# EUROPEAN PATENT APPLICATION

(11) **EP 4 818 168 A1**
(43) Date of publication of application: **07.10.2026**
(21) Application number: 24896793.7
(22) Date of filing: 02.12.2024
(51) Int. Cl.: C07D 487/04, A61K 47/66, A61P 35/00

(54) **FUSED RING COMPOUND, PHARMACEUTICAL COMPOSITION THEREOF AND USE THEREOF**

(30) Priority: 01.12.2023 CN 202311637621; 06.03.2024 CN 202410254237; 13.08.2024 CN 202411106833
(71) Applicant: Evopoint Biosciences Co., Ltd., Suzhou, Jiangsu 215000 (CN)
(72) Inventor: CHEN, Liqun, Suzhou, Jiangsu 215000 (CN); LI, Haoran, Suzhou, Jiangsu 215000 (CN); ZHANG, Xiuchun, Suzhou, Jiangsu 215000 (CN); PENG, Chong, Suzhou, Jiangsu 215000 (CN); WEI, Haiyang, Suzhou, Jiangsu 215000 (CN)
(74) Representative: Hirons, Daniel Stuart
(86) International application number: PCT/CN2024/136230
(87) International publication number: WO 2025/113714

(57) **Abstract**

A fused ring compound, a pharmaceutical composition thereof, and use thereof. Specifically, the present invention relates to a compound represented by formula (I), a pharmaceutically acceptable salt thereof, a solvate thereof, or a solvate of a pharmaceutically acceptable salt thereof. The fused ring compound has excellent inhibitory activity or protein degradation activity on AURKA.

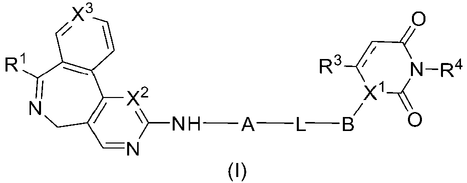

## Description

The present application claims priority to the following Chinese applications:

Chinese Patent Application No. 2023116376216 filed on December 1, 2023;

Chinese Patent Application No. 2024102542376 filed on March 06, 2024;

Chinese Patent Application No. 2024111068336 filed on August 13, 2024.

The entire contents of the above Chinese patent applications are incorporated herein by reference.

### TECHNICAL FIELD

The present invention relates to a fused ring compound, a pharmaceutical composition thereof, and use thereof.

### BACKGROUND

Aurora-A (also known as AURKA, Aurora kinase A) is a serine/threonine kinase that plays an important role in cell mitosis. It is a member of the Aurora Kinases family and participates in the centrosome maturation process, promoting the transition of cells from G2 to mitosis. It is a key mitotic regulator required to maintain chromosomal stability.

Aurora-A is involved in regulating a variety of molecules and signaling pathways by phosphorylating multiple substrates, including p53/p73, p27, PP1, BRCA, Ras, MEK/ERK pathway, PLK1, TPX2, NF-xB pathway, Hippo pathway, P13K/Akt/mTOR pathway, RIPK1/3, MLKL, Wnt/β-catenin, p38 MAPK pathway, etc., thus participating in tumor progression, including tumorigenesis, proliferation, migration, invasion and metastasis, and it is considered to be an oncogene. Aurora-A is overexpressed in a variety of tumors, such as neuroblastoma, lymphoma, breast cancer, colorectal cancer, ovarian cancer and prostate cancer. High levels of Aurora-A have been shown to be associated with tumor proliferation, epithelial-mesenchymal transition, drug resistance and metastasis. Therefore, inhibiting the catalytic activity of Aurora-A is beneficial to inhibiting tumor progression.

The MYC oncogene family consists of three members: MYCC, MYCN and MYCL, which encode c-Myc, N-Myc and L-Myc proteins respectively. They mainly play the role of transcription regulators and their expression in normal tissues is strictly controlled. MYC abnormalities are a driving factor in tumorigenesis. MYC gene mutations are found in about 70% of human malignancies, the most common of which is gene amplification. Inhibiting the expression or function of Myc can reverse carcinogenic processes, including restoring blocked cell cycles, triggering apoptosis, inducing cell senescence, promoting cell differentiation, inhibiting angiogenesis, and upregulating anti-tumor immune responses, thereby reversing tumor occurrence. Aurora-A has the function of stabilizing C-Myc. Studies have found that in liver cancer cells that are dependent on PKA (protein kinase A) or have p53 gene mutations, Aurora-A relies on its structure to directly bind to C-Myc and mediate the stability of C-Myc protein. Some inhibitors can induce changes in the conformation of Aurora-A, thereby interfering with the protein-protein interaction between Aurora-A and C-Myc, affecting the formation of complexes, leading to proteasome degradation of C-Myc and causing cell death, while cells are not sensitive to inhibitors that inhibit the activity of Aurora-A kinase alone. Aurora-A also promotes c-Myc expression as a transactivator in a non-kinase dependent manner. Transcription of the human MYCC gene is mainly initiated by two tandem promoters, P1 and P2. The 283-333 domain of Aurora-A can interact with the KI domain of hnRNP K (MYCC promoter regulatory protein) to form a complex, thereby activating the P1 promoter and promoting c-Myc expression. In addition, Aurora-A can stabilize N-Myc. The proteasome degradation pathway of N-Myc depends on the triggering of the E3 ubiquitin ligase complex SCFFBXW7, while the Aurora-A catalytic domain competes with SCFFBXW7 for binding to the MBI region of N-Myc, which can interfere with the interaction between N-Myc and SCFFBXW7, thereby affecting the degradation of N-Myc. Therefore, eliminating the structurally dependent carcinogenic effect of Aurora-A is conducive to inhibiting tumor progression.

Currently, small molecule inhibitors targeting Aurora-A for cancer therapy are in clinical trials. The known ones include VE465, tozasertib (VX-680), MK-0457, MK-5108, alisertib (MLN-8237), LY3295668, etc., but the treatment effect is unsatisfactory and no related drugs have been approved for marketing. Simultaneous inhibition of Aurora-A's kinase activity and scaffold protein function may improve the treatment effect on related cancers.

PROTAC (proteolysis targeting chimera) is a bifunctional compound whose molecular structure consists of a target protein binding ligand and an E3 ubiquitin ligase ligand; the two ligands are connected by a linker. PROTAC molecules can simultaneously bind to a target protein and an E3 ubiquitin ligase to form a ternary complex. Ubiquitination and labeling of the target protein using the E3 ubiquitin ligase system can induce the degradation of the target protein under the action of the proteasome to achieve the purpose of removing pathogenic proteins. Unlike small molecule kinase inhibitors that can only inhibit the kinase activity of the target protein, PROTAC molecules, by inducing the degradation of the target protein, can not only inhibit its kinase activity, but also eliminate the scaffold protein function of the target protein.

The development of PROTAC molecules that induce Aurora-A degradation and eliminate the promotion effect of Aurora-A enzyme activity and scaffold protein function on tumors is likely to have better tumor inhibition performance than small molecule inhibitors, and has the potential to be used in tumor indications related to Aurora-A activity (head and neck cancer, lung cancer, breast cancer, gastroesophageal adenocarcinoma, uterine leiomyosarcoma, urothelial carcinoma, ovarian cancer, prostate cancer, advanced or metastatic sarcoma, lymphoma, acute myeloid leukemia) and Myc amplification-related tumor indications (colorectal cancer, non-small cell lung cancer, triple-negative breast cancer), becoming a more effective new treatment option.

### SUMMARY OF THE INVENTION

The technical problem to be solved by the present invention is to provide a fused ring compound, a pharmaceutical composition thereof and use thereof in view of the defect that there are insufficient types of AURKA inhibitors or degraders in the prior art. The fused ring compound has excellent inhibitory activity or protein degradation activity on AURKA.

The present invention solves the above technical problems by the following technical solutions.

The present invention provides a compound represented by formula (I), a pharmaceutically acceptable salt thereof, a solvate thereof, or a solvate of the pharmaceutically acceptable salt thereof: 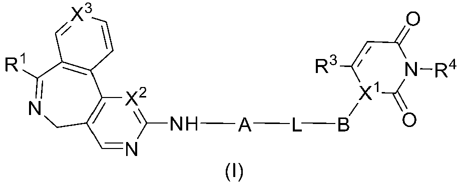 wherein,
" 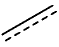 " represents a single bond or a double bond;
X¹ is CH, CD or N;
R³ is H, D, C₁-C₆ alkyl or optionally substituted C₁-C₆ alkyl;
R⁴ is H or C₁-C₆ alkyl;
X² is N, CH or CD;
X³ is CR² or N;
R² is H, D, cyano, halogen, -OR²⁻⁷, -P(=O)(R²⁻¹)₂, -S(=O)₂R²⁻², optionally substituted C₁-C₆ alkyl, optionally substituted C₁-C₆ alkoxy, optionally substituted cycloalkyl or optionally substituted heterocycloalkyl;
R²⁻¹ and R²⁻² are each independently optionally substituted C₁-C₆ alkyl;
R²⁻⁷ is optionally substituted cycloalkyl or optionally substituted heterocycloalkyl;
R¹ is optionally substituted aryl, optionally substituted heteroaryl, optionally substituted cycloalkyl or optionally substituted heterocycloalkyl;
R¹⁻¹ and R¹⁻² are each independently halogen, optionally substituted C₁-C₆ alkyl or optionally substituted C₁-C₆ alkoxy;
A is -R^{A1}C(=O)-, -R^{A2}CH₂-, -R^{A2}CD₂-, -R^{A2}CHD-, optionally substituted arylene, optionally substituted heteroarylene, optionally substituted cycloalkylene or optionally substituted heterocycloalkylene;
R^{A1} and R^{A2} are each independently optionally substituted aryl, optionally substituted heteroarylene, optionally substituted cycloalkylene or optionally substituted heterocycloalkylene;
L is a linker;
B is optionally substituted arylene, optionally substituted heteroarylene, optionally substituted cycloalkylene or optionally substituted heterocycloalkylene;
and the compound represented by formula (I) is not any one of the following compounds:

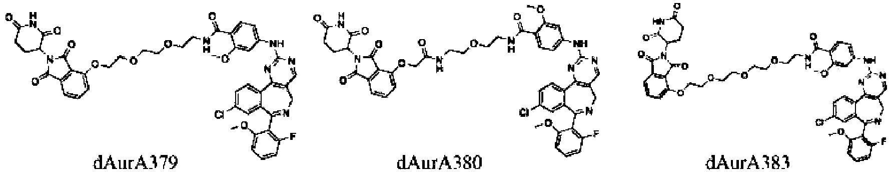

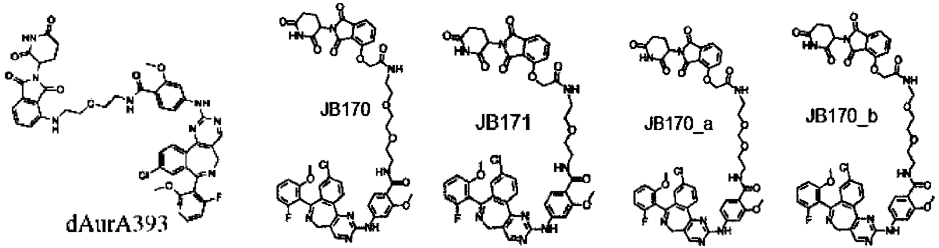

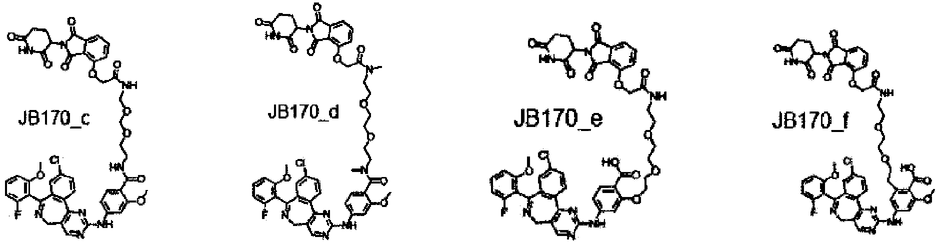

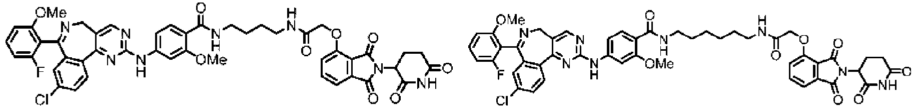

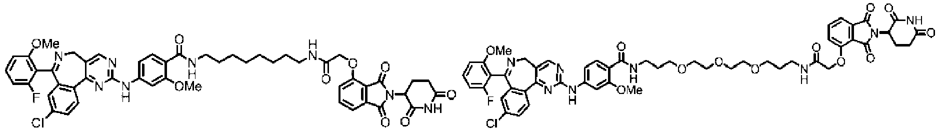

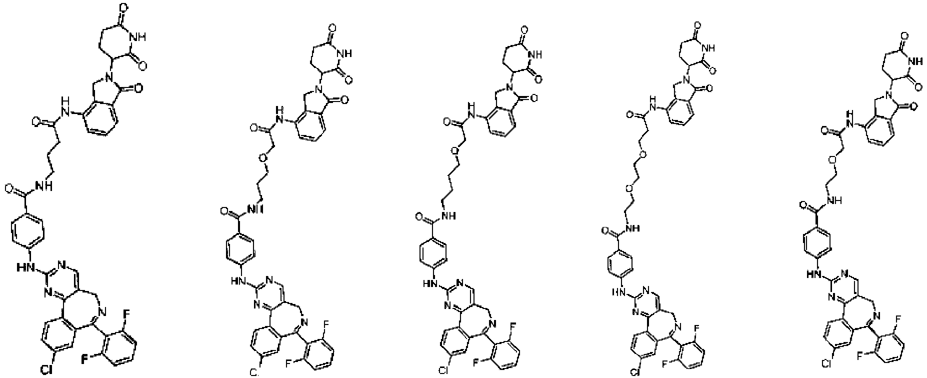

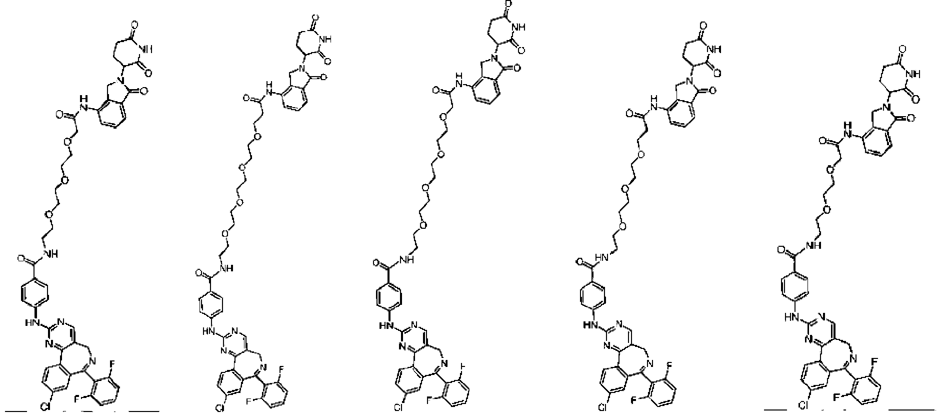

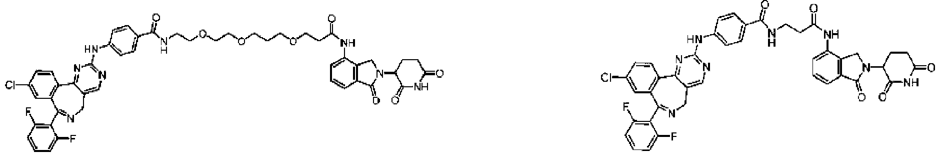

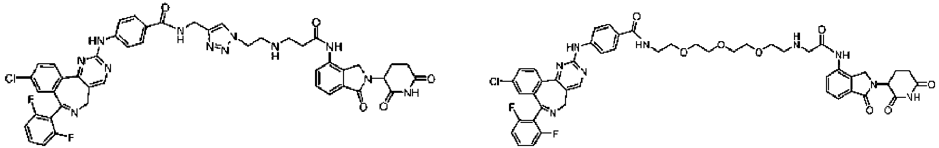

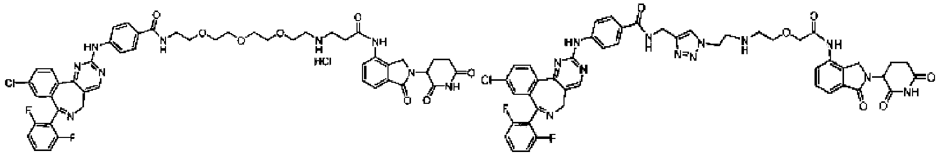

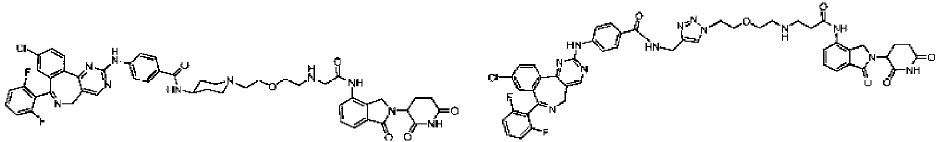

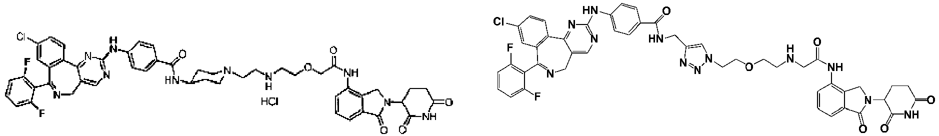

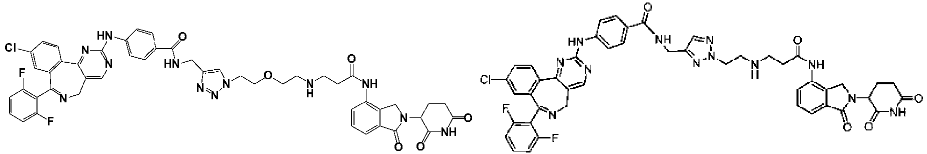

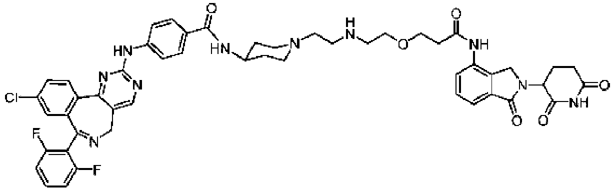

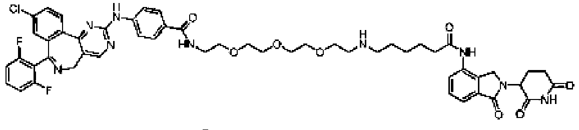

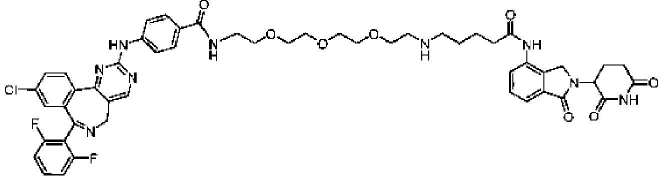

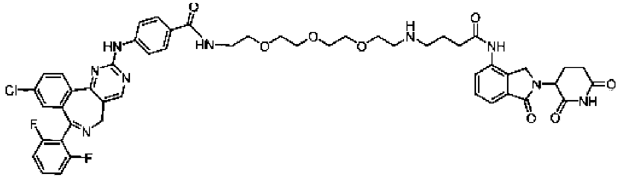

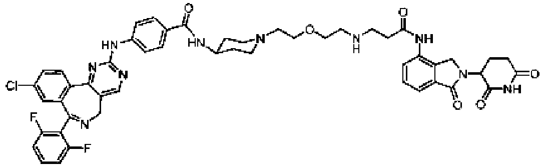

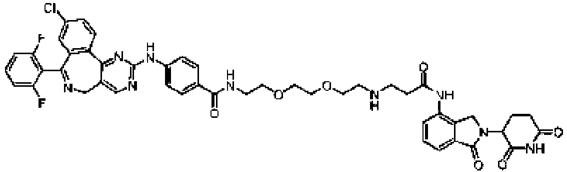

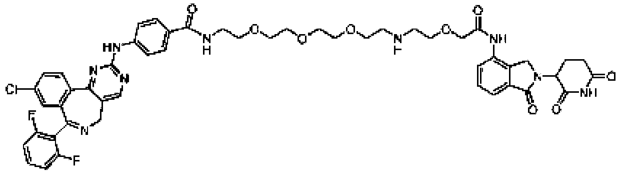

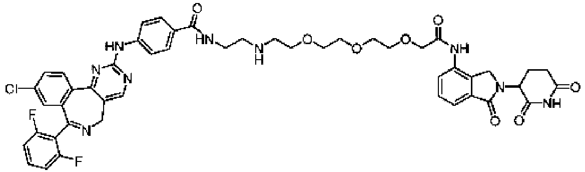

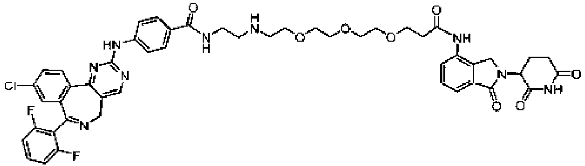

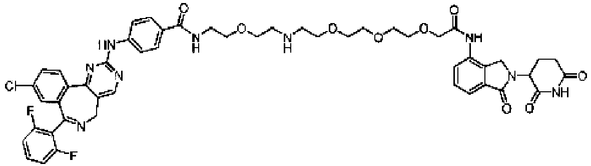

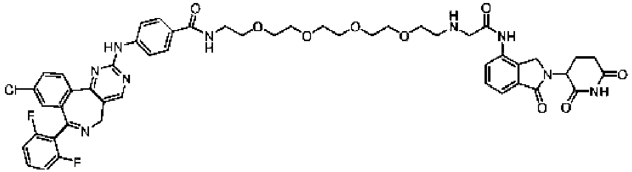

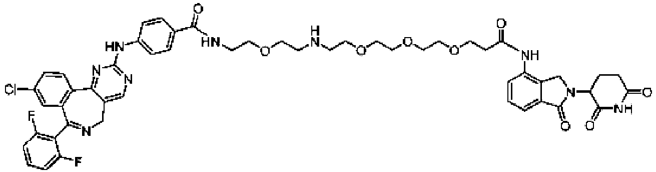

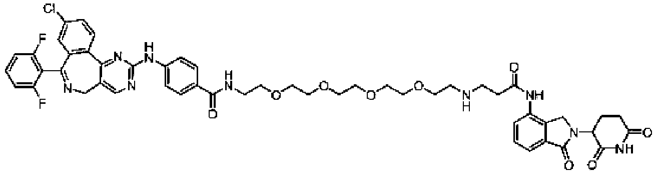

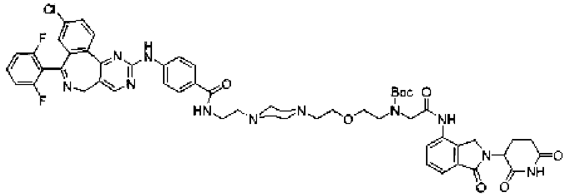

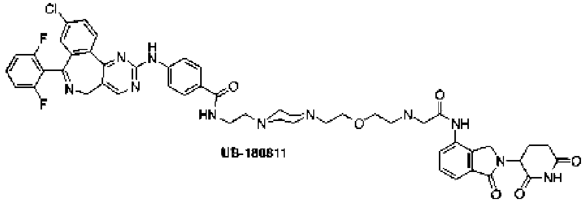

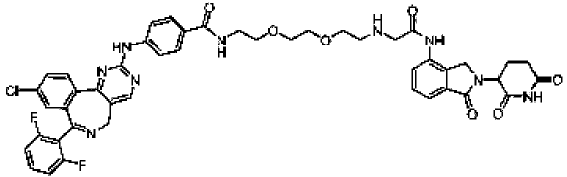

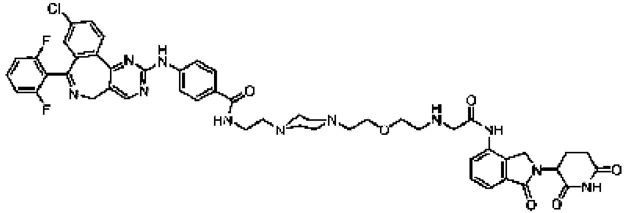

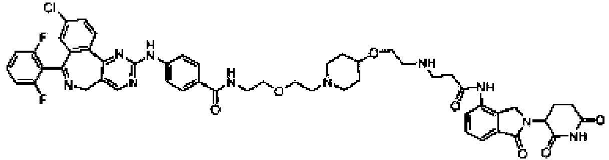

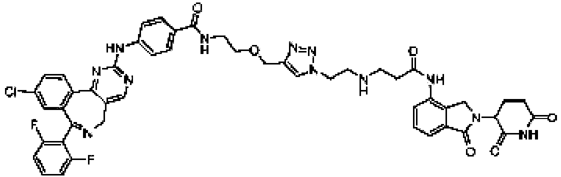

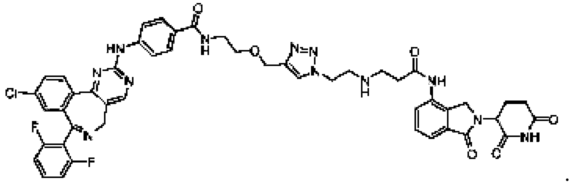

In certain preferred embodiment above of the present invention, certain groups of the compound represented by formula (I), the pharmaceutically acceptable salt thereof, the solvate thereof and the solvate of the pharmaceutically acceptable salt thereof are defined as follows, and groups not mentioned are the same as those described in any embodiment of the present invention (hereinafter referred to as "in one embodiment of the present invention").

In one embodiment of the present invention,
" 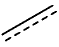 " represents a single bond or a double bond;
X¹ is CH, CD or N;
R³ is H, D, C₁-C₆ alkyl or optionally substituted C₁-C₆ alkyl;
R⁴ is H;
X² is N, CH or CD;
X³ is CR² or N;
R² is H, D, cyano, halogen, -OR²⁻⁷, -P(=O)(R²⁻¹)₂, -S(=O)₂R²⁻², optionally substituted C₁-C₆ alkyl, optionally substituted C₁-C₆ alkoxy, optionally substituted cycloalkyl or optionally substituted heterocycloalkyl;
R²⁻¹ and R²⁻² are each independently optionally substituted C₁-C₆ alkyl;
R²⁻⁷ is optionally substituted cycloalkyl or optionally substituted heterocycloalkyl;
R¹ is optionally substituted aryl, optionally substituted heteroaryl, optionally substituted cycloalkyl or optionally substituted heterocycloalkyl;
R¹⁻¹ and R¹⁻² are each independently halogen, optionally substituted C₁-C₆ alkyl or optionally substituted C₁-C₆ alkoxy;
A is -R^{A1}C(=O)-, -R^{A2}CH₂-, -R^{A2}CD₂-, -R^{A2}CHD-, optionally substituted arylene, optionally substituted heteroarylene, optionally substituted cycloalkylene or optionally substituted heterocycloalkylene;
R^{A1} and R^{A2} are each independently optionally substituted aryl, optionally substituted heteroarylene, optionally substituted cycloalkylene or optionally substituted heterocycloalkylene;
L is a linker;
B is optionally substituted arylene, optionally substituted heteroarylene, optionally substituted cycloalkylene or optionally substituted heterocycloalkylene;
and the compound represented by formula (I) is not any one of the following compounds:

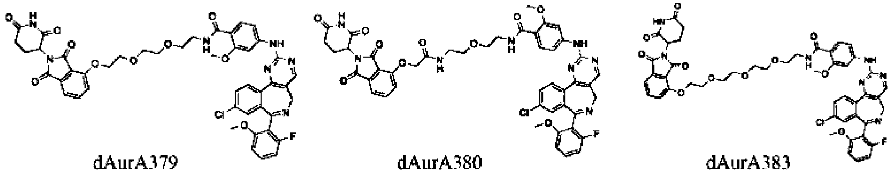

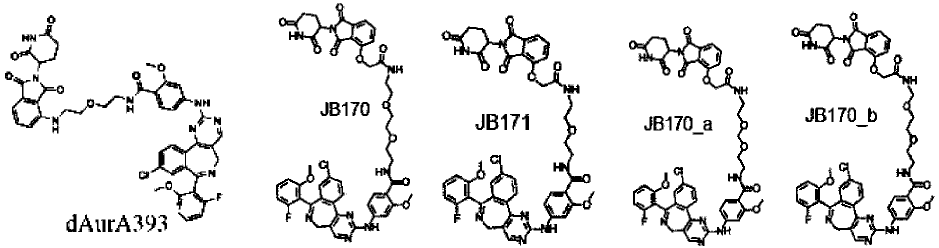

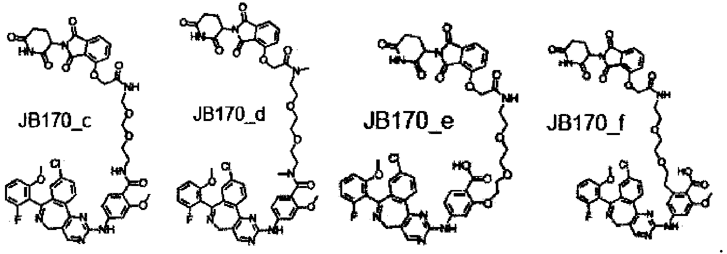

In one embodiment of the present invention,
" 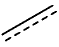 " represents a single bond or a double bond;
X¹ is CH, CD or N;
R³ is H, D, C₁-C₆ alkyl or optionally substituted C₁-C₆ alkyl;
R⁴ is H;
X² is N, CH or CD;
X³ is CR² or N;
R² is H, D, halogen, -OR²⁻⁷, -P(=O)(R²⁻¹)₂, -S(=O)₂R²⁻², optionally substituted C₁-C₆ alkyl, optionally substituted C₁-C₆ alkoxy, optionally substituted cycloalkyl or optionally substituted heterocycloalkyl;
R²⁻¹ and R²⁻² are each independently optionally substituted C₁-C₆ alkyl;
R²⁻⁷ is optionally substituted cycloalkyl or optionally substituted heterocycloalkyl;
R¹ is optionally substituted aryl, optionally substituted heteroaryl, optionally substituted cycloalkyl or optionally substituted heterocycloalkyl;
R¹⁻¹ and R¹⁻² are each independently halogen, optionally substituted C₁-C₆ alkyl or optionally substituted C₁-C₆ alkoxy;
A is -R^{A1}C(=O)-, -R^{A2}CH₂-, -R^{A2}CD₂-, -R^{A2}CHD-, optionally substituted arylene, optionally substituted heteroarylene, optionally substituted cycloalkylene or optionally substituted heterocycloalkylene;
R^{A1} and R^{A2} are each independently optionally substituted aryl, optionally substituted heteroarylene, optionally substituted cycloalkylene or optionally substituted heterocycloalkylene;
L is a linker;
B is optionally substituted arylene, optionally substituted heteroarylene, optionally substituted cycloalkylene or optionally substituted heterocycloalkylene;
and the compound represented by formula (I) is not any one of the following compounds:

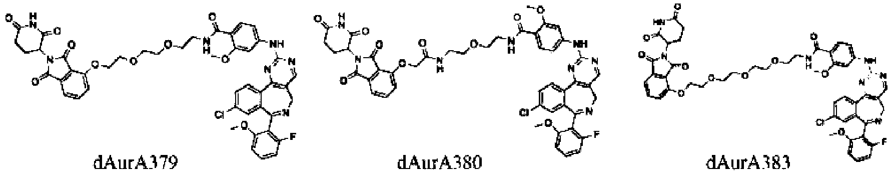

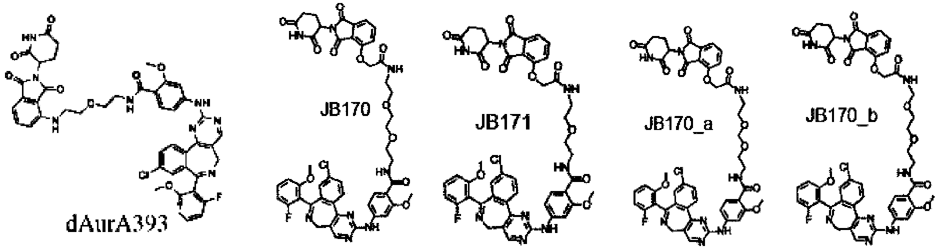

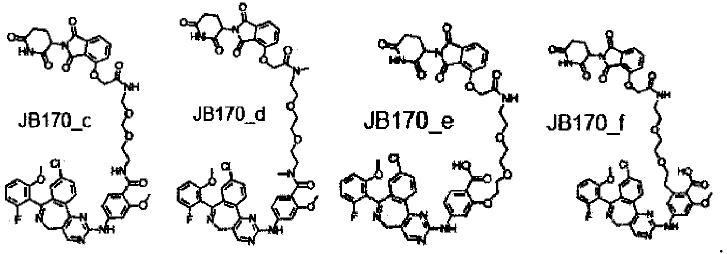

In one embodiment of the present invention,
" 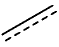 " represents a single bond or a double bond;
X¹ is CH or N;
R³ is H or C₁-C₆ alkyl;
R⁴ is H or C₁-C₆ alkyl;
X² is N or CH;
X³ is CR² or N;
R² is H, cyano, halogen, C₁-C₆ alkyl, C₁-C₆ alkoxy, saturated C₃-C₁₁ cycloalkyl, saturated 3- to 11-membered heterocycloalkyl, -P(=O)(R²⁻¹)₂, -S(=O)₂R²⁻², -OR²⁻⁷, C₁-C₆ alkyl substituted with one or more R²⁻³, C₁-C₆ alkoxy substituted with one or more R²⁻⁴, saturated C₃-C₁₁ cycloalkyl substituted with one or more R²⁻⁵ or saturated 3- to 11-membered heterocycloalkyl substituted with one or more R²⁻⁶, wherein the saturated 3-to 11-membered heterocycloalkyl and the 3- to 11-membered heterocycloalkyl in the saturated 3- to 11-membered heterocycloalkyl substituted with one or more R²⁻⁶ each independently have 1, 2 or 3 heteroatoms selected from one or more of N, O, S and C(=O);
R²⁻¹ and R²⁻² are each independently C₁-C₆ alkyl;
R²⁻⁷ is saturated C₃-C₁₁ cycloalkyl;
R²⁻³ and R²⁻⁴ are each independently -N(R²⁻³⁻¹)₂, saturated C₃-C₁₁ cycloalkyl, saturated 3- to 11-membered heterocycloalkyl, saturated C₃-C₁₁ cycloalkyl substituted with one or more R²⁻³⁻², or saturated 3- to 11-membered heterocycloalkyl substituted with one or more R²⁻³⁻³, wherein the saturated 3- to 11-membered heterocycloalkyl and the 3- to 11-membered heterocycloalkyl in the saturated 3- to 11-membered heterocycloalkyl substituted with one or more R²⁻³⁻³ each independently have 1, 2 or 3 heteroatoms selected from one or more of N, O, S and C(=O);
R²⁻³⁻¹ is independently H or C₁-C₆ alkyl;
R²⁻³⁻² and R²⁻³⁻³ are each independently C₁-C₆ alkyl;
R²⁻⁵ and R²⁻⁶ are each independently C₁-C₆ alkyl;
R¹ is C₆-C₁₀ aryl, 5- to 10-membered heteroaryl, saturated C₃-C₁₁ cycloalkyl, saturated 3- to 11-membered heterocycloalkyl, C₆-C₁₀ aryl substituted with one or more R¹⁻¹, 5- to 10-membered heteroaryl substituted with one or more R¹⁻², saturated C₃-C₁₁ cycloalkyl substituted with one or more R¹⁻³, or saturated 3- to 11-membered heterocycloalkyl substituted with one or more R¹⁻⁴, wherein the saturated 3- to 11-membered heterocycloalkyl has 1, 2 or 3 heteroatoms selected from one or more of N, O, S and C(=O), and the 5- to 10-membered heteroaryl and the 5- to 10-membered heteroaryl in the 5- to 10-membered heteroaryl substituted with one or more R¹⁻² each independently have 1, 2 or 3 heteroatoms selected from one or more of N, O and S;
R¹⁻¹, R¹⁻², R¹⁻³ and R¹⁻⁴ are each independently halogen, C₁-C₆ alkyl or C₁-C₆ alkoxy;
A is -R^{A1}C(=O)-, -R^{A2}CH₂-, C₆-C₁₀ arylene, 5- to 10-membered heteroarylene, saturated C₃-C₁₁ cycloalkylene, saturated 3- to 11-membered heterocycloalkylene, 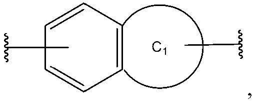 C₆-C₁₀ arylene substituted with one or more R^{A3}, or 5- to 10-membered heteroarylene substituted with one or more R^{A4}, wherein the saturated 3- to 11-membered heterocycloalkylene has 1, 2 or 3 heteroatoms selected from one or more of N, O, S and C(=O) and the 5- to 10-membered heteroarylene and the 5- to 10-membered heteroarylene in the 5- to 10-membered heteroarylene substituted with one or more R^{A4} each independently have 1, 2 or 3 heteroatoms selected from one or more of N, O and S;
R^{A1} and R^{A2} are each independently C₆-C₁₀ arylene, 5- to 10-membered heteroarylene, saturated C₃-C₁₁ cycloalkylene, saturated 3- to 11-membered heterocycloalkylene, C₆-C₁₀ arylene substituted with one or more R^{A1-1}, or 5- to 10-membered heteroarylene substituted with one or more R^{A1-2}, wherein the 5- to 10-membered heteroarylene and the 5- to 10-membered heteroarylene in the 5- to 10-membered heteroarylene substituted with one or more R^{A1-2} each independently have 1, 2 or 3 heteroatoms selected from one or more of N, O and S, and the 3- to 11-membered heterocycloalkylene has 1, 2 or 3 heteroatoms selected from one or more of N, O, S and C(=O);
R^{A1-1} and R^{A1-2} are each independently CN, -P(=O)(R^{A3-1})₂, C₁-C₆ alkyl or C₁-C₆ alkoxy;
ring C₁ is a partially unsaturated 3- to 8-membered heterocycle or a partially unsaturated 3- to 8-membered heterocycle substituted with one or more R^{C1}, wherein the partially unsaturated 3- to 8-membered heterocycle and the 3- to 8-membered heterocycle in the partially unsaturated 3- to 8-membered heterocycle substituted with one or more R^{C1} each independently have 1, 2 or 3 heteroatoms selected from one or more of N, O, S and C(=O);
R^{C1} is independently C₁-C₆ alkyl;
R^{A3} and R^{A4} are each independently CN, -P(=O)(R^{A3-1})₂, C₁-C₆ alkyl or C₁-C₆ alkoxy;
R^{A3-1} is independently C₁-C₆ alkyl;
L is -(CH₂)ₙ-, wherein n is any integer from 1 to 18, and 1 or more of the -(CH₂)- in -(CH₂)ₙ- are replaced by one or more of the following groups: -NR^{L1}-, -O-, -S-, -CR^{L6}R^{L7}-, -C(=O)-, 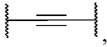 saturated C₃-C₁₂ cycloalkylene, saturated 3- to 12-membered heterocycloalkylene, C₆-C₁₀ arylene, 5- to 10-membered heteroarylene, 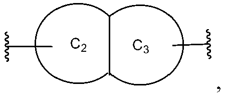 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4} or 5- to 10-membered heteroarylene substituted with one or more R^{L5}, wherein the saturated 3- to 12-membered heterocycloalkylene and the 3- to 12-membered heterocycloalkyl in the saturated 3- to 12-membered heterocycloalkylene substituted with one or more R^{L3} each independently have 1, 2 or 3 heteroatoms selected from one or more of N, O, S and C(=O) and the 5- to 10-membered heteroarylene and the 5- to 10-membered heteroarylene in the 5- to 10-membered heteroarylene substituted with one or more R^{L5} each independently have 1, 2 or 3 heteroatoms selected from one or more of N, O and S;
R^{L1} is H or C₁-C₆ alkyl;
ring C₂ is benzene ring or 5- to 6-membered heteroaromatic ring, wherein the 5- to 6-membered heteroaromatic ring has 1, 2 or 3 heteroatoms selected from one or more of N, O and S;
ring C₃ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
ring C₄ is a saturated C₃-C₇ carbon ring or a saturated 3- to 7-membered heterocycle, wherein the saturated 3- to 7-membered heterocycle has 1, 2 or 3 heteroatoms selected from one or more of N, O, S and C(=O);
R^{L2} and R^{L3} are each independently CN, OH, halogen, C₁-C₆ alkyl, C₃-C₇ cycloalkyl or 3- to 7-membered heterocycloalkyl;
R^{L4} and R^{L5} are each independently C₁-C₆ alkyl;
R^{L6} is H or C₁-C₆ alkyl;
R^{L7} is C₁-C₆ alkyl;
B is 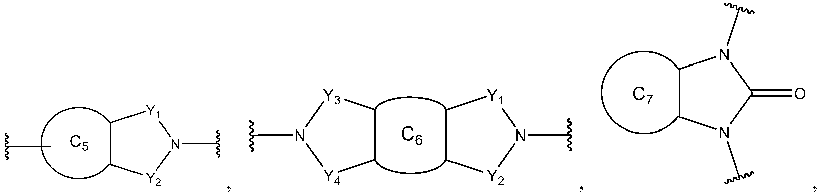 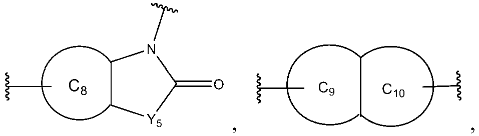 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1}, or 5- to 10-membered heteroarylene substituted with one or more R^{B2}, wherein the 5-to 10-membered heteroarylene and the 5- to 10-membered heteroarylene in the 5- to 10-membered heteroarylene substituted with one or more R^{B2} each independently have 1, 2 or 3 heteroatoms selected from one or more of N, O and S, and the saturated 3- to 12-membered heterocycloalkylene independently has 1, 2 or 3 heteroatoms selected from one or more of N, O, S and C(=O);
Y₁. Y₂, Y₃ and Y₄ are each independently CH₂ or C(=O);
Y₅ is NY⁵⁻¹, O or S, and Y⁵⁻¹ is H or C₁-C₆ alkyl;
ring C₅, ring C₆, ring C₇, ring C₈ and ring C₉ are each independently a benzene ring or a 5- to 6-membered heteroaromatic ring, wherein the 5- to 6-membered heteroaromatic ring has 1, 2 or 3 heteroatoms selected from one or more of N, O and S;
ring C₁₀ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
R^{B1} and R^{B2} are each independently halogen, cyano, -P=O(C₁-C₆ alkyl)₂, -C(=O)NH₂, C₁-C₆ alkyl, C₁-C₆ alkoxy, C₁-C₆ alkyl substituted with one or more R^{B1-1}, or C₁-C₆ alkoxy substituted with one or more R^{B1}-²;
R^{B1-1} and R^{B1-2} are each independently halogen;
moreover, the compound represented by formula (I) satisfies one or both of the following conditions:
   i: B is 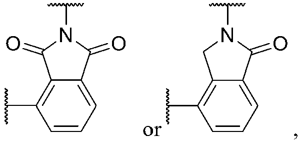 L is -(CH₂)ₙ-, wherein n is any integer from 1 to 18, and
      at least one -(CH₂)- in -(CH₂)ₙ- is replaced by the following groups: saturated C₃-C₁₂ cycloalkylene, saturated 3- to 5-membered heterocycloalkylene, saturated 7- to 12-membered heterocycloalkylene, C₆-C₁₀ arylene, 6- to 10-membered heteroarylene, 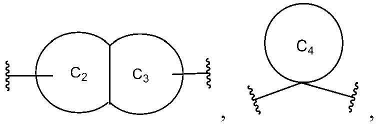 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4}, or 5- to 10-membered heteroarylene substituted with one or more R^{L5};
      alternatively, at least 2 -(CH₂)- of -(CH₂)n- are replaced by the following groups: saturated C₃-C₁₂ cycloalkylene, saturated 3- to 12-membered heterocycloalkylene, C₆-C₁₀ arylene, 5- to 10-membered heteroarylene, 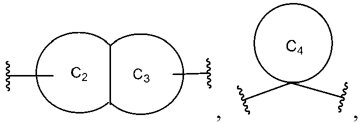 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4}, or 5- to 10-membered heteroarylene substituted with one or more R^{L5};
   ii: B is 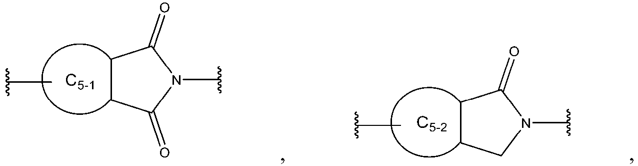 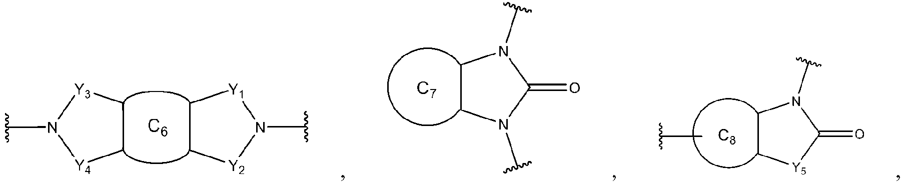 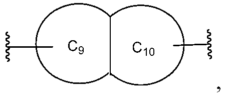 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1} or 5- to 10-membered heteroarylene substituted with one or more R^{B2}, ring C₅₋₁ same as the 5- to 6-membered heteroaromatic ring in ring C₅, and ring C₅₋₂ same as the 5- to 6-membered heteroaromatic ring in ring C₅.

In one embodiment of the present invention,
" 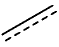 " represents a single bond or a double bond;
X¹ is CH or N;
R³ is H or C₁-C₆ alkyl;
R⁴ is H;
X² is N or CH;
X³ is CR² or N;
R² is H, cyano, halogen, C₁-C₆ alkyl, C₁-C₆ alkoxy, saturated C₃-C₁₁ cycloalkyl, saturated 3- to 11-membered heterocycloalkyl, -P(=O)(R²⁻¹)₂, -S(=O)₂R²⁻², -OR²⁻⁷, C₁-C₆ alkyl substituted with one or more R²⁻³, C₁-C₆ alkoxy substituted with one or more R²⁻⁴, saturated C₃-C₁₁ cycloalkyl substituted with one or more R²⁻⁵ or saturated 3- to 11-membered heterocycloalkyl substituted with one or more R²⁻⁶, wherein the saturated 3-to 11-membered heterocycloalkyl and the 3- to 11-membered heterocycloalkyl in the saturated 3- to 11-membered heterocycloalkyl substituted with one or more R²⁻⁶ each independently have 1, 2 or 3 heteroatoms selected from one or more of N, O, S and C(=O);
R²⁻¹ and R²⁻² are each independently C₁-C₆ alkyl;
R²⁻⁷ is saturated C₃-C₁₁ cycloalkyl;
R²⁻³ and R²⁻⁴ are each independently -N(R²⁻³⁻¹)₂, saturated C₃-C₁₁ cycloalkyl, saturated 3- to 11-membered heterocycloalkyl, saturated C₃-C₁₁ cycloalkyl substituted with one or more R²⁻³⁻², or saturated 3- to 11-membered heterocycloalkyl substituted with one or more R²⁻³⁻³, wherein the saturated 3- to 11-membered heterocycloalkyl and the 3- to 11-membered heterocycloalkyl in the saturated 3- to 11-membered heterocycloalkyl substituted with one or more R²⁻³⁻³ each independently have 1, 2 or 3 heteroatoms selected from one or more of N, O, S and C(=O);
R²⁻³⁻¹ is independently H or C₁-C₆ alkyl;
R²⁻³⁻² and R²⁻³⁻³ are each independently C₁-C₆ alkyl;
R²⁻⁵ and R²⁻⁶ are each independently C₁-C₆ alkyl;
R¹ is C₆-C₁₀ aryl, 5- to 10-membered heteroaryl, saturated C₃-C₁₁ cycloalkyl, saturated 3- to 11-membered heterocycloalkyl, C₆-C₁₀ aryl substituted with one or more R¹⁻¹, or 5- to 10-membered heteroaryl substituted with one or more R¹⁻², wherein the saturated 3- to 11-membered heterocycloalkyl has 1, 2 or 3 heteroatoms selected from one or more of N, O, S and C(=O), and the 5- to 10-membered heteroaryl and the 5- to 10-membered heteroaryl in the 5- to 10-membered heteroaryl substituted with one or more R¹⁻² each independently have 1, 2 or 3 heteroatoms independently selected from one or more of N, O and S;
R¹⁻¹ and R¹⁻² are each independently halogen, C₁-C₆ alkyl or C₁-C₆ alkoxy;
A is -R^{A1}C(=O)-, -R^{A2}CH₂-, C₆-C₁₀ arylene, 5- to 10-membered heteroarylene, saturated C₃-C₁₁ cycloalkylene, saturated 3- to 11-membered heterocycloalkylene, 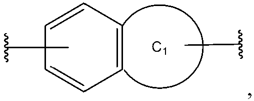 C₆-C₁₀ arylene substituted with one or more R^{A3}, or 5- to 10-membered heteroarylene substituted with one or more R^{A4}, wherein the saturated 3- to 11-membered heterocycloalkylene has 1, 2 or 3 heteroatoms selected from one or more of N, O, S and C(=O) and the 5- to 10-membered heteroarylene and the 5- to 10-membered heteroarylene in the 5- to 10-membered heteroarylene substituted with one or more R^{A4} each independently have 1, 2 or 3 heteroatoms independently selected from one or more of N, O and S;
R^{A1} and R^{A2} are each independently C₆-C₁₀ arylene, 5- to 10-membered heteroarylene, saturated C₃-C₁₁ cycloalkylene, saturated 3- to 11-membered heterocycloalkylene, C₆-C₁₀ arylene substituted with one or more R^{A1-1}, or 5- to 10-membered heteroarylene substituted with one or more R^{A1-2}, wherein the 5- to 10-membered heteroarylene and the 5- to 10-membered heteroarylene in the 5- to 10-membered heteroarylene substituted with one or more R^{A1-2} each independently have 1, 2 or 3 heteroatoms selected from one or more of N, O and S, and the 3- to 11-membered heterocycloalkylene has 1, 2 or 3 heteroatoms selected from one or more of N, O, S and C(=O);
R^{A1-1} and R^{A1-2} are each independently CN, -P(=O)(R^{A3-1})₂, C₁-C₆ alkyl or C₁-C₆ alkoxy;
ring C₁ is a partially unsaturated 3- to 8-membered heterocycle or a partially unsaturated 3- to 8-membered heterocycle substituted with one or more R^{C1}, wherein the partially unsaturated 3- to 8-membered heterocycle and the 3- to 8-membered heterocycle in the partially unsaturated 3- to 8-membered heterocycle substituted with one or more R^{C1} each independently have 1, 2 or 3 heteroatoms selected from one or more of N, O, S and C(=O);
R^{C1} is independently C₁-C₆ alkyl;
R^{A3} and R^{A4} are each independently CN, -P(=O)(R^{A3-1})₂, C₁-C₆ alkyl or C₁-C₆ alkoxy;
R^{A3-1} is independently C₁-C₆ alkyl;
L is -(CH₂)n-, wherein n is any integer from 1 to 18, and 1 or more of the -(CH₂)- in -(CH₂)ₙ- are replaced by one or more of the following groups: -NR^{L1}-, -O-, -S-, -C(=O)-, 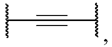 saturated C₃-C₁₂ cycloalkylene, saturated 3- to 12-membered heterocycloalkylene, C₆-C₁₀ arylene, 5- to 10-membered heteroarylene, 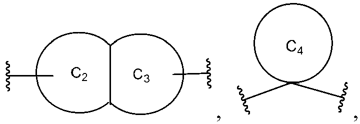 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4} or 5- to 10-membered heteroarylene substituted with one or more R^{L5}, wherein the saturated 3- to 12-membered heterocycloalkylene and the 3- to 12-membered heterocycloalkyl in the saturated 3- to 12-membered heterocycloalkylene substituted with one or more R^{L3} each independently have 1, 2 or 3 heteroatoms independently selected from one or more of N, O, S and C(=O) and the 5- to 10-membered heteroarylene and the 5- to 10-membered heteroarylene in the 5- to 10-membered heteroarylene substituted with one or more R^{L5} each independently have 1, 2 or 3 heteroatoms independently selected from one or more of N, O and S;
R^{L1} is H or C₁-C₆ alkyl;
ring C₂ is benzene ring or 5- to 6-membered heteroaromatic ring, wherein the 5- to 6-membered heteroaromatic ring has 1, 2 or 3 heteroatoms selected from one or more of N, O and S;
ring C₃ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
ring C₄ is a saturated C₃-C₇ carbon ring or a saturated 3- to 7-membered heterocycle, wherein the saturated 3- to 7-membered heterocycle has 1, 2 or 3 heteroatoms selected from one or more of N, O, S and C(=O);
R^{L2} and R^{L3} are each independently CN, OH, halogen, C₁-C₆ alkyl, C₃-C₇ cycloalkyl or 3- to 7-membered heterocycloalkyl;
R^{L4} and R^{L5} are each independently C₁-C₆ alkyl;
B is 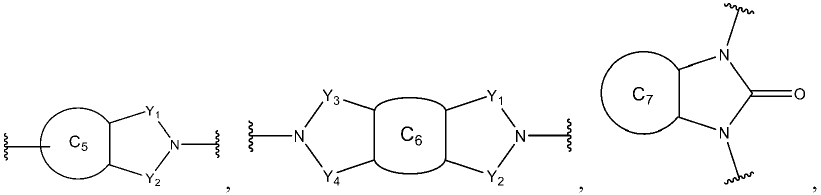 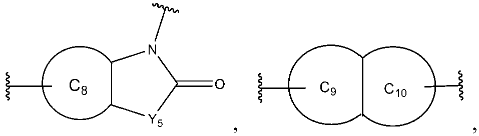 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1}, or 5- to 10-membered heteroarylene substituted with one or more R^{B2}, wherein the 5-to 10-membered heteroarylene and the 5- to 10-membered heteroarylene in the 5- to 10-membered heteroarylene substituted with one or more R^{B2} each independently have 1, 2 or 3 heteroatoms selected from one or more of N, O and S, and the saturated 3- to 12-membered heterocycloalkylene independently has 1, 2 or 3 heteroatoms selected from one or more of N, O, S and C(=O);
Y₁. Y₂, Y₃ and Y₄ are each independently CH₂ or C(=O);
Y₅ is NY⁵⁻¹, O or S, and Y⁵⁻¹ is H or C₁-C₆ alkyl;
ring C₅, ring C₆, ring C₇, ring C₈ and ring C₉ are each independently a benzene ring or a 5- to 6-membered heteroaromatic ring, wherein the 5- to 6-membered heteroaromatic ring has 1, 2 or 3 heteroatoms selected from one or more of N, O and S;
ring C₁₀ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
R^{B1} and R^{B2} are each independently halogen, cyano, -P=O(CH₃)₂, C₁-C₆ alkyl, C₁-C₆ alkoxy, C₁-C₆ alkyl substituted with one or more R^{B1-1}, or C₁-C₆ alkoxy substituted with one or more R^{B1-2};
R^{B1-1} and R^{B1-2} are each independently halogen;
moreover, the compound represented by formula (I) satisfies one or both of the following conditions:
   i: B is 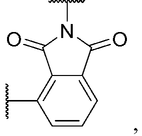 L is -(CH₂)ₙ-, n is any integer from 1 to 18, and at least 1 of -(CH₂)- in - (CH₂)ₙ- is replaced by the following groups: saturated C₃-C₁₂ cycloalkylene, saturated 3- to 12-membered heterocycloalkylene, C₆-C₁₀ arylene, 5- to 10-membered heteroarylene, 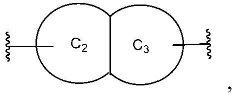 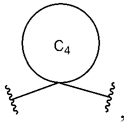 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4}, or 5-to 10-membered heteroarylene substituted with one or more R^{L5};
   ii: B is 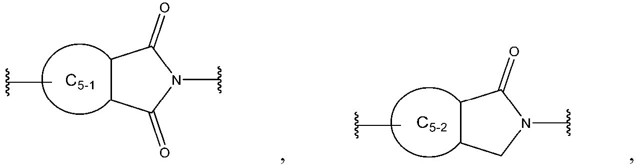 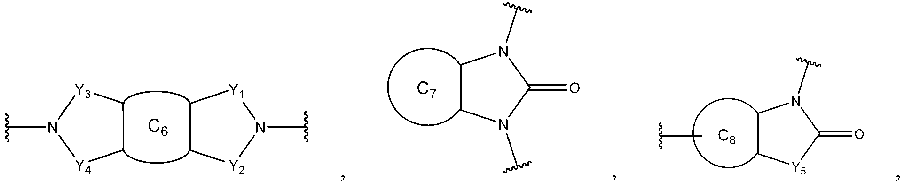 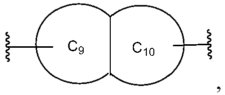 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1} or 5- to 10-membered heteroarylene substituted with one or more R^{B2}, ring C₅₋₁ same as the 5- to 6-membered heteroaryl ring in ring C5, and ring C₅₋₂ same as ring C₅.

In one embodiment of the present invention,
" 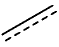 " represents a single bond or a double bond;
X¹ is CH or N;
R³ is H or C₁-C₆ alkyl;
R⁴ is H;
X² is N or CH;
X³ is CR² or N;
R² is H, halogen, C₁-C₆ alkyl, C₁-C₆ alkoxy, saturated C₃-C₁₁ cycloalkyl, saturated 3- to 11-membered heterocycloalkyl, -P(=O)(R²⁻¹)₂, -S(=O)₂R²⁻², -OR²⁻⁷, C₁-C₆ alkyl substituted with one or more R²⁻³, C₁-C₆ alkoxy substituted with one or more R²⁻⁴, saturated C₃-C₁₁ cycloalkyl substituted with one or more R²⁻⁵ or saturated 3- to 11-membered heterocycloalkyl substituted with one or more R²⁻⁶, wherein the saturated 3-to 11-membered heterocycloalkyl and the 3- to 11-membered heterocycloalkyl in the saturated 3- to 11-membered heterocycloalkyl substituted with one or more R²⁻⁶ each independently have 1, 2 or 3 heteroatoms independently selected from one or more of N, O, S and C(=O);
R²⁻¹ and R²⁻² are each independently C₁-C₆ alkyl;
R²⁻⁷ is saturated C₃-C₁₁ cycloalkyl;
R²⁻³ and R²⁻⁴ are each independently -N(R²⁻³⁻¹)₂, saturated C₃-C₁₁ cycloalkyl, saturated 3- to 11-membered heterocycloalkyl, saturated C₃-C₁₁ cycloalkyl substituted with one or more R²⁻³⁻², or saturated 3- to 11-membered heterocycloalkyl substituted with one or more R²⁻³⁻³, wherein the saturated 3- to 11-membered heterocycloalkyl and the 3- to 11-membered heterocycloalkyl in the saturated 3- to 11-membered heterocycloalkyl substituted with one or more R²⁻³⁻³ each independently have 1, 2 or 3 heteroatoms selected from one or more of N, O, S and C(=O);
R²⁻³⁻¹ is independently H or C₁-C₆ alkyl;
R²⁻³⁻² and R²⁻³⁻³ are each independently C₁-C₆ alkyl;
R²⁻⁵ and R²⁻⁶ are each independently C₁-C₆ alkyl;
R¹ is C₆-C₁₀ aryl, 5- to 10-membered heteroaryl, saturated C₃-C₁₁ cycloalkyl, saturated 3- to 11-membered heterocycloalkyl, C₆-C₁₀ aryl substituted with one or more R¹⁻¹, or 5- to 10-membered heteroaryl substituted with one or more R¹⁻², wherein the saturated 3- to 11-membered heterocycloalkyl has 1, 2 or 3 heteroatoms selected from one or more of N, O, S and C(=O), and the 5- to 10-membered heteroaryl and the 5- to 10-membered heteroaryl in the 5- to 10-membered heteroaryl substituted with one or more R¹⁻² each independently have 1, 2 or 3 heteroatoms independently selected from one or more of N, O and S;
R¹⁻¹ and R¹⁻² are each independently halogen, C₁-C₆ alkyl or C₁-C₆ alkoxy;
A is -R^{A1}C(=O)-, -R^{A2}CH₂-, C₆-C₁₀ arylene, 5- to 10-membered heteroarylene, saturated C₃-C₁₁ cycloalkylene, saturated 3- to 11-membered heterocycloalkylene, 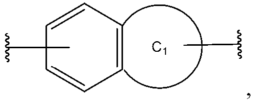 C₆-C₁₀ arylene substituted with one or more R^{A3,} or 5- to 10-membered heteroarylene substituted with one or more R^{A4}, wherein the saturated 3- to 11-membered heterocycloalkylene has 1, 2 or 3 heteroatoms selected from one or more of N, O, S and C(=O) and the 5- to 10-membered heteroarylene and the 5- to 10-membered heteroarylene in the 5- to 10-membered heteroarylene substituted with one or more R^{A4} each independently have 1, 2 or 3 heteroatoms selected from one or more of N, O and S;
R^{A1} and R^{A2} are each independently C₆-C₁₀ arylene, 5- to 10-membered heteroarylene, saturated C₃-C₁₁ cycloalkylene, saturated 3- to 11-membered heterocycloalkylene, C₆-C₁₀ arylene substituted with one or more R^{A1-1}, or 5- to 10-membered heteroarylene substituted with one or more R^{A1-2}, wherein the 5- to 10-membered heteroarylene and the 5- to 10-membered heteroarylene in the 5- to 10-membered heteroarylene substituted with one or more R^{A1-2} each independently have 1, 2 or 3 heteroatoms selected from one or more of N, O and S, and the 3- to 11-membered heterocycloalkylene has 1, 2 or 3 heteroatoms selected from one or more of N, O, S and C(=O);
R^{A1-1} and R^{A1-2} are each independently CN, -P(=O)(R^{A3-1})₂, C₁-C₆ alkyl or C₁-C₆ alkoxy;
ring C₁ is a partially unsaturated 3- to 8-membered heterocycle or a partially unsaturated 3- to 8-membered heterocycle substituted with one or more R^{C1}, wherein the partially unsaturated 3- to 8-membered heterocycle and the 3- to 8-membered heterocycle in the partially unsaturated 3- to 8-membered heterocycle substituted with one or more R^{C1} each independently have 1, 2 or 3 heteroatoms selected from one or more of N, O, S and C(=O);
R^{C1} is independently C₁-C₆ alkyl;
R^{A3} and R^{A4} are each independently CN, -P(=O)(R^{A3-1})₂, C₁-C₆ alkyl or C₁-C₆ alkoxy;
R^{A3-1} is independently C₁-C₆ alkyl;
L is -(CH₂)ₙ-, wherein n is any integer from 1 to 18, and 1 or more of the -(CH₂)- in -(CH₂)ₙ- are replaced by one or more of the following groups: -NR^{L1}-, -O-, -S-, -C(=O)-, 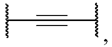 saturated C₃-C₁₂ cycloalkylene, saturated 3- to 12-membered heterocycloalkylene, C₆-C₁₀ arylene, 5- to 10-membered heteroarylene, 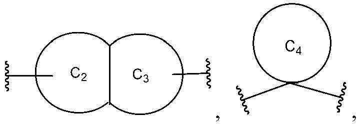 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4} or 5- to 10-membered heteroarylene substituted with one or more R^{L5}, wherein the saturated 3- to 12-membered heterocycloalkylene and the 3- to 12-membered heterocycloalkyl in the saturated 3- to 12-membered heterocycloalkylene substituted with one or more R^{L3} each independently have 1, 2 or 3 heteroatoms selected from one or more of N, O, S and C(=O) and the 5- to 10-membered heteroarylene and the 5- to 10-membered heteroarylene in the 5- to 10-membered heteroarylene substituted with one or more R^{L5} each independently have 1, 2 or 3 heteroatoms selected from one or more of N, O and S;
R^{L1} is H or C₁-C₆ alkyl;
ring C₂ is benzene ring or 5- to 6-membered heteroaromatic ring, wherein the 5- to 6-membered heteroaromatic ring has 1, 2 or 3 heteroatoms selected from one or more of N, O and S;
ring C₃ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
ring C₄ is a saturated C₃-C₇ carbon ring or a saturated 3- to 7-membered heterocycle, wherein the saturated 3- to 7-membered heterocycle has 1, 2 or 3 heteroatoms selected from one or more of N, O, S and C(=O);
R^{L2} and R^{L3} are each independently CN, OH, halogen, C₁-C_{6 alkyl,} C₃-C₇ cycloalkyl or 3- to 7-membered heterocycloalkyl;
R^{L4} and R^{L5} are each independently C₁-C₆ alkyl;
B is 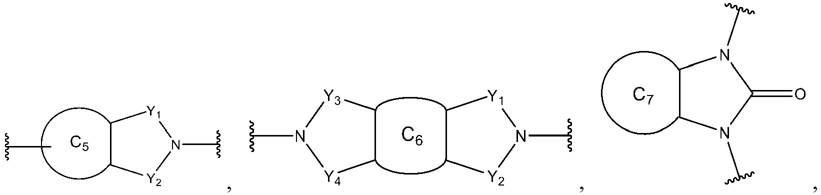 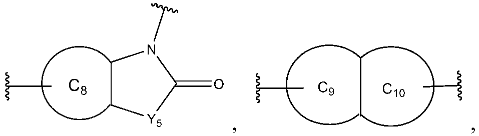 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1}, or 5- to 10-membered heteroarylene substituted with one or more R^{B2}, wherein the 5-to 10-membered heteroarylene and the 5- to 10-membered heteroarylene in the 5- to 10-membered heteroarylene substituted with one or more R^{B2} each independently have 1, 2 or 3 heteroatoms selected from one or more of N, O and S, and the saturated 3- to 12-membered heterocycloalkylene independently has 1, 2 or 3 heteroatoms selected from one or more of N, O, S and C(=O);
Y₁. Y₂, Y₃ and Y₄ are each independently CH₂ or C(=O);
Y₅ is NY⁵⁻¹, O or S, and Y⁵⁻¹ is H or C₁-C₆ alkyl;
ring C₅, ring C₆, ring C₇, ring C₈ and ring C₉ are each independently a benzene ring or a 5- to 6-membered heteroaromatic ring, wherein the 5- to 6-membered heteroaromatic ring has 1, 2 or 3 heteroatoms selected from one or more of N, O and S;
ring C₁₀ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
R^{B1} and R^{B2} are each independently halogen, C₁-C₆ alkyl, C₁-C₆ alkoxy, C₁-C₆ alkyl substituted with one or more R^{B1-1}, or C1-C6 alkoxy substituted with one or more R^{B1-2};
R^{B1-1} and R^{B1-2} are each independently halogen;
moreover, the compound represented by formula (I) satisfies one or both of the following conditions:
   i: B is 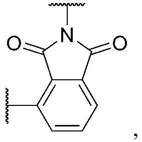 L is -(CH₂)ₙ-, n is any integer from 1 to 18, and at least 1 of -(CH₂)- in - (CH₂)ₙ- is replaced by the following groups: saturated C₃-C₁₂ cycloalkylene, saturated 3- to 12-membered heterocycloalkylene, C₆-C₁₀ arylene, 5- to 10-membered heteroarylene, 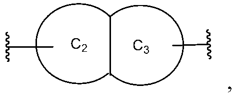 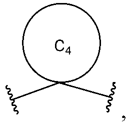 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4}, or 5-to 10-membered heteroarylene substituted with one or more R^{L5};
   ii: B is 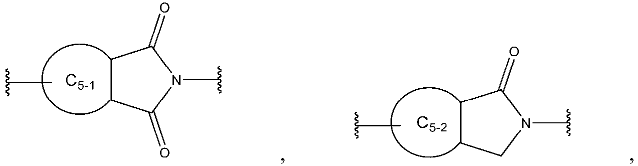 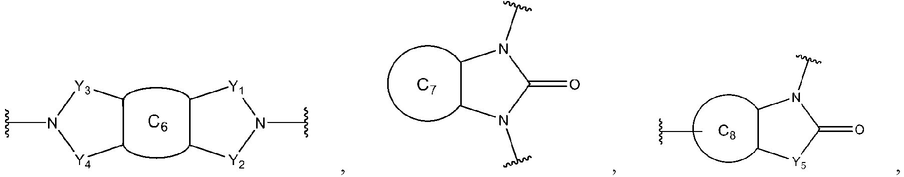 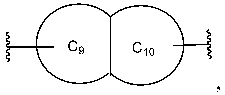 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1} or 5- to 10-membered heteroarylene substituted with one or more R^{B2}, ring C₅₋₁ same as the 5- to 6-membered heteroaryl ring in ring C5, and ring C₅₋₂ same as ring C₅.

In one embodiment of the present invention, R³ is H or methyl.

In one embodiment of the present invention, R² is -H, -CN, -F, -Cl, -CH₃, cyclopropyl, -OCH₃,

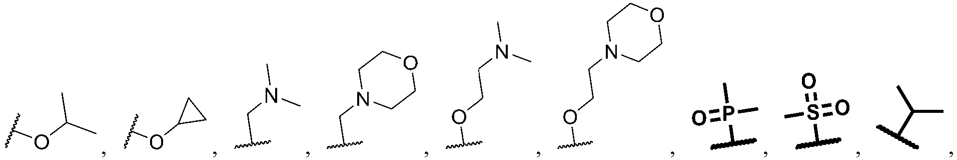

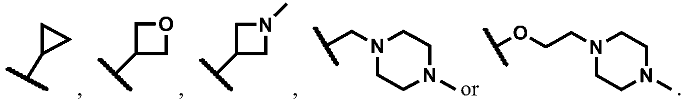

In one embodiment of the present invention, R² is -H, -F, -Cl, - CH₃, cyclopropyl, -OCH₃,

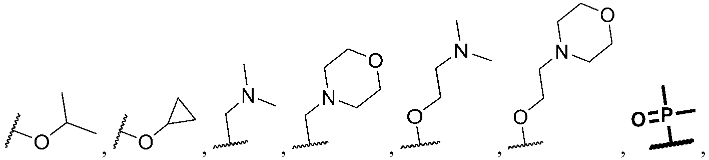

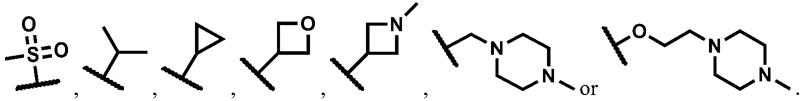

In one embodiment of the present invention, A is 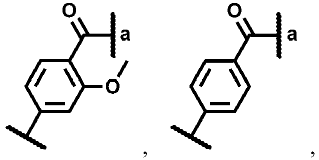 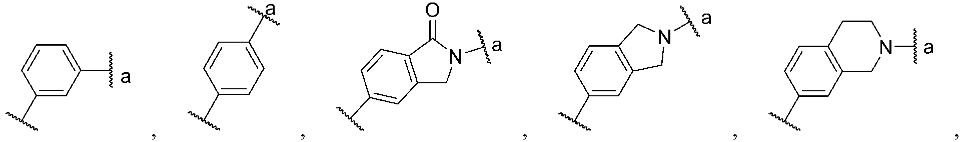 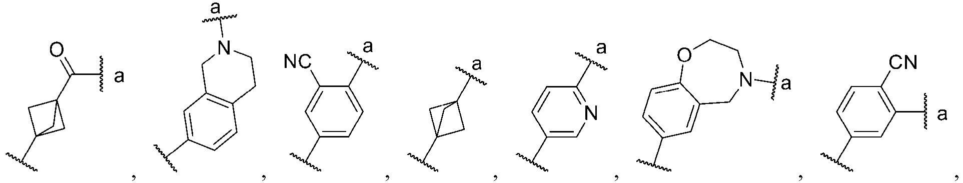 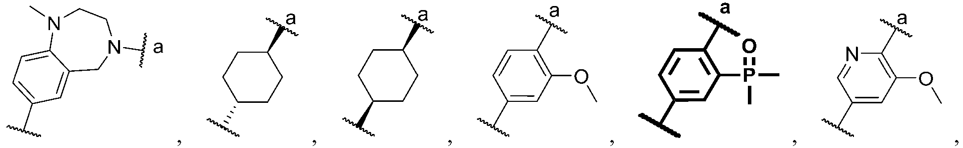 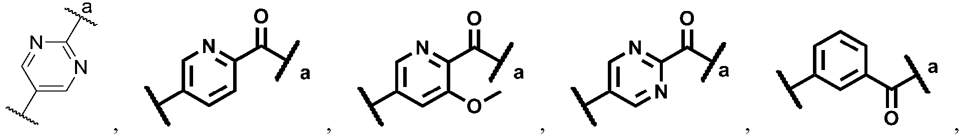 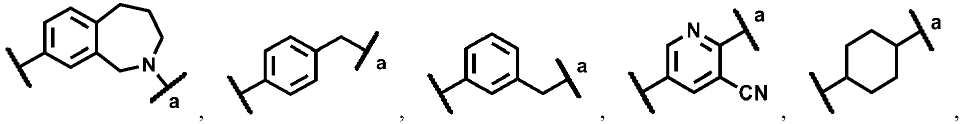 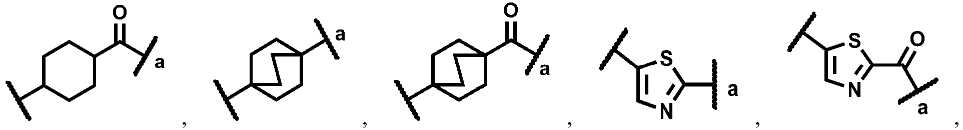 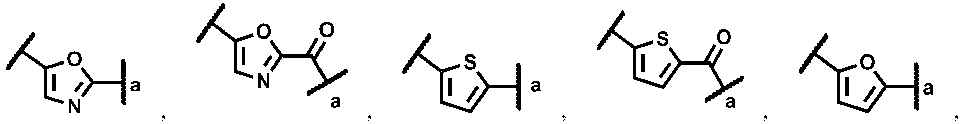 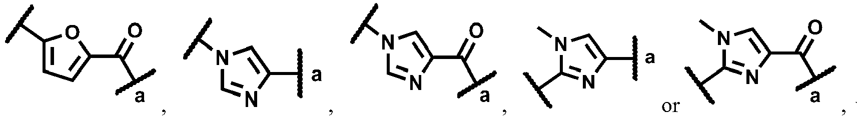 with side a connected to L.

In one embodiment of the present invention, in condition ii, L is: 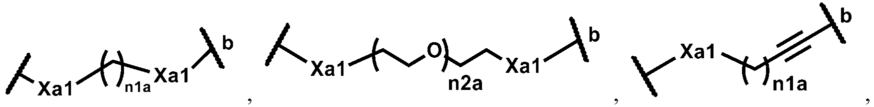 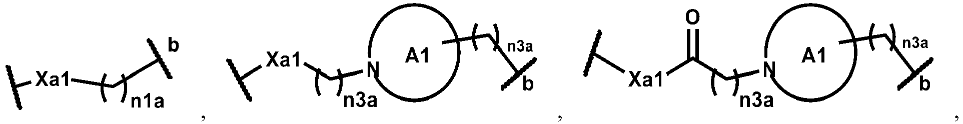 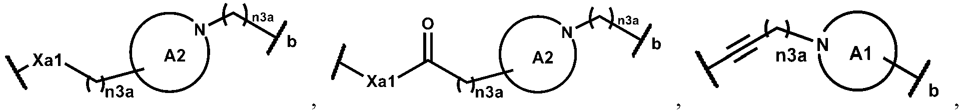 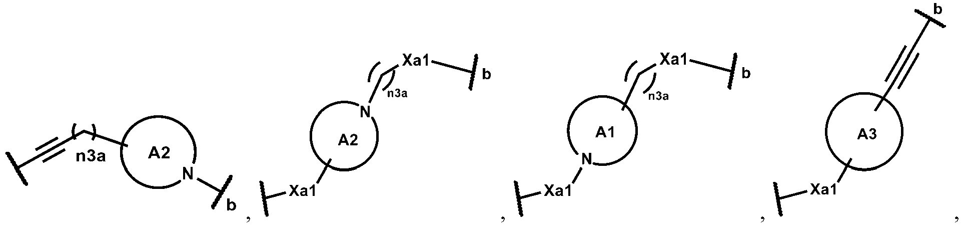 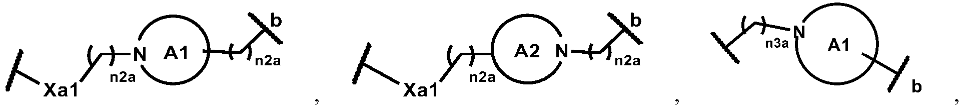 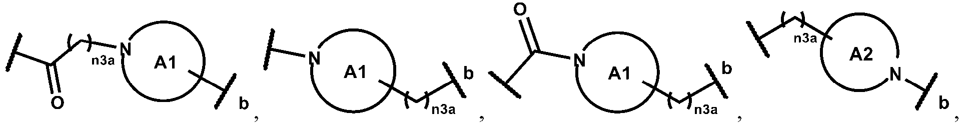 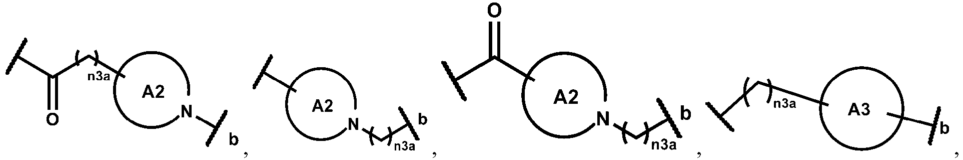 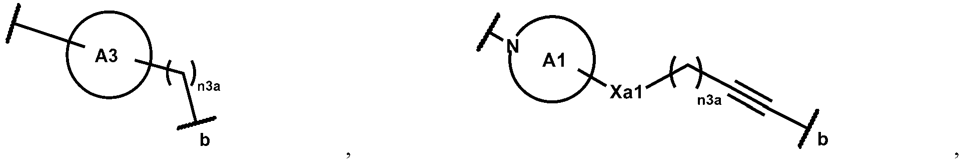 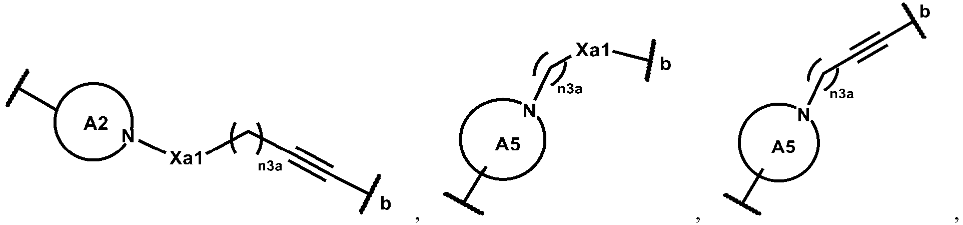 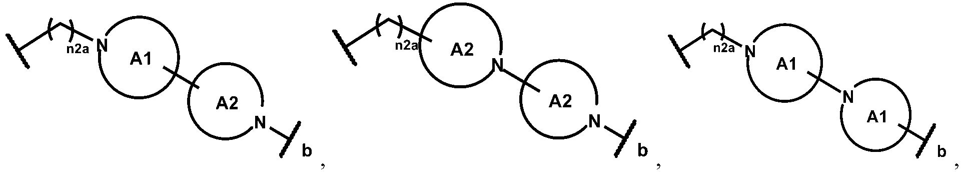 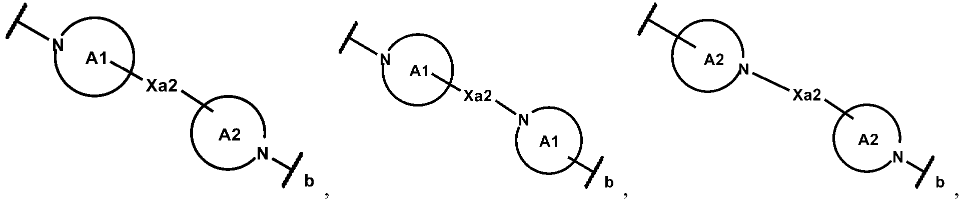 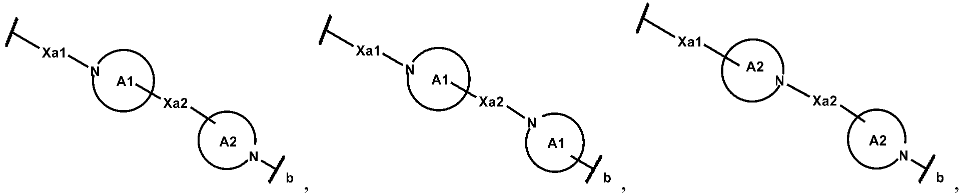 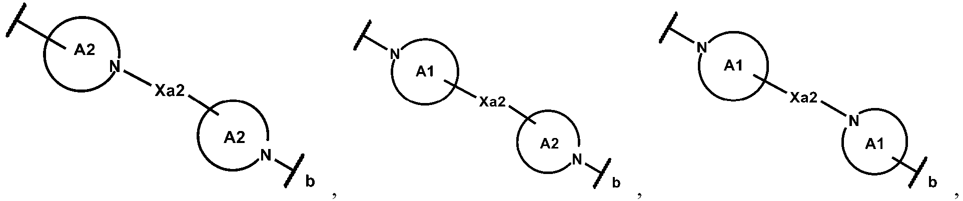 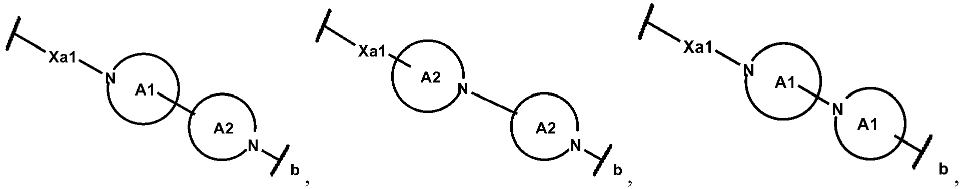 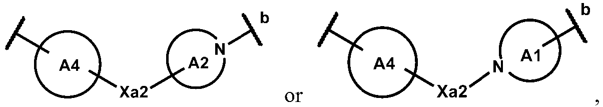
wherein side b is connected to B;
Xa1 is O or -NR^{L1}-, R^{L1} is H or C₁-C₆ alkyl;
Xa2 is 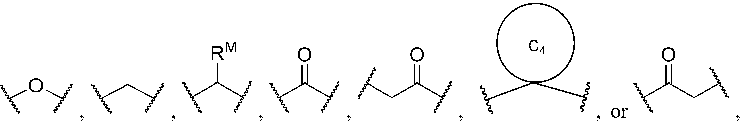 R^{M} is C₁-C₆ alkyl, ring C4 is saturated C₃-C₆ carbon ring, or saturated 3- to 6-membered heterocyclic ring having 1, 2 or 3 heteroatoms selected from one or more of N, O, S and C(=O);
ring A1 and ring A2 are saturated 3- to 12-membered nitrogen-containing heterocycles or saturated 3- to 12-membered nitrogen-containing heterocycles substituted with one or more R^{L3}, R^{L3} is independently CN, OH, halogen, C₁-C₆ alkyl, C₃-C₇ cycloalkyl or 3- to 7-membered heterocycloalkyl, wherein the saturated 3- to 12-membered nitrogen-containing heterocycle or the 3- to 12-membered nitrogen-containing heterocycle in the substituted saturated 3- to 12-membered nitrogen-containing heterocycle is independently 3- to 6-membered monocyclic nitrogen-containing heterocycle, 7- to 12-membered spirocyclic ring or 7- to 12-membered bridged nitrogen-containing heterocyclic ring, with 1, 2 or 3 heteroatoms selected from one or more of O, S and C(=O) in addition to N;
ring A3 is independently saturated C₃-C₁₂ carbon ring or saturated C₃-C₁₂ carbon ring substituted with one or more R^{L2}, and R^{L2} is independently CN, OH, halogen, C₁-C₆ alkyl, C₃-C₇ cycloalkyl or 3- to 7-membered heterocycloalkyl, wherein the C₃-C₁₂ carbon ring in the saturated C₃-C₁₂ carbon ring or the substituted saturated C₃-C₁₂ carbon ring is independently C₃-C₆ monocyclic carbon ring or C₅-C₁₂ spirocyclic carbon ring;
ring A4 is independently 5- to 10-membered heteroaromatic ring or 5- to 10-membered heteroaromatic ring substituted with one or more R^{L5}, and R^{L5} is independently C₁-C₆ alkyl; the 5- to 10-membered heteroaromatic ring or the 5- to 10-membered heteroaromatic ring in the 5- to 10-membered heteroaromatic ring substituted with one or more R^{L5} independently has 1, 2 or 3 heteroatoms selected from one or more of N, O and S;
ring A5 is independently 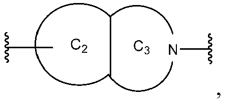 and ring C₂ is benzene ring or 5- to 6-membered heteroaromatic ring wherein the 5- to 6-membered heteroaromatic ring has 1, 2 or 3 heteroatoms selected from one or more of N, O and S; ring C₃ is partially unsaturated 3- to 7-membered nitrogen-containing heterocyclic ring, which has 1, 2 or 3 heteroatoms selected from one or more of O, S and C(=O) in addition to N;
n1a is independently any integer from 0 to 8;
n2a is independently any integer from 0 to 3;
n3a is independently any integer from 0 to 5;
preferably: 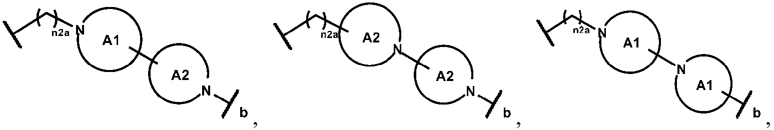 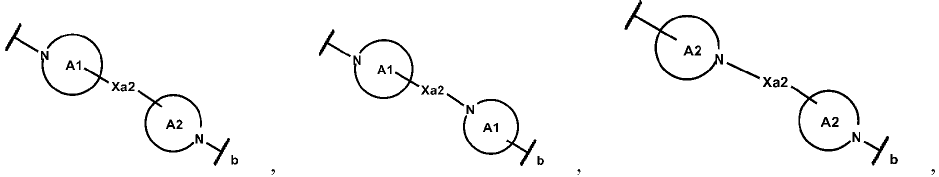 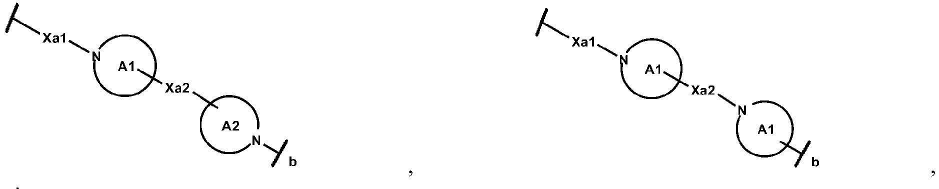 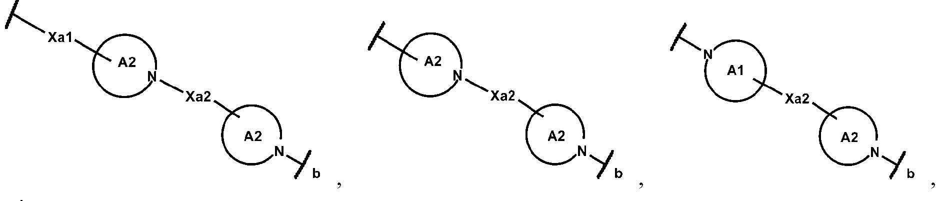 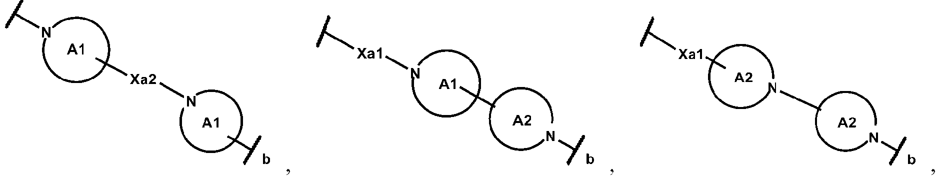 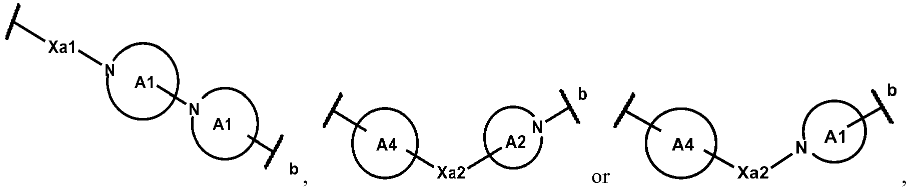
wherein side b is connected to B.

In one embodiment of the present invention, in condition ii, L is 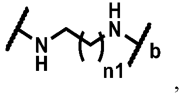 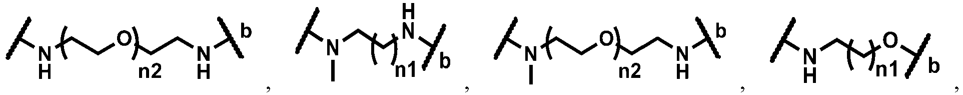 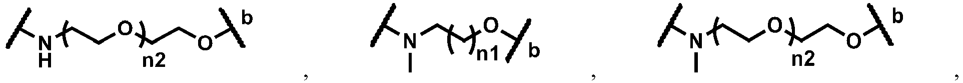 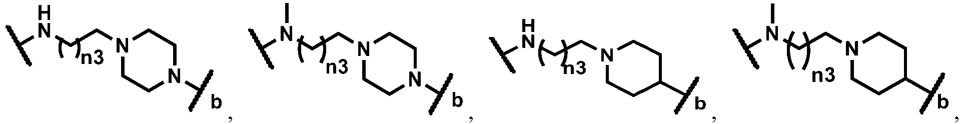 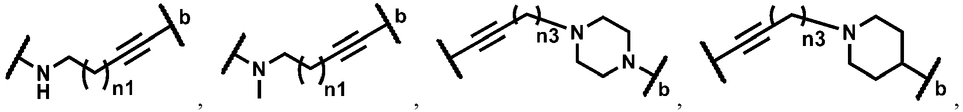 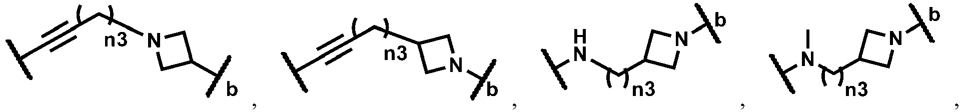 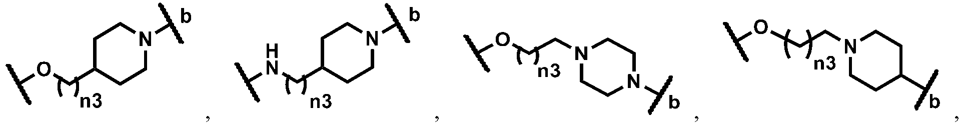 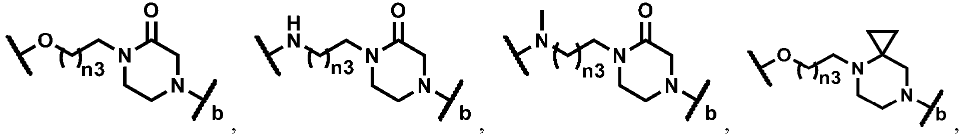 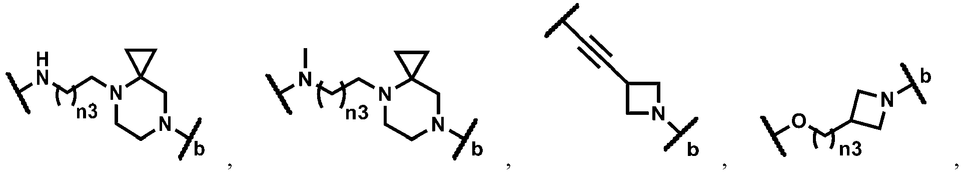 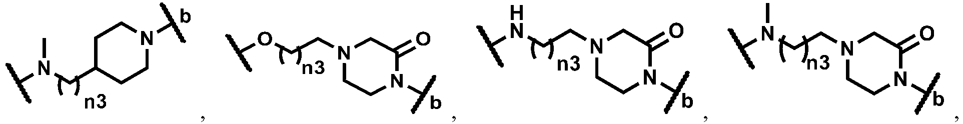 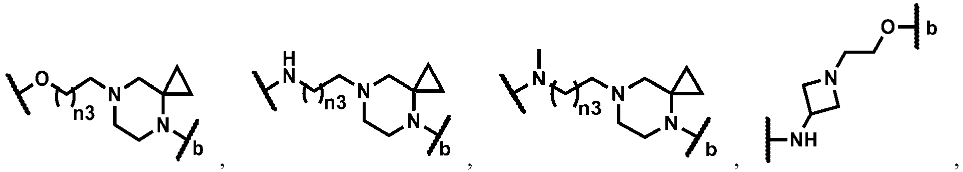 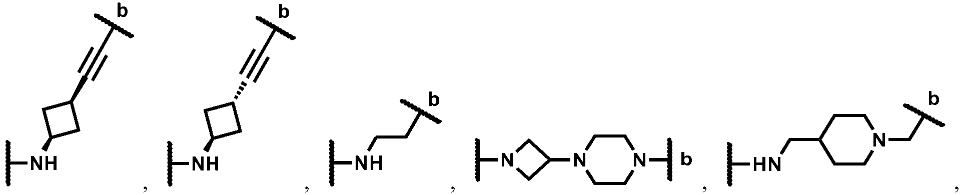 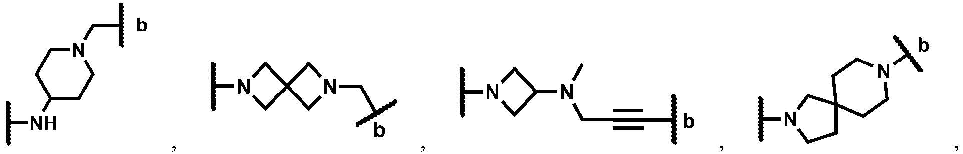 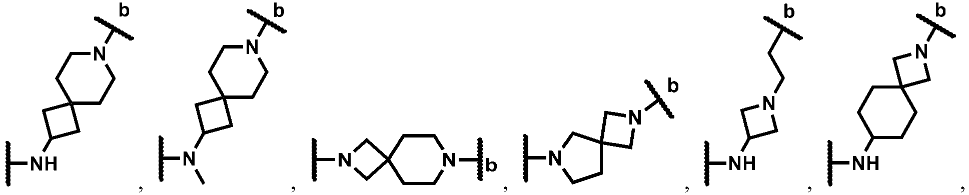 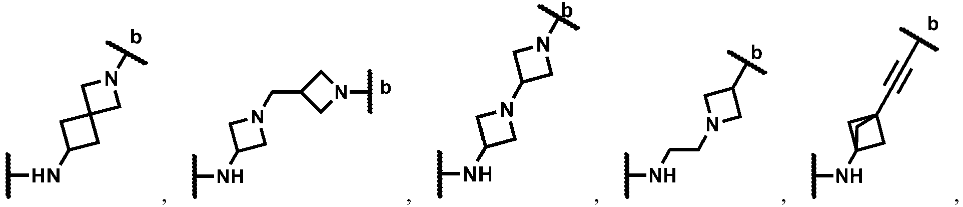 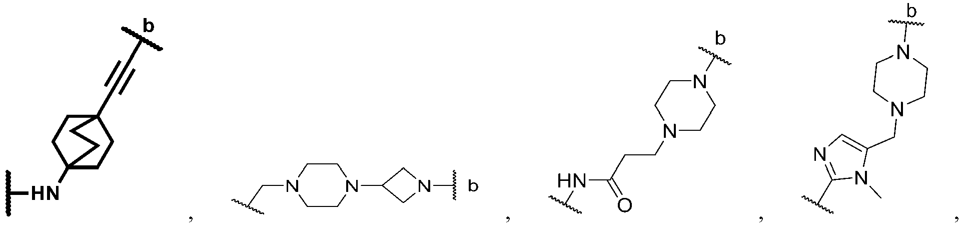 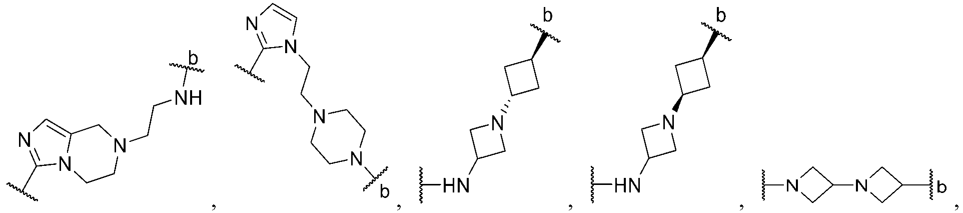 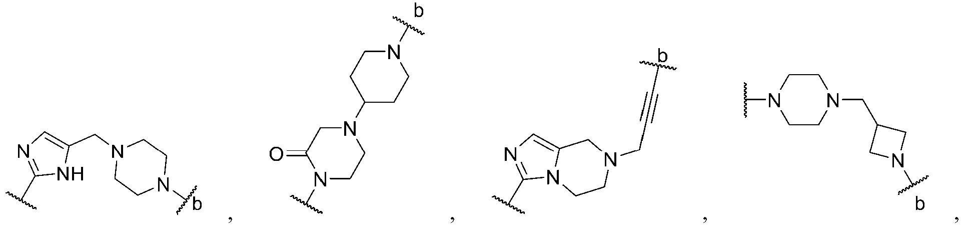 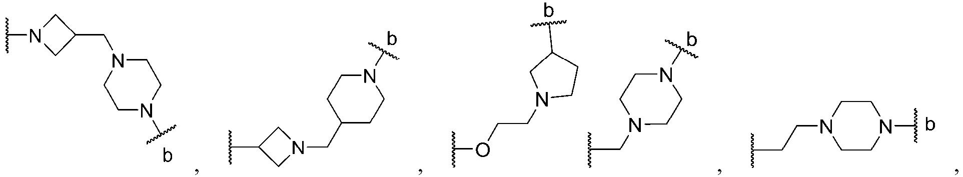 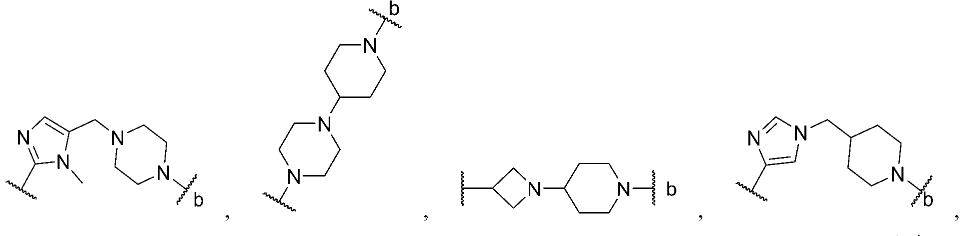 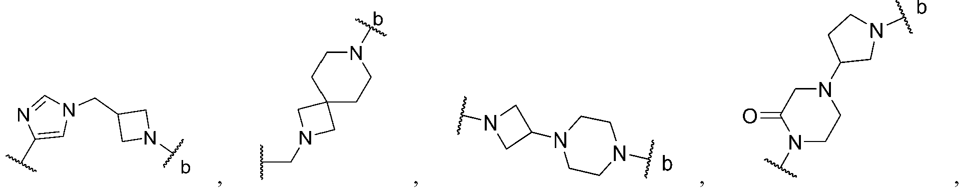 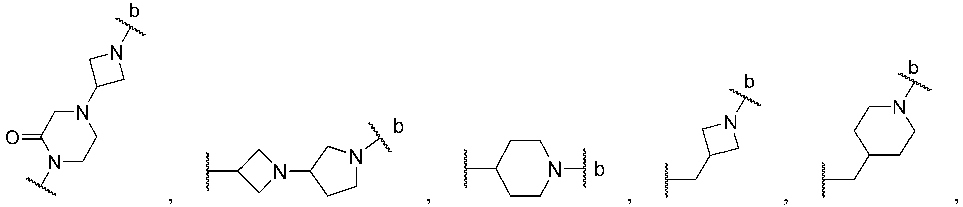 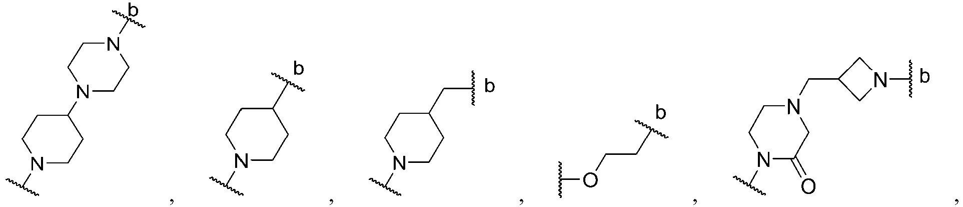 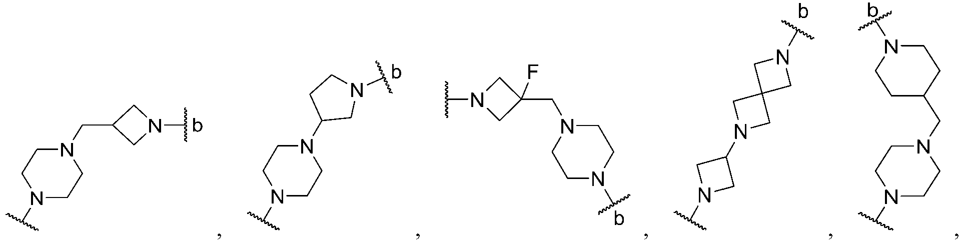 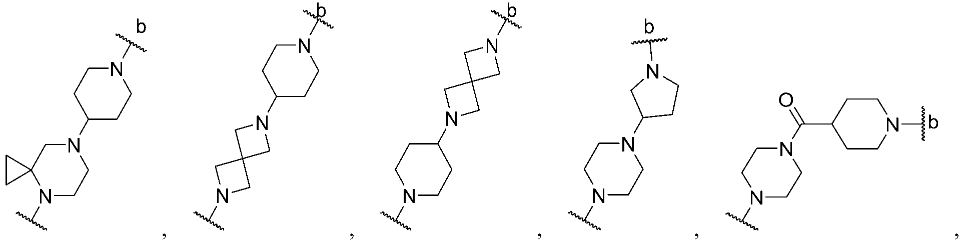 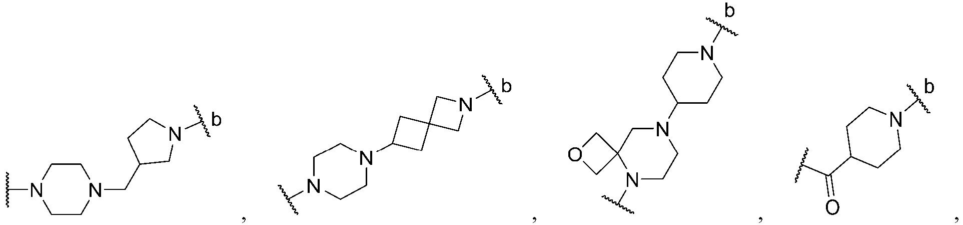 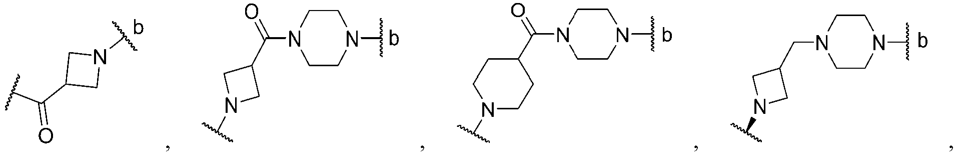 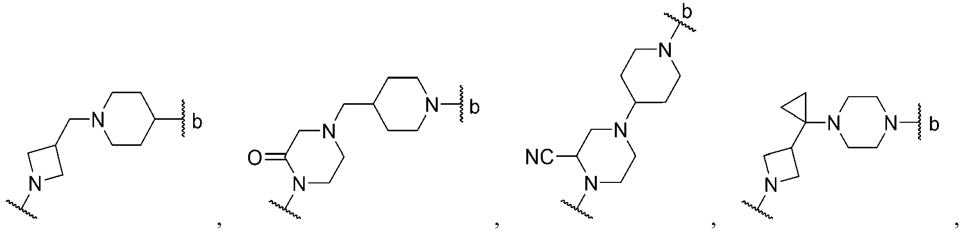 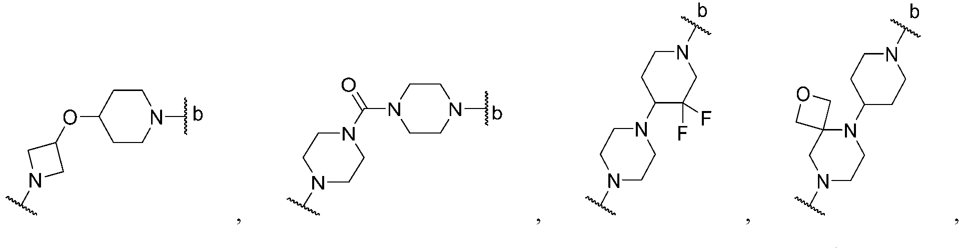 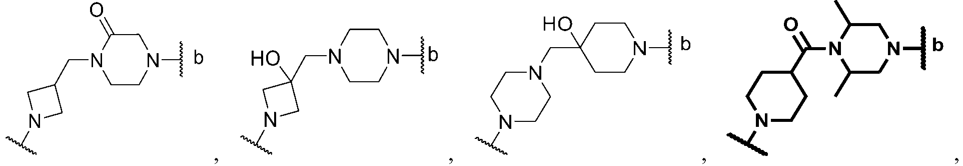 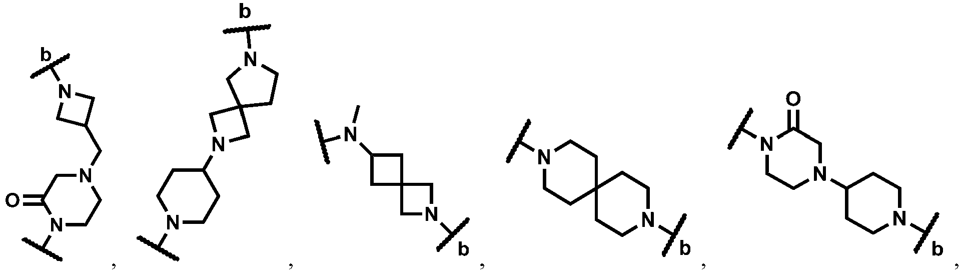 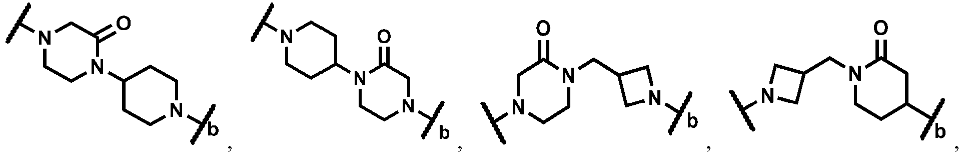 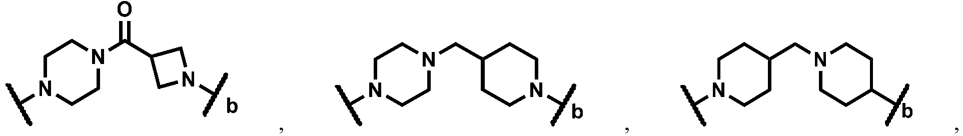 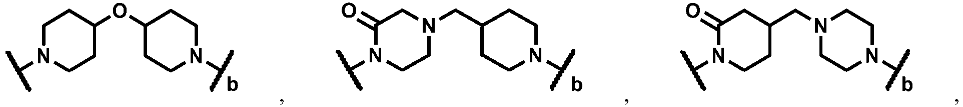 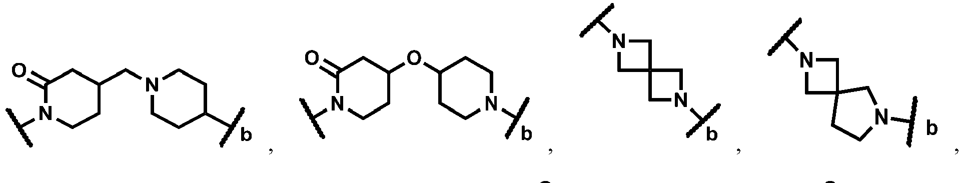 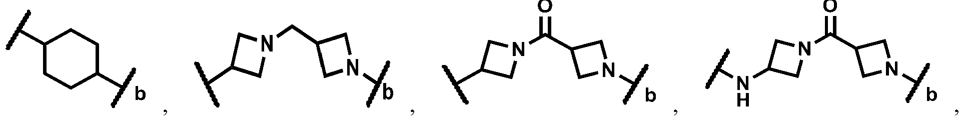 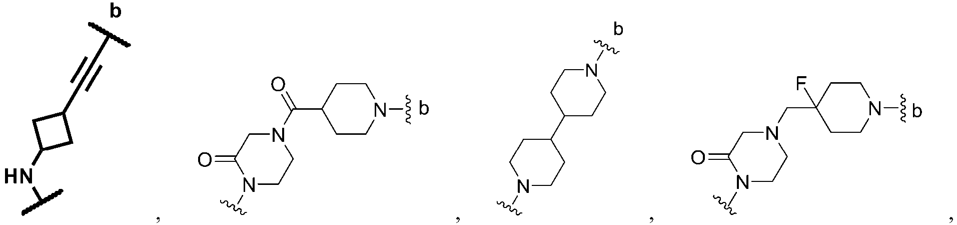 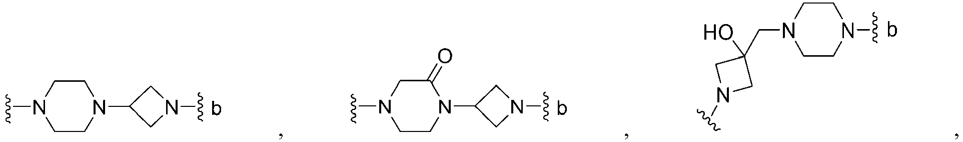 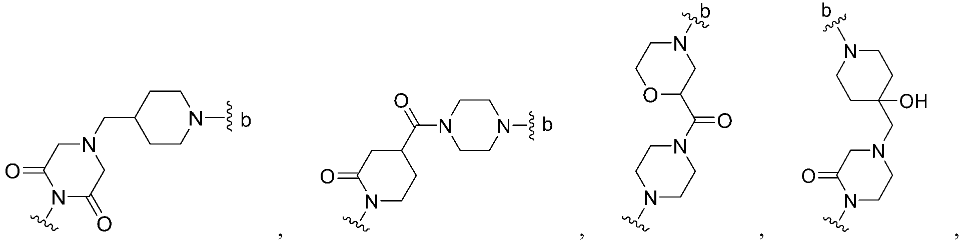 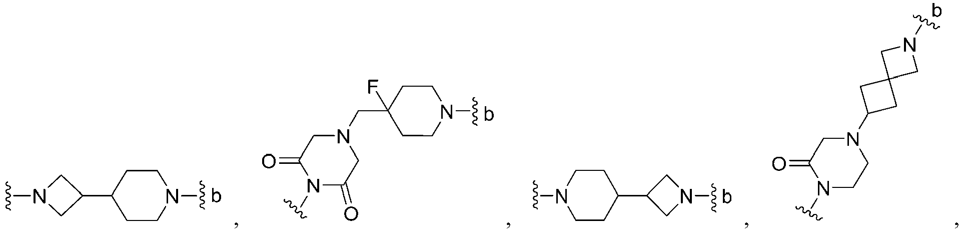 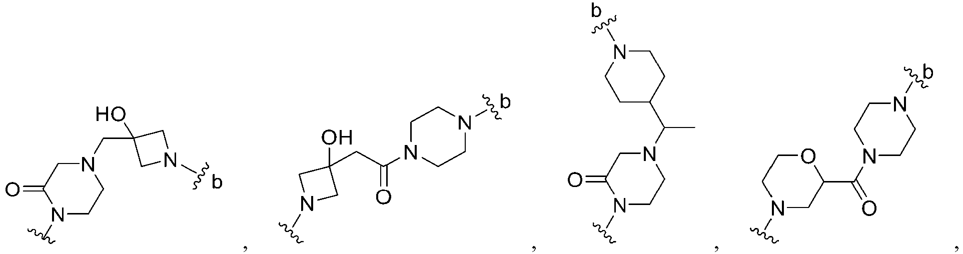 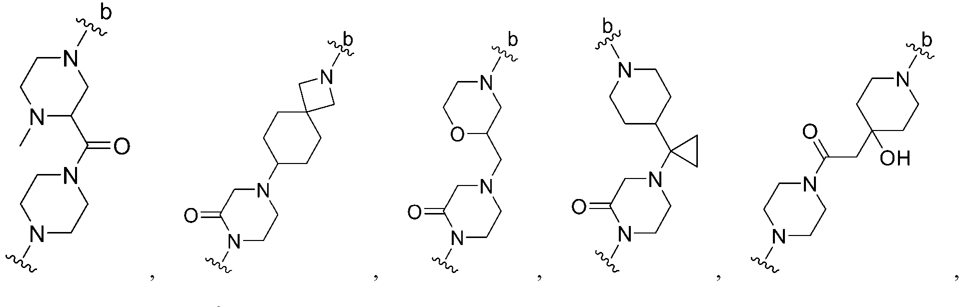 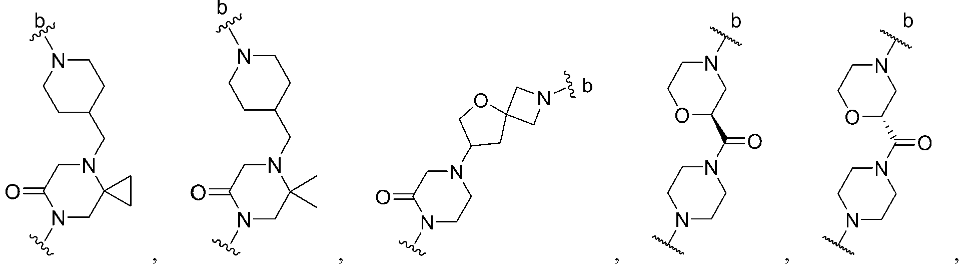 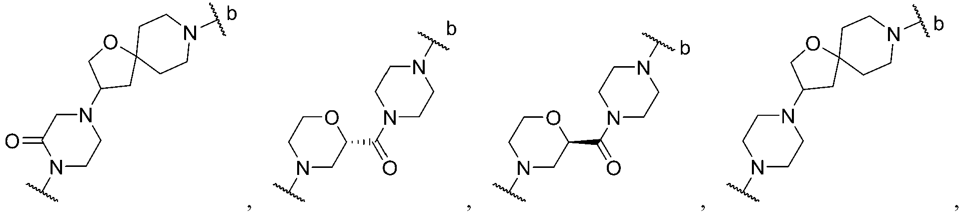 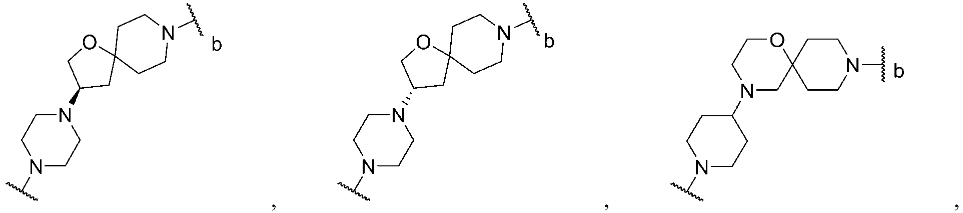 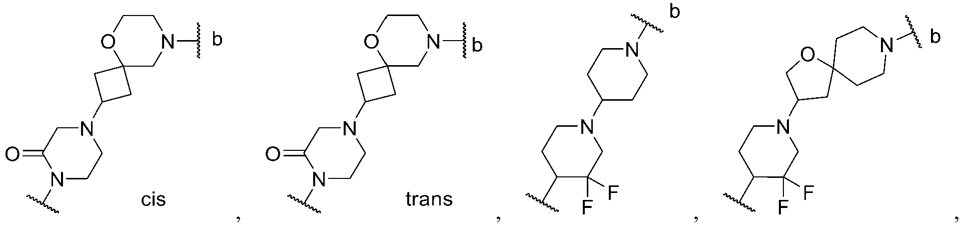 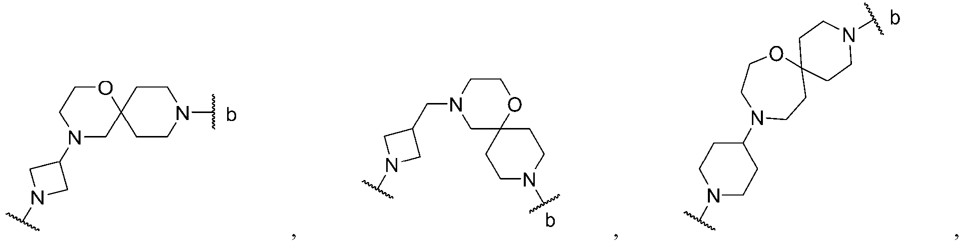 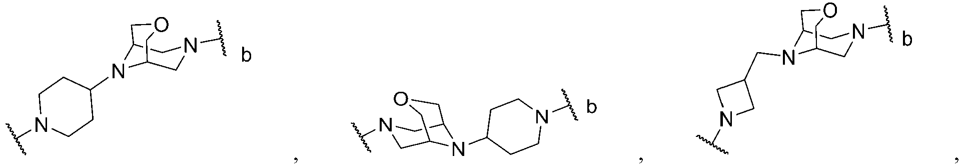 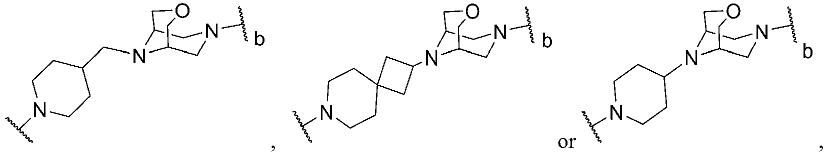 wherein n1 is any integer from 1 to 8, n2 is any integer from 1 to 3, n3 is any integer from 1 to 5, and side b is connected to B;
preferably 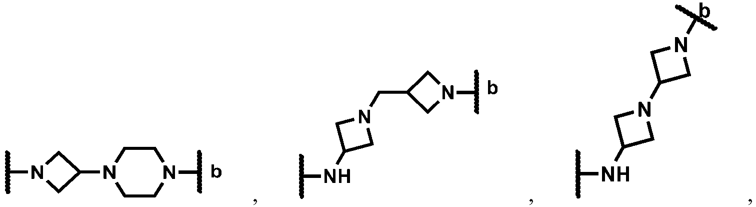 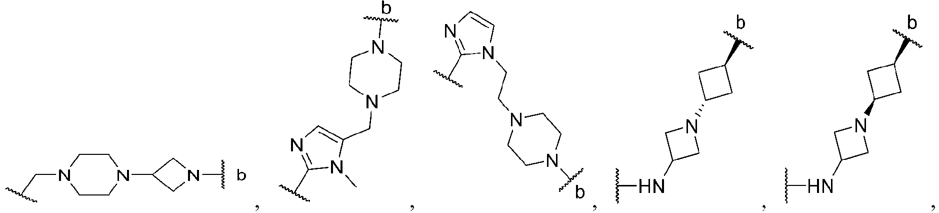 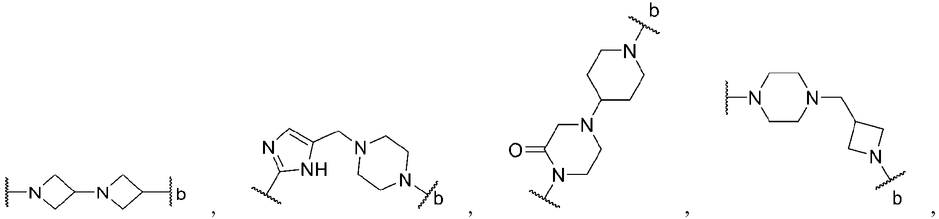 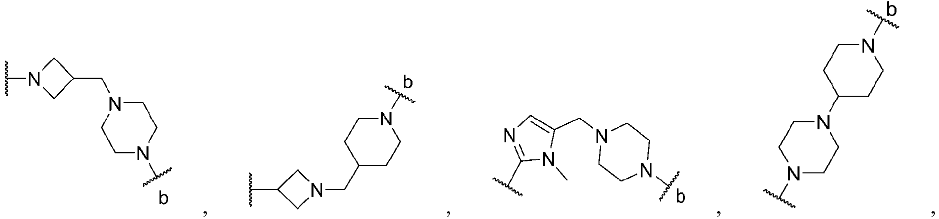 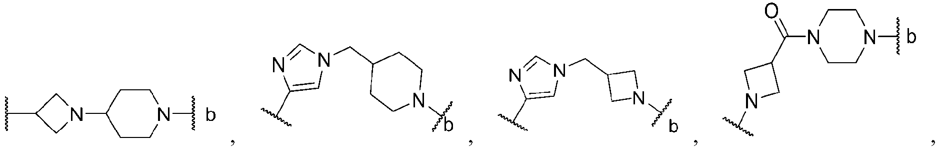 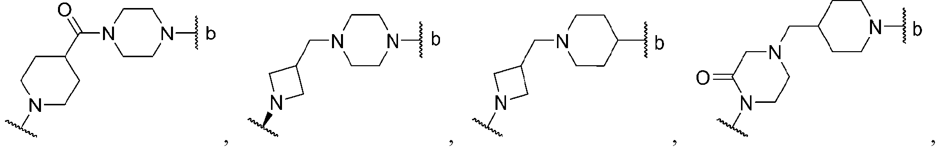 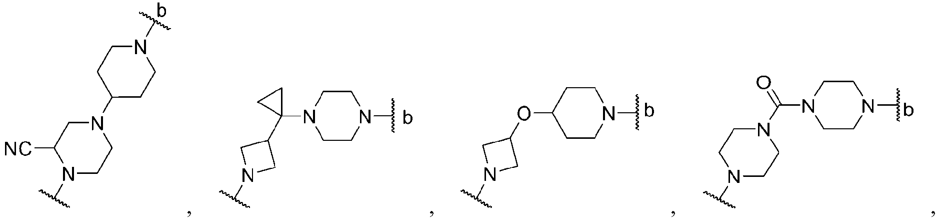 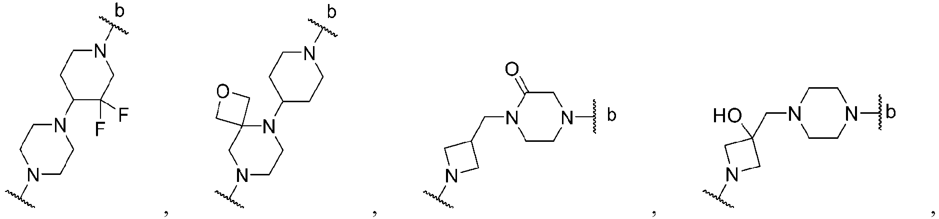 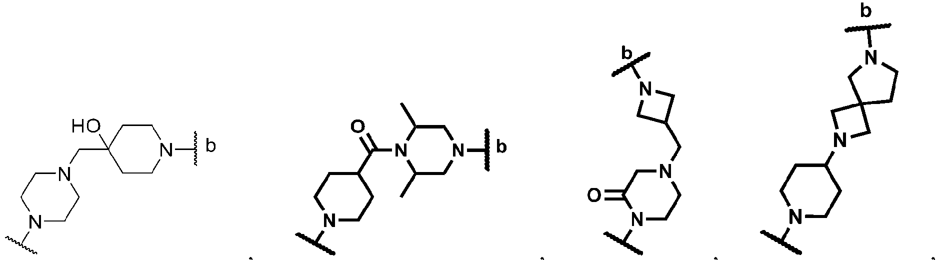 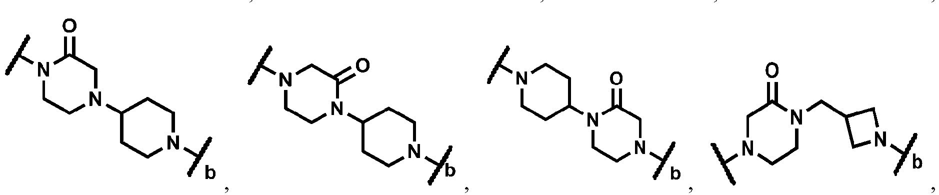 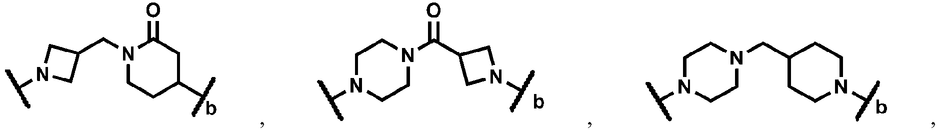 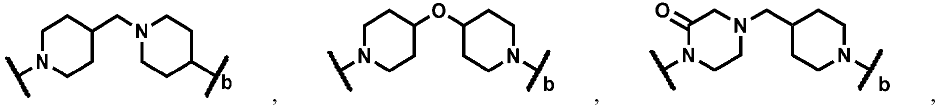 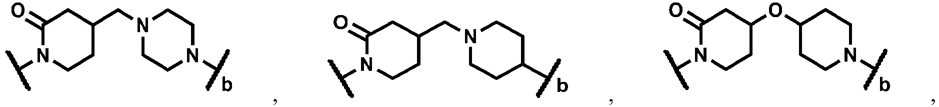 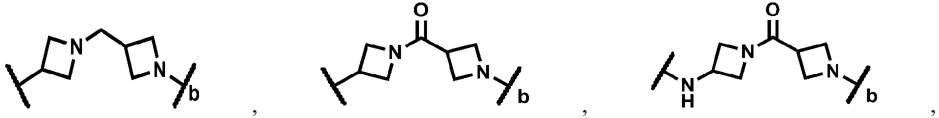 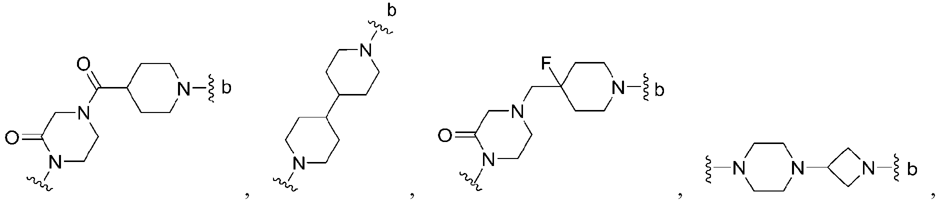 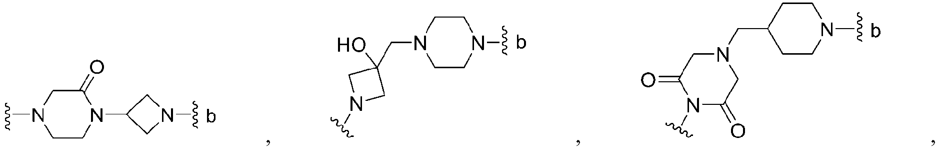 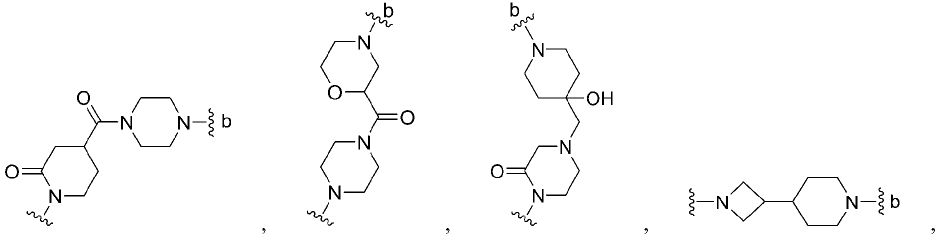 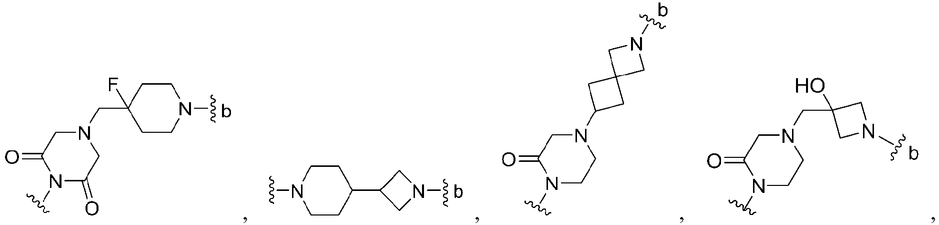 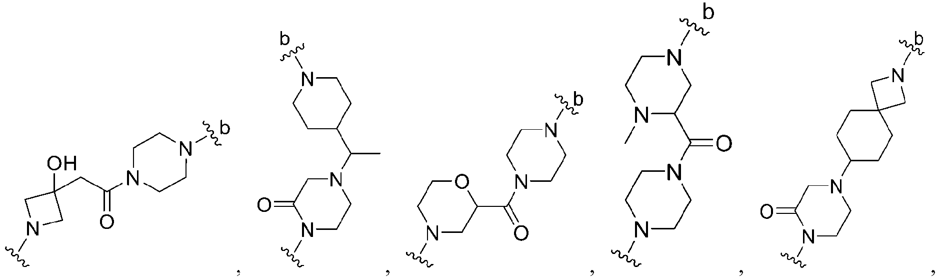 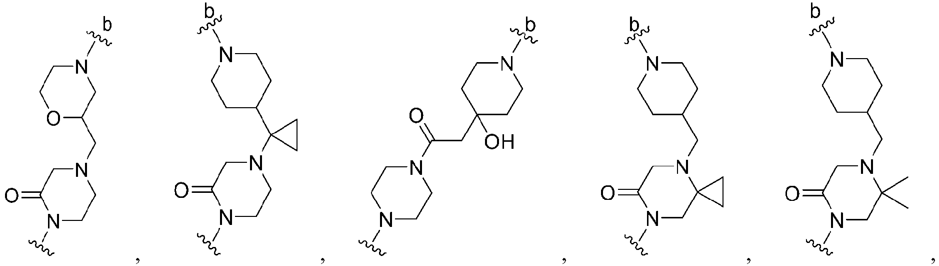 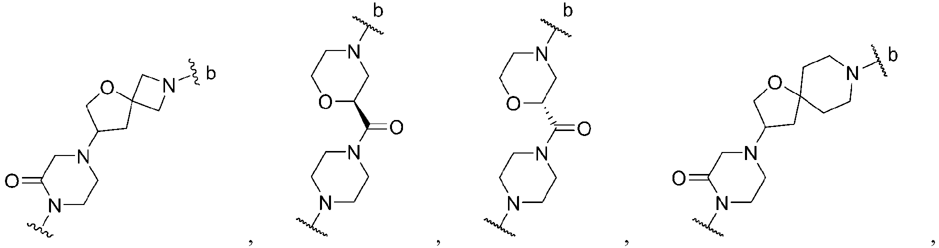 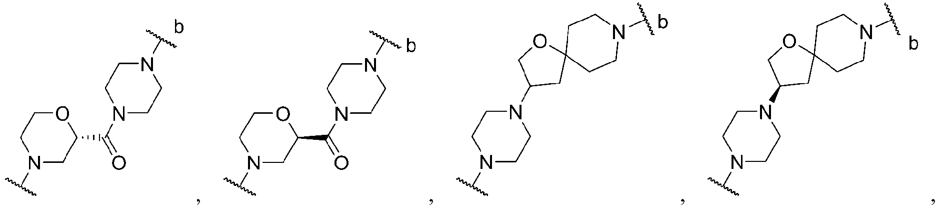 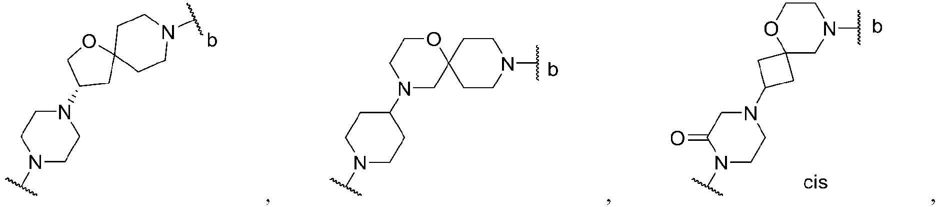 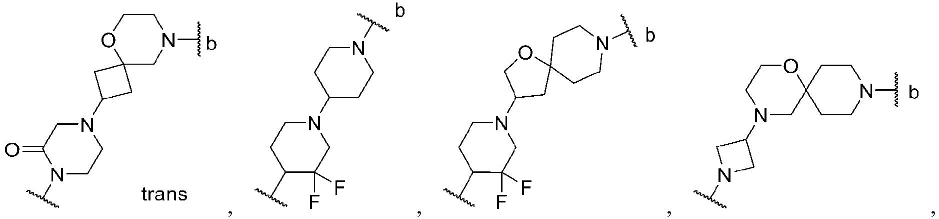 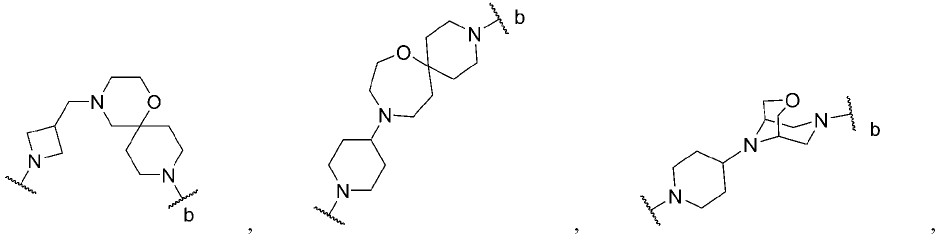 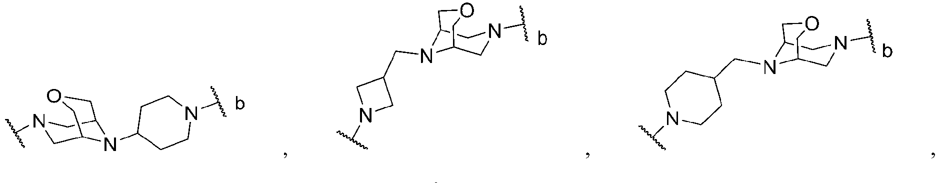 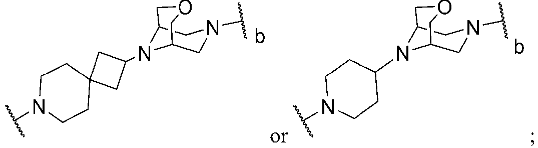
side b is connected to B.

In one embodiment of the present invention, in condition i, L is G1 or G2:
G1 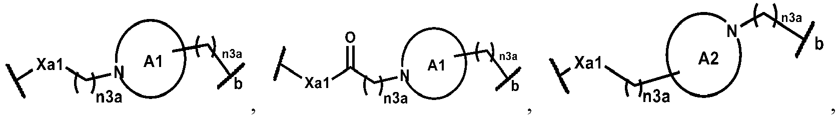 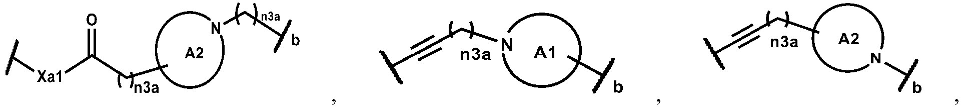 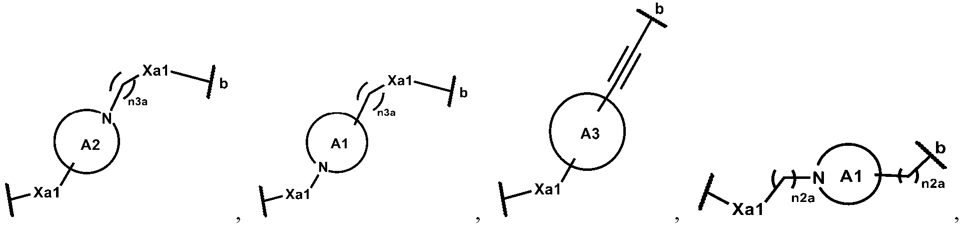 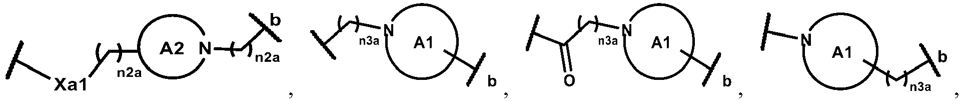 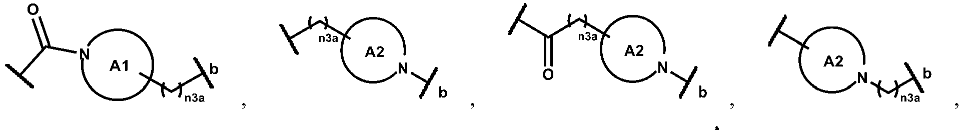 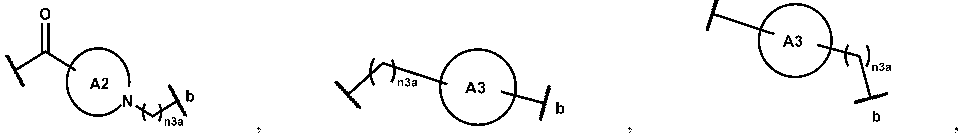 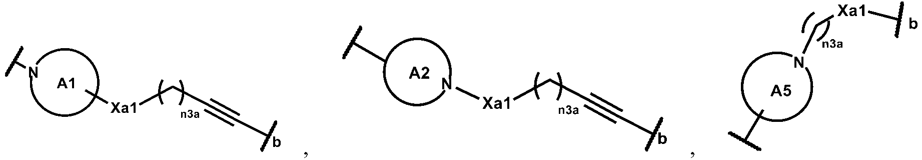 or 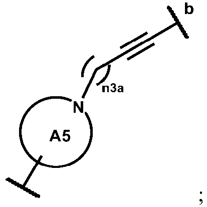
wherein side b is connected to B;
Xa1 is O or -NR^{L1}-, R^{L1} is H or C₁-C₆ alkyl;
ring A1 and ring A2 are saturated 3- to 12-membered nitrogen-containing heterocycles or saturated 3- to 12-membered nitrogen-containing heterocycles substituted with one or more R^{L3}, R^{L3} is independently CN, OH, halogen, C₁-C₆ alkyl, C₃-C₇ cycloalkyl or 3- to 7-membered heterocycloalkyl, wherein the saturated 3- to 12-membered nitrogen-containing heterocycle or the 3- to 12-membered nitrogen-containing heterocycle in the substituted saturated 3- to 12-membered nitrogen-containing heterocycle is independently 4-membered monocyclic nitrogen-containing heterocycle, 7- to 12-membered spirocyclic nitrogen-containing heterocycle or 7- to 12-membered bridged nitrogen-containing heterocycle, with 1, 2 or 3 heteroatoms selected from one or more of O, S and C(=O) in addition to N;
ring A3 is independently saturated C₃-C₁₂ carbon ring or saturated C₃-C₁₂ carbon ring substituted with one or more R^{L2}, and R^{L2} is independently CN, OH, halogen, C₁-C₆ alkyl, C₃-C₇ cycloalkyl or 3- to 7-membered heterocycloalkyl, wherein the C₃-C₁₂ carbon ring in the saturated C₃-C₁₂ carbon ring or the substituted saturated C₃-C₁₂ carbon ring is independently C₃-C₆ monocyclic carbon ring, C₇-C₁₂ spirocyclic carbon ring or C₇-C₁₂ bridged carbon ring;
ring A5 is independently 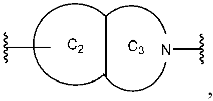 and ring C₂ is benzene ring or 5- to 6-membered heteroaromatic ring wherein the 5- to 6-membered heteroaromatic ring has 1, 2 or 3 heteroatoms selected from one or more of N, O and S; ring C₃ is partially unsaturated 3- to 7-membered nitrogen-containing heterocyclic ring, which has 1, 2 or 3 heteroatoms selected from one or more of O, S and C(=O) in addition to N;
n2a is independently any integer from 0 to 3;
n3a is independently any integer from 0 to 5;
G2: 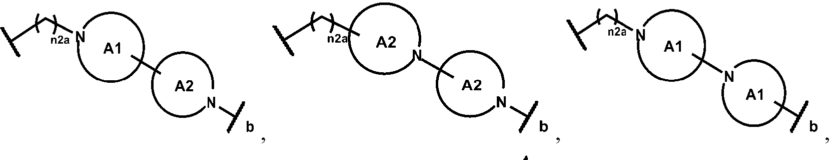 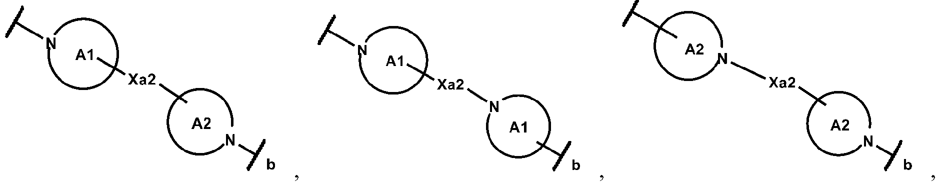 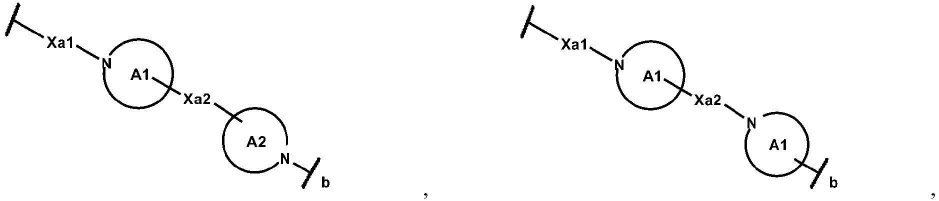 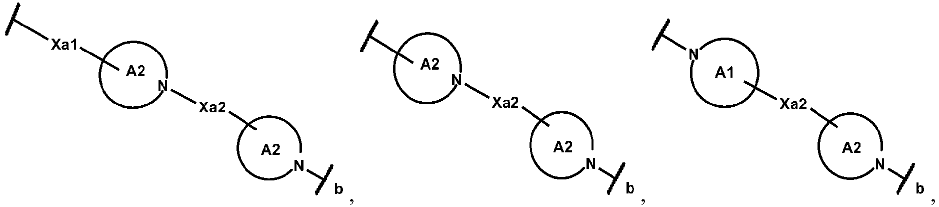 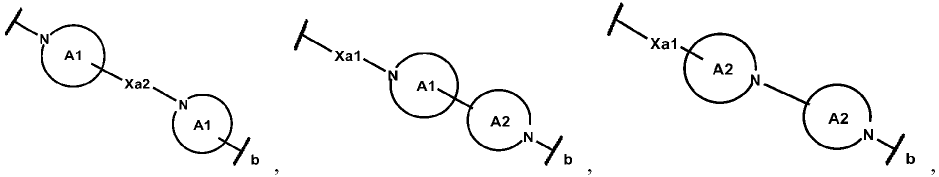 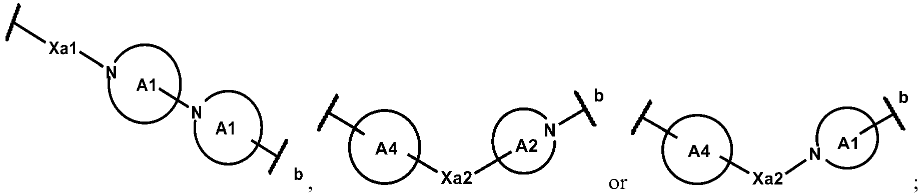
wherein side b is connected to B;
Xa1 is O or -NR^{L1}-, R^{L1} is H or C₁-C₆ alkyl;
Xa2 is 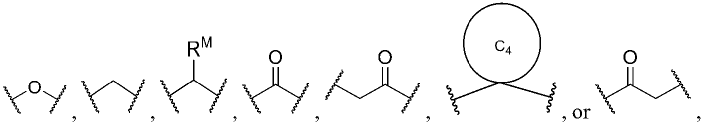 R^{M} is C₁-C₆ alkyl, ring C4 is saturated C₃-C₆ carbon ring, or saturated 3- to 6-membered heterocyclic ring having 1, 2 or 3 heteroatoms selected from one or more of N, O, S and C(=O);
ring A1 and ring A2 are saturated 3- to 12-membered nitrogen-containing heterocycles or saturated 3- to 12-membered nitrogen-containing heterocycles substituted with one or more R^{L3}, R^{L3} is independently CN, OH, halogen, C₁-C₆ alkyl, C₃-C₇ cycloalkyl or 3- to 7-membered heterocycloalkyl, wherein the saturated 3- to 12-membered nitrogen-containing heterocycle or the 3- to 12-membered nitrogen-containing heterocycle in the substituted saturated 3- to 12-membered nitrogen-containing heterocycle is independently 3- to 6-membered monocyclic nitrogen-containing heterocycle or 5- to 12-membered spirocyclic nitrogen-containing heterocycle, with 1, 2 or 3 heteroatoms selected from one or more of O, S and C(=O) in addition to N;
ring A4 is independently 5- to 10-membered heteroaromatic ring or 5- to 10-membered heteroaromatic ring substituted with one or more R^{L5}, and R^{L5} is independently C₁-C₆ alkyl; the 5- to 10-membered heteroaromatic ring or the 5- to 10-membered heteroaromatic ring in the 5- to 10-membered heteroaromatic ring substituted with one or more R^{L5} independently has 1, 2 or 3 heteroatoms selected from one or more of N, O and S;
n2a is independently any integer from 0 to 3.

In one embodiment of the present invention, in condition i, L is G1 or G2:
G1: 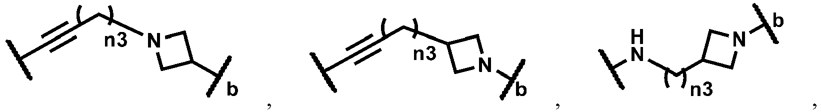 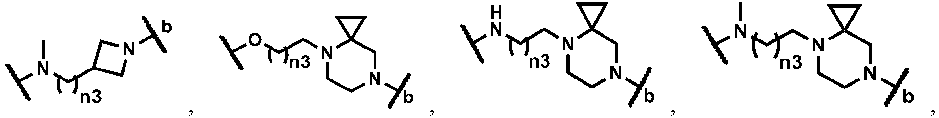 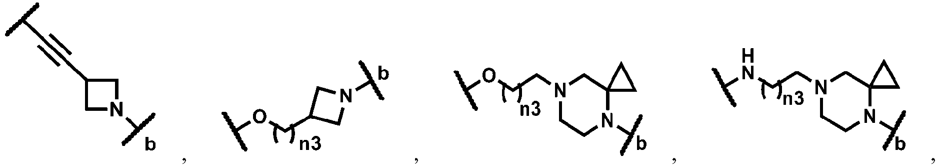 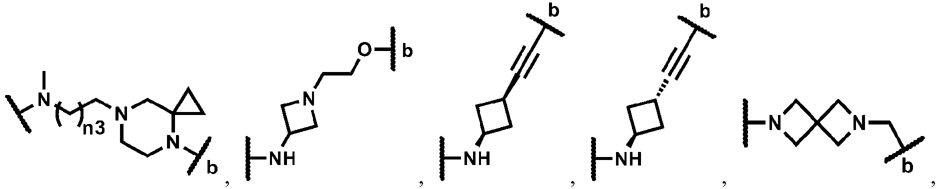 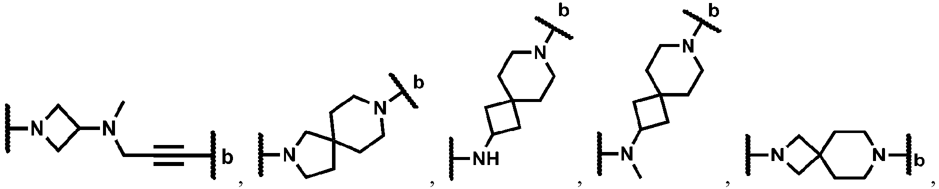 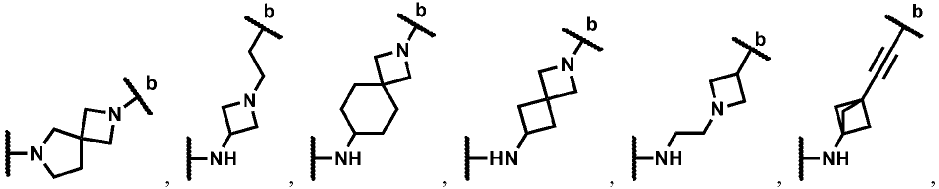 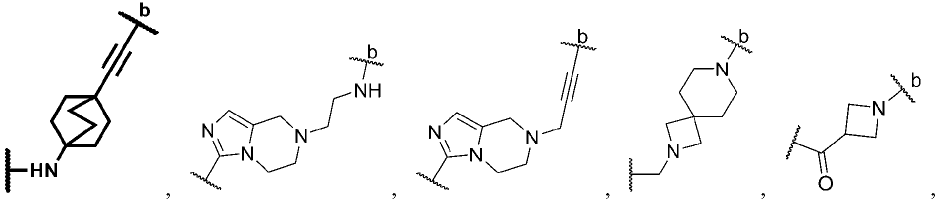 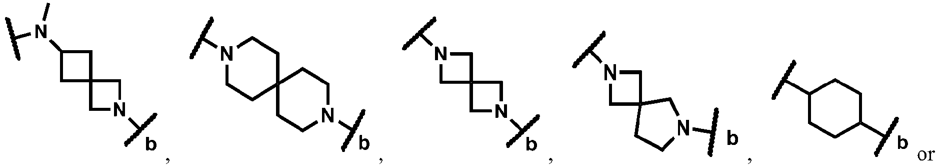 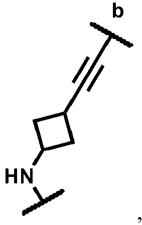 wherein n1 is any integer from 1 to 8, n2 is any integer from 1 to 3, n3 is any integer from 1 to 5, and side b is connected to B;
G2: 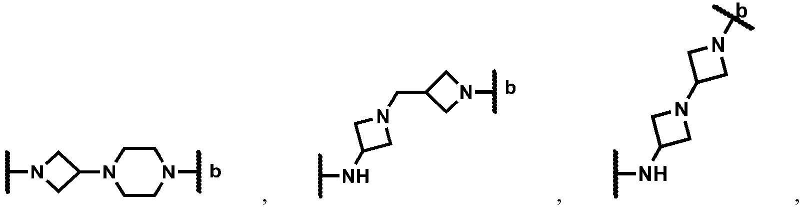 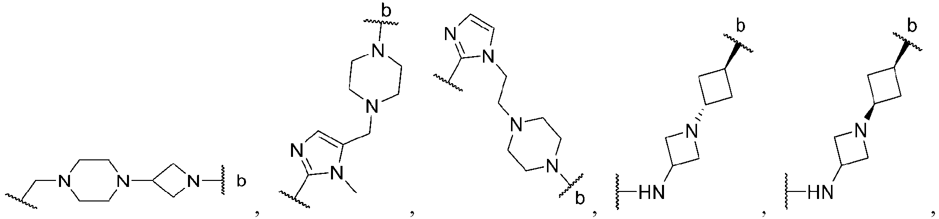 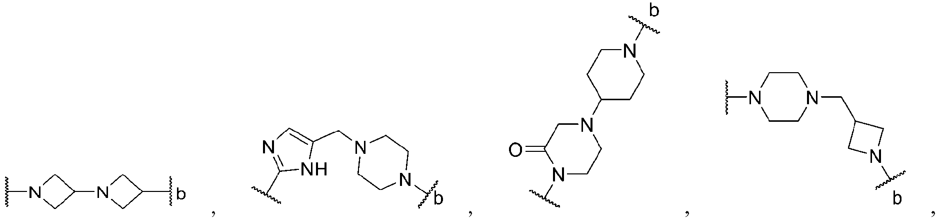 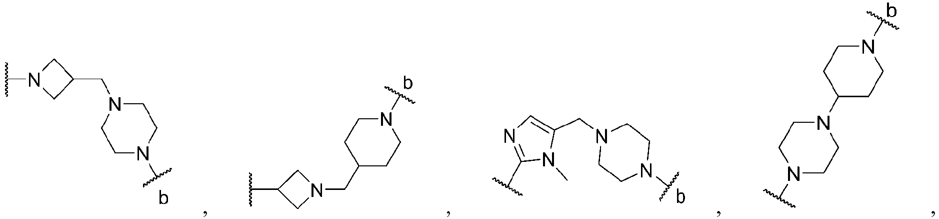 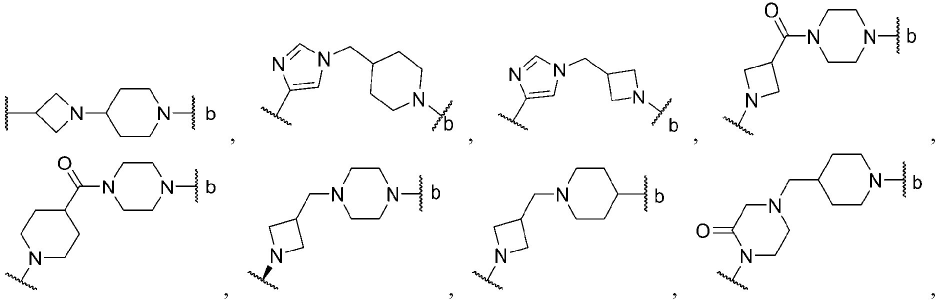 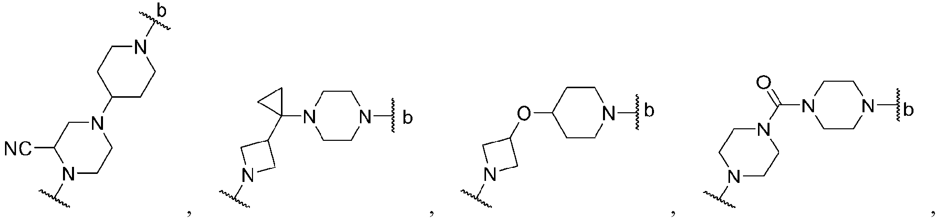 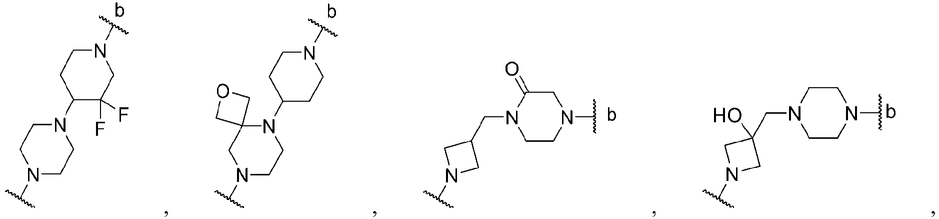 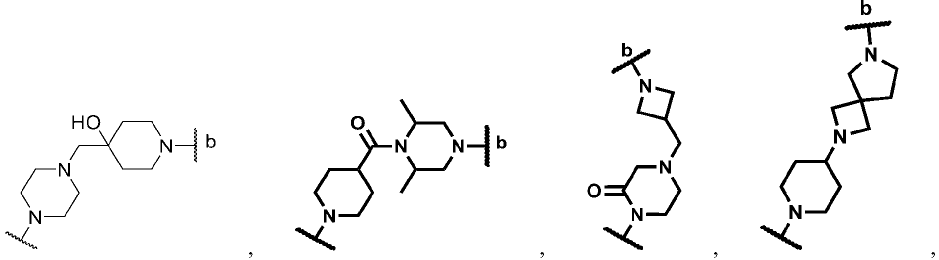 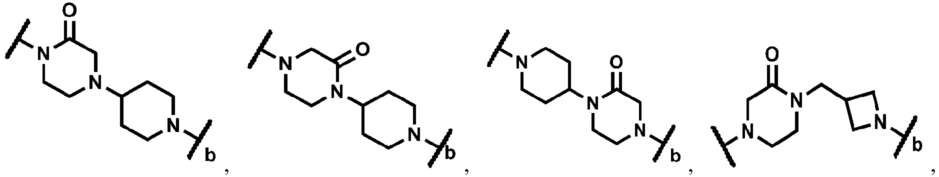 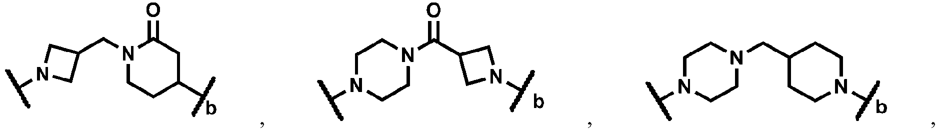 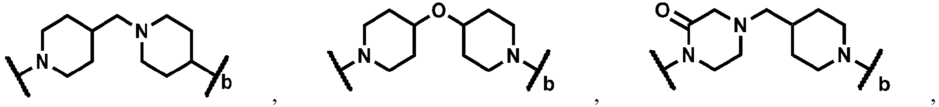 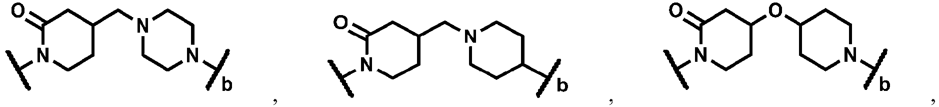 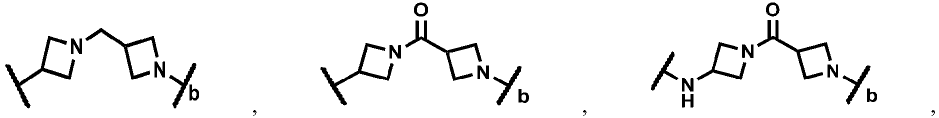 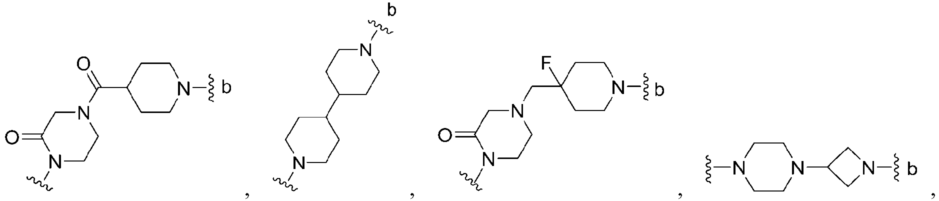 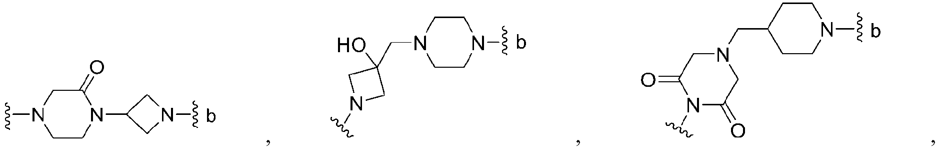 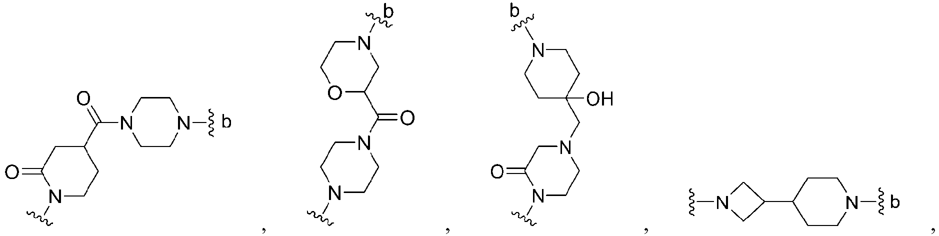 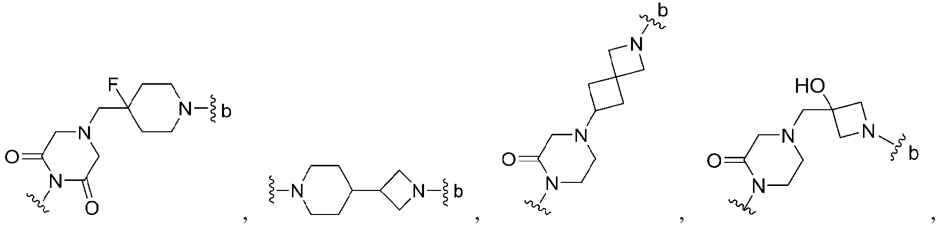 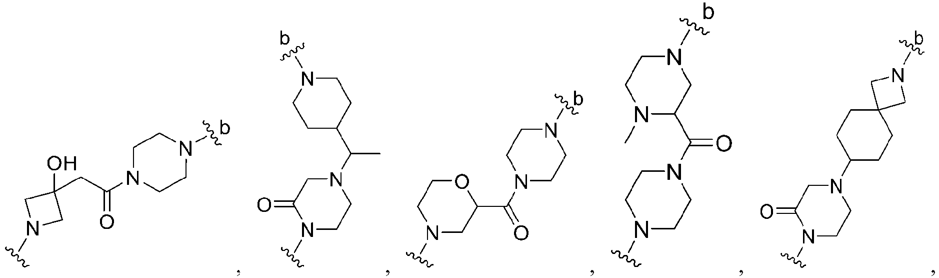 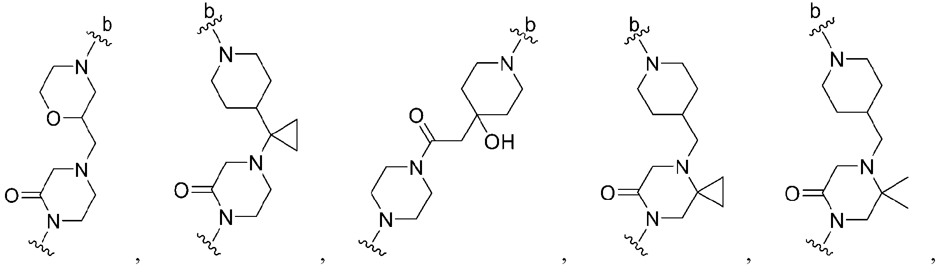 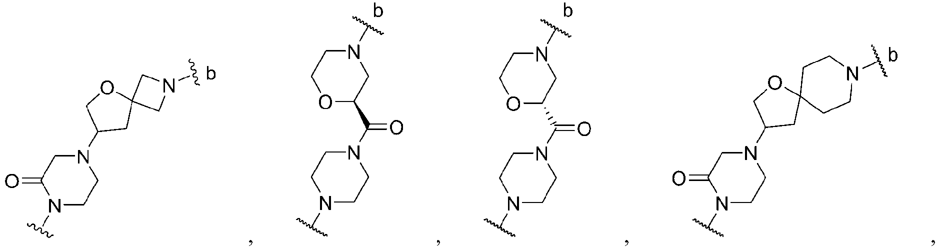 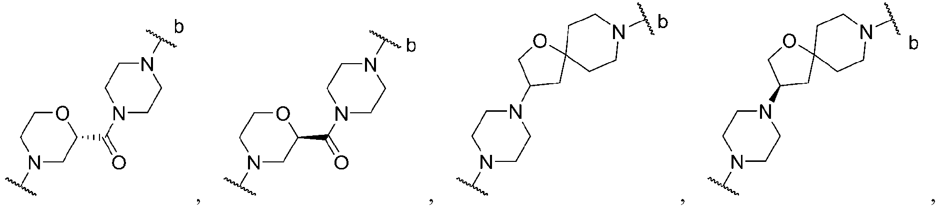 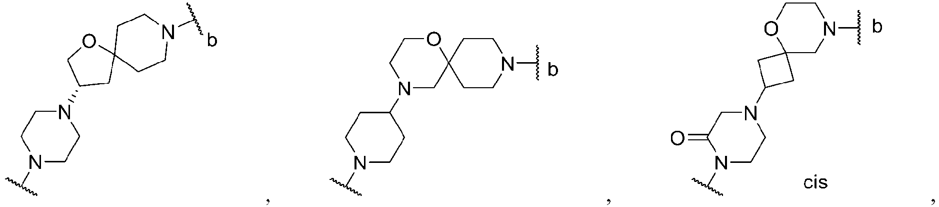 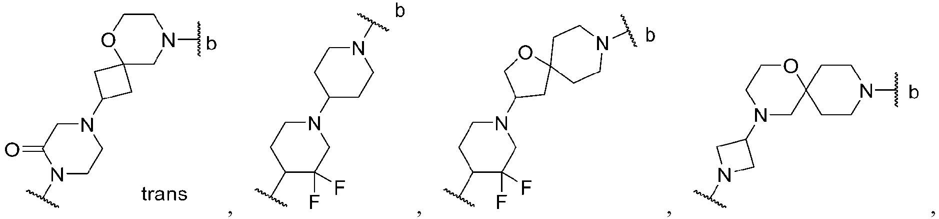 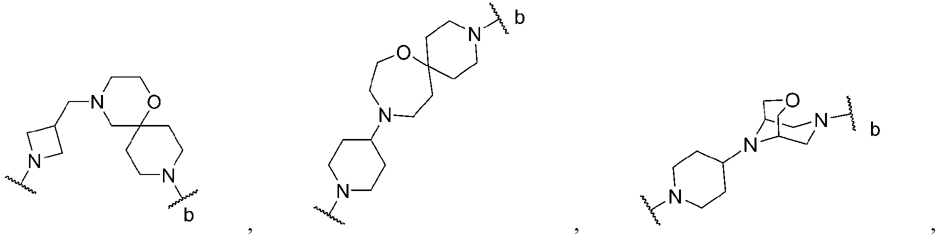 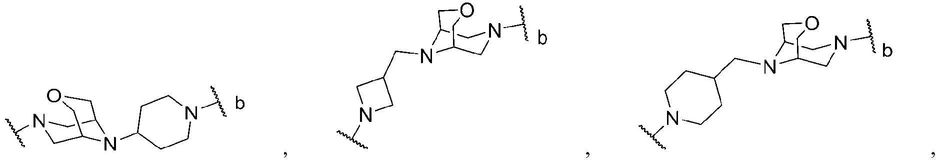 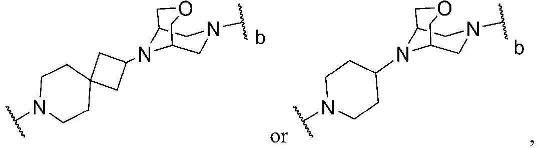
side b is connected to B.

In one embodiment of the present invention, L is 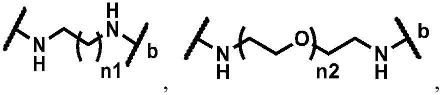 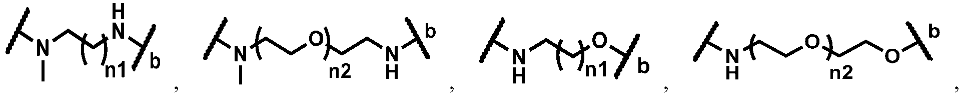 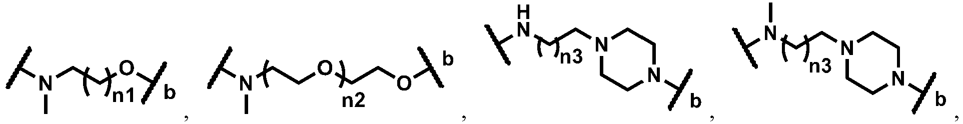 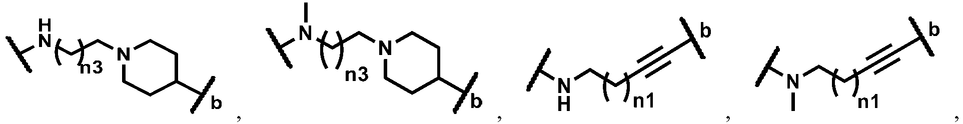 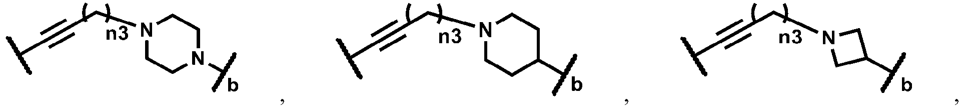 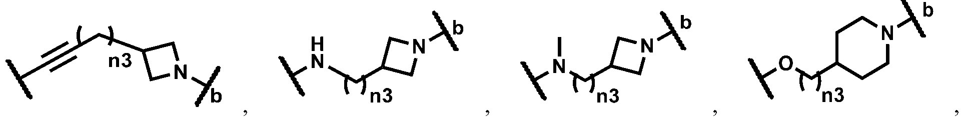 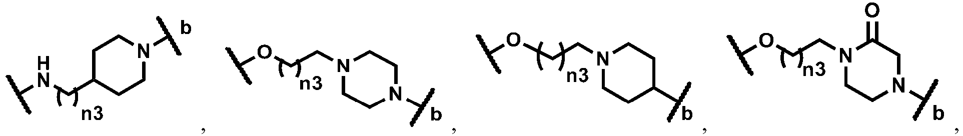 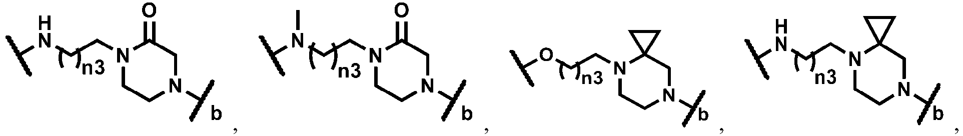 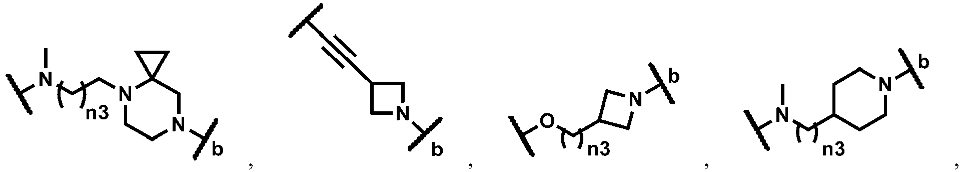 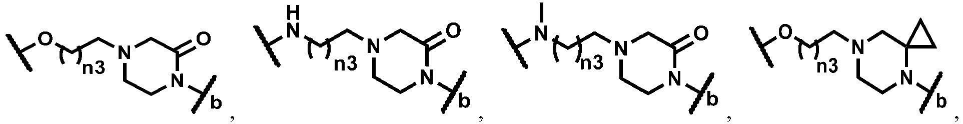 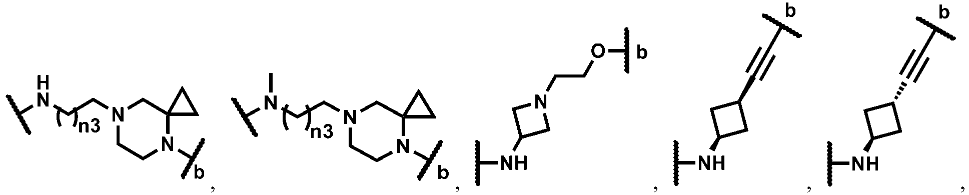 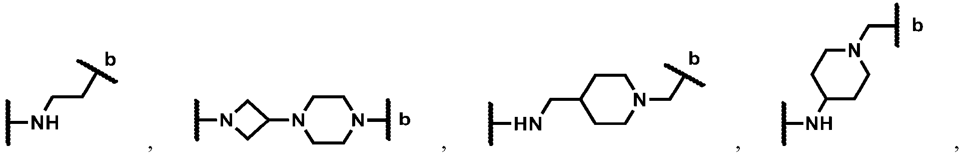 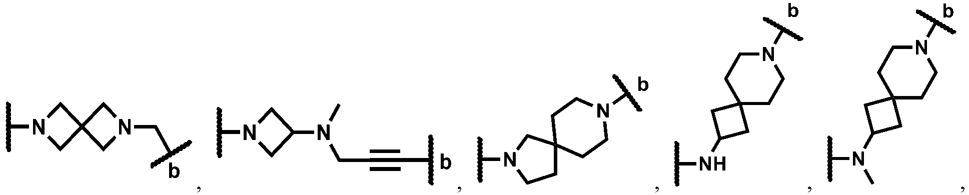 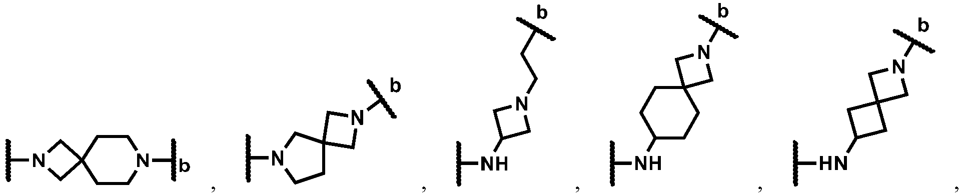 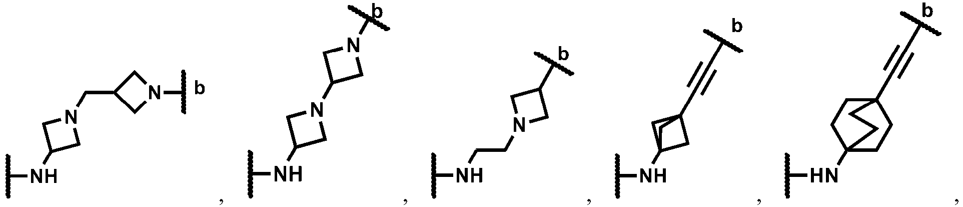 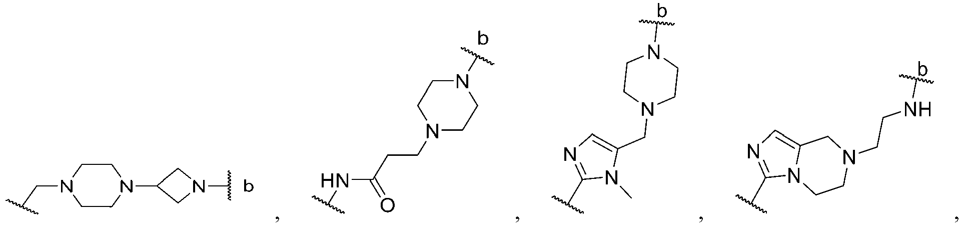 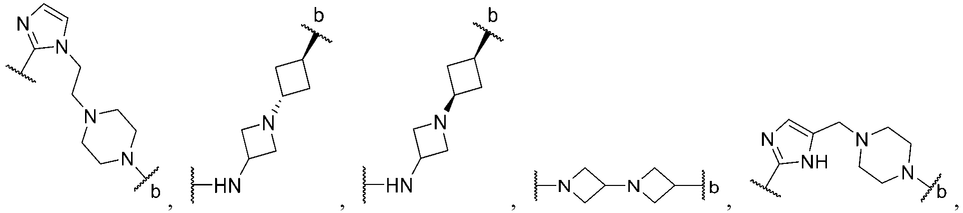 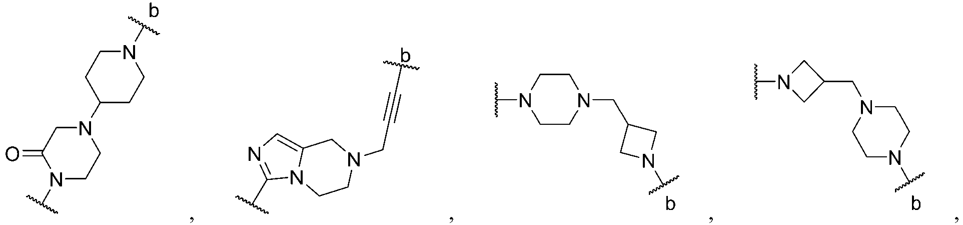 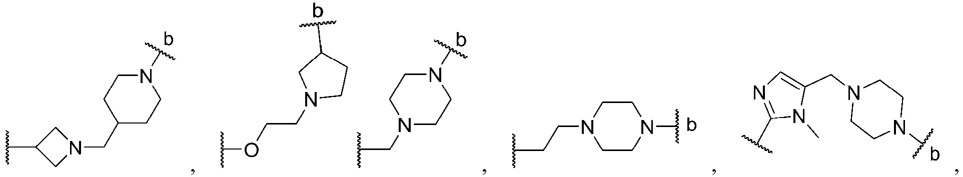 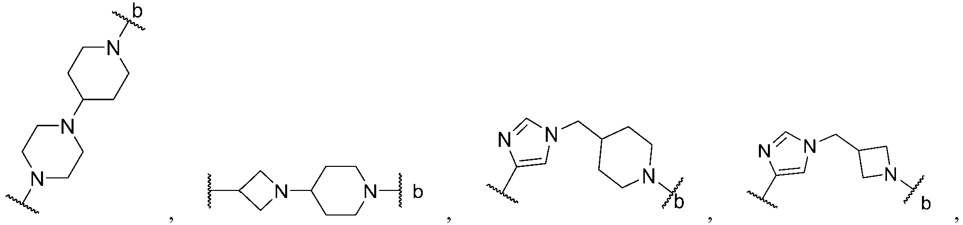 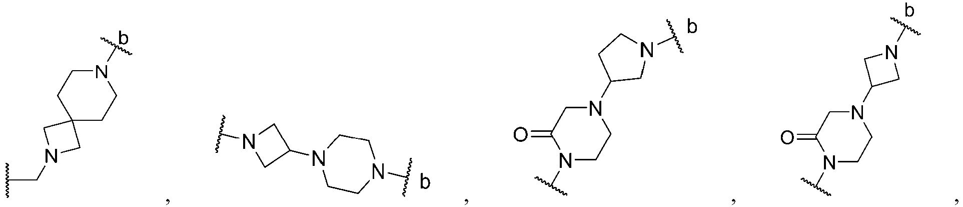 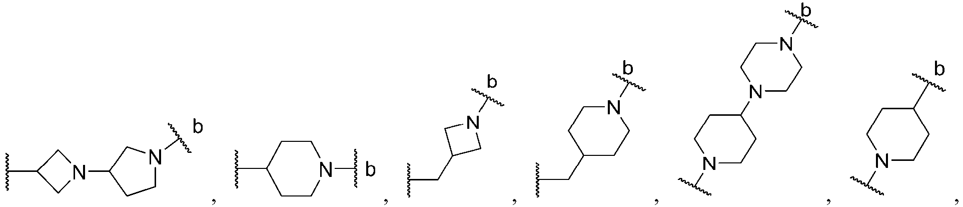 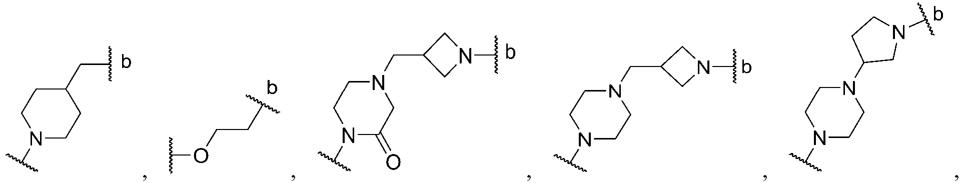 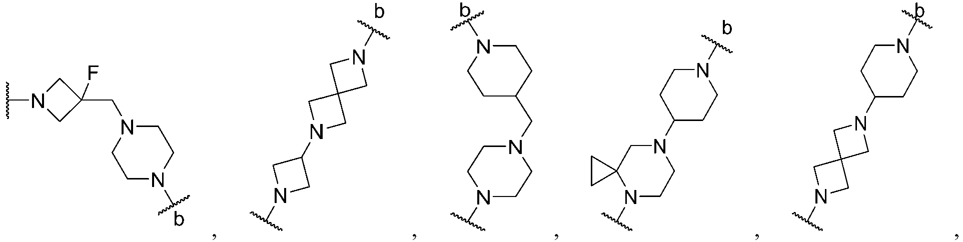 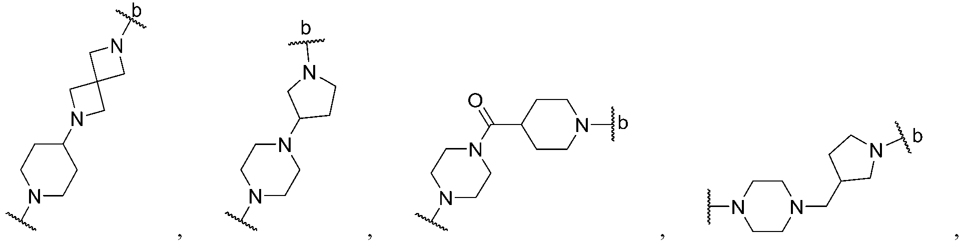 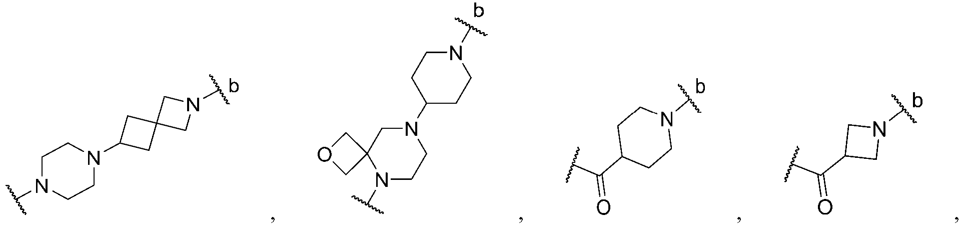 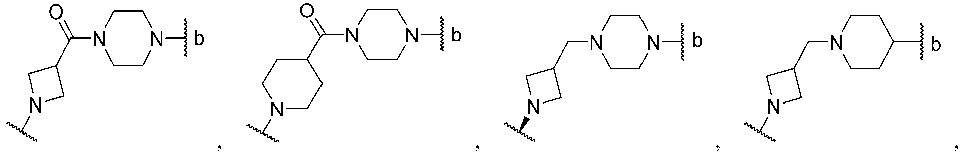 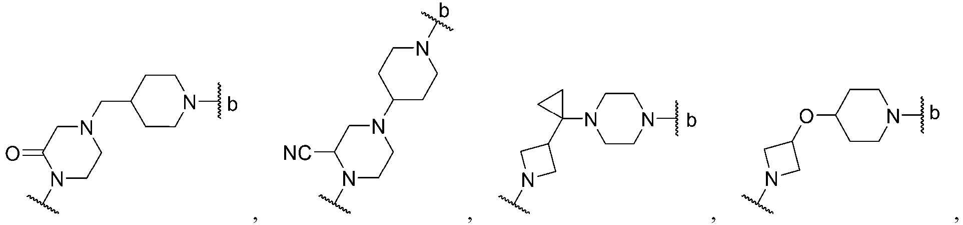 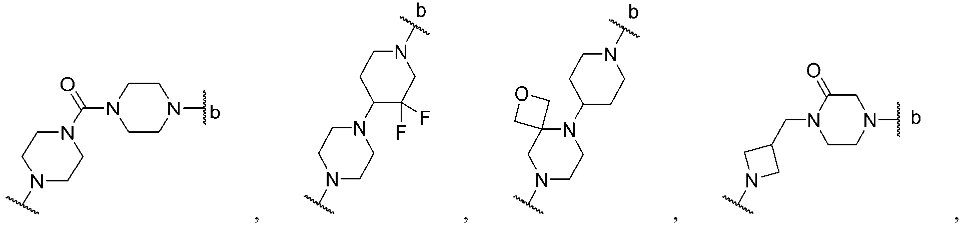 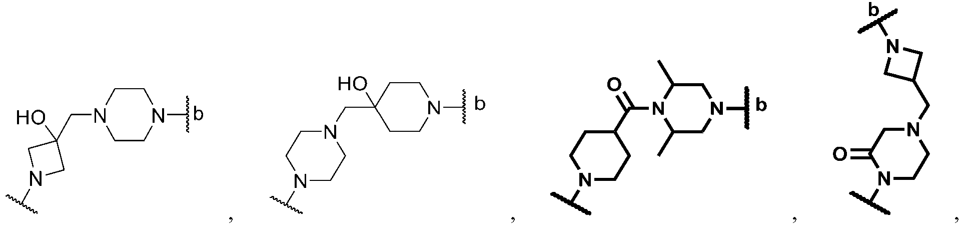 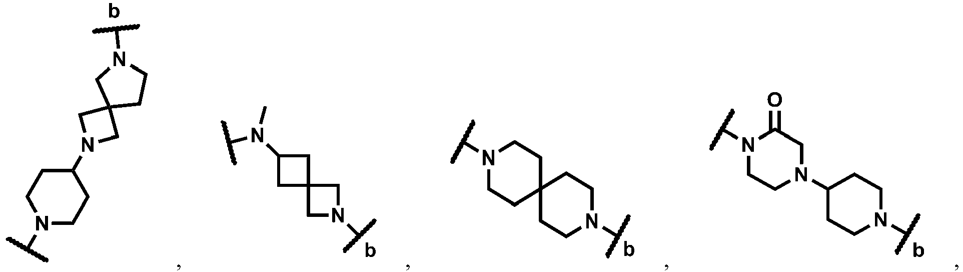 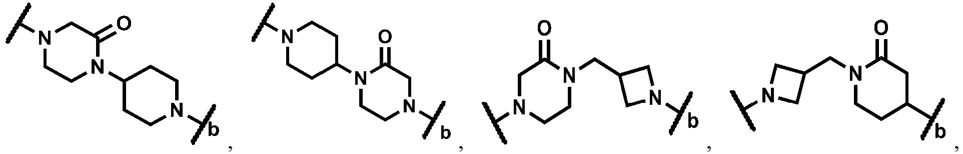 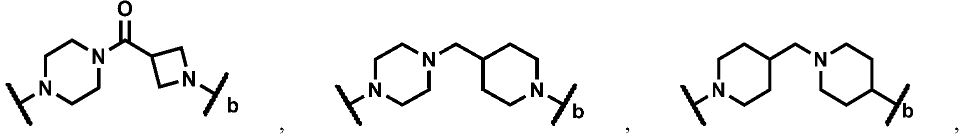 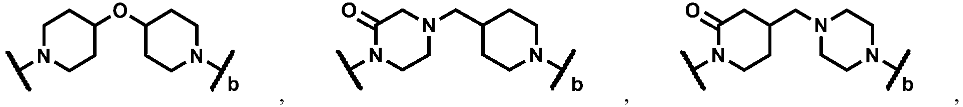 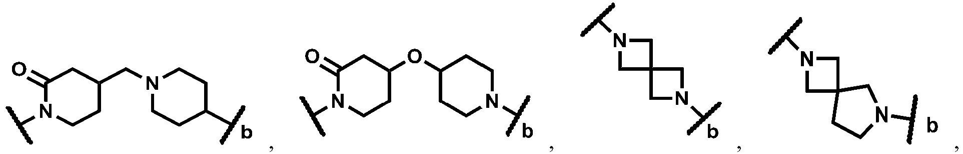 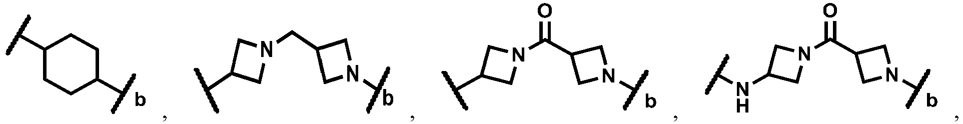 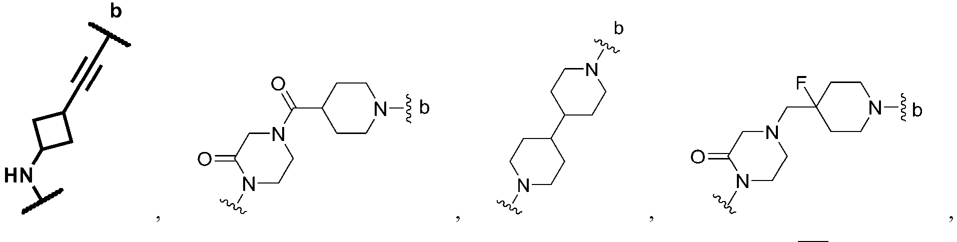 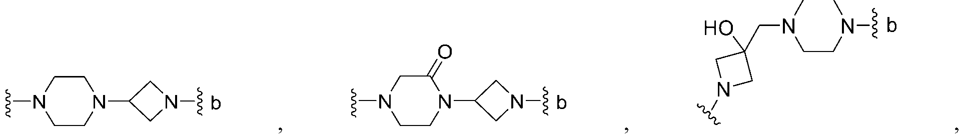 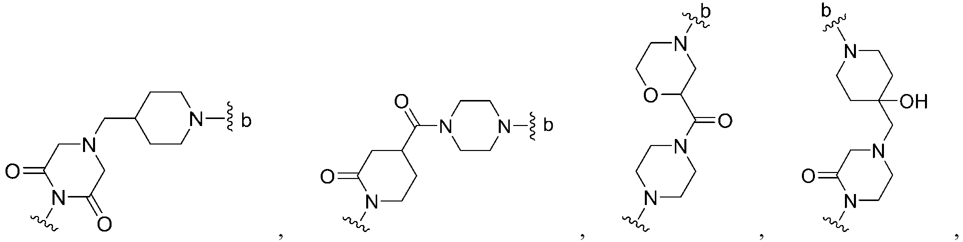 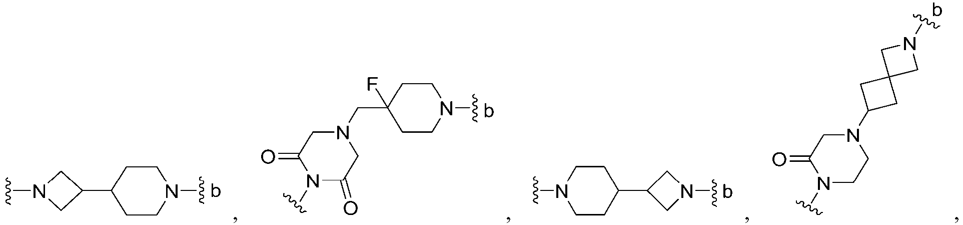 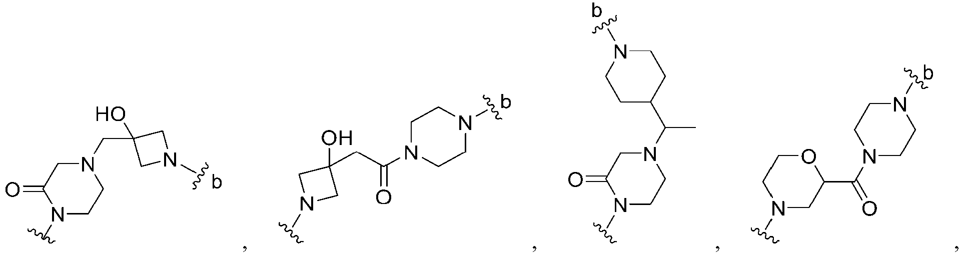 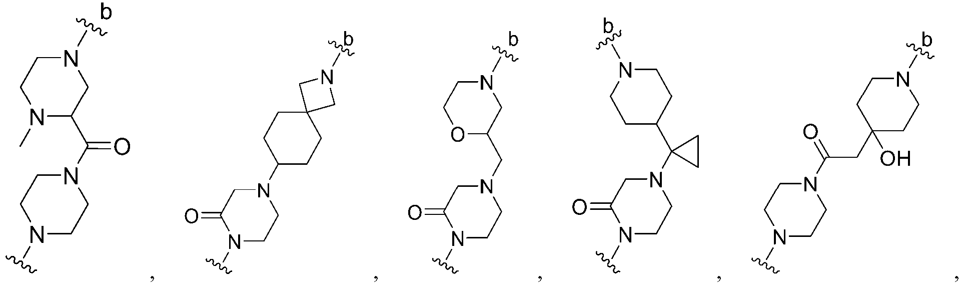 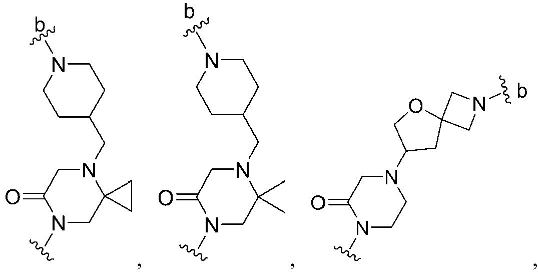 wherein n1 is any integer from 1 to 8, n2 is any integer from 1 to 3, n3 is any integer from 1 to 5, and side b is connected to B; preferably, in Scheme i, L is any group of the above groups except 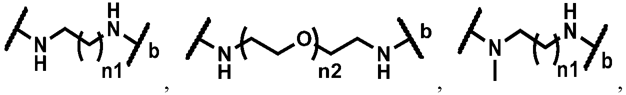 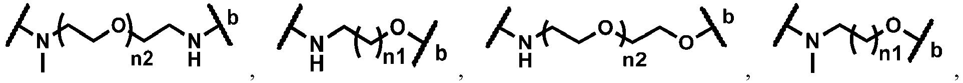 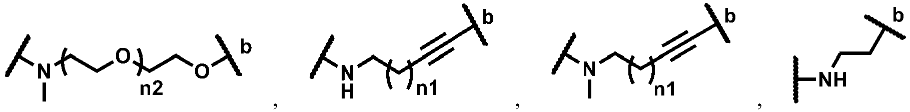 and

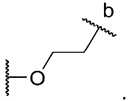

In one embodiment of the present invention, L is 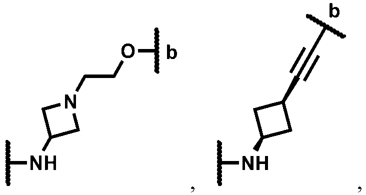 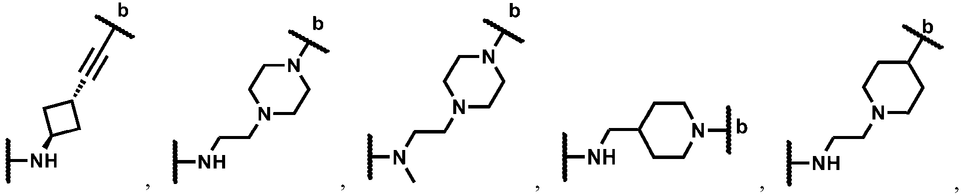 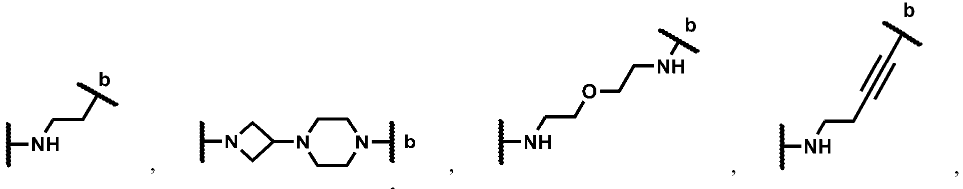 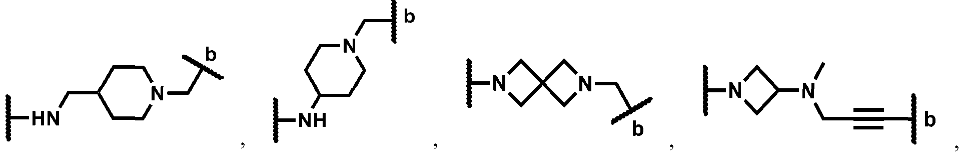 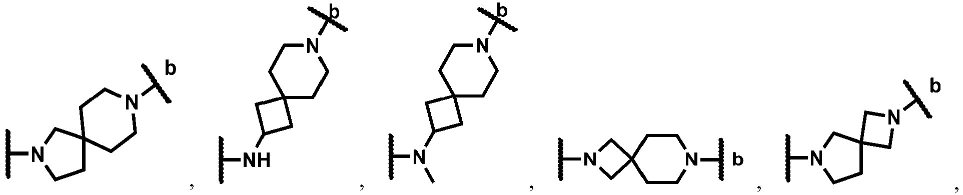 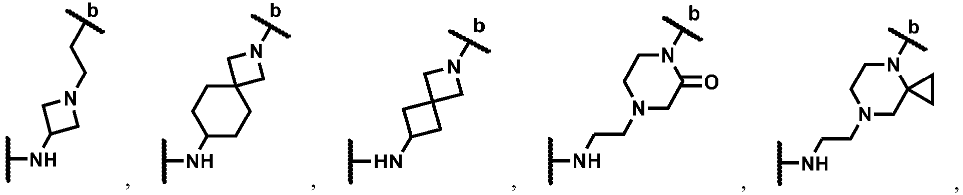 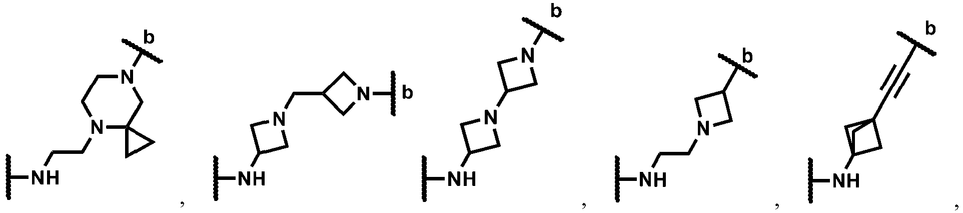 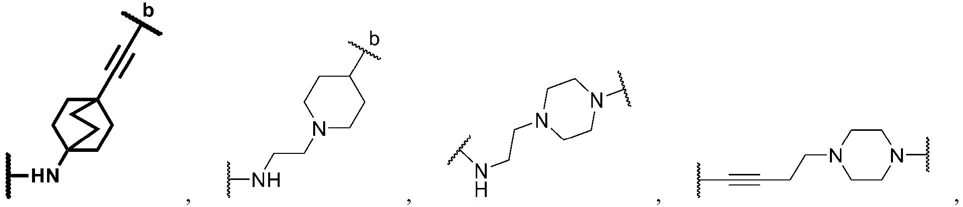 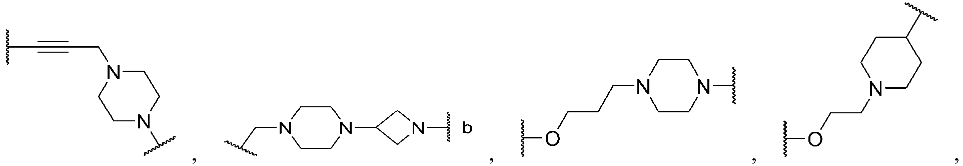 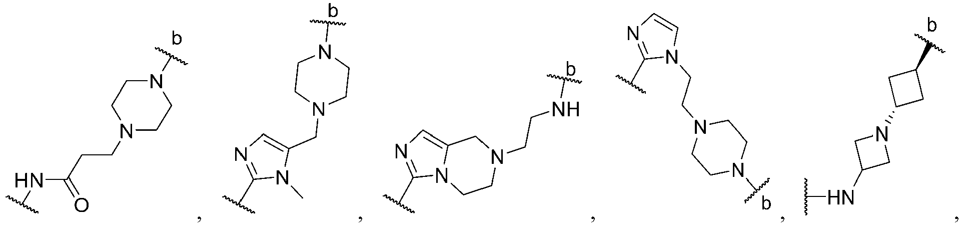 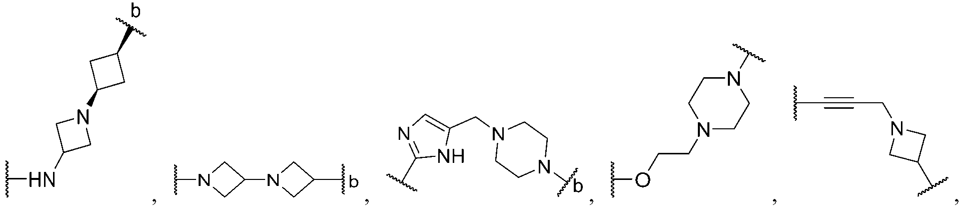 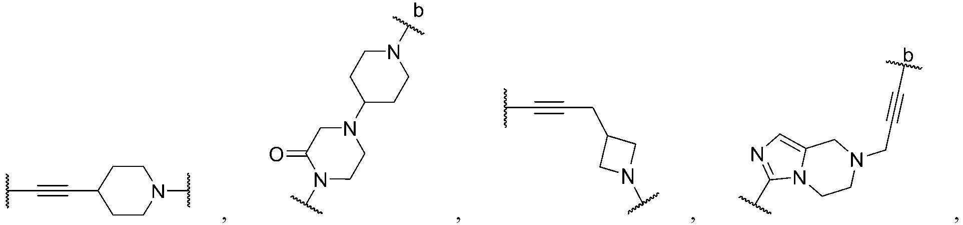 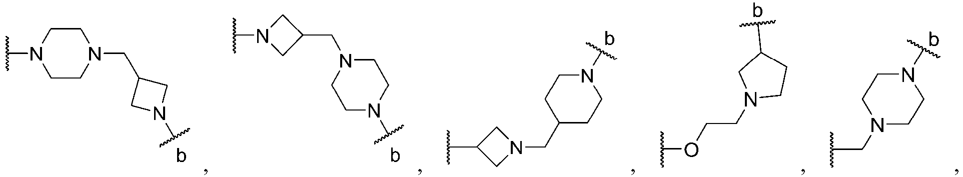 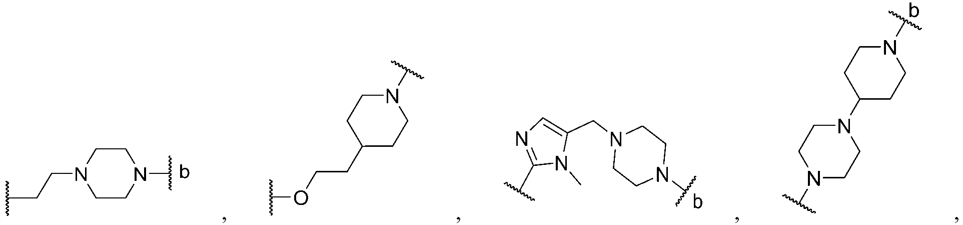 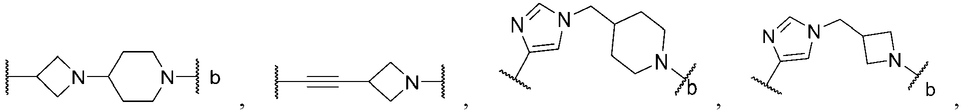 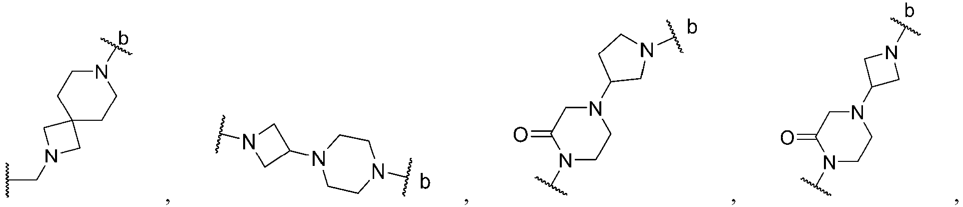 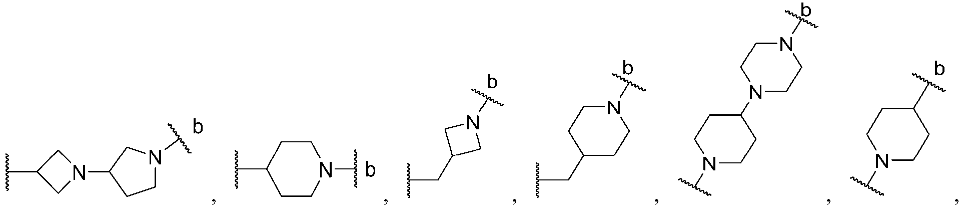 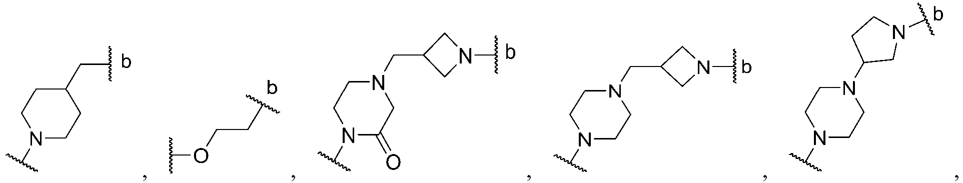 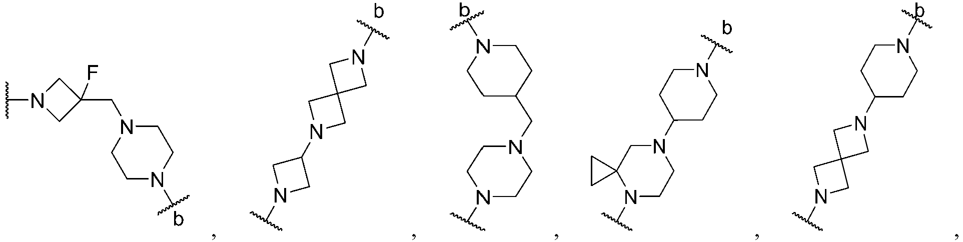 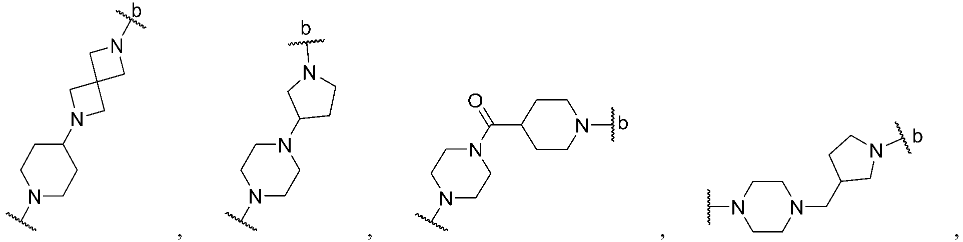 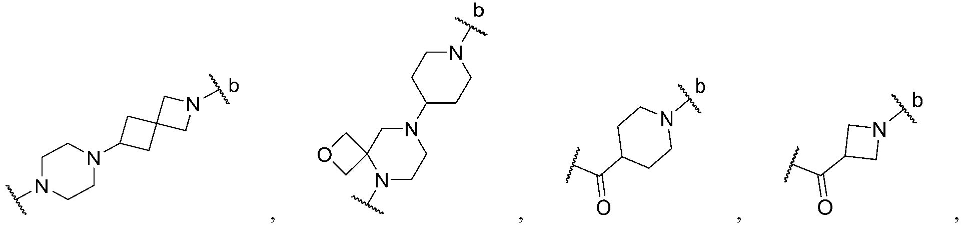 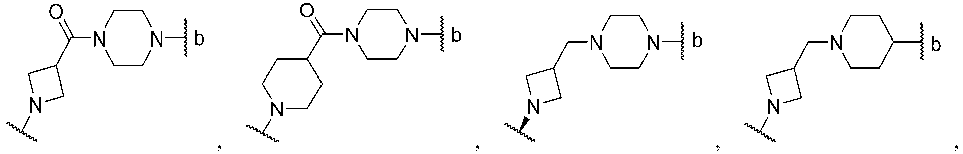 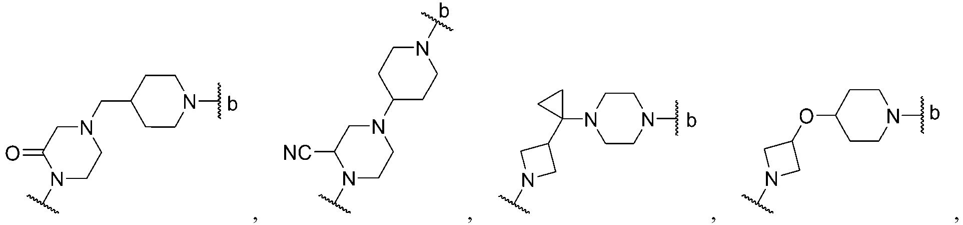 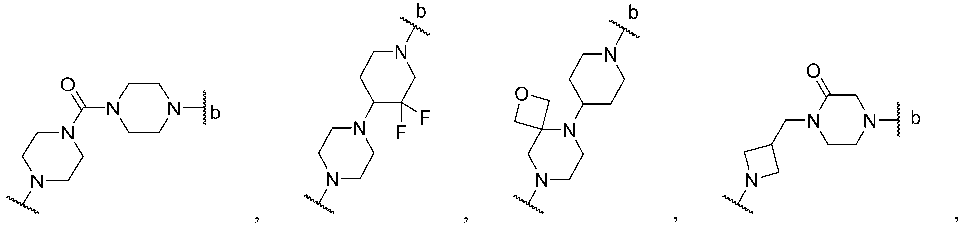 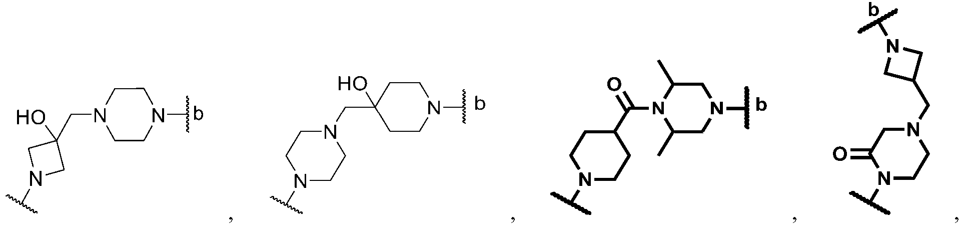 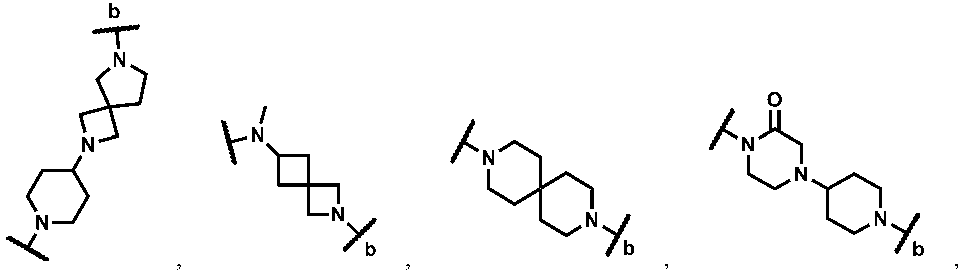 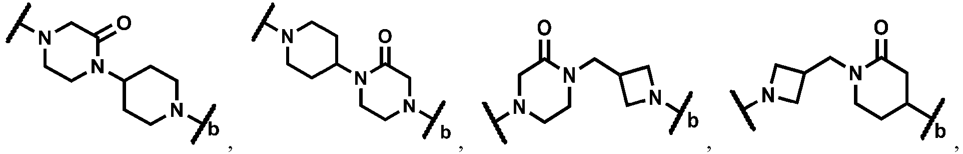 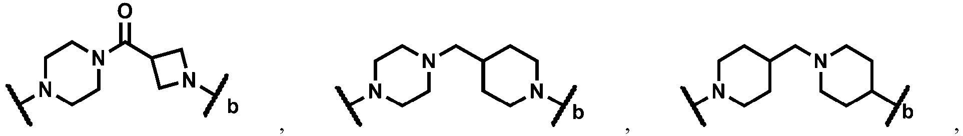 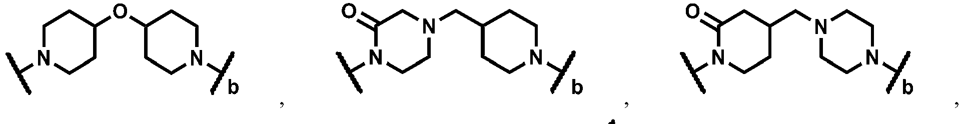 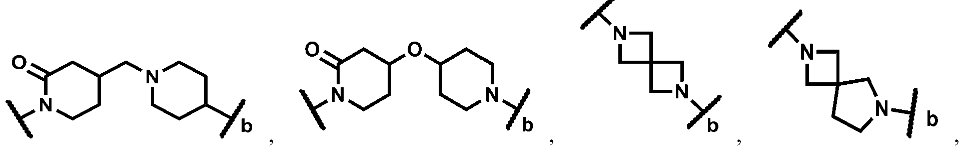 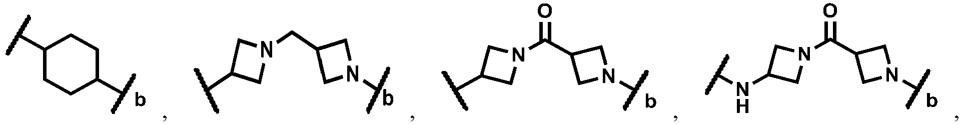 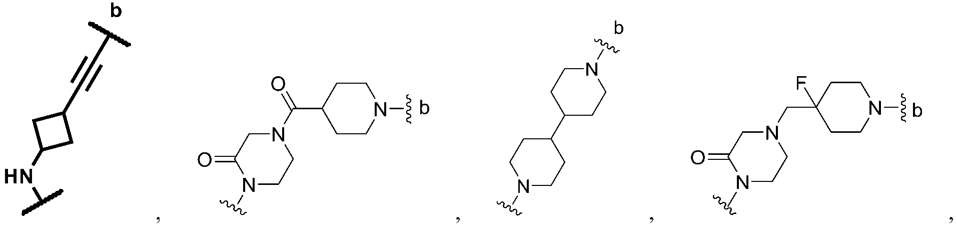 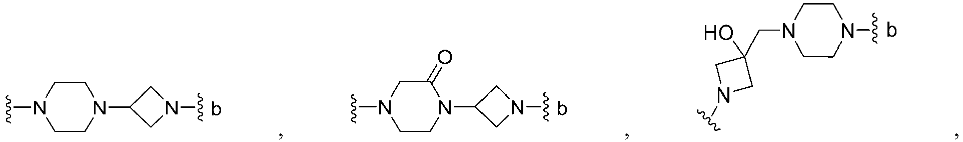 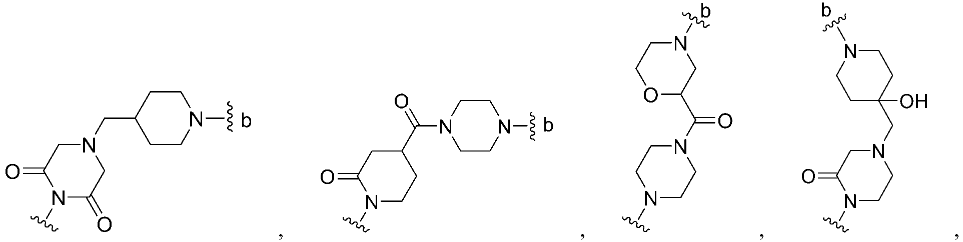 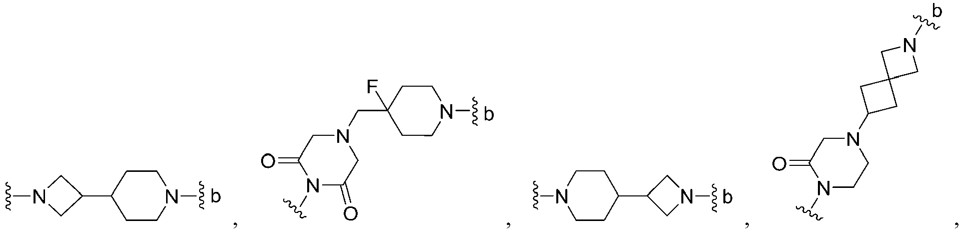 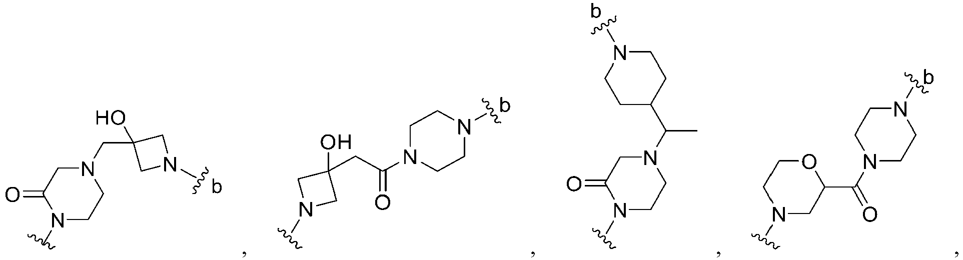 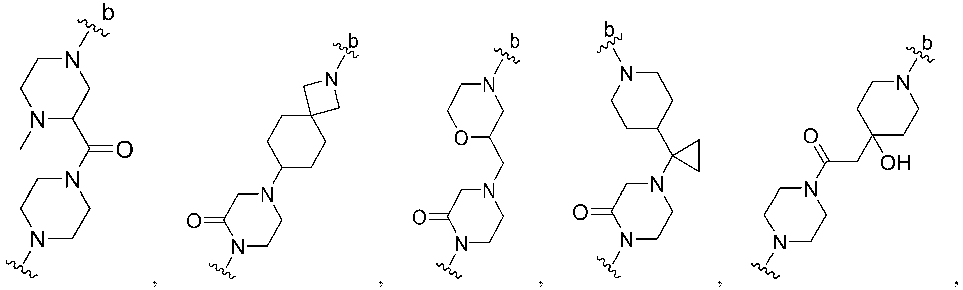 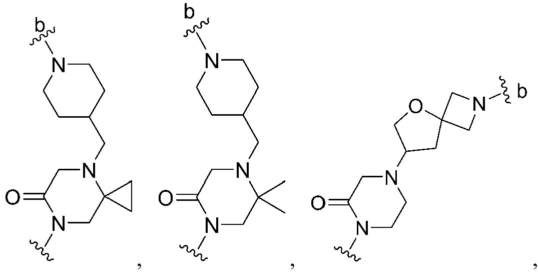 wherein side b is connected to B; preferably, in Scheme i, L is any one of the above groups except

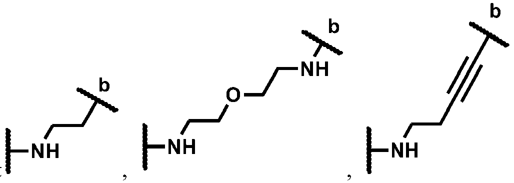

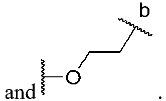

In one embodiment of the present invention, L is: 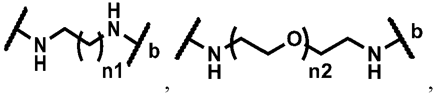 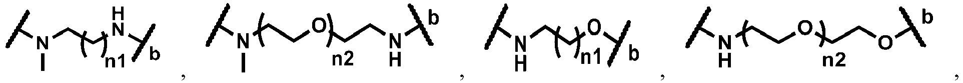 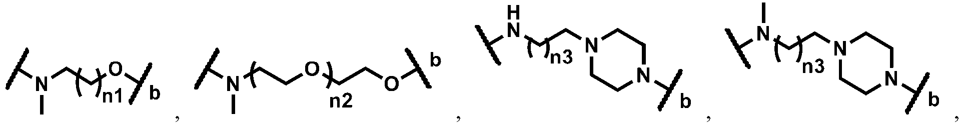 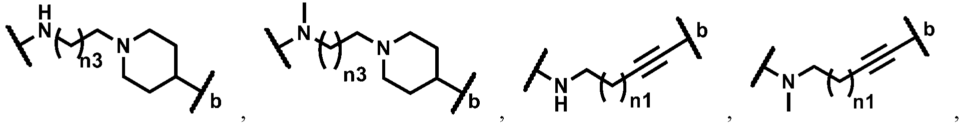 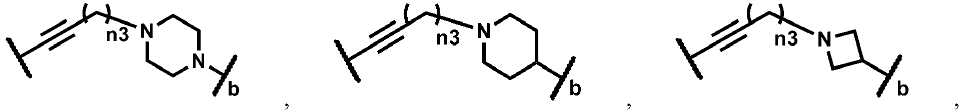 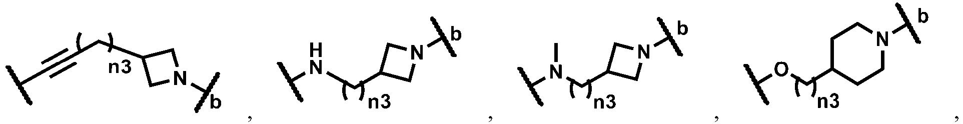 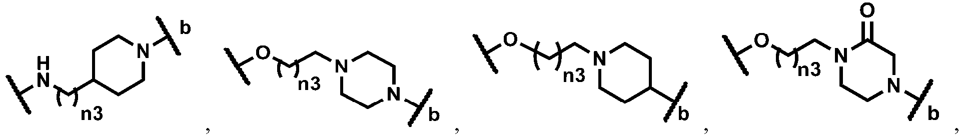 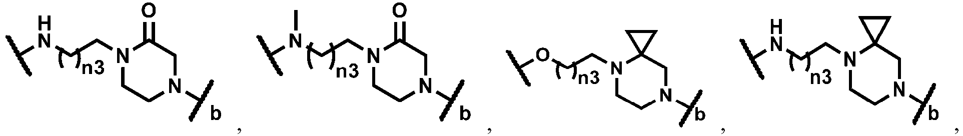 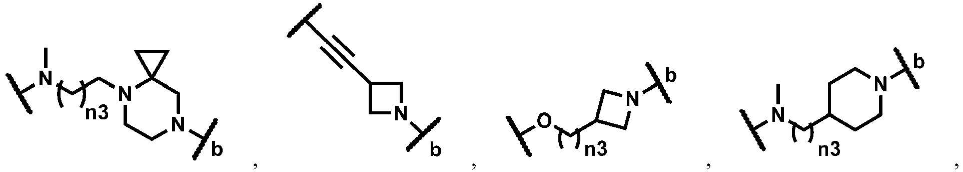 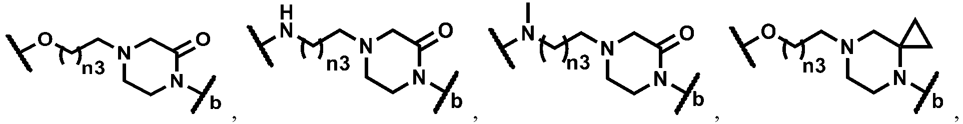 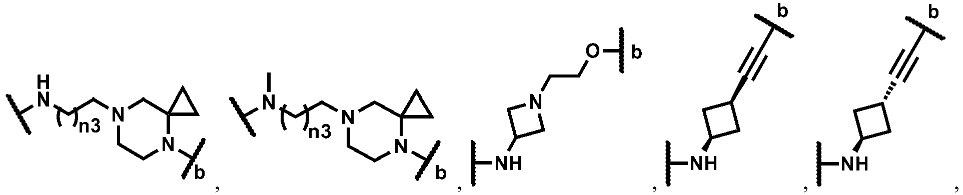 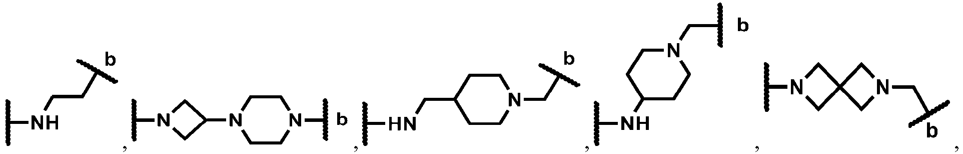 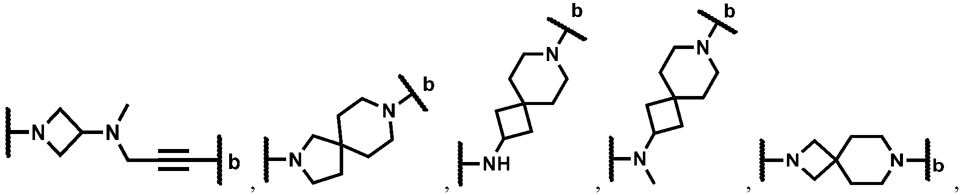 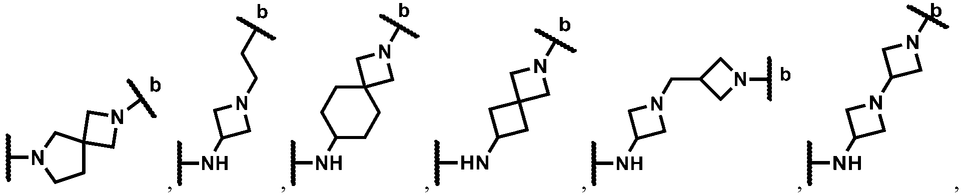 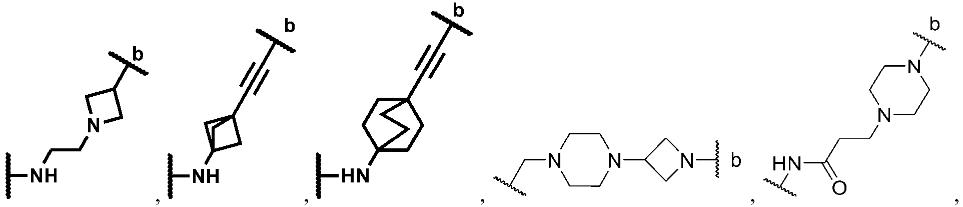 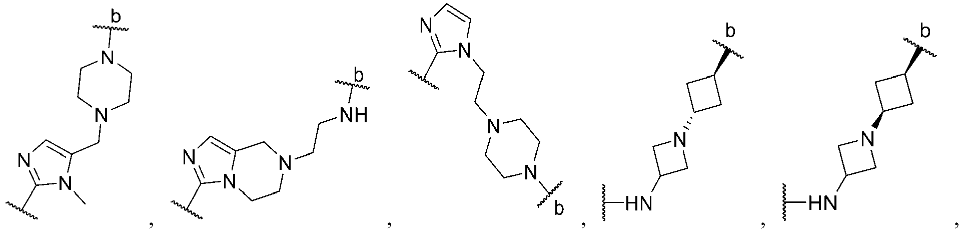 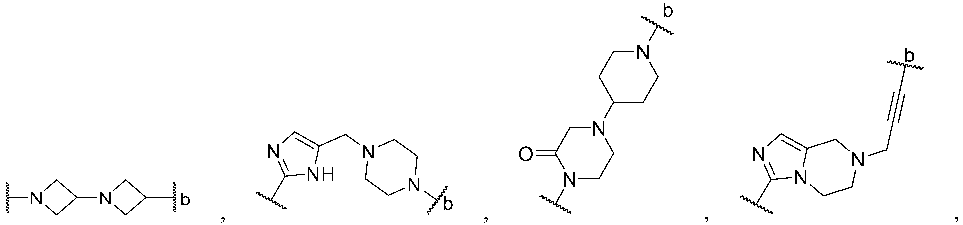 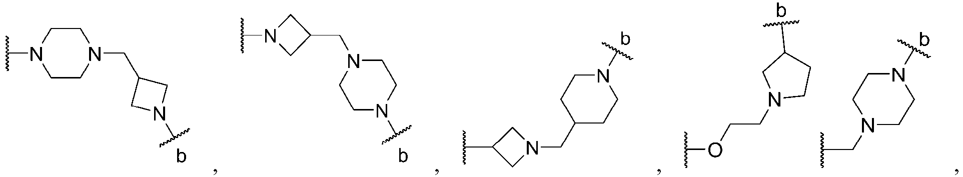 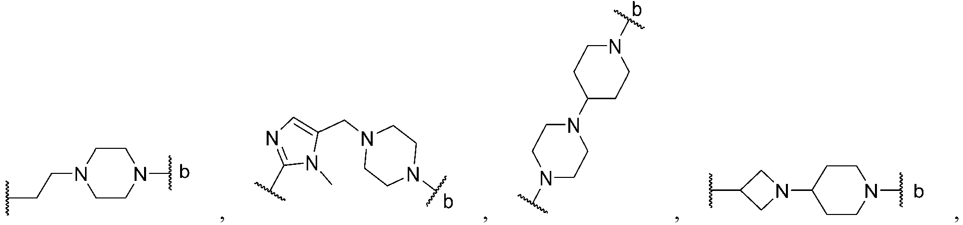 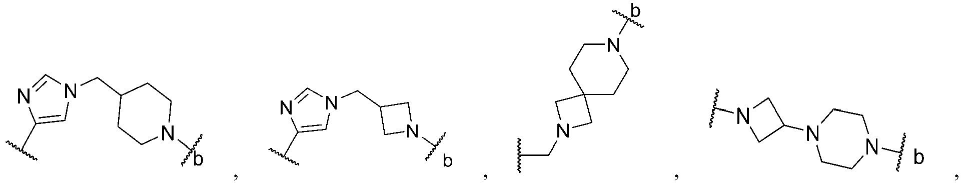 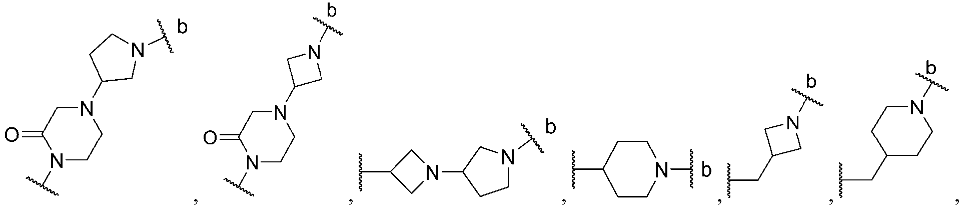 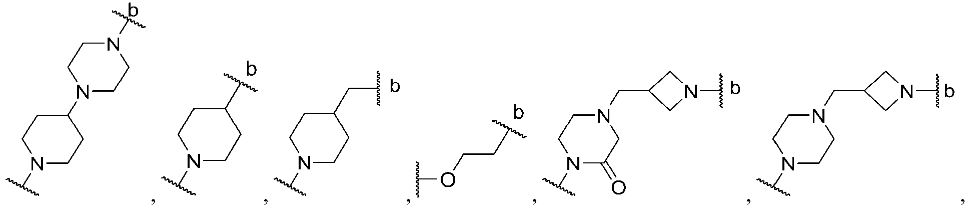 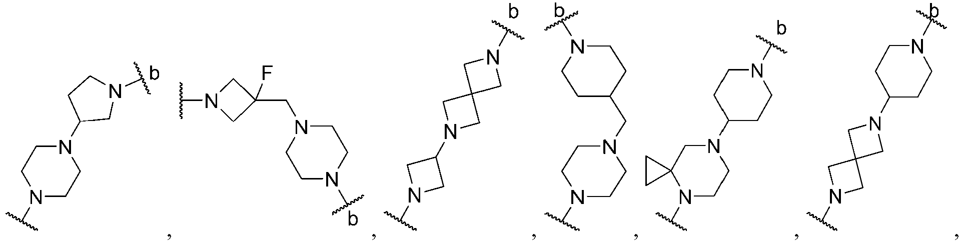 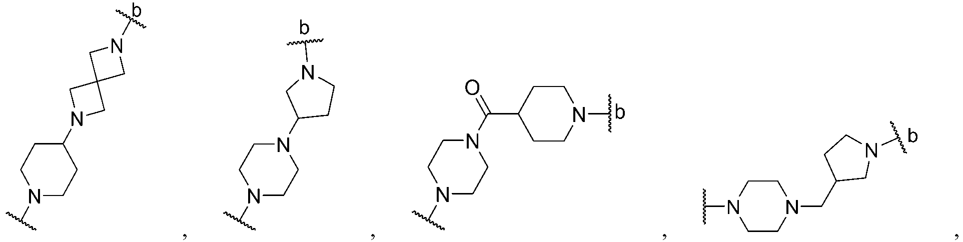 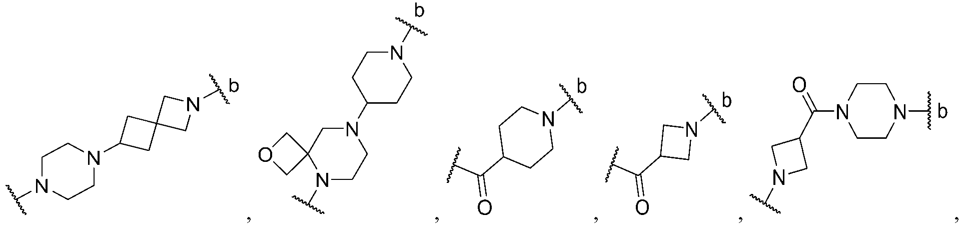 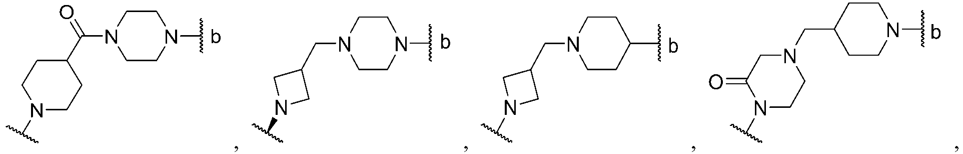 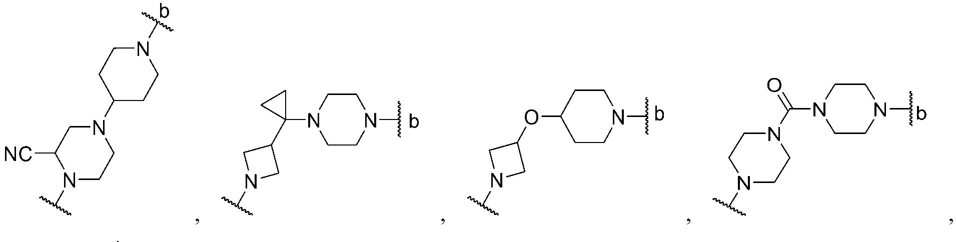 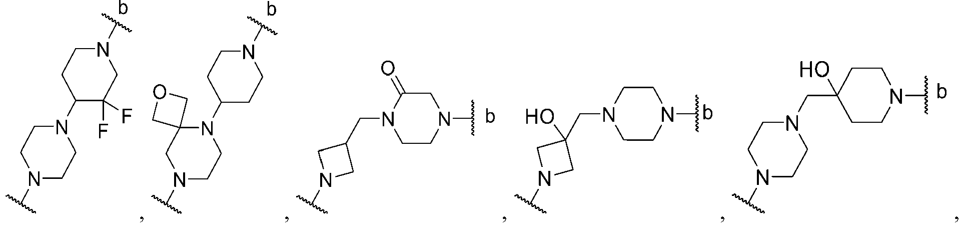 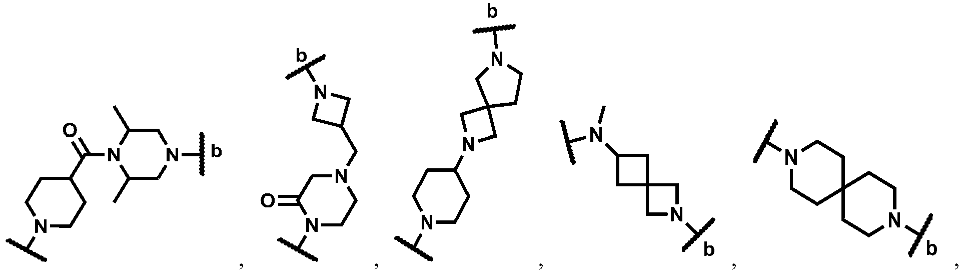 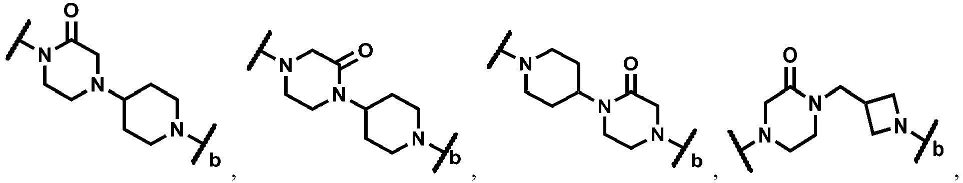 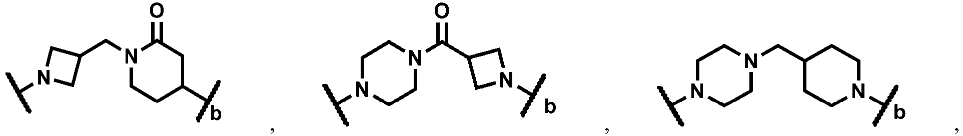 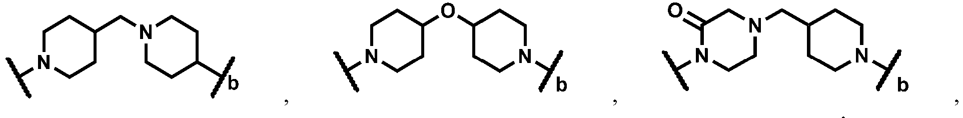 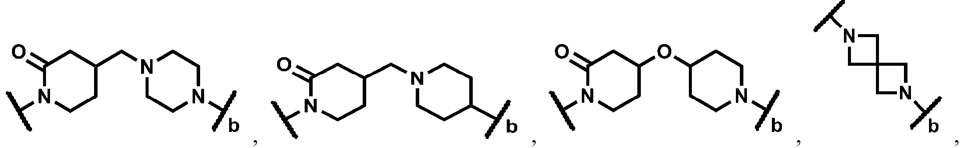 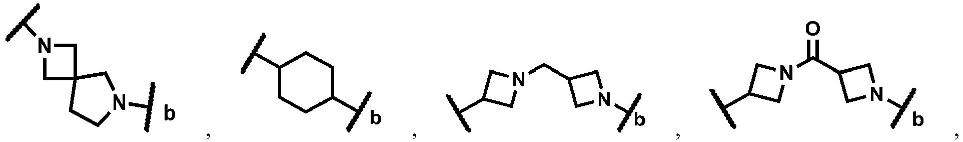 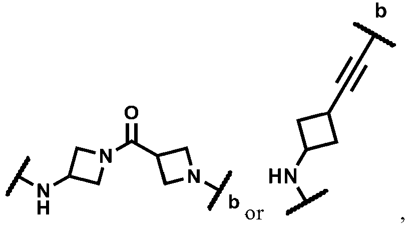 wherein n1 is any integer from 1 to 8, n2 is any integer from 1 to 3, n3 is any integer from 1 to 5, and side b is connected to B; preferably, in Scheme i, L is any one of the above groups except

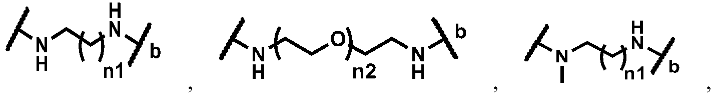

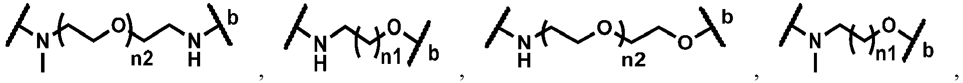

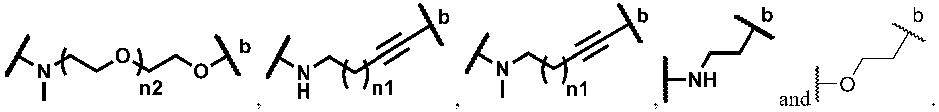

In one embodiment of the present invention, L is: 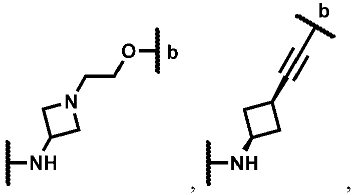 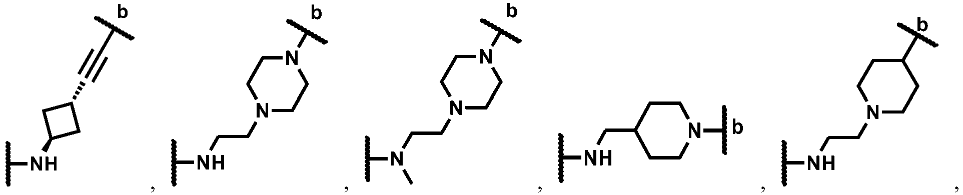 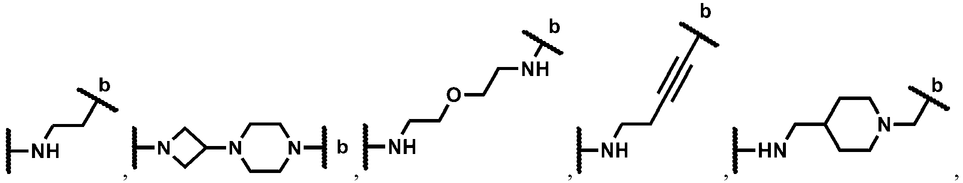 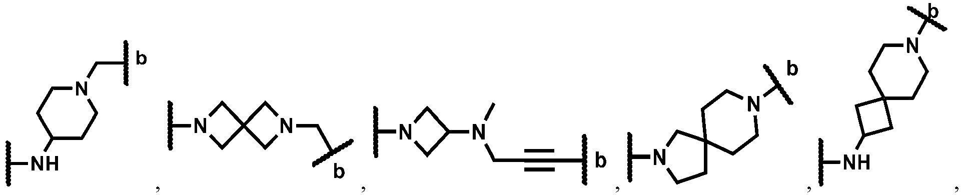 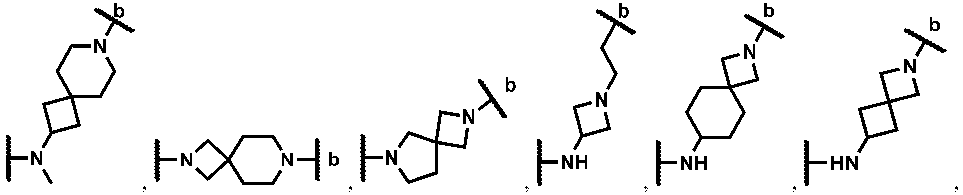 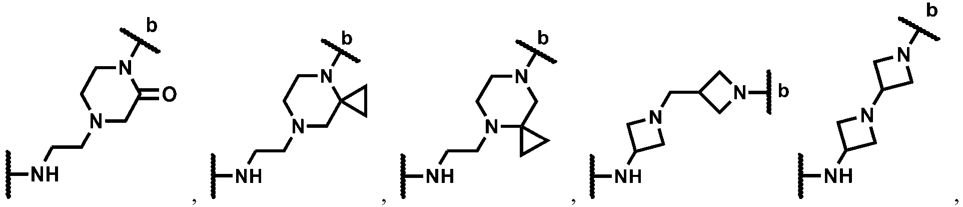 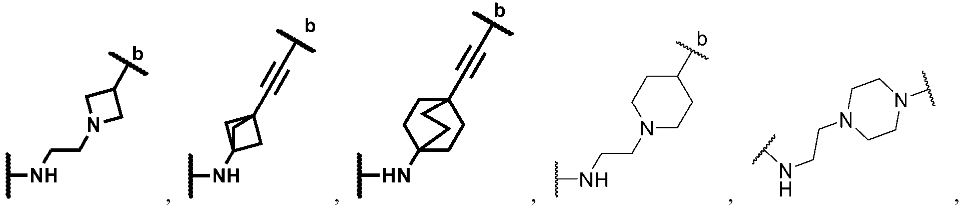 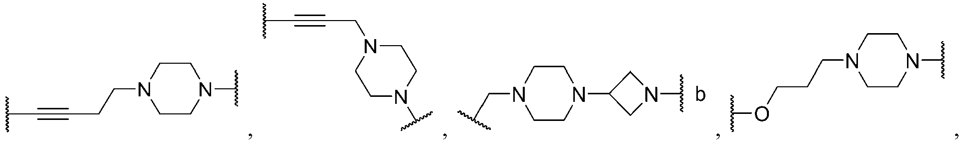 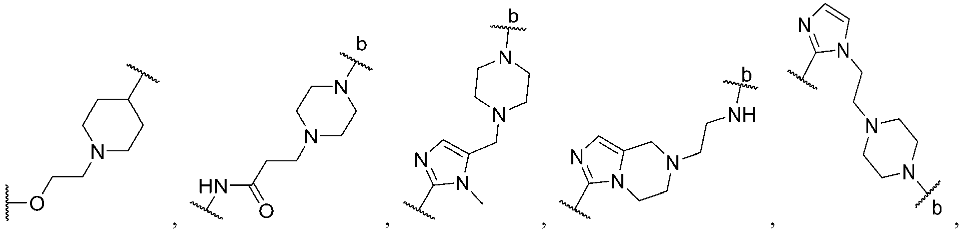 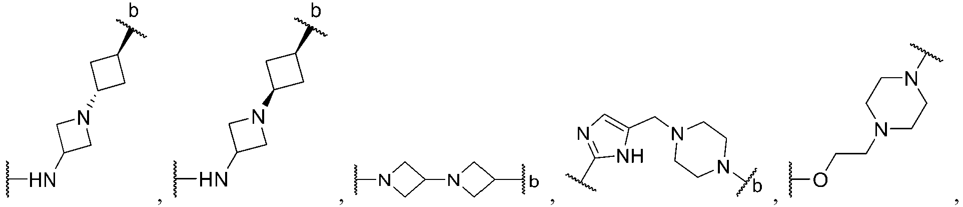 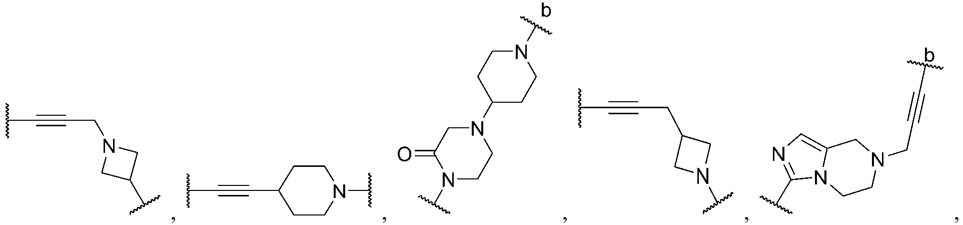 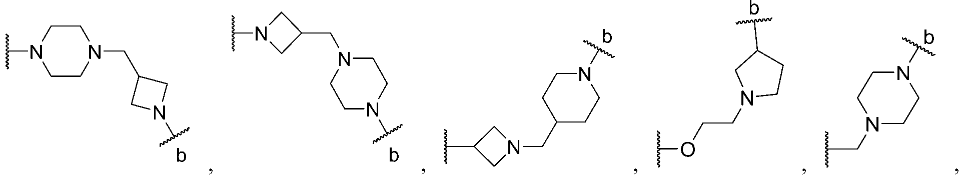 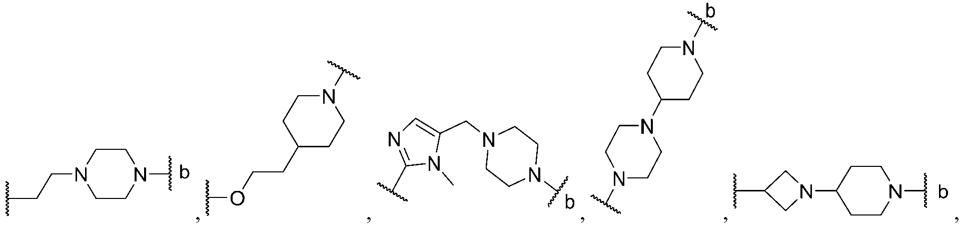 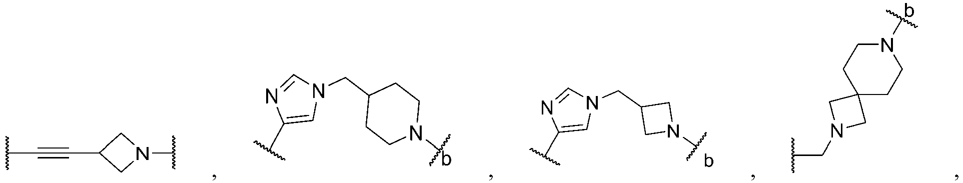 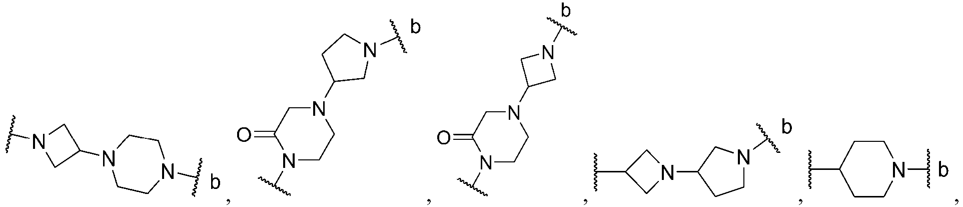 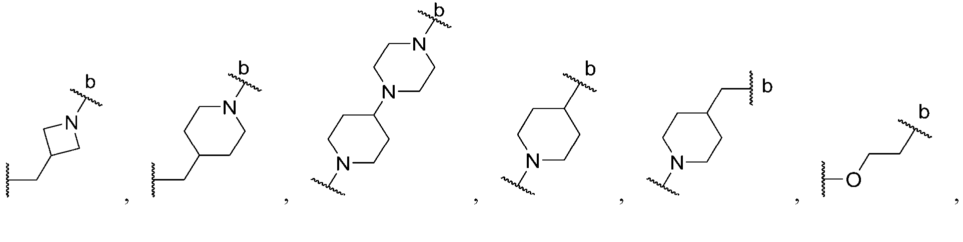 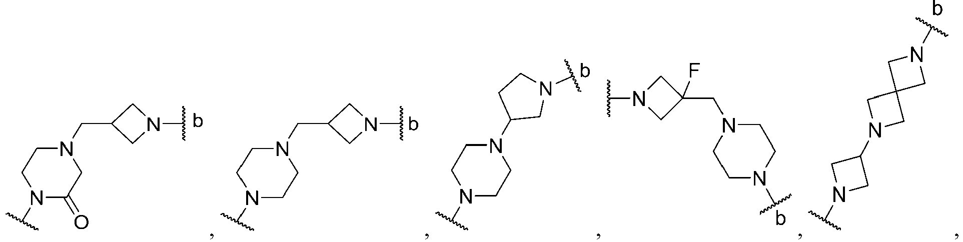 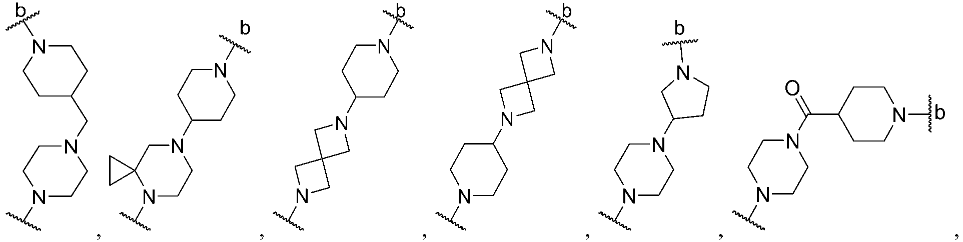 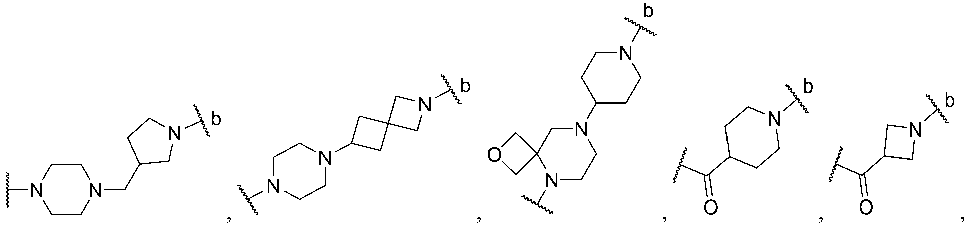 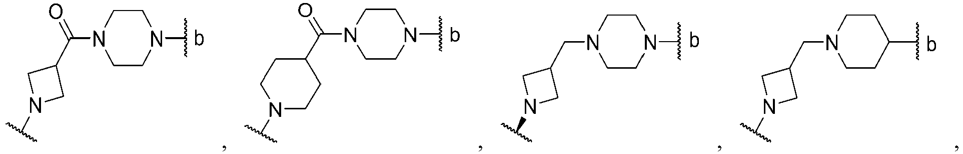 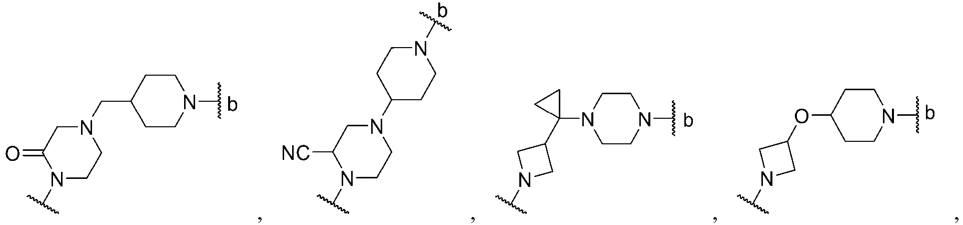 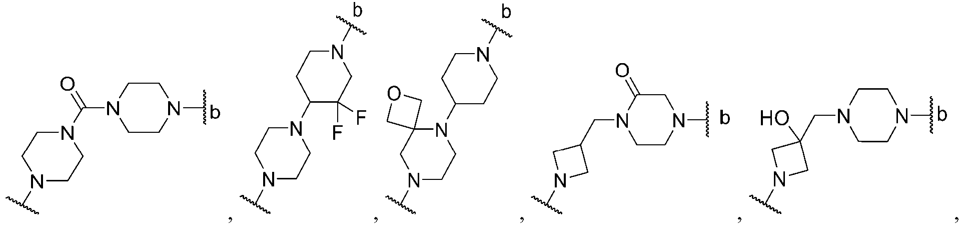 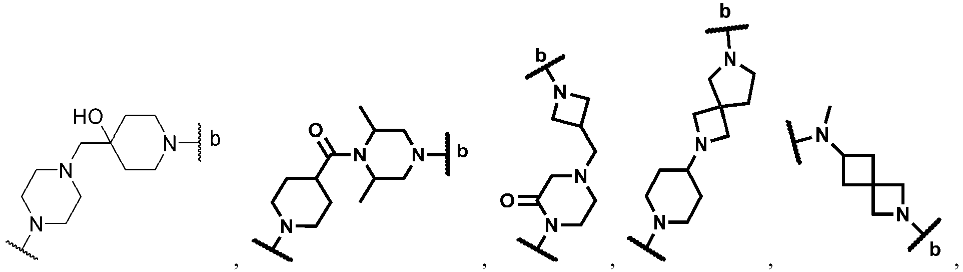 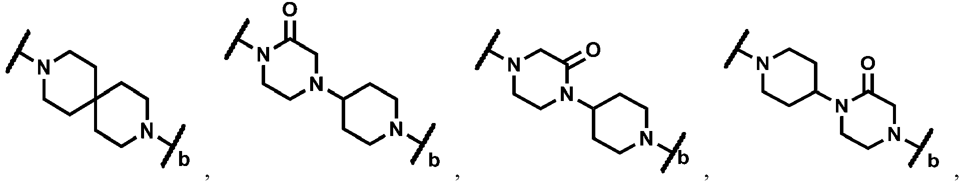 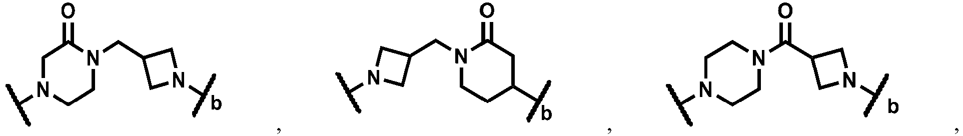 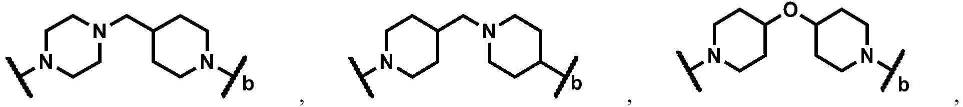 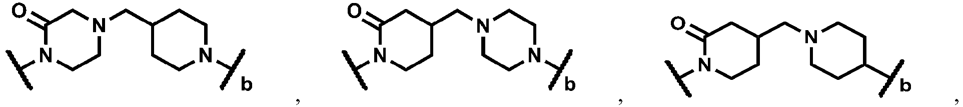 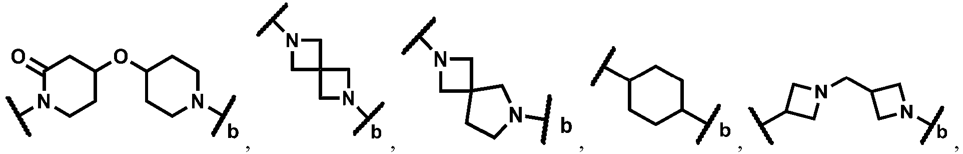 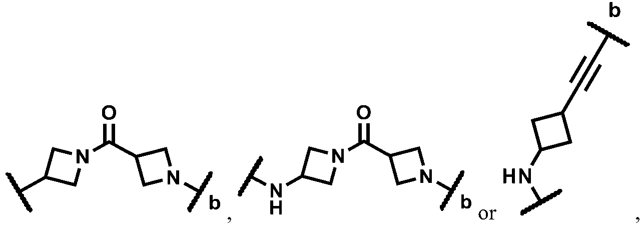 wherein side b is connected to B; preferably, in Scheme i, L is any one of the above groups except

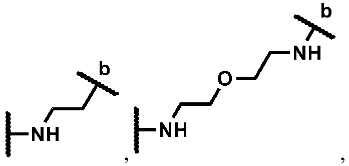

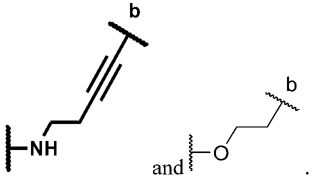

In one embodiment of the present invention, B is: 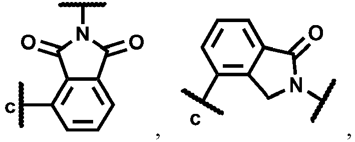 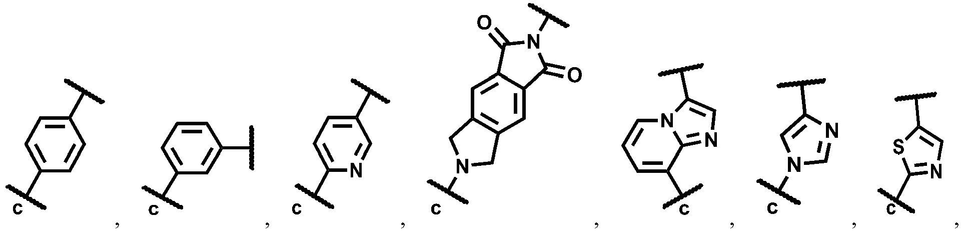 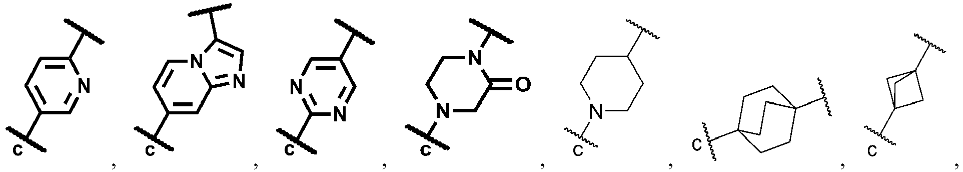 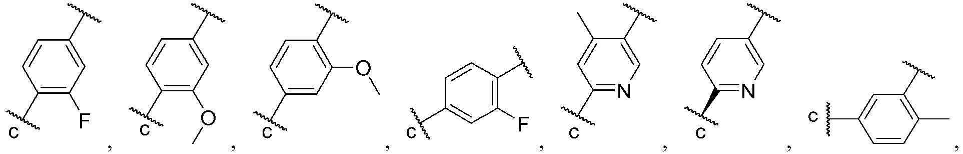 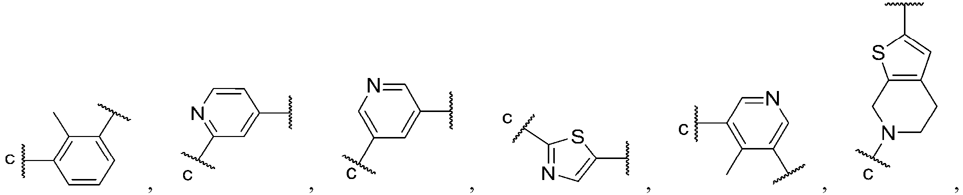 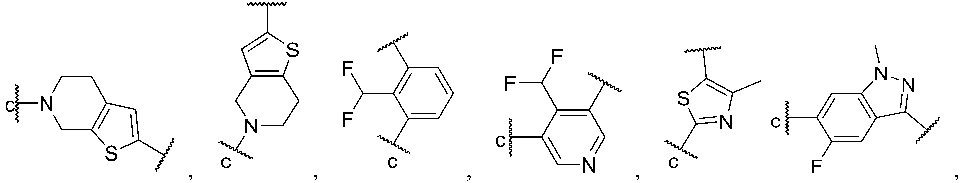 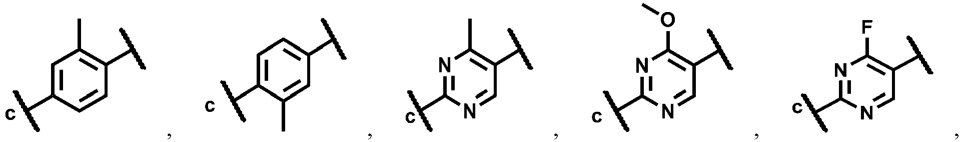 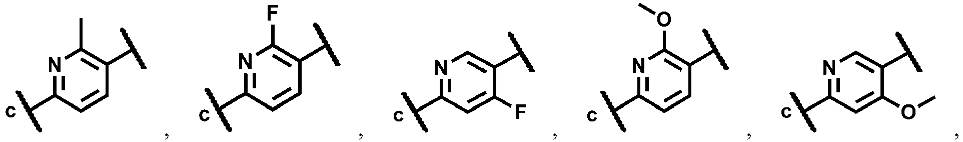 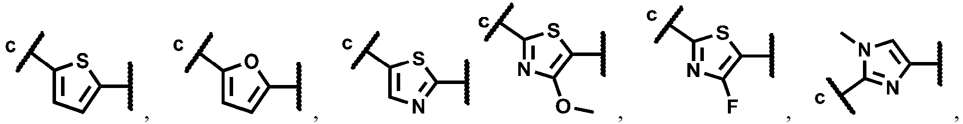 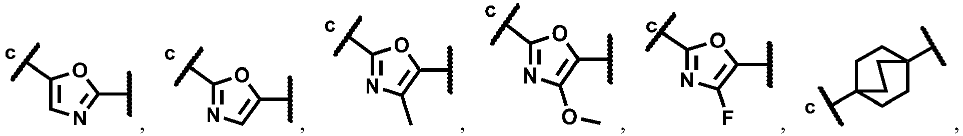 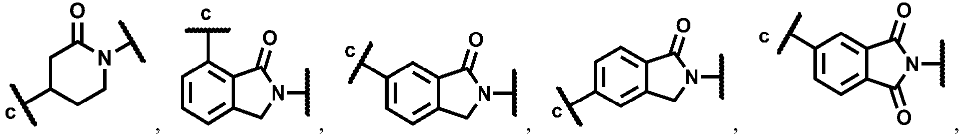 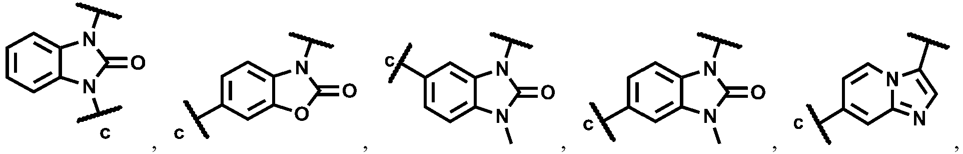 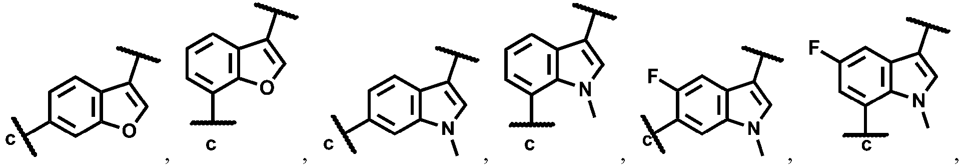 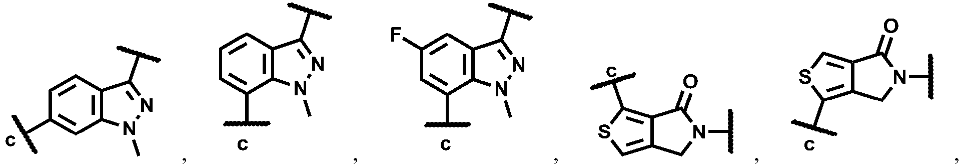 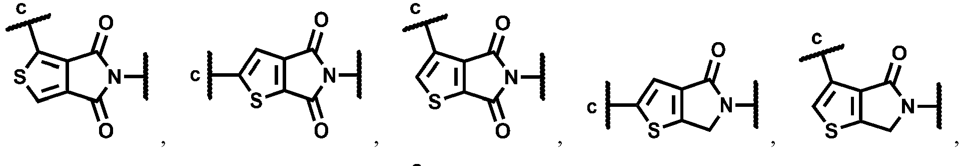 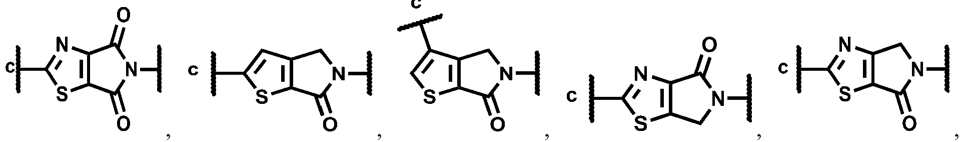 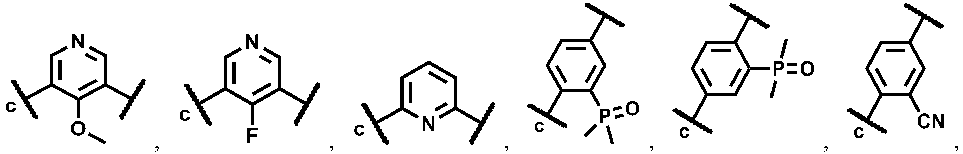 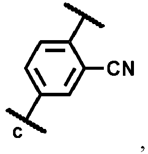 wherein side c is connected to L; in Scheme ii, B is any one of the above groups other than

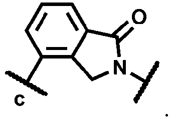

In one embodiment of the present invention, B is: 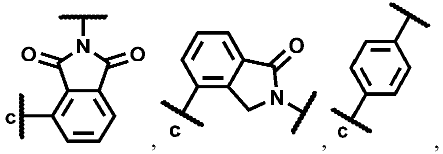 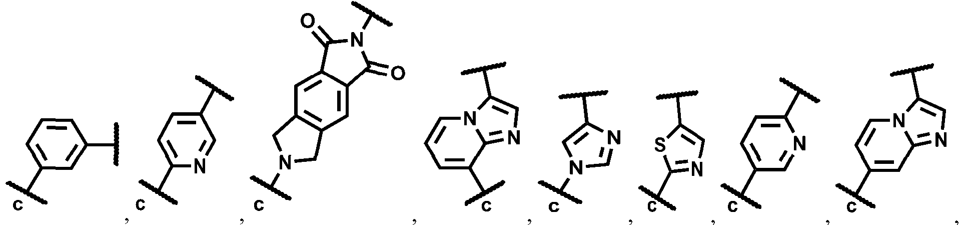 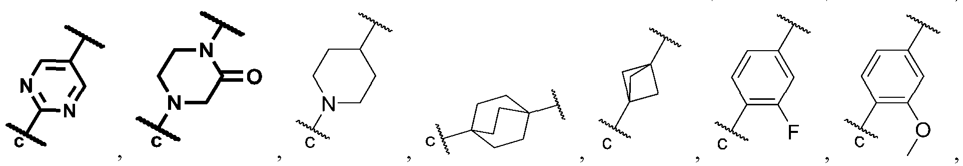 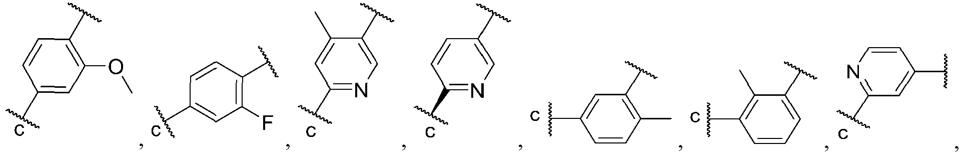 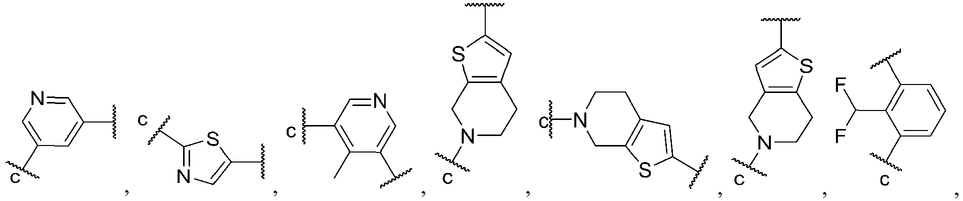 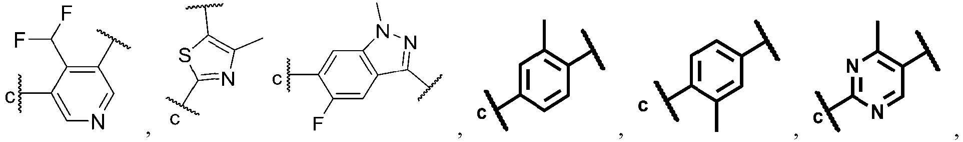 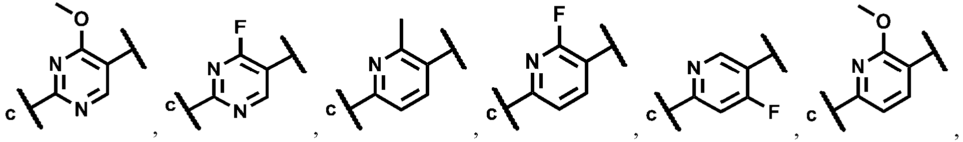 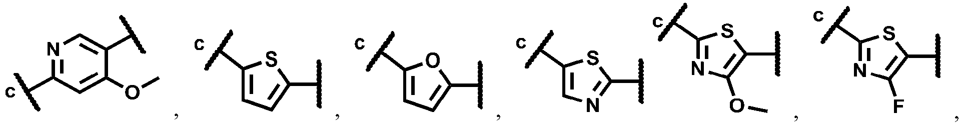 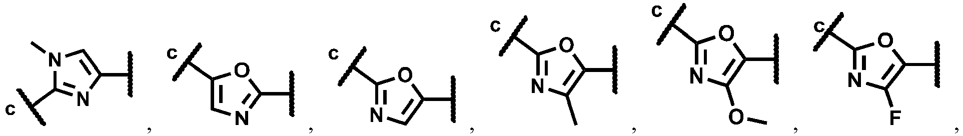 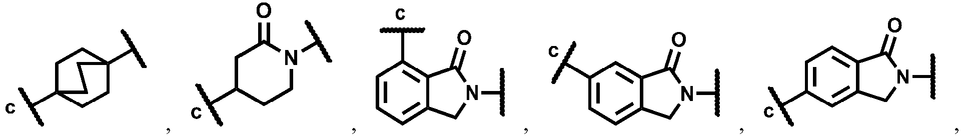 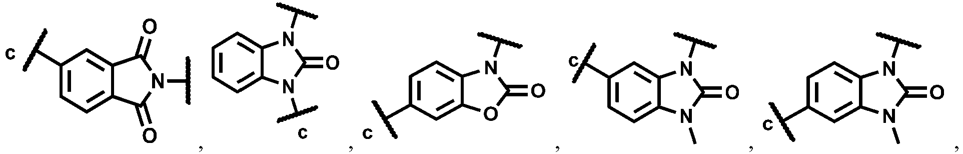 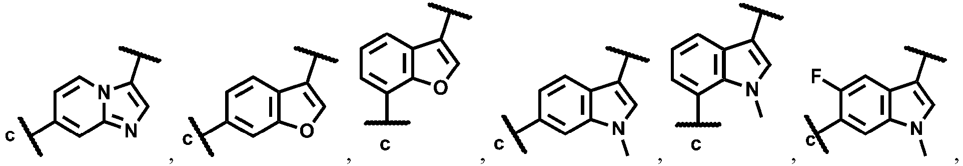 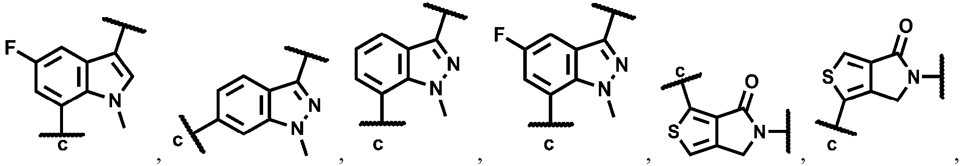 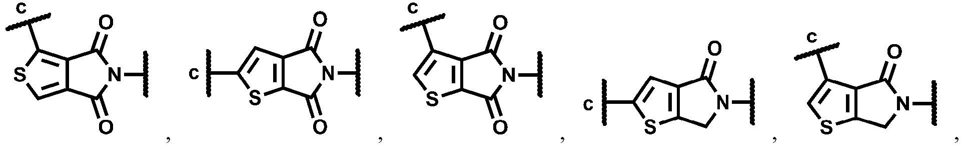 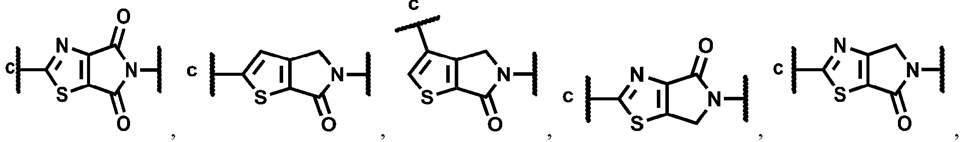 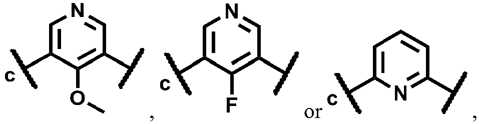 wherein side c is connected to L; in Scheme ii, B is any one of the above groups other than

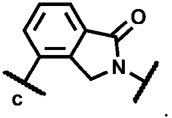

In one embodiment of the present invention, in condition ii, -A-L-B- is G11 or G12:
G11: 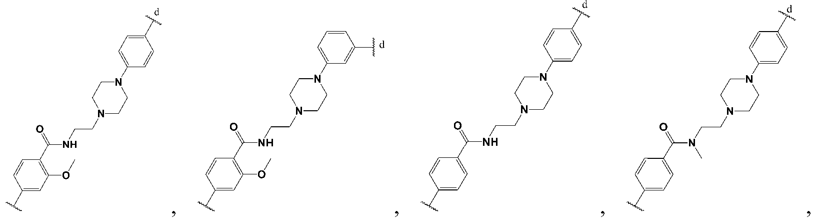 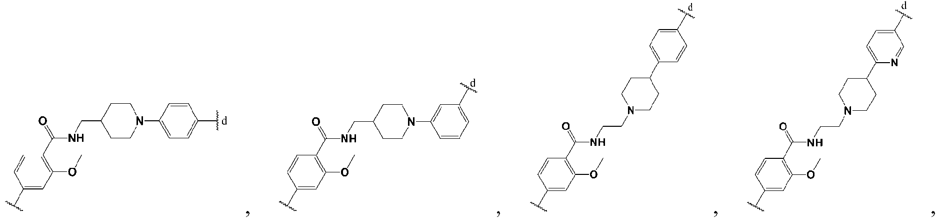 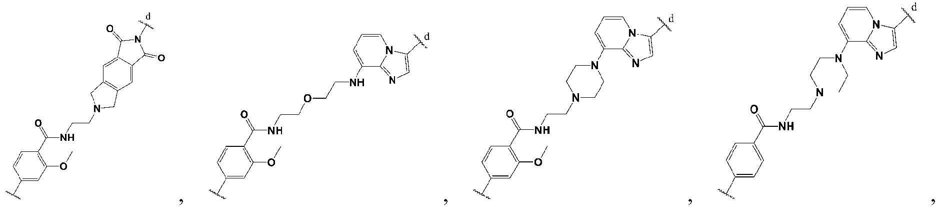 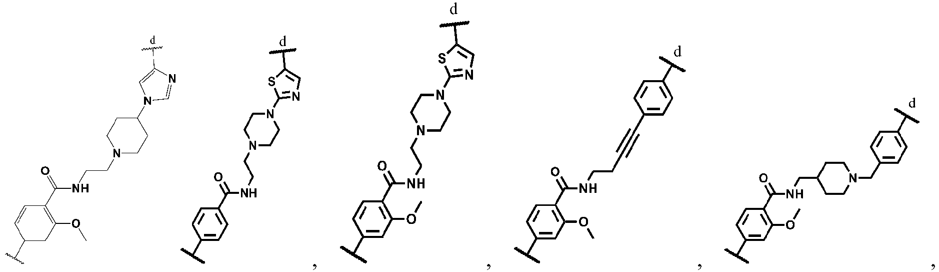 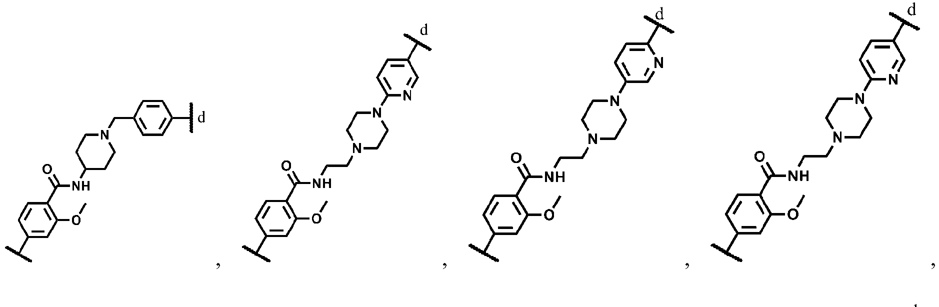 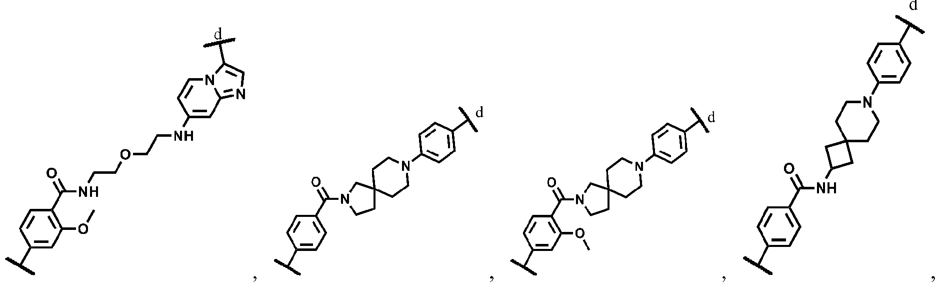 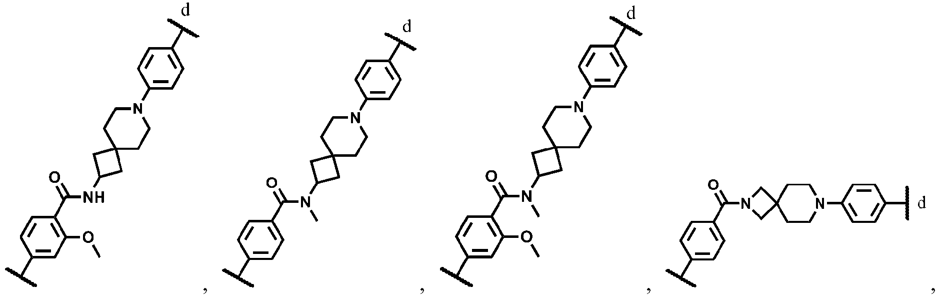 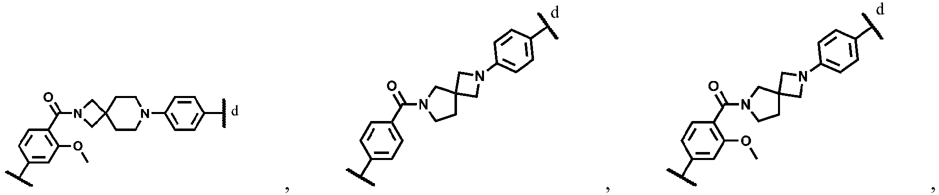 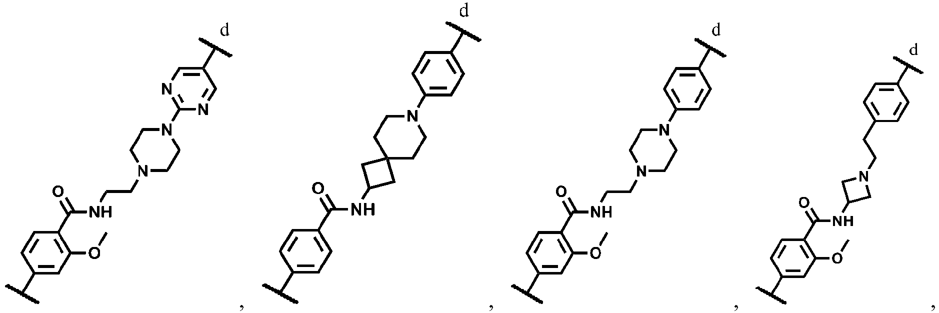 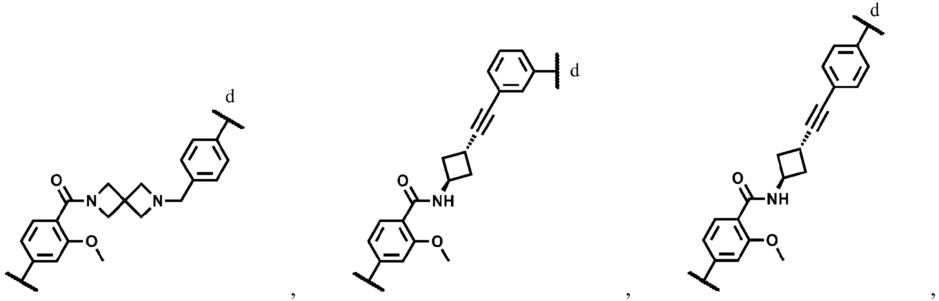 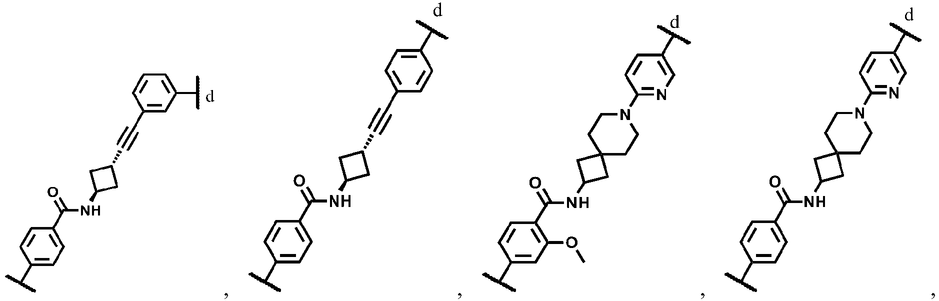 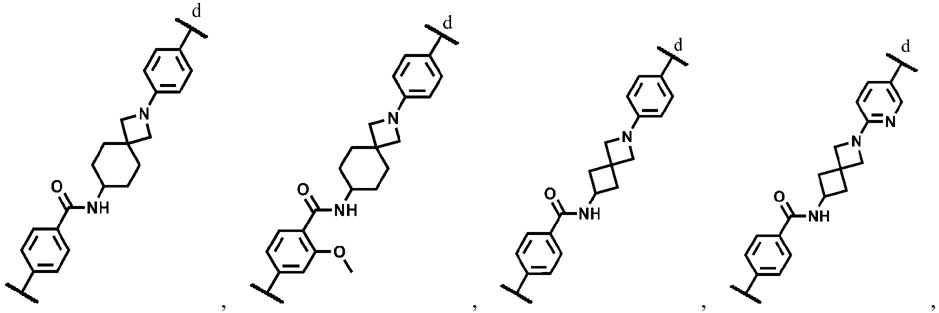 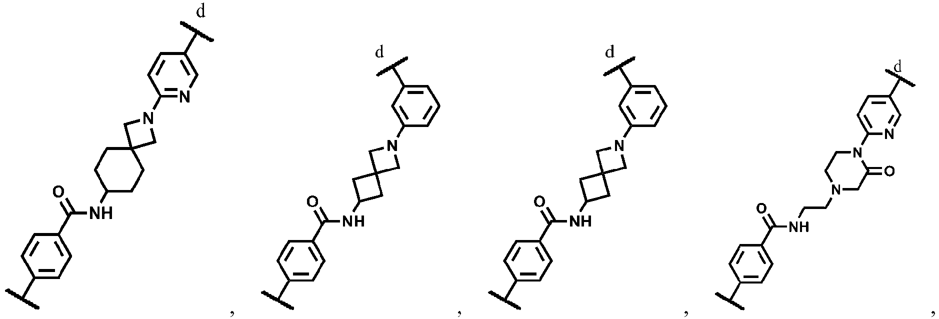 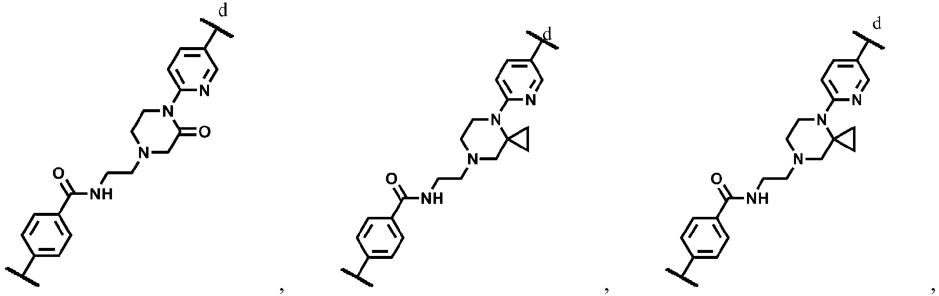 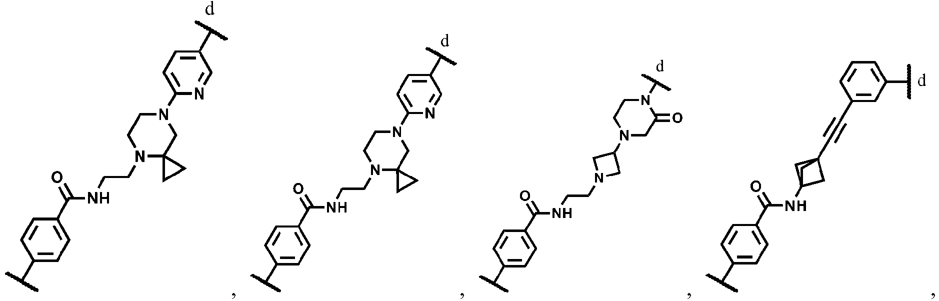 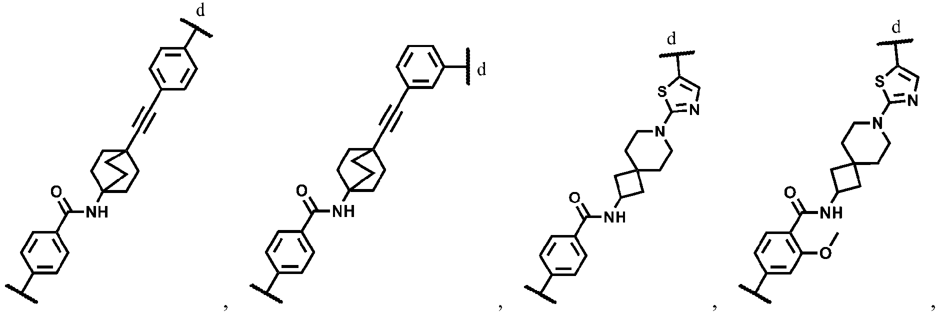 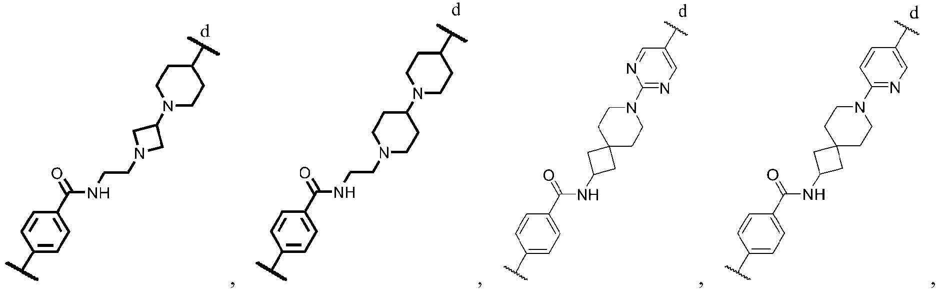 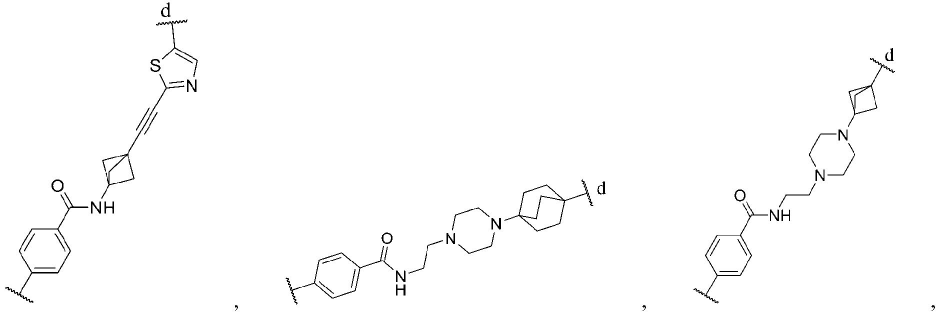 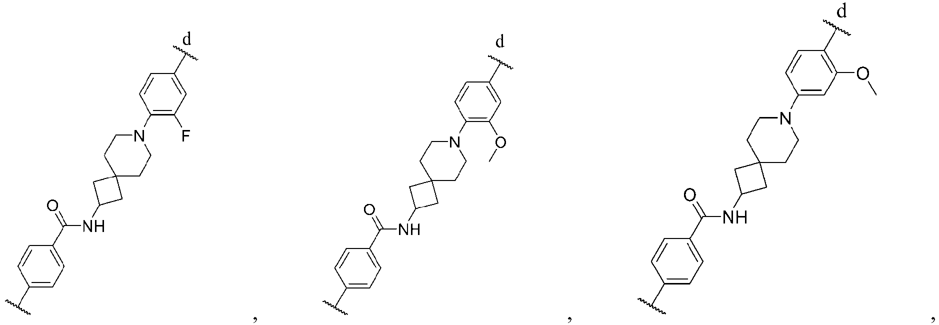 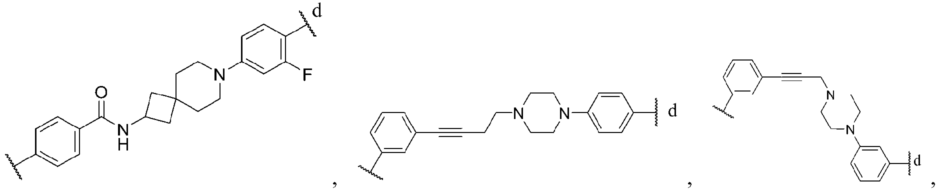 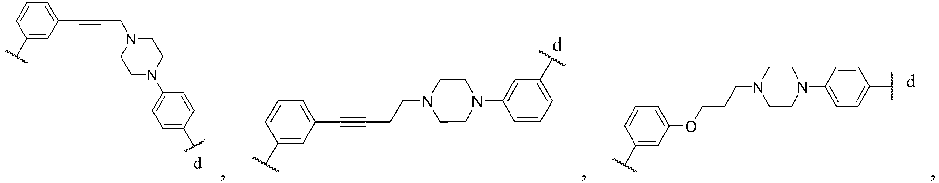 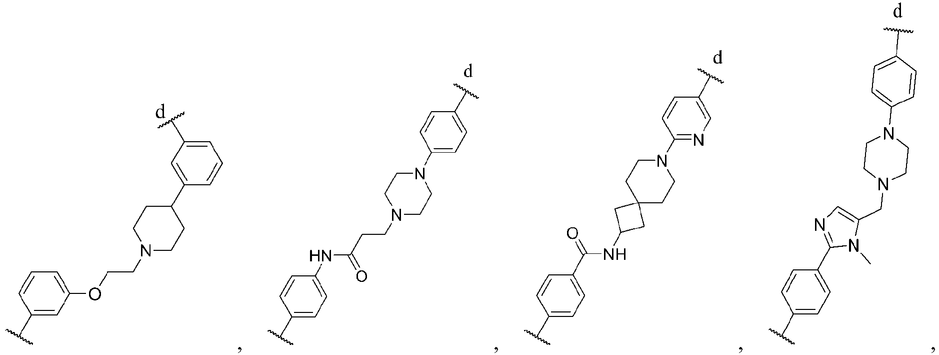 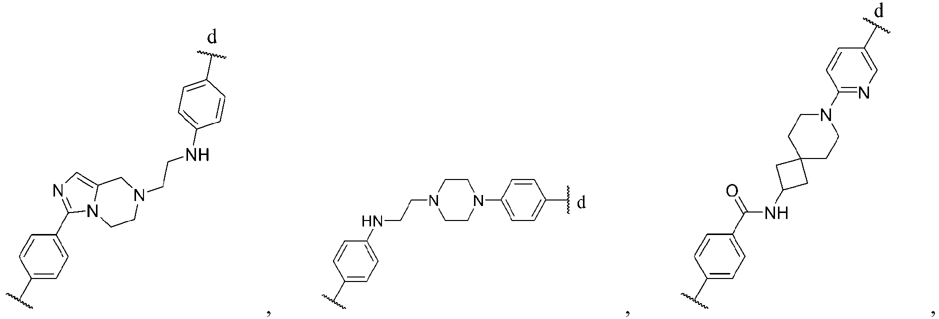 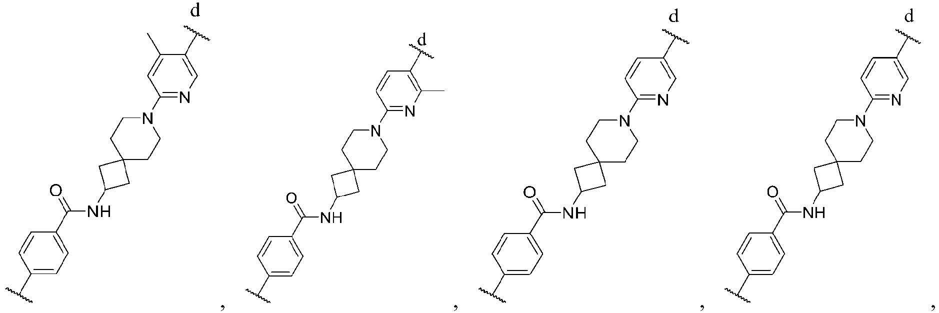 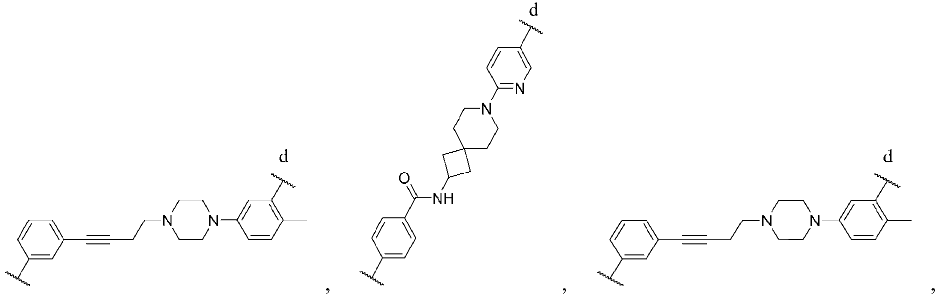 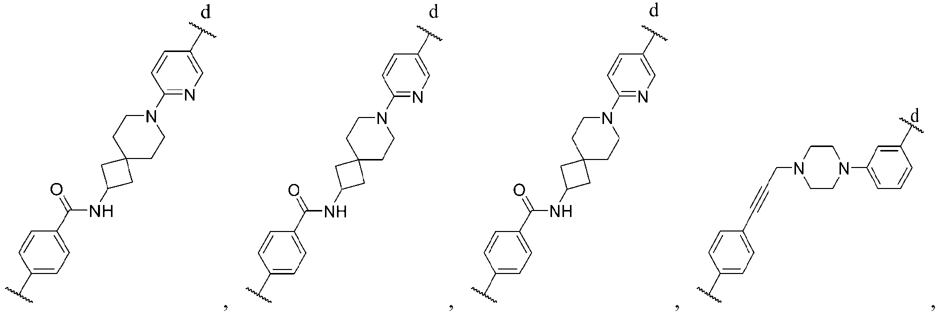 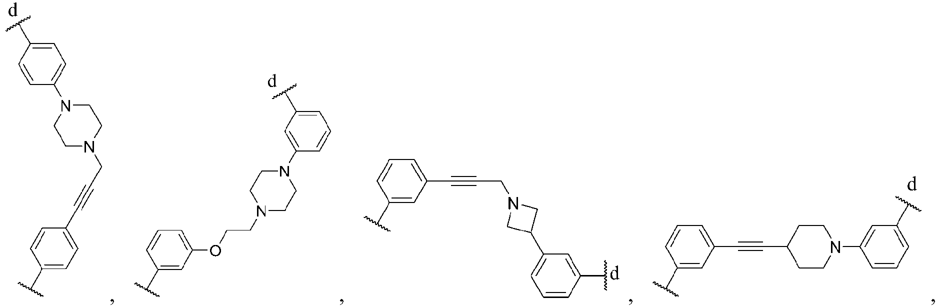 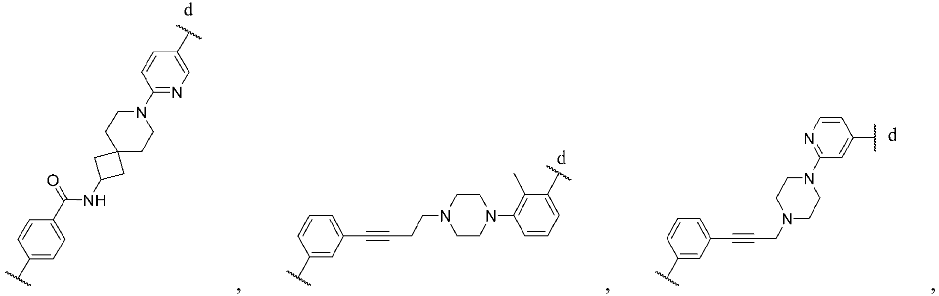 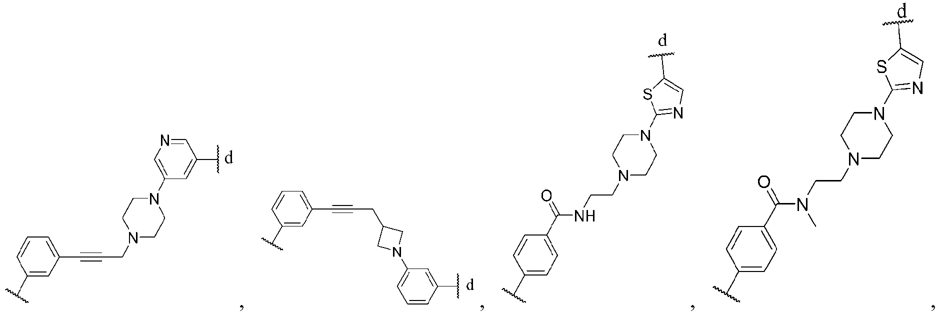 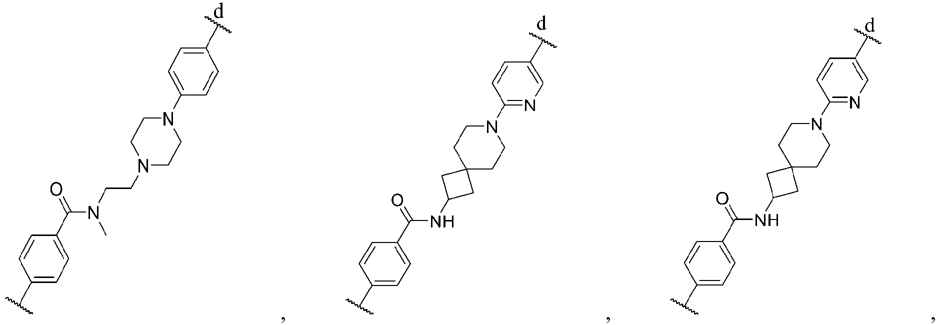 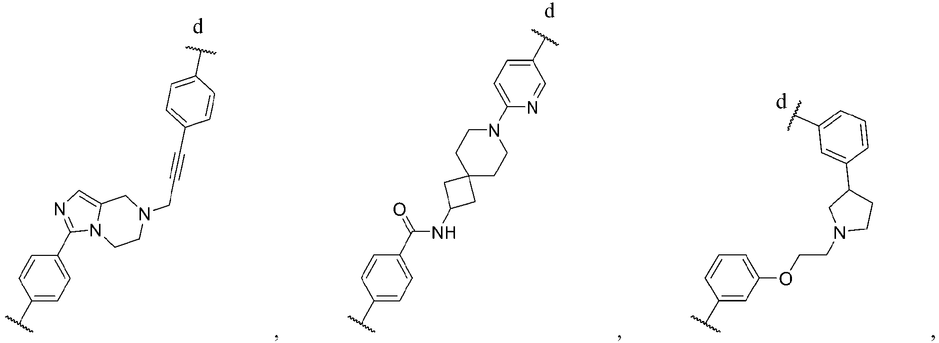 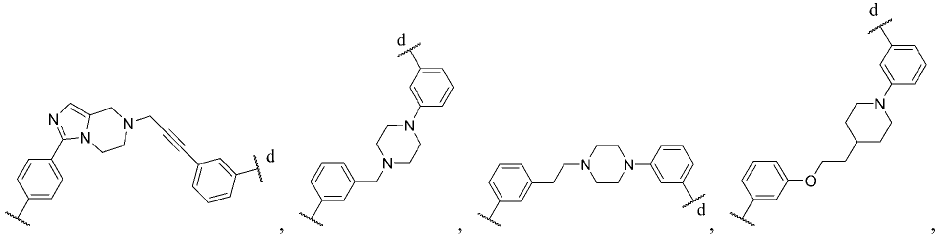 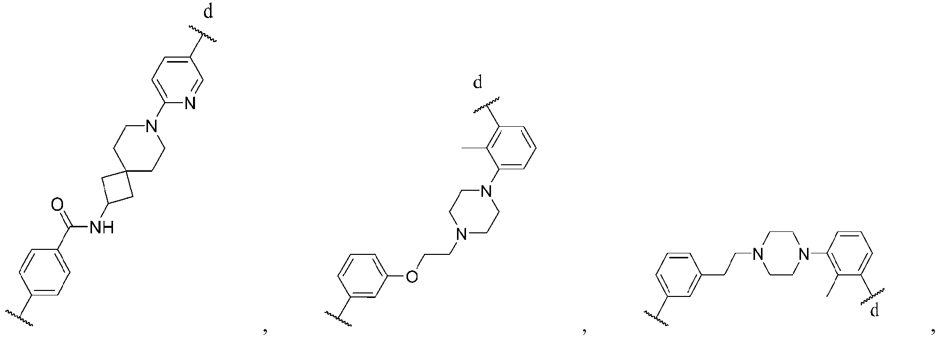 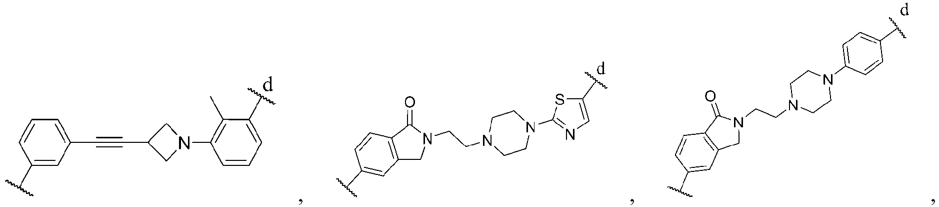 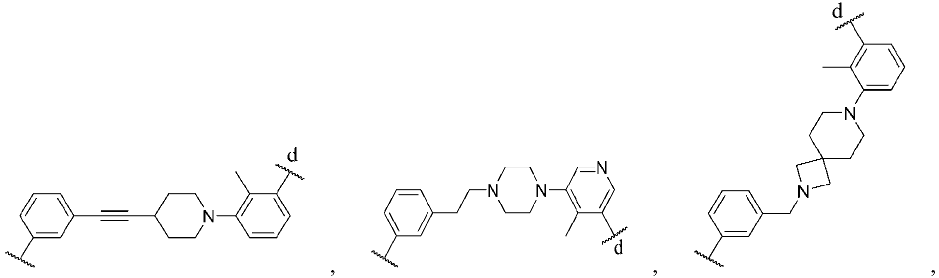 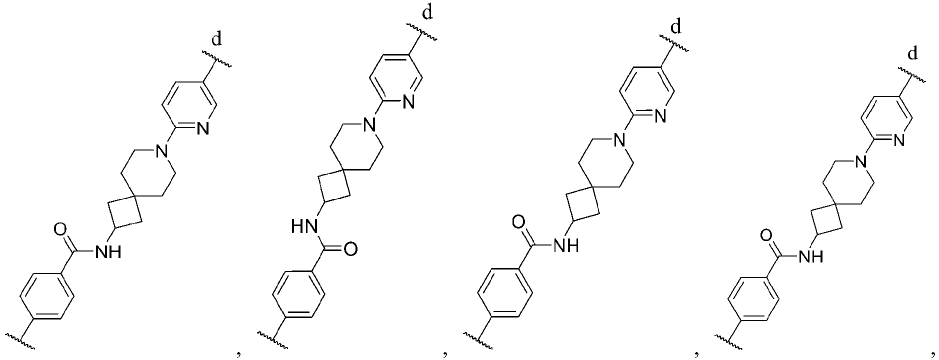 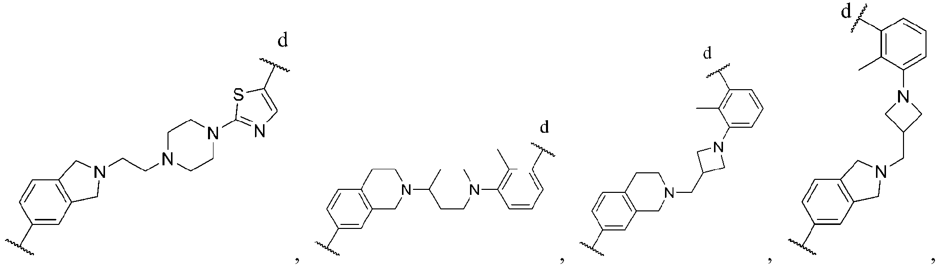 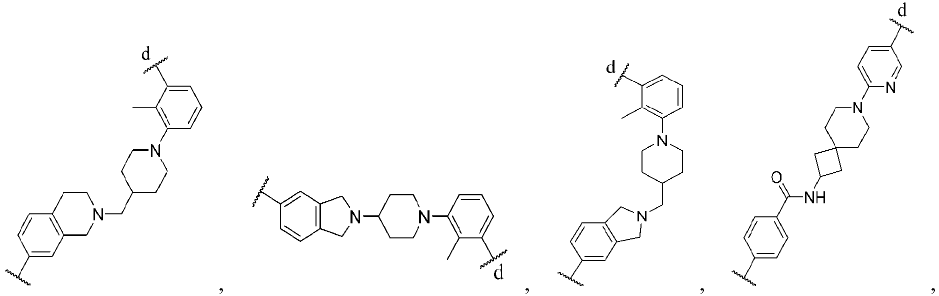 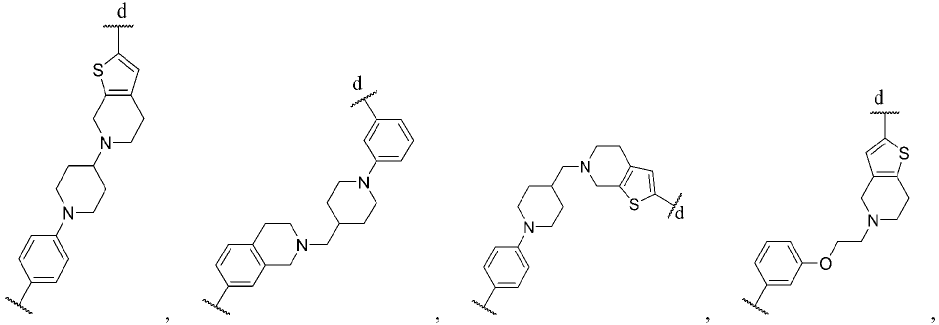 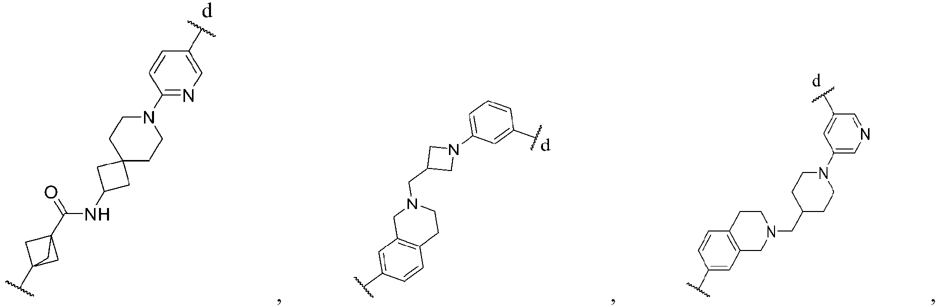 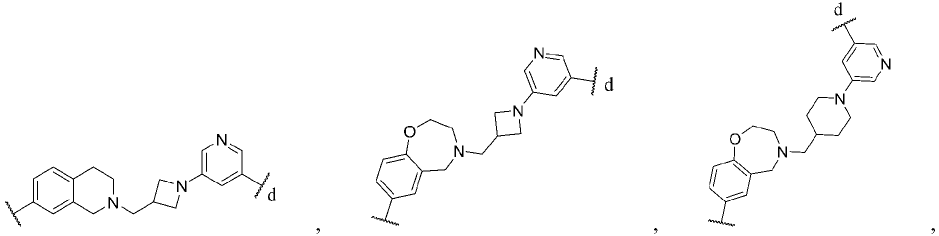 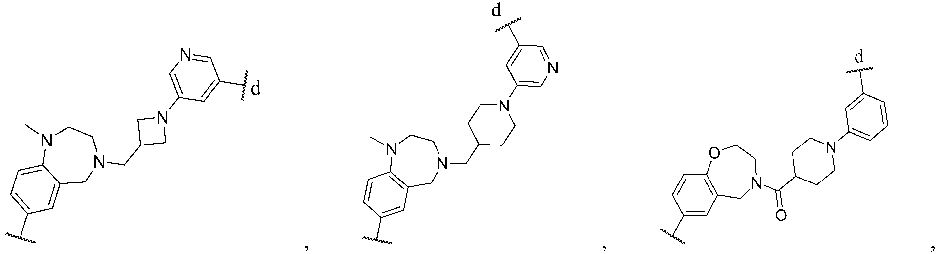 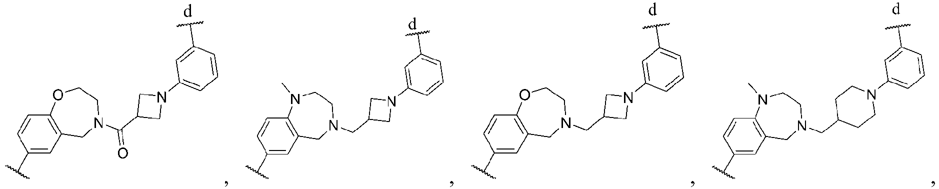 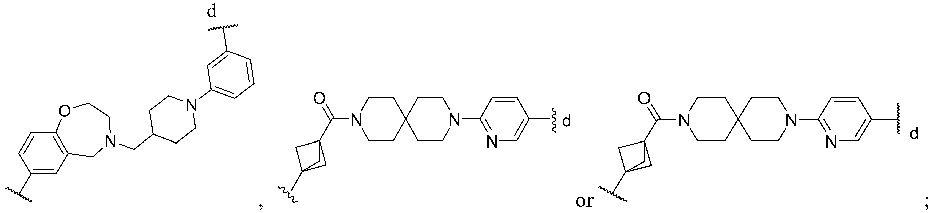
G12: 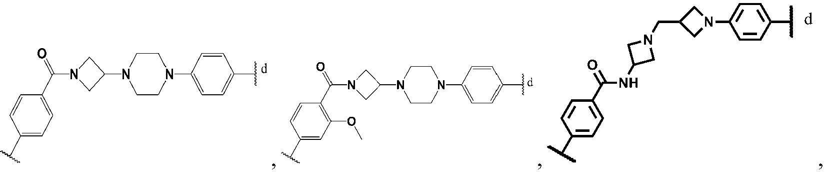 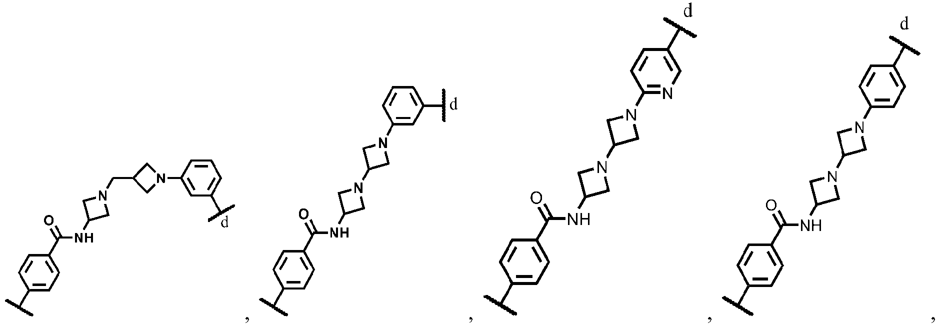 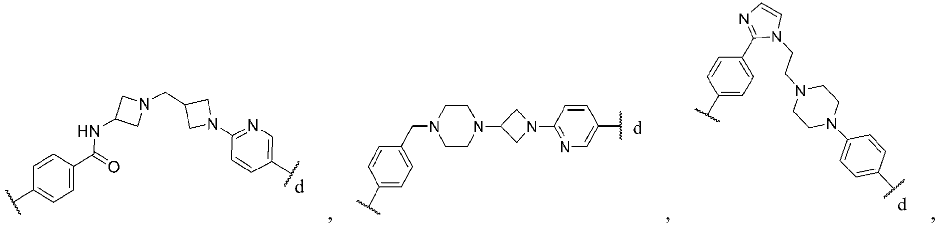 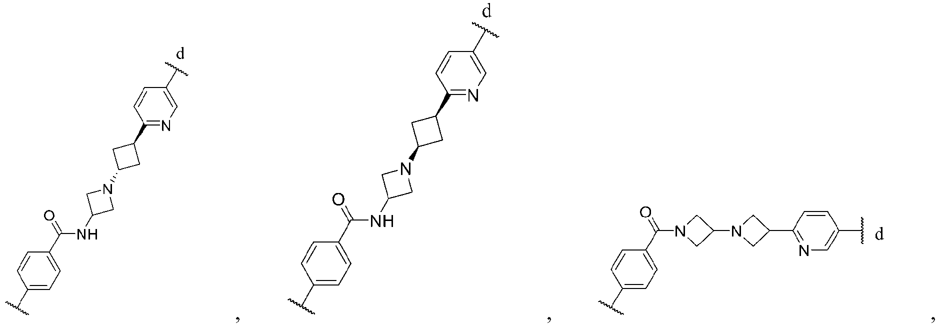 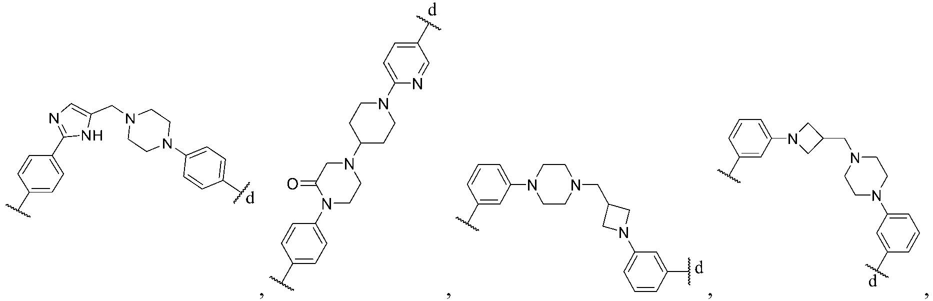 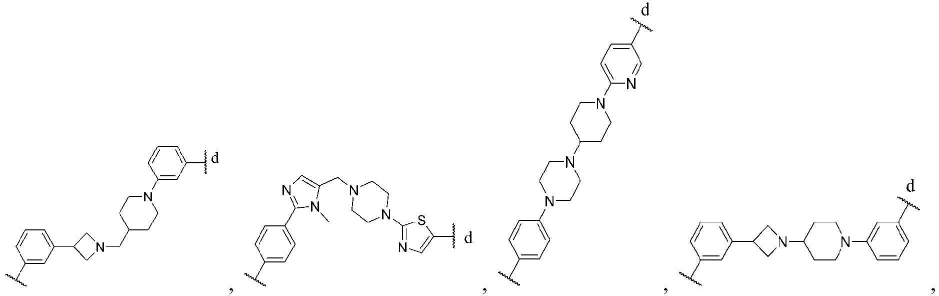 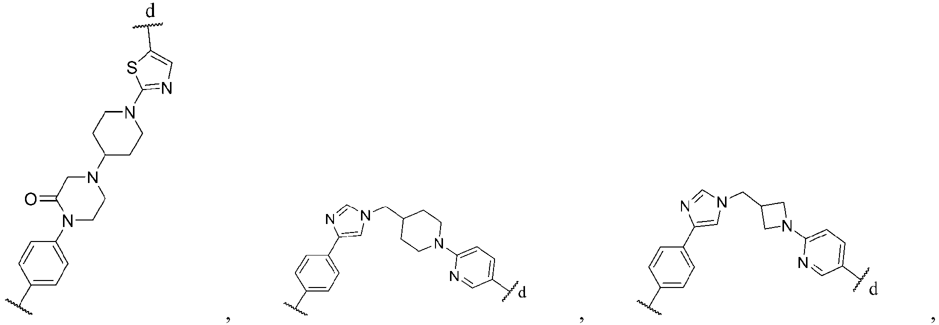 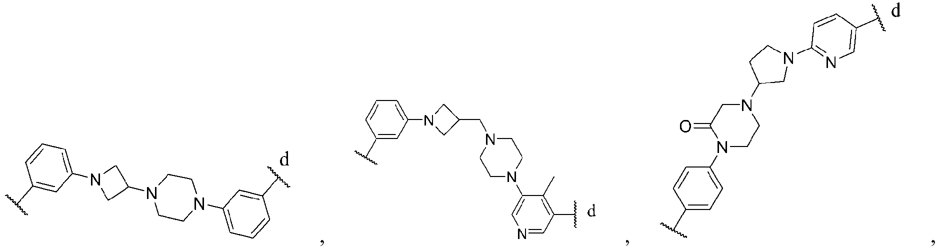 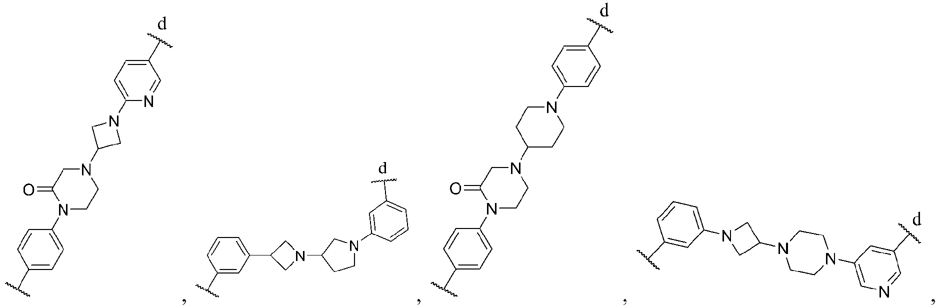 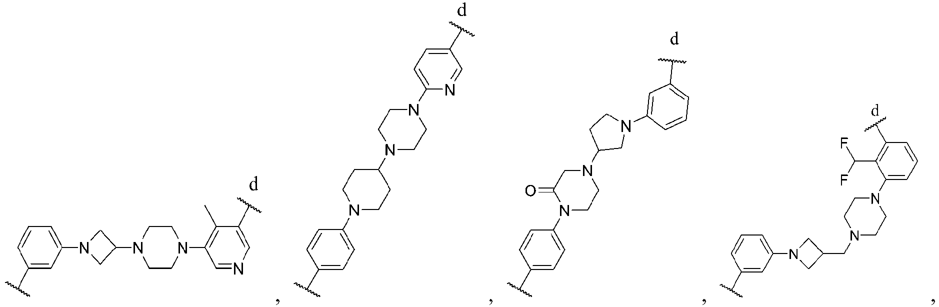 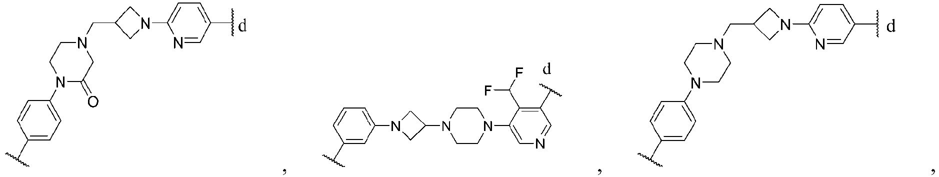 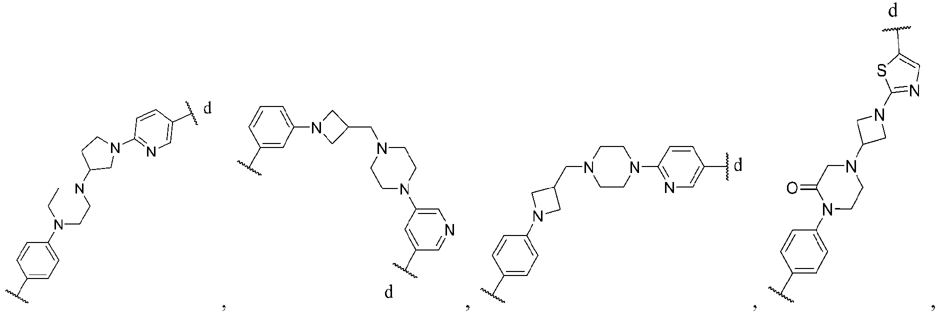 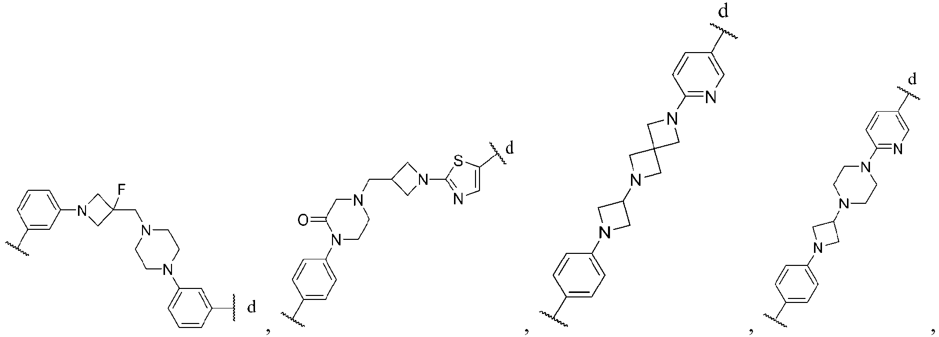 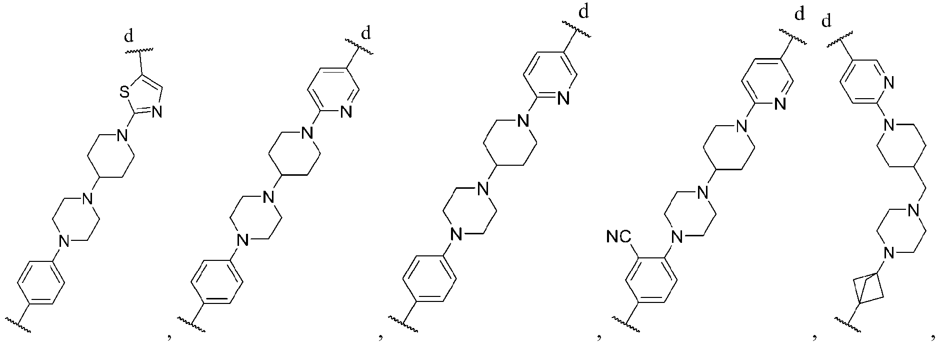 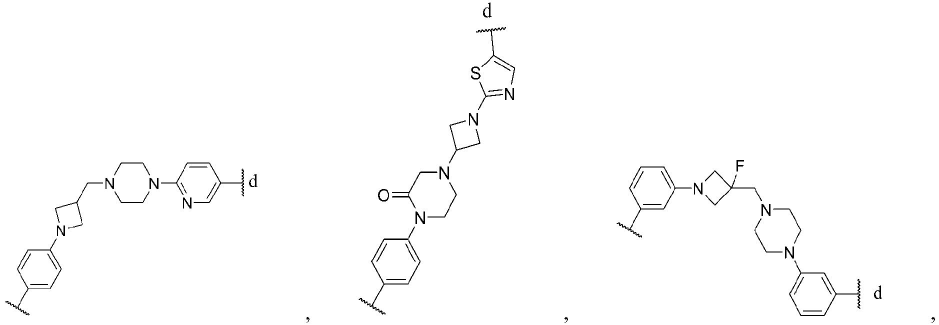 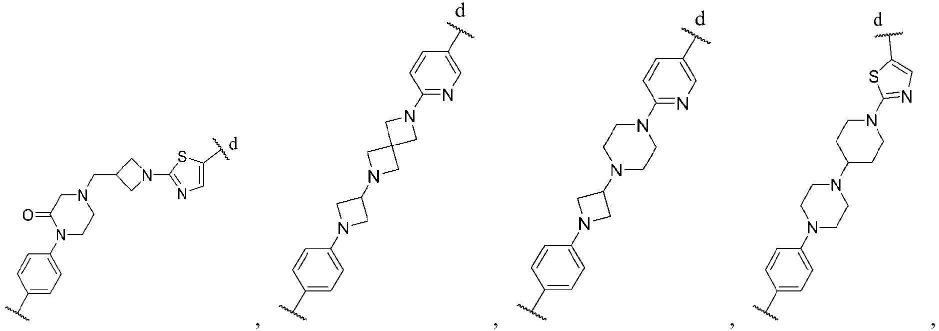 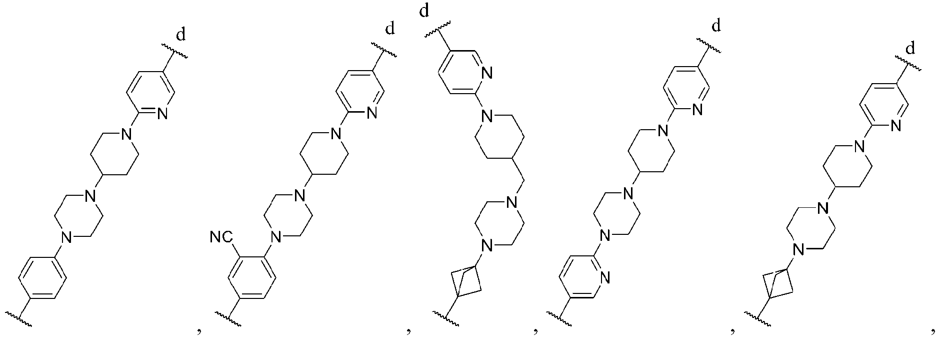 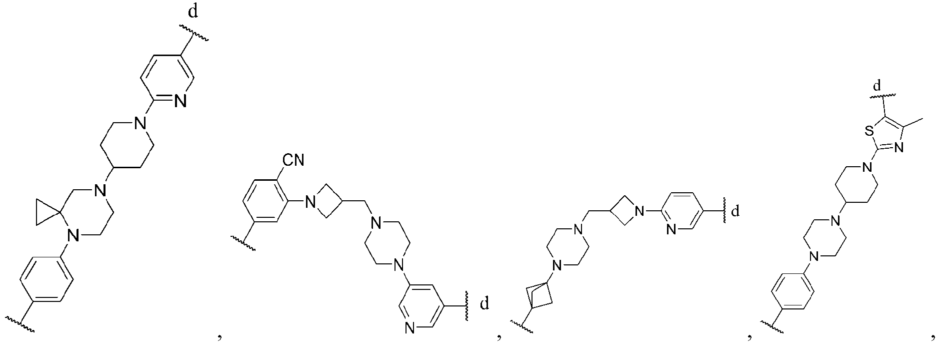 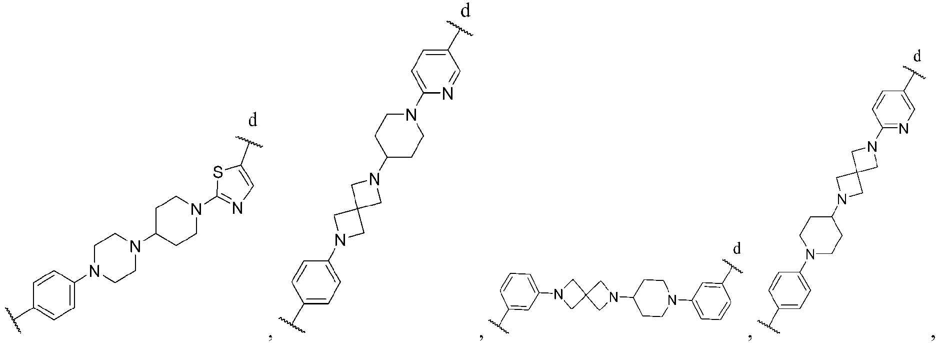 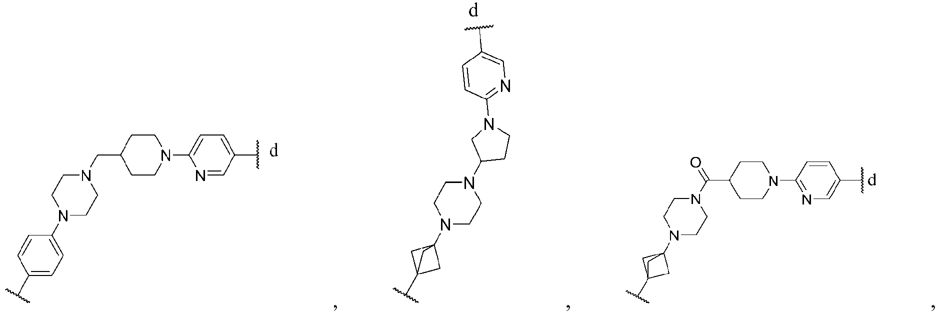 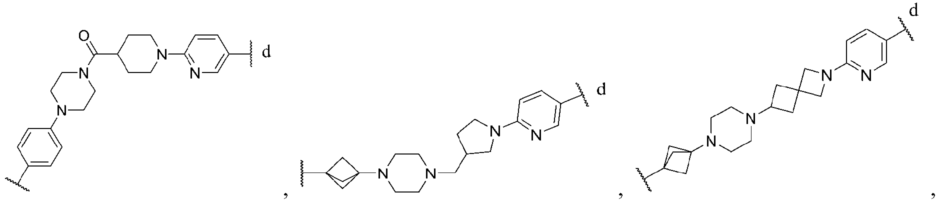 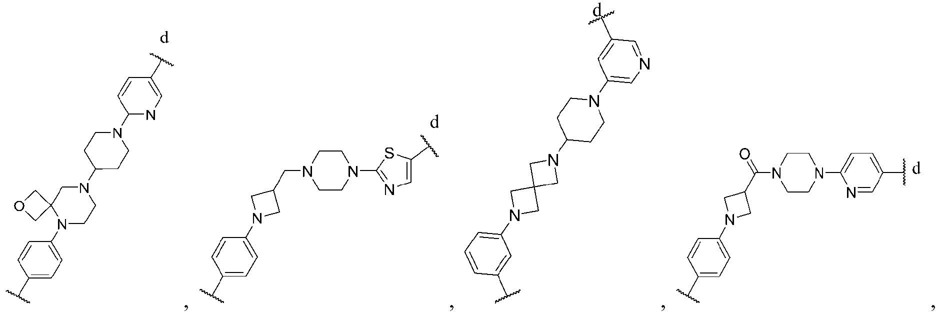 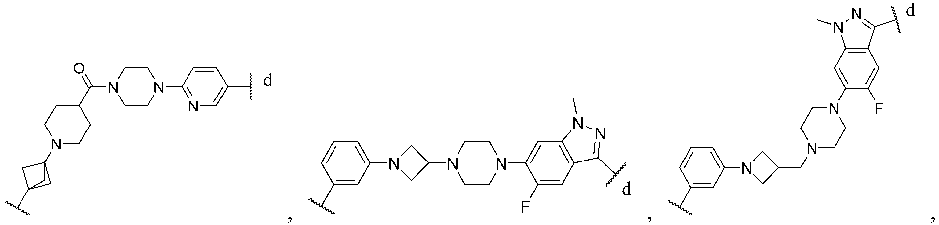 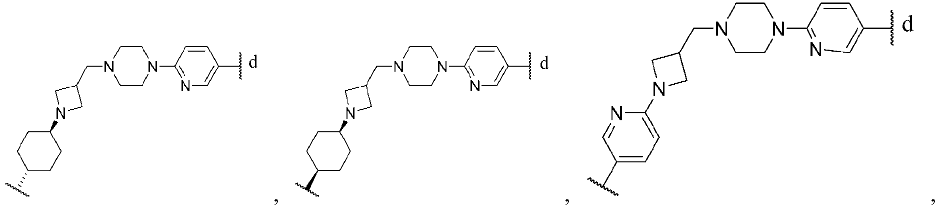 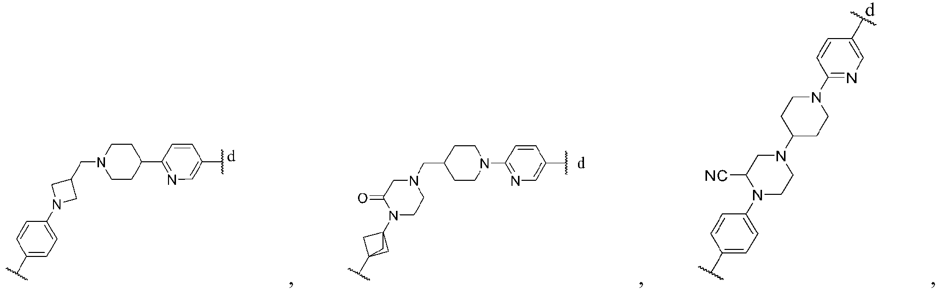 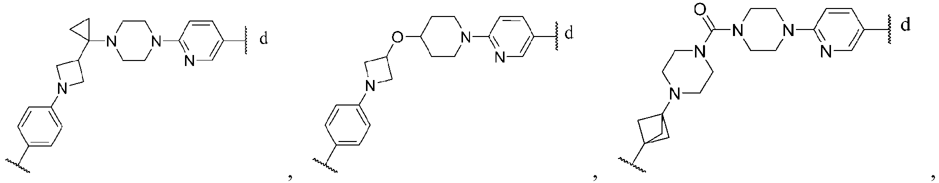 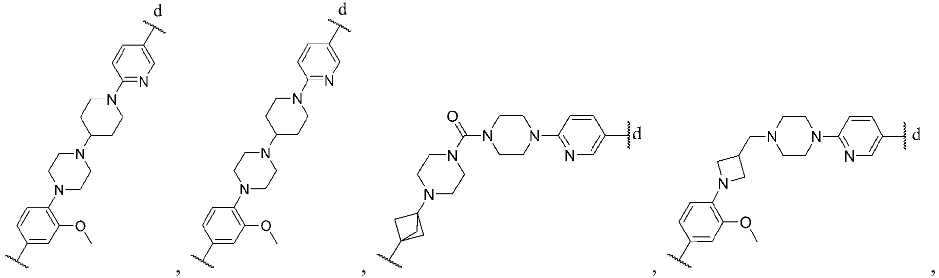 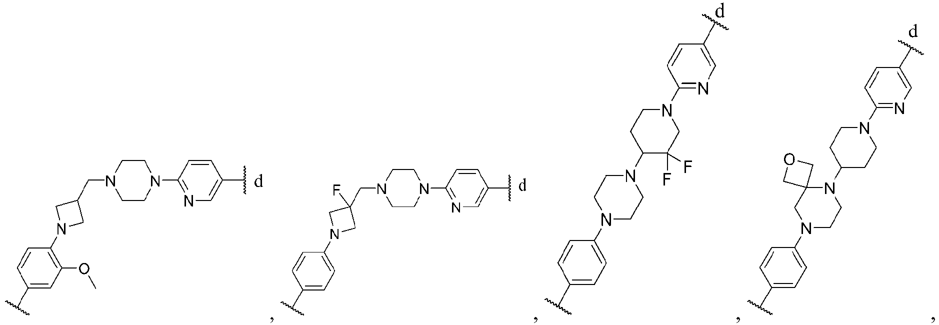 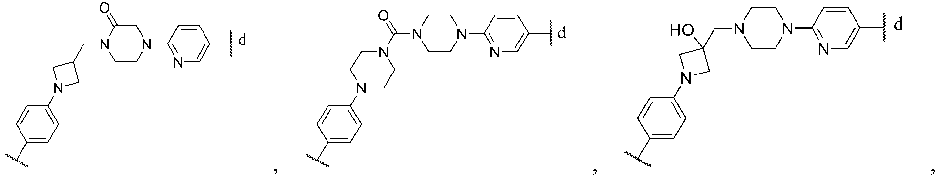 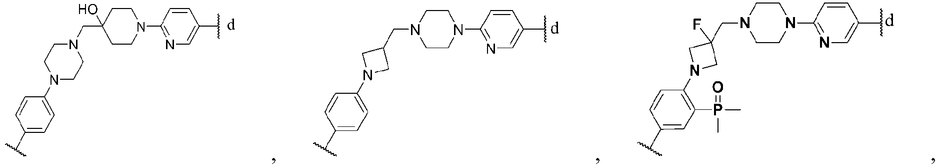 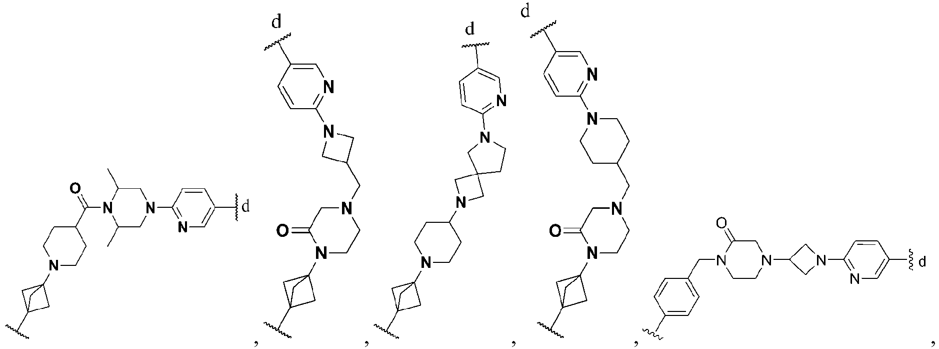 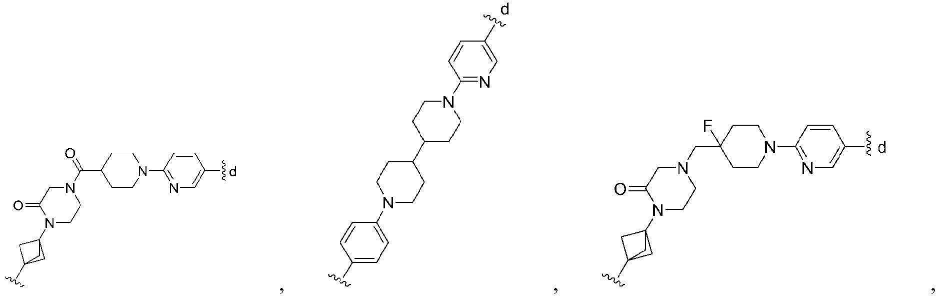 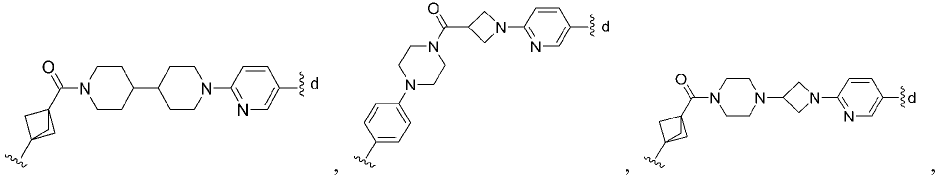 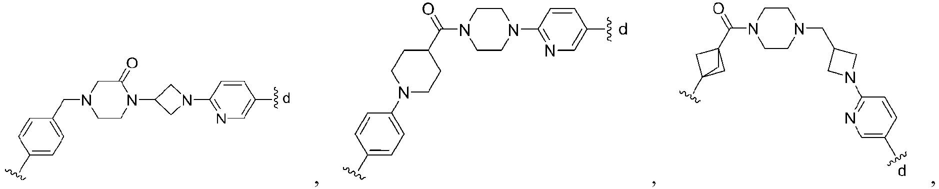 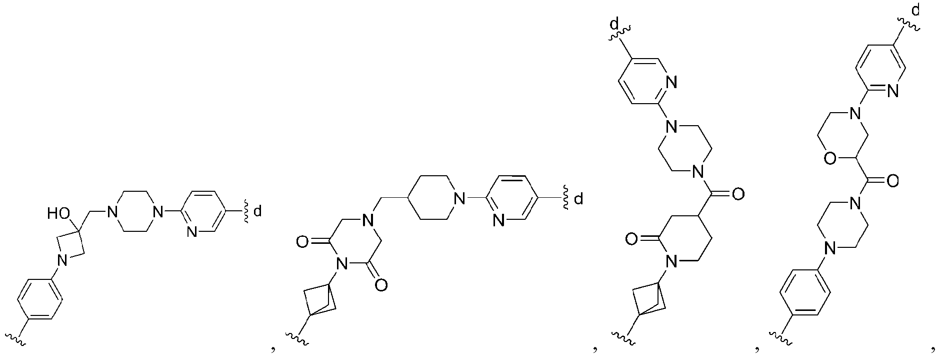 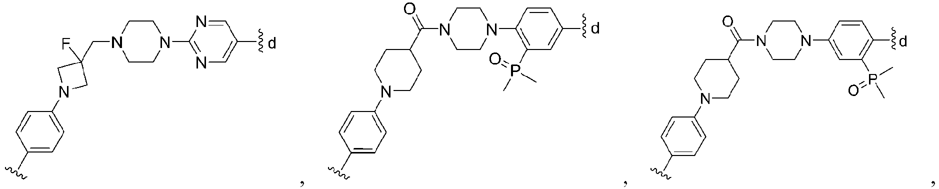 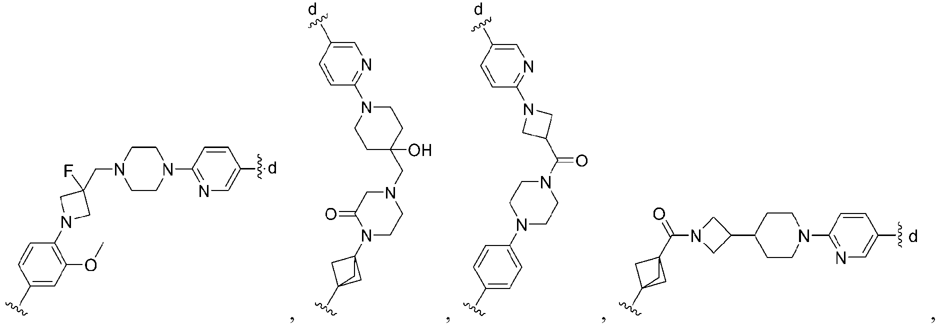 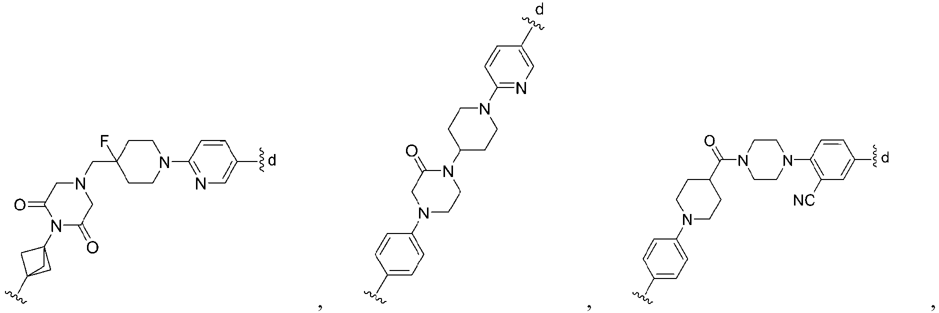 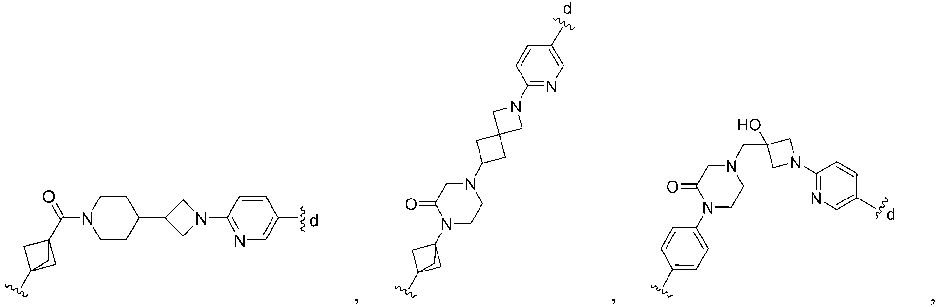 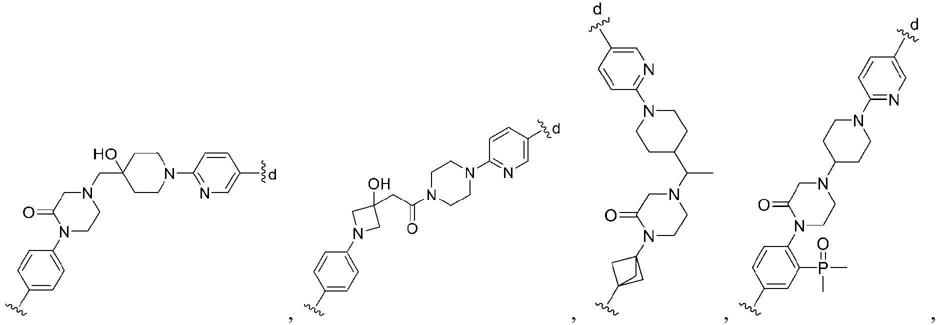 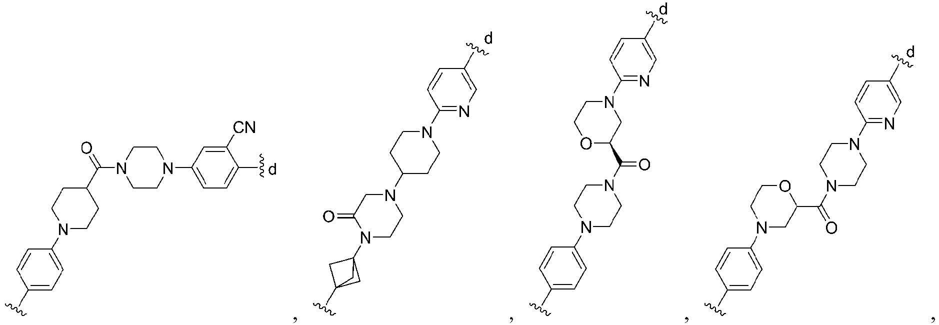 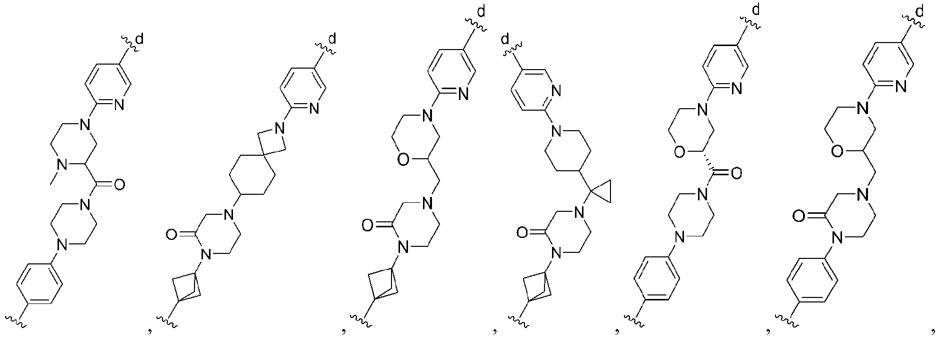 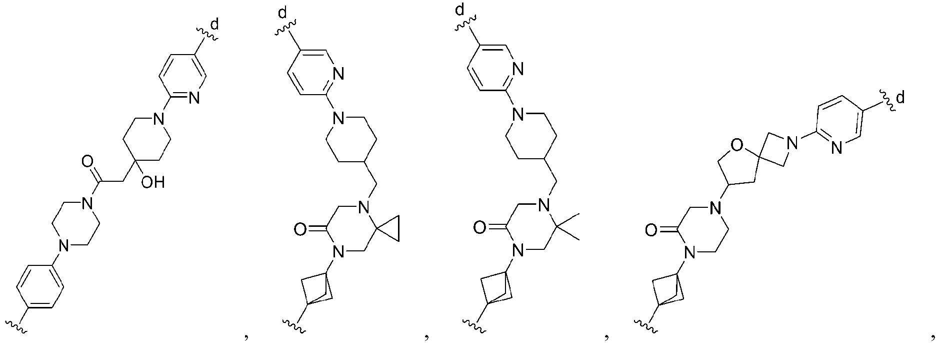 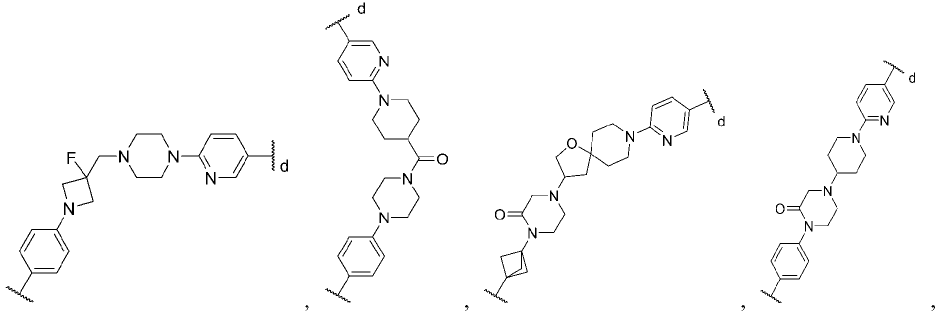 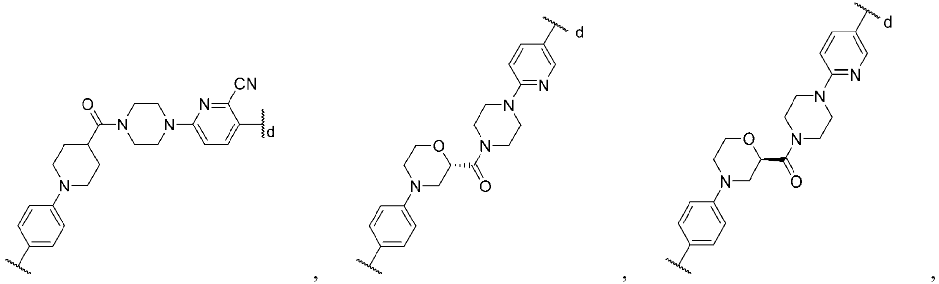 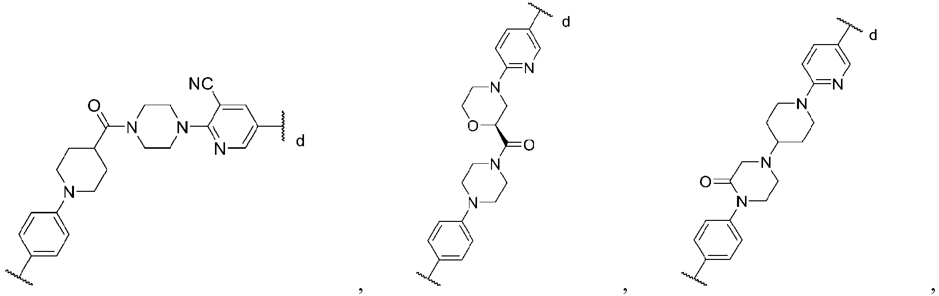 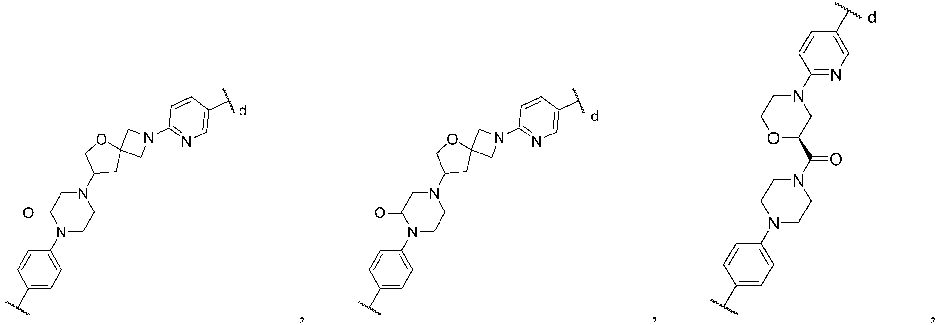 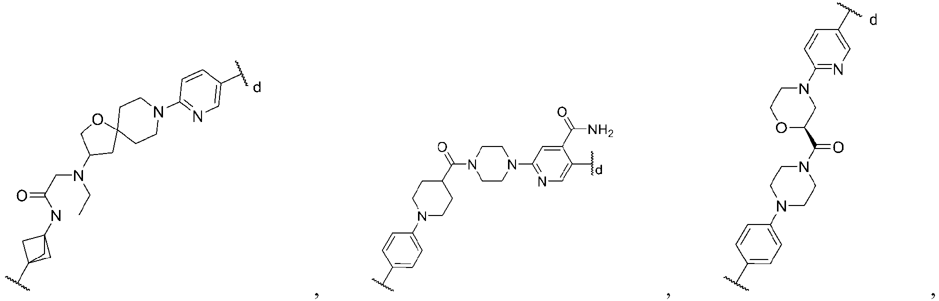 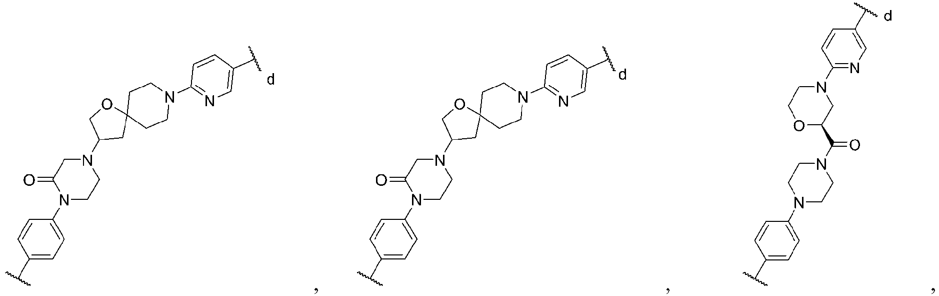 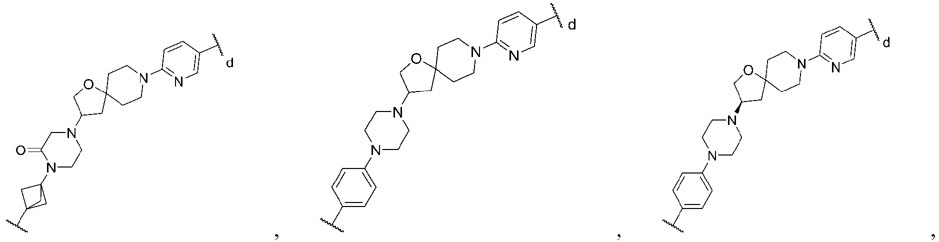 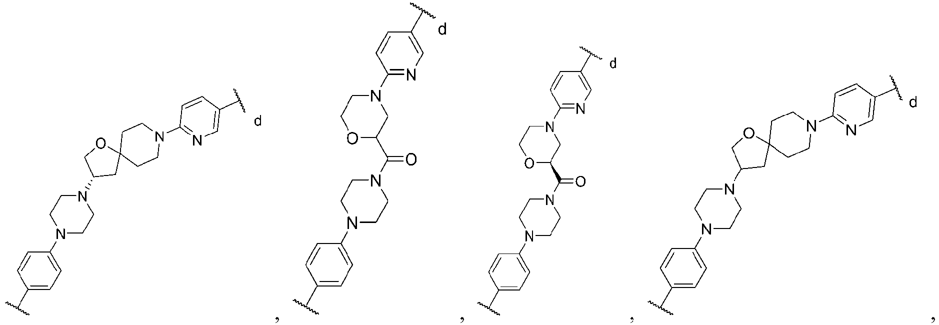 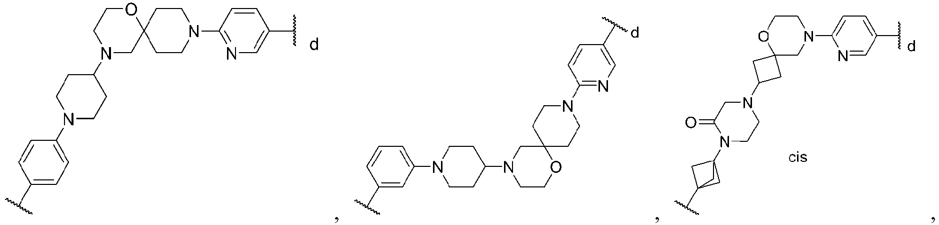 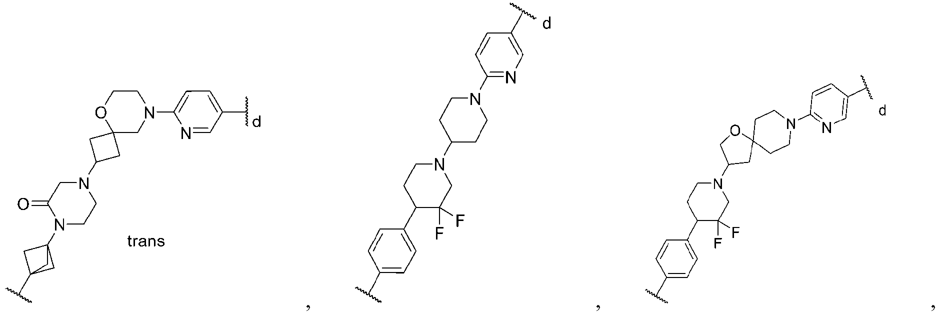 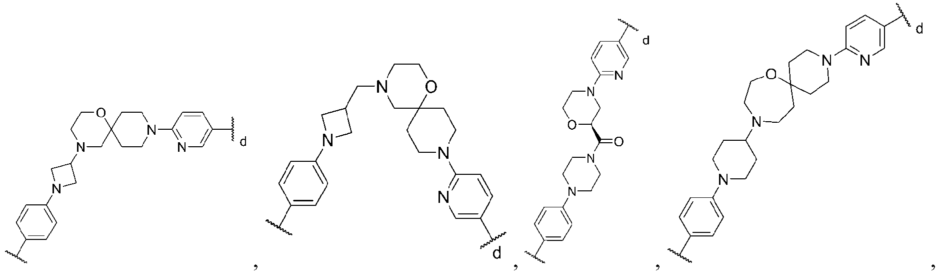 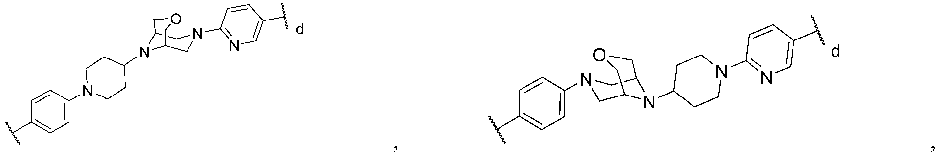 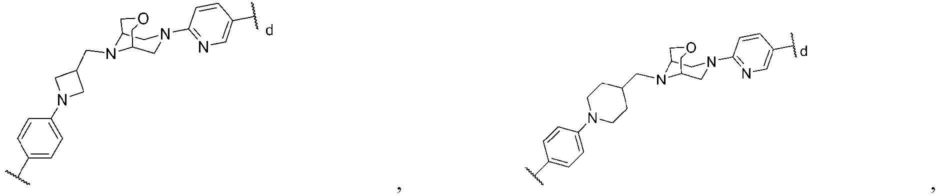 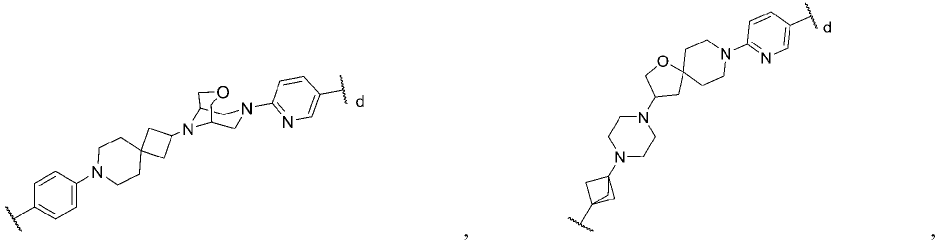 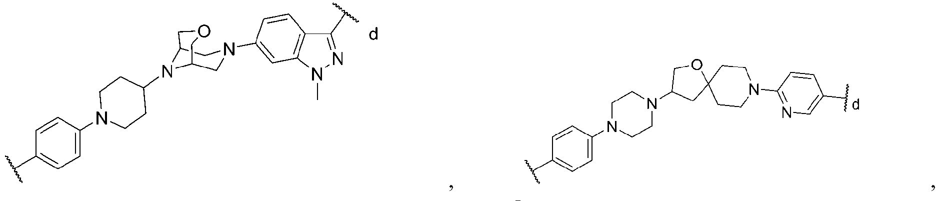 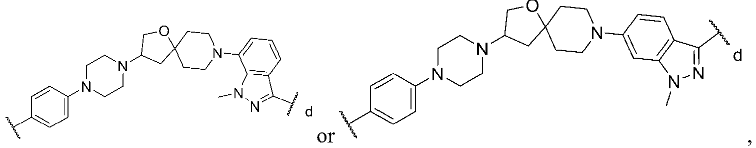
wherein side d is connected to

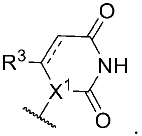

In one embodiment of the present invention, in Scheme i, -A-L-B- is: 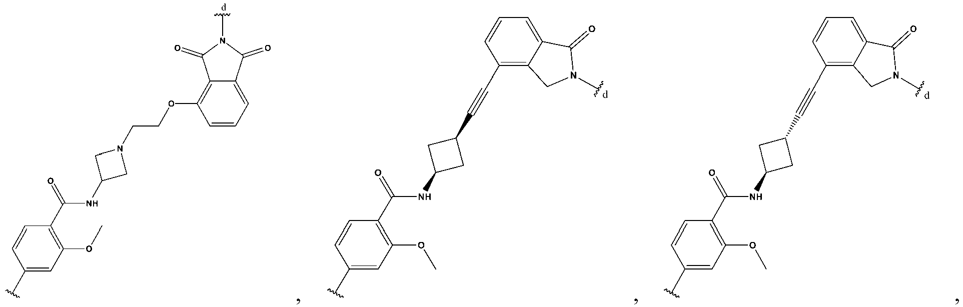 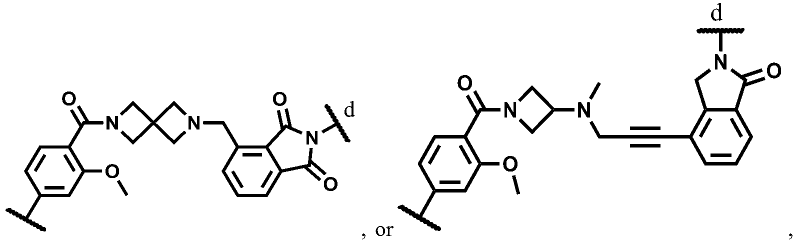 wherein side d is connected to

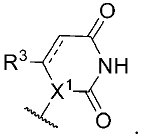

In one embodiment of the present invention, -A-L-B- is: 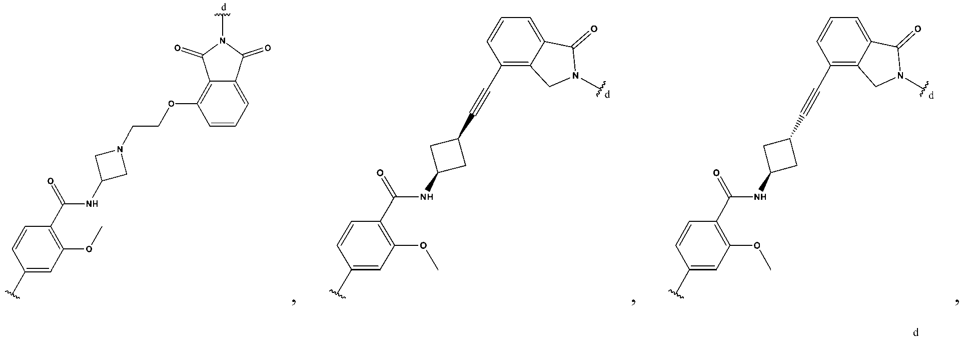 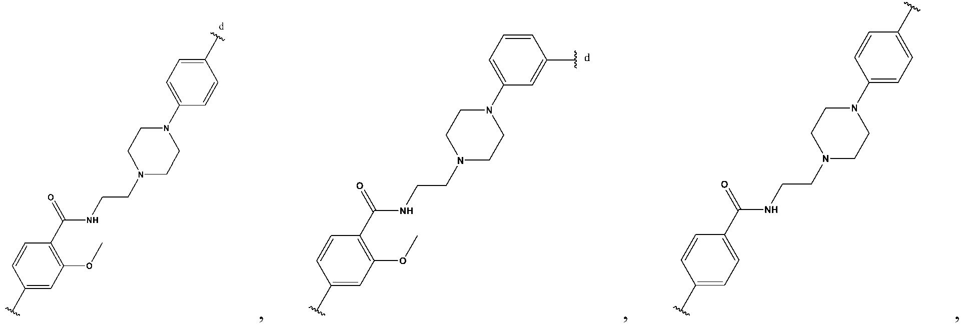 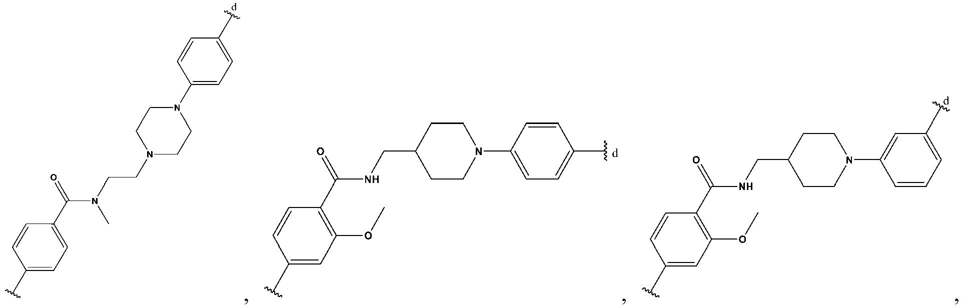 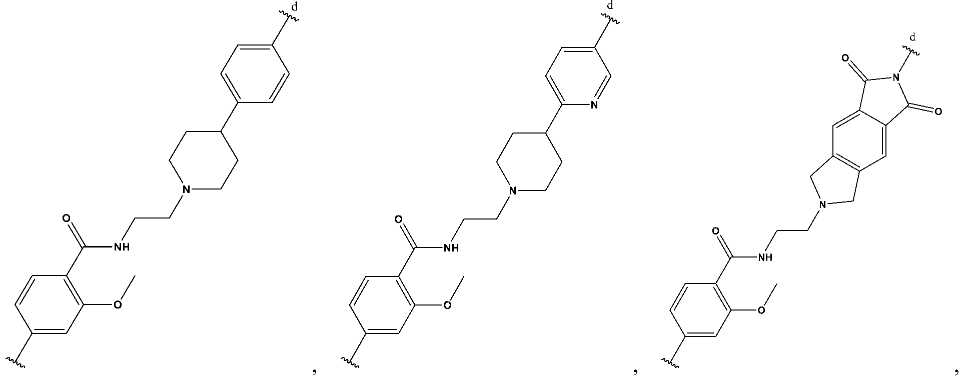 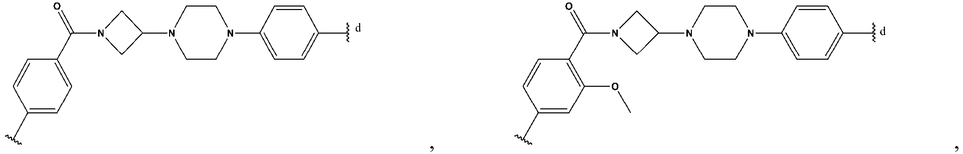 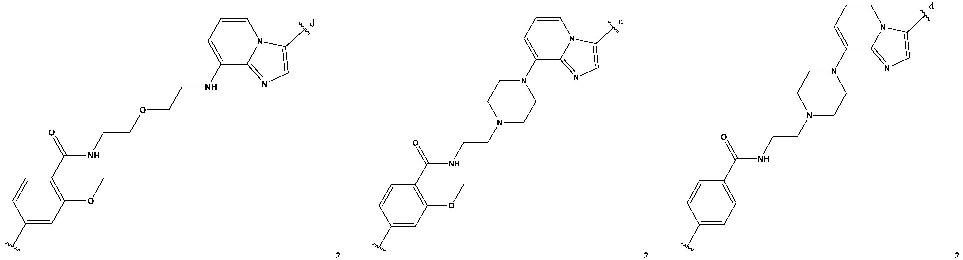 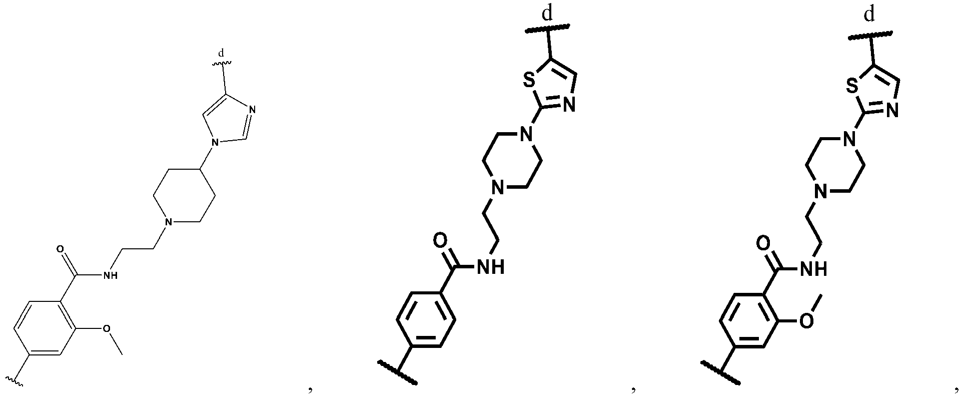 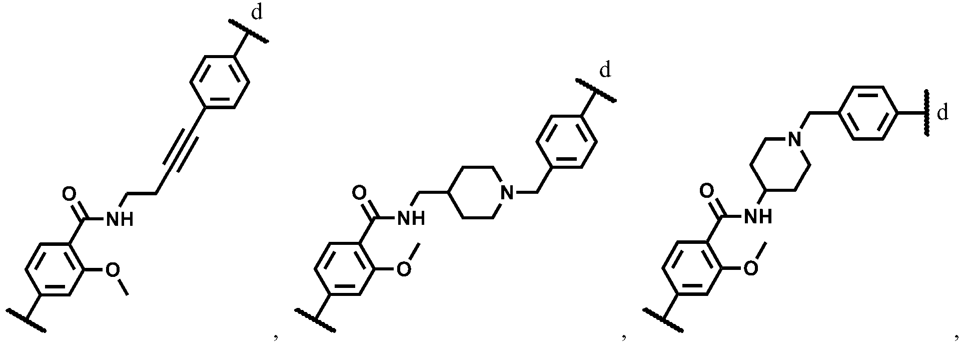 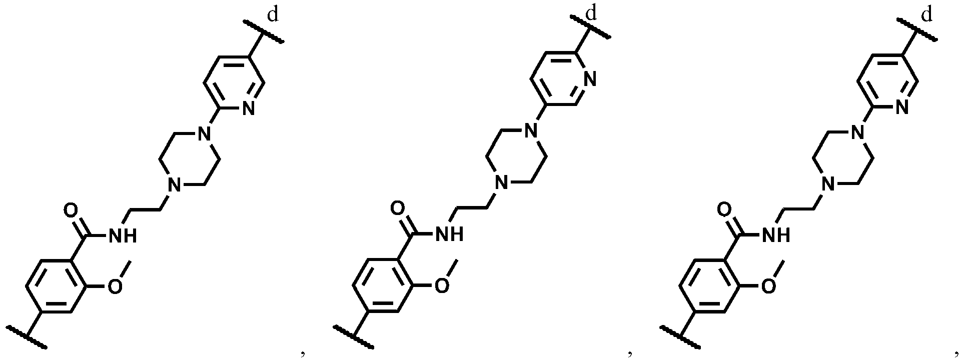 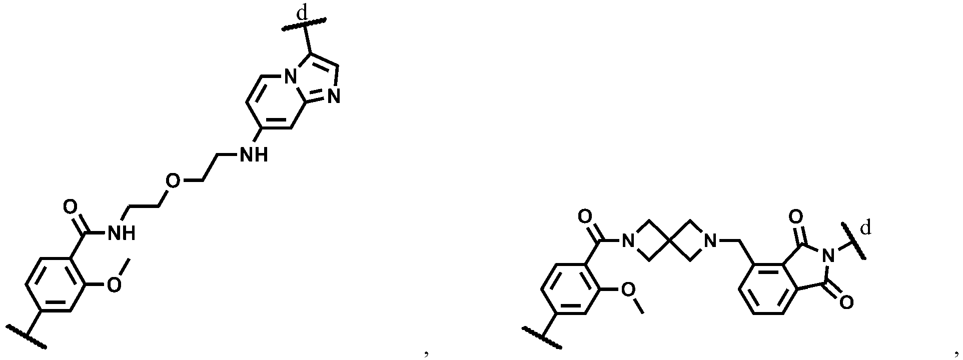 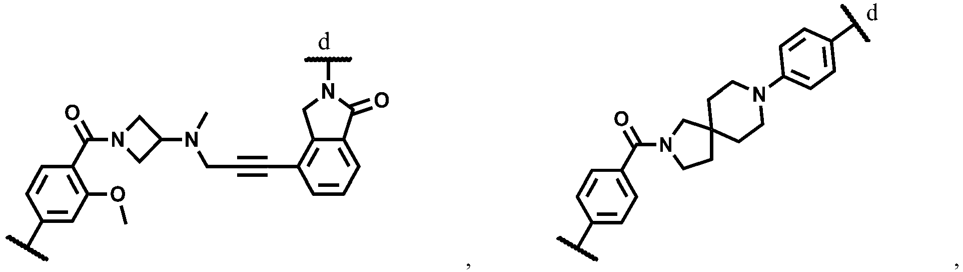 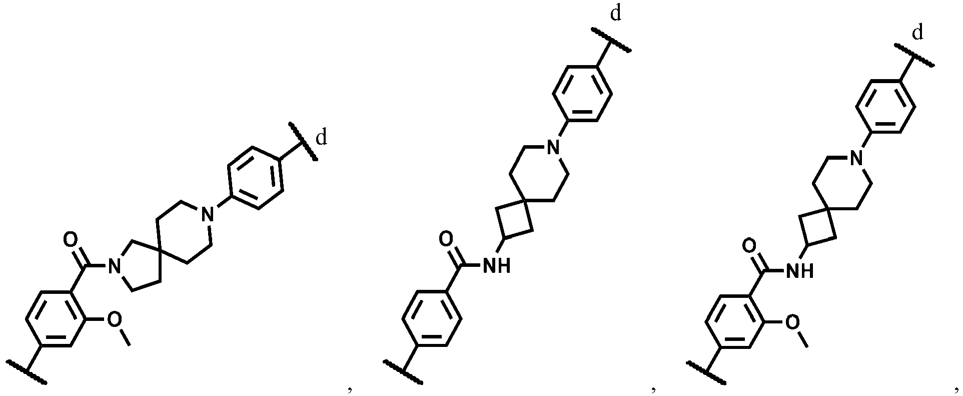 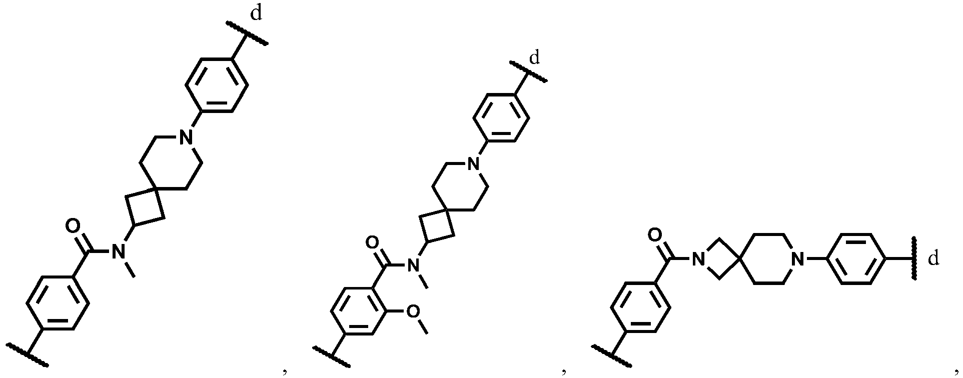 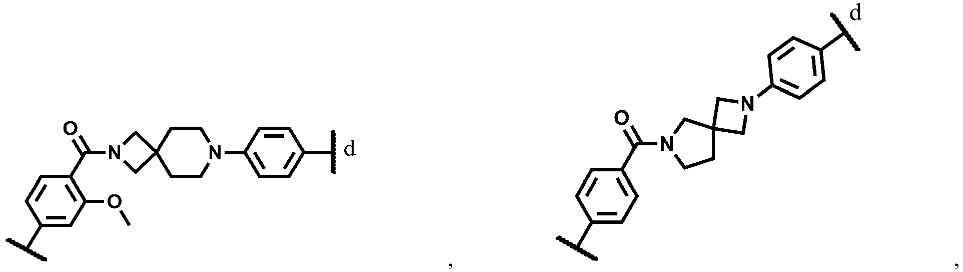 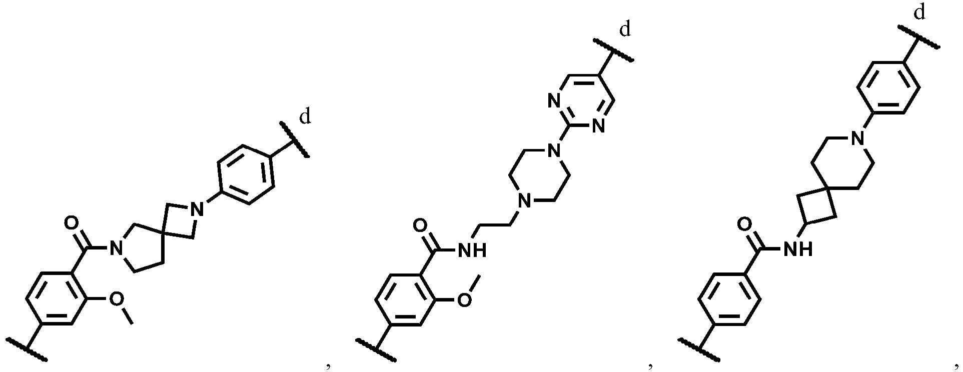 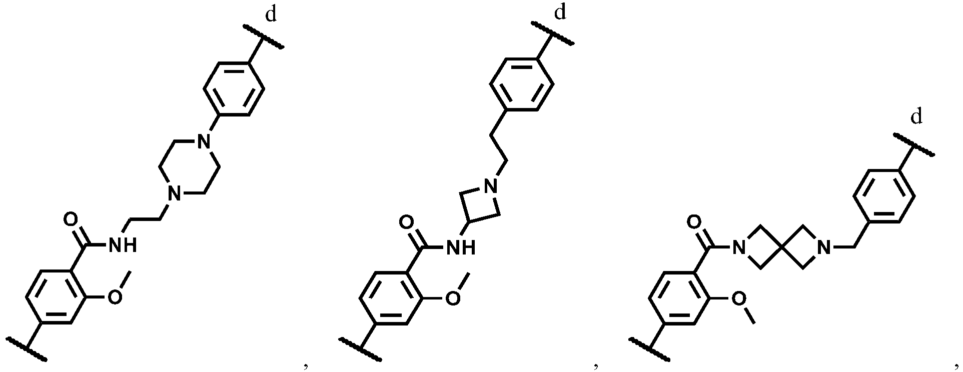 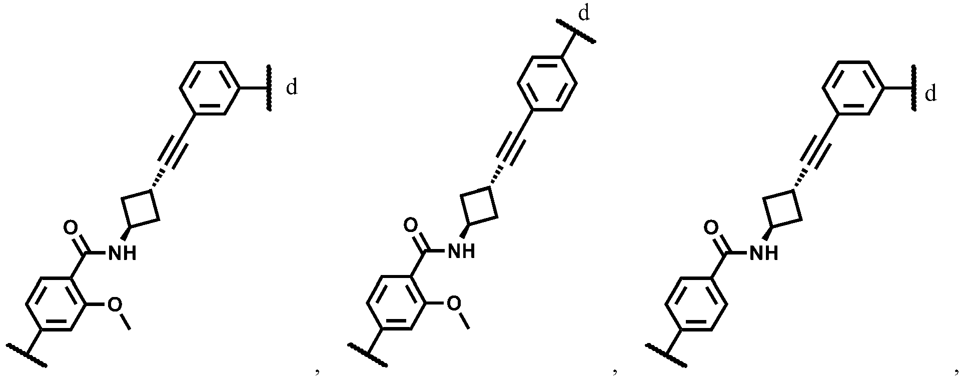 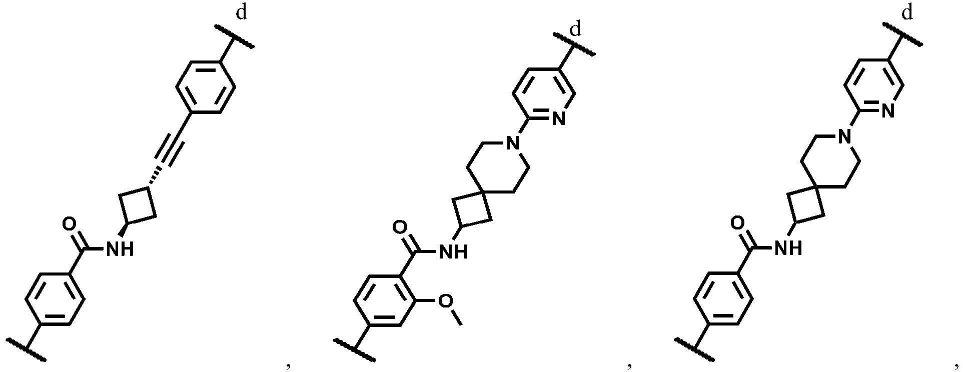 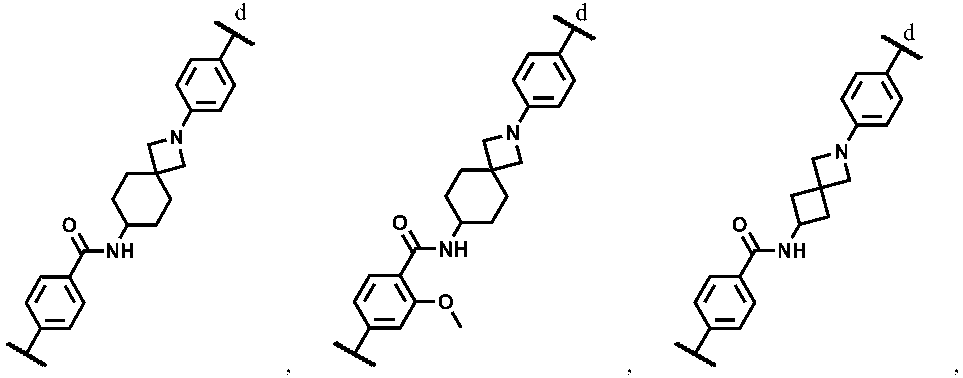 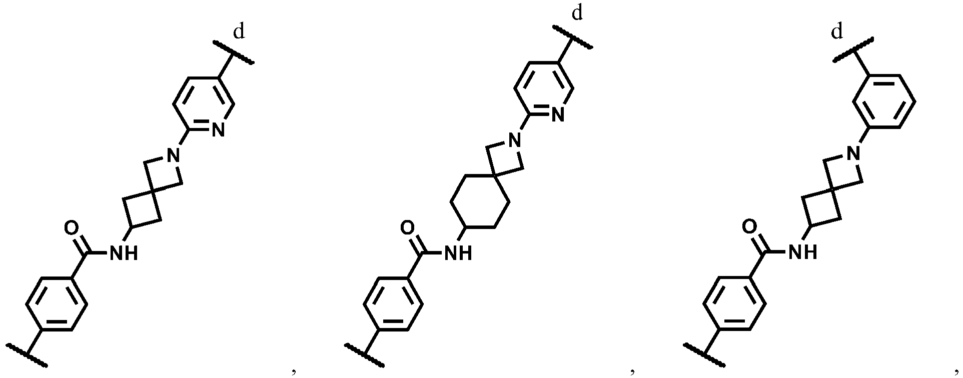 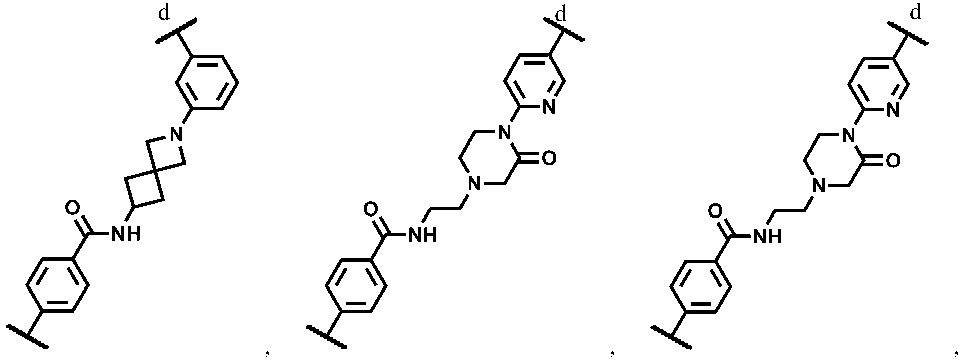 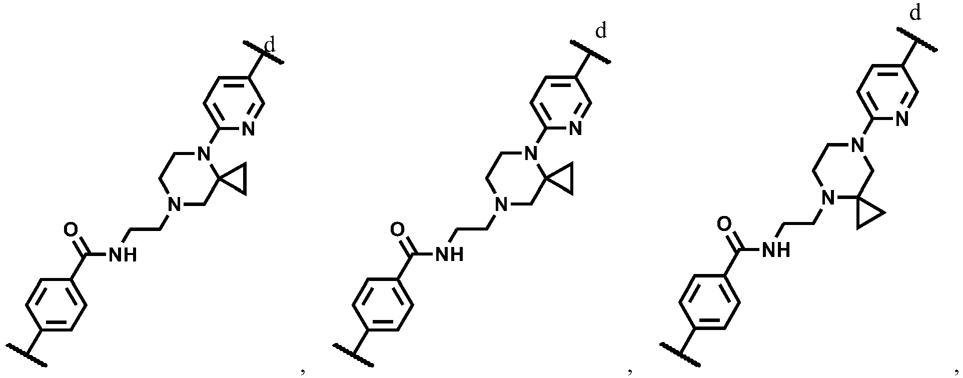 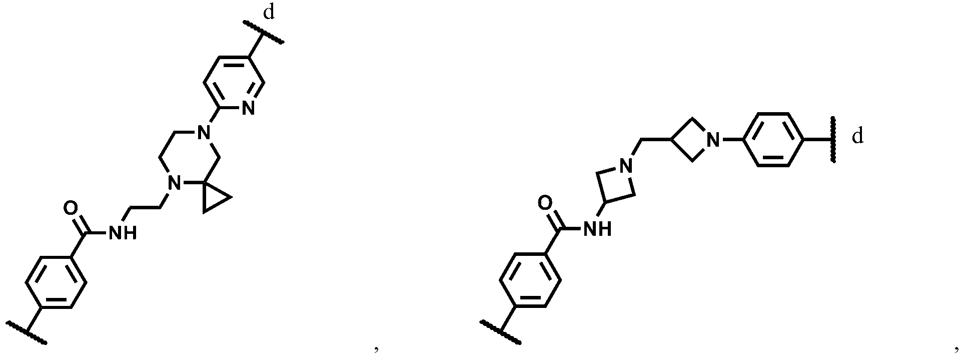 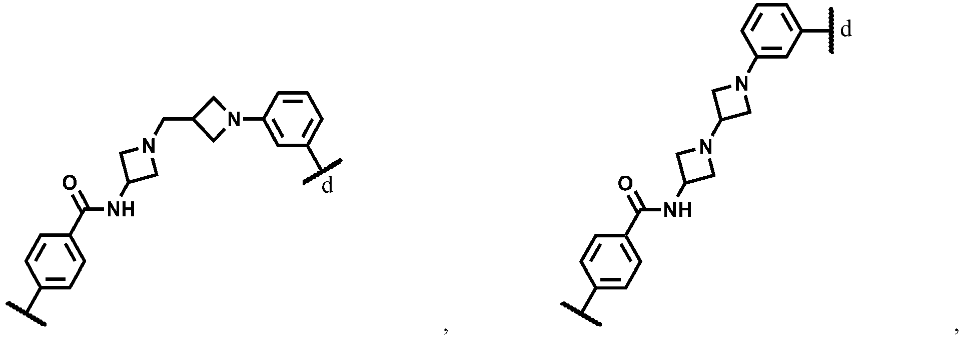 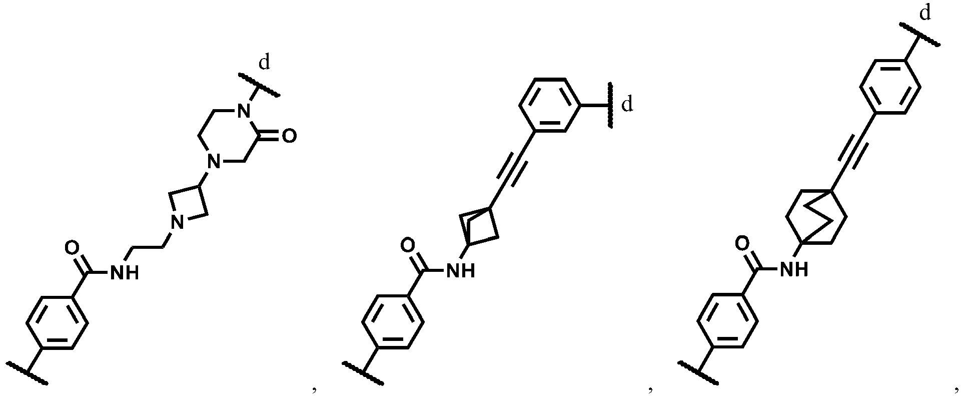 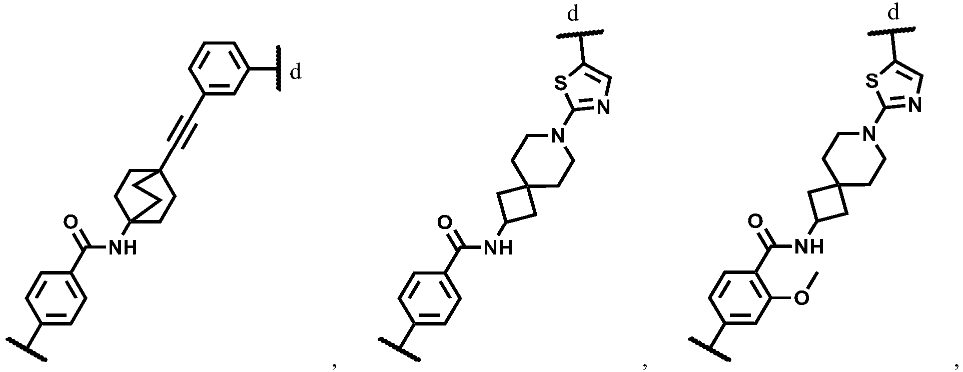 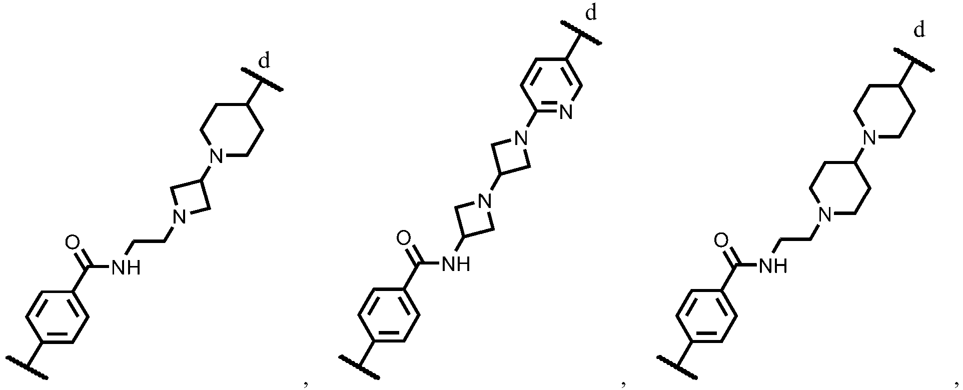 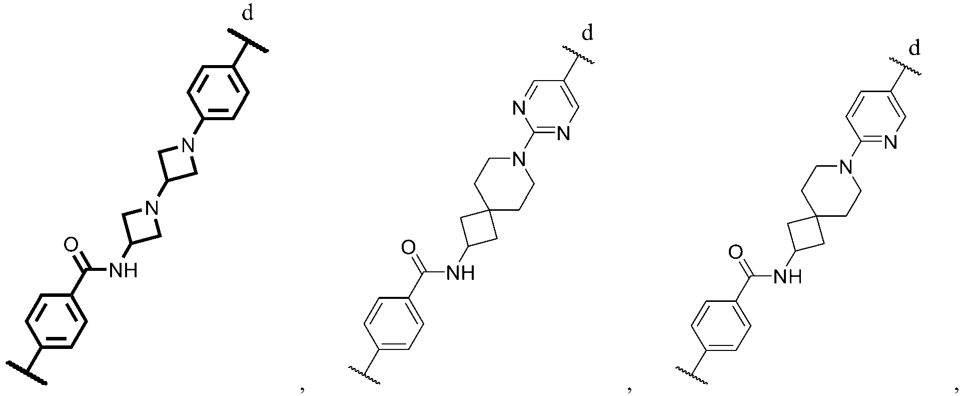 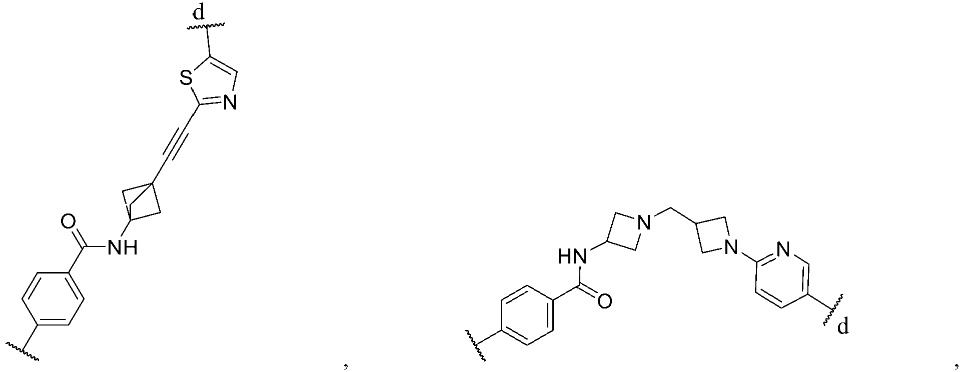 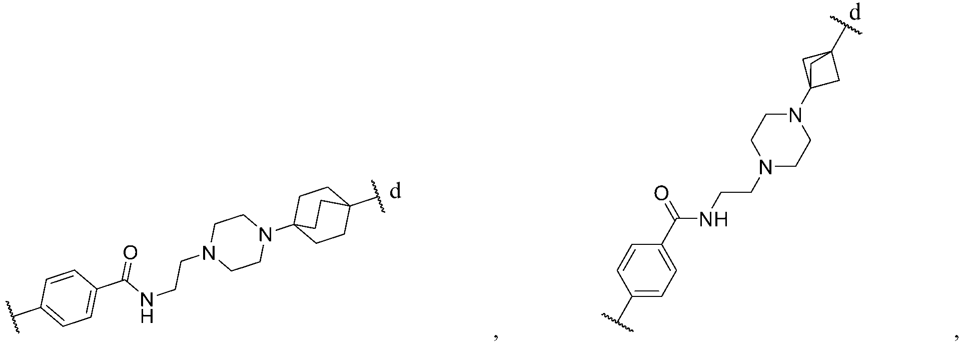 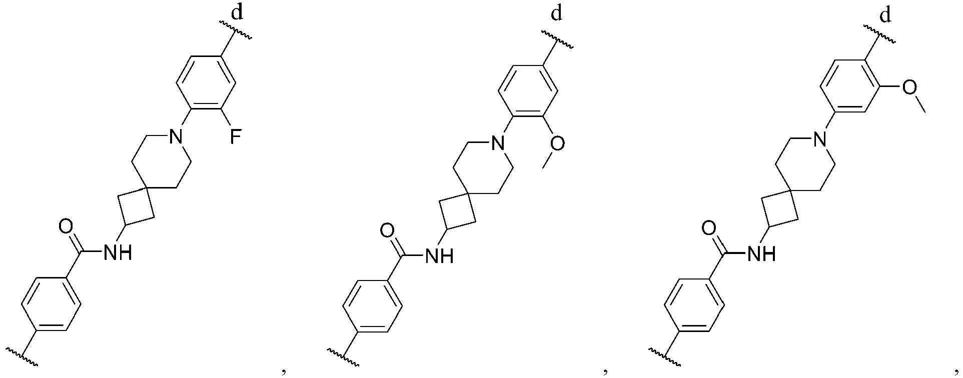 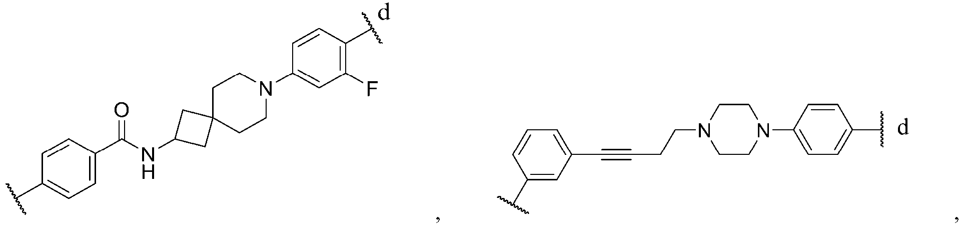 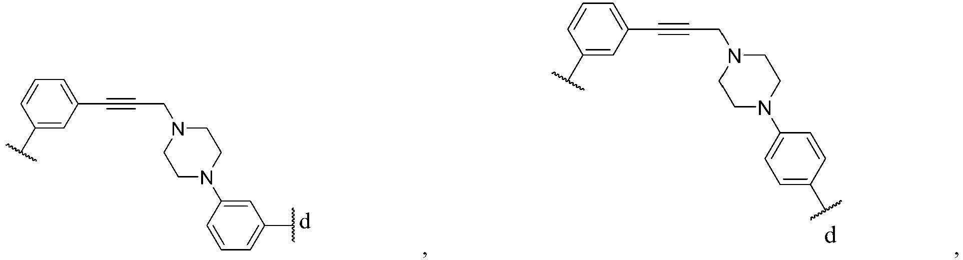 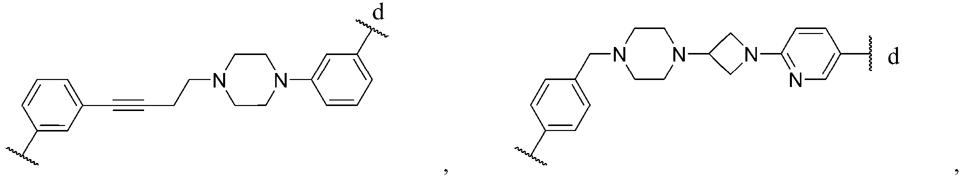 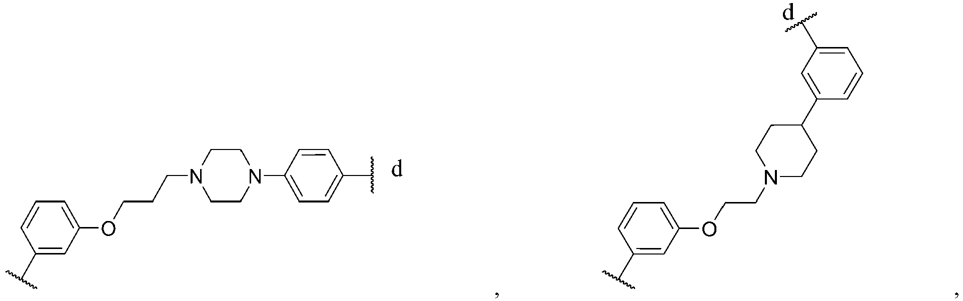 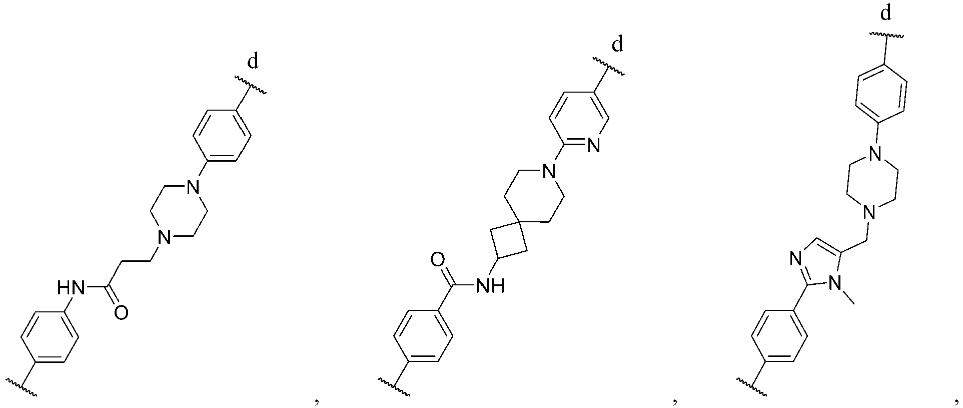 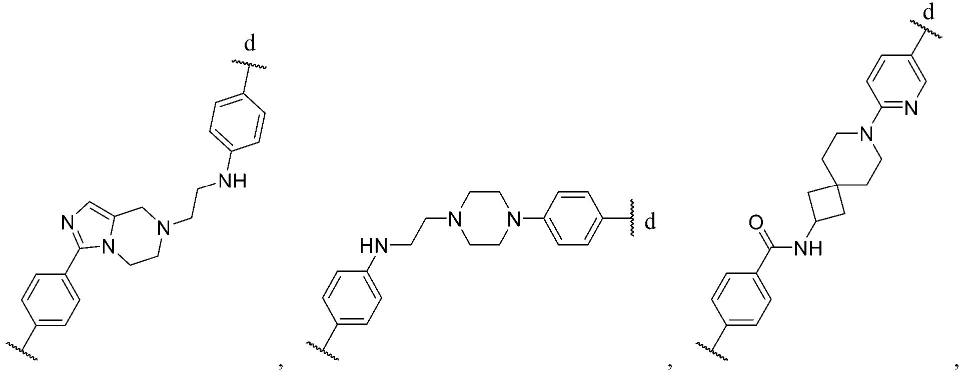 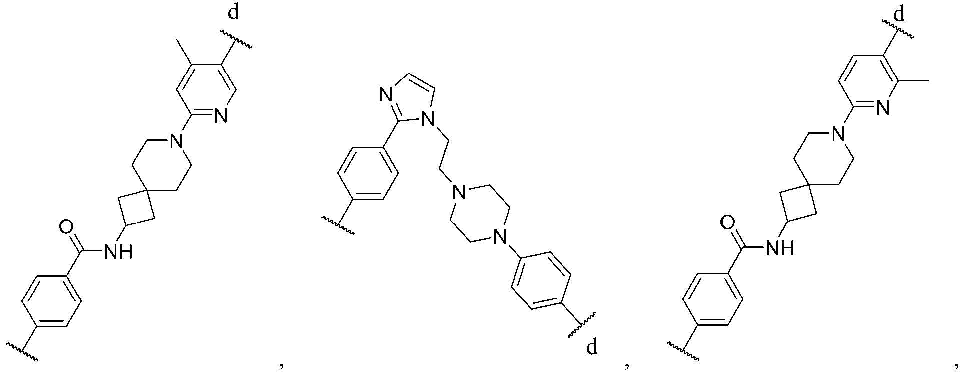 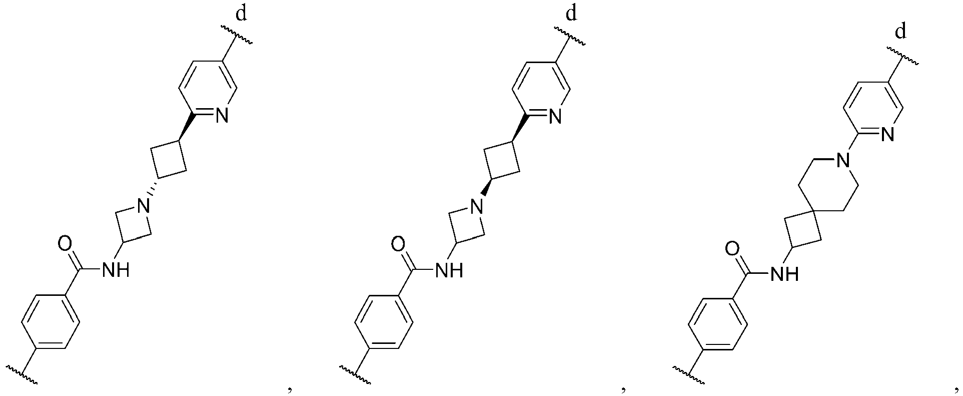 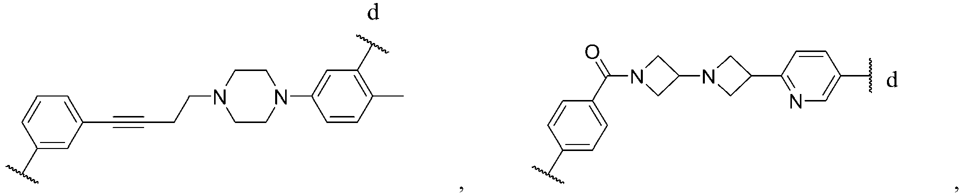 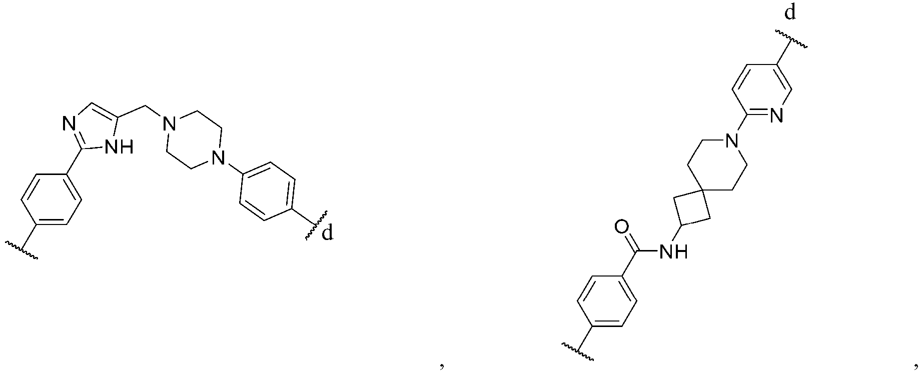 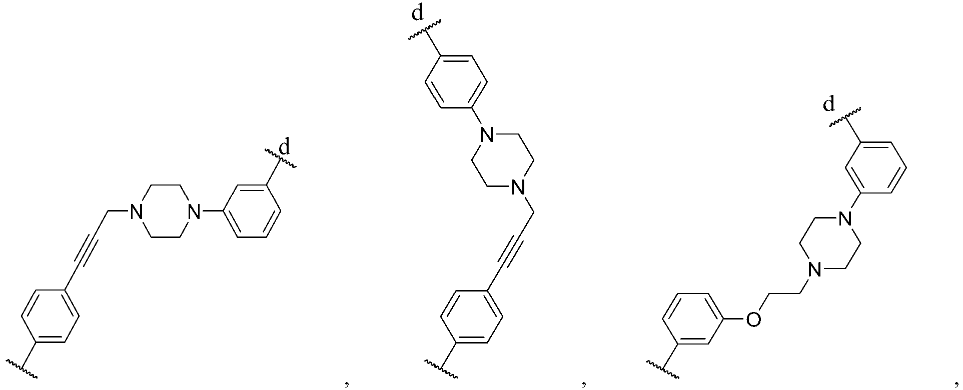 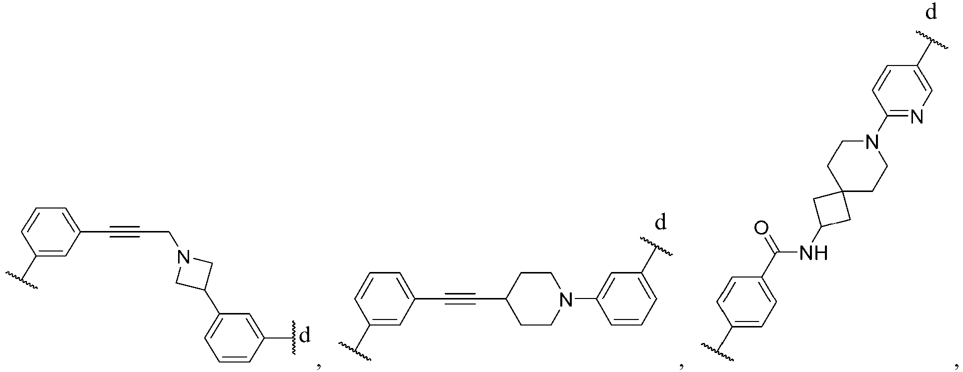 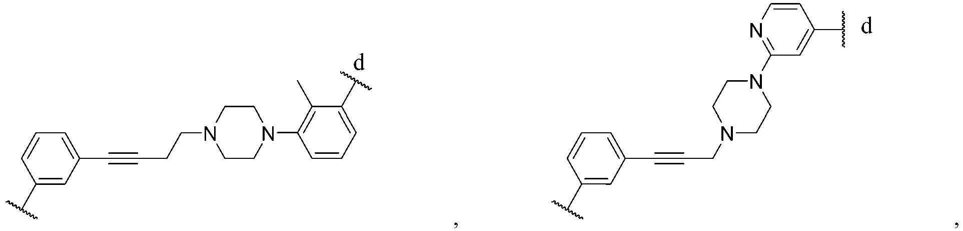 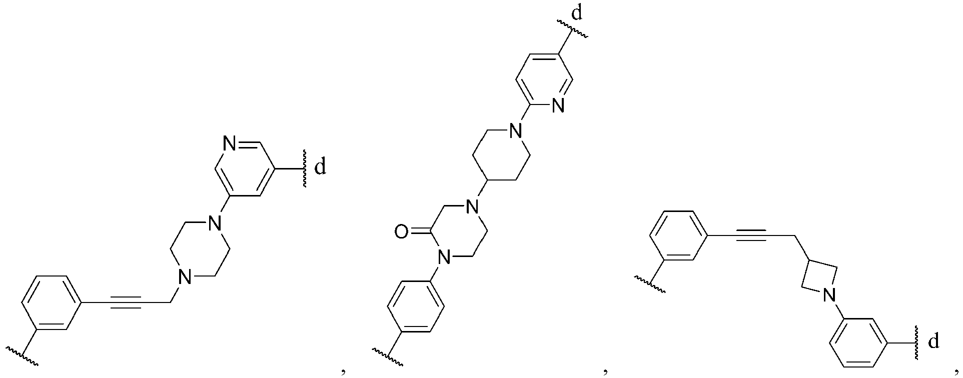 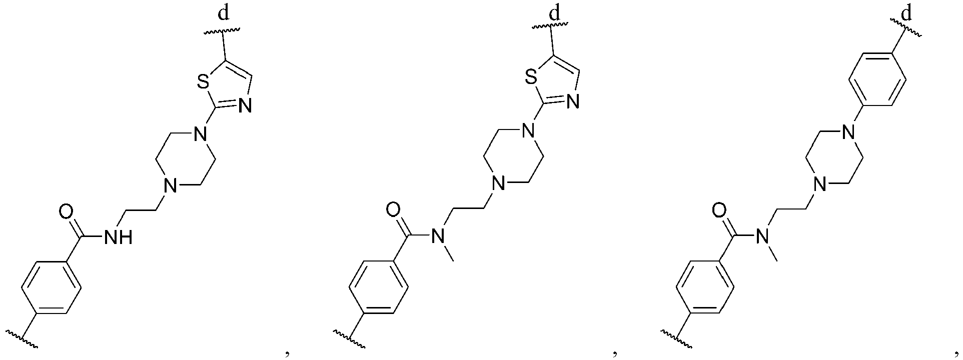 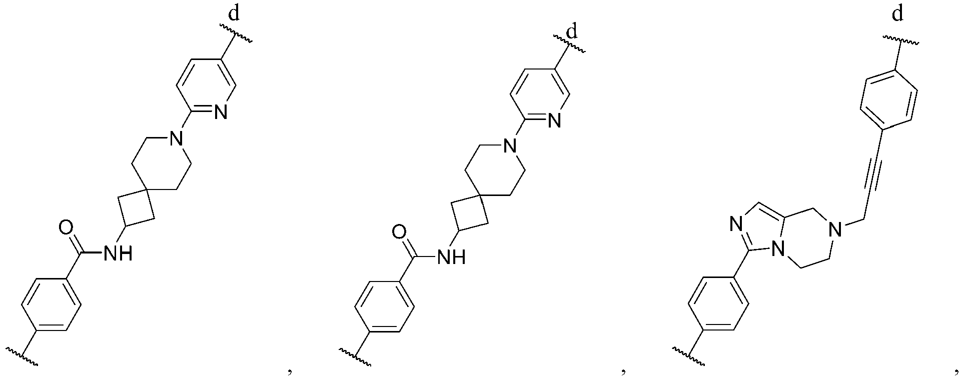 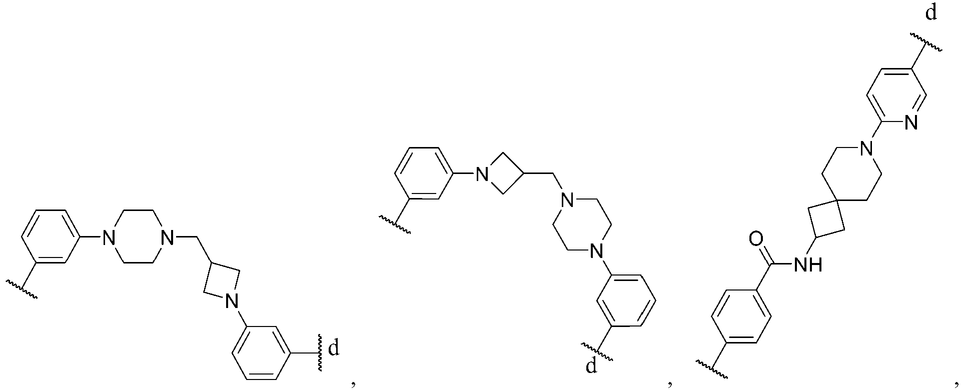 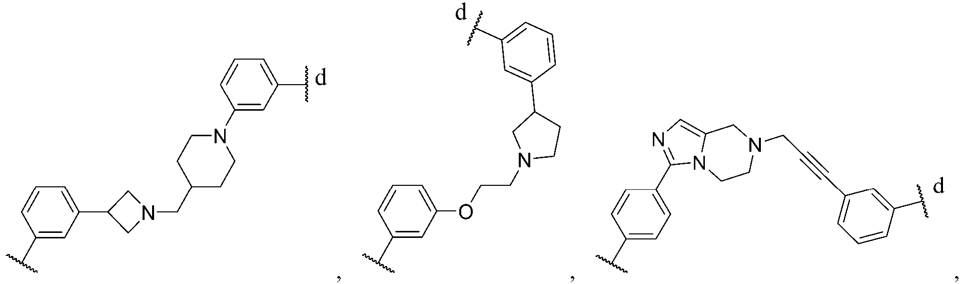 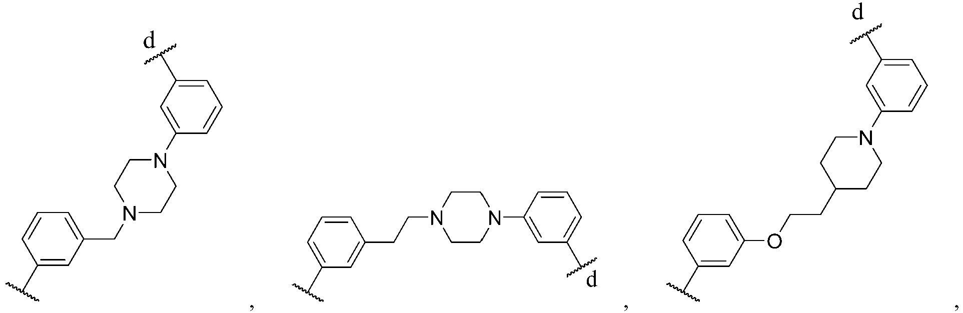 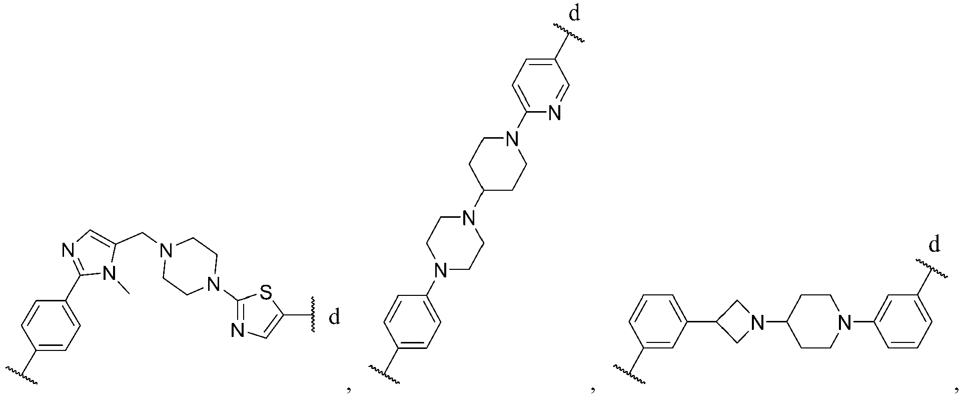 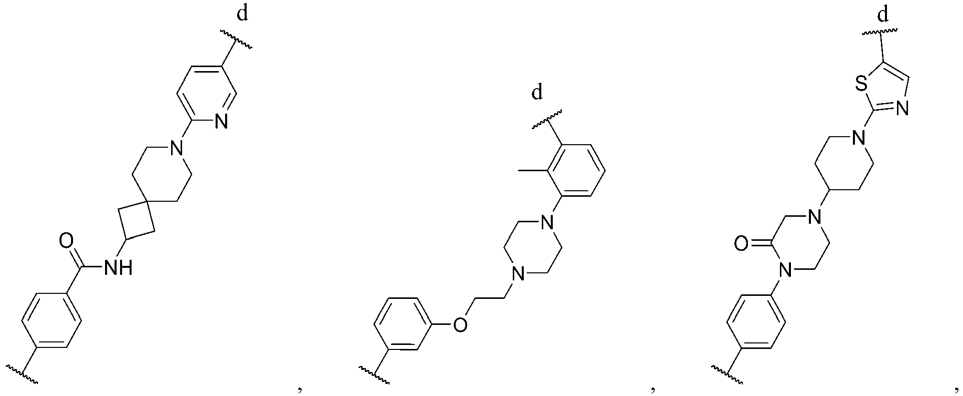 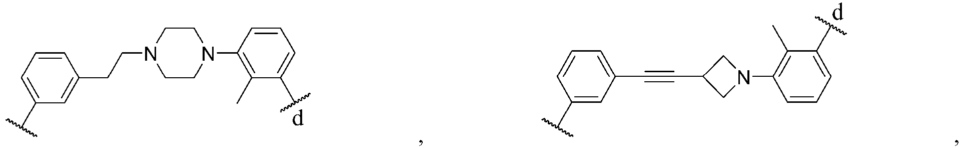 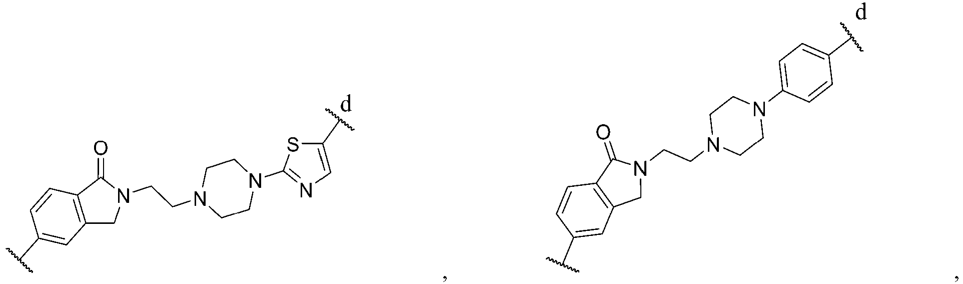 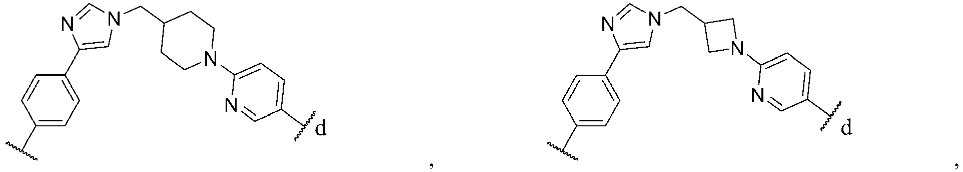 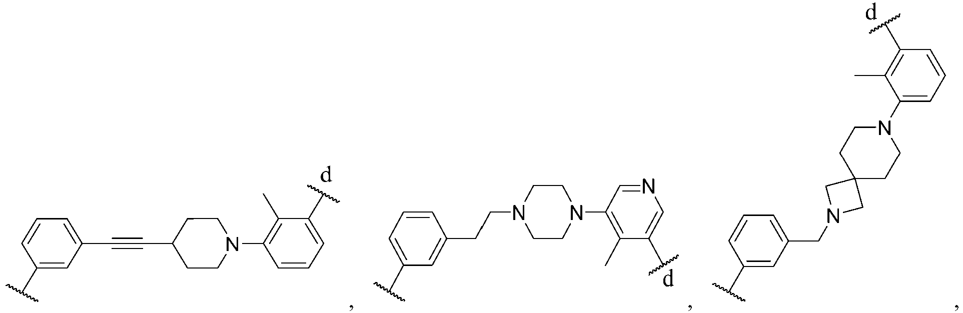 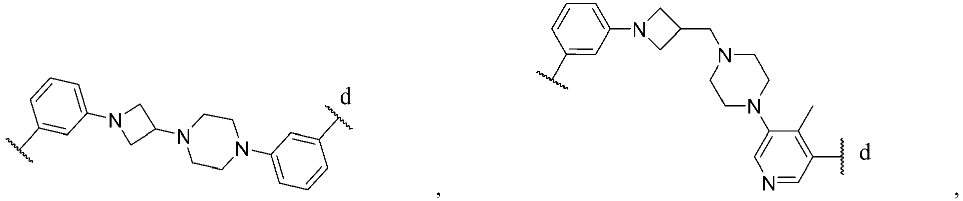 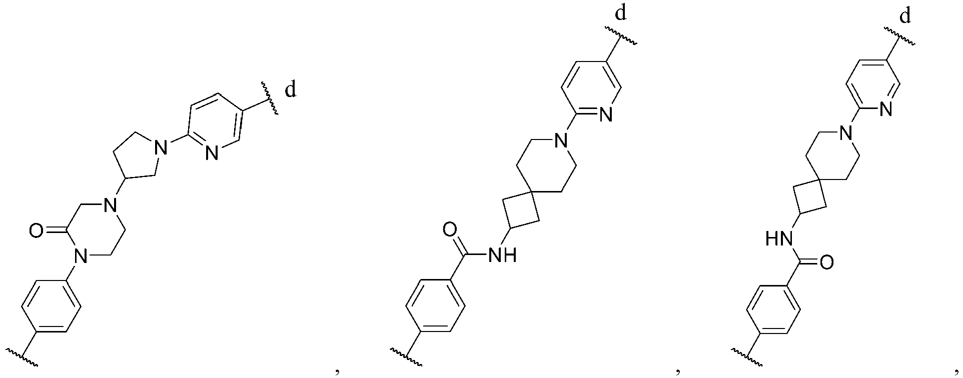 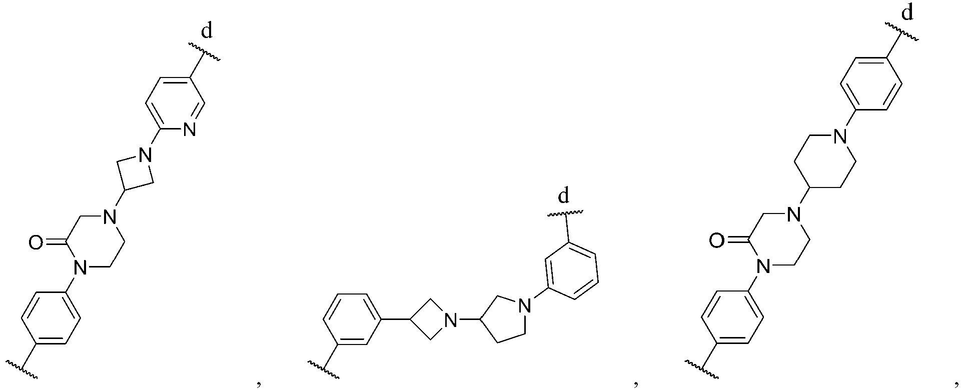 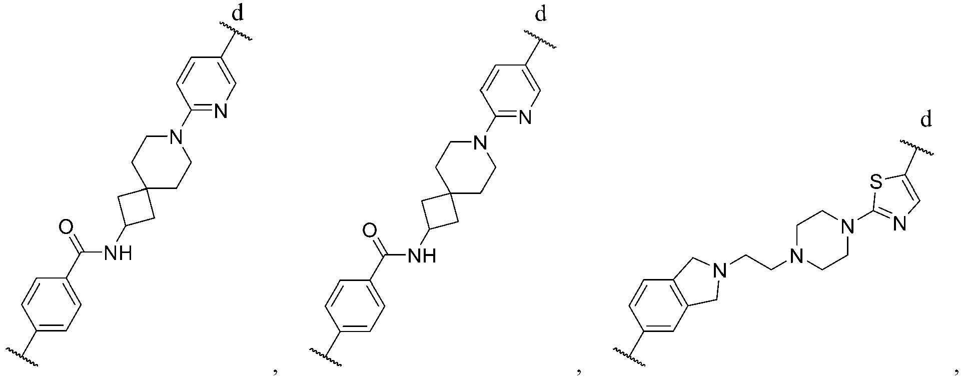 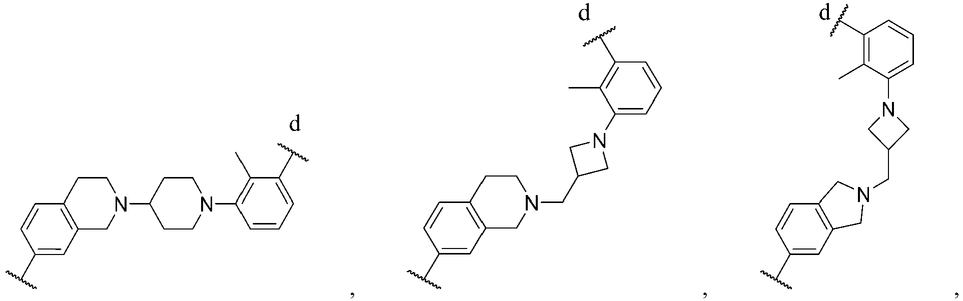 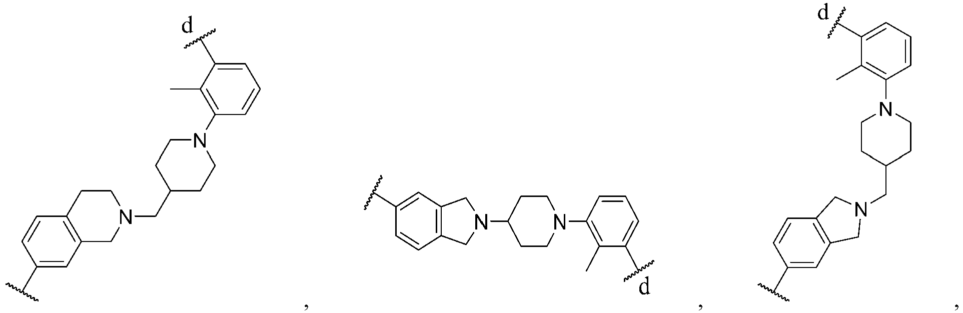 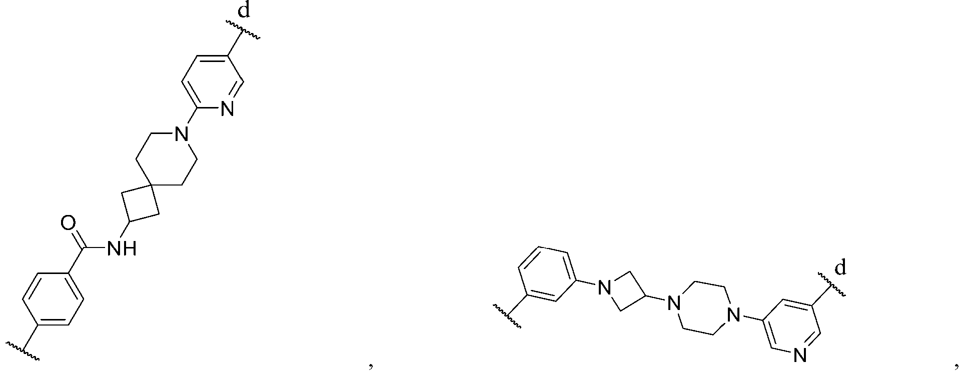 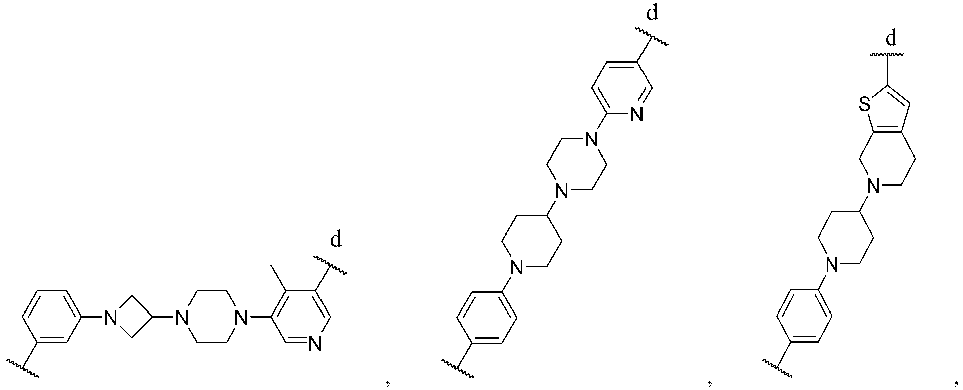 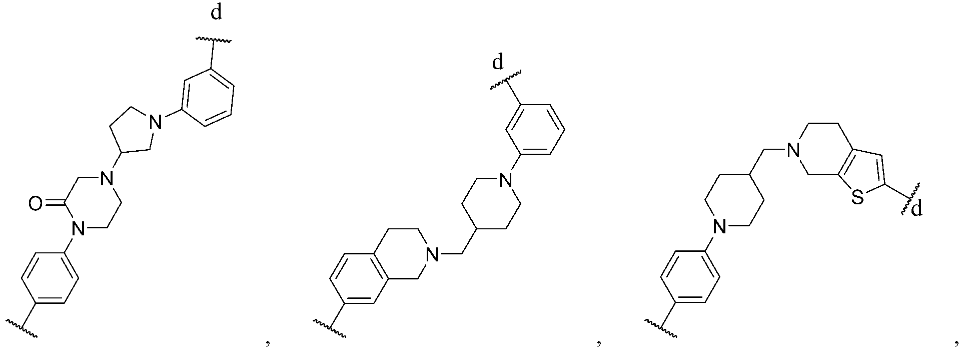 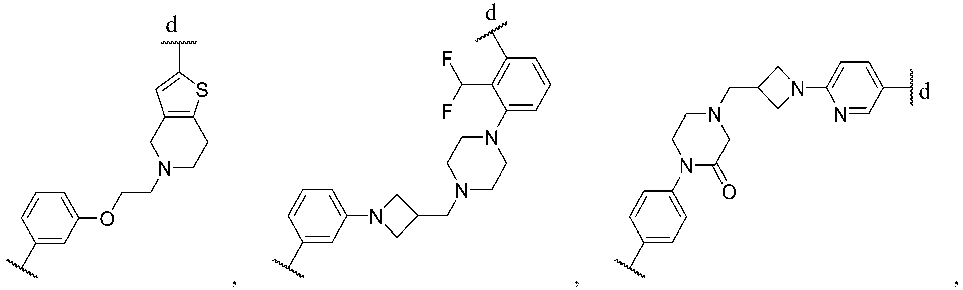 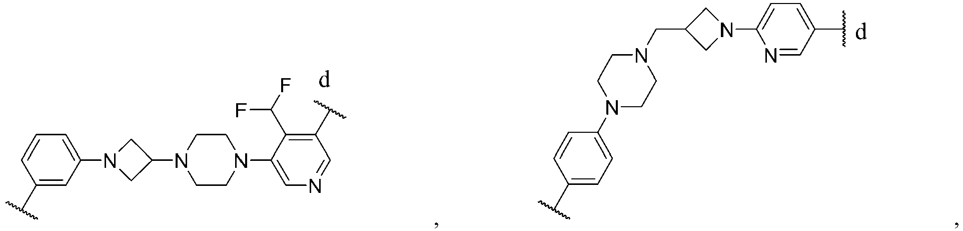 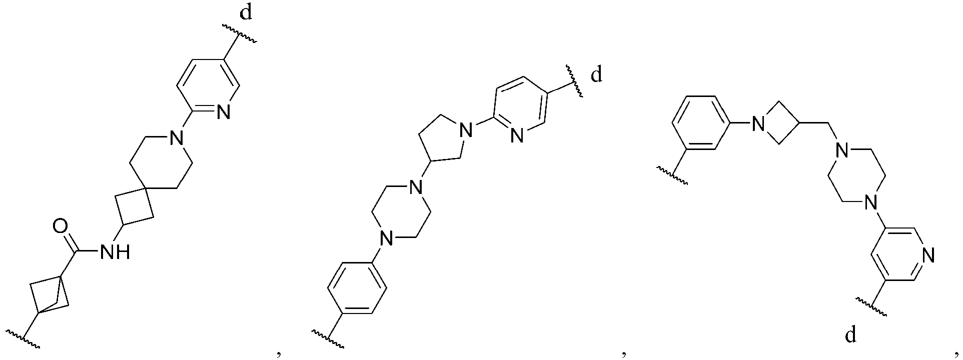 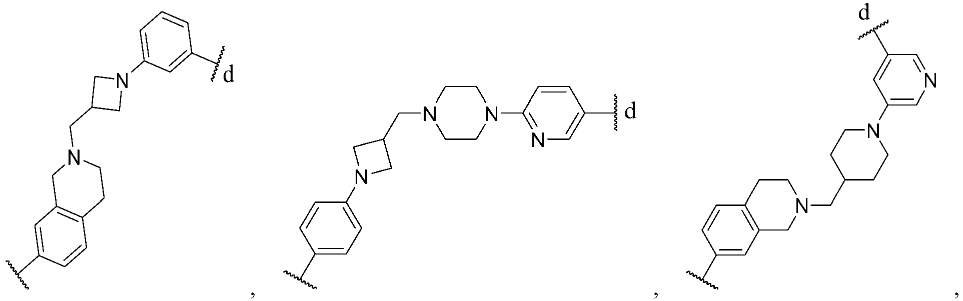 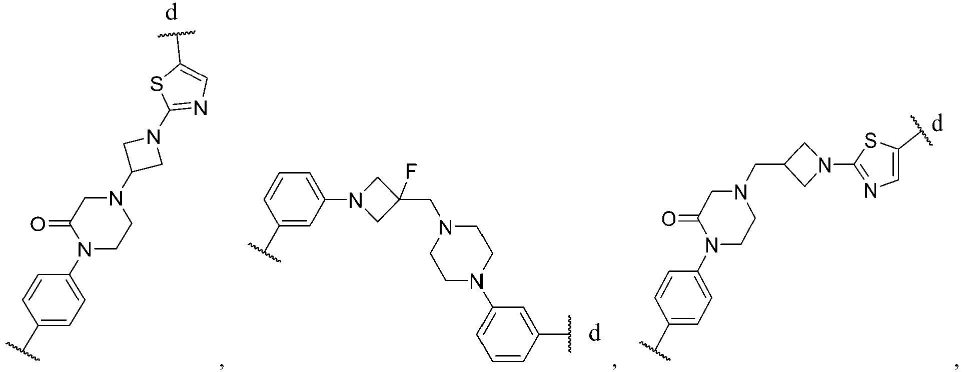 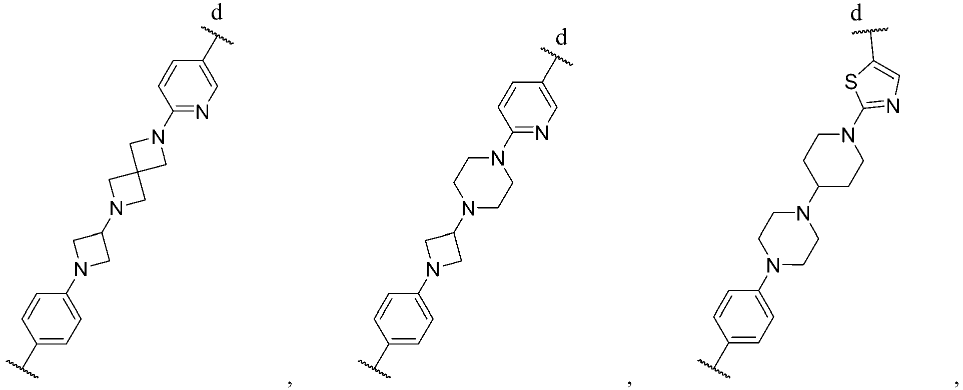 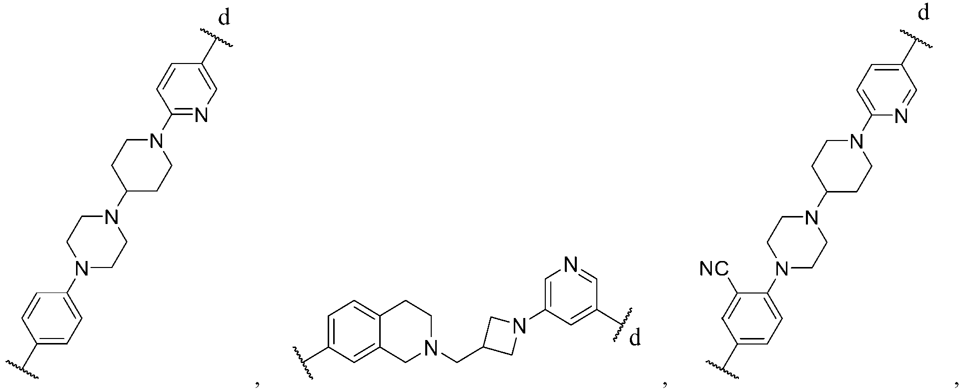 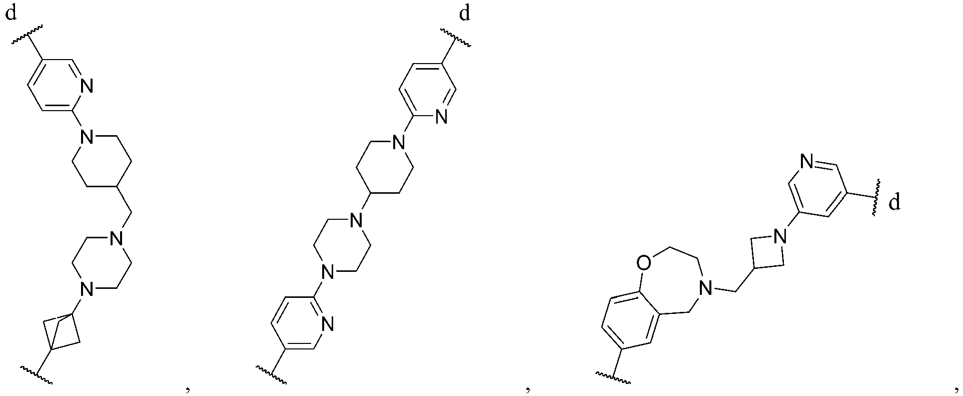 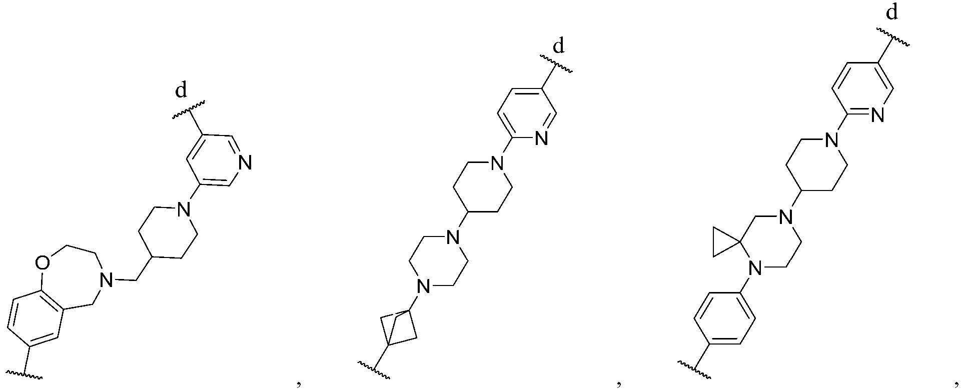 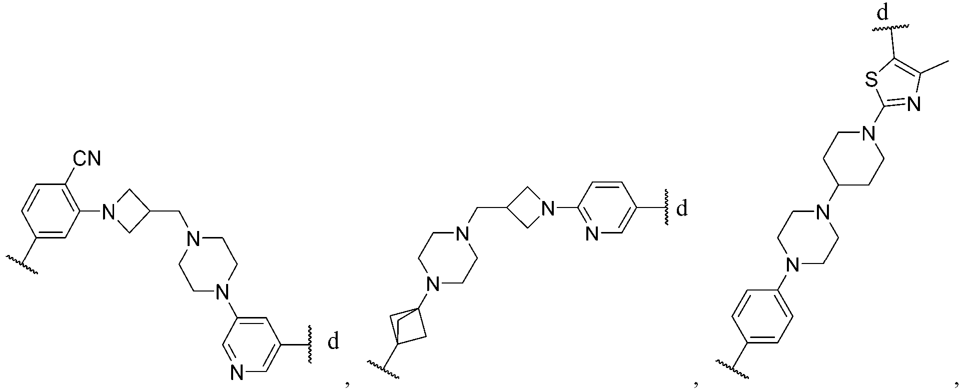 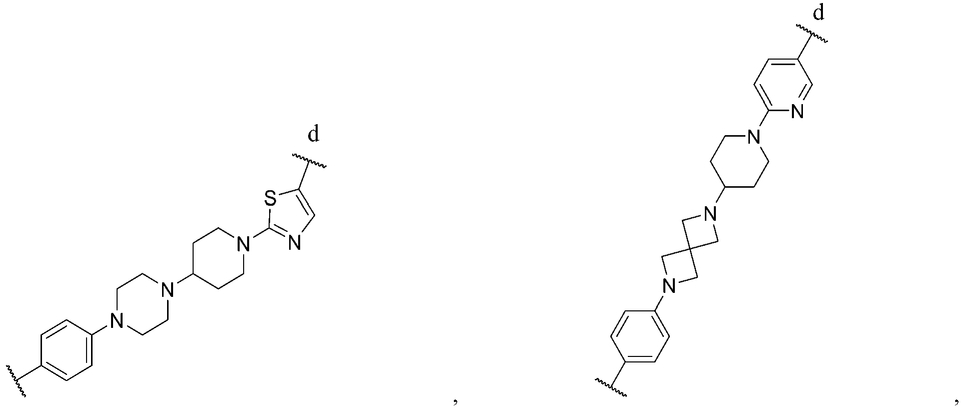 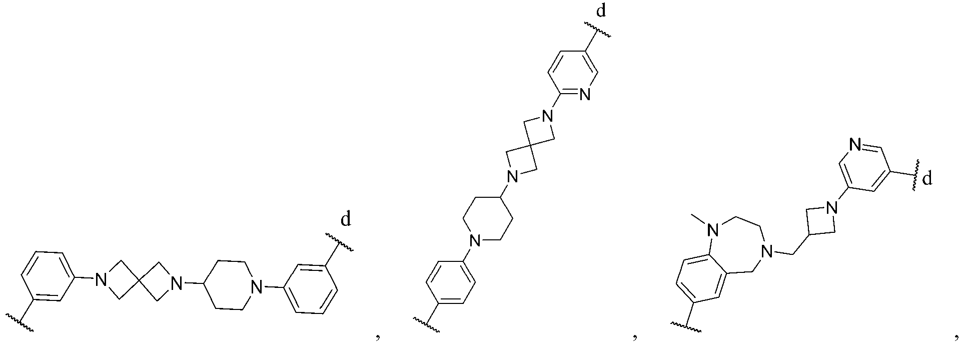 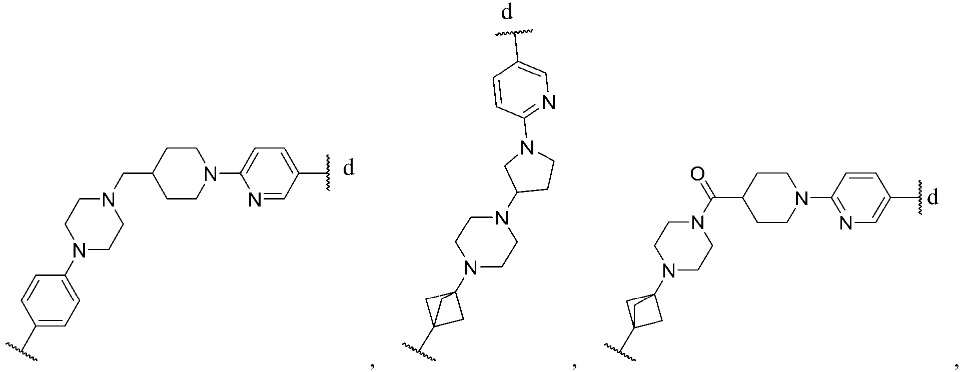 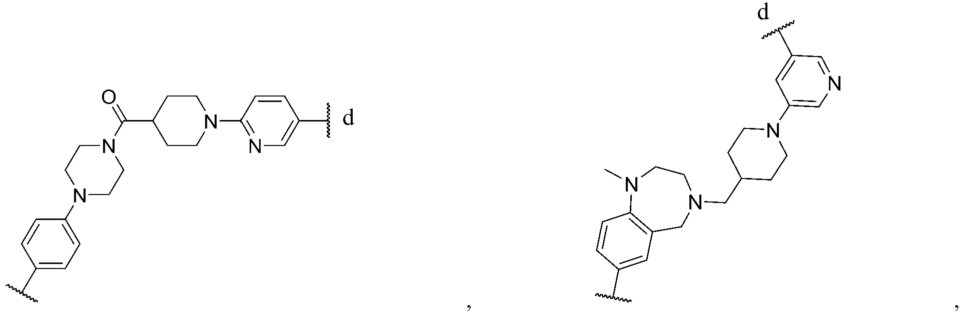 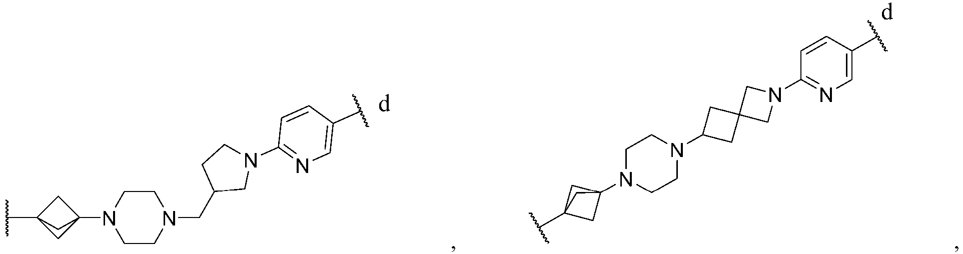 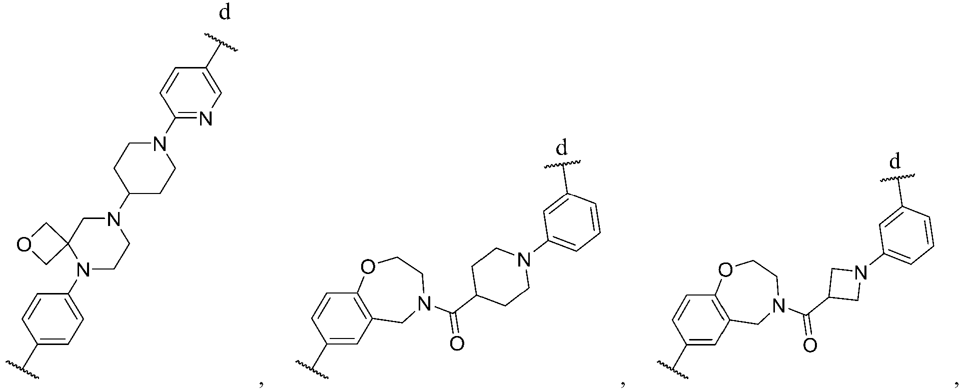 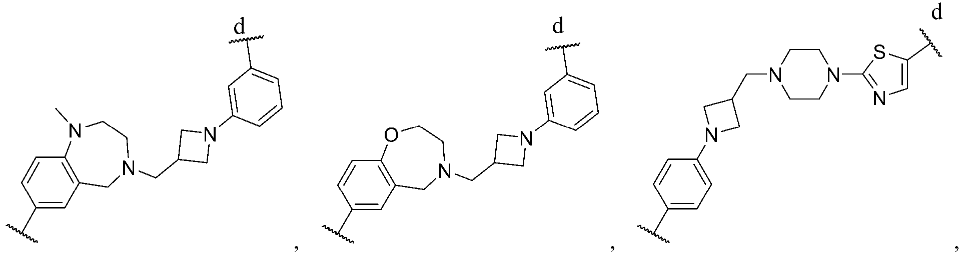 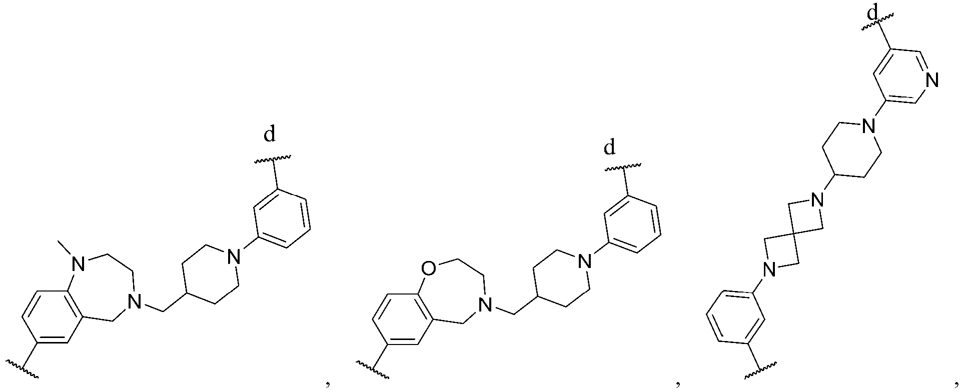 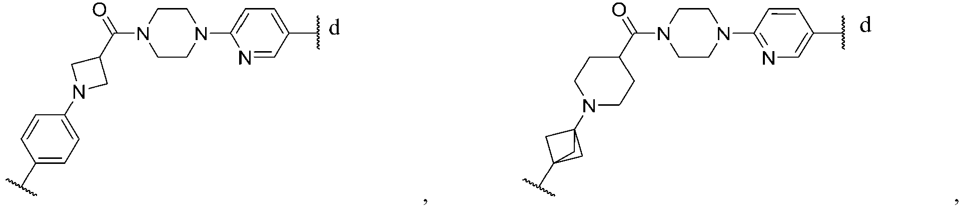 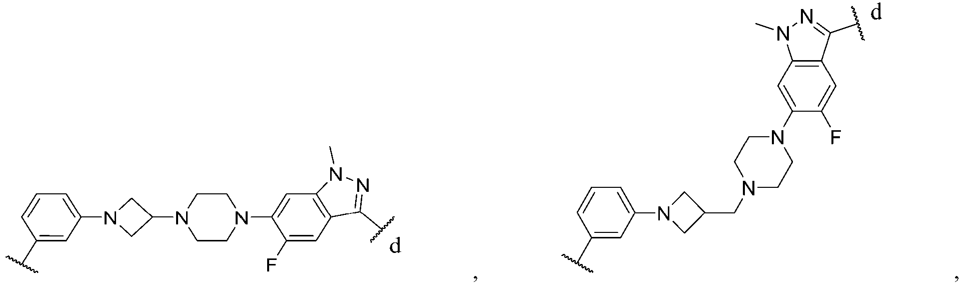 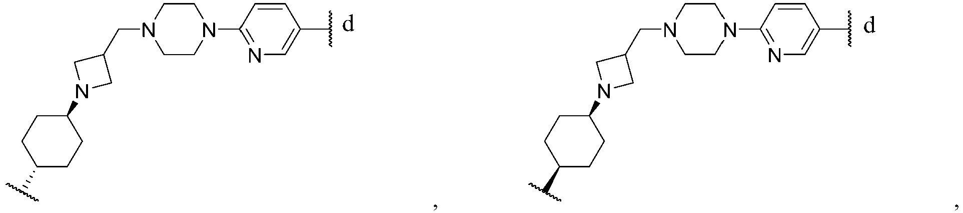 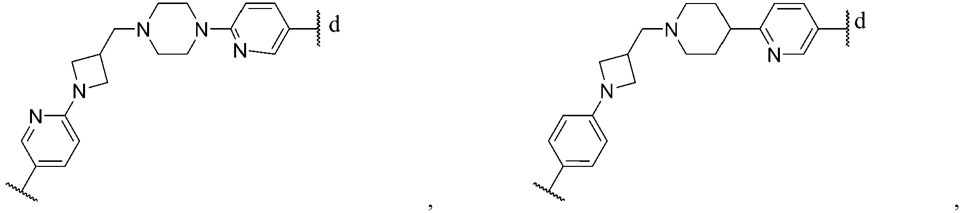 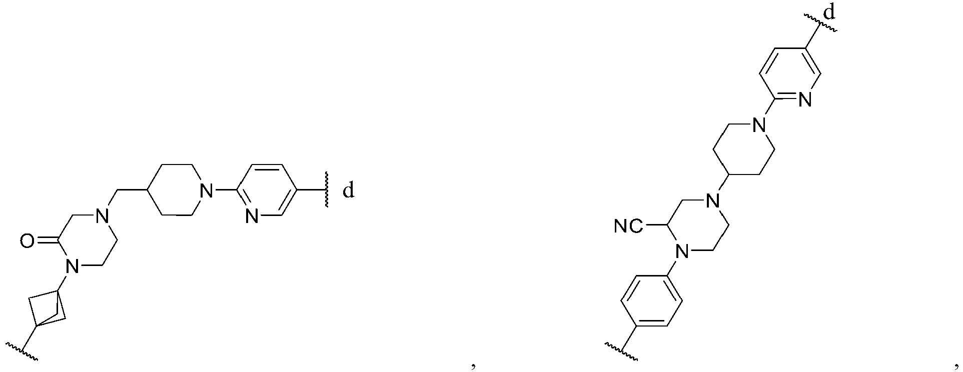 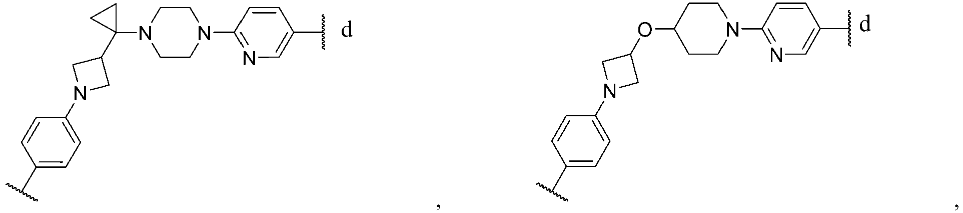 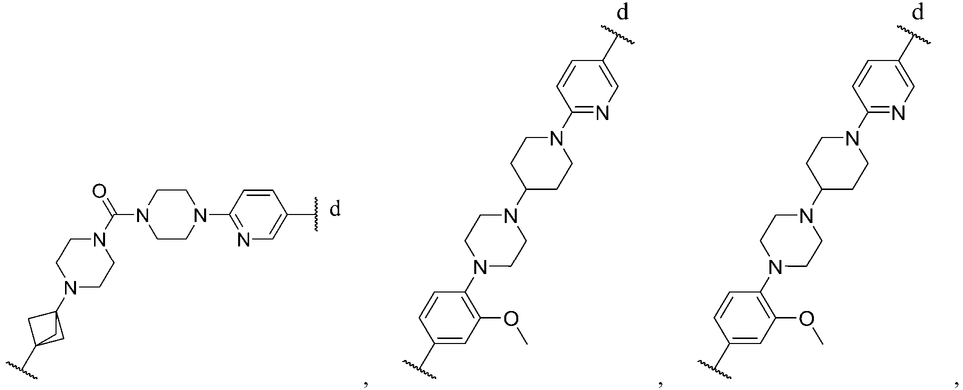 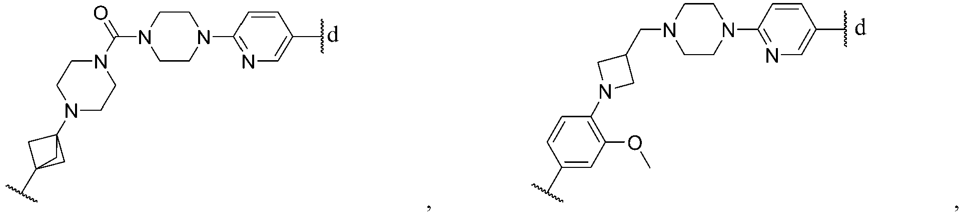 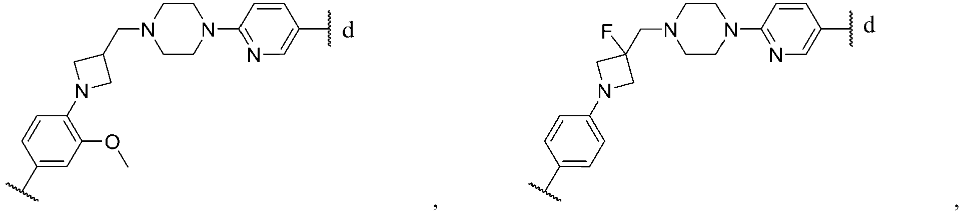 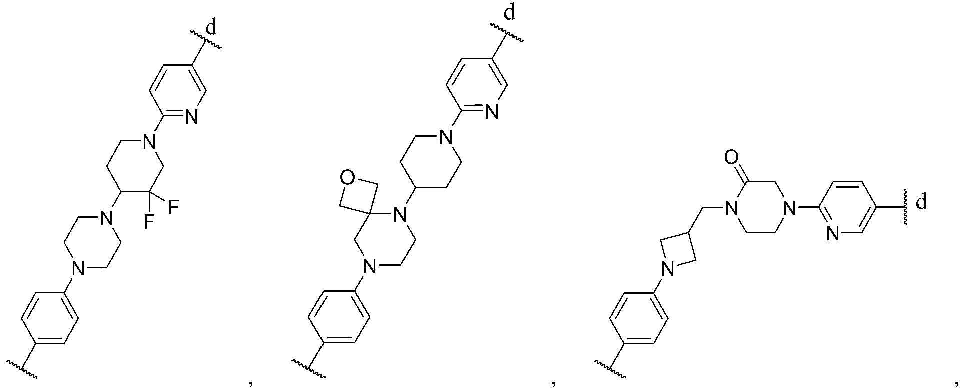 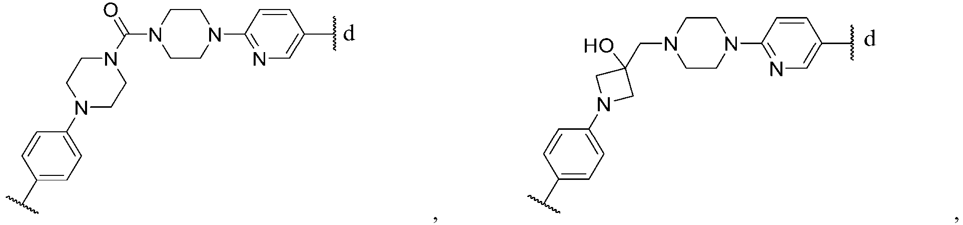 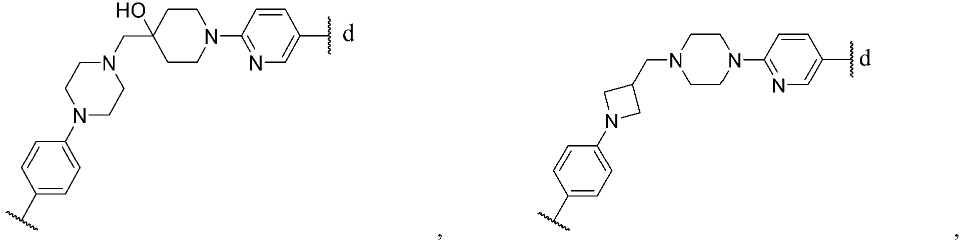 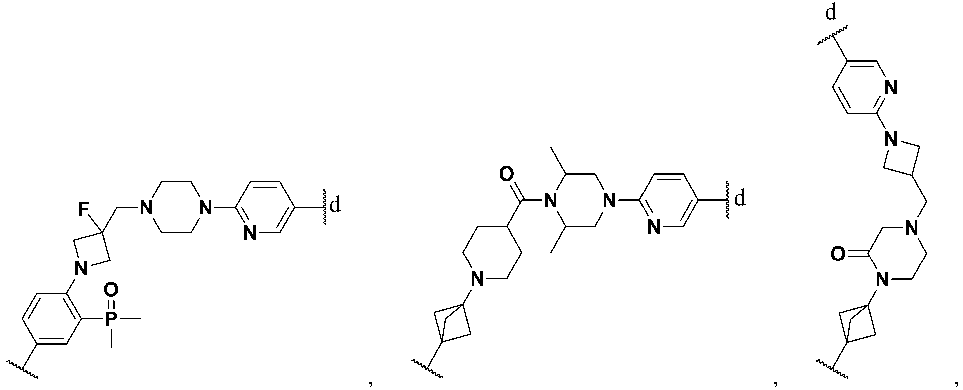 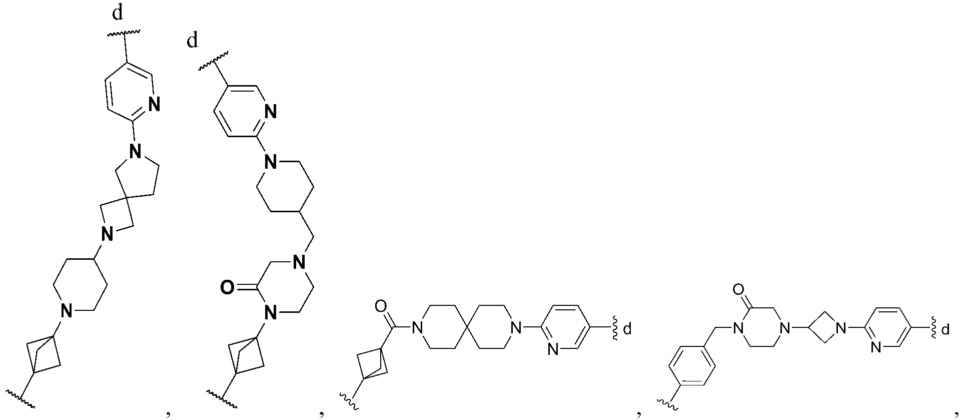 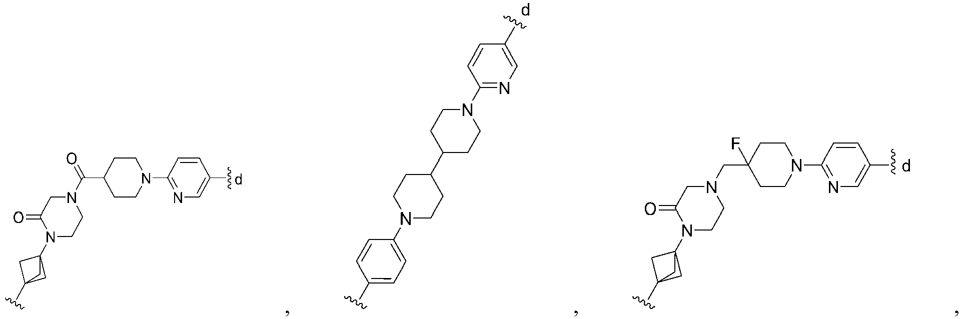 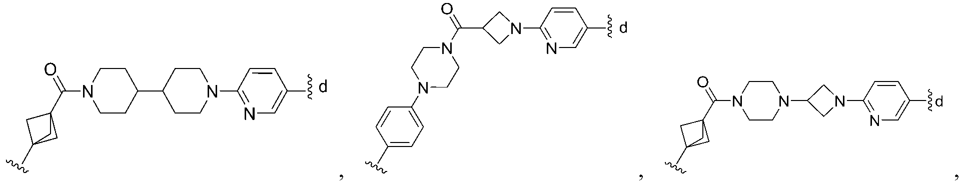 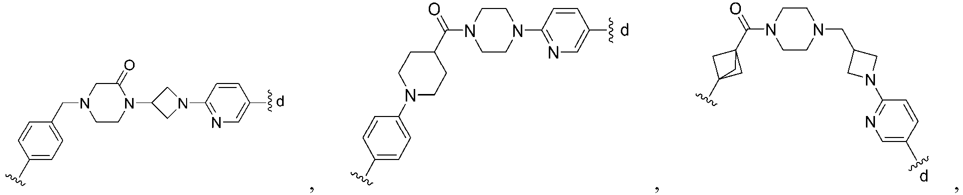 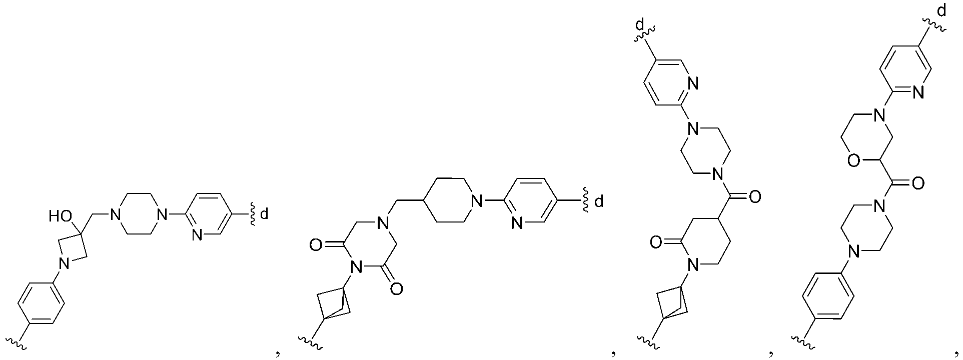 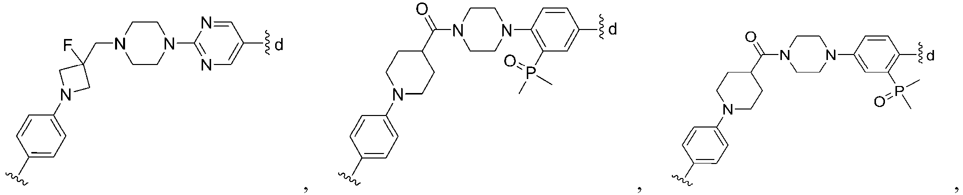 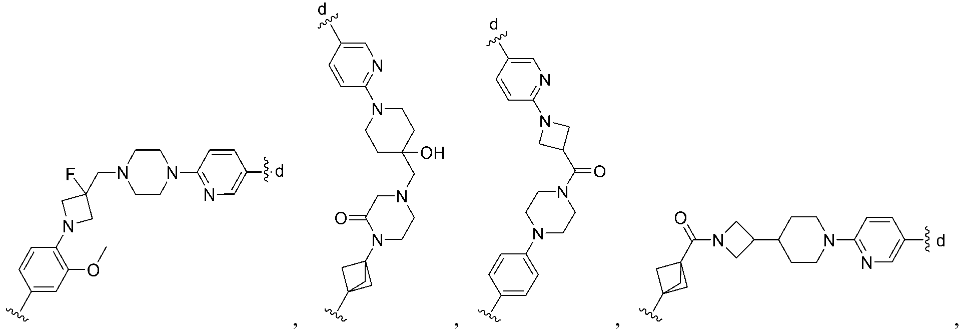 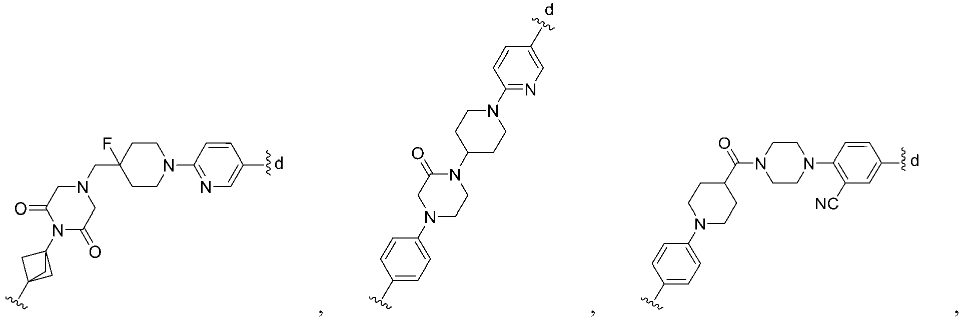 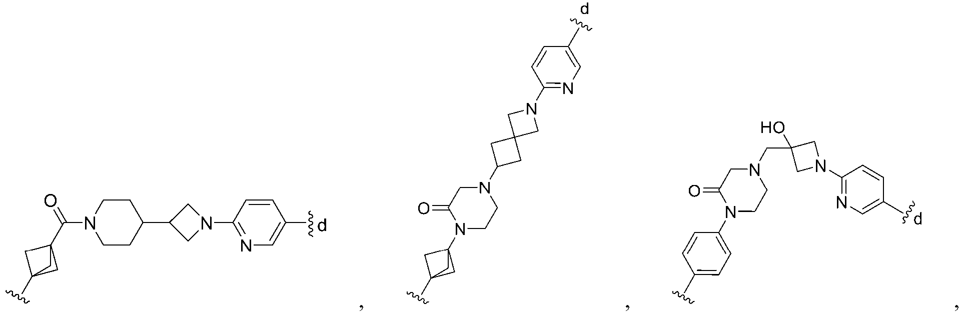 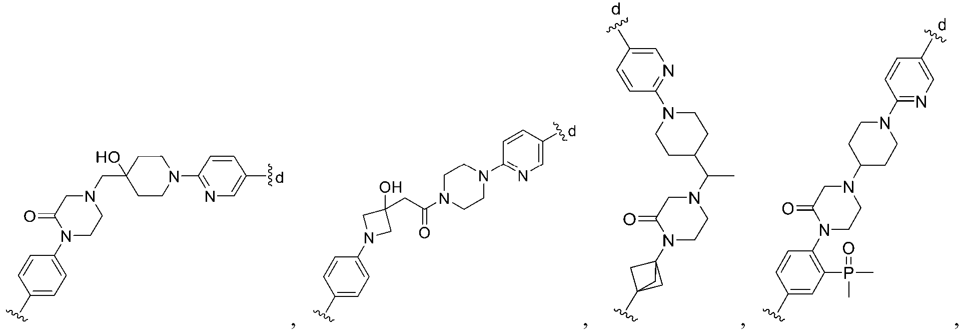 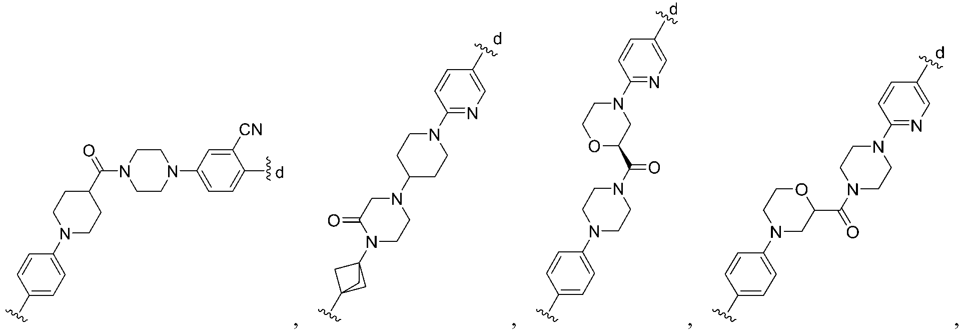 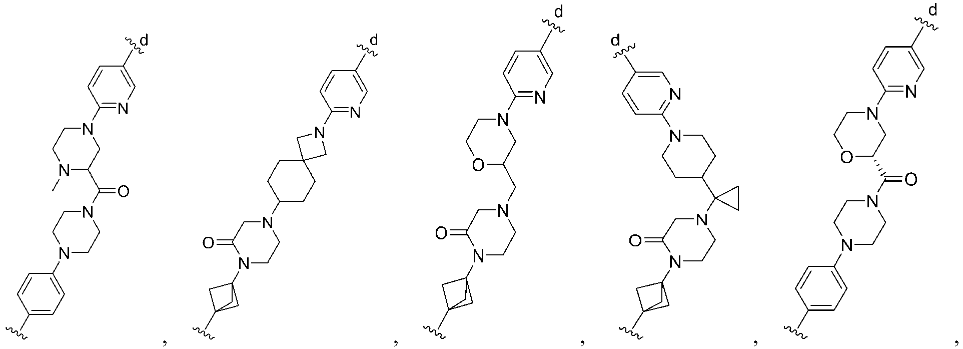 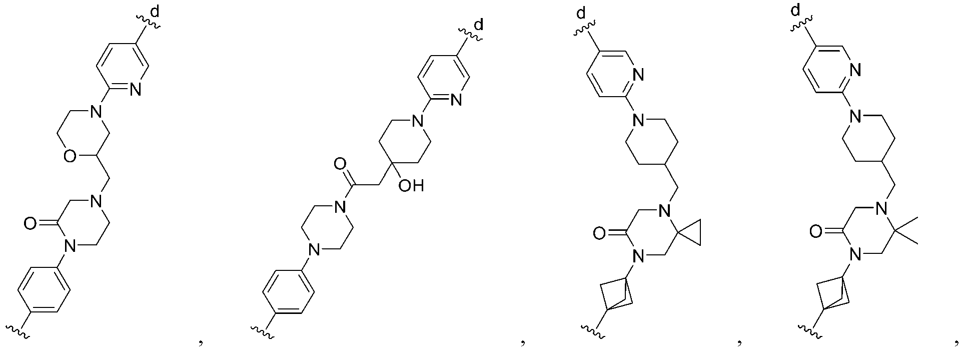 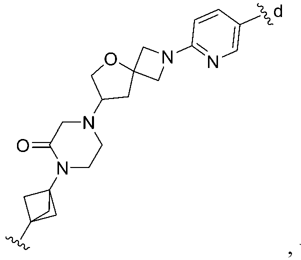 wherein side d is connected to 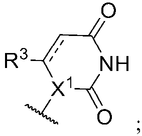 preferably, in Scheme ii, - A-L-B- is any one of the above groups except 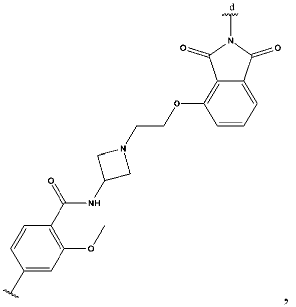 and in Scheme i, -A-L-B- is

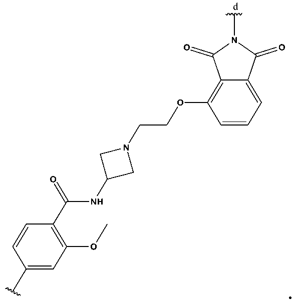

In one embodiment of the present invention, -A-L-B- is: 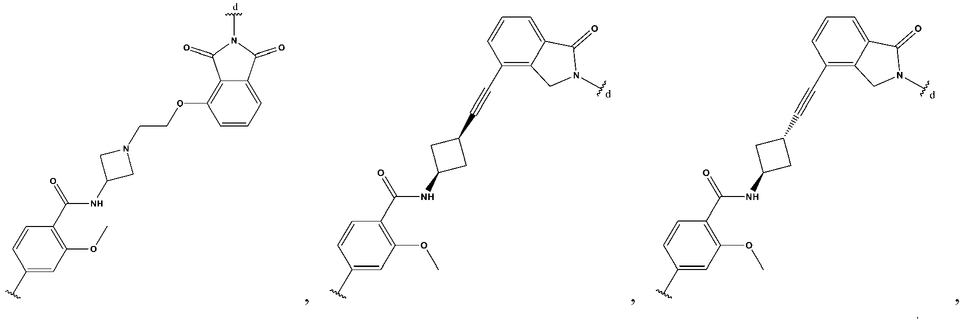 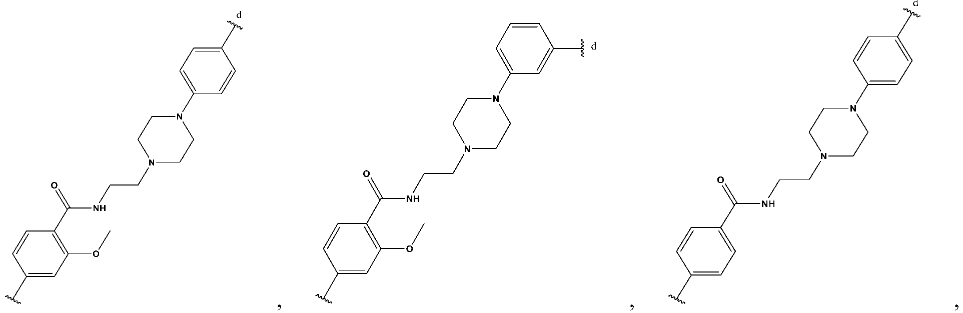 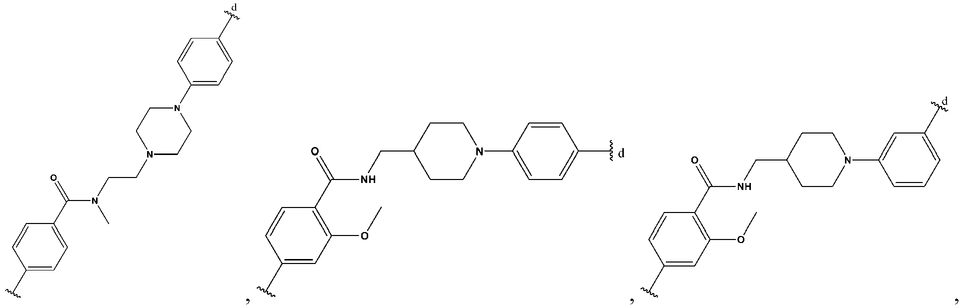 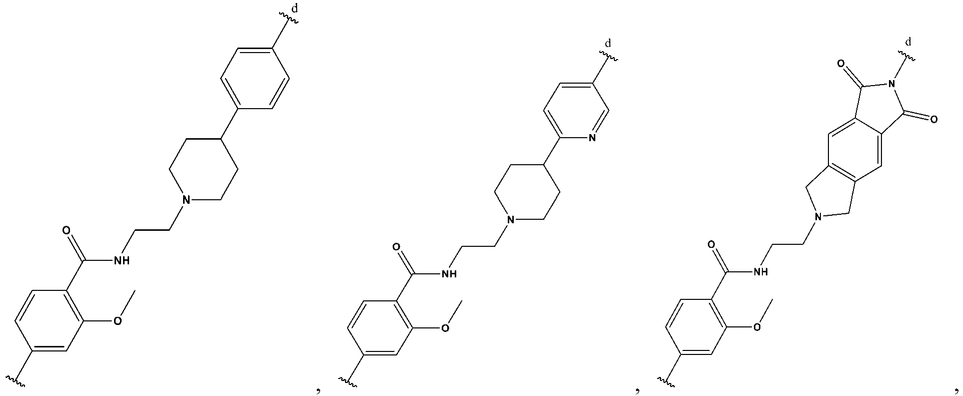 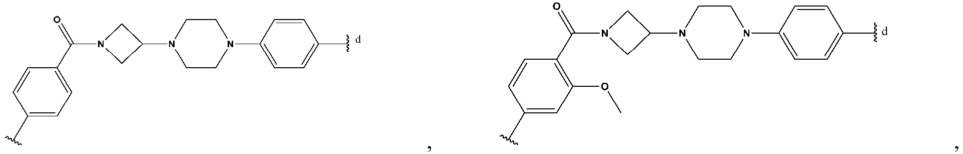 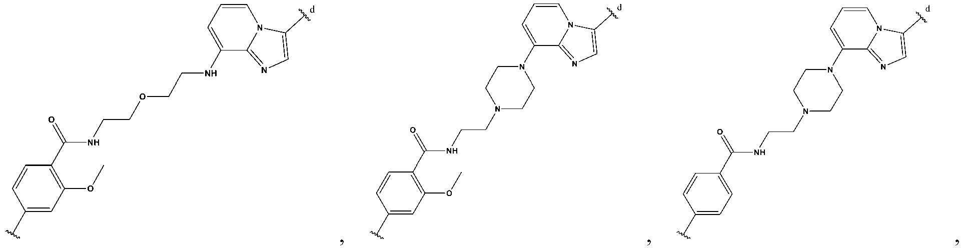 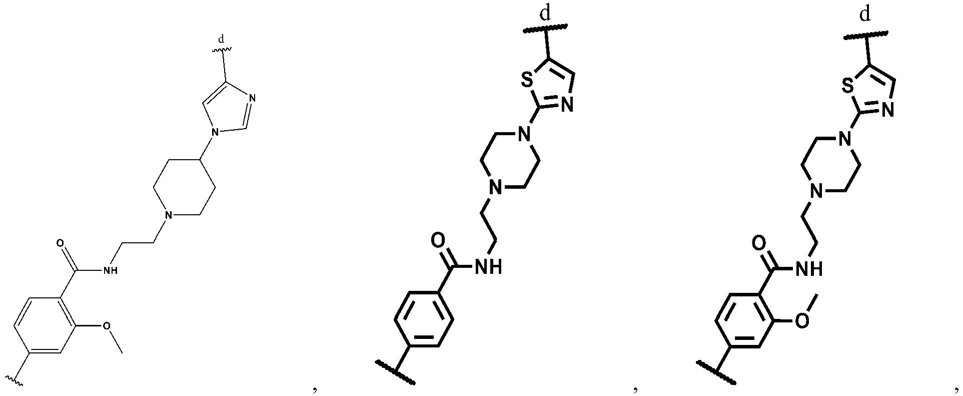 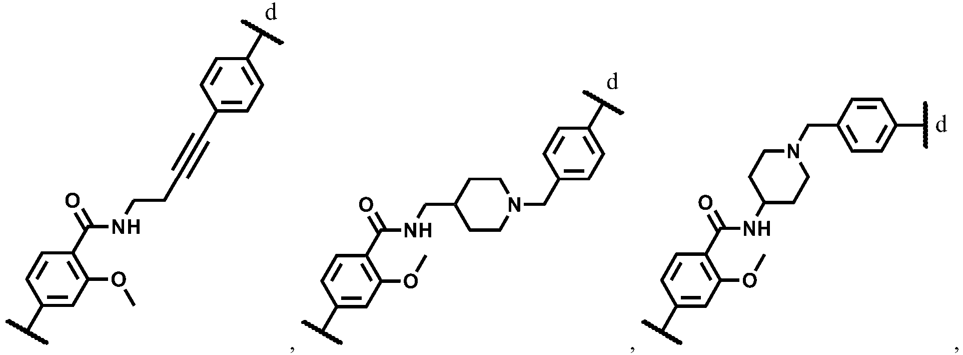 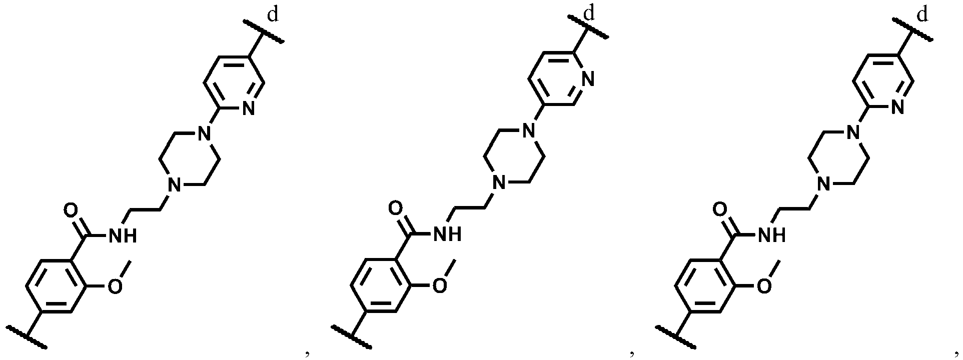 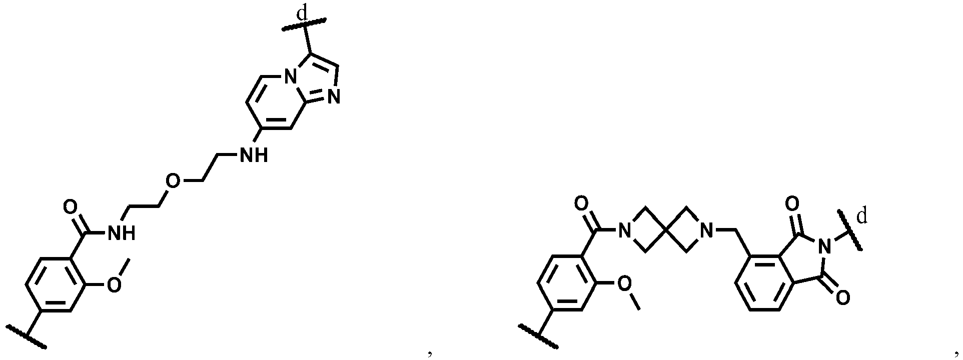 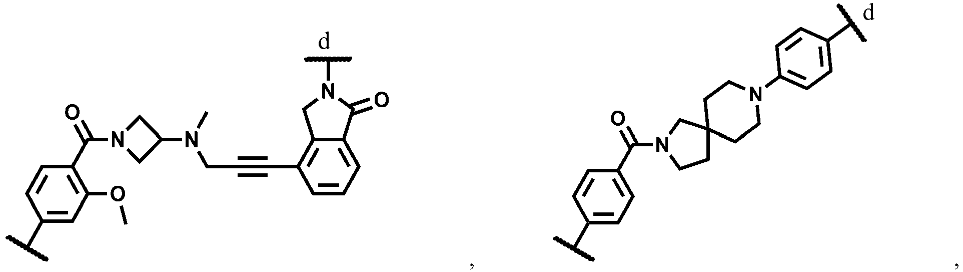 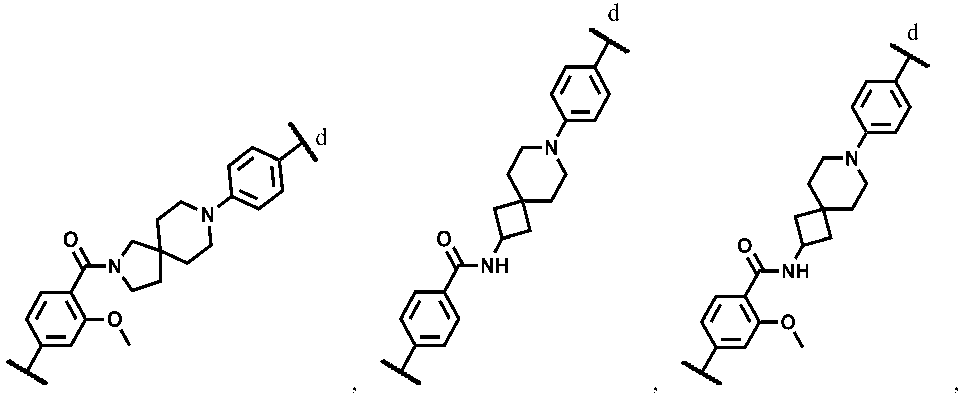 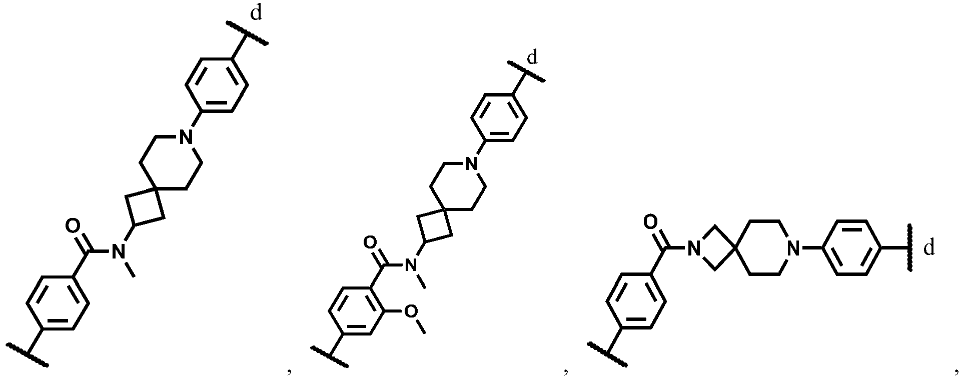 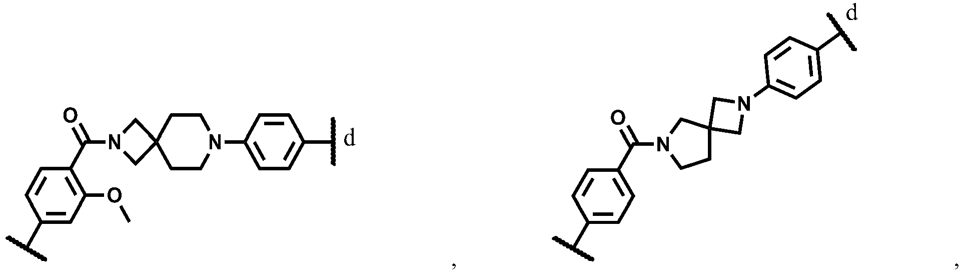 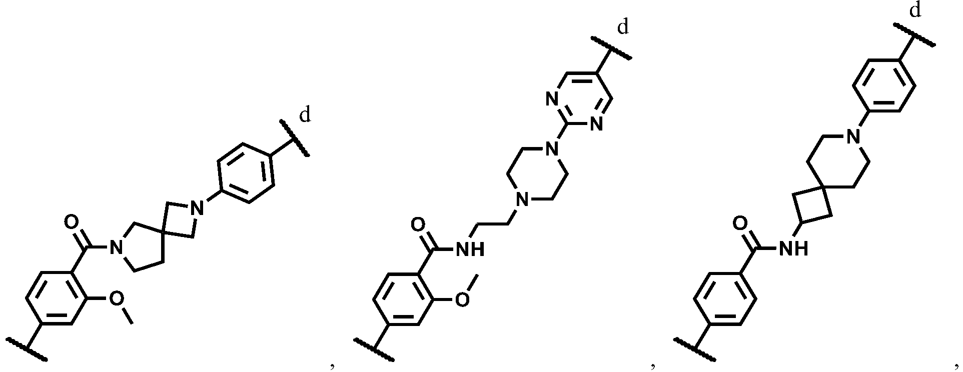 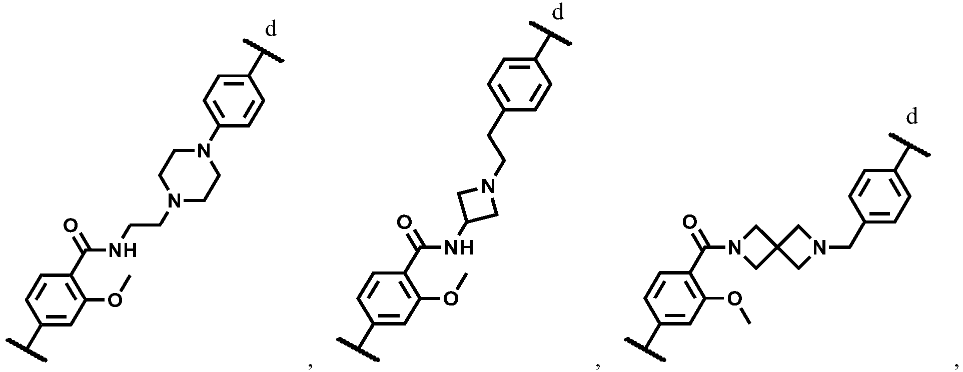 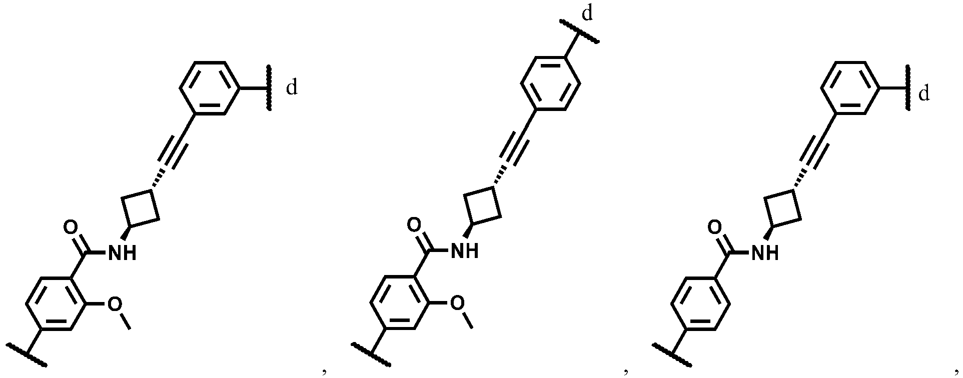 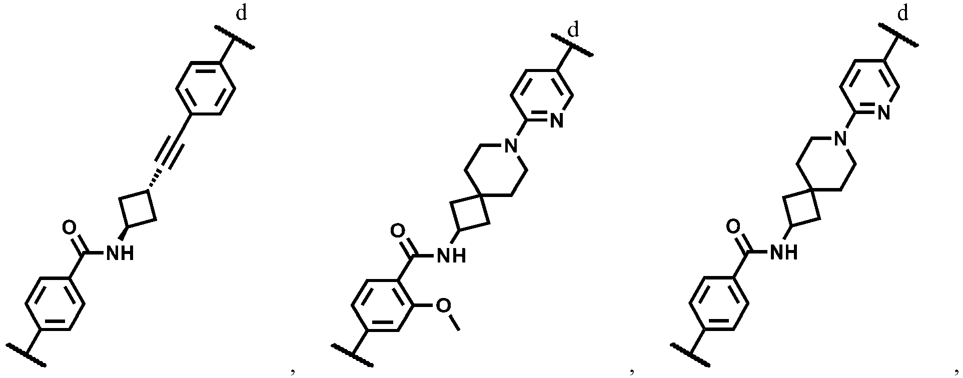 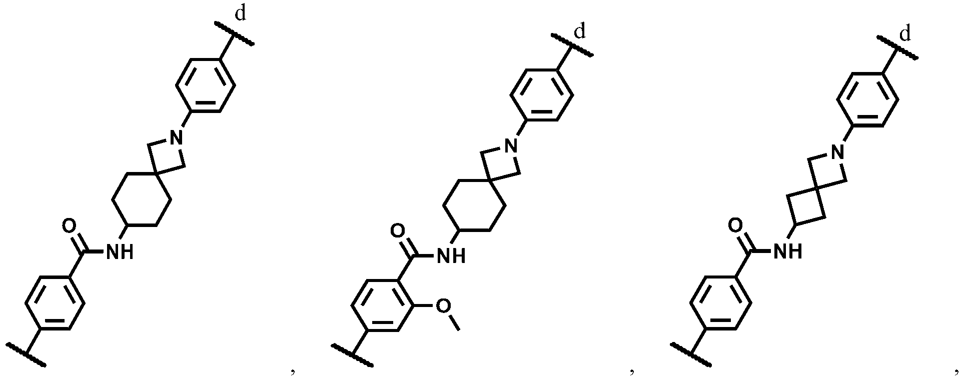 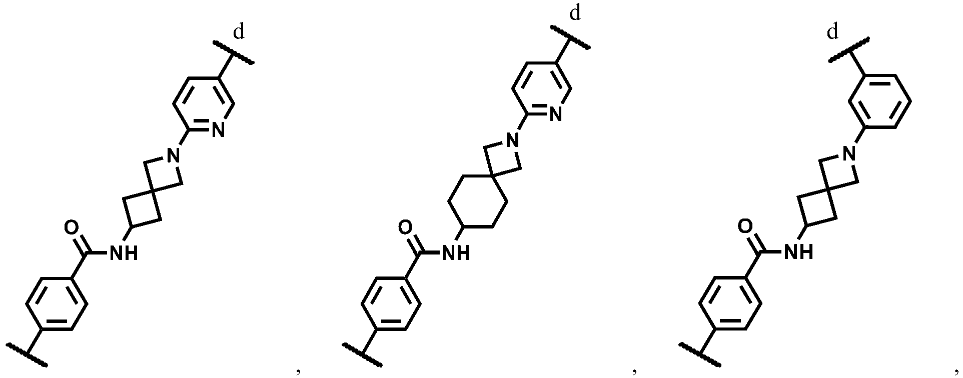 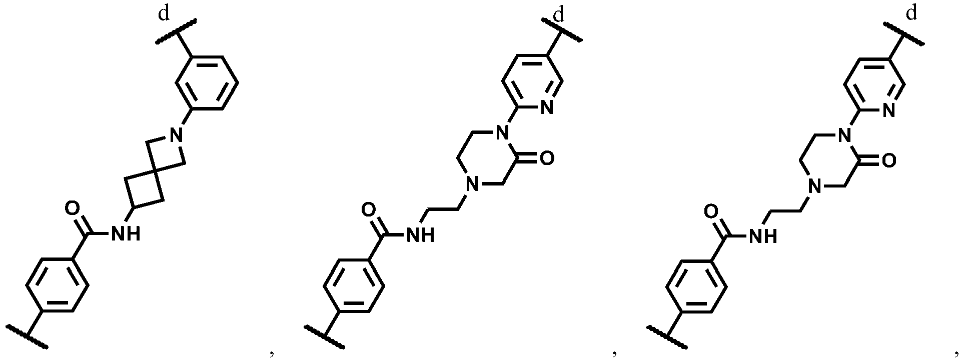 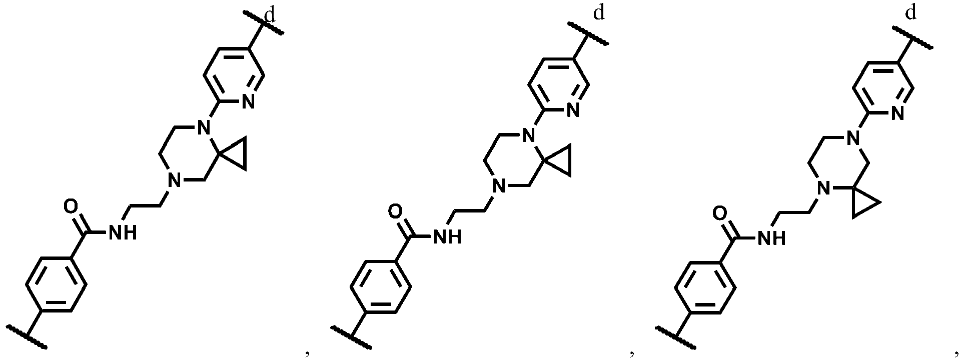 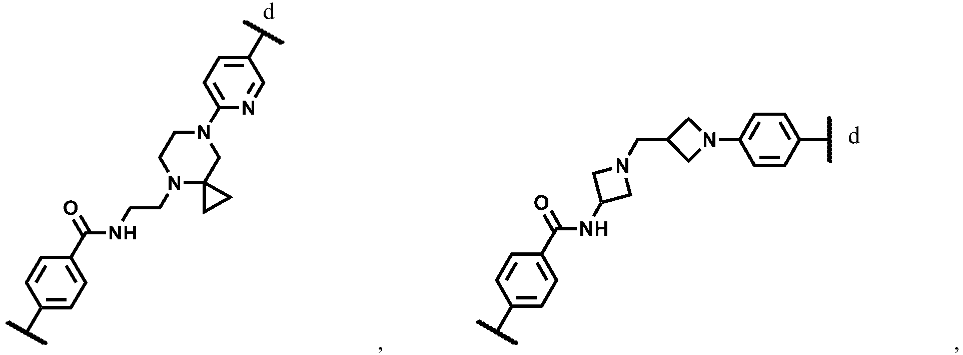 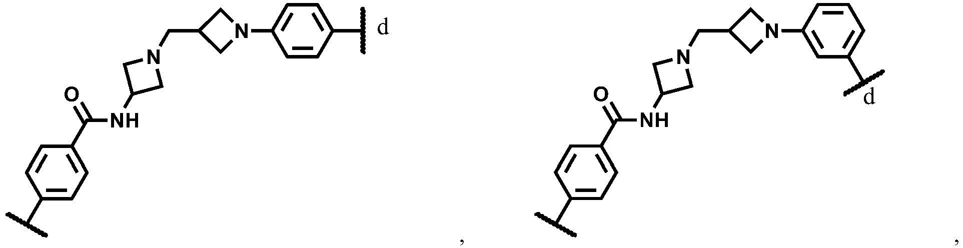 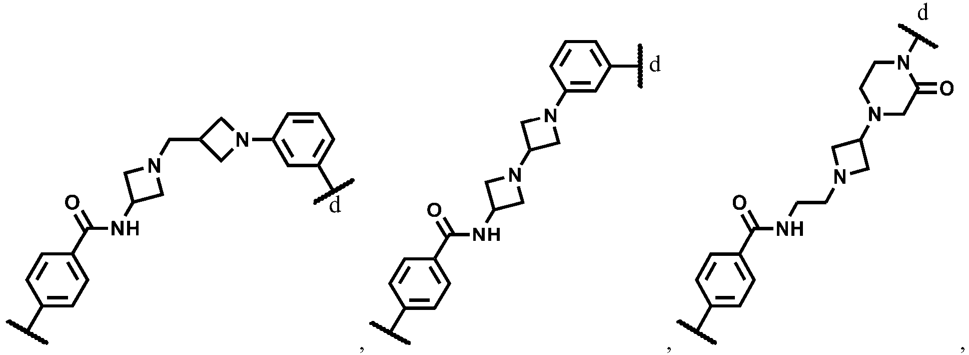 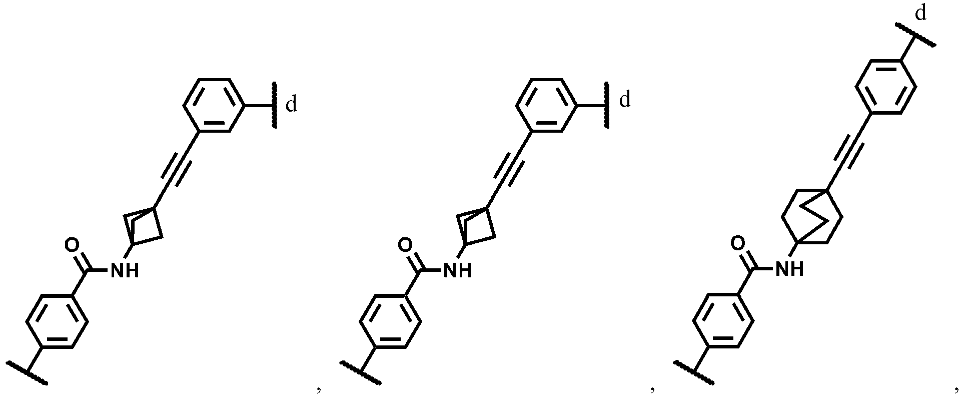 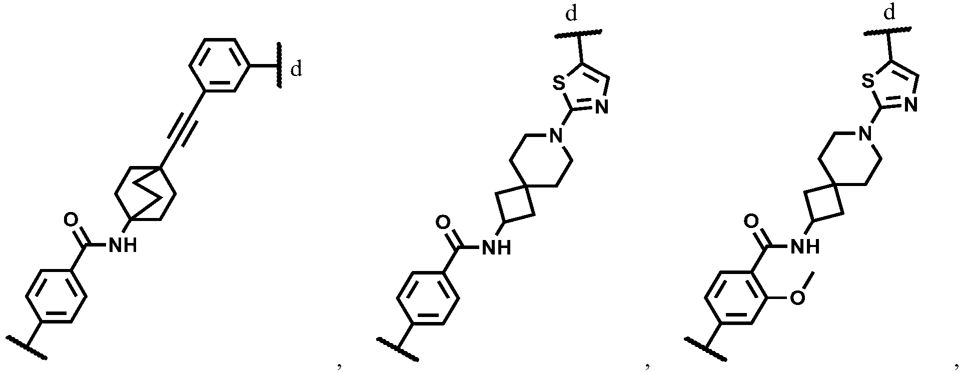 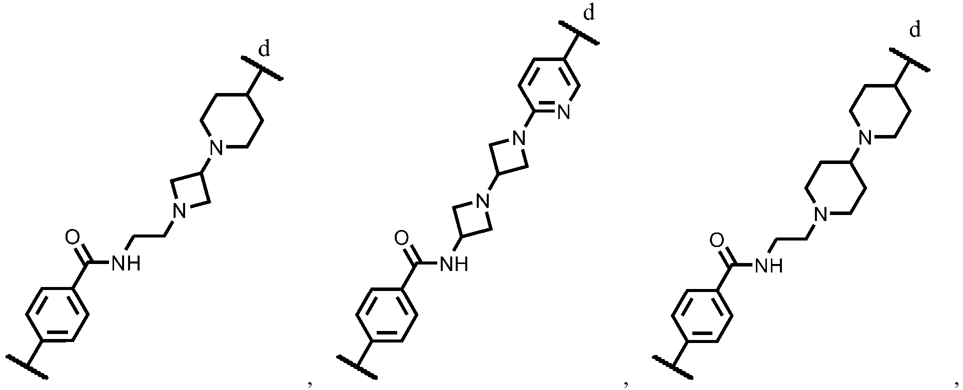 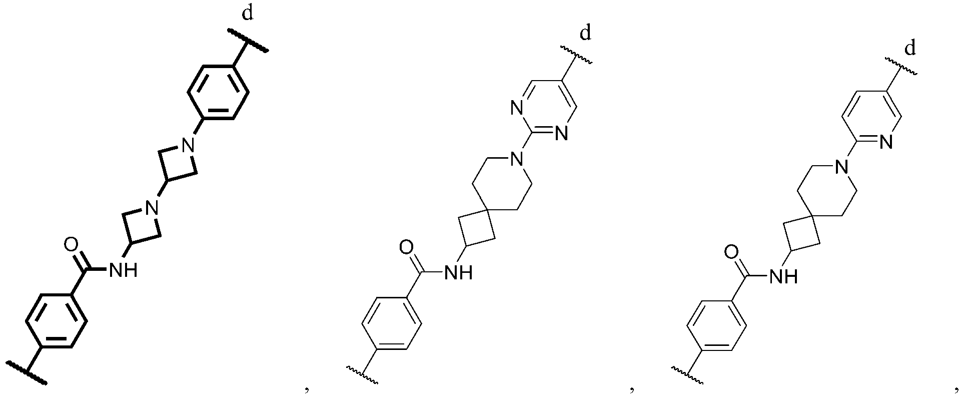 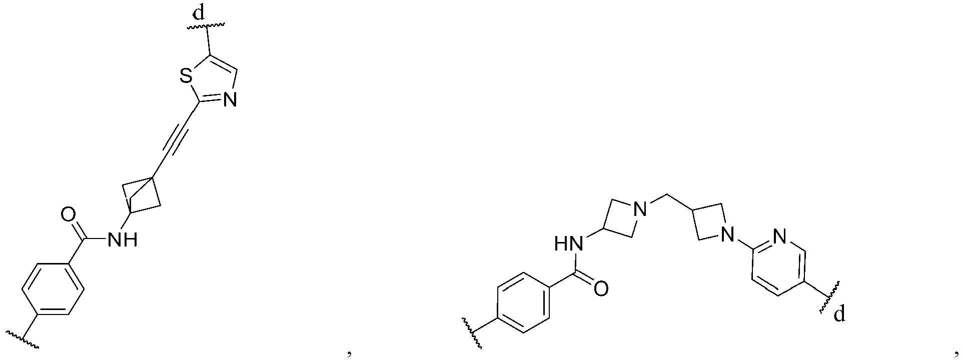 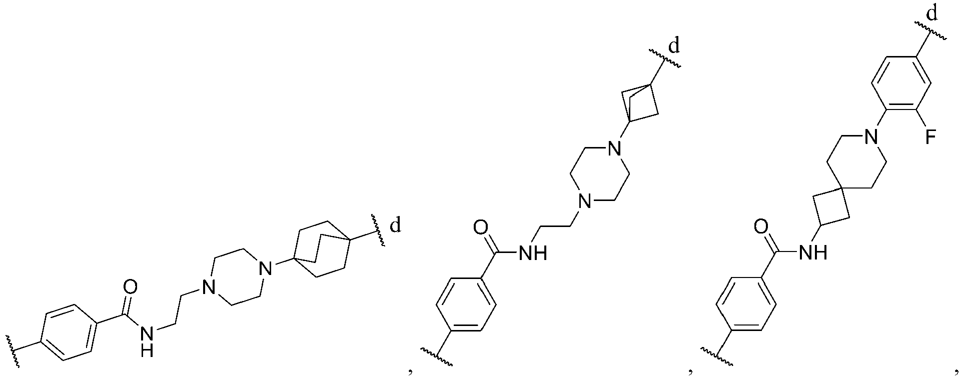 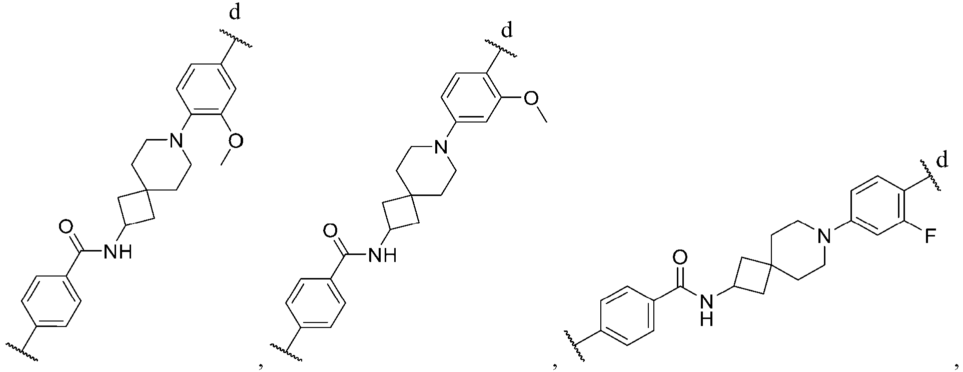 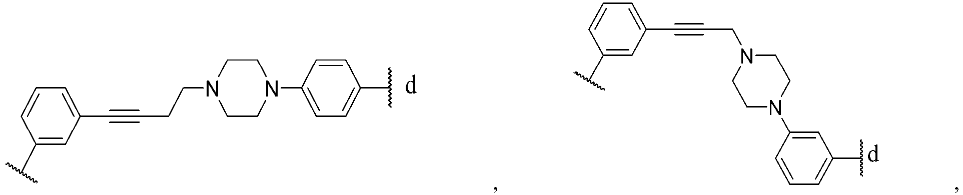 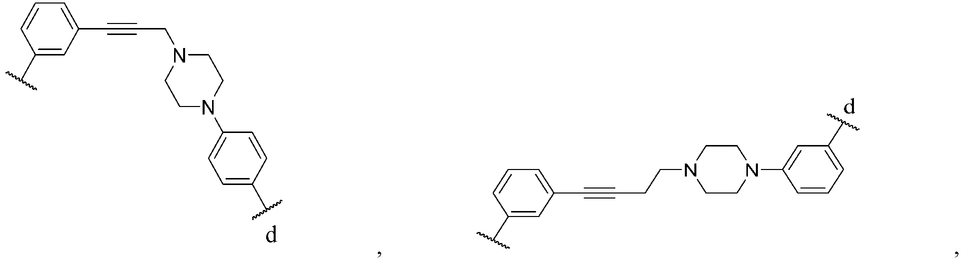 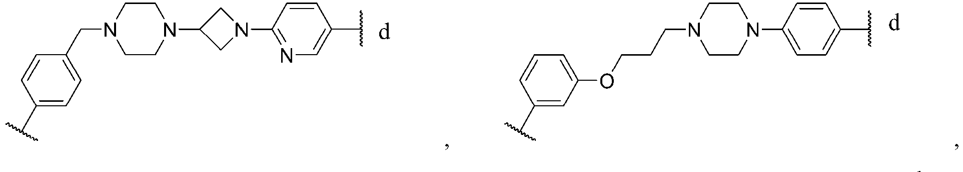 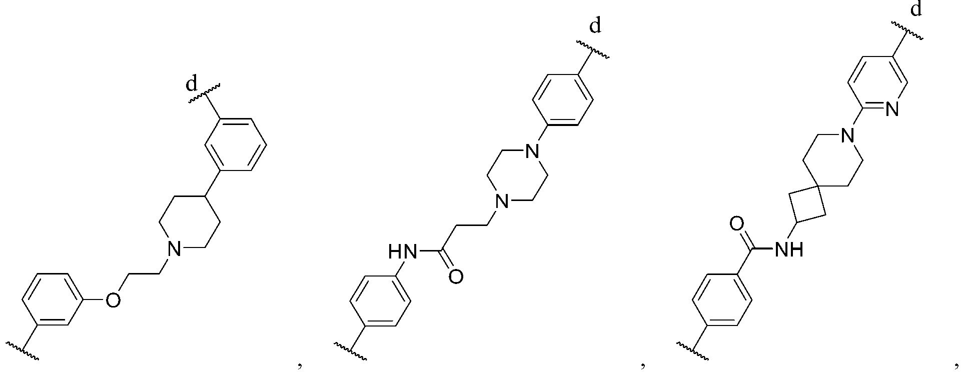 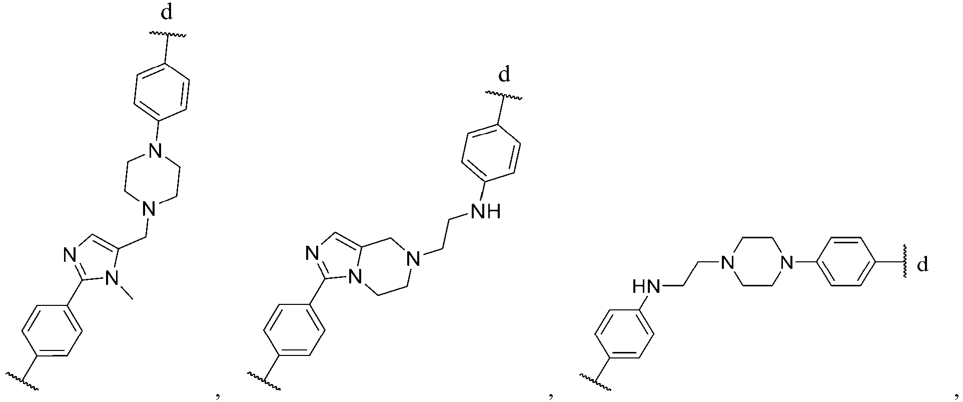 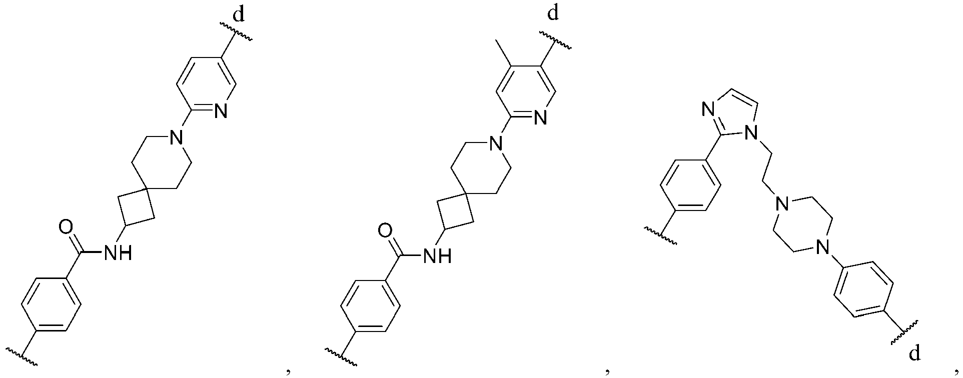 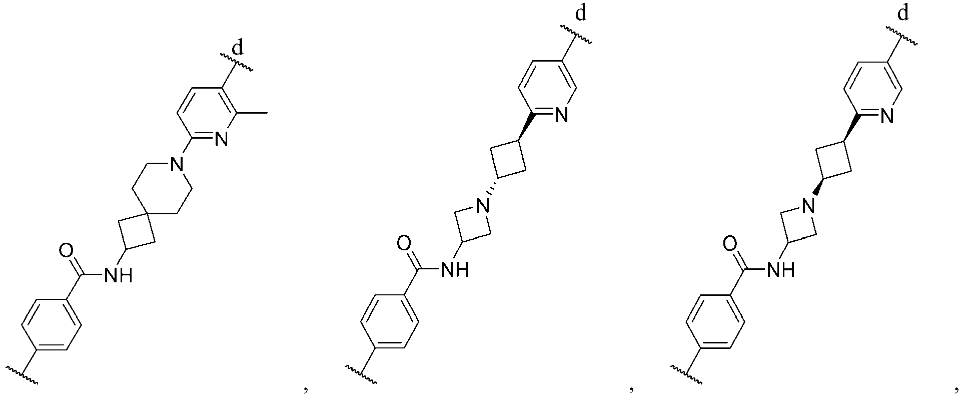 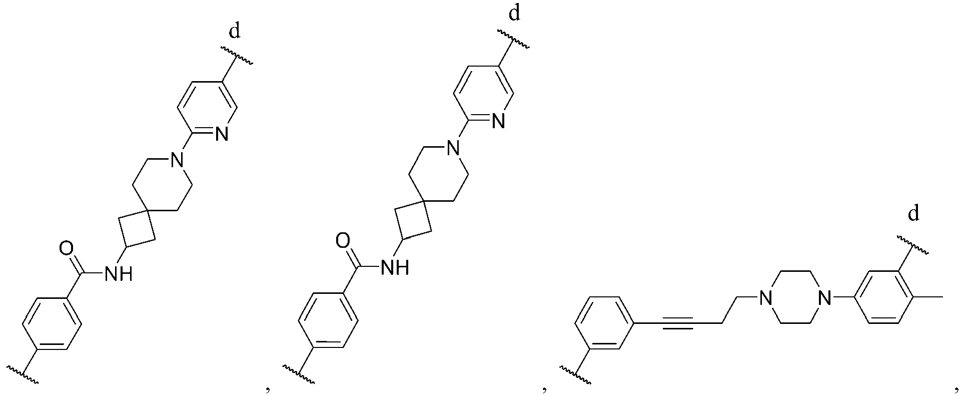 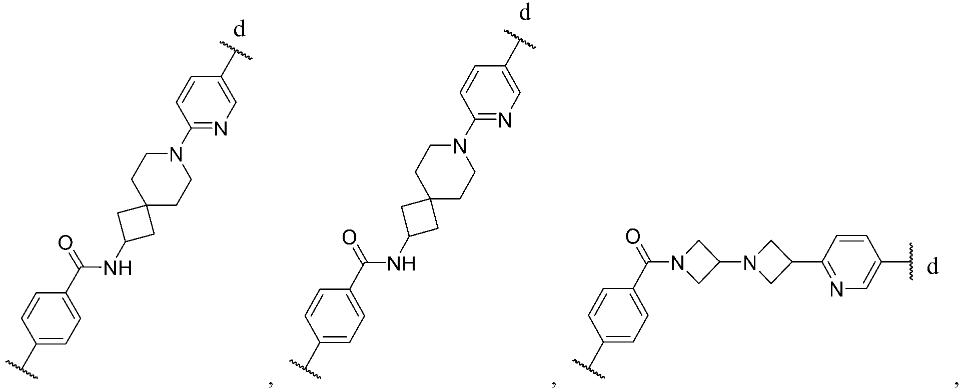 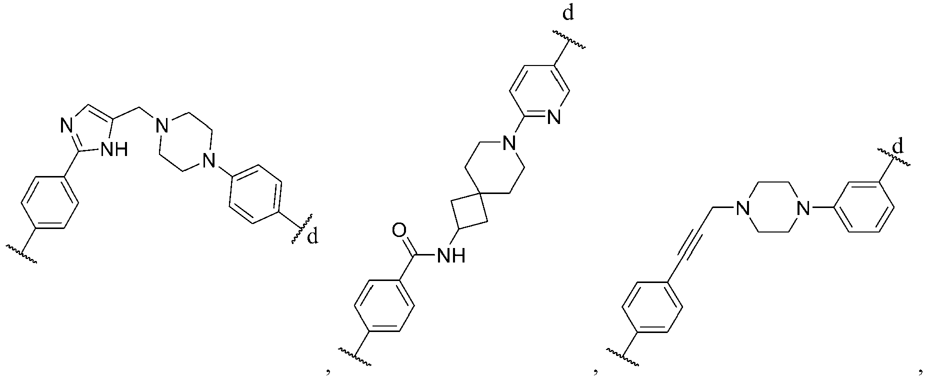 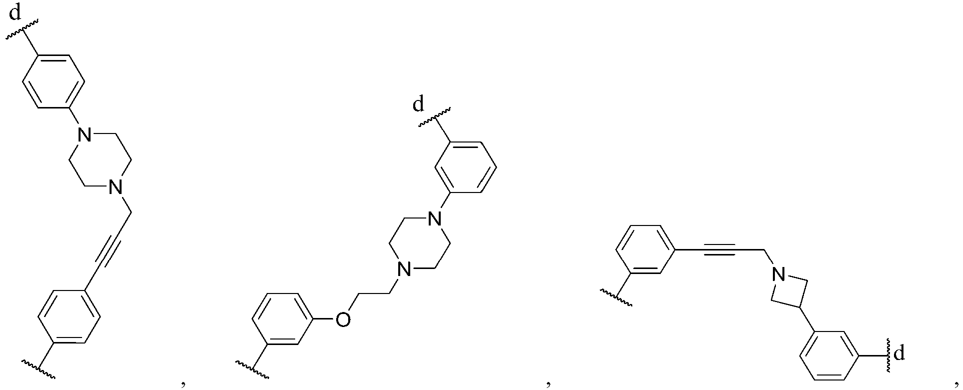 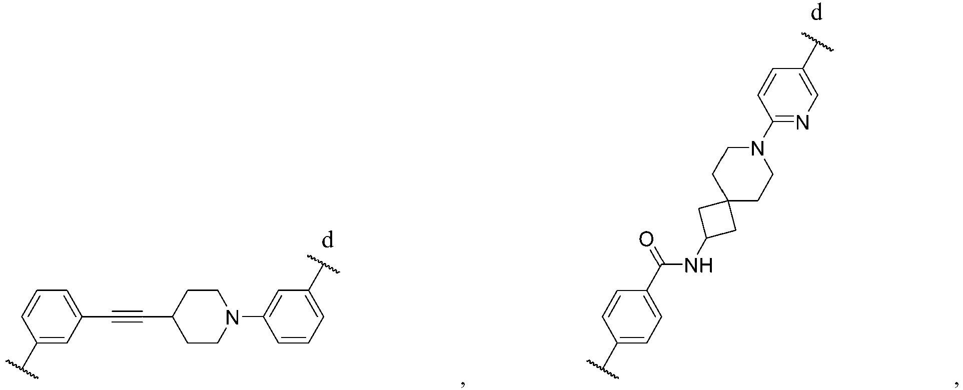 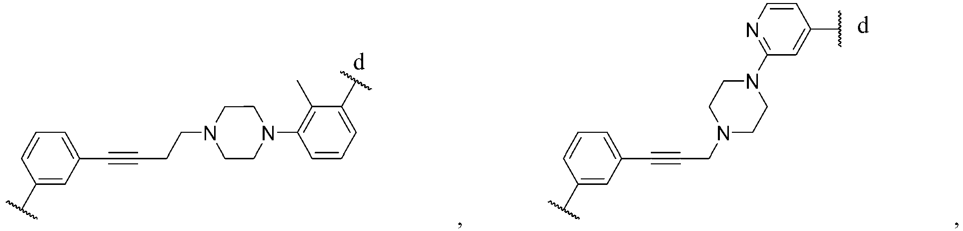 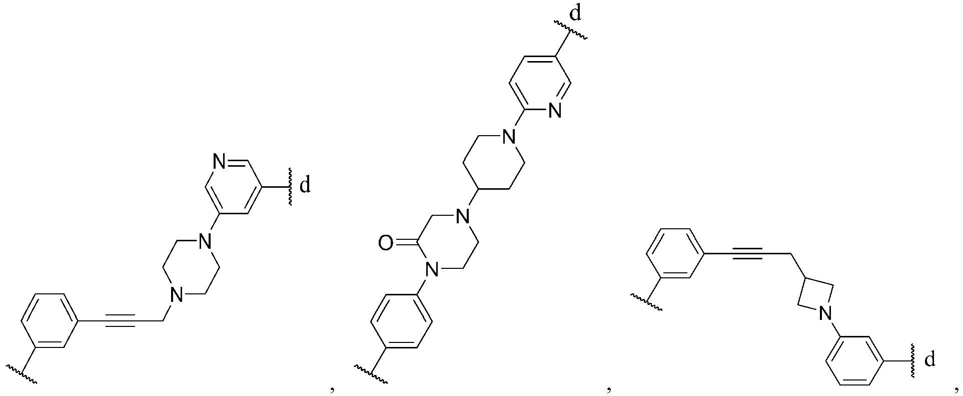 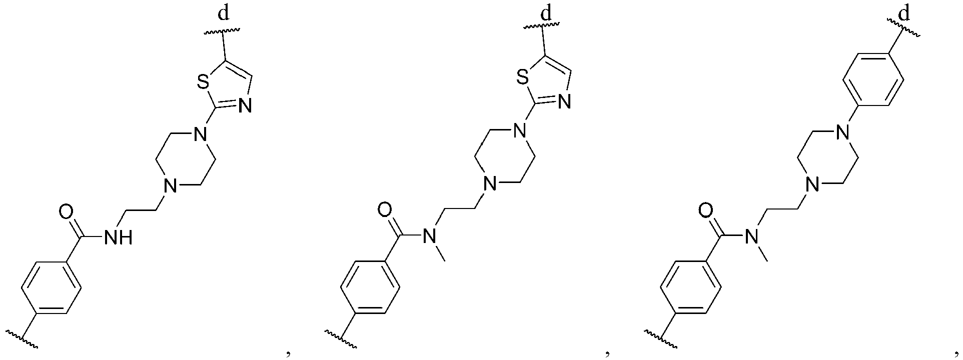 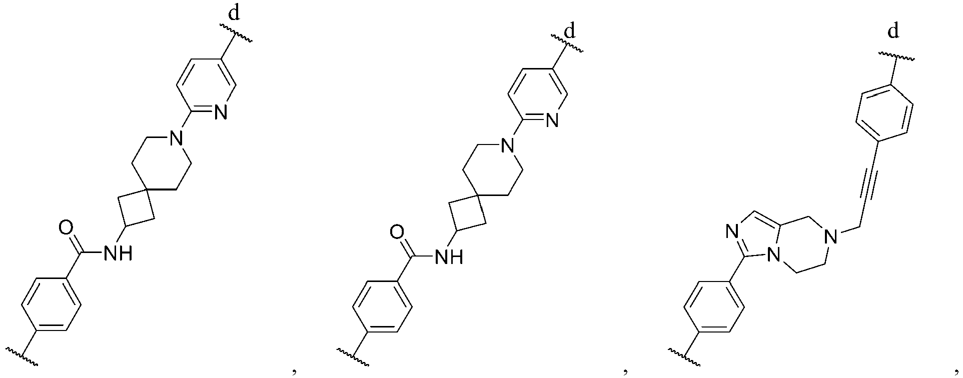 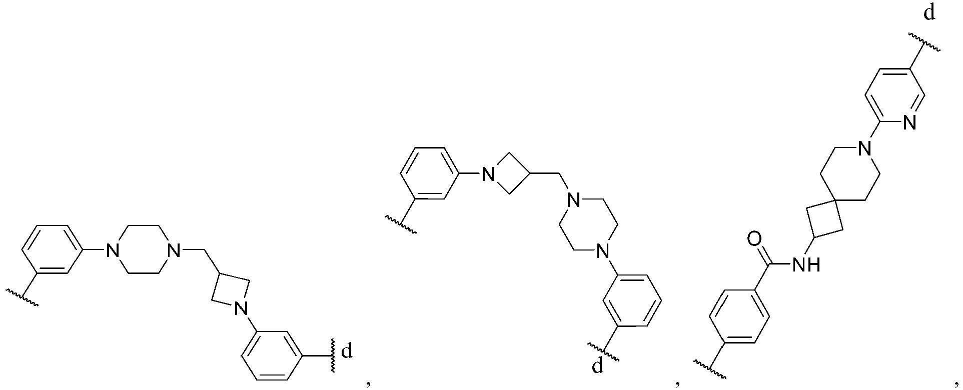 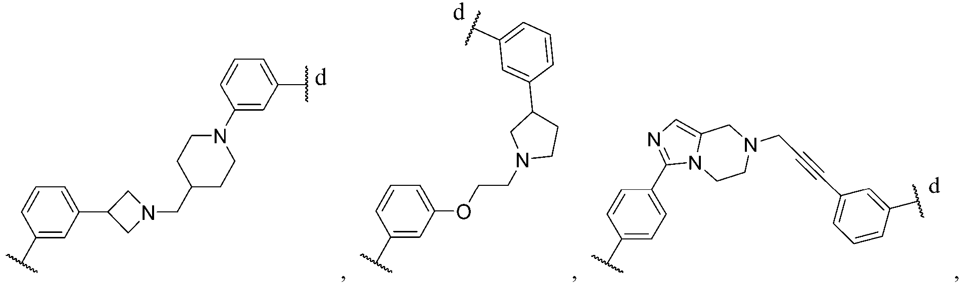 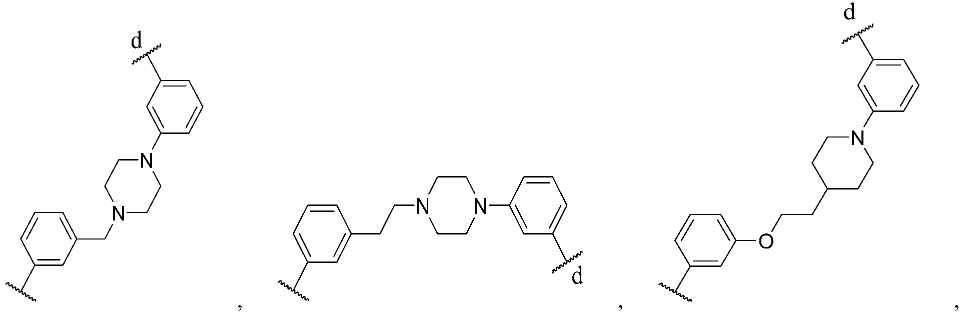 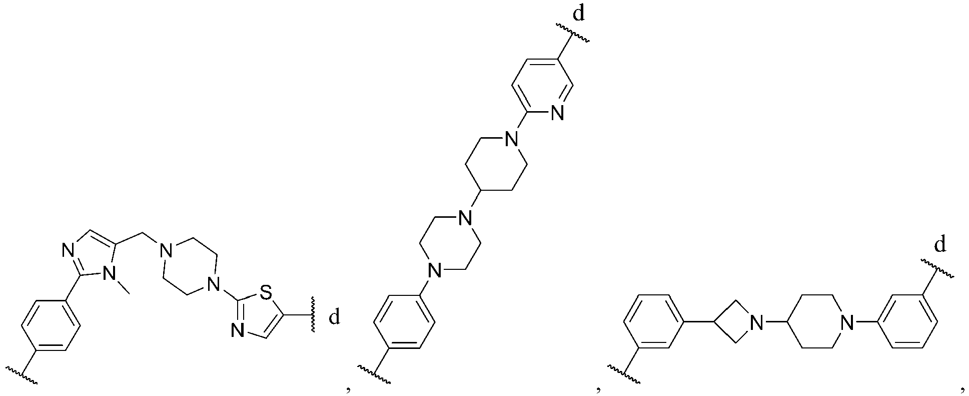 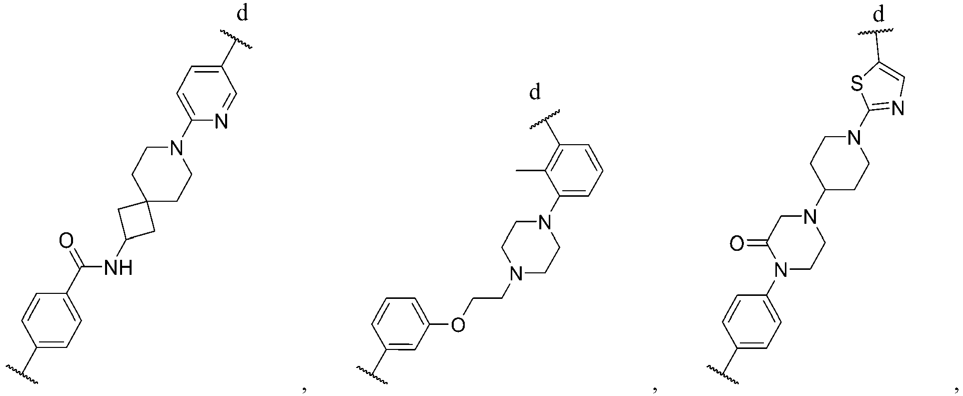 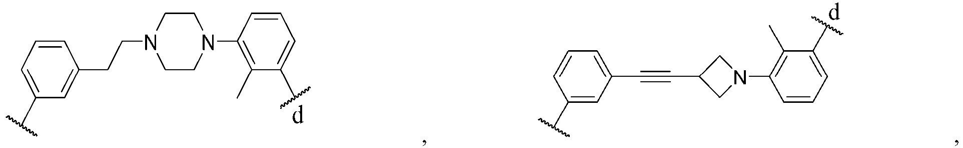 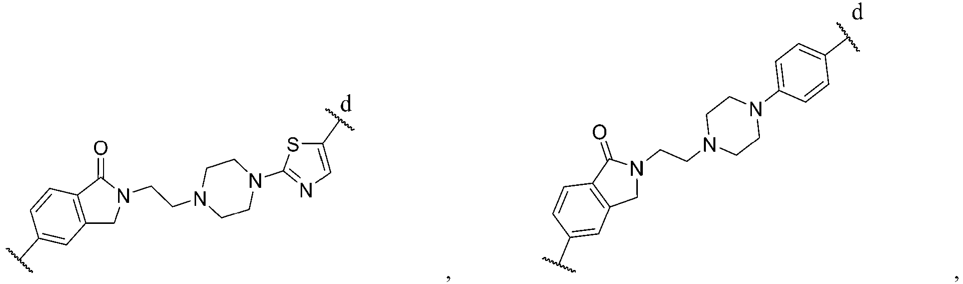 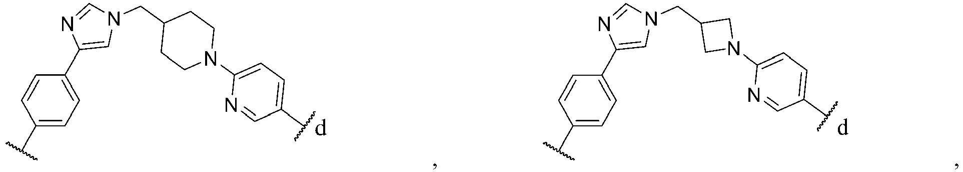 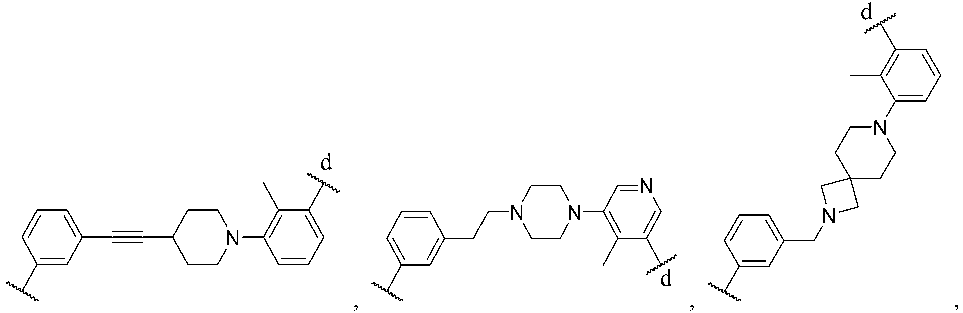 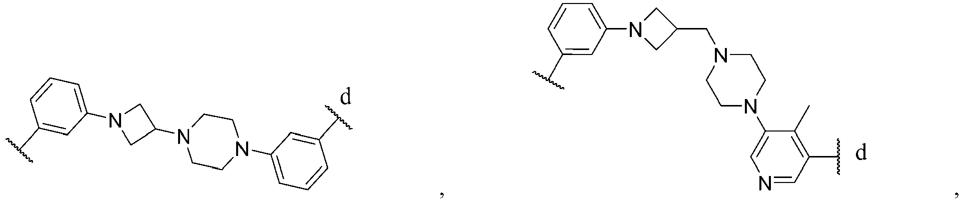 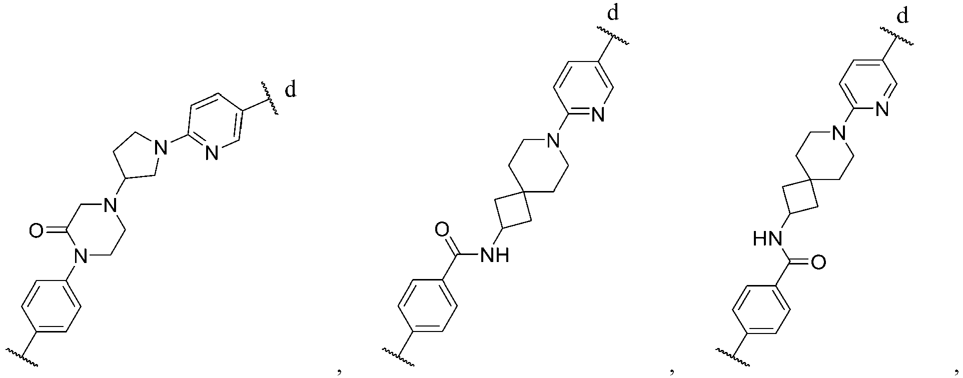 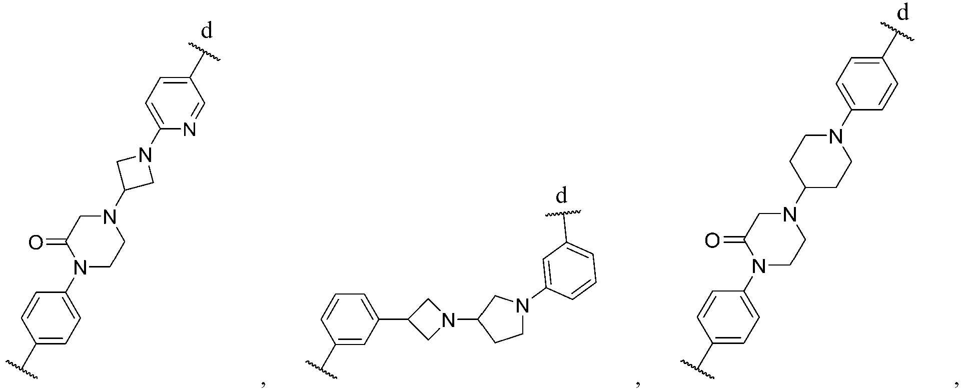 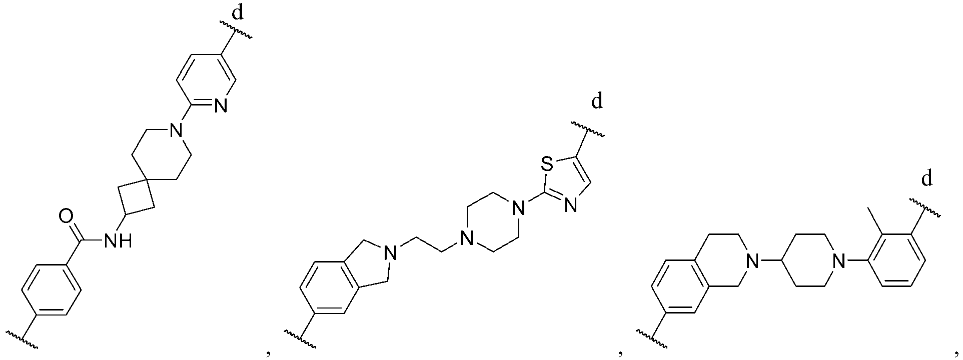 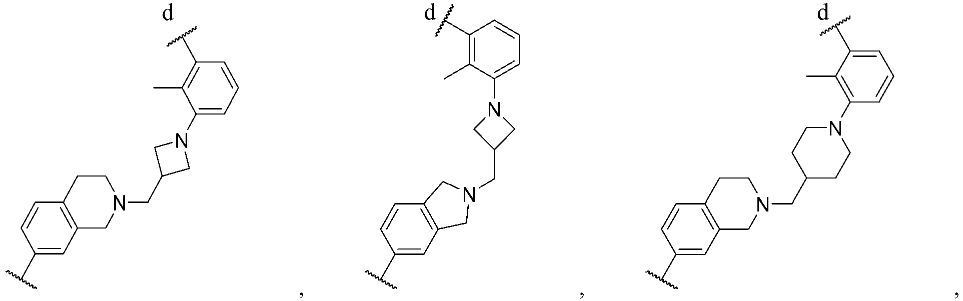 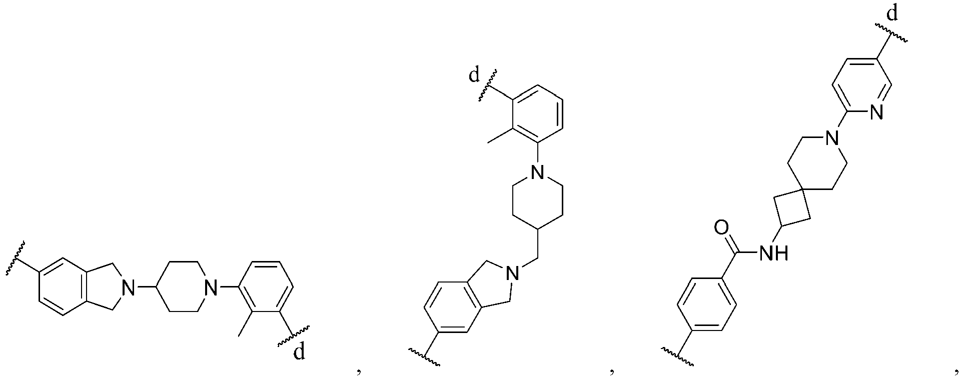 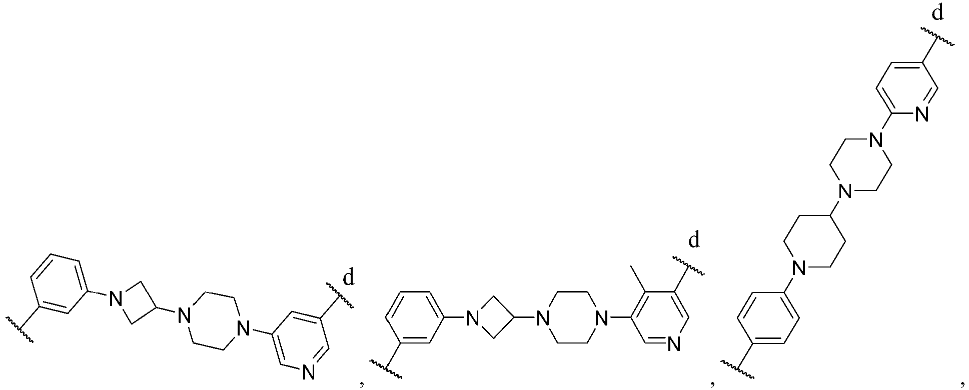 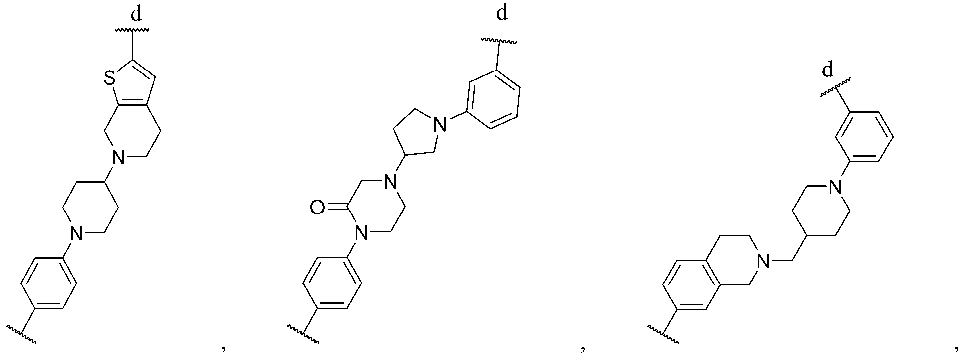 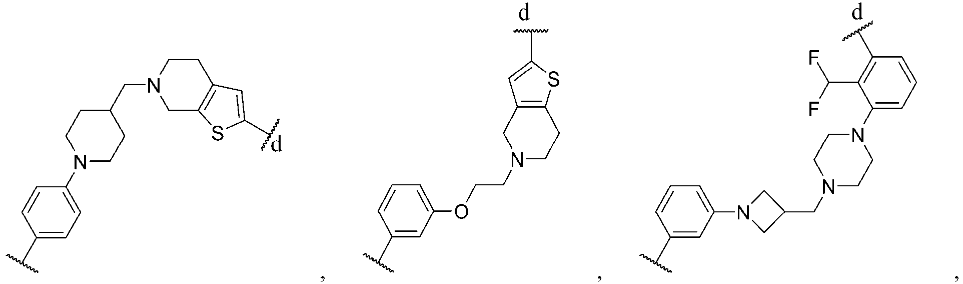 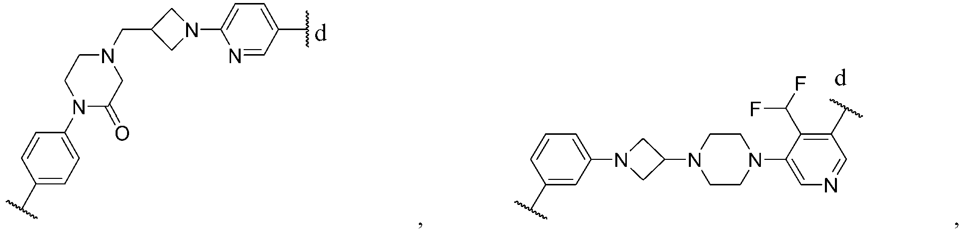 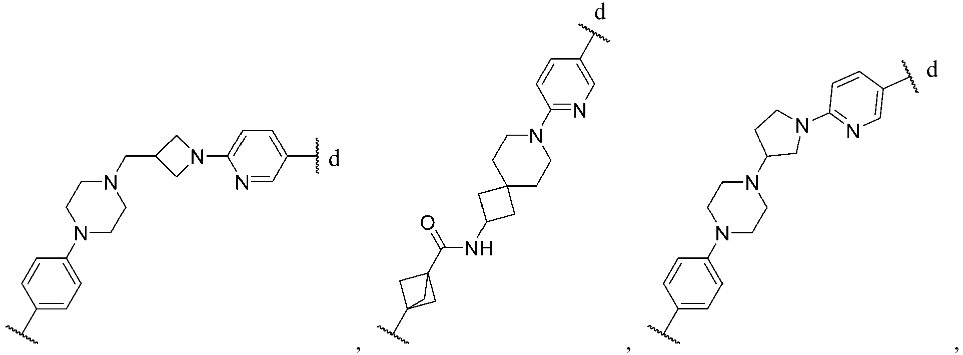 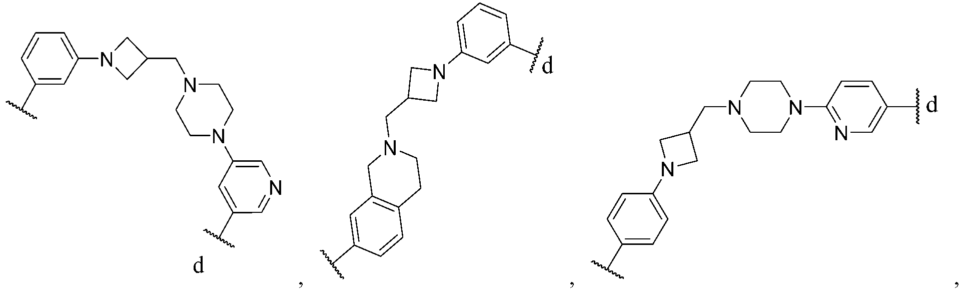 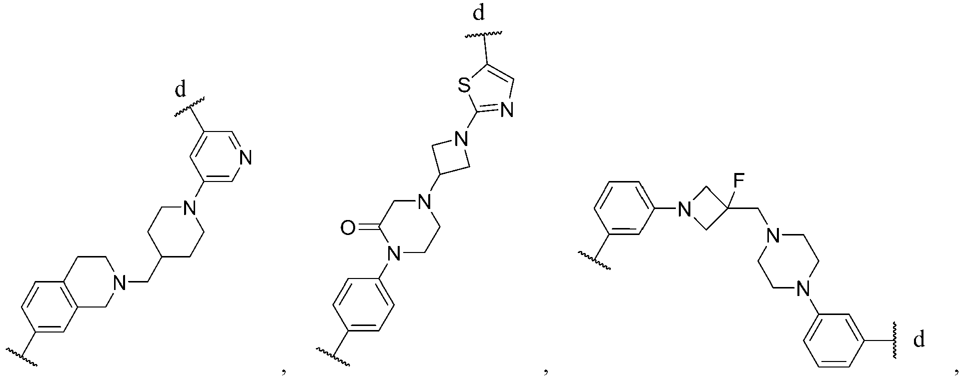 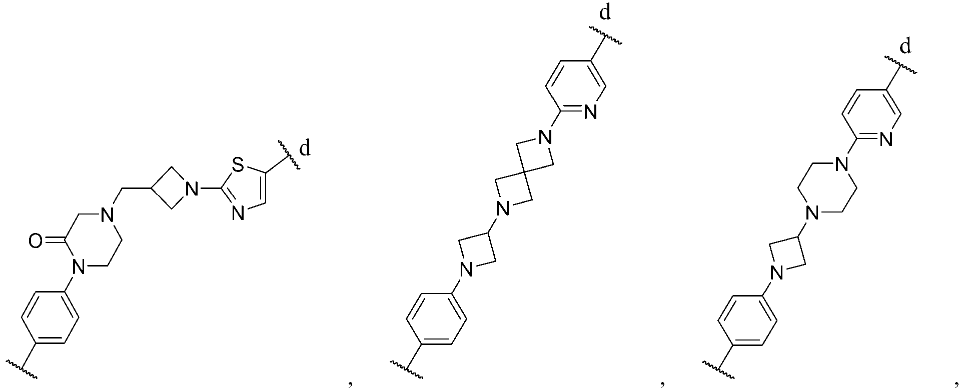 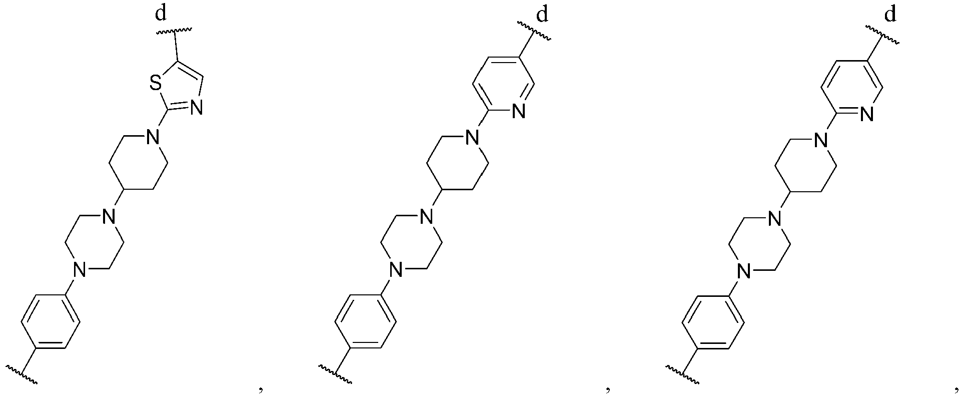 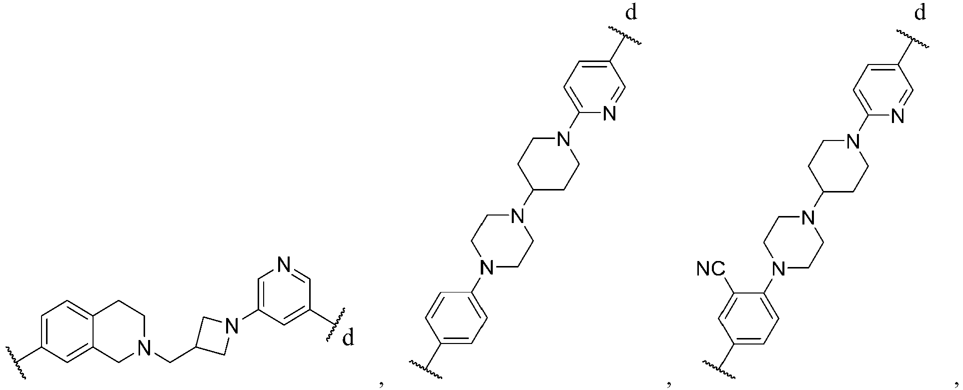 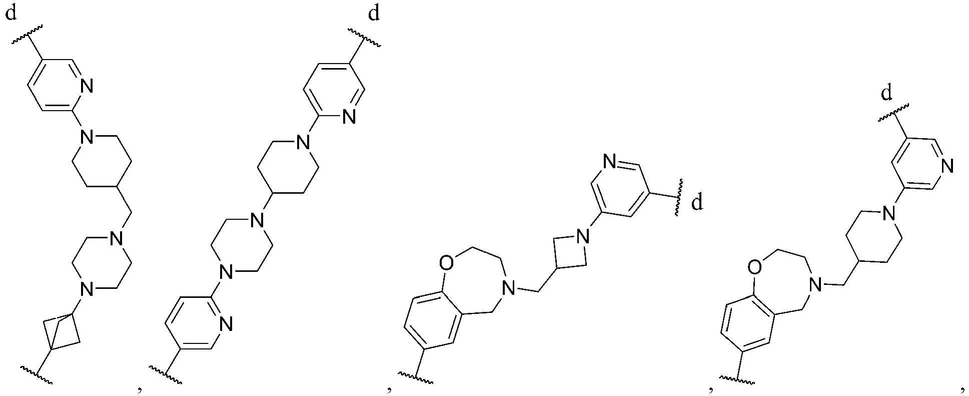 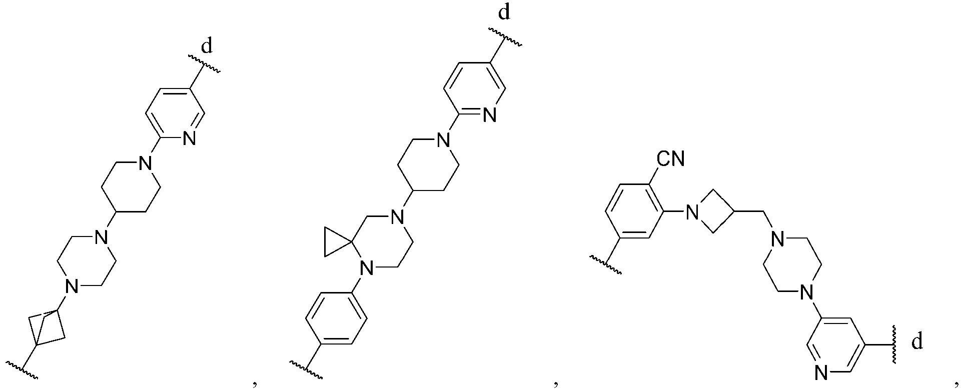 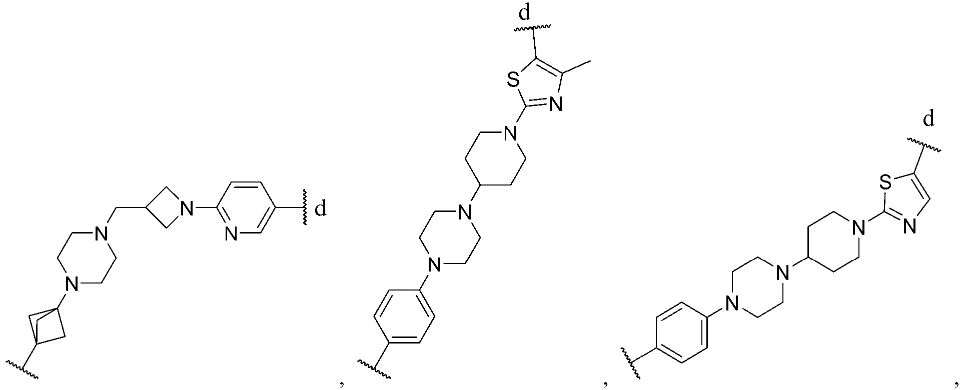 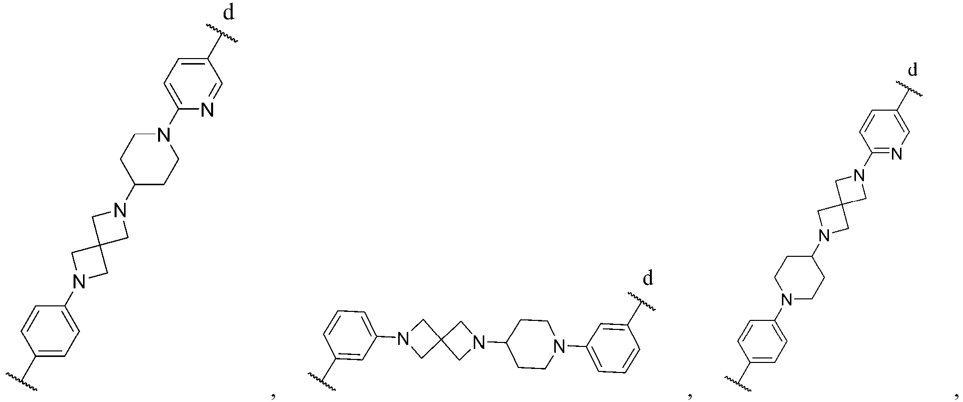 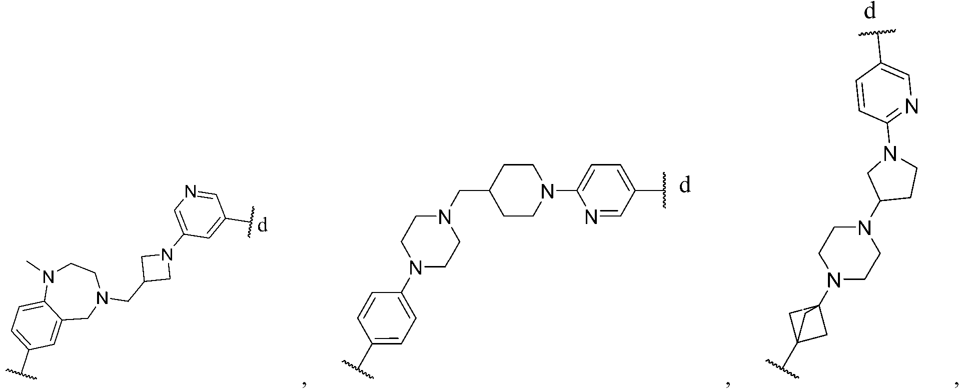 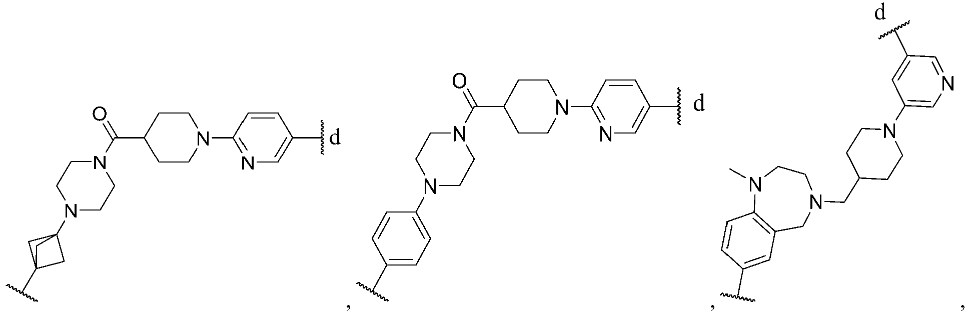 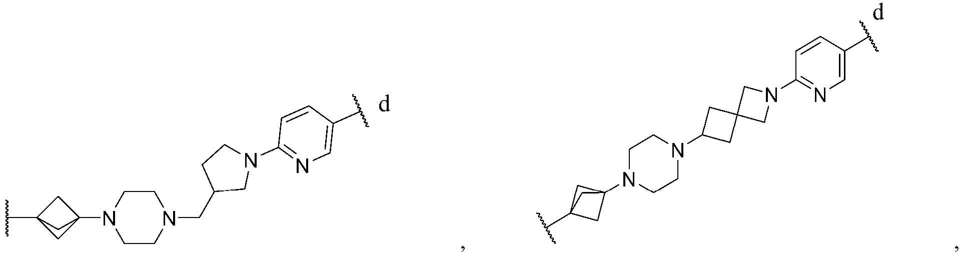 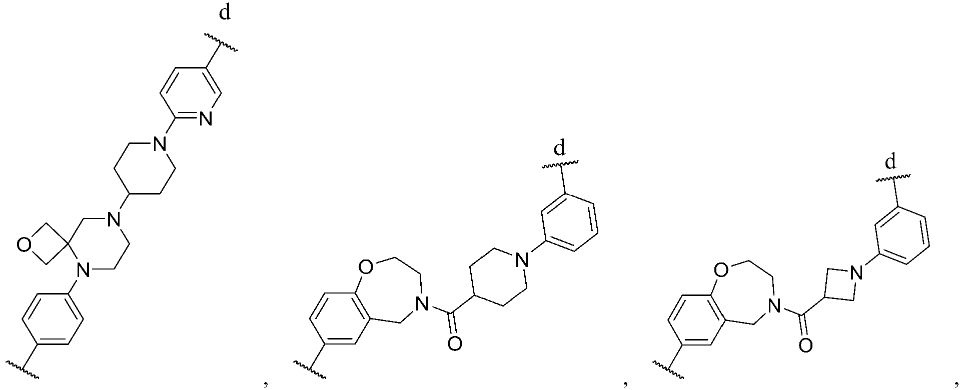 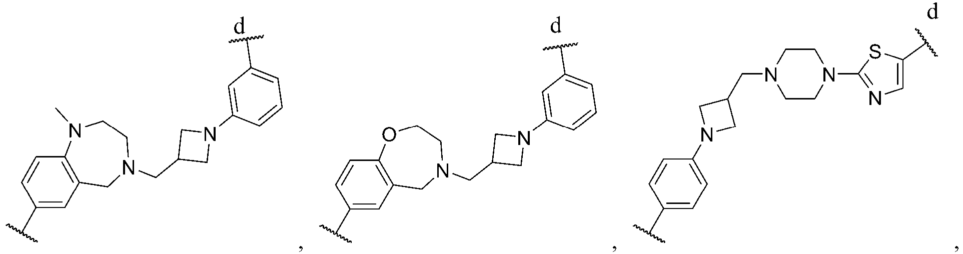 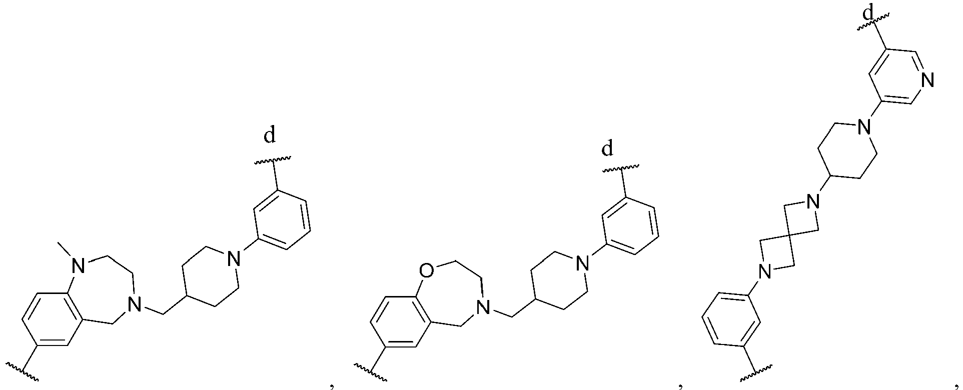 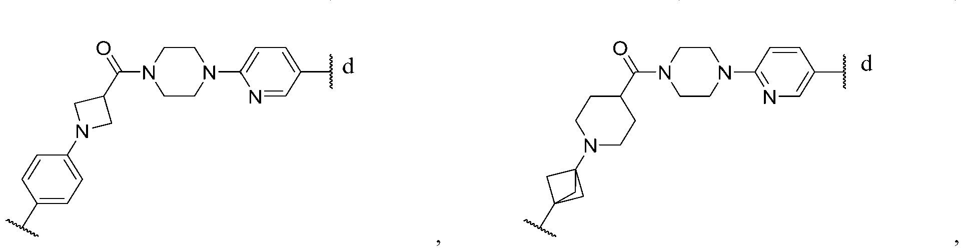 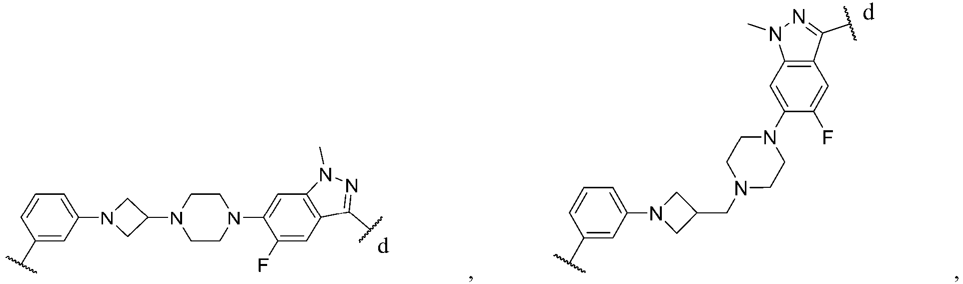 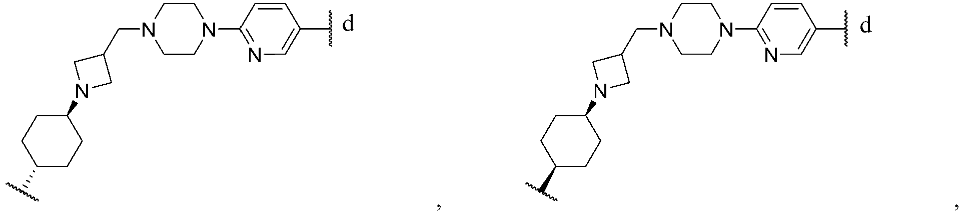 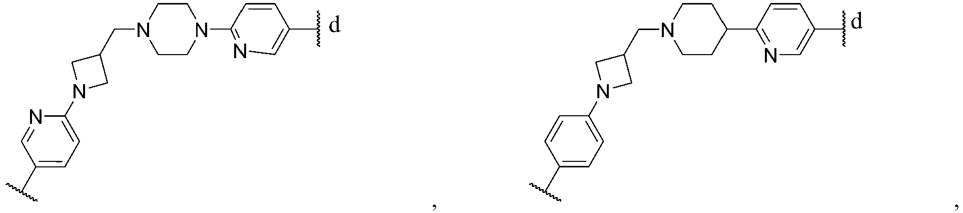 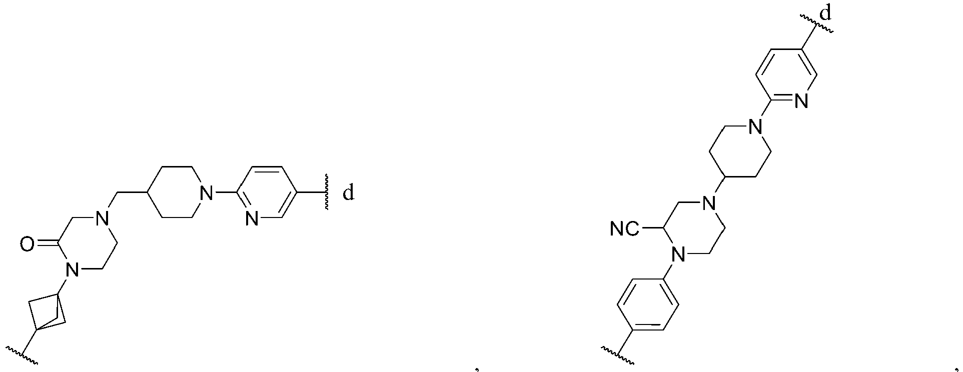 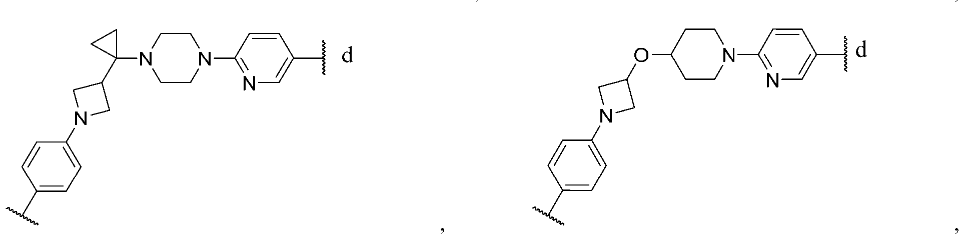 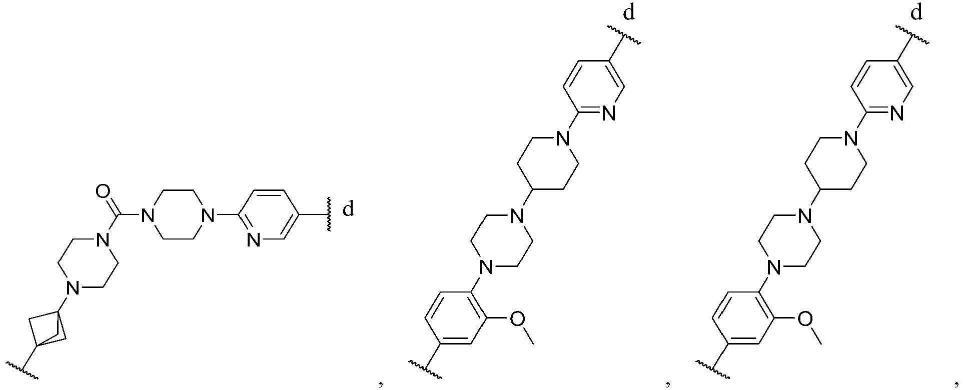 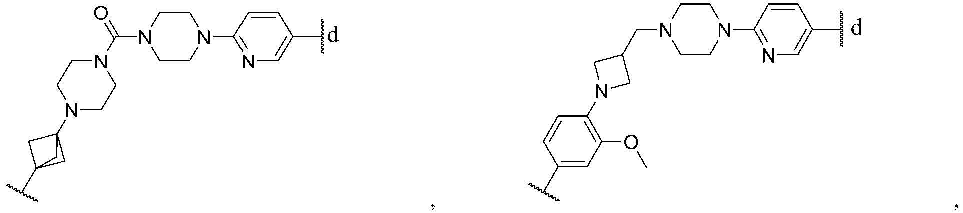 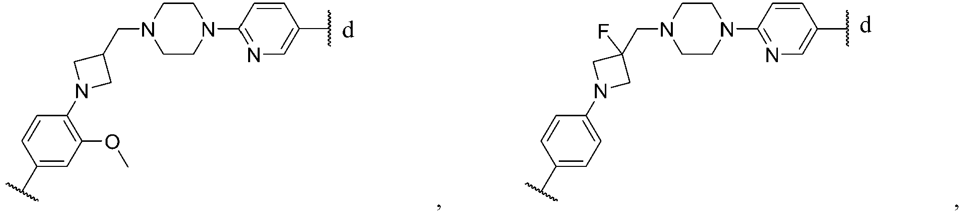 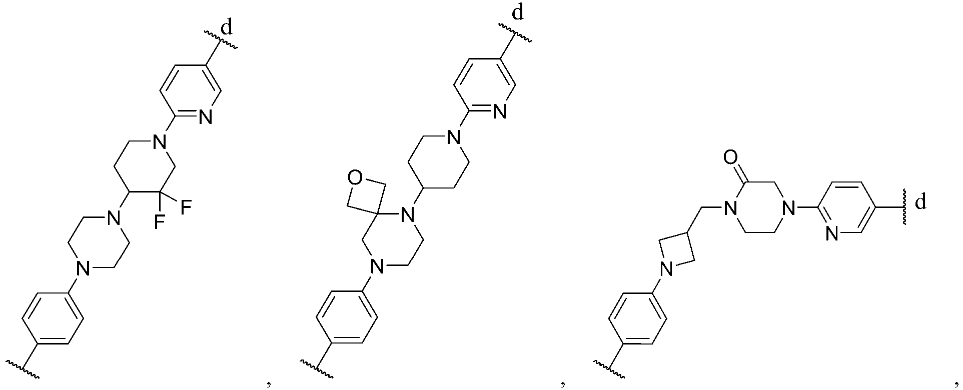 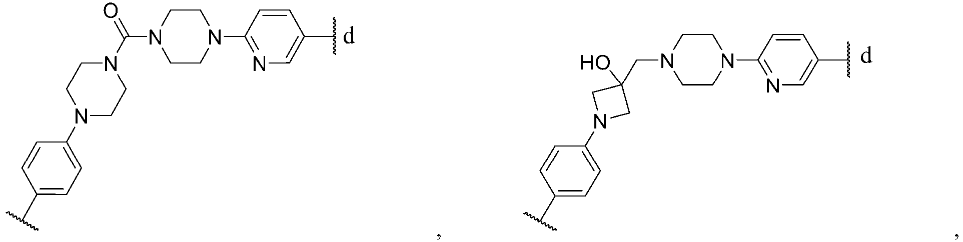 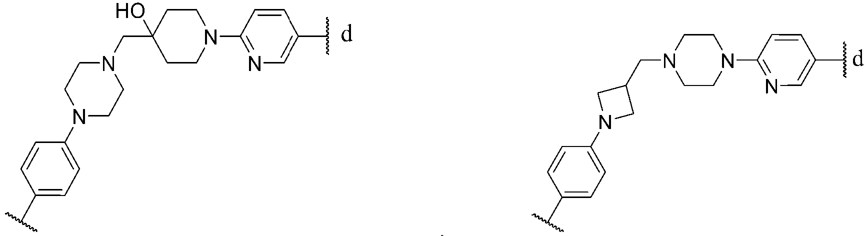 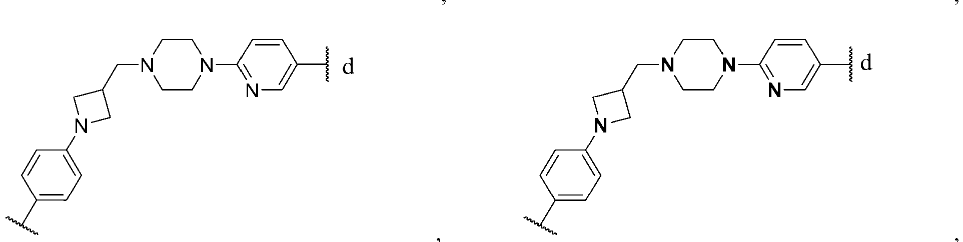 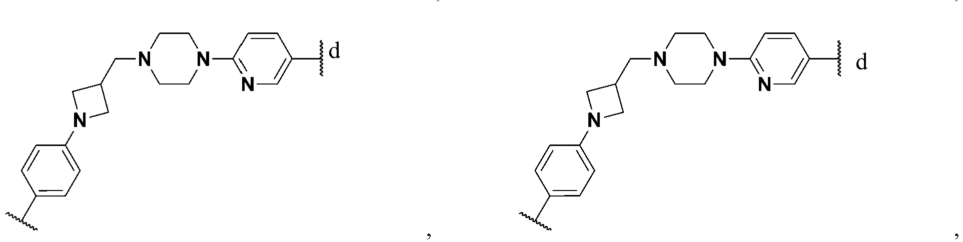 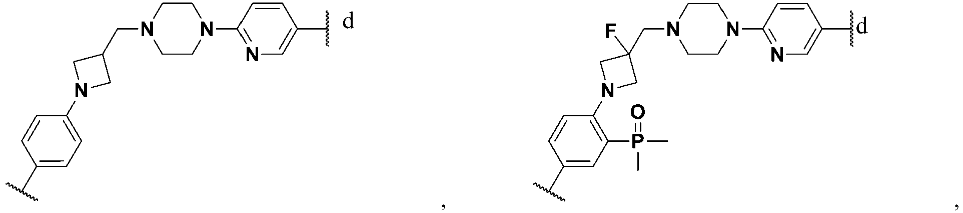 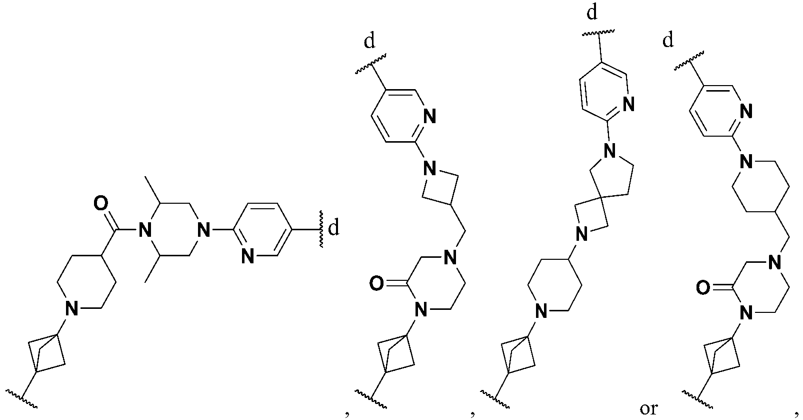 wherein side d is connected to 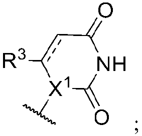 preferably, in Scheme ii, -A-L-B- is any one of the above groups except 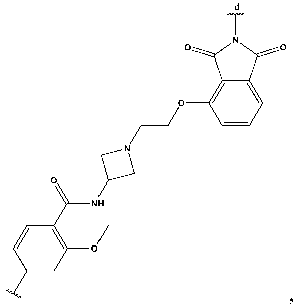 and in Scheme i, -A-L-B- is

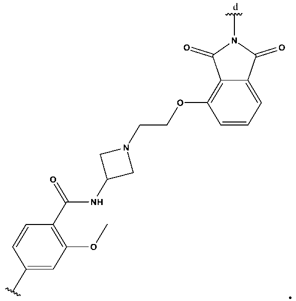

In one embodiment of the present invention,

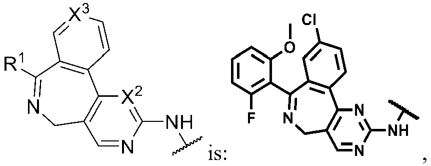

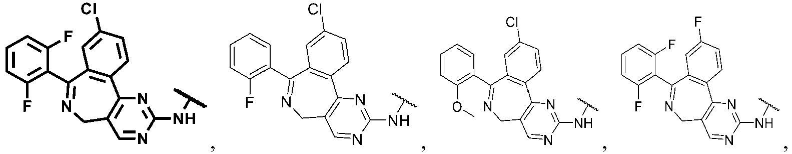

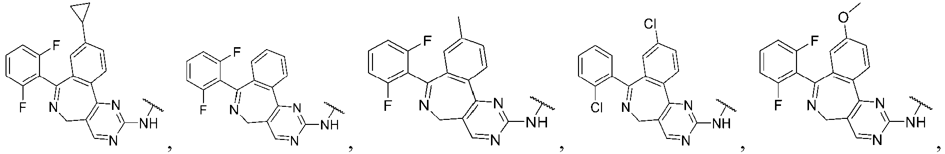

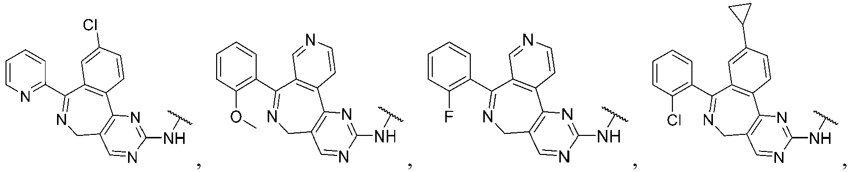

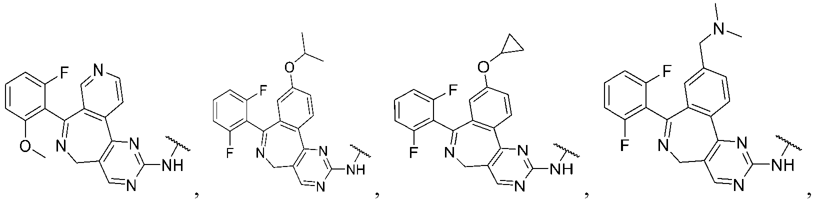

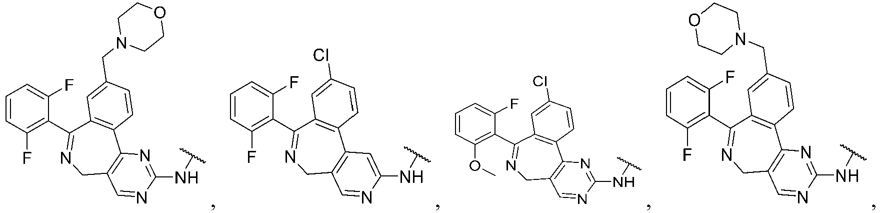

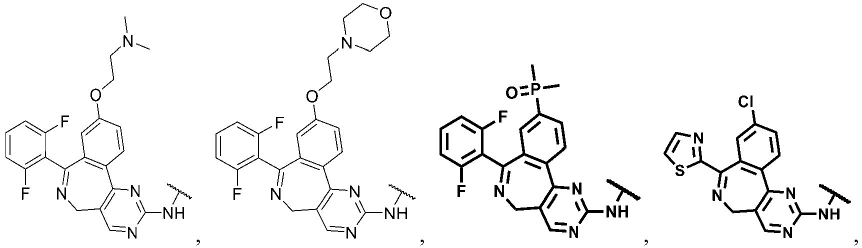

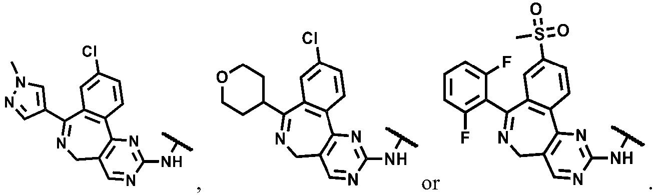

In one embodiment of the present invention,

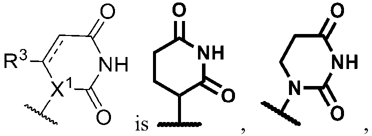

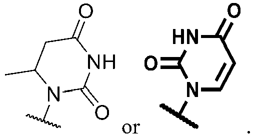

In one embodiment of the present invention, a compound represented by formula (I) is any one of the following compounds:

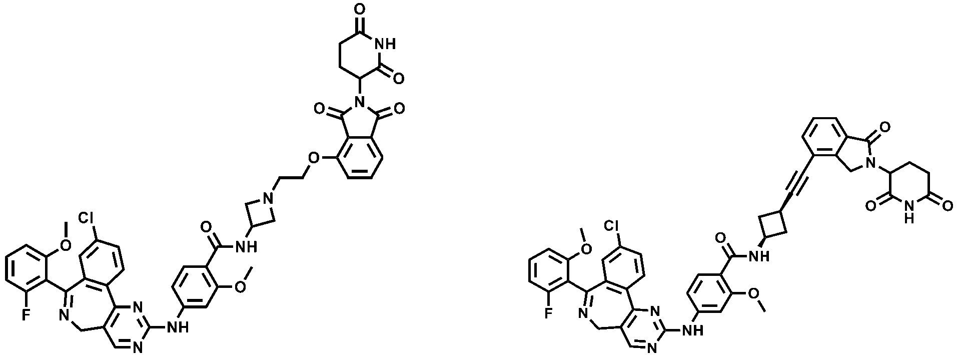

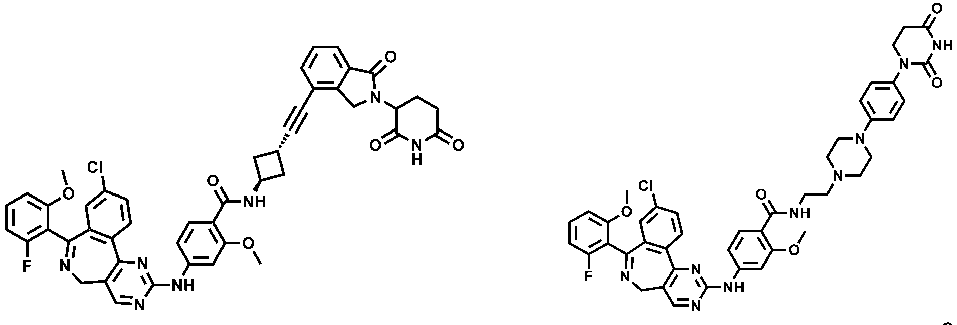

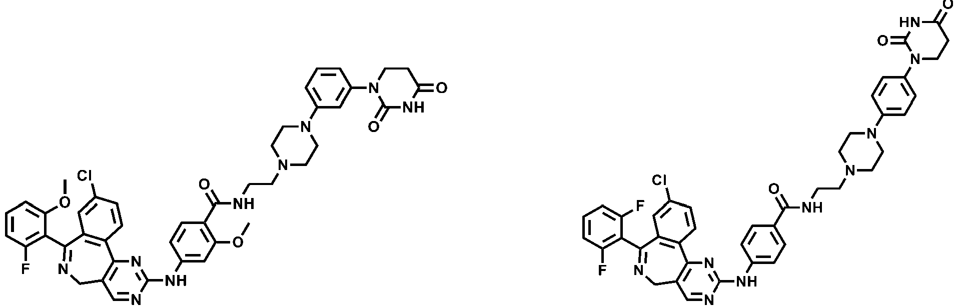

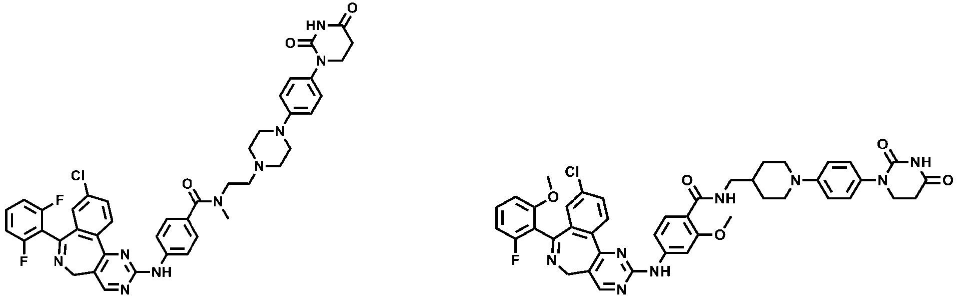

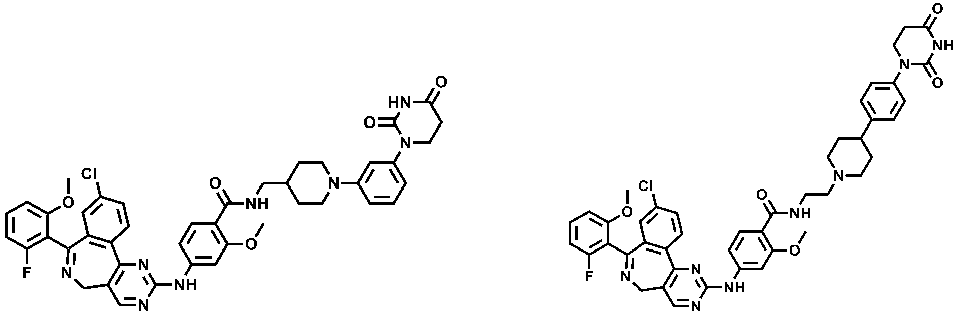

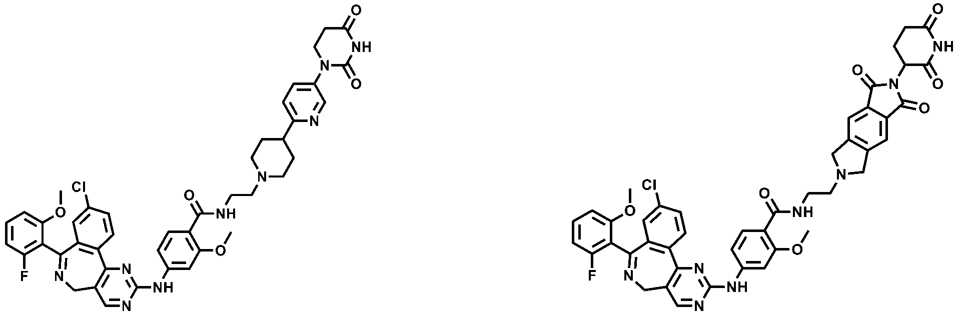

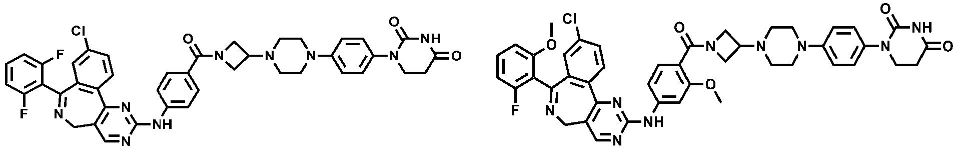

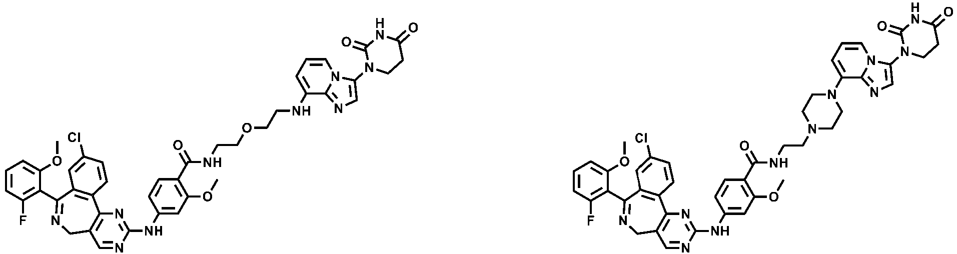

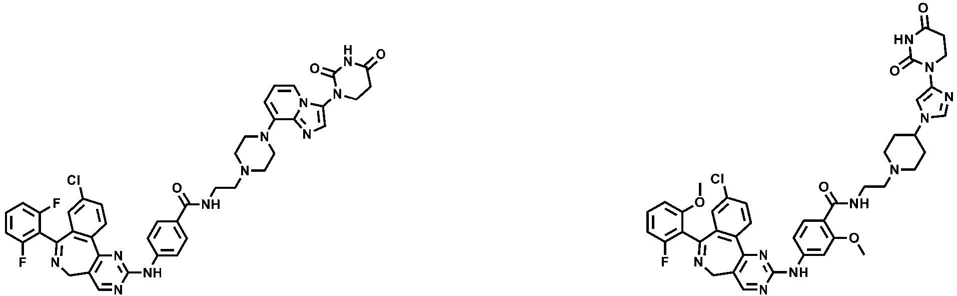

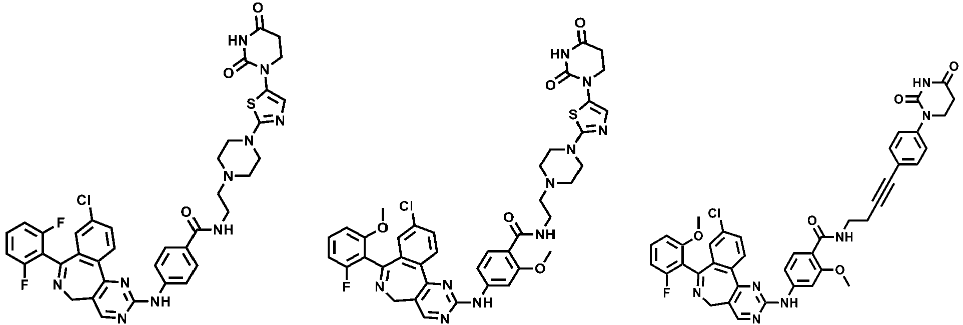

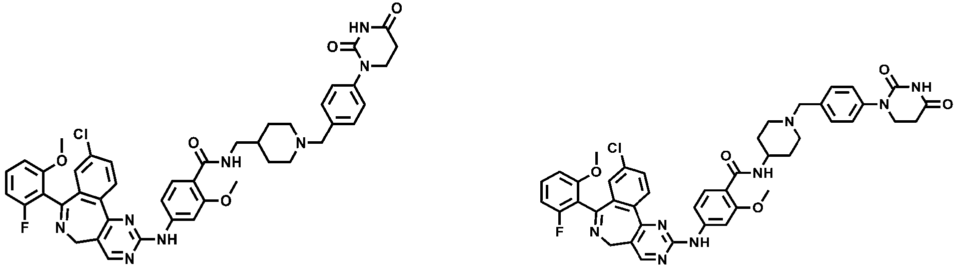

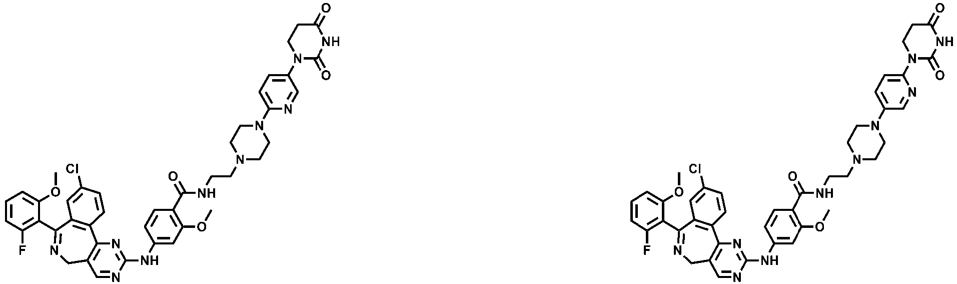

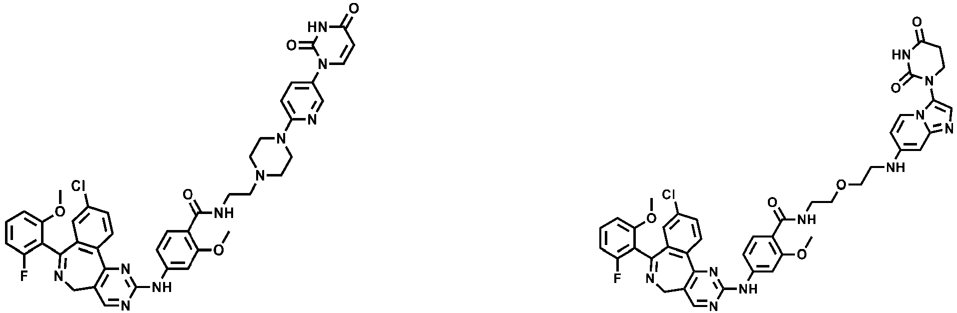

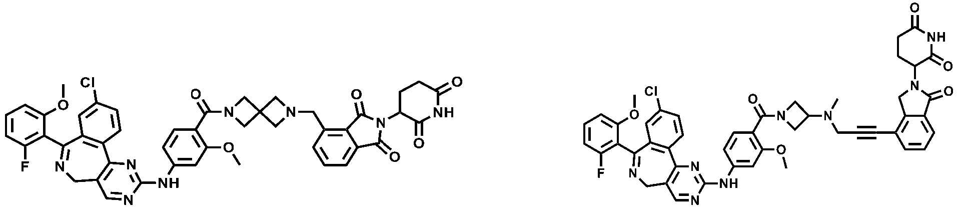

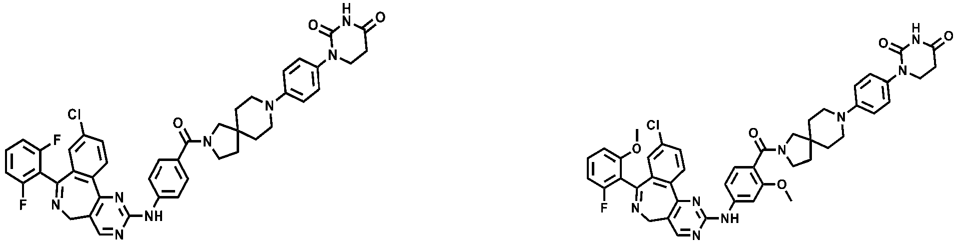

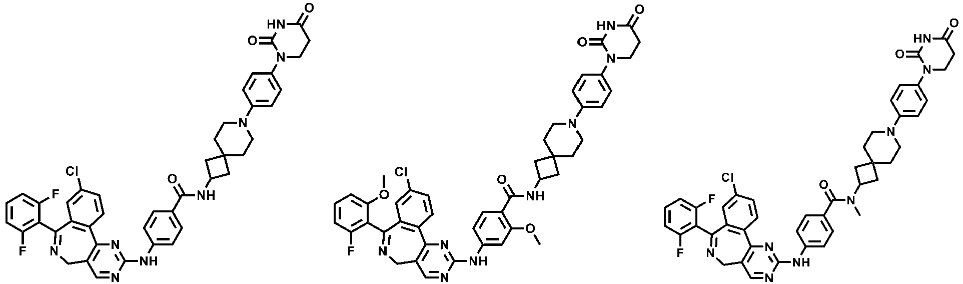

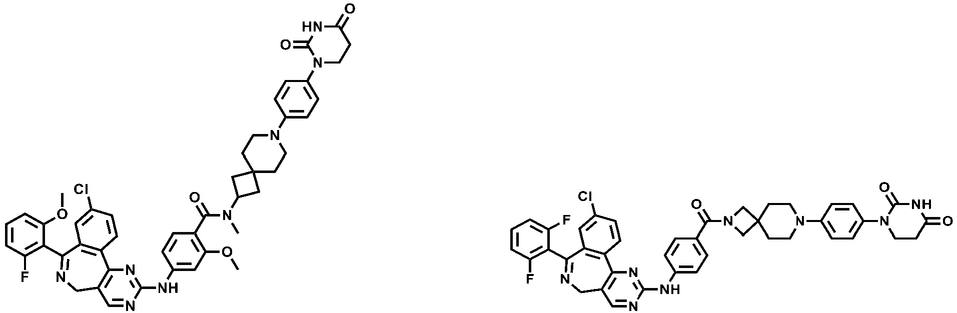

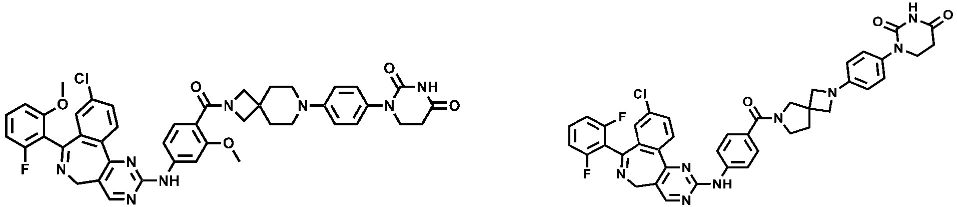

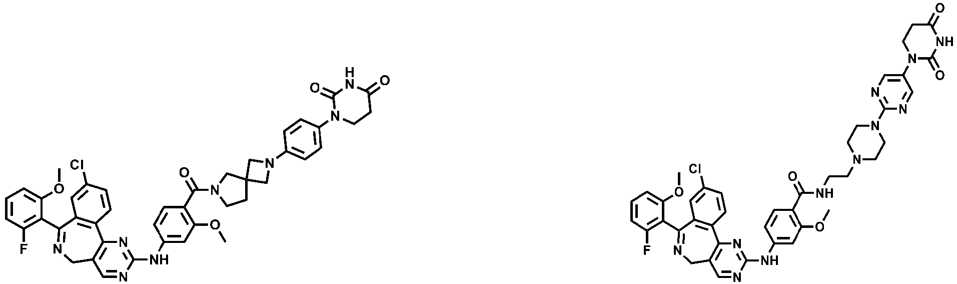

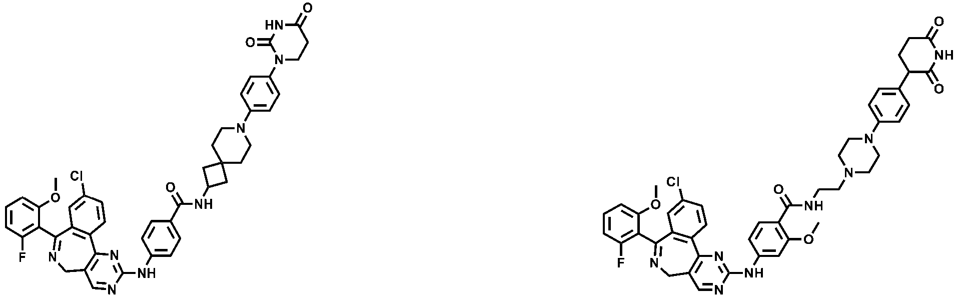

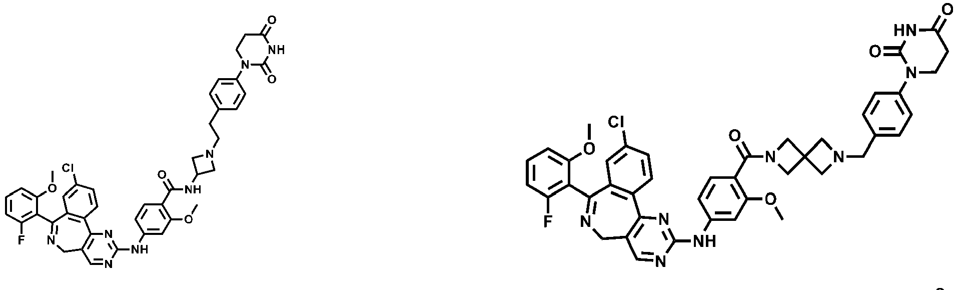

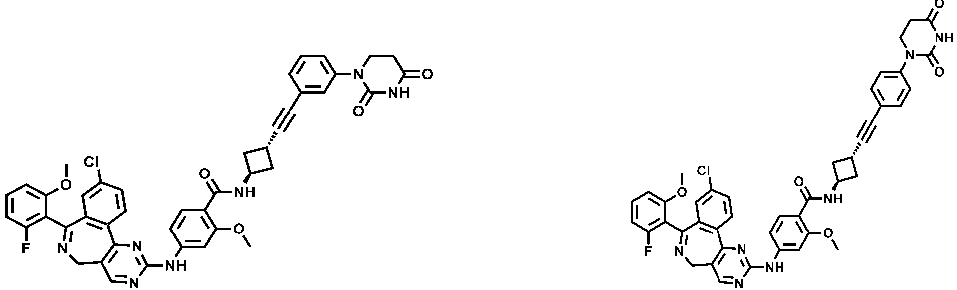

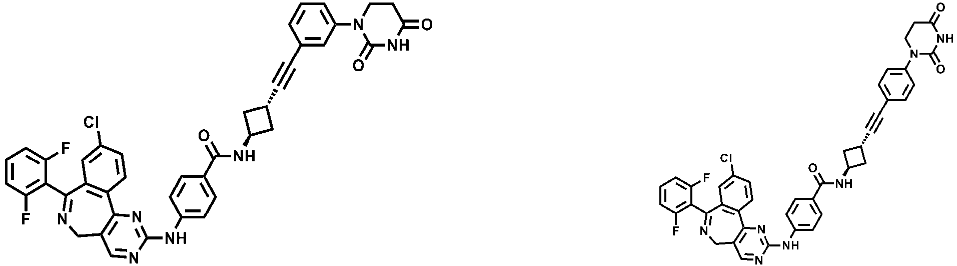

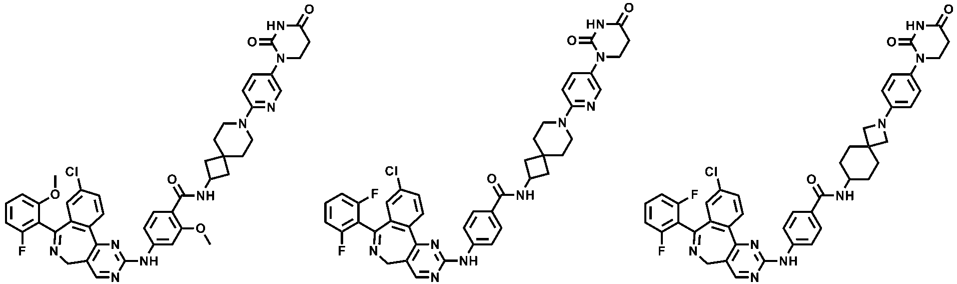

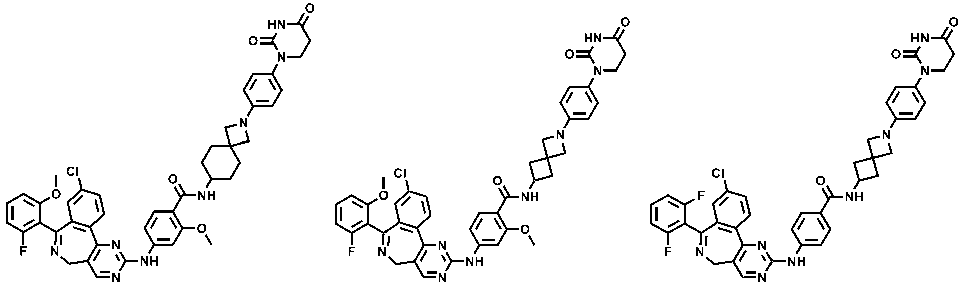

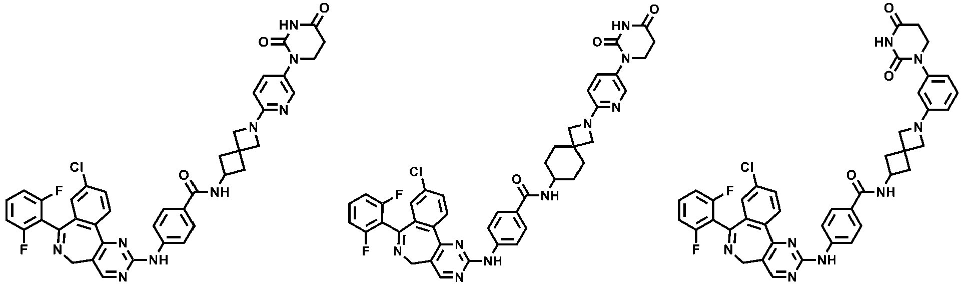

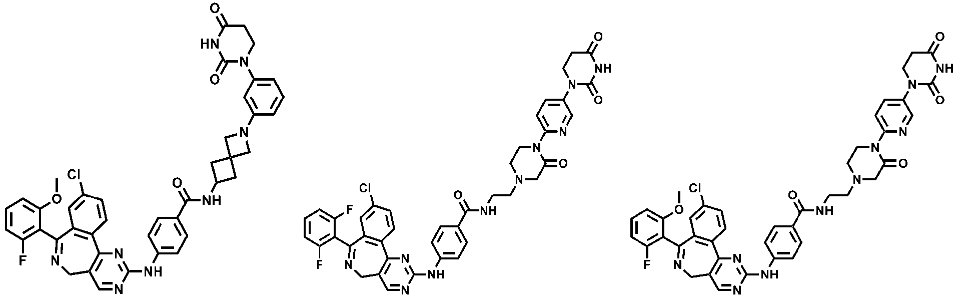

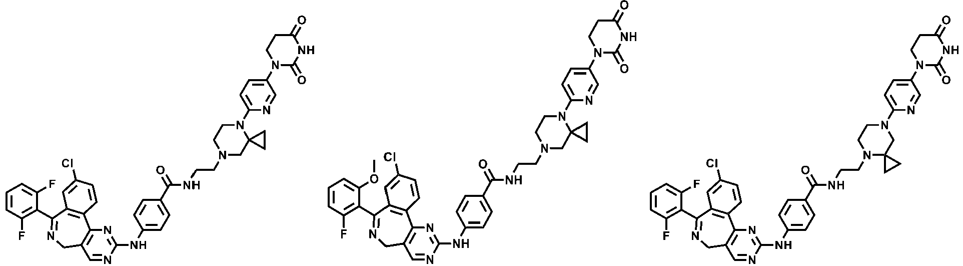

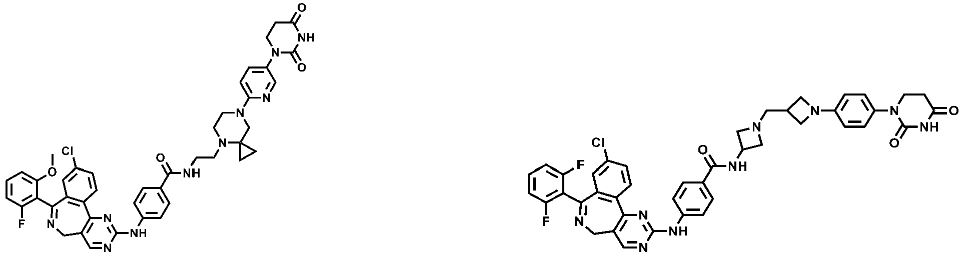

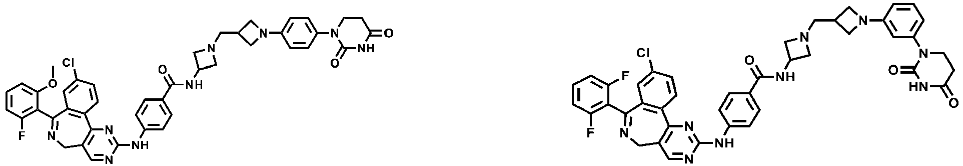

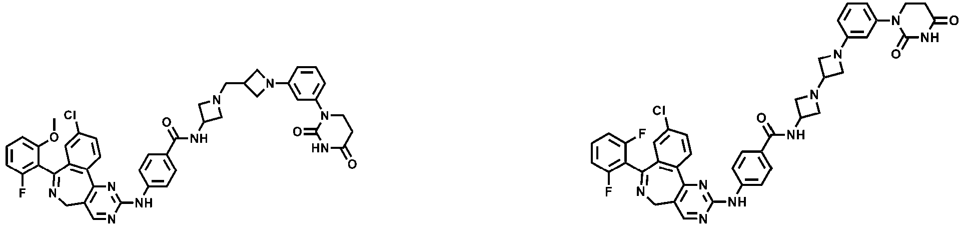

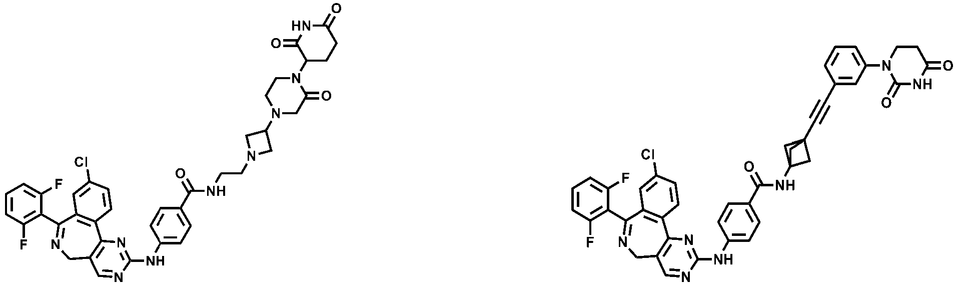

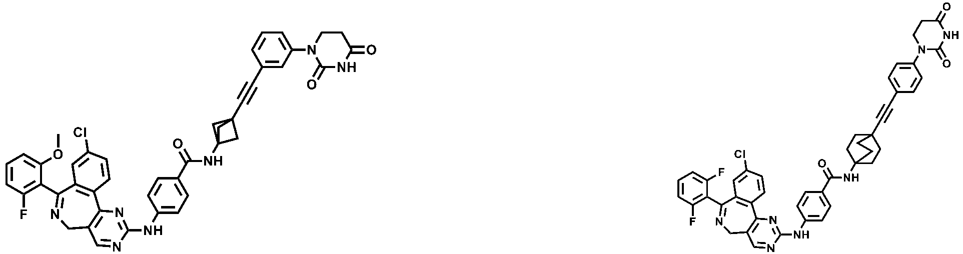

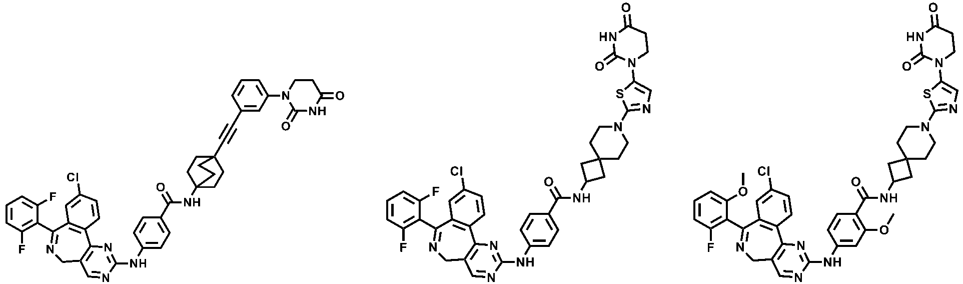

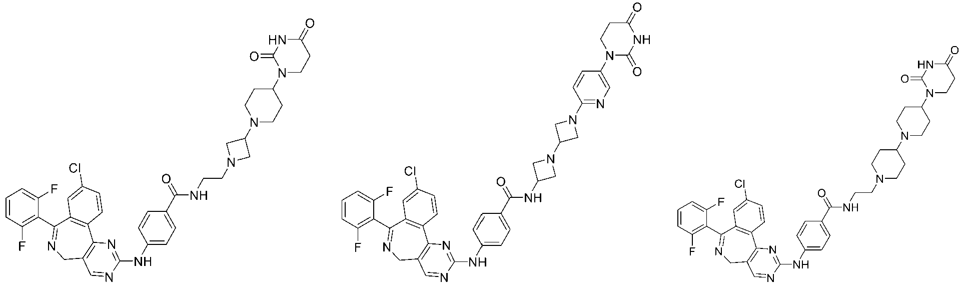

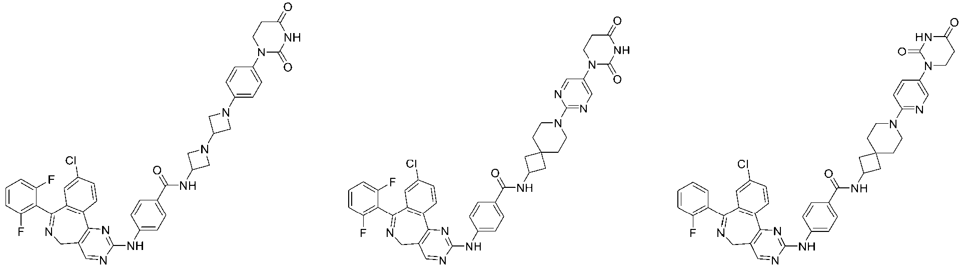

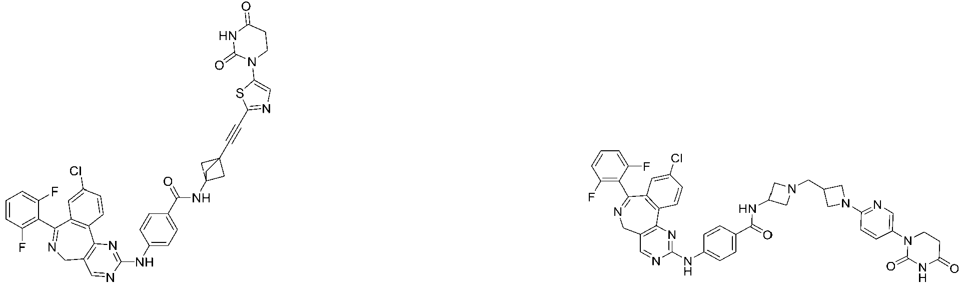

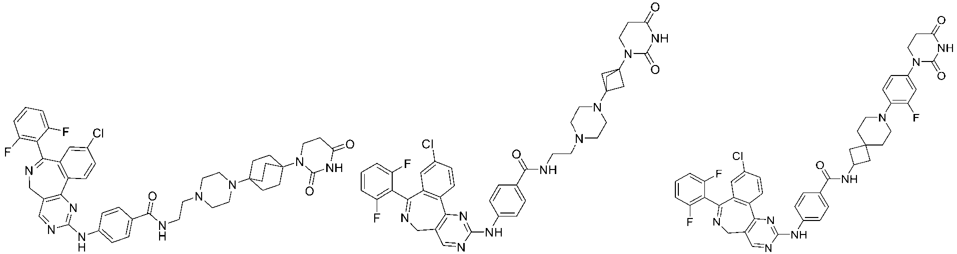

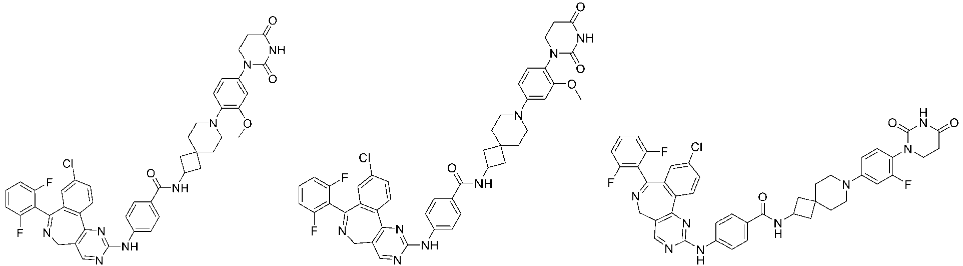

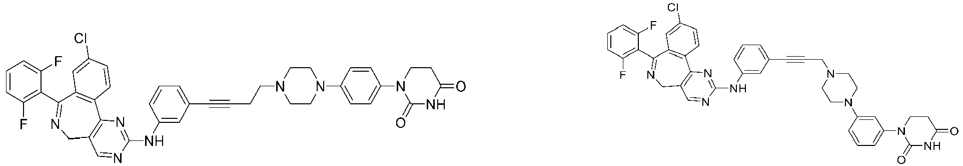

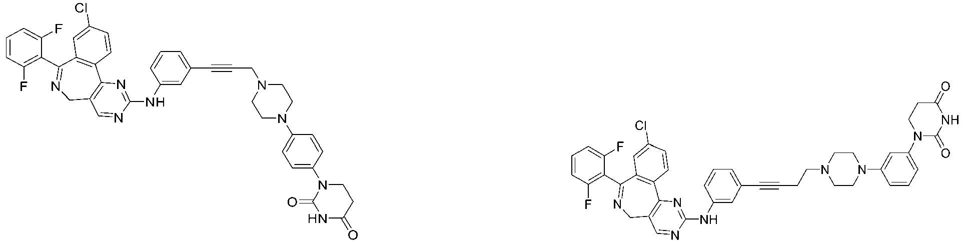

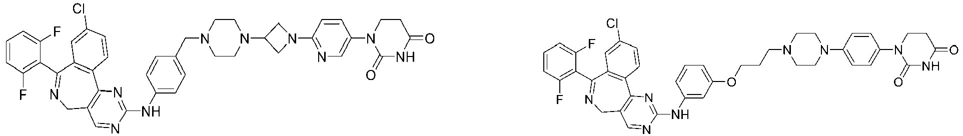

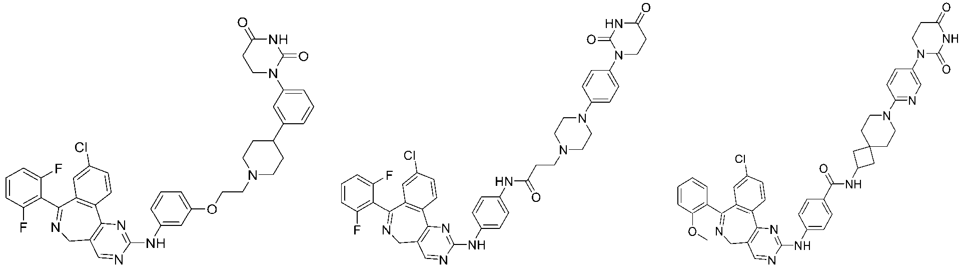

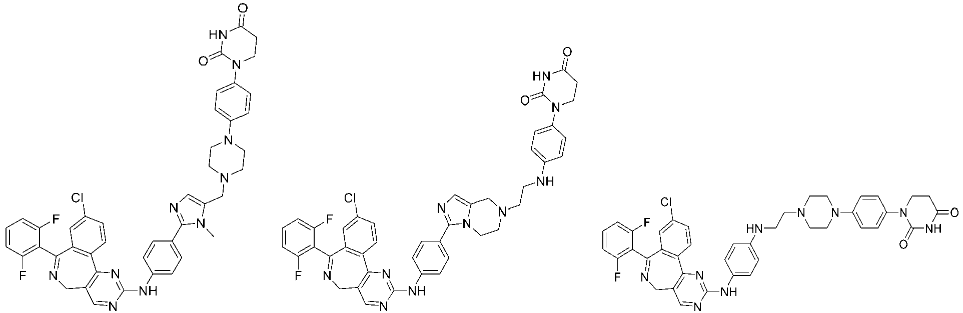

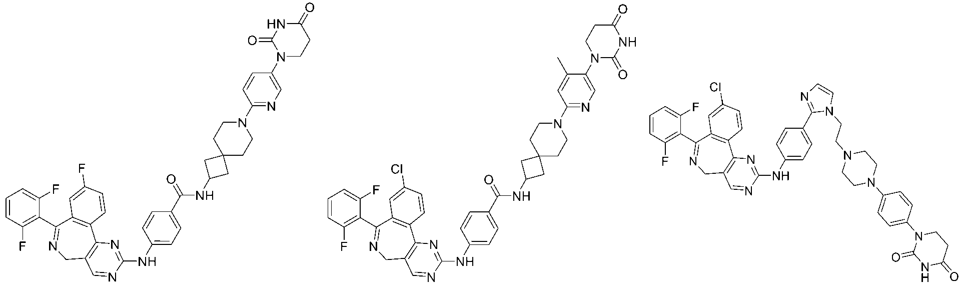

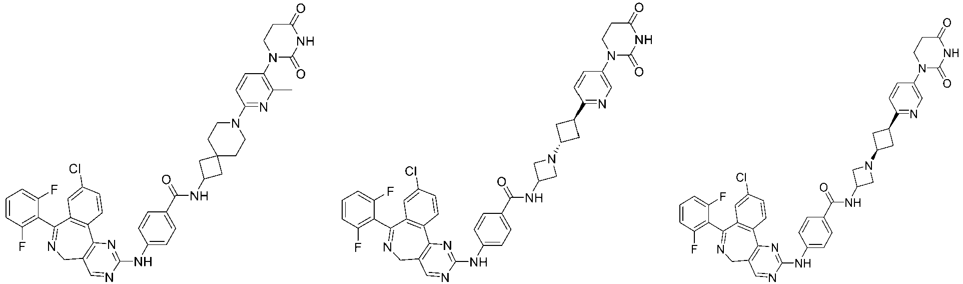

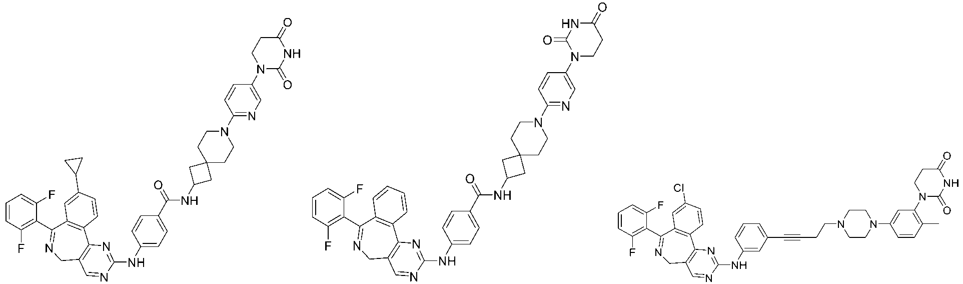

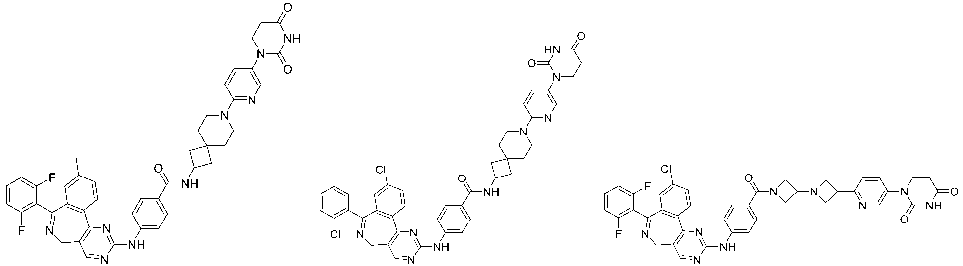

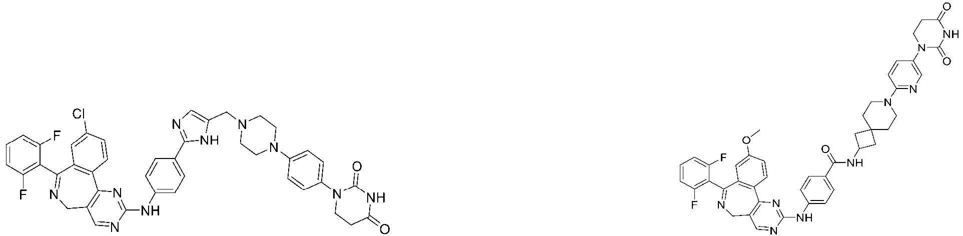

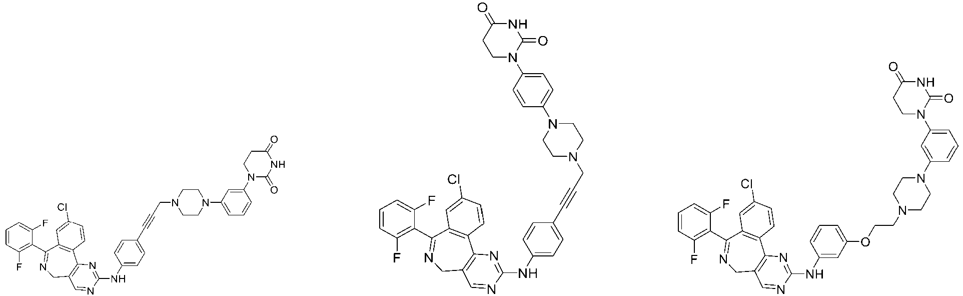

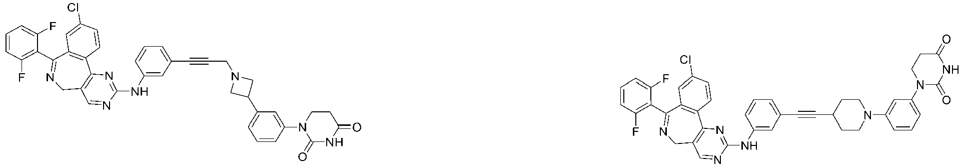

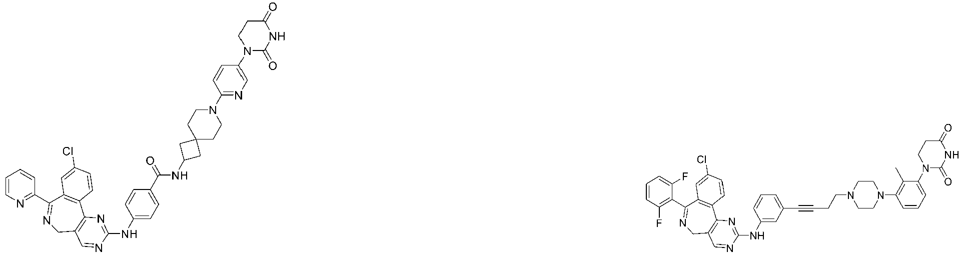

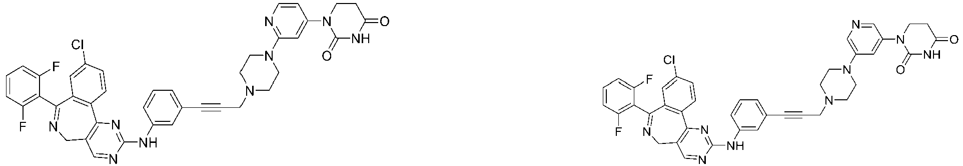

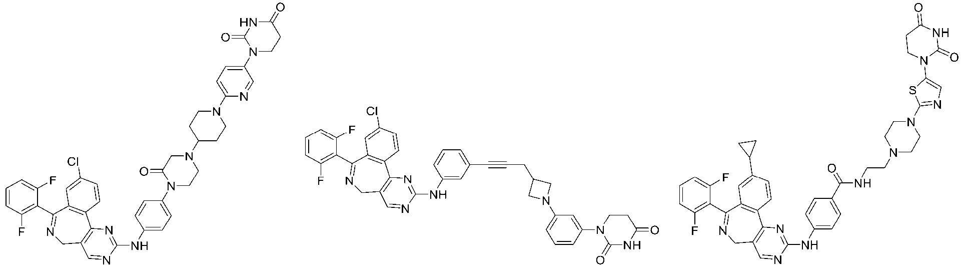

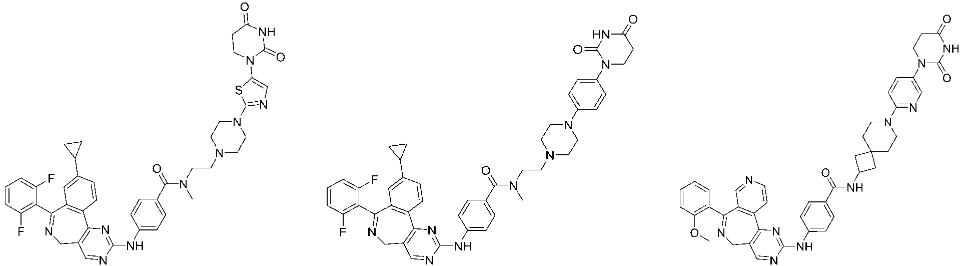

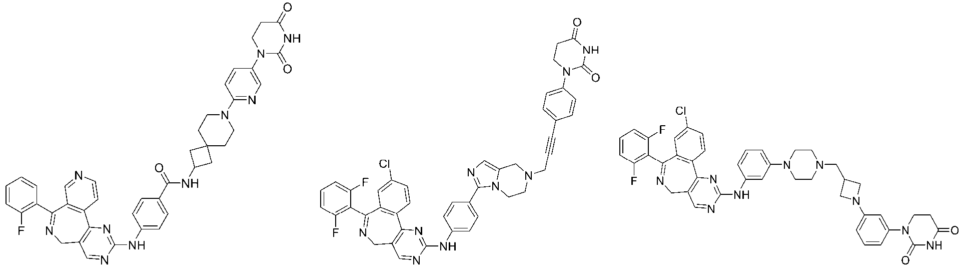

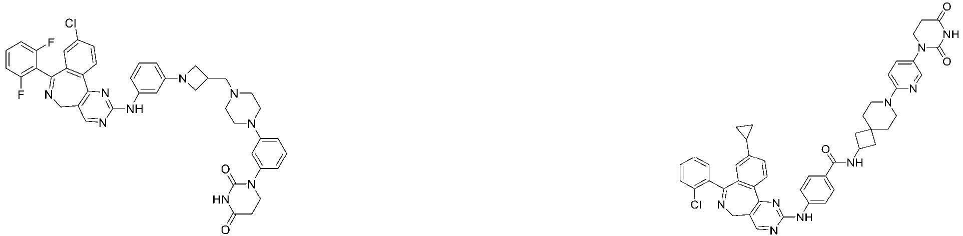

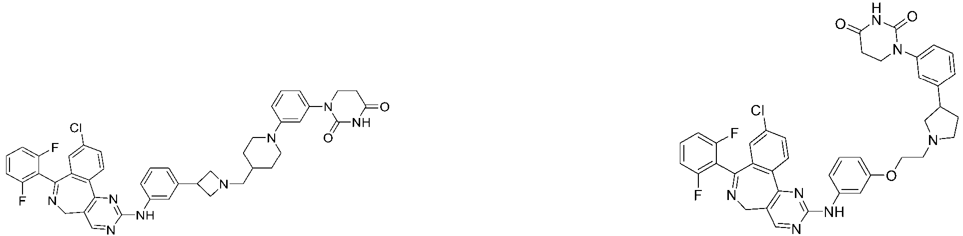

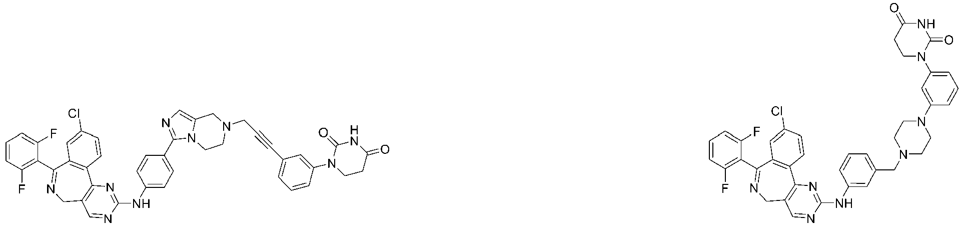

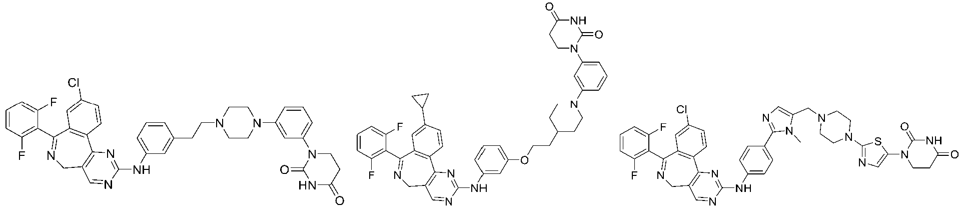

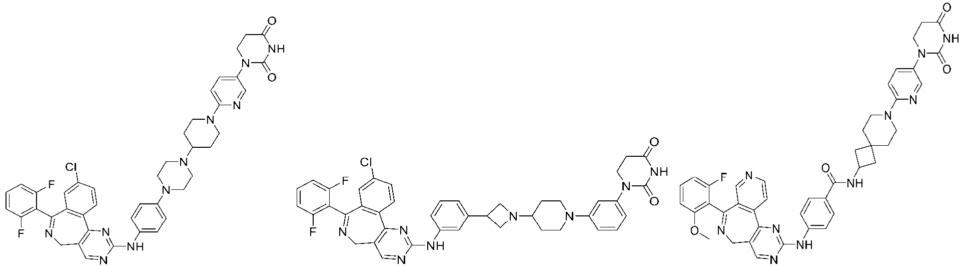

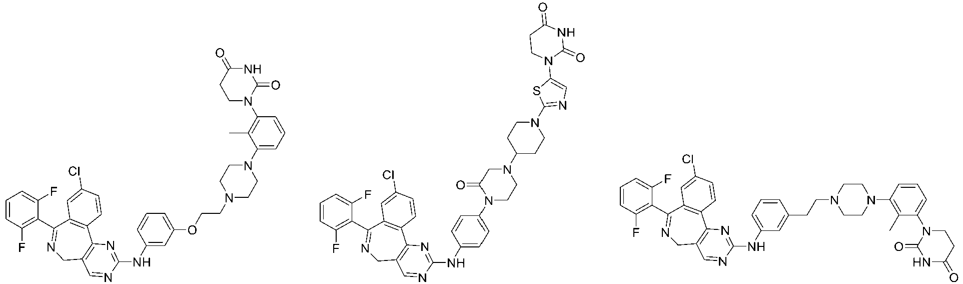

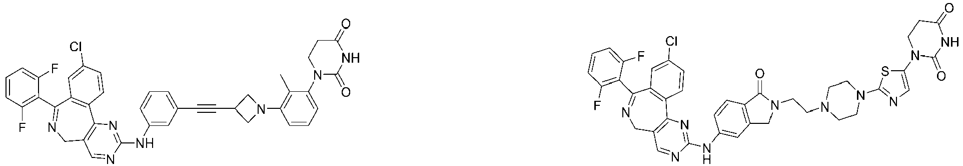

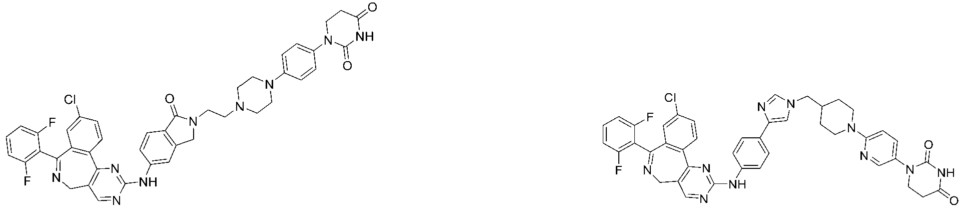

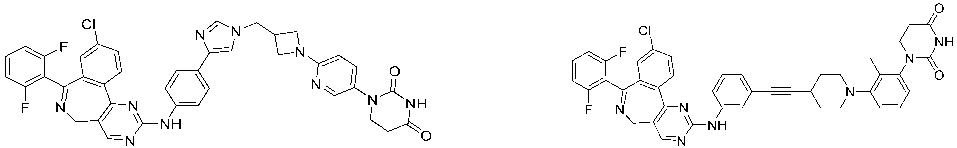

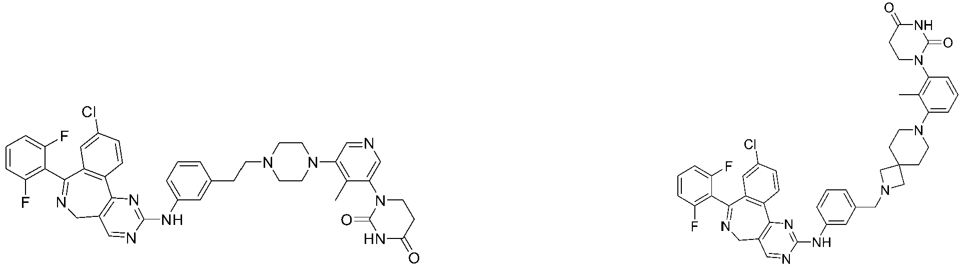

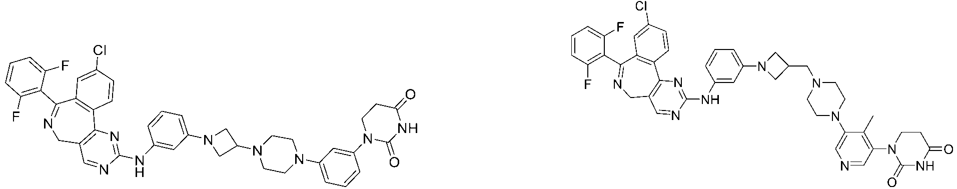

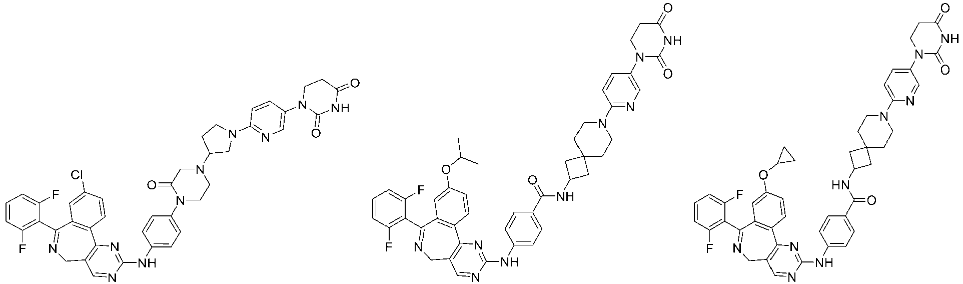

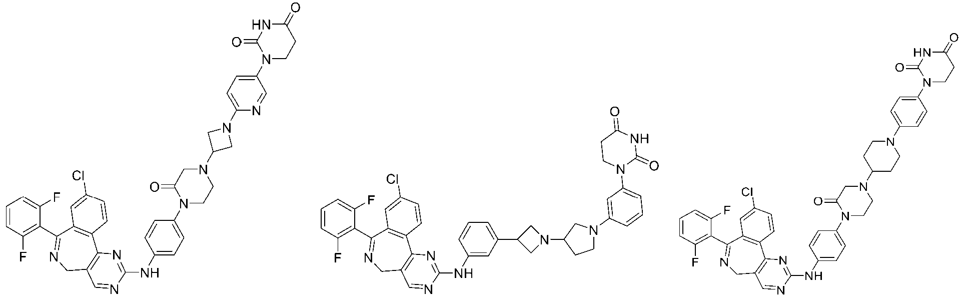

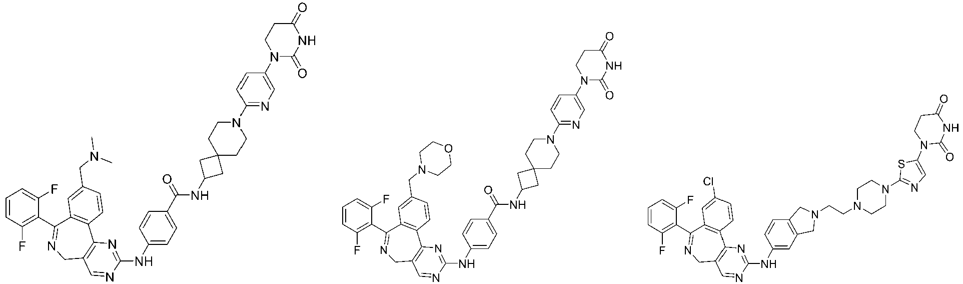

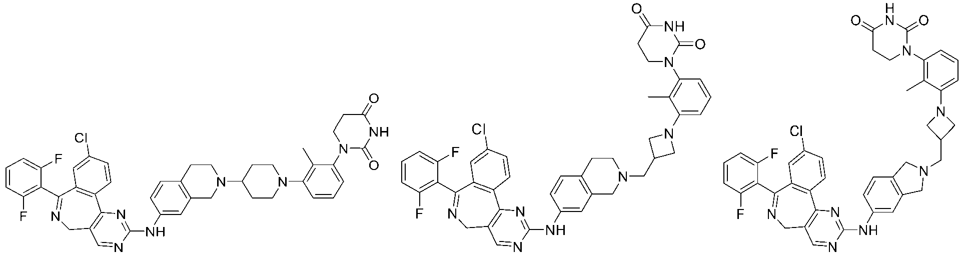

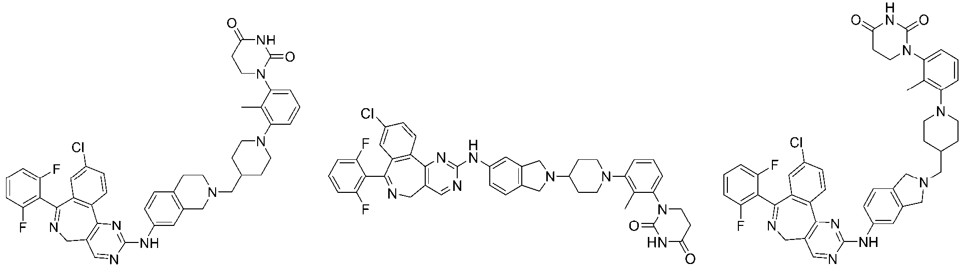

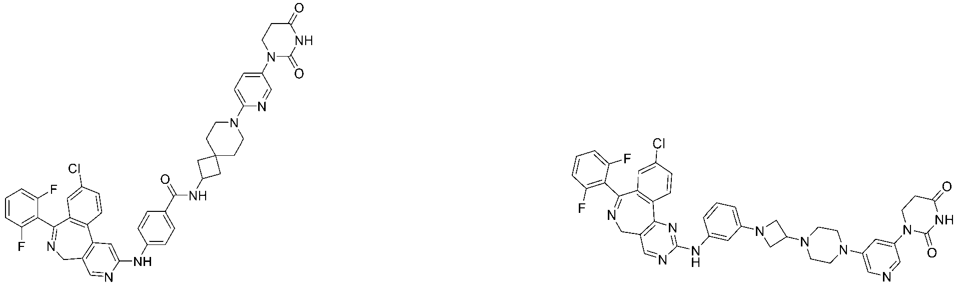

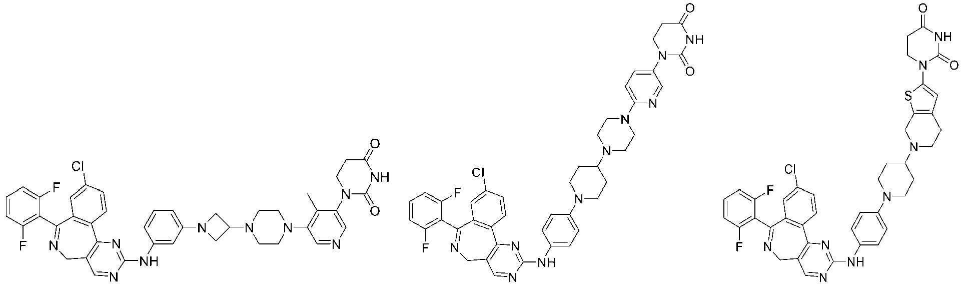

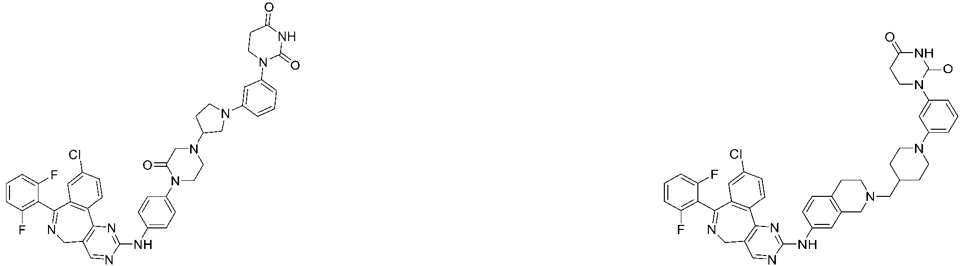

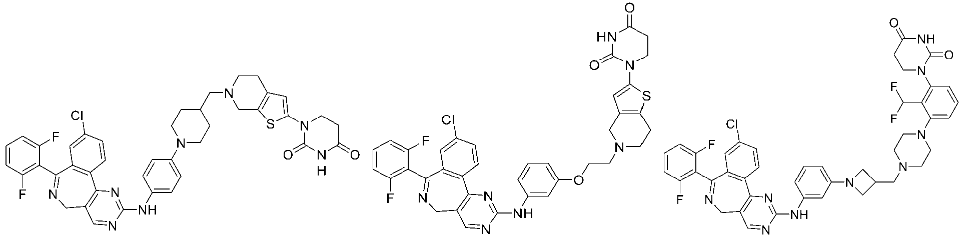

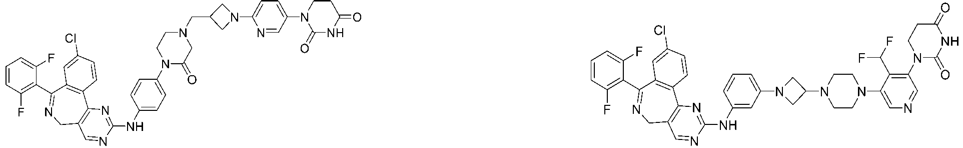

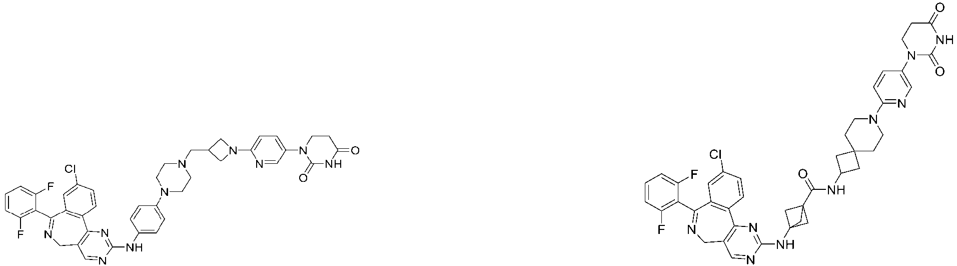

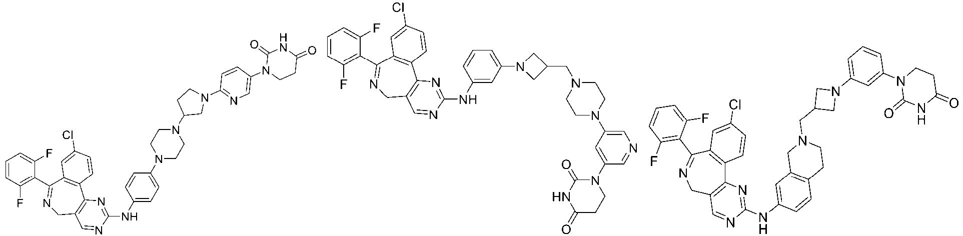

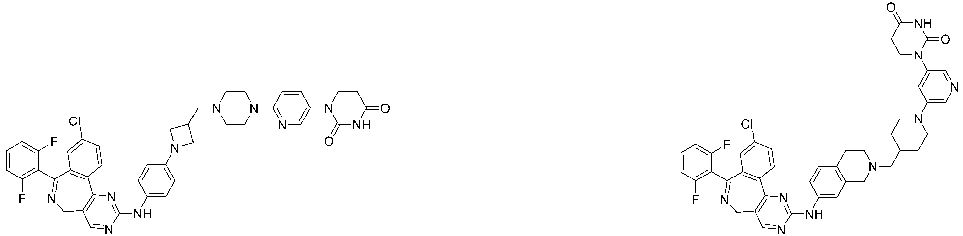

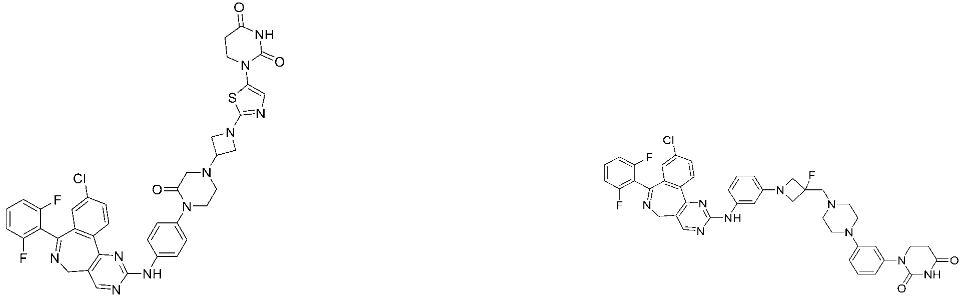

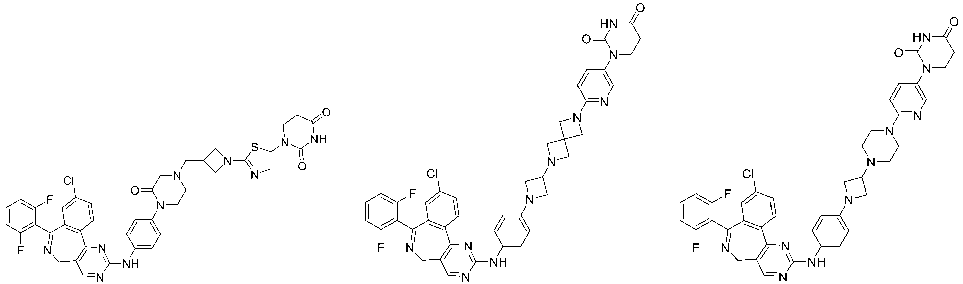

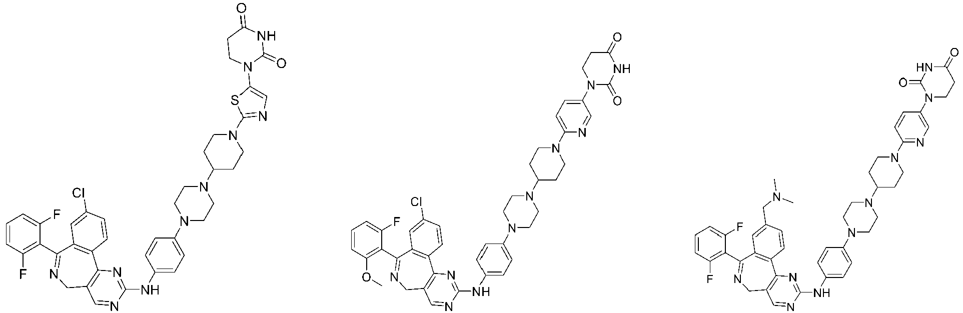

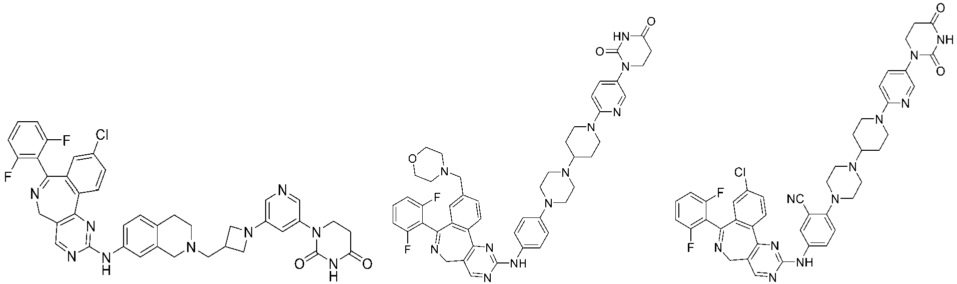

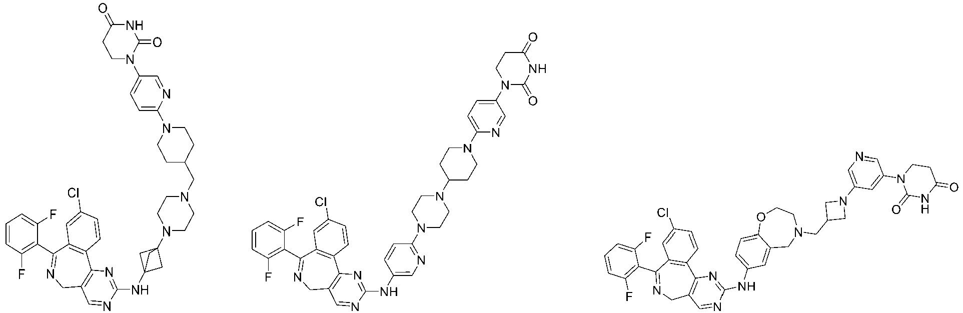

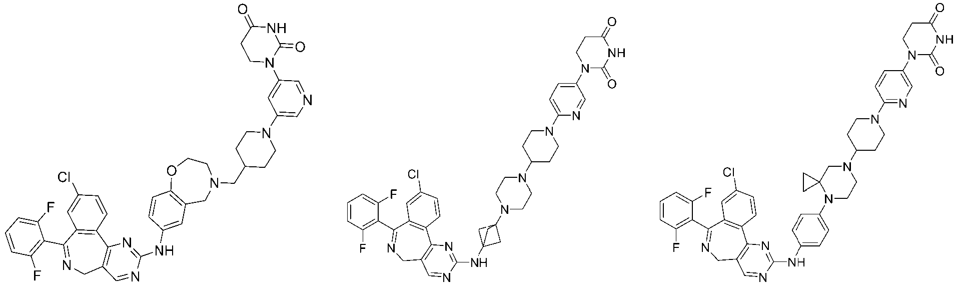

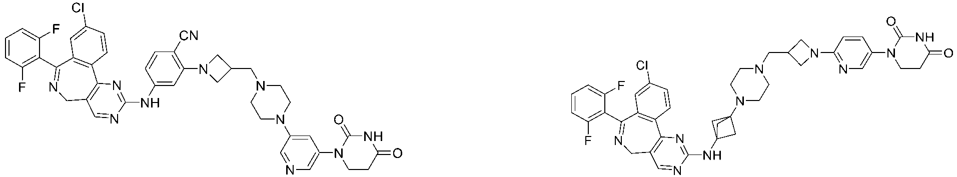

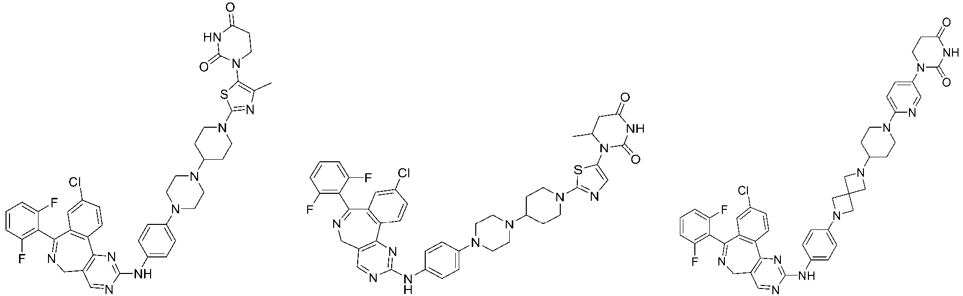

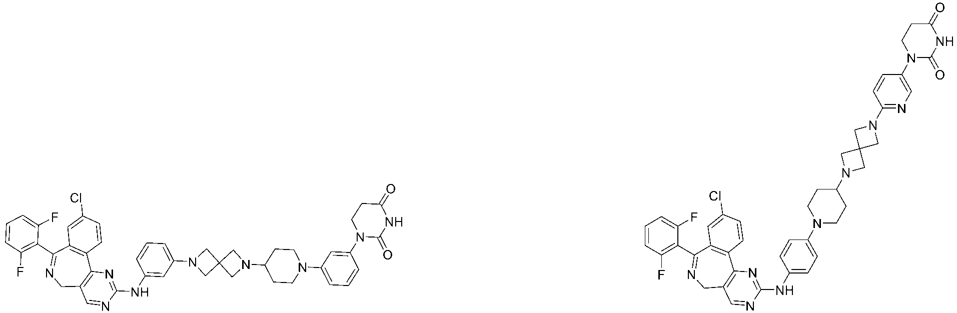

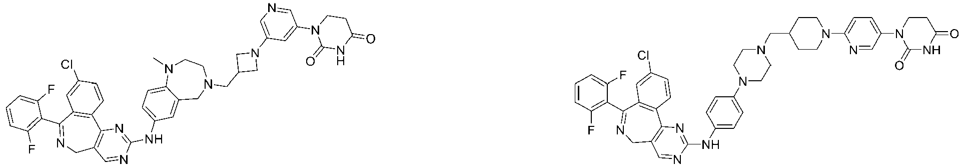

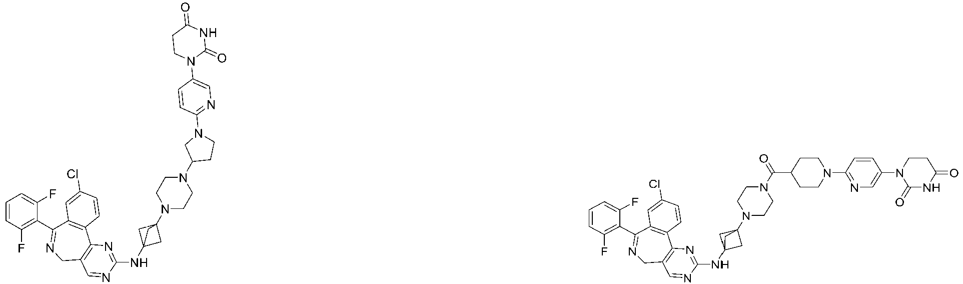

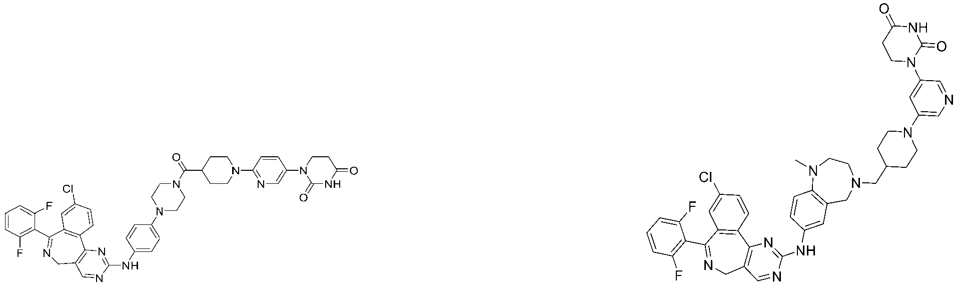

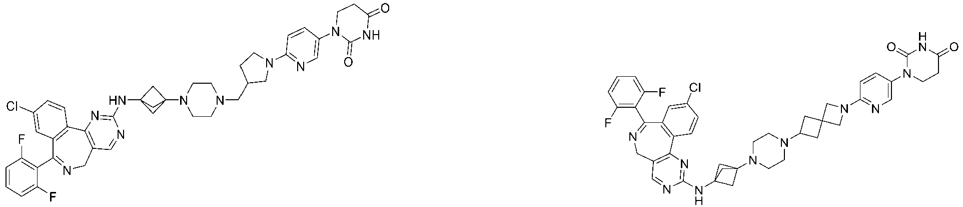

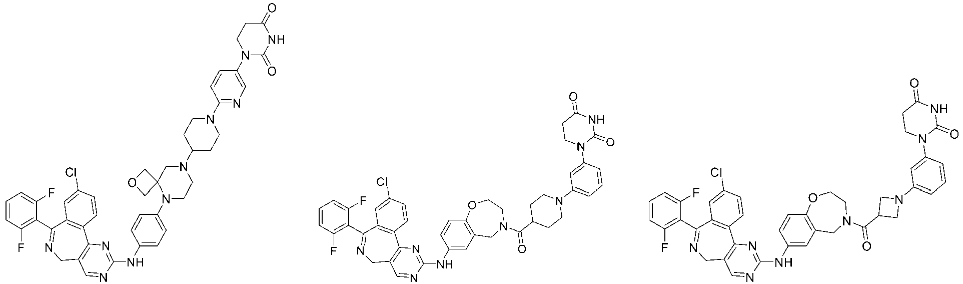

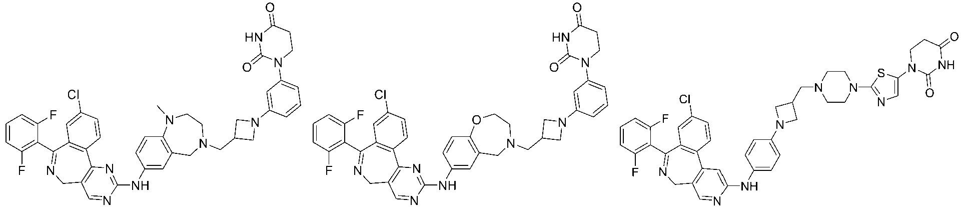

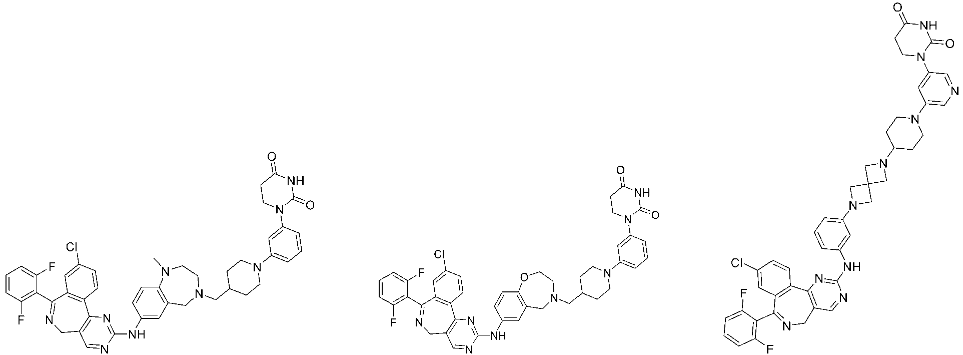

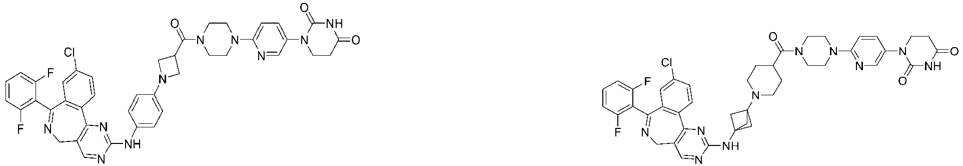

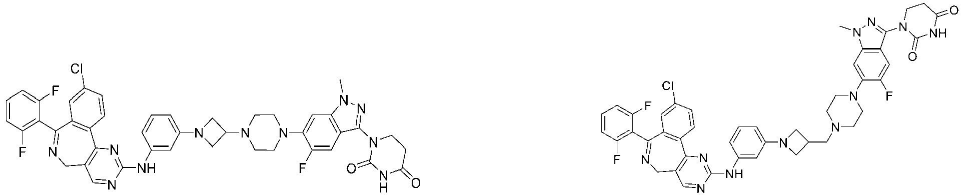

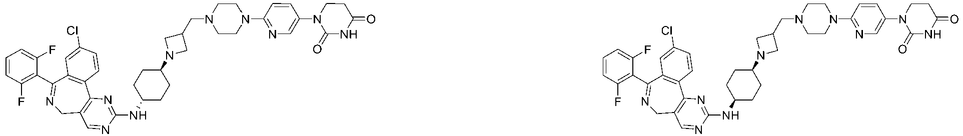

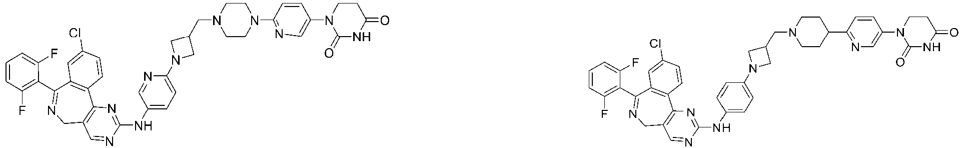

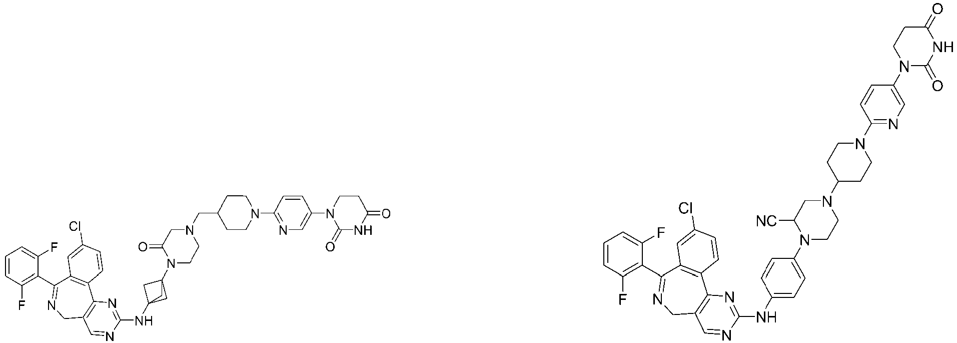

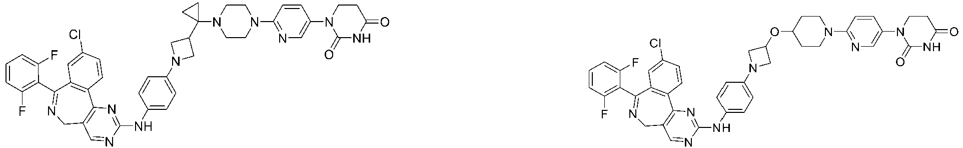

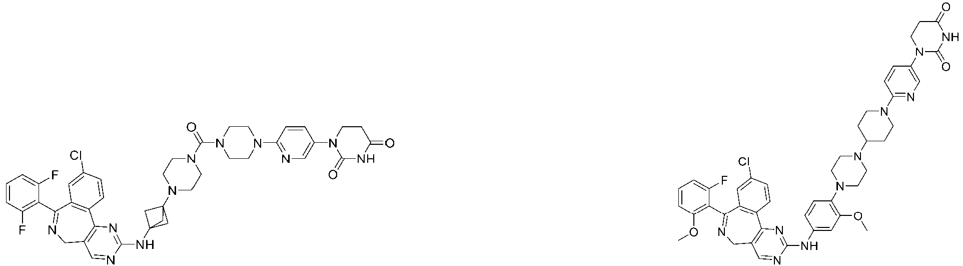

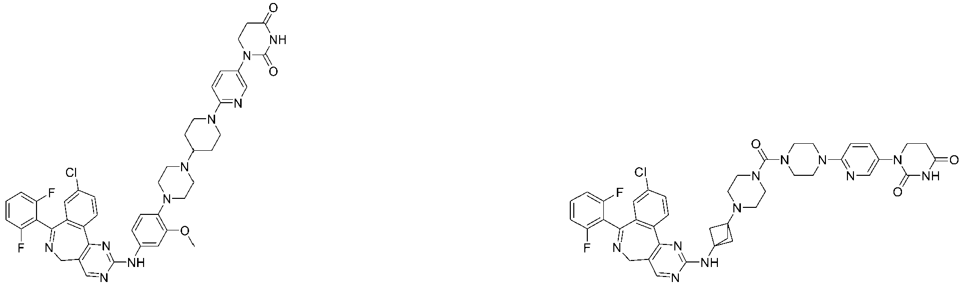

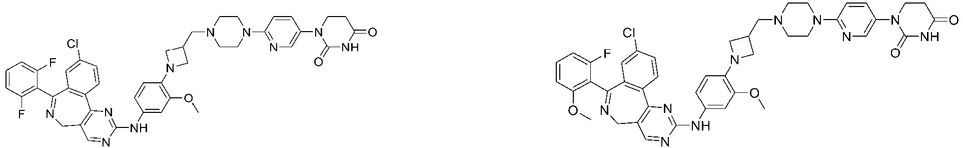

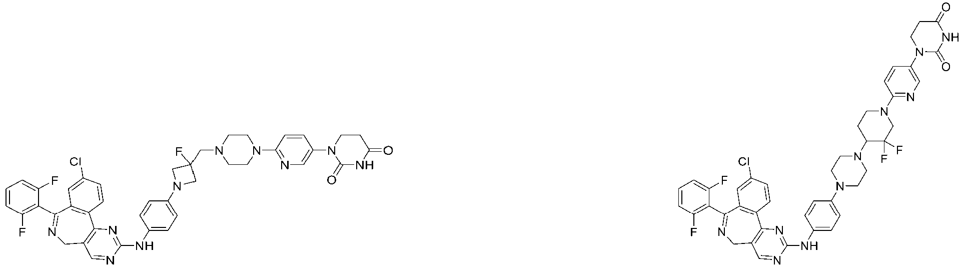

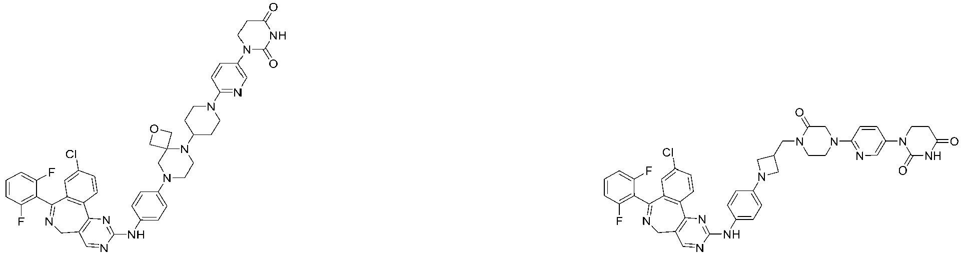

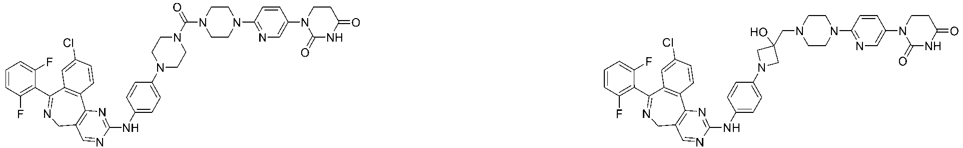

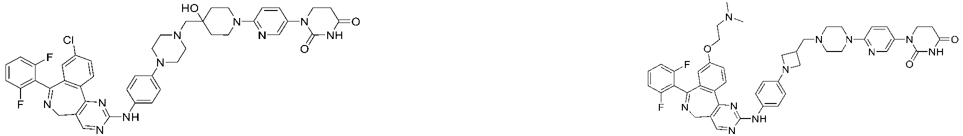

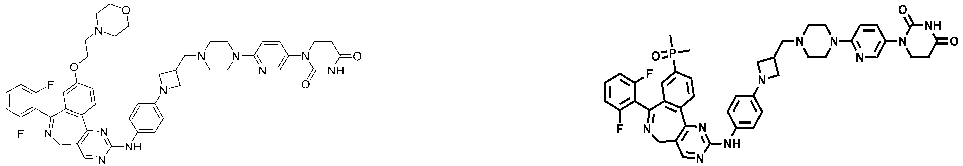

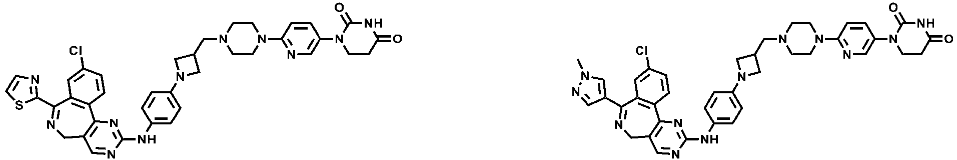

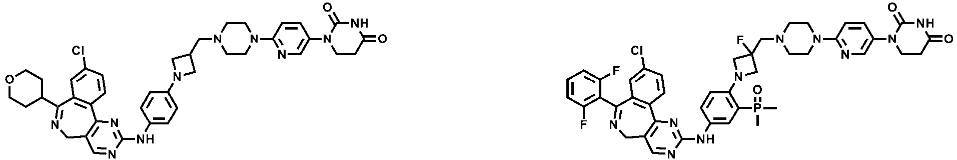

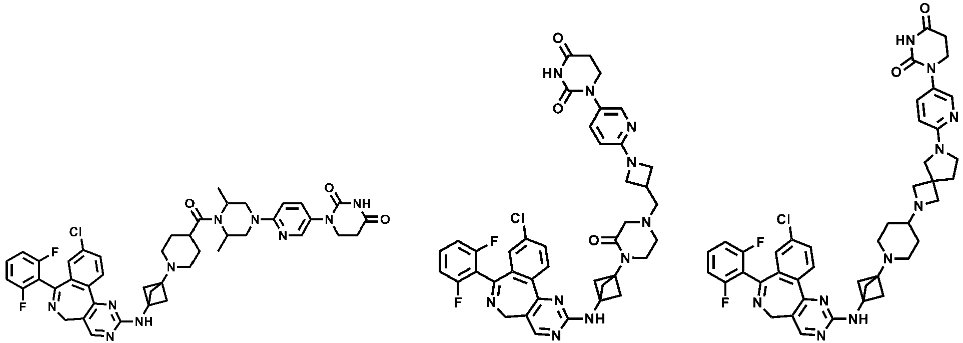

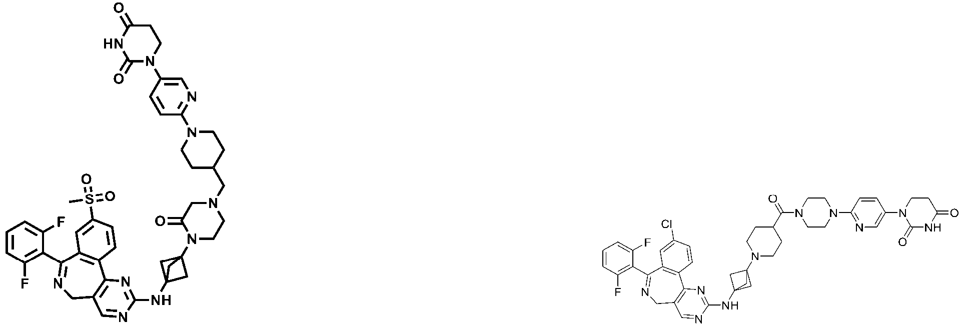

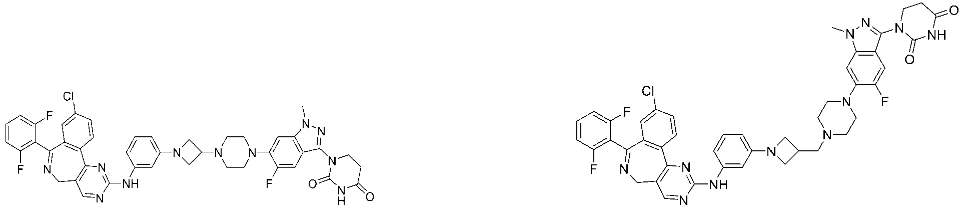

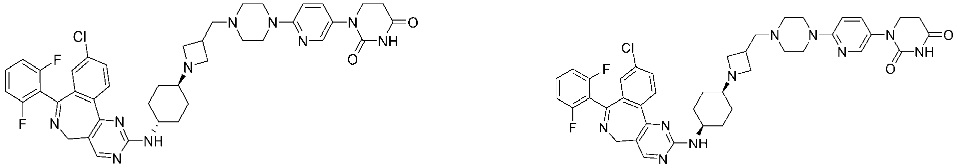

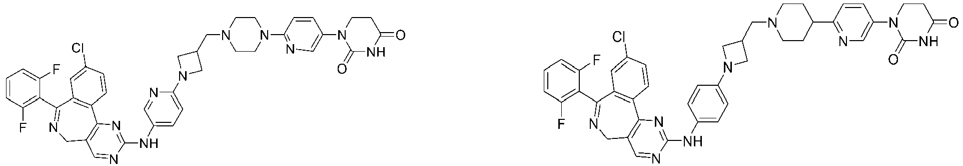

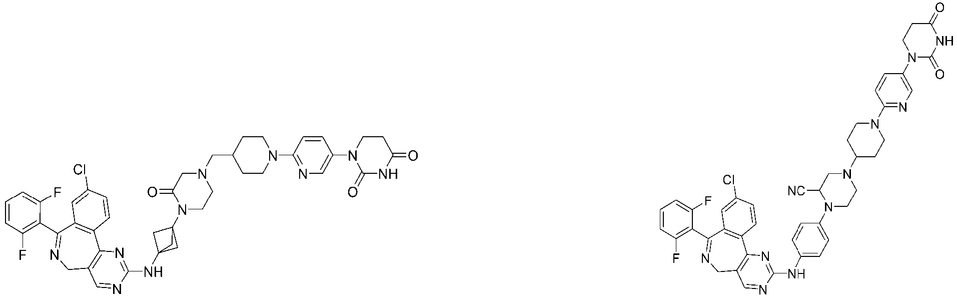

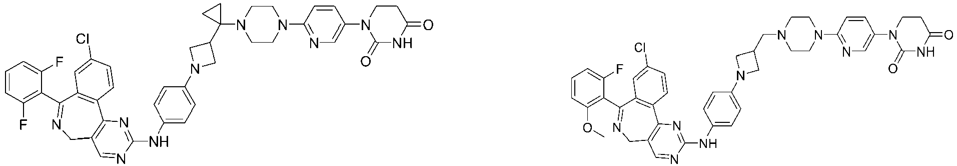

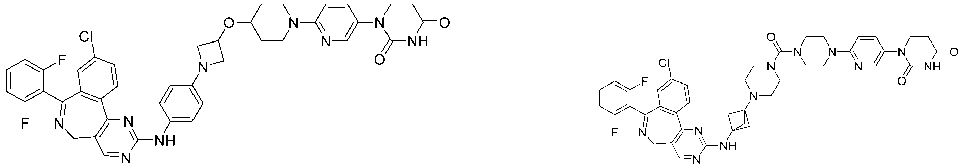

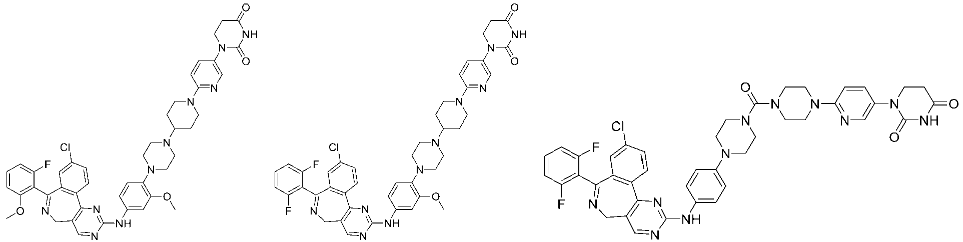

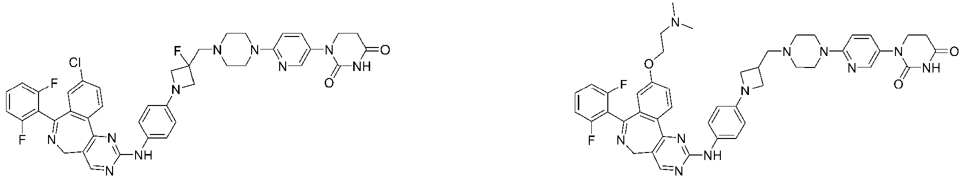

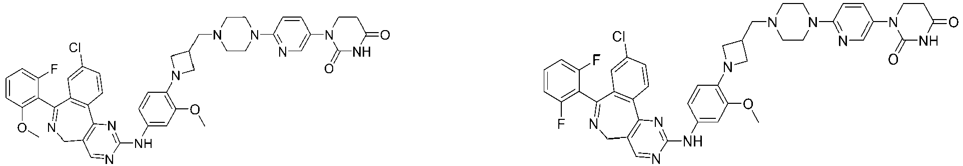

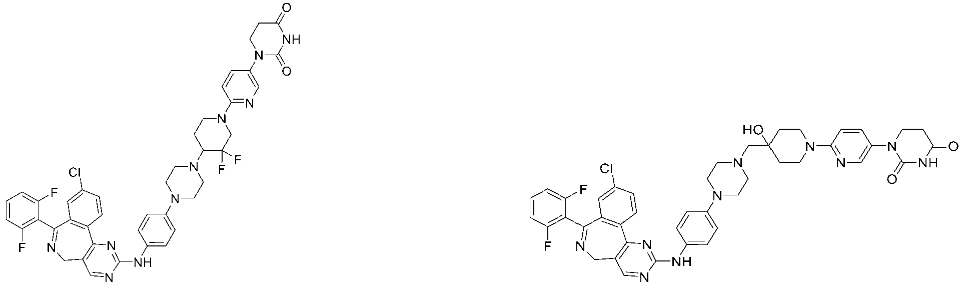

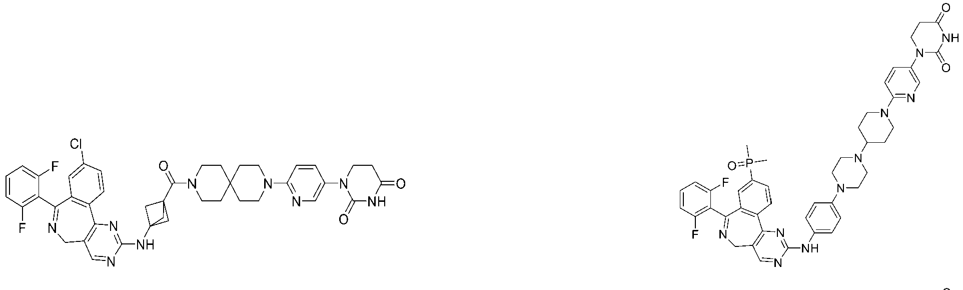

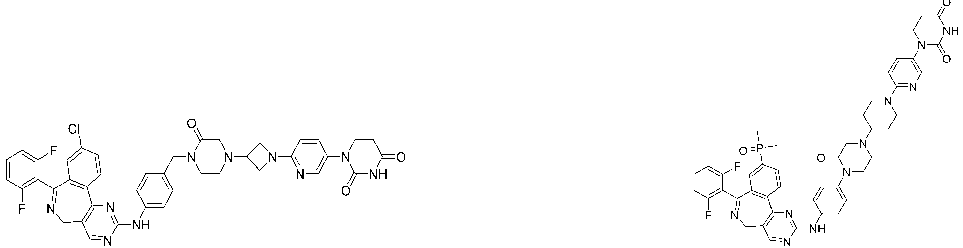

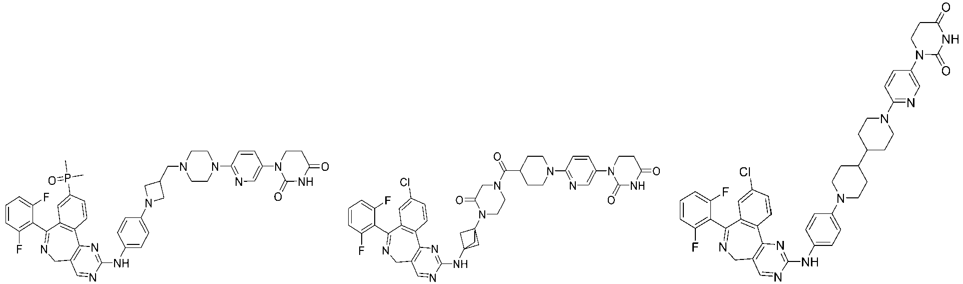

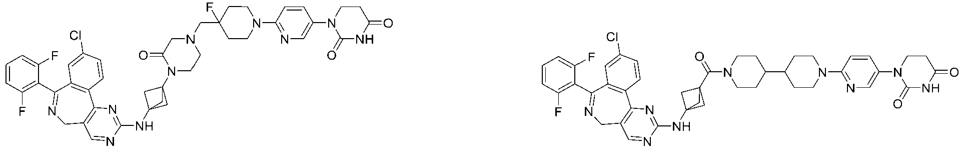

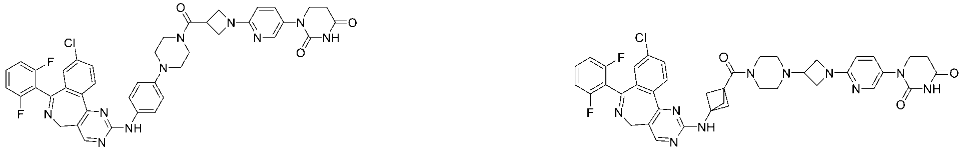

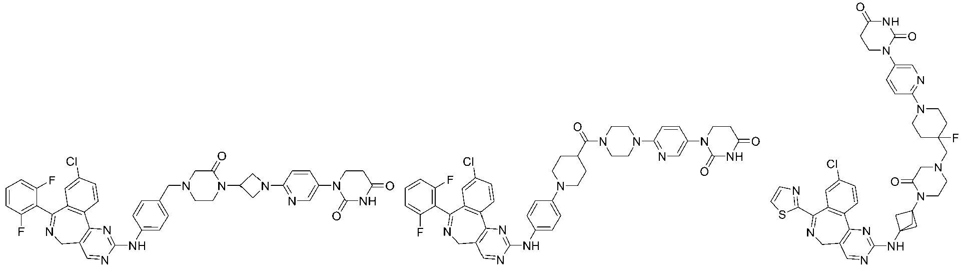

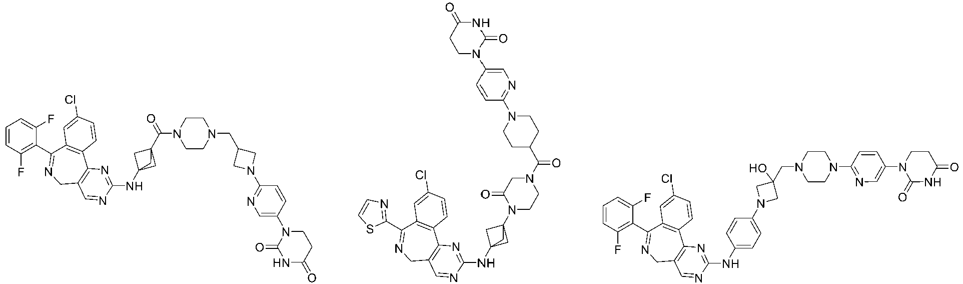

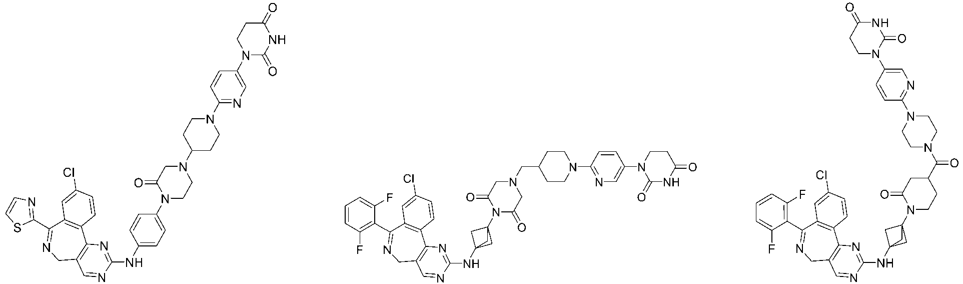

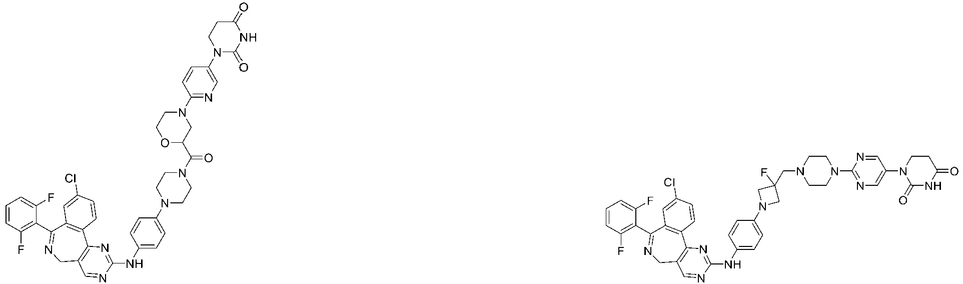

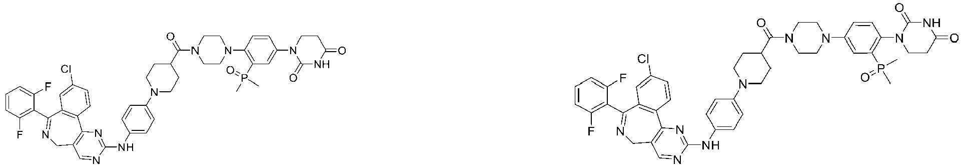

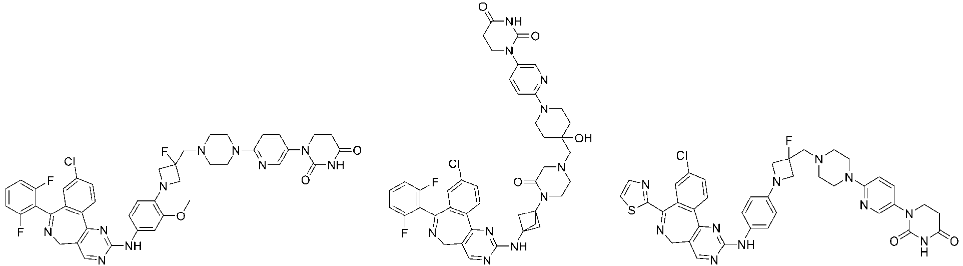

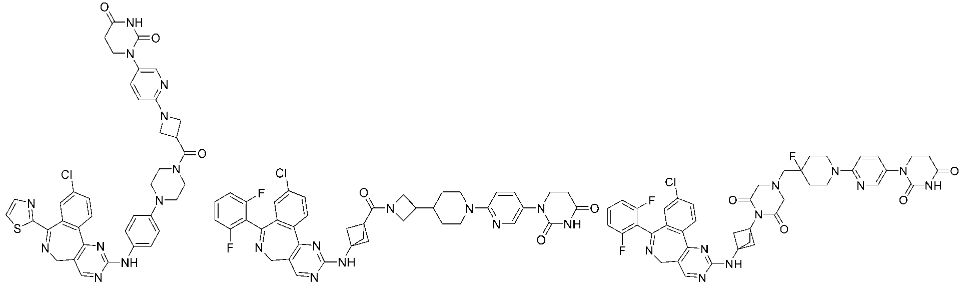

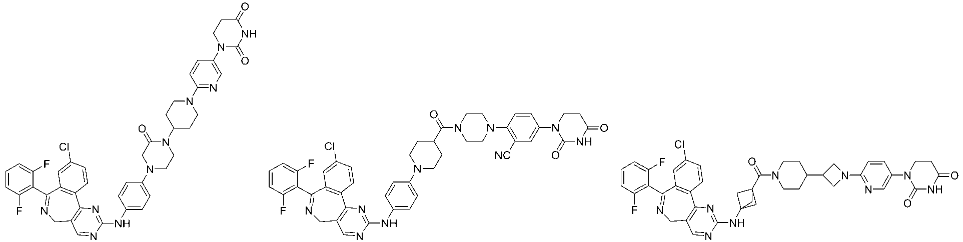

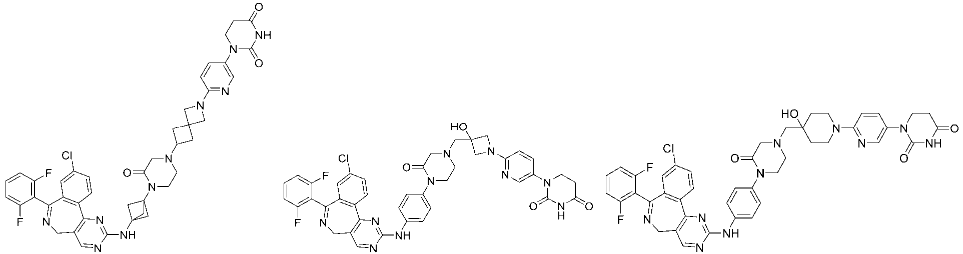

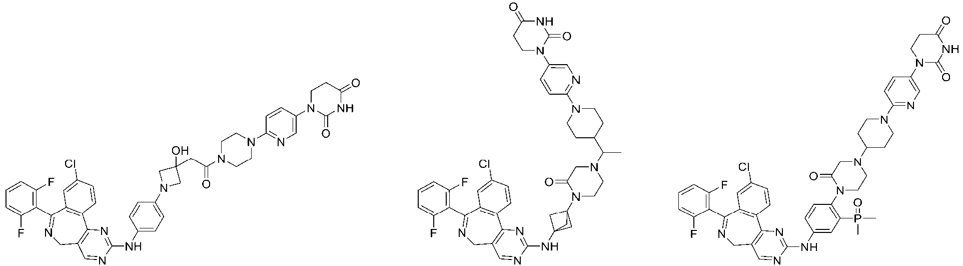

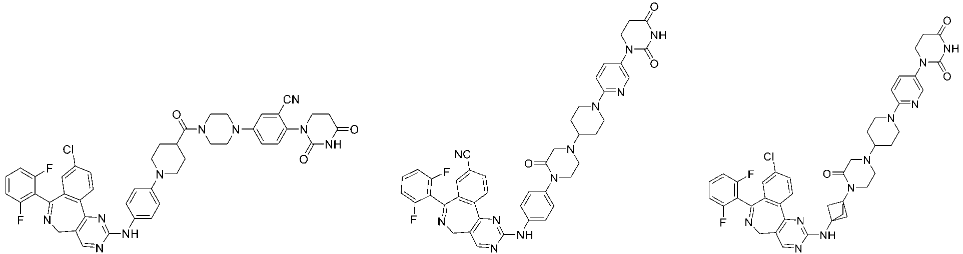

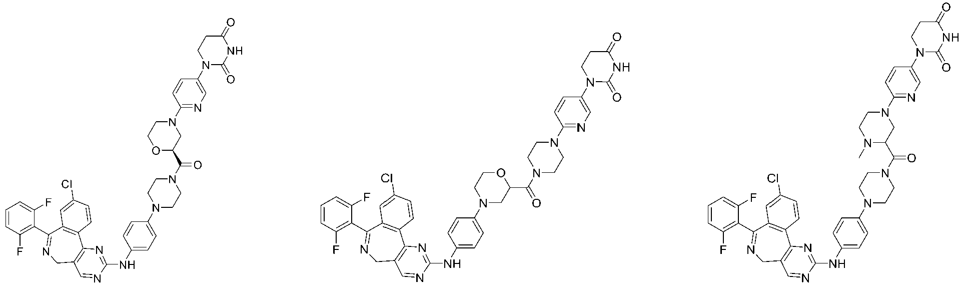

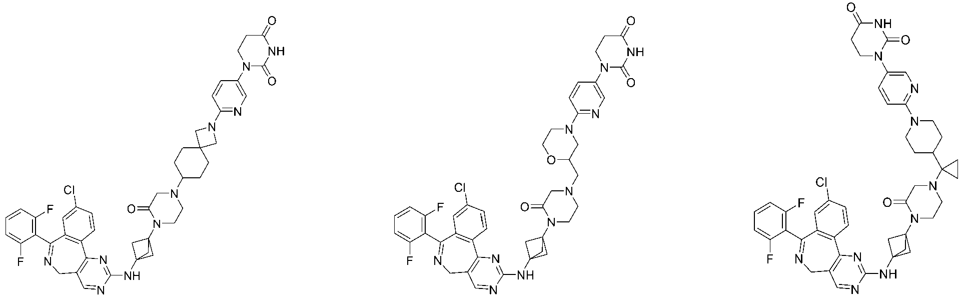

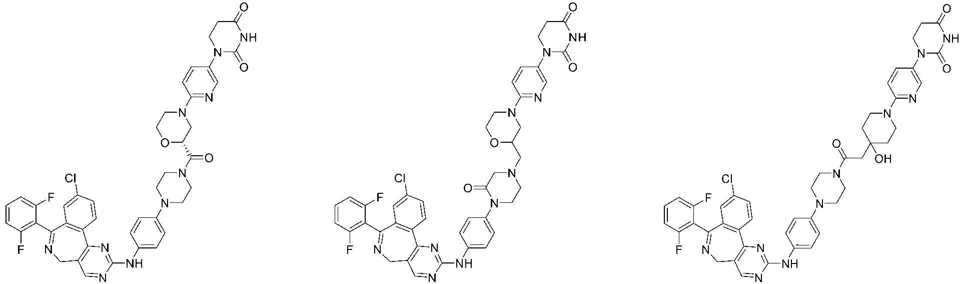

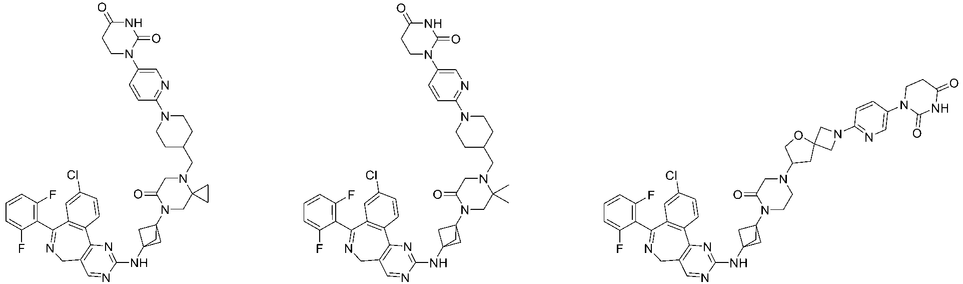

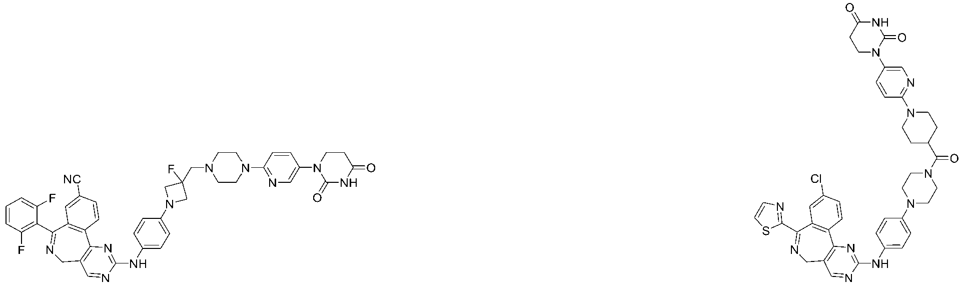

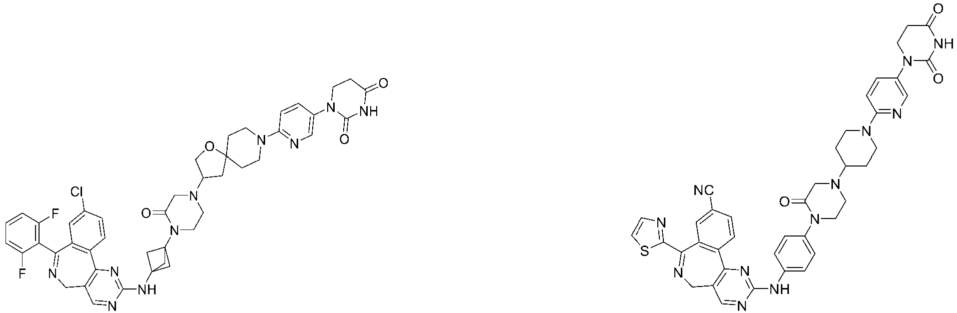

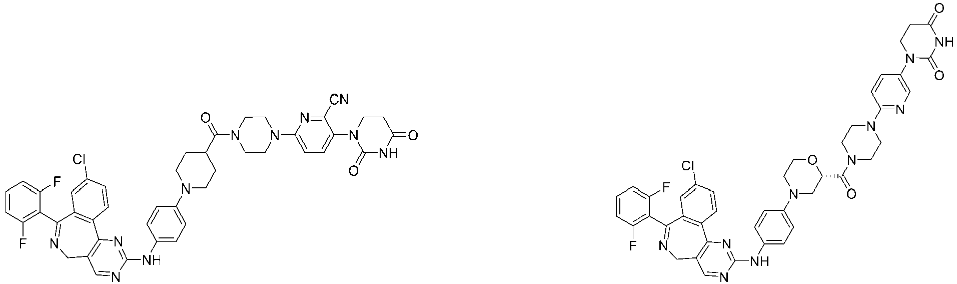

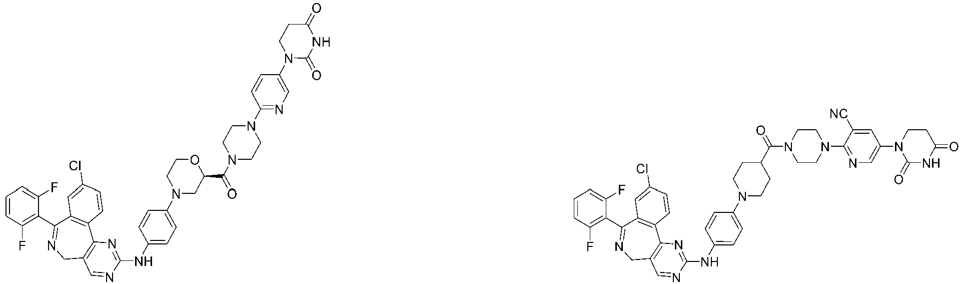

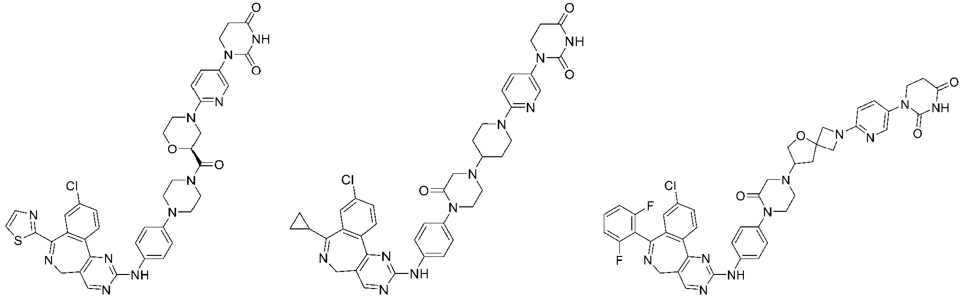

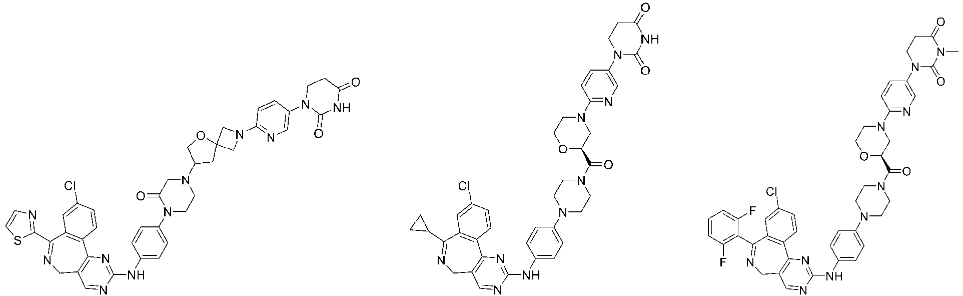

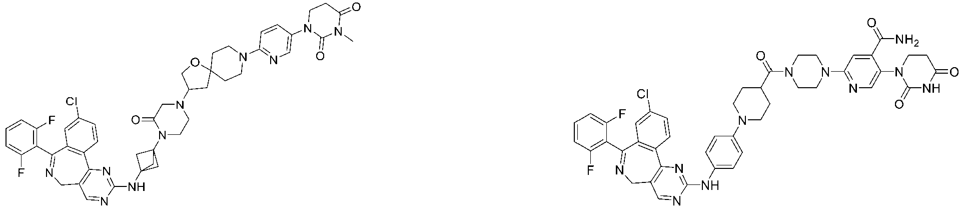

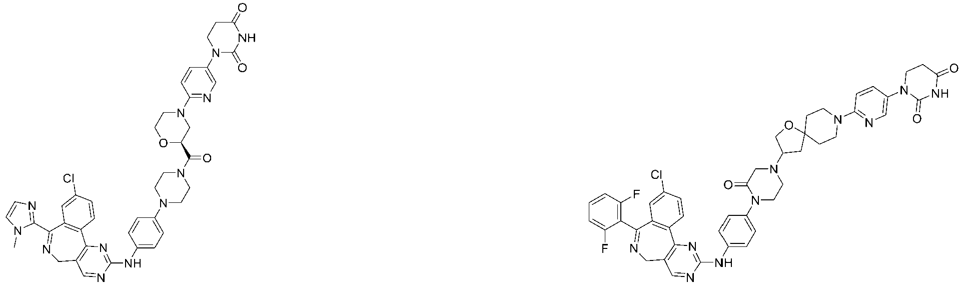

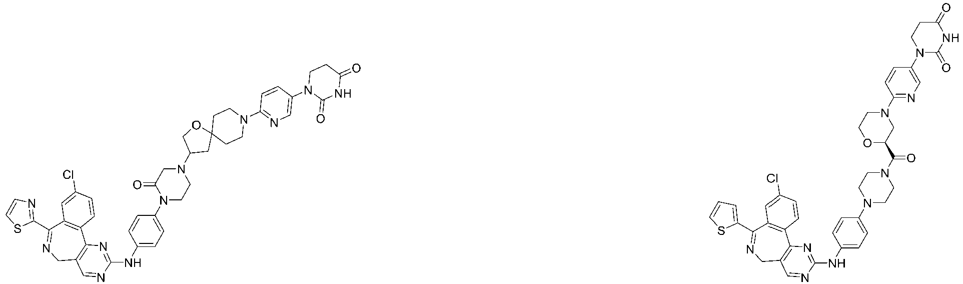

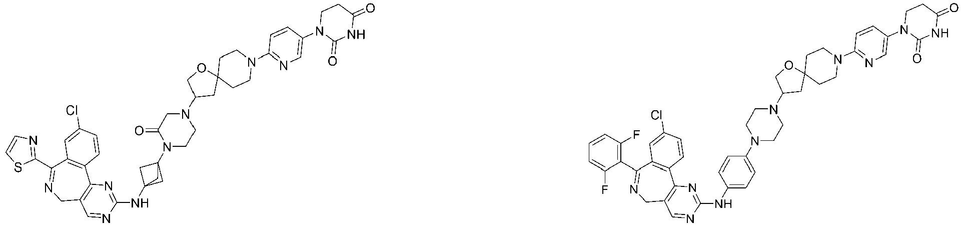

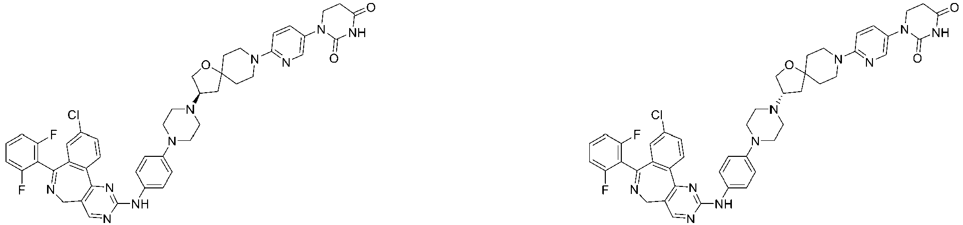

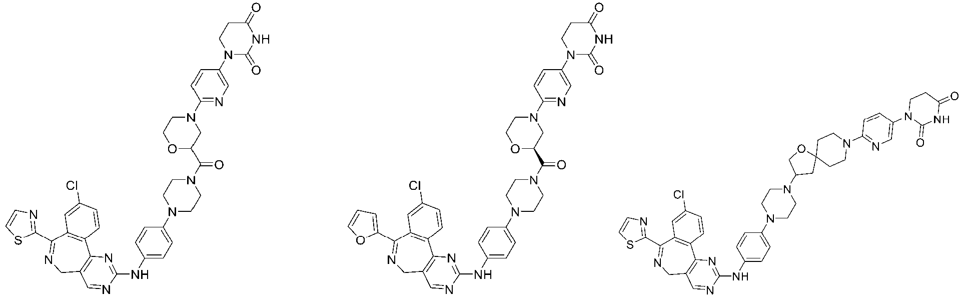

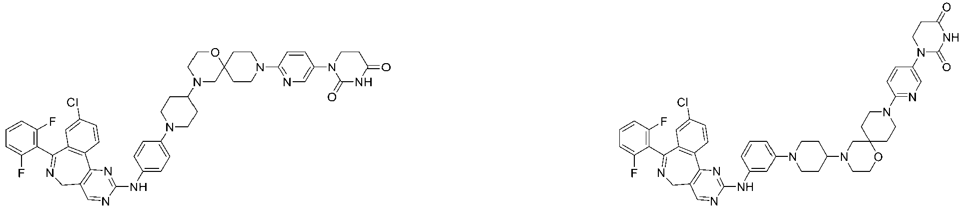

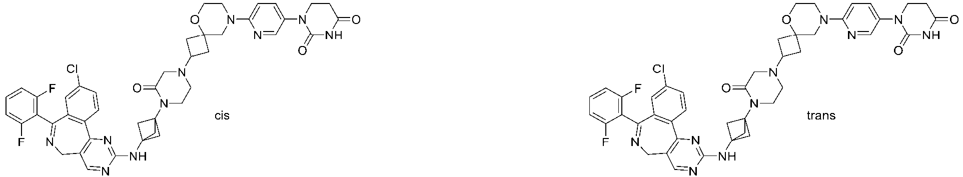

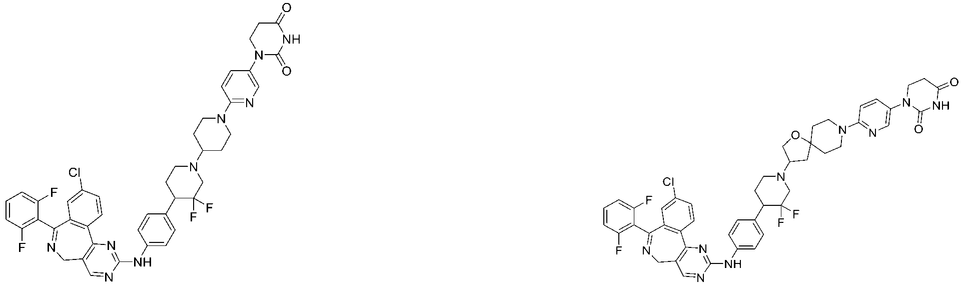

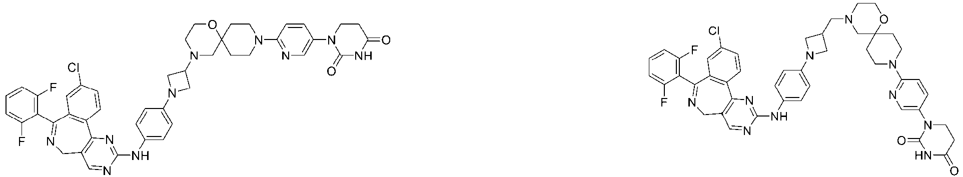

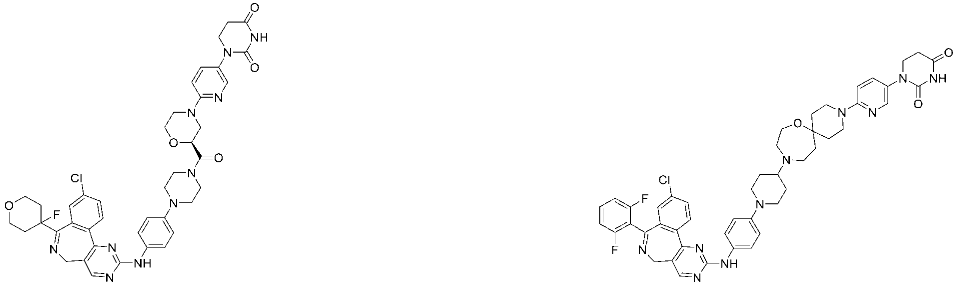

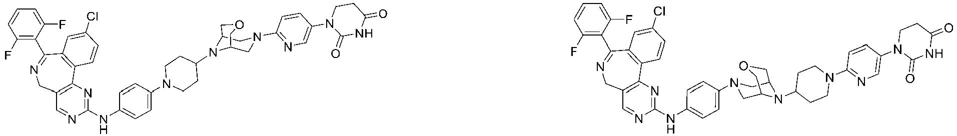

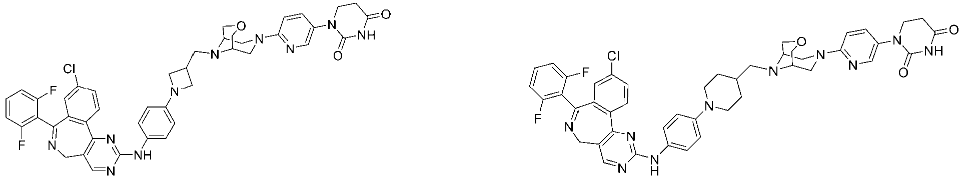

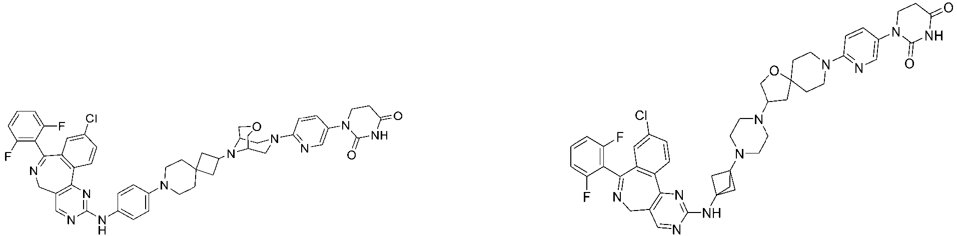

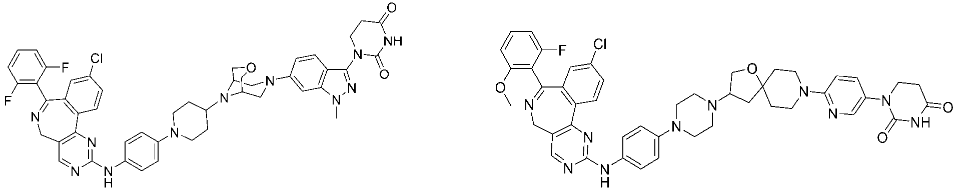

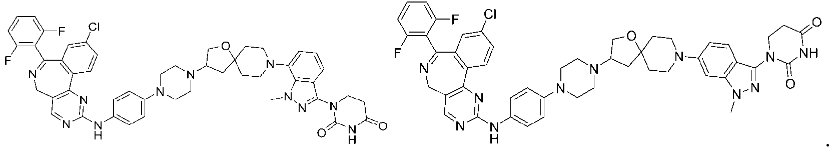

The present invention also provides a compound as shown below: 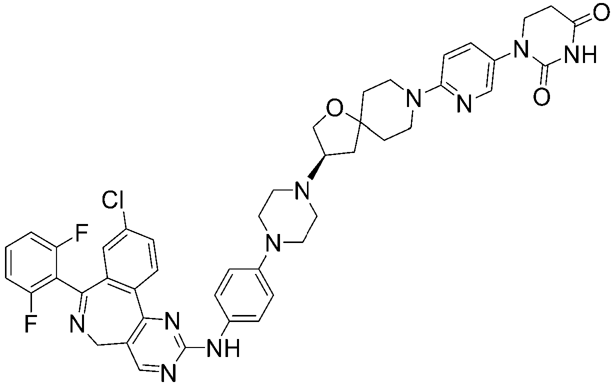 and 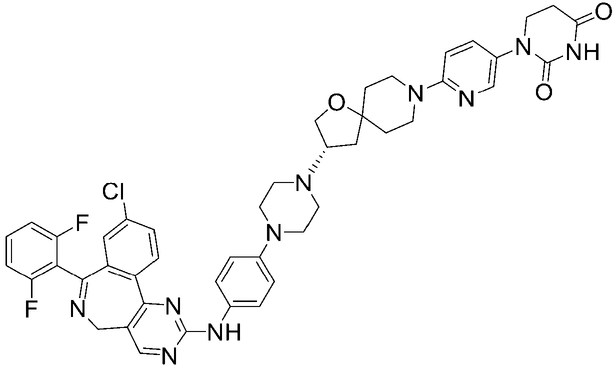 the compound with the first peak under the following conditions from: chromatographic column: CHIRALPAK IE-3, mobile phase: 0.1% diethylamine in acetonitrile: 0.1% diethylamine in isopropanol (40:60), flow rate: 1.0 mL/min; preferably, under the above conditions, the retention time of the compound with the first peak is 9.78 min; 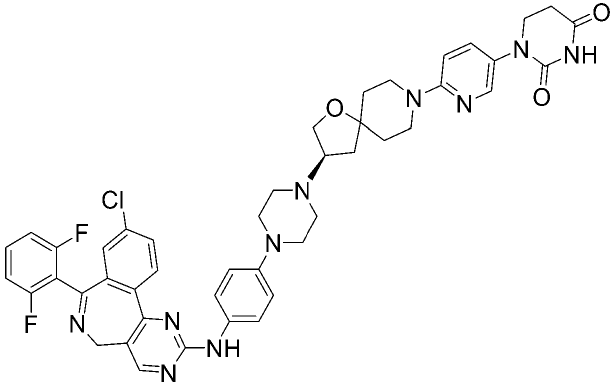 and 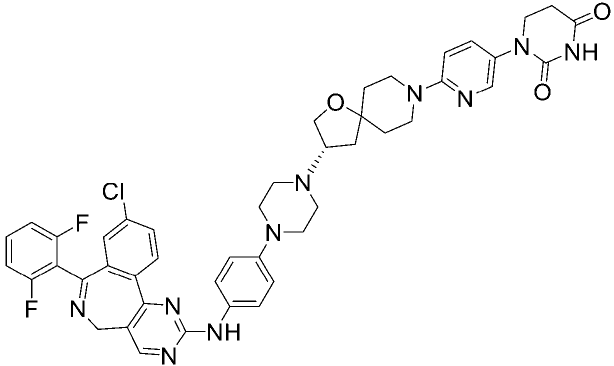 the compound with the later peak under the following conditions from: chromatographic column: CHIRALPAK IE-3, mobile phase: 0.1% diethylamine in acetonitrile: 0.1% diethylamine in isopropanol (40:60), flow rate: 1.0 mL/min; preferably, under the above conditions, the retention time of the compound with the later peak is 11.36 min.

The test conditions for above retention time are not a limitation on the compound. As long as the above test conditions are used for measurement, the obtained retention time is the same as or within the error range described above, and the compound is a stereoisomer in the above compounds defined by the retention time, the compound falls within the scope of protection of the present invention.

The present invention also provides a pharmaceutical composition, which comprises a compound represented by formula (I), a pharmaceutically acceptable salt thereof, a solvate thereof or a solvate of the pharmaceutically acceptable salt thereof as described in any of the above embodiments, and pharmaceutically acceptable excipients.

The present invention also provides use of a compound represented by formula (I), a pharmaceutically acceptable salt thereof, a solvate thereof, a solvate of a pharmaceutically acceptable salt thereof as described in any of the above embodiments, or an above-mentioned pharmaceutical composition in the preparation of AURKA inhibitors or degraders.

The present invention also provides use of a compound represented by formula (I), a pharmaceutically acceptable salt thereof, a solvate thereof, a solvate of a pharmaceutically acceptable salt thereof as described in any of the above embodiments, or an above-mentioned pharmaceutical composition in the preparation of drugs for treating and/or preventing diseases associated with AURKA. Preferably, the disease associated with AURKA is neuroblastoma, retinoblastoma, colorectal cancer, breast cancer, lung cancer, kidney cancer or blood cancer.

The present invention also provides use of a compound represented by formula (I), a pharmaceutically acceptable salt thereof, a solvate thereof, a solvate of a pharmaceutically acceptable salt thereof as described in any of the above embodiments, or an above-mentioned pharmaceutical composition in the preparation of drugs for treating and/or preventing diseases mediated by AURKA inhibitors or degraders. Preferably, the disease mediated by AURKA inhibitors or degraders is neuroblastoma, retinoblastoma, colorectal cancer, breast cancer, lung cancer, kidney cancer or blood cancer.

The present invention also provides use of a compound represented by formula (I), a pharmaceutically acceptable salt thereof, a solvate thereof, a solvate of a pharmaceutically acceptable salt thereof as described in any of the above embodiments, or an above-mentioned pharmaceutical composition in the preparation of drugs for treating and/or preventing neuroblastoma, retinoblastoma, colorectal cancer, breast cancer, lung cancer, kidney cancer or blood cancer.

The present invention also provides a substance X used as a drug, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition.

The present invention also provides a substance X for treatment, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition.

The present invention also provides a substance X for preventing and/or treating diseases associated with AURKA, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition. Preferably, the disease associated with AURKA is neuroblastoma, retinoblastoma, colorectal cancer, breast cancer, lung cancer, kidney cancer or blood cancer.

The present invention also provides a substance X for preventing and/or treating diseases mediated by AURKA inhibitors or degraders, wherein the substance X is a compound represented by formula (I), a pharmaceutically acceptable salt thereof, a solvate thereof, a solvate of a pharmaceutically acceptable salt thereof, or an above-mentioned pharmaceutical composition as described in any of the above embodiments. Preferably, the disease mediated by AURKA inhibitors or degraders is neuroblastoma, retinoblastoma, colorectal cancer, breast cancer, lung cancer, kidney cancer or blood cancer.

The present invention also provides a substance X for preventing and/or treating neuroblastoma, retinoblastoma, colorectal cancer, breast cancer, lung cancer, kidney cancer or blood cancer, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition.

The present invention also provides a method for treating an AURKA-related disease in a subject in need thereof, comprising administering to the subject a therapeutically effective amount of substance X, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition. Preferably, the AURKA-related disease is neuroblastoma, retinoblastoma, colorectal cancer, breast cancer, lung cancer, kidney cancer or blood cancer.

The present invention also provides a method for treating an AURKA inhibitor or degrader-mediated disease in a subject in need thereof, comprising administering to the subject a therapeutically effective amount of substance X, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition. Preferably, the disease mediated by the AURKA inhibitor or degrader is neuroblastoma, retinoblastoma, colorectal cancer, breast cancer, lung cancer, kidney cancer or blood cancer.

The present invention also provides a method for treating neuroblastoma, retinoblastoma, colorectal cancer, breast cancer, lung cancer, kidney cancer or blood cancer in a subject in need thereof, comprising administering to the subject a therapeutically effective amount of substance X, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition.

The present invention also provides a method for treating an AURKA-related disease in a non-human subject, comprising administering to the subject a therapeutically effective amount of substance X, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition. Preferably, the AURKA-related disease is neuroblastoma, retinoblastoma, colorectal cancer, breast cancer, lung cancer, kidney cancer or blood cancer.

The present invention also provides a method for treating an AURKA inhibitor or degrader-mediated disease in a non-human subject, comprising administering to the subject a therapeutically effective amount of substance X, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition. Preferably, the disease mediated by the AURKA inhibitor or degrader is neuroblastoma, retinoblastoma, colorectal cancer, breast cancer, lung cancer, kidney cancer or blood cancer.

The present invention also provides a method for treating neuroblastoma, retinoblastoma, colorectal cancer, breast cancer, lung cancer, kidney cancer or blood cancer in a non-human subject, comprising administering to the subject a therapeutically effective amount of substance X, wherein the substance X is a compound represented by formula (I), a pharmaceutically acceptable salt thereof, a solvate thereof, a solvate of a pharmaceutically acceptable salt thereof as described in any of the above embodiments, or an above-mentioned pharmaceutical composition.

In the above use, the AURKA inhibitor or degrader can be used in mammalian organisms; or used in vitro, mainly for experimental purposes, for example: as a standard sample or control sample to provide comparison, or made into a kit according to conventional methods in this field to provide detection for the inhibition or degradation effect of AURKA.

It can be understood by those skilled in the art that, according to the conventions used in the art, " 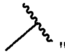 as used in the structural formula of a group described herein means that the corresponding group is linked to other fragments or groups in the compound through this site.

As used herein, a substituent may be preceded by a single dash "-" indicating that the named substituent is connected to a parent moiety by a single bond. When a linking direction of the linking group listed in the present invention is not specified, the linking direction is the same as the reading order from left to right.

The term "pharmaceutically acceptable salt" refers to a salt prepared from a compound of the present invention and a relatively non-toxic, pharmaceutically acceptable acid or base. When a compound of the present invention contains relatively acidic functional groups, base addition salts can be obtained by contacting a neutral form of such compound with a sufficient amount of a pharmaceutically acceptable base in a pure solution or in a suitable inert solvent. When a compound of the present invention contains relatively basic functional groups, acid addition salts can be obtained by contacting a neutral form of such compound with a sufficient amount of a pharmaceutically acceptable acid in a pure solution or in a suitable inert solvent.

The term "solvate" refers to a substance formed by combining a compound of the present invention with a stoichiometric or non-stoichiometric solvent. The solvent molecules in the solvate can exist in an ordered or non-ordered arrangement. The solvents include but are not limited to: water, methanol, ethanol, etc.

The "pharmaceutically acceptable salt" and "solvate" in the term "a solvate of a pharmaceutically acceptable salt" as described above refer to a substance prepared from a compound of the present invention with (1) a relatively non-toxic, pharmaceutically acceptable acid or base and with (2) a stoichiometric or non-stoichiometric solvent.

When any variable (for example, R²⁻³) appears multiple times in the definition of a compound, the definition of the variable appearing at each position is independent of the definitions of the variable appearing at the remaining positions. Their meanings are independent of each other and do not affect each other. Therefore, if a group is substituted by 1, 2 or 3 R²⁻³ groups, that is to say, the group may be substituted by up to 3 R²⁻³ groups, where the definition of R²⁻³ at a certain position is independent of the definition of R²⁻³ at the remaining positions. Additionally, combinations of substituents and/or variables are permitted only if such combinations result in stable compounds.

The term "A- or B-membered", wherein A and B are integers, describes a range in which the number of ring-forming atoms is from A to B.

The term "alkyl" refers to a linear or branched saturated hydrocarbon group having a specified number of carbon atoms. In some embodiments, the alkyl is C₁₋₆ alkyl, such as C₁₋₅ alkyl, C₁₋₄ alkyl, C₁₋₃ alkyl, C₁₋₂ alkyl, etc. Examples of alkyls include, but are not limited to, methyl, ethyl, n-propyl, isopropyl, n-butyl, tert-butyl, isobutyl, sec-butyl, n-pentyl, n-hexyl and similar alkyls.

The term "alkoxyl" refers to a group -O-R^{X}, wherein R^{X} is an alkyl as defined above.

The term "cycloalkyl" refers to a non-aromatic, saturated, or partially unsaturated monocyclic, fused, bridged or spirocyclic group whose ring atoms consist only of carbon atoms.

The term "cycloalkylene" refers to a group formed by the elimination of two hydrogen atoms from a non-aromatic, saturated or partially unsaturated monocyclic, fused, bridged or spirocyclic alkane whose ring atoms consist only of carbon atoms. The two eliminated hydrogen atoms can be on the same atom or on different atoms.

The term "carbon ring" meets any of the following conditions, and the remaining conditions are the same as those for the term "cycloalkyl": 1. The ring is connected to the rest of the molecule through two or more single bonds; 2. The ring shares two atoms and one bond with the rest of the molecule.

The term "heterocycloalkyl" refers to a non-aromatic, saturated, partially unsaturated, monocyclic, fused, bridged or spirocyclic group whose ring atoms contain at least 1 heteroatom independently selected from N, O, S and C(=O).

The term "heterocycloalkylene" refers to a group formed by eliminating two hydrogens from a non-aromatic, saturated, partially unsaturated, monocyclic, fused, bridged or spirocyclic alkane containing at least 1 heteroatom independently selected from N, O, S and C(=O) in the ring atoms, and the two eliminated hydrogen atoms may be from the same atom or from different atoms.

The term "heterocycle" meets any of the following conditions, and the remaining definitions are the same as those for the term "heterocycloalkyl": 1. The heterocycle is connected to the rest of the molecule through two or more single bonds; 2. The heterocycle shares two atoms and one bond with the rest of the molecule.

The term "halogen" refers to F, Cl, Br, and I.

In the present invention, the term "optional" refers to an event or condition described subsequently that may but is not required to occur, and the description includes a situation in which the event or condition occurs and a situation in which the event or condition does not occur. For example, the term "optionally substituted" means that the group can be substituted or not, unless otherwise specified, and the type and number of substituents can be arbitrary on a chemically achievable basis.

The term "aryl" refers to a cyclic aromatic group having a specified number of carbon atoms, which is monocyclic or fused, and for fused rings, each ring has aromaticity.

The term "arylene" refers to a divalent aryl, and the definition of "aryl" is as described above.

The term "heteroaryl" refers to a cyclic aromatic group having a specified number of ring atoms, a specified number of heteroatoms (e.g. 1, 2 or 3), and a specified type of heteroatom (1, 2 or 3 of N, O and S), which is monocyclic or fused, and for fused rings, each ring has aromaticity.

The term "heteroarylene" refers to a divalent heteroaryl, and the definition of "heteroaryl" is as described above.

The term "heteroaryl ring" meets any of the following conditions, and the remaining conditions are the same as those for the term "heteroaryl": 1. The ring is connected to the rest of the molecule through two or more single bonds; 2. The ring shares two atoms and one bond with the rest of the molecule.

The term "pharmaceutically acceptable excipients" refers to the vehicles and additives used in the production of drugs and formulations, which are all substances contained in drug preparations except active ingredients. Refer to Volume IV, Chinese Pharmacopoeia (2020 Edition) or Handbook of Pharmaceutical Excipients (Raymond C Rowe, 2009 Sixth Edition).

The term "treatment" refers to therapeutic treatments. When it comes to specific conditions, treatment refers to: (1) alleviating one or more biological manifestations of a disease or condition, (2) interfering with (a) one or more points in the biological cascade that causes or contributes to the condition or (b) one or more biological manifestations of the condition, (3) improving one or more symptoms, effects or side effects associated with the condition or its treatment, or (4) slowing down the development of the condition or one or more biological manifestations of the condition.

The term "prevention" refers to the reduction in the risk of acquiring or developing a disease or disorder.

The term "therapeutically effective amount" means an amount of a compound sufficient to effectively treat the disease or condition described herein when administered to a subject. The "therapeutically effective amount" varies depending on the compound, the condition and its severity, and the age of the subject to be treated, but can be adjusted as needed by those skilled in the art.

Without departing from common knowledge in the art, the above preferred conditions can be arbitrarily combined to obtain the preferred examples of the present invention.

The reagents and raw materials used in the present invention are commercially available.

The positive and progressive effects of the present invention are: the compounds of the present invention have good inhibitory activity against AURKA (the IC₅₀ of the example compounds in HCC1806 cells or H82 cells is preferably below 1000 nM) and/or degradation activity (the percentage of AURKA degradation by the example compounds in Jurkat cells at a concentration of 1 µM or 100 nM is preferably above 70%).

### DETAILED DESCRIPTION

The present invention is further described through the following specific Examples, which are not intended to limit the scope of the present invention to these Examples. Experimental methods with no specific conditions indicated in the following Examples should be selected according to conventional methods and conditions, or product instructions.

### Example 1 Synthesis of Compound A1:

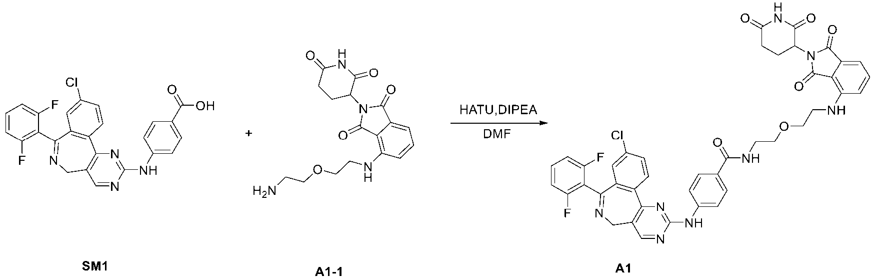

### Step 1:

At room temperature, **SM1** (15 mg, 0.03 mmol, synthesized with reference to ACS Medicinal Chemistry Letters (2015), 6(6), 630-634), HATU (14 mg, 0.04 mmol) and DIPEA (12.2 mg, 0.09 mmol) were added to a solution of compound **A1-1** (20 mg, 0.055 mmol) in N,N-dimethylformamide (2 mL). The reaction mixture was stirred at room temperature for 2 h. The reaction mixture was separated for purification by reverse phase chromatography (acetonitrile/0.1% aqueous formic acid solution) to give a light yellow solid **A1** (10.6 mg, yield 43%). LCMS (ESI) m/z = 819.4 [M+H]⁺. ¹H NMR (400 MHz, DMSO-*d*₆) δ 11.07 (s, 1H), 10.14 (s, 1H), 8.72 (s, 1H), 8.34 - 8.28 (m, 2H), 7.90 - 7.86 (m, 3H), 7.81 (d, *J =* 8.8 Hz, 2H), 7.59 - 7.51 (m, 2H), 7.33 (d, *J* = 4.0 Hz, 1H), 7.21 - 7.10 (m, 3H), 7.03 (d, *J* = 4.0 Hz, 1H), 6.63 (t, *J =* 6.0 Hz, 1H), 5.07 - 5.01 (m, 1H), 3.65 (t, *J =* 5.6 Hz, 2H), 3.59 (t, *J =* 6.0 Hz, 2H), 3.52 - 3.40 (m, 4H), 2.92 - 2.80 (m, 1H), 2.69 - 2.54 (m, 2H), 2.04 - 1.96 (m, 1H).

### Example 2 Synthesis of Compound A2:

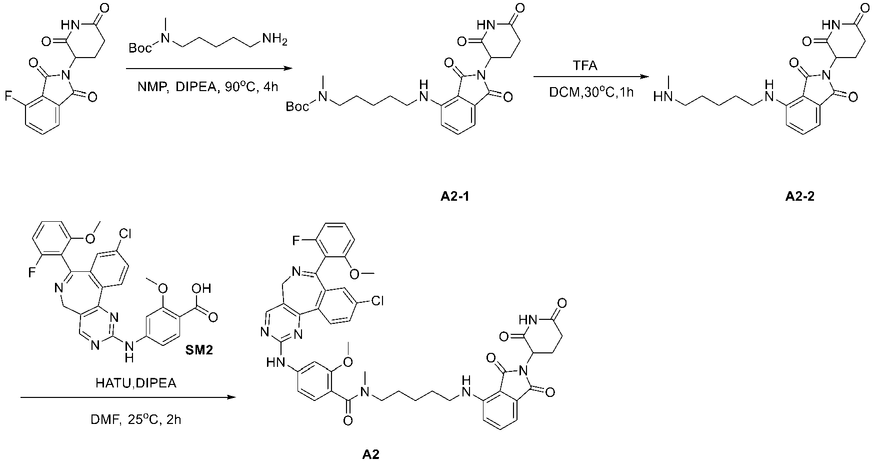

### Step 1:

At room temperature, tert-butyl (5-aminopentyl)(methyl)carbamate (86 mg, 0.4 mmol) and DIPEA (93 mg, 0.72 mmol) were added to a solution of compound 2-(2,6-dioxo-piperidin-3-yl)-4-fluoro-isoindole-1,3-dione (100 mg, 0.36 mmol) in N-methylpyrrolidone (5 mL). The reaction mixture was heated to 90°C under argon protection and stirred for 4 h. After the addition of water, the reaction mixture was extracted with ethyl acetate, dried over anhydrous sodium sulfate, and concentrated under reduced pressure. The residue was separated for purification by silica gel column chromatography (methanol/dichloromethane = 1:30) to give a yellow solid **A2-1** (120 mg, yield 70%). LCMS (ESI) m/z = 473.1 [M+H]⁺.

### Step 2:

Trifluoroacetic acid (0.5 mL) was added to a solution of compound **A2-1** (30 mg, 0.06 mmol) in dichloromethane (2 mL) at room temperature. The reaction mixture was stirred at 30°C for 1 h. The reaction mixture was concentrated under reduced pressure to give a light yellow solid **A2-2** (23 mg, yield 99%). LCMS (ESI) m/z = 373.1 [M+H]⁺.

### Step 3:

**SM2** (15 mg, 0.03 mmol, synthesized with reference to WO2008063525A1), HATU (15.5 mg, 0.04 mmol) and DIPEA (12.2 mg, 0.09 mmol) were added to a solution of compound **A2-2** (23 mg, 0.06 mmol) in *N,N*-dimethylformamide (2 mL) at room temperature. The reaction mixture was stirred at room temperature for 2 h. The reaction mixture was separated for purification by reverse phase chromatography (acetonitrile/0.1% aqueous formic acid solution) to give a light yellow solid **A2** (11.4 mg, yield 43.5%). LCMS (ESI) m/z = 873.1 [M+H]⁺. ¹H NMR (400 MHz, DMSO-*d*₆) δ 11.10 (s, 1H), 10.02 (d, *J =* 12.0 Hz, 1H), 8.70 (d, *J =* 4.0 Hz, 1H), 8.31 (t, *J =* 8.4 Hz, 1H), 7.88 (s, 1H), 7.84 - 7.79 (m, 1H), 7.67 - 7.34 (m, 3H), 7.25 - 6.96 (m, 3H), 6.64 - 6.44 (m, 1H), 5.14 - 5.01 (m, 1H), 3.81 (d, *J* = 4.0 Hz, 3H), 3.52 - 3.39 (m, 1H), 3.26 - 3.04 (m, 2H), 2.98 - 2.76 (m, 4H), 2.65 - 2.54 (m, 2H), 2.10 - 1.96 (m, 1H), 1.71 - 1.60 (m, 2H), 1.55 - 1.39 (m, 3H), 1.21 - 1.13 (m, 1H).

### Example 3 Synthesis of Compound A3:

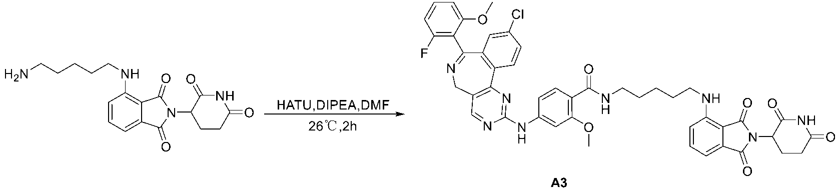

Compound **A3** (6.0 mg, yield 27%) was synthesized according to the synthesis method of compound **A1** described in **Example 1.** LCMS (ESI) m/z =859.1 [M+H]⁺.

### Example 4 Synthesis of Compound A4:

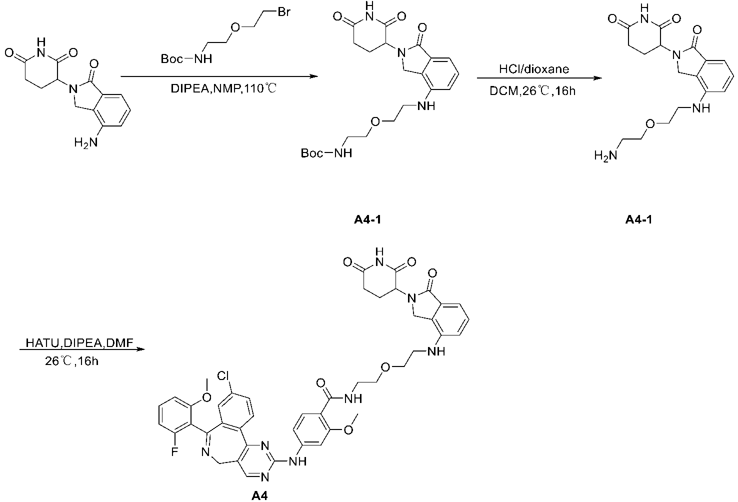

At room temperature, N-Boc-2-(2-bromoethoxy)ethylamine (113.8 mg, 0.40 mmol) was added to a solution of compound lenalidomide (100.0 mg, 0.43 mmol) in N-methylpyrrolidone and DIPEA (3 mL/0.2 mL), and the reaction mixture was heated to 110°C and stirred for 5 h. The reaction mixture was washed with water, and extracted with ethyl acetate. The organic phase was concentrated to remove the solvent, and the residue was separated for purification by silica gel column chromatography (petroleum ether/ethyl acetate = 2:1) to give a white solid **A4-1** (100.0 mg, yield 90%). LCMS (ESI) m/z = 447.21 [M+H]⁺.

### Step 2:

HCl (4.0 M in 1,4-dioxane) was added to a dichloromethane solution (3 mL) of compound **A4-1** (100.0 mg, 0.20 mmol), and the reaction mixture was stirred at room temperature for 16 h. The reaction mixture was directly spin-dried to give a yellow solid **A4-2** (60.0 mg, yield 77%). LCMS (ESI) m/z = 347.13 [M+H]⁺.

### Step 3:

To a solution of compound **A4-2** (20.0 mg, 0.052 mmol) in N,N-dimethylformamide (2 mL), SM2 (15.0 mg, 0.026 mmol), HATU (15.0 mg, 0.039 mmol) and DIPEA (10.1 mg, 0.078 mmol) were added. The reaction mixture was stirred at room temperature for 2 h. The reaction mixture was separated for purification by reverse phase chromatography (acetonitrile/0.1% aqueous formic acid solution = 2/1) to give a yellow solid **A4** (5.0 mg, yield 20%). LCMS (ESI) m/z = 847.2 [M+H]⁺. ¹H NMR (400 MHz, DMSO-*d*₆) δ 11.22 (s, 1H), 10.40 (s, 1H), 8.73 (s, 1H), 8.33 (d, *J* = 8.4 Hz, 1H), 8.15 (d, *J =* 5.6 Hz, 1H), 7.96 (s, 1H), 7.88 (d, *J =* 8.4 Hz, 1H), 7.85 (dd, *J*₁ = 8.4 Hz, *J*₁ = 2.0 Hz, 1H), 7.45 - 7.41 (m, 3H), 7.31 (d, *J =* 7.6 Hz, 1H), 6.96 (d, *J =* 7.2 Hz, 1H), 6.88 (d, *J =* 8.0 Hz, 1H), 5.64 (t*, J* = 5.6 Hz, 1H), 5.12 (dd, *J*₁ = 13.2 Hz, *J*₂ = 5.2 Hz, 1H), 4.43 - 4.30 (m, 2H), 3.91 (s, 3H), 3.69 - 3.66 (m, 2H), 3.62-3.60 (m, 2H), 3.53 - 3.48 (m, 2H), 3.40-3.37 (m, 2H), 2.97-2.89 (m ,1H), 2.69-2.60 (m, 2H), 2.35-2.27 (m, 2H), 2.06 - 2.03 (m, 1H).

### Example 5 Synthesis of Compound A5:

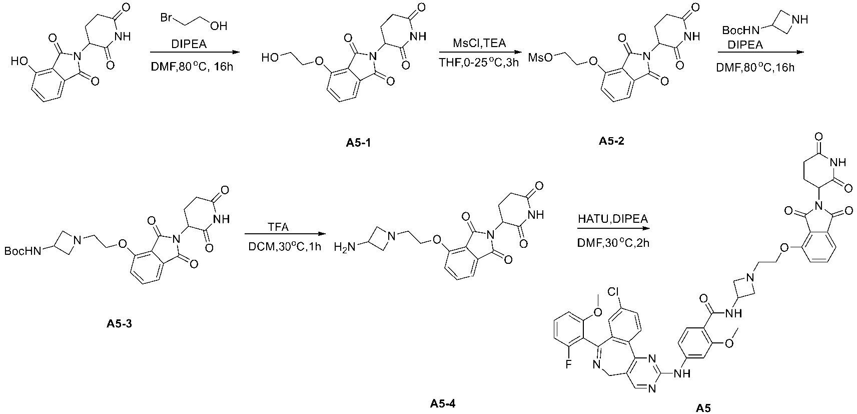

### Step 1:

At room temperature, 2-bromoethanol (912 mg, 7.3 mmol) and DIPEA (2.8 g, 21.9 mmol) were added to a solution of 2-(2,6-dioxo-piperidin-3-yl)-4-hydroxy-isoindole-1,3-dione (2 g, 7.3 mmol) in *N,N-*dimethylformamide (10 mL). The reaction mixture was heated to 80°C under argon protection and stirred for 16 h. After the addition of water, the reaction mixture was extracted with ethyl acetate, dried over anhydrous sodium sulfate, and concentrated under reduced pressure. The residue was separated for purification by silica gel column chromatography (methanol/dichloromethane = 1:30) to give a yellow oil **A5-1** (620 mg, yield 27%). LCMS (ESI) m/z = 319.1 [M+H]⁺.

### Step 2:

To a solution of compound **A5-1** (620 mg, 1.95 mmol) in tetrahydrofuran (10 mL) in an ice bath, methanesulfonyl chloride (290 mg, 2.54 mmol) and DIPEA (756 mg, 5.85 mmol) were added. The reaction mixture was stirred at 25°C for 3 h. After the addition of water, the reaction mixture was extracted with ethyl acetate, dried over anhydrous sodium sulfate, and concentrated under reduced pressure. The residue was separated for purification by silica gel column chromatography (methanol/dichloromethane = 1:30) to give a yellow oil **A5-2** (465 mg, yield 60%). LCMS (ESI) m/z = 397.1 [M+H]⁺.

### Step 3:

At room temperature, 3-*N*-tert-butoxycarbonylaminocyclobutylamine (135 mg, 0.78 mmol) and DIPEA (302 mg, 2.34 mmol) were added to a solution of compound **A5-2** (310 mg, 0.78 mmol) in *N,N-*dimethylformamide (5 mL). The reaction mixture was stirred at 80°C for 16 h. After the addition of water, the reaction mixture was extracted with ethyl acetate, dried over anhydrous sodium sulfate, and concentrated under reduced pressure. The residue was separated for purification by silica gel column chromatography (methanol/dichloromethane = 1:20) to give a yellow oil **A5-3** (96 mg, yield 26%). LCMS (ESI) m/z = 473.2 [M+H]⁺.

### Step 4:

Trifluoroacetic acid (0.5 mL) was added to a solution of compound **A5-3** (35 mg, 0.07 mmol) in dichloromethane (2 mL) at room temperature. The reaction mixture was stirred at 30°C for 1 h. The reaction mixture was concentrated under reduced pressure to give a light yellow solid **A5-4** (28 mg, yield 99%). LCMS (ESI) m/z = 373.1 [M+H]⁺.

### Step 5:

**SM2** (15 mg, 0.03 mmol), HATU (14 mg, 0.04 mmol) and DIPEA (12.2 mg, 0.09 mmol) were added to a solution of compound **A5-4** (28 mg, 0.07 mmol) in *N,N*-dimethylformamide (2 mL) at room temperature. The reaction mixture was stirred at room temperature for 2 h. The reaction mixture was separated for purification by reverse phase chromatography (acetonitrile/0.1% aqueous formic acid solution) to give a light yellow solid **A5** (11.3 mg, yield 43%). LCMS (ESI) m/z = 872.9 [M+H]⁺. ¹H NMR (400 MHz, DMSO-*d*₆) δ 11.08 (s, 1H), 10.18 (s, 1H), 8.72 (s, 1H), 8.33 (d, *J* = 8.0 Hz,1H), 8.27 (d, *J* = 8.0 Hz, 1H), 7.94 (s, 1H), 7.85 - 7.78 (m, 2H), 7.74 (d, *J =* 8.0 Hz, 1H), 7.51 (d, *J =* 8.0 Hz, 1H), 7.47 - 7.39 (m, 3H), 7.22 (bs, 1H), 5.12 - 5.06 (m, 1H), 4.49 (q, *J* = 6.8 Hz, 1H), 4.21 (t, *J* = 8.0 Hz, 2H), 3.93 (s, 3H), 3.70 (t, *J* = 8.0 Hz, 2H), 3.14 (t, *J =* 7.2 Hz, 2H), 2.93 - 2.78 (m, 3H), 2.68 - 2.53 (m, 2H), 2.07 - 1.98 (m, 1H).

### Example 6 Synthesis of Compound A6:

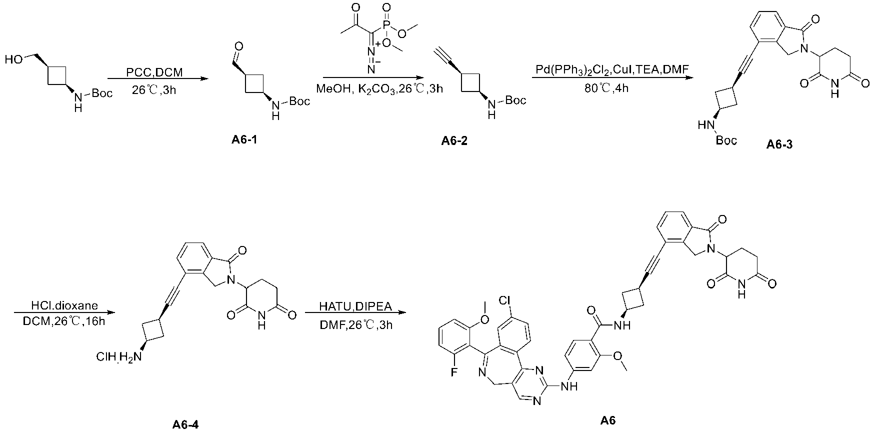

### Step 1:

At room temperature, pyridinium chlorochromate (2.60 g, 11.60 mmol) was added to a solution of compound tert-butyl (cis-3-(hydroxymethyl)cyclobutyl)carbamate (2.22 g, 9.94 mmol) in dichloromethane (20 mL), and the reaction mixture was stirred at room temperature for 3 h. The reaction mixture was washed with water, and extracted with ethyl acetate. The organic phase was concentrated to remove the solvent, and the residue was separated for purification by silica gel column chromatography (petroleum ether/ethyl acetate = 2:1) to give a white solid **A6-1** (1.20 g, yield 54%). LCMS (ESI) m/z = 200.1 [M+H]⁺.

### Step 2:

To a solution of compound **A6-1** (842 mg, 3.80 mmol) in methanol (10 mL), dimethyl (1-diazo-2-oxopropyl)phosphonate (1.22 g, 5.71 mmol) and potassium carbonate (1.06 g, 7.61 mmol) were added, and the mixture was stirred at room temperature for 3 h. The reaction mixture was washed with water, and extracted with ethyl acetate. The organic phase was concentrated to remove the solvent, and the residue was separated for purification by silica gel column chromatography (petroleum ether/ethyl acetate = 10:1) to give a white solid **A6-2** (500.0 mg, yield 57%). LCMS (ESI) m/z = 196.2 [M+H]⁺.

### Step 3:

Compound **A6-2** (55.5 mg, 0.26 mmol), TEA (55.0 mg, 0.52 mmol), CuI (10.0 mg, 0.052 mmol) and Pd(PPh₃)₂Cl₂ (19.6 mg, 0.026 mmol) were dissolved in *N,N*-dimethylformamide (5 mL) at room temperature. The reaction mixture was stirred at 80°C for 4 h. The reaction mixture was washed with water, and extracted with ethyl acetate. The organic phase was concentrated to remove the solvent, and the residue was separated for purification by silica gel column chromatography (petroleum ether/ethyl acetate = 10:1) to give a white solid **A6-3** (50.0 mg, yield 57%). LCMS (ESI) m/z = 438.1 [M+H]⁺.

### Step 4:

HCl (4.0 M in 1,4-dioxane, 2 mL) was added to a dichloromethane solution (2 mL) of compound A6-3 (50.0 mg, 0.10 mmol), and the reaction mixture was stirred at room temperature for 16 h. The reaction mixture was directly spin-dried to give a white solid **A6-4** (30.5 mg, yield 86%). LCMS (ESI) m/z = 338.2 [M+H]⁺.

### Step 5:

To a solution of compound **A6-4** (29.0 mg, 0.077 mmol) in *N,N*-dimethylformamide (1 mL), **SM2** (22.0 mg, 0.039 mmol), HATU (22.6 mg, 0.11 mmol) and DIPEA (15.3 mg, 0.11 mmol) were added. The reaction mixture was stirred at room temperature for 3 h. The reaction mixture was separated for purification by reverse phase chromatography (acetonitrile/0.1% aqueous formic acid solution = 2.3/1) to give a yellow solid **A6** (8.0 mg, yield 23%). LCMS (ESI) m/z = 838.2 [M+H]⁺. ¹H NMR (400 MHz, DMSO-*d*₆) δ 11.04 (s, 1H), 10.42 (s, 1H), 8.77 (s, 1H), 8.33 - 8.24 (m, 2H), 7.96 (s, 1H), 7.74 (dd, *J*₁ = 8.4 Hz, *J*₂ = 2.4 Hz, 1H), 7.80 - 7.60 (m, 3H), 7.56 (d, *J =* 7.6 Hz, 1H), 7.49 - 7.44 (m, 2H), 7.24 (s, 1H), 5.20 - 5.15 (m, 1H), 4.75 (d, *J =* 7.6 Hz, 1H), 4.56 - 4.36 (m, 2H), 3.96 (s, 3H), 3.10 - 2.95 (m, 2H), 2.70 - 2.63 (m, 4H), 2.35 - 2.29 (m, 3H).

### Example 7 Synthesis of Compound A7:

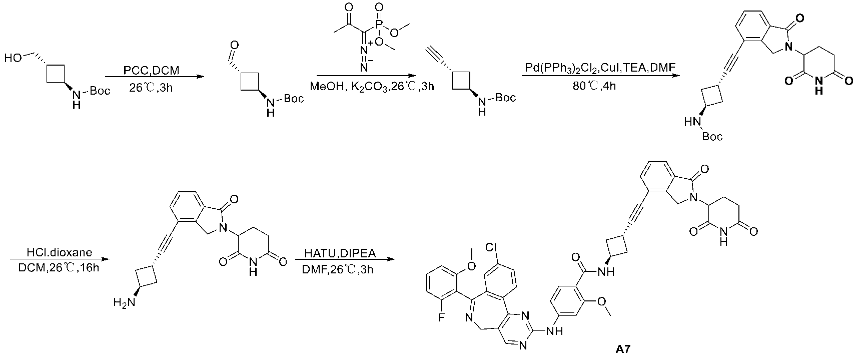

Compound **A7** (6.0 mg, yield 13%) was synthesized according to the synthesis method of compound A6 described in **Example 6.** LCMS (ESI) m/z = 838.2 [M+H]⁺. ¹H NMR (400 MHz, DMSO-*d*₆) δ 11.04 (s, 1H), 10.42 (s, 1H), 8.77 (s, 1H), 8.32 (d, *J =* 8.0 Hz, 1H), 8.27 (d, *J =* 8.0 Hz, 1H ), 7.96 (s, 1H), 7.74 (dd, *J*₁ = 8.4 Hz, *J*₂ =2.4 Hz, 1H), 7.80 - 7.60 (m, 3H), 7.56 (d, *J* = 7.6 Hz, 1H), 7.49 - 7.44 (m, 2H), 7.24 (s, 1H), 5.20 - 5.15 (m, 2H), 4.75 (d, *J =* 7.6 Hz, 1H), 4.56 - 4.36 (m, 2H), 3.96 (s, 3H), 3.04 - 2.92 (m, 2H), 2.72 - 2.60 (m, 4H), 2.35 - 2.29 (m, 3H).

### Example 8 Synthesis of Compound A8:

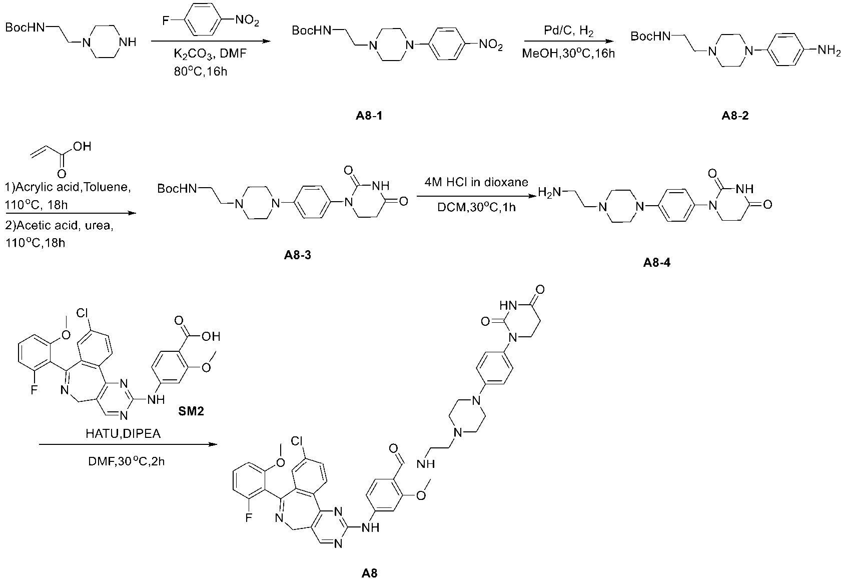

### Step 1:

At room temperature, 4-nitrofluorobenzene (5 g, 35.4 mmol) and potassium carbonate (9.8 g, 70.8 mmol) were added to a solution of compound 1-(N-Boc-aminoethyl)piperazine (10 g, 43.6 mmol) in *N,N-*dimethylformamide (50 mL). The reaction mixture was heated to 80°C under argon protection and stirred for 16 h. After the addition of water, the reaction mixture was extracted with ethyl acetate, dried over anhydrous sodium sulfate, concentrated under reduced pressure, and the residue was separated for purification by silica gel column chromatography (ethyl acetate/petroleum ether = 1:2) to give a yellow solid **A8-1** (10.2 g, yield 82%). LCMS (ESI) m/z = 350.8 [M+H]⁺.

### Step 2:

At room temperature, 10% palladium on carbon (910 mg, 1.71 mmol) was added to a solution of compound **A8-1** (3 g, 8.56 mmol) in methanol (30 mL), the atmosphere was replaced with hydrogen 3 times, and the reaction mixture was stirred at 30°C for 16 h. The reaction mixture was filtered and concentrated under reduced pressure to give a brown solid **A8-2** (2.7 g, yield 98%). LCMS (ESI) m/z = 321.1 [M+H]⁺.

### Step 3:

Acrylic acid (910 mg, 12.6 mmol) was added to a solution of compound **A8-2** (2.7 g, 8.43 mmol) in toluene (30 mL) at room temperature. The reaction mixture was heated to 110°C under argon protection and stirred for 18 h. The mixture was cooled to room temperature, and urea (2.53 g, 42.2 mmol) and acetic acid (30 mL) were added. The mixture was heated to 110°C and stirred for 18 h. After the reaction was completed, the solvent was removed by concentration under reduced pressure. Ethyl acetate and saturated aqueous sodium bicarbonate solution were added for extraction, and the extract was dried over anhydrous sodium sulfate and concentrated under reduced pressure. The residue was separated for purification by silica gel column chromatography (methanol/dichloromethane = 1:20) to give a white solid A8-3 (617 mg, yield 18%). LCMS (ESI) m/z = 418.1 [M+H]⁺.

### Step 4:

HCl (4 M in dioxane, 0.5 mL) was added to a solution of compound **A8-3** (30 mg, 0.07 mmol) in dichloromethane (2 mL) at room temperature. The reaction mixture was stirred at 30°C for 1 h. The reaction mixture was concentrated under reduced pressure to give a light yellow solid **A8-4** (23 mg, yield 99%). LCMS (ESI) m/z = 318.1 [M+H]⁺.

### Step 5:

**SM2** (15 mg, 0.03 mmol), HATU (14 mg, 0.04 mmol) and DIPEA (12.2 mg, 0.09 mmol) were added to a solution of compound **A8-4** (23 mg, 0.08 mmol) in N,N-dimethylformamide (2 mL) at room temperature. The reaction mixture was stirred at room temperature for 2 h. The reaction mixture was separated for purification by reverse phase chromatography (acetonitrile/0.1% aqueous formic acid solution) to give a light yellow solid **A8** (12.1 mg, yield 46%). LCMS (ESI) m/z = 818.5 [M+H]⁺. ¹H NMR (400 MHz, DMSO-*d*₆) δ 10.24 (s, 1H), 10.17 (s, 1H), 8.71 (s, 1H), 8.33 - 8.27 (m, 2H), 7.97 (s, 1H), 7.89 (d, *J =* 8.0 Hz, 1H), 7.82 (dd, *J*₁ = 8.0 Hz, *J*₂ = 2.0 Hz, 1H), 7.45 - 7.38 (m, 2H), 7.21 (s, 1H), 7.18 - 7.14 (m, 2H), 6.99 - 6.93 (m, 2H), 3.94 (s, 3H), 3.70 (t, *J* = 4.0 Hz, 2H), 3.50-3.46 (m, 2H), 3.21-3.19 (m, 4H), 2.68 (t, *J* = 4.0 Hz, 2H), 2.64 - 2.58 (m, 4H), 2.56 (t, *J =* 4.0 Hz, 2H).

### Example 9 Synthesis of Compound A9:

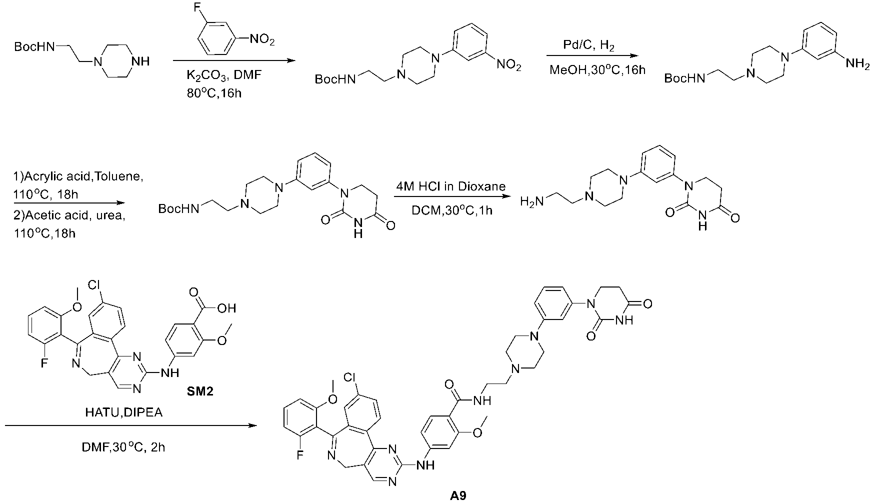

Compound **A9** (9.8 mg, yield 39.9%) was synthesized according to the synthesis method of compound **A8** described in **Example 8.** LCMS (ESI) m/z = 818.5 [M+H]⁺. ¹H NMR (400 MHz, DMSO-*d*₆) δ 10.29 (s, 1H), 10.18 (s, 1H), 8.71 (s, 1H), 8.32 - 8.27 (m, 2H), 7.99 - 7.95 (m, 1H), 7.88 (d, *J =* 8.0 Hz, 1H), 7.82 (dd, *J*₁ = 8.0 Hz, *J*₂ =2.0 Hz, 1H), 7.46 - 7.38 (m, 2H), 7.21 (t, *J =* 8.0 Hz, 2H), 6.91 (d, *J* = 4.0 Hz, 1H), 6.86 - 6.82 (m, 1H), 6.75 - 6.70 (m, 1H), 3.94 (s, 3H), 3.75 (t, *J =* 8.0 Hz, 2H), 3.50 - 3.43 (m, 2H), 3.23 - 3.16 (m, 4H), 2.68 (t, *J* = 6.4 Hz, 2H), 2.63 - 2.58 (m, 4H), 2.56 (d, *J =* 8.0 Hz, 2H).

### Example 10 Synthesis of Compound A10:

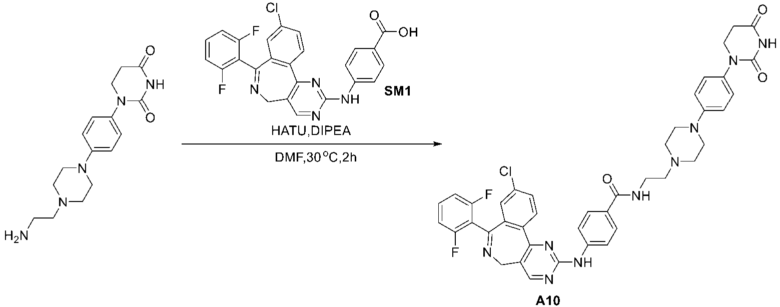

Compound **A10** (16.7 mg, yield 72%) was synthesized according to the synthesis method of compound **A1** described in **Example 1.** LCMS (ESI) m/z = 776.2 [M+H]⁺. ¹H NMR (400 MHz, DMSO-*d*₆) δ 10.25 (s, 1H), 10.15 (s, 1H), 8.72 (s, 1H), 8.31 - 8.25 (m, 2H), 7.92 - 7.81 (m, 5H), 7.58 - 7.51 (m, 1H), 7.33 (d, *J =* 4.0 Hz, 1H), 7.22 - 7.12 (m, 4H), 6.93 (d, *J =* 8.0 Hz, 2H), 3.69 (t, *J =* 8.0 Hz, 2H), 3.42 (q, *J =* 6.4 Hz, 2H), 3.14 (t, *J =* 4.0 Hz, 4H), 2.68 (t, *J* = 6.4 Hz, 2H), 2.62 - 2.56 (m, 4H), 2.55 - 2.52 (m, 2H).

### Example 11 Synthesis of Compound A11:

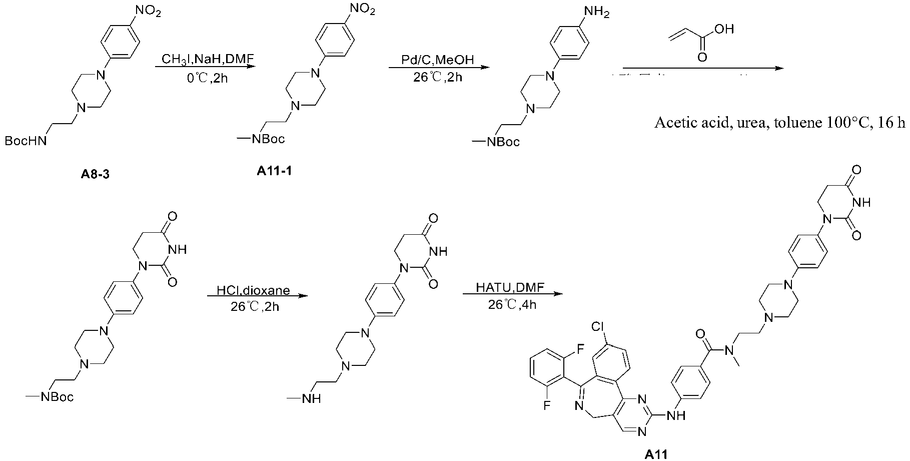

### Step 1:

At room temperature, sodium hydride (137.0 mg, 3.42 mmol) was added to a solution of compound **A8-3** (1.11 g, 2.85 mmol) in N,N-dimethylformamide (10 mL) at 0°C, and the reaction mixture was stirred continuously for 0.5 h. Then, methyl iodide (388.7 mg, 2.71 mmol) was added. The reaction mixture was stirred at room temperature for 2 h. The reaction mixture was washed with water, and extracted with ethyl acetate. The organic phase was concentrated to remove the solvent, and the residue was separated for purification by silica gel column chromatography (petroleum ether/ethyl acetate = 5:1) to give a white solid **A11-1** (600.0 mg, yield 51%). LCMS (ESI) m/z = 365.2 [M+H]⁺.

### Step 2:

Compound **A11** (5.6 mg, yield 26%) was synthesized according to the synthesis method of compound **A8** described in **Example 8.** LCMS (ESI) m/z = 790.2 [M+H]⁺. ¹H NMR (400 MHz, DMSO-*d*₆) δ 10.27 (s, 1H), 10.11 (s, 1H), 8.73 (s, 1H), 8.32 (d, *J =* 8.4 Hz, 1H), 7.91 (d, *J* = 8.4 Hz, 2H), 7.87 (d, *J =* 8.4 Hz, 1H), 7.60 - 7.53 (m, 1H), 7.42 (d, *J =* 8.0 Hz, 2H), 7.35 (d, *J =* 2.4 Hz, 1H), 7.17 - 7.10 (m, 4H), 6.94 (bs, 2H), 3.71 (t, *J* = 7.2 Hz, 2H), 3.62 - 3.51 (m, 2H), 3.12 - 3.01 (m, 4H), 2.98 (s, 3H), 2.70 (d, *J =* 5.9 Hz, 2H), 2.69 - 2.59 (m, 6H).

### Example 12 Synthesis of Compound A12:

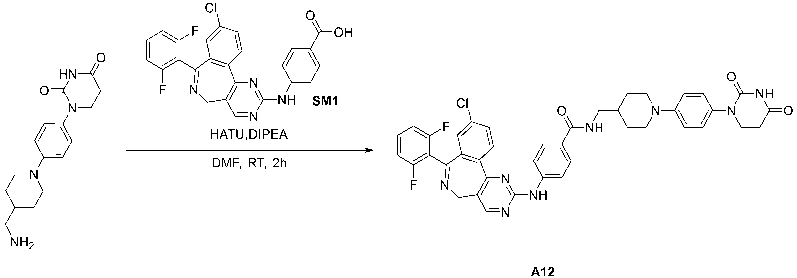

Compound **A12** (16.38 mg, yield 67.31%) was synthesized according to the synthesis method of compound A1 described in **Example 1.** LCMS (ESI) m/z = 761.1 [M+H]⁺. ¹H NMR (400 MHz, DMSO-*d*₆) δ 10.24 (s, 1H), 10.15 (s, 1H), 8.73 (s, 1H), 8.41 - 8.26 (m, 2H), 7.93 - 7.80 (m, 5H), 7.61 - 7.49 (m, 1H), 7.34 (d, *J =* 2.1 Hz, 1H), 7.24 - 7.07 (m, 4H), 6.93 (d, *J =* 8.8 Hz, 2H), 3.77 - 3.62 (m, 4H), 3.19 (m, 2H), 2.75 - 2.51 (m, 4H), 1.87 - 1.74 (d, *J* = 13.2 Hz, 3H), 1.38 - 1.25 (m, 2H).

### Example 13 Synthesis of Compound A13:

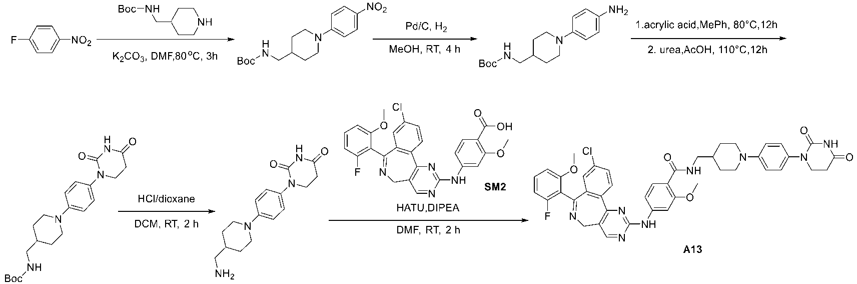

Compound **A13** (15.33 mg, yield 63.45%) was synthesized according to the synthesis method of compound **A8** described in **Example 8.** LCMS (ESI) m/z = 803.5 [M+H]⁺. ¹H NMR (400 MHz, DMSO-*d*₆) δ 10.24 (s, 1H), 10.16 (s, 1H), 8.72 (s, 1H), 8.30 (d, *J =* 8.4 Hz, 1H), 8.08 (t, *J =* 6.0 Hz, 1H), 7.96 (s, 1H), 7.86 - 7.75 (m, 2H), 7.48 - 7.35 (m, 2H), 7.22 (s, 1H), 7.17 - 7.07 (m, 2H), 7.00 - 6.87 (m, 2H), 3.92 (s, 3H), 3.77 - 3.63 (m, 4H), 3.31 - 3.29 (m, 3H), 3.25 (m, 2H), 2.67 (dd, *J*₁ = 8.8 Hz, *J*₂ =4.8 Hz, 4H), 2.52 (d, *J =* 3.2 Hz, 2H), 1.76 (d, *J =* 12.8 Hz, 3H), 1.39 - 1.21 (m, 2H).

### Example 14 Synthesis of Compound A14:

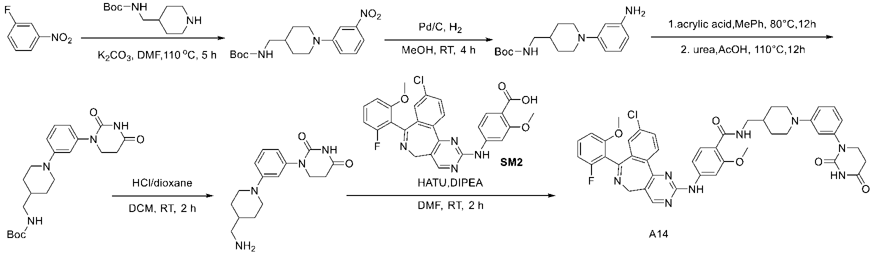

Compound **A14** (8.27 mg, yield 33.09%) was synthesized according to the synthesis method of compound **A8** described in **Example 8.** LCMS (ESI) m/z = 803.4 [M+H]⁺. ¹H NMR (400 MHz, DMSO-*d*₆) δ 10.31(s, 1H), 10.19 (s, 1H), 8.74 (s, 1H), 8.33(d, *J =* 7.2 Hz, 1H), 8.12 - 8.09 (m, 1H), 7.90 (s, 1H), 7.86 - 7.81 (m, 2H), 7.47 - 7.41 (m, 2H), 7.24 - 7.91 (m, 1H), 6.91 - 6.83 (m, 2H), 6.71 - 6.69 (m, 1H), 3.95 (s, 3H),3.78 - 3.73 (m, 4H), 3.29 - 3.25 (, 2H), 2.72-2.61 (m, 4H), 1.79 - 1.71 (m, 3H), 1.34 - 1.31 (m, 2H).

### Example 15 Synthesis of Compound A15:

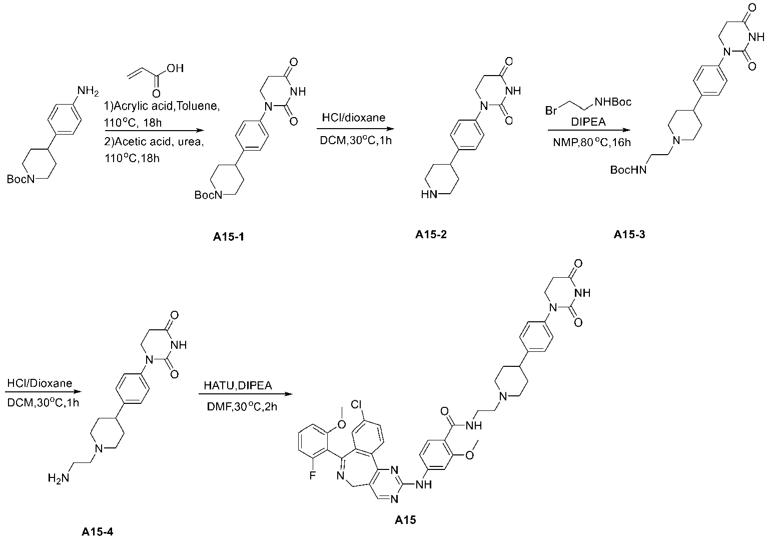

### Step 1:

Acrylic acid (573 mg, 8.0 mmol) was added to a solution of compound 1-BOC-4-(4-aminophenyl)piperidine (2 g, 7.23 mmol) in toluene (20 mL) at room temperature. The reaction mixture was heated to 110°C under argon protection and stirred for 18 h. The mixture was cooled to room temperature, and urea (2.16 g, 36 mmol) and acetic acid (20 mL) were added. The mixture was heated to 110°C and stirred for 18 h. After the reaction was completed, the solvent was removed by concentration under reduced pressure, ethyl acetate and saturated aqueous sodium bicarbonate solution were added for extraction, anhydrous sodium sulfate was used for drying, and concentration under reduced pressure was performed. The residue was separated for purification by silica gel column chromatography (methanol/dichloromethane = 1:20) to give a white solid **A15-1** (300 mg, yield 12%). LCMS (ESI) m/z = 374.2 [M+H]⁺.

### Step 2:

HCl (4 M in dioxane, 1 mL) was added to a solution of compound **A15-1** (300 mg, 0.8 mmol) in dichloromethane (5 mL) at room temperature. The reaction mixture was stirred at 30°C for 1 h. The reaction mixture was concentrated under reduced pressure to give a light yellow solid **A15-2** (220 mg, yield 99%). LCMS (ESI) m/z = 274.1 [M+H]⁺.

### Step 3:

*N*-Boc-bromoethylamine (270 mg, 1.2 mmol) and DIPEA (312 mg, 2.4 mmol) were added to a solution of compound **A15-2** (220 mg, 0.8 mmol) in *N*-methylpyrrolidone (5 mL) at room temperature. The reaction mixture was heated to 80°C under argon protection and stirred for 16 h. After the addition of water, the reaction mixture was extracted with ethyl acetate, dried over anhydrous sodium sulfate, and concentrated under reduced pressure. The residue was separated for purification by silica gel column chromatography (methanol/dichloromethane = 1:20) to give a yellow solid **A15-3** (80 mg, yield 24%). LCMS (ESI) m/z = 417.1 [M+H]⁺.

### Step 4:

HCl (4 M in dioxane, 1 mL) was added to a solution of compound **A15-3** (80 mg, 0.19 mmol) in dichloromethane (2 mL) at room temperature. The reaction mixture was stirred at 30°C for 1 h. The reaction mixture was concentrated under reduced pressure to give a light yellow solid **A15-4** (60 mg, yield 99%). LCMS (ESI) m/z = 317.0 [M+H]⁺.

### Step 5:

**SM2** (15 mg, 0.03 mmol), HATU (15.5 mg, 0.04 mmol) and DIPEA (12.2 mg, 0.09 mmol) were added to a solution of compound **A15-4** (20 mg, 0.06 mmol) in *N,N*-dimethylformamide (2 mL) at room temperature. The reaction mixture was stirred at room temperature for 2 h. The reaction mixture was separated for purification by reverse phase chromatography (acetonitrile/10 M aqueous ammonium bicarbonate solution) to give a light yellow solid **A15** (11.6 mg, yield 47.3%). LCMS (ESI) m/z = 817.5 [M+H]⁺. ¹H NMR (400 MHz, DMSO-*d*₆) δ 10.33 (s, 1H), 10.20 (s, 1H), 8.72 (s, 1H), 8.34 - 8.28 (m, 2H), 8.00 (s, 1H), 7.89 (d, *J =* 12.0 Hz, 1H), 7.83 (dd, *J*₁ = 8.4Hz, *J*₂ = 2.4 Hz, 1H), 7.46 - 7.39 (m, 2H), 7.32 - 7.21 (m, 5H), 3.98 (s, 3H), 3.76 (t, *J =* 6.8 Hz, 2H), 3.46 - 3.40 (m, 2H), 3.04 (d, *J =* 12.0 Hz, 2H), 2.68 (t, *J =* 6.8 Hz, 2H), 2.61 - 2.56 (m, 3H), 2.11 (t, *J* = 8.0 Hz, 2H), 1.86 - 1.77 (m, 2H), 1.74 - 1.63 (m, 2H).

### Example 16 Synthesis of Compound A16:

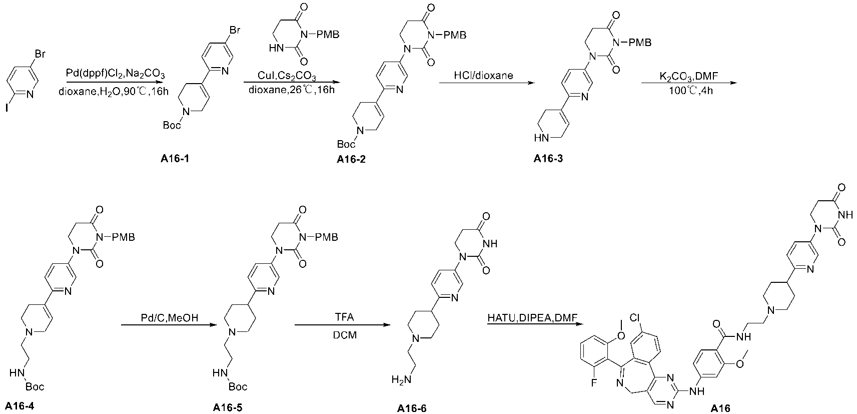

### Step 1:

At room temperature, the compounds 5-bromo-2-iodopyridine (11.11 g, 35.22 mmol), N-Boc-1,2,5,6-tetrahydropyridine-4-boronate pinacol ester (14.52 g, 42.27 mmol), Pd(dppf)Cl₂ (2.71 g, 3.52 mmol) and sodium carbonate (7.86 g, 70.45 mmol) were dissolved in 1,4-dioxane/H₂O (100 mL/20 mL). The reaction mixture was stirred at 80°C for 16 h. The reaction mixture was washed with water, and extracted with ethyl acetate. The organic phase was concentrated to remove the solvent, and the residue was separated for purification by silica gel column chromatography (petroleum ether/ethyl acetate = 5:1) to give a white solid **A16-1** (10.00 g, yield 75%). LCMS (ESI) m/z = 339.2 [M+H]⁺.

### Step 2:

At room temperature, compound **A16-1** (923.8 mg, 2.72 mmol), cesium carbonate (1.77 g, 5.45 mmol), trans-(1S, 2S)-*N,N*-dimethylcyclohexanediamine (77.5 mg, 0.55 mmol), cuprous iodide (103.7 mg, 3.45 mmol), and 3-(4-methoxybenzyl)-dihydropyrimidine-2,4 (1*H*,3*H*)-dione (4.49 g, 0.55 mmol, synthesized with reference to patent WO2022125790) were dissolved in dioxane (5 mL), and the reaction mixture was stirred at 80°C for 16 h. The reaction mixture was concentrated to remove the solvent, and the residue was separated for purification by silica gel column chromatography (dichloromethane/methanol = 10:1) to give a white solid **A16-2** (1.00 g, 74%). LCMS (ESI) m/z = 493.2 [M+H]⁺.

### Step 3:

HCl (4.0 M in 1,4-dioxane, 2 mL) was added to a dichloromethane solution (2 mL) of compound **A16-2** (900.0 mg, 1.83 mmol), and the reaction mixture was stirred at room temperature for 2 h. The reaction mixture was directly spin-dried to give a white solid **A16-3** (600.0 mg, yield 83%). LCMS (ESI) m/z = 393.1 [M+H]⁺.

### Step 4:

At room temperature, DIPEA (65.8 mg, 0.50 mmol) and N-BOC-bromoethylamine (57.1 mg, 0.25 mmol) were added to a solution of compound **A16-3** (100.0 mg, 0.25 mmol) in N,N-dimethylformamide (3 mL), and the reaction mixture was heated to 100°C and stirred for 4 h. The reaction mixture was washed with water, and extracted with ethyl acetate. The organic phase was concentrated to remove the solvent, and the residue was separated for purification by silica gel column chromatography (petroleum ether/ethyl acetate = 2:1) to give a white solid **A16-4** (100.0 mg, yield 73%). LCMS(ESI)m/z=536.2 [M+H]⁺.

### Step 5:

Pd/C (20.0 mg, 10%) was added to a solution of compound **A16-4** (80.0 mg, 0.15 mmol) in methanol (3 mL) at room temperature, and the reaction mixture was stirred at room temperature for 16 h. The reaction mixture was directly suction-filtered and spin-dried to give a white solid **A16-5** (75.0 mg, yield 93%). LCMS (ESI) m/z = 538.2 [M+H]⁺.

### Step 6:

Trifluoroacetic acid (0.5 mL) was added to a solution of compound **A16-5** (75.0 mg, 0.14 mmol) in dichloromethane (2 mL), and the reaction mixture was stirred at room temperature for 16 h. The reaction mixture was directly spin-dried to give a white solid **A16-6** (65.0 mg, yield 90%). LCMS (ESI) m/z = 318.2 [M+H]⁺.

### Step 7:

To a solution of compound **A16-6** (15.3 mg, 0.048 mmol) in N,N-dimethylformamide (2 mL), **SM2** (15.0 mg, 0.029 mmol), HATU (16.5 mg, 0.043 mmol) and DIPEA (11.2 mg, 0.086 mmol) were added. The reaction mixture was stirred at room temperature for 2 h. The reaction mixture was separated for purification by reverse phase chromatography (acetonitrile/0.1% aqueous formic acid solution = 2.3/1) to give a yellow solid **A16** (7.0 mg, yield 30%). LCMS (ESI) m/z = 818.2 [M+H]⁺. ¹H NMR (400 MHz, DMSO-*d*₆) δ 10.50 (s, 1H), 10.23 (s, 1H), 8.75 (s, 1H), 8.51 (d, *J =* 2.4 Hz, 1H), 8.39 - 8.32 (m, 2H), 8.03 (s, 1H), 7.92 (d, *J =* 8.4 Hz, 1H), 7.85 (dd, *J*₁ = 8.4 Hz, *J*₂ = 2.0 Hz, 1H), 7.71 (d, *J* =2.4 Hz, 1H), 7.50 - 7.42 (m, 2H), 7.38 (d, *J* = 8.4 Hz, 1H), 7.25 (s, 1H), 4.02 (s, 3H), 3.85 (t, *J* = 6.4 Hz, 2H), 3.50 - 3.44 (m, 2H), 3.06 (d, *J =* 10.8 Hz, 2H), 2.75 (t, *J =* 6.4 Hz, 2H), 2.65 - 2.54 (m, 3H), 2.15 (t, *J =* 11.2 Hz, 2H), 1.93 - 1.81 (m, 4H).

### Example 17 Synthesis of Compound A17:

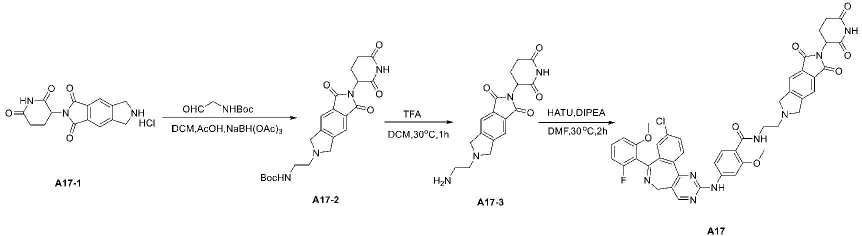

### Step 1:

At room temperature, to a solution of compound **A17-1** (200 mg, 0.67 mmol, synthesized with reference to patent WO2021041664) in dichloromethane (10 mL), N-tert-butoxycarbonyl-2-aminoacetaldehyde (117 mg, 0.74 mmol) and acetic acid (120 mg, 2 mmol) were added. The reaction mixture was stirred at 30°C for 1 h. Then sodium triacetoxyborohydride (425 mg, 2 mmol) was added, and the reaction mixture was stirred at 30°C for 16 h. The reaction mixture was concentrated under reduced pressure, and the residue was separated for purification by silica gel column chromatography (methanol/dichloromethane = 1:20) to give a white solid **A17-2** (90 mg, yield 30%). LCMS(ESI) m/z = 443.2[M+H]⁺.

### Step 2:

Trifluoroacetic acid (0.5 mL) was added to a solution of compound **A17-2** (30 mg, 0.07 mmol) in dichloromethane (2 mL) at room temperature. The reaction mixture was stirred at 30°C for 1 h. The reaction mixture was concentrated under reduced pressure to give a light yellow solid **A17-3** (23 mg, yield 99%). LCMS (ESI) m/z = 343.2 [M+H]⁺.

### Step 3:

**SM2** (15 mg, 0.03 mmol), HATU (15.5 mg, 0.04 mmol) and DIPEA (12.2 mg, 0.09 mmol) were added to a solution of compound **A17-3** (23 mg, 0.07 mmol) in N,N-dimethylformamide (2 mL) at room temperature. The reaction mixture was stirred at room temperature for 2 h. The reaction mixture was separated for purification by reverse phase chromatography (acetonitrile/10 M aqueous ammonium bicarbonate solution) to give a light yellow solid A17 (11.4 mg, yield 45%). LCMS (ESI) m/z = 843.0 [M+H]⁺. ¹H NMR (400 MHz, DMSO-*d*₆) δ 11.15 (s, 1H), 10.29 (s, 1H), 8.74 (s, 1H), 8.32 - 8.30 (m, 3H), 7.97 (s, 1H), 7.89 - 7.81 (m, 6H),7.47 - 7.41 (m, 3H),7.24 (bs, 1H), 5.19 - 5.14 (m, 1H), , 4.19 (s, 3H), 3.87 - 3.81(m, 4H), 3.53 - 3.52 (m, 2H), 2.95 - 2.89 (m, 3H), 2.64 - 2.58 (m, 2H), 2.10 - 2.04 (m, 1H).

### Example 18 Synthesis of Compound A18:

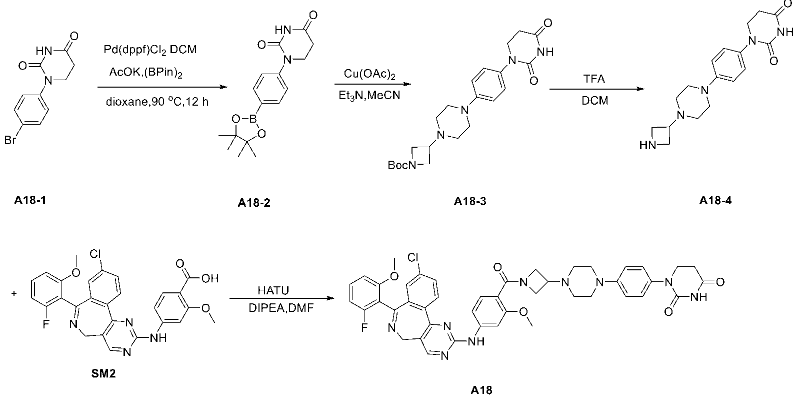

### Step 1:

Bis(pinacolato)diboron (2.82 g, 11.15 mmol), [1,1'-bis(diphenylphosphino)ferrocene]dichloropalladium, complex with dichloromethane (455.21 mg, 557.43 µmol), and potassium acetate (1.37 g, 13.94 mmol) were added to a solution of compound **A18-1** (1.50 g, 5.57 mmol, synthesized with reference to **Example 8)** in 1,4-dioxane (15.0 mL) at room temperature. The reaction mixture was stirred at 90°C for 12 h under an argon atmosphere. Solid impurities were removed by suction filtration, the filtrate was concentrated, and the residue was separated for purification by a silica gel column (petroleum ether/ethyl acetate = 20:1) to give a yellow solid **A18-2** (746 mg, yield 42.33%). LCMS (ESI) m/z = 317.2 [M+H]⁺.

### Step 2:

1- tert-Butoxycarbonyl-3-(1-piperazinyl)azetidine (343.49 mg, 1.42 mmol) was dissolved in acetonitrile, and **A18-2,** copper acetate (377.66 mg, 1.90 mmol), triethylamine (191.68 mg, 1.90 mmol), and 4Å molecular sieves (191.68 mg, 1.90 mmol) were added sequentially, and the mixture was stirred at 60°C for 3 h. After the reaction was completed, the mixture was directly spin-dried, and purified by flash silica gel column chromatography (EA: MeOH=10% to 10%) to give **A18-3** (320 mg). LCMS (ESI) m/z = 430.2 [M+H]⁺.

### Step 2:

**A18-3** (40 mg) obtained in the previous step was dissolved in dichloromethane (2 mL), trifluoroacetic acid (1 mL) was added, and the reaction mixture was stirred at room temperature for 2 h. The reaction mixture was concentrated to remove the solvent to give an oily substance **A18-4,** which was used directly in the next step. LCMS (ESI) m/z = 330.18 [M+H]⁺.

### Step 3:

**A18-4** (30 mg, 96.46 µmol) obtained in the previous step was dissolved in N,N-dimethylformamide (1 mL), and **SM2** (23 mg, 48.23 µmol), HATU (36.68 mg, 96.46 µmol) and DIPEA (18.70 mg, 144.70 µmol, 25.20 µL) were added successively. The reaction mixture was stirred overnight at room temperature. After the reaction was completed, the reaction mixture was separated for purification by reverse phase chromatography (acetonitrile/0.1% aqueous formic acid solution = 2.3/1) to give a yellow solid **A18** (10.2 mg, yield 26%). LCMS (ESI) m/z = 820.25 [M+H]⁺. ¹H NMR (400 MHz, DMSO-*d*₆) δ 10.28 (s, 1H), 10.23 (s, 1H), 8.76 (s, 1H), 8.33 (d, *J =* 8.4 Hz, 1H), 7.96 - 7.89 (m, 3H), 7.68 (d, *J =* 8.4 Hz, 1H), 7.61 - 7.56 (m, 1H), 7.36 (d, *J =* 2.4 Hz, 1H), 7.19 - 7.17 (m, 4H), 6.98 - 6.96 (m, 2H), 3.72 (t, *J =* 6.8 Hz, 2H), 3.19 - 3.17 (m, 4H), 3.27 - 2.98 (m, 2H).

### Example 19 Synthesis of Compound A19:

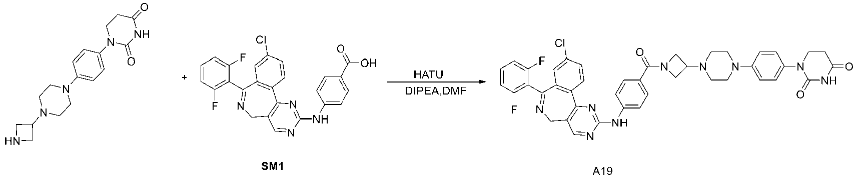

Compound **A19** (5.6 mg, yield 34%) was synthesized according to the synthesis method of compound **A1** described in **Example 1.** LCMS (ESI) m/z = 788.21 [M+H]⁺. ¹H NMR (400 MHz, CDCl₃) δ 8.73 (s, 1H), 8.64 (s, 1H), 8.60 (s, 1H), 8.31 - 8.29 (m, 1H), 8.01 (s, 1H), 7.89 - 7.76(m, 2H), 7.66 - 7.63 (m, 2H), 7.56 - 7.52 (m, 2H), 7.43 - 7.37 (m, 2H), 7.29 - 7.24 (m, 2H), 7.19 - 7.16 (m, 1H), 7.04 - 7.01 (1H),4.51 - 4.32 (m, 2H), 4.01 - 3.86 (m, 5H), 3.91 - 3.86 (m, 4H), 2.90 - 2.87 (m, 4H).

### Example 20 Synthesis of Compound A20:

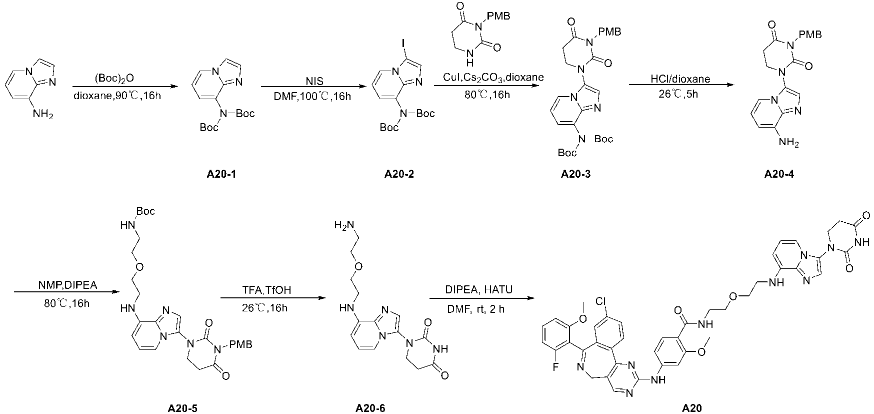

### Step 1:

To a solution of compound imidazo[1,2-*a*]pyridine-8-amine (5.56 g, 37.55 mmol) in dioxane (100 mL), di-tert-butyl dicarbonate (33.11 g, 150.21 mmol) was added at room temperature, and the reaction mixture was stirred at 90°C for 16 h. After the addition of water, the reaction mixture was extracted with ethyl acetate, dried over anhydrous sodium sulfate, and the crude product was separated for purification by silica gel column chromatography (petroleum ether:ethyl acetate = 2:1) to give a yellow solid **A20-1** (10.00 g, yield 90%). LCMS (ESI) m/z = 334.2 [M+H]⁺.

### Step 2:

At room temperature, N-iodosuccinimide (7.07 g, 31.10 mmol) was added to a solution of compound **A20-1** (9.00 g, 25.92 mmol) in DMF (100 mL), and the reaction mixture was stirred at 100°C for 16 h. The reaction mixture was washed with water and extracted with ethyl acetate, and the organic phase was concentrated to remove the solvent to give a yellow solid **A20-2** (8.00 g, yield 90%). LCMS (ESI) m/z = 460.1 [M+H]⁺.

### Step 3:

At room temperature, compound **A20-2** (8.00 g, 17.24 mmol), cesium carbonate (11.35 g, 34.49 mmol), trans-(1S, 2S)-*N,N*-dimethylcyclohexanediamine (495.0 mg, 3.45 mmol), cuprous iodide (663.0 mg, 3.45 mmol), and 3-(4-methoxybenzyl)-dihydropyrimidine-2,4 (1H,3H)-dione (4.49 g, 18.97 mmol) were dissolved in dioxane (100 mL), and the reaction mixture was stirred at 80°C for 16 h. The reaction mixture was concentrated to remove the solvent, and the residue was separated for purification by silica gel column chromatography (dichloromethane/methanol = 10:1) to give a white solid **A20-3** (3.00 g, 27%). LCMS (ESI) m/z = 566.1 [M+H]⁺.

### Step 4:

HCl (4.0 M in 1,4-dioxane, 5 mL) was added to a solution of compound **A20-3** (1.11 g, 1.77 mmol) in 1,4-dioxane (5 mL), and the reaction mixture was stirred at room temperature for 5 h. The reaction mixture was directly spin-dried to give a yellow solid **A20-4** (600.0 mg, yield 84%). LCMS (ESI) m/z = 366.2 [M+H]⁺.

### Step 5:

At room temperature, N-Boc-2-(2-bromoethoxy)ethylamine (250.5 mg, 0.92 mmol) and DIPEA (150.0 mg, 1.15 mmol) were added to a solution of compound A20-4 (212.0 mg, 0.57 mmol) in N-methylpyrrolidone (2 mL). The reaction mixture was stirred at 80°C for 16 h. The reaction mixture was washed with water and extracted with ethyl acetate, and the organic phase was concentrated to remove the solvent to give a yellow solid **A20-5** (111.0 mg, yield 34%). LCMS (ESI) m/z = 553.1 [M+H]⁺.

### Step 6:

At 0°C, trifluoroacetic acid (3 mL) and trifluoromethanesulfonic acid (1.5 mL) were added to compound **A20-5** (111.0 mg, 0.18 mmol), and the reaction mixture was stirred at 25°C for 16 h. The reaction mixture was adjusted to about pH 8 with saturated potassium bicarbonate, and separated for purification by reverse phase chromatography (acetonitrile/10 M aqueous ammonium bicarbonate solution) to give a white solid **A20-6** (30.0 mg, 50%). LCMS (ESI) m/z = 333.3 [M+H]⁺.

### Step 7:

To a solution of compound **A20-6** (13.0 mg, 0.035 mmol) in N,N-dimethylformamide (1 mL), **SM2** (12.2 mg, 0.021 mmol), HATU (12.1 mg, 0.032 mmol) and DIPEA (8.3 mg, 0.064 mmol) were added. The reaction mixture was stirred at room temperature for 2 h. The reaction mixture was separated for purification by reverse phase chromatography (acetonitrile/0.1% aqueous formic acid solution = 2.3/1) to give a yellow solid **A20** (5.0 mg, yield 27%). LCMS (ESI) m/z = 833.2 [M+H]⁺. ¹H NMR (400 MHz, DMSO-*d*₆) δ 10.34 (s, 1H), 10.24 (s, 1H), 8.75 (s, 1H), 8.33 (d, *J =* 2.8 Hz, 2H), 8.11 (d, *J* = 2.8 Hz, 1H), 8.00 (s, 1H), 7.92 (d, *J* = 8.4 Hz, 1H), 7.85 (dd, *J =* 8.4, 2.4 Hz, 1H), 7.55 (d, *J* = 9.2 Hz, 1H), 7.51 - 7.44 (m, 3H),7.40 (s, 1H), 6.79 - 6.75 (m, 1H), 6.27 - 6.25 (m, 1H), 5.94 - 5.92 (m, 1H), 3.94 (s, 3H), 3.80 - 3.72 (m, 4H), 3.65 - 3.53 (m, 2H), 3.52 - 3.49 (m, 4H), 2.84 - 2.81 (m, 2H).

### Example 21 Synthesis of Compound A21:

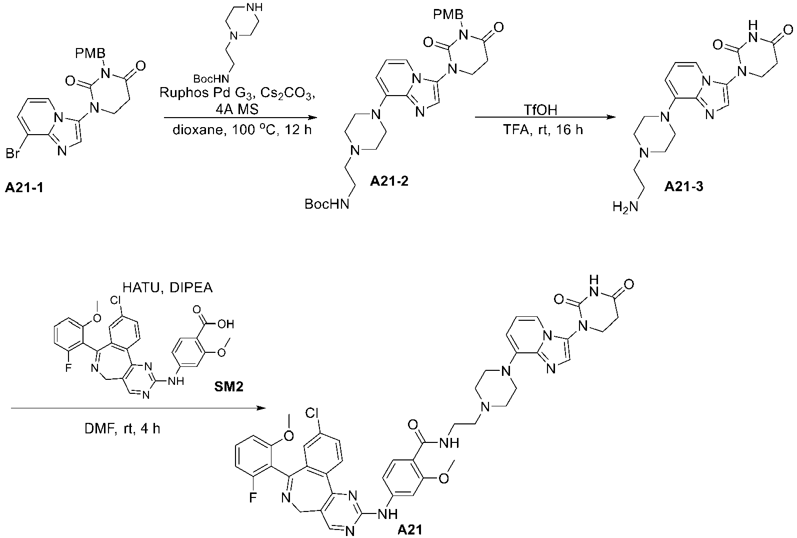

### Step 1:

Compound **A21-1** (3.31 g, 0.41 mmol, synthesized with reference to patent WO2022125790) and compound 1-(N-Boc-aminoethyl)piperazine (180.9 mg, 0.81 mmol) were dissolved in dioxane solution (4 mL), then 4Å molecular sieve (200.0 mg), cesium carbonate (396.2 mg, 1.22 mmol) and RuPhos Pd G3 (33.9 mg, 0.041 mmol) were added. The reaction mixture was stirred at 100°C for 12 h under argon protection. After the addition of water (20 mL), the reaction mixture was extracted with ethyl acetate (50 mL*3). The organic phases were pooled, washed with saturated brine (50 mL), dried over anhydrous sodium sulfate, filtered, and the filtrate was concentrated to give a crude product. The crude product was purified by column chromatography to give compound **A21-2** (171.0 mg, yellow solid). LCMS (ESI) m/z = 578.2 [M+H]⁺.

### Step 4:

Compound **A21-2** (171.0 mg, 0.30 mmol) was dissolved in trifluoroacetic acid (2 mL), trifluoromethanesulfonic acid (1 mL) was added, and the reaction mixture was stirred at 25°C for 16 h. The reaction mixture was adjusted to pH = 5-6 with a saturated sodium bicarbonate solution, and separated for purification by reverse phase chromatography (acetonitrile/10 M aqueous ammonium bicarbonate solution) to give compound **A21-3** (100.0 mg, brown solid). LCMS (ESI) m/z = 358.3 [M+H]⁺.

### Step 5:

To a solution of compound **A21-3** (20.7 mg, 0.058 mmol) in N,N-dimethylformamide (2 mL), **SM2** (15.0 mg, 0.029 mmol), HATU (16.5 mg, 0.043 mmol) and DIPEA (11.2 mg, 0.087 mmol) were added. The reaction mixture was stirred at room temperature for 4 h. The reaction mixture was separated for purification by reverse phase chromatography (methanol/10 M aqueous ammonium bicarbonate solution) to give a yellow solid **A21** (7.0 mg, yield 28%). LCMS (ESI) m/z = 857.9 [M+H]⁺. ¹H NMR (400 MHz, DMSO-*d*₆) δ 10.68 (s, 1H), 10.24 (s, 1H), 8.75 (s, 1H), 8.46 - 8.23 (m, 3H), 8.02 (s, 1H), 7.98 - 7.78 (m, 3H), 7.55 - 7.39 (m, 3H), 7.24 (s, 1H), 6.87 (t, *J* = 7.2 Hz, 1H), 6.58 (d, *J* = 7.6 Hz, 1H), 3.98 (s, 3H), 3.81 - 3.79 (m, 2H), 3.61 - 3.53 (m, 4H), 3.54 - 3.48 (m, 6H), 2.90 - 2.84 (m, 1H),2.73 - 2.70 (m, 2H), 2.66 - 2.58 (m, 1H).

### Example 22 Synthesis of Compound A22:

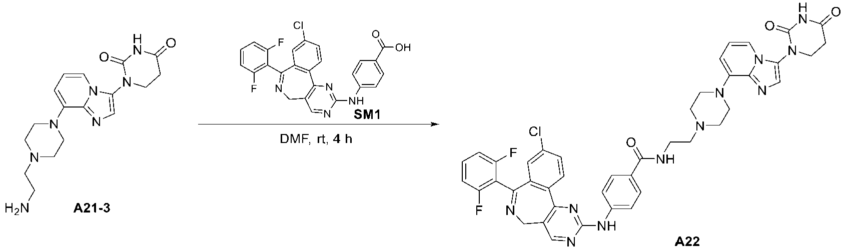

To a solution of compound **A21-3** (22.5 mg, 0.063 mmol) in N,N-dimethylformamide (2 mL), **SM1** (15.0 mg, 0.031 mmol), HATU (17.9 mg, 0.047 mmol) and DIPEA (12.2 mg, 0.094 mmol) were added. The reaction mixture was stirred at room temperature for 4 h. The reaction mixture was separated for purification by reverse phase chromatography (methanol/10 M aqueous ammonium bicarbonate solution) to give **A22** (19.0 mg, yield 74%). LCMS (ESI) m/z = 815.9 [M+H]⁺. ¹H NMR (400 MHz, DMSO-*d*₆) δ 10.67 (s, 1H), 10.20 (s, 1H), 8.76 (s, 1H), 8.39 - 8.29 (m, 2H), 7.98 - 7.82 (m, 6H), 7.63 - 7.53 (m, 1H), 7.49 (s, 1H), 7.37 (d, *J* = 2.0 Hz, 1H), 7.20 (t, *J =* 8.8 Hz, 2H), 6.85 (t, *J =* 6.8 Hz, 1H), 6.54 (d, *J =* 7.6 Hz, 1H), 3.79 - 3.70 (m, 2H), 3.59 - 3.35 (m, 10H), 2.89 - 2.82 (m, 1H), 2.69 - 2.64 (m, 2H), 2.64 - 2.56 (m, 1H).

### Example 23 Synthesis of Compound A23:

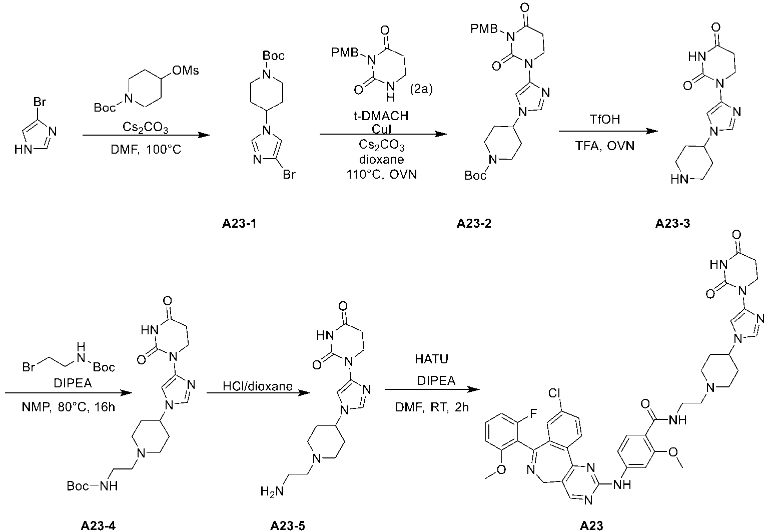

### Step 1:

To a solution of compound 4-bromo-1H-imidazole (5.0 g, 34.02 mmol) in *N,N*-dimethylformamide (70 mL), cesium carbonate (22.17 g, 68.04 mmol) and tert-butyl 4-(methanesulfonyloxy)piperidine-1-carboxylate (9.5 g, 34.02 mmol) were added at room temperature. The reaction mixture was heated to 100°C under argon protection and stirred for 16 h. The reaction mixture was separated for purification by reverse phase chromatography (acetonitrile/0.1% aqueous formic acid solution) to give a yellow solid **A23-1** (1.5 g, yield 13%). LCMS (ESI) m/z = 331.0 [M+H]⁺. ¹H NMR (400 MHz, DMSO-*d*₆) δ 7.75 (d, *J =* 1.4 Hz, 1H), 7.49 (d, *J=* 1.5 Hz, 1H), 4.28 - 4.20 (m, 1H), 4.14 - 4.02 (m, 2H), 2.84 (bs, 2H), 1.95 (s, 1H), 1.91 (s, 1H), 1.82 - 1.69 (m, 2H), 1.44 (s, 9H).

### Step 2:

At room temperature, 3-(4-methoxybenzyl)-dihydropyrimidine-2,4 (1*H*,3*H*)-dione (1.33 g, 5.66 mmol), trans-(1R, 2R)-*N,N'*-dimethyl-1,2-cyclohexanediamine (732.2 mg, 5.15 mmol), cesium carbonate (3.35 g, 10.30 mmol) and cuprous iodide (196.09 mg, 1.03 mmol) were added to a solution of compound **A23-1 (1.7** g, 5.15 mmol) in 1,4-dioxane (20 mL). The reaction mixture was stirred at 110°C for 16 h. The reaction mixture was concentrated to remove the solvent, and the residue was separated for purification by silica gel column chromatography (dichloromethane/methanol = 10:1) to give a yellow solid **A23-2** (2.2 g, 91%). LCMS (ESI) m/z = 484.1 [M+H]⁺. ¹H NMR (400 MHz, DMSO-*d*₆) δ 7.21 (d, *J =* 8.2 Hz, 2H), 6.84 (d, *J =* 7.7 Hz, 2H), 4.82 (s, 2H), 4.21 (t, *J =* 12.0 Hz, 1H), 4.10 - 3.99 (m, 4H), 3.71 (s, 3H), 2.83 (t, *J =* 6.4 Hz, 3H), 1.95 (d, *J* = 12.3 Hz, 2H), 1.81 - 1.63 (m, 2H), 1.41 (s, 9H).

### Step 3:

At 0°C, trifluoroacetic acid (5 mL) and trifluoromethanesulfonic acid (1 mL) were added to compound **A23-2** (1.0 g, 2.07 mmol), and the reaction mixture was stirred at 25°C for 24 h. The reaction mixture was adjusted to about pH 8 with saturated potassium bicarbonate, and separated for purification by reverse phase chromatography (acetonitrile/10 M aqueous ammonium bicarbonate solution) to give a white solid **A23-3** (150 mg, 27%). LCMS (ESI) m/z = 264.0 [M+H]⁺.

### Step 4:

At room temperature, *N*-Boc-2-bromoethyl-amine (255.3 mg, 1.14 mmol) and DIPEA (736.3 mg, 5.7 mmol) were added to a solution of compound **A23-3** (150.0 mg, 0.56 mmol) in *N*-methylpyrrolidone (6 mL). The reaction mixture was stirred at 80°C for 16 h. After the addition of water, the reaction mixture was extracted with ethyl acetate, dried over anhydrous sodium sulfate, and the crude product was separated for purification by silica gel column chromatography (dichloromethane/methanol = 10:1) to give a yellow solid **A23-4** (77 mg, yield 33%). LCMS (ESI) m/z = 407.2 [M+H]⁺. ¹H NMR (400 MHz, DMSO-*d*₆) δ 10.33 (s, 1H), 7.70 - 7.60 (m, 0.5H), 7.31 - 7.20 (m, 1H), 6.69 - 6.64 (m, 1H), 4.06 - 3.97 (m, 2H), 3.04 (q, *J* = 6.6 Hz, 2H), 2.95 (d, *J* = 11.2 Hz, 2H), 2.64 (t, *J* = 6.7 Hz, 1H), 2.36 (t, *J* = 6.8 Hz, 2H), 2.08 (t, *J* = 11.6 Hz, 2H), 1.97 - 1.79 (m, 4H), 1.37(s, 9H).

### Step 5:

At room temperature, hydrochloric acid (4.0 M solution in 1,4-dioxane) (2 mL) was added to compound **A23-4** (77.0 mg, 0.19 mmol), and the reaction mixture was stirred at 25°C for 2 h. The reaction mixture was concentrated to remove the solvent to give a yellow solid **A23-5** (78 mg). LCMS (ESI) m/z = 307.1 [M+H]⁺.

### Step 6:

To a solution of compound **A23-5** (13.3 mg, 0.043 mmol) in N,N-dimethylformamide (1 mL), **SM2** (15.0 mg, 0.029 mmol), HATU (13.2 mg, 0.034 mmol) and DIPEA (18.7 mg, 0.14 mmol) were added. The reaction mixture was stirred at room temperature for 2 h. The reaction mixture was separated for purification by reverse phase chromatography (acetonitrile/0.1% aqueous formic acid solution = 2.3/1) to give a yellow solid **A23** (20.0 mg, yield 87%). LCMS (ESI) m/z = 807.1 [M+H]⁺. ¹H NMR (400 MHz, DMSO-*d*₆) δ 10.32 (s, 1H), 10.19 (s, 1H), 8.72 (s, 1H), 8.31 (d, *J =* 8.4 Hz 1H), 8.25 (t, *J* = 5.2 Hz, 1H), 7.96 (s, 1H), 7.87 (d, *J =* 8.6 Hz, 1H), 7.83 (dd, *J*₁ = 8.4 Hz, *J*₂ = 2.2 Hz, 1H), 7.64 (d, *J =* 1.6 Hz, 1H), 7.47 - 7.37 (m, 2H), 7.33 (d, *J* = 1.6 Hz, 1H), 7.21 (s, 1H), 4.02 (t, *J =* 6.6 Hz, 2H), 3.96 (s, 3H), 3.46-3.40 (m, 2H), 3.01 (d, *J =* 11.2 Hz, 2H), 2.64 (t, *J =* 6.8 Hz, 2H), 2.62 - 2.55 (m, 3H), 2.20 - 2.15 (m, 2H), 2.02 (d, *J =* 12.0 Hz, 2H), 1.91 - 1.81 (m, 2H).

### Example 24 Synthesis of Compound A24:

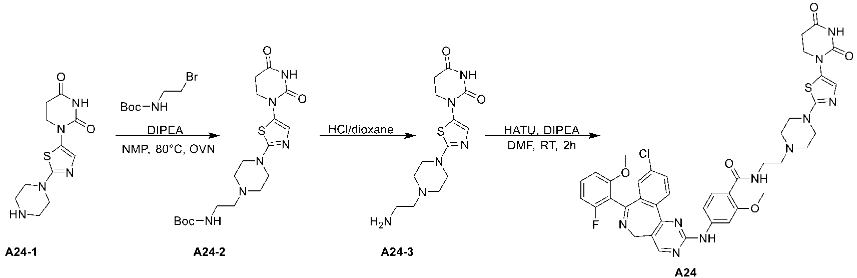

### Step 1:

At room temperature, *N*-Boc-2-bromoethyl-amine (95.6 mg, 0.42 mmol) and DIPEA (137.8 mg, 1.07 mmol) were added to a solution of compound **A24-1** (60.0 mg, 0.21 mmol) in N-methylpyrrolidone (1 mL). The reaction mixture was stirred at 80°C for 16 h. After the addition of water, the reaction mixture was extracted with ethyl acetate, and dried over anhydrous sodium sulfate. The crude product was separated for purification by silica gel column chromatography (dichloromethane/methanol = 10:1) to give a yellow solid **A24-2** (50 mg, yield 55%). LCMS (ESI) m/z = 425.2 [M+H]⁺. ¹H NMR (400 MHz, DMSO-*d*₆) δ 10.59 (s, 1H), 6.99 (s, 1H), 6.69 (t, *J =* 5.8 Hz, 1H), 3.78 (t, *J* = 6.8 Hz, 2H), 3.31 - 3.29 (m, 4H), 3.06 (q, *J* = 6.4 Hz, 2H), 2.72 (t, *J =* 6.8 Hz, 2H), 2.48 - 2.46 (m, 4H), 2.36 (t, *J =* 6.9 Hz, 2H), 1.37 (s, 9H).

### Step 2:

At room temperature, HCl (4 M in dioxane, 2 mL) was added to compound **A24-2** (77.0 mg, 0.19 mmol), and the reaction mixture was stirred at 25°C for 2 h. The reaction mixture was concentrated to remove the solvent to give a yellow solid **A24-3** (50 mg). LCMS (ESI) m/z = 325.2 [M+H]⁺.

### Step 3:

To a solution of compound **A24-3** (14.0 mg, 0.043 mmol) in N,N-dimethylformamide (1 mL), **SM2** (15.0 mg, 0.029 mmol), HATU (13.2 mg, 0.034 mmol) and DIPEA (18.7 mg, 0.14 mmol) were added. The reaction mixture was stirred at room temperature for 2 h. The reaction mixture was separated for purification by reverse phase chromatography (acetonitrile/0.1% aqueous formic acid solution = 2.3/1) to give a yellow solid **A24** (14.0 mg, yield 58%). LCMS (ESI) m/z = 825.6 [M+H]⁺. ¹H NMR (400 MHz, DMSO-*d*₆) δ 10.60 (s, 1H), 10.20 (s, 1H), 8.72 (s, 1H), 8.35-8.25 (m, 2H), 7.99 (s, 1H), 7.88 (d, *J =* 8.6 Hz, 1H), 7.83 (dd, *J*₁ *=* 8.4 Hz, *J*₂ = 2.2 Hz, 1H), 7.45 - 7.37 (m, 2H), 7.21 (s, 1H), 7.01 (s, 1H), 7.19 - 6.81 (m, 2H), 3.94 (s, 3H), 3.79 (t, *J =* 6.8 Hz, 2H), 3.78 - 3.69 (m, 1H), 3.47 - 3.42 (m, 2H), 3.39 (t, *J =* 5.0 Hz, 5H), 2.73 (t, *J* = 6.8 Hz, 2H), 2.56 (t, *J =* 5.4 Hz, 4H).

### Example 25 Synthesis of Compound A25:

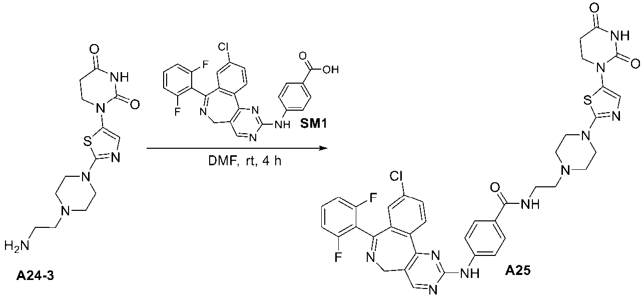

Compound A25 (10.0 mg, yield 41%) was synthesized according to the synthesis method of compound A24 described in Example 24. LCMS (ESI) m/z = 783.1 [M+H]⁺. ¹H NMR (400 MHz, DMSO-*d*₆) δ 10.58 (s, 1H), 10.15 (s, 1H), 8.71 (s, 1H), 8.33 - 8.20 (m, 2H), 7.94 - 7.84 (m, 3H), 7.84 - 7.75 (m, 2H), 7.59 - 7.44 (m, 1H), 7.32 (d, *J =* 2.0 Hz, 1H), 7.16 (t, *J* = 8.8 Hz, 2H), 6.98 (s, 1H), 3.77 (t, *J* = 6.8 Hz, 2H), 3.45 - 3.33 (m, 6H), 2.71 (t, *J* = 6.8 Hz, 2H), 2.59 - 2.51 (m, 6H).

### Example 26 Synthesis of Compound A26:

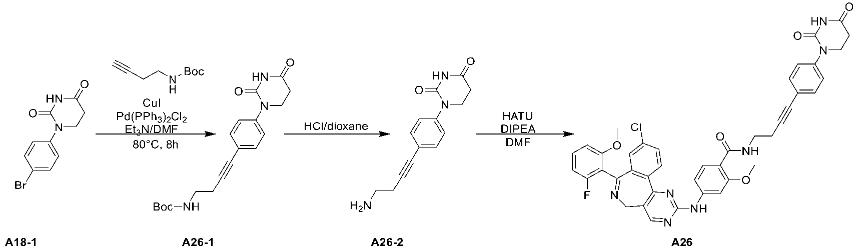

### Step 1:

At room temperature, tert-butyl 3-butynylcarbamate (157.2 mg, 0.92 mmol), cuprous iodide (35.4 mg, 0.18 mmol) and bis(triphenylphosphine)palladium dichloride (65.2 mg, 0.092 mmol) were added to a solution of compound **A18-1** (250.0 mg, 0.92 mmol, synthesized with reference to **Example 8)** in *N,N-*dimethylformamide and triethylamine (2 mL/2 mL). The reaction mixture was heated to 80°C under argon protection and stirred for 8 h. The reaction mixture was separated for purification by reverse phase chromatography (acetonitrile/0.1% aqueous formic acid solution) to give a yellow solid **A26-1** (129 mg, yield 38%). LCMS (ESI) m/z = 358.1 [M+H]⁺. ¹H NMR (400 MHz, DMSO-*d*₆) δ 10.41 (s, 1H), 7.40 (d, *J =* 8.6 Hz, 2H), 7.31 (d, *J =* 8.6 Hz, 2H), 7.01 (t, *J =* 6.0 Hz, 1H), 3.79 (t, *J =* 6.6 Hz, 2H), 3.14 (q, *J =* 6.6 Hz, 2H), 2.70 (t, *J* = 6.6 Hz, 2H), 2.53 (q, *J =* 6.6 Hz, 2H), 1.38 (s, 9H).

### Step 2:

At room temperature, HCl (4.0 M in 1,4-dioxane, 2 mL) was added to compound A26-1 (125.0 mg, 0.34 mmol), and the reaction mixture was stirred at 25°C for 2 h. The reaction mixture was concentrated to remove the solvent to give a yellow solid **A26-2** (100 mg, 98%). LCMS (ESI) m/z = 258.0 [M+H]⁺.

### Step 3:

To a solution of compound **A26-2** (14.8 mg, 0.057 mmol) in N,N-dimethylformamide (1 mL), **SM2** (15.0 mg, 0.029 mmol), HATU (12.1 mg, 0.031 mmol) and DIPEA (18.7 mg, 0.14 mmol) were added. The reaction mixture was stirred at room temperature for 2 h. The reaction mixture was separated for purification by reverse phase chromatography (acetonitrile/10 M aqueous ammonium bicarbonate solution = 2.3/1) to give a yellow solid **A26** (16.0 mg, yield 73%). LCMS (ESI) m/z = 758.4 [M+H]⁺. ¹H NMR (400 MHz, DMSO-*d*₆) δ 10.40 (s, 1H), 10.18 (s, 1H), 8.72 (s, 1H), 8.36 - 8.27 (m, 2H), 7.96 (s, 1H), 7.87 (d, *J =* 8.6 Hz, 1H), 7.81 (dd, *J*₁ = 8.5 Hz, *J*₂ =2.2 Hz, 1H), 7.45 - 7.38 (m, 4H), 7.36 - 7.30 (m, 2H), 7.22 (bs, 1H), 3.90 (s, 3H), 3.80 (t, *J* = 6.6 Hz, 2H), 3.53 (q, *J =* 6.6 Hz, 2H), 2.71 - 2.67 (m, 4H).

### Example 27 Synthesis of Compound A27:

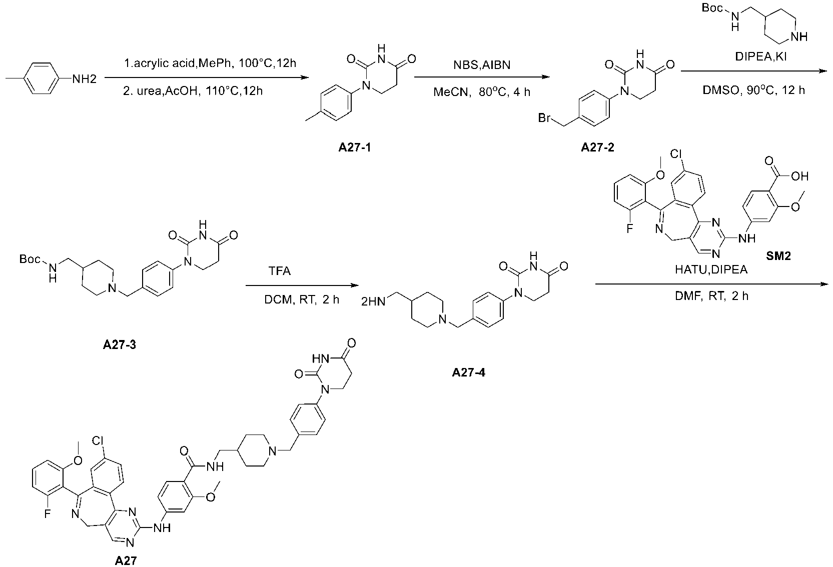

### Step 1:

At room temperature, acrylic acid (2.42 g, 33.60 mmol) was added to a solution of compound p-toluidine (3.00 g, 28.00 mmol) in toluene (25.0 mL). The reaction mixture was stirred at 100°C for 12 h. The reaction mixture was cooled to room temperature, and urea (8.41 g, 139.99 mmol) and acetic acid (25.0 mL) were added. The reaction mixture was heated to 110°C and stirred for 12 h. Toluene was removed by concentration under reduced pressure, and the pH was adjusted to about 8 with a saturated aqueous sodium bicarbonate solution. The mixture was extracted with ethyl acetate, and the layers were separated. The organic phase was washed with saturated brine, dried over anhydrous sodium sulfate, concentrated under reduced pressure to remove the solvent, and separated for purification by a silica gel column (dichloromethane/methanol = 20:1) to give a yellow solid **A27-1** (1.78 g, yield 31.13%). LCMS (ESI) m/z = 204.9 [M+H]⁺.

### Step 2:

At room temperature, to a solution of compound **A27-1** (500.00 mg, 2.45 mmol) in acetonitrile (5.0 mL), N-bromosuccinimide (522.89 mg, 2.94 mmol) and azobisisobutyronitrile (80.41 mg, 489.66 µmol) were added. The reaction mixture was stirred at 80°C for 4 h. The mixture was concentrated under reduced pressure to remove the solvent, and separated for purification by silica gel column (dichloromethane/methanol = 20:1) to give a yellow solid **A27-2** (345 mg, yield 24.85%). LCMS (ESI) m/z = 283.01/284.1 [M+H]⁺.

### Step 3:

To a solution of compound **A27-2** (150.00 mg, 529.81 µmol) in N,N-dimethylformamide (5.0 mL), tert-butyl (piperidin-4-ylmethyl)carbamate (113.54 mg, 529.81 µmol), DIPEA (205.42 mg, 1.59 mmol), and potassium iodide (17.59 mg, 105.96 µmol, 5.64 µL) were added at room temperature. The reaction mixture was stirred at 90°C for 12 h. After the addition of water, the reaction mixture was extracted with ethyl acetate, and the layers were separated. The organic phase was washed with saturated brine, dried over anhydrous sodium sulfate, concentrated under reduced pressure to remove the solvent, and separated for purification by a silica gel column (dichloromethane/methanol = 10:1) to give a light yellow solid **A27-3** (118 mg, yield 53.47%). LCMS (ESI) m/z = 417.2 [M+H]+.

### Step 4:

To a solution of compound **A27-3** (20.00 mg, 48.02 µmol) in dichloromethane (6.0 mL) at room temperature, trifluoroacetic acid (1.5 mL) was added. The reaction mixture was stirred at 25°C for 2 h. The solvent was removed by concentration under reduced pressure to give a yellow solid **A27-4** (16 mg, yield 100%). LCMS (ESI) m/z = 317.1 [M+H]⁺.

### Step 5:

To a solution of compound **A27-4** (16.00 mg, 48.02 µmol) in N,N-dimethylformamide (2.0 mL) at room temperature, **SM2** (15.00 mg, 28.91 µmol), HATU (21.98 mg, 57.81 µmol), and DIPEA (22.41 mg, 173.44 µmol) were added. The reaction mixture was stirred at room temperature for 2 h. Reverse-phase preparative chromatography (acetonitrile/10 M aqueous ammonium bicarbonate solution = 1.5/1) was used to separate for purification to give a white solid **A27** (5.45 mg, yield 23.07%). LCMS (ESI) m/z = 817.56 [M+H]⁺. ¹H NMR (400 MHz, DMSO-*d*₆) δ 10.35 (s, 1H), 10.17 (s, 1H), 8.71 (s, 1H), 8.30 (d, *J =* 8.4 Hz, 1H), 7.96 (s, 1H), 7.92 (d, *J* = 6.0 Hz, 1H), 7.88 - 7.73 (m, 2H), 7.47 - 7.36 (m, 2H), 7.34 - 7.24 (m, 4H), 7.22 (s, 1H), 3.91 (s, 3H), 3.87 - 3.81 (m, 2H), 3.77 (t, *J* = 6.4 Hz, 2H), 3.43 (s, 2H), 3.19 (t, *J* = 6.4 Hz, 2H), 2.71 - 2.53 (m, 2H), 2.51 - 2.49 (m, 1H), 2.20 - 2.11 (m, 2H), 1.87 - 1.84 (m, 2H), 1.59 - 1.57 (m, 2H).

### Example 28 Synthesis of Compound A28:

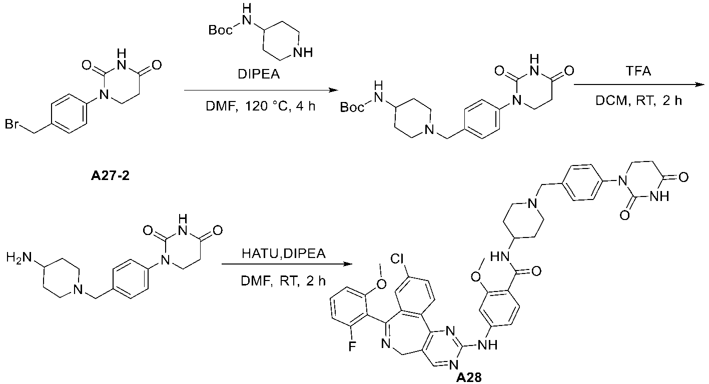

Compound **A28** (10.55 mg, yield 45.44%) was synthesized according to the synthesis method of compound **A27** described in Example 27. LCMS (ESI) m/z = 803.5 [M+H]⁺. ¹H NMR (400 MHz, DMSO-*d*₆) δ 10.36 (s, 1H), 10.18 (s, 1H), 8.72 (s, 1H), 8.30 (d, *J =* 8.8 Hz, 1H), 7.96 (s, 1H), 7.90 (d, *J* = 7.6 Hz, 1H), 7.86 - 7.75 (m, 2H), 7.49 - 7.37 (m, 2H), 7.30 (q, *J =* 8.8 Hz, 4H), 7.22 (s, 1H), 3.92 (s, 3H), 3.83 (bs, 2H), 3.78 (t, *J* = 6.8 Hz, 2H), 3.47 (s, 2H),3.33(s, 1H), 2.70 (t, *J* = 6.8 Hz, 4H), 2.14 (t, *J* = 8.8 Hz, 2H), 1.83 - 1.79 (m, 2H), 1.55 (q, *J =* 10.8 Hz, 2H).

### Example 29 Synthesis of Compound A29:

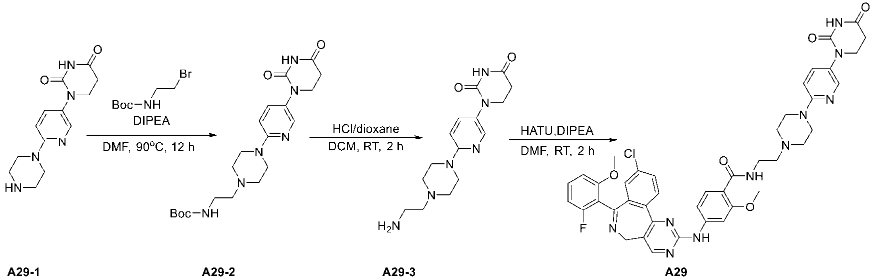

### Step 1:

To a solution of compound **A29-1** (100.00 mg, 363.23 µmol, synthesized with reference to patent WO2020132561) in *N,N*-dimethylformamide (5.0 mL) at room temperature, tert-butyl (2-bromoethyl)carbamate (81.40 mg, 363.23 µmol) and DIPEA (140.83 mg, 1.09 mmol) were added. The reaction mixture was stirred at 90°C for 12 h. After the addition of water, the reaction mixture was extracted with ethyl acetate, and the layers were separated. The organic phase was washed with saturated brine, dried over anhydrous sodium sulfate, concentrated under reduced pressure to remove the solvent, and separated for purification by a silica gel column (dichloromethane/methanol = 10:1) to give a light yellow solid **A29-2** (30 mg, yield 19.74%). LCMS (ESI) m/z = 419.3 [M+H]⁺.

### Step 2:

At room temperature, HCl (4 M in dioxane, 1.0 mL) was added to a solution of compound **A29-2** (29.00 mg, 69.30 µmol) in dichloromethane (2.0 mL). The reaction mixture was stirred at 25°C for 2 h. The solvent was removed by concentration under reduced pressure to give a yellow solid **A29-3** (30 mg, yield 100%). LCMS (ESI) m/z = 319.2 [M+H]⁺.

### Step 3:

To a solution of compound **A29-3** (30.00 mg, 69.30 µmol) in *N,N-*dimethylformamide (2.0 mL) at room temperature, **SM2** (15.00 mg, 28.91 µmol), HATU (21.98 mg, 57.81 µmol), and DIPEA (22.41 mg, 173.44 µmol) were added. The reaction mixture was stirred at room temperature for 2 h. Reverse-phase preparative chromatography (acetonitrile/10 M aqueous ammonium bicarbonate solution = 1.5/1) was used to separate for purification to give a light yellow solid **A29** (14.25 mg, yield 58.97%). LCMS (ESI) m/z = 819.1 [M+H]⁺. ¹H NMR (400 MHz, DMSO-*d*₆) δ 10.34 (s, 1H), 10.18 (s, 1H), 8.72 (s, 1H), 8.37 - 8.23 (m, 2H), 8.08 (d, *J =* 2.8 Hz, 1H), 7.99 (s, 1H), 7.89 (d, *J =* 8.8 Hz, 1H), 7.83 (dd, *J*₁ = 8.4 Hz, *J*₂ = 2.0 Hz, 1H), 7.51 (dd, *J*₁ = 8.8 Hz, *J*₂ = 2.8 Hz, 1H), 7.42 (td, *J*₁ = 8.8 Hz, *J*₂ = 6.8 Hz, 2H), 7.21 (s, 1H), 6.91 (s, 1H), 6.88 (d, *J* = 9.2 Hz, 1H), 3.94 (s, 3H), 3.70 (t, *J* = 6.8 Hz, 2H), 3.53 (bs, 4H), 3.49 - 3.41 (m, 2H), 2.70 (t, *J* = 6.8 Hz, 2H), 2.55 (d*, J* = 6.8 Hz, 6H).

### Example 30 Synthesis of Compound A30:

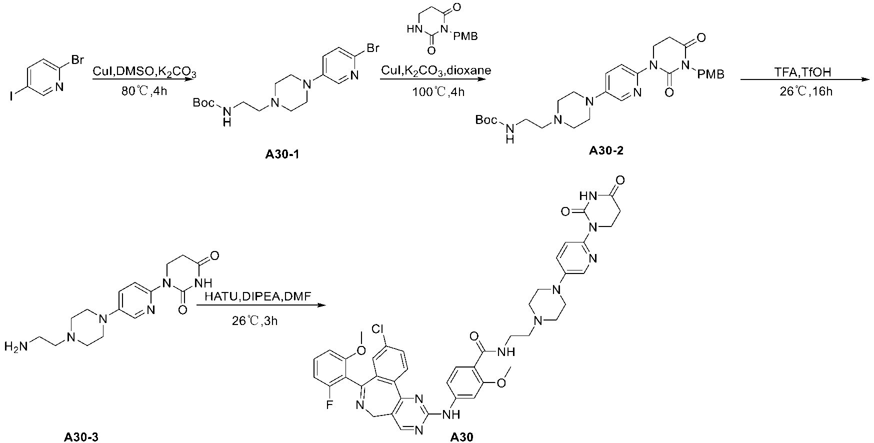

### Step 1:

At room temperature, potassium carbonate (4.87 g, 34.87 mmol), cuprous iodide (670.9 mg, 3.49 mmol), L-proline (405.5 mg, 3.49 mmol) and 1-(N-Boc-aminoethyl)piperazine (4.40 g, 19.18 mmol) were added to a solution of compound 2-bromo-5-iodopyridine (5.00 g, 17.44 mmol) in dimethyl sulfoxide (50 mL). The reaction mixture was heated to 80°C under argon protection and stirred for 4 h. The reaction mixture was washed with water and extracted with ethyl acetate, and the organic phase was spin-dried to remove the solvent. The residue was separated for purification by silica gel column chromatography (dichloromethane/methanol = 20:1) to give a white solid **A30-1** (6.00 g, 90%). LCMS (ESI) m/z = 385.1 [M+H]⁺.

### Step 2:

At room temperature, compound **A30-1** (500.0 mg, 1.30 mmol), cesium carbonate (845.5 mg, 2.60 mmol), trans-(1S, 2S)-N,N-dimethylcyclohexanediamine (37.0 mg, 0.26 mmol), cuprous iodide (49.0 mg, 0.26 mmol), 3-(4-methoxybenzyl)-dihydropyrimidine-2,4 (1H,3H)-dione (395.0 mg, 1.69 mmol) were dissolved in dioxane (5 mL), and the reaction mixture was stirred at 80°C for 4 h. The reaction mixture was concentrated to remove the solvent, and the residue was separated for purification by silica gel column chromatography (dichloromethane/methanol = 10:1) to give a white solid **A30-2** (400.0 mg, 57%). LCMS (ESI) m/z = 539.2 [M+H]⁺.

### Step 3:

At 0°C, trifluoroacetic acid (5 mL) and trifluoromethanesulfonic acid (1 mL) were added to compound **A30-2** (400.0 mg, 0.74 mmol), and the reaction mixture was stirred at 25°C for 16 h. The reaction mixture was adjusted to pH≈8 with saturated aqueous potassium bicarbonate solution, and separated for purification by reverse phase chromatography (acetonitrile/10 M aqueous ammonium bicarbonate solution) to give a white solid **A30-3** (100.0 mg, 42%). LCMS (ESI) m/z = 319.1 [M+H]⁺.

### Step 4:

To a solution of compound **A30-3** (15.4 mg, 0.048 mmol) in N,N-dimethylformamide (1 mL), **SM2** (15.0 mg, 0.029 mmol), HATU (13.2 mg, 0.034 mmol) and DIPEA (18.7 mg, 0.14 mmol) were added. The reaction mixture was stirred at room temperature for 3 h. The reaction mixture was separated for purification by reverse phase chromatography (acetonitrile/0.1% aqueous formic acid solution = 2.3/1) to give a yellow solid **A30** (3.9 mg, yield 16%). LCMS (ESI) m/z = 819.2 [M+H]⁺. ¹H NMR (400 MHz, DMSO-*d*₆) δ 10.45 (s, 1H), 10.23 (s, 1H), 8.77 (s, 1H), 8.31 (d, *J =* 2.8 Hz, 2H), 8.12 (d, *J =* 2.8 Hz, 1H), 8.00 (s, 1H), 7.93 (d, *J* = 8.4 Hz, 1H), 7.85 (dd, *J*₁ = 8.4 Hz, *J*₂ =2.4 Hz, 1H), 7.57 (d, *J* = 9.2 Hz, 1H), 7.52 - 7.44 (m, 3H), 7.26 (s, 1H), 3.98 (s, 3H), 3.96 - 3.94 (m, 2H), 3.47 - 3.42 (m, 2H), 3.30 - 3.22 (m, 4H), 3.24 (s, 3H), 2.68 (t, 2H), 2.55 - 2.50 (m, 4H), 2.06 - 2.01 (m, 2H).

### Example 31 Synthesis of Compound A31:

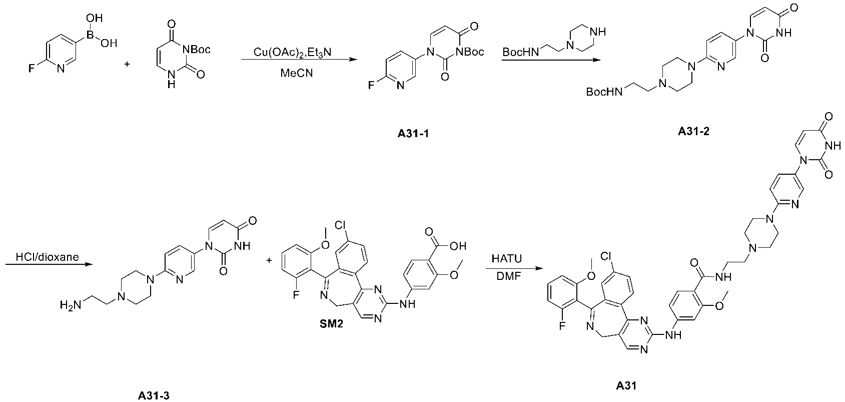

### Step 1:

2-Fluoro-5-pyridineboronic acid (600 mg, 4.26 mmol) was dissolved in acetonitrile, and tert-butyl 2,4-dicarbonyl-1H-pyrimidine-3-carboxylate (903.58 mg, 4.26 mmol, synthesized with reference to WO2021255212), copper acetate monohydrate (170.03 mg, 851.62 µmol) and triethylamine (860.14 mg, 8.52 mmol) were added in sequence. The reaction system was stirred at 60°C for 2 h. After the reaction was completed, the mixture was concentrated under reduced pressure to give a crude product, which was purified by flash silica gel column chromatography (PE:EA=50% to 100%) to give **A31-1** (320 mg). LCMS (ESI) m/z = 308.1 [M+H]⁺.

### Step 2:

**A31-1** (300 mg, 976.32 µmol) was dissolved in DMSO (1 mL), and 1-(*N*-Boc-aminoethyl)piperazine (447.78 mg, 1.95 mmol) and triethylamine (197.22 mg, 1.95 mmol) were added successively, and the mixture was stirred at 100°C for 2 h. After the reaction was completed, water (10 mL) and ethyl acetate (20 mL) were added to the system, the organic phase was separated, dried over sodium sulfate, and concentrated under reduced pressure to give crude product **A31-2,** which was directly used in the next step. LCMS (ESI) m/z = 317.1 [M+H]⁺.

### Step 3:

**A31-2** obtained in the previous step was dissolved in HCl (4.0 M in dioxane, 2 mL) and stirred at room temperature for 1 h. The reaction mixture was concentrated to remove the solvent to give a white solid **A31-3,** which was used directly in the next step. LCMS (ESI) m/z = 317.2 [M+H]⁺.

### Step 4:

**A31-3** (20 mg, 63.22 µmol) obtained in the previous step was dissolved in *N,N*-dimethylformamide (1 mL), and **SM2** (16.40 mg, 31.61 µmol), HATU (18.03 mg, 47.41 µmol) and DIPEA (12.26 mg, 94.83 µmol, 16.52 µL) were added successively. The reaction mixture was stirred overnight at room temperature. The reaction mixture was separated for purification by reverse phase chromatography (acetonitrile/0.1% aqueous formic acid solution = 2.3/1) to give a yellow solid A31 (2 mg, yield 9%). LCMS (ESI) m/z = 817.2 [M+H]⁺. ¹H NMR (400 MHz, DMSO-*d*₆) δ 11.44 (s, 1H), 10.21 (s, 1H), 8.74 (s, 1H), 8.36 - 8.29 (m, 2H), 8.14 (d, *J* = 2.4 Hz, 1H), 8.02 (s, 1H), 7.91 (d, *J =* 8.4 Hz, 1H), 7.85 (dd, *J*₁ = 8.2 Hz, *J*₂ = 2.6 Hz 1H), 7.47 - 7.41 (m, 2H), 7.24 (s, 1H), 7.01 (s, 1H), 6.95 (d, *J =* 9.2 Hz, 1H), 5.67 (d, *J =* 8.0 Hz, 1H), 3.97 (s, 3H), 3.61 - 3.59 (m, 4H), 3.51 - 3.46 (m, 2H), 2.59 - 2.53 (m, 6H).

### Example 32 Synthesis of Compound A32:

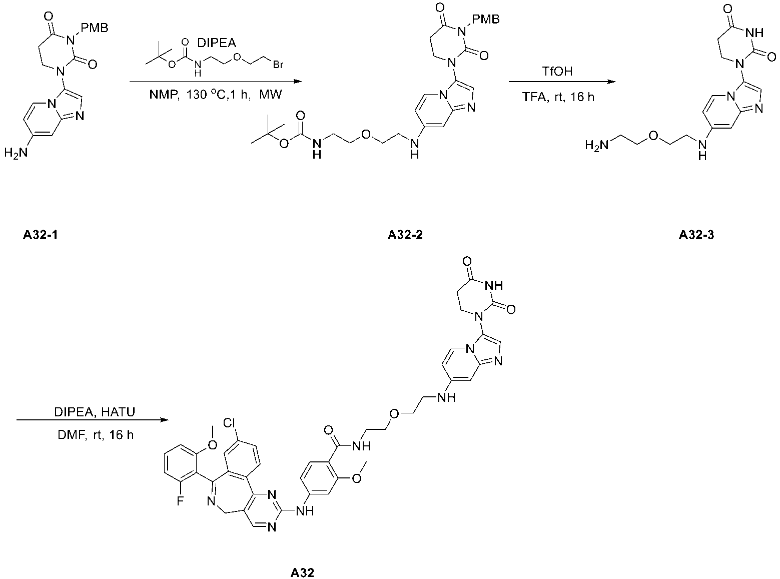

### Step 1:

Compound **A32-1** (440.0 mg, 1.07 mmol, synthesized with reference to patent WO2021188948) was dissolved in N-methylpyrrolidone solution (3 mL), and tert-butyl (2-(2-bromoethoxy)ethyl)carbamate (353.4 mg, 1.32 mmol) and DIPEA (227.1 mg, 1.76 mmol) were added. The mixture was reacted under microwave conditions at 130°C for 1 h. After the addition of water (20 mL), the reaction mixture was extracted with dichloromethane (50 mL*3). The organic phases were pooled, washed with saturated brine (50 mL), dried over anhydrous sodium sulfate, filtered, and the filtrate was concentrated to give a crude product. The crude product was purified by column chromatography to give compound **A32-2** (306.0 mg, yellow solid). LCMS (ESI) m/z = 553.2 [M+H].

### Step 2:

At room temperature, compound **A32-2** (306.0 mg, 0.55 mmol) was dissolved in trifluoroacetic acid (6 mL), trifluoromethanesulfonic acid (2 mL) was added, and the reaction mixture was stirred at 25°C for 16 h. The reaction mixture was adjusted to pH ~5 with a saturated sodium bicarbonate solution, and separated for purification by reverse phase chromatography (acetonitrile/10 M aqueous ammonium bicarbonate solution) to give compound **A32-3** (76.0 mg, white solid). LCMS (ESI) m/z = 333.40 [M+H]⁺.

### Step 3:

To a solution of compound **A32-3** (19.2 mg, 0.058 mmol) in N,N-dimethylformamide (2 mL), **SM2** (15.0 mg, 0.029 mmol), HATU (16.5 mg, 0.043 mmol) and DIPEA (11.2 mg, 0.087 mmol) were added. The reaction mixture was stirred at room temperature for 16 h. The reaction mixture was separated for purification by reverse phase chromatography (methanol/10 M aqueous ammonium bicarbonate solution) to give a white solid **A32** (7.0 mg, yield 29%). LCMS (ESI) m/z = 833.5 [M+H]⁺. ¹H NMR (400 MHz, DMSO-*d*₆) δ 10.22 (s, 1H), 8.75 (s, 1H), 8.52 (s, 1H), 8.40 - 8.28 (m, 2H), 8.13 (t, *J* = 5.6 Hz, 1H), 7.96 (s, 1H), 7.89 - 7.78 (m, 3H), 7.52-7.40 (m, 2H), 7.26 (s, 3H), 6.86 (dd, *J* = 7.6, 2.0 Hz, 1H), 6.77 (d, *J* = 2.0 Hz, 1H), 4.33 (t, *J* = 4.8 Hz, 2H), 3.91 (s, 3H), 3.85 (t, *J =* 4.8 Hz, 2H), 3.77 (t, *J =* 6.4 Hz, 2H), 3.60 (t, *J =* 6.0 Hz, 2H), 3.50 - 3.44 (m, 2H), 2.81 (bs, 2H).

### Example 33 Synthesis of Compound A33:

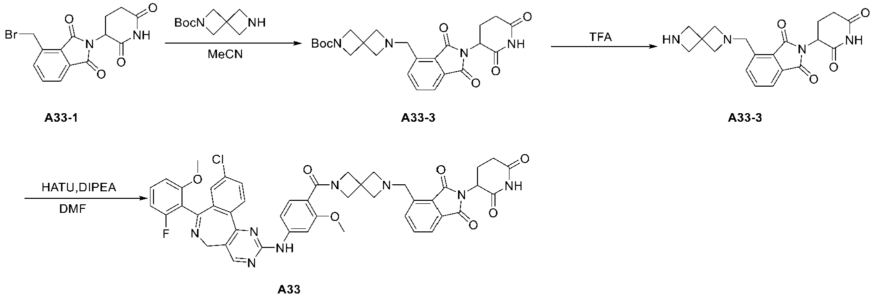

### Step 1:

**A33-1** (200 mg, 569.55 µmol, synthesized with reference to WO2020173440) was dissolved in DMSO (2 mL), tert-butyl 2,6-diazaspiro[3.3]heptane-2-carboxylate (112.92 mg, 569.55 µmol) and DIPEA (368.05 mg, 2.85 mmol, 496.03 µL) were added successively, and the mixture was stirred overnight at room temperature. After the reaction was completed, water (10 mL) and ethyl acetate (20 mL) were added to the system, the organic phase was separated and dried over sodium sulfate, and concentrated under reduced pressure to give a crude product which was purified by flash silica gel column chromatography (PE:EA=40% to 100%) to give **A33-2** (30 mg). LCMS (ESI) m/z = 469.1 [M+H]⁺.

### Step 2:

**A33-2** obtained in the previous step was dissolved in dichloromethane (2 mL), trifluoroacetic acid (1 mL) was added, and the reaction mixture was stirred at room temperature for 1 h. The reaction mixture was concentrated to remove the solvent to give an oily substance **A33-3,** which was used directly in the next step. LCMS (ESI) m/z = 369.1 [M+H]⁺.

### Step 3:

**A33-3** (23.58 mg, 64 µmol) obtained in the previous step was dissolved in N,N-dimethylformamide (1 mL), and **SM2** (20.86 mg, 42.24 µmol), HATU (36.50 mg, 96.00 µmol) and DIPEA (24.81 mg, 192.00 µmol, 33.44 µL) were added successively. The reaction mixture was stirred at room temperature for 2 h. The reaction mixture was separated for purification by reverse phase chromatography (acetonitrile/0.1% aqueous formic acid solution = 2.3/1) to give a yellow solid **A33** (12.2 mg, yield 21%). LCMS (ESI) m/z = 869.2 [M+H]⁺. ¹H NMR (400 MHz, CD₃OD-d₄) δ 8.62 (s, 1H), 8.39 (d, *J =* 8.2 Hz, 1H),7.96 (s, 1H), 7.85 - 7.81 (m, 3H), 7.75 (dd, *J*₁ = 8.4 Hz, *J*₂ = 2.4 Hz, 1H), 7.49 - 7.45 (m, 1H), 7.38 - 7.31 (m, 3H), 5.19 - 5.14 (m, 1H), 4.61 (s, 1H), 4.24 - 4.16 (m, 6H), 3.94 (s, 3H), 3.60 - 3.52 (m, 4H), 2.91 - 2.75 (m, 3H), 2.17 - 2.16 (m, 1H).

### Example 34 Synthesis of Compound A34:

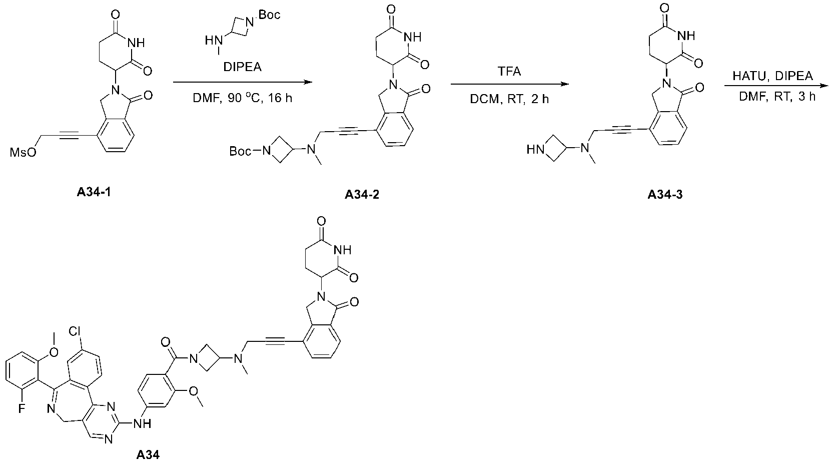

### Step 1:

To a solution of **A34-1** (63.00 mg, 167.38 µmol, synthesized with reference to patent CN112079866) in *N,N-*dimethylformamide (2.0 mL) at room temperature, tert-butyl 3-(methylamino)azetidine-1-carboxylate (46.76 mg, 251.07 µmol) and DIPEA (64.90 mg, 502.15 µmol) were added. The reaction mixture was stirred at 90°C for 16 h. After the addition of water, the reaction mixture was extracted with ethyl acetate, and the layers were separated. The organic phase was washed with saturated brine, dried over anhydrous sodium sulfate, concentrated under reduced pressure to remove the solvent, and separated for purification by a silica gel column (dichloromethane/methanol = 10:1) to give a yellow solid **A34-2** (37 mg, yield 47.38%). LCMS (ESI) m/z = 467.1 [M+H]⁺.

### Step 2:

To a solution of compound **A34-2** (37.00 mg, 79.31 µmol) in dichloromethane (4.0 mL) at room temperature, trifluoroacetic acid (1.0 mL) was added. The reaction mixture was stirred at 25°C for 2 h. The solvent was removed by concentration under reduced pressure to give a brown solid **A34-3** (30 mg, yield 100%). LCMS (ESI) m/z = 367.2 [M+H]⁺.

### Step 3:

To a solution of compound **A34-3** (30.00 mg, 61.79 µmol) in *N,N*-dimethylformamide (2.0 mL) at room temperature, **SM2** (15.00 mg, 28.91 µmol), HATU (21.98 mg, 57.81 µmol), and DIPEA (22.41 mg, 173.44 µmol) were added. The reaction mixture was stirred at room temperature for 2 h. Reverse-phase preparative chromatography (acetonitrile/10 M aqueous ammonium bicarbonate solution = 1.5/1) was used to separate for purification to give a light yellow solid **A34** (21.91 mg, yield 86.67%). LCMS (ESI) m/z = 867.1 [M+H]+. ¹H NMR (400 MHz, DMSO-*d*₆) δ 10.98 (s, 1H), 10.08 (s, 1H), 8.70 (s, 1H), 8.29 (d, *J=* 8.4 Hz, 1H), 7.90 (s, 1H), 7.80 (dd, *J*₁ = 8.4 Hz, *J*₂ = 2.4 Hz, 1H), 7.71 (dd, *J*₁ = 19.2 Hz, *J*₂ = 7.2 Hz, 2H), 7.53 (t, *J =* 7.6 Hz, 1H), 7.47 - 7.30 (m, 2H), 7.30 - 7.16 (m, 2H), 5.12 (dd, *J*₁ = 13.2 Hz, *J*₂ *=* 5.2 Hz, 1H), 4.54 - 4.29 (m, 2H), 3.99-3.95 (m, 2H), 3.91-3.89 (m, 1H), 3.86 (s, 3H), 3.65 (s, 2H), 3.51-3.49 (m, 1H), 3.03 - 2.82 (m, 1H), 2.70 - 2.55 (m, 2H), 2.26 (s, 3H), 2.07-2.03 (m, 1H).

### Example 35 Synthesis of Compound A35:

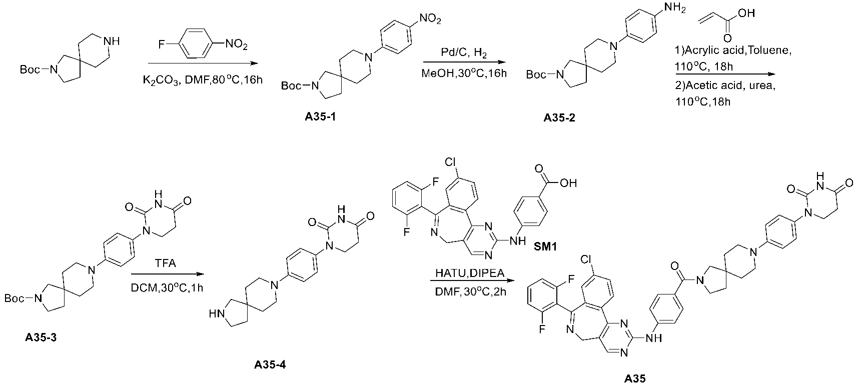

Compound **A35** (11.6 mg, yield 46.8%) was synthesized according to the synthesis method of compound **A8** described in **Example 8.** LCMS (ESI) m/z = 787.1 [M+H]⁺. ¹H NMR (400 MHz, DMSO-*d*₆) δ 10.24 (d, *J =* 8.0 Hz, 1H), 10.13 (s, 1H), 8.71 (s, 1H), 8.30 (d, *J =* 8.0 Hz, 1H), 7.92 - 7.84 (m, 3H), 7.55 (t, *J* = 8.0 Hz, 3H), 7.35 - 7.31 (m, 1H), 7.22 - 7.08 (m, 4H), 7.00 - 6.87 (m, 2H), 3.68 (t, *J =* 7.2 Hz, 2H), 3.58 (d, *J* = 7.2 Hz, 2H), 3.40 (s, 2H), 3.28 - 3.07 (m, 4H), 2.70 - 2.64 (m, 2H), 1.87 - 1.77 (m, 2H), 1.70 (s, 2H), 1.58 (s, 2H).

### Example 36 Synthesis of Compound A36:

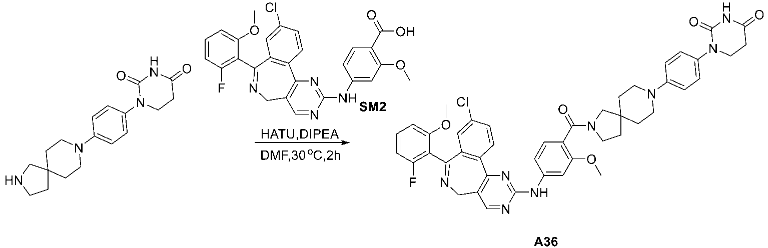

Compound **A36** (14.5 mg, yield 58%) was synthesized according to the synthesis method of compound A1 described in **Example 1.** LCMS (ESI) m/z = 829.1 [M+H]⁺. ¹H NMR (400 MHz, DMSO-*d*₆) δ 10.24 (d, *J =* 8.0 Hz, 1H), 10.03 (d, *J =* 4.0 Hz, 1H), 8.68 (d, *J =* 4.0 Hz, 1H), 8.33 - 8.26 (m, 1H), 7.89 (s, 1H), 7.83 - 7.77 (m, 1H), 7.45 - 7.34 (m, 2H), 7.21 (s, 1H), 7.17 - 7.08 (m, 3H), 6.96 (d, *J* = 8.0 Hz, 1H), 6.90 (d, *J =* 8.0 Hz, 1H), 3.82 (s, 3H), 3.72 - 3.63 (m, 2H), 3.50 (d, *J =* 8.0 Hz, 1H), 3.38 - 3.35 (m, 1H), 3.31 - 3.06 (m, 5H), 3.05 - 2.96 (m, 1H), 2.70 - 2.63 (m, 2H), 1.84 - 1.72 (m, 2H), 1.70 - 1.50 (m, 4H).

### Example 37 Synthesis of Compound A37:

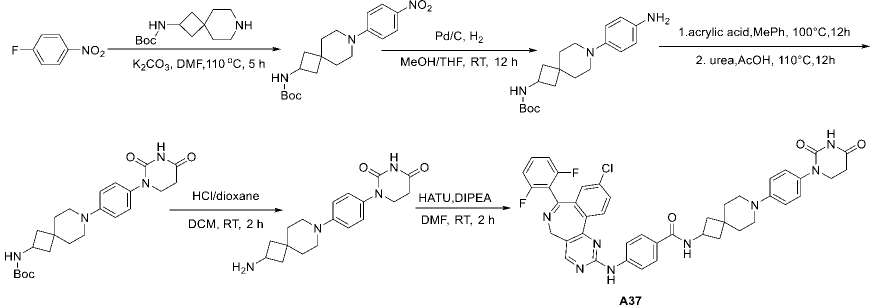

Compound **A37** (18.19 mg, yield 72.43%) was synthesized according to the synthesis method of compound **A8** described in **Example 8.** LCMS (ESI) m/z = 787.5 [M+H]⁺. ¹H NMR (400 MHz, DMSO-*d*₆) δ 10.23 (s, 1H), 10.13 (s, 1H), 8.73 (s, 1H), 8.42 (d, *J =* 8.8 Hz, 1H), 8.30 (d, *J =* 8.3 Hz, 1H), 7.97 - 7.78 (m, 5H), 7.61-7.53 (m, 1H), 7.34 (d, *J =* 2.0 Hz, 1H), 7.22 - 7.09 (m, 4H), 6.94 (d, *J =* 8.8 Hz, 2H), 4.52 - 4.43 (m, 1H), 3.69 (t, *J* = 6.8 Hz, 2H), 3.18 - 3.14 (m, 2H), 3.11 (m, 2H), 2.68 (t, *J* = 6.8 Hz, 2H), 2.28 - 2.23 (m, 2H), 1.93 - 1.88 (m, 2H), 1.75 - 1.73 (m, 2H), 1.70 - 1.68 (m, 2H).

### Example 38 Synthesis of Compound A38:

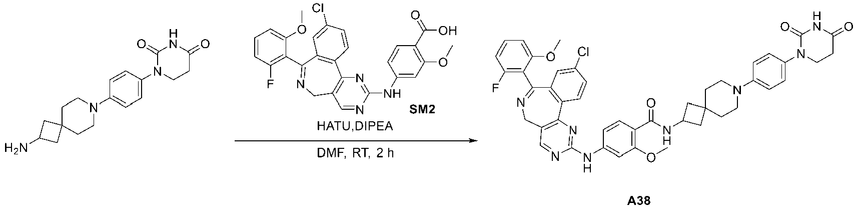

Compound **A38** (18.57 mg, yield 77.46%) was synthesized according to the synthesis method of compound **A1** described in **Example 1.** LCMS (ESI) m/z = 829.1 [M+H]⁺. ¹H NMR (400 MHz, DMSO-*d*₆) δ 10.24 (s, 1H), 10.16 (s, 1H), 8.72 (s, 1H), 8.30 (d, *J* = 8.4 Hz, 1H), 8.11 (d, *J* = 7.6 Hz, 1H), 7.94 (s, 1H), 7.82 (dd, *J*₁ = 6.0 Hz, *J*₁ = 2.4 Hz, 1H), 7.75 (d, *J =* 8.4 Hz, 1H), 7.50 - 7.36 (m, 2H), 7.22 (s, 1H), 7.13 (d, *J =* 8.4 Hz, 2H), 6.94 (d, *J* = 8.8 Hz, 2H), 4.42 (m, 1H), 3.93 (s, 3H), 3.69 (t, *J* = 6.4 Hz, 2H), 3.24 - 2.97 (m, 4H), 2.68 (t, *J* = 6.4 Hz, 2H), 2.32-2.27 (m, 2H), 1.87-1.82 (m, 2H), 1.76 - 1.58 (m, 4H).

### Example 39 Synthesis of Compound A39:

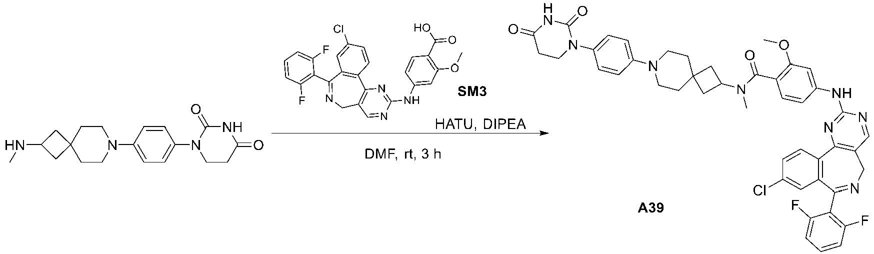

Compound **A39** (9.0 mg, yield 36%) was synthesized according to the synthesis method of compound A1 described in **Example 1.** LCMS (ESI) m/z = 801.1 [M+H]⁺. ¹H NMR (400 MHz, DMSO-*d*₆) δ 10.26 (s, 1H), 10.14 (s, 1H), 8.74 (s, 1H), 8.33 (d, *J =* 8.4 Hz, 1H), 7.97 - 7.82 (m, 3H), 7.66 - 7.48 (m, 1H), 7.44 - 7.31 (m, 3H), 7.25 - 7.09 (m, 4H), 6.93 (d, *J =* 8.8 Hz, 2H), 4.59 (s, 1H), 3.71 (t, *J* = 6.4 Hz, 2H), 3.20 - 3.03 (m, 4H), 2.99 (s, 3H), 2.70 (t, *J* = 6.8 Hz, 2H), 2.11 - 2.02 (d, *J* = 9.8 Hz, 4H), 1.74 - 1.53 (m, 4H).

### Example 40 Synthesis of Compound A40:

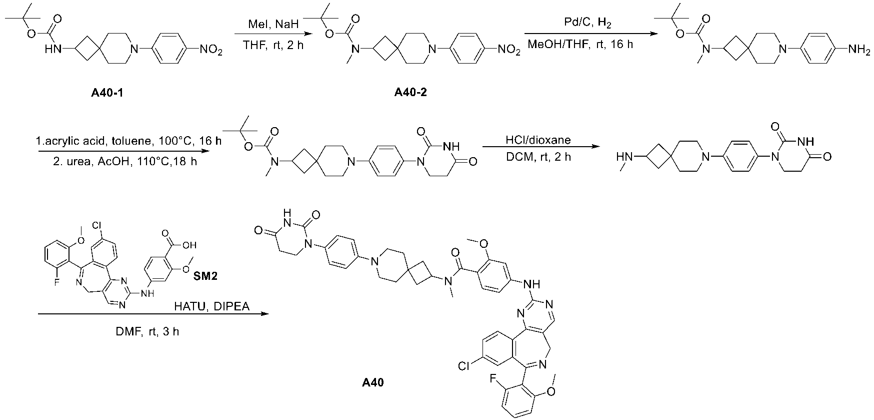

### Step 1:

Compound **A40-1** (269.0 mg, 0.74 mmol, synthesized with reference to patent WO2020147702) was dissolved in tetrahydrofuran (5 mL), and sodium hydride (119.1 mg, 2.98 mmol) was added under argon protection at 0°C. The reaction mixture was stirred at 0°C for 10 min under argon protection. Methyl iodide (316.9 mg, 2.23 mmol) was added, and the reaction mixture was stirred at room temperature for 2 h under argon protection. After the addition of water (50 mL), the reaction mixture was extracted with dichloromethane (50 mL*3). The organic phases were pooled, washed with saturated brine (100 mL), dried over anhydrous sodium sulfate, and filtered. The filtrate was concentrated to give compound **A40-2** (380.0 mg, yellow solid, crude). LCMS (ESI) m/z = 376.3 [M+H]⁺.

### Step 2:

Compound **A40** (11.0 mg, yield 45%) was synthesized according to the synthesis method of compound **A8** described in **Example 8.** LCMS (ESI) m/z = 843.1 [M+H]⁺. ¹H NMR (400 MHz, DMSO-*d*₆) δ 10.22 (s, 1H), 10.03 (s, 1H), 8.68 (s, 1H), 8.28 (d, *J =* 8.4 Hz, 1H), 7.93 - 7.72 (m, 2H), 7.48 - 7.29 (m, 2H), 7.20 (s, 1H), 7.17 - 6.98 (m, 3H), 6.98 - 6.76 (m, 3H), 4.24 - 4.05 (m, 1H), 3.76 (s, 3H), 3.70 - 3.63 (m, 2H), 3.14 - 2.91 (m, 6H), 2.76 (s, 1H), 2.72 - 2.60 (m, 2H), 2.15 - 1.79 (m, 4H), 1.77 - 1.39 (m, 4H).

### Example 41 Synthesis of Compound A41:

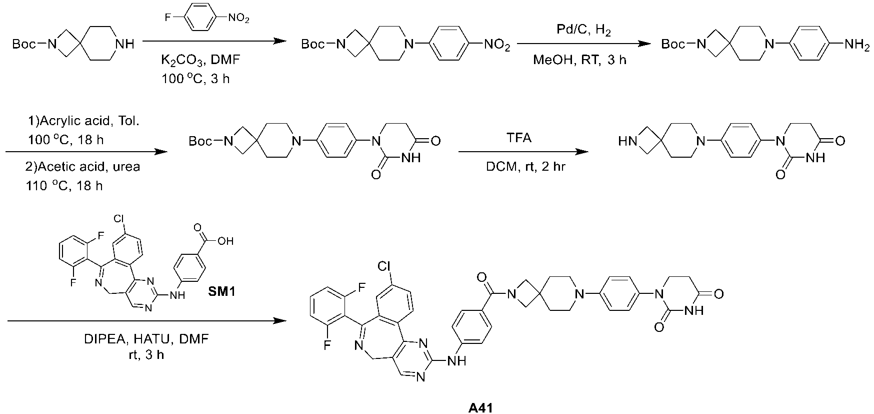

Compound **A41** (11 mg, yield 45.2%) was synthesized according to the synthesis method of compound **A8** described in **Example 8.** LCMS (ESI) m/z = 773.1 [M+H]⁺. ¹H NMR (400 MHz, DMSO-*d*₆) δ 10.24 (s, 1H), 10.17 (s, 1H), 8.72 (s, 1H), 8.30 (d, *J =* 8.4 Hz, 1H), 7.97 - 7.85 (m, 3H), 7.67 (d, *J =* 8.4 Hz, 2H), 7.58 - 7.54 (m, 1H), 7.33 (d, *J =* 2.0 Hz, 1H), 7.21 - 7.15 (m, 4H), 6.94 (d, *J =* 8.8 Hz, 2H), 4.13 (s, 2H), 3.79 (s, 2H), 3.69 (t, *J* = 6.8 Hz, 2H), 3.20 - 3.13 (m, 4H), 2.67 (t, *J* = 6.8 Hz, 2H), 1.83 (s, 4H).

### Example 42 Synthesis of Compound A42:

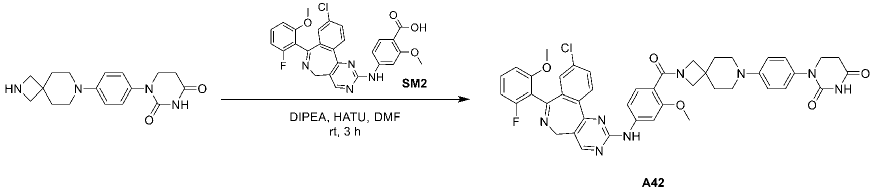

Compound **A42** (9.4 mg, yield 39.8%) was synthesized according to the synthesis method of compound **A1** described in **Example 1.** LCMS (ESI) m/z = 815.1 [M+H]⁺. ¹H NMR (400 MHz, DMSO-*d*₆) δ 10.23 (s, 1H), 10.06 (s, 1H), 8.69 (s, 1H), 8.29 (d, *J =* 8.4 Hz, 1H), 7.89 (s, 1H), 7.81 (dd, *J*₁ = 8.4 Hz, *J*₂ = 2.2 Hz, 1H), 7.46 - 7.34 (m, 2H), 7.29 - 7.19 (m, 2H), 7.12 (d, *J =* 8.8 Hz, 2H), 7.01 - 6.89 (m, 3H), 3.84 (s, 3H), 3.76 - 3.62 (m, 6H), 3.22 - 3.03 (m, 4H), 2.67 (t, *J* = 6.6 Hz, 2H), 1.78 (s, 4H).

### Example 43 Synthesis of Compound A43:

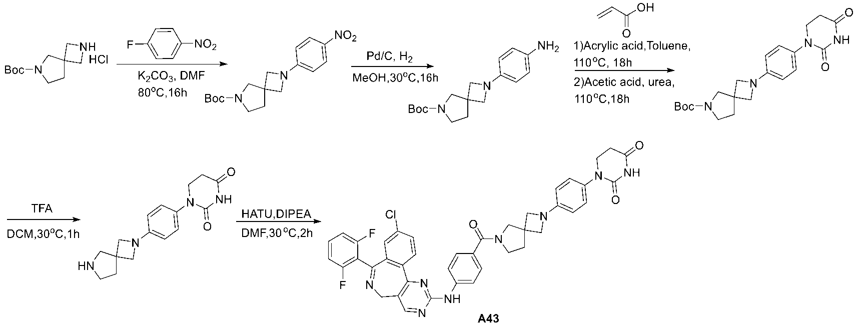

Compound **A43** (16.2 mg, yield 67.8%) was synthesized according to the synthesis method of compound **A8** described in **Example 8.** LCMS (ESI) m/z = 759.1 [M+H]⁺. ¹H NMR (400 MHz, DMSO-*d*₆) δ 10.20 (s, 1H), 10.12 (s, 1H), 8.71 (s, 1H), 8.30 (d, *J =* 8.0 Hz, 1H), 7.93 - 7.85 (m, 3H), 7.58 - 7.52 (m, 3H), 7.33 (d, *J =* 2.0 Hz, 1H), 7.20 - 7.06 (m, 4H), 6.50 - 6.38 (m, 2H), 3.89 - 3.54 (m, 10H), 2.70 - 2.64 (m, 2H), 2.14 (s, 2H).

### Example 44 Synthesis of Compound A44:

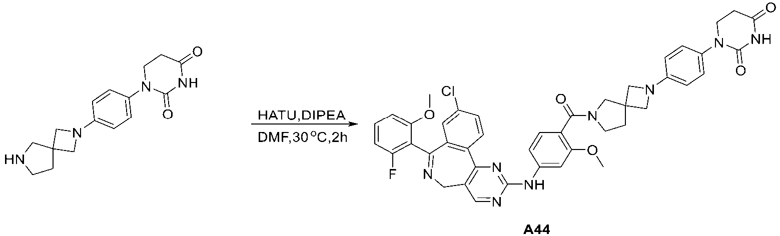

Compound **A44** (11.6 mg, yield 48.2%) was synthesized according to the synthesis method of compound **A1** described in **Example 1.** LCMS (ESI) m/z = 801.1 [M+H]⁺. ¹H NMR (400 MHz, DMSO-*d*₆) δ 10.22 (d, *J =* 8.0 Hz, 1H), 10.04 (s, 1H), 8.69 (d, *J =* 4.0 Hz, 1H), 8.32 - 8.26 (m, 1H), 7.90 (s, 1H), 7.83 - 7.76 (m, 1H), 7.46 - 7.34 (m, 2H), 7.21 (s, 1H), 7.17 - 7.06 (m, 3H), 6.54 - 6.44 (m, 2H), 3.86 - 3.65 (m, 9H), 3.56 - 3.29 (m, 4H), 2.70 - 2.63 (m, 2H), 2.17 - 2.06 (m, 2H).

### Example 45 Synthesis of Compound A45:

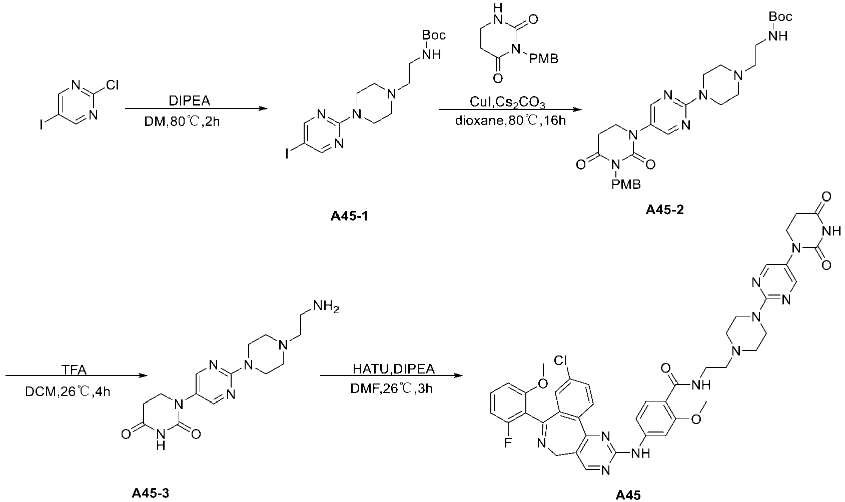

### Step 1:

At room temperature, 1-(*N*-Boc-aminoethyl)piperazine (77.5 mg, 0.26 mmol) was added to a solution of compound 2-chloro-5-iodopyrimidine (1.00 g, 4.16 mmol) in *N,N*-dimethylformamide and DIEA (10 mL/2 mL), and the reaction mixture was heated to 80°C and stirred for 2 h. The reaction mixture was washed with water, and extracted with ethyl acetate. The organic phase was concentrated to remove the solvent. The residue was separated for purification by silica gel column chromatography (dichloromethane/methanol = 10:1) to give a white solid **A45-1** (1.18 g, 65%). LCMS (ESI) m/z = 434.1 [M+H]⁺.

### Step 2:

At room temperature, compound **A45-1** (1.18 g, 2.72 mmol), cesium carbonate (1.77 g, 5.45 mmol), trans-(1S, 2S)-*N,N*-dimethylcyclohexanediamine (1.77 g, 0.54 mmol), cuprous iodide (103.0 mg, 0.54 mmol), 3-(4-methoxybenzyl)-dihydropyrimidine-2,4(1H,3H)-dione (637.5 mg, 2.72 mmol) were dissolved in dioxane (15 mL), and the reaction mixture was stirred at 80°C for 16 h. The reaction mixture was concentrated to remove the solvent, and the residue was separated for purification by silica gel column chromatography (dichloromethane/methanol = 10:1) to give a white solid **A45-2** (1.20 g, 81%). LCMS (ESI) m/z = 540.1 [M+H]⁺.

### Step 3:

Trifluoromethanesulfonic acid solution (3 mL) was added to a solution of compound **A45-2** (300.0 mg, 0.56 mmol) in trifluoroacetic acid (3 mL), and the reaction mixture was stirred at room temperature for 4 h. The reaction mixture was separated for purification by reverse phase chromatography (acetonitrile/10 M aqueous ammonium bicarbonate solution = 2.3/1) to give a white solid **A45-3** (100.0 mg, yield 56%). LCMS (ESI) m/z = 340.3 [M+H]⁺.

### Step 4:

To a solution of compound **A45-3** (15.4 mg, 0.048 mmol) in N,N-dimethylformamide (1 mL), **SM2** (15.0 mg, 0.029), HATU (13.2 mg, 0.034 mmol) and DIPEA (18.7 mg, 0.14 mmol) were added. The reaction mixture was stirred at room temperature for 3 h. The reaction mixture was separated for purification by reverse phase chromatography (acetonitrile/0.1% aqueous formic acid solution = 2.3/1) to give a yellow solid A45 (5.8 mg, yield 24%). LCMS (ESI) m/z = 820.1 [M+H]⁺. ¹H NMR (400 MHz, DMSO-*d*₆) δ 10.47 (s, 1H), 10.23 (s, 1H), 8.76 (s, 1H), 8.30 (s, 2H), 8.38 - 8.32 (m, 2H), 8.04 (s, 1H), 7.92 (d, *J =* 8.8 Hz, 1H), 7.85 (dd, *J*₁ *=* 8.4 Hz, *J*₂ = 2.4 Hz, 1H), 7.48 - 7.44 (m, 2H), 7.25 (s, 1H), 3.96 (s, 3H), 3.83 - 3.80(m, 4H), 3.74 (t, *J* = 6.8 Hz, 2H), 3.49 - 3.47 (m, 2H), 2.76 (t, *J =* 6.8 Hz, 2H), 2.58 - 2.51 (m, 2H).

### Example 46 Synthesis of Compound A46:

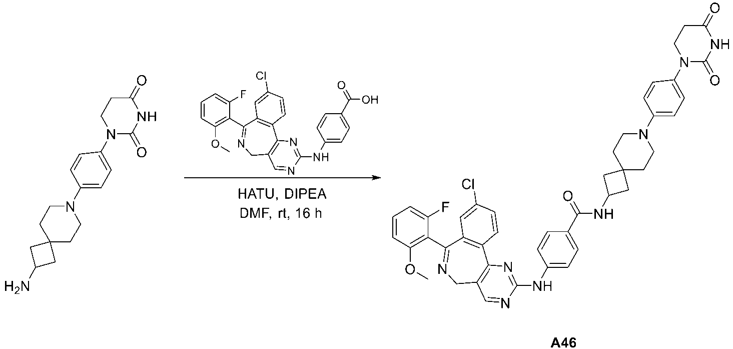

Compound **A46** (15.0 mg, yield 61%) was synthesized according to the synthesis method of compound **A1** described in **Example 1.** LCMS (ESI) m/z = 799.5 [M+H]⁺. ¹H NMR (400 MHz, DMSO-*d*₆) δ 10.29 (s, 1H), 10.18 (s, 1H), 8.74 (s, 1H), 8.47 (d, *J =* 7.6 Hz, 1H), 8.29 (d, *J =* 8.4 Hz, 1H), 7.95 (d, *J =* 8.4 Hz, 2H), 7.91 - 7.80 (m, 3H), 7.45 (q, *J =* 8.0 Hz, 1H), 7.25 (s, 1H), 7.16 (d, *J =* 8.8 Hz, 2H), 6.97 (d, *J* = 9.2 Hz, 2H), 4.51 - 4.44 (m, 1H), 3.72 (t, *J =* 6.4 Hz, 2H), 3.24 - 3.14 (m, 2H), 3.14 - 3.03 (m, 2H), 2.71 (t, *J* = 6.8 Hz, 2H), 2.27 - 2.22 (m, 2H), 1.93 - 1.88 (m, 2H), 1.80 - 1.62 (m, 4H).

### Example 47 Synthesis of Compound A47:

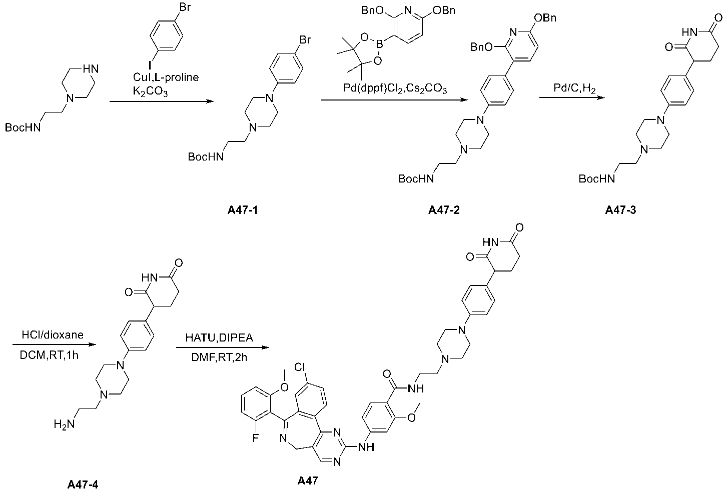

### Step 1:

To a solution of compound 1-(N-Boc-aminoethyl)piperazine (2.7 g, 11.7 mmol) in dimethyl sulfoxide (15 mL) at room temperature, 4-bromoiodobenzene (3 g, 10.6 mmol), potassium carbonate (2.93 g, 21.2 mmol), cuprous iodide (404 mg, 2.12 mmol), and L-proline (244 mg, 2.12 mmol) were added. The reaction mixture was heated to 80°C under argon protection and stirred for 16 h. After the addition of water, the reaction mixture was extracted with ethyl acetate, dried over anhydrous sodium sulfate, and concentrated under reduced pressure. The residue was separated for purification by silica gel column chromatography (ethyl acetate/petroleum ether = 1:3) to give a white solid **A47-1** (2.8 g, yield 69%). LCMS (ESI) m/z = 384.9 [M+H]⁺.

### Step 2:

To a solution of compound **A47-1** (400 mg, 1.04 mmol) in dioxane (10 mL) and water (1 mL), 2,6-bis(phenylmethoxy)-3-(4,4,5,5-tetramethyl-1,3,2-dioxaborolan-2-yl)-pyridine (456 mg, 10.6 mmol), cesium carbonate (678 mg, 2.08 mmol), and [1,1'-bis(diphenylphosphino)ferrocene]dichloropalladium, complex with dichloromethane (85 mg, 0.1 mmol) were added at room temperature. The reaction mixture was heated to 100°C under argon protection and stirred for 16 h. After the addition of water, the reaction mixture was extracted with ethyl acetate, dried over anhydrous sodium sulfate, and concentrated under reduced pressure. The residue was separated for purification by silica gel column chromatography (ethyl acetate/petroleum ether = 1:2) to give a white solid **A47-2** (298 mg, yield 48%). LCMS (ESI) m/z = 595.2 [M+H]⁺.

### Step 3:

10% palladium on carbon (50 mg, 0.05 mmol) was added to a solution of compound **A47-2** (298 mg, 0.5 mmol) in methanol (5 mL) at room temperature. The atmosphere was replaced with hydrogen 3 times, and the mixture was heated to 35°C and stirred for 16 h. The reaction mixture was filtered, and the filtrate was concentrated under reduced pressure to give a white solid **A47-3** (153 mg, yield 73%). LCMS (ESI) m/z *=* 417.1 [M+H]⁺.

### Step 4:

HCl (4 M in dioxane, 0.5 mL) was added to a solution of compound **A47-3** (30 mg, 0.07 mmol) in dichloromethane (2 mL) at room temperature. The reaction mixture was stirred at 30°C for 1 h. The reaction mixture was concentrated under reduced pressure to give a light yellow solid **A47-4** (23 mg, yield 99%). LCMS (ESI) m/z = 317.2 [M+H]⁺.

### Step 5:

**SM2** (15 mg, 0.03 mmol), HATU (14 mg, 0.04 mmol) and DIPEA (12.2 mg, 0.09 mmol) were added to a solution of compound **A47-4** (23 mg, 0.07 mmol) in N,N-dimethylformamide (2 mL) at room temperature. The reaction mixture was stirred at room temperature for 2 h. The reaction mixture was separated for purification by reverse phase chromatography (acetonitrile/10 M aqueous ammonium bicarbonate solution) to give a light yellow solid **A47** (13.3 mg, yield 54.2%). LCMS (ESI) m/z = 817.1 [M+H]⁺. ¹H NMR (400 MHz, DMSO-*d*₆) δ 10.77 (s, 1H), 10.19 (s, 1H), 8.71 (s, 1H), 8.34 - 8.27 (m, 2H), 7.97 (s, 1H), 7.89 (d, *J =* 8.8 Hz, 1H), 7.82 (dd, *J*₁ = 8.0 Hz, *J*₂ = 2.0 Hz, 1H), 7.46 - 7.38 (m, 2H), 7.21 (s, 1H), 7.06 (d, *J =* 8.0 Hz, 2H), 6.92 (d, *J =* 8.0 Hz, 2H), 3.93 (s, 3H), 3.76 - 3.70 (m, 1H), 3.49 - 3.41 (m, 2H), 3.20 - 3.12 (m, 4H), 2.69 - 2.63 (m, 2H), 2.62 - 2.57 (m, 4H), 2.57 - 2.53 (m, 2H), 2.18 - 2.08 (m, 1H), 2.06 - 1.96 (m, 1H).

### Example 48 Synthesis of Compound A48:

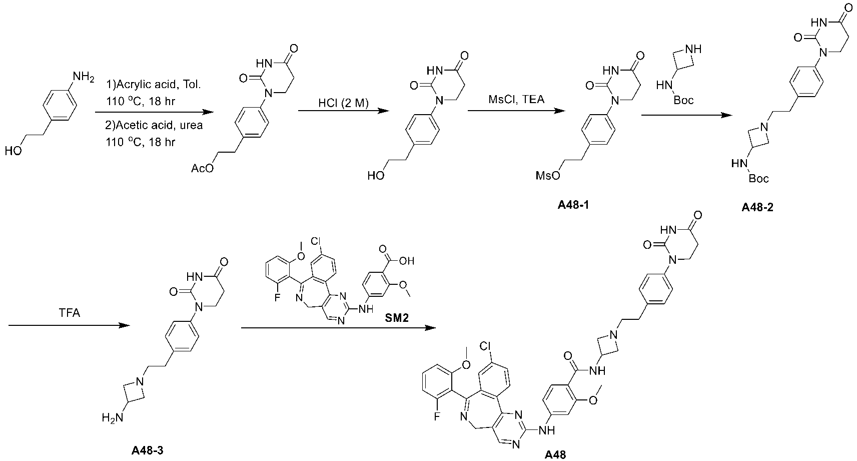

### Step 1:

Compound **A48-1** was synthesized according to the synthesis method of compound **A8** described in **Example 8.** LCMS (ESI) m/z = 313.1 [M+H]⁺.

### Step 2:

DIPEA (496.54 mg, 3.84 mmol, 669.20 µL) and 3-*N*-tert-butoxycarbonylaminocyclobutylamine (441.12 mg, 2.56 mmol) were added to a solution of **A48-1** (400 mg, 1.28 mmol) in *N,N*-dimethylformamide (4 mL) at room temperature. The reaction mixture was stirred at 80°C for 4 h. With addition of saturated brine, the reaction mixture was extracted with ethyl acetate, and the organic phase then was dried over anhydrous sodium sulfate, filtered, and concentrated. The crude product was separated for purification by silica gel column chromatography (dichloromethane/methanol = 20:1) to give a white solid **A48-2** (150 mg, yield 30.1%). LCMS (ESI) m/z = 389.0 [M+H]⁺.

### Step 3:

Trifluoroacetic acid (1.49 g, 13.07 mmol, 1 mL) was added to a solution of **A48-2** (60 mg, 154.46 µmol) in dichloromethane (2 mL) at room temperature. The reaction mixture was stirred at room temperature for 2 h. The reaction mixture was directly concentrated under reduced pressure to give a crude colorless oil **A48-3** (60 mg) which was directly used in the next step. LCMS (ESI) m/z = 289.1 [M+H]⁺.

### Step 4:

Compound **A48** (10 mg, yield 43.8%) was synthesized according to the synthesis method of compound A1 described in **Example 1.** LCMS (ESI) m/z = 789.5 [M+H]⁺. ¹H NMR (400 MHz, DMSO-*d*₆) δ 10.33 (s, 1H), 10.18 (s, 1H), 8.72 (s, 1H), 8.32 (d, *J =* 7.8 Hz, 1H), .8.25 (d, *J =* 7.8 Hz, 1H), 7.94 (s, 1H), 7.86 - 7.71 (m, 2H), 7.46 - 7.37 (m, 2H), 7.23 (s, 6H), 4.44 (m, 1H), 3.93 (s, 3H), 3.76 (t, *J =* 6.6 Hz, 2H), 3.58 (t, *J =* 7.0 Hz, 2H), 2.93 (t, *J =* 7.0 Hz, 2H), 2.74 - 2.54 (m, 6H).

### Example 49 Synthesis of Compound A49:

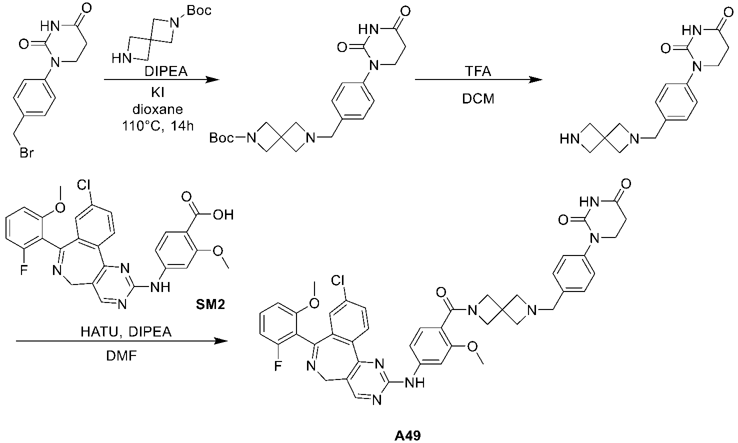

Compound **A49** (5.0 mg, yield 21%) was synthesized according to the synthesis method of compound **A27** described in **Example 27.** LCMS (ESI) m/z = 801.5 [M+H]⁺. ¹H NMR (400 MHz, DMSO-*d*₆) δ 10.34 (s, 1H), 10.08 (s, 1H), 8.70 (s, 1H), 8.29 (d, *J =* 8.4 Hz, 1H), 7.89 (s, 1H), 7.81 (d, *J =* 8.4 Hz, 1H), 7.47 - 7.38 (m, 1H), 7.37 - 7.30 (m, 1H), 7.28 - 7.19 (m, 6H), 4.05 (s, 2H), 4.00 (s, 2H), 3.82 (s, 3H), 3.76 (t, *J = 6.6* Hz, 2H), 3.49 (s, 2H), 3.29 (s, 2H), 3.24 (d, *J =* 7.4 Hz, 2H), 2.69 (t, *J* = 6.6 Hz, 2H).

### Example 50 Synthesis of Compound A50:

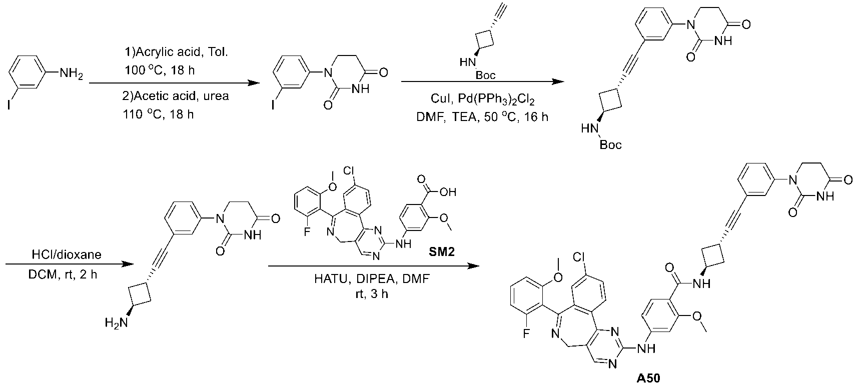

Compound **A50** (5.5 mg, yield 24.2%) was synthesized according to the synthesis method of compound A6 described in **Example 6.** LCMS (ESI) m/z = 784.4 [M+H]⁺. ¹H NMR (400 MHz, DMSO-*d*₆) δ 10.40 (s, 1H), 10.17 (s, 1H), 8.71 (s, 1H), 8.32 (d, *J* = 8.0 Hz, 1H), 8.28 (d, *J =* 8.0 Hz, 1H), 7.94 (s, 1H), 7.85 - 7.73 (m, 2H), 7.47 - 7.26 (m, 6H), 7.22 (s, 1H), 4.72 (m, 1H), 3.93 (s, 3H), 3.80 (t, *J* = 6.6 Hz, 2H), 3.38 - 3.34 (m, 1H), 2.70 (t, *J* = 6.6 Hz, 2H), 2.47 - 2.38 (m, 4H).

### Example 51 Synthesis of Compound A51:

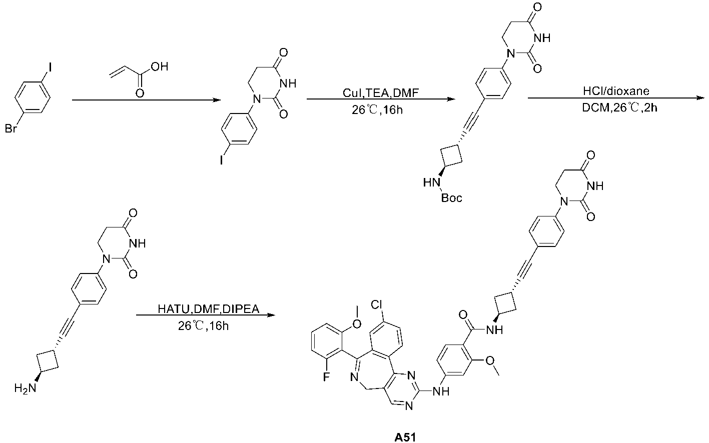

Compound **A51** (5.0 mg, yield 22%) was synthesized according to the synthesis method of compound **A6** described **in Example 6.** LCMS (ESI) m/z = 784.2 [M+H]⁺. ¹H NMR (400 MHz, DMSO-*d*₆) δ 10.45 (s, 1H), 10.21 (s, 1H), 8.75 (s, 1H), 8.35 - 8.30 (m, *J* = 7.6 Hz, 2H), 7.97 (s, 1H), 7.85 (d, *J =* 8.4 Hz, 1H), 7.78 (d, *J* = 8.4 Hz, 1H), 7.50 - 7.43 (m, *J* = 7.6 Hz, 4H), 7.38 (s, 1H), 7.35 (s, 1H), 7.25 (s, 1H), 4.75 - 4.70 (m, 1H), 3.96 (s, 3H), 3.84 (t, *J* = 6.4 Hz, 2H), 3.38 - 3.34 (m,1H), 2.76 - 2.74 (t, *J* = 6.4 Hz, 2H), 2.49 - 2.45 (m, 4H).

### Example 52 Synthesis of Compound A52:

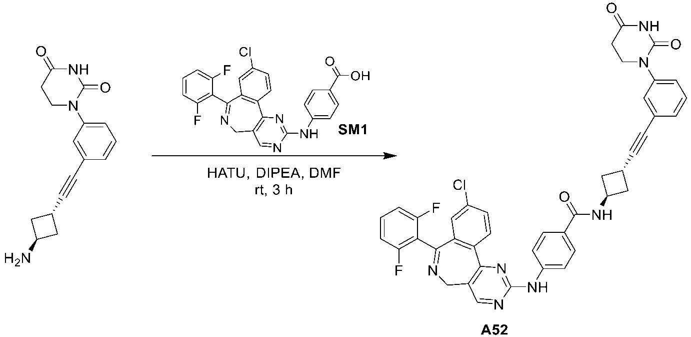

Compound **A52** (13 mg, yield 55.6%) was synthesized according to the synthesis method of compound A6 described in **Example 6.** LCMS (ESI) m/z = 742.0 [M+H]⁺. ¹H NMR (400 MHz, DMSO-*d*₆) δ 10.41 (s, 1H), 10.18 (s, 1H), 8.73 (s, 1H), 8.63 (d, *J =* 7.6 Hz, 1H ), 8.32 (d, *J* = 7.6 Hz, 1H), 7.94 - 7.82 (m, 5H), 7.61 - 7.53 (m, 1H), 7.43 - 7.24 (m, 5H), 7.17 (t, *J =* 8.9 Hz, 2H), 4.77 - 4.65 (m, 0.7H), 4.48 - 4.35 (m, 0.3 H), 3.84 - 3.75 (m, 2H), 3.38 - 3.34 (m, 0.7 H), 3.05 - 2.95 (m, 0.3 H), 2.74 - 2.62 (m, 3H), 2.55 - 2.51 (m, 1H), 2.48 - 2.37 (m, 1.4H), 2.34 - 2.22 (m, 0.6H).

### Example 53 Synthesis of Compound A53:

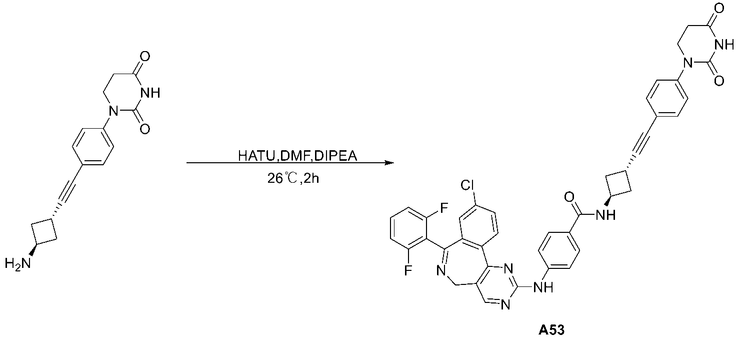

Compound **A53** (6.0 mg, yield 27%) was synthesized according to the synthesis method of compound **A6** described in **Example 6.** LCMS (ESI) m/z = 742.23 [M+H]⁺. ¹H NMR (400 MHz, DMSO-*d*₆) δ 10.46 (s, 1H), 10.21 (s, 1H), 8.77 (s, 1H), 8.62 (d, *J =* 7.2 Hz, 1H), 8.37 - 8.33 (m, 1H), 7.95 - 7.83 (m, 6H), 7.60 - 7.56 (m, 1H), 7.48 - 7.42 (m, 1H),7.37 - 7.31 (m, 3H), 7.22 - 7.17 (m, 2H), 4.74 - 4.72 (m, 1H), 3.85 (t, *J =* 6.4 Hz, 2H), 3.31 - 3.29 (m, 1H), 2.74 - 2.65 (m, 3H), 2.51 - 2.34 (m, 3H).

### Example 54 Synthesis of Compound A54:

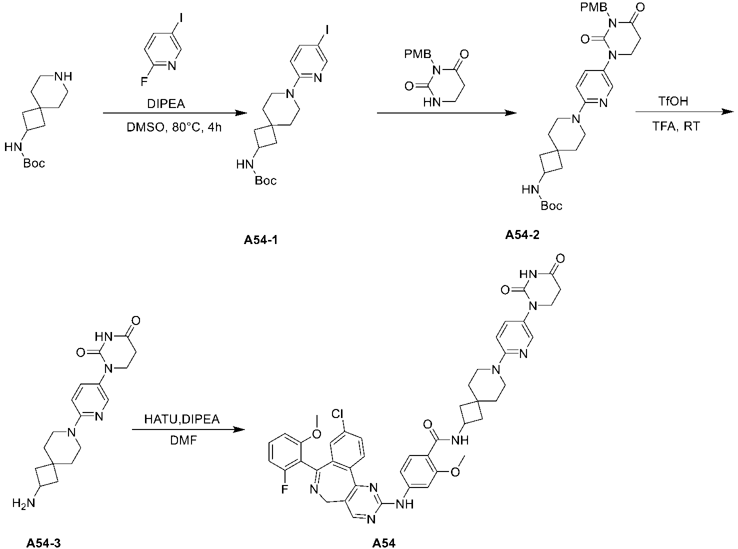

### Step 1:

To a solution of compound tert-butyl 7-azaspiro[3.5]nonane-2-carbamate (200.0 mg, 0.89 mmol) in dimethyl sulfoxide (2 mL), 2-fluoro-5-iodopyridine (215.6 mg, 0.89 mmol) and DIPEA (1.3 g, 2.69 mmol) were added at room temperature. The reaction mixture was stirred at 110°C for 4 h. The reaction mixture was separated for purification by reverse phase chromatography (acetonitrile/0.1% aqueous formic acid solution) to give a yellow solid **A54-1** (284 mg, 71%). LCMS (ESI) m/z = 444.0 [M+H]⁺. ¹H NMR (400 MHz, DMSO-*d*₆) δ 8.21 (d, *J =* 2.4 Hz, 1H), 7.70 (dd, *J*₁ = 9.0 Hz, *J*₂ = 2.4 Hz, 1H), 7.11 (d, *J =* 8.0 Hz, 1H), 6.73 (d, *J=* 9.0 Hz, 1H), 3.98 - 3.88 (m, 1H), 3.47 - 3.41 (m, 2H), 3.39 - 3.34 (m, 2H), 2.13-2.08 (m, 2H), 1.68 - 1.63 (m, 2H), 1.52 (t, *J =* 5.6 Hz, 2H), 1.49 - 1.43 (m, 2H), 1.37 (s, 9H).

### Step 2:

At room temperature, 3-(4-methoxybenzyl)-dihydropyrimidine-2,4 (1H,3H)-dione (162.7 mg, 0.69 mmol), trans-(1R, 2R)-*N,N*'-dimethyl-1,2-cyclohexanediamine (89.8 mg, 0.63 mmol), cesium carbonate (411.5 mg, 1.26 mmol) and cuprous iodide (24.0 mg, 0.12 mmol) were added to a solution of compound **A54-1** (280 mg, 0.63 mmol) in 1,4-dioxane (10 mL). The reaction mixture was stirred at 110°C for 14 h. After the addition of water, the reaction mixture was extracted with ethyl acetate, dried over anhydrous sodium sulfate and concentrated to remove the solvent, and the residue was separated for purification by silica gel column chromatography (dichloromethane/methanol = 10:1) to give a yellow solid **A54-2** (270 mg, 77%). ¹H NMR (400 MHz, DMSO-*d*₆) δ 8.03 (d, *J* = 2.6 Hz, 1H), 7.46 (dd, *J*₁ = 9.0 Hz, *J*₂ = 2.8 Hz, 1H), 7.21 (d, *J* = 8.2 Hz, 2H), 7.11 (d, *J =* 7.9 Hz, 1H), 6.86 - 6.81 (m, 3H), 4.78 (s, 2H), 3.94 (q, *J =* 8.0 Hz, 1H), 3.72 (s, 3H), 3.70 - 3.68 (m,2H), 3.46 (t, *J =* 3.6 Hz, 2H), 3.38 t, *J =* 3.76 Hz, 2H), 2.87 (t, *J =* 6.6 Hz, 2H), 2.12 (t, *J =* 9.6 Hz, 2H), 1.67 (t, *J =* 10.0 Hz, 2H), 1.55 (t, *J* = 6.0 Hz, 2H), 1.48 (t, *J* = 6.0 Hz, 2H), 1.37 (s, 9H).

### Step 3:

At 0°C, trifluoroacetic acid (1 mL) and trifluoromethanesulfonic acid (0.2 mL) were added to compound **A54-2** (220.0 mg, 0.40 mmol), and the reaction mixture was stirred at 25°C for 14 h. The reaction mixture was adjusted to about pH 8 with saturated potassium bicarbonate, and separated for purification by reverse phase chromatography (acetonitrile/10 M aqueous ammonium bicarbonate solution) to give a white solid **A54-3** (100 mg, 75%). LCMS (ESI) m/z = 330.1 [M+H]⁺.

### Step 4:

To a solution of compound **A54-3** (19.0 mg, 0.057 mmol) in N,Ndimethylformamide (1 mL), **SM2** (15.0 mg, 0.029 mmol), HATU (13.2 mg, 0.034 mmol) and DIPEA (18.7 mg, 0.14 mmol) were added. The reaction mixture was stirred at room temperature for 2 h. The reaction mixture was separated for purification by reverse phase chromatography (acetonitrile/0.1% aqueous formic acid solution = 2.3/1) to give a yellow solid A54 (13.7 mg, yield 57%). LCMS (ESI) m/z = 830.6 [M+H]⁺. ¹H NMR (400 MHz, DMSO-*d*₆) δ 10.33 (s, 1H), 10.17 (s, 1H), 8.72 (s, 1H), 8.30 (d, *J =* 8.5 Hz, 1H), 8.12 (d, *J =* 7.4 Hz, 1H), 8.04 (d, *J* = 2.6 Hz, 1H), 7.94 (s, 1H), 7.82 (dd, *J*₁ = 8.4 Hz, *J*₂ = 2.2 Hz, 1H), 7.75 (d, *J* = 8.6 Hz, 1H), 7.49 - 7.39 (m, 3H), 7.22 (s, 1H), 6.85 (d, *J* = 9.0 Hz, 2H), 4.47 - 4.37 (m, 1H), 3.93 (s, 3H), 3.69 (t, *J =* 6.6 Hz, 2H), 3.54 - 3.52 (m, 2H), 3.48 - 3.45 (m, 2H), 2.69 (t, *J* = 6.6 Hz, 2H), 2.29 - 2.24 (m, 2H), 1.85 - 1.79 (m, 2H), 1.64 - 1.62 (m, 2H), 1.58 - 1.54 (m 2H).

### Example 55 Synthesis of Compound A55:

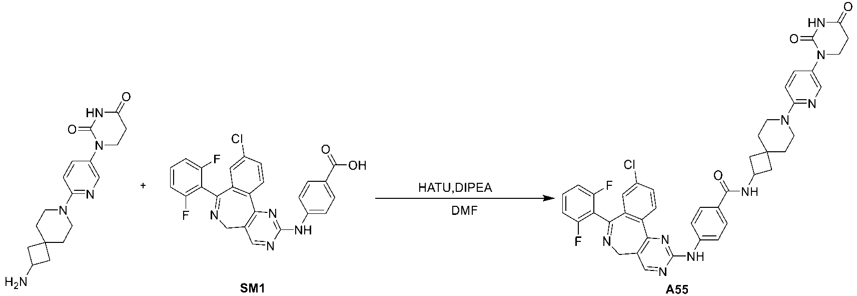

Compound **A55** (5.7 mg, yield 22%) was synthesized according to the synthesis method of compound **A1** described in **Example 1.** LCMS (ESI) m/z = 788.5 [M+H]⁺. ¹H NMR (400 MHz, DMSO-*d*₆) δ 10.33 (s, 1H), 10.16 (s, 1H), 8.73 (s, 1H), 8.44 (d, *J* = 7.4 Hz, 1H), 8.30 (d, *J =* 8.4 Hz, 1H), 8.04 (d, *J* = 2.7 Hz, 1H), 7.93-7.81 (m, 5H), 7.58 - 7.51 (m, 1H), 7.47 (dd, *J* = 9.0, 2.6 Hz, 1H), 7.34 (d, *J* = 2.0 Hz, 1H), 7.17 (t, *J =* 8.6 Hz, 2H), 6.85 (d, *J* = 9.0 Hz, 1H), 4.45 (q, *J* = 8.0 Hz, 1H), 3.69 (t, *J* = 6.6 Hz, 2H), 3.53 - 3.50 (m, 2H), 3.45 - 3.43 (m, 2H), 2.69 (t, *J* = 6.8 Hz, 2H), 2.29 - 2.24 (m, 2H), 1.94 - 1.89 (m, 2H), 1.67 - 1.62 (m, 2H), 1.59 - 1.57 (m, 2H).

### Example 56 Synthesis of Compound A56:

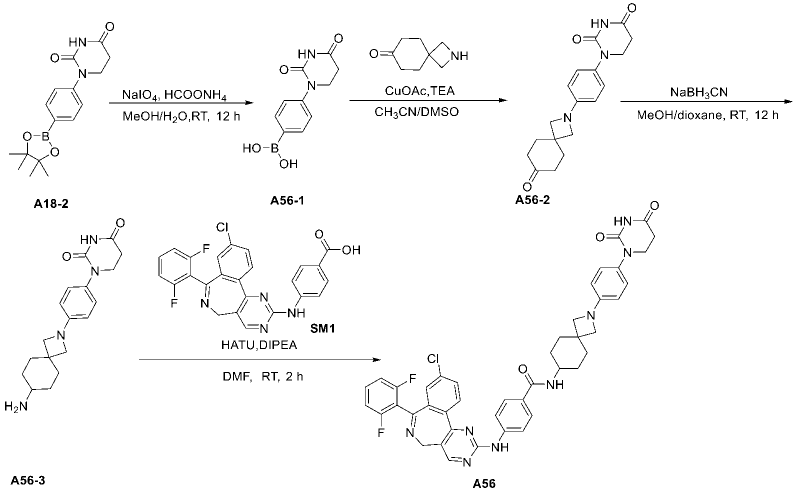

### Step 1:

Sodium periodate (449.55 mg, 2.10 mmol) and ammonium acetate (1.08 g, 14.01 mmol) were added to a mixed solution of compound **A18-2** (443.00 mg, 1.40 mmol) in methanol (20.0 mL) and water (10.0 mL) at room temperature. The reaction mixture was stirred at 25°C for 12 h. Solid impurities were removed by suction filtration, and the filtrate was concentrated, and separated for purification by a silica gel column (dichloromethane/methanol = 10:1) to give a white solid **A56-1** (285 mg, yield 86.92%). LCMS (ESI) m/z = 235.1 [M+H]⁺.

### Step 2:

To a mixed solution of compound **A56-1** (250.00 mg, 1.07 mmol) in dimethyl sulfoxide (7.0 mL) and acetonitrile (30.0 mL), 2-azaspiro[3.5]nonan-7-one (446.11 mg, 3.20 mmol), copper acetate (250 mg, 1.38 mmol), triethylamine (1.08 g, 10.68 mmol), and 4 Å molecular sieve (1.5 g) were added at room temperature. The reaction mixture was stirred at 25°C for 12 h. The reaction mixture was filtered to remove solid impurities, and the filtrate was concentrated. After the addition of water, the reaction mixture was extracted with dichloromethane, and the layers were separated. The organic phase was washed with saturated brine, dried over anhydrous sodium sulfate, concentrated under reduced pressure to remove the solvent, and separated for purification by a silica gel chromatography column (dichloromethane/methanol = 10:1) to give a green solid **A56-3** (256 mg, yield 73.20%). LCMS (ESI) m/z = 328.1 [M+H]⁺.

### Step 3:

To a mixed solution of compound **A56-3** (58.00 mg, 177.17 µmol) in methanol (5.0 mL) and acetonitrile (2.0 mL) at room temperature, ammonium acetate (68.21 mg, 885.83 µmol) and sodium cyanoborohydride (33.40 mg, 531.50 µmol) were added. The reaction mixture was stirred at 25°C for 12 h. The solvent was removed by concentration under reduced pressure, and the residue was separated for purification by reverse-phase preparative chromatography (acetonitrile/0.1% aqueous formic acid solution = 25%) to give a light green solid **A56-4** (15 mg, yield 25.78%). LCMS (ESI) m/z = 328.2 [M+H]⁺.

### Step 4:

To a solution of compound **A56-4** (15.00 mg, 45.67 µmol) in N,N-dimethylformamide (2 mL) at room temperature, **SM1** (5.00 mg, 10.49 µmol), HATU (7.97 mg, 20.97 µmol), and DIPEA (8.13 mg, 62.91 µmol) were added. The reaction mixture was stirred at room temperature for 2 h. Reverse-phase preparative chromatography (acetonitrile/10 M aqueous ammonium bicarbonate solution = 1.5/1) was used to separate for purification to give a white solid **A56** (6.36 mg, yield 77.05%). LCMS (ESI) m/z = 787.51 [M+H]⁺. ¹H NMR (400 MHz, DMSO-*d*₆) δ 10.23 (s, 1H), 10.15 (s, 1H), 8.73 (s, 1H), 8.30 (d, *J =* 8.4 Hz, 1H), 8.04 (d, *J =* 8.0 Hz, 1H), 7.96 - 7.86 (m, 3H), 7.83 (d, *J =* 8.8 Hz, 2H), 7.61 - 7.46 (m, 1H), 7.34 (s, 1H), 7.18 (d, *J =* 8.4 Hz, 2H), 7.10 (d, *J =* 8.0 Hz, 2H), 6.43 (d, *J =* 8.4 Hz, 2H), 3.79 (bs, 1H), 3.67 (t, *J* = 6.8 Hz, 2H), 3.59 (s, 2H), 3.51 (s, 2H), 2.67 (t, *J* = 6.8 Hz, 2H), 1.99 - 1.95 (m, 2H), 1.82 - 1.80 (m, 2H), 1.66 - 1.60 (m, 2H), 1.50 - 1.43 (m, 2H).

### Example 57 Synthesis of Compound A57:

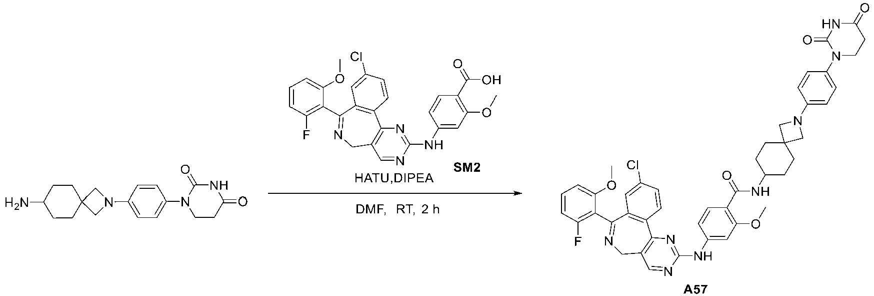

Compound A57 (6.29 mg, yield 78.72%) was synthesized according to the synthesis method of compound **A1** described in Example 1. LCMS (ESI) m/z = 829.01 [M+H]⁺. ¹H NMR (400 MHz, DMSO-*d*₆) δ 10.24 (s, 1H), 10.19 (s, 1H), 8.72 (s, 1H), 8.31 (d, *J =* 8.8 Hz, 1H), 7.96 (s, 1H), 7.89 - 7.76 (m, 3H), 7.50 - 7.38 (m, 2H), 7.22 (s, 1H), 7.10 (d, *J =* 8.0 Hz, 2H), 6.42 (d, *J =* 8.0 Hz, 2H), 3.93 (s, 3H), 3.82 (bs, 1H), 3.67 (t, *J* = 6.4 Hz, 2H), 3.58 (s, 2H), 3.52 (s, 2H), 2.67 (t, *J* = 6.8 Hz, 2H), 1.94 - 1.83 (m, 4H), 1.70 - 1.64 (m, 2H), 1.46 - 1.43 (m, 2H).

### Example 58 Synthesis of Compound A58:

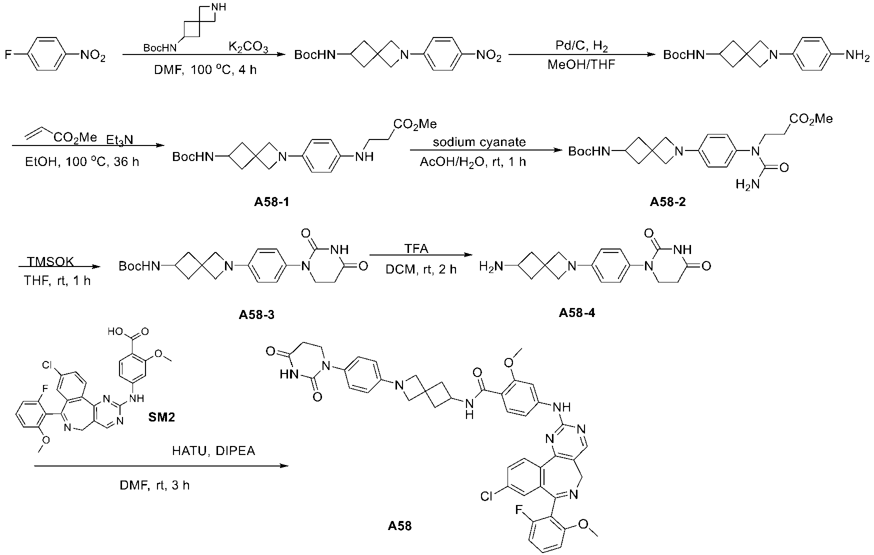

### Step 1:

Compound **A58-1** (157.0 mg, 0.40 mmol, synthesized with reference to **Example 8)** was dissolved in a mixed solution of acetic acid (2 mL) and water (0.4 mL), sodium cyanate (52.4 mg, 0.81 mmol) was added, and the reaction mixture was stirred at room temperature for 1 h. The reaction mixture was concentrated to give compound **A58-2** (200.0 mg, yellow solid, crude product). LCMS (ESI) m/z = 433.1 [M+H]⁺.

### Step 2:

Compound **A58-2** (174.0 mg, 0.40 mmol) was dissolved in tetrahydrofuran (4 mL), potassium trimethylsilanolate was added to adjust the pH to about 9, and the reaction mixture was stirred at room temperature for 1 h. The reaction mixture was adjusted to weak acidity with acetic acid, and concentrated to give a crude product. The crude product was purified by column chromatography to give compound **A58-3** (167.0 mg, yellow solid, crude product). LCMS (ESI) m/z = 401.2 [M+H]⁺.

### Step 3:

Compound **A58-3** (80.0 mg, 0.20 mmol) was dissolved in dichloromethane (3 mL), trifluoroacetic acid (1.5 mL) was added, and the reaction mixture was stirred at 25°C for 2 h. The reaction mixture was concentrated to remove the solvent to give **A58-4** (100.0 mg, yellow solid, crude product, trifluoroacetate). LCMS (ESI) m/z = 301.3 [M+H]⁺.

### Step 7:

To a solution of compound **A58-4** (23.9 mg, 0.058 mmol) in N,Ndimethylformamide (2 mL), **SM2** (15.0 mg, 0.029 mmol), HATU (16.5 mg, 0.043 mmol) and DIPEA (11.2 mg, 0.087 mmol) were added. The reaction mixture was stirred at room temperature for 3 h. The reaction mixture was separated for purification by reverse phase chromatography (acetonitrile/10 M aqueous ammonium bicarbonate solution) to give yellow solid A58 (10.0 mg, yield 43%). LCMS (ESI) m/z = 800.9 [M+H]⁺. ¹H NMR (400 MHz, DMSO-*d*₆) δ 10.26 (s, 1H), 10.21 (s, 1H), 8.75 (s, 1H), 8.33 (d, *J =* 8.4 Hz, 1H), 8.17 (d, *J =* 7.6 Hz, 1H), 7.97 (s, 1H), 7.85 (dd, *J*₁ = 8.4 Hz, *J*₂ = 2.4 Hz, 1H), 7.79 (d, *J =* 8.4 Hz, 1H), 7.51 - 7.36 (m, 2H), 7.25 (s, 1H), 7.18-7.06 (m, 2H), 6.51 - 6.36 (m, 2H), 4.48 - 4.28 (m, 1H), 3.96 (s, 3H), 3.91 (s, 2H), 3.80 (s, 2H), 3.69 (t, *J =* 6.4 Hz, 2H), 2.70 (t, *J* = 6.8 Hz, 2H), 2.60 - 2.55 (m, 2H), 2.33 - 2.28 (m, 2H).

### Example 59 Synthesis of Compound A59:

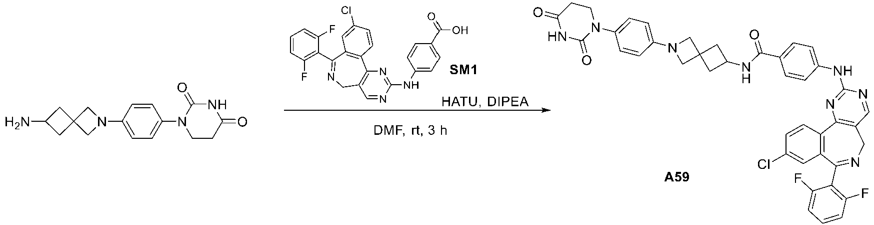

Compound **A59** (14.5 mg, yield 61%) was synthesized according to the synthesis method of compound **A1** described in **Example 1.** LCMS (ESI) m/z =758.96 [M+H]⁺. ¹H NMR (400 MHz, DMSO-*d*₆) δ 10.26 (s, 1H), 10.20 (s, 1H), 8.76 (s, 1H), 8.50 (d, *J =* 7.2 Hz, 1H), 8.33 (d, *J =* 8.4 Hz, 1H), 8.00-7.77 (m, 5H), 7.61 - 7.53 (m, 1H), 7.37 (d, *J =* 2.0 Hz, 1H), 7.27 - 7.17 (m, 2H), 7.13 (d, *J =* 8.0 Hz, 2H), 6.45 (d, *J =* 8.4 Hz, 2H), 4.41 - 4.35 (m, 1H), 3.92 (s, 2H), 3.81 (s, 2H), 3.69 (t, *J* = 6.4 Hz, 2H), 2.70 (t, *J* = 6.8 Hz, 2H), 2.63 - 2.55 (m, 2H), 2.36 - 2.31 (m, 2H).

### Example 60 Synthesis of Compound A60:

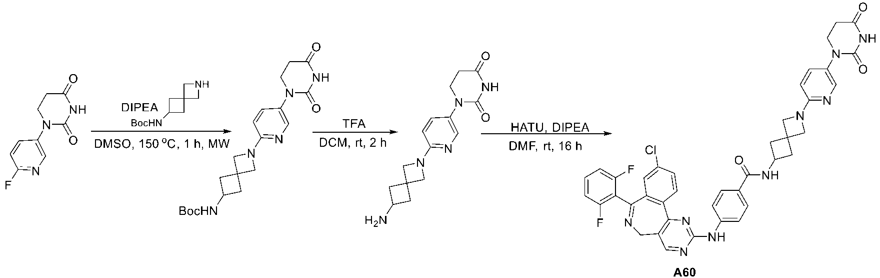

Compound **A60** (21.0 mg, yield 88%) was synthesized according to the synthesis method of compound A54 described in **Example 54.** LCMS (ESI) m/z = 760.5 [M+H]⁺. ¹H NMR (400 MHz, DMSO-*d*₆) δ 10.36 (s, 1H), 10.20 (s, 1H), 8.76 (s, 1H), 8.49 (d, *J* = 7.2 Hz, 1H), 8.33 (d, *J =* 8.4 Hz, 1H), 8.04 (d, *J =* 2.4 Hz, 1H), 7.99 - 7.82 (m, 5H), 7.65 - 7.47 (m, 2H), 7.37 (d, *J =* 2.0 Hz, 1H), 7.20 (t, *J* = 8.8 Hz, 2H), 6.43 (d, *J* = 8.8 Hz, 1H), 4.41 - 4.39 (m, 1H), 4.05 (s, 2H), 3.94 (s, 2H), 3.71 (t, *J* = 6.4 Hz, 2H), 2.72 (t, *J* = 6.8 Hz, 2H), 2.61 - 2.56 (m, 2H), 2.41 - 2.25 (m, 2H).

### Example 61 Synthesis of Compound A61:

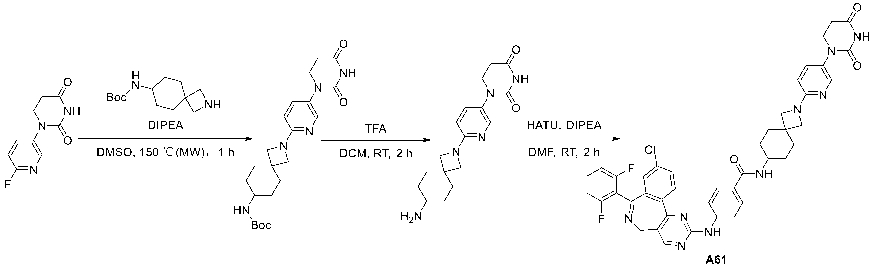

Compound **A61** (7.82 mg, yield 47.31%) was synthesized according to the synthesis method of compound **A54** described in **Example 54.** LCMS (ESI) m/z = 788.4 [M+H]⁺. ¹H NMR (400 MHz, DMSO-*d*₆) δ 10.33 (s, 1H), 10.15 (s, 1H), 8.73 (s, 1H), 8.30 (d, *J =* 8.4 Hz, 1H), 8.10 - 7.97 (m, 2H), 7.92 - 7.80 (m, 5H), 7.62 - 7.43 (m, 2H), 7.34 (d, *J =* 2.4 Hz, 1H), 7.23 - 7.09 (m, 2H), 6.41 (d, *J =* 8.8 Hz, 1H), 3.87 - 3.74 (m, 1H), 3.74 - 3.65 (m, 4H), 3.63 (s, 2H), 2.70 (t, *J* = 6.8 Hz, 2H), 1.99 - 1.96 (m, 2H), 1.82 - 1.80 (m, 2H), 1.66 - 1.61 (m, 2H), 1.52 - 1.44 (m, 2H).

### Example 62 Synthesis of Compound A62:

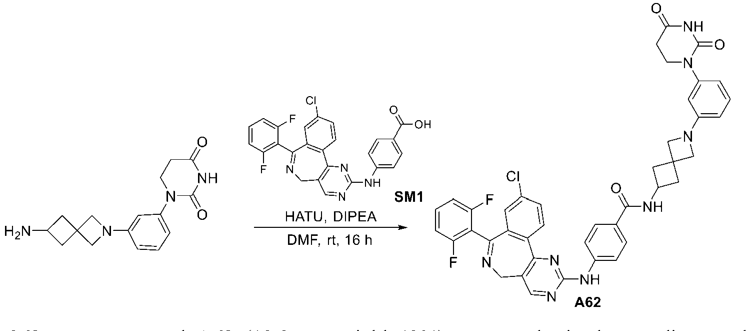

Compound **A62** (10.0 mg, yield 42%) was synthesized according to the synthesis method of compound **A1** described in **Example 1.** LCMS (ESI) m/z = 758.9 [M+H]⁺. ¹H NMR (400 MHz, DMSO-*d*₆) δ 10.33 (s, 1H), 10.20 (s, 1H), 8.76 (s, 1H), 8.50 (d, *J* = 7.6 Hz, 1H), 8.33 (d, *J =* 8.8 Hz, 1H), 8.00-7.78 (m, 5H), 7.58 (t, *J* = 7.6 Hz, 1H), 7.37 (d, *J =* 2.0 Hz, 1H), 7.26 - 7.13 (m, 3H), 6.64 (d, *J =* 8.0 Hz, 1H), 6.40 (s, 1H), 6.34 (d, *J =* 8.4 Hz, 1H), 4.44 - 4.39 (m, 1H), 3.92 (s, 2H), 3.80 (s, 2H), 3.78 - 3.74 (m, 2H), 2.74 - 2.69 (m, 2H), 2.62 - 2.56 (m, 2H), 2.40 - 2.33 (m, 2H).

### Example 63 Synthesis of Compound A63:

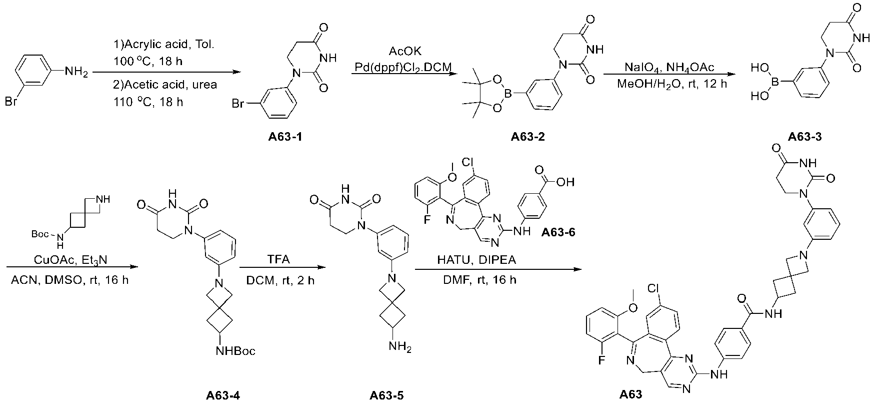

### Step 1:

Compound **A63-1** (4.00 g, 14.86 mmol, synthesized with reference to **Example 8)** and compound bis(pinacolato)diboron (7.52 g, 29.73 mmol) were dissolved in a dioxane solution (40 mL), and potassium acetate (3.65 g, 37.16 mmol) and [1,1'-bis(diphenylphosphino)ferrocene]dichloropalladium, complex with dichloromethane (1.21 g, 1.49 mmol) were added. The reaction mixture was stirred at 90°C for 16 h. After the addition of water (100 mL), the reaction mixture was extracted with ethyl acetate (150 mL*3). The organic phases were pooled, washed with saturated brine (150 mL), dried over anhydrous sodium sulfate, filtered, and the filtrate was concentrated to give a crude product. The crude product was purified by column chromatography to give compound **A63-2** (3.45 g, brown solid). LCMS (ESI) m/z = 317.3 [M+H]⁺.

### Step 2:

Compound **A63-2** (3.45 g, 10.91 mmol) was dissolved in a mixed solution of methanol (100 mL) and water (50 mL), and sodium periodate (3.50 g, 16.37 mmol) and ammonium acetate (8.40 g, 109.12 mmol) were added. The reaction mixture was stirred at 25°C for 12 h. The reaction mixture was concentrated to give a crude product. The crude product was separated for purification by reverse phase chromatography (acetonitrile/0.1% aqueous formic acid solution) to give **A63-3** (0.40 g, white solid). LCMS (ESI) m/z = 235.2 [M+H]⁺.

### Step 3:

Compound **A63-3** (50.0 mg, 0.21 mmol) was dissolved in acetonitrile (2 mL) and dimethyl sulfoxide (0.2 mL), and tert-butyl *N*-(2-azaspiro[3.3]heptan-6-yl)carbamate (45.4 mg, 0.21 mmol), copper acetate (34.1 mg, 0.28 mmol) and triethylamine (216.2 mg, 2.14 mmol) were added. The reaction mixture was stirred at 25°C for 16 h. The reaction mixture was concentrated, after the addition of water (20 mL), the mixture was then extracted with dichloromethane (20 mL*3). The organic phases were pooled, washed with saturated brine (30 mL), dried over anhydrous sodium sulfate, filtered, and the filtrate was concentrated to give a crude product. The crude product was purified by column chromatography to give compound **A63-4** (45 mg, yellow solid). LCMS (ESI) m/z = 401.3 [M+H]⁺.

### Step 4:

At room temperature, compound **A63-4** (91.0 mg, 0.23 mmol) was dissolved in dichloromethane (3 mL), trifluoroacetic acid (1.5 mL) was added, and the reaction mixture was stirred at 25°C for 2 h. The reaction mixture was concentrated to remove the solvent to give **A63-5** (100.0 mg, yellow solid, crude product, trifluoroacetate). LCMS (ESI) m/z = 301.3 [M+H]⁺.

### Step 5:

**A63-6** (15.0 mg, 0.031 mmol, synthesized with reference to patent WO2008063525), HATU (17.5 mg, 0.046 mmol) and *N,N-*diisopropylethylamine (11.9 mg, 0.092 mmol) were added to a solution of compound **A63-5** (25.4 mg, 0.061 mmol) in N,N-dimethylformamide (2 mL). The reaction mixture was stirred at room temperature for 16 h. The reaction mixture was separated for purification by reverse phase chromatography (acetonitrile/10 M aqueous ammonium bicarbonate solution) to give yellow solid A63 (10.0 mg, yield 42%). LCMS (ESI) m/z = 771.4 [M+H]⁺. ¹H NMR (400 MHz, DMSO-*d*₆) δ 10.33 (s, 1H), 10.19 (s, 1H), 8.73 (s, 1H), 8.49 (d, *J =* 7.2 Hz, 1H), 8.29 (d, *J =* 8.4 Hz, 1H), 7.94 (d, *J =* 8.8 Hz, 2H), 7.91 - 7.80 (m, 3H), 7.45 (q, *J =* 8.0 Hz, 1H), 7.24 (s, 1H), 7.19 (t, *J =* 8.0 Hz, 1H), 6.71 - 6.57 (m, 1H), 6.40 (t, *J =* 2.0 Hz, 1H), 6.33 (d, *J =* 8.4 Hz, 1H), 4.41 - 4.39 (m, 1H), 3.91 (s, 2H), 3.80 (s, 2H), 3.76 (t, *J* = 6.4 Hz, 2H), 2.71 (t, *J* = 6.8 Hz, 2H), 2.62 - 2.64 (m, 2H), 2.35 - 2.31 (m, 2H).

### Example 64 Synthesis of Compound A64:

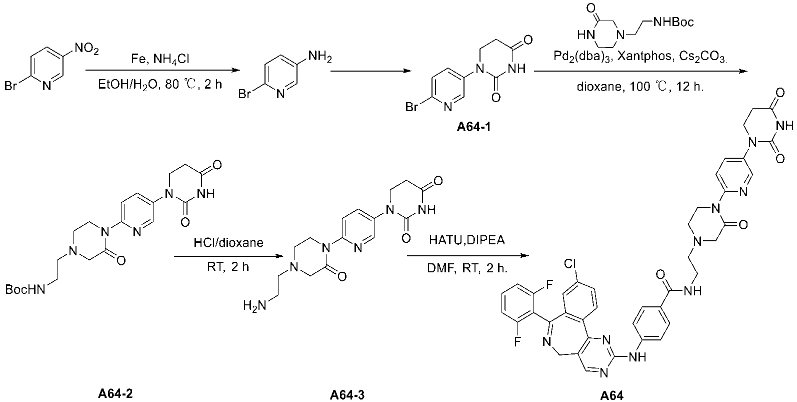

### Step 1:

At room temperature, to a solution of compound **A64-1** (100.00 mg, 370.26 µmol, synthesized with reference to **Example 8)** in 1,4-dioxane (2.0 mL), tert-butyl (2-(3-oxopiperazin-1-yl)ethyl)carbamate (180.17 mg, 740.51 µmol), tris(dibenzylideneacetone) dipalladium (67.81 mg, 74.05 µmol), and cesium carbonate (361.91 mg, 1.11 mmol) were added. The reaction mixture was stirred at 100°C for 12 h under argon protection. Solid impurities were removed by suction filtration, the filtrate was concentrated, and separated for purification by a silica gel column (dichloromethane/methanol = 10:1) to give a gray-yellow solid **A64-2** (81 mg, yield 50.59%). LCMS (ESI) m/z = 433.2 [M+H]⁺.

### Step 2:

To a solution of compound **A64-2** (20.00 mg, 46.25 µmol) in dichloromethane (3.0 mL) at room temperature, a solution of hydrochloric acid in 1,4-dioxane (4 M, 1.0 mL) was added. The reaction mixture was stirred at 25°C for 2 h. The solvent was removed by concentration under reduced pressure to give a yellow solid **A64-3** (15 mg, yield 97.59%). LCMS (ESI) m/z = 333.3 [M+H]⁺.

### Step 3:

To a solution of compound **A64-3** (15.00 mg, 46.25 µmol) in N,N-dimethylformamide (2.0 mL) at room temperature, **SM1** (8.00 mg, 16.78 µmol), HATU (12.76 mg, 33.55 µmol), and DIPEA (13.01 mg, 100.7 µmol) were added. The reaction mixture was stirred at room temperature for 2 h. Reverse-phase preparative chromatography (acetonitrile/10 M aqueous ammonium bicarbonate solution = 1.5/1) was used to separate for purification to give a white solid A64 (10.78 mg, yield 81.21%). LCMS (ESI) m/z = 791.4 [M+H]⁺. ¹H NMR (400 MHz, DMSO-*d*₆) δ 10.50 (s, 1H), 10.17 (s, 1H), 8.73 (s, 1H), 8.44 (d, *J =* 2.8 Hz, 1H), 8.38 - 8.27 (m, 2H), 7.97 - 7.74 (m, 7H), 7.63 - 7.47 (m, 1H), 7.34 (d, *J =* 2.4 Hz, 1H), 7.17 (t, *J =* 9.2 Hz, 2H), 3.88 (t, *J =* 4.4 Hz, 2H), 3.83 (t, *J* = 6.8 Hz, 2H), 3.49 - 3.40 (m, 2H), 3.35 (s, 2H), 2.88 (t, *J =* 5.6 Hz, 2H), 2.73 (t, *J =* 6.8 Hz, 2H), 2.62 (t, *J =* 6.4 Hz, 2H).

### Example 65 Synthesis of Compound A65:

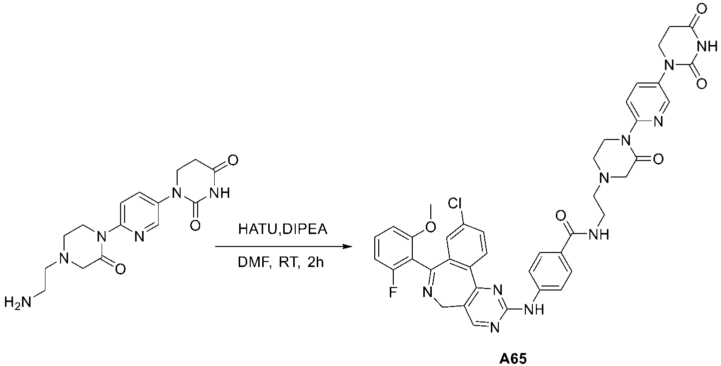

Compound **A65** (10.28 mg, yield 76.29%) was synthesized according to the synthesis method of compound **A1** described in Example 1. LCMS (ESI) m/z = 803.5 [M+H]⁺. ¹H NMR (400 MHz, DMSO-*d*₆) δ 10.51 (s, 1H), 10.16 (s, 1H), 8.70 (s, 1H), 8.43 (d, *J =* 2.4 Hz, 1H), 8.34 (t, *J =* 6.0 Hz, 1H), 8.26 (d, *J* = 8.4 Hz, 1H), 7.94 - 7.77 (m, 7H), 7.42 (q, *J =* 8.0 Hz, 1H), 7.21 (s, 1H), 3.86 - 3.79 (m, 4H), 3.45 (q, *J =* 6.0 Hz, 2H), 3.35 (s, 2H), 2.88 (t, *J =* 5.2 Hz, 2H), 2.73 (t, *J* = 6.8 Hz, 2H), 2.62 (t, *J* = 6.8 Hz, 2H).

### Example 66 Synthesis of Compound A66:

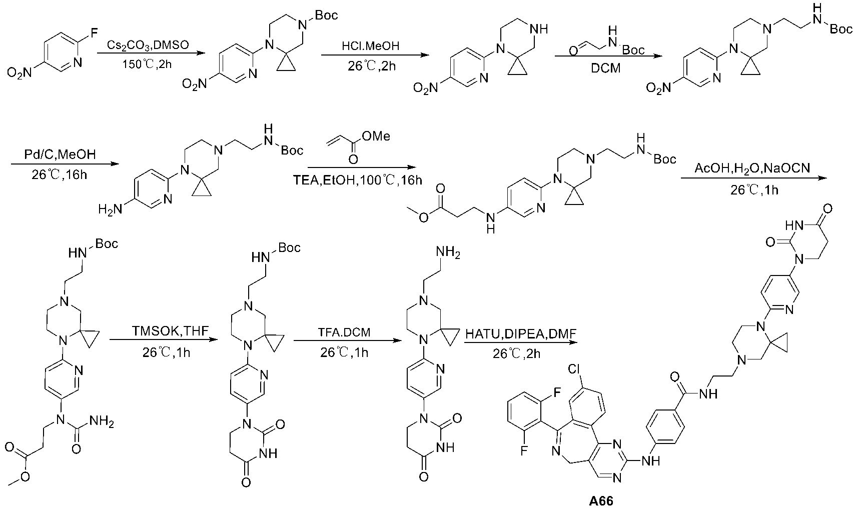

Compound **A66** (6.0 mg, yield 39%) was synthesized according to the synthesis method of compound A58 described in **Example 58.** LCMS (ESI) m/z = 803.2 [M+H]⁺. ¹H NMR (400 MHz, DMSO-*d*₆) δ 10.39 (s, 1H), 10.19 (s, 1H), 8.75 (s, 1H), 8.33 (d, *J =* 8.4 Hz, 1H), 8.22 (t, *J* = 5.6 Hz, 1H), 8.13 (d, *J* = 2.8 Hz, 1H), 7.95 - 7.88 (m, 3H), 7.83 (d, *J* = 8.8 Hz, 2H), 7.60 - 7.52 (m, 2H), 7.36 (d, *J* = 2.4 Hz, 1H), 7.20 (d, *J =* 8.8 Hz, 2H), 7.07 (d, *J* = 9.2 Hz, 1H), 3.68 - 3.56 (m, 2H), 3.46 - 3.42 (m, 2H), 2.72 (d, *J* = 6.6 Hz, 2H)), 2.48 - 2.35 (m, 3H), 1.23 (s, 4H).

### Example 67 Synthesis of Compound A67:

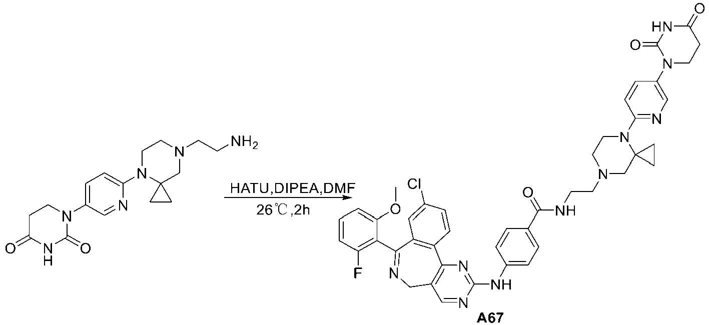

Compound **A67** (7.0 mg, yield 49%) was synthesized according to the synthesis method of compound **A1** described in **Example 1.** LCMS (ESI) m/z = 815.2 [M+H]+. ¹H NMR (400 MHz, DMSO-*d*₆) δ 10.39 (s, 1H), 10.19 (s, 1H), 8.75 (s, 1H), 8.33 (d, *J =* 8.4 Hz, 1H), 8.22 (t, *J =* 5.6 Hz, 1H), 8.13 (d, *J =* 2.8 Hz, 1H), 7.95 - 7.88 (m, 3H), 7.83 (d, *J =* 8.8 Hz, 2H), 7.60 - 7.52 (m, 2H), 7.36 (d, *J =* 2.4 Hz, 1H), 7.20 (d, *J = 8.8* Hz, 2H), 7.07 (d, *J* = 9.2 Hz, 1H), 3.74 (d, *J* = 6.6 Hz, 2H), 3.46 - 3.42 (m, 2H), 2.72(d, *J* = 6.6 Hz, 2H), 2.45 - 2.43 (m, 3H), 0.98 - 0.82 (m, 4H).

### Example 68 Synthesis of Compound A68:

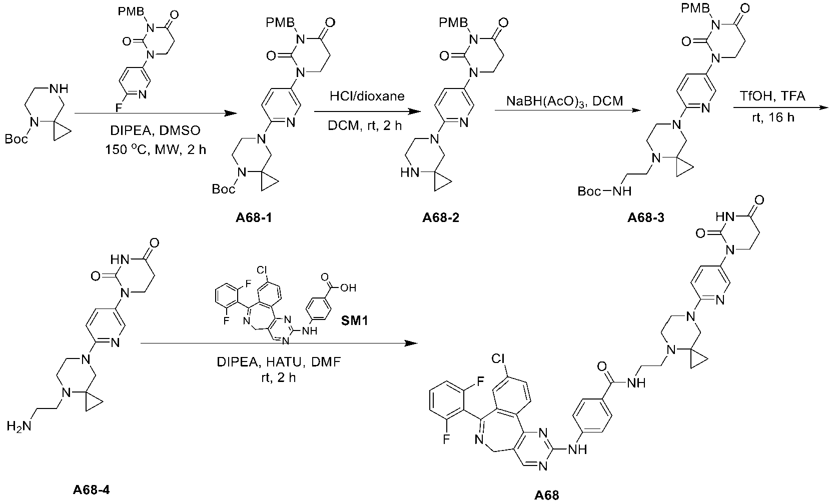

### Step 1:

DIPEA (608.81 mg, 4.71 mmol, 820.49 µL) was added to a solution of tert-butyl 4,7-diazaspiro[2.5]octane-4-carboxylate (1 g, 4.71 mmol) and 1-(6-fluoropyridin-3-yl)-3-(4-methoxybenzyl)dihydropyrimidine-2,4(1H,3H)-dione (1.41 g, 4.28 mmol) in dimethyl sulfoxide (7 mL) at room temperature. The reaction mixture was stirred at 150°C for 2 h under microwave conditions. With addition of saturated brine for quenching, the reaction mixture was extracted with ethyl acetate, and the organic phase then was dried over anhydrous sodium sulfate, filtered, and concentrated. The crude product was separated for purification by silica gel column chromatography (dichloromethane/methanol = 50:1) to give a yellow solid **A68-1** (1.5 g, yield 53.7%). LCMS (ESI) m/z = 522.1 [M+H]⁺.

### Step 2:

HCl (4 M in dioxane, 3.5 mL) was added to a solution of compound **A68-1** (1 g, 1.92 mmol) in dichloromethane (7 mL) at room temperature. The reaction mixture was stirred at room temperature for 2 h. The reaction mixture was directly concentrated under reduced pressure to give a crude yellow solid **A68-2** (1 g) which was directly used in the next step. LCMS (ESI) m/z = 422.1 [M+H]⁺.

### Step 3:

DIPEA (306.63 mg, 2.37 mmol, 413.24 µL), acetic acid (284.95 mg, 4.75 mmol), N-tert-butoxycarbonyl-2-aminoacetaldehyde (566.50 mg, 3.56 mmol) and sodium triacetoxyborohydride (1.51 g, 7.12 mmol) were added to a solution of **A68-2** (1 g, 2.37 mmol) in dichloromethane (10 mL) at room temperature. The reaction mixture was stirred at room temperature for 16 h. The reaction mixture was quenched with saturated aqueous sodium bicarbonate solution, and extracted with dichloromethane. The organic phase was dried over anhydrous sodium sulfate, filtered, and concentrated. The crude product was separated for purification by silica gel column chromatography (dichloromethane/methanol = 50:1) to give a yellow oil **A68-3** (1.1 g, yield 82.1%). LCMS (ESI) m/z = 565.2 [M+H]⁺.

### Step 4:

**A68-3** (300 mg, 531.28 µmol) was dissolved in trifluoromethanesulfonic acid solution (2 mL) and trifluoroacetic acid solution (2 mL) at room temperature. The reaction mixture was stirred at room temperature for 16 h. The reaction mixture was adjusted to pH 6 with a saturated sodium carbonate solution in an ice bath, and the resulting solution was directly purified by a reverse phase column to give a white solid **A68-4** (130 mg, yield 71.0%). LCMS (ESI) m/z = 345.0 [M+H]⁺.

### Step 5:

To a solution of **A68-4** (21.67 mg, 62.91 µmol) in N,N-dimethylformamide (3 mL), DIPEA (12.20 mg, 94.37 µmol, 16.44 µL), **SM1** (15 mg, 31.46 µmol), and HATU (23.92 mg, 62.91 µmol) were added at room temperature. The reaction mixture was stirred at room temperature for 3 h. The reaction mixture was separated for purification by preparative high performance liquid chromatography to give a white solid A68 (15 mg, yield 59.3%). LCMS (ESI) m/z = 802.9 [M+H]⁺. ¹H NMR (400 MHz, DMSO-*d*₆) δ 10.35 (s, 1H), 10.18 (s, 1H), 8.72 (s, 1H), 8.32 - 8.29 (m, 2H), 8.04 (s, 1H), 7.92 - 7.87 (m, 3H), 7.87 - 7.79 (m, 2H), 7.59 - 7.53 (m, 2H), 7.34 (d, *J* = 2.0 Hz, 1H), 7.16 (t, *J* = 8.6 Hz, 2H), 6.87 (bs, 1H), 3.69 (t*, J =* 6.8 Hz, 2H), 3.49 (s, 2H), 3.29 - 3.22 (m, 4H), 3.02 (s, 2H), 2.88 (s, 2H), 2.69 (t, *J =* 6.8 Hz, 2H), 0.56 (d, *J =* 21.2 Hz, 4H).

### Example 69 Synthesis of Compound A69:

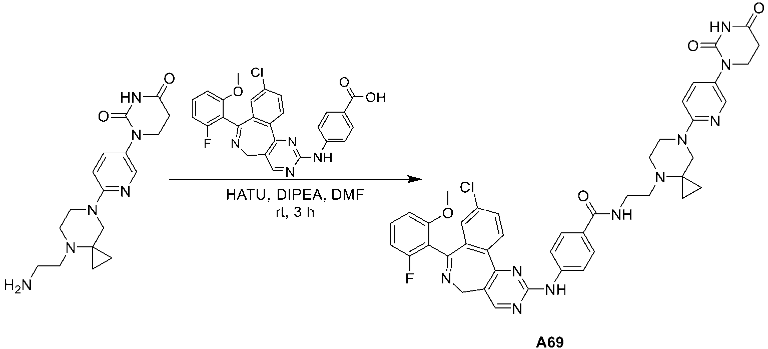

Compound **A69** (15 mg, yield 59.9%) was synthesized according to the synthesis method of compound **A1** described in **Example 1.** LCMS (ESI) m/z = 814.9 [M+H]⁺. ¹H NMR (400 MHz, DMSO-*d*₆) δ 10.34 (s, 1H), 10.16 (s, 1H), 8.70 (s, 1H), 8.30 - 8.19 (m, 2H), 8.03 (d, *J =* 2.8 Hz, 1H), 7.90 (d, *J =* 8.8 Hz, 2H), 7.85 - 7.75 (m, 3H), 7.52 - 7.36 (m, 2H), 7.21 (s, 1H), 6.80 (d, *J =* 9.1 Hz, 2H), 3.68 (t, *J =* 6.8 Hz, 2H), 3.48 (s, 2H), 3.30 - 3.29 (m, 4H), 3.01 (s, 2H), 2.87 (t, *J =* 7.0 Hz, 2H), 2.69 (t, *J* = 6.8 Hz, 2H), 0.61 - 0.50 (m, 4H).

### Example 70 Synthesis of Compound A70:

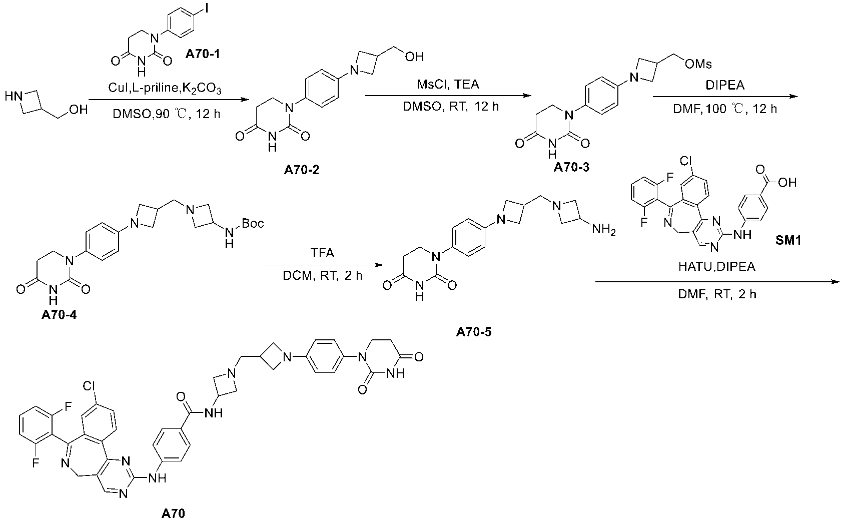

### Step 1:

To a solution of compound **A70-1** (400.00 mg, 1.27 mmol, synthesized with reference to **Example** 8) in dimethyl sulfoxide (3.0 mL) at room temperature, azetidin-3-ylmethanol hydrochloride (234.58 mg, 1.90 mmol), cuprous iodide (48.20 mg, 0.25 mmol), L-proline (58.26 mg, 0.51 mmol), and potassium carbonate (524.69 mg, 3.80 mmol) were added. The reaction mixture was stirred at 90°C for 12 h. After the addition of water, the reaction mixture was extracted with ethyl acetate, the layers were separated, the organic phase was washed with saturated brine, dried over anhydrous sodium sulfate, concentrated under reduced pressure to remove the solvent, and separated for purification by a silica gel column (dichloromethane/methanol = 10:1) to give a yellow solid **A70-2** (203 mg, yield 58.27%). LCMS (ESI) m/z = 276.2 [M+H]⁺.

### Step 2:

To a solution of compound **A70-2** (150.00 mg, 544.86 µmol) in dimethyl sulfoxide (3.0 mL) at room temperature, methanesulfonyl chloride (74.90 mg, 653.83 µmol) and triethylamine (165.40 mg, 1.63 mmol) were added. The reaction mixture was stirred at 25°C for 12 h. After the addition of water, the reaction mixture was extracted with dichloromethane, the layers were separated, the organic phase was washed with saturated brine, dried over anhydrous sodium sulfate, and concentrated under reduced pressure to remove the solvent to give a crude product. The crude product was treated with dichloromethane for slurry washing and filtered to give a yellow solid **A70-3** (113 mg, yield 56.69%). LCMS (ESI) m/z = 354.2 [M+H]⁺.

### Step 3:

To a solution of compound **A70-3** (93.00 mg, 263.16 µmol) in *N,N-*dimethylformamide (3.0 mL) at room temperature, 3-*N-*tert-butoxycarbonylaminocyclobutylamine (67.98 mg, 394.74 µmol) and DIPEA (102.03 mg, 789.49 µmol) were added. The reaction mixture was stirred at 100°C for 12 h. After the addition of water, the reaction mixture was extracted with dichloromethane, and the layers were separated. The organic phase was washed with saturated brine, dried over anhydrous sodium sulfate, concentrated under reduced pressure to remove the solvent, and separated for purification by a silica gel column (dichloromethane/methanol = 10:1) to give a yellow solid **A70-4** (42 mg, yield 37.16%). LCMS (ESI) m/z = 430.3 [M+H]⁺.

### Step 4:

To a solution of compound **A70-4** (21.00 mg, 49.01 µmol) in dichloromethane (4.0 mL) at room temperature, trifluoroacetic acid (1.0 mL) was added. The reaction mixture was stirred at 25°C for 2 h. The solvent was removed by concentration under reduced pressure to give a brown solid **A70-5** (17 mg, yield 100%). LCMS (ESI) m/z = 330.3 [M+H]⁺.

### Step 5:

To a solution of compound **A70-5** (17.00 mg, 49.01 µmol) in N,N-dimethylformamide (2.0 mL) at room temperature, **SM1** (10.00 mg, 20.97 µmol), HATU (15.95 mg, 41.94 µmol), and DIPEA (8.13 mg, 61.91 µmol) were added. The reaction mixture was stirred at room temperature for 2 h. Reverse-phase preparative chromatography (acetonitrile/10 M aqueous ammonium bicarbonate solution = 1.5/1) was used to separate for purification to give a light yellow solid **A70** (8.93 mg, yield 54.09%). LCMS (ESI) m/z = 787.9 [M+H]⁺. ¹H NMR (400 MHz, DMSO-*d*₆) δ 10.24 (s, 1H), 10.18 (s, 1H), 8.73 (s, 1H), 8.60 (d, *J =* 6.8 Hz, 1H), 8.30 (d, *J =* 8.4 Hz, 1H), 7.95 - 7.80 (m, 5H), 7.60 - 7.48 (m, 1H), 7.34 (d, *J =* 2.4 Hz, 1H), 7.17 (t, *J =* 8.8 Hz, 2H), 7.09 (d, *J =* 8.4 Hz, 2H), 6.40 (d, *J* = 8.4 Hz, 2H), 4.47-4.45 (m, 1H), 3.89 (d, *J* = 6.8 Hz, 2H), 3.66 (t, *J =* 6.8 Hz, 2H), 3.58 (t, *J* = 6.4 Hz, 2H), 3.48 - 3.46 (m, 2H), 3.02 (t, *J* = 7.2 Hz, 2H), 2.76 - 2.62 (m, 5H).

### Example 71 Synthesis of Compound A71:

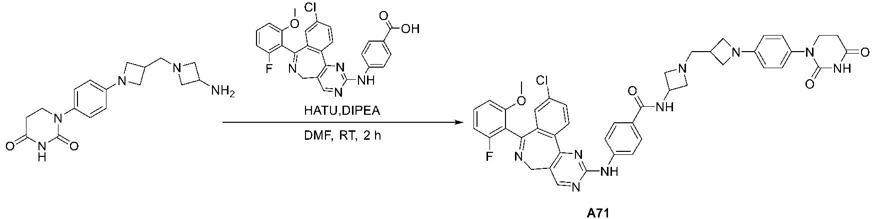

Compound **A71** (9.27 mg, yield 56.70%) was synthesized according to the synthesis method of compound **A1** described in **Example 1.** LCMS (ESI) m/z = 800.0 [M+H]⁺. ¹H NMR (400 MHz, DMSO-*d*₆) δ 10.24 (s, 1H), 10.17 (s, 1H), 8.71 (s, 1H), 8.59 (d, *J =* 7.2 Hz, 1H), 8.26 (d, *J =* 8.4 Hz, 1H), 7.92 (d, *J =* 8.4 Hz, 2H), 7.88 - 7.78 (m, 3H), 7.42 (q, *J =* 8.0 Hz, 1H), 7.21 (s, 1H), 7.09 (d, *J =* 8.8 Hz, 2H), 6.46 - 6.34 (m, 2H), 4.47 - 4.42 (m, 1H), 3.88 (t, *J* = 7.2 Hz, 2H), 3.66 (t, *J* = 6.8 Hz, 2H), 3.58 (t, *J* = 6.8 Hz, 2H), 3.48 - 3.46 (m, 2H), 3.02 (t, *J* = 7.2 Hz, 2H), 2.74 - 2.62 (m, 5H).

### Example 72 Synthesis of Compound A72:

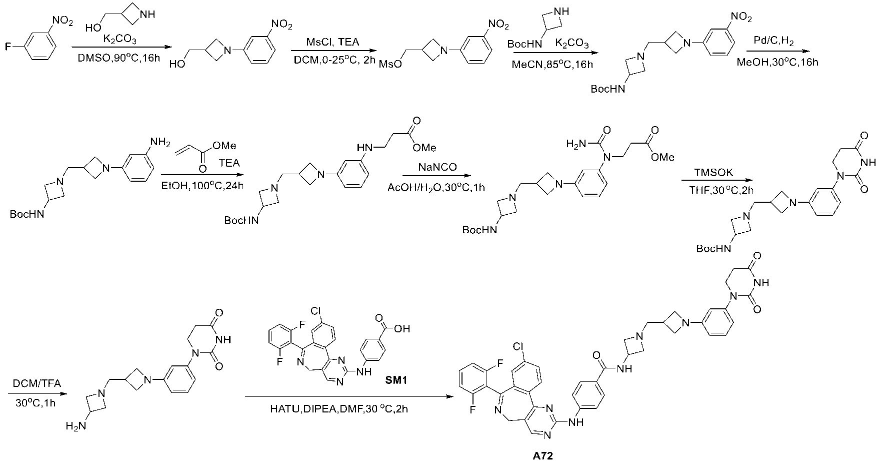

Compound **A72** (10.8 mg, yield 45.6%) was synthesized according to the synthesis method of compound **A58** described in **Example 58.** LCMS (ESI) m/z = 787.98 [M+H]⁺. ¹H NMR (400 MHz, DMSO-*d*₆) δ 10.29 (s, 1H), 10.18 (s, 1H), 8.73 (s, 1H), 8.59 (d, *J =* 8.0 Hz, 1H), 8.30 (d, *J =* 8.0 Hz, 1H), 7.94 - 7.81 (m, 5H), 7.59 - 7.50 (m, 1H), 7.34 (s, 1H), 7.22 - 7.10 (m, 3H), 6.61 - 6.58 (m, 1H), 6.35 (d, *J =* 4.0 Hz, 1H), 6.29 (d, *J =* 8.0 Hz, 1H), 4.52 - 4.42 (m, 1H), 3.88 (t, *J =* 4.0 Hz, 2H), 3.73 (t, *J =* 8.0 Hz, 2H), 3.58 (t, *J =* 8.0 Hz, 2H), 3.48 - 3.43 (m, 2H), 3.03 (t, *J =* 4.0 Hz, 2H), 2.71 - 2.64 (m, 5H).

### Example 73 Synthesis of Compound A73:

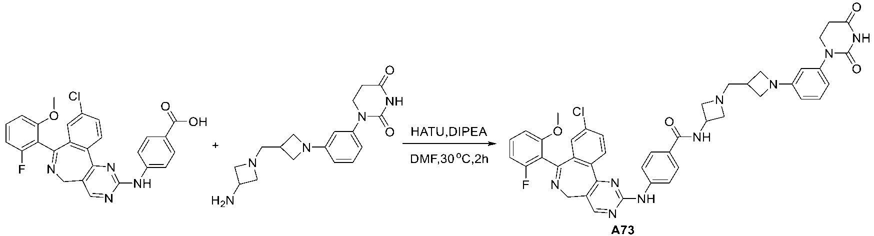

Compound **A73** (12.8 mg, yield 53%) was synthesized according to the synthesis method of compound **A1** described in **Example 1.** LCMS (ESI) m/z = 800.0 [M+H]⁺. ¹H NMR (400 MHz, DMSO-*d*₆) δ 10.30 (s, 1H), 10.17 (s, 1H), 8.71 (s, 1H), 8.59 (d, *J =* 8.0 Hz, 1H), 8.26 (d, *J =* 8.0 Hz, 1H), 7.92 (d, *J =* 8.8 Hz, 2H), 7.87 - 7.79 (m, 3H), 7.46 - 7.38 (m, 1H), 7.21 (s, 1H), 7.15 (t, *J =* 8.0 Hz, 1H), 6.60 (dd, *J*₁ = 7.2 Hz, *J*₂ = 2.0 Hz, 1H), 6.35 (t, *J* = 4.0 Hz, 1H), 6.28 (dd, *J* = 7.8, 2.0 Hz, 1H), 4.53 - 4.42 (m, 1H), 3.88 (t, *J =* 4.0 Hz, 2H), 3.73 (t, *J =* 4.0 Hz, 2H), 3.59 (t, *J =* 4.0 Hz, 2H), 3.46 - 3.42 (m, 2H), 3.03 (t, *J =* 8.0 Hz, 2H), 2.71 - 2.65 (m, 5H).

### Example 74 Synthesis of Compound A74:

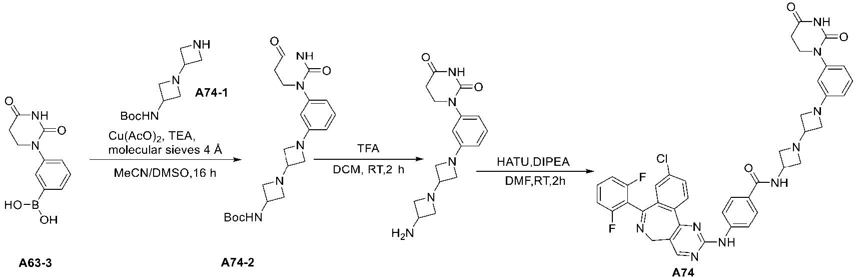

### Step 1:

To a mixed solution of compound **A74-1** (100.00 mg, 427.32 µmol, synthesized with reference to patent WO2020150385) in dimethyl sulfoxide (3.0 mL) and acetonitrile (15.0 mL) at room temperature, **A63-3** (145.70 mg, 640.98 µmol), copper acetate (100.00 mg, 500.88 µmol), triethylamine (432.41 mg, 4.27 mmol), and 4Å molecular sieve (350.0 mg) were added. The reaction mixture was stirred at 25°C for 12 h. The reaction mixture was filtered to remove solid impurities, and the filtrate was concentrated. After the addition of water, the reaction mixture was extracted with dichloromethane, and the layers were separated. The organic phase was washed with saturated brine, dried over anhydrous sodium sulfate, concentrated under reduced pressure to remove the solvent, and separated for purification by a silica gel chromatography column (dichloromethane/methanol = 10:1) to give a green solid **A74-2** (56 mg, yield 31.54%). LCMS (ESI) m/z = 416.2 [M+H]⁺.

### Step 2:

Compound **A77** (9.55 mg, yield 65.36%) was synthesized according to the synthesis method of compound **A8** described in **Example 8.** LCMS (ESI) m/z = 774.1 [M+H]⁺. ¹H NMR (400 MHz, DMSO-*d*₆) δ 10.29 (s, 1H), 10.18 (s, 1H), 8.73 (s, 1H), 8.62 (d, *J =* 7.2 Hz, 1H), 8.30 (d, *J =* 8.4 Hz, 1H), 7.99 - 7.79 (m, 5H), 7.67 - 7.48(m, 1H), 7.34 (d, *J =* 2.4 Hz, 1H), 7.17 (t, *J* =8.0 Hz, 3H), 6.61 (d, *J =* 8.0 Hz 1.2 Hz, 1H), 6.38 (t, *J* = 2.4 Hz, 1H), 6.36 - 6.26 (m, 1H), 4.47 - 4.62 (m, 1H), 3.84 (t, *J* = 6.8 Hz, 2H), 3.74 (t, *J =* 6.8 Hz, 2H), 3.70 - 3.59 (m, 3H), 3.55 (t, *J* = 6.8 Hz, 2H), 3.17 (t, *J* = 7.2 Hz, 2H), 2.68 (t, *J* = 6.8 Hz, 2H).

### Example 75 Synthesis of Compound A75:

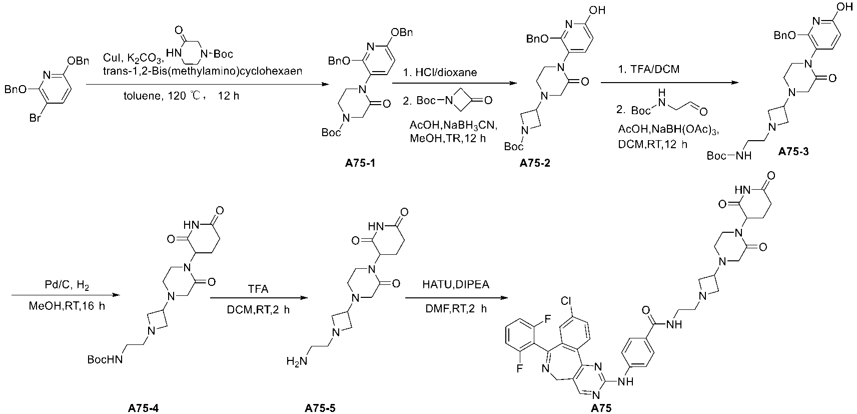

### Step 1:

To a solution of compound 2,6-bis(benzyloxy)-3-bromopyridine (500.00 mg, 1.35 mmol) in toluene (15.0 mL) at room temperature, tert-butyl 3-oxo-1-piperazinecarboxylate (405.62 mg, 2.03 mmol), (1R,2R)-(-)-N,N-dimethyl-1,2-cyclohexanediamine (96.05 mg, 675.24 µmol), cuprous iodide (128.60 mg, 675.24 µmol), and potassium carbonate (559.95 mg, 4.05 mmol) were added. The reaction mixture was stirred at 120°C for 12 h. The reaction mixture was filtered to remove solid impurities, concentrated under reduced pressure to remove the solvent, and separated for purification by a silica gel column (petroleum ether/ethyl acetate = 3:2) to give a yellow solid **A75-1** (368 mg, yield 63.01%). LCMS (ESI) m/z = 490.1 [M+H]⁺.

### Step 2:

At room temperature, HCl (4 M in dioxane, 3.0 mL) was added to a solution of compound **A75-1** (300.00 mg, 612.79 µmol) in dichloromethane (12.0 mL). The reaction mixture was stirred at 25°C for 2 h. The solvent was removed by concentration under reduced pressure. Methanol (5.0 mL), 1-tert-butoxycarbonyl-3-azetidinone (419.77 mg, 2.45 mmol) and sodium cyanoborohydride (231.13 mg, 3.68 mmol) were added. The reaction mixture was stirred at 25°C for 12 h. The solvent was removed by concentration under reduced pressure, and the residue was separated for purification by a silica gel column (dichloromethane/methanol *=* 20:1) to give a white solid **A75-2** (266 mg, yield 95.47%). LCMS (ESI) m/z = 455.2 [M+H]⁺.

### Step 3:

To a solution of compound **A75-2** (266.01 mg, 488.40 µmol) in dichloromethane (15.0 mL) at room temperature, trifluoroacetic acid (3.0 mL) was added. The reaction mixture was stirred at 25°C for 2 h. The solvent was removed by concentration under reduced pressure. Dichloromethane (25.0 mL), N-tert-butoxycarbonyl-2-aminoacetaldehyde (126.23 mg, 792.98 µmol) and sodium triacetoxyborohydride (224.09 mg, 1.06 mmol) were added. The reaction mixture was stirred at 25°C for 12 h. The solvent was removed by concentration under reduced pressure, and the residue was separated for purification by a silica gel column (dichloromethane/methanol = 20:1) to give a yellow solid **A75-3** (226 mg, yield 72.74%). LCMS (ESI) m/z = 498.3 [M+H]⁺.

### Step 4:

Reducing palladium on carbon (10%, 150 mg, 10% w/w) was added to a solution of compound **A75-3** (300.00 mg, 510.46 µmol) in methanol (30.0 mL) at room temperature. The mixture was stirred at 25°C for 12 h under hydrogen protection (1.0 atm). Solid impurities were removed by suction filtration, and reverse-phase purification (methanol/water with 0.01% formic acid = 1:1) was performed to give a purple solid **A75-4** (58 mg, yield 27.75%). LCMS (ESI) m/z = 410.1 [M+H]⁺.

### Step 5:

To a solution of compound **A75-4** (30.00 mg, 73.26 µmol) in dichloromethane (4.0 mL) at room temperature, trifluoroacetic acid (1.0 mL) was added. The reaction mixture was stirred at 25°C for 2 h. The solvent was removed by concentration under reduced pressure to give a brown solid **A75-5** (25 mg, yield 100%). LCMS (ESI) m/z = 310.2 [M+H]⁺.

### Step 6:

To a solution of compound **A75-5** (20.00 mg, 58.21 µmol) in N,N-dimethylformamide (2.0 mL) at room temperature, **SM1** (9 mg, 18.87 µmol), HATU (14.35 mg, 37.75 µmol), and DIPEA (14.64 mg, 113.24 µmol) were added. The reaction mixture was stirred at room temperature for 2 h. Reverse-phase preparative chromatography (acetonitrile/10 M aqueous ammonium bicarbonate solution = 1.5/1) was used to separate for purification to give a white solid A75 (9.56 mg, yield 65.94%). LCMS (ESI) m/z = 768.1 [M+H]⁺. ¹H NMR (400 MHz, DMSO-*d*₆) δ 10.85 (s, 1H), 10.16 (s, 1H), 8.73 (s, 1H), 8.30 (d, *J =* 8.4 Hz, 1H), 8.22 (t, *J =* 5.6 Hz, 1H), 7.96 - 7.85 (m, 3H), 7.81 (d, *J =* 8.8 Hz, 2H), 7.62 - 7.48 (m, 1H), 7.34 (d, *J =* 2.0 Hz, 1H), 7.17 (t, *J* = 8.8 Hz, 2H), 4.92 (bs, 1H), 3.45 - 3.42 (m, 2H), 3.34 - 3.20 (m, 4H), 3.06 - 2.93 (m, 3H), 2.94-2.89 (m, 2H), 2.82 - 2.65 (m, 1H), 2.64 - 2.52 (m, 3H), 2.39 - 2.20 (m, 1H), 1.91 - 1.74 (m, 1H).

### Example 76 Synthesis of Compound A76:

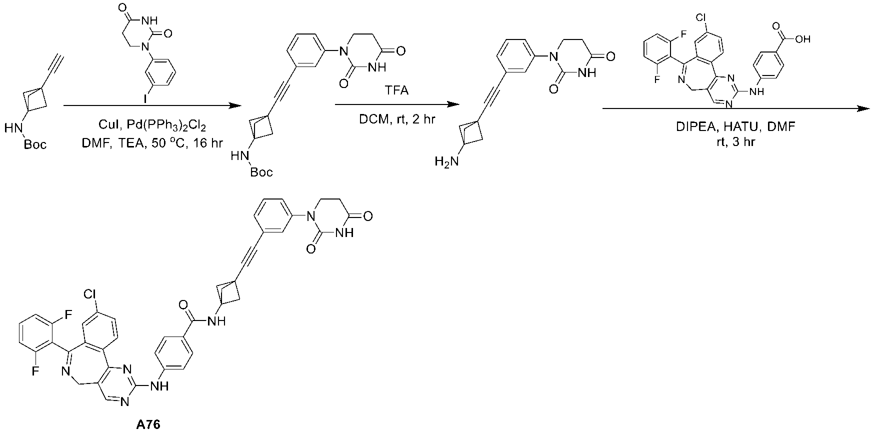

Compound **A76** (13 mg, yield 54.8%) was synthesized according to the synthesis method of compound A6 described in **Example 6.** LCMS (ESI) m/z = 753.9 [M+H]⁺. ¹H NMR (400 MHz, DMSO-*d*₆) δ 10.42 (s, 1H), 10.20 (s, 1H), 8.95 (s, 1H), 8.73 (s, 1H), 8.29 (d, *J =* 8.4 Hz, 1H), 7.93 - 7.78 (m, 5H), 7.55 (m, 1H), 7.40 (m, 1H), 7.39 - 7.32 (m, 3H), 7.28 (m, 1H), 7.17 (t, *J =* 8.8 Hz, 2H), 3.80 (t, *J =* 6.6 Hz, 2H), 2.70 (t, *J* = 6.6 Hz, 2H), 2.44 (s, 6H).

### Example 77 Synthesis of Compound A77:

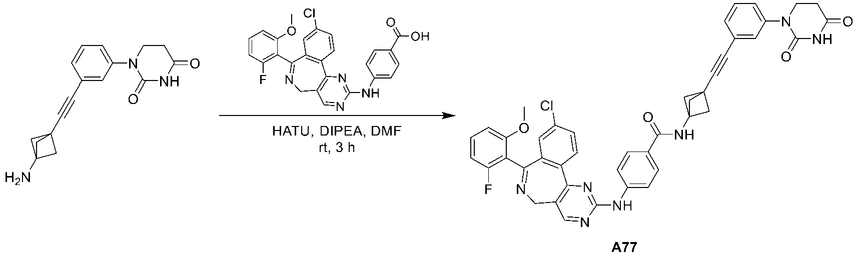

Compound A77 (13 mg, yield 55.3%) was synthesized according to the synthesis method of compound **A6** described in **Example 6.** LCMS (ESI) m/z = 765.9 [M+H]⁺. ¹H NMR (400 MHz, DMSO-*d*₆) δ 10.42 (s, 1H), 10.19 (s, 1H), 8.94 (s, 1H), 8.71 (s, 1H), 8.25 (d, *J =* 8.4 Hz, 1H), 7.91 (d, *J =* 8.8 Hz, 2H), 7.84 - 7.78 (m, 3H), 7.47 - 7.39 (m, 2H), 7.38 - 7.32 (m, 2H), 7.28 (m, 1H), 7.21 (s, 1H), 6.88 (s, 1H), 3.80 (t, *J =* 6.6 Hz, 2H), 2.70 (t, *J* = 6.6 Hz, 2H), 2.44 (s, 6H).

### Example 78 Synthesis of Compound A78:

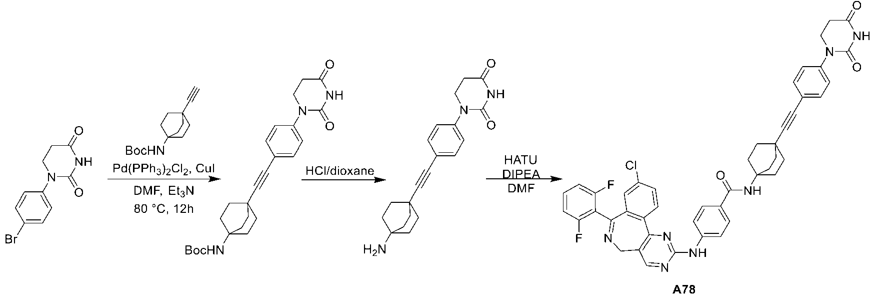

Compound **A78** (18.0 mg, yield 53%) was synthesized according to the synthesis method of compound **A26** described in **Example 26.** LCMS (ESI) m/z = 758.4 [M+H]⁺. ¹H NMR (400 MHz, DMSO-*d*₆) δ 10.42 (s, 1H), 10.14 (s, 1H), 8.72 (s, 1H), 8.29 (d, *J =* 8.4 Hz, 1H), 7.88 (dd, *J* = 8.6, 2.5 Hz, 3H), 7.76 (d, *J* = 8.6 Hz, 2H), 7.63 - 7.45 (m, 2H), 7.43 - 7.25 (m, 5H), 7.17 (t, *J* = 9.0 Hz, 2H), 3.79 (t, *J* = 6.6 Hz, 2H), 2.70 (t, *J =* 6.6 Hz, 2H), 2.03 - 1.88 (m, 12H).

### Example 79 Synthesis of Compound A79:

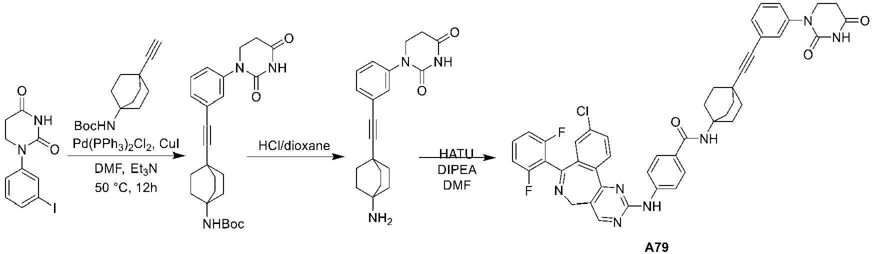

Compound **A79** (18.0 mg, yield 53%) was synthesized according to the synthesis method of compound **A26** described in **Example 26.** LCMS (ESI) m/z = 758.4 [M+H]⁺. ¹H NMR (400 MHz, DMSO-*d*₆) δ 10.40 (s, 1H), 10.14 (s, 1H), 8.72 (s, 1H), 8.44 (s, 1H), 8.29 (d, *J* = 8.4 Hz, 1H), 7.88 (dd, *J*₁ = 8.6 Hz, *J*₂ = 2.2 Hz, 3H), 7.76 (d, *J =* 8.8 Hz, 2H), 7.62 - 7.47 (m, 2H), 7.39 - 7.25 (m, 4H), 7.24-7.09 (m, 3H), 3.78 (t, *J* = 6.6 Hz, 2H), 2.70 (t, *J* = 6.6 Hz, 2H), 2.03 - 1.89 (m, 12H).

### Example 80 Synthesis of Compound A80:

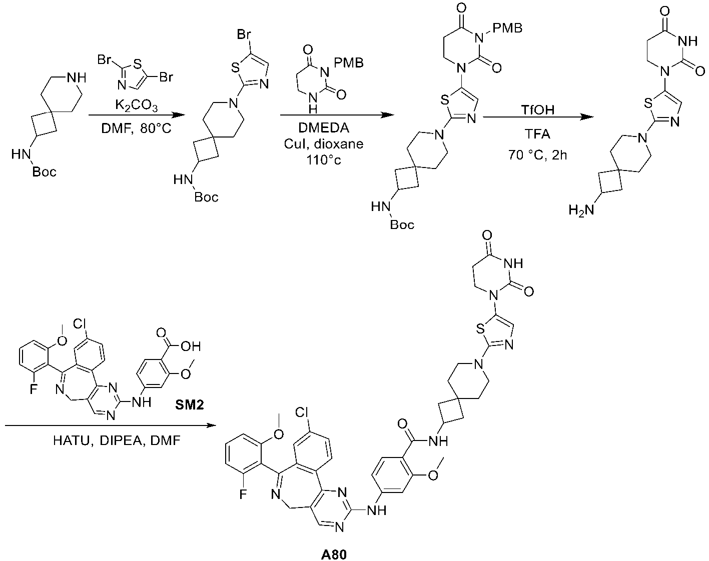

Compound **A80** (17.0 mg, yield 70%) was synthesized according to the synthesis method of compound A30 described in **Example** 30. LCMS (ESI) m/z = 836.2 [M+H]⁺. ¹H NMR (400 MHz, DMSO-*d*₆) δ 10.58 (s, 1H), 10.18 (s, 1H), 8.72 (s, 1H), 8.30 (d, *J =* 8.4 Hz, 1H), 8.12 (d, *J =* 7.4 Hz, 1H), 7.94 (s, 1H), 7.82 (dd, *J*₁ = 8.6 Hz, *J*₂ = 2.2 Hz, 1H), 7.74 (d, *J* = 8.4 Hz, 1H), 7.42 (d, *J* = 8.2 Hz, 2H), 7.22 (s, 1H), 6.97 (s, 1H), 4.42 (q, *J =* 8.0 Hz, 1H), 3.93 (s, 3H), 3.78 (t, *J* = 6.8 Hz, 2H), 3.3-3.25 (m, 4H), 2.72 (t, *J =* 6.8 Hz, 2H), 2.27 (t, *J =* 9.4 Hz, 2H), 1.83 (t, *J* = 9.8 Hz, 2H), 1.71 - 1.66 (m, 4H).

### Example 81 Synthesis of Compound A81:

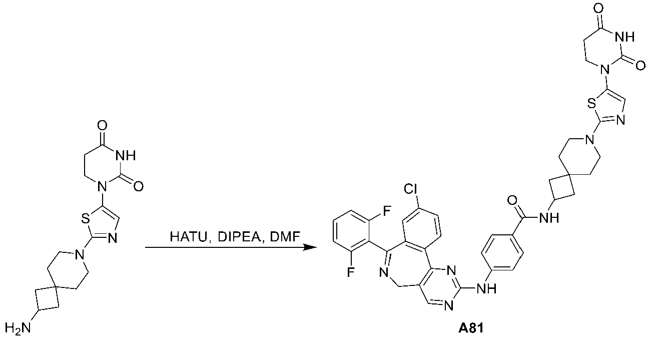

Compound **A81** (16.0 mg, yield 64%) was synthesized according to the synthesis method of compound **A1** described in **Example 1.** LCMS (ESI) m/z = 794.1 [M+H]⁺. ¹H NMR (400 MHz, DMSO-*d*₆) δ 10.17 (s, 1H), 8.73 (s, 1H), 8.45 (d, *J =* 7.4 Hz, 1H), 8.30 (d, *J =* 8.4 Hz, 1H), 7.92 - 7.82 (m, 5H), 7.58 - 7.50 (m, 1H), 7.34 (d, *J* = 2.2 Hz, 1H), 7.17 (t, *J =* 9.2 Hz, 2H), 6.97 (s, 1H), 4.49 - 4.39 (m, 1H), 3.78 (t, *J = 6.8* Hz, 2H), 3.30 - 3.27 (m, 4H), 2.72 (t, *J* = 6.8 Hz, 2H), 2.24 (t, *J* = 9.8 Hz, 2H), 1.89 (t, *J* = 10.0 Hz, 2H), 1.70 - 1.61 (m, 4H).

### Example 82 Synthesis of Compound A82:

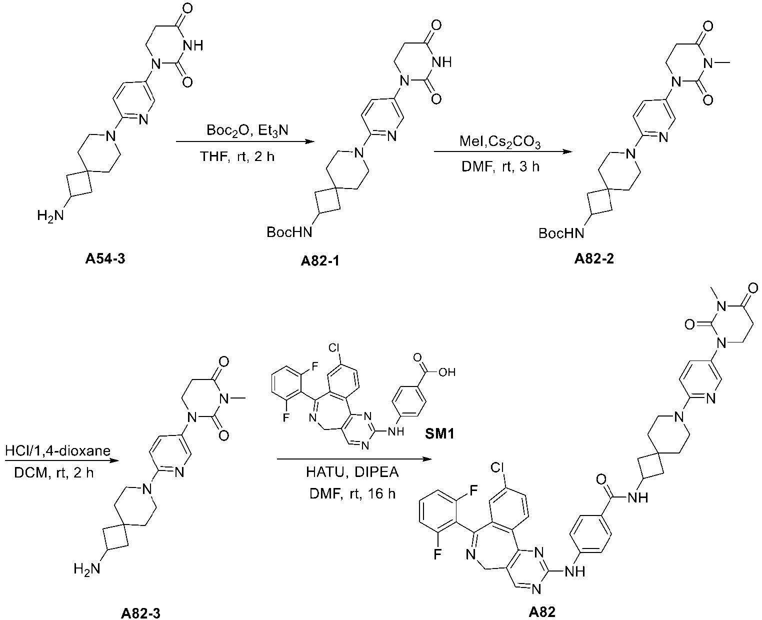

### Step 1:

Compound **A54-3** (60.0 mg, 0.18 mmol) was dissolved in tetrahydrofuran (2 mL), and triethylamine (36.9 mg, 0.36 mmol) and di-tert-butyl dicarbonate (59.6 mg, 0.27 mmol) were added. The reaction mixture was stirred at 25°C for 2 h. After the addition of water (10 mL), the reaction mixture was extracted with ethyl acetate (20 mL*3). The organic phases were pooled, washed with saturated brine (30 mL), dried over anhydrous sodium sulfate and filtered, and the filtrate was concentrated to give a crude product. The crude product was purified by column chromatography to give compound **A82-1** (45.0 mg, white solid). LCMS (ESI) m/z = 430.4 [M+H]⁺.

### Step 2:

At room temperature, compound **A82-1** (40.0 mg, 0.093 mmol) was dissolved in *N,N-*dimethylformamide (2 mL), cesium carbonate (60.7 mg, 0.19 mmol) and methyl iodide (19.8 mg, 0.14 mmol) were added, and the reaction mixture was stirred at 25°C for 3 h. The reaction mixture was extracted by adding water (10 mL) and ethyl acetate (20 mL*3). The organic phases were pooled, washed with saturated brine (30 mL), dried over anhydrous sodium sulfate, and filtered. The filtrate was concentrated to give compound **A82-2** (70.0 mg, yellow solid, crude product). LCMS (ESI) m/z = 444.1 [M+H]⁺.

### Step 3:

At room temperature, compound **A82-2** (70.0 mg, crude product) was dissolved in dichloromethane (2 mL), hydrogen chloride (4.0 M in dioxane, 1 mL) was added, and the reaction mixture was stirred at 25°C for 2 h. The reaction mixture was concentrated to remove the solvent to give **A82-3** (50.0 mg, yellow solid, crude product, hydrochloride). LCMS (ESI) m/z = 344.3 [M+H]⁺.

### Step 4:

To a solution of compound **A82-3** (23.9 mg, 0.063 mmol) in N,Ndimethylformamide (2 mL), **SM1** (15.0 mg, 0.031 mmol), HATU (17.9 mg, 0.047 mmol) and N,N-diisopropylethylamine (12.2 mg, 0.094 mmol) were added. The reaction mixture was stirred at room temperature for 16 h. The reaction mixture was separated for purification by reverse phase chromatography (acetonitrile/10 M aqueous ammonium bicarbonate solution) to give yellow solid **A82** (14.0 mg, yield 55%). LCMS (ESI) m/z = 802.1 [M+H]⁺. ¹H NMR (400 MHz, DMSO-*d*₆) δ 10.18 (s, 1H), 8.76 (s, 1H), 8.47 (d, *J* = 7.2 Hz, 1H), 8.33 (d, *J =* 8.8 Hz, 1H), 8.08 (d, *J* = 2.8 Hz, 1H), 7.98 - 7.81 (m, 5H), 7.66 - 7.53 (m, 1H), 7.50 (dd, *J*₁ = 8.8 Hz, *J*₂ = 2.8 Hz, 1H), 7.37 (d, *J* = 2.4 Hz, 1H), 7.20 (t, *J =* 8.8 Hz, 2H), 6.89 (d, *J* = 9.2 Hz, 1H), 4.50 (p, *J =* 8.0 Hz, 1H), 3.71 (t, *J* = 6.8 Hz, 2H), 3.62 - 3.52 (m, 2H), 3.52 - 3.42 (m, 2H), 3.07 (s, 3H), 2.85 (t, *J =* 6.8 Hz, 2H), 2.27 (t, *J* = 10.0 Hz, 2H), 1.92 (t, *J* = 10.0 Hz, 2H), 1.74 - 1.64 (m, 2H), 1.64 - 1.55 (m, 2H).

### Example 83 Synthesis of Compound A83:

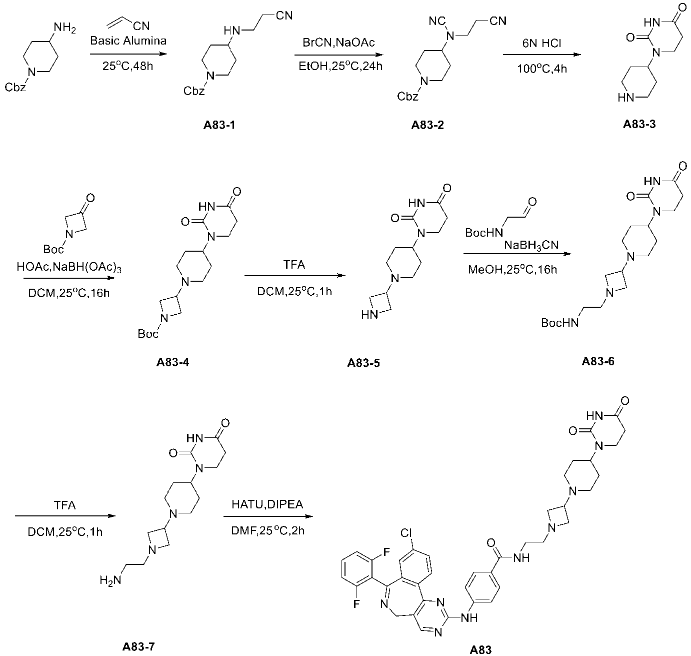

### Step 1:

At room temperature, acrylonitrile (2.26 g, 42.68 mmol) and basic aluminum oxide (21.8 g, 213.4 mmol) were added to a solution of 4-aminopiperidine-1-carboxylic acid benzyl ester (5 g, 21.34 mmol) in ethanol (50 mL). The reaction mixture was stirred at room temperature for 48 h. The reaction mixture was filtered, and concentrated under reduced pressure. The residue was separated for purification by silica gel column chromatography (petroleum ether/ethyl acetate = 10:1) to give a colorless oil **A83-1** (6 g, yield 97.8%). LCMS (ESI) m/z = 288.3 [M+H]⁺.

### Step 2:

To a solution of **A83-1** (6 g, 20.9 mmol) in ethanol (50 mL), cyanogen bromide (2.65 g, 25.0 mmol) and sodium acetate (3.43 g, 41.76 mmol) were added in an ice bath. The reaction mixture was stirred at room temperature for 24 h. Ethyl acetate (200 mL) was added to the reaction mixture, and the mixture was washed with saturated brine (2 * 60 mL). The organic phase was dried over anhydrous sodium sulfate, filtered, and concentrated under reduced pressure. The crude product was separated for purification by silica gel column chromatography (petroleum ether/ethyl acetate = 10:1) to give a colorless oil **A83-2** (6.3 g, yield 96.5%). LCMS (ESI) m/z = 312.8 [M+H]⁺.

### Step 3:

**A83-2** (6.3 g, 20.1 mmol) was dissolved in 6 N HCl (33.6 mL), and the mixture was heated to 100°C for reaction for 4 h. The mixture was concentrated to remove most of the solvent and adjusted to pH=8 with saturated sodium bicarbonate. The reaction mixture was separated for purification by reverse phase chromatography (acetonitrile/10 mM aqueous ammonium bicarbonate solution) to give a white solid **A83-3** (2.8 g, yield 70.3%).

### Step 4:

At room temperature, 1-Boc-3-azetidinone (1.43 g, 8.36 mmol), glacial acetic acid (1.36 g, 22.8 mmol) and sodium triacetoxyborohydride (4.83 g, 22.8 mmol) were added to a solution of **A83-3** (1.5 g, 7.6 mmol) in dichloromethane (60 mL), and the reaction mixture was stirred at room temperature for 16 h. The reaction mixture was washed with saturated sodium bicarbonate solution (50 mL) and saturated brine (50 mL), and then dried over anhydrous sodium sulfate. The crude product was separated for purification by silica gel column chromatography (dichloromethane/methanol = 30:1) to give a light yellow solid **A83-4** (0.4 g, yield 15%). LCMS (ESI) m/z = 353.1 [M+H]⁺.

### Step 5:

Trifluoroacetic acid (1 mL) was added to a solution of compound **A83-4** (130 mg, 0.37 mmol) in dichloromethane (2 mL) at room temperature. The reaction mixture was stirred at room temperature for 1 h. The reaction mixture was directly concentrated under reduced pressure to give **A83-5** (93 mg, yield 99%). LCMS (ESI) m/z = 253.1 [M+H]⁺.

### Step 6:

At room temperature, N-tert-butoxycarbonyl-2-aminoacetaldehyde (70 mg, 0.44 mmol), glacial acetic acid (66 mg, 1.1 mmol) and sodium cyanoborohydride (70 mg, 1.1 mmol) were added to **A83-5** (93 mg, 0.37 mmol) in methanol (5 mL). The reaction mixture was stirred at room temperature for 16 h. Ethyl acetate (50 mL) was added to the reaction mixture, which was washed with saturated sodium bicarbonate solution (20 mL) and saturated brine (20 mL), and then dried over anhydrous sodium sulfate. The crude product was separated for purification by silica gel column chromatography (dichloromethane/methanol = 20: 1) to give a light yellow solid **A83-6** (120 mg, yield 82.3%). LCMS (ESI) m/z = 396.2 [M+H]⁺.

### Step 7:

Trifluoroacetic acid (1 mL) was added to a solution of compound **A83-6** (30 mg, 0.076 mmol) in dichloromethane (2 mL) at room temperature. The reaction mixture was stirred at room temperature for 1 h. The reaction mixture was directly concentrated under reduced pressure to give **A83-7** (22 mg, yield 99%). LCMS (ESI) m/z = 296.1 [M+H]⁺.

### Step 8:

**SM1** (15 mg, 0.03 mmol), HATU (16 mg, 0.04 mmol) and *N,N-*diisopropylethylamine (12 mg, 0.09 mmol) were added to a solution of compound **A83-7** (22 mg, 0.07 mmol) in *N,N-*dimethylformamide (2 mL) at room temperature. The reaction mixture was stirred at room temperature for 2 h. The reaction mixture was separated for purification by reverse phase chromatography (acetonitrile/10 mM aqueous ammonium bicarbonate solution) to give a light yellow solid **A83** (14.7 mg, yield 61.9%). LCMS (ESI) m/z = 754.5 [M+H]⁺. ¹H NMR (400 MHz, DMSO-*d*₆) δ 10.17 (s, 1H), 10.06 (s, 1H), 8.73 (s, 1H), 8.30 (d, *J =* 8.0 Hz, 1H), 8.26 - 8.20 (m, 1H), 7.92 - 7.86 (m, 3H), 7.83 - 7.78 (m, 2H), 7.59 - 7.50 (m, 1H), 7.34 (d, *J =* 4.0 Hz, 1H), 7.22 - 7.10 (m, 2H), 4.07 - 3.97 (m, 1H), 3.46 - 3.40 (m, 2H), 3.30 - 3.25 (m, 2H), 3.24 - 3.16 (m, 2H), 2.86 - 2.80 (m, 1H), 2.78 - 2.69 (m, 4H), 2.55 - 2.51 (m, 2H), 2.46 (t, *J* = 4.0 Hz, 2H), 1.83 - 1.74 (m, 2H), 1.72 - 1.60 (m, 2H), 1.53 - 1.46 (m, 2H).

### Example 84 Synthesis of Compound A84:

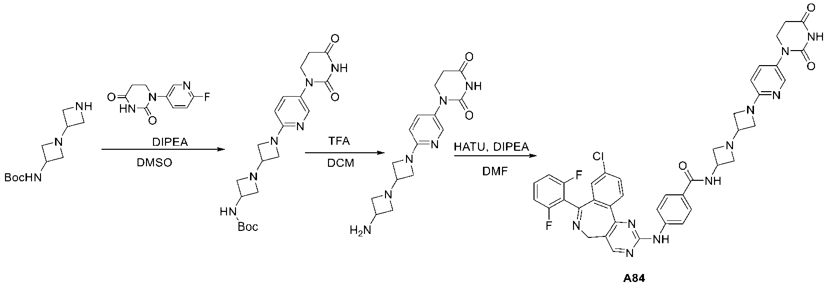

Compound **A84** (2.6 mg, yield 15%) was synthesized according to the synthesis method of compound **A54** described in **Example 54.** LCMS (ESI) m/z = 775.1 [M+H]⁺. ¹H NMR (400 MHz, DMSO-*d*₆) δ 10.34 (s, 1H), 10.18 (s, 1H), 8.73 (s, 1H), 8.63 (d, *J* = 8.0 Hz, 1H), 8.30 *(d, J=* 8.0 Hz, 1H), 8.02 (d, *J =* 4.0 Hz, 1H), 7.94 - 7.81 (m, 6H), 7.58 - 7.47 (m, 2H), 7.34 (d, *J* = 2.2 Hz, 1H), 7.17 (t, *J* = 8.0 Hz, 2H), 6.41 (d, *J =* 8.0 Hz, 1H), 4.59 - 4.51 (m, 1H), 3.96 (t, *J =* 8.0 Hz, 2H), 3.82 - 3.79 (m, 2H), 3.69 (t, *J* = 6.8 Hz, 2H), 3.64 - 3.61 (m, 1H), 3.56 (t, *J =* 6.8 Hz, 2H), 3.16 (t, *J* = 6.8 Hz, 2H), 2.69 (t, *J =* 6.8 Hz, 2H).

### Example 85 Synthesis of Compound A85:

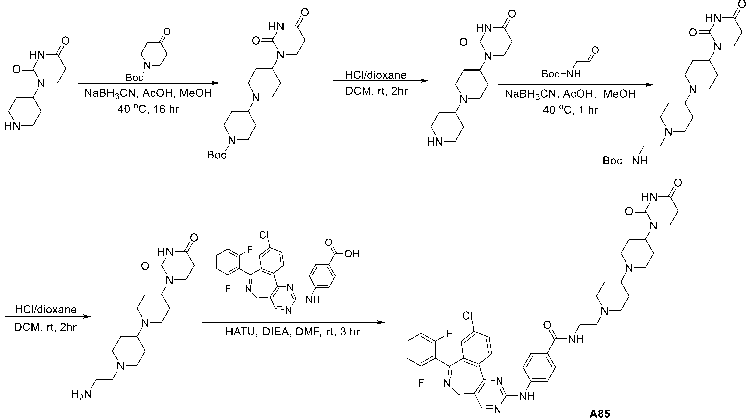

Compound A85 (5.8 mg, yield 35.3%) was synthesized according to the synthesis method of compound A83 described in Example 83. LCMS (ESI) m/z = 782.4 [M+H]⁺. ¹H NMR (400 MHz, DMSO-*d*₆) δ 10.20 (s, 1H), 10.08 (s, 1H), 8.76 (s, 1H), 8.33 (d, *J* = 8.0 Hz, 1H), 8.26 - 8.24 (m, 1H), 7.94 - 7.90 (m, 3H), 7.85 - 7.83 (m, 2H), 7.62 - 7.53 (m, 1H), 7.37 (d, *J =* 2.4 Hz, 1H), 7.24 - 7.15 (m, 2H), 4.11 - 3.80 (m, 2H), 3.43 - 3.38 (m, 3H), 3.30 (t, *J* = 6.8 Hz, 2H), 3.0 - 2.90 (m, 4H), 2.50 - 2.40 (m, 3H), 2.31 - 2.15 (m, 2H), 2.03 - 1.91 (m, 2H), 1.77 - 1.60 (m, 4H), 1.58 - 1.37 (m, 4H).

### Example 86 Synthesis of Compound A86:

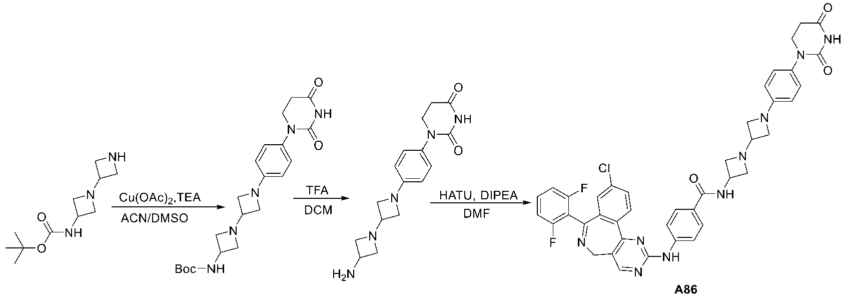

Compound **A86** (2.8 mg, yield 17.2%) was synthesized according to the synthesis method of compound **A56** described in **Example 56.** LCMS (ESI) m/z = 774.4 [M+H]⁺. ¹H NMR (400 MHz, DMSO-*d*₆) δ 10.24 (s, 1H), 10.19 (s, 1H), 8.73 (s, 1H), 8.63 (d, *J =* 8.0 Hz, 1H), 8.30 (d, *J* = 8.0 Hz, 1H), 7.92 - 7.83 (m, 5H), 7.58 - 7.51 (m, 1H), 7.34 (d, *J =* 2.2 Hz, 1H), 7.19 - 7.15 (m, 2H), 7.12 - 7.10 (m, 2H), 6.44 - 6.42 (m, 2H), 4.56 - 4.51 (m, 1H), 3.84 (t, *J =* 7.0 Hz, 2H), 3.68 - 3.60 (m, 5H), 3.56 - 3.53 (m, 2H), 3.17 - 3.13 (m, 2H), 2.69 - 2.65 (m, 2H).

### Example 87 Synthesis of Compound A87:

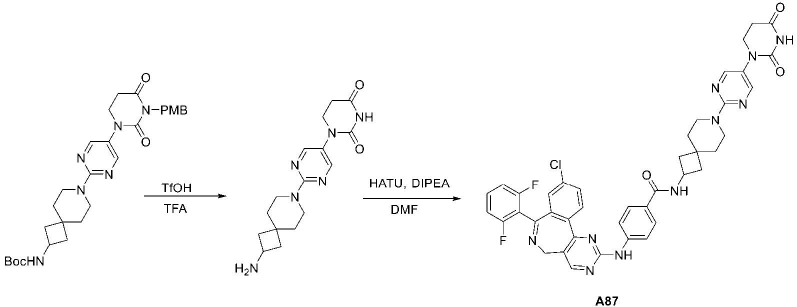

Compound **A87** (2.5 mg, yield 15.1%) was synthesized according to the synthesis method of compound **A54** described in **Example 54.** LCMS (ESI) m/z = 789.4 [M+H]⁺. ¹H NMR (400 MHz, DMSO-*d*₆) δ 10.47 (s, 1H), 10.20 (s, 1H), 8.76 (s, 1H), 8.49 (d, *J =* 8.0 Hz, 1H), 8.35 (s, 2H), 8.33 (d, *J =* 8.0 Hz, 1H), 7.96 - 7.86 (m, 5H), 7.62 - 7.54 (m, 1H), 7.37 (d, *J* = 2.2 Hz, 1H), 7.22 - 7.17 (m, 2H), 4.52 - 4.46 (m, 1H), 3.79 - 3.69 (m, 6H), 2.75 - 2.69 (m, 2H), 2.36 - 2.26 (m, 2H), 1.95 - 1.90 (m, 2H), 1.66 - 1.58 (m, 4H).

### Example 88 Synthesis of Compound A88:

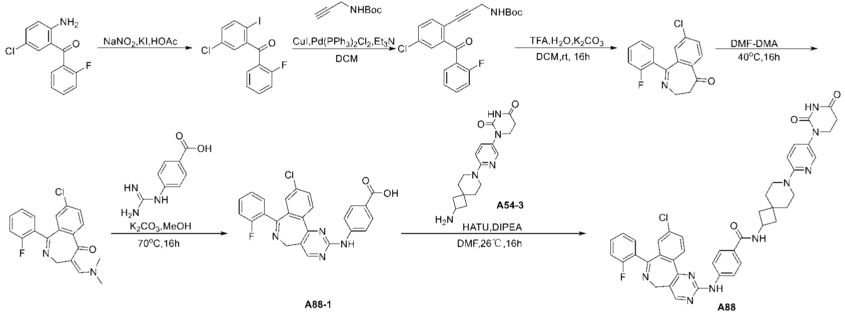

At room temperature, **A54-3** (9.69 mg, 29.42 µmol), HATU (15 mg, 39.23 µmol) and DIPEA (8 mg, 58.84 µmol) were added to compound **A88-1** (9.0 mg, 19.61 µmol; refer to ACS Medicinal Chemistry Letters (2015), 6(6), 630-634 for the synthesis method). The reaction mixture was stirred at 26°C for 16 h. After the addition of water, the reaction mixture was extracted with ethyl acetate, dried over anhydrous sodium sulfate, and concentrated under reduced pressure. The residue was separated for purification by silica gel column chromatography (methanol/dichloromethane = 1:20) to give a yellow oil **A88** (6 mg, yield 39%). LCMS (ESI) m/z = 770.2 [M+H]⁺. ¹H NMR (400 MHz, DMSO-*d*₆) δ 10.37 (s, 1H), 10.17 (s, 1H), 8.75 (s, 1H), 8.47 (d, *J =* 7.6 Hz, 1H), 8.31 (d, *J =* 8.4 Hz, 1H), 8.07 (d, *J =* 2.8 Hz, 1H), 7.95 - 7.91 (m, 3H), 7.89 - 7.86 (m, 3H), 7.67 - 7.63 (m, 1H), 7.56 - 7.49 (m, 1H), 7.38 - 7.33 (m, 2H), 7.24 - 7.17 (m, 1H), 6.89 (d, *J =* 9.2 Hz, 1H), 3.84 - 3.71 (m, 2H), 3.57 - 3.54 (m, 2H), 3.50 - 3.47 (m, 2H), 2.72 (t, *J =* 6.8 Hz, 2H), 2.27 (t, *J =* 9.6 Hz, 2H), 1.91 (t, *J* = 10.0 Hz, 2H), 1.68 - 1.65 (m, 2H), 1.64 - 1.61 (m, 2H).

### Example 89 Synthesis of Compound A89:

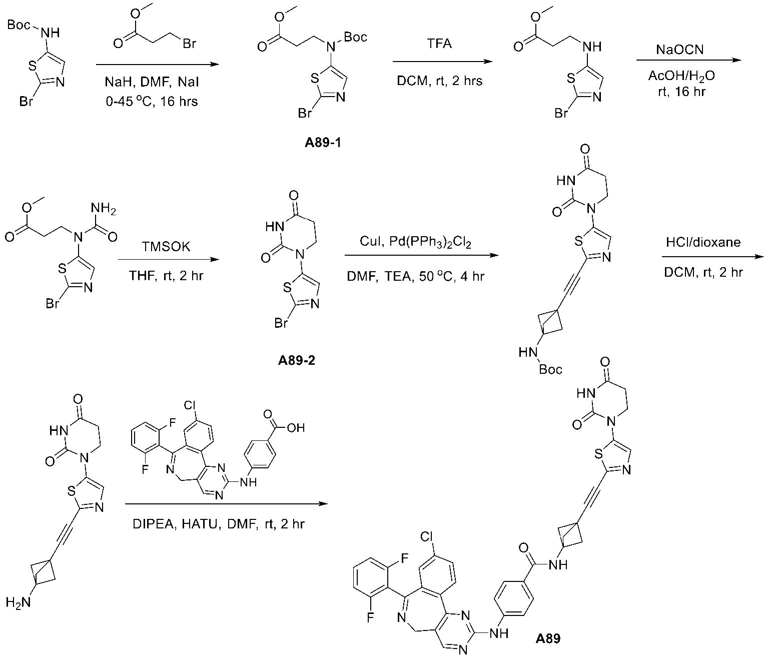

### Step 1:

Sodium hydride (1.07 g, 26.87 mmol, 60% purity) was added to a solution of tert-butyl (2-bromothiazol-5-yl)carbamate (5.0 g, 17.91 mmol) in N,N-dimethylformamide (30 mL) at 0°C. The reaction mixture was stirred at 0°C for 2 h. Methyl 3-bromopropionate (3.29 g, 19.70 mmol) and sodium iodide (1.34 g, 8.96 mmol) were added to the above solution sequentially, and the reaction mixture was heated to 45°C and stirred for 16 h. After LCMS showed that the reaction was completed, the reaction mixture was cooled to room temperature, quenched with a saturated aqueous ammonium chloride solution, extracted with ethyl acetate, and the organic phase was dried over anhydrous sodium sulfate, filtered, and concentrated. The crude product was purified by reverse phase column separation to give a yellow solid **A89-1** (3.5 g, yield 53.5%). LCMS (ESI) m/z = 364.9/366.9 [M+H]⁺.

### Step 2:

Compound **A89-2** (160 mg, yield 74.4%) was synthesized according to the synthesis method of compound **A58** described in **Example 58.** LCMS (ESI) m/z =275.9/277.9 [M+H]⁺.

### Step 3:

Compound **A89** (5.1 mg, yield 31.9%) was synthesized according to the synthesis method of compound **A6** described in **Example 6.** LCMS (ESI) m/z = 761.4 [M+H]⁺. ¹H NMR (400 MHz, DMSO-*d*₆) δ 10.86 (s, 1H), 10.20 (s, 1H), 8.98 (s, 1H), 8.73 (s, 1H), 8.30 (d, *J =* 8.4 Hz, 1H), 7.93 - 7.86 (m, 3H), 7.84 - 7.78 (m, 2H), 7.71 (s, 1H), 7.59 - 7.50 (m, 1H), 7.34 (d, *J =* 2.0 Hz, 1H), 7.21 - 7.13 (m, 2H), 4.04 (t, *J* = *6.8* Hz, 2H), 2.80 (t, *J =* 6.8 Hz, 2H), 2.48 (s, 6H).

### Example 90 Synthesis of Compound A90:

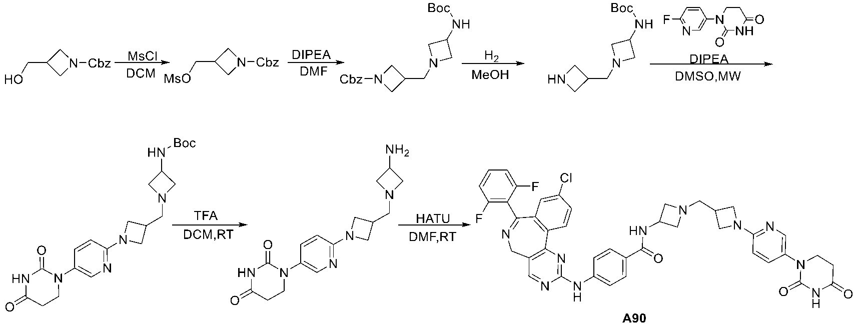

Compound **A90** (10.09 mg, yield 67.74%) was synthesized according to the synthesis method of compound **A54** described in **Example 54.** LCMS (ESI) m/z = 789.3 [M+H]⁺. ¹H NMR (400 MHz, DMSO-*d*₆) δ 10.34 (s, 1H), 10.18 (s, 1H), 8.73 (s, 1H), 8.60 (d, *J =* 6.8 Hz, 1H), 8.30 (d, *J =* 8.4 Hz, 1H), 8.00 (d, *J* = 2.8 Hz, 1H), 7.95 - 7.81 (m, 5H), 7.60 - 7.50 (m, 1H), 7.47 (dd, *J*₁ = 8.8 Hz, *J*₂ = 2.4 Hz, 1H), 7.34 (d, *J* = 2.0 Hz, 1H), 7.17 (t, *J* = 8.8 Hz, 2H), 6.38 (d, *J* = 8.8 Hz, 1H), 4.52 - 4.42 (m, 1H), 4.01 - 3.97 (m, 2H), 3.68 (t, *J =* 6.8 Hz, 2H), 3.63 - 3.53 (m, 4H), 3.03 (t, *J =* 6.8 Hz, 2H), 2.75 - 2.65 (m, 5H).

### Example 91 Synthesis of Compound A91:

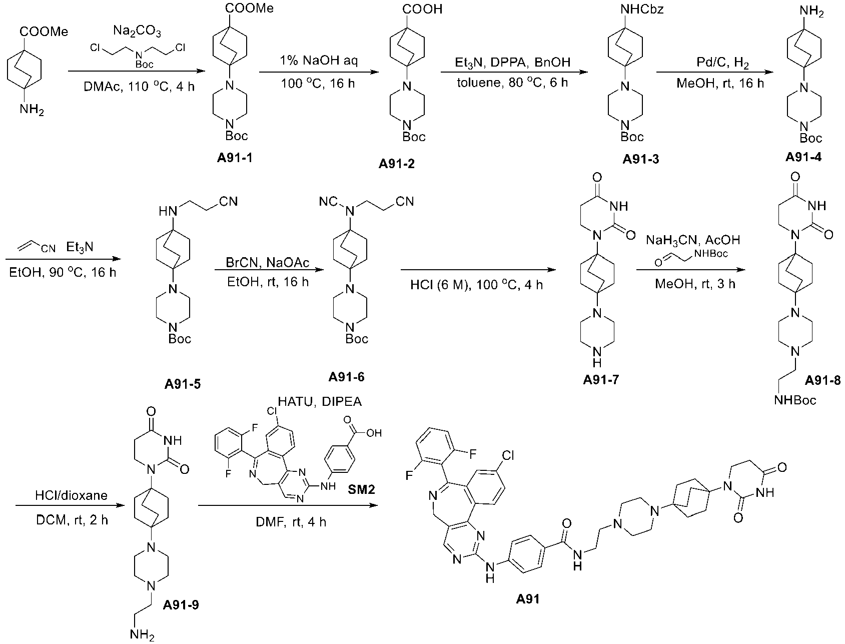

### Step 1:

At room temperature, tert-butyl N,N-bis(2-chloroethyl)carbamate (5.39 g, 22.27 mmol) and DIPEA (5.19 g, 7 mL) were added to a solution of compound methyl 4-aminobicyclo[2.2.2]octane-1-carboxylate (3.76 g, 17.72 mmol, synthesized with reference to WO2023067388A1) in *N,N*-dimethylacetamide (14 mL). The reaction mixture was heated to 110°C under argon protection and stirred for 4 h. After the addition of water, the reaction mixture was extracted with ethyl acetate, washed with saturated brine, dried over anhydrous sodium sulfate, concentrated under reduced pressure, and the residue was separated for purification by silica gel column chromatography to give a yellow liquid **A91-1** (0.83 g, yield 32%). LCMS (ESI) m/z = 353.4 [M+H]⁺.

### Step 2:

1% sodium hydroxide solution (2.5 mL) was added to compound **A91-1** (0.83 g, 2.35 mmol), and the reaction mixture was stirred at 100°C for 16 h. The reaction mixture was adjusted to pH 4-5 with a 3 M hydrochloric acid solution, and the resulting solution was directly separated for purification by reverse phase chromatography (acetonitrile/0.1% aqueous formic acid solution) to give a yellow oil **A91-2** (268 mg, yield 34%). LCMS (ESI) m/z = 339.3 [M+H]⁺.

### Step 5:

To a solution of **A91-2** (268 mg, 0.79 mmol) in toluene (5 mL), diphenyl phosphoryl azide (240 mg, 0.87 mmol, 0.19 mL) and triethylamine (96 mg, 0.95 mmol, 0.13 mL) were added at room temperature. The reaction mixture was heated to 70°C under argon protection and stirred for 1 h. Benzyl alcohol (94 mg, 0.87 mmol, 0.09 mL) and triethylamine (96 mg, 0.95 mmol, 0.13 mL) were added. The reaction mixture was stirred at 80°C for 6 h under argon protection. The reaction mixture was concentrated under reduced pressure, and the residue was separated for purification by silica gel column chromatography to give a white solid **A91-3** (228 mg, yield 65%). LCMS (ESI) m/z = 444.3 [M+H]⁺.

### Step 6:

At room temperature, 10% palladium on carbon (167 mg) was added to a solution of compound A91-3 (228 mg, 0.51 mmol) in methanol (5 mL), the atmosphere was replaced with hydrogen 3 times, and the reaction mixture was stirred at room temperature for 16 h. The reaction mixture was filtered and concentrated under reduced pressure to give a white solid **A91-4** (133 mg, yield 84%). LCMS (ESI) m/z = 309.3[M+H]⁺.

### Step 7:

To a solution of compound **A91-4** (133 mg, 0.43 mmol) in ethanol (2 mL), acrylonitrile (34 mg, 0.64 mmol, 0.04 mL) and triethylamine (130 mg, 1.29 mmol, 0.18 mL) were added at room temperature. The reaction mixture was heated to 90°C under argon protection and stirred for 16 h. The reaction mixture was concentrated under reduced pressure. Water was added to the residue, and the mixture was extracted with dichloromethane. The extract was washed with saturated brine, dried over anhydrous sodium sulfate, and concentrated under reduced pressure to give crude compound **A91-5** (155 mg, yield 100%). LCMS (ESI) m/z = 362.4 [M+H]⁺.

### Step 8:

Sodium acetate (56 mg, 0.68 mmol) and cyanogen bromide (75 mg, 0.68 mmol) were added to a solution of compound **A91-5** (155 mg, 0.43 mmol) in ethanol (3 mL) at room temperature. The reaction mixture was stirred at room temperature for 16 h under argon protection. The reaction mixture was concentrated under reduced pressure. Water was added to the residue, and the mixture was extracted with dichloromethane. The extract was washed with saturated brine, dried over anhydrous sodium sulfate, and concentrated under reduced pressure. The residue was purified by silica gel column chromatography to give crude **A91-6** (121 mg, yield 73%) as a yellow solid. LCMS (ESI) m/z = 388.4 [M+H]⁺.

### Step 9:

6 M hydrochloric acid (2 mL) was added to compound **A91-6** (121 mg, 0.31 mmol), and the reaction mixture was stirred at 100°C for 4 h. The reaction mixture was concentrated under reduced pressure to give crude compound **A91-7** (105 mg, yield 100%). LCMS (ESI) m/z = 307.3 [M+H]⁺.

### Step 10:

To a solution of compound **A91-7** (95 mg, 0.31 mmol) in methanol (2 mL) and acetic acid (0.3 mL), N-tert-butoxycarbonyl-2-aminoacetaldehyde (99 mg, 0.62 mmol) and sodium cyanoborohydride (39 mg, 0.62 mmol) were added at room temperature. The reaction mixture was stirred at room temperature for 3 h under argon protection. The reaction mixture was concentrated under reduced pressure, and the residue was separated for purification by silica gel column chromatography to give crude product **A91-8** (60 mg, yield 43%) as a white solid. LCMS (ESI) m/z = 450.2 [M+H]⁺.

### Step 11:

HCl (1 mL, 4.0 M in 1,4-dioxane) was added to a dichloromethane solution (2 mL) of compound **A91-8** (40 mg, 0.09 mmol), and the reaction mixture was stirred at room temperature for 2 h. The reaction mixture was directly spin-dried to give crude product **A91-9** (35 mg, yield 100%). LCMS (ESI) m/z = 350.3 [M+H]⁺.

### Step 12:

**SM2** (15 mg, 0.03 mmol), HATU (18 mg, 0.05 mmol) and DIPEA (12 mg, 0.09 mmol) were added to a solution of compound **A91-9** (24 mg, 0.06 mmol) in N,N-dimethylformamide (2 mL) at room temperature. The reaction mixture was stirred at room temperature for 4 h. The reaction mixture was separated for purification by reverse phase chromatography (acetonitrile/0.1% aqueous formic acid solution) to give a yellow solid **A91** (7 mg, yield 28%). LCMS (ESI) m/z = 807.4 [M+H]⁺. ¹H NMR (400 MHz, DMSO-*d*₆) δ 10.20 (s, 1H), 9.86 (s, 1H), 8.76 (s, 1H), 8.36 - 8.29 (m, 1H), 8.29 - 8.18 (m, 1H), 7.98- 7.87 (m, 3H), 7.87 - 7.79 (m, 2H), 7.65 - 7.51 (m, 1H), 7.37 (d, *J =* 2.4 Hz, 1H), 7.26 - 7.12 (m, 2H), 3.36 - 3.27 (m, 6H), 2.51 - 2.29 (m, 10H), 2.11 - 1.92 (m, 6H), 1.70 - 1.51 (m, 2H).

### Example 92 Synthesis of Compound A92:

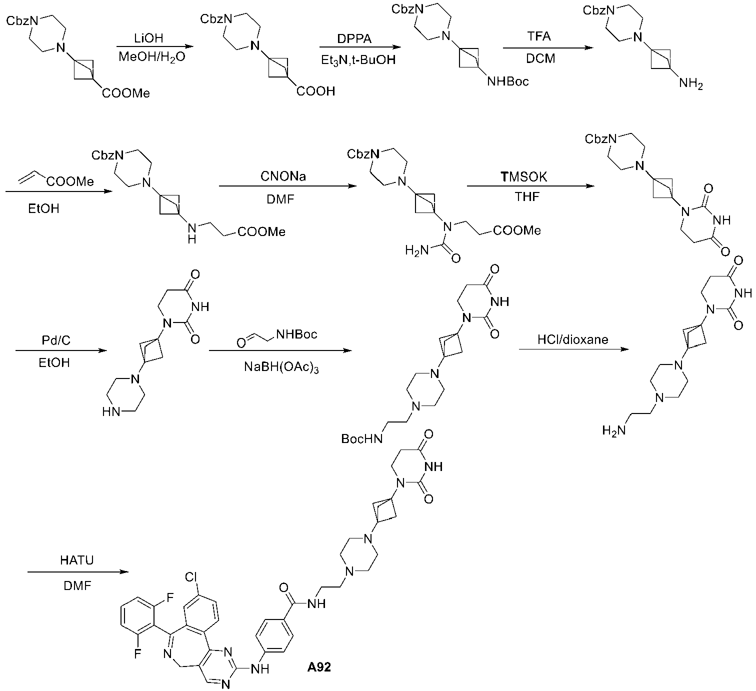

Compound **A92** (21 mg, yield 67%) was synthesized according to the synthesis method of compound **A91** described in **Example 91.** LCMS (ESI) m/z = 766.2 [M+H]⁺. ¹H NMR (400 MHz, DMSO-*d*₆) δ 10.20 (s, 1H), 10.09 (s, 1H), 8.76 (s, 1H), 8.32 (d, *J =* 7.8 Hz, 1H), 8.24 (t, *J* = 7.2 Hz, 1H), 7.93- 7.88 (m, 3H), 7.84 - 7.80 (m, 2H), 7.61 - 7.53 (m, 1H), 7.36 (d, *J=* 2.4 Hz, 1H), 7.26 - 7.12 (m, 2H), 3.36 - 3.27 (m, 6H), 2.51 - 2.29 (m, 10H).

### Example 93 Synthesis of Compound A93:

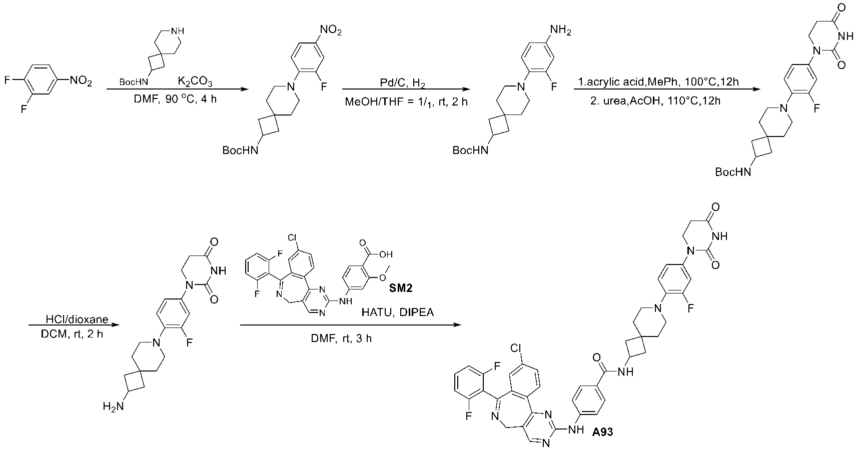

Compound **A93** (10 mg, yield 38%) was synthesized according to the synthesis method of compound **A8** described in **Example 8.** LCMS (ESI) m/z = 805.4 [M+H]⁺. ¹H NMR (400 MHz, DMSO-*d*₆) δ 10.40 (s, 1H), 10.21 (s, 1H), 8.77 (s, 1H), 8.49 (d, *J* = 7.6 Hz, 1H), 8.39 - 8.29 (m, 1H), 7.99 - 7.84 (m, 5H), 7.67 - 7.52 (m, 1H), 7.38 (d, *J* = 2.0 Hz, 1H), 7.28 - 7.15 (m, 3H), 7.15 - 7.06 (m, 2H), 4.59 - 4.39 (m, 1H), 3.78 (t, *J* = 6.8 Hz, 2H), , 3.08 - 2.97 (m, 2H), 2.97 - 2.86 (m, 2H), 2.73 (t, *J* = 6.8 Hz, 2H), 2.34 - 2.21 (m, 2H), 1.99 - 1.86 (m, 2H), 1.85- 1.67 (m, 4H).

### Example 94 Synthesis of Compound A94:

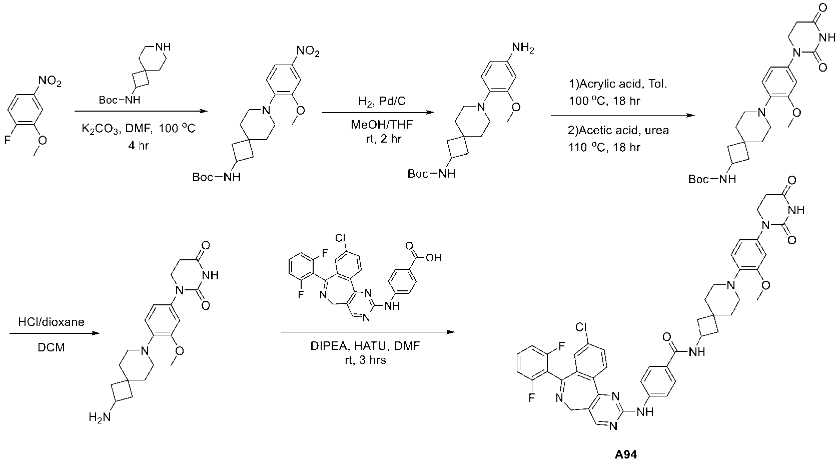

Compound **A94** (7.0 mg, yield 34.0%) was synthesized according to the synthesis method of compound **A8** described in **Example** 8. LCMS (ESI) m/z = 817.3 [M+H]⁺. ¹H NMR (400 MHz, DMSO-*d*₆) δ 10.30 (s, 1H), 10.17 (s, 1H), 8.73 (s, 1H), 8.44 (d, *J* = 7.6 Hz, 1H), 8.30 (d, *J =* 8.4 Hz, 1H), 7.94 - 7.82 (m, 5H), 7.60 - 7.50 (m, 1H), 7.34 (d, *J =* 2.0 Hz, 1H), 7.21 - 7.13 (m, 2H), 6.92 - 6.86 (m, 2H), 6.82 - 6.78 (m, 1H), 4.49 - 4.40 (m, 1H), 3.77 (s, 3H), 3.73 (t, *J* = 6.8 Hz, 2H), 2.94 - 2.79 (m, 4H), 2.69 (t, *J* = 6.8 Hz, 2H), 2.27 - 2.17 (m, 2H), 1.91 - 1.82 (m, 2H), 1.75 - 1.64 (m, 4H).

### Example 95 Synthesis of Compound A95:

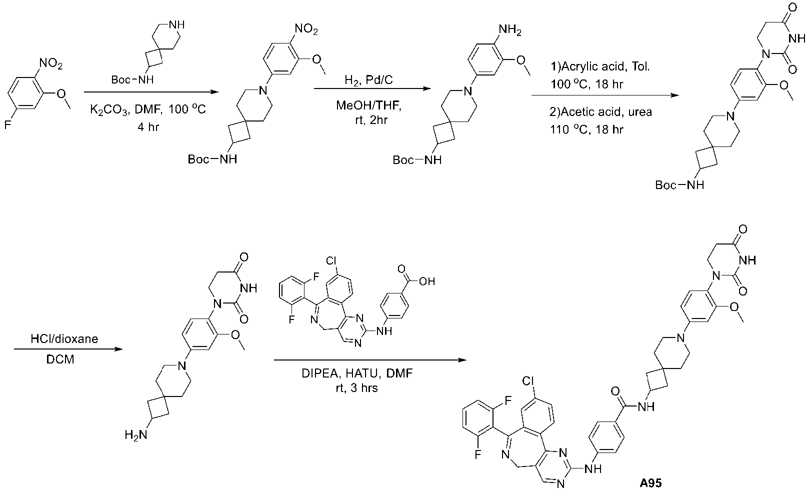

Compound **A95** (9.0 mg, yield 43.7%) was synthesized according to the synthesis method of compound A8 described in **Example 8.** LCMS (ESI) m/z = 817.2 [M+H]⁺. ¹H NMR (400 MHz, DMSO-*d*₆) δ 10.20 (s, 1H), 10.17 (s, 1H), 8.73 (s, 1H), 8.45 (d, *J* = 7.6 Hz, 1H), 8.30 (d, *J =* 8.4 Hz, 1H), 7.94 - 7.82 (m, 5H), 7.59 - 7.50 (m, 1H), 7.34 (d, *J =* 2.0 Hz, 1H), 7.21 - 7.13 (m, 2H), 7.01 (d, *J =* 8.6 Hz, 1H), 6.61 - 6.58 (m, 1H), 6.51 - 6.46 (m, 1H), 4.51 - 4.40 (m, 1H), 3.77 (s, 3H), 3.49 (t, *J =* 6.4 Hz, 2H), 3.23 - 3.16 (m, 2H), 3.13 - 3.06 (m, 2H), 2.64 (t, *J* = 6.4 Hz, 2H), 2.23 (t, *J* = 9.8 Hz, 2H), 1.88 (t, *J =* 9.8 Hz, 2H), 1.75 - 1.62 (m, 4H).

### Example 96 Synthesis of Compound A96:

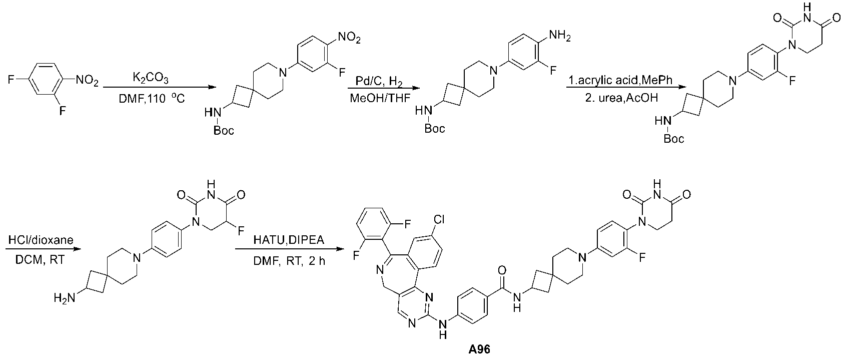

Compound **A96** (10.09 mg, yield 67.74%) was synthesized according to the synthesis method of **Example 8.** LCMS (ESI) m/z = 805.6 [M+H]⁺. ¹H NMR (400 MHz, DMSO-*d*₆) δ 10.37 (s, 1H), 10.16 (s, 1H), 8.73 (s, 1H), 8.44 (d, *J* = 7.6 Hz, 1H), 8.30 (d, *J =* 8.8 Hz, 1H), 7.98 - 7.76 (m, 5H), 7.62 - 7.48 (m, 1H), 7.34 (d, *J =* 2.4 Hz, 1H), 7.25 - 7.11 (m, 3H), 6.89 - 6.73 (m, 2H), 4.51 - 4.39 (m, 1H), 3.62 (t, *J =* 6.8 Hz, 2H), 3.25 - 3.16 (m, 2H), 3.15 - 3.08 (s, 2H), 2.68 (t, *J* = 6.8 Hz, 2H), 2.26 - 2.15 (m, 2H), 1.95 - 1.83 (m, 2H), 1.75 - 1.58 (m, 4H).

### Example 97 Synthesis of Compound A97:

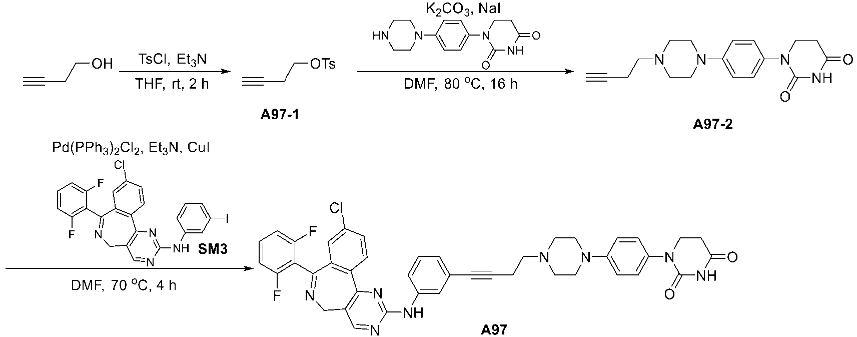

### Step 1:

Triethylamine (866 mg, 8.56 mmol) and p-toluenesulfonyl chloride (979 mg, 5.14 mmol) were added to a solution of compound butynol (300 mg, 4.28 mmol) in tetrahydrofuran (5 mL) at room temperature. The reaction mixture was stirred at room temperature for 2 h under argon protection. After the addition of water, the reaction mixture was extracted with ethyl acetate, washed with saturated brine, dried over anhydrous sodium sulfate, concentrated under reduced pressure, and the residue was separated for purification by silica gel column chromatography to give a colorless liquid **A97-1** (700 mg, yield 73%). LCMS (ESI) m/z = 225.3 [M+H]⁺.

### Step 2:

To a solution of compound **A97-1** (130 mg, 0.58 mmol) in N,N-dimethylformamide (3 mL) at room temperature, 1-(4-(piperazin-1-yl)phenyl)dihydropyrimidine-2,4(1H,3H)-dione (120 mg, 0.39 mmol, synthesized with reference to WO2023017446A1), sodium iodide (116 mg, 0.77 mmol) and potassium carbonate (160 mg, 1.16 mmol) were added. The reaction mixture was stirred at 80°C for 16 h under argon protection. After the addition of water, the reaction mixture was extracted with dichloromethane, washed with saturated brine, dried over anhydrous sodium sulfate, concentrated under reduced pressure, and the residue was separated for purification by silica gel column chromatography to give a white solid **A97-2** (90 mg, yield 71%). LCMS (ESI) m/z = 327.2 [M+H]⁺.

### Step 3:

To a solution of compound **A97-2** (23 mg, 0.07 mmol) in N,N-dimethylformamide (2 mL) at room temperature were added **SM3** (40 mg, 0.07 mmol; see **SM5** for the synthesis method), cuprous iodide (3 mg, 0.01 mmol), triethylamine (22 mg, 0.21 mmol) and tetrakis(triphenylphosphine)palladium (17 mg, 0.01 mmol). The reaction mixture was stirred at room temperature for 3 h under argon protection. The reaction mixture was filtered, and the filtrate was separated for purification by reverse phase chromatography (acetonitrile/10 mM ammonium bicarbonate solution) to give a yellow solid **A112** (17 mg, yield 31%). LCMS (ESI) m/z = 757.3 [M+H]⁺. ¹H NMR (400 MHz, DMSO-*d*₆) δ 10.33 (s, 1H), 10.06 (s, 1H), 9.75 (s, 1H), 8.73 (s, 1H), 8.30 (d, *J* = 8.4 Hz, 1H), 8.03 - 7.96 (m, 1H), 7.95 - 7.83 (m, 2H), 7.71 - 7.52 (m, 2H), 7.41 - 7.33 (m, 2H), 7.28 - 7.16 (m, 4H), 7.10 - 7.03 (m, 3H), 3.99 - 3.88 (m, 4H), 3.75 - 3.69 (m, 4H), 3.59 - 3.46 (m, 2H), 3.42 - 3.20 (m, 1H), 3.14 - 2.96 (m, 4H), 2.77 - 2.66 (m, 2H).

### Example 98 Synthesis of Compound A98:

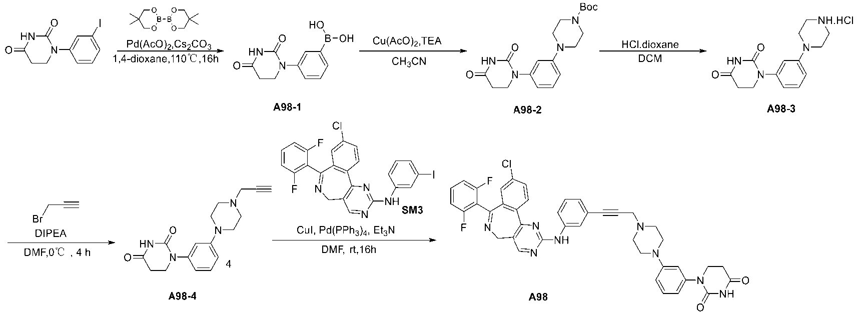

### Step 1:

To a solution of compound 1-(3-iodophenyl)dihydropyrimidine-2,4(1H,3H)-dione (1.00 g, 3.16 mmol, synthesized with reference to WO2023080732A1) in 1,4-dioxane (15.0 mL), bis(neopentyl glycolato)diboron (3.57 g, 15.82 mmol), palladium acetate (142.2 mg, 632.72 µmol), and potassium acetate (931.3 mg, 9.49 mmol) were added at room temperature. The reaction mixture was stirred at 110°C for 16 h under an argon atmosphere. Solid impurities were removed by suction filtration, the filtrate was concentrated, and the residue was separated for purification by a silica gel column (petroleum ether/ethyl acetate = 20:1) to give a yellow solid **A98-1** (500 mg, yield 67%). LCMS (ESI) m/z = 235.2 [M+H]⁺.

### Step 2:

2- Boc-piperazine (374 mg, 2.01 mmol) was dissolved in acetonitrile, and **A98-1** (470.0 mg, 2.01 mmol), copper acetate (729.29 mg, 4.02 mmol), triethylamine (406.46 mg, 4.02 mmol) and 4Å molecular sieve (191.68 mg, 1.90 mmol) were added sequentially. The mixture was stirred at 26°C for 16 h. After the reaction was completed, the mixture was directly spin-dried, and purified by flash silica gel column chromatography (EA: MeOH=10% to 10%) to give **A98-2** (300 mg, yield 54%). LCMS (ESI) m/z = 375.3 [M+H]⁺.

### Step 3:

At room temperature, **A98-2** (250.0 mg, 667.28 µmol) was dissolved in dichloromethane (10 mL), and a solution of HCl in dioxane (4 M, 5 mL) was added. The reaction mixture was stirred at 26°C for 2 h. The solvent was removed by concentration under reduced pressure to give a yellow solid **A98-3** (200 mg, yield 96%). LCMS (ESI) m/z = 275.1 [M+H]⁺.

### Step 4:

Bromopropyne (59 mg, 492.13 µmol) and DIPEA (141.5 mg, 1.09 mmol) were added to a solution of compound **A98-3** (150.0 mg, 546.81 µmol) in N,N-dimethylformamide (5 mL) at 0°C. The reaction mixture was heated to 0°C and stirred for 4 h. After the addition of water, the reaction mixture was extracted with ethyl acetate, dried over anhydrous sodium sulfate, concentrated under reduced pressure, and the residue was separated for purification by silica gel column chromatography (ethyl acetate/petroleum ether = 1:2) to give a yellow solid **A98-4** (100.0 mg, yield 58%). LCMS (ESI) m/z = 313.2 [M+H]⁺.

### Step 5:

Compounds **A98-4** (32 mg, 102.44 µmol), **SM3** (57.24 mg, 102.44 µmol), triethylamine (20.7 mg, 204.89 µmol), cuprous iodide (3.9 mg, 20.49 µmol) and Pd(PPh₃)₄ (23.7 mg, 20.49 µmol) were dissolved in N,N-dimethylformamide (5 mL) at room temperature. The reaction mixture was stirred at 80°C for 4 h. The reaction mixture was washed with water, and extracted with ethyl acetate. The organic phase was concentrated to remove the solvent, and the residue was separated for purification by silica gel column chromatography (petroleum ether/ethyl acetate = 10:1) to give a white solid **A98** (10.0 mg, yield 13%). LCMS (ESI) m/z *=* 743.5 [M+H]⁺. ¹H NMR (400 MHz, DMSO-*d*₆) δ 10.33 (s, 1H), 10.02 (s, 1H), 8.73 (s, 1H), 8.30 (d, *J =* 8.4 Hz, 1H), 8.01 (s, 1H),7.89 (dd, *J*₁ = 8.4 Hz, *J*₂ = 2.0 Hz, 1H), 7.85 (d, *J =* 8.4 Hz, 1H), 7.61 - 7.52 (m, 1H), 7.37 - 7.32 (m, 2H), 7.24 - 7.18 (m, 3H), 7.08 (d, *J* = 7.6 Hz, 1H), 6.92 (s, 1H), 6.84 (d, *J* = 8.4 Hz, 1H), 6.75 *(d, J* = 7.6 Hz, 1H), 3.78 - 3.75 (t, *J =* 6.8 Hz, 2H), 3.64 - 3.60 (m, 2H), 3.23 - 3.20 (m, 4H), 2.75 - 2.71 (m, 4H).

### Example 99 Synthesis of Compound A99:

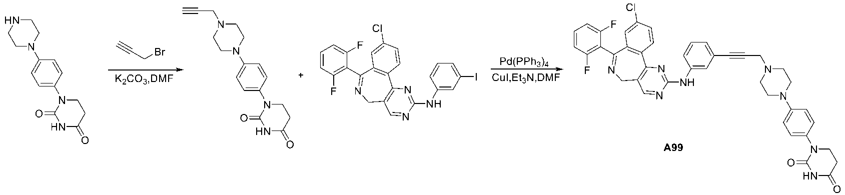

Compound **A99** (9.56 mg, yield 7.5%) was synthesized according to the synthesis method of compound **A97** described in **Example 97.** LCMS (ESI) m/z = 743.2 [M+H]⁺. ¹H NMR (400 MHz, DMSO-*d*₆) δ 10.29 (s, 1H), 10.02 (s, 1H), 8.73 (s, 1H), 8.30 (d, *J =* 8.4 Hz, 1H), 8.04 (s, 1H), 7.89 (dd, *J*₁ = 8.4 Hz, *J*₂ *=* 2.0 Hz, 1H), 7.83 (d, *J =* 8.8 Hz, 2H), 7.57 - 7.54 (m, 1H), 7.34 - 7.32 (m, 2H), 7.17 - 7.06 (m, 5H), 6.95 (d, *J* = 8.8 Hz, 2H), 3.71 (t, *J* = 6.8 Hz, 2H), 3.19 (bs, 4H), 2.72 - 2.68 (m, 6H).

### Example 100 Synthesis of Compound A100:

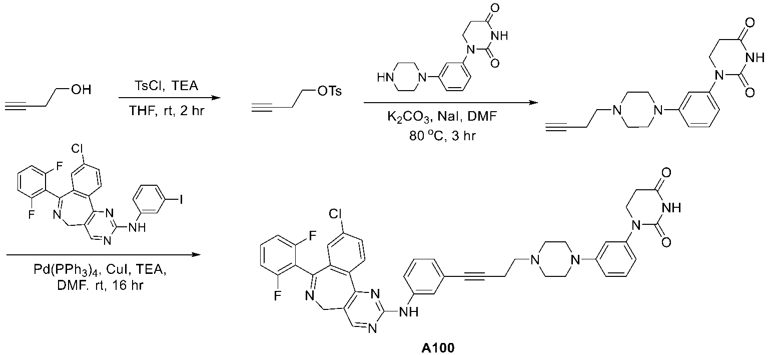

Compound **A100** (10.09 mg, yield 67.74%) was synthesized according to the synthesis method of compound A97 described in **Example 97.** (15 mg, yield 11.0%). LCMS (ESI) m/z = 757.3 [M+H]⁺. ¹H NMR (400 MHz, DMSO-*d*₆) δ 10.29 (s, 1H), 9.97 (s, 1H), 8.69 (s, 1H), 8.27 (d, *J =* 8.4 Hz, 1H), 7.91 (s, 1H), 7.87 (d, *J* = 8.4 Hz, 1H), 7.80 (d, *J =* 8.0 Hz, 1H), 7.58 - 7.48 (m, 1H), 7.33 (s, 1H), 7.28 (t, *J =* 7.6 Hz, 1H), 7.22 - 7.10 (m, 3H), 7.02 - 6.97 (m, 1H), 6.89 (s, 1H), 6.84 - 6.79 (m, 1H), 6.74 - 6.68 (m, 1H), 3.74 (t, *J =* 6.8 Hz, 2H), 3.18 - 3.12 (m, 4H), 2.70 - 2.60 (m, 10H).

### Example 101 Synthesis of Compound A101:

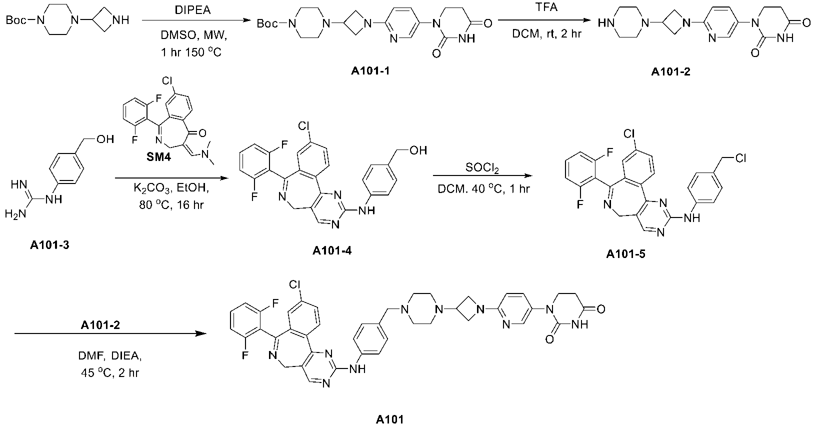

### Step 1:

Compound **A101-1** (60 mg, crude) was synthesized according to the synthesis method of compound A68-2 described in **Example 68.** LCMS (ESI) m/z =331.5 [M+H]⁺.

### Step 2:

Potassium carbonate (250.99 mg, 1.82 mmol) was added to a solution of **A101-3** (100 mg, 605.36 µmol, synthesized with reference to Bioorganic & Medicinal Chemistry Letters (2017), 27(18), 4440-4445) and **SM4** (218.40 mg, 605.36 µmol, synthesized with reference to ACS Medicinal Chemistry Letters (2015), 6(6), 630-634) in ethanol (4 mL) at room temperature. The reaction mixture was heated to 80°C and stirred for 16 h. After LCMS showed that the reaction was completed, the reaction mixture was quenched with a saturated aqueous ammonium chloride solution, extracted with ethyl acetate, and the organic phase was dried over anhydrous sodium sulfate, filtered, and concentrated. The crude product was separated for purification by silica gel column to give a yellow solid **A101-4** (140 mg, yield 49.9%). LCMS (ESI) m/z = 463.2 [M+H]⁺.

### Step 3:

Sulfoxide chloride (13.37 mg, 112.34 µmol, 8.19 µL) was added to a solution of **A101-4** (26 mg, 56.17 µmol) in dichloromethane (2 mL) at room temperature. The reaction mixture was heated to 40°C and stirred for 2 h. After LCMS showed that the reaction was complete, the reaction mixture was concentrated to give a yellow solid **A101-5** (28 mg, crude). LCMS (ESI) m/z = 481.3 [M+H]⁺.

### Step 4:

N,N-diisopropylethylamine (34.91 mg, 270.09 µmol, 47.04 µL) was added to a solution of **A101-5** (26 mg, 54.02 µmol) and **A101-2** (23.95 mg, 54.02 µmol, TFA) in N,N-dimethylformamide (3 mL) at room temperature. The reaction mixture was heated to 45°C and stirred for 16 h. After LCMS showed that the reaction was complete, the reaction mixture was filtered, and the filtrate was separated for purification by high-performance liquid chromatography to give a white solid **A101** (8 mg, yield 19.1%). LCMS (ESI) m/z = 775.4 [M+H]⁺. ¹H NMR (400 MHz, DMSO-*d*₆) δ 10.34 (s, 1H), 9.85 (s, 1H), 8.65 (s, 1H), 8.28 (d, *J =* 8.4 Hz, 1H), 8.02 - 7.98 (m, 1H), 7.89 - 7.83 (m, 1H), 7.76 (d, *J =* 8.4 Hz, 2H), 7.58 - 7.44 (m, 2H), 7.33 - 7.31 (m, 1H), 7.22 (d, *J* = 8.4 Hz, 2H), 7.19 - 7.12 (m, 2H), 6.39 (d, *J =* 8.8 Hz, 1H), 3.97 (t, *J =* 7.6 Hz, 2H), 3.75 - 3.64 (m, 4H), 3.41 (s, 2H), 3.26 - 3.20 (m, 1H), 2.69 (t, *J =* 6.8 Hz, 2H), 2.46 - 2.18 (m, 8H).

### Example 102 Synthesis of Compound A102:

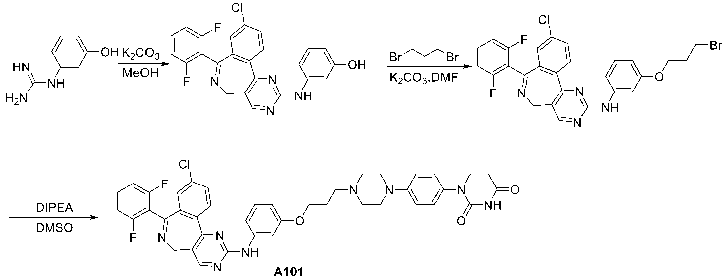

Compound **A102** (7 mg, yield 9.1%) was synthesized according to the synthesis method of compound **A101** described in **Example 101.** LCMS (ESI) m/z = 763.3 [M+H]⁺. ¹H NMR (400 MHz, DMSO-*d*₆) δ 10.29 (s, 1H), 9.91 (s, 1H), 8.70 (s, 1H), 8.33 (d, *J =* 8.4 Hz, 1H), 7.90 (dd, *J*₁ = 8.4 Hz, *J*₂ = 2.0 Hz, 1H), 7.71 (s, 1H), 7.57 - 7.54 (m, 1H), 7.36 - 7.32 (m, 2H), 7.22 (t, *J =* 8.0 Hz, 1H), 7.17 - 7.15 (m, 4H), 6.93 (d, *J* = 8.8 Hz, 2H), 6.59 (dd, *J*₁ = 8.8 Hz, *J*₂ = 2.0 Hz, 1H), 4.06 (t, *J* = 6.0 Hz, 2H), 3.72 (t, *J* = 6.8 Hz, 2H), 3.12 (bs, 4H), 2.72 (t, *J* = 6.8 Hz, 2H), 2.50 - 2.35 (m, 6H), 1.98 - 1.94 (m, 2H).

### Example 103 Synthesis of Compound A103:

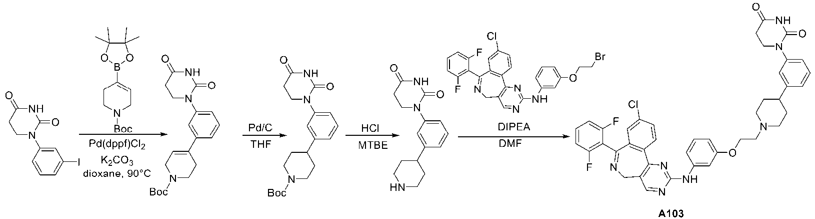

Compound **A103** (10.0 mg, yield 50.9%) was synthesized according to the synthesis method of compound **A101** described in **Example 101.** LCMS (ESI) m/z = 789.5 [M+H]⁺. ¹H NMR (400 MHz, DMSO-*d*₆) δ 10.34 (s, 1H), 9.89 (s, 1H), 8.68 (s, 1H), 8.31 (d, *J* = 8.4 Hz, 1H), 7.87 (dd, *J*₁ = 8.6 Hz, *J*₂ = 2.2 Hz, 1H), 7.71 (s, 1H), 7.56 - 7.49 (m, 1H), 7.33 - 7.26 (m, 3H), 7.21 - 7.08 (m, 6H), 6.59 - 6.55 (m, 1H), 4.13 - 4.08 (m, 2H), 3.77 (t, *J* = 6.6 Hz, 2H), 3.07 - 3.00 (m, 2H), 2.77 - 2.73 (m, 2H), 2.69 (t, *J* = 6.6 Hz, 2H), 1.74 - 1.65 (m, 4H).

### Example 104 Synthesis of Compound A104:

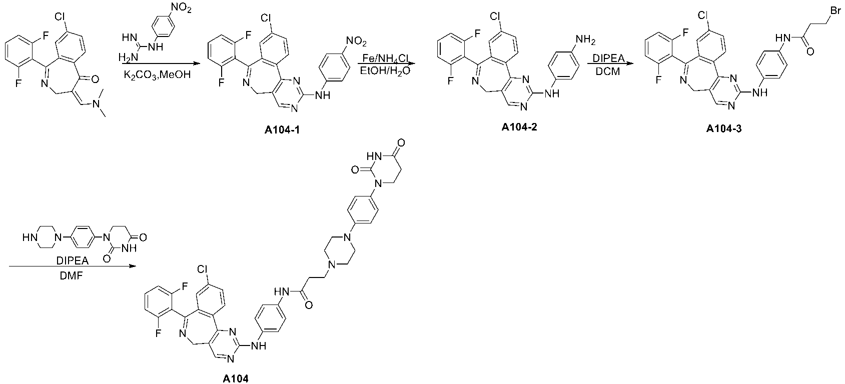

### Step 1:

To a solution of compound **SM4** (1.0 g, 2.077 mmol) in methanol (20 mL) at room temperature, 1-(4-nitrophenyl)guanidine nitrate (660.5 mg, 3.05 mmol) and potassium carbonate (957.7 mg, 6.93 mmol) were added. The reaction mixture was stirred at 70°C for 12 h. Solid impurities were removed by suction filtration, and the filtrate was concentrated and purified on a silica gel column to give a yellow solid **A104-1** (183 mg, yield 13.82%). LCMS (ESI) m/z = 478.1 [M+H]⁺.

### Step 2:

Compound **A104-2** (135 mg, yield 78.71%) was synthesized according to the synthesis method of compound **A64-1** described in **Example 64.** LCMS (ESI) m/z = 477.8 [M+H]⁺.

### Step 3:

At room temperature, 3-bromopropionyl chloride (41.3 mg, 0.24 mmol) and *N,N-*diisopropylethylamine (93.5 mg, 0.72 mmol) were added to a solution of compound **A104-2** (108 mg, 0.24 mmol) in dichloromethane (5 mL). The reaction mixture was stirred at 25°C for 12 h. The reaction mixture was concentrated and purified by normal phase column to give **A104-3** (53.6 mg, yield 38.14%). LCMS (ESI) m/z = 581.6/583.7 [M+H]⁺.

### Step 4:

To a solution of compound **A104-3** (53.6 mg, 0.096 mmol) in N,N-dimethylformamide (3 mL) at room temperature, 1-(4-(piperazin-1-yl)phenyl)dihydropyrimidine-2,4(1H,3H)-dione (52.71 mg, 0.192 mmol) and N,N-diisopropylethylamine (37.2 mg, 0.288 mmol) were added. The reaction mixture was stirred at 25°C for 12 h. The reaction mixture was separated for purification by reverse phase chromatography (acetonitrile/0.1% aqueous formic acid solution = 2.3/1) to give a yellow solid **A104** (7.72 mg, yield 10.0%). LCMS (ESI) m/z = 775.8 [M+H]⁺. ¹H NMR (400 MHz, DMSO-*d*₆) δ 10.26 (s, 1H), 9.99 (s, 1H), 9.79 (s, 1H), 8.63 (s, 1H), 8.27 (d, *J* = 8.4 Hz, 1H), 7.87 (dd, *J*₁ = 8.4 Hz, *J*₂ = 2.0 Hz, 1H), 7.71 (d, *J =* 8.8 Hz, 2H), 7.60 - 7.48 (m, 3H), 7.31 (d, *J =* 2.0 Hz, 1H), 7.21 - 7.11 (m, 4H), 6.98 - 6.91 (m, 2H), 3.69 (t, *J =* 6.4 Hz, 2H), 3.17 - 3.12 (m, 4H), 2.74 - 2.64 (m, 4H), 2.61 - 2.56 (m, 4H), 2.54 - 2.51 (m, 2H).

### Example 105 Synthesis of Compound A105:

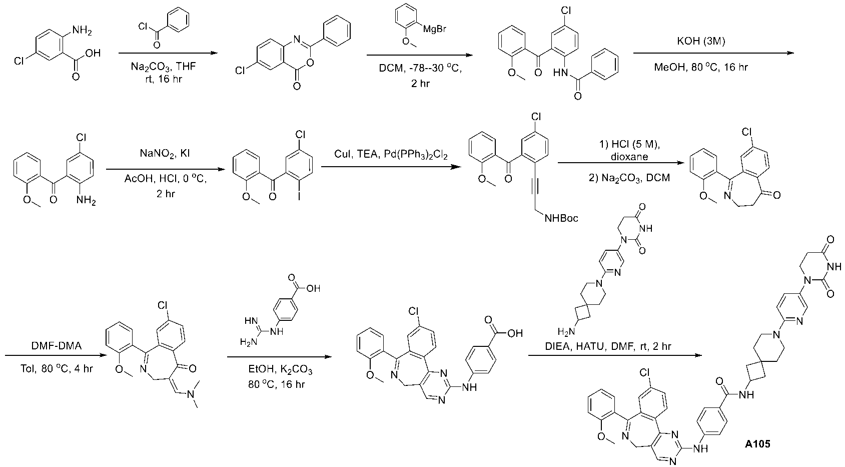

Compound **A105** (8.0 mg, yield 40.1%) was synthesized according to the synthesis method of compound A6 described in **Example 6.** LCMS (ESI) m/z = 782.5 [M+H]⁺. ¹H NMR (400 MHz, DMSO-*d*₆) δ 10.33 (s, 1H), 10.14 (s, 1H), 8.69 (s, 1H), 8.44 (d, *J =* 7.6 Hz, 1H), 8.24 (d, *J =* 8.4 Hz, 1H), 8.04 (d, J = 2.8 Hz, 1H), 7.94 - 7.89 (m, 2H), 7.86 - 7.82 (m, 2H), 7.81 - 7.77 (m, 1H), 7.51 - 7.40 (m, 3H), 7.15 (d, *J =* 2.4 Hz, 1H), 7.06 (t, *J = 7.6* Hz, 1H), 6.98 (d, *J* = 8.4 Hz, 1H), 6.85 (d, *J = 9.2* Hz, 1H), 4.85 (bs, 1H), 4.50 - 4.42 (m, 1H), 3.79 (bs, 1H), 3.69 (t, *J* = 6.8 Hz, 2H), 3.55 - 3.41 (m, 4H), 3.30 (s, 3H), 2.69 (t, *J* = 6.8 Hz, 2H), 2.28 - 2.19 (m, 2H), 1.93-1.83 (m, 2H), 1.67 - 1.55 (m, 4H).

### Example 106 Synthesis of Compound A106:

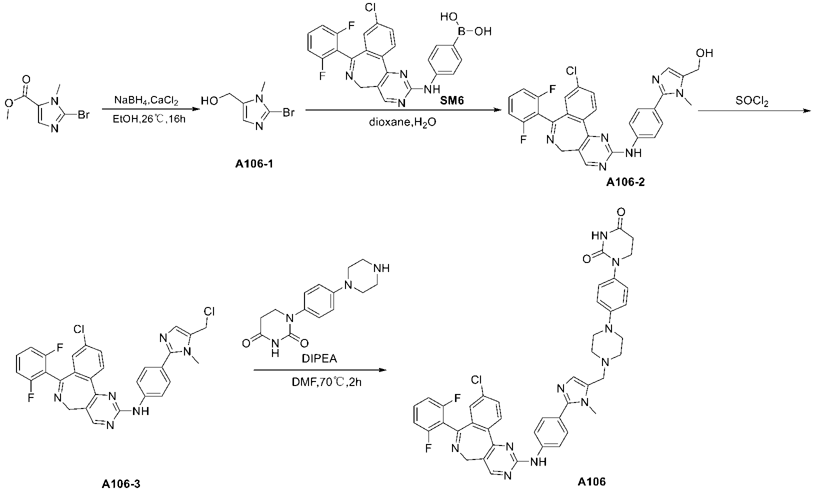

### Step 1:

Sodium borohydride (504 mg, 13.7 mmol) and calcium chloride (1.52 g, 13.7 mmol) were added to a solution of 2-bromo-3-methyl-3H-imidazole-4-carboxylic acid methyl ester (1.0 g, 4.57 mmol) in ethanol (20 mL) at room temperature. The reaction mixture was stirred at 26°C for 16 h. The reaction mixture was filtered and concentrated. The crude product was separated for purification by silica gel column chromatography (petroleum ether/ethyl acetate = 5:1) to give a white solid **A106-1** (750 mg, yield 86%). LCMS (ESI) m/z = 191.2 [M+H]⁺.

### Step 2:

Compound **A106-1** (600 mg, 3.14 mmol), cesium carbonate (2.05 g, 6.28 mmol), tetrakis(triphenylphosphine)palladium (362 mg, 314 µmol) and SM6 (1.8 g, 3.77 mmol) were dissolved in dioxane/H₂O = 10/1 (20 mL) at room temperature, and the reaction mixture was stirred at 90°C for 16 h under argon protection. The reaction mixture was concentrated to remove the solvent, and the residue was separated for purification by silica gel column chromatography (dichloromethane/methanol = 10:1) to give a white solid **A106-2** (1.0 g, 58%). LCMS (ESI) m/z = 543.2 [M+H]⁺.

### Step 3:

At room temperature, compound **A106-2** (600 mg, 1.11 mmol) was dissolved in dichloromethane (10 mL), and sulfoxide chloride (2 mL) was slowly added dropwise and stirred at 26°C for 2 h. The reaction mixture was concentrated to remove the solvent to give a yellow solid **A106-3** (400 mg, 64%). LCMS (ESI) m/z = 561.1 [M+H]⁺.

### Step 4:

DIPEA (13.8 mg, 106.87 µmol) and 1-(4-(piperazin-1-yl)phenyl)dihydropyrimidine-2,4(1H,3H)-dione (16.6 mg, 53.44 µmol) were added to a solution of **A106-3** (30 mg, 53.44 µmol) in *N,N-*dimethylformamide (10 mL) at room temperature. The reaction mixture was stirred at 70°C for 2 h. With addition of saturated brine, the reaction mixture was extracted with ethyl acetate, and the organic phase then was dried over anhydrous sodium sulfate, filtered, and concentrated. The crude product was separated for purification by silica gel column chromatography (dichloromethane/methanol = 10:1) to give a white solid A106 (9 mg, yield 21%). LCMS (ESI) m/z = 799.2 [M+H]⁺. ¹H NMR (400 MHz, DMSO-*d*₆) δ 10.28 (s, 1H), 10.17 (s, 1H), 8.74 (s, 1H), 8.34 (d, *J =* 8.4 Hz, 1H), 7.97 (d, *J =* 8.4 Hz, 2H), 7.90 (dd, *J*₁ = 8.0 Hz, *J*₂ = 2.4 Hz, 1H), 7.65 (d, *J =* 8.4 Hz, 2H), 7.59 - 7.51 (m, 1H), 7.36 (d, *J =* 2.4 Hz, 1H), 7.22 - 7.16 (m, 4H), 6.96 (d, *J* = 9.2 Hz, 2H), 3.75 (s, 3H), 3.72 (t, *J =* 6.8 Hz, 2H), 3.59 - 3.56 (m, 2H), 3.19 - 3.16 (m, 4H), 2.70 (t, *J =* 6.8 Hz, 2H), 2.68 - 2.64 (m, 2H).

### Example 107 Synthesis of Compound A107:

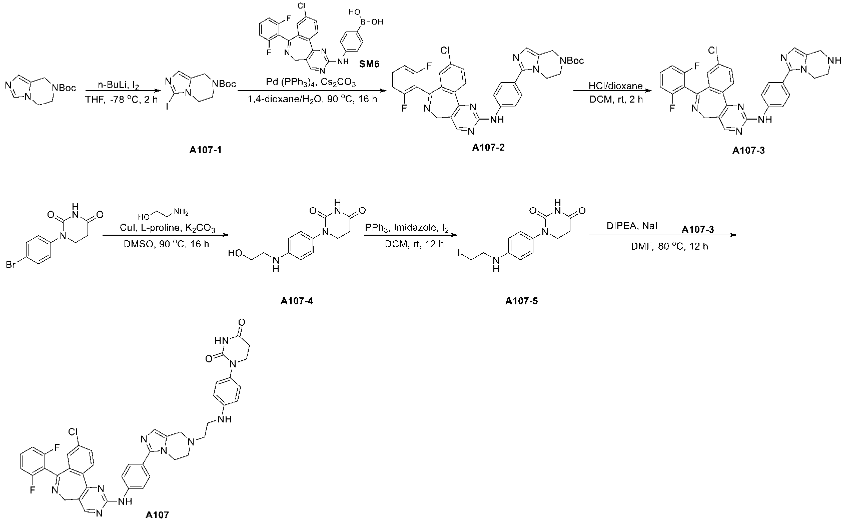

### Step 1:

To a solution of tert-butyl 5,6-dihydroimidazo[1,5-A]pyrazine-7(8H)-carboxylate (200 mg, 0.90 mmol) in tetrahydrofuran (2 mL) was added n-butyl lithium (1.6 M in hexanes, 0.67 mL, 1.07 mmol) under - 78°C argon protection. The reaction mixture was stirred at -78°C for 1 h under argon protection. A solution of iodine (273 mg, 1.07 mmol) in tetrahydrofuran (1 mL) was slowly added dropwise, and stirring was continued for 1 h after the addition. The reaction mixture was quenched with saturated ammonium chloride and extracted with ethyl acetate. The organic phase was washed with saturated brine, dried over anhydrous sodium sulfate, and concentrated under reduced pressure. The residue was purified by silica gel column chromatography to give a yellow solid **A107-1** (150 mg, yield 48%). LCMS (ESI) m/z = 450.1 [M+H]⁺.

### Step 2:

**SM6** (60 mg, 0.13 mmol), cesium carbonate (82 mg, 0.25 mmol) and tetrakis(triphenylphosphine)palladium (7 mg, 0.006 mmol) were added to a mixed solution of compound **A107-1** (44 mg, 0.13 mmol) in 1,4-dioxane (2 mL) and water (0.4 mL) at room temperature. The reaction mixture was heated to 90°C under argon protection and stirred for 16 h. After the addition of water, the reaction mixture was extracted with ethyl acetate, washed with saturated brine, dried over anhydrous sodium sulfate, concentrated under reduced pressure, and the residue was separated for purification by silica gel column chromatography to give a yellow solid **A107-2** (44 mg, yield 53%). LCMS (ESI) m/z = 654.1 [M+H]⁺.

### Step 3:

HCl (4 M in dioxane, 1 mL) was added to a solution of compound **A107-2** (44 mg, 0.07 mmol) in dichloromethane (2 mL) at room temperature. The reaction mixture was stirred at room temperature for 2 h. The reaction mixture was concentrated under reduced pressure to give a light yellow solid **A107-3** (40 mg, yield 100%, crude product). LCMS (ESI) m/z = 554.2 [M+H]⁺.

### Step 4:

Ethanolamine (454 mg, 7.43 mmol), *L*-proline (428 mg, 3.72 mmol), potassium carbonate (1.54 g, 11.15 mmol) and cuprous iodide (142 mg, 0.74 mmol) were added to a solution of compound 1-(4-bromophenyl)dihydropyrimidine-2,4(1H,3H)-dione (1 g, 3.72 mmol) in dimethyl sulfoxide (6 mL) at room temperature. The reaction mixture was heated to 90°C under argon protection and stirred for 16 h. The reaction mixture was purified by reverse phase separation to give a yellow solid **A107-4** (95 mg, yield 10%). LCMS (ESI) m/z = 250.2 [M+H]⁺.

### Step 5:

Imidazole (46 mg, 0.68 mmol), triphenylphosphine (179 mg, 0.68 mmol) and iodine (173 mg, 0.68 mmol) were added to a solution of compound **A107-4** (85 mg, 0.34 mmol) in dichloromethane (1 mL) at room temperature. The reaction mixture was stirred at room temperature for 12 h. The reaction mixture was concentrated under reduced pressure, and the residue was separated for purification by silica gel column chromatography to give a yellow solid **A107-5** (72 mg, yield 59%). LCMS (ESI) m/z =360.1 [M+H]⁺.

### Step 6:

Sodium iodide (17 mg, 0.11 mmol), DIPEA (22 mg, 0.17 mmol) and **A107-3** (33 mg, 0.06 mmol) were added to a solution of compound **A107-5** (40 mg, 0.11 mmol) in *N,N*-dimethylformamide (1 mL) at room temperature. The reaction mixture was heated to 80°C under argon protection and stirred for 12 h. The reaction mixture was separated for purification by reverse phase chromatography (acetonitrile/0.1% aqueous formic acid solution) to give a light yellow solid **A107** (6 mg, yield 8%). LCMS (ESI) m/z = 785.3 [M+H]⁺. ¹H NMR (400 MHz, DMSO-*d*₆) δ 10.23 (s, 1H), 10.09 (s, 1H), 8.73 (s, 1H), 8.39 - 8.26 (m, 1H), 8.00 - 7.83 (m, 3H), 7.75 - 7.65 (m, 2H), 7.65 - 7.51 (m, 1H), 7.36 (d, *J =* 2.4 Hz, 1H), 7.28 - 7.12 (m, 2H), 7.10 - 6.98 (m, 2H), 6.79 (s, 1H), 670 - 6.58 (m, 2H), 5.59 (t, *J =* 5.6 Hz, 1H), 4.19 (t, *J =* 5.2 Hz, 2H), 3.78 (s, 2H), 3.68 (t, *J =* 6.4 Hz, 2H), 3.31 - 3.20 (m, 2H), 2.97 - 2.84 (m, 2H), 2.82 - 2.72 (m, 2H), 2.72 - 2.63 (m, 2H).

### Example 108 Synthesis of Compound A108:

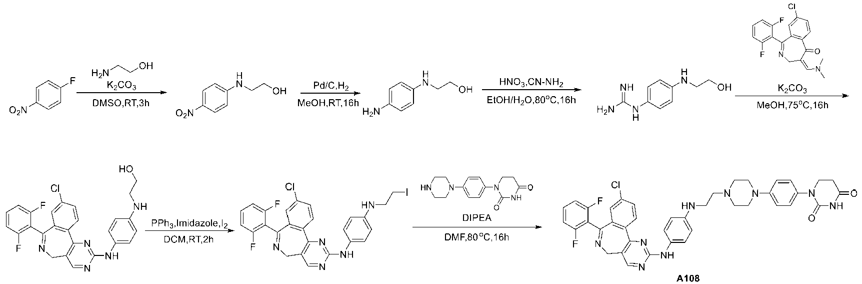

Compound **A108** (15.8 mg, yield 17.6%) was synthesized according to the synthesis method of compound **A101** described in **Example 101.** LCMS (ESI) m/z = 748.2 [M+H]⁺. ¹H NMR (400 MHz, DMSO-*d*₆) δ 10.26 (s, 1H), 9.38 (s, 1H), 8.53 (s, 1H), 8.23 (d, *J =* 8.5 Hz, 1H), 7.86 - 7.80 (m, 1H), 7.59 - 7.50 (m, 1H), 7.45 (d, *J =* 8.0 Hz, 2H), 7.30 - 7.27 (m, 1H), 7.21 - 7.12 (m, 4H), 6.98 - 6.91 (m, 2H), 6.61 - 6.56 (m, 2H), 5.18 (t, *J =* 5.6 Hz, 1H), 3.70 (t, *J =* 6.8 Hz, 2H), 3.20 - 3.13 (m, 6H), 2.71 - 2.65 (t, *J =* 6.8 Hz, 2H), 2.61- 2.55 (m, 6H).

### Example 109 Synthesis of Compound A109:

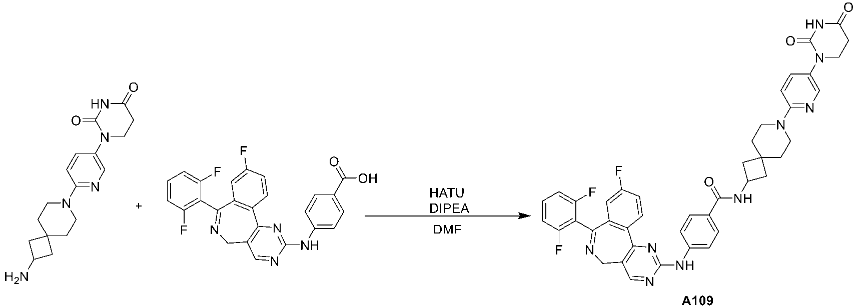

Compound **A109** (15.0 mg, yield 59.6%) was synthesized according to the synthesis method of compound **A1** described in **Example 1.** LCMS (ESI) m/z = 772.5 [M+H]⁺. ¹H NMR (400 MHz, DMSO-*d*₆) δ 10.15 (s, 1H), 8.72 (s, 1H), 8.45 (d, *J =* 8.0 Hz, 1H), 8.38 - 8.34 (m, 1H), 8.04 - 8.02 (m, 1H), 7.92 - 7.90 (m, 2H), 7.86 - 7.83 (m, 2H), 7. 71 - 7.66 (m, 1H), 7.58 - 7.52 (m, 1H), 7.48 - 7.44 (m, 1H), 7.18 - 7.15 (m, 2H), 6.87 - 6.82 (m, 1H), 4.51 - 4.40 (m, 1H), 3.71 - 3.67 (m, 2H), 3.53 - 3.39 (m, 4H), 2.71 - 2.67 (m, 3H), 2.26 - 2.18 (m, 2H), 1.91 - 1.86 (m, 1H), 1.66 - 1.52 (m, 4H).

### Example 110 Synthesis of Compound A110:

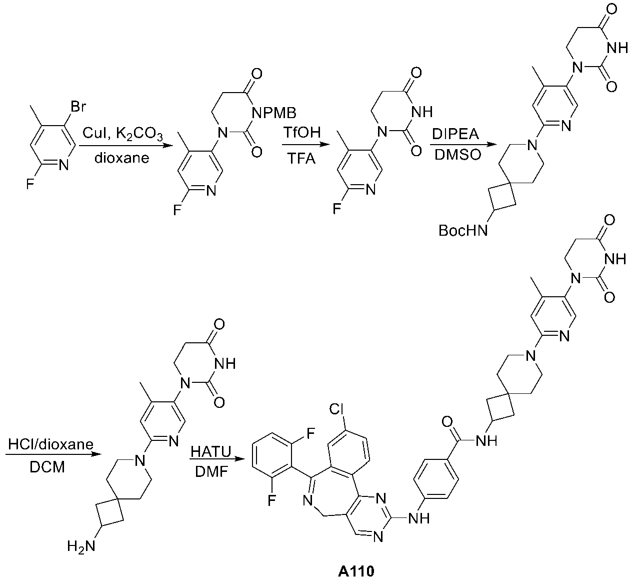

Compound **A110** (13.46 mg, yield 43.09%) was synthesized according to the synthesis method of compound **A54** described in **Example A54.** LCMS (ESI) m/z = 802.16 [M+H]⁺. ¹H NMR (400 MHz, DMSO-*d*₆) *δ* 10.33 (s, 1H), 10.17 (s, 1H), 8.73 (s, 1H), 8.44 (d, *J =* 7.6 Hz, 1H), 8.30 (d, *J =* 8.4 Hz, 1H), 7.95 - 7.81 (m, 6H), 7.61 - 7.49 (m, 1H), 7.34 (d, *J* = 2.0 Hz, 1H), 7.22 - 7.13 (m, 2H), 6.75 (s, 1H), 4.51 - 4.40 (m, 1H), 3.72 - 3.61 (m, 1H), 3.57 - 3.48 (m, 3H), 3.47 - 3.42 (m, 2H), 2.75 - 2.66 (m, 2H), 2.24 (t, *J* = 10.0 Hz, 2H), 2.10 (s, 3H), 1.88 (t, *J =* 10.0 Hz, 2H), 1.69 - 1.53 (m, 4H).

### Example 111 Synthesis of Compound A111:

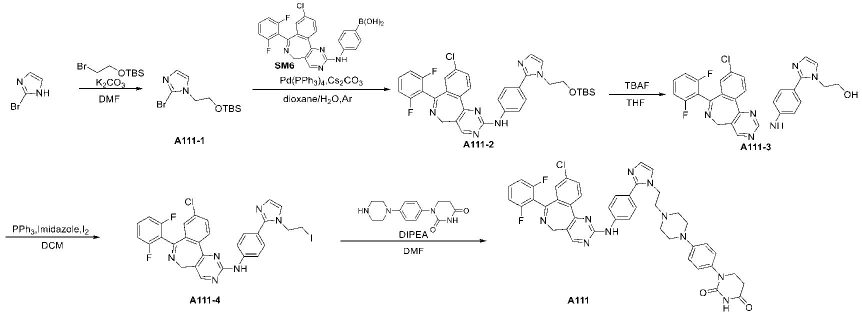

### Step 1:

Potassium carbonate (1.4 g, 10.2 mmol) and (2-bromoethoxy)-tert-butyldimethylsilane (980 mg, 4.1 mmol) were added to a solution of 2-bromo-1H-imidazole (500 mg, 3.4 mmol) in *N,N*-dimethylformamide (5 mL) at room temperature. The reaction mixture was stirred at room temperature for 16 h, and thin layer chromatography showed that the raw materials reacted completely. Water was added, ethyl acetate was added for extraction, anhydrous sodium sulfate was used for drying, and concentration under reduced pressure was performed. The residue was separated for purification by silica gel column chromatography (ethyl acetate/petroleum ether = 1:20) to give a colorless oil **A111-1** (1 g, yield 96.3%). LCMS (ESI) m/z = 305.4 [M+H]⁺.

### Step 2:

**A111-1** (128 mg, 0.42 mmol), tetrakis(triphenylphosphine)palladium (48.5 mg, 0.04 mmol) and cesium carbonate (273 mg, 0.84 mmol) were added to a solution of **SM6** (200 mg, 0.42 mmol) in 1,4-dioxane (10 mL) and water (1 mL) at room temperature. The atmosphere was replaced with argon 3 times, and the mixture was heated to 100°C and stirred for 16 h. After the addition of water, the mixture was extracted with ethyl acetate , dried over anhydrous sodium sulfate, and concentrated under reduced pressure. The residue was separated for purification by silica gel column chromatography (ethyl acetate/petroleum ether = 1:5) to give a yellow solid **A111-2** (120 mg, yield 43.5%). LCMS (ESI) m/z = 657.6 [M+H]⁺.

### Step 3:

M tetrabutylammonium fluoride (0.4 mL, 0.4 mmol) was added to a solution of **A111-2** (120 mg, 0.18 mmol) in tetrahydrofuran (3 mL) at room temperature, and the mixture was stirred for 3 h. LC-MS showed that the raw materials reacted completely. Water was added, ethyl acetate was added for extraction, anhydrous sodium sulfate was used for drying, and concentration under reduced pressure was performed. The residue was separated for purification by silica gel column chromatography (ethyl acetate/petroleum ether = 1:3) to give a yellow solid **A111-3** (85 mg, yield 85.7%). LCMS (ESI) m/z = 543.1 [M+H]⁺.

### Step 4:

Triphenylphosphine (82 mg, 0.3 mmol), imidazole (21 mg, 0.3 mmol) and elemental iodine (80 mg, 0.3 mmol) were added to a solution of **A111-3** (85 mg, 0.15 mmol) in dichloromethane (3 mL) at room temperature. The reaction mixture was stirred at room temperature for 2 h. After the addition of water, the mixture was extracted with dichloromethane, dried over anhydrous sodium sulfate, and concentrated under reduced pressure. The residue was separated for purification by silica gel column chromatography (ethyl acetate/petroleum ether = 1:3) to give a yellow solid **A111-4** (60 mg, yield 58.7%). LCMS (ESI) m/z = 653.2 [M+H]⁺.

### Step 5:

To a solution of **A111-4** (60 mg, 0.09 mmol) in N,N-dimethylformamide (3 mL), 1-(4-(piperazin-1-yl)phenyl)dihydropyrimidine-2,4(1H,3H)-dione (25 mg, 0.09 mmol) and DIPEA (36 mg, 0.27 mmol) were added at room temperature. The mixture was heated to 80°C and stirred to react for 16 h. LCMS showed a product yield of about 30%. The reaction mixture was filtered, and the crude product was separated for purification by reverse phase chromatography (acetonitrile/10 mM aqueous ammonium bicarbonate solution) to give a yellow solid **A111** (7.6 mg, yield 10.3%). LCMS (ESI) m/z = 799.2 [M+H]⁺. ¹H NMR (400 MHz, DMSO-*d*₆) δ 10.27 (s, 1H), 10.11 (s, 1H), 8.71 (s, 1H), 8.30 (d, *J =* 8.8 Hz, 1H), 7.97 - 7.92 (m, 2H), 7.88 - 7.82 (m, 1H), 7.63 - 7.51 (m, 3H), 7.38 - 7.32 (m, 2H), 7.21 - 7.07 (m, 4H), 6.97 (s, 1H), 6.91 - 6.85 (m, 2H), 4.21 - 4.14 (m, 2H), 3.67 (t, *J =* 6.8 Hz, 2H), 3.10 - 2.99 (m, 4H), 2.70 - 2.63 (m, 4H).

### Example 112 Synthesis of Compound A112:

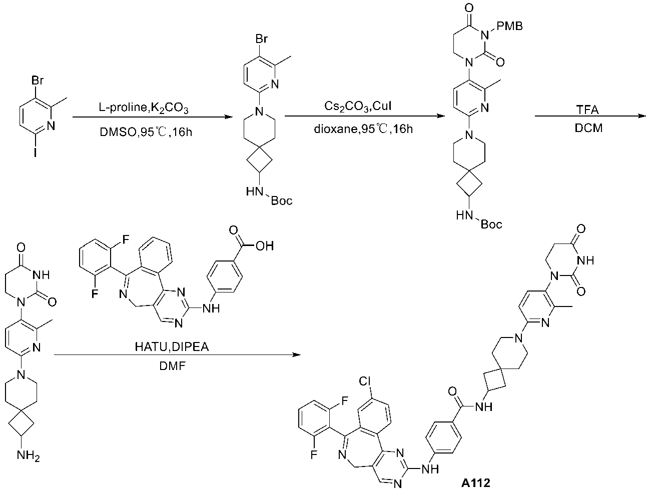

Compound **A112** (6 mg, yield 23%) was synthesized according to the synthesis method of compound A16 described in **Example A16.** LCMS (ESI) m/z = 802.3 [M+H]⁺. ¹H NMR (400 MHz, DMSO-*d*₆) δ 10.34 (s, 1H), 10.21 (s, 1H), 8.76 (s, 1H), 8.49 (d, *J =* 7.6 Hz, 1H), 8.33 (d, *J =* 8.4 Hz, 1H), 7.96 - 7.85 (m, 4H), 7.61 - 7.54 (m, 1H), 7.42 - 7.36 (m, 2H), 7.22 - 7.21 (m, 2H), 6.71 (d, *J =* 8.8 Hz, 1H), 4.51 - 4.45 (m, 1H), 3.71-3.64 (m ,1H), 3.54 - 3.39 (m, 5H), 2.81-2.72 (m, 2H), 2.29 - 2.26 (m, 2H), 2.23 (s, 3H), 1.93 - 1.88 (m, 2H), 1.67 - 1.61 (m, 4H).

### Example 113 Synthesis of Compounds A113 and A114:

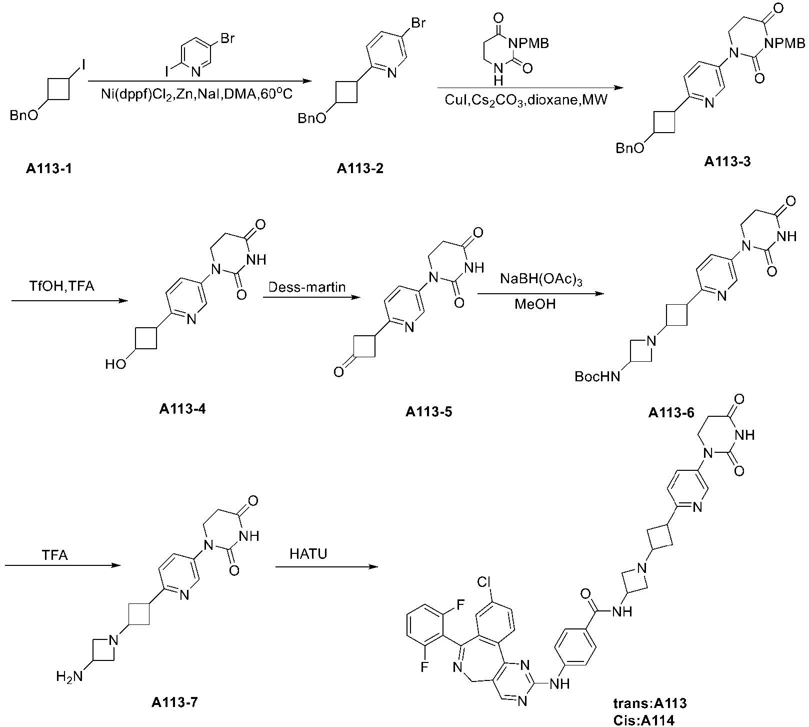

### Step 1:

**A113-1** (3 g, 10.4 mmol, synthesized with reference to WO2019032743A1) and 5-bromo-2-iodopyridine (2.96 g, 10.41 mmol) were dissolved in DMA (80 mL), and zinc powder (676 mg, 10.41 mmol), 1,1'-bis(diphenylphosphino)ferrocene)dichloronickel (711 mg, 1.1 mmol), 4,4'-di-tert-butyl-2,2'-bipyridine (558.09 mg, 2.08 mmol), and sodium iodide (780 mg, 5.21 mmol) were added in sequence under argon protection. The reaction mixture was stirred at 60°C for 2 h. After the reaction was completed, the reaction mixture was directly spin-dried and purified by flash silica gel column chromatography (EA: PE = 10% to 50%) to give **A113-2** (320 mg). LCMS (ESI) m/z = 318.2 [M+H]⁺.

### Step 2:

**A113-2** (700 mg, 2.2 mmol), 3-(4-methoxybenzyl)-dihydropyrimidine-2,4(1H,3H)-dione (772 mg, 3.3 mmol), cesium carbonate (1.08 g, 3.3 mmol), (1R,2R)-N,N-dimethyl-1,2-cyclohexanediamine (156 mg, 1.1 mmol), cuprous iodide (209 mg, 1.1 mmol) and dioxane (10 mL) were added to the microwave reaction vial in sequence, and then the mixture was reacted under microwave conditions at 120°C for 2 h under argon protection. After the reaction was completed, the mixture was directly spin-dried, and purified by flash silica gel column chromatography (EA: PE = 50% to 100%) to give **A113-3** (600 mg). LCMS (ESI) m/z = 471.5 [M+H]⁺.

### Step 3:

**A113-3** (400 mg) was dissolved in trifluoroacetic acid (4 mL), trifluoromethanesulfonic acid (2 mL) was slowly added dropwise, and the mixture was stirred at room temperature for 3 h. Upon completion, the reaction was quenched with ice water. Saturated sodium bicarbonate was added to adjust the pH of the system to 7-8, extraction was performed twice with ethyl acetate, and the aqueous phase was directly purified by reversed-phase medium pressure preparative chromatography to give **A113-4** (150 mg). LCMS (ESI) m/z = 261.2 [M+H]⁺.

### Step 4:

**A113-4** (50 mg, 0.19 mmol) was dissolved in tetrahydrofuran (5 mL), Dess-Martin oxidizer (162 mg, 0.38 mmol) was added, and the mixture was stirred overnight at room temperature. After the reaction was completed, the mixture was directly spin-dried and purified by flash silica gel column chromatography (EA: PE = 50% to 100%) to give **A113-5** (49 mg). LCMS (ESI) m/z = 259.2 [M+H]⁺.

### Step 5:

**A113-6** (49 mg, 0.18 mmol) was dissolved in methanol (2 mL), 3-N-tert-butoxycarbonylaminocyclobutylamine (39 mg, 0.22 mmol), sodium triacetoxyborohydride (120 mg, 0.56 mmol) and 1 drop of acetic acid were added successively, and the mixture was stirred overnight at 50°C. After the reaction was completed, water was added for quenching, ethyl acetate was used for extraction, the organic phase was spin-dried and purified by flash silica gel column chromatography (EA: PE = 50% to 100%) to give a cis product (10 mg) and a trans product (5 mg). LCMS (ESI) m/z = 415.5 [M+H]⁺.

### Step 6:

The product obtained in the previous step was dissolved in dichloromethane (1 mL), trifluoroacetic acid (0.5 mL) was added, and then the mixture was stirred at room temperature for 1 h. After the reaction was completed, the mixture was spin-dried directly for the next step. LCMS (ESI) m/z = 315.2 [M+H]⁺.

### Step 7:

The compound was synthesized according to the synthesis method of compound **A1** described in **Example 1.** Isolation and purification resulted in the trans product **A113** (4 mg, yield 24.6%). LCMS (ESI) m/z = 774.2 [M+H]⁺. ¹H NMR (400 MHz, DMSO-*d*₆) δ 10.52 (s, 1H), 10.23 (s, 1H), 8.76 (s, 1H), 8.55 (d, *J* = 8.4 Hz, 1H), 8.34 (d, *J =* 8.4 Hz, 1H), 7.96 - 7.87 (m, 5H), 7.70 - 7.68 (m, 1H), 7.60 - 7.56 (m, 1H), 7.37 - 7.32 (m, 2H), 7.22 - 7.19 (m, 2H), 3.84 (t, *J =* 6.8 Hz, 2H), 2.74 (t, *J =* 6.8 Hz, 2H), 1.28 - 1.25 (m, 4H).

And the cis product **A114** (2 mg, yield 12.3%). LCMS (ESI) m/z = 774.2 [M+H]⁺. ¹H NMR (400 MHz, DMSO-*d*₆) δ 10.51 (s, 1H), 10.25 (s, 1H), 8.79 (s, 1H), 8.67 (d, *J =* 8.4 Hz, 1H), 8.57 (d, *J =* 8.4 Hz, 1H), 8.36 (d, *J =* 8.8 Hz, 1H), 7.97 - 7.89 (m, 5H), 7.73 - 7.70 (m, 1H), 7.62 - 7.58 (m, 1H), 7.40 - 7.34 (m, 2H), 7.24 - 7.21 (m, 2H), 3.86 (t, *J* = 6.8 Hz, 2H), 3.52 - 3.49 (m, 2H), 3.29 - 3.26 (m, 1H), 3.19 (bs, 1H), 2.78 (t, *J=* 6.8 Hz, 2H ), 2.38 - 2.32 (m, 3H), 2.23 - 2.20 (m, 2H).

### Example 114 Synthesis of Compound A115:

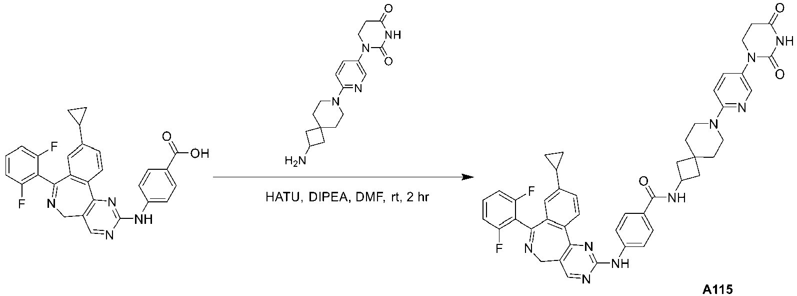

Compound **A115** (10.0 mg, yield 50.6%) was synthesized according to the synthesis method of compound A6 described in **Example 6.** LCMS (ESI) m/z = 793.9 [M+H]⁺. ¹H NMR (400 MHz, DMSO-*d*₆) δ 10.34 (s, 1H), 10.08 (s, 1H), 8.67 (s, 1H), 8.43 (d, *J =* 7.6 Hz, 1H), 8.18 (d, *J =* 8.2 Hz, 1H), 8.04 (d, *J =* 2.8 Hz, 1H), 7.95 - 7.88 (m, 2H), 7.85 - 7.80 (m, 2H), 7.56 - 7.44 (m, 2H), 7.39 - 7.35 (m, 1H), 7.21 - 7.10 (m, 2H), 7.07 - 7.03 (m, 1H), 6.85 (d, *J = 9.1* Hz, 1H), 4.50 - 4.39 (m, 1H), 3.69 (t, *J* = 6.8 Hz, 2H), 3.56 - 3.48 (m, 2H), 3.47 - 3.42 (m, 2H), 2.69 (t, *J =* 6.8 Hz, 2H), 2.28 - 2.19 (m, 2H), 2.04 - 1.96 (m, 1H), 1.92 - 1.85 (m, 2H), 1.66 - 1.62 (m, 2H), 1.60 - 1.56 (m, 2H), 1.03 - 0.97 (m, 2H), 0.78 - 0.60 (m, 2H).

### Example 115 Synthesis of Compound A116:

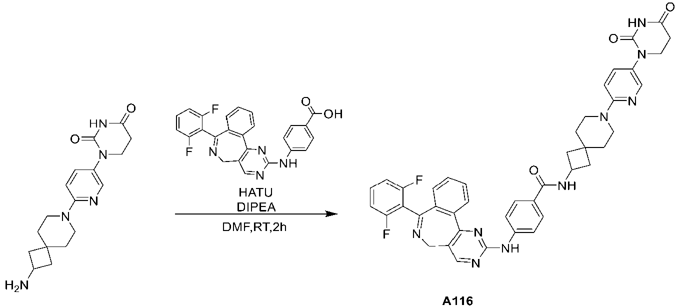

Compound **A116** (9.3 mg, yield 45.6%) was synthesized according to the synthesis method of compound A6 described in **Example 6.** LCMS (ESI) m/z = 754.2 [M+H]⁺. ¹H NMR (400 MHz, DMSO-*d*₆) δ 10.34 (s, 1H), 10.12 (s, 1H), 8.72 (s, 1H), 8.44 (d, *J =* 8.0 Hz, 1H), 8.32 - 8.27 (m, 1H), 8.04 (d, *J =* 2.8 Hz, 1H), 7.93 (d, *J =* 8.8 Hz, 2H), 7.84 (d, *J =* 8.8 Hz, 2H), 7.82 - 7.76 (m, 1H), 7.68 - 7.62 (m, 1H), 7.57 - 7.44 (m, 2H), 7.33 (d, *J =* 7.8 Hz, 1H), 7.15 (s, 2H), 6.88 - 6.82 (m, 1H), 4.51 - 4.40 (m, 1H), 3.69 (t, *J =* 8.0 Hz, 2H), 3.55 - 3.48 (m, 2H), 3.47 - 3.30 (m, 2H), 2.69 (t, *J =* 8.0 Hz, 2H), 2.28 - 2.19 (m, 2H), 1.94 - 1.83 (m, 2H), 1.69 - 1.53 (m, 4H).

### Example 116 Synthesis of Compound A117:

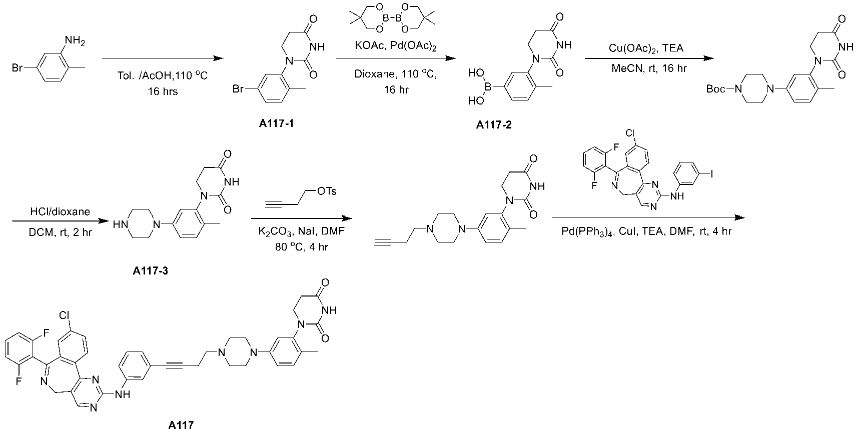

### Step 1:

Compound **A117-1** (1.5 g, 5.30 mmol, synthesized with reference to **Example 8)** and bis(neopentyl glycolato)diboron (7.18 g, 31.79 mmol) were dissolved in dioxane solution (15 mL), and then potassium acetate (1.56 g, 15.89 mmol) and palladium acetate (594.74 mg, 2.65 mmol, 271.57 µL) were added. The reaction mixture was stirred at 110°C for 16 h. After the addition of water (100 mL), the reaction mixture was extracted with ethyl acetate (150 mL*3). The organic phases were pooled, washed with saturated brine (150 mL), dried over anhydrous sodium sulfate, filtered, and the filtrate was concentrated to give a crude product. The crude product was purified by column chromatography to give compound **A117-2** (0.3 g, 22.8% yield). LCMS (ESI) m/z = 249.1 [M+H]⁺.

### Step 2:

Compound **A117-3** (100 mg, hydrochloride) was synthesized according to the synthesis method of compound **A63-5** described in **Example 63.** LCMS (ESI) m/z = 289.4 [M+H]⁺.

### Step 3:

Compound **A117** (5 mg, yield 5.5%) was synthesized according to the synthesis method of compound **A98** described in **Example 98.** LCMS (ESI) m/z = 771.4 [M+H]⁺. ¹H NMR (400 MHz, DMSO-*d*₆) δ 10.28 (s, 1H), 9.98 (s, 1H), 8.69 (s, 1H), 8.27 (d, *J =* 8.4 Hz, 1H), 7.94 - 7.90 (m, 1H), 7.88 - 7.84 (m, 1H), 7.80 (d, *J =* 8.4 Hz, 1H), 7.58 - 7.50 (m, 1H), 7.34 - 7.32 (m, 1H), 7.28 (t, *J =* 8.0 Hz, 1H), 7.20 - 7.11 (m, 2H), 7.08 (d, *J =* 8.4 Hz, 1H), 6.99 (d, *J =* 7.6 Hz, 1H), 6.86 - 6.84 (m, 1H), 6.83 - 6.79 (m, 1H), 3.78 - 3.70 (m, 1H), 3.52 - 3.43 (m, 1H), 3.16 - 3.06 (m, 4H), 2.73 - 2.57 (m, 10H), 2.06 (s, 3H).

### Example 117 Synthesis of Compound A118:

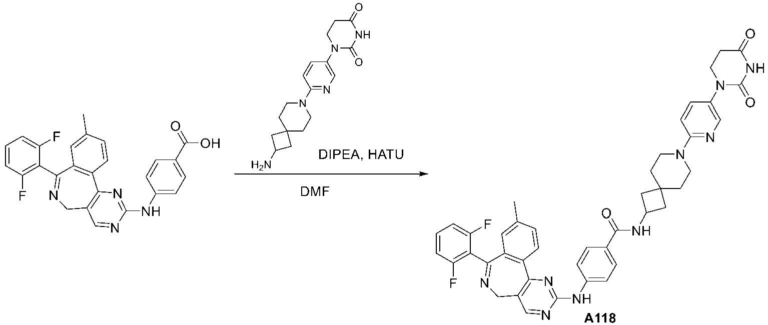

Compound **A118** (16 mg, yield 48%) was synthesized according to the synthesis method of compound **A6** described in **Example 6.** LCMS (ESI) m/z = 768.1 [M+H]⁺. ¹H NMR (400 MHz, DMSO-*d*₆) δ 10.37 (s, 1H), 10.12 (s, 1H), 8.72 (s, 1H), 8.46 (d, *J =* 7.6 Hz, 1H), 8.27 - 8.16 (m, 1H), 8.08 (d, *J =* 2.8 Hz, 1H), 8.01 - 7.91 (m, 2H), 7.91 - 7.81 (m, 2H), 7.69 - 7.61 (m, 1H), 761- 7.42 (m, 2H), 7.27 - 7.05 (m, 2H), 6.97 - 6.79 (m, 1H), 4.58 - 4.36 (m, 1H), 3.73 (t, *J* = 6.4 Hz, 2H), 3.63 - 3.51 (m, 2H), 3.51 - 3.42 (m, 2H), 2.73 (t, *J =* 6.8 Hz, 2H), 2.41 (s, 3H), 2.34 - 2.19 (m, 2H), 2.00 - 1.86 (m, 2H), 1.75 - 1.54 (m, 4H).

### Example 118 Synthesis of Compound A119:

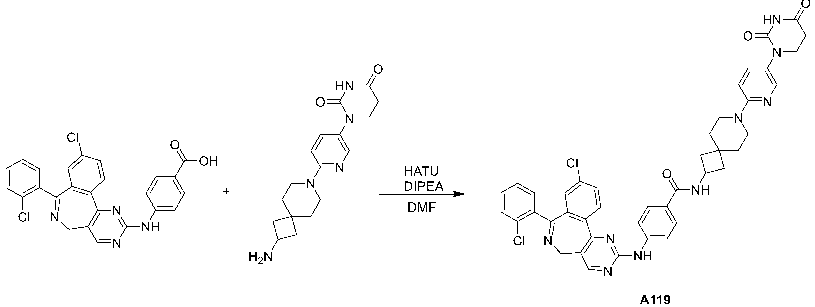

Compound **A119** (2.0 mg, yield 8.0%) was synthesized according to the synthesis method of compound 4 described in **Example 4.** LCMS (ESI) m/z = 786.1 [M+H]⁺. ¹H NMR (400 MHz, DMSO-*d*₆) δ 10.33 (s, 1H), 10.15 (s, 1H), 8.73 (s, 1H), 8.44 (d, *J =* 8.0 Hz, 1H), 8.28 (d, *J =* 8.0 Hz, 1H), 8.03 (d, *J* = 2.7 Hz, 1H), 7.91 - 7.82 (m, 5H), 7.66 - 7.63 (m, 1H), 7.50 - 7.46 (m, 3H), 7.42 - 7.40 (m, 1H), 7.13 (d, *J* = 2.2 Hz, 1H), 6.85 (d, *J* = 12 Hz, 1H), 4.48 - 4.42 (m, 1H), 3.69 (t, *J =* 8 Hz, 2H), 3.53 - 3.50 (m, 2H), 3.46 - 3.42 (m, 2H), 2.71 - 2.68 (m, 3H), 2.26 - 2.22 (m, 2H), 1.91 - 1.86 (m, 2H), 1.66 - 1.58 (m, 4H).

### Example 119 Synthesis of Compound A120:

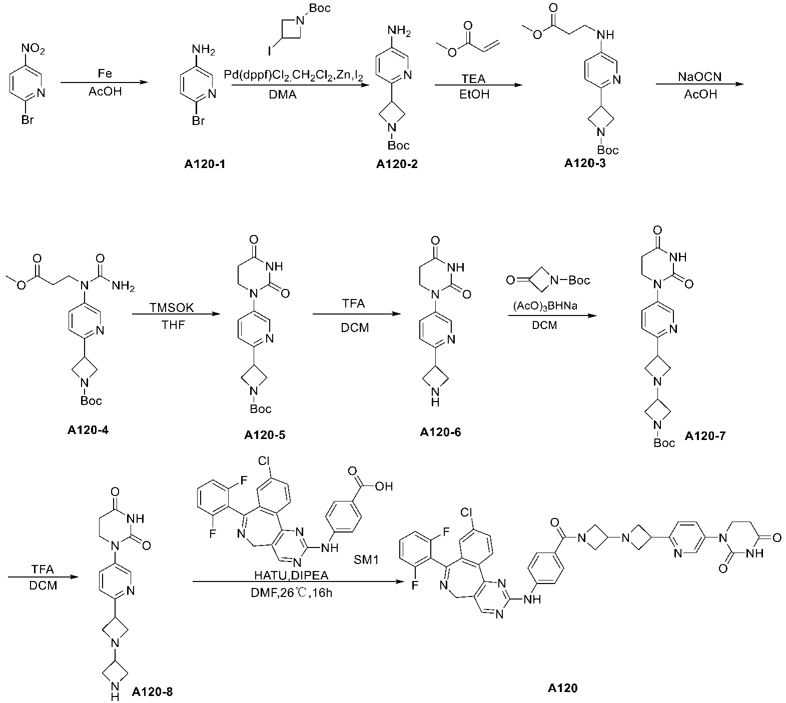

### Step 1:

At room temperature, iron powder (11.17 g, 200 mmol) was added to a solution of compound 2-bromo-5-nitropyridine (8.1 g, 40 mmol) in acetic acid (30 mL), and the reaction mixture was stirred at 90°C for 2 h. The reaction mixture was filtered and concentrated under reduced pressure. After the addition of water, the mixture was extracted with ethyl acetate, and the organic phase was dried over anhydrous sodium sulfate, filtered, and concentrated. The crude product was separated for purification by silica gel column chromatography (dichloromethane/methanol = 20:1) to give a brown solid **A120-1** (6 g, yield 86%). LCMS (ESI) m/z = 173.1 [M+H]⁺.

### Step 2:

At room temperature, compound **A120-1** (4.85 g, 22.04 mmol) and [1,1'-bis(diphenylphosphino)ferrocene]dichloropalladium, complex with dichloromethane (1.8 g, 2.2 mmol) were dissolved in *N,N-*dimethylacetamide solution (50 mL), and the mixture was stirred continuously at 65°C under argon protection. In another conical flask, 1-Boc-3-iodoazetidine (18.72 g, 66.13 mmol), elemental iodine (560 mg, 2.2 µmol) and zinc powder (4.3 g, 66.13 µmol) were dissolved in *N,N*-dimethylacetamide solution (50 mL). The reaction mixture was stirred at room temperature for 2 h, and then added to another reaction system. The temperature was raised to 85°C, and the mixture was stirred continuously overnight. After the addition of water, the reaction mixture was extracted with ethyl acetate, dried over anhydrous sodium sulfate, and concentrated under reduced pressure. The residue was separated for purification by silica gel column chromatography (methanol/dichloromethane = 1:20) to give a yellow oil **A120-2** (3 g, yield 54%). LCMS (ESI) m/z = 250.3 [M+H]⁺.

### Step 3:

At room temperature, methyl acrylate (932 mg, 10.83 mmol) and triethylamine (731 mg, 7.22 mmol) were added to a solution of compound **A120-2** (900 mg, 3.61 mmol) in ethanol (20 mL), and the reaction mixture was stirred at 100°C for 16 h. The mixture was concentrated under reduced pressure. The crude product was separated for purification by silica gel column chromatography (petroleum ether/ethyl acetate = 5:1) to give a brown solid **A120-3** (1 g, yield 82%). LCMS (ESI) m/z = 336.2 [M+H]⁺.

### Step 4:

Compound **A120-3** (1.0 g, 2.86 mmol) was dissolved in a mixed solution of acetic acid (20 mL) and water (0.4 mL), sodium cyanate (930 mg, 14.31 mmol) was added, and the reaction mixture was stirred at room temperature for 1 h. The reaction mixture was concentrated to give compound **A120-4** (1.0 g, yellow solid, crude product). LCMS (ESI) m/z = 379.3 [M+H]⁺.

### Step 5:

Compound **A120-4** (400 mg, 1.02 mmol) was dissolved in tetrahydrofuran (10 mL), potassium trimethylsilanolate was added to adjust the pH to about 9, and the reaction mixture was stirred at room temperature for 1 h. The reaction mixture was adjusted to weak acidity with acetic acid, and concentrated to give a crude product. The crude product was purified by column chromatography to give compound **A120-5** (250.0 mg, yellow solid, crude product). LCMS (ESI) m/z = 347.2 [M+H]⁺

### Step 6:

Compound **A120-5** (300.0 mg, 866.10 µmol) was dissolved in dichloromethane (3 mL), trifluoroacetic acid (1 mL) was added, and the reaction mixture was stirred at 25°C for 2 h. The reaction mixture was concentrated to remove the solvent to give **A120-6** (250.0 mg, yellow solid, crude product, trifluoroacetate). LCMS (ESI) m/z = 247.2 [M+H]⁺.

### Step 7:

Compound **A120-6** (213 mg, 864.92 µmol) was dissolved in dichloromethane, 1-(tert-butoxycarbonyl)-3-azetidinone (296 mg, 1.73 mmol) and acetic acid (2.52 mg, 42.20 µmol) were added sequentially, and the mixture was stirred at 26°C for 1 h, then sodium borohydride acetate (367 mg, 1.73 µmol) was added. After the reaction was completed, the mixture was directly spin-dried and purified by flash silica gel column chromatography (EA:MeOH = 10% to 10%) to give **A120-7** (200 mg, yield 57%). LCMS (ESI) m/z = 402.4 [M+H]⁺.

### Step 8:

Compound **A120-7** (40 mg, 99.64 µmol) was dissolved in dichloromethane (3 mL), trifluoroacetic acid (0.5 mL) was added, and the reaction mixture was stirred at 25°C for 2 h. The reaction mixture was concentrated to remove the solvent to give **A120-8** (30.0 mg, yellow solid, crude product, trifluoroacetate). LCMS (ESI) m/z = 302.3 [M+H]⁺.

### Step 9:

To a solution of compound **A120-8** (15 mg, 31.46 µmol) in N,N-dimethylformamide (5 mL), **SM1** (14.22 mg, 47.18 µmol), HATU (23.9 mg, 62.91 µmol) and DIPEA (4.0 mg, 31.46 µmol) were added at room temperature. The reaction mixture was stirred at 26°C for 16 h. After the addition of water, the reaction mixture was extracted with ethyl acetate, dried over anhydrous sodium sulfate, and concentrated under reduced pressure. The residue was separated for purification by silica gel column chromatography (methanol/dichloromethane = 1:20) to give a yellow oil **A120** (4 mg, yield 16%). LCMS (ESI) m/z = 760.3 [M+H]⁺. ¹H NMR (400 MHz, DMSO-*d*₆) δ 10.51 (s, 1H), 10.23 (s, 1H), 8.76 (s, 1H), 8.56 (d, *J =* 2.4 Hz, 1H), 8.33 (d, *J =* 8.4 Hz, 1H), 7.95 - 7.90 (m, 3H), 7.73 (dd, *J*₁ = 8.4 Hz, *J*₂ = 2.6 Hz, 1H), 7.62 - 7.54 (m, 2H), 7.60 - 7.54 (m, 1H), 7.44 (d, *J =* 8.4 Hz, 1H), 7.37 (d, *J =* 2.4 Hz, 1H), 7.22 - 7.18 (m, 2H), 4.42 - 4.40 (m, 1H), 4.12 - 4.08 (m, 2H), 3.86 - 3.78 (m, 4H), 3.72 - 3.67 (m, 2H), 3.60 - 3.54 (m, 1H), 3.40 - 3.35 (m, 4H), 2.77 - 2.74 (m, 2H).

### Example 120 Synthesis of Compound A121:

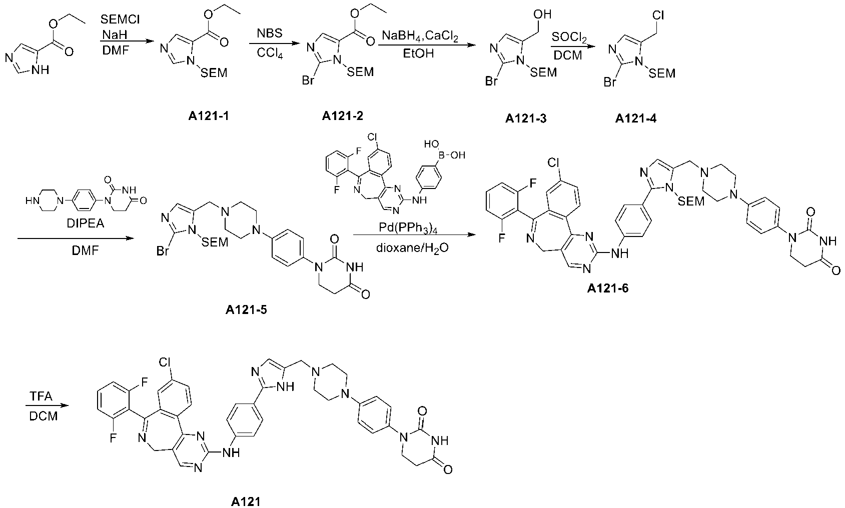

### Step 1:

Sodium hydride (314 mg, 7.85 mmol) was added to a solution of compound ethyl imidazole-4-carboxylate (1.0 g, 7.14 mmol) in N,N-dimethylformamide (5 mL) at 0°C. The reaction mixture was stirred for 0.5 h. And 2-(trimethylsilyl)ethoxymethyl chloride (1.42 g, 8.52 mmol) was added. The reaction mixture was stirred at room temperature for 3 h. The reaction was quenched with saturated ammonium chloride, the reaction mixture was extracted with ethyl acetate, concentrated, separated for purification by silica gel column (petroleum ether/ethyl acetate = 10:1) to give a white solid **A121-1** (1.1 g, yield 57.01%). LCMS (ESI) m/z = 271.21 [M+H]⁺.

### Step 2:

To a solution of compound **A121-1** (1.0 g, 3.70 mmol) in carbon tetrachloride (5 mL), N-bromosuccinimide (658 mg, 3.70 mmol) and azobisisobutyronitrile (30.36 mg, 0.18 mmol) were added at room temperature. The reaction mixture was stirred at 60°C for 3 h. The reaction mixture was filtered, the filtrate was concentrated, and separated for purification by a silica gel column (petroleum ether/ethyl acetate = 10:1) to give a white solid **A121-2** (1.02 mg, yield 78.95%). LCMS (ESI) m/z = 348.9 [M+H]⁺.

### Step 3:

Sodium borohydride (650 mg, 17.18 mmol) and calcium chloride (1.91 g, 17.8 mmol) were added to a solution of compound **A121-2** (1.0 g, 2.86 mmol) in ethanol (20 mL) at room temperature. The reaction mixture was stirred at 25°C for 12 h. The reaction mixture was filtered, the filtrate was concentrated, and separated for purification by a silica gel column (petroleum ether/ethyl acetate = 1:1) to give a white solid **A121-3** (721 mg, yield 81.96%). LCMS (ESI) m/z = 307.0 [M+H]⁺.

### Step 4:

Thionyl chloride (2 mL) was added to a solution of compound **A121-3** (300 mg, 0.98 mmol) in dichloromethane (5 mL) at room temperature. The reaction mixture was stirred at 25°C for 3 h. The reaction mixture was concentrated, and separated for purification by a silica gel column (petroleum ether/ethyl acetate = 5:1) to give a white solid **A121-4** (120 mg, yield 37.73%). LCMS (ESI) m/z = 325.2 [M+H]⁺.

### Step 5:

Compound **A121-5** (42 mg, yield 97.69%) was synthesized according to the synthesis method of compound **A5-2** described in **Example A5.** LCMS (ESI) m/z = 563.1 [M+H]⁺.

### Step 6:

Compound **A121-5** (142.38 mg, 251.75 µmol), **SM6** (100 mg, 0.21 mmol), cesium carbonate (205.06 mg, 0.63 mmol) and tetrakis(triphenylphosphine)palladium (48.46 mg, 0.04 mmol) were dissolved in dioxane (5 mL) and water (0.2 mL) at room temperature. The reaction mixture was stirred at 90°C for 8 h. The reaction mixture was filtered, the filtrate was concentrated to remove the solvent, and the residue was separated for purification by silica gel column chromatography (dichloromethane/methanol = 30:1) to give a yellow solid **A121-6** (53 mg, 27.53%). LCMS (ESI) m/z = 915.2 [M+H]⁺.

### Step 7:

**A121-6** (30 mg, 0.03 mmol) obtained in the previous step was dissolved in dichloromethane (4 mL), and trifluoroacetic acid (2 mL) was added. After stirring at room temperature for 12 h, the reaction mixture was separated for purification by reverse phase chromatography (acetonitrile/0.1% aqueous formic acid solution = 2.3/1) to give a yellow solid **A121** (12.2 mg, yield 21%). LCMS (ESI) m/z = 785.0 [M+H]⁺. ¹H NMR (400 MHz, DMSO-*d*₆) δ 12.2 (s, 1H), 10.30 (s, 1H), 10.07 (s, 1H), 8.73 (s, 1H), 8.34 (d, *J =* 8.4 Hz, 1H), 7.98 - 7.85 (m, 5H), 7.64 - 7.52 (m, 1H), 7.36 (d, *J =* 2.0 Hz, 1H), 7.26 - 7.05 (m, 5H), 6.96 (d, *J* = 8.9 Hz, 3H), 3.72 (t, *J =* 6.8 Hz, 2H), 3.62 - 3.44 (m, 2H), 3.23 - 3.08 (m, 4H), 2.70 (t, *J =* 6.8 Hz, 2H), 2.67 - 2.56 (m, 4H).

### Example 121 Synthesis of Compound A122:

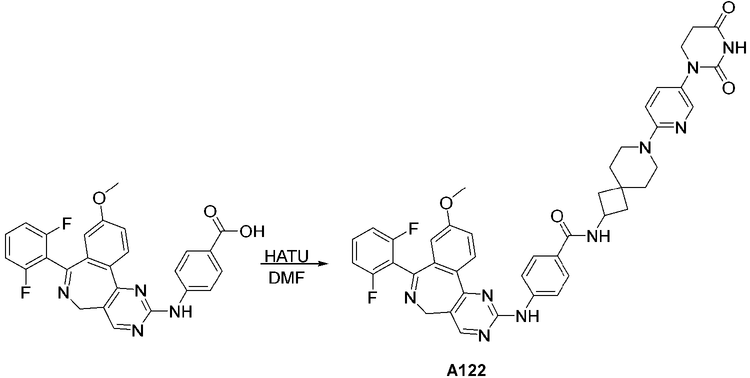

Compound **A122** (8.25 mg, yield 49.73%) was synthesized according to the synthesis method of compound **A54** described in **Example 54.** LCMS (ESI) m/z = 784.2 [M+H]⁺. ¹H NMR (400 MHz, DMSO-*d*₆) δ 10.37 (s, 1H), 10.10 (s, 1H), 8.69 (s, 1H), 8.47 (d, *J =* 7.6 Hz, 1H), 8.30 (d, *J=* 8.8 Hz, 1H), 8.09 - 8.04 (m, 1H), 7.99 - 7.80 (m, 4H), 7.62 - 7.39 (m, 3H), 7.18 (s, 2H), 6.88 (d, *J=* 8.8 Hz, 1H), 6.74 (s, 1H), 4.57 - 4.43 (m, 1H), 3.81 (s, 3H), 3.72 (t, *J =* 6.8 Hz, 2H), 3.60 - 3.45 (m, 4H), 2.72 (t, *J =* 6.8 Hz, 2H), 2.32 - 2.19 (m, 2H), 1.99 - 1.82 (m, 2H), 1.71 - 1.52 (m, 4H).

### Example 122 Synthesis of Compound A123:

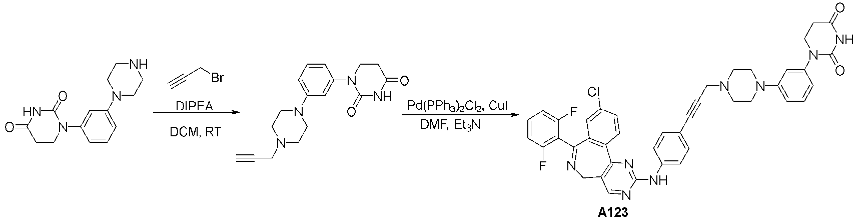

Compound **A123** (2.0 mg, yield 4.2%) was synthesized according to the synthesis method of compound **A98** described in **Example 98.** LCMS (ESI) m/z = 743.4 [M+H]. ¹H NMR (400 MHz, DMSO-*d*₆) δ 10.35 (s, 1H), 10.14 (s, 1H), 8.74 (s, 1H), 8.31 (d, *J=* 8.4 Hz, 1H), 7.88 - 7.83 (m, 3H), 7.61 - 7.54 (m, 1H), 7.43 *(d, J=* 8.4 Hz, 2H), 7.36 *(d, J=* 2.2 Hz, 1H), 7.28 - 7.15 (m, 3H), 6.93 (s, 1H), 6.87 (d, *J =* 8.4 Hz, 1H), 6.76 (d, *J= 7.8* Hz, 1H), 3.78 (t, *J =* 6.6 Hz, 2H), 3.61 (s, 2H), 3.22 (s, 4H), 2.72 - 2.70 (m, 6H).

### Example 124 Synthesis of Compound A124:

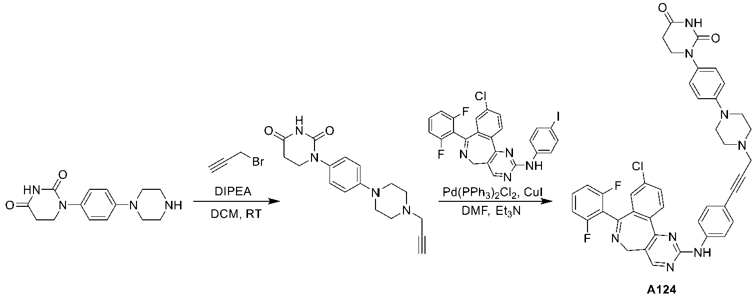

Compound **A124** (4.0 mg, yield 8.4%) was synthesized according to the synthesis method of compound **A98** described in **Example 98.** LCMS (ESI) m/z = 743.4 [M+H]. ¹H NMR (400 MHz, DMSO-*d*₆) δ 10.29 (s, 1H), 10.12 (s, 1H), 8.70 (s, 1H), 8.28 (d, *J =* 8.0 Hz, 1H), 7.91 - 7.86 (m, 3H), 7.68 - 7.61 (m, 1H), 7.40 (d, *J =* 8.0 Hz, 2H), 7.33 (d, *J =* 2.2 Hz, 1H), 7.19 - 7.14 (m, 4H), 6.95 (d, *J =* 8.0 Hz, 2H), 3.70 (t, *J =* 6.6 Hz, 2H), 3.57 (s, 2H), 3.18 (bs, 4H), 3.19 - 3.16 (m, 6H).

### Example 124 Synthesis of Compound A125:

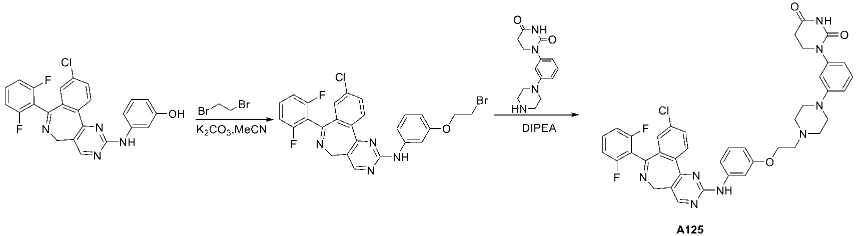

Compound **A125** (7 mg, yield 14.2%) was synthesized according to the synthesis method of compound **A101** described in **Example 101.** LCMS (ESI) m/z = 749.2 [M+H]⁺. ¹H NMR (400 MHz, DMSO-*d*₆) δ 10.31 (s, 1H), 9.92 (s, 1H), 8.71 (s, 1H), 8.33 (d, *J* = 8.4 Hz, 1H), 7.90 (dd, *J*₁ = 8.4 Hz, *J*₂ = 2.0 Hz, 1H), 7.71 (s, 1H), 7.60 - 7.52 (m, 1H), 7.36 - 7.33 (m, 2H), 7.24 - 7.18 (m, 4H), 6.89 (bs, 1H), 6.83 - 6.80 (m, 1H ), 6.75 - 6.73 (m, 1H), 6.60 (dd, *J*₁ = 8.0 Hz, *J*₂ = 2.0 Hz, 1H), 4.17 (bs, 2H), 3.75 *(t, J=* 6.8 Hz, 2H), 3.17 - 3.15 (m, 4H), 2.79 (t, *J =* 6.8 Hz, 2H), 2.70 (t, *J* = 6.8 Hz, 2H ), 2.66 - 2.64 (m, 4H).

### Example 125 Synthesis of Compound A126:

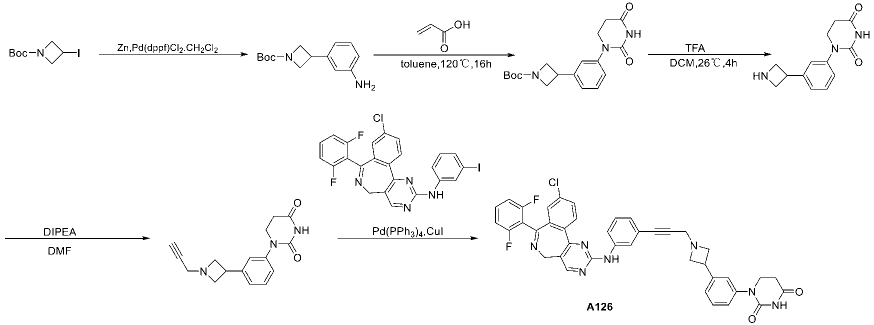

Compound **A126** (20.0 mg, yield 18%) was synthesized according to the synthesis method of compound **A98** described in **Example 98.** LCMS (ESI) m/z = 714.3 [M+H]⁺. LCMS (ESI) m/z = 819.4 [M+H]⁺. ¹H NMR (400 MHz, DMSO-*d*₆) δ 10.38 (s, 1H), 10.04 (s, 1H), 8.72 (s, 1H), 8.28 (d, *J =* 8.4 Hz, 1H), 8.03 - 8.01 (m, 1H), 7.88 - 7.82 (m, 2H), 7.61 - 7.53 (m, 1H), 7.38 - 7.26 (m, 5H), 7.19 - 7.16 (m, 3H), 7.11 - 7.07 (m, 1H), 3.74 - 3.70 (m, 4H), 3.68 - 3.63 (m, 1H), 3.59 (s, 2H), 3.42 - 3.38 (m, 2H), 2.65 (t, *J* = 6.8 Hz, 2H).

### Example 126 Synthesis of Compound A127:

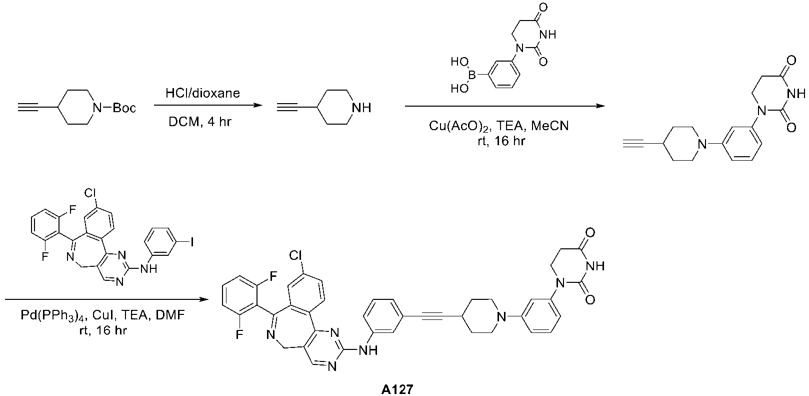

Compound **A127** (20 mg, yield 6.8%) was synthesized according to the synthesis method of compound **A98** described in **Example 98.** LCMS (ESI) m/z =728.3 [M+H]⁺. ¹H NMR (400 MHz, DMSO-*d*₆) δ 10.30 (s, 1H), 9.95 (s, 1H), 8.69 (s, 1H), 8.27 (d, *J =* 8.4 Hz, 1H), 7.91 - 7.88 (m, 1H), 7.86 - 7.79 (m, 2H), 7.58 - 7.50 (m, 1H), 7.34 - 7.31 (m, 1H), 7.30 - 7.25 (m, 1H), 7.22 - 7.13 (m, 3H), 7.00 (d, *J* = 7.2 Hz, 1H), 6.92 - 6.89 (m, 1H), 6.86 - 6.81 (m, 1H), 6.73 - 6.69 (m, 1H), 3.75 (t, *J =* 6.8 Hz, 2H), 3.54 - 3.46 (m, 2H), 3.06 - 2.99 (m, 2H), 2.91-2.84 (m, 1H), 2.68 (t, *J =* 6.8 Hz, 2H), 2.02 - 1.94 (m, 2H), 1.78 - 1.68 (m, 2H).

### Example 127 Synthesis of Compound A128:

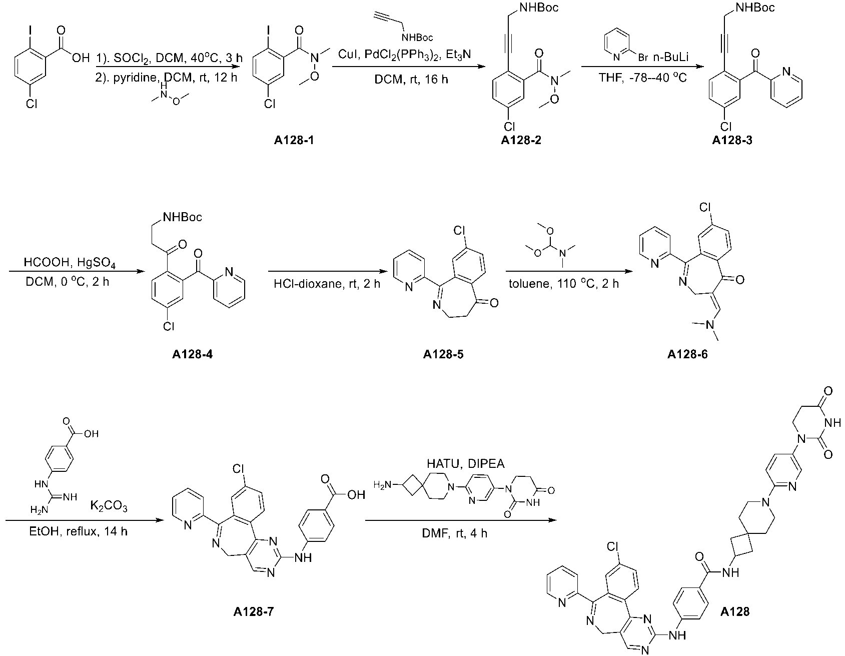

### Step 1:

At room temperature, N,N-dimethylformamide (1 mL) and sulfoxide chloride (4.21 g, 35.40 mmol) were added to a solution of compound 5-chloro-2-iodobenzoic acid (5 g, 17.70 mmol) in dichloromethane (50 mL). The reaction mixture was stirred at 40°C for 3 h under argon protection. The reaction mixture was concentrated, co-evaporated with toluene twice, the residue was dissolved in dichloromethane (20 mL), methoxymethylamine (1.9 g, 19.47 mmol) and pyridine (5.6 g, 70.81 mol, 5.70 mL) were added, and the reaction mixture was stirred at room temperature under argon for 12 h. After the addition of water, the reaction mixture was extracted with dichloromethane, washed with saturated brine, dried over anhydrous sodium sulfate, concentrated under reduced pressure, and the residue was separated for purification by silica gel column chromatography to give a white solid **A128-1** (5.23 g, yield 91%). LCMS (ESI) m/z = 326.1 [M+H]⁺.

### Step 2:

To a solution of compound **A128-1** (5.23 g, 16.07 mmol) in dichloromethane (100 mL), N-Boc-propargylamine (2.74 g, 17.67 mmol), cuprous iodide (306 mg, 1.61 mmol) and bis(triphenylphosphine)palladium dichloride (1.13 g, 1.61 mmol) were added at room temperature. Triethylamine (7.72 g, 76.31 mmol) was slowly added dropwise to the reaction mixture at 0°C under argon protection. After the addition, the mixture was warmed to room temperature and stirred for 16 h. The reaction mixture was filtered, the filtrate was concentrated, and the residue was separated for purification by silica gel column chromatography to give an orange solid **A128-2** (2.88 g, yield 51%). LCMS (ESI) m/z = 353.2 [M+H]⁺.

### Step 3:

Under argon protection at -78°C, n-butyl lithium (1.57 g, 24.49 mmol, 9.8 mL, 2.5 M in n-hexane) was slowly added dropwise to a solution of compound 2-bromopyridine (4.51 g, 28.57 mmol) in tetrahydrofuran (65 mL), the reaction mixture was kept at -78°C and stirred for 0.5 h, then **A128-2** (2.88 g, 8.16 mmol) was added. The reaction mixture was warmed to -40°C and stirring was continued for 1 h. The reaction mixture was quenched with a saturated ammonium chloride solution, extracted with dichloromethane, washed with saturated brine, dried over anhydrous sodium sulfate, concentrated under reduced pressure, and the residue was separated for purification by silica gel column chromatography to give a yellow solid **A128-3** (1.26 g, yield 42%). LCMS (ESI) m/z = 371.3 [M+H]⁺.

### Step 4:

To a solution of compound **A128-3** (1.2 g, 3.24 mmol) in dichloromethane (12 mL) under argon protection at 0°C, formic acid (0.4 mL) and mercury sulfate (288 mg, 0.97 mmol) were added. The reaction mixture was stirred at 0°C for 2 h under argon protection. After the addition of saturated sodium bicarbonate, the reaction mixture was extracted with ethyl acetate, washed with saturated brine, dried over anhydrous sodium sulfate, and concentrated under reduced pressure. The residue was separated for purification by silica gel column chromatography to give a brown oily liquid **A128-4** (168 mg, yield 13%). LCMS (ESI) m/z = 389.4 [M+H]⁺.

### Step 5:

Compound **A128-4** (168 mg, 0.43 mmol) was dissolved in HCl (4 M in dioxane, 3 mL) at room temperature. The reaction mixture was stirred at room temperature for 2 h. The reaction mixture was concentrated under reduced pressure, and the residue was separated for purification by silica gel column chromatography to give a brown oily liquid **A128-5** (100 mg, yield 86%). LCMS (ESI) m/z = 271.2 [M+H]⁺.

### Step 6:

N,N-dimethylformamide dimethyl acetal (1 mL) was added to a solution of compound **A128-5** (100 mg, 0.37 mmol) in toluene (2 mL) at room temperature. The reaction mixture was stirred at 110°C for 2 h. The reaction mixture was concentrated under reduced pressure to give a brown solid **A128-6** (80 mg, yield 66%). LCMS (ESI) m/z = 326.3 [M+H]⁺.

### Step 7:

To a solution of compound **A128-6** (47 mg, 0.14 mmol) in ethanol (2 mL), p-guanidinobenzoic acid (62 mg, 0.29 mmol) and potassium carbonate (50 mg, 0.36 mmol) were added at room temperature. The reaction mixture was refluxed and stirred at 80°C for 14 h. The reaction mixture was concentrated under reduced pressure, and the residue was separated for purification by silica gel column chromatography to give a white solid **A128-7** (37 mg, yield 58%). LCMS (ESI) m/z = 442.3 [M+H]⁺.

### Step 8:

**A54-3** (39 mg, 0.12 mmol), HATU (34 mg, 0.09 mmol) and DIPEA (23 mg, 0.18 mmol) were added to a solution of compound **A128-7** (26 mg, 0.06 mmol) in N,N-dimethylformamide (2 mL) at room temperature. The reaction mixture was stirred at room temperature for 4 h. The reaction mixture was separated for purification by reverse phase chromatography (acetonitrile/10 mM ammonium bicarbonate solution) to give a white solid **A128** (2 mg, yield 5%). LCMS (ESI) m/z = 753.4 [M+H]⁺. ¹H NMR (400 MHz, DMSO-*d*₆) δ 10.37 (s, 1H), 10.13 (s, 1H), 8.74 (s, 1H), 8.63 - 8.39 (m, 2H), 8.25 (d, *J =* 8.4 Hz, 1H), 8.20 - 8.02 (m, 2H), 8.02 - 7.80 (m, 6H), 7.58 - 7.39 (m, 3H), 6.88 (d, *J =* 9.2 Hz, 1H), 5.03 - 4.82 (m, 1H), 4.63 - 4.35 (m, 1H), 3.99 - 3.82 (m, 1H), 3.78 - 3.68 (m, 2H), 3.58 - 3.52 (m, 2H), 2.78 - 2.66 (m, 2H), 2.33 - 2.17 (m, 2H), 2.01 - 1.81 (m, 2H), 1.73 - 1.54 (m, 4H).

### Example 128 Synthesis of Compound A129:

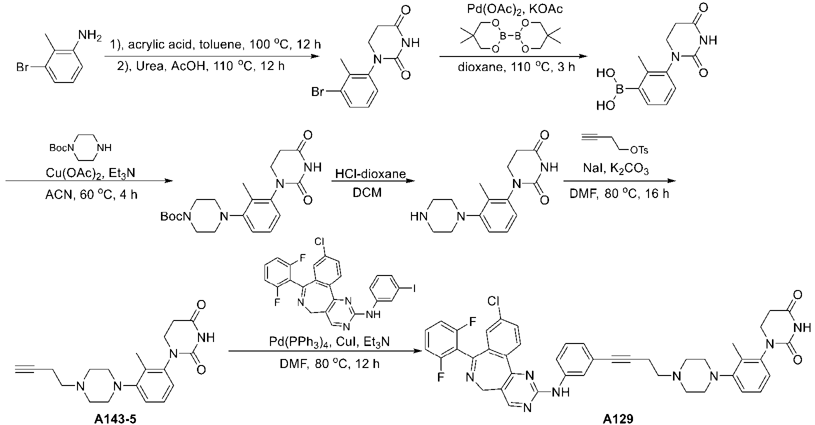

Compound A129 (2 mg, yield 4%) was synthesized according to the synthesis method of compound **A98** described in **Example 98.** LCMS (ESI) m/z = 771.3 [M+H]⁺. ¹H NMR (400 MHz, DMSO-*d*₆) δ 10.35 (s, 1H), 10.01 (s, 1H), 8.72 (s, 1H), 8.30 (d, *J =* 8.4 Hz, 1H), 7.97 (s, 1H), 7.94 - 7.86 (m, 1H), 7.86 - 7.77 (m, 1H), 7.65 - 7.50 (m, 1H), 7.41 - 7.27 (m, 2H), 7.27 - 7.12 (m, 3H), 7.08 - 6.93 (m, 3H), 3.85 - 3.66 (m, 1H), 3.62 - 3.45 (m, 1H), 2.97 - 2.83 (m, 4H), 2.81 - 2.59 (m, 10H), 2.13 (s, 3H).

### Example 129 Synthesis of Compound A130:

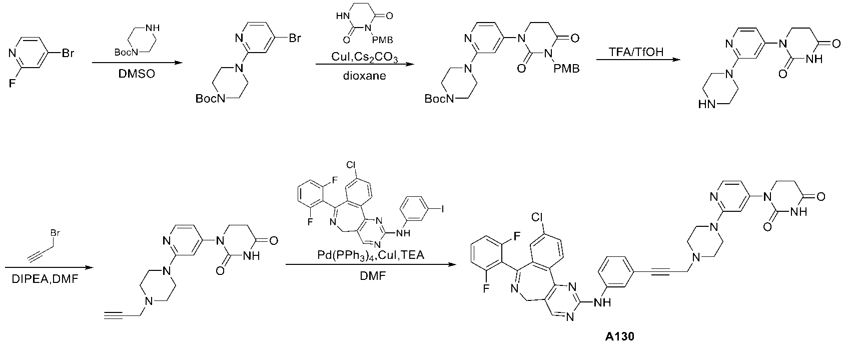

Compound **A130** (39.2 mg, yield 32.7%) was synthesized according to the synthesis method of compound **A98** described in **Example 98.** LCMS (ESI) m/z = 744.4 [M+H]⁺. ¹H NMR (400 MHz, DMSO-*d*₆) δ 10.48 (s, 1H), 9.99 (s, 1H), 8.69 (s, 1H), 8.26 (d, *J =* 8.8 Hz, 1H), 8.04 (d, *J =* 4.0 Hz, 1H), 7.96 - 7.93 (m, 1H), 7.89 - 7.80 (m, 2H), 7.58 - 7.50 (m, 1H), 7.35 - 7.28 (m, 2H), 7.20 - 7.10 (m, 2H), 7.05 - 7.01 (m, 1H), 6.76 - 6.73 (m, 1H), 6.70 - 6.66 (m, 1H), 3.85 - 3.77 (m, 2H), 3.60 (s, 2H), 3.56 - 3.47 (m, 4H), 2.69 - 2.61 (m, 6H).

### Example 130 Synthesis of Compound A131:

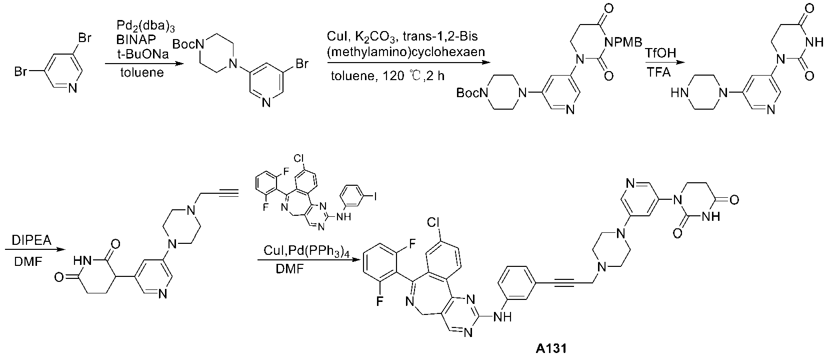

Compound **A131** (24.6 mg, yield 36.94%) was synthesized according to the synthesis method of compound **A98** described in **Example 98.** LCMS (ESI) m/z = 744.3 [M+H]⁺. ¹H NMR (400 MHz, DMSO-*d*₆) δ 10.44 (s, 1H), 9.99 (s, 1H), 8.70 (s, 1H), 8.27 (d, *J =* 8.4 Hz, 1H), 8.16 (s, 1H), 8.06 - 7.93 (m, 2H), 7.90 - 7.79 (m, 2H), 7.59 - 7.47 (m, 1H), 7.35 - 7.27 (m, 3H), 7.19 - 7.10 (m, 2H), 7.08 - 7.01 (m, 1H), 3.78 (t, *J =* 6.8 Hz, 2H), 3.62 (s, 2H), 3.28 - 3.24 (m, 4H), 2.74 - 2.66 (m, 6H).

### Example 131 Synthesis of Compound A132:

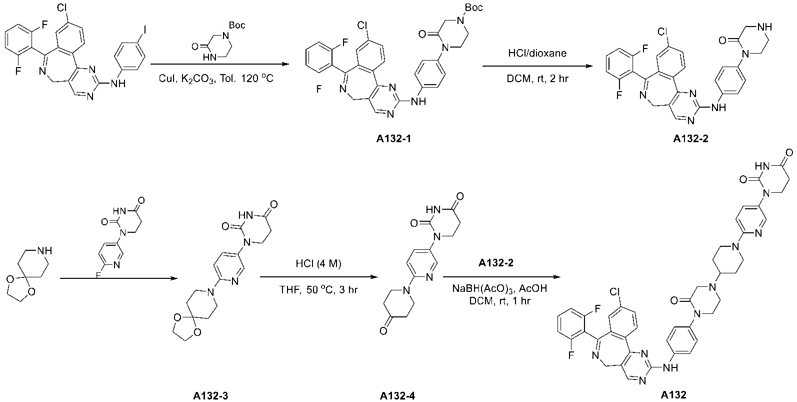

### Step 1:

Potassium carbonate (142.23 mg, 1.03 mmol), cuprous iodide (31.36 mg, 164.65 µmol, 5.58 µL) and trans-(1R,2R)-N,N'-dimethyl-1,2-cyclohexanediamine (58.55 mg, 411.63 µmol) were added to a solution of **SM5** (230 mg, 411.63 µmol) and tert-butyl 3-oxo-1-piperazinecarboxylate (164.85 mg, 823.27 µmol) in toluene (3 mL) at room temperature. The reaction mixture was heated to 110°C and stirred for 16 h in an argon atmosphere. After LCMS showed that the reaction was completed, the reaction mixture was quenched with a saturated aqueous ammonium chloride solution, extracted with ethyl acetate, and the organic phase was dried over anhydrous sodium sulfate, filtered, and concentrated. The crude product was separated for purification by silica gel column to give a yellow solid **A132-1** (200 mg, yield 76.9%). LCMS (ESI) m/z = 631.2 [M+H]⁺.

### Step 2:

Hydrogen chloride (4.0 M in dioxane, 1 mL) was added to a solution of **A132-1** (200 mg, 316.92 µmol) in dichloromethane (3 mL) at room temperature. The reaction mixture was stirred at room temperature for 2 h. After LCMS showed that the reaction was complete, the reaction mixture was concentrated to give a yellow solid **A132-2** (200 mg, crude). LCMS (ESI) m/z = 531.3 [M+H]⁺.

### Step 3:

Compound **A132-3** (300 mg, 94.4% yield) was synthesized according to the synthesis method of compound **A68-1** described in **Example 68.** LCMS (ESI) m/z = 333.2 [M+H]⁺.

### Step 4:

Aqueous hydrochloric acid solution (4 M, 752.21 µL) was added to a solution of **A132-3** (200 mg, 601.77 µmol) in tetrahydrofuran (2 mL) at room temperature. The reaction mixture was heated to 50°C and stirred for 3 h. After LCMS showed that the reaction was complete, the reaction mixture was quenched with a saturated aqueous sodium bicarbonate solution, extracted with ethyl acetate, and the organic phase was dried over anhydrous sodium sulfate, filtered, and concentrated. The crude product was separated for purification by silica gel column to give an off-white solid **A132-4** (90 mg, yield 51.9%). LCMS (ESI) m/z = 289.3 [M+H]⁺.

### Step 5:

N,N-diisopropylethylamine (51.12 mg, 395.51 µmol, 68.89 µL), **A132-4** (171.04 mg, 593.27 µmol), acetic acid (47.46 mg, 791.03 µmol) and sodium borohydride acetate (167.65 mg, 791.03 µmol) were added to a solution of **A132-2** (210 mg, 395.51 µmol) in dichloromethane (4 mL) at room temperature. The reaction mixture was stirred at room temperature for 1 h. After LCMS showed that the reaction was complete, the reaction mixture was quenched with a saturated aqueous sodium bicarbonate solution, extracted with dichloromethane, and the organic phase was dried over anhydrous sodium sulfate, filtered, and concentrated. The crude product was separated for purification by high performance liquid chromatography to give a yellow solid **A132** (32 mg, yield 10.0%). LCMS (ESI) m/z = 803.4 [M+H]⁺. ¹H NMR (400 MHz, DMSO-*d*₆) δ 10.35 (s, 1H), 9.95 (s, 1H), 8.67 (s, 1H), 8.28 (d, *J =* 8.4 Hz, 1H), 8.06 (d, *J =* 2.7 Hz, 1H), 7.87 - 7.83 (m, 1H), 7.82 - 7.78 (m, 2H), 7.59 - 7.52 (m, 1H), 7.51 - 7.46 (m, 1H), 7.32 (d, *J =* 2.1 Hz, 1H), 7.28 - 7.24 (m, 2H), 7.19 - 7.12 (m, 2H), 6.88 (d, *J =* 9.2 Hz, 1H), 4.35 - 4.27 (m, 2H), 3.70 (t, *J =* 6.8 Hz, 2H), 3.63 - 3.57 (m, 2H), 3.31 - 3.29 (m, 2H), 2.90 - 2.81 (m, 4H), 2.70 (t, *J =* 6.8 Hz, 2H), 2.62 - 2.54 (m, 1H), 1.95 - 1.86 (m, 2H), 1.48 - 1.35 (m, 2H).

### Example 132 Synthesis of Compound A133:

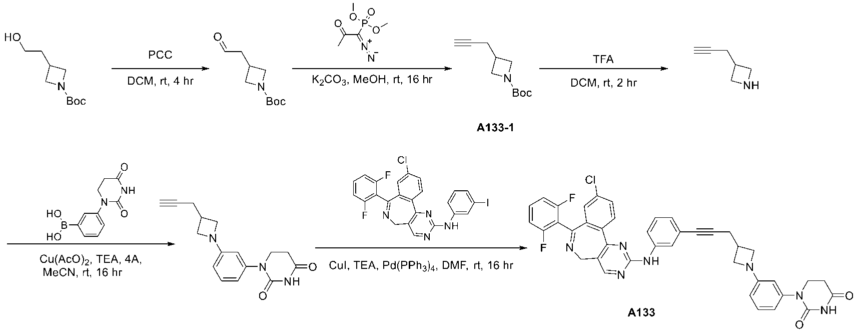

### Step 1:

Compound **A133-1** (2.3 g, 55.8% yield) was synthesized according to the synthesis method of compound **A6-2** described in **Example 6.** LCMS (ESI) m/z = 196.3 [M+H]⁺.

### Step 2:

Compound **A133** (8 mg, yield 6.3%) was synthesized according to the synthesis method of compound **A98** described in **Example 98.** LCMS (ESI) m/z = 714.3 [M+H]⁺. ¹H NMR (400 MHz, DMSO-*d*₆) δ 10.30 (s, 1H), 9.93 (s, 1H), 8.68 (s, 1H), 8.26 (d, *J =* 8.5 Hz, 1H), 7.89 - 7.77 (m, 3H), 7.59 - 7.49 (m, 1H), 7.33 (d, *J =* 2.1 Hz, 1H), 7.26 (t, *J =* 7.9 Hz, 1H), 7.20 - 7.09 (m, 3H), 6.93 - 6.89 (m, 1H), 6.61 - 6.57 (m, 1H), 6.41 - 6.38 m, 1H), 6.32 - 6.28 (m, 1H), 3.97 (t, *J* = 7.6 Hz, 2H), 3.71 (t, *J* = 6.8 Hz, 2H), 3.63 - 3.57 (m, 2H), 3.0 - 2.92 (m, 1H), 2.81 - 2.76 (m, 2H), 2.66 (t, *J* = 6.8 Hz, 2H).

### Example 133 Synthesis of Compound A134:

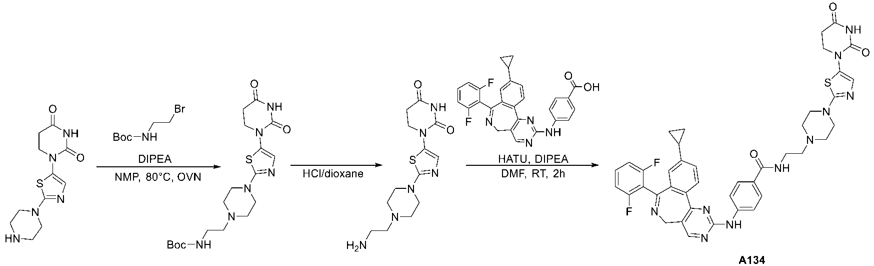

Compound **A134** (10.0 mg, yield 50.9%) was synthesized according to the synthesis method of compound **A16** described in **Example 16.** LCMS (ESI) m/z = 789.5 [M+H]⁺. ¹H NMR (400 MHz, DMSO-*d*₆) δ 10.60 (s, 1H), 10.10 (s, 1H), 8.70 (s, 1H), 8.30 *(t, J =* 5.6 Hz, 1H), 8.20 (d, *J =* 8.2 Hz, 2H), 7.94 (d, *J =* 8.6 Hz, 2H), 7.85 (d, *J =* 8.6 Hz, 2H), 7.58 - 7.50 (m, 1H), 7.41 - 7.38 (m, 1H), 7.19 - 7.15 (m, 2H), 7.08 (s, 1H), 7.02 (s, 1H), 3.81 (t, *J =* 6.8 Hz, 2H), 3.46 - 3.40 (m, 6H), 2.75 (t, *J =* 6.8 Hz, 2H), 2.59 - 2.56 (m, 6H), 2.05 - 1.99 (m, 1H), 1.04 - 1.02 (m, 2H), 0.72 (s, 2H).

### Example 134 Synthesis of Compound A135:

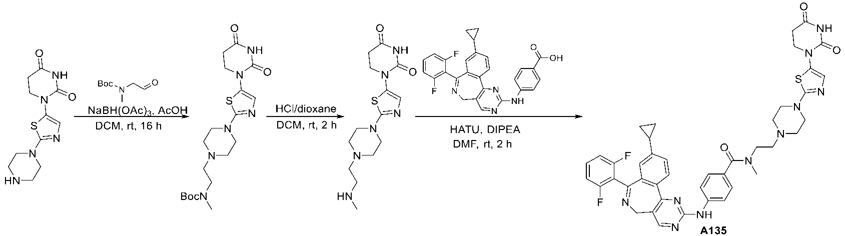

Compound **A135** (9 mg, yield 36%) was synthesized according to the synthesis method of compound **A75** described in **Example 75.** LCMS (ESI) m/z = 803.4 [M+H]⁺. ¹H NMR (400 MHz, DMSO-*d*₆) δ 10.60 (s, 0.5H), 10.03 (s, 1H), 8.68 (s, 1H), 8.29 - 8.12 (m, 1H), 8.01 - 7.79 (m, 2H), 7.63 - 7.48 (m, 1H), 7.48 - 7.34 (m, 3H), 727- 7.12 (m, 2H), 7.12 - 6.95 (m, 2H), 3.81 (t, *J =* 6.8 Hz, 2H), 3.02 (s, 3H), 2.75 (t, *J =* 6.8 Hz, 2H), 2.66 - 2.55 (m, 2H).

### Example 135 Synthesis of Compound A136:

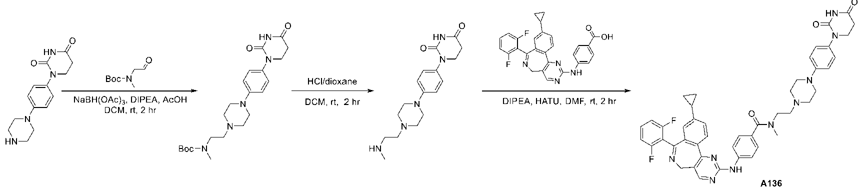

Compound **A136** (9 mg, yield 54.5%) was synthesized according to the synthesis method of compound **A75** described in **Example 75.** LCMS (ESI) m/z = 796.6 [M+H]⁺. ¹H NMR (400 MHz, DMSO-*d*₆) δ 10.24 (s, 1H), 10.00 (s, 1H), 8.65 (s, 1H), 8.17 (d, *J =* 8.2 Hz, 1H), 7.91 - 7.86 (m, 2H), 7.56 - 7.46 (m, 1H), 7.41 - 7.32 (m, 3H), 7.18 - 7.07 (m, 4H), 7.05 - 7.05 (m, 1H), 6.94 - 6.85 (m, 2H), 3.68 (t, *J=* 6.8 Hz, 2H), 3.61 - 3.42 (m, 2H), 3.30 - 3.23 (m, 2H), 3.18 - 3.01 (m, 4H), 2.99 (s, 3H), 2.67 (t, *J =* 6.8 Hz, 2H), 2.62 - 2.52 (m, 4H), 2.03 - 1.94 (m, 1H), 1.01 - 0.95 (m, 2H), 0.74 - 0.60 (m, 2H).

### Example 136 Synthesis of Compound A137:

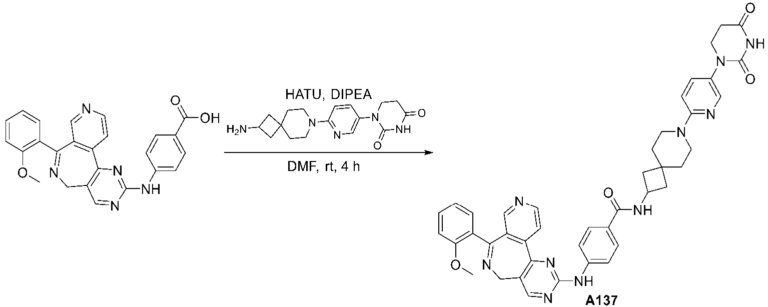

Compound A137 (8 mg, yield 31 %) was synthesized according to the synthesis method of compound **A75** described in **Example 75.** LCMS (ESI) m/z = 749.4 [M+H]⁺. ¹H NMR (400 MHz, DMSO-*d*₆) δ 10.35 (s, 1H), 10.24 (s, 1H), 8.89 (d, *J =* 5.2 Hz, 1H), 8.80 (s, 1H), 8.53 - 8.38 (m, 2H), 8.17 (d, *J =* 5.2 Hz, 1H), 8.07 (d, *J =* 2.4 Hz, 1H), 8.01 - 7.92 (m, 2H), 7.92 - 7.83 (m, 2H), 7.63 - 7.54 (m, 1H), 7.54 - 7.42 (m, 2H), 7.11 (t, J= 7.2 Hz, 1H), 8.01 (d, *J=* 8.0 Hz, 1H), 6.88 (d, J= 9.2 Hz, 1H), 4.60 - 4.34 (m, 1H), 3.72 (t, J= 6.8 Hz, 2H), 3.61 - 3.51 (m, 2H), 3.51 - 3.45 (m, 2H), 3.31 (s, 1H), 2.73 (t, *J =* 6.4 Hz, 2H), 2.33 - 2.21 (m, 2H), 1.98 - 1.83 (m, 2H), 1.72 - 1.64 (m, 2H), 1.64 - 1.56 (m, 2H).

### Example 137 Synthesis of Compound A138:

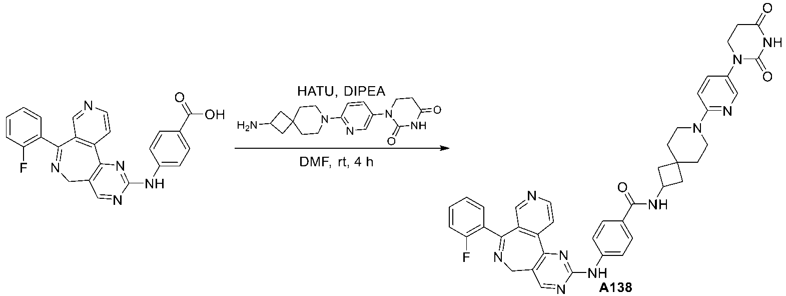

Compound **A138** (4 mg, yield 15%) was synthesized according to the synthesis method of compound **A75** described in **Example 75.** LCMS (ESI) m/z = 737.4 [M+H]⁺. ¹H NMR (400 MHz, DMSO-*d*₆) δ 10.35 (s, 1H), 10.23 (s, 1H), 8.87 (d, *J =* 4.8 Hz, 1H), 8.80 (s, 1H), 8.58 (s, 1H), 8.47 (d, *J* = 7.6 Hz, 1H), 8.21 (d, *J =* 5.2 Hz, 1H), 8.07 (d, *J =* 2.8 Hz, 1H), 7.99 - 7.92 (m, 2H), 7.92 - 7.85 (m, 2H), 7.74 - 7.65 (m, 1H), 7.62 - 7.53 (m, 1H), 7.53 - 7.47 (m, 1H), 7.40- 7.33 (m, 1H), 7.26 - 7.16 (m, 1H), 6.93 - 6.83 (m, 1H), 4.59 - 4.38 (m, 1H), 3.72 (t, *J* = 6.4 Hz, 2H), 3.62 - 3.51 (m, 2H), 3.51 - 3.44 (m, 2H), 2.73 (t, *J =* 6.8 Hz, 2H), 2.33 - 2.19 (m, 2H), 1.98 - 1.85 (m, 2H), 1.74 - 1.65 (m, 2H), 1.64 - 1.55 (m, 2H).

### Example 138 Synthesis of Compound A139:

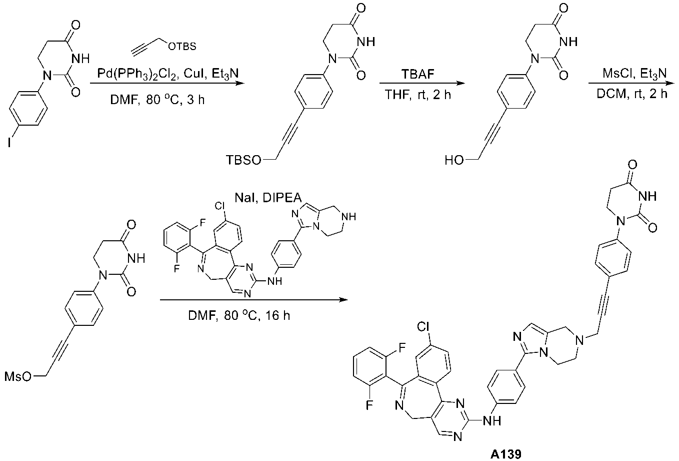

Compound **A139** (1 mg, yield 2%) was synthesized according to the synthesis method of compound **A26** described in **Example 26.** LCMS (ESI) m/z = 780.4 [M+H]⁺. ¹H NMR (400 MHz, DMSO-*d*₆) δ 10.44 (s, 1H), 10.09 (s, 1H), 8.73 (s, 1H), 8.34 (d, *J* = 8.4 Hz, 1H), 7.99 - 7.85 (m, 3H), 7.76 - 7.67 (m, 2H), 7.64 - 7.54 (m, 1H), 7.54 - 7.47 (m, 2H), 7.41 - 7.32 (m, 3H), 7.25 - 7.14 (m, 2H), 6.82 (s, 1H), 4.31 - 4.16 (m, 2H), 3.93 - 3.77 (m, 6H), 3.03 - 2.89 (m, 2H), 2.77 - 2.71 (m, 2H).

### Example 139 Synthesis of Compound A140:

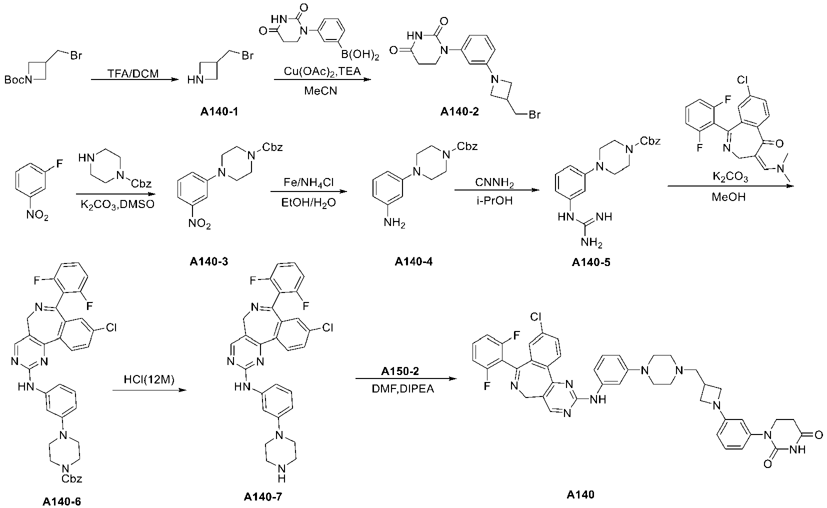

### Step 1:

At room temperature, trifluoroacetic acid (2 mL) was added to a solution of tert-butyl 3-(bromomethyl)azetidine-1-carboxylate (600 mg, 2.4 mmol) in dichloromethane (5 mL), and the reaction mixture was stirred at room temperature for 2 h. The reaction mixture was concentrated under reduced pressure to give a light yellow oil **A140-1** (360 mg, yield 100%). The crude product was directly used in the next reaction.

### Step 2:

**A98-1** (450 mg, 1.92 mmol), copper acetate (436 mg, 2.4 mmol), triethylamine (728 mg, 7.2 mmol) and 4Å molecular sieve (300 mg) were added to a solution of **A140-1** (360 mg, 2.4 mmol) in acetonitrile (10 mL) at room temperature, and the reaction mixture was stirred at room temperature for 3 h. LC-MS showed that the raw materials reacted completely. The reaction mixture was filtered through diatomaceous earth, the filtrate was concentrated under reduced pressure, and the residue was separated for purification by silica gel column chromatography (methanol/dichloromethane = 1:20) to give a yellow solid **A140-2** (120 mg, yield 15.4%). LCMS (ESI) m/z = 338.4 [M+H]⁺.

### Step 3:

At room temperature, potassium carbonate (19.6 g, 142 mmol) and benzyl piperazine-1-carboxylate (15.6 g, 70.9 mmol) were added to a solution of m-nitrofluorobenzene (10 g, 70.9 mmol) in dimethyl sulfoxide (60 mL), and the mixture was heated to 100°C and stirred to react for 16 h. After the addition of water, the mixture was extracted with ethyl acetate, dried over anhydrous sodium sulfate, and concentrated under reduced pressure. The residue was separated for purification by silica gel column chromatography (ethyl acetate/petroleum ether = 1:10) to give a yellow solid **A140-3** (4.8 g, yield 19.8%).

### Step 4:

At room temperature, reduced iron powder (3.93 g, 70.0 mmol) and ammonium chloride (3.76 g, 70.0 mmol) were added to a solution of **A140-3** (4.8 g, 14.0 mmol) in ethanol (50 mL) and water (10 mL), and the mixture was heated to 80°C and stirred to react for 3 h. The reaction mixture was filtered through diatomaceous earth and concentrated under reduced pressure. After the addition of water, the mixture was extracted with ethyl acetate, dried over anhydrous sodium sulfate, and concentrated under reduced pressure. The residue was separated for purification by silica gel column chromatography (ethyl acetate/petroleum ether = 1:5) to give a yellow solid **A140-4** (4 g, yield 91.3%). LCMS (ESI) m/z = 311.5 [M+H]⁺.

### Step 5:

At room temperature, cyanamide (270 mg, 6.4 mmol) and p-toluenesulfonic acid monohydrate (916 mg, 4.8 mmol) were added to a solution of **A140-4** (1 g, 3.2 mmol) in isopropanol (10 mL), and the mixture was heated to 80°C and stirred to react for 16 h. LCMS showed a product yield of about 60%. The reaction mixture was concentrated. Water was added to the residue, and the mixture was extracted with ethyl acetate, dried over anhydrous sodium sulfate, and concentrated under reduced pressure. The residue was separated for purification by silica gel column chromatography (methanol/dichloromethane = 1:10) to give an off-white solid **A140-5** (580 mg, yield 51.1%). LCMS (ESI) m/z = 353.5 [M+H]⁺.

### Step 6:

At room temperature, potassium carbonate (567 mg, 4.1 mmol) and **SM4** (592 mg, 1.64 mmol) were added to a solution of **A140-5** (580 mg, 1.64 mmol) in methanol (20 mL), and the mixture was heated to 70°C and stirred to react for 16 h. LC-MS showed that the raw materials reacted completely. The reaction mixture was filtered, concentrated under reduced pressure, and the residue was separated for purification by silica gel column chromatography (methanol/dichloromethane = 1:60) to give a yellow solid **A140-6** (560 mg, yield 52.4%). LCMS (ESI) m/z = 652.2 [M+H]⁺.

### Step 7:

At room temperature, concentrated hydrochloric acid (0.25 mL, 3 mmol) was added to **A140-6** (200 mg, 0.30 mmol), and the mixture was heated to 100°C and stirred to react for 4 h. LC-MS showed that the raw materials reacted completely. Saturated sodium bicarbonate was added to the reaction mixture to adjust the pH value to 8. The mixture was extracted with ethyl acetate, dried over anhydrous sodium sulfate, and concentrated under reduced pressure. The residue was separated for purification by silica gel column chromatography (methanol/dichloromethane = 1:20) to give a yellow solid A140-7 (100 mg, yield 62.9%). LCMS (ESI) m/z = 517.2 [M+H]⁺.

### Step 8:

At room temperature, **A140-2** (49 mg, 0.14 mmol) and N,N-diisopropylethylamine (37.5 mg, 0.27 mmol) were added to a solution of **A140-7** (50 mg, 0.09 mmol) in N,N-dimethylformamide (5 mL), and the mixture was heated to 80°C and stirred to react for 6 h. LC-MS showed a product yield of about 50%. The reaction mixture was filtered, and the crude product was separated for purification by reverse phase chromatography (acetonitrile/10 mM ammonium bicarbonate solution) to give a yellow solid **A140** (23 mg, yield 30.7%). LCMS (ESI) m/z = 774.5 [M+H]⁺. ¹H NMR (400 MHz, DMSO-*d*₆) δ 10.30 (s, 1H), 9.72 (s, 1H), 8.66 (s, 1H), 8.29 (d, *J =* 8.8 Hz, 1H), 7.88 - 7.82 (m, 1H), 7.62 - 7.51 (m, 2H), 7.33 (d, *J =* 4.0 Hz, 1H), 7.24 - 7.20 (m, 1H), 7.19 - 7.10 (m, 4H), 6.63 - 6.57 (m, 2H), 6.39 - 6.36 (m, 1H), 6.32 - 6.28 (m, 1H), 3.99 - 3.92 (m, 2H), 3.50 - 3.45 (m, 2H), 3.51 - 3.44 (m, 2H), 3.18 - 3.09 (m, 4H), 3.02 - 2.93 (m, 1H), 2.70 - 2.62 (m, 4H), 2.58 - 2.52 (m, 4H).

### Example 140 Synthesis of Compound A141:

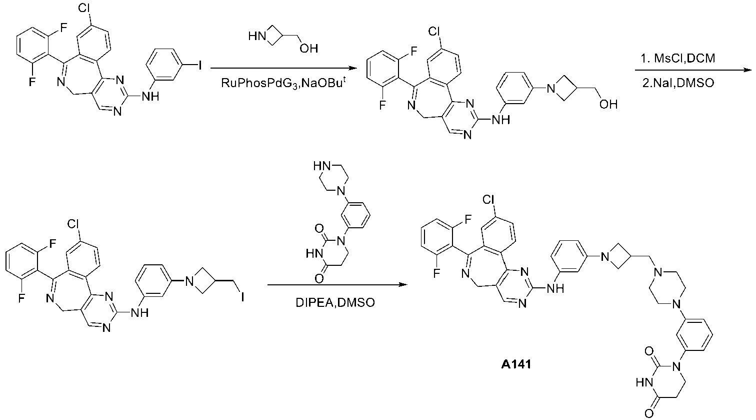

Compound **A141** (1.21 mg, yield 4%) was synthesized according to the synthesis method of compound **A126** described in **Example 125.** LCMS (ESI) m/z = 775.5 [M+H]⁺. ¹H NMR (400 MHz, DMSO-*d*₆) *δ* 10.33 (s, 1H), 9.74 (s, 1H), 8.68 (s, 1H), 8.35 (d, *J =* 7.8 Hz, 1H), 7.87 (dd, *J*₁ = 7.8 Hz, *J*₂ = 2.4 Hz, 1H), 7.60 - 7.52 (m, 1H), 7.37 (s, 1H), 7.34 (d, *J =* 7.8 Hz, 1H), 7.24 - 7.16 (m, 3H), 7.09 (t, *J =* 7.4 Hz, 1H), 6.98 - 6.96 (m, 1H), 6.90 (bs, 1H), 6.84 - 6.82 (m, 1H), 6.75 - 6.73 (m, 1H), 6.10 - 6.08 (m, 1H), 4.00 - 3.96 (m, 2H), 3.76 (t, *J* = 6.8 Hz, 2H), 3.54 - 3.51 (m, 2H), 3.21 - 3.18 (m, 4H), 3.05 - 2.95 (m, 1H), 2.72 - 2.67 (m, 4H), 2.56 - 2.52 (m, 4H).

### Example 141 Synthesis of Compound A142:

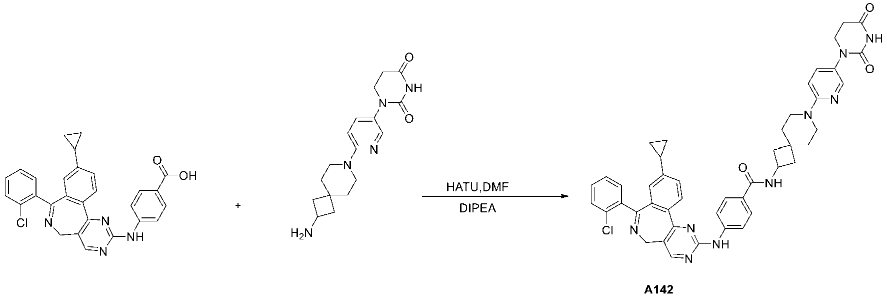

Compound **A142** (6.7 mg, yield 15%) was synthesized according to the synthesis method of compound **A1** described in **Example 1.** LCMS (ESI) m/z = 793.4 [M+H]⁺. 1H NMR (400 MHz, DMSO-*d*₆) δ 10.36 (s, 1H), 10.09 (s, 1H), 8.69 (s, 1H), 8.45 (d, *J* = 8.4 Hz, 1H), 8.18 (d, *J =* 8.4 Hz, 1H), 8.07 (d, *J* = 2.4 Hz, 1H), 7.96 - 7.93 (m, 2H), 7.87 - 7.84 (m, 2H), 7.63 - 7.60 (m, 1H), 7.51 - 7.46 (m, 3H), 7.43 - 7.38 (m, 2H), 6.91 - 6.87 (m, 2H), 4.51 - 4.45 (m 1H), 3.72 (t, *J* = 6.8 Hz, 2H), 3.54 - 3.46 (m, 4H), 2.72 (t, *J* = 6.8 Hz, 2H), 2.29 - 2.24 (m, 2H), 1.98 - 1.88 (m, 3H), 1.86 - 1.60 (m, 4H), 1.03 - 0.97 (m, 2H).

### Example 142 Synthesis of Compound A143:

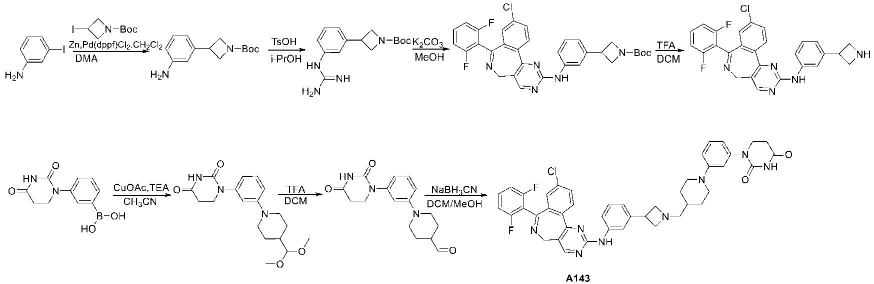

Compound **A143** (9.56 mg, yield 16.76%) was synthesized according to the synthesis method of compound **A132** described in **Example 131.** LCMS (ESI) m/z = 773.3 [M+H]⁺. ¹H NMR (400 MHz, DMSO-*d*₆) δ 10.29 (s, 1H), 9.85 (s, 1H), 8.67 (s, 1H), 8.30 (d, *J =* 8.4 Hz, 1H), 8.02 (s, 1H), 7.85 (dd, *J*₁ = 8.4 Hz, *J*₂ = 2.0 Hz, 1H), 7.59 - 7.47 (m, 2H), 7.33 (d, *J =* 2.0 Hz, 1H), 7.28 - 7.13 (m, 4H), 6.91 (d, *J =* 7.6 Hz, 1H), 6.85 (d, *J=* 2.0 Hz, 1H), 6.77 (dd, *J =* 8.0, 2.4 Hz, 1H), 6.67 (dd, *J*₁ = 7.6 Hz, *J*₂ = 2.0 Hz, 1H), 3.73 (t, *J =* 6.8 Hz, 2H), 3.70 - 3.55 (m, 4H), 3.14 - 2.96 (m, 1H), 2.68 (t, *J =* 6.8 Hz, 2H), 2.65 - 2.55 (m, 2H), 2.34 - 2.18 (m, 1H), 1.77 - 1.68 (m, 2H), 1.46 - 1.32 (m, 2H), 1.33 - 1.11 (m, 2H).

### Example 143 Synthesis of Compound A144:

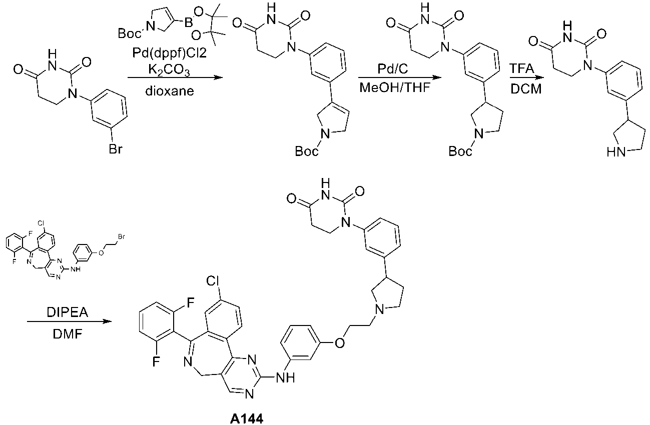

Compound **A144** (4.26 mg, yield 16.12%) was synthesized according to the synthesis method of compound **A56** described in **Example 56.** LCMS (ESI) m/z = 733.8 [M+H]⁺. ¹H NMR (400 MHz, DMSO-*d*₆) δ 10.33 (s, 1H), 9.87 (s, 1H), 8.67 (s, 1H), 8.29 (d, *J =* 8.4 Hz, 1H), 7.84 (dd, *J*₁ = 8.4 Hz, *J*₂ = 2.0 Hz, 1H), 7.65 (d, *J =* 2.8 Hz, 1H), 7.60 - 7.48 (m, 1H), 7.37 - 7.07 (m, 10H), 6.57 (dd, *J*₁ = 8.0 Hz, *J*₂ = 2.4 Hz, 1H), 4.10 (t, *J =* 5.8 Hz, 2H), 3.75 (t, *J =* 6.8 Hz, 2H), 3.05 (s, 1H), 2.97 - 2.71 (m, 4H), 2.67 (t, *J =* 6.8 Hz, 2H), 2.58 - 2.53 (m, 1H), 2.31 - 2.21 (m, 1H), 1.81 - 1.72 (m, 1H).

### Example 144 Synthesis of Compound A145:

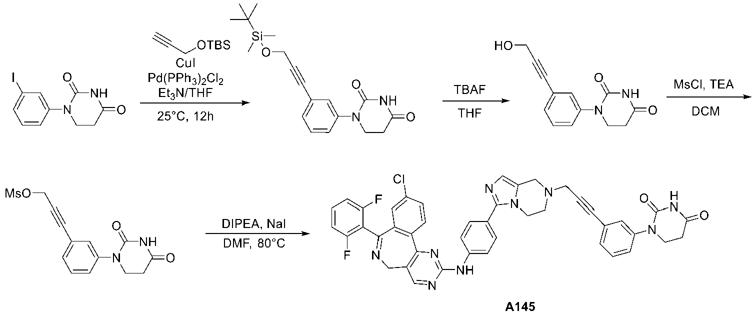

Compound **A145** (15.0 mg, yield 42.6%) was synthesized according to the synthesis method of compound **A139** described in **Example 138.** LCMS (ESI) m/z = 780.5 [M+H]⁺. ¹H NMR (400 MHz, DMSO-*d*₆) δ 10.41 (s, 1H), 10.07 (s, 1H), 8.70 (s, 1H), 8.30 (d, *J* = 8.4 Hz, 1H), 7.97 - 7.84 (m, 3H), 7.67 (d, *J =* 8.4 Hz, 2H), 7.58 - 7.50 (m, 1H), 7.44 - 7.31 (m, 5H), 7.19 - 7.14 (m, 2H), 6.79 (s, 1H), 4.22 - 4.19 (m, 2H), 3.85 (s, 2H), 3.80 - 3.77 (m, 4H), 2.96 - 2.93 (m, 2H), 2.69 (t, *J* = 6.6 Hz, 2H).

### Example 145 Synthesis of Compound A146:

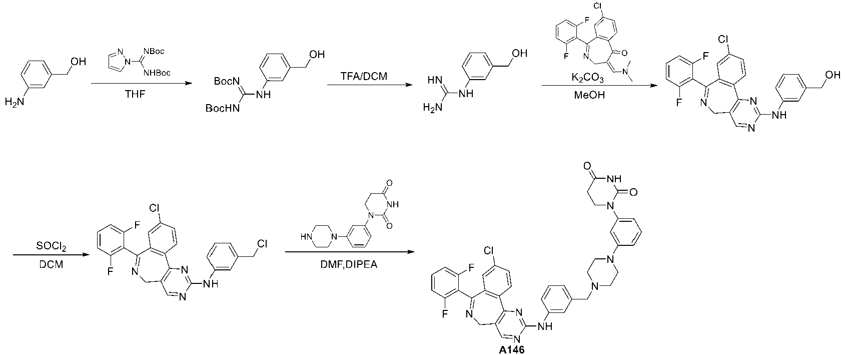

Compound **A146** (14.3 mg, yield 18.4%) was synthesized according to the synthesis method of compound **A101** described in **Example 101.** LCMS (ESI) m/z = 719.4 [M+H]⁺. ¹H NMR (400 MHz, DMSO-*d*₆) δ 10.29 (s, 1H), 9.85 (s, 1H), 8.66 (s, 1H), 8.28 (d, *J =* 8.8 Hz, 1H), 7.89 - 7.82 (m, 2H), 7.70 - 7.65 (m, 1H), 7.56 - 7.47 (m, 1H), 7.32 - 7.24 (m, 2H), 7.20 - 7.06 (m, 3H), 6.97 - 6.92 (m, 1H), 6.87 - 6.84 (m, 1H), 6.78 - 6.73 (m, 1H), 6.73 - 6.68 (m, 1H), 3.72 (t, *J =* 8.0 Hz, 2H), 3.53 (s, 2H), 3.15 - 3.05 (m, 4H), 2.69 - 2.64 (m, 2H), 2.58 - 2.52 (m, 4H).

### Example 146 Synthesis of Compound A147:

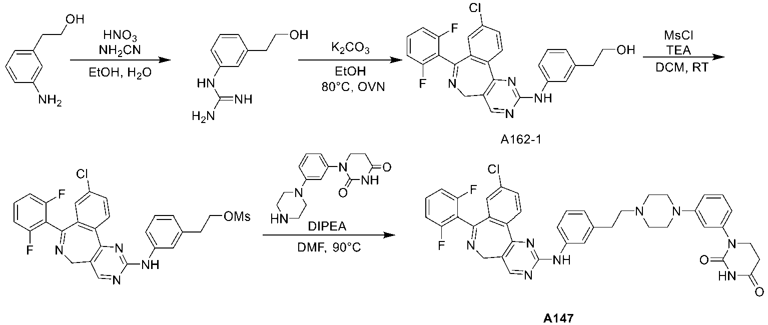

Compound **A147** (22.0 mg, yield 42.7%, yellow solid) was synthesized according to the synthesis method of compound **A101** described in **Example 101.** LCMS (ESI) m/z = 733.2 [M+H]⁺. ¹H NMR (400 MHz, DMSO-*d*₆) δ 10.32 (s, 1H), 9.82 (s, 1H), 8.69 (s, 1H), 8.32 (d, *J =* 8.4 Hz, 1H), 7.89 (dd, *J*₁ = 8.4 Hz, *J*₂ = 2.2 Hz, 2H), 7.76 (s, 1H), 7.67 *(d, J=* 8.2 Hz, 1H), 7.32 - 7.51 (m, 1H), 7.35 (d, *J =* 2.2 Hz, 1H), 7.29 7.14 (m, 4H), 6.95 - 6.89 (m, 2H), 6.83 (dd, *J*₁ = 8.0 Hz, *J*₂ = 2.4 Hz, 1H), 6.77 - 6.71 (m, 1H), 3.77 (t, *J* = 4.0 Hz, 2H) 3.18 - 3.16 (m, 2H), 2.82 - 2.78 (m, 2H), 2.71 (t, *J =* 6.6 Hz, 2H), 2.64 - 2.60 (m, 6H).

### Example 147 Synthesis of Compound A148:

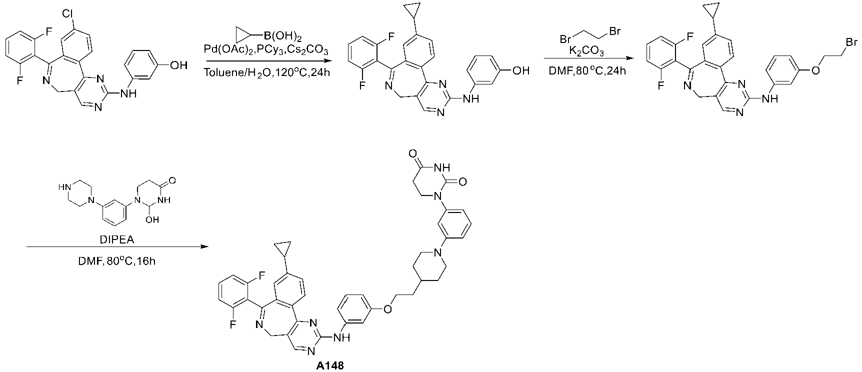

Compound **A148** (5.8 mg, yield 7.7%) was synthesized according to the synthesis method of compound **A101** described in **Example 101.** LCMS (ESI) m/z = 755.3 [M+H]⁺. ¹H NMR (400 MHz, DMSO-*d*₆) δ 10.29 (s, 1H), 9.78 (s, 1H), 8.63 (s, 1H), 8.18 (d, *J =* 8.0 Hz, 1H), 7.70 (t, *J =* 2.2 Hz, 1H), 7.56 - 7.47 (m, 1H), 7.38 - 7.30 (m, 2H), 7.21 - 7.10 (m, 4H), 7.04 (d, *J =* 1.8 Hz, 1H), 6.88 - 6.85 (m, 1H), 6.81 - 6.76 (m, 1H), 6.73 - 6.69 (m, 1H), 6.59 - 6.53 (m, 1H), 4.12 (t, *J =* 6.0 Hz, 2H), 3.73 (t, *J =* 8.0 Hz, 2H), 3.16 - 3.09 (m, 4H), 2.77 (t, *J =* 6.0 Hz, 2H), 2.67 (t, *J =* 6.0 Hz, 2H), 2.64 - 2.60 (m, 4H), 2.02 - 1.94 (m, 1H), 1.00 - 0.93 (m, 2H), 0.73 - 0.56 (m, 2H).

### Example 148 Synthesis of Compound A149:

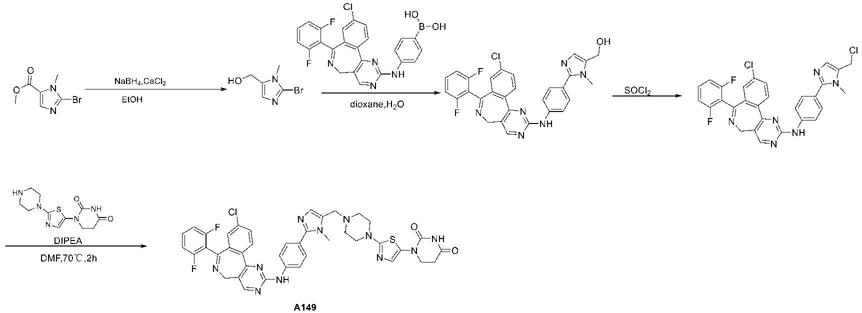

Compound **A149** (6.0 mg, yield 27%) was synthesized according to the synthesis method of compound **A106** described in **Example 106.** LCMS (ESI) m/z = 806.2 [M+H]⁺. ¹H NMR (400 MHz, DMSO-*d*₆) δ 10.61 (s, 1H), 10.12 (s, 1H), 8.74 (s, 1H), 8.34 (d, *J =* 8.4Hz, 1H), 7.98 - 7.96 (m, 2H), 7.92 - 7.89 (m, 1H), 7.66 - 7.63 (m, 2H), 7.63 - 7.54 (m, 1H), 7.36 (d, *J =* 2.4 Hz, 1H), 7.22 - 7.18 (m, 1H), 7.02 (s, 1H), 6.91 (s, 1H), 3.82 (t, *J* = 6.8 Hz, 2H), 3.75 (s, 3H), 3.58 (s, 2H), 3.42 - 3.38 (m, 4H), 2.75 (t, *J* = 6.8 Hz, 2H), 2.57 - 2.54 (m, 4H).

### Example 149 Synthesis of Compound A150:

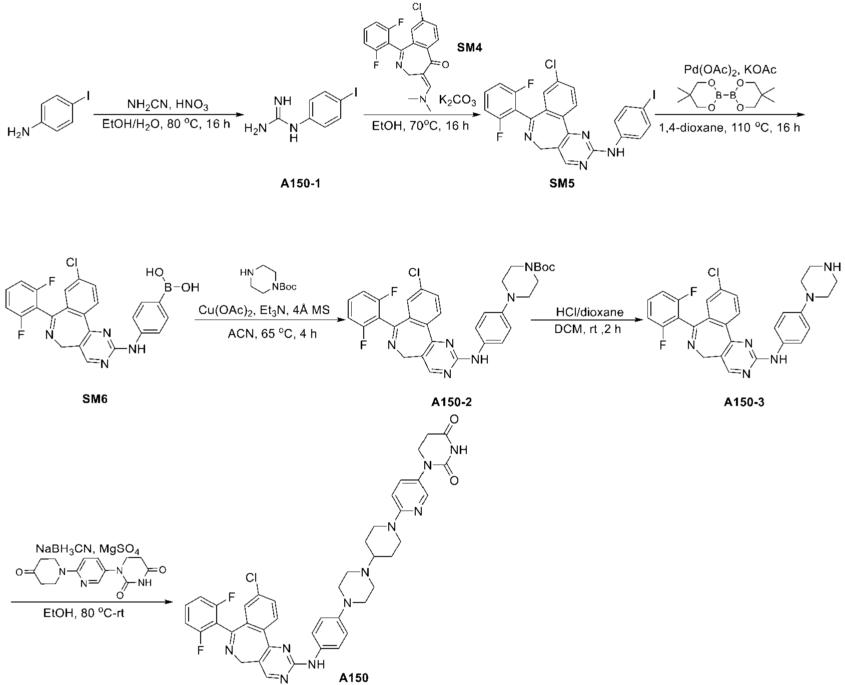

### Step 1:

To a solution of compound p-iodoaniline (5 g, 22.83 mmol) in ethanol (35 mL), a solution of 66% nitric acid (2.29 g, 23.97 mmol, 1.62 mL) and cyanamide (1.92 g, 45.66 mmol) in water (2 mL) was slowly added at 0°C. The reaction mixture was heated to 80°C and stirred for 16 h. The reaction mixture was concentrated under reduced pressure to remove ethanol (20 mL). The residue was stirred at 0°C for 1 h and filtered, and the resulting solid was washed with a small amount of cold ethanol and dried by an oil pump to give a light yellow solid **A150-1** (3.4 g, yield 57%). LCMS (ESI) m/z = 262.1 [M+H]⁺.

### Step 2:

To a solution of compound **A150-1** (1.82 g, 6.98 mmol) in ethanol (34 mL) were added **SM4** (1.68 g, 4.66 mmol) and potassium carbonate (1.61 g, 11.64 mmol) at room temperature. The reaction mixture was heated to 70°C and stirred for 16 h. After the addition of water, the reaction mixture was extracted with ethyl acetate, washed with saturated brine, dried over anhydrous sodium sulfate, concentrated under reduced pressure, and the residue was separated for purification by silica gel column chromatography to give a brown solid **SM5** (2 g, yield 77%). LCMS (ESI) m/z = 559.2 [M+H]⁺.

### Step 3:

Bis(neopentyl glycolato)diboron (4.85 g, 21.48 mmol), palladium acetate (402 mg, 1.79 mmol) and potassium acetate (1.05 g, 10.74 mmol) were added to a solution of compound **SM5** (2 g, 3.58 mmol) in 1,4-dioxane (40 mL) at room temperature. The reaction mixture was heated to 110°C under argon protection and stirred for 16 h. The reaction mixture was filtered, the filtrate was concentrated under reduced pressure, and the residue was separated for purification by silica gel column chromatography to give a yellow solid **SM6** (2.3 g, yield 94%, purity 70%). LCMS (ESI) m/z = 477.3 [M+H]⁺.

### Step 4:

At room temperature, 1-Boc-piperazine (1.53 g, 8.22 mmol), copper acetate (308 mg, 1.17 mmol), triethylamine (356 mg, 3.52 mmol) and 4Å molecular sieve (1.6 g) were added to compound **SM6** (800 mg, 1.17 mmol) in acetonitrile (16 mL). The reaction mixture was heated to 65°C and stirred for 4 h. The reaction mixture was filtered, the filtrate was concentrated under reduced pressure, and the residue was separated for purification by silica gel column chromatography to give a yellow solid **A150-2** (320 mg, yield 44%). LCMS (ESI) m/z = 617.4 [M+H]⁺.

### Step 5:

HCl (4 M in dioxane, 3 mL) was added to a solution of compound **A150-2** (320 mg, 0.52 mmol) in dichloromethane (6 mL) at room temperature. The reaction mixture was stirred at room temperature for 2 h. The reaction mixture was concentrated under reduced pressure, and saturated aqueous sodium bicarbonate solution (10 mL) was added to the residue. The mixture was extracted with dichloromethane, washed with saturated brine, dried over anhydrous sodium sulfate, and concentrated under reduced pressure to give a yellow solid **A150-3** (205 mg, yield 76%). LCMS (ESI) m/z = 516.3 [M+H]⁺.

### Step 6:

**A132-4** (56 mg, 0.19 mmol) and magnesium sulfate (233 mg, 1.93 mmol) were added to a solution of compound **A150-5** (50 mg, 0.10 mmol) in ethanol (2 mL) at room temperature. The reaction mixture was heated to 80°C under argon protection and stirred for 16 h. The mixture was cooled to room temperature. After the addition of sodium cyanoborohydride (18 mg, 0.29 mmol), the reaction mixture was stirred at room temperature for 16 h under argon protection. After the addition of water, the reaction mixture was extracted with dichloromethane, washed with saturated brine, dried over anhydrous sodium sulfate, and concentrated under reduced pressure. The residue was separated for purification by reverse phase chromatography (acetonitrile/10 mM ammonium bicarbonate solution) to give a yellow solid **A150** (7 mg, yield 9%). LCMS (ESI) m/z = 780.4 [M+H]⁺. ¹H NMR (400 MHz, DMSO-*d*₆) δ 10.36 (s, 1H), 9.61 (s, 1H), 8.61 (s, 1H), 8.27 (d, *J =* 8.4 Hz, 1H), 8.08 (d, *J =* 2.8 Hz, 1H), 7.92 - 7.82 (m, 1H), 7.65 (d, *J =* 8.8 Hz, 1H), 7.62 - 7.54 (m, 1H), 7.54 - 7.46 (m, 1H), 7.33 (d, *J =* 2.0 Hz, 1H), 7.25 - 7.11 (m, 2H), 6.98 - 6.85 (m, 3H), 4.42 - 4.26 (m, 2H), 3.73 (t, *J* = 6.8 Hz, 2H), 3.15 - 3.03 (m, 4H), 2.91 - 2.78 (m, 2H), 2.77 - 2.62 (m, 6H), 1.97 - 1.83 (m, 2H), 1.53 - 1.35 (m, 2H).

### Example 150 Synthesis of Compound A151:

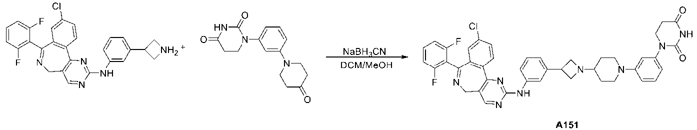

Compound **A151** (9.01 mg, yield 32.17%) was synthesized according to the synthesis method of compound **A132** described in **Example 131.** LCMS (ESI) m/z = 759.3 [M+H]⁺. ¹H NMR (400 MHz, DMSO-*d*₆) *δ* 10.32 (s, 1H), 9.88 (s, 1H), 8.69 (s, 1H), 8.32 (d, *J* = 8.4 Hz, 1H), 8.07 (s, 1H), 7.89 (dd, *J =* 8.4, 2.0 Hz, 1H), 7.60 - 7.45 (m, 2H), 7.35 (d, *J =* 2.0 Hz, 1H), 7.28 - 7.06 (m, 4H), 6.95 (d, *J =* 7.6 Hz, 1H), 6.90 - 6.86 (m, 1H), 6.80 (dd, *J*₁ = 8.0 Hz, *J*₂ = 2.4 Hz, 1H), 6.75 - 6.64 (m, 1H), 3.80 - 3.51 (m, 6H), 3.19 - 3.04 (m, 1H), 2.75 - 2.67 (m, 4H), 1.75 - 1.69 (m, 2H), 1.35 - 1.21 (m, 2H).

### Example 151 Synthesis of Compound A152:

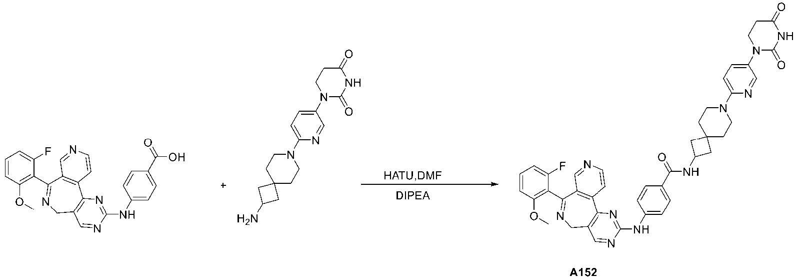

Compound **A152** (5.3 mg, yield 23%) was synthesized according to the synthesis method of compound **A1** described in **Example 1.** LCMS (ESI) m/z = 766.4 [M+H]⁺. ¹H NMR (400 MHz, DMSO-*d*₆) δ 10.35 (s, 1H), 10.25 (s, 1H), 8.92 (d, *J* = 4.8 Hz, 1H), 8.80 (s, 1H), 8.51 (s, 1H), 8.45 (d, *J* = 7.8 Hz, 1H), 8.18 (d, *J = 8.2* Hz, 2H), 8.06 (d, *J* = 5.6 Hz, 1H), 7.94 (d, *J* = 8.6 Hz, 2H), 7.88 (d, *J* = 8.6 Hz, 2H), 7.51 - 7.43 (m, 1H), 6.92 (bs, 1H), 6.87 (d, *J =* 6.8 Hz, 1H), 4.51 (m, 1H), 3.72 (t, *J =* 4.8 Hz, 2H), 3.55 - 3.53 (m, 2H), 3.48 - 3.45 (m, 2H), 2.72 (t, *J =* 6.8 Hz, 2H), 2.29 - 2.24 (m, 2H), 1.94 - 1.89 (m, 2H), 1.68 - 1.60 (m, 4H).

### Example 152 Synthesis of Compound A153:

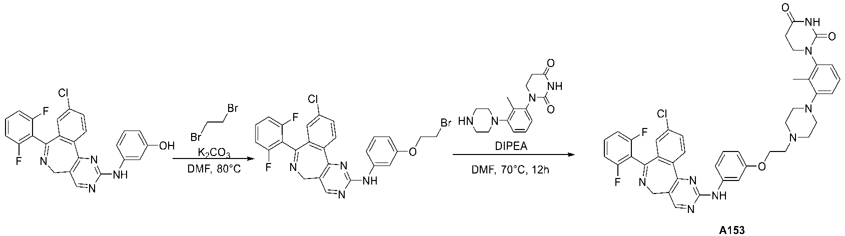

Compound **A153** (30.0 mg, yield 72.8%, yellow solid) was synthesized according to the synthesis method of compound **A101** described in **Example 101.** LCMS (ESI) m/z = 733.2 [M+H]. ¹H NMR (400 MHz, DMSO-*d*₆) δ 10.30 (s, 1H), 9.87 (s, 1H), 8.68 (s, 1H), 8.31 (d, *J =* 8.4 Hz, 1H), 7.87 (dd, *J*₁ = 8.4 Hz, *J*₂ = 2.2 Hz, 1H), 7.71 (s, 1H), 7.56 - 7.49 (m, 1H), 7.34 - 7.26 (m, 2H), 7.23 -7.10 (m, 4H), 7.01 - 6.93 (m, 2H), 6.61 - 6.55 (m, 1H), 4.13 - 4.10 (m, 2H), 3.76 - 3.69 (m, 1H), 3.52 - 3.46 (m, 1H), 2.9 - 2.57 (m, 12H), 2.10 (s, 3H).

### Example 153 Synthesis of Compound A154:

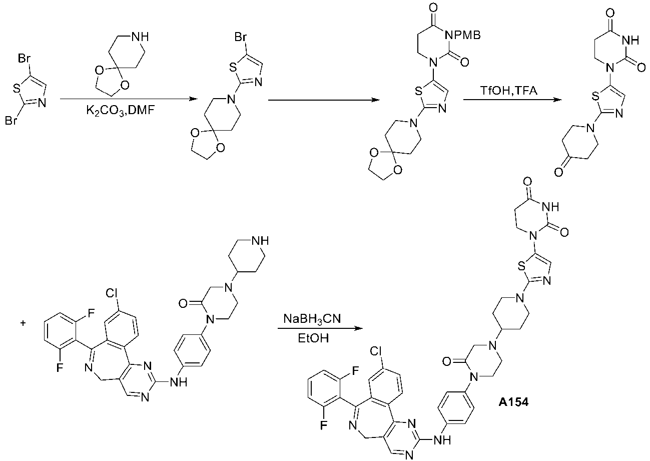

Compound **A154** (9 mg, yield 23%) was synthesized according to the synthesis method of compound **A150** described in **Example 149.** LCMS (ESI) m/z = 809.3 [M+H]. ¹H NMR (400 MHz, DMSO-*d*₆) δ 10.61 (s, 1H), 9.97 (s, 1H), 8.69 (s, 1H), 8.31 (d, *J =* 8.4 Hz, 1H), 7.90 - 7.82 (m, 2H), 7.61 - 7.55 (m, 1H), 7.35 (d, *J* = 2.4 Hz, 1H), 7.30 - 7.28 (m, 2H), 7.21 - 7.17 (m, 2H), 7.01 (s, 1H), 3.91 - 3.88 (m, 2H), 3.81 (t, *J* = 6.8 Hz, 2H), 3.65 - 3.62 (m, 2H), 3.34 (s, 2H), 3.04 - 2.98 (m, 2H), 2.91 - 2.89 (m, 2H), 2.75 (t, *J =* 6.4 Hz, 2H), 2.63 - 2.57 (m, 1H), 1.96 - 1.93 (m, 2H), 1.59 - 1.51 (m, 2H).

### Example 154 Synthesis of Compound A155:

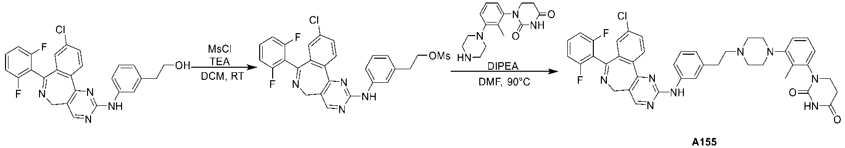

Compound **A155** (22.0 mg, yield 58.3%, yellow solid) was synthesized according to the synthesis method of compound **A101** described in **Example 101.** LCMS (ESI) m/z = 747.5 [M+H]. ¹H NMR (400 MHz, DMSO-*d*₆) δ 10.30 (s, 1H), 9.79 (s, 1H), 8.66 (s, 1H), 8.29 (d, *J =* 8.4 Hz, 1H), 7.86 (dd, *J*₁ = 8.4 Hz, *J*₂ = 2.2 Hz, 1H), 7.76 (s, 1H), 7.62 - 7.60 (m, 1H), 7.57 - 7.50 (m, 1H), 7.32 (d, *J =* 2.2 Hz, 1H), 7.24 - 7.13 (m, 4H), 7.01 - 6.96 (m, 2H), 6.89 - 6.87 (m, 1H), 3.76 - 3.69 (m, 1H), 3.52 - 3.46 (m, 1H), 2.85 - 2.62 (m, 14H), 2.11 (s, 3H).

### Example 155 Synthesis of Compound A156:

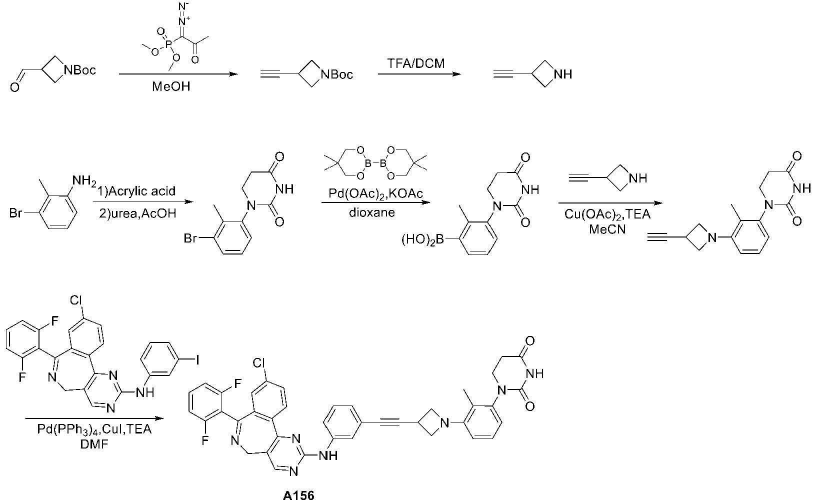

Compound **A156** (8.6 mg, yield 16.8%) was synthesized according to the synthesis method of compound **A18** described in **Example 18.** LCMS (ESI) m/z =714.4 [M+H]⁺. ¹H NMR (400 MHz, DMSO-*d*₆) δ 10.29 (s, 1H), 9.98 (s, 1H), 8.70 (s, 1H), 8.28 (d, *J =* 8.8 Hz, 1H), 7.97 (s, 1H), 7.89 - 7.78 (m, 2H), 7.60 - 7.49 (m, 1H), 7.36 - 7.26 (m, 2H), 7.20 - 7.08 (m, 3H), 7.07 - 7.01(m, 1H), 6.82 - 6.73 (m, 1H), 6.58 - 6.50 (m, 1H), 4.31 - 4.21 (m, 2H), 3.90 - 3.70 (m, 4H), 3.51 - 3.42 (m, 1H), 2.83 - 2.72 (m, 1H), 2.71 - 2.62 (m, 1H), 1.99 (s, 3H).

### Example 156 Synthesis of Compound A157:

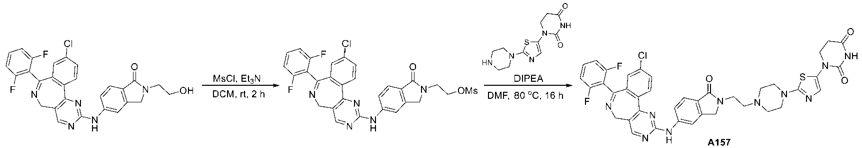

Compound **A157** (9 mg, yield 22%) was synthesized according to the synthesis method of compound **A101** described in **Example 101.** LCMS (ESI) m/z = 795.2 [M+H]⁺. ¹H NMR (400 MHz, DMSO-*d*₆) δ 10.61 (s, 1H), 10.30 (s, 1H), 8.76 (s, 1H), 8.33 (d, *J =* 8.4 Hz, 1H), 8.26 - 8.09 (m, 1H), 8.03 - 7.77 (m, 2H), 7.77 - 7.48 (m, 2H), 7.36 (s, 1H), 7.27 - 7.10 (m, 2H), 7.02 (s, 1H), 4.58 (s, 2H), 3.93 - 3.75 (m, 2H), 3.75 - 3.58 (m, 2H), 2.84 - 2.70 (m, 2H), 2.70 - 2.56 (m, 6H).

### Example 157 Synthesis of Compound A158:

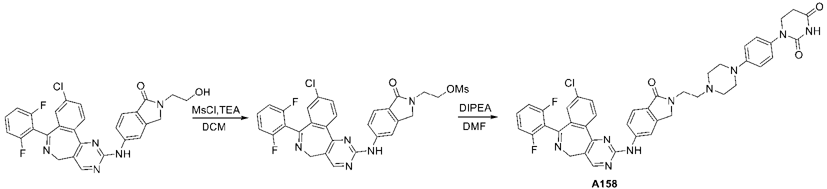

Compound **A158** (5.04 mg, yield 17.73%) was synthesized according to the synthesis method of compound **A48** described in **Example 48.** LCMS (ESI) m/z = 788.3 [M+H]⁺. ¹H NMR (400 MHz, DMSO-*d*₆) δ 10.29 (s, 1H), 10.28 (s, 1H), 8.76 (s, 1H), 8.33 (d, *J =* 8.4 Hz, 1H), 8.18 (d, *J =* 2.0 Hz, 1H), 7.90 - 7.85 (m, 2H), 7.64 - 7.57 (m, 2H), 7.36 (d, *J =* 2.0 Hz, 1H), 7.21 - 7.15 (m, 4H), 6.96 - 6.94 (m, 1H), 4.58 (s, 2H), 3.73 - 3.67 (m, 4H), 3.15 - 3.14 (m, 4H), 2.70 (t, *J* = 6.8 Hz, 2H), 2.66 - 2.63 (m, 6H).

### Example 158 Synthesis of Compound A159:

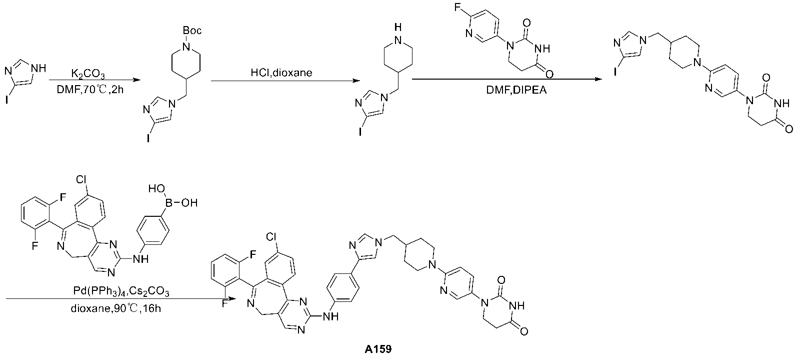

Compound **A159** (5.0 mg, yield 31%) was synthesized according to the synthesis method of compound **A107** described in **Example 107.** LCMS (ESI) m/z = 785.3 [M+H]⁺. ¹H NMR (400 MHz, DMSO-*d*₆) δ 10.32 (s, 1H), 9.86 (s, 1H), 8.66 (s, 1H), 8.30 (d, *J =* 8.4 Hz, 1H), 8.04 (d, *J =* 2.8 Hz, 1H), 7.89 - 7.86 (m, 1H), 7.81 - 7.79 (m, 2H), 7.70 - 7.68 (m, 3H), 7.63 (s, 1H), 7.55 (s, 1H), 7.49 - 7.46 (m, 1H), 7.32 (d, *J* = 2.0 Hz, 1H), 7.19 - 7.15 (m, 2H), 6.85 (d, *J* = 9.2 Hz, 1H), 4.32 - 4.28 (m, 2H), 3.90 (d, *J =* 7.2 Hz, 2H), 3.70 - 7.67 (m, 2H), 2.83 - 2.77 (m, 2H), 2.71 - 2.67 (m, 2H), 2.10 - 1.97 (m, 1H), 1.59 - 1.56 (m, 2H), 1.23 - 1.19 (m, 2H).

### Example 159 Synthesis of Compound A160:

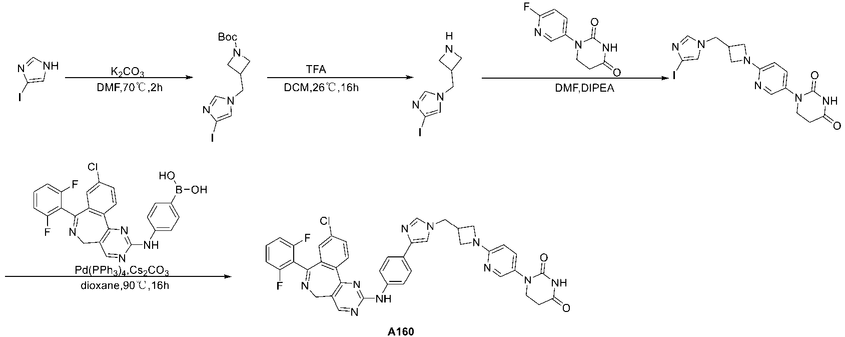

Compound **A160** (10.0 mg, yield 24.5%) was synthesized according to the synthesis method of compound **A107** described in **Example 107.** LCMS (ESI) m/z = 757.3 [M+H]⁺. ¹H NMR (400 MHz, DMSO-*d*₆) δ 10.35 (s, 1H), 9.90 (s, 1H), 8.70 (s, 1H), 8.06 (d, *J* = 2.8 Hz, 1H), 7.93 - 7.91 (m, 1H), 7.84 - 7.82 (m, 2H), 7.77 - 7.71 (m, 3H), 7.64 (s, 1H), 7.55 (s, 1H), 7.49 - 7.46 (m, 2H), 7.35 (d, *J =* 2.0 Hz, 1H), 7.20 - 7.17 (m, 2H), 6.46 (d, *J* = 8.8 Hz, 1H), 4.32 (d, *J* = 7.6 Hz, 2H), 4.08 - 4.04 (m, 2H), 3.81 - 3.78 (m, 2H), 3.73 - 3.69 (m, 2H), 2.74 - 2.73 (m, 2H).

### Example 160 Synthesis of Compound A161:

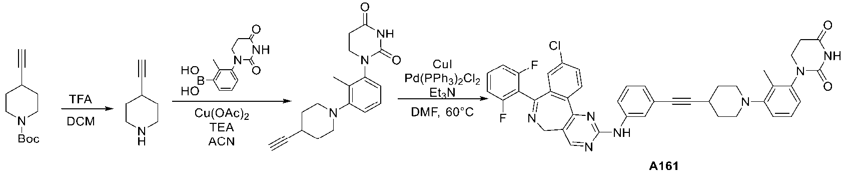

Compound **A161** (2.0 mg, yield 7.5%) was synthesized according to the synthesis method of compound **A6** described in **Example 6.** LCMS (ESI) m/z = 742.2 [M+H]⁺.¹H NMR (400 MHz, DMSO-*d*₆) δ 10.30 (s, 1H), 9.96 (s, 1H), 8.70 (s, 1H), 8.48 (s, 1H), 8.28 (d, *J =* 8.4 Hz, 1H), 7.95 - 7.94 (m, 1H), 7.87 - 7.78 (m, 2H), 7.58 - 7.50 (m, 1H), 7.35 - 7.26 (m, 2H), 7.19 - 7.14 (m, 3H), 7.03 - 6.95 (m, 3H), 3.53 - 3.47 (m, 1H), 3.06 - 3.03 (m, 2H), 2.83 - 2.64 (m, 4H), 2.33 - 2.31 (m, 1H), 2.12 (s, 3H), 2.07 - 1.98 (m, 3H), 1.85 - 1.79 (m, 2H).

### Example 161 Synthesis of Compound A162:

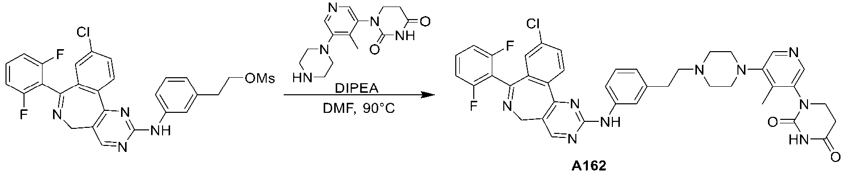

Compound **A162** (20.0 mg, yield 29.6%, yellow solid) was synthesized according to the synthesis method of compound **A101** described in **Example 101.** LCMS (ESI) m/z = 748.2 [M+H]⁺. ¹H NMR (400 MHz, DMSO-*d*₆) δ 9.81 (s, 1H), 8.66 (s, 1H), 8.34 (s, 3H), 8.29 (d, *J =* 8.4 Hz, 1H), 8.20 (s, 2H), 7.86 (dd, *J*₁ = 8.4 Hz, *J*₂ = 2.2 Hz, 1H), 7.76 (s, 1H), 7.63 - 7.60 (m, 1H), 7.57 - 7.48 (m, 1H), 7.32 (d, *J* = 2.2 Hz, 1H), 7.23 (t, *J =* 7.8 Hz, 1H), 7.17 - 7.13 (m, 2H), 6.88 (d, *J =* 7.6 Hz, 1H), 2.96 - 2.93 (m, 4H), 2.82 - 2.60 (m, 10H), 2.13 (s, 3H).

### Example 162 Synthesis of Compound A163:

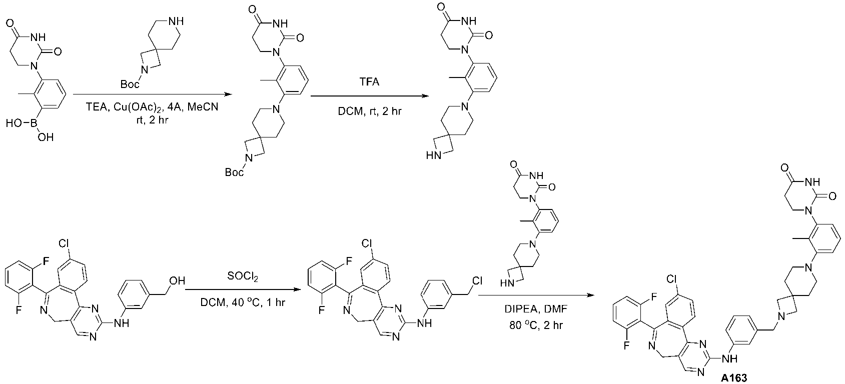

Compound **A163** (3 mg, yield 9.3%) was synthesized according to the synthesis method of compound **A101** described in **Example 101.** LCMS (ESI) m/z = 773.4 [M+H]⁺. ¹H NMR (400 MHz, DMSO-*d*₆) δ 10.33 (s, 1H), 9.86 (s, 1H), 8.70 (s, 1H), 8.32 (d, *J =* 8.4 Hz, 1H), 7.91 - 7.86 (m, 2H), 7.68 - 7.62 (m, 1H), 7.60 - 7.51 (m, 1H), 7.36 - 7.32 (m, 1H), 7.27 (t, *J =* 7.6 Hz, 1H), 7.22 - 7.13 (m, 3H), 6.99 - 6.88 (m, 3H), 3.79 - 3.70 (m, 1H), 3.63 (s, 2H), 3.56 - 3.47 (m, 1H), 3.04 (s, 4H), 2.83 - 2.64 (m, 6H), 2.09 (s, 3H), 1.84 - 1.73 (m, 4H).

### Example 163 Synthesis of Compound A164:

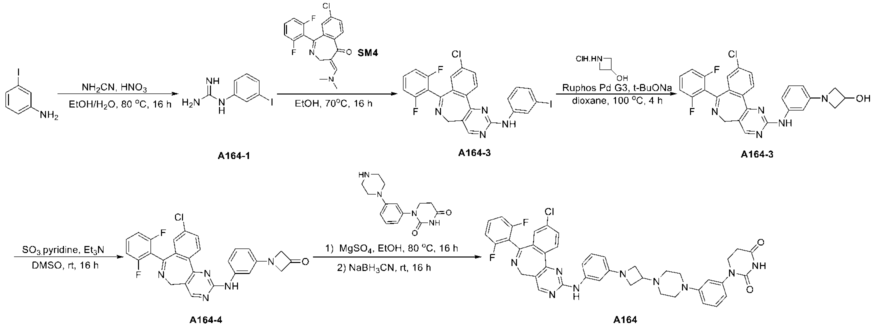

### Step 1:

To a solution of compound p-iodoaniline (10 g, 45.66 mmol) in ethanol (70 mL), a solution of 66% nitric acid (4.58 g, 47.94 mmol, 3.25 mL) and cyanamide (3.84 g, 91.31 mmol) in water (4 mL) was slowly added at 0°C. The reaction mixture was heated to 80°C and stirred for 16 h. The reaction mixture was concentrated under reduced pressure to remove ethanol (50 mL), the residue was stirred at 0°C for 1 h and filtered, the resulting solid was washed with a small amount of cold ethanol, and dried by an oil pump to give a light yellow solid **A164-1** (7.93 g, yield 67%). LCMS (ESI) m/z = 262.2 [M+H]⁺.

### Step 2:

To a solution of compound **A164-1** (868 mg, 3.33 mmol) in ethanol (14 mL) were added **SM4** (1 g, 2.77 mmol) and potassium carbonate (958 mg, 6.93 mmol) at room temperature. The reaction mixture was heated to 70°C and stirred for 16 h. After the addition of water, the reaction mixture was extracted with ethyl acetate, washed with saturated brine, dried over anhydrous sodium sulfate, concentrated under reduced pressure, and the residue was separated for purification by silica gel column chromatography to give a brown solid **A164-2** (1.29 g, yield 83%). LCMS (ESI) m/z = 559.3 [M+H]⁺.

### Step 3:

To a solution of compound **A164-2** (500 mg, 0.89 mmol) in 1,4-dioxane (10 mL), 3-hydroxyazetidine hydrochloride (294 mg, 2.68 mmol), methanesulfonato(2-dicyclohexylphosphino-2',6'-diisopropoxy-1,1'-biphenyl)(2-amino-1,1'-biphenyl-2-yl)palladium(II) (75 mg, 0.09 mmol) and sodium tert-butoxide (258 mg, 2.68 mmol) were added at room temperature. The reaction mixture was heated to 100°C under argon protection and stirred for 4 h. The reaction mixture was filtered, the filtrate was concentrated under reduced pressure, and the residue was separated for purification by silica gel column chromatography to give a yellow solid **A164-3** (210 mg, yield 47%). LCMS (ESI) m/z = 504.2 [M+H]⁺.

### Step 4:

At room temperature, a solution of sulfur trioxide pyridine complex (632 mg, 3.97 mmol) in dimethyl sulfoxide (0.5 mL) was added to a solution of compound **A164-3** (200 mg, 0.40 mmol) and triethylamine (1.2 g, 11.91 mmol) in dimethyl sulfoxide (4 mL), and the reaction mixture was stirred at room temperature for 16 h under argon protection. After the addition of water, the reaction mixture was extracted with dichloromethane, washed with saturated brine, dried over anhydrous sodium sulfate, concentrated under reduced pressure, and the residue was separated for purification by silica gel column chromatography to give a yellow solid **A164-4** (100 mg, yield 50%). LCMS (ESI) m/z = 502.4 [M+H]⁺.

### Step 5:

At room temperature, 1-(3-(piperazin-1-yl)phenyl)dihydropyrimidine-2,4(1H,3H)-dione (26 mg, 0.09 mmol) and magnesium sulfate (224 mg, 1.86 mmol) were added to a solution of compound **A164-4** (56 mg, 0.11 mmol) in ethanol (2 mL). The reaction mixture was heated to 80°C under argon protection and stirred for 16 h. The mixture was cooled to room temperature. With sodium cyanoborohydride (18 mg, 0.28 mmol) added, the reaction mixture was stirred at room temperature for 16 h under argon protection. After the addition of water, the reaction mixture was extracted with dichloromethane, washed with saturated brine, dried over anhydrous sodium sulfate, and concentrated under reduced pressure. The residue was separated for purification by reverse phase chromatography (acetonitrile/10 mM ammonium bicarbonate solution) to give a yellow solid **A164** (5 mg, yield 7%). LCMS (ESI) m/z = 760.4 [M+H]⁺. ¹H NMR (400 MHz, DMSO-*d*₆) δ 10.37 (s, 1H), 9.83 (s, 1H), 8.69 (s, 1H), 8.33 (d, *J =* 8.8 Hz, 1H), 7.97 - 7.82 (m, 1H), 7.64 - 7.50 (m, 1H), 7.36 (d, *J =* 2.0 Hz, 1H), 7.33 - 7.25 (m, 3H), 7.22 - 7.16 (m, 3H), 7.13 (s, 1H), 7.01 (s, 2H), 6.96 - 6.90 (m, 1H), 6.89 - 6.81 (m, 1H), 6.27 - 6.08 (m, 1H), 4.61 - 4.55 (m, 1H), 4.35 - 4.20 (m, 1H), 3.79 (t, *J* = 6.8 Hz, 2H), 2.73 (t, *J =* 6.4 Hz, 2H).

### Example 164 Synthesis of Compound A165:

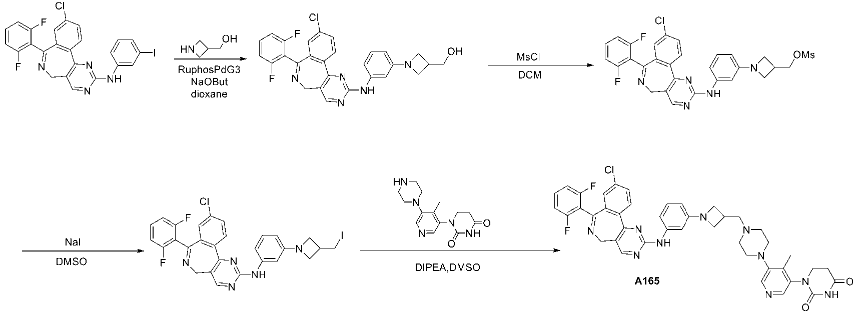

Compound **A165** (5 mg, yield 7%) was synthesized according to the synthesis method of compound **A141** described in **Example 140.** LCMS (ESI) m/z = 789.2 [M+H]⁺. ¹H NMR (400 MHz, DMSO-*d*₆) δ 10.45 (s, 1H), 9.74 (s, 1H), 8.68 (s, 1H), 8.35 (d, *J =* 8.4 Hz, 1H), 8.23 (s, 2H), 7.89 - 7.87 (m, 1H), 7.60 - 7.52 (m, 1H), 7.37 - 7.35 (m, 2H), 7.20 - 7.15 (m, 2H), 7.10 (t, *J =* 8.0 Hz, 1H), 6.98 - 6.96 (m, 1H), 6.11 - 6.08 (m, 1H), 3.40 - 3.98 (m, 2H), 3.84 - 3.78 (m, 1H), 3.62 - 3.51 (m, 1H), 3.54 - 3.51 (m, 2H), 3.05 - 2.95 (m, 5H), 2.84 - 2.70 (m, 4H), 2.61 - 2.59 (m, 4H), 2.15 (s, 3H).

### Example 165 Synthesis of Compound A166:

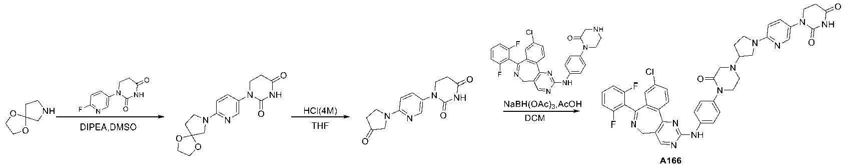

Compound **A166** (15.4 mg, yield 10.3%) was synthesized according to the synthesis method of compound **A132** described in **Example 131.** LCMS (ESI) m/z = 789.2 [M+H]⁺. ¹H NMR (400 MHz, DMSO-*d*₆) δ 10.31 (s, 1H), 9.95 (s, 1H), 8.67 (s, 1H), 8.28 (d, *J =* 8.8 Hz, 1H), 8.02 (d, *J =* 4.0 Hz, 1H), 7.89 - 7.80 (m, 3H), 7.59 - 7.51 (m, 1H), 7.49 - 7.45 (m, 1H), 7.32 (d, *J =* 2.2 Hz, 1H), 7.30 - 7.25 (m, 2H), 7.17 (t, *J =* 8.0 Hz, 2H), 6.51 (d, *J =* 8.0 Hz, 1H), 3.80 - 3.57 (m, 6H), 3.41 - 3.35 (m, 2H), 3.28 - 3.21 (m, 2H), 3.14 - 3.05 (m, 1H), 2.92 - 2.85 (m, 2H), 2.70 (t, *J =* 8.0 Hz, 2H), 2.31 - 2.20 (m, 1H), 1.98 - 1.87 (m, 1H).

### Example 166 Synthesis of Compound A167:

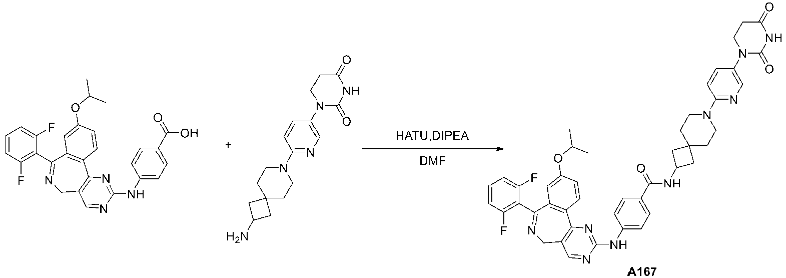

Compound **A167** (12.4 mg, yield 50.9%) was synthesized according to the synthesis method of compound **A1** described in **Example 1.** LCMS (ESI) m/z = 812.6 [M+H]⁺. ¹H NMR (400 MHz, DMSO-*d*₆) δ 10.32 (s, 1H), 10.04 (s, 1H), 8.65 (s, 1H), 8.42 (d, *J =* 8.0 Hz, 1H), 8.25 (d, *J =* 8.0 Hz, 1H), 8.05 - 8.03 (m, 1H), 7.94 -7.90 (m, 2H), 7.86 - 7.81 (m, 2H), 7.56 - 7.45 (m, 2H), 7.40 - 7.36 (m, 1H), 7.20 - 7.11 (m, 2H), 6.85 (d, *J = 8.0* Hz, 1H), 6.66 (d, *J =* 4.0 Hz, 1H), 4.70 - 4.63 (m, 1H), 4.51 - 4.43 (m, 1H), 3.73 - 3.66 (m, 2H), 3.55 - 3.48 (m, 2H), 3.48 - 3.40 (m, 2H), 2.73 - 2.66 (m, 2H), 2.27 - 2.21 (m, 2H), 1.93 - 1.85 (m, 2H), 1.67 - 1.62 (m, 2H), 1.61 - 1.55 (m, 2H), 1.23 (d, *J =* 6.0 Hz, 6H).

### Example 167 Synthesis of Compound A168:

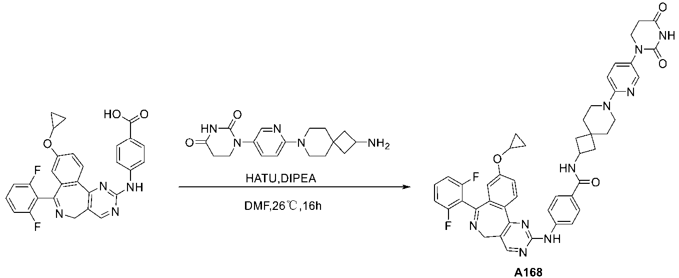

Compound **A168** (96 mg, yield 26%) was synthesized according to the synthesis method of compound **A1** described in **Example 1.** LCMS (ESI) m/z = 810.2 [M+H]⁺. ¹H NMR (400 MHz, DMSO-*d*₆) δ 10.35 (s, 1H), 10.08 (s, 1H), 8.70 (s, 1H), 8.46 (d, *J =* 7.6 Hz, 1H), 8.32 (d, *J =* 8.8 Hz, 1H), 8.07 (d, *J =* 2.8 Hz, 1H), 7.97 - 7.95 (m, 2H), 7.88 - 7.86 (m, 2H), 7.57 - 7.54 (m, 2H), 7.53 - 7.49 (m, 1H), 7.21 - 7.17 (m, 1H), 6.87 (s, 1H), 4.52 - 4.46 (m, 1H), 3.95 - 3.92 (m, 1H), 3.72 (t, *J =* 6.8 Hz, 2H), 3.57 - 3.54 (m, 2H), 3.52 - 3.48 (m, 2H), 2.88 - 2.86 (m, 2H), 2.27 (t, *J =* 10.0 Hz, 2H), 1.93 (t, *J* = 9.6 Hz, 2H), 1.68 - 1.65 (m, 2H), 1.64 - 1.62 (m, 2H), 0.76 - 0.68 (m, 4H).

### Example 168 Synthesis of Compound A169:

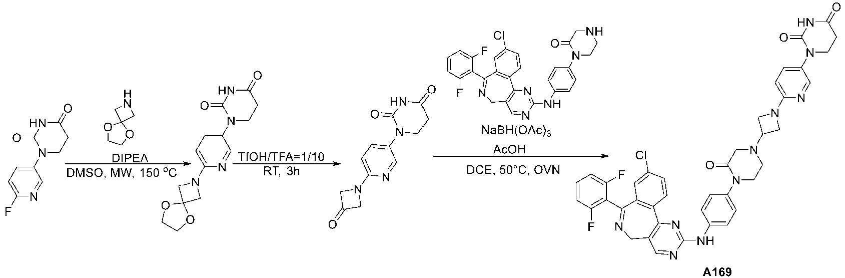

Compound **A169** (9.0 mg, yield 20.5%, yellow solid) was synthesized according to the synthesis method of compound **A132** described in **Example 131.** LCMS (ESI) m/z = 775.5 [M+H]⁺. ¹H NMR (400 MHz, DMSO-*d*₆) δ 10.33 (s, 1H), 9.94 (s, 1H), 8.67 (s, 1H), 8.28 (d, *J =* 8.4 Hz, 1H), 8.03 (d, *J =* 2.6 Hz, 1H), 7.87 - 7.79 (m, 3H), 7.59 - 7.48 (m, 2H), 7.32 (d, *J =* 2.2 Hz, 1H), 7.27 - 7.25 (m, 2H), 7.18- - 7.14 (m, 2H), 6.43 (d, *J =* 8.8 Hz, 1H), 4.05 (t, *J =* 8.0 Hz, 2H), 3.87 - 3.84 (m, 2H), 3.72 - 3.63 (m, 3H), 3.49 - 3.43 (m, 1H), 3.22 - 3.17 (m, 2H), 2.84 - 2.77 (m, 2H), 2.71 - 2.06 (m, 3H).

### Example 169 Synthesis of Compound A170:

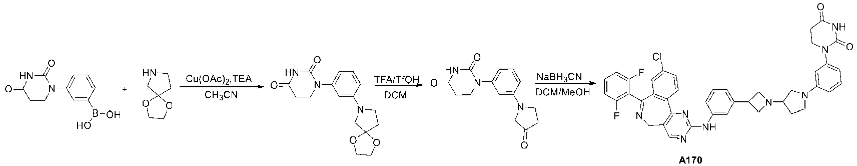

Compound **A170** (4.65 mg, yield 15.22%) was synthesized according to the synthesis method of compound **A101** described in **Example 101.** LCMS (ESI) m/z = 745.18 [M+H]⁺. ¹H NMR (400 MHz, DMSO-*d*₆) δ 10.26 (s, 1H), 9.84 (s, 1H), 8.66 (s, 1H), 8.29 (d, *J =* 8.4 Hz, 1H), 7.99 (s, 1H), 7.88 (dd, *J*₁ = 8.4 Hz, *J*₂ = 2.0 Hz, 1H), 7.67 - 7.42 (m, 2H), 7.33 (d, *J =* 2.4 Hz, 1H), 7.24 (t, *J =* 8.0 Hz, 1H), 7.14 (t, *J =* 8.0 Hz, 2H), 6.91 (d, *J* = 7.6 Hz, 1H), 6.55 - 6.48 (m, 1H), 6.46 - 6.42 (m, 1H), 6.40 - 6.35 (m, 1H), 3.79 - 3.64 (m, 4H), 3.64 - 3.52 (m, 1H), 3.27 - 3.15 (m, 3H), 3.14 - 3.04 (m, 2H), 3.02 - 2.90 (m, 2H), 2.68 (t, *J=* 6.8 Hz, 2H), 1.94 - 1.71 (m, 2H).

### Example 170 Synthesis of Compound A171:

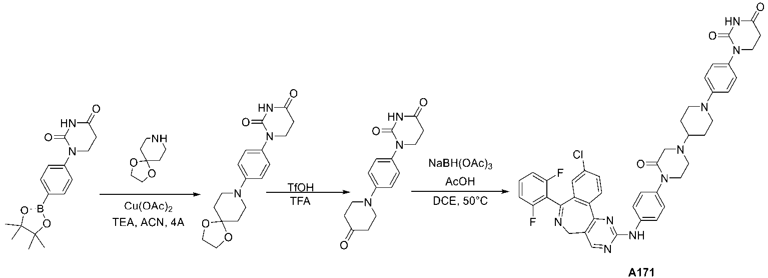

Compound **A171** (6.0 mg, yield 13.2%) was synthesized according to the synthesis method of compound **A132** described in **Example 131.** LCMS (ESI) m/z = 802.5 [M+H]⁺. ¹H NMR (400 MHz, DMSO-*d*₆) δ 10.25 (s, 1H), 9.94 (s, 1H), 8.67 (s, 1H), 8.42 (s, 2H), 8.28 (d, *J =* 8.4 Hz, 1H), 7.89 - 7.78 (m, 3H), 7.87 - 7.78 (m, 2H), 7.62 - 7.48 (m, 1H), 7.32 (d, *J =* 2.2 Hz, 1H), 7.27 (d, *J =* 8.8 Hz, 2H), 7.18 - 7.13 (m, 3H), 6.96 (d, *J =* 9.0 Hz, 2H), 3.78 - 3.67 (m, 4H), 3.62 - 3.60 (m, 2H), 2.90 - 2.87 (m, 2H), 2.7 - 2.66 (m, 4H), 1.94 - 1.91 (m, 2H), 1.59 - 1.50 (m, 2H).

### Example 171 Synthesis of Compound A172:

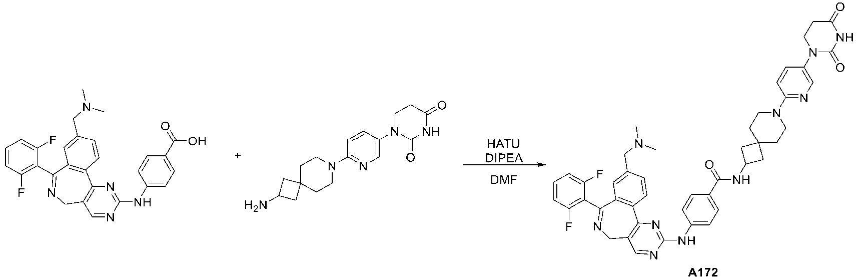

Compound **A172** (15.0 mg, yield 46.2%) was synthesized according to the synthesis method of compound **A1** described in **Example 1.** LCMS (ESI) m/z = 811.6 [M+H]. ¹H NMR (400 MHz, DMSO-*d*₆) δ 10.32 (s, 1H), 10.09 (s, 1H), 8.70 (s, 1H), 8.42 (d, *J =* 7.4 Hz, 1H), 8.26 (d, *J =* 8.0 Hz, 1H), 8.04 (d, *J =* 2.8 Hz, 1H), 7.93 (d, *J =* 8.8 Hz, 2H), 7.84 (d, *J =* 8.8 Hz, 2H), 7.69 - 7.67 (m, 1H), 7.57 - 7.43 (m, 2H), 7.22 (s, 1H), 7.17 - 7.12 (m, 2H), 6.85 (d, *J =* 9.0 Hz, 1H), 4.50 - 4.42 (m, 1H), 3.69 (t, *J* = 6.6 Hz, 2H), 3.52 - 3.50 (m, 2H), 3.45 - 3.43 (m, 4H), 2.72 (t, *J =* 6.6 Hz, 2H ), 2.26 - 2.21 (m, 2H), 2.08 (s, 6H), 1.91 - 1.86 (m, 2H), 1.65 - 1.57 (m, 4H).

### Example 172 Synthesis of Compound A173:

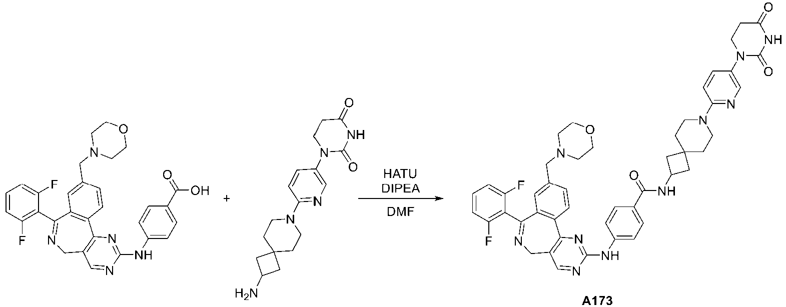

Compound **A173** (20.3 mg, yield 64.4%) was synthesized according to the synthesis method of compound **A1** described in **Example 1.** LCMS (ESI) m/z = 853.7 [M+H]. ¹H NMR (400 MHz, DMSO-*d*₆) δ 10.32 (s, 1H), 10.08 (s, 1H), 8.70 (s, 1H), 8.42 (d, *J =* 7.4 Hz, 1H), 8.27 (d, *J =* 8.0 Hz, 1H), 8.04 (d, *J =* 2.8 Hz, 1H), 7.93 (d, *J =* 8.8 Hz, 2H), 7.84 (d, *J =* 8.8 Hz, 2H), 7.71 - 7.68 (m, 1H), 7.57 - 7.45 (m, 2H), 7.28 (s, 1H), 7.17 - 7.12 (m, 2H), 6.85 (d, *J = 9.0* Hz, 1H), 4.50 - 4.40 (m, 1H), 3.69 (t, *J* = 6.6 Hz, 2H), 3.55 - 3.43 (m, 10H), 2.69 (t, *J* = 6.6 Hz, 2H), 2.30 - 2.21 (m, 6H), 1.91 - 1.86 (m, 2H), 1.65 - 1.57 (m, 4H).

### Example 173 Synthesis of Compound A174:

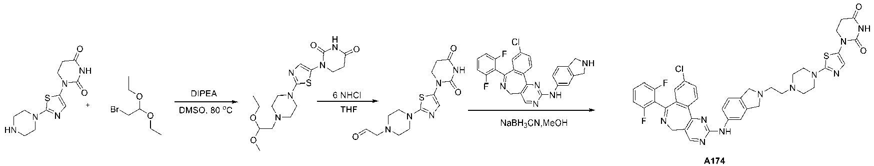

Compound **A174** (6.0 mg, yield 13.2%) was synthesized according to the synthesis method of **compound A132** described in **Example 131.** LCMS (ESI) m/z = 781.2 [M+H]⁺. ¹H NMR (400 MHz, DMSO-*d*₆) δ 10.61 (s, 1H), 9.84 (s, 1H), 8.67 (s, 1H), 8.28 (d, *J =* 8.4 Hz, 1H), 7.89 - 7.88 (m, 1H), 7.74 (s, 1H), 7.62 - 7.55 (m, 2H), 7.34 (d, *J =* 2.2 Hz, 1H), 7.21 - 7.17 (m, 3H), 7.02 (s, 1H), 3.91 (bs, 2H), 3.86 (bs, 2H), 3.81 (t, *J* = 6.6 Hz, 2H), 2.88 - 2.85 (m, 2H), 2.75 (t, *J* = 6.6 Hz, 2H), 2.59 - 2.57 (m, 6H).

### Example 174 Synthesis of Compound A175:

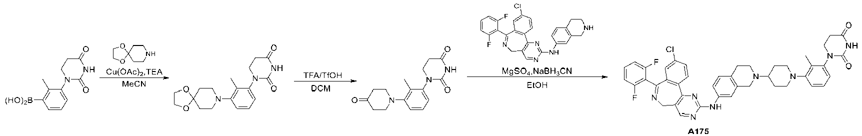

Compound **A175** (13.7 mg, yield 21.6%) was synthesized according to the synthesis method of compound **A132** described in **Example 131.** LCMS (ESI) m/z = 773.4 [M+H]⁺. ¹H NMR (400 MHz, DMSO-*d*₆) *δ* 10.31 (s, 1H), 9.72 (s, 1H), 8.63 (s, 1H), 8.26 (d, *J =* 8.8 Hz, 1H), 7.88 - 7.84 (m, 1H), 7.59 - 7.48 (m, 3H), 7.31 (d, *J =* 4.0 Hz, 1H), 7.21 - 7.13 (m, 3H), 7.06 - 7.00 (m, 2H), 6.98 - 6.94 (m, 1H), 3.78 - 3.70 (m, 3H), 3.56 - 3.48 (m, 1H), 3.16 - 3.08 (m, 2H), 2.86 - 2.55 (m, 9H), 2.13 (s, 3H), 1.99 - 1.91 (m, 2H), 1.79 - 1.68 (m, 2H).

### Example 175 Synthesis of Compound A176:

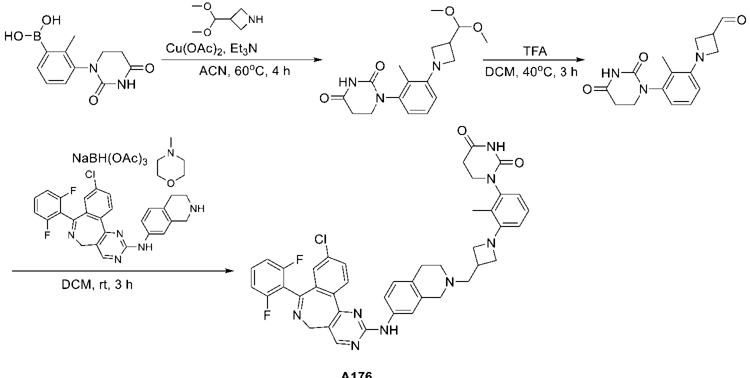

Compound **A176** (8 mg, yield 13%) was synthesized according to the synthesis method of compound **A132** described in **Example 131.** LCMS (ESI) m/z = 759.4 [M+H]⁺. ¹H NMR (400 MHz, DMSO-*d*₆) δ 10.30 (s, 1H), 9.76 (s, 1H), 8.66 (s, 1H), 8.30 (d, *J=* 8.4 Hz, 1H), 7.97 - 7.81 (m, 1H), 7.65 - 7.48 (m, 3H), 7.34 (d, *J* = 2.4 Hz, 1H), 7.26 - 7.14 (m, 2H), 7.14 - 7.02 (m, 2H), 6.73 (d, *J* = 7.6 Hz, 1H), 6.49 (d, *J =* 8.0 Hz, 1H), 4.17 - 3.98 (m, 2H), 3.82 - 3.68 (m, 1H), 3.67 - 3.54 (m, 4H), 3.54 - 3.43 (m, 1H), 3.07 - 2.92 (m, 1H), 2.87 - 2.62 (m, 8H), 2.01 (s, 3H).

### Example 176 Synthesis of Compound A177:

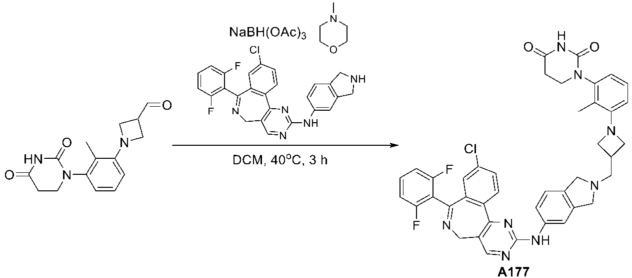

Compound **A177** (12 mg, yield 19%) was synthesized according to the synthesis method of compound A132 described in **Example 131.** LCMS (ESI) m/z = 745.2 [M+H]⁺. ¹H NMR (400 MHz, DMSO-*d*₆) δ 10.30 (s, 1H), 9.85 (s, 1H), 8.67 (s, 1H), 8.29 (d, *J =* 8.4 Hz, 1H), 7.95 - 7.85 (m, 1H), 7.80 - 7.70 (m, 1H), 7.68 - 7.51 (m, 2H), 7.34 (d, *J =* 2.4 Hz, 1H), 7.26 - 7.15 (m, 3H), 7.15 - 7.07 (m, 1H), 6.73 (d, *J = 7.6* Hz, 1H), 6.50 (d, *J =* 8.0 Hz, 1H), 4.16 - 4.02 (m, 2H), 3.98 - 3.81 (m, 4H), 3.81 - 3.68 (m, 1H), 3.69 - 3.55 (m, 2H), 3.55 - 3.44 (m, 1H), 3.02 - 2.86 (m, 3H), 2.86 - 2.73 (m, 1H), 2.73 - 2.62 (m, 1H), 2.01 (s, 3H).

### Example 177 Synthesis of Compound A178:

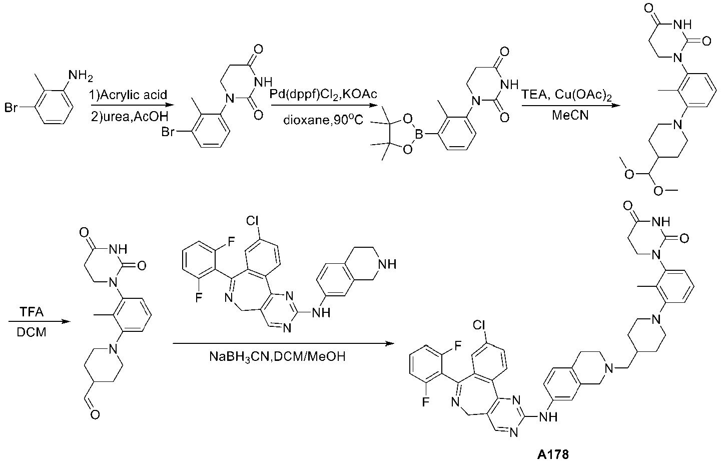

Compound **A178** (8.62 mg, yield 26.71%) was synthesized according to the synthesis method of compound **A132** described in **Example 131.** LCMS (ESI) m/z = 787.1 [M+H]⁺. ¹H NMR (400 MHz, DMSO-*d*₆) δ 10.29 (s, 1H), 9.72 (s, 1H), 8.63 (s, 1H), 8.26 (d, *J =* 8.4 Hz, 1H), 7.86 (dd, *J*₁ = 8.4 Hz, *J*₂ = 2.0 Hz, 1H), 7.60 - 7.48 (m, 3H), 7.31 (d, *J =* 2.0 Hz, 1H), 7.17 (q, *J =* 7.4, 6.9 Hz, 3H), 7.02 (dd, *J*₁ = 12.9 Hz, *J*₂ = 8.1 Hz, 2H), 6.95 (d, *J =* 8.0 Hz, 1H), 3.79 - 3.67 (m, 1H), 3.58 - 3.45 (m, 3H), 3.08 - 3.01 (m, 2H), 2.79 - 2.57 (m, 8H), 2.43 - 2.37 (m, 2H), 1.90 - 1.82 (m, 2H), 1.83 - 1.72 (m, 1H), 1.41 - 1.26 (m, 2H).

### Example 178 Synthesis of Compound A179:

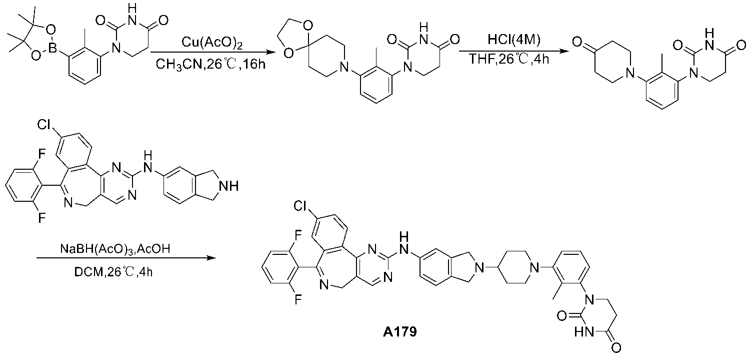

Compound **A179** (5.0 mg, yield 15.6%) was synthesized according to the synthesis method of compound **A132** described in **Example 131.** LCMS (ESI) m/z = 759.3 [M+H]⁺. ¹H NMR (400 MHz, DMSO-*d*₆) δ 10.35 (s, 1H), 10.08 (s, 1H), 9.85 (s, 1H), 8.68 (s, 1H), 8.30 (d, *J =* 8.8 Hz, 1H), 7.92 - 7.89 (m, 1H), 7.79 (s, 1H), 7.63 - 7.56 (m, 2H), 7.34 (d, *J =* 8.4 Hz, 1H), 7.24 - 7.18 (m, 4H), 7.06 (d, *J =* 3.2 Hz, 1H), 6.99 *(d, J= 3.6* Hz, 1H), 3.97 (s, 2H), 3.92 (s, 2H), 3.79 - 3.74 (m, 1H), 3.57 - 3.51 (m, 2H), 3.15 - 3.10 (m, 2H), 2.80 - 2.70 (m, 4H), 2.15 (s, 3H), 2.10 - 2.05 (m, 2H).

### Example 179 Synthesis of Compound A180:

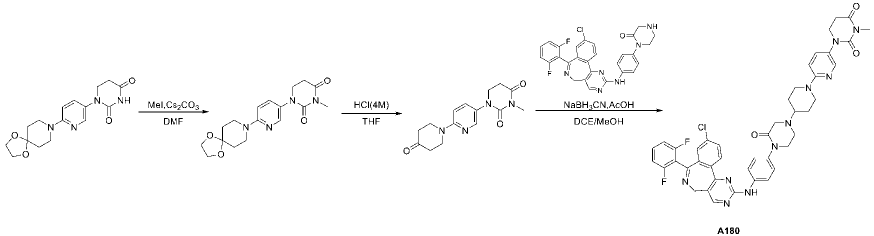

Compound **A180** (16.2 mg, yield 10.5%) was synthesized according to the synthesis method of compound **A132** described in **Example 131.** LCMS (ESI) m/z = 817.5 [M+H]⁺. ¹H NMR (400 MHz, DMSO-*d*₆) δ 9.94 (s, 1H), 8.67 (s, 1H), 8.28 (d, *J =* 8.8 Hz, 1H), 8.08 - 8.05 (m, 1H), 7.88 - 7.84 (m, 1H), 7.84 - 7.78 (m, 2H), 7.59 - 7.52 (m, 1H), 7.51 - 7.48 (m, 1H), 7.33 - 7.31 (m, 1H), 7.29 - 7.23 (m, 2H), 7.21 - 7.10 (m, 2H), 6.91 - 6.85 (m, 1H), 4.36 - 4.28 (m, 2H), 3.68 (t, *J =* 8.0 Hz, 2H), 3.62 - 3.57 (m, 2H), 3.31 - 3.29 (m, 2H), 3.04 (s, 3H), 2.90 - 2.80 (m, 6H), 2.62 - 2.53 (m, 1H), 1.95 - 1.86 (m, 2H), 1.48 - 1.36 (m, 2H).

### Example 180 Synthesis of Compound A181:

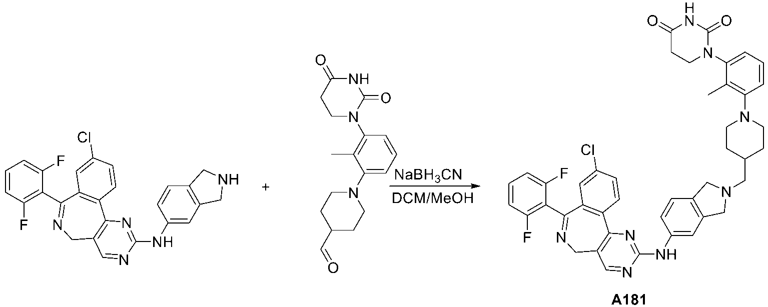

Compound **A181** (10.65 mg, yield 32.63%) was synthesized according to the synthesis method of compound **A132** described in **Example 131.** LCMS (ESI) m/z = 773.1 [M+H]⁺. ¹H NMR (400 MHz, DMSO-*d*₆) δ 10.30 (s, 1H), 9.82 (s, 1H), 8.64 (s, 1H), 8.26 (d, *J* = 8.4 Hz, 1H), 7.87 (dd, *J*₁ = 8.4 Hz, *J*₂ = 2.0 Hz, 1H), 7.76 - 7.71 (m, 1H), 7.62 - 7.48 (m, 2H), 7.32 (d, *J =* 2.0 Hz, 1H), 7.25 - 7.13 (m, 4H), 7.01 (d, *J =* 8.0 Hz, 1H), 6.95 (d, *J* = 8.0 Hz, 1H), 3.93 - 3.79 (m, 4H), 3.79 - 3.68 (m, 1H), 3.56 - 3.45 (m, 1H), 3.06 (d, *J* = 12.0 Hz, 2H), 2.81 - 2.55 (m, 6H), 2.11 (s, 3H), 1.93 - 1.86 (m, 2H), 1.72 - 1.61 (m, 1H), 1.41 - 1.27 (m, 2H).

### Example 181 Synthesis of Compound A182:

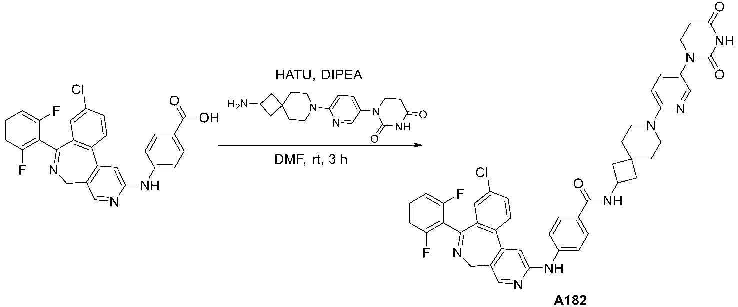

Compound **A182** (12 mg, yield 48%) was synthesized according to the synthesis method of compound **A1** described in **Example 1.** LCMS (ESI) m/z = 787.4 [M+H]⁺. ¹H NMR (400 MHz, DMSO-*d*₆) δ 10.35 (s, 1H), 9.55 (s, 1H), 8.46 - 8.32 (m, 2H), 8.12 - 8.03 (m, 1H), 7.93 (d, *J =* 8.4 Hz, 1H), 7.89 - 7.73 (m, 5H), 7.64 - 7.43 (m, 2H), 7.37 - 7.28 (m, 1H), 7.28 - 7.12 (m, 3H), 6.88 (d, *J =* 9.2 Hz, 1H), 4.99 (d, *J* = 11.2 Hz, 1H), 4.57- 4.30 (m, 1H), 3.93 (d, *J =* 11.2 Hz, 1H), 3.72 (t, *J =* 6.8 Hz, 1H), 3.62 - 3.46 (m, 4H), 2.72 (t, *J=* 6.4 Hz, 2H), 2.34 - 2.13 (m, 2H), 2.03 - 1.82 (m, 2H), 1.75 - 1.50 (m, 4H).

### Example 182 Synthesis of Compound A183:

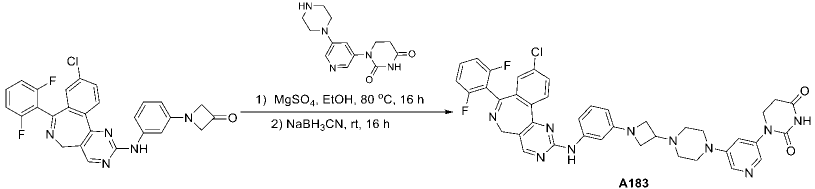

Compound **A183** (2 mg, yield 6%) was synthesized according to the synthesis method of compound **A132** described in **Example 131.** LCMS (ESI) m/z = 761.3 [M+H]⁺. ¹H NMR (400 MHz, DMSO-*d*₆) δ 10.46 (s, 1H), 9.73 (s, 1H), 8.68 (s, 1H), 8.33 (d, *J =* 8.4 Hz, 1H), 8.24 - 8.13 (m, 1H), 8.07 - 7.96 (m, 1H), 7.94 - 7.83 (m, 1H), 7.64 - 7.50 (m, 1H), 7.40 - 7.29 (m, 2H), 7.26 - 7.16 (m, 3H), 7.16 - 7.02 (m, 2H), 6.18 - 6.07 (m, 1H), 3.99 (t, *J =* 6.8 Hz, 2H), 3.83 (t, *J =* 6.8 Hz, 2H), 3.70 - 3.64 (m, 2H), 3.32 - 3.22 (m, 6H), 2.74 (t, *J* = 6.8 Hz, 2H), 2.57 - 2.55 (m, 1H).

### Example 183 Synthesis of Compound A184:

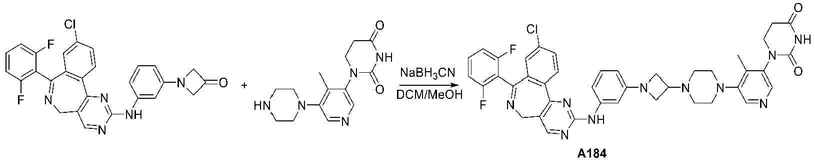

Compound **A184** (10.65 mg, yield 32.63%) was synthesized according to the synthesis method of compound **A132** described in **Example 131.** LCMS (ESI) m/z = 775.3 [M+H]⁺. ¹H NMR (400 MHz, DMSO-*d₆*) δ 10.46 (s, 1H), 9.72 (s, 1H), 8.63 (s, 1H), 8.34 (d, *J =* 8.6 Hz, 1H), 8.23 (s, 2H), 7.86 (dd, *J*₁ = 8.8 Hz, *J*₂ = 2.4 Hz, 1H), 7.60 - 7.48 (m, 1H), 7.31 (d, *J =* 2.4 Hz, 1H), 7.23 - 7.60 (m, 5H), 6.15 - 6.13 (m, 1H), 4.00 - 3.97 (m, 2H), 3.84 - 3.78 (m, 1H), 3.68 - 3.57 (m, 3H), 3.44 - 3.25 (m, 1H), 3.01 (bs, 4H), 2.81 - 2.70 (m, 2H), 2.57 - 2.55 (bs, 4H), 2.10 (s, 3H).

### Example 184 Synthesis of Compound A185:

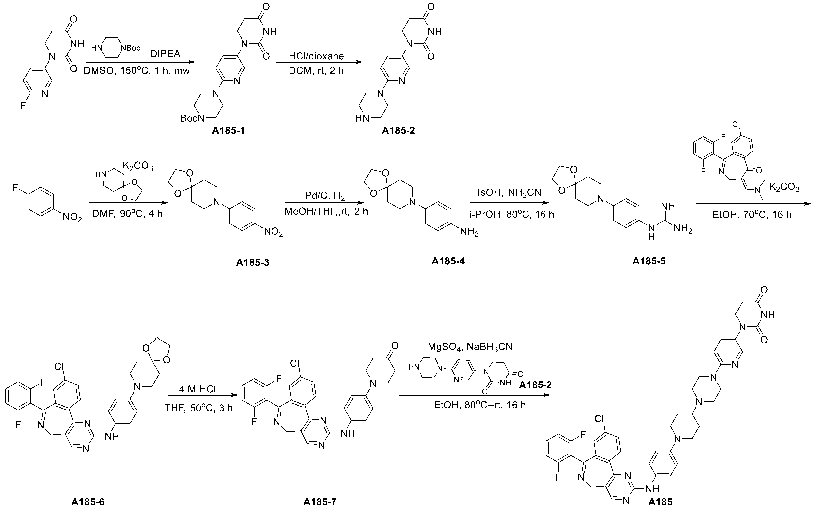

### Step 1:

At room temperature, 1-Boc-piperazine (196 mg, 1.05 mmol) and DIPEA (618 mg, 4.78 mmol) were added to a solution of compound 1-(6-fluoropyridin-3-yl)dihydropyrimidine-2,4(1H,3H)-dione (200 mg, 0.96 mmol) in dimethyl sulfoxide (2 mL). The reaction mixture was stirred at 150°C for 1 h under microwave conditions. After the addition of water, the reaction mixture was extracted with dichloromethane, washed with saturated brine, dried over anhydrous sodium sulfate, concentrated under reduced pressure, and the residue was separated for purification by silica gel column chromatography to give a brown solid **A185-1** (86 mg, yield 24%). LCMS (ESI) m/z = 376.3 [M+H]⁺.

### Step 2:

HCl (4 M in dioxane, 0.5 mL) was added to a solution of compound **A185-1** (86 mg, 0.23 mmol) in dichloromethane (1 mL) at room temperature. The reaction mixture was stirred at room temperature for 2 h. The reaction mixture was concentrated under reduced pressure, and saturated aqueous sodium bicarbonate solution (10 mL) was added to the residue. The mixture was extracted with dichloromethane, washed with saturated brine, dried over anhydrous sodium sulfate, and concentrated under reduced pressure to give a brownish-yellow solid **A185-2** (60 mg, yield 95%). LCMS (ESI) m/z = 276.2 [M+H]⁺.

### Step 3:

To a solution of compound p-fluoronitrobenzene (2 g, 14.17 mmol) in N,Ndimethylformamide (20 mL) were added 4-piperidone ethylene ketal (3.04 g, 21.26 mmol) and potassium carbonate (3.92 g, 28.35 mmol) at room temperature. The reaction mixture was heated to 90°C and stirred for 4 h. After the addition of water, the reaction mixture was extracted with ethyl acetate, washed with saturated brine, dried over anhydrous sodium sulfate, concentrated under reduced pressure, and the residue was separated for purification by silica gel column chromatography to give a brown solid **A185-3** (3.65 g, yield 97%). LCMS (ESI) m/z = 265.3 [M+H]⁺.

### Step 4:

To a mixed solution of **A185-3** (3.65 g, 13.81 mmol) in methanol (40 mL) and tetrahydrofuran (40 mL) was added palladium on carbon (10%) (wetted with approximately 55% water) (1 g) at room temperature. The reaction mixture was stirred at room temperature for 2 h under hydrogen protection. The reaction mixture was filtered, and the filtrate was concentrated under reduced pressure to give a colorless liquid **A185-4** (3.22 g, yield 100%, crude product). LCMS (ESI) m/z = 235.3 [M+H]⁺.

### Step 5:

To a solution of compound **A185-4** (1 g, 4.27 mmol) in isopropanol (30 mL) were added p-toluenesulfonic acid hydrate (1.22 g, 6.40 mmol) and cyanamide (449 mg, 10.67 mmol) at room temperature. The reaction mixture was heated to 80°C and stirred for 16 h. The reaction mixture was concentrated under reduced pressure, and the residue was separated for purification by silica gel column chromatography to give a brown solid **A185-5** (1.1 g, yield 93%). LCMS (ESI) m/z = 277.2 [M+H]⁺.

### Step 6:

To a solution of compound **A185-5** (306 mg, 1.11 mmol) in ethanol (2 mL), **SM4** (200 mg, 0.55 mmol) and potassium carbonate (192 mg, 1.39 mmol) were added at room temperature. The reaction mixture was heated to 70°C and stirred for 16 h. After the addition of water, the reaction mixture was extracted with ethyl acetate, washed with saturated brine, dried over anhydrous sodium sulfate, concentrated under reduced pressure, and the residue was separated for purification by silica gel column chromatography to give a brown solid **A185-6** (305 mg, yield 96%). LCMS (ESI) m/z = 574.1 [M+H]⁺.

### Step 7:

At room temperature, HCl (4 M, 0.6 mL) was added to a solution of compound **A185-6** (285 mg, 0.50 mmol) in tetrahydrofuran (3 mL), and the reaction mixture was heated to 50°C and stirred for 3 h. The reaction mixture was adjusted to weak alkalinity with a saturated aqueous sodium bicarbonate solution, extracted with dichloromethane, washed with saturated brine, dried over anhydrous sodium sulfate, and concentrated under reduced pressure. The residue was purified by silica gel column chromatography to give a brown solid **A185-7** (232 mg, yield 88%). LCMS (ESI) m/z = 530.2 [M+H]⁺.

### Step 8:

**A185-2** (35 mg, 0.13 mmol) and magnesium sulfate (306 mg, 2.54 mmol) were added to a solution of compound **A185-7** (81 mg, 0.15 mmol) in ethanol (2 mL) at room temperature. The reaction mixture was heated to 80°C under argon protection and stirred for 16 h. The mixture was cooled to room temperature. With sodium cyanoborohydride (24 mg, 0.38 mmol) added, the reaction mixture was stirred at room temperature for 16 h under argon protection. After the addition of water, the reaction mixture was extracted with dichloromethane, washed with saturated brine, dried over anhydrous sodium sulfate, and concentrated under reduced pressure. The residue was separated for purification by reverse phase chromatography (acetonitrile/10 mM ammonium bicarbonate solution) to give a yellow solid **A185** (5 mg, yield 5%). LCMS (ESI) m/z = 789.4 [M+H]⁺. ¹H NMR (400 MHz, DMSO-*d*₆) δ 10.37 (s, 1H), 9.61 (s, 1H), 8.61 (s, 1H), 8.28 (d, *J =* 8.4 Hz, 1H), 8.09 (d, *J =* 2.8 Hz, 1H), 7.91 - 7.82 (m, 1H), 7.68 - 7.61 (m, 2H), 7.61 - 7.48 (m, 2H), 7.33 (d, *J =* 2.0 Hz, 1H), 7.19 (t, *J =* 8.8 Hz, 2H), 7.00 - 6.90 (m, 2H), 6.86 (d, J = 8.8 Hz, 1H), 3.78 - 3.63 (m, 4H), 3.55 - 3.44 (m, 4H), 2.72 (t, *J =* 6.8 Hz, 2H), 2.69 - 2.59 (m, 6H), 2.44 - 2.29 (m, 1H), 1.97 - 1.85 (m, 2H), 1.67 - 1.52 (m, 2H).

### Example 185 Synthesis of Compound A186:

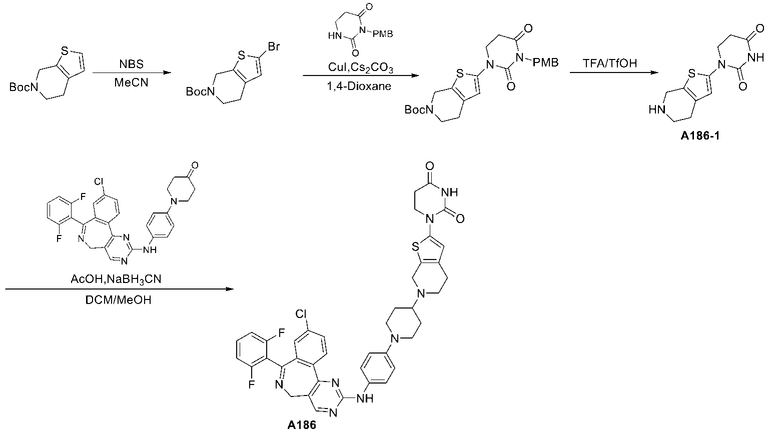

Compound **A186** (10.3 mg, yield 23.8%) was synthesized according to the synthesis method of compound **A132** described in **Example 131.** LCMS (ESI) m/z = 765.4 [M+H]⁺. ¹H NMR (400 MHz, DMSO-*d*₆) δ 10.57 (s, 1H), 9.58 (s, 1H), 8.58 (s, 1H), 8.25 (d, *J =* 8.8 Hz, 1H), 7.86 - 7.81 (m, 1H), 7.65 - 7.59 (m, 2H), 7.57 - 7.49 (m, 1H), 7.31 - 7.28 (m, 1H), 7.20 - 7.12 (m, 2H), 6.96 - 6.89 (m, 2H), 6.50 (s, 1H), 3.92 - 3.84 (m, 2H), 3.69 - 3.62 (m, 4H), 2.84 - 2.78 (m, 2H), 2.75 - 2.70 (m, 2H), 2.68 - 2.60 (m, 2H), 2.54 (bs, 3H), 1.93 - 1.85 (m, 2H), 1.68 - 1.56 (m, 2H).

### Example 186 Synthesis of Compound A187:

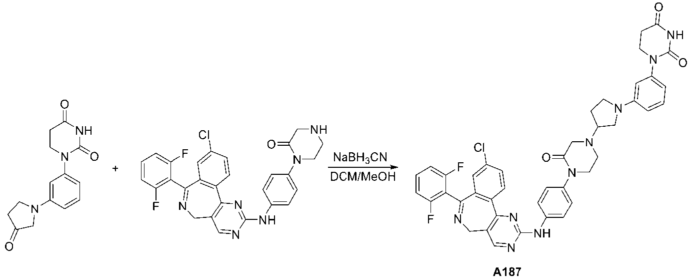

Compound **A187** (10.65 mg, yield 32.63%) was synthesized according to the synthesis method of compound **A132** described in **Example 131.** LCMS (ESI) m/z = 788.11 [M+H]⁺. ¹H NMR (400 MHz, DMSO-*d*₆) δ 10.28 (s, 1H), 9.95 (s, 1H), 8.67 (s, 1H), 8.28 (d, *J =* 8.4 Hz, 1H), 7.90 - 7.79 (m, 3H), 7.60 - 7.48 (m, 1H), 7.35 - 7.24 (m, 3H), 7.21 - 7.12 (m, 3H), 6.59 - 6.50 (m, 2H), 6.47 (d, *J =* 8.4 Hz, 1H), 3.75 (t, *J* = 6.8 Hz, 2H), 3.70 - 3.63 (m, 2H), 3.57 - 3.49 (m, 1H), 3.43 - 3.35 (m, 1H), 3.31 - 3.21 (m, 3H), 3.19 - 3.05 (m, 2H), 2.95 - 2.83 (m, 2H), 2.69 (t, *J =* 6.8 Hz, 2H), 2.31 - 2.22 (m, 1H), 1.98 - 1.87 (m, 1H).

### Example 187 Synthesis of Compound A188:

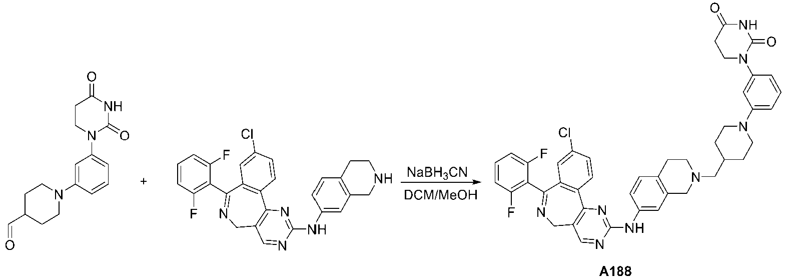

Compound **A187** (10.65 mg, yield 32.63%) was synthesized according to the synthesis method of compound **A132** described in **Example 131.** LCMS (ESI) m/z = 773.12 [M+H]⁺. ¹H NMR (400 MHz, DMSO-*d*₆) δ 10.28 (s, 1H), 9.73 (s, 1H), 8.63 (s, 1H), 8.26 (d, *J =* 8.4 Hz, 1H), 7.86 (dd, *J*₁ = 8.8 Hz, *J*₂ = 2.4 Hz, 1H), 7.59 - 7.45 (m, 3H), 7.31 (d, *J =* 2.4 Hz, 1H), 7.22 - 7.12 (m, 3H), 7.04 (d, *J =* 8.4 Hz, 1H), 6.89 - 6.85 (m, 1H), 6.84 - 6.78 (m, 1H), 6.71 - 6.65 (m, 1H), 3.78 - 3.66 (m, 4H), 3.54 (s, 2H), 2.79 - 2.63 (m, 8H), 2.34 (d, *J=* 6.8 Hz, 2H), 1.87 - 1.80 (m, 3H), 1.27 - 1.21 (m, 2H).

### Example 188 Synthesis of Compound A189:

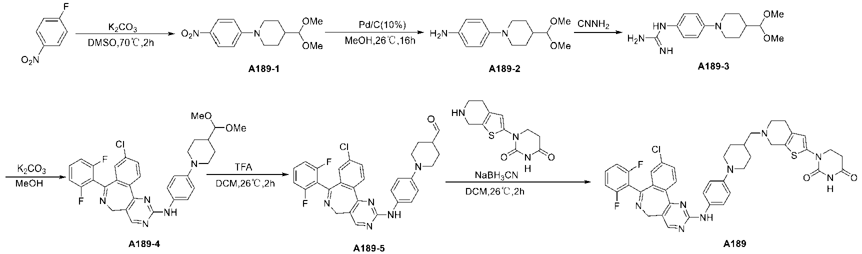

### Step 1:

At room temperature, 4-nitrofluorobenzene (1 g, 7.09 mmol) and potassium carbonate (1.96 g, 14.2 mmol) were added to a solution of compound 4-(dimethoxymethyl)piperidine (1.69 g, 10.63 mmol) in dimethyl sulfoxide (15 mL). The reaction mixture was stirred at 70°C for 2 h. After the addition of water, the reaction mixture was extracted with ethyl acetate, dried over anhydrous sodium sulfate, concentrated under reduced pressure, and the residue was separated for purification by silica gel column chromatography (ethyl acetate/petroleum ether = 1:2) to give a yellow solid **A189-1** (1.3 g, yield 65%). LCMS (ESI) m/z = 281.2 [M+H]⁺.

### Step 2:

At room temperature, 10% palladium on carbon (99 mg, 0.93 mmol) was added to a solution of compound **A189-1** (1.3 g, 4.62 mmol) in methanol (20 mL), the atmosphere was replaced with hydrogen 3 times, and the reaction mixture was stirred at 26°C for 16 h. The reaction mixture was filtered and concentrated under reduced pressure to give a brown solid **A189-2** (1.0 g, yield 86%). LCMS (ESI) m/z = 251.3 [M+H]⁺.

### Step 3:

At room temperature, an aqueous cyanamide solution (420 mg, 10.0 mmol) was slowly added dropwise to a solution of compound **A189-2** (500 mg, 2.0 mmol) in isopropanol (10 mL), and the reaction mixture was stirred at 90°C for 16 h. The reaction mixture was concentrated under reduced pressure, and the residue was separated for purification by silica gel column chromatography (methanol/dichloromethane = 1:20) to give a yellow solid **A189-3** (350 mg, yield 59%). LCMS (ESI) m/z = 293.4 [M+H]⁺.

### Step 4:

**A189-3** (195 mg, 0.66 mmol) obtained in the previous step was dissolved in methanol (5 mL), potassium carbonate (192 mg, 1.39 mmol) was added, and the mixture was stirred at 70°C for 16 h. After the reaction was completed, the mixture was directly spin-dried and purified by flash silica gel column chromatography (ethyl acetate/petroleum ether = 1:2) to give **A189-4** (250 mg, yield 76%). LCMS (ESI) m/z = 590.2 [M+H]⁺.

### Step 5:

Trifluoroacetic acid (0.5 mL) was added to a solution of compound **A189-4** (250 mg, 0.42 mmol) in dichloromethane (10 mL) at room temperature. The reaction mixture was stirred at 26°C for 2 h. The reaction mixture was concentrated under reduced pressure to give a brown oil **A189-5** (200 mg, yield 86%). LCMS (ESI) m/z = 544.3 [M+H]⁺.

### Step 6:

**A189-5** (17 mg, 31.25 µmol) was dissolved in dichloromethane, **A186-1** (11.78 mg, 46.88 µmol) and acetic acid (2.52 mg, 42.20 µmol) were added successively, and the mixture was stirred at 26°C for 2 h; then sodium cyanoborohydride (3.93 mg, 62.50 µmol) was added. After the reaction was completed, the mixture was directly spin-dried and purified by flash silica gel column chromatography (EA: MeOH=10% to 10%) to give **A189** (10.0 mg, yield 41%). LCMS (ESI) m/z = 779.3 [M+H]⁺. ¹H NMR (400 MHz, DMSO-*d*₆) δ 10.61 (s, 1H), 9.60 (s, 1H), 8.61 (s, 1H), 8.28 (d, *J* = 8.4 Hz, 1H), 7.90 - 7.87 (m, 1H), 7.65 - 7.62 (m, 2H), 7.59 - 7.53 (m, 1H), 7.33 (d, *J =* 2.4 Hz, 1H), 7.22 - 7.17 (m, 2H), 6.94 (d, *J* = 9.2 Hz, 2H), 6.54 (s, 1H), 3.90 (t, *J* = 6.8 Hz, 2H), 3.64 - 3.61 (m, 2H), 3.53 (s, 2H), 3.33 - 3.31 (m, 2H), 2.78 - 2.74 (m, 2H), 2.71 - 2.59 (m, 4H), 2.41 - 2.39 (m, 2H), 2.37 - 2.32 (m, 1H), 1.86 - 1.83 (m, 2H), 1.32 - 1.25 (m, 2H).

### Example 189 Synthesis of Compound A190:

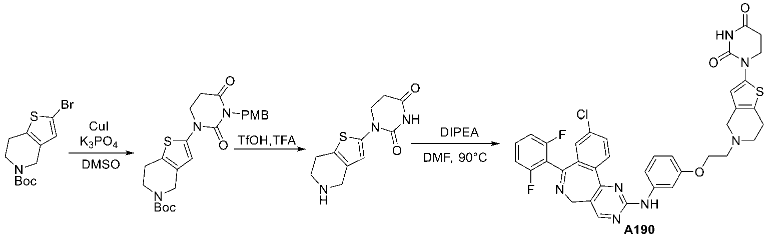

Compound **A190** (15.2 mg, yield 28.6%) was synthesized according to the synthesis method of compound **A101** described in **Example 101.** LCMS (ESI) m/z = 726.4 [M+H. ¹H NMR (400 MHz, DMSO-*d₆*) δ 10.56 (s, 1H), 9.86 (s, 1H), 8.68 (s, 1H), 8.30 (d, *J* = 8.4 Hz, 1H), 7.83 (dd, *J*₁ = 8.4 Hz, *J*₂ = 2.2 Hz, 1H), 7.68 - 7.67 (m, 1H), 7.57 - 7.50 (m, 1H), 7.33 - 7.31 (m, 2H), 7.22 - 7.13 (m, 3H), 6.61 - 6.56 (m, 1H), 6.44 (s, 1H), 4.16 - 4.13 (m, 2H), 3.83 (t, *J* = 6.8 Hz, 2H), 3.51 (s, 2H), 2.93 - 2.89 (m, 2H), 2.84 - 2.80 (m, 2H), 2.73 - 2.66 (m, 4H).

### Example 190 Synthesis of Compound A191:

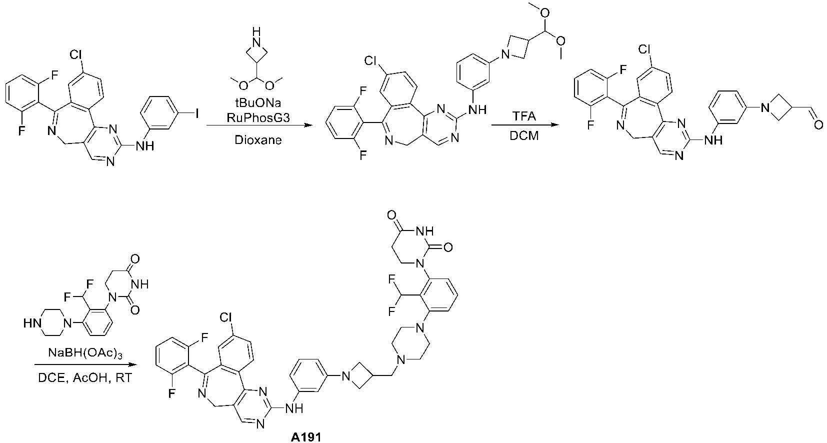

Compound **A191** (15.2 mg, yield 28.6%) was synthesized according to the synthesis method of compound **A132** described in **Example 131.** LCMS (ESI) m/z = 726.4 [M+H]. ¹H NMR (400 MHz, DMSO-*d*₆) δ 10.56 (s, 1H), 9.86 (s, 1H), 8.68 (s, 1H), 8.30 (d, *J* = 8.4 Hz, 1H), 7.83 (dd, *J*₁ = 8.4 Hz, *J*₂ = 2.2 Hz, 1H), 7.68 - 7.67 (m, 1H), 7.57 - 7.50 (m, 1H), 7.33 - 7.31 (m, 2H), 7.22 - 7.13 (m, 3H), 6.61 - 6.56 (m, 1H), 6.44 (s, 1H), 4.16 - 4.13 (m, 2H), 3.83 (t, *J* = 6.8 Hz, 2H), 3.51 (s, 2H), 2.93 - 2.89 (m, 2H), 2.84 - 2.80 (m, 2H), 2.73 - 2.66 (m, 4H).

### Example 191 Synthesis of Compound A192:

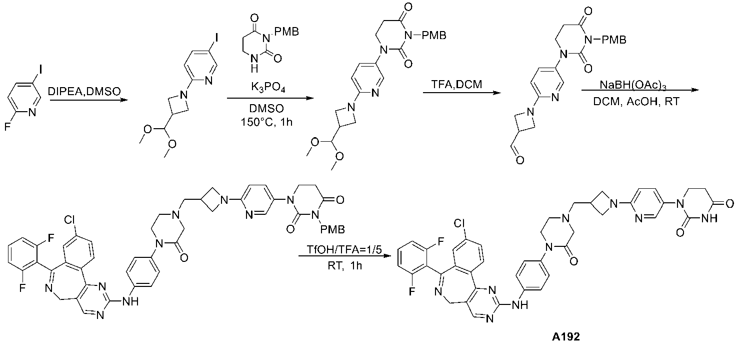

Compound **A192** (15.0 mg, yield 57.6%) was synthesized according to the synthesis method of compound **A132** described in **Example 131.** LCMS (ESI) m/z = 789.5 [M+H]. ¹H NMR (400 MHz, DMSO-*d*₆) *δ* 10.35 (s, 1H), 9.97 (s, 1H), 8.70 (s, 1H), 8.31 *(d, J =* 8.4 Hz, 1H), 8.04 (d, *J* = 2.6 Hz, 1H), 7.88 (dd, *J*₁ = 8.4 Hz, *J*₂ = 2.2 Hz, 1H), 7.84 (d, *J* = 8.8 Hz, 2H), 7.61 - 7.53 (m, 1H), 7.51 (dd, *J*₁ = 8.8 Hz, *J*₂ = 2.6 Hz, 1H), 7.35 (d, *J* =2.0 Hz, 1H), 7.29 (d, *J* = 8.8 Hz, 2H), 7.21 - 7.16 (m, 2H), 6.43 (d, *J* = 8.8 Hz, 1H), 4.09 (t, *J* = 8.0 Hz, 2H), 3.71 (t, *J* = 8.0 Hz, 2H), 3.68 - 3.64 (m, 4H), 3.22 (s, 2H), 3.05 - 2.98 (m, 1H), 2.85 - 2.82 (m, 2H), 2.76 - 2.69 (m, 4H).

### Example 192 Synthesis of Compound A193:

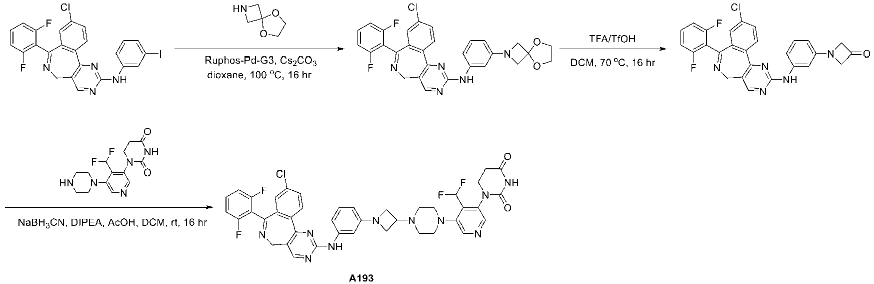

Compound **A193** (3 mg, yield 6.1%) was synthesized according to the synthesis method of compound **A132** described in **Example 131.** LCMS (ESI) m/z = 811.4 [M+H]⁺. ¹H NMR (400 MHz, DMSO-*d*₆) δ 10.52 (s, 1H), 9.74 (s, 1H), 8.68 (s, 1H), 8.63 (s, 1H), 8.48 (s, 1H), 8.33 (d, *J* = 8.0 Hz, 1H), 7.90 - 7.85 (m, 1H), 7.61 - 7.52 (m, 1H), 7.36 - 7.33 (m, 1H), 7.24 - 7.07 (m, 4H), 6.14 (d, *J* = 7.6 Hz, 1H), 4.02 - 3.94 (m, 2H), 3.89 - 3.80 (m, 1H), 3.70 - 3.62 (m, 2H), 3.61 - 3.52 (m, 1H), 3.14 - 3.05 (m, 4H), 2.76 - 2.68 (m, 3H), 2.65 - 2.58 (m, 4H).

### Example 193 Synthesis of Compound A194:

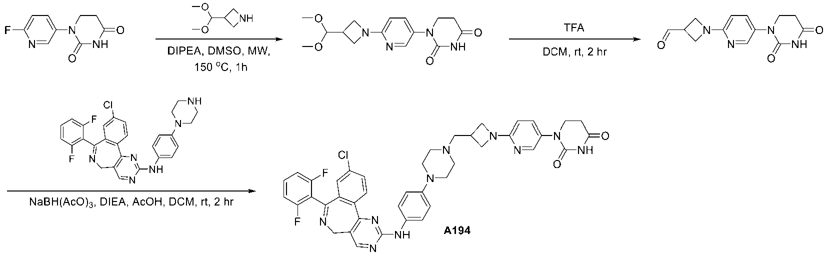

Compound **A194** (10 mg, yield 17.6%) was synthesized according to the synthesis method of compound **A132** described in **Example 131.** LCMS (ESI) m/z = 775.4 [M+H]⁺. ¹H NMR (400 MHz, DMSO-*d*₆) δ 10.36 (s, 1H), 9.62 (s, 1H), 8.61 (s, 1H), 8.28 (d, *J* = 8.4 Hz, 1H), 8.05 - 8.02 (m, 1H), 7.90 - 7.85 (m, 1H), 7.69 - 7.62 (m, 2H), 7.61 - 7.53 (m, 1H), 7.52 - 7.48 (m, 1H), 7.35 - 7.31 (m, 1H), 7.23 - 7.14 (m, 2H), 6.97 - 6.92 (m, 2H), 6.43 (d, *J* = 8.8 Hz, 1H), 4.08 (t, *J* = 8.0 Hz, 2H), 3.79 - 3.75 (m, 1H), 3.71 (t, *J* = 6.8 Hz, 2H), 3.67 - 3.61 (m, 2H), 3.13 - 3.07 (m, 4H), 3.03 - 2.96 (m, 1H), 2.72 (t, *J* = 6.8 Hz, 2H), 2.68 - 2.64 (m, 2H), 2.59 - 2.56 (m, 3H).

### Example 194 Synthesis of Compound A195:

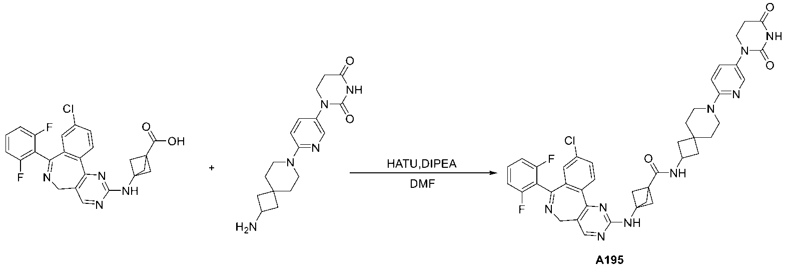

Compound **A195** (11.6 mg, yield 17.6%) was synthesized according to the synthesis method of compound **A1** described in **Example 1.** LCMS (ESI) m/z = 778.3 [M+H]⁺. ¹H NMR (400 MHz, DMSO-*d*₆) δ 10.33 (s, 1H), 8.48 (s, 1H), 8.21 - 8.19 (m, 1H), 8.12 (s, 1H), 8.03 (d, *J* = 2.0 Hz, 1H), 7.96 (d, *J* = 8.0 Hz, 1H), 7.86 (d, *J =* 8.4 Hz, 1H), 7.59 - 7.51 (m, 1H), 7.48 - 7.45 (m, 1H), 7.29 (d, *J =* 2.0 Hz, 1H), 7.17 (t, *J =* 8.2 Hz, 1H), 6.85 (d, *J* = 8.8 Hz, 1H), 4.28 - 4.22 (m ,1H), 3.69 (t, *J* = 6.8 Hz, 2H), 3.49 - 3.48 (m, 2H), 3.42 - 3.41 (m, 2H), 2.70 (t, *J =* 6.8 Hz, 2H), 2.27 (s, 6H), 2.17 - 2.12 (m, 2H), 1.81 - 1.76 (m, 2H), 1.61 - 1.53 (m, 4H).

### Example 195 Synthesis of Compound A196:

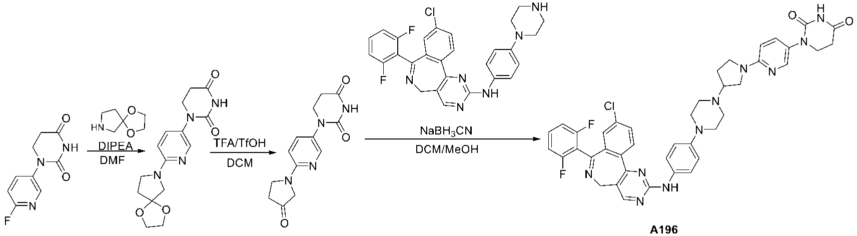

Compound **A196** (8.59 mg, yield 28.64%) was synthesized according to the synthesis method of compound **A132** described in **Example 131.** LCMS (ESI) m/z = 775.3 [M+H]⁺. ¹H NMR (400 MHz, DMSO-*d*₆) δ 10.31 (s, 1H), 9.60 (s, 1H), 8.58 (s, 1H), 8.25 (d, *J =* 8.4 Hz, 1H), 8.01 (d, *J* = 2.4 Hz, 1H), 7.85 (dd, *J*₁ = 8.4 Hz, *J*₂ = 2.0 Hz, 1H), 7.63 (d, *J* = 8.8 Hz, 2H), 7.60 - 7.49 (m, 1H), 7.46 (dd, *J*₁ = 8.4 Hz, *J*₂ = 2.8 Hz, 1H), 7.30 (d, *J* = 2.0 Hz, 1H), 7.21 - 7.12 (m, 2H), 6.93 (d, *J =* 8.8 Hz, 2H), 6.49 (d, *J =* 8.8 Hz, 1H), 3.81 - 3.50 (m, 4H), 3.30 - 3.04 (m, 6H), 3.00 - 2.92 (m, 1H), 2.74 - 2.57 (m, 6H), 2.30 - 2.17 (m, 1H), 1.91 - 1.81 (m, 1H).

### Example 196 Synthesis of Compound A197:

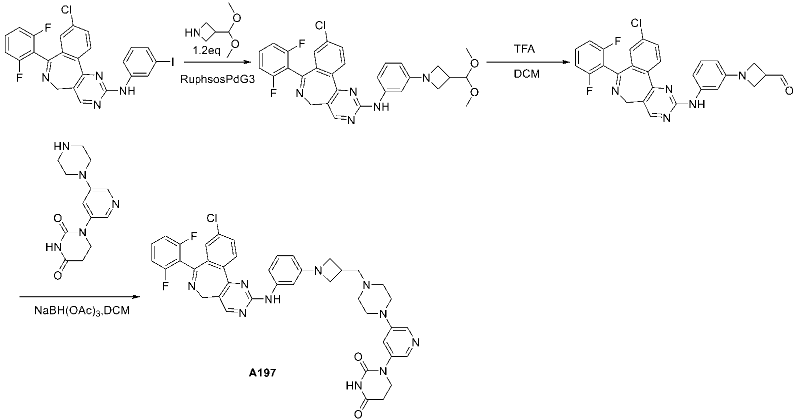

Compound **A197** (3.3 mg, yield 28.64%) was synthesized according to the synthesis method of compound **A132** described in **Example 131.** LCMS (ESI) m/z = 775.3 [M+H]⁺. ¹H NMR (400 MHz, DMSO-*d*₆) δ 10.46 (s, 1H), 9.74 (s, 1H), 8.67 (s, 1H), 8.35 (d, *J* = 8.4 Hz, 1H), 8.17 (s, 1H), 8.07 (s, 1H), 7.85 (dd, *J*₁ = 8.4 Hz, *J*₂ = 2.0 Hz, 1H), 7.57 - 7.54 (m, 2H), 7.37 - 7.31 (m, 3H), 7.21 - 7.12 (m, 2H), 7.09 (d, *J* = 8.8 Hz, 2H), 6.98 (d, *J* = 8.8 Hz, 1H), 6.09 (d, *J* = 8.8 Hz, 1H), 3.99 - 3.96 (m, 2H), 3.81 (t, *J* = 6.8 Hz, 2H), 3.54 - 3.49 (m, 2H), 3.26 (bs, 4H), 3.02 - 2.98 (m, 1H), 2.73 (t, *J* = 6,8 Hz, 2H), 2.69 - 2.67 (m, 2H), 2.58 - 2.57 (m, 4H).

### Example 197 Synthesis of Compound A198:

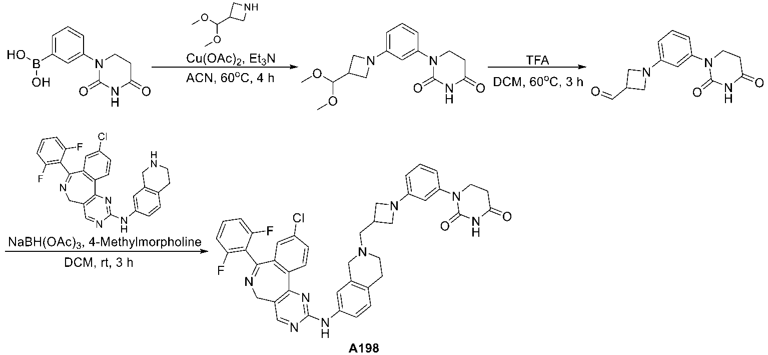

Compound **A198** (15 mg, yield 28%) was synthesized according to the synthesis method of compound **A132** described in **Example 131.** LCMS (ESI) m/z = 745.4 [M+H]⁺. ¹H NMR (400 MHz, DMSO-*d*₆) δ 10.31 (s, 1H), 9.76 (s, 1H), 8.66 (s, 1H), 8.29 (d, *J* = 8.8 Hz, 1H), 7.96 - 7.82 (m, 1H), 7.66 - 7.49 (m, 3H), 7.34 (d, *J* = 2.4 Hz, 1H), 7.29 - 7.12 (m, 3H), 7.12 - 7.01 (m, 1H), 6.69 - 6.56 (m, 1H), 6.44 - 6.37 (m, 1H), 6.37 - 6.25 (m, 1H), 3.99 (t, *J* = 7.6 Hz, 2H), 3.76 (t, *J* = 6.8 Hz, 2H), 3.60 (s, 2H), 3.57 - 3.48 (m, 2H), 3.15 - 2.97 (m, 1H), 2.86 - 2.75 (m, 4H), 2.75 - 2.63 (m, 4H).

### Example 198 Synthesis of Compound A199:

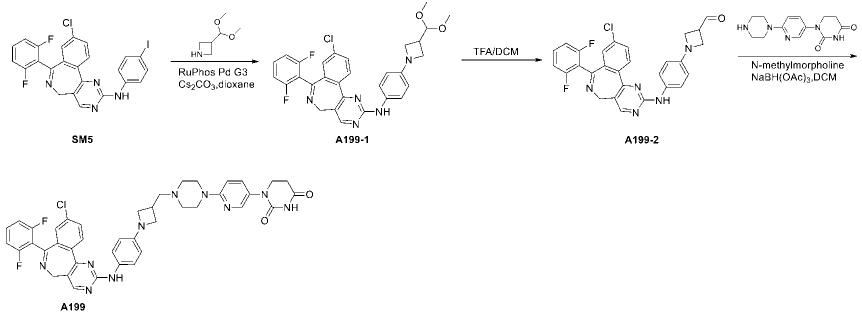

### Step 1:

3-(Dimethoxymethyl)azetidine (235 mg, 1.07 mmol, 60% purity), methanesulfonato(2-dicyclohexylphosphino-2',6'-diisopropoxy-1,1'-biphenyl)(2-amino-1,1'-biphenyl-2-yl) palladium (II) (75 mg, 0.09 mmol) and cesium carbonate (875 mg, 2.68 mmol) were added to a solution of **SM5** (500 mg, 0.89 mmol) in dioxane (10 mL) at room temperature. The atmosphere was replaced with argon 3 times, and the reaction mixture was stirred at 110°C for 16 h. LC-MS showed that the raw materials reacted completely. After the reaction mixture was filtered and the filtrate was concentrated under reduced pressure, the residue was separated for purification by silica gel column chromatography (ethyl acetate/petroleum ether = 1:5) to give a yellow solid **A199-1** (420 mg, yield 83.5%). LCMS (ESI) m/z = 562.0 [M+H]⁺.

### Step 4:

Trifluoroacetic acid (0.5 mL) was added to a solution of compound **A199-1** (30 mg, 0.05 mmol) in dichloromethane (1 mL) at room temperature, and the reaction mixture was stirred at room temperature for 1 h. N-methylmorpholine was added to the reaction mixture to adjust the pH to 8, and the mixture was then concentrated under reduced pressure to give **A199-2** as a yellow oil (27.5 mg, yield 100%). LCMS (ESI) m/z = 516.1 [M+H]⁺. The crude product was directly used in the next reaction.

### Step 5:

At room temperature, 1-(6-(piperazin-1-yl)pyridin-3-yl)dihydropyrimidine-2,4(1H,3H)-dione (14.6 mg, 0.05 mmol) and sodium triacetoxyborohydride (22.6 mg, 0.1 mmol) were added to a solution of compound A199-2 (27.5 mg, 0.05 mmol) in dichloromethane (2 mL), and the reaction mixture was stirred at room temperature for 1 h. LC-MS showed that the reaction was complete. After the addition of water, the reaction mixture was extracted with dichloromethane, dried over anhydrous sodium sulfate, concentrated under reduced pressure, and the crude product was separated for purification by reverse phase chromatography (acetonitrile/10 mM aqueous ammonium bicarbonate solution) to give a yellow solid **A199** (9.8 mg, yield 23.7%). LCMS (ESI) m/z = 775.5 [M+H]⁺. ¹H NMR (400 MHz, DMSO-*d*₆) δ 10.34 (s, 1H), 9.48 (s, 1H), 8.55 (s, 1H), 8.23 (d, *J* = 8.8 Hz, 1H), 8.08 - 8.06 (m, 1H), 7.86 - 7.81 (m, 1H), 7.57 - 7.48 (m, 4H), 7.30 - 7.28 (m, 1H), 7.20 - 7.12 (m, 2H), 6.85 (d, *J* = 8.0 Hz, 1H), 6.42 (d, *J* = 8.0 Hz, 2H), 3.95 - 3.87 (m, 2H), 3.73 - 3.67 (m, 2H), 3.49 - 3.42 (m, 6H), 2.99 - 2.88 (m, 1H), 2.72 - 2.66 (m, 2H), 2.64 - 2.60 (m, 2H), 2.48 - 2.44 (m, 4H).

### Example 199 Synthesis of Compound A200:

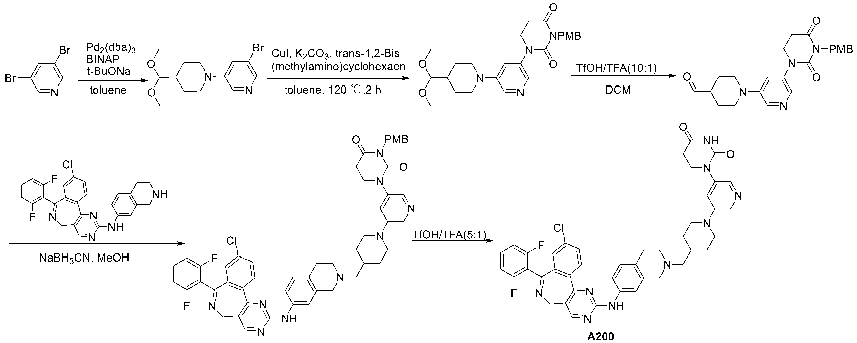

Compound A200 (4.23 mg, yield 8.24%) was synthesized according to the synthesis method of compound A132 described in Example 131. LCMS (ESI) m/z = 774.2 [M+H]⁺. ¹H NMR (400 MHz, DMSO-*d*₆) δ 10.42 (s, 1H), 9.73 (s, 1H), 8.63 (s, 1H), 8.26 (d, *J* = 8.4 Hz, 1H), 8.15 (d, *J* = 2.4 Hz, 1H), 7.94 (d, *J* = 2.0 Hz, 1H), 7.86 (dd, *J*₁ = 8.4 Hz, *J*₂ =2.0 Hz, 1H), 7.60 - 7.47 (m, 3H), 7.34 - 7.25 (m, 2H), 7.21 - 7.12 (m, 2H), 7.04 (d, *J* = 8.4 Hz, 1H), 3.83 - 3.74 (m, 4H), 3.57 - 3.52 (m, 2H), 2.85 - 2.61 (m, 8H), 2.39 - 2.32 (m, 2H), 1.88 - 1.81 (m, 3H), 1.28 - 1.21 (m, 2H).

### Example 200 Synthesis of Compound A201:

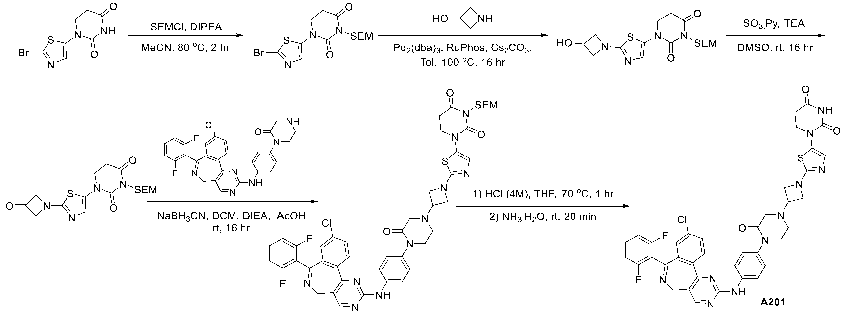

Compound **A201** (3 mg, yield 20.6%) was synthesized according to the synthesis method of compound **A132** described in **Example 131.** LCMS (ESI) m/z = 781.4 [M+H]⁺. ¹H NMR (400 MHz, DMSO-*d*₆) δ 10.63 (s, 1H), 9.98 (s, 1H), 8.70 (s, 1H), 8.31 (d, *J* = 8.4 Hz, 1H), 7.90 - 7.87 (m, 1H), 7.86 - 7.82 (m, 2H), 7.61 - 7.52 (m, 1H), 7.35 (d, *J* = 2.4 Hz, 1H), 7.31 - 7.26 (m, 2H), 7.24 - 7.15 (m, 2H), 7.02 (s, 1H), 4.13 - 4.05 (m, 2H), 3.95 - 3.90 (m, 2H), 3.83 (t, *J* = 6.8 Hz, 2H), 3.71 - 3.65 (m, 2H), 3.61 - 3.54 (m, 1H), 3.23 (s, 2H), 2.85 - 2.80 (m, 2H), 2.76 (t, *J* = 6.8 Hz, 2H).

### Example 201 Synthesis of Compound A202:

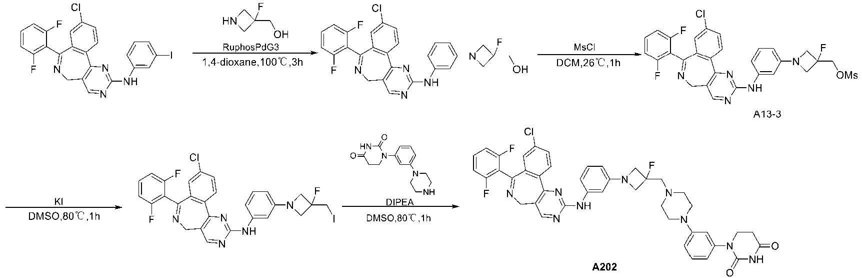

Compound **A202** (4.0 mg, yield 62%) was synthesized according to the synthesis method of compound **A126** described in **Example 125.** LCMS (ESI) m/z = 792.2 [M+H]⁺.¹H NMR (400 MHz, DMSO-*d*₆) δ 10.33 (s, 1H), 9.01 (s, 1H), 8.70 (s, 1H), 8.37 (d, *J* = 8.4 Hz, 1H), 7.90 (dd, *J*₁ = 6.0 Hz, *J*₂ = 2.4 Hz, 1H), 7.56 - 7.54 (m, 1H), 7.47 (s, 1H), 7.34 (d, *J* = 8.4 Hz, 2H), 7.25 - 7.12 (m, 3H), 7.05 (d, *J* = 8.4 Hz, 1H), 6.95 - 6.91 (m, 2H), 6.93 (d, *J* = 8.4 Hz, 1H), 6.75 (d, *J* = 8.8 Hz, 1H), 6.18 (d, *J* = 8.4 Hz, 1H), 4.10 - 4.03 (m, 2H), 3.76 (t, *J* = 6.8 Hz, 2H), 3.20 - 3.18 (m, 4H), 3.01 (s, 1H), 2.94 (s, 1H), 2.72 - 2.68 (m, 6H).

### Example 202 Synthesis of Compound A203:

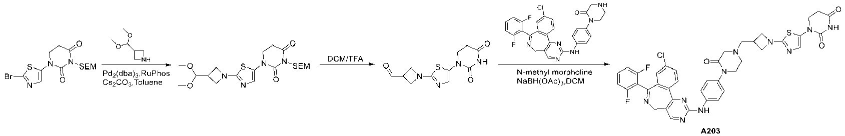

Compound **A203** (6.38 mg, yield 18.5%) was synthesized according to the synthesis method of compound **A132** described in **Example 131.** LCMS (ESI) m/z = 795.5 [M+H]⁺. ¹H NMR (400 MHz, DMSO-*d*₆) δ 10.57 (s, 1H), 9.94 (s, 1H), 8.67 (s, 1H), 8.28 (d, *J* = 8.8 Hz, 1H), 7.88 - 7.78 (m, 3H), 7.58 - 7.50 (m, 1H), 7.33 - 7.30 (m, 1H), 7.29 - 7.23 (m, 2H), 7.21 - 7.10 (m, 2H), 6.97 (s, 1H), 4.11 - 4.04 (m, 2H), 3.82 - 3.76 (m, 2H), 3.69 - 3.59 (m, 4H), 3.20 - 3.15 (m, 2H), 3.12 - 3.00 (m, 1H), 2.83 - 2.78 (m, 2H), 2.75 - 2.68 (m, 4H).

### Example 203 Synthesis of Compound A204:

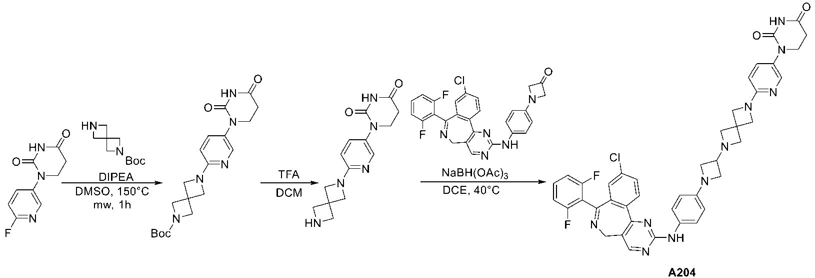

Compound **A204** (2.0 mg, yield 8.6%) was synthesized according to the synthesis method of compound **A132** described in **Example 131.** LCMS (ESI) m/z = 773.5 [M+H]. ¹H NMR (400 MHz, DMSO-*d*₆) δ 10.32 (s, 1H), 9.47 (s, 1H), 8.55 (s, 1H), 8.23 (d, *J* = 8.4 Hz, 1H), 8.01 (d, *J* = 2.6 Hz, 1H), 7.84 (dd, *J₁* = 8.4, *J*₂ =2.2 Hz, 1H), 7.57-7.45 (m, 4H), 7.29 (d, *J* = 2.2 Hz, 1H), 7.18 - 7.14 (m, 2H), 6.42 - 6.39 (m, 3H), 4.01 (s, 4H), 3.81 - 3.74 (m, 2H), 3.67 (t, *J =* 6.8 Hz, 2H), 3.55 - 3.50 (m, 3H), 3.35 (s, 4H), 2.69 (t, *J =* 6.8 Hz, 2H).

### Example 204 Synthesis of Compound A205:

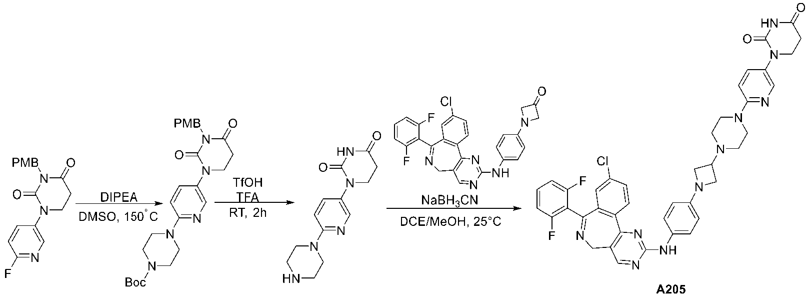

Compound **A205** (4.0 mg, yield 17.2%) was synthesized according to the synthesis method of compound **A132** described in **Example 131.** LCMS (ESI) m/z = 789.5 [M+H]. ¹H NMR (400 MHz, DMSO-*d₆*) δ 10.33 (s, 1H), 9.48 (s, 1H), 8.56 (s, 1H), 8.24 (d, *J* = 4.0 Hz, 1H), 8.06 (d, *J* = 2.8 Hz, 1H), 7.84 (dd, *J*₁ = 8.0 Hz, *J*₂ =2.2 Hz, 1H), 7.57 - 7.49 (m, 1H), 7.29 *(d, J =* 4.0 Hz, 1H), 7.18 - 7.14 (m, 2H), 6.86 (d, *J =* 9.0 Hz, 1H), 6.44 (d, *J =* 8.8 Hz, 2H), 3.92 (t, *J =* 6.8 Hz, 2H), 3.70 (t, *J* = 6.8 Hz, 2H), 3.61 - 3.58 (m, 2H), 3.51 - 3.48 (m, 4H), 2.71 - 2.67 (m, 3H), 2.48 - 2.43 (m, 4H).

### Example 205 Synthesis of Compound A206:

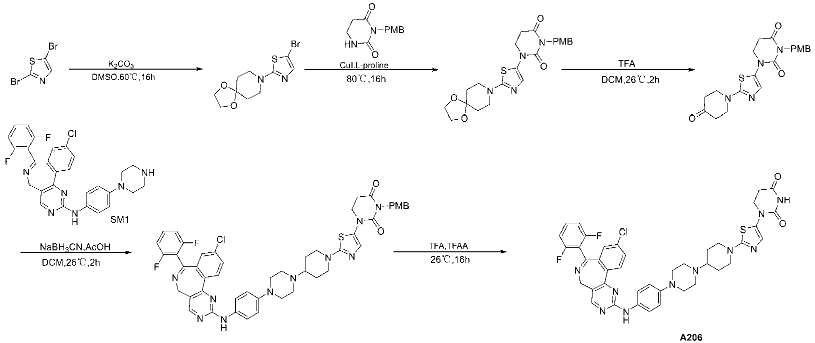

Compound **A206** (15.0 mg, yield 17%) was synthesized according to the synthesis method of compound **A132** described in **Example 131.** LCMS (ESI) m/z = 795.2 [M+H]⁺. ¹H NMR (400 MHz, DMSO-*d*₆) δ 10.61 (s, 1H), 9.62 (s, 1H), 8.61 (s, 1H), 8.28 (d, *J* = 8.4 Hz, 1H), 7.87 (dd, *J*₁ = 8.4 Hz, *J*₂ = 2.2 Hz, 1H), 7.65 (d, *J* = 8.8 Hz, 2H), 7.62 - 7.53 (m, 1H), 7.33 (d, *J =* 2.8 Hz, 1H), 7.19 (t, *J* = 8.8 Hz, 2H), 7.01 (s, 1H), 6.94 (d, *J* = 9.2 Hz, 2H), 3.92 - 3.90 (m, 2H), 3.82 - 3.80 (m, 2H), 3.13 - 3.10 (m, 4H), 2.99 (t, *J* = 12.4 Hz, 2H), 2.75 (t, *J* = 6.8 Hz, 2H), 2.69 - 2.67 (m, 4H), 2.36 - 2.32 (m, 1H), 1.91 - 1.89 (m, 2H), 1.54 - 1.52 (m, 2H).

### Example 206 Synthesis of Compound A207:

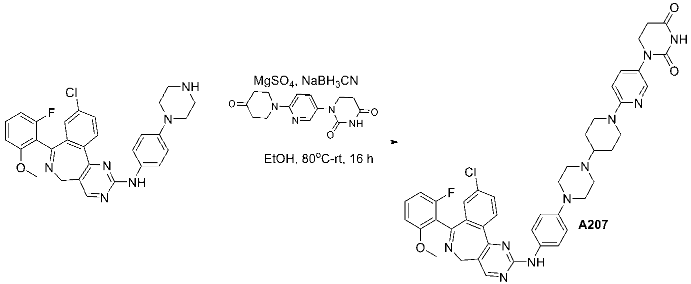

Compound **A207** (21.1 mg, yield 45%) was synthesized according to the synthesis method of **compound A132** described in **Example 131.** LCMS (ESI) m/z = 801.3 [M+H]⁺. ¹H NMR (400 MHz, DMSO-*d*₆) δ 10.37 (s, 1H), 9.61 (s, 1H), 8.59 (s, 1H), 8.24 (d, *J* = 8.4 Hz, 1H), 8.08 (d, *J* = 2.8 Hz, 1H), 7.85 - 7.77 (m, 1H), 7.67 (d, *J* = 8.8 Hz, 2H), 7.56 - 7.49 (m, 1H), 7.49 - 7.39 (m, 1H), 7.21 (s, 1H), 6.99 - 6.85 (m, 4H), 4.45 - 4.24 (m, 2H), 3.73 (t, *J* = 6.8 Hz, 2H), 3.17 - 3.03 (m, 4H), 2.93 - 2.80 (m, 2H), 2.78 - 2.61 (m, 7H), 1.99 - 1.81 (m, 2H), 1.55 - 1.40 (m, 2H).

### Example 207 Synthesis of Compound A208:

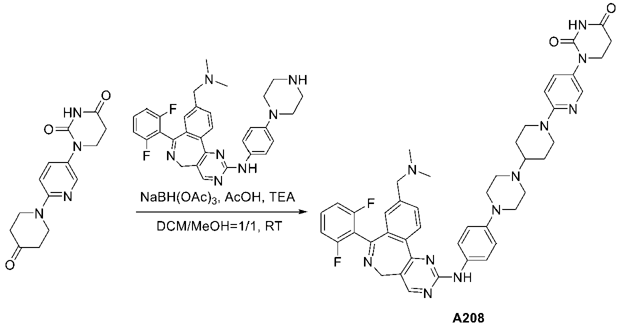

Compound **A208** (23.0 mg, yield 40.8%) was synthesized according to the synthesis method of compound **A132** described in **Example 131.** LCMS (ESI) m/z = 812.6 [M+H]. ¹H NMR (400 MHz, DMSO-*d*₆) δ 10.33 (s, 1H), 9.51 (s, 1H), 8.55 (s, 1H), 8.20 (d, *J* = 8.0 Hz, 1H), 8.05 (d, *J* = 2.8 Hz, 1H), 7.65 - 7.62 (m, 3H), 7.56 - 7.43 (m, 2H), 7.19 (s, 1H), 7.17 - 7.10 (m, 1H), 6.91 - 6.85 (m, 3H), 4.31 (d, *J* = 12.0 Hz, 2H), 3.70 (t, *J* = 6.8 Hz, 2H), 3.43 (s, 2H), 3.06 (s, 4H), 2.82 (t, *J =* 12.0 Hz, 2H), 2.70 (t, *J* = 6.8 Hz, 2H), 2.66 - 2.63 (m, 4H), 2.07 (s, 6H), 1.88 - 1.85 (m, 2H), 1.45 - 1.37 (m, 2H).

### Example 208 Synthesis of Compound A209:

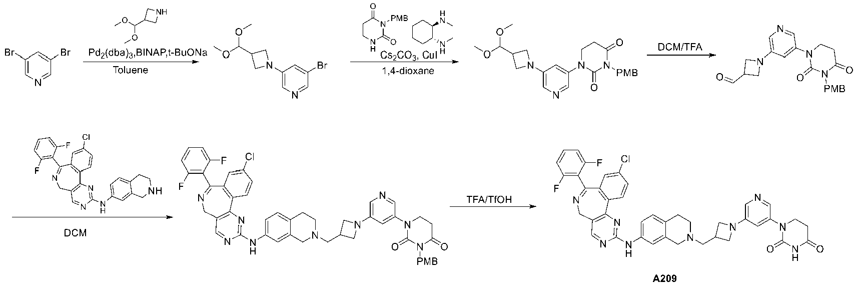

Compound **A209** (8.2 mg, yield 29.7%) was synthesized according to the synthesis method of compound **A132** described in **Example 131.** LCMS (ESI) m/z = 746.4 [M+H]⁺. ¹H NMR (400 MHz, DMSO-*d*₆) δ 10.42 (s, 1H), 9.74 (s, 1H), 8.63 (s, 1H), 8.26 (d, *J* = 8.8 Hz, 1H), 7.91 - 7.85 (m, 2H), 7.70 - 7.66 (m, 1H), 7.57 - 7.50 (m, 3H), 7.31 (d, *J=* 4.0 Hz, 1H), 7.21 - 7.12 (m, 2H), 7.06 - 7.01 (m, 1H), 6.81 - 6.79 (m, 1H), 4.07 - 4.00 (m, 2H), 3.80 - 3.75 (m, 2H), 3.63 - 3.53 (m, 4H), 3.15 - 3.06 (m, 1H), 2.80 - 2.73 (m, 4H), 2.73 - 2.65 (m, 4H).

### Example 209 Synthesis of Compound A210:

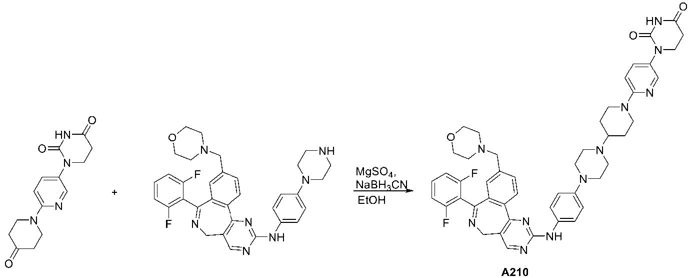

**Compound A209** (15.64 mg, yield 35.51%) was synthesized according to the synthesis method of compound **A132** described in **Example 131.** LCMS (ESI) m/z = 854.7 [M+H]⁺. ¹H NMR (400 MHz, DMSO-*d*₆) δ 10.33 (s, 1H), 9.51 (s, 1H), 8.56 (s, 1H), 8.22 (d, *J* = 8.0 Hz, 1H), 8.05 (d, *J* = 2.8 Hz, 1H), 7.69 - 7.61 (m, 3H), 7.59 - 7.43 (m, 2H), 7.25 (d, *J* = 1.6 Hz, 1H), 7.22 - 7.06 (m, 2H), 6.94 - 6.83 (m, 3H), 4.31 (d, *J =* 12.8 Hz, 2H), 3.70 (t, *J =* 6.8 Hz, 2H), 3.60 - 3.40 (m, 6H), 3.13 - 3.00 (m, 4H), 2.88 - 2.77 (m, 2H), 2.74 - 2.58 (m, 7H), 2.30 - 2.25 (m, 4H), 1.93 - 1.82 (m, 2H), 1.48 - 1.35 (m, 2H).

### Example 210 Synthesis of Compound A211:

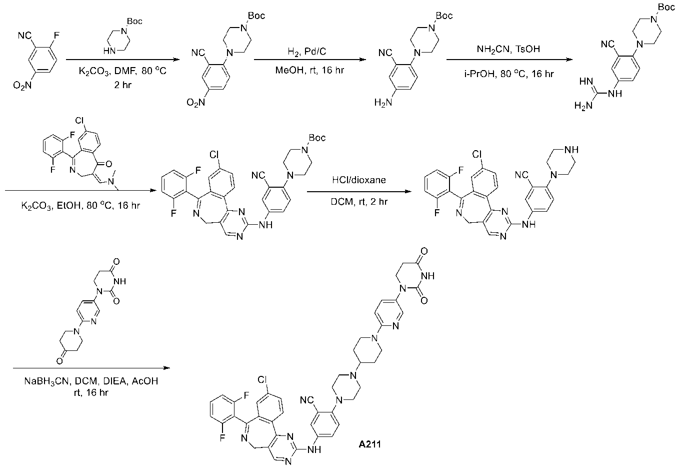

Compound **A211** (4 mg, yield 5.6%) was synthesized according to the synthesis method of compound **A132** described in **Example 131.** LCMS (ESI) m/z = 814.3 [M+H]⁺. ¹H NMR (400 MHz, DMSO-*d*₆) δ 10.35 (s, 1H), 10.04 (s, 1H), 8.71 (s, 1H), 8.28 (d, *J* = 8.4 Hz, 1H), 8.15 (d, *J =* 2.8 Hz, 1H), 8.08 (d,*J* = 2.8 Hz, 1H), 8.04 - 8.0 (m, 1H), 7.90 - 7.85 (m, 1H), 7.62 - 7.54 (m, 1H), 7.53 - 7.48 (m, 1H), 7.35 (d, *J =* 2.1 Hz, 1H), 7.24 - 7.16 (m, 3H), 6.89 (d, *J* = 9.1 Hz, 1H), 4.38 - 4.28 (m, 2H), 3.73 (t, *J* = 6.8 Hz, 2H), 3.14 - 3.06 (m, 4H), 2.91 - 2.80 (m, 2H), 2.75 - 2.71 (m, 6H), 2.60 - 2.55 (m, 1H), 1.95 - 1.85 (m, 2H), 1.52 - 1.39 (m, 2H).

### Example 211 Synthesis of Compound A212:

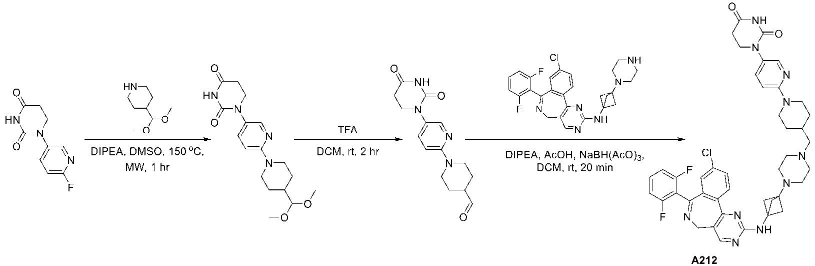

Compound **A212** (8 mg, yield 12.7%) was synthesized according to the synthesis method of compound **A132** described in **Example 131.** LCMS (ESI) m/z = 793.4 [M+H]⁺. ¹H NMR (400 MHz, DMSO-*d*₆) δ 10.34 (s, 1H), 8.49 (s, 1H), 8.26 - 8.18 (m, 1H), 8.09 (s, 1H), 8.06 (d, *J=* 2.8 Hz, 1H), 7.89 - 7.84 (m, 1H), 7.60 - 7.52 (m, 1H), 7.50 - 7.46 (m, 1H), 7.30 (d, *J* = 2.4 Hz, 1H), 7.19 (t, *J* = 8.8 Hz, 2H), 6.85 (d, *J =* 9.2 Hz, 1H), 4.28 (d, *J* = 12.8 Hz, 2H), 3.71 (t, *J* = 6.8 Hz, 2H), 2.81 (t, *J* = 12.0 Hz, 2H), 2.72 (t, *J* = 6.8 Hz, 2H), 2.50 - 2.30 (m, 8H), 2.19 - 2.13 (m, 2H), 2.07 (s, 6H), 1.82 - 1.72 (m, 3H), 1.15 - 1.02 (m, 2H).

### Example 212 Synthesis of Compound A213:

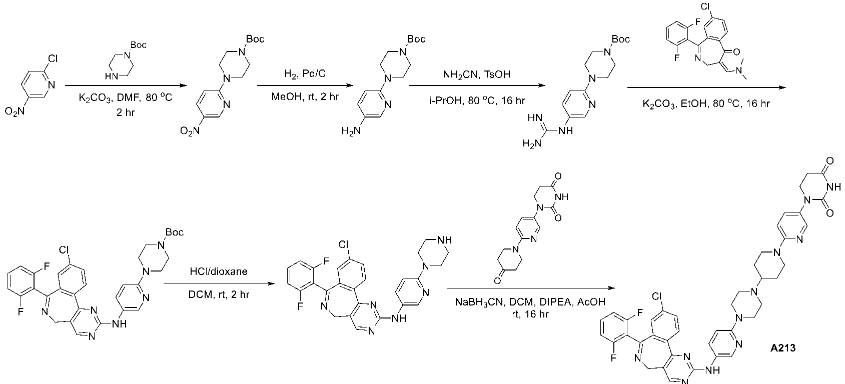

Compound **A213** (20 mg, yield 28.0%) was synthesized according to the synthesis method of compound **A132** described in **Example 131.** LCMS (ESI) m/z = 790.4 [M+H]⁺. ¹H NMR (400 MHz, DMSO-*d*₆) δ 10.35 (s, 1H), 9.60 (s, 1H), 8.61 (s, 1H), 8.53 - 8.49 (m, 1H), 8.25 (d, *J* = 8.4 Hz, 1H), 8.08 (d, *J* = 2.8 Hz, 1H), 7.94 - 7.90 (m, 1H), 7.89 - 7.85 (m, 1H), 7.61 - 7.53 (m, 1H), 7.52 - 7.48 (m, 1H), 7.33 (d, *J* = 2.4 Hz, 1H), 7.23 - 7.15 (m, 2H), 6.91 - 6.83 (m, 2H), 4.39 - 4.29 (m, 2H), 3.73 (t, *J* = 6.8 Hz, 2H), 3.45 - 3.39 (m, 4H), 2.90 - 2.78 (m, 2H), 2.72 (t, *J* = 6.8 Hz, 2H), 2.67 - 2.60 (m, 4H), 2.56 - 2.53 (m, 1H), 1.93 - 1.84 (m, 2H), 1.50 - 1.38 (m, 2H).

### Example 213 Synthesis of Compound A214:

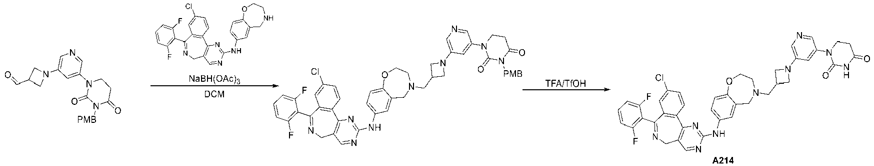

Compound **A214** (13.2 mg, yield 30.5%) was synthesized according to the synthesis method of compound **A132** described in **Example 131.** LCMS (ESI) m/z = 762.4 [M+H]⁺. ¹H NMR (400 MHz, DMSO-*d*₆) δ 10.39 (s, 1H), 9.71 (s, 1H), 8.62 (s, 1H), 8.29 (d, *J* = 8.8 Hz, 1H), 7.88 - 7.83 (m, 2H), 7.70 - 7.66 (m, 1H), 7.64 - 7.61 (m, 1H), 7.57 - 7.49 (m, 2H), 7.30 - 7.27 (m, 1H), 7.19 - 7.05 (m, 2H), 6.93 (d, *J* = 8.0 Hz, 1H), 6.75 - 6.72 (m, 1H), 4.01 - 3.84 (m, 4H), 3.79 (s, 2H), 3.76 - 3.70 (m, 2H), 3.56 - 3.48 (m, 2H), 3.08 - 2.93 (m, 3H), 2.74 - 2.65 (m, 4H).

### Example 214 Synthesis of Compound A215:

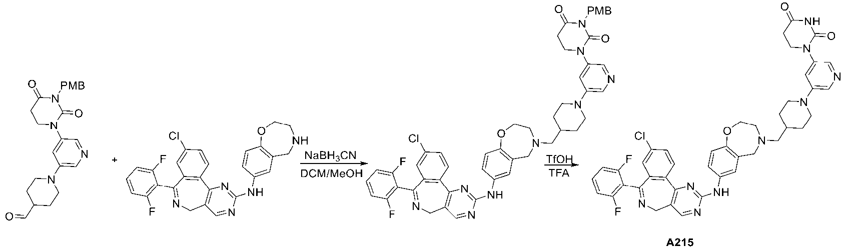

Compound **A215** (8.99 mg, yield 21.14%) was synthesized according to the synthesis method of compound **A132** described in **Example 131.** LCMS (ESI) m/z = 790.55 [M+H]⁺. ¹H NMR (400 MHz, DMSO-*d*₆) δ 10.41 (s, 1H), 9.73 (s, 1H), 8.62 (s, 1H), 8.27 (d, *J* = 8.4 Hz, 1H), 8.07 (d, *J* = 2.4 Hz, 1H), 7.92 (d, *J =* 2.0 Hz, 1H), 7.86 (dd, *J*₁ = 8.4 Hz, *J*₂ =2.0 Hz, 1H), 7.73 (s, 1H), 7.58 - 7.46 (m, 2H), 7.29 (d, *J* = 2.0 Hz, 1H), 7.22 (t, *J =* 2.4 Hz, 1H), 7.17 - 7.06 (m, 2H), 6.93 *(d, J =* 8.4 Hz, 1H), 4.01 - 3.88 (m, 2H), 3.79 - 3.66 (m, 6H), 3.06 - 2.96 (m, 2H), 2.74 - 2.55 (m, 4H), 2.33 - 2.27 (m, 2H), 1.83 - 1.59 (m, 3H), 1.26 - 1.13 (m, 2H).

### Example 215 Synthesis of Compound A216:

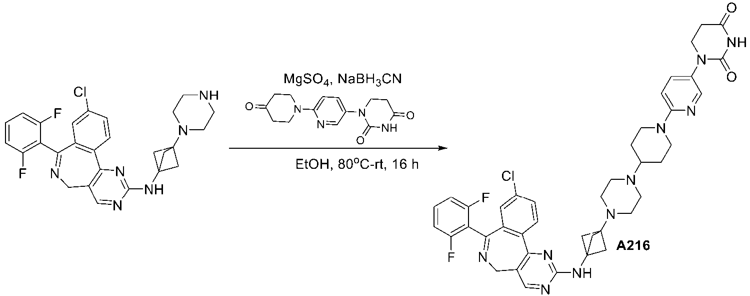

Compound **A216** (8.5 mg, yield 24%) was synthesized according to the synthesis method of compound **A132** described in **Example 131.** LCMS (ESI) m/z = 779.4 [M+H]⁺. ¹H NMR (400 MHz, DMSO-*d*₆) δ 10.36 (s, 1H), 8.49 (s, 1H), 8.30 - 8.14 (m, 1H), 8.14 - 8.02 (m, 2H), 7.87 (d, *J =* 8.4 Hz, 1H), 7.62 - 7.45 (m, 2H), 7.30 (d, *J* = 2.4 Hz, 1H), 7.19 (t, *J =* 8.8 Hz, 2H), 6.88 (d, *J* = 8.8 Hz, 1H), 4.40 - 4.23 (m, 2H), 3.72 (t, *J* = 6.4 Hz, 2H), 2.90 - 2.76 (m, 2H), 2.76 - 2.66 (m, 2H), 2.59 - 2.54 (m, 4H), 2.49 - 2.37 (m, 5H), 2.06 (s, 6H), 1.92 - 1.75 (m, 2H), 1.48 - 1.32 (m, 2H).

### Example 216 Synthesis of Compound A217:

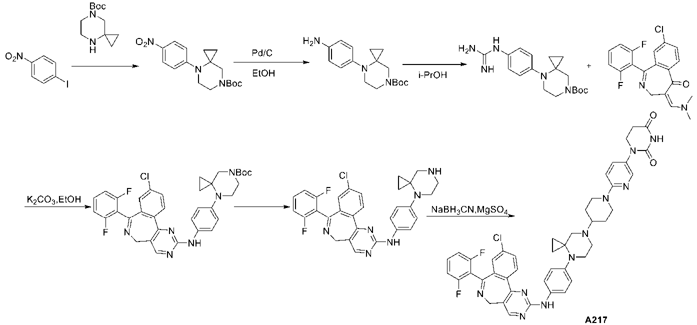

Compound **A217** (3.7 mg, yield 12%) was synthesized according to the synthesis method of compound **A132** described in **Example 131.** LCMS (ESI) m/z = 815.3 [M+H]⁺. ¹H NMR (400 MHz, DMSO-*d*₆) δ 10.36 (s, 1H), 9.55 (s, 1H), 8.59 (s, 1H), 8.28 (d, *J* = 8.4 Hz, 1H), 8.05 (d, *J* = 2.4 Hz, 1H), 7.89 - 7.86 (m, 1H), 7.61 -7.55 (m, 3H),7.50 - 7.47 (m, 1H), 7.32 (d, *J* = 2.4 Hz, 1H), 7.19 (t, *J* = 8.8 Hz, 2H), 7.00 (d, *J* = 10.0 Hz, 1H), 6.85 (d, *J* = 9.2 Hz, 1H ), 4.26 - 4.23 (m, 2H), 3.72 (t, *J* = 6.8 Hz, 2H), 2.84 - 2.78 (m, 2H), 2.71 (t, *J* = 6.8 Hz, 2H), 2.37 - 2.35 (m, 1H), 1.18 - 1.78 (m, 2H), 1.37 - 1.35 (m, 2H).

### Example 217 Synthesis of Compound A218:

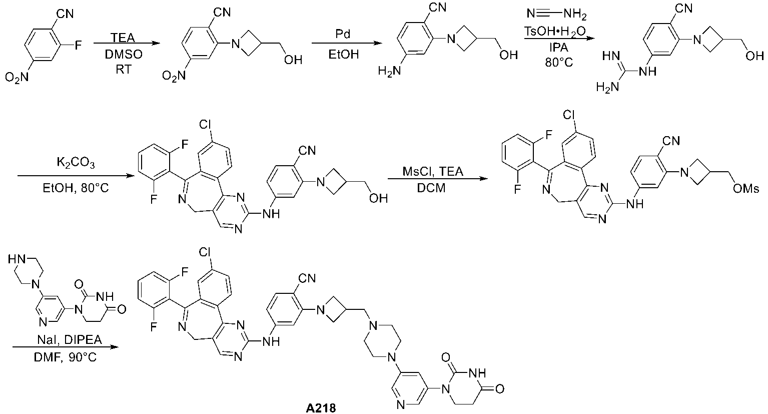

Compound **A218** (6.0 mg, yield 4.6%) was synthesized according to the synthesis method of compound **A211** described in **Example 210.** LCMS (ESI) m/z = 800.5 [M+H]⁺. ¹H NMR (400 MHz, DMSO-*d*₆) δ 10.43 (s, 1H), 10.22 (s, 1H), 8.75 (s, 1H), 8.35 (d, *J* = 8.4 Hz, 1H), 8.13 (d, *J* = 2.6 Hz, 1H), 7.98 (s, 1H), 7.87 - 7.84 (m, 1H), 7.65 (s, 1H), 7.56 - 7.48 (m, 1H), 7.38 - 7.11 (m, 5H), 6.97 (d, *J* = 8.6 Hz, 1H), 4.25 - 4.21 (m, 1H), 3.79 - 3.76 (m, 3H), 3.24 - 3.21 (m, 4H), 3.05 - 2.96 (m, 1H), 2.72 - 2.66 (m, 4H), 2.56 - 2.53 (m, 4H).

### Example 218 Synthesis of Compound A219:

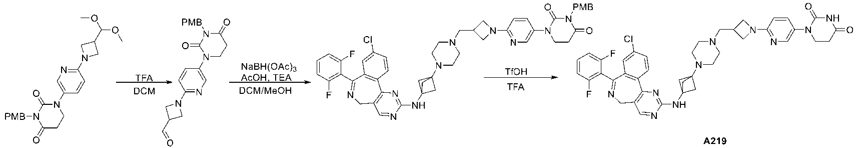

Compound **A219** (30.0 mg, yield 69.4%) was synthesized according to the synthesis method of compound **A132** described in **Example 131.** LCMS (ESI) m/z = 765.5 [M+H]⁺. ¹H NMR (400 MHz, DMSO-*d*₆) δ 10.32 (s, 1H), 8.47 (s, 1H), 8.20 - 8.17 (m, 1H), 8.08 (s, 1H), 8.00 (d, *J* = 2.6 Hz, 1H), 7.84 (d, *J =* 8.4 Hz, 1H), 7.57 - 7.50 (m, 1H), 7.47 (dd, *J*₁ = 8.8 Hz, *J*₂ = 2.6 Hz, 1H), 7.27 (d, *J* = 2.2 Hz, 1H), 7.16 (t,*J* = 8.8 Hz, 2H), 6.38 (d, *J* = 8.8 Hz, 1H), 4.01 (t, *J* = 8.0 Hz, 2H), 3.67 (t, *J* = 6.8 Hz, 2H), 3.59 - 3.55 (m, 2H), 2.94 - 2.87 (m, 1H), 2.69 (t, *J* = 6.8 Hz, 2H), 2.57 (d, *J* = 7.4 Hz, 2H), 2.41 (bs, 6H), 2.04 (s, 6H).

### Example 219 Synthesis of Compound A220:

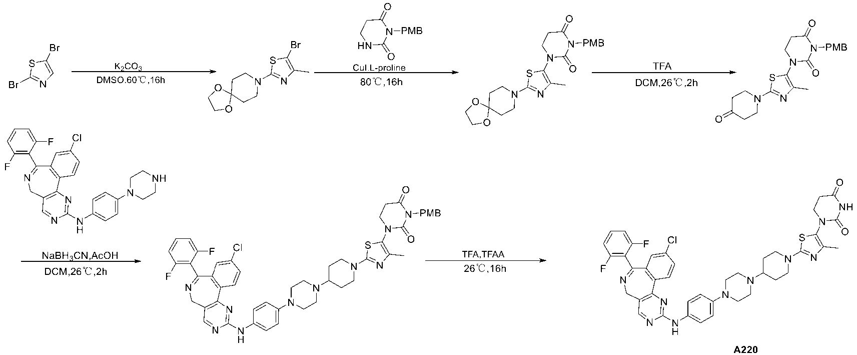

Compound **A220** (10.0 mg, yield 24.5%) was synthesized according to the synthesis method of compound **A132** described in **Example 131.** LCMS (ESI) m/z = 809.3 [M+H]⁺. ¹H NMR (400 MHz, DMSO-*d*₆) δ 10.48 (s, 1H), 9.61 (s, 1H), 8.61 (s, 1H), 8.28 (d, *J* = 8.4Hz, 1H), 7.91 - 7.86 (m, 1H), 7.65 (d, *J* = 8.8 Hz, 2H), 7.57 - 7.55 (m, 1H), 7.33 (d, *J* = 2.4 Hz, 1H), 7.19 (t, *J* = 8.8 Hz, 2H), 6.94 (d, *J* = 8.8 Hz, 2H), 3.90 - 3.88 (m, 2H), 3.62 - 3.60 (m, 2H), 3.12 - 3.10 (m, 4H), 3.00 (t, *J* = 11.6 Hz, 2H), 2.74 (t, *J* = 6.8 Hz, 2H), 2.69 - 2.66 (m, 4H), 2.02 (s, 3H), 1.92 - 1.90 (m, 2H), 1.54 - 1.50 (m, 2H).

### Example 220 Synthesis of Compound A221:

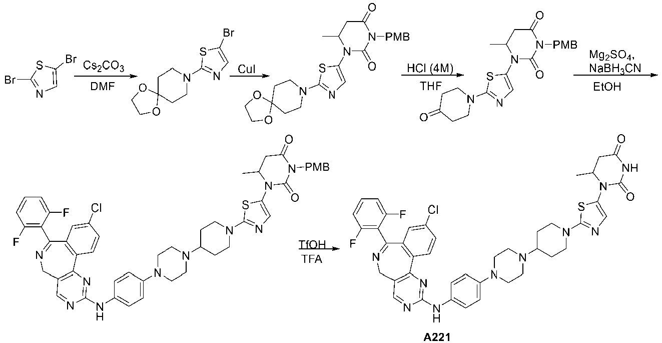

Compound **A221** (7.61 mg, yield 21.87%) was synthesized according to the synthesis method of compound **A132** described in **Example 131.** LCMS (ESI) m/z = 890.5 [M+H]⁺. ¹H NMR (400 MHz, DMSO-*d*₆) δ 10.62 (s, 1H), 9.58 (s, 1H), 8.58 (s, 1H), 8.25 (d, *J* = 8.4 Hz, 1H), 7.84 (dd, *J*₁ = 8.4, *J*₂ = 2.0 Hz, 1H), 7.62 (d, *J* = 8.8 Hz, 2H), 7.59 - 7.48 (m, 1H), 7.30 (d, *J* = 2.0 Hz, 1H), 7.19 - 7.12 (m, 2H), 7.04 (s, 1H), 6.91 (d, *J* = 8.8 Hz, 2H), 4.14 - 4.03 (m, 1H), 3.88 (d, *J* = 12.0 Hz, 2H), 3.12 - 2.91 (m, 7H), 2.68 - 2.64 (m, 4H), 2.47 - 2.28 (m, 2H), 1.93 - 1.86 (m, 2H), 1.54 - 1.48 (m, 2H), 1.18 (d, *J* = 6.4 Hz, 3H).

### Example 221 Synthesis of Compound A222:

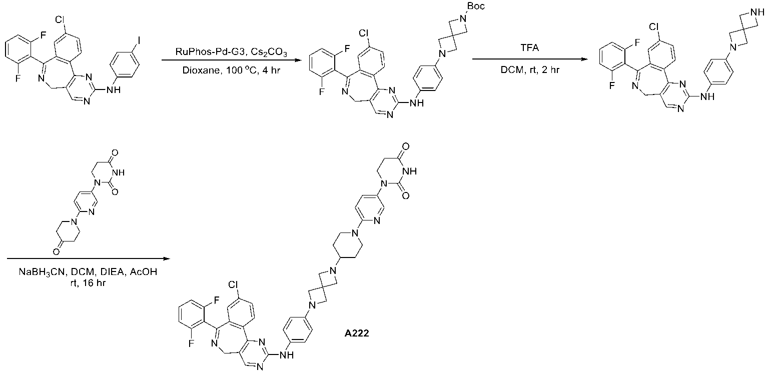

Compound **A222** (8 mg, yield 16.0%) was synthesized according to the synthesis method of compound **A132** described in **Example 131.** LCMS (ESI) m/z = 801.5 [M+H]⁺. ¹H NMR (400 MHz, DMSO-*d*₆) δ 10.15 (s, 1H), 9.30 (s, 1H), 8.56 (s, 1H), 8.27 (d, *J* = 8.4 Hz, 1H), 8.08 (s, 1H), 7.86 - 7.81 (m, 1H), 7.61 - 7.46 (m, 4H), 7.30 (s, 1H), 7.20 - 7.12 (m, 2H), 6.84 (d, *J =* 9.2 Hz, 1H), 6.45 (d, *J =* 8.4 Hz, 2H), 4.09 - 4.00 (m, 2H), 3.87 (s, 4H), 3.73 (t, *J* = 6.8 Hz, 2H), 3.32 (s, 4H), 3.08 - 3.00 (m, 2H), 2.74 (d, *J* = 6.8 Hz, 2H), 2.31 - 2.17 (m, 1H), 1.75 - 1.67 (m, 2H), 1.24 - 1.16 (m, 2H).

### Example 222 Synthesis of Compound A223:

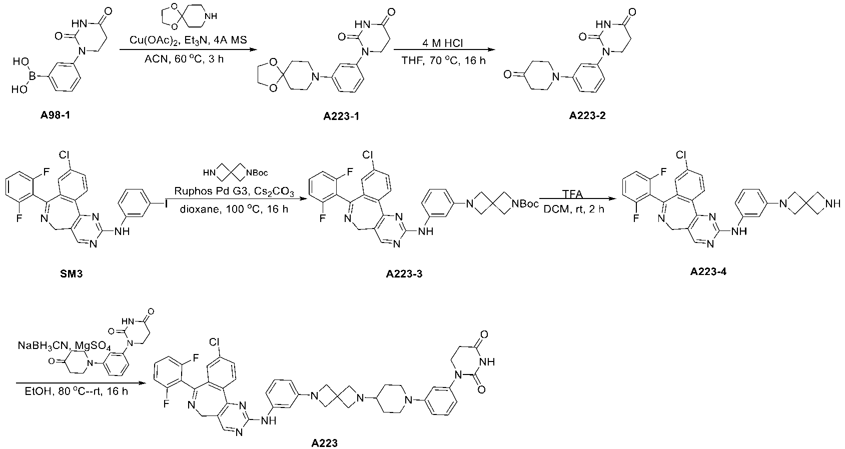

### Step 1:

At room temperature, 4-piperidone ethylene ketal (685 mg, 4.79 mmol), copper acetate (627 mg, 2.39 mmol), triethylamine (725 mg, 7.18 mmol) and 4Å molecular sieve (1.6 g) were added to compound **A98-1** (800 mg, 2.39 mmol, 70%) in acetonitrile (16 mL). The reaction mixture was heated to 60°C and stirred for 4 h. The reaction mixture was filtered, the filtrate was concentrated under reduced pressure, and the residue was separated for purification by silica gel column chromatography to give a yellow solid **A223-1** (330 mg, yield 42%). LCMS (ESI) m/z = 332.4 [M+H]⁺.

### Step 2:

At room temperature, HCl (4 M, 2 mL) was added to a solution of compound **A223-1** (330 mg, 1.00 mmol) in tetrahydrofuran (4 mL), and the reaction mixture was heated to 70°C and stirred for 16 h. The reaction mixture was adjusted to weak alkalinity with a saturated aqueous sodium bicarbonate solution, extracted with dichloromethane, washed with saturated brine, dried over anhydrous sodium sulfate, and concentrated under reduced pressure. The residue was purified by silica gel column chromatography to give a yellow solid **A223-2** (150 mg, yield 52%). LCMS (ESI) m/z = 288.2 [M+H]⁺.

### Step 3:

To a solution of compound **SM3** (250 mg, 0.45 mmol) in 1,4-dioxane (5 mL), tert-butyl 2,6-diazaspiro[3.3]heptane-2-carboxylate (159 mg, 0.67 mmol), methanesulfonato (2-dicyclohexylphosphino-2',6'-diisopropoxy-1,1'-biphenyl)(2-amino-1,1'-biphenyl-2-yl) palladium (II) (37 mg, 0.04 mmol) and cesium carbonate (437 mg, 1.34 mmol) were added at room temperature. The reaction mixture was heated to 100°C under argon protection and stirred for 16 h. The reaction mixture was filtered, the filtrate was concentrated under reduced pressure, and the residue was separated for purification by silica gel column chromatography to give a yellow solid **A223-3** (130 mg, yield 46%). LCMS (ESI) m/z = 429.2 [M+H]⁺.

### Step 4:

Trifluoroacetic acid (1 mL) was added to a solution of compound **A223-3** (130 mg, 0.21 mmol) in dichloromethane (2 mL) at room temperature. The reaction mixture was stirred at room temperature for 2 h. The reaction mixture was concentrated under reduced pressure, and saturated aqueous sodium bicarbonate solution (10 mL) was added to the residue. The mixture was extracted with dichloromethane, washed with saturated brine, dried over anhydrous sodium sulfate, and concentrated under reduced pressure to give a yellow solid **A223-4** (110 mg, yield 100%, crude product). LCMS (ESI) m/z = 529.3 [M+H]⁺.

### Step 5:

**A223-2** (33 mg, 0.11 mmol) and magnesium sulfate (137 mg, 1.13 mmol) were added to a solution of compound **A223-4** (30 mg, 0.06 mmol) in ethanol (2 mL) at room temperature. The reaction mixture was heated to 80°C under argon protection and stirred for 16 h. The mixture was cooled to room temperature. With sodium cyanoborohydride (11 mg, 0.17 mmol) added, the reaction mixture was stirred at room temperature for 16 h under argon protection. After the addition of water, the reaction mixture was extracted with dichloromethane, washed with saturated brine, dried over anhydrous sodium sulfate, and concentrated under reduced pressure. The residue was separated for purification by reverse phase chromatography (acetonitrile/10 mM ammonium bicarbonate solution) to give a yellow solid **A223** (5 mg, yield 11%). LCMS (ESI) m/z = 800.4 [M+H]⁺. ¹H NMR (400 MHz, DMSO-*d*₆) δ 10.31 (s, 1H), 9.72 (s, 1H), 8.67 (s, 1H), 8.32 (d, *J* = 8.4 Hz, 1H), 7.99 - 7.82 (m, 1H), 7.68 - 7.49 (m, 1H), 7.35 (d, *J =* 2.4 Hz, 1H), 7.29 - 7.04 (m, 6H), 6.94 - 6.87 (m, 1H), 6.87 - 6.80 (m, 1H), 6.78 - 6.66 (m, 1H), 6.15 - 6.04 (m, 1H), 3.89 (s, 4H), 3.77 (t, *J* = 6.4 Hz, 2H), 3.67 - 3.52 (m, 2H), 3.32 (s, 4H), 2.88 - 2.74 (m, 2H), 2.71 (t, *J =* 6.4 Hz, 2H), 2.24 - 2.11 (m, 1H), 1.82 - 1.66 (m, 2H), 1.36 - 1.21 (m, 2H).

### Example 223 Synthesis of Compound A224:

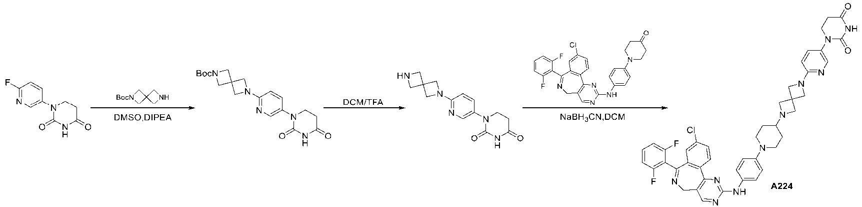

Compound **A224** (29.8 mg, yield 32.4%) was synthesized according to the synthesis method of compound **A132** described in **Example 131.** LCMS (ESI) m/z = 801.4 [M+H]⁺. ¹H NMR (400 MHz, DMSO-*d*₆) δ 10.33 (s, 1H), 9.57 (s, 1H), 8.58 (s, 1H), 8.25 (d, *J* = 8.0 Hz, 1H), 8.02 - 8.00 (m, 1H), 7.87 - 7.82 (m, 1H), 7.61 (d, *J* = 8.0 Hz, 2H), 7.57 - 7.51 (m, 1H), 7.50 - 7.46 (m, 1H), 7.32 - 7.29 (m, 1H), 7.21 - 7.12 (m, 2H), 6.91 (d, *J* = 8.8 Hz, 2H), 6.42 - 6.38 (m, 1H), 3.99 (s, 4H), 3.68 (t, *J* = 6.8 Hz, 2H), 3.54 - 3.44 (m, 2H), 3.29 (s, 4H), 2.72 - 2.63 (m, 4H), 2.14 - 2.03 (m, 1H), 1.76 - 1.65 (m, 2H), 1.28 - 1.20 (m, 2H).

### Example 224 Synthesis of Compound A225:

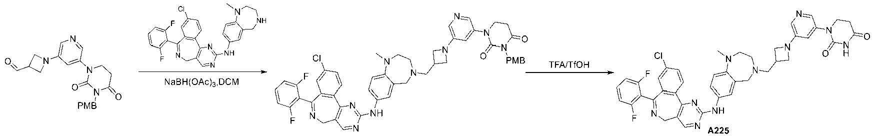

Compound **A225** (11.2 mg, yield 23.1%) was synthesized according to the synthesis method of compound **A132** described in **Example 131.** LCMS (ESI) m/z = 775.6 [M+H]⁺. ¹H NMR (400 MHz, DMSO-*d*₆) δ 10.41 (s, 1H), 9.59 (s, 1H), 8.59 (s, 1H), 8.29 (d, *J* = 8.0 Hz, 1H), 7.89 - 7.81 (m, 2H), 7.65 - 7.60 (m, 2H), 7.59 - 7.48 (m, 2H), 7.29 - 7.24 (m, 1H), 7.14 (t, *J* = 8.8 Hz, 2H), 6.89 - 6.83 (m, 1H), 6.74 - 6.70 (m, 1H), 5.75 (s, 3H), 3.87 (s, 2H), 3.76 - 3.71 (m, 4H), 3.49 (s, 2H), 3.03 - 2.94 (m, 1H), 2.90 - 2.84 (m, 4H), 2.80 (s, 3H), 2.70 - 2.65 (m, 2H), 2.64 - 2.59 (m, 2H).

### Example 225 Synthesis of Compound A226:

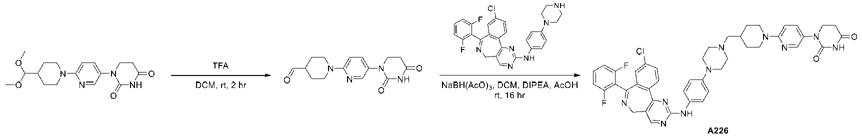

Compound **A226** (17 mg, yield 31.2%) was synthesized according to the synthesis method of compound **A132** described in **Example 131.** LCMS (ESI) m/z = 803.5 [M+H]⁺. ¹H NMR (400 MHz, DMSO-*d*₆) δ 10.36 (s, 1H), 9.62 (s, 1H), 8.61 (s, 1H), 8.28 (d, *J* = 8.4 Hz, 1H), 8.07 (d, *J* = 2.8 Hz, 1H), 7.89 - 7.85 (m, 1H), 7.68 - 7.63 (m, 2H), 7.60 - 7.54 (m, 1H), 7.52 - 7.46 (m, 1H), 7.34 - 7.32 (m, 1H), 7.23 - 7.15 (m, 2H), 6.97 - 6.92 (m, 2H), 6.86 (d, *J* = 9.2 Hz, 1H), 4.30 (d, *J* = 12.8 Hz, 2H), 3.72 (t, *J* = 6.8 Hz, 2H), 3.39 - 3.35 (m, 1H), 3.33 - 3.29 (m, 1H), 3.14 - 3.07 (m, 4H), 2.88 - 2.78 (m, 2H), 2.73 (t, *J* = 6.8 Hz, 2H), 2.56 - 2.54 (m, 2H), 2.26 - 2.19 (m, 2H), 1.89 - 1.76 (m, 3H), 1.19 - 1.06 (m, 2H).

### Example 226 Synthesis of Compound A227:

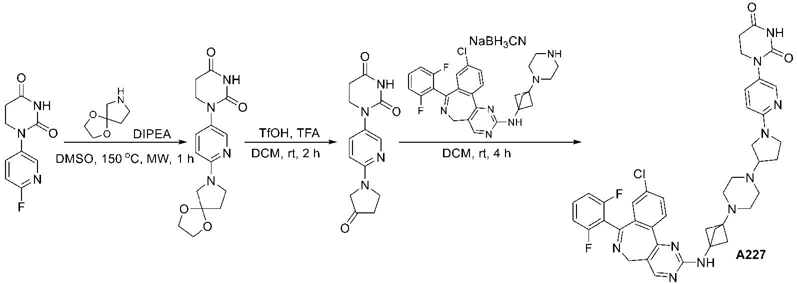

Compound **A227** (7 mg, yield 15%) was synthesized according to the synthesis method of compound **A132** described in **Example 131.** LCMS (ESI) m/z = 765.4 [M+H]⁺. ¹H NMR (400 MHz, DMSO-*d*₆) δ 10.33 (s, 1H), 8.50 (s, 1H), 8.31 - 8.15 (m, 1H), 8.11 (s, 1H), 8.03 (d, *J* = 2.4 Hz, 1H), 7.98 - 7.81 (m, 1H), 7.64 - 7.51 (m, 1H), 7.51 - 7.43 (m, 1H), 7.30 (d, *J* = 2.0 Hz, 1H), 7.19 (t, *J* = 8.8 Hz, 2H), 6.49 (d, *J* = 8.8 Hz, 1H), 3.80 - 3.64 (m, 3H), 3.64 - 54 (m, 1H), 3.14 (t, *J* = 8.8 Hz, 1H), 2.98 - 2.84 (m, 1H), 2.72 (t, *J* = 6.4 Hz, 2H), 2.63 - 2.54 (m, 3H), 2.52 - 2.35 (m, 6H), 2.28 - 2.16 (m, 1H), 2.08 (s, 6H), 1.92 - 1.71 (m, 1H).

### Example 227 Synthesis of Compound A228:

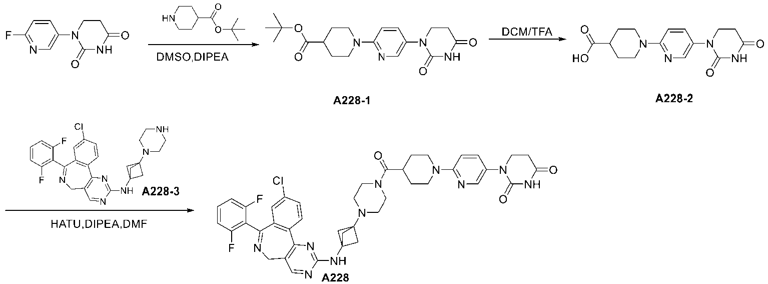

### Step 1:

1-(6-fluoropyridin-3-yl)dihydropyrimidine-2,4(1H,3H)-dione (300 mg, 1.43 mmol), tert-butyl piperidine-4-carboxylate (320 mg, 1.72 mmol), *N,N*-diisopropylethylamine (370 mg, 2.87 mmol) and dimethyl sulfoxide (2 mL) were added to a 5 mL microwave tube at room temperature. The reaction mixture was stirred at 150°C under microwave conditions for 1 h. LC-MS showed that the raw materials reacted completely. The reaction mixture was filtered, and the filtrate was separated for purification by reverse phase chromatography (acetonitrile/10 mM aqueous ammonium bicarbonate solution) to give a yellow solid **A228-1** (420 mg, yield 78.2%). LCMS (ESI) m/z = 375.0 [M+H]⁺.

### Step 2:

Trifluoroacetic acid (1 mL) was added to a solution of compound **A228-1** (30 mg, 0.08 mmol) in dichloromethane (2 mL) at room temperature. The reaction mixture was stirred for 2 h. The reaction mixture was concentrated under reduced pressure to give a yellow solid **228-2** (25 mg, yield 99%). This crude product was directly used in the next reaction.

### Step 3:

**A228-2** (9.5 mg, 0.03 mmol), HATU (12 mg, 0.03 mmol) and *N,N*-diisopropylethylamine (8 mg, 0.06 mmol) were added to a solution of compound **A228-3** (10 mg, 0.02 mmol) in *N,N*-dimethylformamide (2 mL) at room temperature. The reaction mixture was stirred at room temperature for 2 h. The reaction mixture was separated for purification by reverse phase chromatography (acetonitrile/10 mM aqueous ammonium bicarbonate solution) to give a light yellow solid **A228** (5.3 mg, yield 33.2%). LCMS (ESI) m/z = 807.6 [M+H]⁺. ¹H NMR (400 MHz, DMSO-*d*₆) δ 10.33 (s, 1H), 8.47 (s, 1H), 8.19 (s, 1H), 8.10 (s, 1H), 8.06 - 8.03 (m, 1H), 7.86 - 7.80 (m, 1H), 7.58 - 7.51 (m, 1H), 7.50 - 7.46 (m, 1H), 7.29 - 7.25 (m, 1H), 7.16 (t, *J* = 8.8 Hz, 2H), 6.88 - 6.83 (m, 1H), 4.32 - 4.24 (m, 2H), 3.70 (t, *J* = 6.8 Hz, 2H), 3.57 (s, 2H), 3.48 (s, 2H), 2.93 - 2.86 (m, 3H), 2.70 (t, *J* = 6.8 Hz, 2H), 2.43 (s, 2H), 2.35 (s, 2H), 2.06 (s, 6H), 1.69 - 1.61 (m, 2H), 1.59 - 1.46 (m, 2H).

### Example 228 Synthesis of Compound A229:

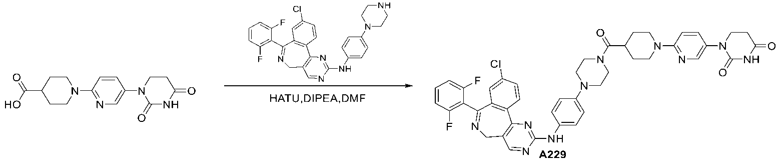

Compound **A229** (10.2 mg, yield 43.0%) was synthesized according to the synthesis method of compound **A228** described in **Example 227.** LCMS (ESI) m/z = 817.6 [M+H]⁺. ¹H NMR (400 MHz, DMSO-*d*₆) δ 10.33 (s, 1H), 9.64 (s, 1H), 8.59 (s, 1H), 8.25 (d, *J* = 8.0 Hz, 1H), 8.07 - 8.04 (m, 1H), 7.87 - 7.82 (m, 1H), 7.70 - 7.62 (m, 2H), 7.59 - 7.46 (m, 2H), 7.32 - 7.28 (m, 1H), 7.21 - 7.10 (m, 2H), 6.99 - 6.93 (m, 2H), 6.89 - 6.83 (m, 1H), 4.34 - 4.23 (m, 2H), 3.74 - 3.68 (m, 4H), 3.61 (s, 2H), 3.10 (s, 2H), 3.03 (s, 2H), 3.00 - 2.84 (m, 3H), 2.72 - 2.67 (m, 2H), 1.73 - 1.66 (m, 2H), 1.62 - 1.50 (m, 2H).

### Example 229 Synthesis of Compound A230:

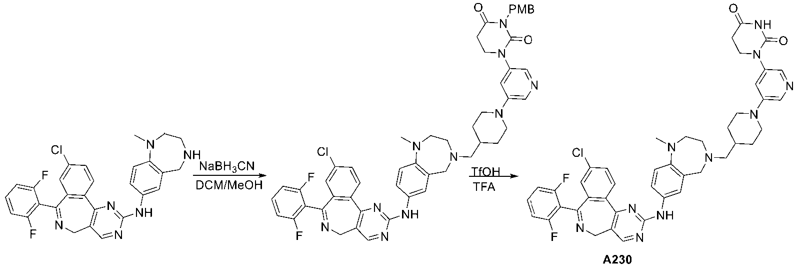

Compound **A227** (7.81 mg, yield 22.99%) was synthesized according to the synthesis method of compound **A132** described in **Example 131.** LCMS (ESI) m/z = 803.63 [M+H]⁺. ¹H NMR (400 MHz, DMSO-*d*₆) δ 10.41 (s, 1H), 9.60 (s, 1H), 8.59 (s, 1H), 8.28 (d, *J* = 8.4 Hz, 1H), 8.07 (d, *J* = 2.4 Hz, 1H), 7.92 (d, *J =* 2.0 Hz, 1H), 7.86 (dd, *J*₁ = 8.4 Hz, *J*₂ = 2.0 Hz, 1H), 7.68 - 7.62 (m, 1H), 7.61 - 7.56 (m, 1H), 7.55 - 7.46 (m, 1H), 7.28 (d, *J =* 2.0 Hz, 1H), 7.24 - 7.19 (m, 1H), 7.14 - 7.09 (m, 2H), 6.86 (d, *J =* 8.8 Hz, 1H), 3.76 (t, *J =* 6.4 Hz, 2H), 3.73 - 3.62 (m, 4H), 2.92 - 2.83 (m, 4H), 2.80 (s, 3H), 2.69 (t, *J =* 6.4 Hz, 2H), 2.67 - 2.53 (m, 2H), 2.25 - 2.19 (m, 2H), 1.82 - 1.59 (m, 3H), 1.21 - 1.07 (m, 2H).

### Example 230 Synthesis of Compound A231:

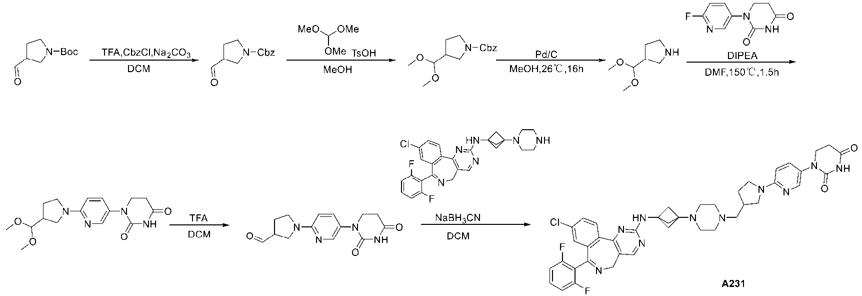

Compound **A231** (15.0 mg, yield 48%) was synthesized according to the synthesis method of compound **A132** described in **Example 131.** LCMS (ESI) m/z = 779.3 [M+H]⁺. ¹H NMR (400 MHz, DMSO-*d*₆) δ 10.31 (s, 1H), 8.49 (s, 1H), 8.10 (s, 1H), 8.01 (d, *J =* 2.8 Hz, 1H), 7.86 (d, *J* = 8.4 Hz, 1H), 7.59 - 7.55 (m, 1H), 7.45 (dd, *J*₁ = 8.4 Hz, *J*₂ = 2.0 Hz, 1H), 7.32 - 7.30 (m, 1H), 7.20 - 7.18 (m, 2H), 6.45 (d, *J* = 9.2 Hz, 1H), 3.70 - 3.67 (m, 4H), 3.57 - 3.52 (m, 2H), 3.50 - 3.48 (m, 2H), 3.13 - 3.09 (m, 2H), 2.73 - 2.69 (m, 4H), 2.45 - 2.42 (m, 4H), 2.36 - 2.34 (m, 2H), 2.10 (s, 6H), 1.73 - 1.68 (m, 1H).

### Example 231 Synthesis of Compound A232:

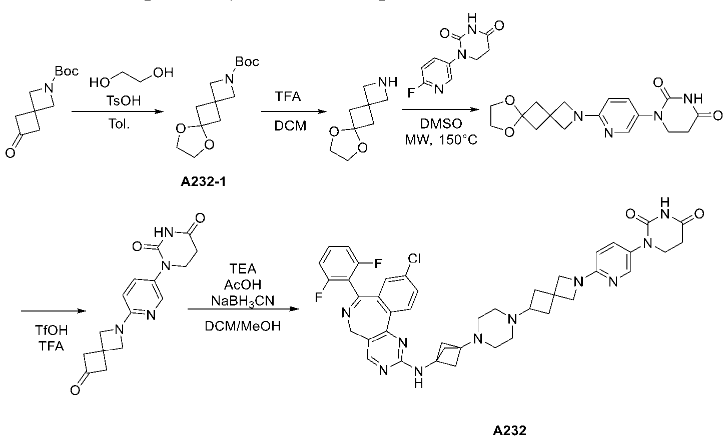

### Step 1:

To a solution of compound tert-butyl 6-oxo-2-azaspiro[3.3]heptane-2-carboxylate (1.0 g, 4.73 mmol) in toluene (20 mL), ethylene glycol (2.35 g, 37.87 mmol) and p-toluenesulfonic acid monohydrate (450.21 mg, 2.37 mmol) were added at room temperature. The reaction mixture was heated to 145°C with a water separator under argon protection and stirred for 4 h. After the addition of water, the reaction mixture was extracted with ethyl acetate, dried over anhydrous sodium sulfate, and concentrated under reduced pressure to give a white solid **A232-1** (530 mg, yield 43.8%). LCMS (ESI) m/z = 200.1 [M+H].

### Step 2:

Compound **A232** (10.5 mg, yield 22.4%) was synthesized according to the method of compound A132 described in **Example 131.** LCMS (ESI) m/z = 791.6 [M+H]. ¹H NMR (400 MHz, DMSO-*d*₆) δ 10.32 (s, 1H), 8.47 (s, 1H), 8.21 - 8.18 (m, 1H), 8.08 (s, 1H), 7.99 (d, *J* = 2.6 Hz, 1H), 7.84 (d, *J* = 8.4 Hz, 1H), 7.57 - 7.49 (m, 1H), 7.46 (dd, *J*₁ = 8.8 Hz, *J*₂ = 2.6 Hz, 1H), 7.27 (d, *J* = 2.1 Hz, 1H), 7.22 - 7.13 (m, 2H), 6.36 (d, *J* = 8.8 Hz, 1H), 3.95 (s, 2H), 3.83 (s, 2H), 3.67 (t, *J* = 6.6 Hz, 2H), 2.69 (t, *J* = 6.6 Hz, 2H), 2.64 - 2.57 (m, 1H), 2.40 (bs, 4H), 2.31 - 2.26 (m, 5H), 2.07 - 1.94 (m, 8H).

### Example 232 Synthesis of Compound A233:

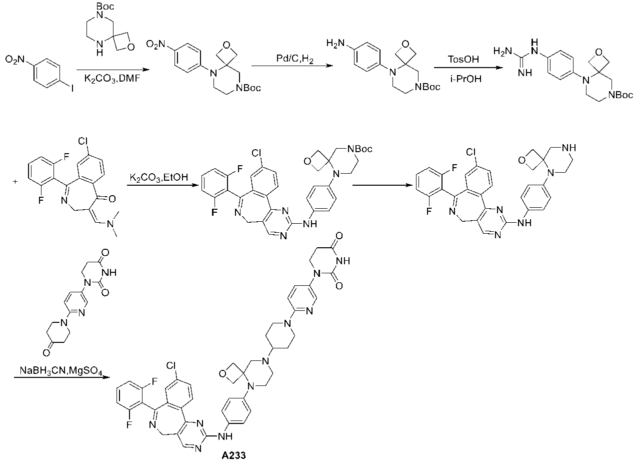

Compound **A233** (6.7 mg, yield 21%) was synthesized according to the method of compound A150 described in **Example 149.** LCMS (ESI) m/z = 831.3 [M+H]. ¹H NMR (400 MHz, DMSO-*d*₆) δ 10.35 (s, 1H), 9.73 (s, 1H), 8.63 (s, 1H), 8.28 (d, *J* = 8.4 H, 1H), 8.06 (d, *J* = 2.0 Hz, 1H), 7.88 (dd, *J*₁ = 8.4 Hz, *J*₂ = 2.2 Hz, 1H), 7.79 (d, *J* = 8.8 Hz, 2H), 7.57 - 7.49 (m, 1H), 7.32 (d, *J* = 3.2 Hz, 1H), 7.22 - 7.16 (m, 2H), 6.88 (d,*J* = 9.2 Hz, 1H), 6.81 (d, *J* = 8.8 Hz, 2H), 4.50 (d, *J* = 7.2 Hz, 2H), 4.37 (d, *J =* 7.0 Hz, 2H),4.33 - 4.30 (m, 2H), 3.71 (t, *J =* 6.8 Hz, 2H), 3.15 (bs, 2H), 2.97 (bs, 2H), 2.85 (t, *J =* 11.6 Hz, 2H), 2.71 (t, *J =* 6.8 Hz, 2H), 2.56 - 2.51 (m, 1H), 2.44 (d, 2H), 1.86 - 1.84 (m, 2H), 1.49 - 1.44 (m, 2H).

### Example 233 Synthesis of Compound A234:

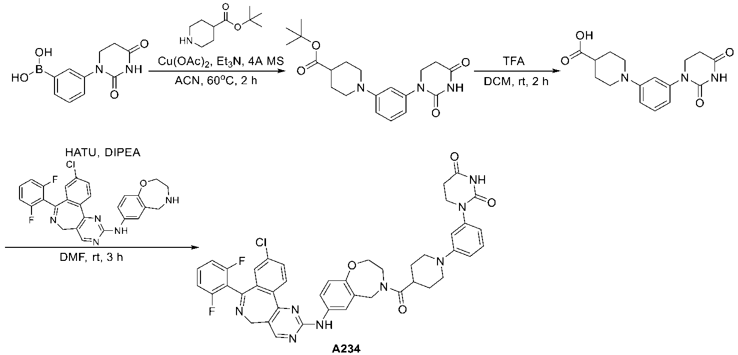

Compound **A234** (13 mg, yield 41%) was synthesized according to the method of compound **A228** described in **Example 227.** LCMS (ESI) m/z = 803.4 [M+H]⁺. ¹H NMR (400 MHz, DMSO-*d*₆) δ 10.30 (d, *J* = 5.6 Hz, 1H), 9.96 - 9.72 (m, 1H), 8.71 - 8.60 (m, 1H), 8.43 - 8.27 (m, 1H), 7.97 - 7.85 (m, 1.5H), 7.78 (s, 0.5H), 7.73 - 7.64 (m, 0.5H), 7.62 - 7.45 (m, 1.5H), 7.38 - 7.30 (m, 1H), 7.26 - 7.05 (m, 3H), 7.04 - 6.93 (m, 1H), 6.92 - 6.78 (m, 1.5H), 6.78 - 6.65 (m, 1.5H), 4.82 - 4.52 (m, 2H), 4.25 - 3.84 (m, 4H), 3.80 - 3.60 (m, 4H), 3.03 - 2.59 (m, 5H), 1.76 - 1.53 (m, 4H).

### Example 234 Synthesis of Compound A235:

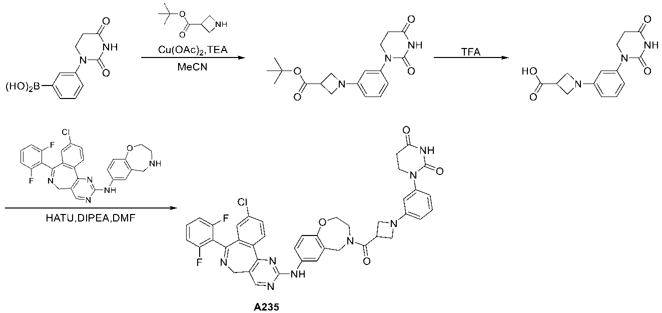

Compound **A235** (5.6 mg, yield 28.0%) was synthesized according to the method of compound **A228** described in **Example 227.** LCMS (ESI) m/z = 775.5 [M+H]⁺. ¹H NMR (400 MHz, DMSO-*d*₆) δ 10.26 (s, 1H), 9.83 - 9.74 (m, 1H), 8.65 - 8.61 (m, 1H), 8.39 - 8.27 (m, 1H), 7.93 - 7.86 (m, 1H), 7.83 - 7.76 (m, 1H), 7.64 - 7.47 (m, 2H), 7.30 - 7.27 (m, 1H), 7.20 - 7.08 (m, 3H), 7.00 - 6.93 (m, 1H), 6.64 - 6.58 (m, 1H), 6.40 - 6.22 (m, 2H), 4.63 - 4.50 (m, 2H), 4.10 - 3.94 (m, 4H), 3.91 - 3.82 (m, 3H), 3.77 - 3.67 (m, 4H), 2.68 - 2.62 (m, 2H).

### Example 235 Synthesis of Compound A236:

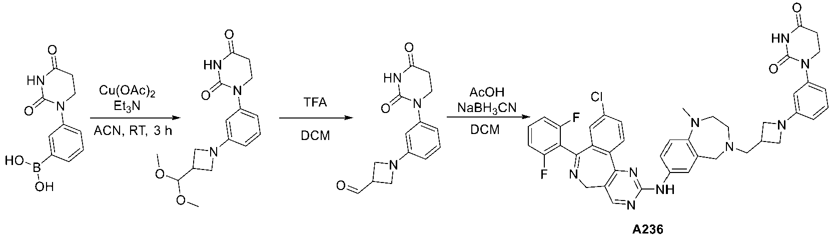

Compound **A236** (20.0 mg, yield 33.3%) was synthesized according to the method of compound **A194** described in **Example 193.** LCMS (ESI) m/z = 774.2 [M+H]. ¹H NMR (400 MHz, DMSO-*d*₆) δ 10.26 (s, 1H), 9.58 (s, 1H), 8.59 (s, 1H), 8.28 (d, *J* = 8.4 Hz, 1H), 7.84 (dd, *J*₁ = 8.4 Hz, *J*₂ = 2.2 Hz, 1H), 7.64 - 7.47 (m, 3H), 7.27 (d, *J* = 2.2 Hz, 1H), 7.16 - 7.08 (m, 3H), 6.86 (d, *J* = 8.7 Hz, 1H), 6.56 (d, *J* = 8.6 Hz, 1H), 6.32 - 6.31 (m, 1H), 6.23 (d, *J* = 8.6 Hz, 1H), 3.81 (s, 2H), 3.73 - 3.63 (m, 4H), 3.42 - 3.38 (m, 2H), 2.95 - 2.83 (m, 5H), 2.80 (s, 3H), 2.69 - 2.56 (m, 4H).

### Example 236 Synthesis of Compound A237:

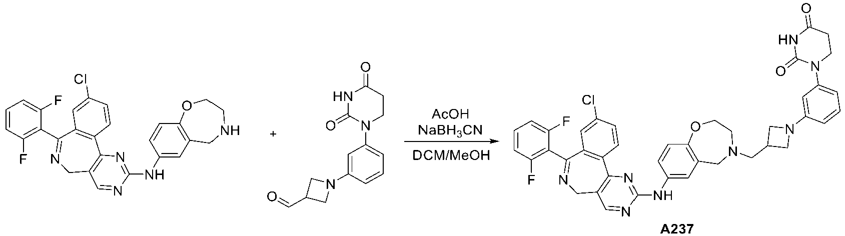

Compound **A237** (6.0 mg, yield 9.7%) was synthesized according to the method of compound A194 described in **Example 193.** LCMS (ESI) m/z = 761.4 [M+H]. ¹H NMR (400 MHz, DMSO-*d*₆) δ 10.26 (s, 1H), 9.71 (s, 1H), 8.62 (s, 1H), 8.28 (d, *J =* 8.4 Hz, 1H), 7.84 (dd, *J*₁ = 8.5 Hz, *J*₂ = 2.2 Hz, 1H), 7.68 (s, 1H), 7.55 - 7.50 (m, 2H), 7.28 (d, *J =* 2.2 Hz, 1H), 7.17 - 7.08 (m, 3H), 6.93 *(d, J =* 8.6 Hz, 1H), 6.57 (d, *J =* 8.4 Hz, 1H), 6.33 - 6.31 (m, 1H), 6.24 (d, *J* = 8.0 Hz, 1H), 3.95 (s, 2H), 3.83 (s, 2H), 3.78 (s, 2H), 3.69 (t, *J* = 6.6 Hz, 2H), 3.44 - 3.41 (m, 2H), 3.03 (s, 2H), 2.98 - 2.88 (m, 1H), 2.71 - 2.62 (m, 4H).

### Example 237 Synthesis of Compound A238:

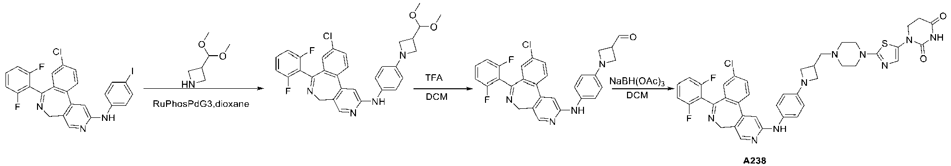

Compound **A238** (2.2 mg, yield 5%) was synthesized according to the method of compound **A194** described in **Example 193.** LCMS (ESI) m/z = 761.4 [M+H]⁺. ¹H NMR (400 MHz, DMSO-*d*₆) δ 10.61 (s, 1H), 9.49 (s, 1H), 8.57 (s, 1H), 8.25 (d, *J =* 8.8 Hz, 1H), 7.86 (d, *J =* 8.4 Hz, 1H), 7.58 - 7.56 (m, 3H), 7.32 (s, 1H), 7.21 - 7.17 (m, 2H), 7.02 (s, 1H), 6.64 (d, *J* = 9.2 Hz, 2H), 3.93 (t, *J =* 7.2 Hz, 2H), 3.81 (t, *J* = 6.8 Hz, 2H), 3.47 - 3.44 (m, 2H), 2.96 - 2.93 (m, 1H), 2.75 (t, *J* = 7.2 Hz, 2H), 2.66 - 2.65 (m, 2H).

### Example 238 Synthesis of Compound A239:

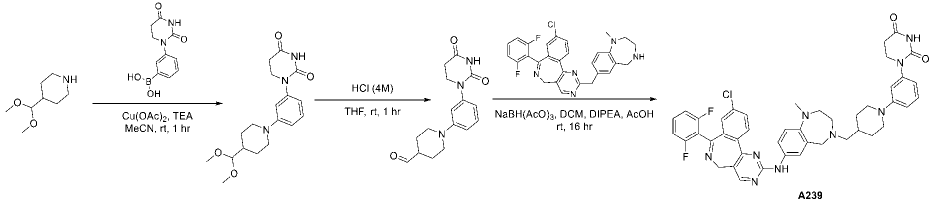

Compound **A239** (15 mg, yield 38.6%) was synthesized according to the synthesis method of compound **A194** described in **Example 193.** LCMS (ESI) m/z = 802.5 [M+H]⁺. ¹H NMR (400 MHz, DMSO-*d*₆) δ 10.29 (s, 1H), 9.63 (s, 1H), 8.62 (s, 1H), 8.31 (d, *J* = 8.4 Hz, 1H), 7.90 - 7.85 (m, 1H), 7.71 - 7.65 (m, 1H), 7.64 - 7.60 (m, 1H), 7.59 - 7.50 (m, 1H), 7.31 - 7.29 (m, 1H), 7.20 - 7.09 (m, 3H), 6.91 - 6.84 (m, 2H), 6.76 - 6.71 (m, 1H), 6.70 - 6.66 (m, 1H), 3.77 - 3.69 (m, 4H), 3.67 - 3.59 (m, 2H), 2.92 - 2.86 (m, 4H), 2.84 (s, 3H), 2.70 (t, *J =* 6.8 Hz, 2H), 2.62 - 2.54 (m, 4H), 2.29 - 2.20 (m, 2H), 1.82 - 1.61 (m, 2H), 1.23 - 1.10 (m, 1H).

### Example 239 Synthesis of Compound A240:

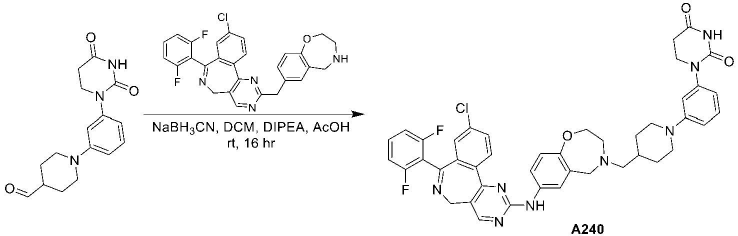

Compound **A240** (6 mg, yield 15.3%) was synthesized according to the synthesis method of compound **A194** described in **Example 193.** LCMS (ESI) m/z = 789.4 [M+H]⁺. ¹H NMR (400 MHz, DMSO-*d*₆) δ 10.29 (s, 1H), 9.75 (s, 1H), 8.65 (s, 1H), 8.30 (d, *J* = 8.4 Hz, 1H), 7.90 - 7.85 (m, 1H), 7.78 - 7.72 (m, 1H), 7.59 - 7.50 (m, 2H), 7.32 - 7.30 (m, 1H), 7.20 - 7.11 (m, 3H), 6.96 (d, *J* = 8.4 Hz, 1H), 6.87 - 6.83 (m, 1H), 6.75 - 6.71 (m, 1H), 6.70 - 6.66 (m, 1H), 4.01 - 3.94 (m, 2H), 3.79 (s, 2H), 3.73 (t, *J* = 6.8 Hz, 2H), 3.68 - 3.59 (m, 2H), 3.07 - 2.98 (m, 2H), 2.69 (t, *J* = 6.8 Hz, 2H), 2.64 - 2.54 (m, 4H), 2.37 - 2.28 (m, 2H), 1.83 - 1.59 (m, 2H), 1.23 - 1.12 (m, 1H).

### Example 240 Synthesis of Compound A241:

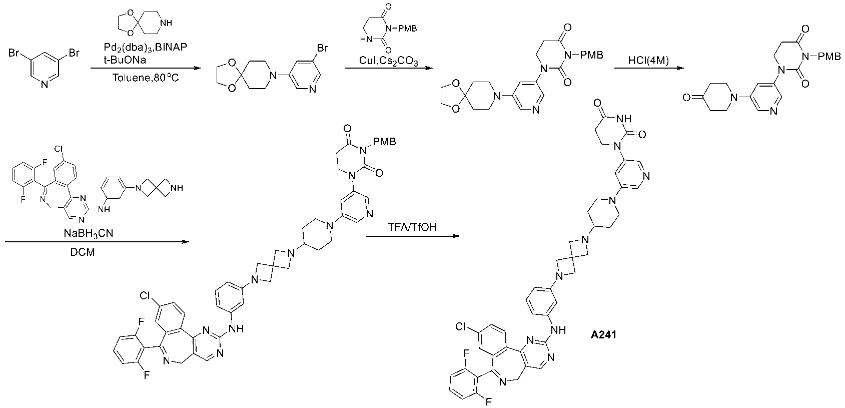

Compound **A241** (9.3 mg, yield 26.7%) was synthesized according to the synthesis method of compound **A194** described in **Example 193.** LCMS (ESI) m/z = 801.6 [M+H]⁺. ¹H NMR (400 MHz, DMSO-*d*₆) δ 10.42 (s, 1H), 9.69 (s, 1H), 8.64 (s, 1H), 8.29 (d, *J* = 8.0 Hz, 1H), 8.16 - 8.10 (m, 1H), 7.98 - 7.93 (m, 1H), 7.92 - 7.83 (m, 1H), 7.59 - 7.50 (m, 1H), 7.33 - 7.31 (m, 1H), 7.29 - 7.25 (m, 1H), 7.22 - 7.10 (m, 3H), 7.09 - 7.03 (m, 2H), 6.09 - 6.04 (m, 1H), 3.86 (s, 4H), 3.81 - 3.75 (m, 2H), 3.65 - 3.58 (m, 2H), 3.31 (s, 4H), 2.90 - 2.80 (m, 2H), 2.73 - 2.68 (m, 2H), 2.18 (s, 1H), 1.76 - 1.66 (m, 2H), 1.34 - 1.20 (m, 2H).

### Example 241 Synthesis of Compound A242:

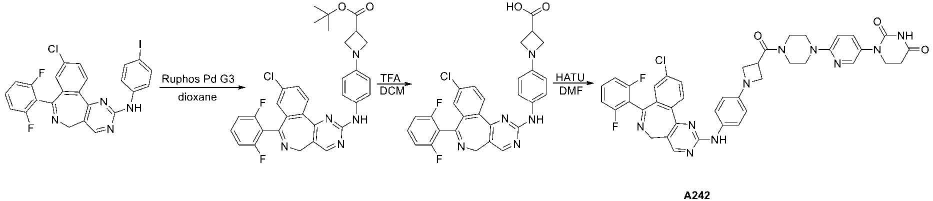

Compound **A242** (55 mg, yield 46.34%) was synthesized according to the synthesis method of compound **A1** described in **Example 1.** LCMS (ESI) m/z = 789.51 [M+H]⁺. ¹H NMR (400 MHz, DMSO-*d*₆) δ 10.34 (s, 1H), 9.49 (s, 1H), 8.56 (s, 1H), 8.24 (d, *J =* 8.4 Hz, 1H), 8.10 (d, *J =* 2.8 Hz, 1H), 7.84 (dd, *J*₁ = 8.4 Hz, *J*₂ = 2.0 Hz, 1H), 7.60 - 7.48 (m, 4H), 7.29 (d, *J* = 2.0 Hz, 1H), 7.20 - 7.12 (m, 2H), 6.90 (d, *J* = 9.2 Hz, 1H), 6.49 - 6.43 (m, 2H), 4.05 - 4.00 (m, 2H), 3.90 - 3.85 (m, 3H), 3.71 (t, *J* = 6.8 Hz, 2H), 3.63 - 3.39 (m, 8H), 2.70 (t, *J* = 6.8 Hz, 2H).

### Example 242 Synthesis of Compound A243:

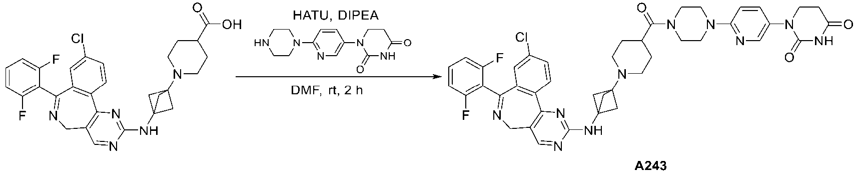

Compound **A243** (2.6 mg, yield 15%) was synthesized according to the synthesis method of compound **A109** described in **Example 109.** LCMS (ESI) m/z = 807.4 [M+H]⁺. ¹H NMR (400 MHz, DMSO-*d*₆) δ 10.36 (s, 1H), 8.50 (s, 1H), 8.23 (d, *J =* 8.4 Hz, 1H), 8.12 (d, *J =* 2.8 Hz, 1H), 8.08 (s, 1H), 7.87 (d, *J =* 8.4 Hz, 1H), 7.64 - 7.50 (m, 2H), 7.30 (d, *J =* 2.0 Hz, 1H), 7.19 (t, *J =* 8.8 Hz, 2H), 6.91 (d, *J* = 9.2 Hz, 1H), 3.74 (t, *J =* 6.8 Hz, 2H), 3.69 - 3.46 (m, 8H), 2.98 - 2.82 (m, 2H), 2.73 (t, *J =* 6.4 Hz, 2H), 2.68 - 2.57 (m, 1H), 2.12 - 2.05 (m, 8H), 1.76 - 1.49 (m, 4H).

### Example 243 Synthesis of Compound A244:

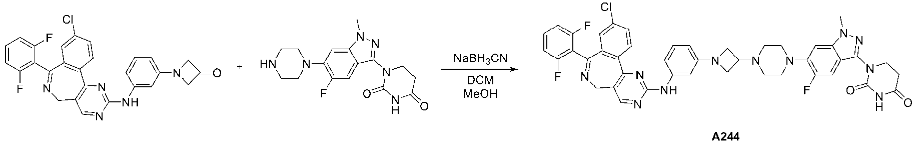

Compound **A244** (7.0 mg, yield 12.0%) was synthesized according to the method of compound **A132** described in **Example 131.** LCMS (ESI) m/z = 832.5 [M+H]⁺. ¹H NMR (400 MHz, DMSO-*d*₆) δ 10.52 (s, 1H), 9.70 (s, 1H), 8.65 (s, 1H), 8.31 (d, *J* = 8.4 Hz, 1H), 7.85 (dd, *J*₁ = 8.4 Hz, *J*₂ = 2.2 Hz, 1H), 7.59 - 7.49 (m, 1H), 7.38 - 7.32 (m, 2H), 7.24 - 7.02 (m, 6H), 6.12 (d, *J* = 7.6 Hz, 1H), 3.98 - 3.95 (m, 2H), 3.94 (s, 3H), 3.89 (t, *J* = 6.6 Hz, 2H), 3.65 - 3.62 (m, 2H), 3.13 - 3.10 (m, 4H), 2.73 (t, *J* = 6.6 Hz, 2H), 2.59 - 2.55 (m, 4H).

### Example 244 Synthesis of Compound A245:

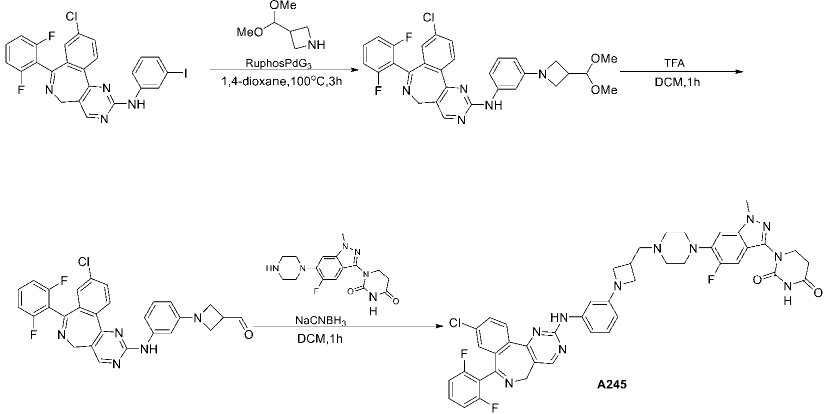

Compound **A245** (10.0 mg, yield 15%) was synthesized according to the synthesis method of compound **A223** described in **Example 222.** LCMS (ESI) m/z = 846.3 [M+H]⁺. ¹H NMR (400 MHz, DMSO-*d*₆) δ 10.55 (s, 1H), 9.74 (s, 1H), 8.68 (s, 1H), 8.37 (d, *J* = 8.4 Hz, 1H), 7.89 (dd, *J*₁ = 8.8 Hz, *J*₂ = 2.8 Hz, 1H), 7.60 - 7.52 (m, 1H), 7.42 - 7.38 (m, 2H), 7.38 - 7.34 (m, 1H), 7.23 - 7.17 (m, 2H), 7.14 - 7.08 (m, 2H), 6.98 (d, *J =* 8.0 Hz, 1H), 6.10 (d, *J* = 7.6 Hz, 1H), 4.05 - 3.98 (m, 2H), 3.96 (s, 3H), 3.92 (t, *J* = 6.8 Hz, 2H), 3.55 (t, *J =* 6.4 Hz, 2H), 3.17 - 3.11 (m, 4H), 3.08 - 2.97 (m, 1H), 2.80 - 2.75 (m, 2H), 2.75 - 2.70 (m, 2H), 2.69 - 2.60 (m, 4H).

### Example 245 Synthesis of Compound A246:

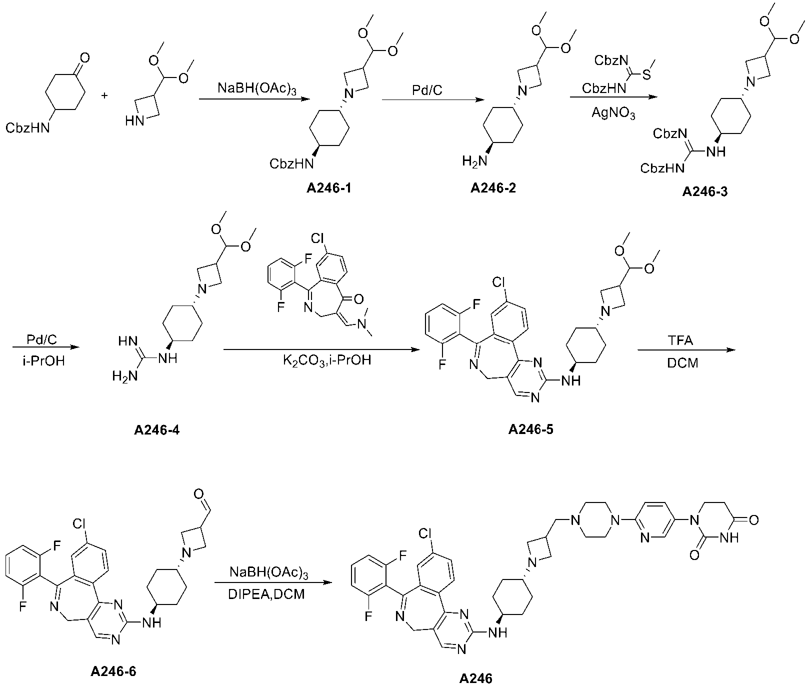

### Step 1:

4-*N*-benzyloxycarbonylaminocyclohexanone (1g, 4.04 mmol) and 3-(dimethoxymethyl)azetidine (795 mg, 6.07 mmol) were dissolved in dichloromethane (20 mL), to which sodium triacetoxyborohydride (1.3 g, 6.07 mmol) was slowly added, and then the mixture was stirred at room temperature for 4 h. After the reaction was completed, the mixture was quenched with water and extracted with dichloromethane. The organic phase was spin-dried and purified by silica gel column chromatography to give 300 mg of cis product and 250 mg of trans product. LCMS (ESI) m/z = 363.3 [M+H]⁺.

### Step 2:

**A246-1** (200 mg, 551 mmol) was dissolved in isopropyl alcohol (5 mL), to which palladium-carbon catalyst (50 mg) was added, and then the mixture was stirred overnight in a hydrogen balloon atmosphere at room temperature. After the reaction was completed, the insoluble matter was filtered out, and the obtained filtrate was spin-dried and directly used in the next step.

### Step 3:

**A246-2** (200 mg, 0.875 mmol), 1,3-di(benzyloxycarbonyl)-2-methylisothiourea (470 mg, 1.31 mmol), silver nitrate (297 mg, 1.75 mmol) and triethylamine (265 mg, 2.65 mmol) were dissolved in dichloromethane (5 mL). Then the mixture was stirred at room temperature for 2 h. After the reaction was completed, the mixture was quenched with water and extracted with dichloromethane, and the organic phase was spin-dried and separated for purification by silica gel column chromatography to give **A246-3** (150 mg). LCMS (ESI) m/z = 539.3 [M+H]⁺.

### Step 4:

**A246-3** (150 mg) was dissolved in isopropyl alcohol (5 mL), palladium-carbon catalyst (20 mg) was added, and then the mixture was stirred overnight in a hydrogen balloon atmosphere at room temperature. After the reaction was completed, the insoluble matter was filtered out, and the obtained filtrate was spin-dried and directly used in the next step.

### Step 5:

**A246-4** (75 mg, 0.27 mmol), **SM4** (100 mg, 0.27 mmol) and potassium carbonate (76 mg, 0.54 mmol) were suspended in isopropyl alcohol (5 mL), and the mixture was stirred overnight at 80°C. After the reaction was completed, **A246-5** (75 mg) was obtained by direct spin-drying to remove solvent and separation for purification by silica gel column chromatography. LCMS (ESI) m/z = 568.2 [M+H]⁺.

### Step 6:

**A246-5** (75 mg) was dissolved in dichloromethane (2 mL), 0.5 mL of trifluoroacetic acid was added, and then the mixture was stirred at room temperature for 2 h. After the reaction was completed, with solvent removed by spin-drying, the product was directly used in the next step.

### Step 7:

**A246-6** (50 mg, 0.095 mmol) and 1-(4-(piperazin-1-yl)phenyl)dihydropyrimidine-2,4(1H,3H)-dione (52 mg, 0.192 mmol) were dissolved in dichloromethane (2 mL), sodium triacetoxyborohydride (40 mg, 0.19 mmol) was added, and the mixture was stirred at room temperature overnight. After the reaction was completed, the mixture was quenched with water and extracted with dichloromethane, the organic phase was spin-dried and purified by preparative reversed-phase high pressure chromatography to give **A246** (12 mg, yield 16.0%). LCMS (ESI) m/z = 781.3 [M+H]⁺. ¹H NMR (400 MHz, DMSO-*d*₆) δ 10.35 (s, 1H), 8.44 (s, 1H), 8.19 (bs, 1H), 8.07 (d, *J =* 2.8 Hz, 1H), 7.81 (d, *J* = 8.8 Hz, 1H),7.58 - 7.50 (m, 2H), 7.28 (d, *J* = 1.6 Hz, 2H), 7.18 (t, *J* = 8.8 Hz, 2H), 6.85 (d, *J* = 8.8 Hz, 1H), 3.72 (t , *J* = 6.8 Hz, 3H), 3.47 (bs, 4H), 2.75 - 2.70 (m, 4H), 2.57 - 2.50 (m, 2H), 2.43 (bs, 4H), 1.94 - 1.92 (m, 3H), 1.77 - 175 (m, 2H), 1.31 - 1.26 (m, 3H), 1.01 - 0.98 (m, 2H).

### Example 246 Synthesis of Compound A247:

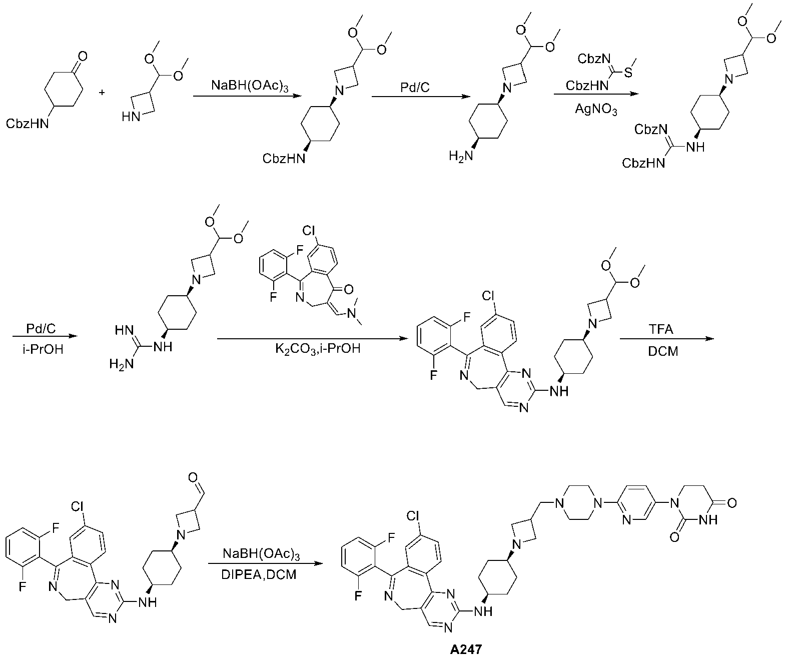

Compound **A247** (17 mg, yield 37%) was synthesized according to the synthesis method of compound **A246** described in **Example 245.** LCMS (ESI) m/z = 781.2 [M+H]⁺. ¹H NMR (400 MHz, DMSO-*d*₆) δ 10.37 (s, 1H), 8.44 (s, 1H), 8.20 (d, *J* = 2.8 Hz, 1H), 8.08 (d, *J* = 2.8 Hz, 1H), 7.81 (dd, *J*₁ = 8.8 Hz, *J*₂ *=* 2.4 Hz, 1H), 7.58 - 7.51 (m, 2H), 7.29 - 7.26 (m, 2H), 7.19 (t, *J* = 8.8 Hz, 2H), 6.86 (d, *J* = 8.8 Hz, 1H),3.85 (bs, 1H), 3.72 (t, *J* = 6.8 Hz, 2H), 3.47 (bs, 4H), 3.40 (bs, 1H), 2.75 - 2.70 (m, 4H), 2.57 - 2.50 (m, 2H), 2.43 (bs, 4H), 2.19 (bs, 1H), 1.94 - 1.92 (m, 3H), 1.77 - 175 (m, 2H), 1.31 - 1.26 (m, 3H), 1.01 - 0.98 (m, 2H).1.69 - 1.26 (m, 8H).

### Example 247 Synthesis of Compound A248:

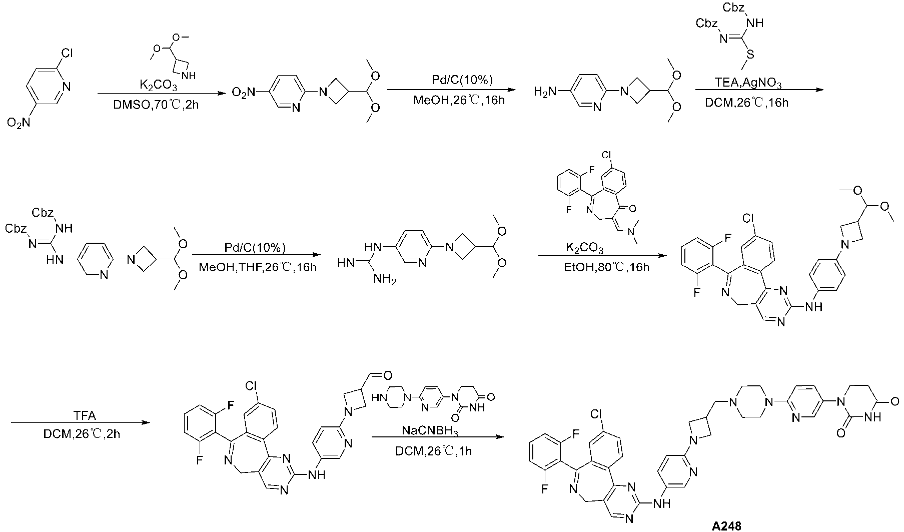

Compound **A248** (15.0 mg, yield 35%) was synthesized according to the synthesis method of compound **A185** described in **Example 184.** LCMS (ESI) m/z = 776.3 [M+H]⁺. ¹H NMR (400 MHz, DMSO-*d*₆) δ 10.37 (s, 1H), 9.56 (s, 1H), 8.61 (s, 1H), 8.46 (s, 1H), 8.25 (d, *J* = 8.4 Hz, 1H), 8.10 (d, *J* = 2.8 Hz, 1H), 7.92 - 7.85 (m, 2H), 7.63 - 7.56 (m, 1H), 7.56 - 7.52 (m, 1H), 7.34 (d, *J* = 2.4 Hz, 1H), 7.25 - 7.15 (m, 2H), 6.89 (d, *J* = 9.2 Hz, 1H), 6.45 (d, *J* = 8.8 Hz, 1H), 4.06 (t, *J* = 7.6 Hz, 2H), 3.74 (t, *J* = 6.8 Hz, 2H), 3.65 - 3.59 (m, 2H), 3.55 - 3.50 (m, 4H), 3.05 - 2.94 (m, 1H), 2.75 - 2.70 (m, 2H), 2.69 - 2.65 (m, 2H), 2.55 - 2.50 (m, 4H).

### Example 248 Synthesis of Compound A249:

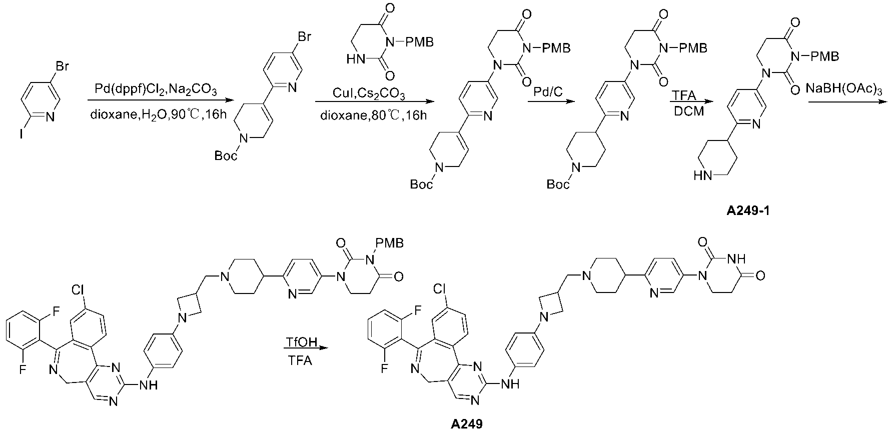

### Step 1:

Compound **A249-1** (158 mg, yield 99.05%) was synthesized according to the synthesis method of compound **A16-6** described in **Example 16.** LCMS (ESI) m/z = 395.17 [M+H]⁺.

### Step 2:

Compound **A249** (8.97 mg, yield 34.5%) was synthesized according to the method of compound **A199** described in **Example 198.** LCMS (ESI) m/z = 774.5 [M+H]⁺. ¹H NMR (400 MHz, DMSO-*d*₆) δ 10.46 (s, 1H), 9.47 (s, 1H), 8.55 (s, 1H), 8.48 (d, *J* = 2.8 Hz, 1H), 8.23 (d, *J =* 8.4 Hz, 1H), 7.84 (dd, *J*₁ = 8.4 Hz, *J*₂ = 2.0 Hz, 1H), 7.68 (dd, *J*₁ = 8.4 Hz, *J*₂ = 2.0 Hz, 1H), 7.60 - 7.48 (m, 3H), 7.36 - 7.27 (m, 2H), 7.16 (t, *J =* 8.8 Hz, 2H), 6.45 - 6.39 (m, 2H), 3.91 (t, *J* = 7.2 Hz, 2H), 3.81 (t, *J* = 6.8 Hz, 2H), 3.47 - 3.39 (m, 2H), 2.98 - 2.86 (m, 3H), 2.76 - 2.56 (m, 5H), 2.11 - 2.01 (m, 2H), 1.86 - 1.66 (m, 4H).

### Example 249 Synthesis of Compound A250:

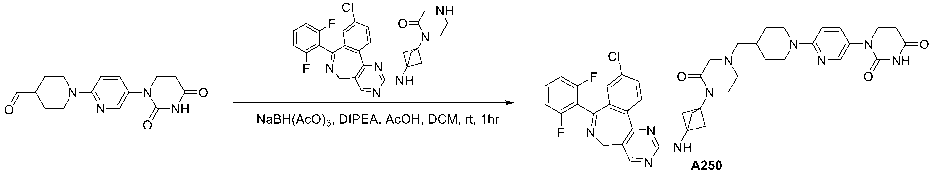

Compound **A250** (25 mg, yield 20.1%) was synthesized according to the synthesis method of compound **A132** described in **Example 131.** LCMS (ESI) m/z = 807.5 [M+H]⁺. ¹H NMR (400 MHz, DMSO-*d*₆) δ 10.35 (s, 1H), 8.51 (s, 1H), 8.26 - 8.19 (m, 1H), 8.16 (s, 1H), 8.06 (d, *J* = 2.8 Hz, 1H), 7.86 (d, *J* = 8.4 Hz, 1H), 7.61 - 7.52 (m, 1H), 7.51 - 7.46 (m, 1H), 7.31 (d, *J* = 2.0 Hz, 1H), 7.19 (t, *J* = 8.8 Hz, 2H), 6.85 (d, *J* = 9.2 Hz, 1H), 4.28 (d, *J* = 12.8 Hz, 2H), 3.72 (t, *J* = 6.8 Hz, 2H), 3.33 - 3.30 (m, 2H), 2.97 (s, 2H), 2.83 (t, *J* = 12.4 Hz, 2H), 2.72 (t, *J* = 6.8 Hz, 2H), 2.66 - 2.61 (m, 2H) , 2.46 (s, 6H), 2.25 - 2.20 (m, 2H), 1.86 - 1.74 (m, 3H), 1.16 - 1.04 (m, 2H).

### Example 250 Synthesis of Compound A251:

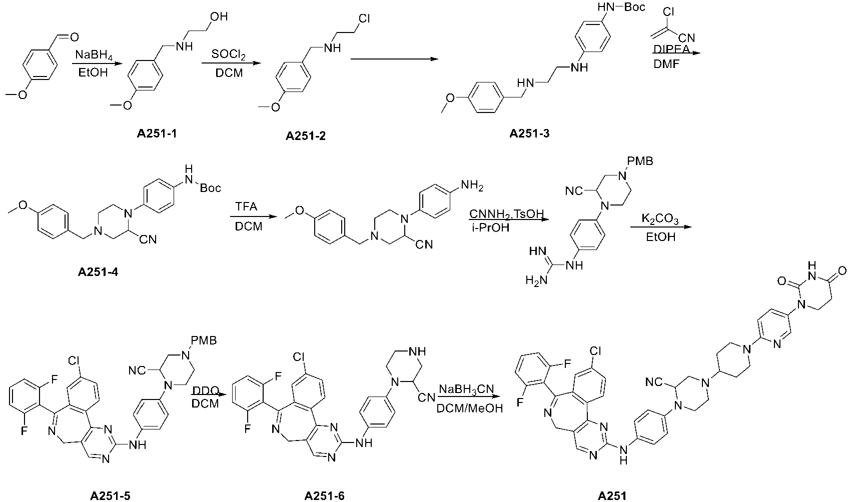

### Step 1:

At room temperature, 2-aminoethanol (2.69 g, 44.1 mmol) was added to a solution of 4-methoxybenzaldehyde (5.0 g, 36.7 mmol) in ethanol (20 mL), the reaction mixture was cooled to 0°C, sodium borohydride (1.53 g, 40.4 mmol) was added , and the mixture was stirred at room temperature for 6 h. The reaction mixture was washed with saturated sodium bicarbonate solution (50 mL) and saturated brine (50 mL), and then dried over anhydrous sodium sulfate. The crude product was separated for purification by silica gel column chromatography (dichloromethane/methanol = 30:1) to give a light yellow solid **A251-1** (5.94 g, yield 89%). LCMS (ESI) m/z = 182.1 [M+H]⁺.

### Step 2:

At 0°C, sulfoxide chloride (7.67 g, 64.45 mmol) was added to a solution of **A251-1** (5.84 g, 32.2 mmol) in 1,2-dichloroethane (20 mL), the reaction mixture was heated to 80°C and stirred for 6 h. The reaction mixture was concentrated, dichloromethane was added for stirring, and a white solid **A251-2** (5.21 g, yield 81%) was obtained by suction filtration. LCMS (ESI) m/z = 200.1 [M+H]⁺.

### Step 3:

At room temperature, *N*-(tert-butoxycarbonyl)-1,4-phenylenediamine (3.91 g, 18.8 mmol) was added to a solution of **A251-2** (2.50 g, 12.5 mmol) in toluene (20 mL), the reaction mixture was heated to 130°C and stirred for 24 h. The reaction mixture was concentrated, and the crude product was separated for purification by silica gel column chromatography (dichloromethane/methanol = 20:1) to give a light yellow solid **A251-3** (2.01 g, yield 43%). LCMS (ESI) m/z = 372.1 [M+H]⁺.

### Step 4:

At room temperature, 2-chloroacrylonitrile (0.49 g, 5.55 mmol) was added to a solution of **A251-3** (1.03 g, 2.77 mmol) in *N,N*-dimethylformamide (5 mL), and the reaction mixture was stirred at room temperature for 12 h. The reaction mixture was concentrated, and the crude product was separated for purification by silica gel column chromatography (dichloromethane/methanol = 30:1) to give a light yellow solid **A251-4** (0.75 g, yield 62%). LCMS (ESI) m/z = 422.9 [M+H]⁺.

### Step 5:

Compound **A251-5** (123 mg, yield 22%) was synthesized according to the synthesis method of compound **A101-4** described in **Example 101.** LCMS (ESI) m/z = 662.1 [M+H]⁺.

### Step 6:

At room temperature, 2,3-dichloro-5,6-dicyanobenzoquinone (0.15 g, 0.66 mmol) was added to a solution of **A251-5** (1.03 g, 2.77 mmol) in dichloromethane (5 mL), and the reaction mixture was stirred at room temperature for 12 h. The reaction mixture was concentrated, and the crude product was separated for purification by silica gel column chromatography (dichloromethane/methanol = 30:1) to give a light yellow solid **A251-6** (28 mg, yield 31%). LCMS (ESI) m/z = 542.1 [M+H]⁺.

### Step 7:

Compound **A251** (4.5 mg, yield 13.0%) was synthesized according to the synthesis method of compound **A132** described in **Example 131.** LCMS (ESI) m/z = 814.2 [M+H]⁺. ¹H NMR (400 MHz, DMSO-*d*₆) δ 10.33 (s, 1H), 9.72 (s, 1H), 8.62 (s, 1H), 8.27 (d, *J* = 8.8 Hz, 1H), 8.06 (d, *J* = 2.8 Hz, 1H), 7.85 (dd, *J*₁ = 8.4 Hz, *J*₂ = 2.0 Hz, 1H), 7.73 (d, *J =* 8.8 Hz, 2H), 7.60 - 7.45 (m, 2H), 7.31 (d, *J* = 2.0 Hz, 1H), 7.16 (t, *J =* 8.8 Hz, 2H), 7.03 (d, *J* = 8.8 Hz, 2H), 6.88 (d, *J =* 9.2 Hz, 1H), 5.03 (s, 1H), 4.32 (d, *J* = 12.4 Hz, 2H), 3.70 (t, *J* = 6.8 Hz, 2H), 3.42 (d, *J =* 11.2 Hz, 1H), 3.15 (d, *J =* 11.2 Hz, 1H), 3.00 (d, *J =* 10.8 Hz, 1H), 2.91 - 2.81 (m, 3H), 2.74 - 2.58 (m, 4H), 2.47 - 2.38 (m, 1H), 1.89 - 1.78 (m, 2H), 1.50 - 1.37 (m, 2H).

### Example 251 Synthesis of Compound A252:

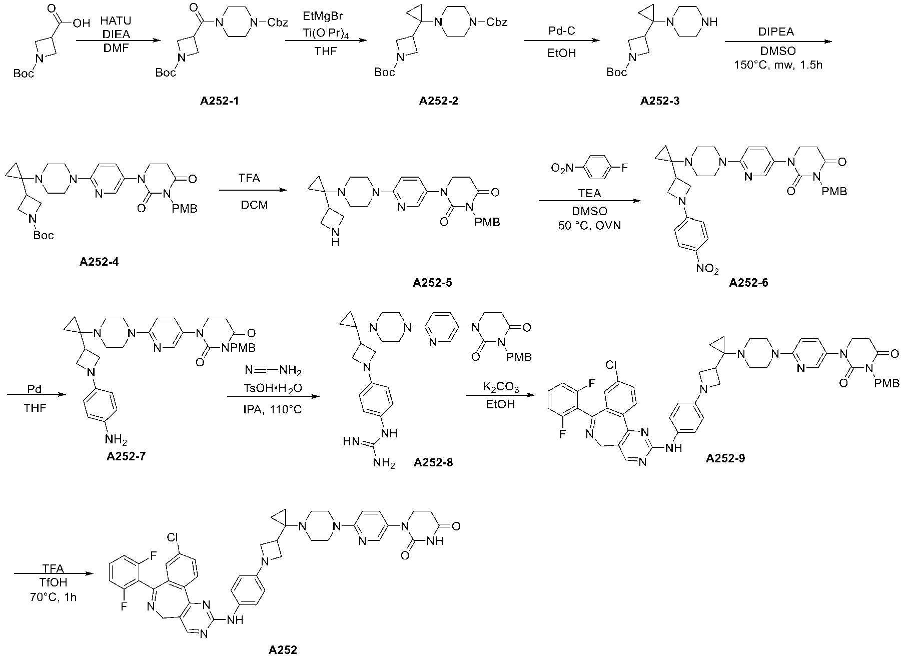

### Step 1:

Benzyl piperazine-1-carboxylate (2.19 g, 9.94 mmol), HATU (3.78 g, 9.94 mmol) and DIPEA (3.85 g, 29.82 mmol) were added to a solution of azetidine-3-carboxylic acid (2.0 g, 9.94 mmol) in *N,N-*dimethylformamide (10 mL) at room temperature. The reaction mixture was stirred at room temperature for 4 h under argon protection. After the addition of water, the reaction mixture was extracted with ethyl acetate, dried over anhydrous sodium sulfate, and concentrated under reduced pressure to give a crude product, which was purified by column chromatography (petroleum ether/ethyl acetate = 1/1) to give a white solid **A252-1** (3.9 g, yield 97.2%). LCMS (ESI) m/z = 404.2 [M+H]⁺. ¹H NMR (400 MHz, DMSO-*d*₆) δ 7.40 - 7.31 (m, 5H), 5.09 (s, 2H), 4.04 - 3.88 (m, 4H), 3.69 - 3.59 (m, 1H), 3.48 - 3.46 (m, 2H), 3.40 - 3.37 (m, 4H), 3.29 - 3.23 (m, 2H), 1.37 (s, 9H).

### Step 2:

At -25°C, ethylmagnesium bromide (6.34 g, 47.59 mmol) and tetraisopropyl titanate (6.76 g, 23.79 mmol) were added dropwise to a solution of compound **A252-1** (3.2 g, 7.93 mmol) in tetrahydrofuran (40 mL), and the reaction mixture was stirred at room temperature for 14 h under argon protection. The reaction mixture was quenched with aqueous ammonium chloride solution, extracted with ethyl acetate, dried over anhydrous sodium sulfate, and concentrated under reduced pressure to give a crude product, which was purified by column chromatography (petroleum ether/ethyl acetate = 2/1) to give a colorless oil **A252-2** (2.1 g, yield 63.7%). LCMS (ESI) m/z = 416.1 [M+H]⁺.

### Step 3:

At room temperature, palladium on carbon (100 mg, 10%) was added to a solution of compound **A252-2** (1.0 g, 2.41 mmol) in tetrahydrofuran (20 mL), and the reaction mixture was stirred at room temperature for 12 h under hydrogen protection. The reaction mixture was filtered through diatomaceous earth to give a yellow oil **A252-3** (600 mg, yield 88.6%). LCMS (ESI) m/z = 282.1 [M+H]⁺.

### Step 9:

Compound **A252-9** (80 mg, yield 40%) was synthesized according to the method of compound **A185** described in **Example 184.** LCMS (ESI) m/z = 921.2 [M+H]⁺.

### Step 10:

Trifluoromethanesulfonic acid (0.2 mL) was added to a solution of compound **A252-9** (30 mg, 0.032 mmol) in trifluoroacetic acid (1 mL) at room temperature. The reaction mixture was reacted at 70°C for 1 h. The reaction mixture was adjusted to pH about 8 by aqueous sodium bicarbonate solution, and the organic phase was dried over sodium sulfate, concentrated, and purified by preparative chromatography to give compound **A252** (6 mg, yield 22.3%). LCMS (ESI) m/z = 801.2 [M+H]⁺. ¹H NMR (400 MHz, DMSO-*d*₆) δ 10.33 (s, 1H), 9.47 (s, 1H), 8.55 (s, 1H), 8.23 (d, *J* = 8.5 Hz, 1H), 8.06 (d, *J* = 2.7 Hz, 1H), 7.83 (dd, *J*₁ = 8.5 Hz, *J*₂ = 2.2 Hz, 1H), 7.58 - 7.46 (m, 4H), 7.29 (d, *J =* 2.1 Hz, 1H), 7.18 - 7.14 (m, 2H), 6.84 (d, *J* = 9.0 Hz, 1H), 6.39 (d, *J* = 8.6 Hz, 2H), 3.88 (t, *J =* 7.4 Hz, 2H), 3.70 (t, *J* = 6.7 Hz, 2H), 3.52 - 3.45 (m, 1H), 3.43 - 3.39 (m, 4H), 3.19 (t, *J* = 6.7 Hz, 2H), 2.71 - 2.67 (m, 6H), 0.62 - 0.55 (m, 4H).

### Example 252 Synthesis of Compound A253:

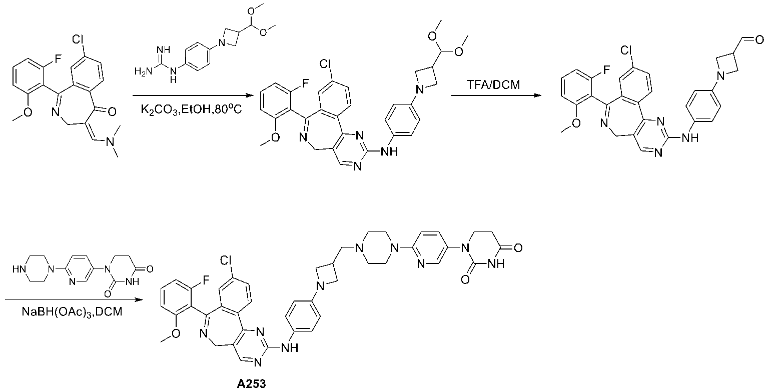

Compound **A253** (13.2 mg, yield 24.6%) was synthesized according to the synthesis method of compound **A185** described in **Example 184.** LCMS (ESI) m/z = 787.6 [M+H]⁺. ¹H NMR (400 MHz, DMSO-*d*₆) δ 10.23 (s, 1H), 9.35 (s, 1H), 8.52 (s, 1H), 8.20 (d, *J* = 8.5 Hz, 1H), 8.08 (s, 1H), 7.76 (dd, *J*₁ = 8.4 Hz, *J*₂ = 2.2 Hz, 1H), 7.57 (d, *J* = 8.3 Hz, 3H), 7.41 (q, *J* = 7.9 Hz, 1H), 7.19 (s, 1H), 6.87 (d, *J* = 20.8 Hz, 3H), 6.43 (d, *J* = 8.3 Hz, 2H), 3.94 (s, 2H), 3.71 (t, *J* = 6.7 Hz, 2H), 3.48 (s, 8H), 2.96 (s, 1H), 2.74 - 2.60 (m, 4H).

### Example 253 Synthesis of Compound A254:

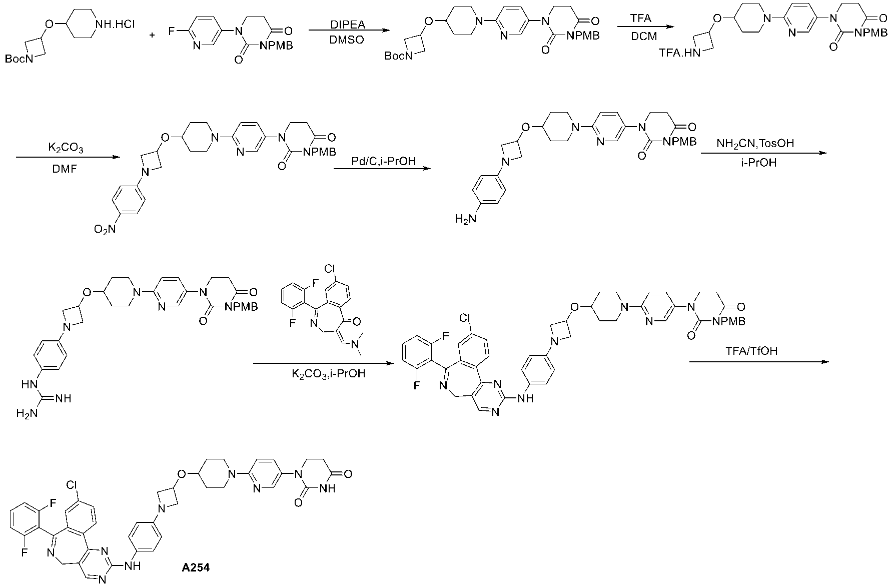

Compound **A254** (13.2 mg, yield 12.1%) was synthesized according to the synthesis method of compound **A252** described in **Example 251.** LCMS (ESI) m/z = 776.3 [M+H]⁺. ¹H NMR (400 MHz, DMSO-*d*₆) δ 10.31 (s, 1H), 9.50 (s, 1H), 8.58 (s, 1H), 8.25 (d, *J* = 8.4 Hz, 1H), 8.07 (d, *J* = 2.4 Hz, 1H), 7.86 (dd, *J*₁ = 8.4 Hz, *J*₂ = 2.2 Hz, 1H), 7.59 - 7.49 (m, 4H), 7.31 (d, *J* = 2.4 Hz, 1H), 7.18 (t, *J* = 7.9 Hz, 2H), 6.89 (d,*J* = 9.2 Hz, 13H), 6.47 (d, *J* = 8.4 Hz, 2H), 4.61 - 4.59 (m, 1H), 4.12 (t, *J* = 6.0 Hz, 2H), 3.68 - 3.64 (m, 1H), 3.56 - 3.53 **(m,** 2H), 3.17 - 3.12 (m, 1H), 2.71 (t, *J* = 6.8 Hz, 2H), 1.92 - 1.89 (m, 2H), 1.50 - 1.45 (m, 2H).

### Example 254 Synthesis of Compound A255:

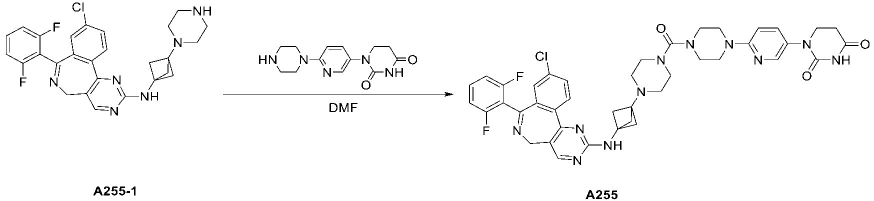

**A255-1** (40 mg, 0.79 mmol) was dissolved in DMF (1 mL) in an ice-water bath, with triphosgene (7 mg, 0.023 mmol) added, the mixture was stirred for 2 h, and then with 1-(4-(piperazin-1-yl)phenyl)dihydropyrimidine-2,4(1H,3H)-dione (22 mg, 0.079 mmol) added, the mixture was stirred at room temperature for 2 h. After the reaction was completed, the mixture was quenched with water and extracted with dichloromethane, the organic phase was spin-dried and purified by preparative reversed-phase high pressure chromatography to give **A255** (3.5 mg, yield 5%). LCMS (ESI) m/z = 808.4 [M+H]⁺. ¹H NMR (400 MHz, DMSO-*d*₆) δ 10.36 (s ,1H), 8.49 (s, 1H), 8.23 - 8.21 (m, 1H), 8.11 - 8.10 (m, 2H), 7.88 (d, *J* = 8.0 Hz, 1H), 7.68 - 7.54 (m, 2H), 7.31 - 7.30 (m, 1H), 7.21 - 7.17 (m, 2H), 6.88 (d, *J* = 8.4 Hz, 1H), 3.73 (t, *J* = 6.8 Hz, 2H), 3.53 (bs, 4H), 3.31 - 3.24 (m, 8H), 2.73 (t, *J* = 6.8 Hz, 2H), 2.45 (bs, 4H), 2.09 (s, 6H).

### Example 255 Synthesis of Compound A256:

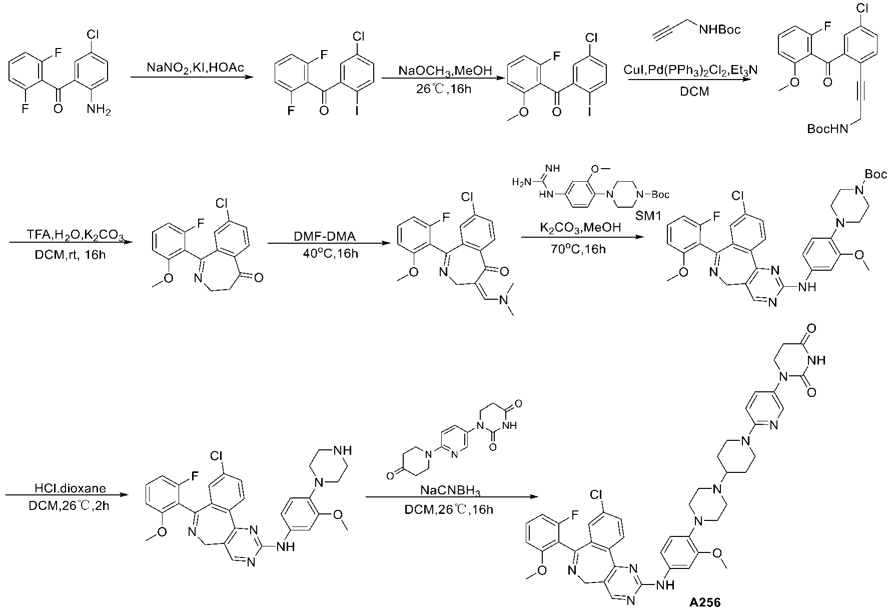

Compound **A256** (9.0 mg, yield 30%) was synthesized according to the synthesis method of compound **A128** described in **Example 127.** LCMS (ESI) m/z = 831.2 [M+H]⁺. ¹H NMR (400 MHz, DMSO-*d*₆) δ 10.35 (s, 1H), 9.67 (s, 1H), 8.62 (s, 1H), 8.29 (d, *J =* 8.4 Hz, 1H), 8.08 (d, *J* = 2.8 Hz, 1H), 7.82 (dd, *J*₁ = 8.8 Hz, *J*₂ = 2.8 Hz, 1H), 7.68 - 7.64 (m, 1H), 7.51 (dd, *J*₁ = 9.2 Hz, *J*₂ = 2.8 Hz, 1H), 7.48 - 7.42 (m, 1H), 7.31 (dd, *J*₁ = 8.4 Hz, *J*₂ = 2.4 Hz, 1H), 7.25 - 7.20 (m, 1H), 6.95 - 6.85 (m, 4H), 4.40 - 4.30 (m, 2H), 3.81 (s, 3H), 3.73 (t, *J =* 6.8 Hz, 2H), 3.35 (s, 3H), 3.00 - 2.90 (m, 4H), 2.85 (t, *J =* 12.4 Hz, 2H), 2.73 (t, *J =* 6.8 Hz, 2H), 2.70 - 2.60 (m, 4H), 2.37 - 2.34 (m, 1H),1.95 - 1.85 (m, 2H), 1.50 - 1.40 (m, 2H).

### Example 256 Synthesis of Compound A257:

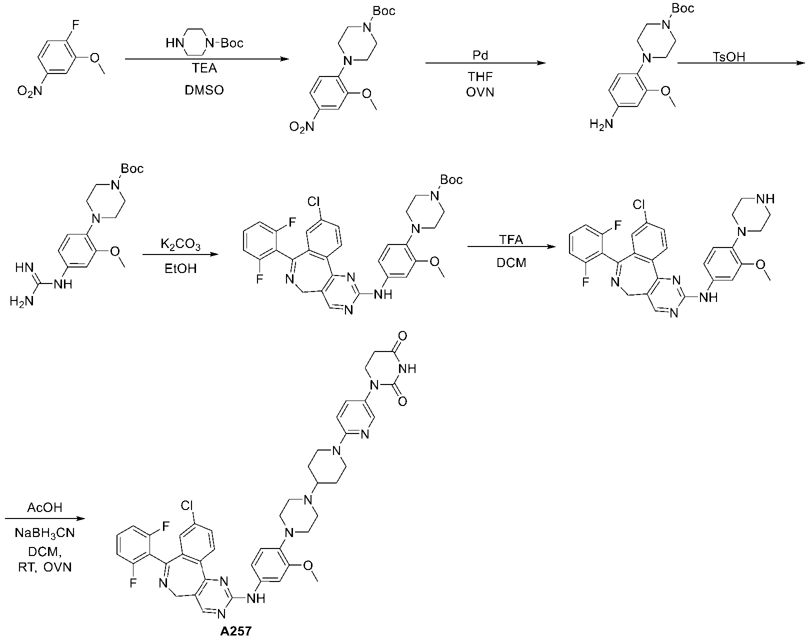

Compound **A257** (6.0 mg, yield 9.7%) was synthesized according to the method of compound **A185** described in **Example 184.** LCMS (ESI) m/z = 761.4 [M+H]⁺. ¹H NMR (400 MHz, DMSO-*d*₆) δ 10.32 (s, 1H), 9.66 (s, 1H), 8.62 (s, 1H), 8.30 (d, *J* = 8.5 Hz, 1H), 8.05 (d, *J* = 2.7 Hz, 1H), 7.85 (dd, *J*₁ = 8.5 Hz, *J*₂ *=* 2.1 Hz, 1H), 7.58 (d, *J* = 4.5 Hz, 1H), 7.57 - 7.51 (m, 1H), 7.48 (dd, *J*₁ = 9.1 Hz, *J*₂ =2.8 Hz, 1H), 7.31 (d, *J =* 2.1 Hz, 1H), 7.29 - 7.26 (m, 1H), 7.18 - 7.14 (m, 2H), 6.87 - 6.82 (m, 2H), 4.31 (d, *J* = 12.5 Hz, 2H), 3.78 (s, 3H), 3.70 (t, *J* = 6.7 Hz, 2H), 2.92 (s, 4H), 2.85 - 2.79 (m, 2H), 2.70 (t, *J* = 6.7 Hz, 2H), 2.65 - 2.62 (m, 4H), 1.88 - 1.85 (m, 2H), 1.47 - 1.33 (m, 2H).

### Example 257 Synthesis of Compound A258:

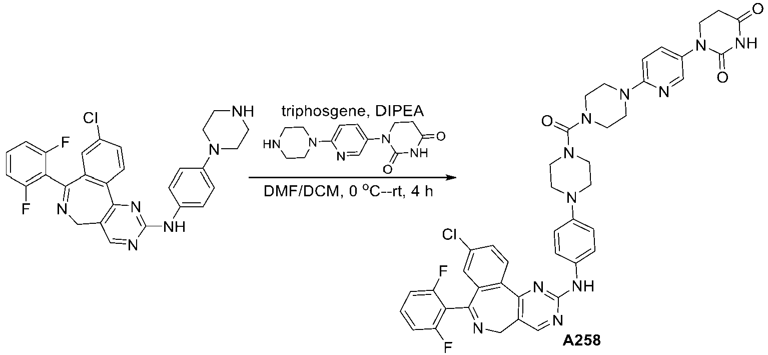

Compound **A258** (7 mg, yield 11%) was synthesized according to the method of compound **A255** described in **Example 254.** LCMS (ESI) m/z = 818.2 [M+H]⁺. ¹H NMR (400 MHz, DMSO-*d*₆) δ 10.37 (s, 1H), 9.65 (s, 1H), 8.63 (s, 1H), 8.29 (d, *J* = 8.4 Hz, 1H), 8.12 (d, *J* = 2.8 Hz, 1H), 7.95 - 7.75 (m, 1H), 7.69 (d, *J* = 8.8 Hz, 2H), 7.63 - 7.50 (m, 2H), 7.34 (d, *J* = 2.0 Hz, 1H), 7.27 - 7.13 (m, 2H), 6.99 (d, *J* = 8.8 Hz, 2H), 6.91 (d, *J* = 8.8 Hz, 1H), 3.74 (t, *J* = 6.8 Hz, 2H), 3.63 - 3.50 (m, 4H), 3.22 - 3.04 (m, 4H), 2.73 (t, *J =* 6.8 Hz, 2H).

### Example 258 Synthesis of Compound A259:

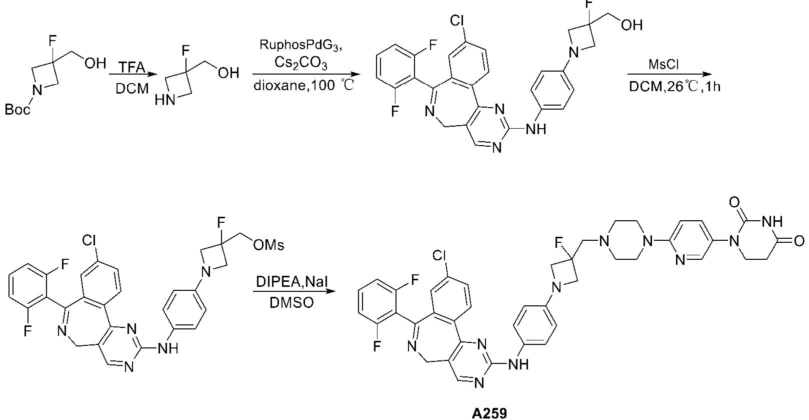

Compound **A259** (16.5 mg, yield 16.0%) was synthesized according to the synthesis method of compound **A202** described in **Example 201** and compound A48-2 described in **Example 48.** LCMS (ESI) m/z = 793.3 [M+H]⁺. ¹H NMR (400 MHz, DMSO-*d*₆) δ 10.33 (s, 1H), 9.52 (s, 1H), 8.57 (s, 1H), 8.24 (d, *J* = 8.4 Hz, 1H), 8.07 (d, *J* = 2.8 Hz, 1H), 7.88 - 7.80 (m, 1H), 7.63 - 7.47 (m, 4H), 7.30 (d, *J =* 2.0 Hz, 1H), 7.16 (t, *J* = 8.8 Hz, 2H), 6.85 (d, *J* = 9.2 Hz, 1H), 6.55 - 6.49 (m, 2H), 4.00 (dd, *J*₁ = 17.2 Hz, *J*₂ = 8.8 Hz, 2H), 3.84 (dd, *J*₁ = 21.2 Hz, *J*₂ = 8.8 Hz, 2H), 3.70 (t, *J* = 6.8 Hz, 2H), 3.54 - 3.44 (m, 4H), 2.91 (d, *J* = 26.0 Hz, 2H), 2.70 (t, *J* = 6.8 Hz, 2H), 2.65 - 2.60 (m, 4H).

### Example 259 Synthesis of Compound A260:

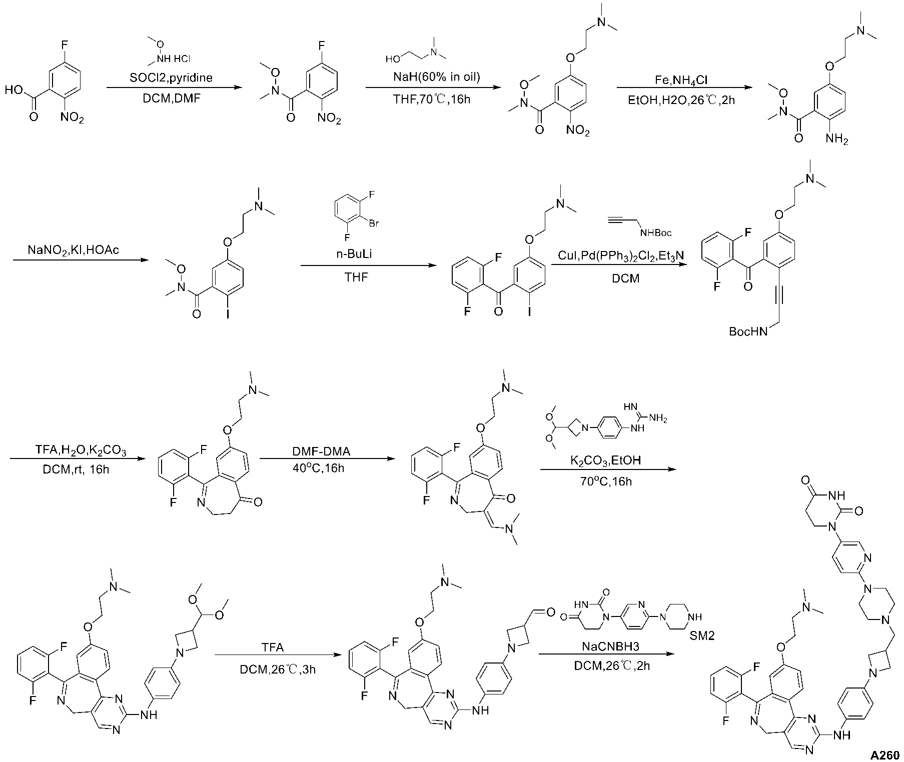

Compound **A260** (10.0 mg, yield 27%) was synthesized according to the synthesis method of compound **A128** described in **Example 127.** LCMS (ESI) m/z = 828.3 [M+H]⁺. ¹H NMR (400 MHz, DMSO-*d*₆) δ 10.42 (s, 1H), 9.47 (s, 1H), 8.54 (s, 1H), 8.26 (d, *J* = 8.8 Hz, 1H), 8.18 (d, *J* = 2.8 Hz, 1H), 7.68 - 7.62 (m, 3H), 7.59 - 7.53 (m, 1H), 7.46 (dd, *J*₁ = 8.8 Hz, *J*₂ = 2.8 Hz, 1H), 7.25 - 7.15 (m, 2H), 7.04 (d, *J* = 9.2 Hz, 1H), 6.80 (d, *J* = 2.4 Hz, 1H), 6.55 - 6.45 (m, 2H), 4.50 - 4.37 (m, 3H), 4.04 (t, *J* = 7.6 Hz, 2H), 3.75 (t, *J =* 6.8 Hz, 3H), 3.70 - 3.56 (m, 6H), 3.29 - 3.06 (m, 5H), 2.86 (d, *J* = 4.4 Hz, 6H), 2.75 (d, *J* = 6.8 Hz, 2H), 2.70 - 2.65 (m, 1H), 2.36 - 2.33 (m, 1H).

### Example 260 Synthesis of Compound A261:

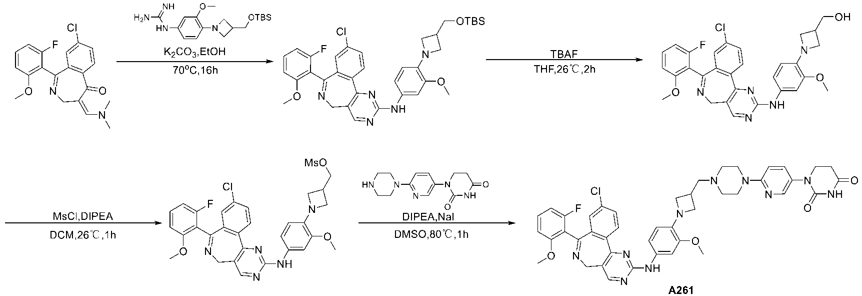

Compound **A261** (10.0 mg, yield 57%) was synthesized according to the method of compound **A185** described in **Example 184.** LCMS (ESI) m/z = 817.3 [M+H]⁺. ¹H NMR (400 MHz, DMSO-*d*₆) δ 10.42 (s, 1H), 9.62 (s, 1H), 8.60 (s, 1H), 8.27 (d, *J* = 8.4 Hz, 1H), 8.17 (d, *J* = 2.8 Hz, 1H), 7.87 - 7.81 (m, 1H), 7.70 - 7.65 (m, 1H), 7.61 (s, 1H), 7.50 - 7.42(m, 1H), 7.30 - 7.20 (m, 3H), 7.08 - 7.02 (m, 2H), 6.43 (d, *J =* 8.4 Hz, 1H), 4.10 - 4.02 (m, 4H), 3.78 (s, 6H), 3.76 - 3.72 (m, 4H), 3.65 - 3.62 (m, 2H), 3.20 - 3.12 (m, 1H), 2.80 - 2.74 (m, 4H).

### Example 261 Synthesis of Compound A262:

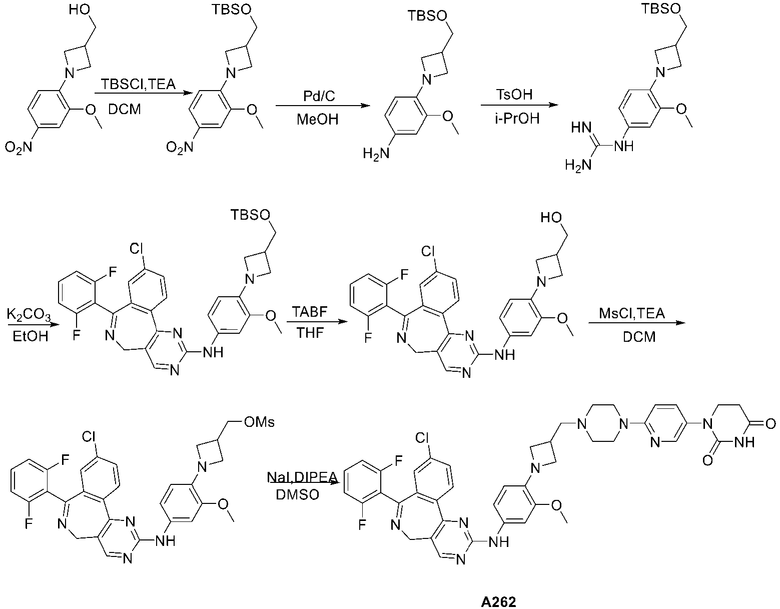

Compound **A262** (1.5 mg, yield 8.3%) was synthesized according to the synthesis method of compound **A211** described in **Example 210** and compound **A48-2** described in **Example 48.** LCMS (ESI) m/z = 805.3 [M+H]⁺. ¹H NMR (400 MHz, DMSO-*d*₆) δ 10.38 (s, 1H), 9.57 (s, 1H), 8.61 (s, 1H), 8.31 (d, *J =* 8.4 Hz, 1H), 8.09 (d, *J* = 2.8 Hz, 1H), 7.88 (dd, *J*₁ = 8.4 Hz, *J*₂ = 2.0 Hz, 1H), 7.62 - 7.50 (m, 3H), 7.33 (d, *J* = 2.0 Hz, 1H), 7.21 (d, *J* = 8.7 Hz, 3H), 6.88 (d, *J* = 9.2 Hz, 1H), 6.39 (d, *J* = 8.4 Hz, 1H), 3.96 (t, *J* = 7.6 Hz, 2H), 3.76 (s, 3H), 3.73 (t, *J* = 6.8 Hz, 2H), 3.53 - 3.43 (m, 6H), 2.77 - 2.67 (m, 5H), 2.64 - 2.60 (m, 2H).

### Example 262 Synthesis of Compound A263:

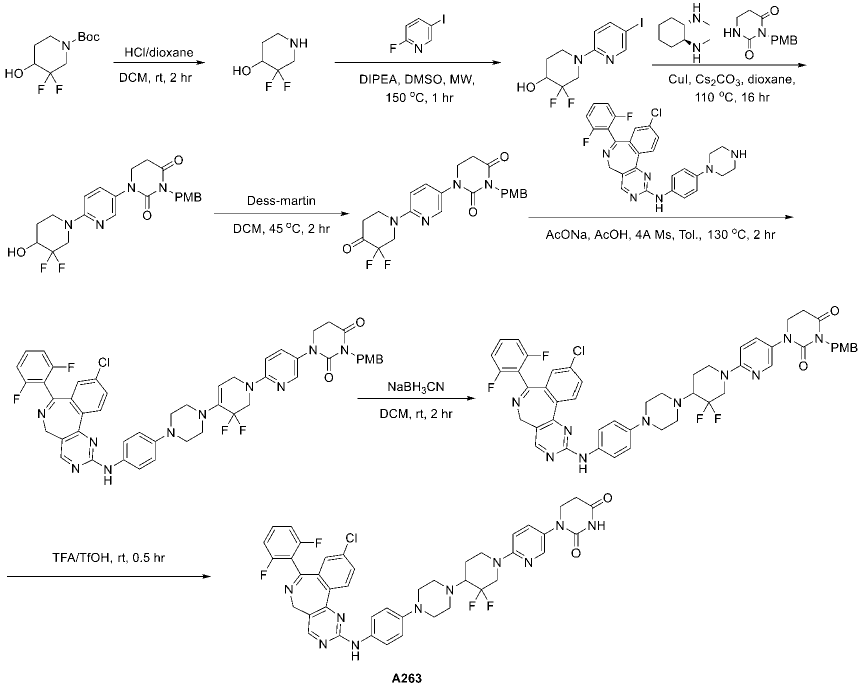

Compound **A261** (8 mg, yield 50.9%) was synthesized according to the method of compound**A249** described in **Example 248.** LCMS (ESI) m/z = 825.3 [M+H]⁺. ¹H NMR (400 MHz, DMSO-*d*₆) δ 10.40 (s, 1H), 9.73(s, 1H), 8.64 (s, 1H), 8.28 (d, *J* = 8.4 Hz, 1H), 8.11 (d, *J* = 2.8 Hz, 1H), 7.88 (dd, *J*₁ = 8.4 Hz, *J*₂ *=* 2.0 Hz, 1H), 7.76 - 7.67 (m, 2H), 7.61 - 7.52 (m, 2H), 7.35-7.32 (m, 1H), 7.25 - 7.15 (m, 2H), 7.10 - 6.99 (m, 3H), 4.84 - 4.39 (m, 1H), 4.02 - 3.78 (m, 1H), 3.74 (t, *J* = 6.8 Hz, 2H), 3.28 - 2.98 (m, 10H), 2.73 (t, *J* = 6.8 Hz, 2H), 2.06 - 1.84 (m, 1H), 1.30 - 1.24 (m, 2H).

### Example 263 Synthesis of Compound A264:

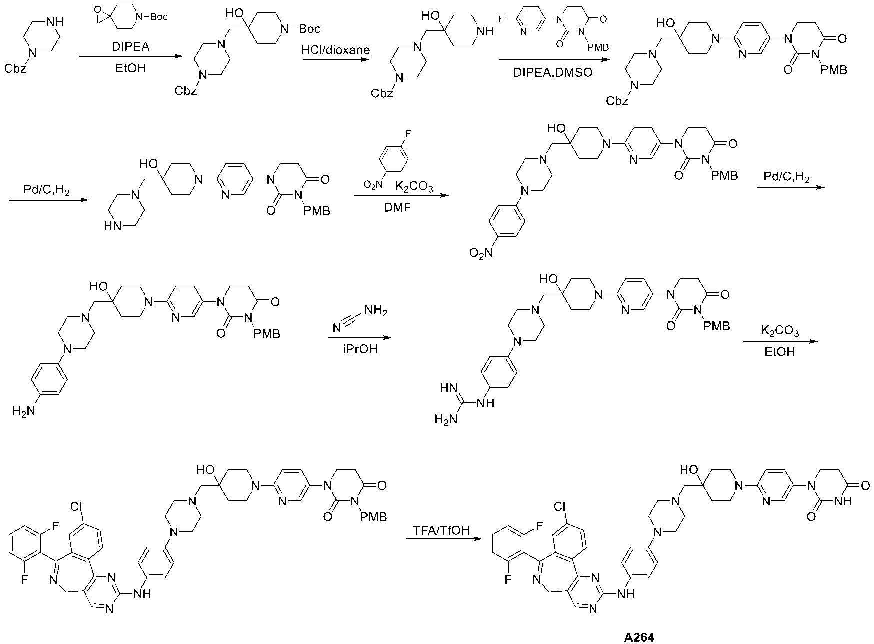

Compound **A264** (19 mg, yield 43.5%) was synthesized according to the synthesis method of compound **A252** described in **Example 251.** LCMS (ESI) m/z = 819.3 [M+H]⁺. ¹H NMR (400 MHz, DMSO-*d*₆) δ 10.32 (s, 1H), 9.58 (s, 1H), 8.58 (s, 1H), 8.25 (d, *J* = 8.0 Hz, 1H), 8.03 (d, *J* = 2.8 Hz, 1H), 7.84 (dd, *J*₁ = 8.4 Hz, *J*₂ = 2.2 Hz, 1H), 7.61 (d, *J* = 8.8 Hz, 2H), 7.58 - 7.50 (m, 1H), 7.48 - 7.43 (m, 1H), 7.30 (d, *J* = 2.0 Hz, 1H), 7.16 (t, *J* = 8.0 Hz, 2H), 6.90 (d, *J* = 8.0 Hz, 2H), 6.84 (d, *J* = 8.0 Hz, 1H), 4.25 (s, 1H), 4.12 (bs, 1H), 3.93 - 3.84 (m, 2H), 3.68 (t, *J* = 6.8 Hz, 2H), 3.30 - 3.25 (m, 2H), 3.09 - 3.00 (m, 4H), 2.72 - 2.64 (m, 6H), 2.34 (s, 2H), 1.60 - 1.52 (m, 4H).

### Example 264 Synthesis of Compound A265:

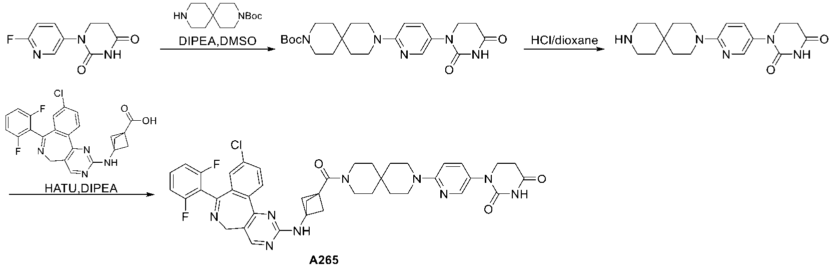

Compound **A265** (18 mg, yield 70.7%) was synthesized according to the synthesis method of compound **A54** described in **Example 54.** LCMS (ESI) m/z = 792.3 [M+H]⁺. ¹H NMR (400 MHz, DMSO-*d*₆) δ 10.33 (s, 1H), 8.49 (s, 1H), 8.22 - 8.14 (m, 2H), 8.04 (d, *J* = 2.8 Hz, 1H), 7.85 (d, *J* = 8.8 Hz, 1H), 7.57 - 7.50 (m, 1H), 7.49 - 7.44 (m, 1H), 7.28 (d, *J* = 2.0 Hz, 1H), 7.16 (t, *J* = 8.8 Hz, 2H), 6.84 (d, *J* = 8.0 Hz, 1H), 3.72 - 3.67 (m, 2H), 3.62 - 3.43 (m, 8H), 2.73 - 2.66 (m, 2H), 2.39 (s, 6H), 1.56 - 1.46 (m, 6H), 1.44 - 1.38 (m, 2H).

### Example 265 Synthesis of Compound A266:

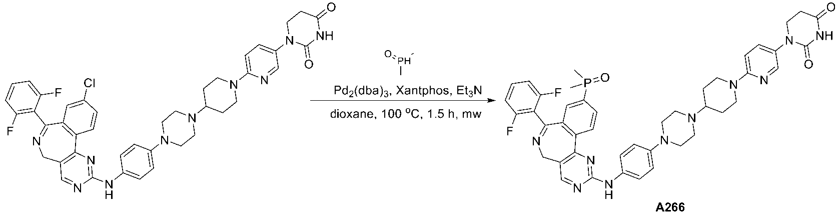

To a solution of compound **A150** (150 mg, 0.19 mmol) in 1,4-dioxane (2 mL) were added dimethylphosphine oxide (30 mg, 0.38 mmol), tris(dibenzylideneacetone)dipalladium (87 mg, 0.10 mmol), 4,5-bis(diphenylphosphino)-9,9-dimethylxanthene (55 mg, 0.1 mmol) and triethylamine (48 mg, 0.48 mmol) at room temperature. The mixture was reacted under microwave conditions at 100°C for 1.5 h under argon protection. After the addition of water, the reaction mixture was extracted with dichloromethane, washed with saturated brine, dried over anhydrous sodium sulfate, and concentrated under reduced pressure. The residue was separated for purification by reverse phase chromatography (acetonitrile/10 M aqueous ammonium bicarbonate solution) to give a yellow solid **A266** (22 mg, yield 14%). LCMS (ESI) m/z = 831.3 [M+H]⁺. ¹H NMR (400 MHz, DMSO-*d*₆) δ 10.37 (s, 1H), 9.62 (s, 1H), 8.63 (s, 1H), 8.45 - 8.29 (m, 1H), 8.19 - 8.10 (m, 1H), 8.08 (d, *J =* 2.4 Hz, 1H), 7.76 - 7.62 (m, 3H), 7.61 - 7.47 (m, 2H), 7.19 (t, *J* = 8.8 Hz, 2H), 6.99 - 6.83 (m, 3H), 4.45 - 4.24 (m, 2H), 3.73 (t, *J* = 6.8 Hz, 2H), 3.10 (s, 4H), 2.92 - 2.79 (m, 2H), 2.79 - 2.59 (m, 6H), 1.99 - 1.83 (m, 2H), 1.77 - 1.60 (m, 6H), 1.56 - 1.38 (m, 2H).

### Example 266 Synthesis of Compound A267:

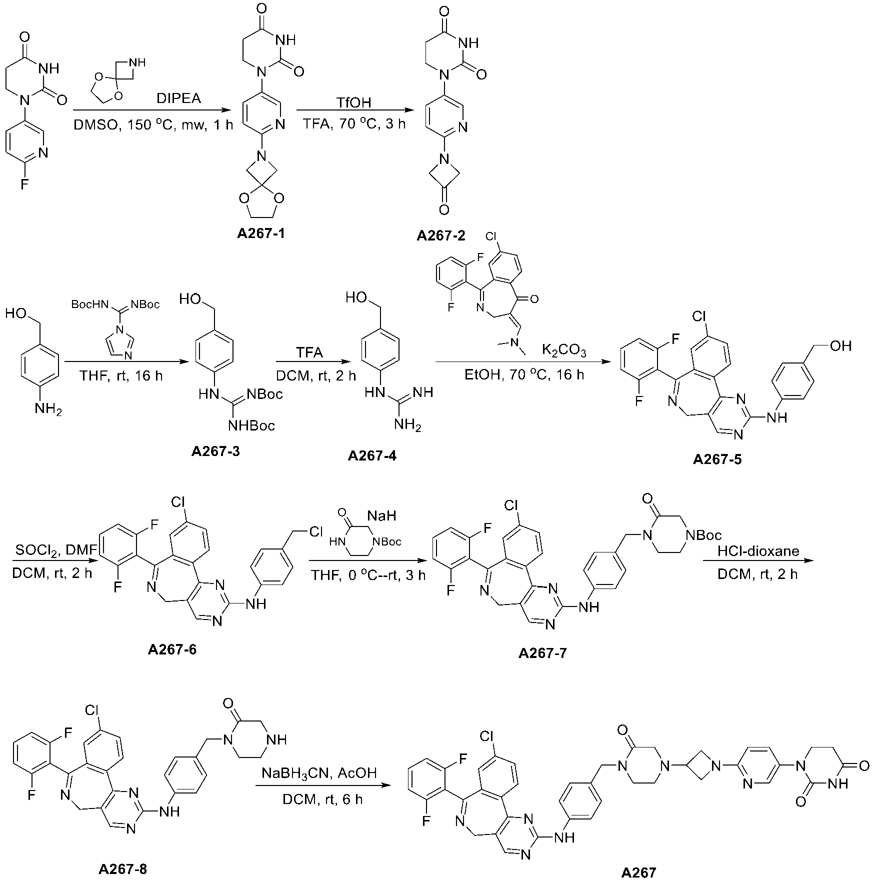

### Step 1:

To 1-(6-fluoropyridin-3-yl)-3-(4-methoxybenzyl)dihydropyrimidine-2,4(1H,3H)-dione (300 mg, 1.43 mmol) in dimethyl sulfoxide (6 mL), 5,8-dioxa-2-azaspiro[3.4]octane (326 mg, 2.15 mmol, HCl salt) and DIPEA (463 mg, 3.59 mmol) were added at room temperature. The reaction mixture was stirred at 150°C for 1 h under microwave conditions. After the addition of water, the reaction mixture was extracted with dichloromethane, washed with saturated brine, dried over anhydrous sodium sulfate, concentrated under reduced pressure, and the residue was separated for purification by silica gel column chromatography to give a yellow solid **A267-1** (230 mg, yield 53%). LCMS (ESI) m/z = 305.3 [M+H]⁺.

### Step 2:

Trifluoromethanesulfonic acid (0.5 mL) was added to a solution of compound **A267-1** (230 mg, 0.76 mmol) in trifluoroacetic acid (2.5 mL) at room temperature. The reaction mixture was stirred at 70°C for 3 h. The reaction mixture was quenched with saturated aqueous sodium bicarbonate solution, extracted with dichloromethane, washed with saturated brine, dried over anhydrous sodium sulfate, and concentrated under reduced pressure. The residue was purified by silica gel column chromatography to give a gray solid **A26-2** (75 mg, yield 38%). LCMS (ESI) m/z = 261.2 [M+H]⁺.

### Step 3:

To a solution of compound 4-aminobenzyl alcohol (2 g, 16.24 mmol) in tetrahydrofuran (20 mL), *N,N'*-di-Boc-1H-1-guanidinopyrazole (5.04 g, 16.24 mmol) was added at room temperature. The reaction mixture was stirred at room temperature for 16 h. The reaction mixture was concentrated under reduced pressure, and the residue was separated for purification by silica gel column chromatography to give a yellow oily liquid **A267-3** (3.75 g, yield 63%). LCMS (ESI) m/z = 366.3 [M+H]⁺.

### Step 4:

Trifluoroacetic acid (19 mL) was added to a solution of compound **A267-3** (3.75 g, 10.26 mmol) in dichloromethane (38 mL) at room temperature. The reaction mixture was stirred at room temperature for 2 h. The reaction mixture was concentrated under reduced pressure to give a brown-yellow liquid **A267-4** (5.7 g, yield 100%, crude product). LCMS (ESI) m/z = 166.2 [M+H]⁺.

### Step 5:

To a solution of compound **A267-4** (1.16 g, 4.16 mmol, TFA salt) in ethanol (20 mL) were added **SM4** (1 g, 2.77 mmol) and potassium carbonate (1.15 g, 8.32 mmol) at room temperature. The reaction mixture was heated to 70°C and stirred for 16 h. After the addition of water, the reaction mixture was extracted with ethyl acetate, washed with saturated brine, dried over anhydrous sodium sulfate, concentrated under reduced pressure, and the residue was separated for purification by silica gel column chromatography to give a brown-yellow solid **A267-5** (794 mg, yield 62%). LCMS (ESI) m/z = 463.3 [M+H]⁺.

### Step 6:

Thionyl chloride (56 mg, 0.47 mmol) and *N,N*-dimethylformamide (0.1 mL) were added to a solution of compound **A267-5** (108 mg, 0.23 mmol) in dichloromethane (2 mL) at room temperature. The reaction mixture was stirred at room temperature for 2 h. The reaction mixture was concentrated under reduced pressure to give a brown-yellow liquid **A267-6** (134 mg, yield 100%, crude product). LCMS (ESI) m/z = 481.2 [M+H]⁺.

### Step 7:

Sodium hydride (17 mg, 0.42 mmol, 60%) was added to a solution of compound tert-butyl 3-oxo-1-piperazinecarboxylate (42 mg, 0.21 mmol) in tetrahydrofuran (3 mL) under argon protection at 0°C. The reaction mixture was stirred at 0°C for 1 h. With compound **A267-6** (80 mg, 0.14 mmol) added, the reaction mixture was stirred at room temperature for 3 h. After the addition of water, the reaction mixture was extracted with dichloromethane, washed with saturated brine, dried over anhydrous sodium sulfate, filtered, concentrated, and the residue was separated for purification by silica gel column chromatography to give a white solid **A267-7** (90 mg, yield 100%, crude product). LCMS (ESI) m/z = 645.3 [M+H]⁺.

### Step 8:

HCl (1 mL, 4.0 M in 1,4-dioxane) was added to a dichloromethane solution (2 mL) of compound **A267-7** (166 mg, 0.26 mmol), and the reaction mixture was stirred at room temperature for 2 h. The reaction mixture was directly spin-dried to give a white solid **A267-8** (120 mg, yield 86%, crude product). LCMS (ESI) m/z = 545.1 [M+H]⁺.

### Step 9:

**A267-2** (30 mg, 0.12 mmol) and acetic acid (0.1 mL) were added to a solution of compound **A267-8** (42 mg, 0.08 mmol) in dichloromethane (2 mL), and sodium cyanoborohydride (14 mg, 0.23 mmol) was added after stirring the reaction mixture at room temperature for 1 h. The reaction mixture was stirred at room temperature for 6 h. After the addition of water, the reaction mixture was extracted with dichloromethane, washed with saturated brine, dried over anhydrous sodium sulfate, filtered and concentrated. The residue was separated for purification by reverse phase chromatography (acetonitrile/10 M aqueous ammonium bicarbonate solution) to give a yellow solid **A267** (10 mg, yield 16%). LCMS (ESI) m/z = 789.3 [M+H]⁺. ¹H NMR (400 MHz, DMSO-*d*₆) δ 10.36 (s, 1H), 9.91 (s, 1H), 8.68 (s, 1H), 8.30 (d, *J =* 8.8 Hz, 1H), 8.03 (d, *J =* 2.4 Hz, 1H), 7.95 - 7.85 (m, 1H), 7.81 (d, *J* = 8.4 Hz, 2H), 7.65 - 7.44 (m, 2H), 7.34 (d, *J* = 2.0 Hz, 1H), 7.30 - 7.11 (m, 4H), 6.41 (d, *J* = 8.8 Hz, 1H), 4.50 (s, 2H), 4.02 (t, *J* = 8.0 Hz, 2H), 3.87 - 3.75 (m, 2H), 3.70 (t, *J* = 6.8 Hz, 2H), 3.28 - 3.19 (m, 2H), 3.14 (s, 2H), 2.79 - 2.59 (m, 4H).

### Example 267 Synthesis of Compound A268:

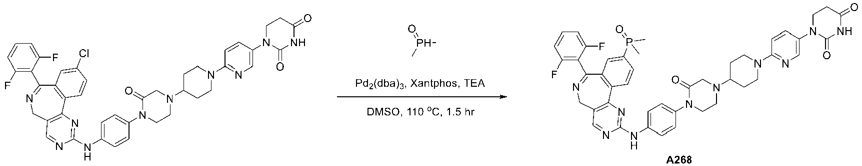

Compound **A268** (18 mg, yield 34.2%) was synthesized according to the synthesis method of compound **A266** described in **Example 265.** LCMS (ESI) m/z = 845.4 [M+H]⁺. ¹H NMR (400 MHz, DMSO-*d*₆) δ 10.38 (s, 1H), 9.98 (s, 1H), 8.72 (s, 1H), 8.41 - 8.37 (m, 1H), 8.20 - 8.12 (m, 1H), 8.09 (d, *J* = 2.7 Hz, 1H), 7.85 (d, *J* = 8.9 Hz, 2H), 7.76 - 7.71 (m, 1H), 7.61 - 7.49 (m, 2H), 7.33 - 7.26 (m, 2H), 7.24 - 7.14 (m, 2H), 6.91 (d, *J* = 9.2 Hz, 1H), 4.39 - 4.30 (m, 2H), 3.73 (t, *J* = 6.8 Hz, 2H), 3.66 - 3.59 (m, 2H), 3.33 (s, 2H), 2.94 - 2.82 (m, 4H), 2.73 (t, *J =* 6.8 Hz, 2H), 2.65 - 2.57 (m, 1H), 1.98 - 1.88 (m, 2H), 1.76 - 1.62 (m, 6H), 1.52 - 1.37 (m, 2H).

### Example 268 Synthesis of Compound A269:

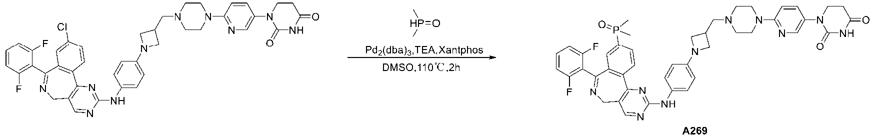

Compound **A269** (23.0 mg, yield 21%) was synthesized according to the synthesis method of compound **A266** described in **Example 265.** LCMS (ESI) m/z = 817.4 [M+H]⁺. ¹H NMR (400 MHz, DMSO-*d*₆) δ 10.37 (s, 1H), 9.51 (s, 1H), 8.60 (s, 1H), 8.34 (dd, *J*₁ = 8.0 Hz, *J*₂ = 2.8 Hz,1H), 8.18 - 8.10 (m, 1H), 8.07 (d, *J* = 11.2 Hz, 1H), 7.71 (d, *J* = 11.6 Hz, 1H), 7.61 - 7.52 (m, 4H), 7.25 - 7.17 (m, 2H), 6.88 (d, *J* = 9.2 Hz, 1H), 6.47 - 6.43 (m, 2H), 3.94 (t, *J* = 7.2 Hz, 2H), 3.73 (t, *J* = 6.8 Hz, 2H), 3.55 - 3.49 (m, 4H), 3.48 - 3.45 (m, 2H), 3.05 - 2.95 (m, 1H), 2.72 (t, J = 6.8 Hz, 2H), 2.65 (d, *J* = 7.2 Hz, 2H), 1.75 - 1.65 (m, 4H).

### Example 269 Synthesis of Compound A270:

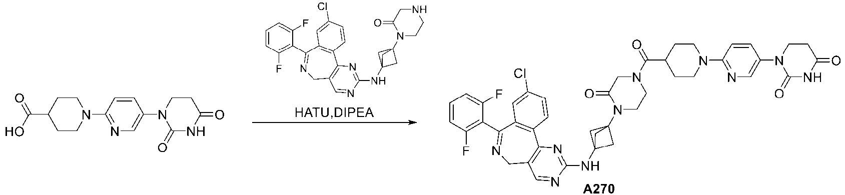

Compound **A270** (15 mg, yield 63.4%) was synthesized according to the synthesis method of compound **A1** described in **Example 1.** LCMS (ESI) m/z = 821.2 [M+H]⁺. ¹H NMR (400 MHz, DMSO-*d*₆) δ 10.34 (s, 1H), 8.49 (s, 1H), 8.22 - 8.13 (m, 2H), 8.05 (d, *J* = 2.8 Hz, 1H), 7.85 - 7.81 (m, 1H), 7.58 - 7.46 (m, 2H), 7.28 (d, *J* = 2.0 Hz, 1H), 7.20 - 7.12 (m, 2H), 6.86 (d, *J* = 8.0 Hz, 1H), 4.35 - 4.25 (m, 2H), 4.22 (s, 1H), 3.98 (s, 1H), 3.83 (s, 1H), 3.74 - 3.65 (m, 3H), 3.42 (s, 1H), 2.98 - 2.85 (m, 3H), 2.69 (t, *J* = 8.0 Hz, 2H), 2.45 (s, 6H), 1.74 - 1.63 (m, 2H), 1.60 - 1.45 (m, 2H).

### Example 270 Synthesis of Compound A271:

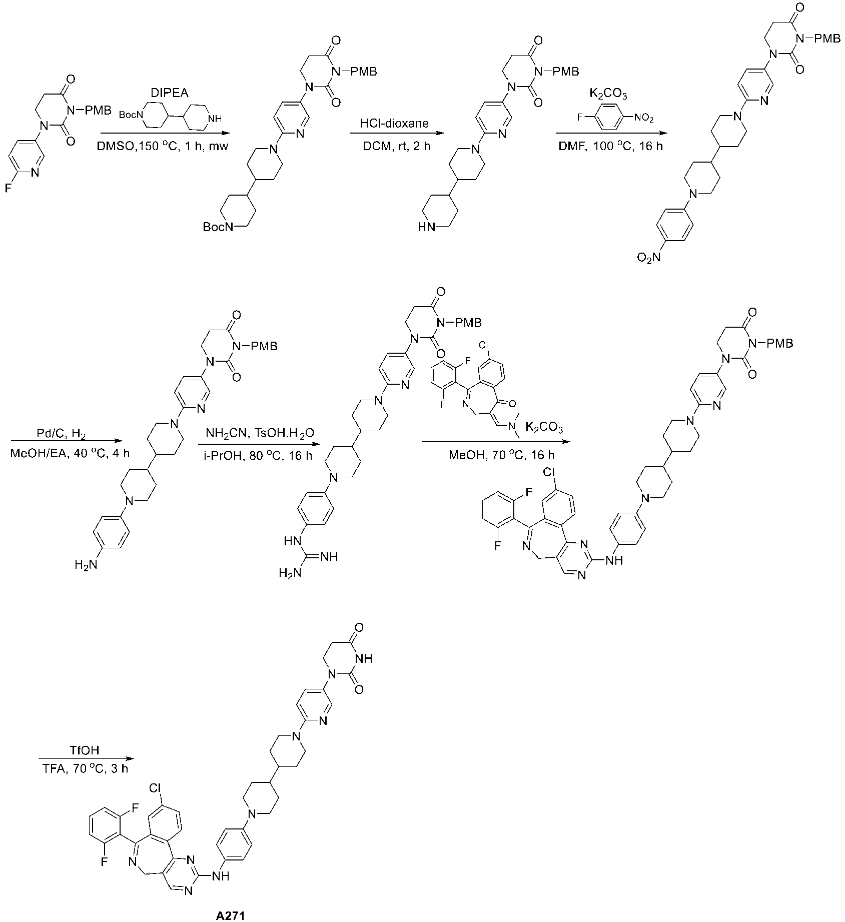

Compound **A271** (2 mg, yield 12%) was synthesized according to the synthesis method of compound **A252** described in **Example 251.** LCMS (ESI) m/z = 788.3 [M+H]⁺. ¹H NMR (400 MHz, DMSO-*d*₆) δ 10.36 (s, 1H), 9.61 (s, 1H), 8.61 (s, 1H), 8.28 (d, *J* = 8.4 Hz, 1H), 8.07 (d, *J* = 2.8 Hz, 1H), 7.91 - 7.83 (m, 1H), 7.64 (d, *J=* 8.8 Hz, 2H), 7.61 - 7.53 (m, 1H), 7.53 - 7.45 (m, 1H), 7.33 (d, *J=* 2.5 Hz, 1H), 7.28 - 7.13 (m, 2H), 6.94 (d, *J = 9.2* Hz, 2H), 6.87 (d, *J* = 9.2 Hz, 1H), 4.50 - 4.18 (m, 2H), 3.80 - 3.57 (m, 4H), 2.86 - 2.64 (m, 4H), 2.63 - 2.55 (m, 2H), 2.10 - 1.98 (m, 1H), 1.91 - 1.68 (m, 4H), 1.37 - 1.30 (m, 2H), 1.25 - 1.20 (m, 2H), 0.96 - 0.76 (m, 1H).

### Example 271 Synthesis of Compound A272:

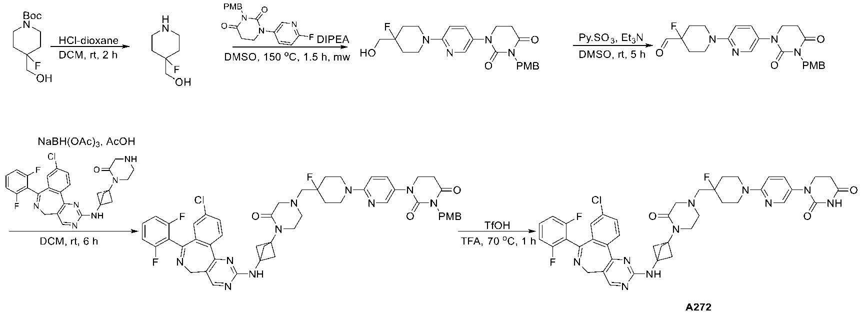

Compound **A272** (9 mg, yield 21%) was synthesized according to the synthesis method of compound **A132** described in **Example 131.** LCMS (ESI) m/z = 825.3 [M+H]⁺. ¹H NMR (400 MHz, DMSO-*d*₆) δ 10.37 (s, 1H), 8.51 (s, 1H), 8.31 - 8.13 (m, 2H), 8.09 (d, *J* = 2.4 Hz, 1H), 7.91 - 7.77 (m, 1H), 7.65 - 7.49 (m, 2H), 7.31 (d, *J* = 2.0 Hz, 1H), 7.19 (t, *J =* 9.2 Hz, 2H), 6.93 (d, *J* = 9.2 Hz, 1H), 4.14 - 3.94 (m, 2H), 3.72 (t, *J =* 6.4 Hz, 2H), 3.34 - 3.29 (m, 2H), 3.29 - 3.18 (m, 2H), 3.11 (s, 2H), 2.83 - 2.60 (m, 6H), 2.46 (s, 6H), 2.00 - 1.84 (m, 2H), 1.82 - 1.54 (m, 2H).

### Example 272 Synthesis of Compound A273:

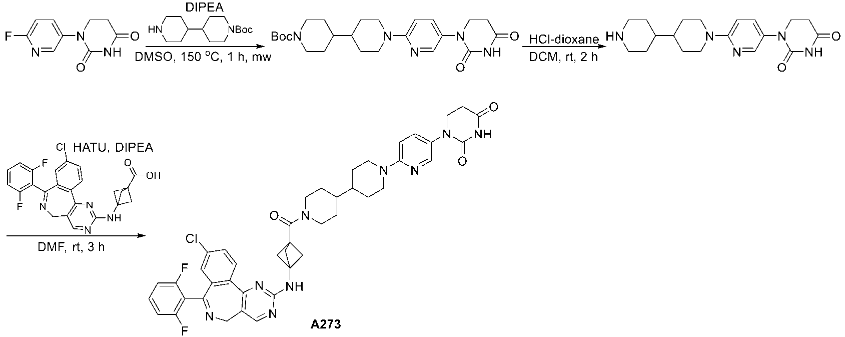

Compound **A273** (10 mg, yield 29%) was synthesized according to the synthesis method of compound **A54** described in **Example 54.** LCMS (ESI) m/z = 806.4 [M+H]⁺. ¹H NMR (400 MHz, DMSO-*d*₆) δ 10.35 (s, 1H), 8.51 (s, 1H), 8.29 - 8.15 (m, 2H), 8.06 (d, *J* = 2.8 Hz, 1H), 7.87 (d, *J* = 8.4 Hz, 1H), 7.62 - 7.52 (m, 1H), 7.52 - 7.44 (m, 1H), 7.31 (d, *J* = 2.0 Hz, 1H), 7.19 (t, *J* = 8.8 Hz, 2H), 6.86 (d, *J* = 9.2 Hz, 1H), 4.51 - 4.25 (m, 3H), 4.22 - 4.02 (m, 1H), 3.71 (t, *J* = 6.8 Hz, 2H), 3.12 - 2.91 (m, 1H), 2.83 - 2.65 (m, 4H), 2.46 - 2.33 (m, 6H), 1.89 - 1.65 (m, 4H), 1.46 - 1.29 (m, 2H), 1.29 - 0.92 (m, 4H).

### Example 273 Synthesis of Compound A274:

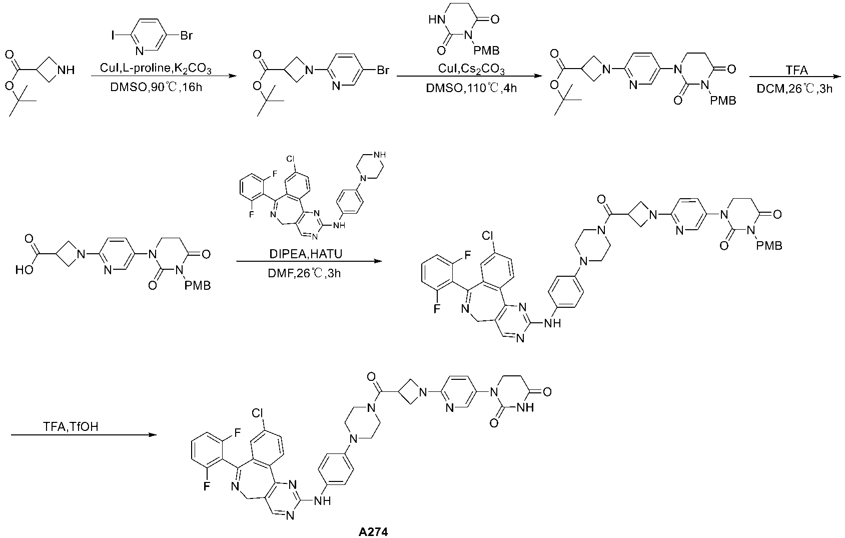

Compound **A274** (12.0 mg, yield 46%) was synthesized according to the synthesis method of compound **A132** described in **Example 131.** LCMS (ESI) m/z = 789.3 [M+H]⁺. ¹H NMR (400 MHz, DMSO-*d*₆) δ 10.37 (s, 1H), 9.67 (s, 1H), 8.62 (s, 1H), 8.28 (d, *J* = 8.4 Hz, 1H), 8.06 (d, *J =* 2.8 Hz, 1H), 7.88 (dd, *J*₁ = 8.0 Hz, *J*₂ = 2.8 Hz, 1H), 7.64 - 7.54 (m, 2H), 7.61 - 7.51 (m, 2H), 7.34 (d, *J* = 2.4 Hz, 1H), 7.25 - 7.18 (m, 2H), 6.99 (d, *J* = 9.2 Hz, 2H), 6.48 (d, *J* = 8.8 Hz, 1H), 4.18 (t, *J* = 8.4 Hz, 2H), 4.08 (t, *J* = 6.8 Hz, 2H), 3.98 - 3.90 (m, 1H), 3.72 (t, *J* = 6.8 Hz, 2H), 3.69 - 3.65 (m, 2H), 3.55 - 3.50 (m, 2H), 3.15 - 3.07 (m, 4H), 2.73 *(t, J* = 6.4 Hz, 2H).

### Example 274 Synthesis of Compound A275:

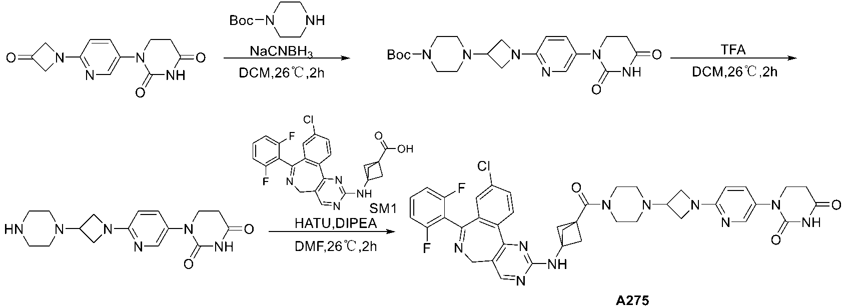

Compound **A275** (12 mg, yield 44%) was synthesized according to the synthesis method of compound **A132** described in **Example 131.** LCMS (ESI) m/z = 779.2 [M+H]⁺. ¹H NMR (400 MHz, DMSO-*d*₆) δ 10.36 (s, 1H), 8.51 (s, 1H), 8.27 - 8.20 (m, 2H), 8.04 (d, *J* = 2.8 Hz, 1H), 7.87 (d, *J* = 8.8 Hz, 1H), 7.57 (t, *J* = 8.4 Hz, 1H), 7.52 (dd, *J*₁ = 8.0 Hz, *J*₂ = 2.8 Hz, 1H), 7.31 (d, *J* = 2.4 Hz, 1H), 7.25 - 7.15 (m, 2H), 6.44 (d, *J* = 8.8 Hz, 1H), 4.03 (t, *J* = 7.6 Hz, 2H), 3.82 - 3.77 (m, 2H), 3.71 (t, *J* = 6.8 Hz, 2H), 3.64 - 3.60 (m, 2H), 3.50 - 3.45 (m, 2H), 2.73 (t, *J =* 6.4 Hz, 2H), 2.42 (s, 6H), 2.38 - 2.30 (m, 2H), 2.08 - 1.97 (m, 1H), 1.29 - 1.20 (m, 2H).

### Example 275 Synthesis of Compound A276:

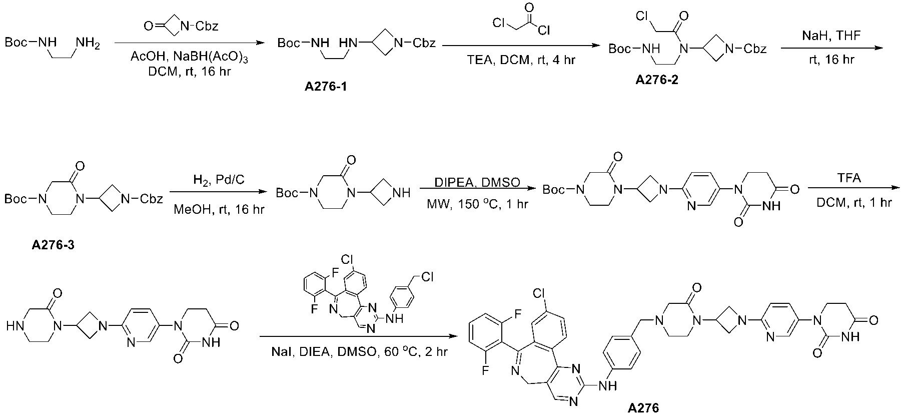

### Step 1:

Acetic acid (1.50 g, 24.97 mmol) and sodium borohydride acetate (7.94 g, 37.45 mmol) were added to a solution of 1-benzyloxycarbonylazetidin-3-one (2.56 g, 12.48 mmol) and N-tert-butoxycarbonyl-1,2-ethylenediamine (2 g, 12.48 mmol) in dichloromethane (50 mL). The reaction mixture was stirred at room temperature for 16 h. After LCMS showed that the reaction was complete, the reaction mixture was quenched with saturated sodium bicarbonate, extracted with dichloromethane, and the organic phase was spin-dried and purified by column chromatography to give **A276-1** (2.7 g, yield 61.90%). LCMS (ESI) m/z = 350.3 [M+H]⁺.

### Step 2:

Chloroacetyl chloride (892.10 mg, 7.90 mmol, 629.57 µL) was added to a solution of **A276-1** (2.3 g, 6.58 mmol) and triethylamine (1.33 g, 13.16 mmol, 1.83 mL) in dichloromethane (30 mL), and the reaction mixture was stirred at room temperature for 4 h. After LCMS showed that the reaction was complete, the reaction mixture was quenched with saturated sodium bicarbonate, extracted with ethyl acetate, and the organic phase was spin-dried and purified by column chromatography to give **A276-2** (1.6 g, yield 57.07%). LCMS (ESI) m/z = 426.3 [M+H]⁺.

### Step 3:

Sodium hydride (169.04 mg, 4.23 mmol, 60% purity) was added to a solution of **A276-2** (1.5 g, 3.52 mmol) in tetrahydrofuran (80 mL) at 0°C, and the reaction mixture was stirred at room temperature for 16 h. After LCMS showed that the reaction was complete, the reaction mixture was quenched with ice-cold saturated ammonium chloride and extracted with ethyl acetate. The organic phase was spin-dried and purified by column chromatography to give **A276-3** (1.3 g, yield 94.78%). LCMS (ESI) m/z = 390.4 [M+H]⁺.

### Step 4:

Compound **A276** (5 mg, yield 3%) was synthesized according to the synthesis method of compound **A101** described in **Example 101.** LCMS (ESI) m/z = 789.3 [M+H]⁺. ¹H NMR (400 MHz, DMSO-*d*₆) δ 10.37 (s, 1H), 9.91 (s, 1H), 8.69 (s, 1H), 8.32 (d, *J* = 8.4 Hz, 1H), 8.06 (d, *J* = 2.4 Hz, 1H), 7.90 (dd, *J*₁ = 8.4 Hz, *J*₂ = 2.0 Hz, 1H), 7.82 (d, *J =* 8.4 Hz, 2H), 7.61 - 7.52 (m, 2H), 7.35 (d, *J =* 2.4 Hz, 1H), 7.31 - 7.26 (m, 2H), 7.24 - 7.15 (m, 2H), 6.47 (d, *J* = 8.8 Hz, 1H), 5.30-5.22 (m, 1H), 4.15 (t, *J* = 8.4 Hz, 2H), 4.09 - 4.02 (m, 2H), 3.71 (t, *J* = 6.8 Hz, 2H), 3.54 (s, 2H), 3.52 - 3.48 (m, 2H), 3.06 (s, 2H), 2.74 - 2.68 (m, 4H).

### Example 276 Synthesis of Compound A277:

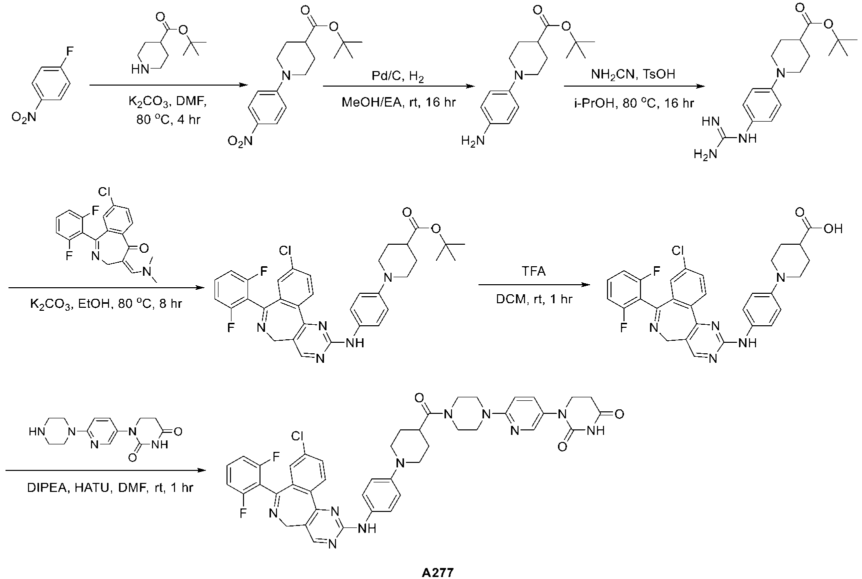

Compound **A277** (17 mg, yield 58.2%) was synthesized according to the synthesis method of compound **A185** described in **Example 184.** LCMS (ESI) m/z = 817.4 [M+H]⁺. ¹H NMR (400 MHz, DMSO-*d*₆) *δ* 10.39 (s, 1H), 9.62 (s, 1H), 8.61 (s, 1H), 8.28 *(d, J =* 8.4 Hz, 1H), 8.13 (d, *J* = 2.8 Hz, 1H), 7.88 (dd, Ji = 8.4 Hz, *J*₂ = 2.0 Hz, 1H), 7.67 - 7.62 (m, 2H), 7.59 - 7.54 (m, 2H), 7.33 (d, *J* = 2.2 Hz, 1H), 7.23 - 7.15 (m, 2H), 6.98 - 6.89 (m, 3H), 3.74 (t, *J =* 6.8 Hz, 2H), 3.72 - 3.68 (m, 2H), 3.67 - 3.64 (m, 2H), 3.62 - 3.56 (m, 4H), 3.54 - 3.48 (m, 2H), 2.89 - 2.79 (m, 1H), 2.75 - 2.69 (m, 4H), 1.80 - 1.73 (m, 4H).

### Example 277 Synthesis of Compound A278:

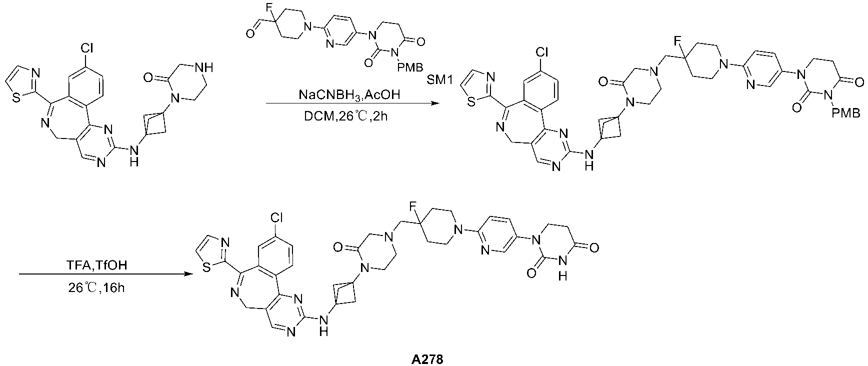

Compound **A278** (15.0 mg, yield 25%) was synthesized according to the synthesis method of compound **A132** described in **Example 131.** LCMS (ESI) m/z = 796.4 [M+H]⁺. ¹H NMR (400 MHz, DMSO-*d*₆) δ 10.37 (s, 1H), 8.53 (s, 1H), 8.16 (s, 1H), 8.09 (d, *J* = 2.8 Hz, 1H), 8.05 - 7.98 (m, 1H), 7.97 (d, *J = 3.2* Hz, 1H), 7.94 - 7.89 (m, 2H), 7.53 (dd, *J*₁ = 8.0 Hz, *J*₂ = 2.8 Hz, 1H), 6.93 (d, *J* = 9.2 Hz, 1H), 4.81 (d, *J =* 10.4 Hz, 1H), 4.08 - 4.01 (m, 2H), 3.86 - 3.82 (m, 1H), 3.73 (t, *J* = 6.8 Hz, 2H), 3.35 - 3.25 (m, 4H), 3.10 (s, 2H), 2.78 (t, *J =* 5.6 Hz, 2H), 2.75 - 2.67 (m, 3H), 2.60 (bs, 1H), 2.43 (s, 6H), 1.95 - 185 (m, 2H).

### Example 278 Synthesis of Compound A279:

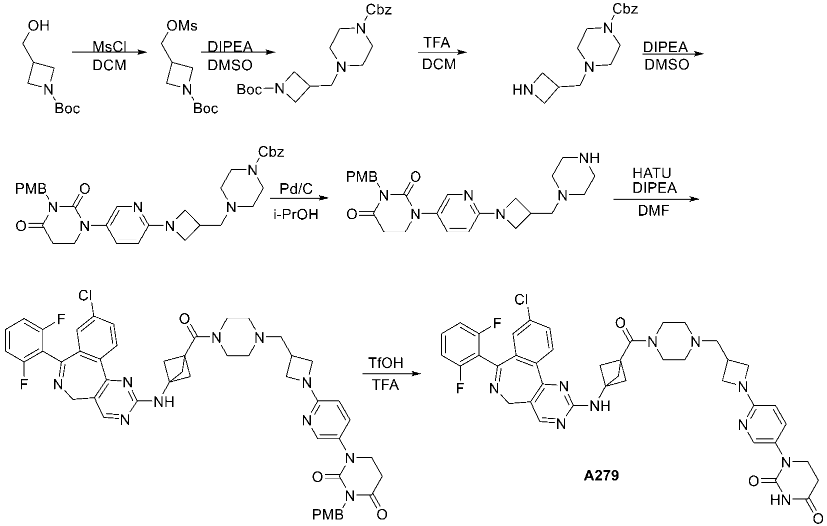

Compound **A279** (8.1 mg, yield 42.3%) was synthesized according to the synthesis method of compound **A68** described in **Example 68** and compound **A70-4** described in **Example 70.** LCMS (ESI) m/z = 793.2 [M+H]⁺. ¹H NMR (400 MHz, DMSO-*d*₆) δ 10.33 (s, 1H), 8.49 (s, 1H), 8.18 (s, 1H), 8.00 (d, *J* = 2.8 Hz, 1H), 7.85 (d, *J* = 8.4 Hz, 1H), 7.58 (s, 1H), 7.60 - 7.48 (m, 1H), 7.47 (dd, *J* = 8.8, 2.8 Hz, 1H), 7.28 (d, *J* = 2.0 Hz, 1H), 7.16 (t, *J* = 8.8 Hz, 2H), 6.38 (d, *J* = 8.8 Hz, 1H), 4.03 (t, *J* = 8.0 Hz, 2H), 3.67 (t, *J* = 6.8 Hz, 2H), 3.63 - 3.55 (m, 4H), 3.49 - 3.41 (m, 2H), 2.97 - 2.89 (m, 1H), 2.69 (t, *J* = 6.8 Hz, 2H), 2.60 (d, *J* = 7.2 Hz, 2H), 2.42 - 2.32 (m, 10H).

### Example 279 Synthesis of Compound A280:

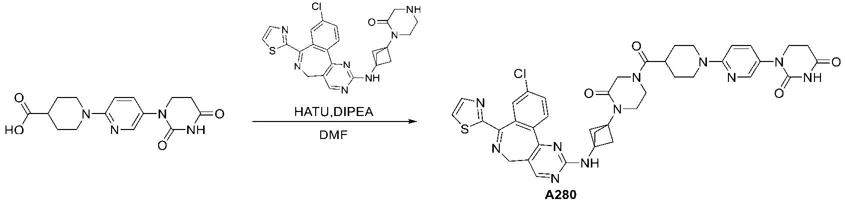

Compound **A280** (10.3 mg, yield 39.9%) was synthesized according to the synthesis method of compound **A1** described in **Example 1.** LCMS (ESI) m/z = 792.2 [M+H]⁺. ¹H NMR (400 MHz, DMSO-*d*₆) δ 10.34 (s, 1H), 8.50 (s, 1H), 8.20 - 8.08 (m, 2H), 8.05 (d, *J* = 2.8 Hz, 1H), 7.97 (d, *J* = 2.0 Hz, 1H), 7.94 (d, *J* = 3.2 Hz, 1H), 7.90 - 7.84 (m, 2H), 7.51 - 7.46 (m, 1H), 6.86 (d, *J =* 8.0 Hz, 1H), 4.78 (d, *J* = 11.6 Hz, 1H), 4.34 - 4.25 (m, 2H), 4.21 (s, 1H), 3.97 (s, 1H), 3.84 - 3.78 (m, 2H), 3.73 - 3.63 (m, 3H), 3.42 - 3.37 (m, 1H), 2.98 - 2.84 (m, 3H), 2.69 (t, *J* = 6.8 Hz, 2H), 2.42 (s, 6H), 1.72 - 1.62 (m, 2H), 1.58 - 1.45 (m, 2H).

### Example 280 Synthesis of Compound A281:

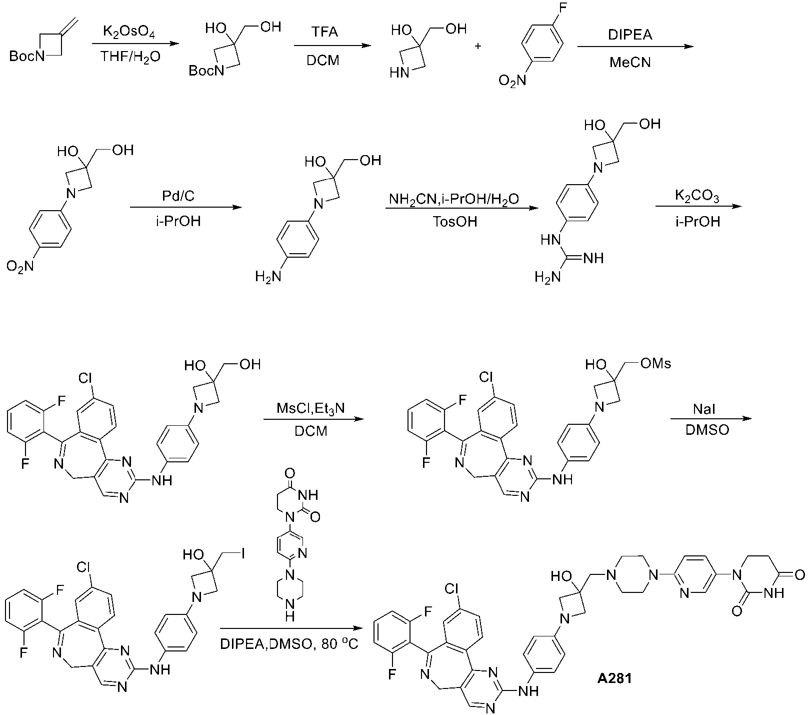

Compound **A281** (11 mg, yield 39.9%) was synthesized according to the synthesis method of compound **A211** described in **Example 210.** LCMS (ESI) m/z = 791.2 [M+H]⁺. ¹H NMR (400 MHz, DMSO-*d*₆) δ 10.37 (s, 1H), 9.22 (s, 1H), 8.58 (s, 1H), 8.25 (d, *J=* 8.4 Hz, 1H), 8.09 (d, *J* = 2.8 Hz, 1H), 7.87 (dd, *J*₁ = 8.4 Hz, *J*₂ = 2.0 Hz, 1H), 7.59 - 7.51 (m, 4H), 7.32 (d, *J = 2.4* Hz, 1H), 7.19 (t, *J* = 3.0 Hz, 2H), 6.86 (d, *J =* 8.0 Hz, 1H), 6.47 (d, *J =* 8.8 Hz, 2H), 5.53 (s, 1H), 3.89 - 3.88 (m, 2H), 3.72 (t, *J =* 6.8 Hz, 2H), 3.58 - 3.56 (m, 2H), 3.52 - 3.49 (m, 4H), 2.74 - 2.65 (m, 8H).

### Example 281 Synthesis of Compound A282:

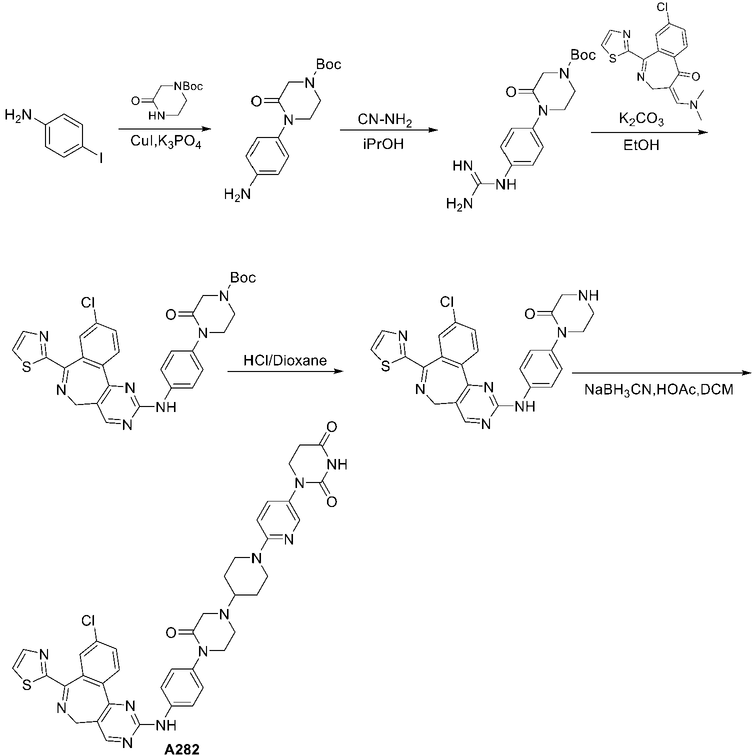

Compound **A282** (12.4 mg, yield 24.3%) was synthesized according to the synthesis method of compound **A211** described in **Example 210.** LCMS (ESI) m/z = 774.2 [M+H]⁺. ¹H NMR (400 MHz, DMSO-*d*₆) δ 10.34 (s, 1H), 9.92 (s, 1H), 8.68 (s, 1H), 8.20 (d, *J* = 8.0 Hz, 1H), 8.06 (d, *J =* 2.0 Hz, 1H), 8.00 (d, *J* = 2.0 Hz, 1H), 7.95 - 7.93 (m, 1H), 7.91 - 7.87 (m, 2H), 7.81 - 7.75 (m, 2H), 7.51 - 7.46 (m, 1H), 7.23 (d, *J =* 8.0 Hz, 2H), 6.88 (d, *J =* 8.0 Hz, 1H), 4.87 (d, *J =* 12.0 Hz, 1H), 4.36 - 4.25 (m, 2H), 3.88 (d, *J =* 12.0 Hz, 1H), 3.70 (t, *J* = 6.8 Hz, 2H), 3.63 - 3.53 (m, 2H), 3.29 (s, 2H), 2.90 - 2.79 (m, 4H), 2.70 (t, *J* = 6.8 Hz, 2H), 2.62 - 2.53 (m, 1H), 1.95 - 1.84 (m, 2H), 1.48 - 1.34 (m, 2H).

### Example 282 Synthesis of Compound A283:

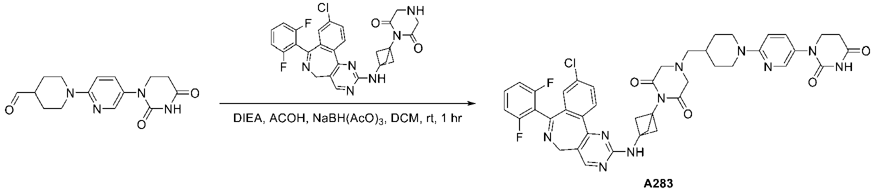

**Compound A283** (5 mg, yield 13%) was synthesized according to the synthesis method of compound **A132** described in **Example 131.** LCMS (ESI) m/z = 821.4 [M+H]⁺. ¹H NMR (400 MHz, DMSO-*d*₆) δ 10.37 (s, 1H), 8.53 (s, 1H), 8.21 (s, 2H), 8.06 (d, *J* = 2.8 Hz, 1H), 7.87 (d, *J* = 8.4 Hz, 1H), 7.61 - 7.53 (m, 1H), 7.52 - 7.47 (m, 1H), 7.32 - 7.29 (m, 1H), 7.23 - 7.16 (m, 2H), 6.86 (d, *J* = 9.2 Hz, 1H), 4.28 (d, *J* = 12.8 Hz, 2H), 3.72 (t, *J* = 6.8 Hz, 2H), 3.43 (s, 4H), 2.84 (t, *J* = 12.4 Hz, 2H), 2.72 (t, *J* = 6.8 Hz, 2H), 2.65 (s, 6H), 2.33 - 2.27 (m, 2H), 1.91 - 1.69 (m, 3H), 1.16 - 1.02 (m, 2H).

### Example 283 Synthesis of Compound A284:

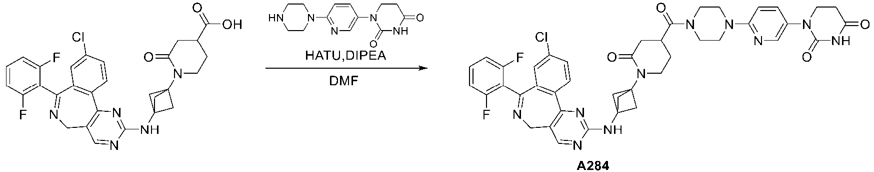

Compound **A284** (11.6 mg, yield 53.1%) was synthesized according to the synthesis method of compound **A1** described in **Example 1.** LCMS (ESI) m/z = 821.3 [M+H]⁺. ¹H NMR (400 MHz, DMSO-*d*₆) δ 10.36 (s, 1H), 8.48 (s, 1H), 8.25 - 8.16 (m, 1H), 8.12 (s, 1H), 8.09 (d, *J* = 2.8 Hz, 1H), 7.86 - 7.80 (m, 1H), 7.58 - 7.50 (m, 2H), 7.28 (d, *J* = 2.0 Hz, 1H), 7.21 - 7.12 (m, 2H), 6.89 (d, *J* = 8.0 Hz, 1H), 3.71 (t, *J* = 6.8 Hz, 2H), 3.63 (s, 2H), 3.61 - 3.52 (m, 4H), 3.50 - 3.45 (m, 2H), 3.32 - 3.19 (m, 5H), 2.70 (t, *J* = 6.8 Hz, 2H), 2.45 - 2.39 (m, 6H), 2.35 - 2.23 (m, 2H), 1.96 - 1.88 (m, 1H), 1.79 - 1.66 (m, 1H).

### Example 284 Synthesis of Compound A285:

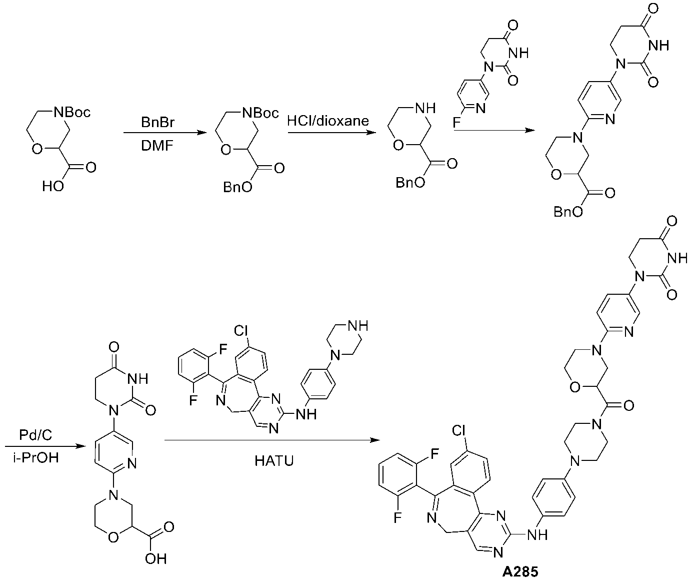

Compound **A285** (3 mg, yield 11.1%) was synthesized according to the synthesis method of compound **A185** described in **Example 184.** LCMS (ESI) m/z = 819.3 [M+H]⁺. ¹H NMR (400 MHz, DMSO-*d*₆) δ 10.39 (s, 1H), 9.67 (s, 1H), 8.62 (s, 1H), 8.29 (d, *J* = 8.4 Hz, 1H), 8.13 (d, *J* = 2.0 Hz, 1H), 7.88 (dd, *J*₁ = 8.4 Hz, *J*₂ = 2.0 Hz, 1H), 7.69 - 7.67 (m, 2H), 7.58 - 7.55 (m, 2H), 7.33 (d, *J* = 2.0 Hz, 1H), 7.21 - 7.17 (m, 2H), 7.00 - 6.93 (m, 3H), 4.42 - 4.39 (m, 1H), 4.21 - 4.18 (m, 1H), 4.08 - 3.98 (m, 2H), 3.75 - 3.65 (m, 7H), 3.20 - 2.95 (m, 6H), 2.73 (t, *J* =6.8 Hz, 2H).

### Example 285 Synthesis of Compound A286:

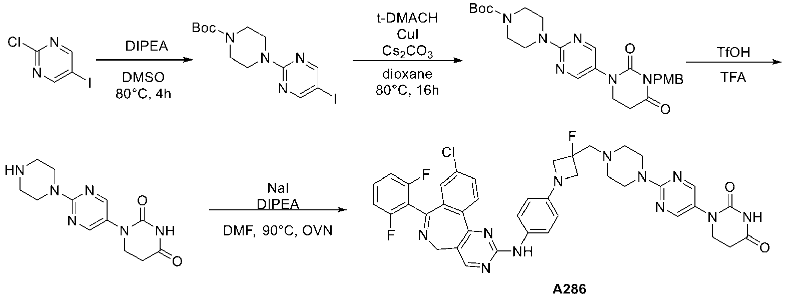

Compound **A286** (50.0 mg, yield 42%) was synthesized according to the method of compound **A101** described in **Example 101.** LCMS (ESI) m/z = 794.2 [M+H]⁺. ¹H NMR (400 MHz, DMSO-*d*₆) δ 10.45 (s, 1H), 9.54 (s, 1H), 8.57 (s, 1H), 8.35 (s, 2H), 8.24 (d, *J =* 8.5 Hz, 1H), 7.84 (dd, *J*₁ = 8.4 Hz, *J*₂ = 2.2 Hz, 1H), 7.66 - 7.50 (m, 3H), 7.30 (d, *J* = 2.1 Hz, 1H), 7.21 - 7.13 (m, 2H), 6.52 (d, *J =* 8.5 Hz, 2H), 4.04 - 3.97 (m, 2H), 3.87 - 3.80 (m, 2H), 3.75 - 3.70 (m, 6H), 2.94 (s, 1H), 2.87 (s, 1H), 2.71 (t, *J* = 6.7 Hz, 2H), 2.60 - 2.58 (m, 4H).

### Example 286 Synthesis of Compound A287:

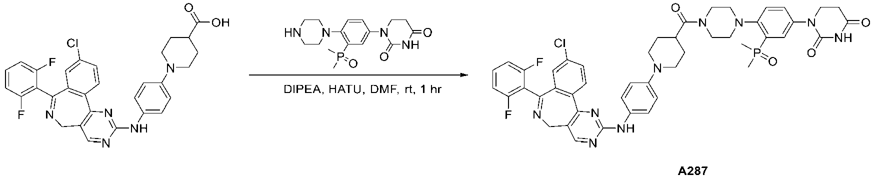

Compound **A287** (20 mg, yield 62.7%) was synthesized according to the synthesis method of compound **A1** described in **Example 1.** LCMS (ESI) m/z = 892.4 [M+H]⁺. ¹H NMR (400 MHz, DMSO-*d*₆) δ 10.46 (s, 1H), 9.64 (s, 1H), 8.62 (s, 1H), 8.29 (d, *J* = 8.4 Hz, 1H), 7.89 (dd, *J*₁ = 8.4 Hz, *J*₂ = 2.0 Hz Hz, 1H), 7.75 (dd, *J*₁ = 13.2 Hz, *J*₂ = 2.4 Hz, 1H), 7.69 ( *J*₁ = 8.4 Hz, *J*₂ = 2.0 Hz , 1H), 7.63 (m, 2H), 7.63 - 7.54 (m, 3H), 7.34 (d, *J* = 2.0 Hz, 1H), 7.24 - 7.15 (m, 2H), 6.97 *(d, J =* 8.8 Hz, 2H), 3.84 (t, *J* = 6.8 Hz, 2H), 3.79 - 3.61 (m, 4H), 3.00 - 2.81 (m, 6H), 2.79 - 2.69 (m, 4H), 1.83 - 1.70 (m, 5H).

### Example 287 Synthesis of Compound A288:

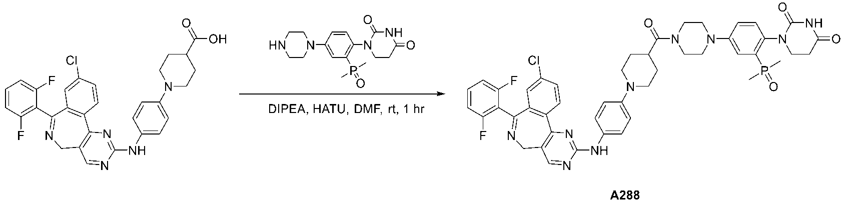

Compound **A288** (20 mg, yield 62.7%) was synthesized according to the synthesis method of compound **A1** described in **Example 1.** LCMS (ESI) m/z = 892.4 [M+H]⁺. ¹H NMR (400 MHz, DMSO-*d*₆) δ 10.34 (s, 1H), 9.63 (s, 1H), 8.61 (s, 1H), 8.28 (d, *J* = 8.4 Hz, 1H), 7.87 (dd, *J*₁ = 8.4 Hz, *J*₂ = 2.0 Hz, 1H), 7.67 - 7.61 (m, 2H), 7.60 - 7.52 (m, 1H), 7.33 (d, *J =* 2.4 Hz, 1H), 7.32 - 7.27 (m, 1H), 7.24 - 7.14 (m, 4H), 6.98 - 6.92 (m, 2H), 3.78 - 3.62 (m, 4H), 3.61 - 3.53 (m, 1H), 3.30 - 3.20 (m, 4H), 2.96 - 2.80 (m, 2H), 2.78 - 2.66 (m, 3H), 2.62 - 2.54 (m, 2H), 1.79 - 1.73 (m, 3H), 1.61 (d, *J* = 13.6 Hz, 2H).

### Example 288 Synthesis of Compound A289:

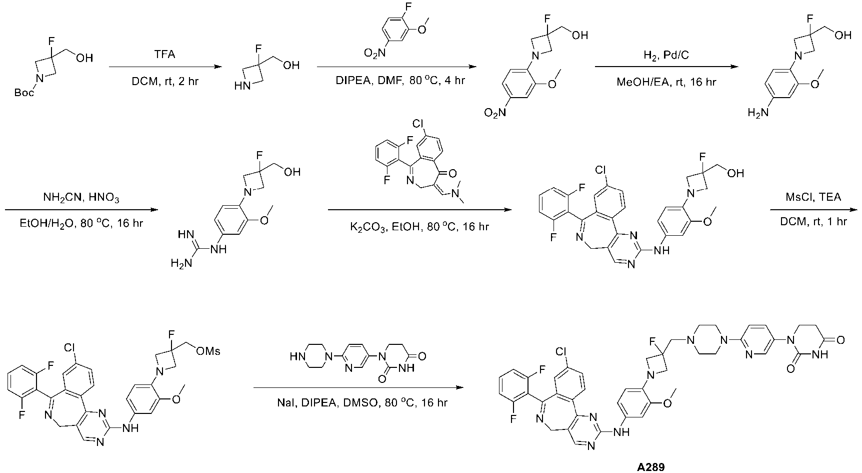

Compound **A289** (16 mg, yield 12.5%) was synthesized according to the synthesis method of compound **A101** described in **Example 101.** LCMS (ESI) m/z = 823.4 [M+H]⁺. ¹H NMR (400 MHz, DMSO-*d*₆) δ 10.18 (s, 1H), 9.42 (s, 1H), 8.60 (s, 1H), 8.31 (d, *J* = 8.4 Hz, 1H), 8.10 (d, *J* = 2.8 Hz, 1H), 7.85 (dd, *J*₁ = 8.4 Hz, *J*₂ = 2.0 Hz, 1H), 7.60 - 7.50 (m, 3H), 7.31 (d, *J =* 2.1 Hz, 1H), 7.27 - 7.22 (m, 1H), 7.19 - 7.12 (m, 2H), 6.85 (d, *J* = 9.2 Hz, 1H), 6.48 (d, *J* = 8.4 Hz, 1H), 4.09 - 4.01 (m, 2H), 3.90 - 3.81 (m, 2H), 3.80 (s, 3H), 3.73 (t, *J* = 6.8 Hz, 2H), 3.56 - 3.52 (m, 4H), 2.93 (d, *J =* 25.8 Hz, 2H), 2.73 (t, *J* = 6.8 Hz, 2H), 2.69 - 2.62 (m, 4H).

### Example 289 Synthesis of Compound A290:

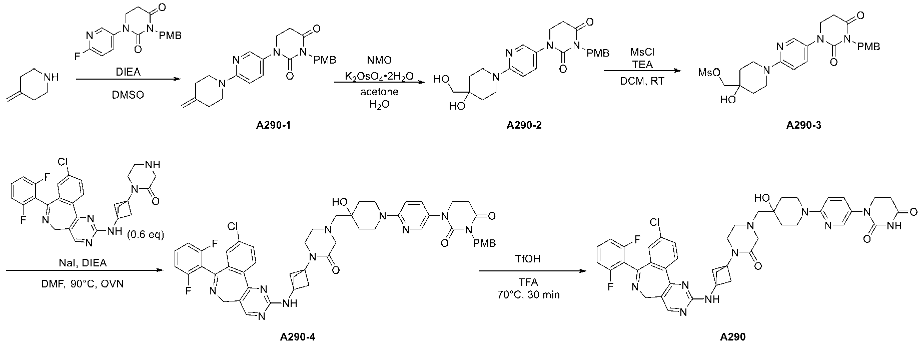

### Step 2:

At room temperature, K₂OsO₄.2H2O (98.0 mg, 0.29 mmol) and NMO (691.7 mg, 5.9 mmol) were added to a solution of compound **A290-1** (1.2 g, 2.95 mmol) in acetone (40 mL) and water (5 mL), and the reaction mixture was stirred at room temperature for 14 h. The reaction mixture was extracted with ethyl acetate, dried over anhydrous sodium sulfate, and concentrated under reduced pressure to give a crude product, which was purified by column chromatography (dichloromethane/methanol = 13/1) to give a yellow solid A290-2 (906 mg, yield 69%). LCMS (ESI) m/z = 441.2 [M+H]⁺.

### Step 5:

Compound **A290** (40.0 mg, yield 57.3%) was synthesized according to the synthesis method of compound **A211** described in **Example 210.** LCMS (ESI) m/z = 823.2 [M+H]⁺. ¹H NMR (400 MHz, DMSO-*d*₆) δ 10.33 (s, 1H), 8.48 (s, 1H), 8.20 - 8.18 (m, 1H), 8.14 (s, 1H), 8.03 (d, *J* = 2.7 Hz, 1H), 7.88 - 7.80 (m, 1H), 7.58 - 7.50 (m, 1H), 7.46 (dd, *J*₁ = 9.1 Hz, *J*₂ = 2.7 Hz, 1H), 7.28 (d, *J* = 2.1 Hz, 1H), 7.18 - 7.14 (m, 2H), 6.84 (d, *J* = 9.1 Hz, 1H), 4.37 (s, 1H), 3.85 - 3.82 (m, 2H), 3.69 (t, *J* = 6.7 Hz, 2H), 3.29 - 3.26 (m, 3H), 3.12 (s, 2H), 2.80 - 2.78 (m, 2H), 2.69 (t, *J =* 6.7 Hz, 2H), 2.42 (s, 6H), 2.35 (s, 2H), 1.57 - 1.47 (m, 4H).

### Example 290 Synthesis of Compound A291:

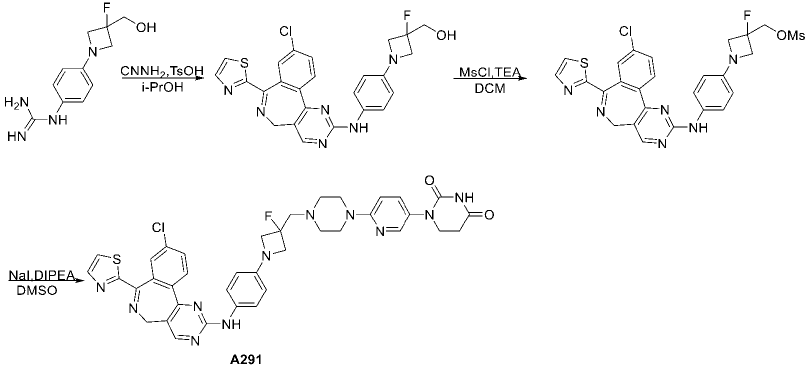

Compound **A291** (7.2 mg, yield 22%) was synthesized according to the synthesis method of compound **A211** described in **Example 210** and compound **A48-2** described in **Example 48.** LCMS (ESI) m/z = 764.2 [M+H]⁺. ¹H NMR (400 MHz, DMSO-*d*₆) δ 10.36 (s, 1H), 9.52 (s, 1H), 8.58 (s, 1H), 8.15 (d, *J* = 8.4 Hz, 1H), 8.07 (d, *J =* 2.8 Hz, 1H), 8.00 - 7.92 (m, 2H), 7.91 - 7.84 (m, 2H), 7.62 - 7.43 (m, 3H), 6.85 (d, *J* = 9.2 Hz, 1H), 6.49 (d, *J* = 8.4 Hz, 2H), 4.82 (d, *J* = 11.6 Hz, 1H), 4.04 - 3.93 (m, 2H), 3.89 - 3.76 (m, 3H), 3.70 (t*, J* = 6.8 Hz, 2H), 3.56 - 3.41 (m, 4H), 2.90 (d, *J* = 26.0 Hz, 2H), 2.70 (t, *J* = 6.8 Hz, 2H), 2.65 - 2.57 (m, 4H).

### Example 291 Synthesis of Compound A292:

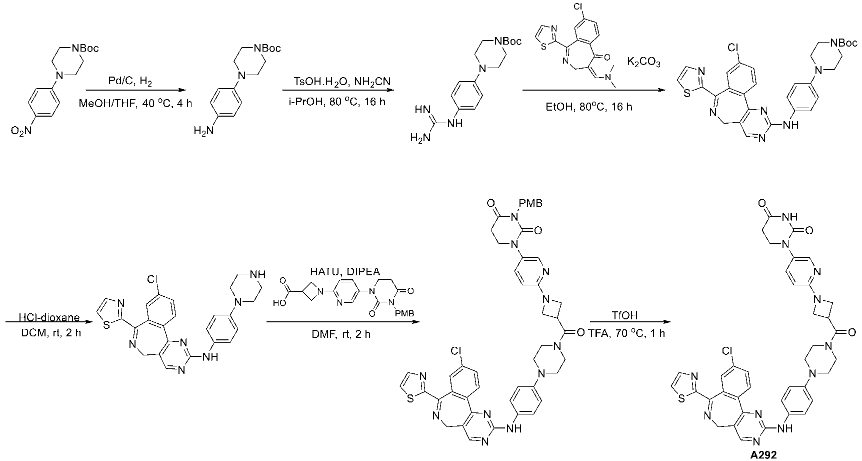

Compound **A292** (24 mg, yield 28%) was synthesized according to the synthesis method of compound **A211** described in **Example 210.** LCMS (ESI) m/z = 760.1 [M+H]⁺. ¹H NMR (400 MHz, DMSO-*d*₆) δ 10.38 (s, 1H), 9.66 (s, 1H), 8.64 (s, 1H), 8.20 (d, *J =* 8.4 Hz, 1H), 8.06 (d, *J* = 2.8 Hz, 1H), 8.02 (d, *J =* 2.0 Hz, 1H), 7.97 (d, *J* = 3.2 Hz, 1H), 7.95 - 7.88 (m, 2H), 7.67 (d, *J* = 8.8 Hz, 2H), 7.61 - 7.47 (m, 1H), 6.96 (d, *J* = 8.8 Hz, 2H), 6.48 (d, *J* = 8.8 Hz, 1H), 4.87 (d, *J* = 11.6 Hz, 1H), 4.18 (t, *J* = 8.4 Hz, 2H), 4.08 (t, *J* = 6.8 Hz, 2H), 3.99 - 3.84 (m, 2H), 3.72 (t, *J=* 6.4 Hz, 2H), 3.69 - 3.60 (m, 2H), 3.57 - 3.45 (m, 2H), 3.16 - 3.02 (m, 4H), 2.72 (t, *J* = 6.4 Hz, 2H).

### Example 292 Synthesis of Compound A293:

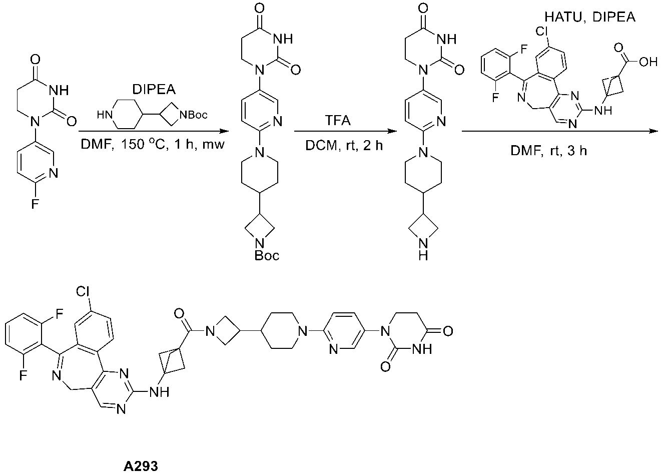

Compound **A293** (20.0 mg, yield 60%, white solid) was synthesized according to the synthesis method of compound **A132** described in **Example 131.** LCMS (ESI) m/z = 778.2 [M+H]⁺. ¹H NMR (400 MHz, DMSO-*d*₆) δ 10.37 (s, 1H), 8.51 (s, 1H), 8.30 - 8.14 (m, 2H), 8.07 (d, *J=* 2.8 Hz, 1H), 7.91 - 7.78 (m, 1H), 7.63 - 7.53 (m, 1H), 7.53 - 7.45 (m, 1H), 7.31 (d, *J* = 2.4 Hz, 1H), 7.26 - 7.12 (m, 2H), 6.88 (d, *J* = 8.8 Hz, 1H), 4.45 - 4.21 (m, 3H), 4.12 - 3.97 (m, 1H), 3.97 - 3.85 (m, 1H), 3.72 (t, *J* = 6.8 Hz, 2H), 3.70 - 3.58 (m, 1H), 2.81 (t, *J* = 12.4 Hz, 2H), 2.72 (t, *J* = 6.4 Hz, 2H), 2.35 (s, 7H), 1.73 (d, *J* = 11.6 Hz, 3H), 1.18 - 0.88 (m, 2H).

### Example 293 Synthesis of Compound A294:

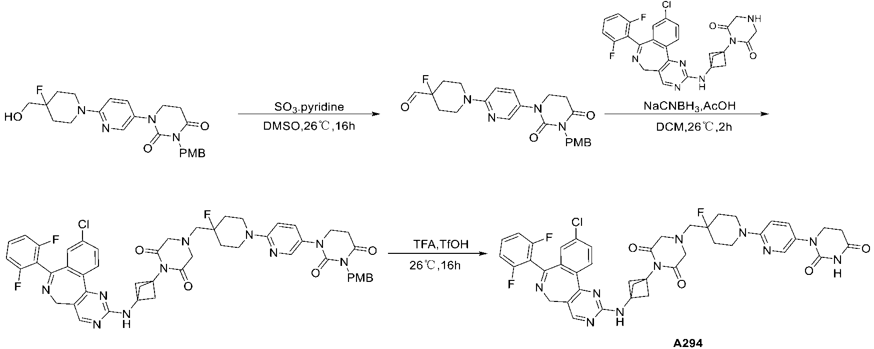

Compound **A294** (15.0 mg, yield 20%) was synthesized according to the synthesis method of compound **A132** described in **Example 131.** LCMS (ESI) m/z = 839.2 [M+H]⁺. ¹H NMR (400 MHz, DMSO-*d*₆) δ 10.38 (s, 1H), 8.53 (s, 1H), 8.21 (s, 1H), 8.09 (d, *J* = 2.8 Hz, 1H), 7.90 - 7.82 (m, 1H), 7.60 - 7.51 (m, 2H), 7.32 (d, *J =* 2.0 Hz, 1H), 7.25 - 7.15 (m, 2H), 6.93 (d, *J* = 9.2 Hz, 1H), 4.10 - 4.00 (m, 2H), 3.72 (t, *J =* 6.8 Hz, 2H), 3.55 (s, 4H), 3.23 (t, *J* = 11.6 Hz, 2H), 2.81 - 2.71 (m, 4H), 2.66 (s, 6H), 1.95 - 1.84 (m, 2H), 1.75 - 1.62 (m, 2H).

### Example 294 Synthesis of Compound A295:

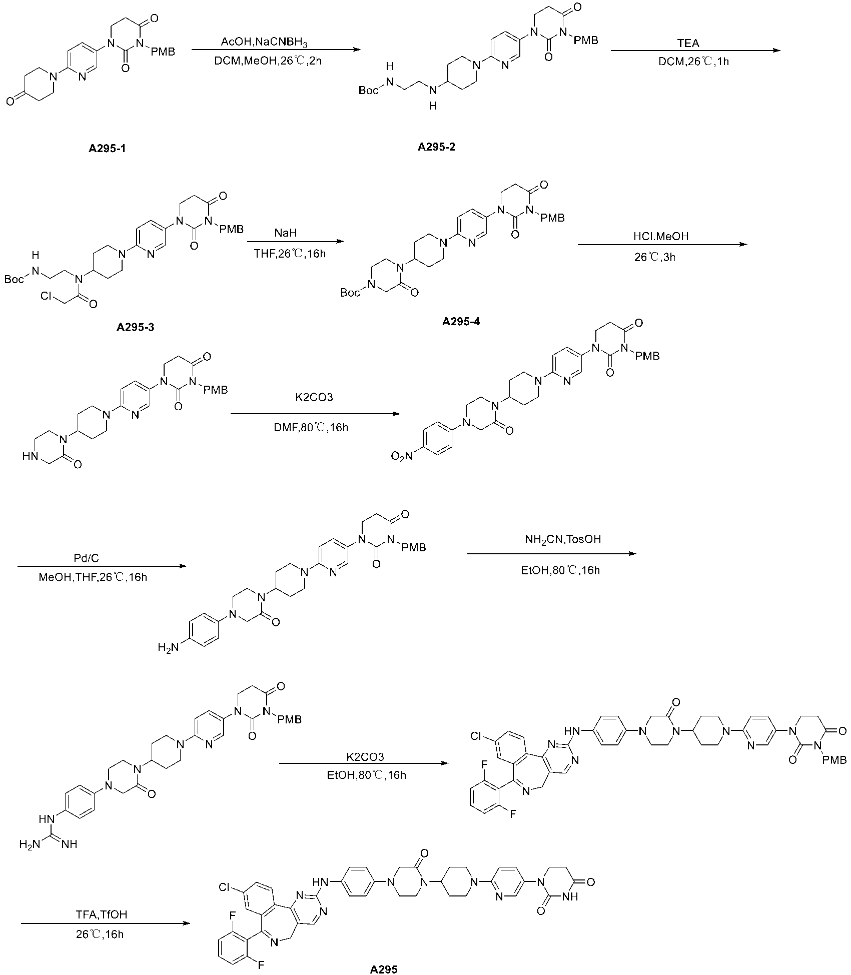

### Step 1:

N-Boc-ethylenediamine (579.2 mg, 2.95 mmol), acetic acid (72.4 mg, 1.23 mmol) and sodium cyanoborohydride (462.6 mg, 7.36 mmol) were added to a mixed solution of compound **A295-1** (1.0 g, 2.45 mmol) in dichloromethane (10 mL) and methanol (2 mL) at room temperature. The reaction mixture was stirred at 26°C for 2 h. The reaction mixture was concentrated, and the residue was separated for purification by silica gel column chromatography to give a white solid **A295-2** (1.00 g, yield 73%). LCMS (ESI) m/z = 553.3 [M+H]⁺.

### Step 2:

At room temperature, chloroacetyl chloride (208.2 mg, 1.84 mmol) and TEA (339.2 mg, 3.35 mmol) were added to a solution of compound **A295-2** (924.8 mg, 1.68 mmol) in dichloromethane (10 mL). The reaction mixture was stirred at 26°C for 1 h. The reaction mixture was concentrated, and the residue was separated for purification by silica gel column chromatography to give a white solid **A295-3** (800.0 mg, yield 75%). LCMS (ESI) m/z = 629.2 [M+H]⁺.

### Step 3:

60% NaH (57.2 mg, 1.43 mmol) was added to a solution of compound **A295-3** (750.0 mg, 1.19 mmol) in tetrahydrofuran (10 mL) at room temperature. The reaction mixture was stirred at 26°C for 16 h. The reaction mixture was concentrated, and the residue was separated for purification by silica gel column chromatography to give a white solid **A295-4** (500.0 mg, yield 70%). LCMS (ESI) m/z = 593.1 [M+H]⁺.

### Step 9:

Compound **A295** (2.3 mg, yield 13%) was synthesized according to the synthesis method of compound **A252** described in **Example 251.** LCMS (ESI) m/z = 803.2 [M+H]⁺. ¹H NMR (400 MHz, DMSO-*d*₆) δ 10.38 (s, 1H), 9.67 (s, 1H), 8.62 (s, 1H), 8.28 (d, *J =* 8.4 Hz, 1H), 8.09 (d, *J =* 2.8 Hz, 1H), 7.91 - 7.86 (m, 1H), 7.74 - 7.69 (m, 2H), 7.61 - 7.51 (m, 2H), 7.34 (d, *J* = 2.4 Hz, 1H), 7.25 - 7.15 (m, 2H), 7.00 - 6.95 (m, 3H), 4.47 - 4.44 (m, 2H), 4.13 - 4.10 (m, 4H), 3.77 (s, 2H), 3.74 - 3.65 (m, 2H), 3.22 - 3.19 (m, 2H), 2.89 - 2.85 (m, 2H), 2.75 - 2.70 (m, 2H), 2.10 (s, 1H), 1.77 - 1.62 (m, 2H), 1.26 (s, 2H).

### Example 295 Synthesis of Compound A296:

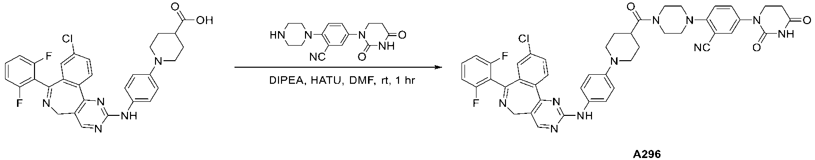

Compound **A296** (18 mg, yield 59.9%) was synthesized according to the synthesis method of compound **A1** described in **Example 1.** LCMS (ESI) m/z = 841.4 [M+H]⁺. ¹H NMR (400 MHz, DMSO-*d*₆) δ 10.48 (s, 1H), 9.63 (s, 1H), 8.61 (s, 1H), 8.28 (d, *J* = 8.4 Hz, 1H), 7.87 (dd, *J*₁ = 8.4 Hz, *J*₂ = 2.0 Hz, 1H), 7.76 (d, *J* = 2.8 Hz, 1H), 7.68 - 7.52 (m, 4H), 7.33 (d, *J* = 2.4 Hz, 1H), 7.26 - 7.15 (m, 3H), 6.98 - 6.92 (m, 2H), 3.84 - 3.75 (m, 4H), 3.72 - 3.61 (m, 4H), 3.24 - 3.10 (m, 4H), 2.89 - 2.80 (m, 1H), 2.77 - 2.67 (m, 4H), 1.82 - 1.69 (m, 4H).

### Example 296 Synthesis of Compound A297:

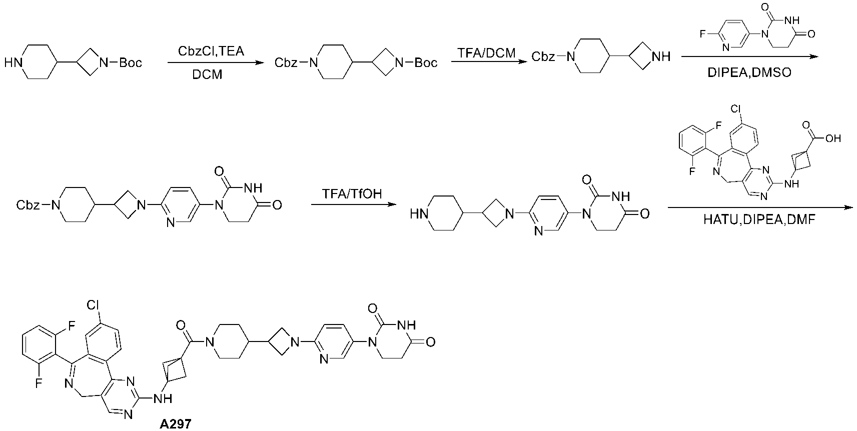

Compound **A297** (6.3 mg, yield 46%) was synthesized according to the synthesis method of compound **A132** described in **Example 131.** LCMS (ESI) m/z = 778.2 [M+H]⁺. ¹H NMR (400 MHz, DMSO-*d*₆) δ 10.33 (s, 1H), 8.49 (s, 1H), 8.23 - 8.15 (m, 2H), 8.00 (d, *J* = 2.8 Hz, 1H), 7.84 (d, *J* = 8.4 Hz, 1H), 7.59 - 7.50 (m, 1H), 7.49 - 7.44 (m, 1H), 7.28 (d, *J* = 2.0 Hz, 1H), 7.21 - 7.12 (m, 2H), 6.38 (d, *J* = 8.0 Hz, 1H), 4.40 - 4.32 (m, 1H), 4.16 - 4.06 (m, 1H), 3.98 (t, *J =* 8.0 Hz, 2H), 3.71 - 3.63 (m, 4H), 3.10 - 2.98 (m, 1H), 2.69 (t, *J=* 6.8 Hz, 2H), 2.60 - 2.53 (m, 1H), 2.46 - 2.30 (m, 7H), 1.81 - 1.63 (m, 3H), 1.09 - 0.84 (m, 2H).

### Example 297 Synthesis of Compound A298:

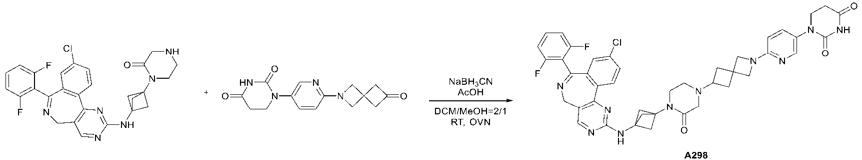

Compound **A313** (18.0 mg, yield 58.2%) was synthesized according to the method of compound **A132** described in **Example 131.** LCMS (ESI) m/z = 805.2 [M+H]⁺. ¹H NMR (400 MHz, DMSO-*d*₆) δ 10.33 (s, 1H), 8.48 (s, 1H), 8.19 (s, 1H), 8.14 (s, 1H), 8.00 (d, *J* = 2.6 Hz, 1H), 7.83 (d, *J* = 8.1 Hz, 1H), 7.57 - 7.50 (m, 1H), 7.47 (dd, *J*₁ = 8.8 Hz, *J*₂ = 2.6 Hz, 1H), 7.28 (d, *J* = 2.2 Hz, 1H), 7.19 - 7.14 (m, 2H), 6.37 (d, *J* = 8.8 Hz, 1H), 3.95 (s, 2H), 3.84 (s, 2H), 3.67 (t, *J* = 6.7 Hz, 2H), 3.29 - 3.26 (m, 2H), 2.85 (s, 2H), 2.73 - 2.66 (m, 3H), 2.53 - 2.51 (m, 2H), 2.42 (s, 6H), 2.34 - 2.29 (m, 2H), 2.05 - 2.00 (m, 2H).

### Example 298 Synthesis of Compound A299:

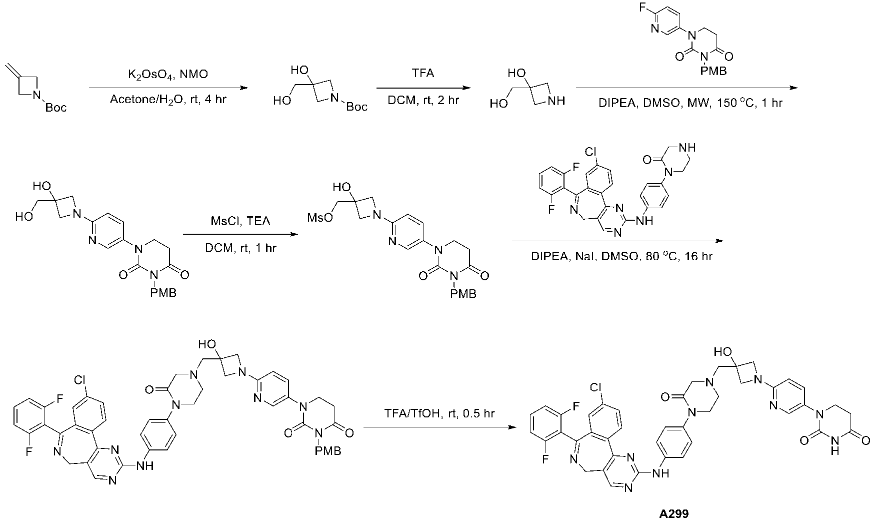

Compound **A299** (7 mg, yield 26.8%) was synthesized according to the synthesis method of compound **A290** described in **Example 289.** LCMS (ESI) m/z = 805.3 [M+H]⁺. ¹H NMR (400 MHz, DMSO-*d*₆) δ 10.37 (s, 1H), 9.97 (s, 1H), 8.70 (s, 1H), 8.32 (d, *J* = 8.4 Hz, 1H), 8.05 (d, *J* = 2.8 Hz, 1H), 7.92 - 7.82 (m, 3H), 7.62 - 7.49 (m, 2H), 7.36 (d, *J* = 2.4 Hz, 1H), 7.32 - 7.27 (m, 2H), 7.24 - 7.15 (m, 2H), 6.49 (d, *J =* 8.8 Hz, 1H), 5.81 (s, 1H), 4.02 (d, *J =* 8.4 Hz, 2H), 3.79 (d, *J =* 8.4 Hz, 2H), 3.71 (t, *J* = 6.8 Hz, 2H), 3.68 - 3.63 (m, 2H), 3.39 - 3.38 (s, 2H), 3.04 - 2.97 (m, 2H), 2.80 (s, 2H), 2.73 (t, *J* = 6.8 Hz, 2H).

### Example 299 Synthesis of Compound A300:

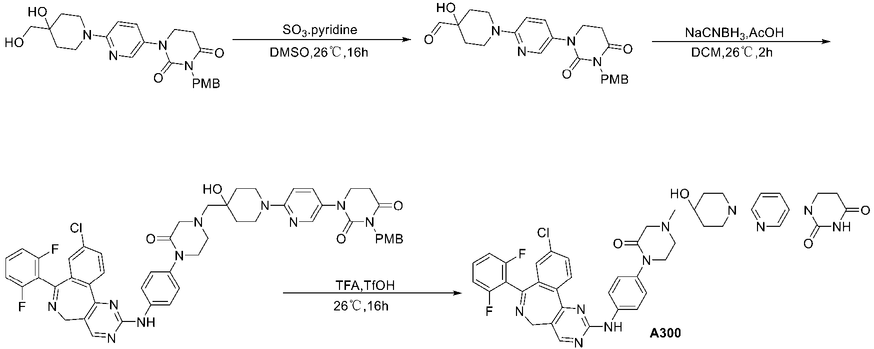

Compound **A300** (15.0 mg, yield 20%) was synthesized according to the synthesis method of compound **A132** described in **Example 131.** LCMS (ESI) m/z = 833.2 [M+H]⁺. ¹H NMR (400 MHz, DMSO-*d*₆) δ 10.37 (s, 1H), 9.99 (s, 1H), 8.71 (s, 1H), 8.32 (d, *J* = 8.4 Hz, 1H), 8.08 (d, *J* = 2.8 Hz, 1H), 7.90 (dd, Ji = 8.4 Hz, *J*₂ = 2.4 Hz, 1H), 7.85 (d, *J* = 8.8 Hz, 2H), 7.60 - 7.51 (m, 1H), 7.50 - 7.45 (m, 1H), 7.36 (d,*J* = 2.4 Hz, 1H), 7.35 - 7.25 (m, 2H), 7.24 - 7.18 (m, 2H), 6.89 (d, *J* = 9.2 Hz, 1H), 4.47 (s, 2H), 3.95 - 3.85 (m, 2H), 3.73 (t, *J* = 6.8 Hz, 2H), 3.68 - 3.62 (m, 2H), 3.05 - 2.95 (m, 2H), 2.73 (t, *J* = 6.4 Hz, 2H), 2.50 - 2.45 (m, 2H), 1.70 - 1.60 (m, 4H).

### Example 300 Synthesis of Compound A301:

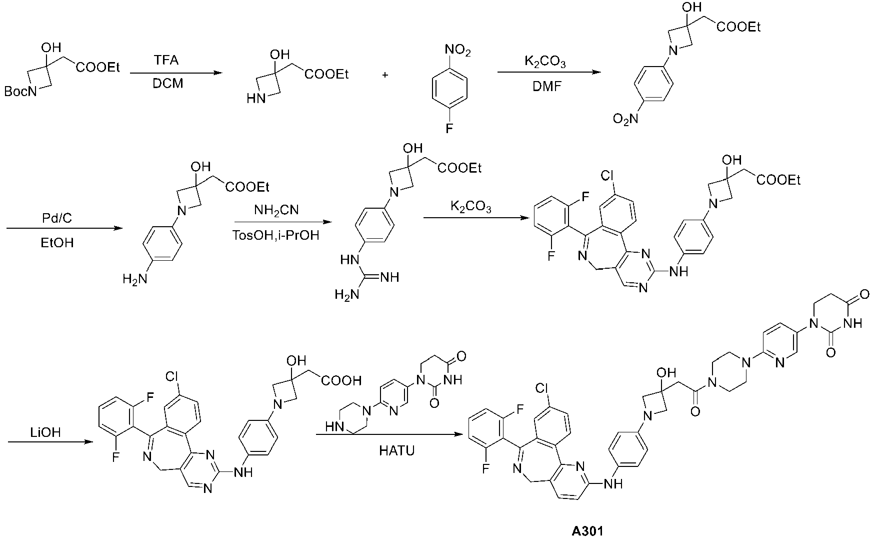

Compound **A292** (15.0 mg, yield 20%) was synthesized according to the synthesis method of compound **A211** described in **Example 210.** LCMS (ESI) m/z = 818.2 [M+H]⁺. ¹H NMR (400 MHz, DMSO-*d*₆) δ 10.37 (s, 1H), 9.52 (s, 1H), 8.58 (s, 1H), 8.25 (d, *J* = 8.4 Hz, 1H), 8.12 (d, *J* = 2.8 Hz, 1H), 7.87 (dd, *J*₁ = 8.4 Hz, *J*₂ = 2.4 Hz, 1H), 7.59 - 7.53 (m, 4H), 7.32 (d, *J* = 2.4 Hz, 1H), 7.21 - 7.17 (m, 2H), 6.48 (d, *J* = 8.8 Hz, 1H), 5.62 (s, 1H), 3.98 - 3.96 (m, 2H), 3.73 (t, *J* = 6.8 Hz, 2H), 3.64 - 3.51 (m, 10H), 2.93 (s, 2H), 2.72 (t, *J* = 6.8 Hz, 2H).

### Example 301 Synthesis of Compound A302:

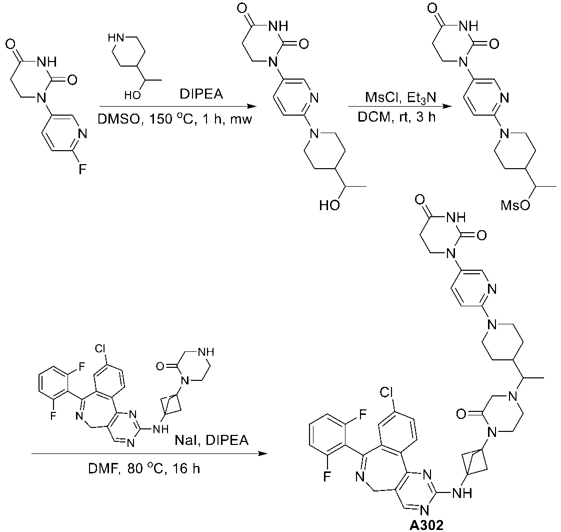

Compound **A302** (2.5 mg, yield 4%, white solid) was synthesized according to the synthesis method of compound **A132** described in **Example 131.** LCMS (ESI) m/z = 821.2 [M+H]⁺. ¹H NMR (400 MHz, DMSO-*d*₆) δ 10.33 (s, 1H), 8.51 (s, 1H), 8.29 - 8.10 (m, 2H), 8.02 (d, *J =* 2.8 Hz, 1H), 7.86 (d, *J =* 8.4 Hz, 1H), 7.64 - 7.50 (m, 1H), 7.49 - 7.38 (m, 1H), 7.30 (d, *J* = 2.0 Hz, 1H), 7.19 (t, *J =* 8.8 Hz, 2H), 6.47 (d, *J =* 8.8 Hz, 1H), 3.89 - 3.75 (m, 1H), 3.70 (t, *J* = 6.4 Hz, 2H), 3.47 - 3.41 (m, 2H), 3.31 - 3.26 (m, 2H), 2.97 (s, 2H), 2.72 (t, *J* = 6.8 Hz, 2H), 2.68 - 2.60 (m, 2H), 2.49 - 2.37 (m, 9H), 2.28 - 2.10 (m, 1H), 2.03 - 1.88 (m, 1H), 1.75 - 1.63 (m, 1H), 1.63 - 1.50 (m, 1H), 1.49 - 1.34 (m, 1H), 1.21 (d, *J* = 6.0 Hz, 3H).

### Example 302 Synthesis of Compound A303:

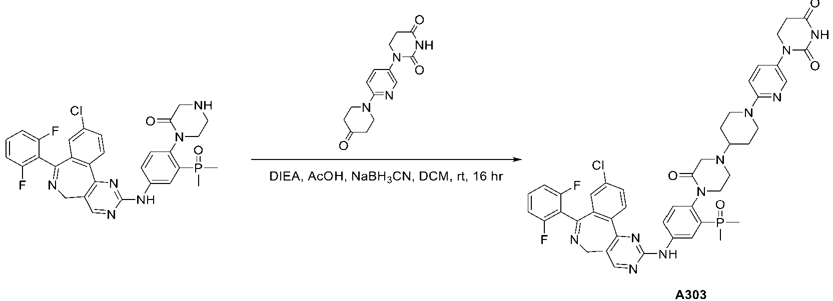

Compound **A303** (8 mg, yield 13.8%) was synthesized according to the synthesis method of compound **A132** described in **Example 131.** LCMS (ESI) m/z = 879.4 [M+H]⁺. ¹H NMR (400 MHz, DMSO-*d*₆) δ 10.37 (s, 1H), 10.15 (s, 1H), 8.74 (s, 1H), 8.43 - 8.35 (m, 2H), 8.09 (d, *J* = 2.8 Hz, 1H), 8.00 - 7.93 (m, 1H), 7.85 (dd, *J*₁ = 8.0 Hz, *J*₂ = 2.4 Hz, 1H), 7.62 - 7.54 (m, 1H), 7.53 - 7.49 (m, 1H), 7.36 (d, *J* = 2.4 Hz, 1H), 7.30 - 7.25 (m, 1H), 7.24 - 7.15 (m, 2H), 6.91 (d, *J=* 9.2 Hz, 1H), 4.39 - 4.29 (m, 2H), 3.77 - 3.66 (m, 3H), 3.59 - 3.49 (m, 1H), 3.45 - 3.39 (m, 1H), 3.25 - 3.16 (m, 1H), 3.07 - 2.98 (m, 1H), 2.93 - 2.83 (m, 2H), 2.82 - 2.76 (m, 1H), 2.73 (t, *J* = 6.8 Hz, 2H), 2.66 - 2.56 (m, 1H), 2.00 - 1.87 (m, 2H), 1.74 - 1.59 (m, 6H), 1.54 - 1.37 (m, 2H).

### Example 303 Synthesis of Compound A304:

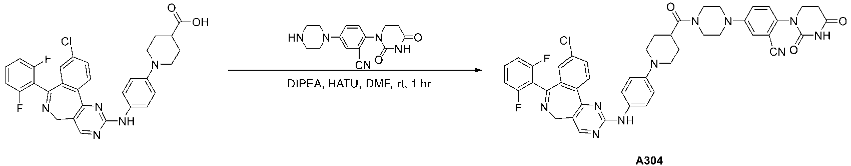

Compound **A304** (6 mg, yield 13.3%) was synthesized according to the synthesis method of compound **A1** described in **Example 1.** LCMS (ESI) m/z = 841.4 [M+H]⁺. ¹H NMR (400 MHz, DMSO-*d*₆) δ 10.57 (s, 1H), 9.63 (s, 1H), 8.62 (s, 1H), 8.28 (d, *J* = 8.4 Hz, 1H), 7.88 (dd, *J*₁ = 8.4 Hz, *J*₂ = 2.4 Hz, 1H), 7.68 - 7.63 (m, 2H), 7.62 - 7.53 (m, 1H), 7.47 - 7.42 (m, 2H), 7.39 - 7.32 (m, 2H), 7.24 - 7.14 (m, 2H), 6.99 - 6.92 (m, 2H), 3.80 - 3.64 (m, 8H), 3.33 - 3.16 (m, 4H), 2.89 - 2.80 (m, 1H), 2.79 - 2.67 (m, 4H), 1.81 - 1.71 (m, 4H).

### Example 304 Synthesis of Compound A305:

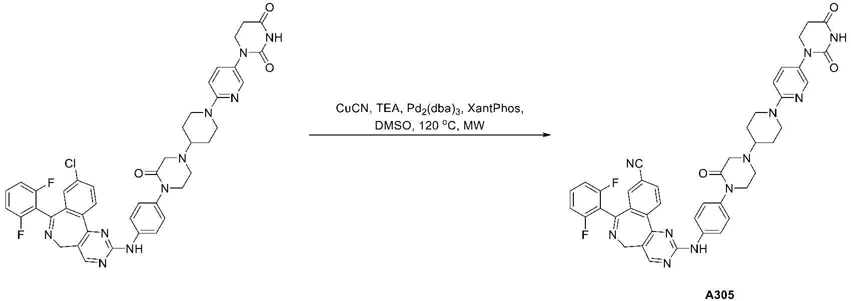

Compound **A305** (18 mg, yield 49.4%) was synthesized according to the synthesis method of compound **A266** described in **Example 265.** LCMS (ESI) m/z = 794.3 [M+H]⁺. ¹H NMR (400 MHz, DMSO-*d*₆) δ 10.38 (s, 1H), 10.05 (s, 1H), 8.75 (s, 1H), 8.44 (d, *J =* 8.4 Hz, 1H), 8.24 (dd, *J*₁ = 8.4 Hz, *J*₂ = 1.6 Hz, 1H), 8.09 (d, *J* = 2.8 Hz, 1H), 7.95-7.93 (m, 1H), 7.86-7.80 (m, 2H), 7.62 - 7.50 (m, 2H), 7.33 - 7.27 (m, 2H), 7.21 - 7.15 (m, 2H), 6.92 (d, *J* = 9.2 Hz, 1H), 4.40 - 4.30 (m, 2H), 3.73 (t, *J* = 6.8 Hz, 2H), 3.66 - 3.59 (m, 2H), 3.34 - 3.32 (m, 2H), 2.94 - 2.83 (m, 4H), 2.73 (t, *J* = 6.8 Hz, 2H), 2.65 - 2.56 (m, 1H), 1.99 - 1.89 (m, 2H), 1.52 - 1.38 (m, 2H).

### Example 305 Synthesis of Compound A306:

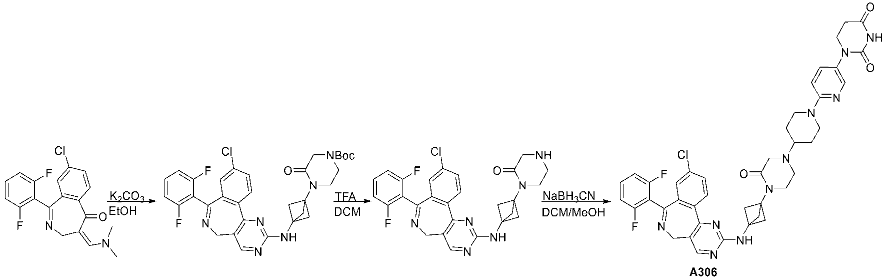

Compound **A306** (15.9 mg, yield 34.9%) was synthesized according to the synthesis method of compound **A132** described in **Example 131.** LCMS (ESI) m/z = 793.2 [M+H]⁺. ¹H NMR (400 MHz, DMSO-*d*₆) δ 10.34 (s, 1H), 8.48 (s, 1H), 8.27 - 8.10 (m, 2H), 8.05 (d, *J* = 2.8 Hz, 1H), 7.83 (d, *J* = 8.8 Hz, 1H), 7.60 - 7.44 (m, 2H), 7.28 (d, *J* = 2.0 Hz, 1H), 7.16 (t, *J* = 8.8 Hz, 2H), 6.86 (d, *J* = 8.8 Hz, 1H), 4.28 (d, *J* = 12.8 Hz, 2H), 3.69 (t, *J* = 6.8 Hz, 2H), 3.29 - 3.20 (m, 2H), 3.09 (s, 2H), 2.90 - 2.60 (m, 7H), 2.42 (s, 6H), 1.87 - 1.80 (m, 2H), 1.42 - 1.30 (m, 2H).

### Example 306 Synthesis of Compound A307:

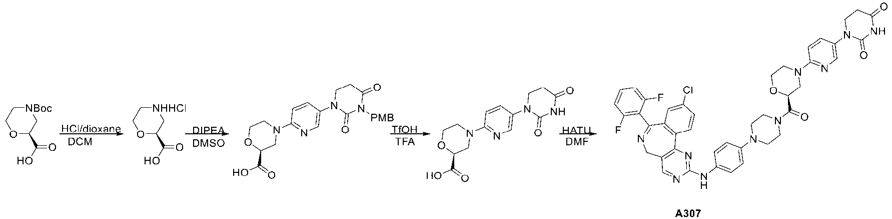

Compound **A307** (33.8 mg, yield 26.5%) was synthesized according to the synthesis method of compound **A54** described in **Example 54.** LCMS (ESI) m/z = 819.1 [M+H]⁺. ¹H NMR (400 MHz, DMSO-*d*₆) δ 10.36 (s, 1H), 9.64 (s, 1H), 8.59 (s, 1H), 8.25 (d, *J* = 8.4 Hz, 1H), 8.11 (d, *J* = 2.8 Hz, 1H), 7.85 (dd, *J*₁ = 8.4 Hz, *J*₂ = 2.0 Hz, 1H), 7.66 (d, *J* = 8.8 Hz, 2H), 7.60 - 7.48 (m, 2H), 7.31 (d, *J* = 2.0 Hz, 1H), 7.17 (t, *J* = 8.8 Hz, 2H), 7.00 - 6.86 (m, 3H), 4.39 (dd, *J*₁ = 10.1 Hz, *J*₂ = 2.8 Hz, 1H), 4.17 (d, *J* = 13.2 Hz, 1H), 4.09 - 3.90 (m, 2H), 3.80 - 3.57 (m, 7H), 3.22 - 2.89 (m, 6H), 2.70 (t, *J* = 6.8 Hz, 2H).

### Example 307 Synthesis of Compound A308:

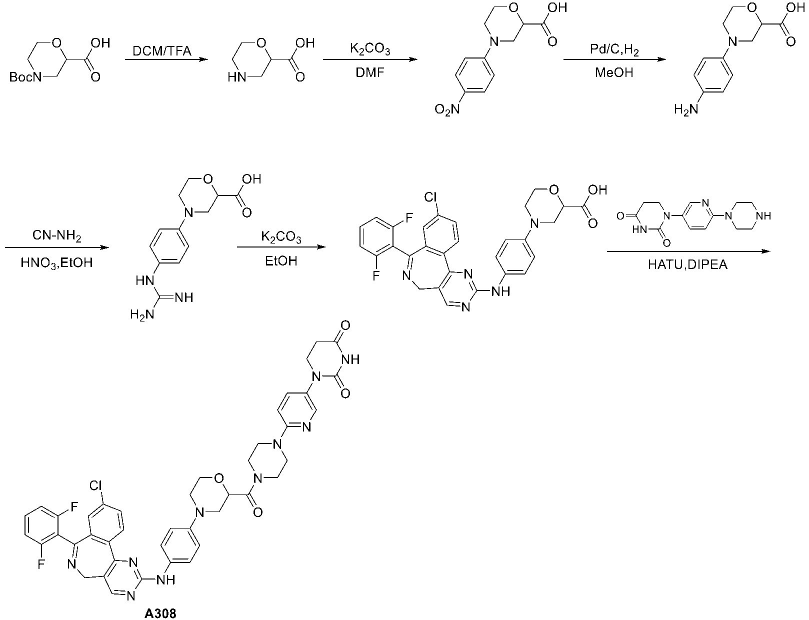

Compound **A308** (10.3 mg, yield 35.3%) was synthesized according to the synthesis method of compound **A101** described in **Example 101.** LCMS (ESI) m/z = 819.1 [M+H]⁺. ¹H NMR (400 MHz, DMSO-*d*₆) δ 10.36 (s, 1H), 9.64 (s, 1H), 8.60 (s, 1H), 8.26 (d, *J* = 8.0 Hz, 1H), 8.10 (d, *J* = 2.8 Hz, 1H), 7.87 - 7.83 (m, 1H), 7.69 - 7.63 (m, 2H), 7.58 - 7.50 (m, 2H), 7.31 (d, *J* = 2.0 Hz, 1H), 7.20 - 7.12 (m, 2H), 6.97 (d, *J* = 8.0 Hz, 2H), 6.90 (d, *J =* 8.0 Hz, 1H), 4.50 - 4.45 (m, 1H), 4.03 - 3.96 (m, 1H), 3.85 - 3.76 (m, 1H), 3.74 - 3.42 (m, 12H), 2.88 - 2.80 (m, 1H), 2.74 - 2.66 (m, 3H).

### Example 308 Synthesis of Compound A309:

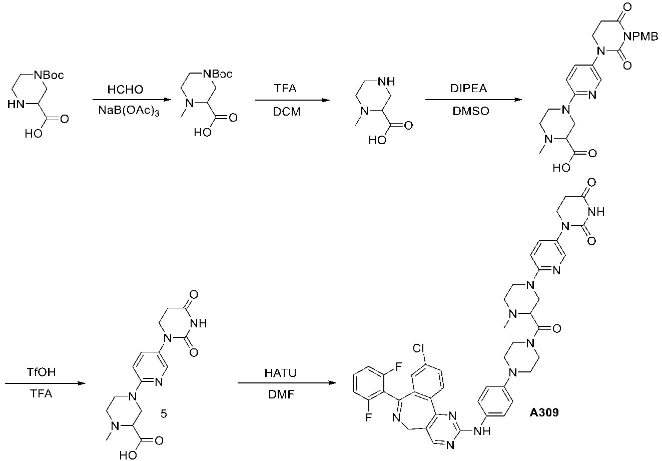

Compound **A309** (12 mg, yield 35.3%) was synthesized according to the synthesis method of compound **A185** described in **Example 184.** LCMS (ESI) m/z = 832.3 [M+H]⁺. ¹H NMR (400 MHz, DMSO-*d*₆) δ 10.36 (s, 1H), 9.64 (s, 1H), 8.60 (s, 1H), 8.28 (d, *J* = 8.0 Hz, 1H), 8.10 (d, *J* = 2.8 Hz, 1H), 7.89 - 7.86 (m, 1H), 7.70 - 7.68 (m, 2H), 7.58 - 7.53 (m, 2H), 7.33 (d, *J* = 2.0 Hz, 1H), 7.20 - 7.17 (m, 2H), 6.96 (d, *J* = 8.0 Hz, 2H), 6.95 (d, *J =* 8.0 Hz, 1H), 4.24 - 4.21 (m, 1H), 4.10 - 3.96 (m, 3H), 3.75 - 3.69 (m, 3H), 3.24 - 3.22 (m, 1H), 3.16 - 3.05 (m, 4H), 2.97 - 2.94 (m, 1H), 2.72 (t, *J* = 2.6 Hz, 2H), 2.28 - 2.23 (m, 4H).

### Example 309 Synthesis of Compound A310:

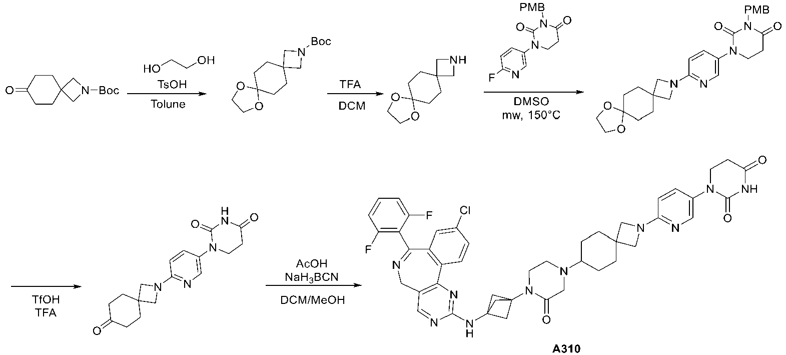

Compound **A310** (18.0 mg, yield 58.2%) was synthesized according to the method of compound A232 described in **Example 231.** LCMS (ESI) m/z = 805.2 [M+H]⁺.¹H NMR (400 MHz, DMSO-*d*₆) δ 10.33 (s, 1H), 8.48 (s, 1H), 8.19 (s, 1H), 8.14 (s, 1H), 8.00 (d, *J* = 2.6 Hz, 1H), 7.83 (d, *J* = 8.1 Hz, 1H), 7.57 - 7.50 (m, 1H), 7.47 (dd, *J*₁ = 8.8 Hz, *J*₂ = 2.6 Hz, 1H), 7.28 (d, *J* = 2.2 Hz, 1H), 7.19 - 7.14 (m, 2H), 6.37 (d, *J* = 8.8 Hz, 1H), 3.69 - 3.65 (m, 2H), 3.65 (s, 2H), 3.59 (s, 2H), 3.25 - 3.24 (m, 2H), 3.07 (s, 2H), 2.71 - 2.67 (m, 4H), 2.42 (s, 6H), 2.33 - 2.25 (m, 1H), 1.93 - 1.90 (m, 2H), 1.73 - 1.70 (m, 2H), 1.52 - 1.45 (m, 2H), 1.33 - 1.26 (m, 2H).

### Example 310 Synthesis of Compound A311:

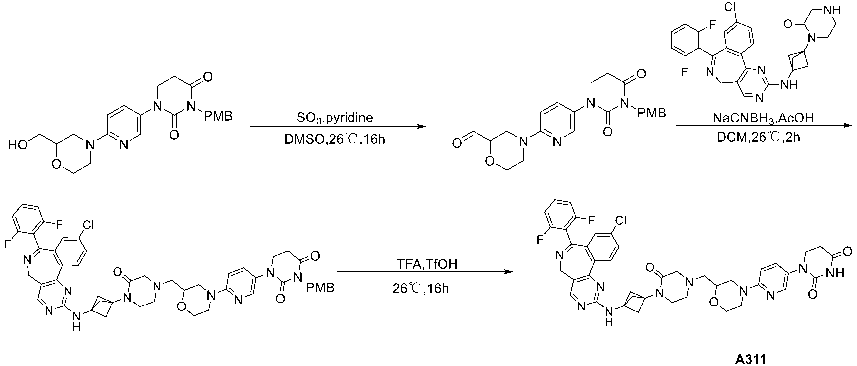

Compound **A311** (15.0 mg, yield 19%) was synthesized according to the method of compound A132 described in **Example 131.** LCMS (ESI) m/z = 807.3 [M+H]⁺. ¹H NMR (400 MHz, DMSO-*d*₆) δ 10.39 (s, 1H), 8.52 (s, 1H), 8.18 (s, 1H), 8.12 (d, *J =* 2.8 Hz, 1H), 7.87 (d, *J =* 8.4 Hz, 1H), 7.60 - 7.55 (m, 2H), 7.31 (d, *J* = 2.4 Hz, 1H), 7.25 - 7.15 (m, 2H), 6.90 (d, *J* = 9.2 Hz, 1H), 4.23 - 4.18 (m, 2H), 4.04 - 3.94 (m, 2H), 3.74 (t, *J=* 6.8 Hz, 2H), 3.71 - 3.65 (m, 2H), 3.64 - 3.58 (m, 1H), 3.11 (s, 2H), 2.93 - 2.68 (m, 8H), 2.46 (s, 6H).

### Example 311 Synthesis of Compound A312:

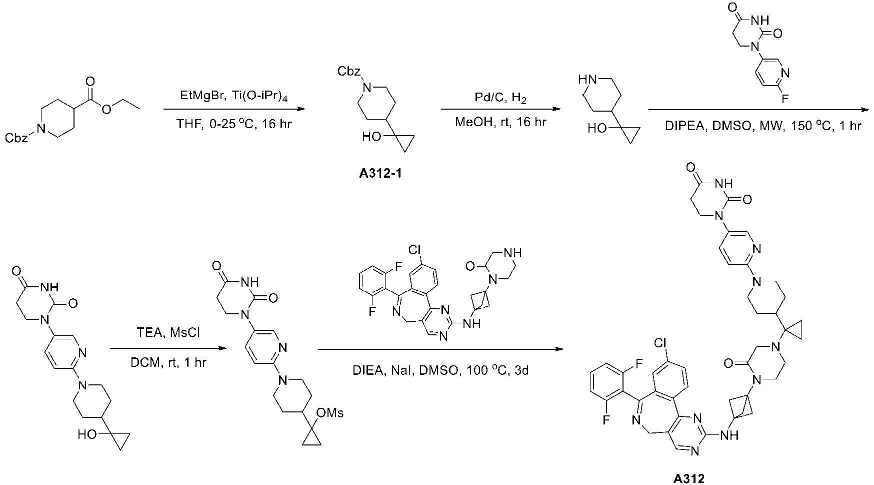

### Step 1:

Ethylmagnesium bromide (1 M, 8.58 mL) was added dropwise to a solution of ethyl N-Cbz-piperidine-4-carboxylate (1 g, 3.43 mmol) in tetrahydrofuran (15 mL) at 0°C, and after the addition was complete, tetraisopropyl titanate (292.66 mg, 1.03 mmol, 306.45 µL) was added dropwise. The reaction mixture was stirred at room temperature for 16 h. After LCMS showed that the reaction was complete, the reaction mixture was quenched with saturated ammonium chloride, extracted with ethyl acetate, and the organic phase was spin-dried and purified by column chromatography to give **A312-1** (300 mg, yield 31.74%). LCMS (ESI) m/z = 276.3 [M+H]⁺.

### Step 2:

Compound **A312** (21 mg, yield 7.3%) was synthesized according to the synthesis method of compound **A132** described in **Example 131.** LCMS (ESI) m/z = 833.4 [M+H]⁺. ¹H NMR (400 MHz, DMSO-*d*₆) δ 10.37 (s, 1H), 8.51 (s, 1H), 8.25 - 8.14 (m, 2H), 8.07 (d, *J* = 2.8 Hz, 1H), 7.88 - 7.82 (m, 1H), 7.61 - 7.53 (m, 1H), 7.50 (dd, *J*₁ = 9.2 Hz, *J*₂ = 2.8 Hz, 1H), 7.31 (d, *J* = 2.0 Hz, 1H), 7.23 - 7.13 (m, 2H), 6.88 (d, *J* = 9.2 Hz, 1H), 5.02 - 4.88 (m, 2H), 4.42 - 4.33 (m, 2H), 3.72 (t, *J* = 6.8 Hz, 2H), 3.34 - 3.27 (m, 2H), 3.02 - 2.92 (m, 4H), 2.82 (t, *J* = 12.4 Hz, 2H), 2.72 (t, *J* = 6.8 Hz, 2H), 2.62 - 2.57 (m, 2H), 2.46 (s, 6H), 2.32 - 2.22 (m, 1H), 1.86 - 1.78 (m, 2H), 1.44 - 1.31 (m, 2H).

### Example 312 Synthesis of Compound A313:

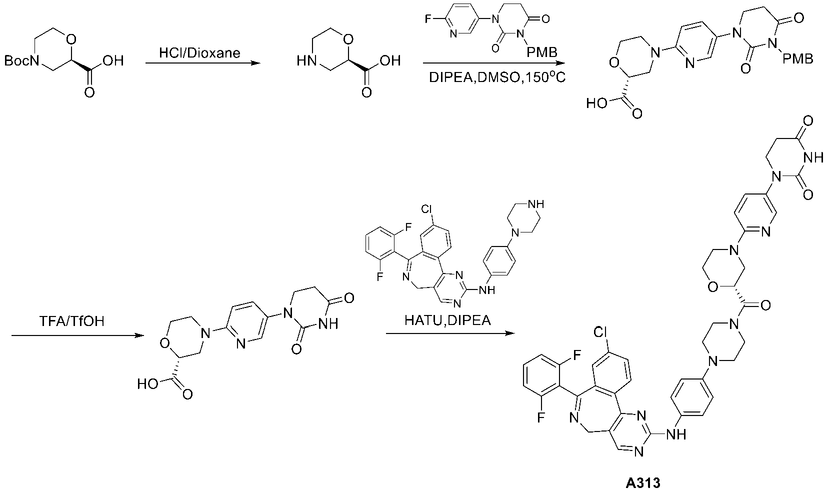

Compound **A313** (33.6 mg, yield 42.4%) was synthesized according to the synthesis method of compound **A54** described in **Example 54.** LCMS (ESI) m/z = 819.1 [M+H]⁺. ¹H NMR (400 MHz, DMSO-*d*₆) δ 10.20 (s, 1H), 9.48 (s, 1H), 8.58 (s, 1H), 8.26 (d, J = 8.0 Hz, 1H), 8.11 (d, *J* = 2.8 Hz, 1H), 7.85 - 7.81 (m, 1H), 7.68 - 7.63 (m, 2H), 7.57 - 7.51 (m, 2H), 7.29 (d, *J* = 2.0 Hz, 1H), 7.19 - 7.09 (m, 2H), 6.99 - 6.93 (m, 2H), 6.90 - 6.87 (m, 1H), 4.42 - 4.36 (m, 1H), 4.20 - 4.15 (m, 1H), 4.05 - 3.95 (m, 2H), 3.78 - 3.59 (m, 8H), 3.15 - 3.08 (m, 4H), 3.05 - 2.96 (m, 1H), 2.71 (t, J = 6.8 Hz, 2H).

### Example 313 Synthesis of Compound A314:

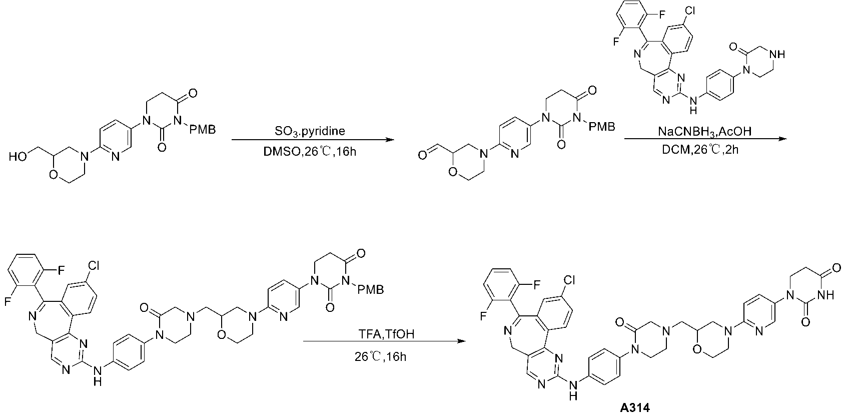

Compound **A314** (15.0 mg, yield 25%) was synthesized according to the synthesis method of compound **A132** described in **Example 131.** LCMS (ESI) m/z = 819.2 [M+H]⁺. ¹H NMR (400 MHz, DMSO-*d*₆) *δ* 10.39 (s, 1H), 9.99 (s, 1H), 8.71 (s, 1H), 8.32 (d, *J* = 8.4 Hz, 1H), 8.14 (d, *J* = 2.8 Hz, 1H), 7.89 (dd, *J*₁ = 8.4 Hz, *J*₂ = 2.4 Hz, 1H), 7.86 - 7.82 (m, 2H), 7.63 - 7.52 (m, 2H), 7.36 (d, *J* = 2.4 Hz, 1H), 7.35 - 7.30 (m, 2H), 7.25 - 7.16 (m, 2H), 6.92 (d, *J* = 9.2 Hz, 1H), 4.22 (d, *J* = 12.8 Hz, 2H), 4.02 (t, *J =* 13.2 Hz, 2H), 3.75 - 3.72 (m, 2H), 3.71 - 3.65 (m, 2H), 3.64 - 3.55 (m, 1H), 3.35 - 3.30 (m, 2H), 2.95 - 2.85 (m, 4H), 2.74 (t, *J* = 6.8 Hz, 2H), 2.65 - 2.61 (m, 2H).

### Example 314 Synthesis of Compound A315:

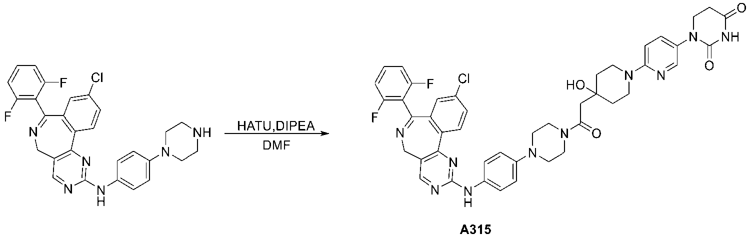

Compound **A315** (16.3 mg, yield 19.1%) was synthesized according to the synthesis method of compound **A1** described in **Example 1.** LCMS (ESI) m/z = 847.2 [M+H]⁺. ¹H NMR (400 MHz, DMSO-*d*₆) δ 10.33 (s, 1H), 9.63 (s, 1H), 8.59 (s, 1H), 8.25 (d, *J* = 8.4 Hz, 1H), 8.03 (d, *J* = 2.8 Hz, 1H), 7.85 (dd, *J*₁ = 8.4 Hz, *J*₂ = 2.0 Hz, 1H), 7.65 (d, *J* = 8.8 Hz, 2H), 7.60 - 7.49 (m, 1H), 7.46 (dd, *J*₁ = 8.8 Hz, *J*₂ = 2.8 Hz Hz, 1H), 7.31 (d, *J* = 2.0 Hz, 1H), 7.16 (t, *J* = 8.8 Hz, 2H), 6.95 (d, *J* = 8.8 Hz, 2H), 6.85 (d, *J* = 9.2 Hz, 1H), 5.02 (s, 1H), 3.93 (d, *J* = 12.8 Hz, 2H), 3.72 - 3.63 (m, 6H), 3.30 - 3.20 (m, 2H), 3.11 - 3.02 (m, 4H), 2.69 (t, *J* = 6.8 Hz, 2H), 2.56 (s, 2H), 1.64 - 1.59 (m, 4H).

### Example 315 Synthesis of Compound A316:

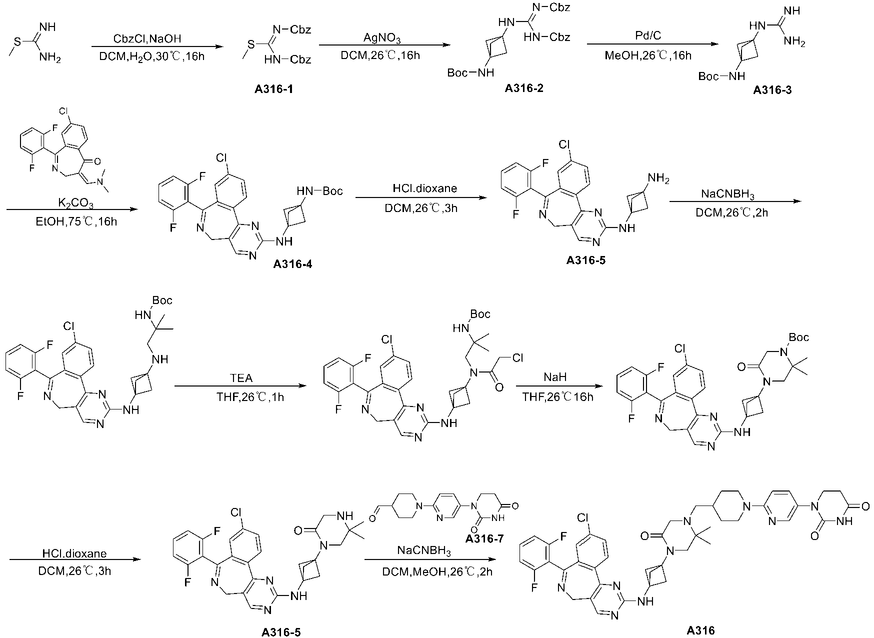

### Step 1:

At room temperature, benzyl chloroformate (24.60 g, 144.21 mmol) and aqueous sodium hydroxide solution (5 M, 30 mL) were added to a solution of compound S-methylisothiourea sulfate (10.00 g, 36.05 mmol) in dichloromethane (150 mL). Then the mixture was stirred at 30°C for 16 h. The reaction mixture was washed with water, extracted with ethyl acetate, and dried over anhydrous sodium sulfate. The residue was purified by silica gel column chromatography to give a white solid **A316-1** (12.00 g, yield 92%). LCMS (ESI) m/z = 359.1 [M+H]⁺.

### Step 2:

To a solution of compound **A316-1** (3.25 g, 9.08 mmol) in dichloromethane (20 mL) at room temperature were added tert-butyl N-{3-aminobicyclo[1.1.1]pentane-1-yl}carbamate (1.50 g, 7.57 mmol), silver nitrate (642.6 mg, 3.78 mmol) and triethylamine (1.53 g, 15.13 mmol), and then the mixture was stirred at 26°C for 16 h. The reaction mixture was washed with water, extracted with ethyl acetate, and dried over anhydrous sodium sulfate. The residue was purified by silica gel column chromatography to give a white solid **A316-2** (3.00 g, yield 77%). LCMS (ESI) m/z = 509.3 [M+H]⁺.

### Step 3:

10% Pd/C (260 mg, 2.46 mmol) was added to a solution of compound **A316-2** (2.50 g, 4.92 mmol) in methanol (25 mL) at room temperature. The reaction mixture was stirred at 26°C for 16 h. After the reaction was completed, the mixture was directly filtered and concentrated to give a white solid **A316-3** (1.00 g, yield 84%). LCMS (ESI) m/z =241.2 [M+H]⁺.

### Step 4:

At room temperature, **SM1** (800.0 mg, 2.22 mmol) and potassium carbonate (612.9 mg, 4.43 mmol) were added to a solution of compound **A316-3** (639.4 mg, 2.66 mmol) in ethanol (5 mL), and the mixture was stirred at 75°C for 16 h. After the reaction was completed, the mixture was directly spin-dried, washed with water, and then extracted with ethyl acetate. The organic phase was washed with saturated brine, and dried over anhydrous sodium sulfate. After concentration under reduced pressure, the residue was purified by silica gel column chromatography (methanol/dichloromethane = 1:20) to give a yellow oil A316-4 (1.00 g, yield 83%). LCMS (ESI) m/z = 538.2 [M+H]⁺.

### Step 5:

At room temperature, **A316-4** (800.0 mg, 1.49 mmol) was dissolved in dichloromethane (10 mL), then a solution of hydrogen chloride in dioxane (5 mL, 4 M) was added, and the mixture was stirred at 26°C for 3 h. After the reaction was completed, the mixture was washed with saturated aqueous sodium bicarbonate solution, extracted with ethyl acetate, and the organic phase was washed with saturated brine, dried over anhydrous sodium sulfate, and directly spin-dried to give **A316-5** (600.0 mg, yield 92%). LCMS (ESI) m/z = 438.1 [M+H]⁺.

### Step 6:

At room temperature, **A316-7** (44.0 mg, 145.72 µmol), acetic acid (2.15 mg, 36.43 µmol) and sodium cyanoborohydride (13.7 mg, 218.57 µmol) were added to a mixed solution of compound **A316-5** (40.0 mg, 72.86 µmol, synthesized with reference to **Example 9)** in dichloromethane (5 mL) and methanol (1 mL). The reaction mixture was stirred at 26°C for 2 h. The reaction mixture was concentrated, and the residue was separated for purification by silica gel column chromatography to give a white solid **A316** (7.0 mg, yield 11%). LCMS (ESI) m/z = 835.3 [M+H]⁺. ¹H NMR (400 MHz, DMSO-*d*₆) δ 10.35 (s, 1H), 8.51 (s, 1H), 8.17 (s, 1H), 8.06 (d, *J* = 2.8 Hz, 1H), 7.87 (d, *J* = 8.4 Hz, 1H), 7.60 - 7.55 (m, 1H), 7.52 - 7.45 (m, 1H), 7.31 (d, *J* = 2.4 Hz, 1H), 7.23 - 7.15 (m, 2H), 6.85 (d, *J* = 9.2 Hz, 1H), 4.29 (d, *J* = 12.8 Hz, 2H), 3.72 (t, *J =* 6.8 Hz, 2H), 3.15 - 3.05 (m, 4H), 2.82 (t, *J =* 12.4 Hz, 2H), 2.75 - 2.68 (m, 4H), 2.44 (s, 6H), 2.25 - 2.20 (m, 2H), 1.85 - 1.79 (m, 2H), 1.74 - 1.67 (m, 1H), 1.04 (s, 6H).

### Example 316 Synthesis of Compound A317:

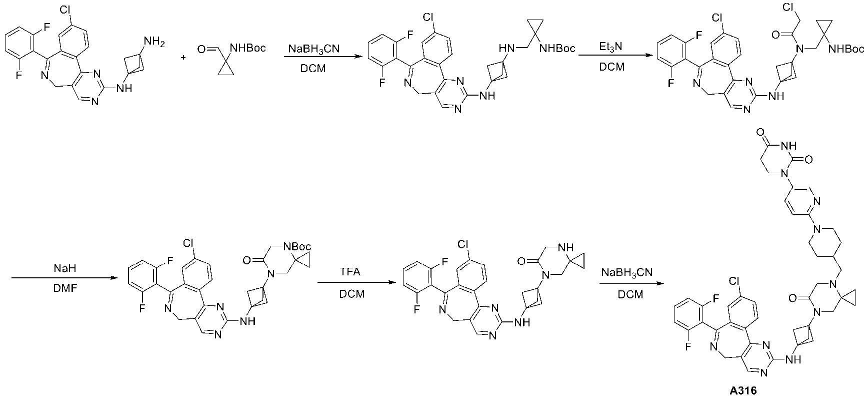

Compound **A317** (14.0 mg, yield 21%) was synthesized according to the synthesis method of compound **A316** described in **Example 315.** LCMS (ESI) m/z = 833.3 [M+H]⁺. ¹H NMR (400 MHz, DMSO-*d*₆) δ 10.35 (s, 1H), 8.51 (s, 1H), 8.20 - 8.16 (m,2H), 8.05 (d, J = 6.4 Hz, 1H), 7.87 (d, *J= 8.4* Hz, 1H), 7.60 - 7.55 (m, 1H), 7.52 - 7.45 (m, 1H), 7.31 (d, *J* = 2.4 Hz, 1H), 7.23 - 7.17 (m, 2H), 6.85 (d, *J* = 9.2 Hz, 1H), 4.27 (d, *J* = 12.8 Hz, 2H), 3.72 (t, *J =* 6.8 Hz, 2H), 3.28 - 3.25 (m, 3H), 2.80 (t, *J* = 12.4 Hz, 2H), 2.72 (t, *J* = 6.8 Hz, 2H), 2.44 (s, 6H), 1.74 - 1.64 (m, 3H), 1.08 - 1.01 (m, 2H), 0.65 - 0.63 (m, 4H).

### Example 317 Synthesis of Compound A318:

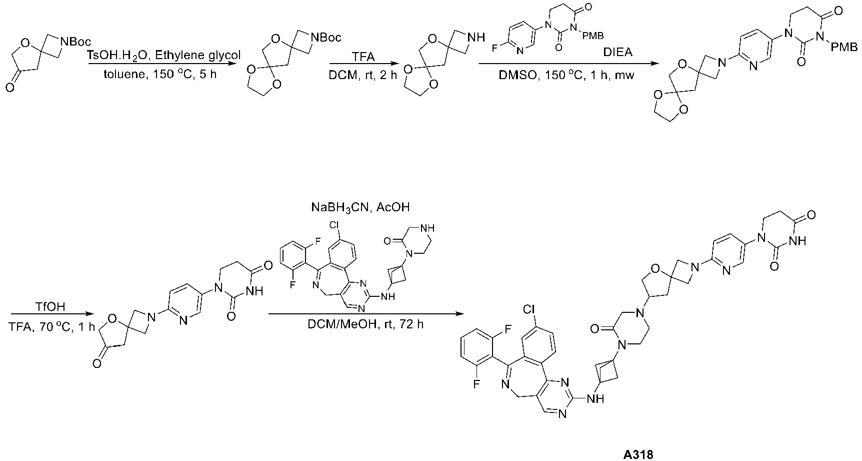

Compound **A318** (17 mg, yield 13%, white solid) was synthesized according to the synthesis method of compound **A132** described in **Example 131.** LCMS (ESI) m/z = 821.3 [M+H]⁺. ¹H NMR (400 MHz, DMSO-*d*₆) δ 10.38 (s, 1H), 8.51 (s, 1H), 8.35 - 8.11 (m, 2H), 8.05 (d, *J* = 2.8 Hz, 1H), 7.86 (d, *J* = 8.8 Hz, 1H), 7.65 - 7.46 (m, 2H), 7.31 (d, *J* = 2.0 Hz, 1H), 7.19 (t, *J* = 8.8 Hz, 2H), 6.46 (d, *J* = 8.8 Hz, 1H), 4.08 (d, *J =* 8.8 Hz, 1H), 4.03 - 3.91 (m, 3H), 3.88 (d, *J* = 8.4 Hz, 1H), 3.76 - 3.63 (m, 3H), 3.33 - 3.29 (m, 2H), 3.14 - 2.99 (m, 3H), 2.72 (t, *J =* 6.8 Hz, 3H), 2.67 - 2.56 (m, 1H), 2.45 (s, 6H), 2.43 - 2.33 (m, 1H), 2.20 - 2.09 (m, 1H).

### Example 318 Synthesis of Compound A319:

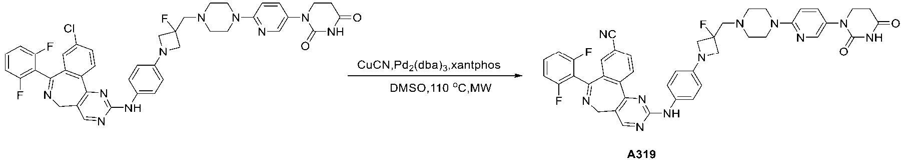

Compound **A319** (21 mg, yield 32%) was synthesized according to the synthesis method of compound **A305** described in **Example 304.** LCMS (ESI) m/z = 784.3 [M+H]⁺. ¹H NMR (400 MHz, DMSO-*d*₆) δ 10.38 (s, 1H), 9.63 (s, 1H), 8.63 (s, 1H), 8.38 (d, J = 8.4 Hz, 1H), 8.22 (dd, *J*₁ = 8.4 Hz, *J*₂ = 2.0 Hz, 1H), 8.09 (d, *J =* 2.8 Hz, 1H), 7.91 (d, *J =* 8.8 Hz, 1H), 7.65 - 7.52 (m, 4H), 7.19 (t, *J =* 8.8 Hz, 2H), 6.87 (d. *J =* 9.2 Hz, 1H), 6.53 (d, *J* = 8.8 Hz, 2H), 4.03 - 3.91 (m, 2H), 3.90 - 3.83 (m, 2H), 3.76 (d, *J =* 6.8 Hz, 2H), 3.52 (bs, 4H), 2.96 (s, 1H), 2.90 (s, 1H), 2.72 (t, *J* = 6.8 Hz, 3H), 2.64 (bs, 4H).

### Example 319 Synthesis of Compound A320:

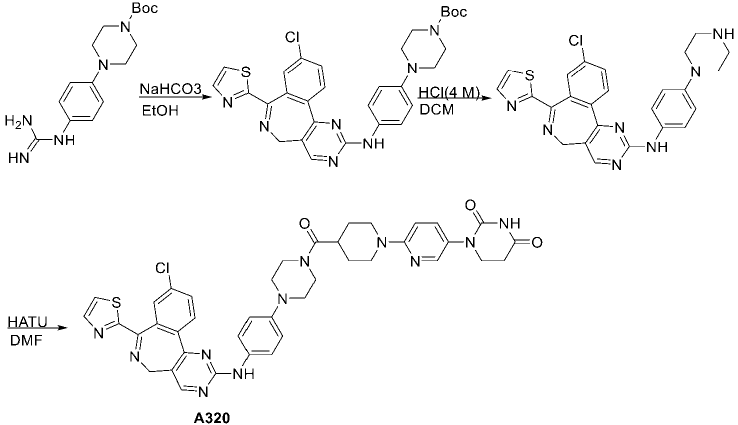

Compound **A320** (49.5 mg, yield 40.8%) was synthesized according to the synthesis method of compound **A140-7** described in **Example 139** and the synthesis method of compound **A1** described in **Example 1.** LCMS (ESI) m/z = 788.5 [M+H]⁺. ¹H NMR (400 MHz, DMSO-*d*₆) δ 10.35 (s, 1H), 9.62 (s, 1H), 8.61 (s, 1H), 8.17 (d, *J* = 8.4 Hz, 1H), 8.05 (d, *J* = 2.8 Hz, 1H), 7.99 (d, *J* = 2.6 Hz, 1H), 7.94 (d, *J* = 3.2 Hz, 1H), 7.92 - 7.85 (m, 2H), 7.64 (d, *J* = 8.8 Hz, 2H), 7.49 (dd, *J* = 9.2, 2.8 Hz, 1H), 6.93 (d, *J* = 9.2 Hz, 2H), 6.86 (d, *J =* 9.2 Hz, 1H), 4.87 - 4.80 (m, 1H), 4.33 - 4.26 (m, 2H), 3.95 - 3.50 (m, 6H), 3.15 - 2.83 (m, 6H), 2.69 (t, *J* = 6.8 Hz, 2H), 1.77 - 1.46 (m, 4H).

### Example 320 Synthesis of Compound A321:

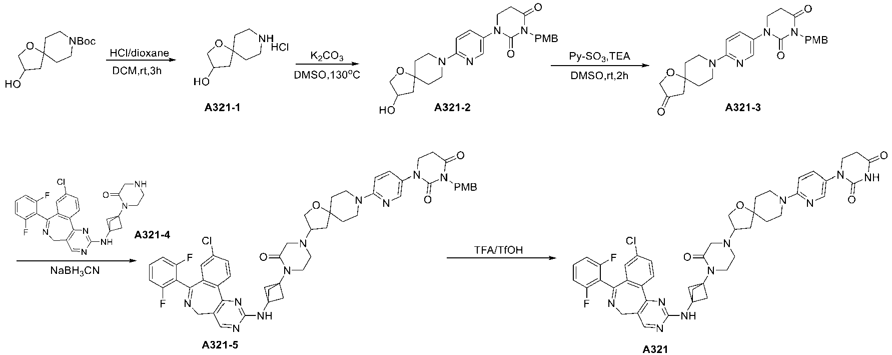

### Step 1:

At room temperature, 4 M hydrochloric acid/dioxane (21.9 mL, 87.5 mmol) was added to a solution of N-tert-butyloxycarbonyl-1-oxa-8-azaspiro[4.5]decane-3-ol (4.5 g, 17.5 mmol) in dichloromethane (50 mL), and the reaction mixture was stirred at room temperature for 3 h. LC-MS showed that the raw materials reacted completely. The reaction mixture was concentrated under reduced pressure to give a white solid A321-1 (2.75 g, yield 100%).

### Step 2:

At room temperature, 1-(6-fluoropyridin-3-yl)-3-(4-methoxybenzyl)dihydropyrimidine-2,4(1H,3H)-dione (2 g, 6.07 mmol), compound **A321-1** (2.55 g, 12.1 mmol), anhydrous potassium carbonate (4.2 g, 3.04 mmol) and anhydrous dimethyl sulfoxide (50 mL) were added to a 100 mL round bottom flask. The mixture was heated in an oil bath at 130°C and reacted for 2 h. LCMS showed a product yield of about 80%. The reaction mixture was washed with water, and extracted with ethyl acetate. The organic phase was separated for purification by a silica gel column to give a light yellow solid **A321-2** (2.22 g, yield 78.4%). LCMS (ESI) m/z = 467.3 [M+H]⁺.

### Step 3:

At room temperature, compound **A321-2** (2.2 g, 4.72 mmol) was dissolved in anhydrous dimethyl sulfoxide (40 mL), pyridine-sulfur trioxide (7.5 g, 47.2 mmol) and triethylamine (9.54 g, 94.3 mmol) were added, and the reaction mixture was stirred at room temperature for 3 h under argon protection. LC-MS showed that the raw materials reacted completely. The reaction mixture was washed with water, and extracted with ethyl acetate. The organic phase was separated for purification by a silica gel column to give a light yellow solid **A321-3** (2 g, yield 91.3%). LCMS (ESI) m/z = 465.2 [M+H]⁺.

### Step 4:

At room temperature, compounds **A321-3** (66.9 mg, 0.14 mmol) and **A321-4** (50 mg, 0.09 mmol) were dissolved in anhydrous dichloromethane (10 mL) and methanol (0.5 mL), acetic acid (6 mg, 0.09 mmol) was added, and the reaction mixture was stirred for half an hour before adding sodium cyanoborohydride (18 mg, 0.27 mmol). The reaction mixture was stirred at room temperature for 4 h. LCMS showed a product yield of about 60%. The reaction mixture was quenched by adding water (10 mL) and extracted with dichloromethane, the organic phase was dried over anhydrous sodium sulfate, filtered and concentrated under reduced pressure, and the crude product was separated by a silica gel column to give a light yellow solid **A321-**5 (42 mg, yield 45.1%). LCMS (ESI) m/z = 969.5 [M+H]⁺.

### Step 5:

At room temperature, compound **A321-5** (42 mg, 0.04 mmol) was dissolved in trifluoroacetic acid (2 mL) and trifluoromethanesulfonic acid (0.5 mL), and the reaction mixture was stirred at room temperature for 2 h. After LCMS showed that the reaction was complete, In an ice bath, saturated sodium bicarbonate solution was added to adjust the pH value to 7-8, and dichloromethane was added for extraction. The organic phase was dried over anhydrous sodium sulfate, filtered, and concentrated under reduced pressure. The crude product was separated for purification by reverse phase chromatography (acetonitrile/10 mM aqueous ammonium bicarbonate solution) to give a yellow solid **A321** (24.6 mg, yield 66.8%). LCMS (ESI) m/z = 849.3 [M+H]⁺. ¹H NMR (400 MHz, DMSO-*d*₆) δ 10.33 (s, 1H), 8.48 (s, 1H), 8.23 - 8.12 (m, 2H), 8.04 (d, *J* = 2.8 Hz, 1H), 7.87 - 7.80 (m, 1H), 7.58 - 7.50 (m, 1H), 7.49 - 7.45 (m, 1H), 7.28 (d, *J=* 2.4 Hz, 1H), 7.16 (t, *J =* 8.8 Hz, 2H), 6.86 (d, *J =* 9.6 Hz, 1H), 3.98 - 3.91 (m, 1H), 3.72 - 3.64 (m, 4H), 3.62 - 3.57 (m, 1H), 3.50 - 3.43 (m, 2H), 3.42 (s, 2H), 3.30 - 3.23 (m, 2H), 3.05 - 2.91 (m, 3H), 2.72 - 2.65 (m, 3H), 2.59 - 2.55 (m, 1H), 2.42 (s, 6H), 2.07 - 1.99 (m, 1H), 1.66 - 1.50 (m, 5H).

### Example 321 Synthesis of Compound A322:

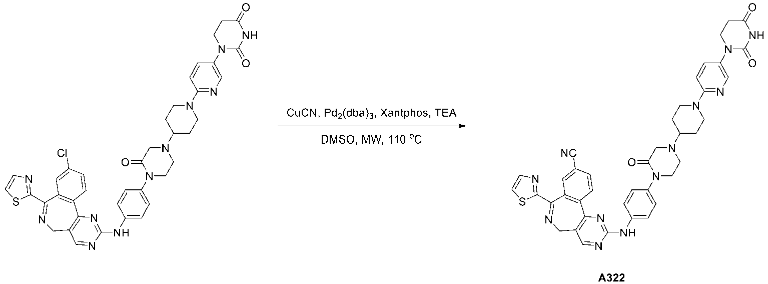

Compound **A322** (6 mg, yield 8.7%) was synthesized according to the synthesis method of compound **A305** described in **Example 304.** LCMS (ESI) m/z = 765.5 [M+H]⁺. ¹H NMR (400 MHz, DMSO-*d*₆) δ 10.38 (s, 1H), 10.02 (s, 1H), 8.76 (s, 1H), 8.47 (d, *J* = 1.6 Hz, 1H), 8.38 - 8.34 (m, 1H), 8.29 - 8.25 (m, 1H), 8.08 (d, *J* = 2.8 Hz, 1H), 7.99 - 7.92 (m, 2H), 7.83 - 7.77 (m, 2H), 7.54 - 7.49 (m, 1H), 7.29 - 7.24 (m, 2H), 6.91 (d, *J* = 9.2 Hz, 1H), 4.96 - 4.88 (m, 1H), 4.38 - 4.28 (m, 2H), 3.96 - 3.90 (m, 1H), 3.73 (t, *J =* 6.8 Hz, 2H), 3.64 - 3.57 (m, 2H), 3.32 - 3.30 (m, 2H), 2.93 - 2.85 (m, 4H), 2.73 (t, *J* = 6.8 Hz, 2H), 2.64 - 2.57 (m, 1H), 1.97 - 1.88 (m, 2H), 1.50 - 1.37 (m, 2H).

### Example 322 Synthesis of Compound A323:

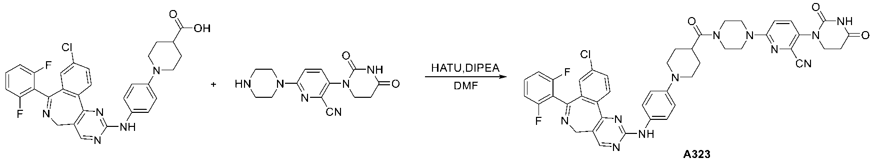

Compound **A323** (20.0 mg, yield 53.1%) was synthesized according to the method of compound **A1** described in **Example 1.** LCMS (ESI) m/z = 842.50 [M+H]⁺. ¹H NMR (400 MHz, DMSO-*d*₆) δ 10.63 (s, 1H), 9.61 (s, 1H), 8.59 (s, 1H), 8.25 (d, *J* = 8.5 Hz, 1H), 7.85 (dd, *J*₁ = 8.5 Hz, *J*₂ = 2.2 Hz, 1H), 7.78 (d, *J* = 9.2 Hz, 1H), 7.62 (d, *J* = 8.8 Hz, 2H), 7.57 - 7.50 (m, 1H), 7.31 - 7.27 (m, 2H), 7.19 - 7.14 (m, 2H), 6.93 (d, *J* = 9.0 Hz, 2H), 3.75 - 3.58 (m, 12H), 2.85 - 2.77 (m, 1H), 2.75 - 2.66 (m, 4H), 1.74 - 1.68 (m, 4H).

### Example 323 Synthesis of Compound A324:

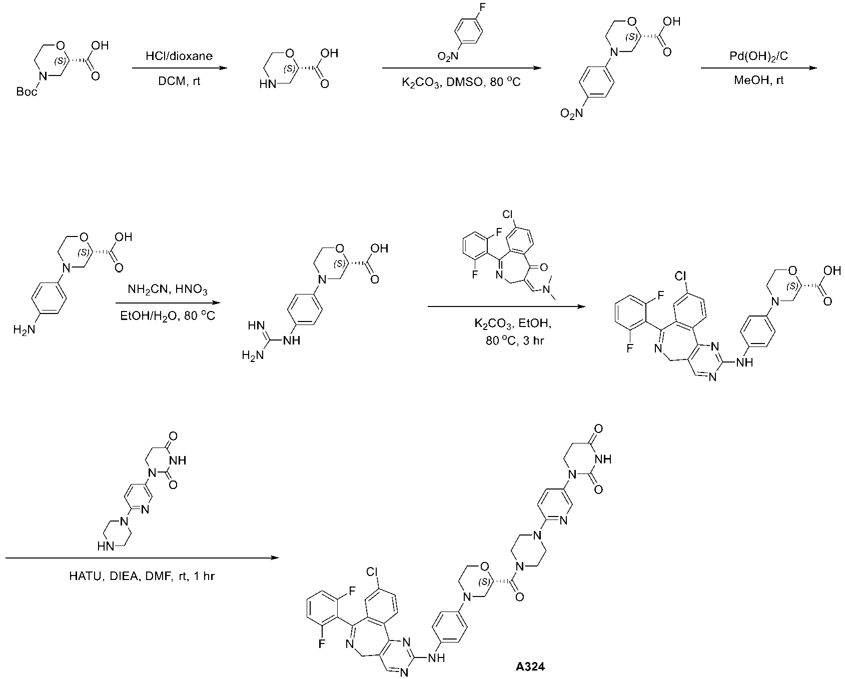

Compound **A324** (21 mg, yield 36%) was synthesized according to the synthesis method of compound **A101** described in **Example 101.** LCMS (ESI) m/z = 819.8 [M+H]⁺. ¹H NMR (400 MHz, DMSO-*d*₆) δ 10.40 (s, 1H), 9.68 (s, 1H), 8.63 (s, 1H), 8.29 (d, *J* = 8.4 Hz, 1H), 8.13 (d, *J* = 2.4 Hz, 1H), 7.90 - 7.86 (m, 1H), 7.73 - 7.67 (m, 2H), 7.61 - 7.52 (m, 2H), 7.34 (d, *J* = 2.0 Hz, 1H), 7.24 - 7.15 (m, 2H), 7.02 - 6.96 (m, 2H), 6.93 (d, *J* = 9.2 Hz, 1H), 4.54 - 4.48 (m, 1H), 4.05 - 3.99 (m, 1H), 3.88 - 3.79 (m, 1H), 3.77 - 3.69 (m, 4H), 3.68 - 3.45 (m, 10H), 2.91 - 2.83 (m, 1H), 2.78 - 2.69 (m, 3H).

### Example 324 Synthesis of Compound A325:

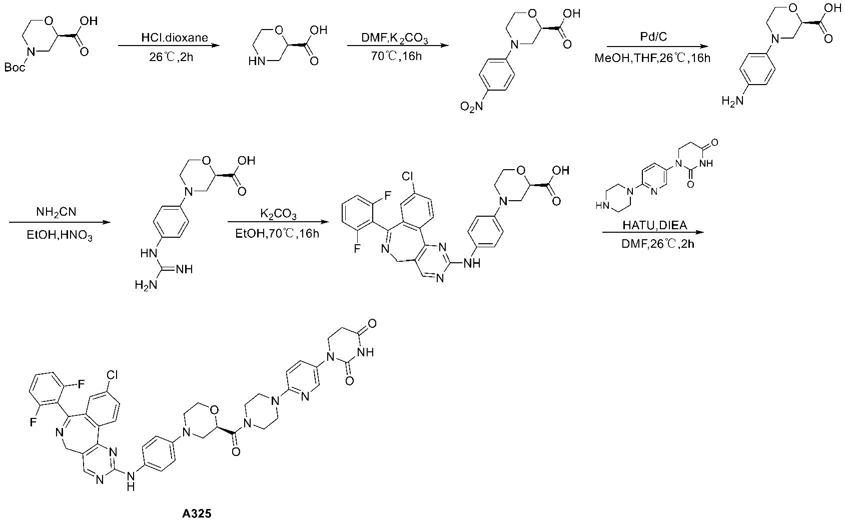

Compound **A325** (5.1 mg, yield 31.9%) was synthesized according to the synthesis method of compound **A101** described in **Example 101.** LCMS (ESI) m/z = 819.2 [M+H]⁺. ¹H NMR (400 MHz, DMSO-*d*₆) δ 10.39 (s, 1H), 9.68 (s, 1H), 8.63 (s, 1H), 8.29 (d, *J* = 8.4 Hz, 1H), 8.13 (d, *J* = 2.8 Hz, 1H), 7.90 - 7.87 (m, 1H), 7.70 (d, *J* = 8.8 Hz, 2H), 7.62 - 7.53 (m, 2H), 7.34 (d, *J* = 2.0 Hz, 1H), 7.20 (t, *J* = 8.4 Hz, 2H), 7.00 (d, *J* = 9.2 Hz, 2H), 6.93 (d, *J =* 9.6 Hz, 1H), 4.55 - 4.47 (m, 2H), 4.08 - 4.00 (m, 2H), 3.88 - 3.80 (m, 2H), 3.78 - 3.73 (m, 4H), 3.65 - 3.58 (m, 4H), 3.55 - 3.48 (m, 1H), 2.90 - 2.83 (m, 2H), 2.75 - 2.70 (m, 2H).

### Example 325 Synthesis of Compound A326:

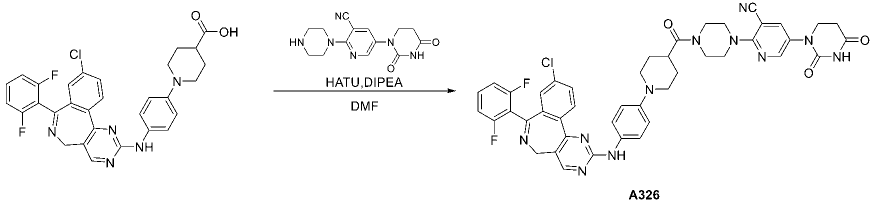

Compound **A326** (8.6 mg, yield 57.1%) was synthesized according to the method of compound **A1** described in **Example 1.** LCMS (ESI) m/z = 842.3 [M+H]⁺. ¹H NMR (400 MHz, DMSO-*d*₆) δ 10.52 (s, 1H), 9.60 (s, 1H), 8.58 (s, 1H), 8.44 (d, *J* = 2.6 Hz, 1H), 8.25 (d, *J =* 8.4 Hz, 1H), 8.14 (d, *J =* 2.8 Hz, 1H), 7.87 - 7.83 (m, 1H), 7.62 (d, *J* = 8.6 Hz, 2H), 7.58 - 7.49 (m, 1H), 7.30 (d, *J* = 2.0 Hz, 1H), 7.17 (t, *J =* 8.8 Hz, 2H), 6.93 (d, *J* = 8.0 Hz, 2H), 3.79 - 3.72 (m, 4H), 3.69 - 3.58 (m, 8H), 2.86 - 2.78 (m, 1H), 2.75 - 2.66 (m, 4H), 1.78 - 1.67 (m, 4H).

### Example 326 Synthesis of Compound A327:

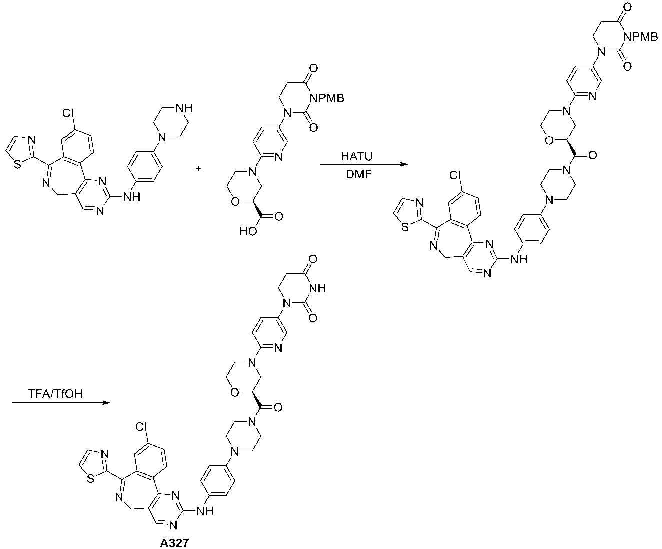

Compound **A327** (5.2 mg, yield 15.2%) was synthesized according to the synthesis method of compound **A101** described in **Example 101.** LCMS (ESI) m/z = 790.3 [M+H]⁺. ¹H NMR (400 MHz, DMSO-*d*₆) δ 10.39 (s, 1H), 9.60 (s, 1H), 8.59 (d, 1H), 8.20 (d, *J* = 8.4 Hz, 1H), 8.13 (*d, J =* 2.4 Hz, 1H), 7.92 (dd, *J =* 8.2, 2.2 Hz, 1H), 7.83 (d, *J* = 2.4 Hz, 1H), 7.72 (d, *J =* 4.8 Hz, 1H), 7.66 (d, *J* = 8.8 Hz, 2H), 7.57 (dd, *J* = 8.8, 2.8 Hz, 1H), 7.10 - 7.08 (m, 1H), 7.01 - 7.00 (m, 1H), 6.97 - 6.93 (m, 3H), 4.72 (d, *J* = 14.8 Hz, 1H), 4.42 (m, 1H), 4.20 (d, *J* = 12.4 Hz, 1H), 4.07 (m, 2H), 3.75 - 3.66 (m, 8H), 3.14 - 2.98 (m, 6H), 2.73 (t, *J* = 7.2 Hz, 2H).

### Example 327 Synthesis of Compound A328:

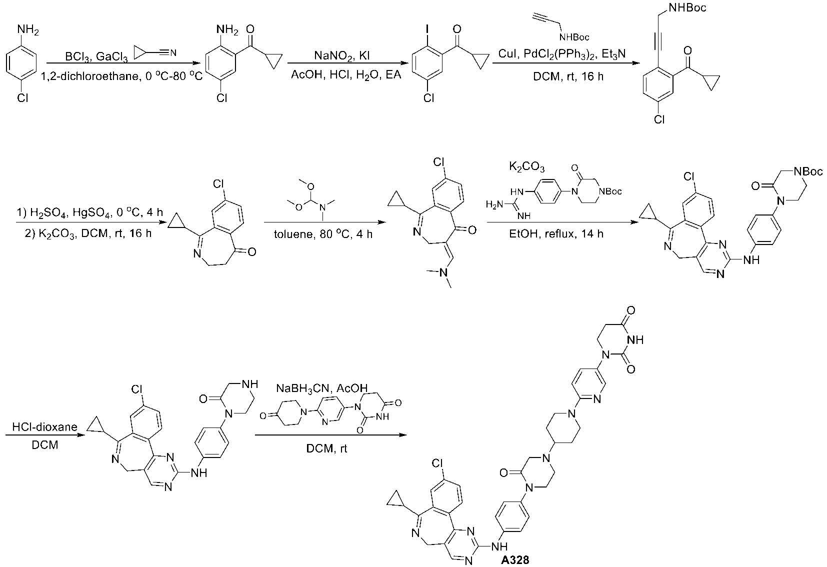

Compound **A328** (10 mg, yield 10%) was synthesized according to the synthesis method of compound **A128** described in **Example 127.** LCMS (ESI) m/z = 731.3 [M+H]⁺. ¹H NMR (400 MHz, DMSO-*d*₆) *δ* 10.38 (s, 1H), 9.88 (s, 1H), 8.59 (s, 1H), 8.19 (d, *J* = 8.4 Hz, 1H), 8.09 (d, *J =* 2.8 Hz, 1H), 8.03 (d, *J* = 2.0 Hz, 1H), 7.88 - 7.76 (m, 3H), 7.57 - 7.48 (m, 1H), 7.30 - 7.20 (m, 2H), 6.91 (d, *J =* 9.2 Hz, 1H), 4.57 (d, *J =* 12.0 Hz, 1H), 4.34 (d, *J* = 12.4 Hz, 2H), 3.73 (t, *J* = 6.4 Hz, 2H), 3.67 - 3.58 (m, 2H), 3.53 (d, *J* = 11.6 Hz, 1H), 3.33 - 3.28 (m, 2H), 2.96 - 2.82 (m, 4H), 2.73 (t, *J =* 6.8 Hz, 2H), 2.65 - 2.56 (m, 1H), 2.02 - 1.88 (m, 3H), 1.54 - 1.37 (m, 2H), 1.14 - 1.03 (m, 1H), 1.02 - 0.84 (m, 2H), 0.80 - 0.64 (s, 1H).

### Example 328 Synthesis of Compound A329:

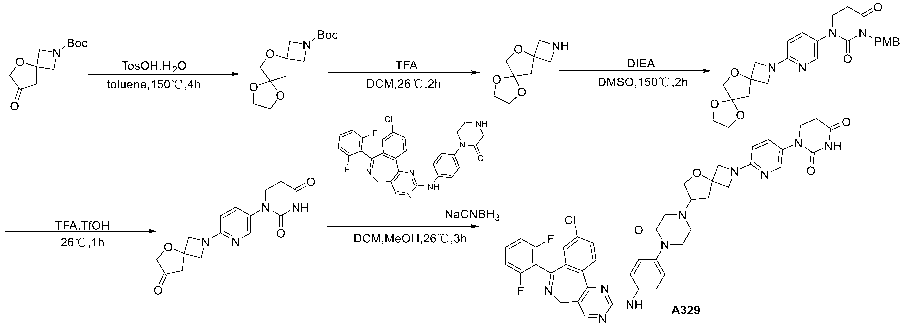

Compound **A329** (15.0 mg, yield 48%) was synthesized according to the synthesis method of compound **A132** described in **Example 131.** LCMS (ESI) m/z = 831.3 [M+H]⁺. ¹H NMR (400 MHz, DMSO-*d*₆) *δ* 10.38 (s, 1H), 10.00 (s, 1H), 8.71 (s, 1H), 8.32 (d, *J* = 8.4 Hz, 1H), 8.06 (d, *J* = 2.8 Hz, 1H), 7.90 (dd, *J* = 8.5, 2.0 Hz, 1H), 7.85 (d, *J =* 8.8 Hz, 2H), 7.62 - 7.56 (m, 1H), 7.54 (dd, *J* = 8.8, 2.4 Hz, 1H), 7.36 - 7.30 (m, 2H), 7.30 (d, *J* = 6.8 Hz, 1H), 7.20 (t, *J* = 8.8 Hz, 2H), 6.49 - 6.45(m, 1H), 4.15 - 4.10 (m, 1H), 4.05 - 3.95 (m, 4H), 3.94 - 3.90 (m, 1H), 3.80 - 3.75 (m, 1H), 3.75 - 3.70 (m, 2H), 3.66 (t, *J* = 5.2 Hz, 2H), 3.24 (d, *J=* 6.8 Hz, 2H), 3.21 - 3.16 (m, 1H), 2.93 - 2.86 (m, 1H), 2.83 - 2.76 (m, 1H), 2.73 (t, *J* = 6.8 Hz, 2H), 2.48 - 2.42 (m, 1H), 2.25 - 2.18 (m, 1H).

### Example 329 Synthesis of Compound A330:

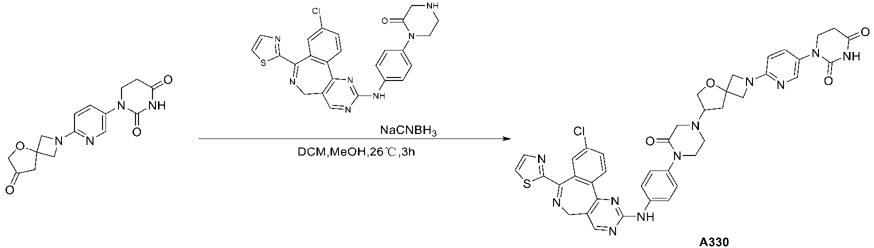

Compound **A330** (15.0 mg, yield 48%) was synthesized according to the synthesis method of compound **A2** described in **Example A2.** LCMS (ESI) m/z = 802.3 [M+H]⁺. ¹H NMR (400 MHz, DMSO-*d*₆) δ 10.37 (s, 1H), 9.96 (s, 1H), 8.71 (s, 1H), 8.23 (d, *J* = 8.5 Hz, 1H), 8.04 (dd, *J* = 10.4, 2.4 Hz, 2H), 7.97 (d, *J* = 3.2 Hz, 1H), 7.95 - 7.90 (m, 2H), 7.82 (d, *J* = 8.8 Hz, 2H), 7.53 (dd, *J* = 8.8, 2.8 Hz, 1H), 7.27 (d, *J* = 8.8 Hz, 2H), 6.47 (d, *J =* 8.8 Hz, 1H), 4.95 - 4.90 (m, 1H), 4.11 (d, *J =* 8.8 Hz, 1H), 4.05 - 3.98 (m, 2H), 3.94 - 3.88 (m, 2H), 3.79 - 3.75 (m, 1H), 3.74 - 3.70 (m, 2H), 3.64 - 3.62 (m, 2H), 3.24 - 3.18 (m, 2H), 2.93 - 2.84 (m, 1H), 2.70 - 2.76 (m, 1H), 2.71 (t, *J* = 6.8 Hz, 2H), 2.55 - 2.41 (m, 1H), 2.24 - 2.16 (m, 1H).

### Example 330 Synthesis of Compound A331:

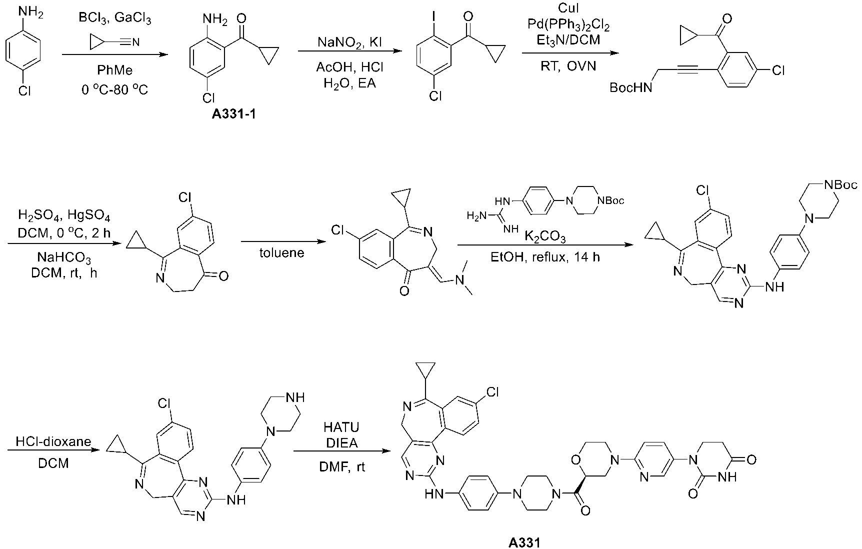

### Step 1:

**A331-1** (2.04 g, yield 63.5%) was synthesized with reference to Tetrahedron Letters (1994), 35(37), 6811-14. LCMS (ESI) m/z = 195.1 [M+H]⁺.

### Step 7:

Compound **A331** (20 mg, yield 62.7%) was synthesized according to the synthesis method of compound **A88** described in **Example 88.** LCMS (ESI) m/z = 747.49 [M+H]⁺. ¹H NMR (400 MHz, DMSO-*d*₆) *δ* 10.36 (s, 1H), 9.54 (s, 1H), 8.49 (s, 1H), 8.18 - 8.08 (m, 2H), 7.99 (d, *J* = 2.2 Hz, 1H), 7.80 (dd, *J*₁ = 8.5 Hz, *J*₂ = 2.2 Hz, 1H), 7.64 (d, *J* = 8.8 Hz, 2H), 7.55 (dd, *J*₁ = 9.0 Hz, *J*₂ = 2.7 Hz, 1H), 6.94 - 6.90 (m, 3H), 4.51 (d, *J* = 12.0 Hz, 1H), 4.38 (dd, *J*₁ = 9.9 Hz, *J*₂ = 2.8 Hz, 1H), 4.18 - 4.15 (m, 1H), 4.05 - 3.95 (m, 2H), 3.73 - 3.62 (m, 7H), 3.50 - 3.47 (m, 1H), 3.08 - 3.03 (m, 3H), 3.00 - 2.91 (m, 1H), 2.70 (t, *J* = 6.7 Hz, 2H), 2.03 - 1.90 (m, 3H), 1.49 - 1.42 (m, 1H), 1.07 - 1.01 (m, 1H), 0.94 - 0.90 (s, 1H), 0.71 - 0.65 (s, 1H).

### Example 331 Synthesis of Compound A332:

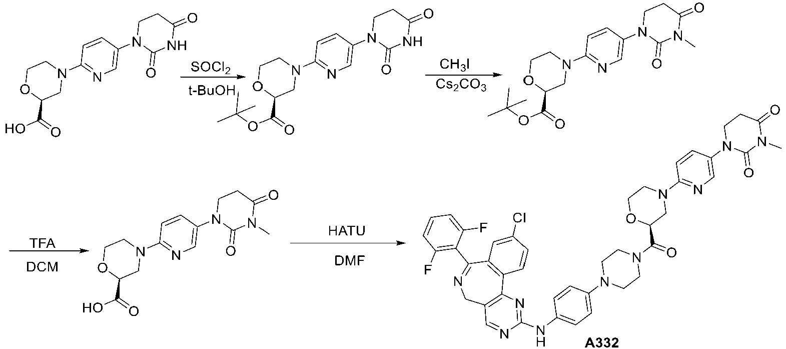

Compound **A332** (54.1 mg, yield 50.5%) was synthesized according to the synthesis method of compound **A82-2** described in **Example 82.** LCMS (ESI) m/z = 833.52 [M+H]⁺. ¹H NMR (400 MHz, DMSO-*d*₆) δ 9.65 (s, 1H), 8.60 (s, 1H), 8.25 (d, *J* = 8.4 Hz, 1H), 8.11 (d, *J* = 2.8 Hz, 1H), 7.85 (dd, *J* = 8.4, 2.0 Hz, 1H), 7.66 (d, *J* = 8.8 Hz, 2H), 7.60 - 7.48 (m, 2H), 7.31 (d, *J* = 2.0 Hz, 1H), 7.21 - 7.12 (m, 2H), 7.01 - 6.89 (m, 3H), 4.43 - 4.35 (m, 1H), 4.18 (d, *J* = 13.2 Hz, 1H), 4.07 - 3.93 (m, 2H), 3.77 - 3.59 (m, 7H), 3.20 - 2.90 (m, 9H), 2.83 (t, *J* = 6.8 Hz, 2H).

### Example 332 Synthesis of Compound A333:

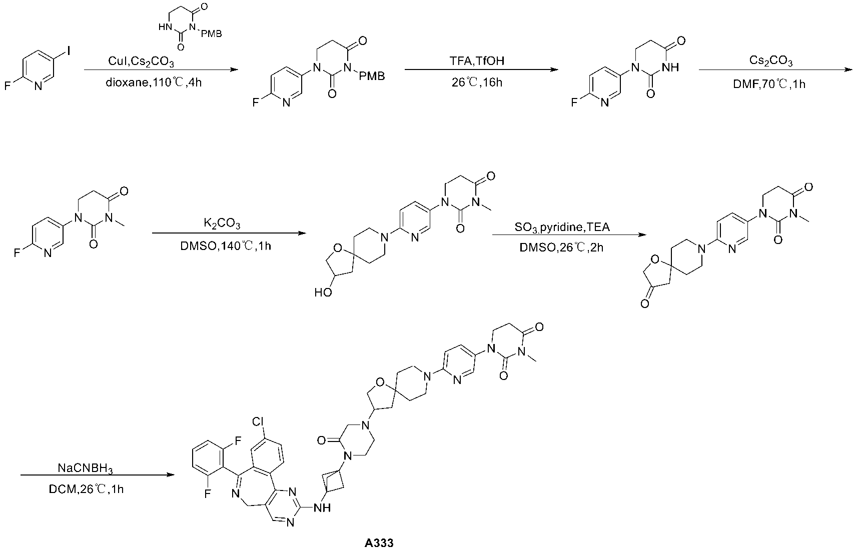

Compound **A333** (3 mg, yield 20.6%) was synthesized according to the synthesis method of compound **A132** described in **Example 131.** LCMS (ESI) m/z = 863.2 [M+H]⁺.¹H NMR (400 MHz, DMSO-*d*₆) δ 8.52 (s, 1H), 8.18 (s, 1H), 8.08 (d, *J* = 2.8 Hz, 1H), 7.86 (dd, *J* = 8.4, 2.0 Hz, 1H), 7.62 - 7.55 (m, 1H), 7.51 (dd, *J* = 9.2, 2.8 Hz, 1H), 7.31 (d, *J* = 2.0 Hz, 1H), 7.20 (t, *J* = 8.8 Hz, 2H), 6.90 (d, *J = 9.2* Hz, 1H), 4.03 - 3.95 (m, 1H), 3.71 (t, *J* = 6.8 Hz, 4H), 3.63 (t, *J* = 8.4 Hz, 1H), 3.55 - 3.43 (m, 2H), 3.35 - 3.25 (m, 2H), 3.05 - 3.00 (m, 2H), 2.98 - 2.90 (m, 1H), 2.85 (t, *J* = 6.8 Hz, 2H), 2.73 - 2.67 (m, 1H), 2.65 - 2.68 (m, 1H), 2.45 (s, 3H), 2.10 - 2.05 (m, 1H), 1.71 - 1.65 (m, 2H), 1.63 - 1.58 (m, 2H).

### Example 333 Synthesis of Compound A334:

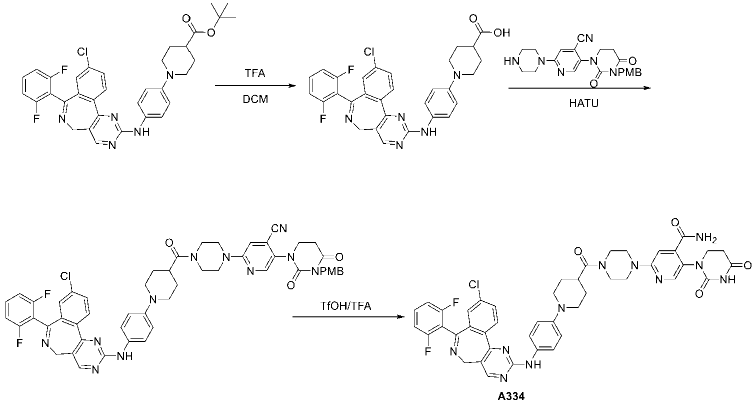

Compound **A334** (2.1 mg, yield 31.9%) was synthesized according to the synthesis method of compound **A101** described in **Example 101.** LCMS (ESI) m/z = 860.2 [M+H]⁺. ¹H NMR (400 MHz, DMSO-*d*₆) *δ* 10.31 (s, 1H), 9.62 (s, 1H), 8.61 (d, 1H), 8.28 (d, *J* = 8.4 Hz, 1H), 8.13 (s, 1H), 7.19 (s, 1H), 7.88 (dd*, J* = 8.4, 2.0 Hz, 1H), 7.65 (d, *J* = 9.2 Hz, 2H), 7.61 - 7.54 (m, 2H), 7.33 (d, *J* = 2.4 Hz, 1H), 7.19 (t, *J* = 8.4 Hz, 2H), 6.97 - 6.94 (m, 3H), 3.70 - 3.58 (m, 11H), 2.85 - 2.83 (m, 1H), 2.77 - 2.68 (m, 4H), 1.78 - 1.72 (m, 4H).

### Example 334 Synthesis of Compound A335:

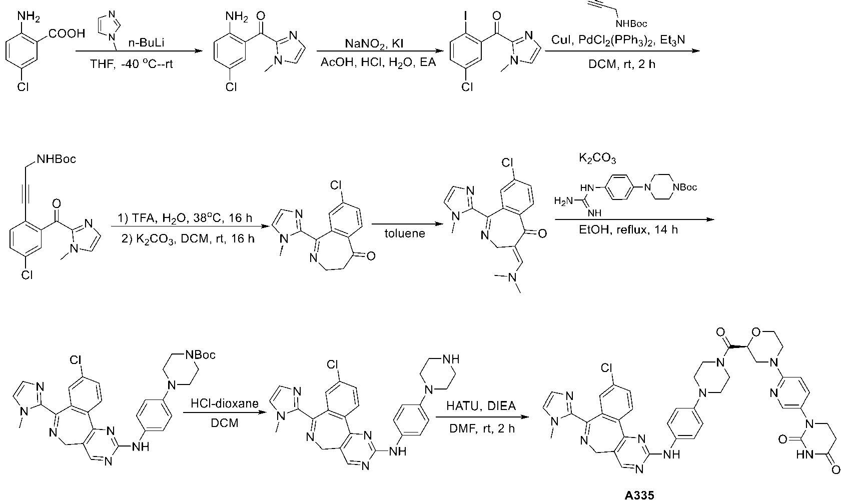

Compound **A335** (24 mg, yield 49%) was synthesized according to the synthesis method of compound **A128** described in **Example 127.** LCMS (ESI) m/z = 787.1 [M+H]⁺. ¹H NMR (400 MHz, DMSO-*d*₆) δ 10.39 (s, 1H), 9.62 (s, 1H), 8.61 (s, 1H), 8.19 - 8.09 (m, 2H), 7.84 (dd, *J* = 8.4, 2.0 Hz, 1H), 7.72 - 7.64 (m, 3H), 7.57 (dd, *J* = 9.2, 2.8 Hz, 1H), 7.36 (s, 1H), 7.00 - 6.89 (m, 4H), 4.85 (d, *J* = 11.6 Hz, 1H), 4.41 (dd, *J=* 10.0, 2.8 Hz, 1H), 4.20 (d, *J* = 13.2 Hz, 1H), 4.06 (d, *J* = 12.6 Hz, 1H), 3.99 (d, *J* = 11.2 Hz, 1H), 3.95 (s, 3H), 3.83 - 3.69 (m, 6H), 3.69 - 3.60 (m, 2H), 3.22 - 2.92 (m, 6H), 2.73 (t, *J* = 6.4 Hz, 2H).

### Example 335 Synthesis of Compound A336:

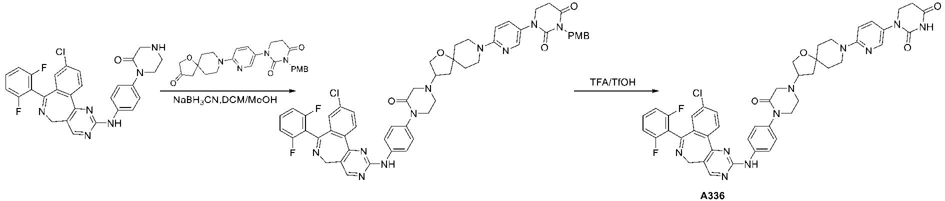

Compound **A336** (25.0 mg, yield 40.7%) was synthesized according to the synthesis method of compound **A321** described in **Example 320.** LCMS (ESI) m/z = 859.6 [M+H]⁺. ¹H NMR (400 MHz, DMSO-*d*₆) δ 10.34 (s, 1H), 9.96 (s, 1H), 8.67 (s, 1H), 8.28 (d, *J* = 8.4 Hz, 1H), 8.05 (d, *J* = 2.4 Hz, 1H), 7.88 - 7.78 (m, 3H), 7.58 - 7.46 (m, 2H), 7.32 (d, *J* = 2.2 Hz, 1H), 7.28 - 7.24 (m, 2H), 7.17 (t, *J* = 8.8 Hz, 2H), 6.87 (d, *J* = 10.0 Hz, 1H), 4.03 - 3.96 (m, 1H), 3.73 - 3.60 (m, 7H), 3.52 - 3.41 (m, 2H), 3.25 - 3.08 (m, 3H), 2.86 - 2.78 (m, 1H), 2.77 - 2.65 (m, 3H), 2.12 - 2.04 (m, 1H), 1.71 - 1.52 (m, 5H).

### Example 336 Synthesis of Compound A337:

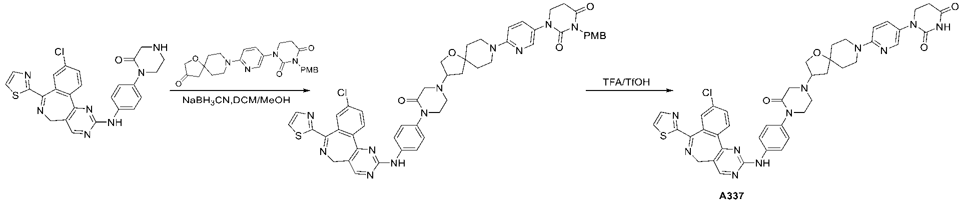

Compound **A337** (12.1 mg, yield 45.8%) was synthesized according to the synthesis method of compound **A321** described in **Example 320.** LCMS (ESI) m/z = 830.4 [M+H]⁺. ¹H NMR (400 MHz, DMSO-*d*₆) δ 10.34 (s, 1H), 9.93 (s, 1H), 8.68 (s, 1H), 8.20 (d, *J* = 8.4 Hz, 1H), 8.04 (d, *J* = 2.4 Hz, 1H), 8.00 (d, *J* = 2.0 Hz, 1H), 7.96 - 7.93 (m, 1H), 7.92 - 7.88 (m, 2H), 7.81 - 7.77 (m, 2H), 7.50 - 7.45 (m, 1H), 7.26 - 7.20 (m, 2H), 6.87 (d, *J* = 8.0 Hz, 1H), 4.87 (d, *J* = 12.0 Hz, 1H), 4.02 - 3.96 (m, 1H), 3.92 - 3.85 (m, 1H), 3.72 - 3.58 (m, 7H), 3.53 - 3.41 (m, 2H), 3.23 - 3.07 (m, 3H), 2.85 - 2.77 (m, 1H), 2.75 - 2.66 (m, 3H), 2.11 - 2.03 (m, 1H), 1.72 - 1.52 (m, 5H).

### Example 337 Synthesis of Compound A338:

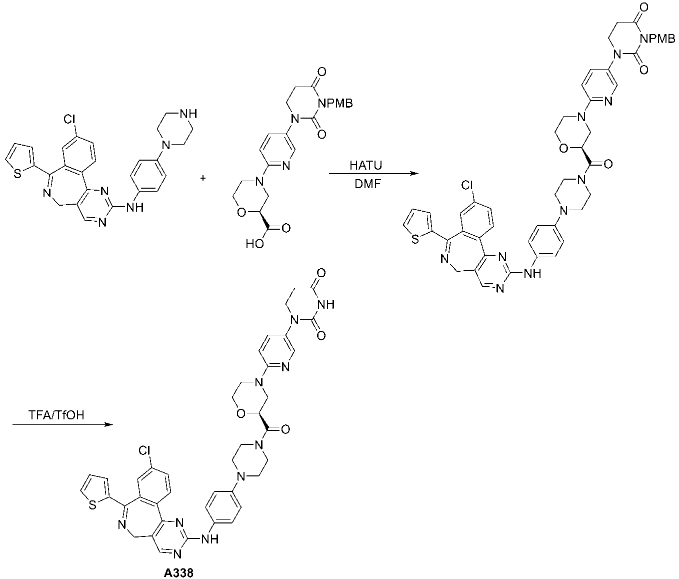

Compound **A338** (5.2 mg, yield 15.2%) was synthesized according to the synthesis method of compound **A101** described in **Example 101.** LCMS (ESI) m/z = 789.2 [M+H]⁺. ¹H NMR (400 MHz, DMSO-*d*₆) δ 10.32 (s, 1H), 9.62 (s, 1H), 8.61 (d, 1H), 8.28 (d, *J* = 8.4 Hz, 1H), 8.13 (d, *J* = 2.4 Hz, 1H), 7.91 (dd, *J =* 8.2, 2.2 Hz, 1H), 7.87 (d, *J =* 2.4 Hz, 1H), 7.65 (d, *J =* 4.8 Hz, 1H), 7.61 - 7.54 (m, 2H), 7.33 (d, *J =* 2.4 Hz, 1H), 7.19 (t, *J* = 8.4 Hz, 2H), 6.97 - 6.94 (m, 3H), 3.71 -3.58 (m, 11H), 2.91 -2.81 (m, 1H), 2.82 - 2.68 (m, 4H), 1.80 - 1.72 (m, 4H).

### Example 338 Synthesis of Compound A339:

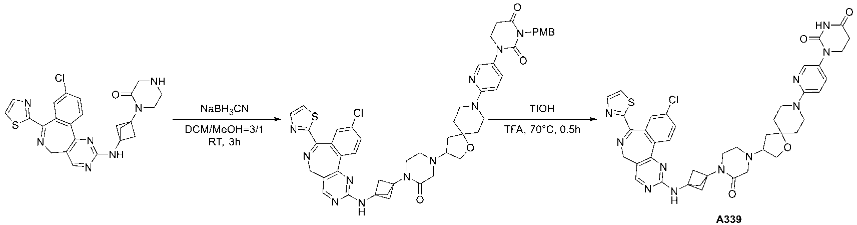

Compound **A354** (10.0 mg, yield 57.3%) was synthesized according to the method of compound **A132** described in **Example 131.** LCMS (ESI) m/z = 820.53 [M+H]⁺. ¹H NMR (400 MHz, DMSO-*d*₆) δ 10.33 (s, 1H), 8.50 (s, 1H), 8.13 (s, 2H), 8.04 (d, *J=* 2.7 Hz, 1H), 7.95 (dd, *J*₁ = 10.8 Hz, *J*₂ = 2.7 Hz, 2H), 7.88 - 7.85 (d, *J* = 3.1 Hz, 2H), 7.47 (dd, *J*₁ = 9.1 Hz, *J*₂ = 2.8 Hz, 1H), 6.86 (d, *J* = 9.1 Hz, 1H), 4.78 (d, *J* = 11.6 Hz, 1H), 3.95 - 3.91 (m, 1H), 3.80 (d, *J* = 11.6 Hz, 1H), 3.70 - 3.57 (m, 5H), 3.49 - 3.41 (m, 2H), 3.27 - 3.24 (m, 2H), 3.04 - 2.90 (m, 3H), 2.70 - 2.62 (m, 3H), 2.58 - 2.54 (m, 1H), 2.39 (s, 6H), 2.04 - 1.97 (m, 1H), 1.65 - 1.52 (m, 5H).

### Example 339 Synthesis of Compounds A340, A341, and A342:

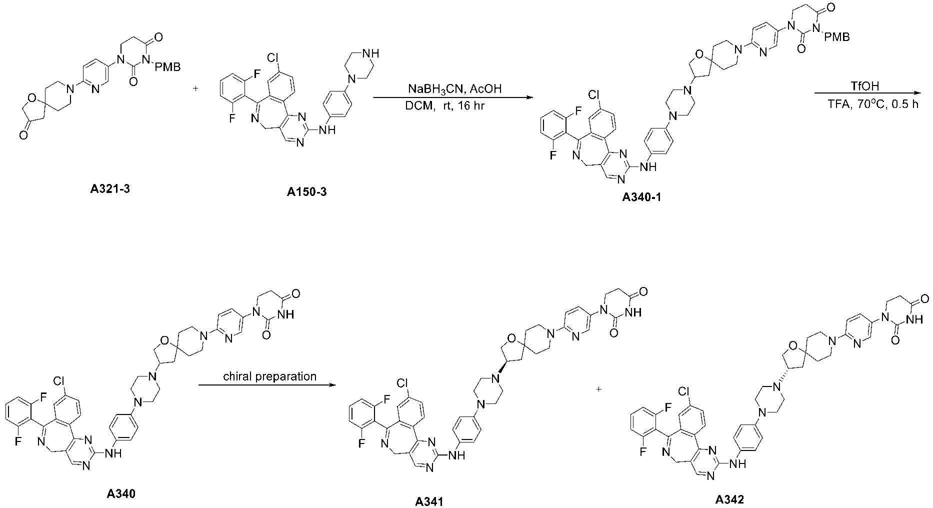

### Step 1:

To a solution of compound **A150-3** (1 g, purity 80%, 1.0 eq) in dichloromethane (60 mL) at room temperature, compound **A321-3** (1.06 g, 1.5 eq) and acetic acid (0.5 mL) were added, the reaction mixture was stirred at room temperature for 0.5 h, and sodium cyanoborohydride (210 mg, 2.2 eq) was added. The reaction mixture was stirred at room temperature for 16 h. After the addition of water, the reaction mixture was extracted with DCM, washed with saturated brine, dried over anhydrous sodium sulfate, filtered, concentrated, and purified by normal phase column to give a yellow solid **A340-1** (0.89 g, yield 60.6%). LCMS (ESI) m/z = 965.2 [M+H]⁺.

### Step 2:

At room temperature, trifluoromethanesulfonic acid (0.4 mL) was added to a solution of compound **A340-1** (182 mg, 0.094 mmol) in trifluoroacetic acid (2 mL), and the reaction mixture was stirred at 70°C for 0.5 h. A saturated sodium bicarbonate solution was slowly added to the reaction mixture in an ice-water bath, the reaction mixture was adjusted to weak alkalinity, extracted with DCM, washed with saturated brine, dried over anhydrous sodium sulfate, filtered, concentrated, and purified by reverse phase column to give a yellow solid **A340** (27 mg, yield 31%). The obtained racemate was resolved by a chiral column to give two configurations, and the detection and analysis conditions were: analytical instrument: Waters e2695, chromatographic column: CHIRALPAK IE-3, mobile phase: a solution of 0.1% diethylamine in acetonitrile: a solution of 0.1% diethylamine in isopropanol (40:60), flow rate: 1.0 mL/min. The R configuration product was **A341,** with a corresponding retention time of 9.78 min. The S configuration product was **A342,** with a corresponding retention time of 11.36 min. The final products of the corresponding configurations prepared using raw materials in R configuration or S configuration were compared with the above A341 and A342 to further confirm that the configurations of A341 and A342 were indeed the above configurations. LCMS (ESI) m/z = 845.2 [M+H]⁺. ¹H NMR (400 MHz, DMSO-*d*₆) δ 10.36 (s, 1H), 9.62 (s, 1H), 8.61 (s, 1H), 8.27 (d, *J* = 8.4 Hz, 1H), 8.07 (d, *J* = 2.8 Hz, 1H), 7.87 (dd, *J* = 8.4, 2.0 Hz, 1H), 7.65 (d, *J* = 8.8 Hz, 2H), 7.62 - 7.45 (m, 2H), 7.33 (d, J = 2.4 Hz, 1H), 7.19 (t, *J* = 8.8 Hz, 2H), 7.00 - 6.81 (m, 3H), 4.00 (t, *J* = 7.6 Hz, 1H), 3.78 - 3.61 (m, 5H), 3.58 - 3.42 (m, 2H), 3.19 - 3.06 (m, 4H), 3.06 - 2.92 (m, 1H), 2.72 (t, *J* = 6.4 Hz, 2H), 2.65 - 2.55 (m, 2H), 2.52 - 2.44 (m, 2H), 2.14 - 2.00 (m, 1H), 1.75 - 1.50 (m, 5H).

### Example 340 Synthesis of Compound A343:

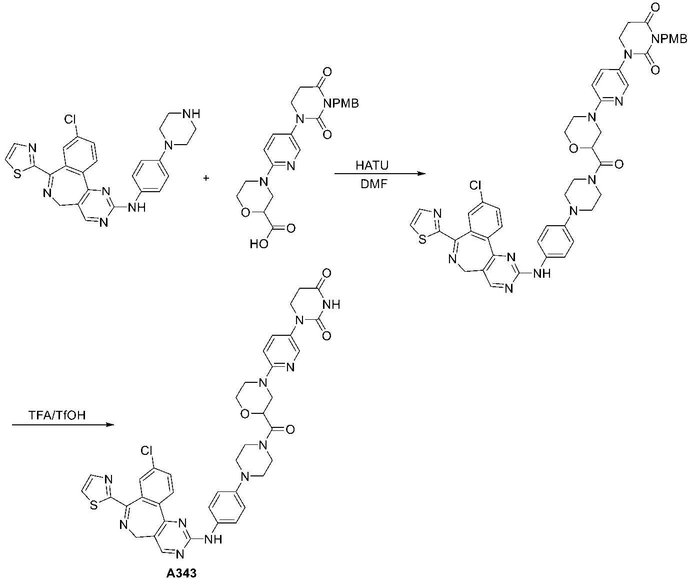

Compound **A343** (5.2 mg, yield 15.2%) was synthesized according to the synthesis method of compound **A101** described in **Example 101.** LCMS (ESI) m/z = 790.3 [M+H]⁺. ¹H NMR (400 MHz, DMSO-*d*₆) δ 10.39 (s, 1H), 9.60 (s, 1H), 8.59 (d, 1H), 8.20 (d, *J* = 8.4 Hz, 1H), 8.13 (d, *J =* 2.4 Hz, 1H), 7.92 (dd, *J =* 8.2, 2.2 Hz, 1H), 7.83 (d, *J* = 2.4 Hz, 1H), 7.72 (d, *J =* 4*.*8 Hz, 1H), 7.66 (d, *J* = 8.8 Hz, 2H), 7.57 (dd, *J* = 8.8, 2.8 Hz, 1H), 7.10 - 7.08 (m, 1H), 7.01 - 7.00 (m, 1H), 6.97 - 6.93 (m, 3H), 4.72 (d, *J* = 14.8 Hz, 1H), 4.42 (m, 1H), 4.20 (d, *J* = 12.4 Hz, 1H), 4.07 (m, 2H), 3.75 - 3.66 (m, 8H), 3.14 - 2.98 (m, 6H), 2.73 (t, *J* = 7.2 Hz, 2H).

### Example 341 Synthesis of Compound A344:

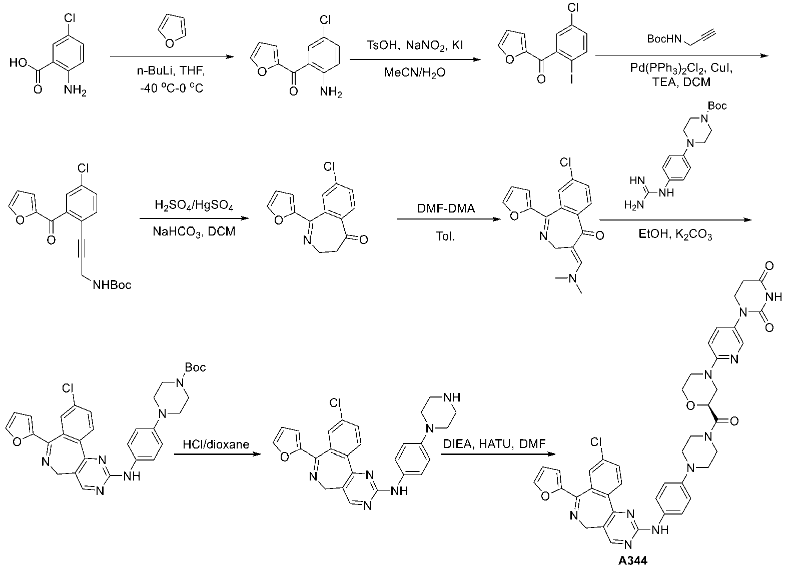

Compound **A344** (28 mg, yield 53%) was synthesized according to the synthesis method of compound **A128** described in **Example 127.** LCMS (ESI) m/z = 773.6 [M+H]⁺. ¹H NMR (400 MHz, DMSO-*d*₆) δ 10.40 (s, 1H), 9.61 (s, 1H), 8.60 (s, 1H), 8.20 (d, *J* = 8.4 Hz, 1H), 8.14 (d, *J* = 2.8 Hz, 1H), 7.93 - 7.89 (m, 1H), 7.87 - 7.85 (m, 1H), 7.80 - 7.78 (m, 1H), 7.69 - 7.64 (m, 2H), 7.60 - 7.55 (m, 1H), 6.99 - 6.92 (m, 3H), 6.74 (d, *J* = 3.6 Hz, 1H), 6.64 - 6.61 (m, 1H), 4.81 - 4.75 (m, 1H), 4.44 - 4.38 (m, 1H), 4.23 - 4.16 (m, 1H), 4.11 - 3.95 (m, 2H), 3.78 - 3.70 (m, 6H), 3.68 - 3.63 (m, 2H), 3.20 - 3.04 (m, 5H), 3.03 - 2.93 (m, 1H), 2.74 (t, *J* = 6.8 Hz, 2H).

### Example 342 Synthesis of Compound A345:

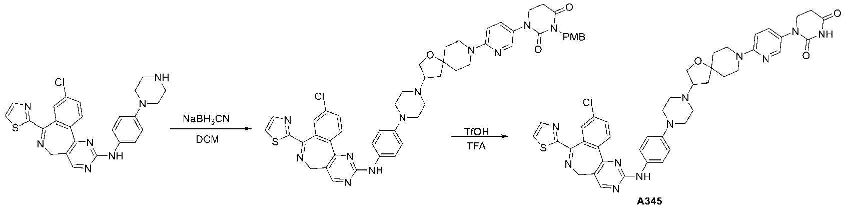

Compound **A345** (31.1 mg, yield 36.3%) was synthesized according to the synthesis method of compound **A132** described in **Example 131.** LCMS (ESI) m/z = 816.4 [M+H]⁺. ¹H NMR (400 MHz, DMSO-*d*₆) δ 10.34 (s, 1H), 9.58 (s, 1H), 8.59 (s, 1H), 8.17 (d, *J =* 8.4 Hz, 1H), 8.04 (d, *J =* 2.8 Hz, 1H), 7.98 (d, *J =* 2.0 Hz, 1H), 7.94 (d, *J* = 3.2 Hz, 1H), 7.91 - 7.85 (m, 2H), 7.60 (d, *J* = 8.8 Hz, 2H), 7.47 (dd, *J* = 9.2, 2.8 Hz, 1H), 6.91 - 6.83 (m, 3H), 4.83 (d, *J* = 11.6 Hz, 1H), 4.01 - 3.93 (m, 1H), 3.85 (d, *J* = 11.6 Hz, 1H), 3.76 - 3.57 (m, 5H), 3.52 - 3.39 (m, 2H), 3.14 - 2.90 (m, 5H), 2.69 (t, *J* = 6.8 Hz, 2H), 2.61 - 2.40 (m, 4H), 2.22 - 1.96 (m, 1H), 1.73 - 1.42 (m, 5H).

### Example 343 Synthesis of Compound A346:

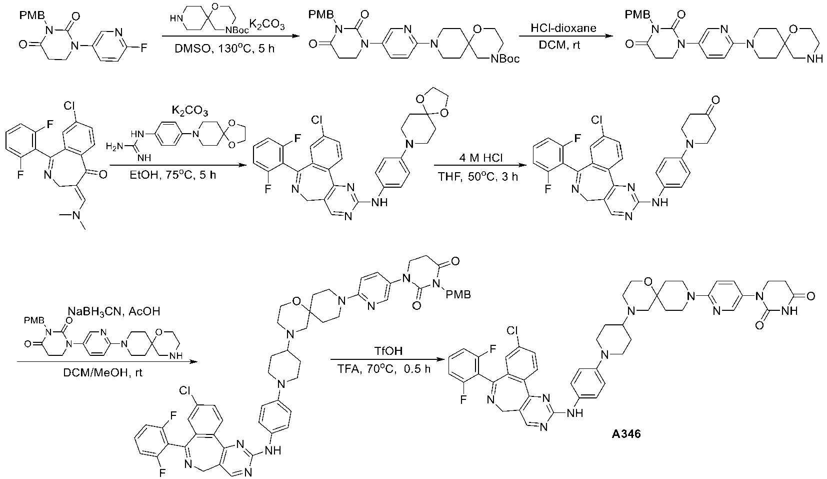

Compound **A346** (107 mg, yield 55%) was synthesized according to the synthesis method of compound **A101** described in **Example 101.** LCMS (ESI) m/z = 859.3 [M+H]⁺. ¹H NMR (400 MHz, DMSO-*d*₆) δ 10.37 (s, 1H), 9.62 (s, 1H), 8.61 (s, 1H), 8.28 (d, *J* = 8.4 Hz, 1H), 8.07 (d, *J* = 2.8 Hz, 1H), 7.88 (dd, *J =* 8.8, 2.4 Hz, 1H), 7.68 - 7.46 (m, 4H), 7.33 (d, *J =* 2.4 Hz, 1H), 7.20 (t, *J* = 8.8 Hz, 2H), 6.94 (d, *J =* 9.2 Hz, 2H), 6.87 (d, *J* = 9.2 Hz, 1H), 3.93 - 3.78 (m, 2H), 3.78 - 3.58 (m, 6H), 3.32 - 3.20 (m, 2H), 2.77 - 2.59 (m, 5H), 2.48 - 2.34 (m, 2H), 2.33 - 2.18 (m, 1H), 1.97 - 1.80 (m, 4H), 1.66 - 1.46 (m, 4H).

### Example 344 Synthesis of Compound A347:

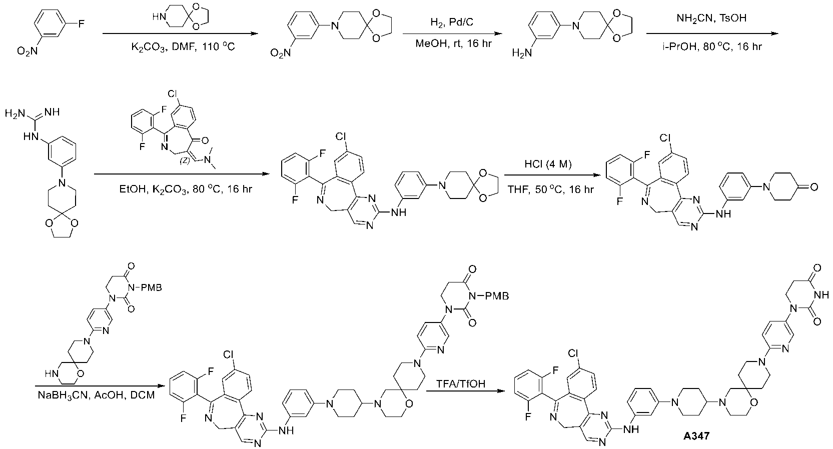

Compound **A347** (59 mg, yield 28%) was synthesized according to the synthesis method of compound **A185** described in **Example 184.** LCMS (ESI) m/z = 859.5 [M+H]⁺. ¹H NMR (400 MHz, DMSO-*d*₆) δ 10.35 (s, 1H), 9.70 (s, 1H), 8.67 (s, 1H), 8.30 (d, *J* = 8.4 Hz, 1H), 8.06 (d, *J* = 2.8 Hz, 1H), 7.88 - 7.84 (m, 1H), 7.62 - 7.59 (m, 1H), 7.58 - 7.51 (m, 1H), 7.50 - 7.46 (m, 1H), 7.35 - 7.32 (m, 1H), 7.23 - 7.10 (m, 4H), 6.85 (d, *J* = 9.2 Hz, 1H), 6.61 - 6.55 (m, 1H), 3.87 - 3.77 (m, 2H), 3.75 - 3.63 (m, 6H), 3.31 - 3.21 (m, 2H), 2.81 - 2.68 (m, 4H), 2.50 - 2.46 (m, 2H), 2.39 (s, 2H), 2.36 - 2.27 (m, 1H), 1.93 - 1.78 (m, 4H), 1.60 - 1.47 (m, 4H).

### Example 345 Synthesis of Compound A348:

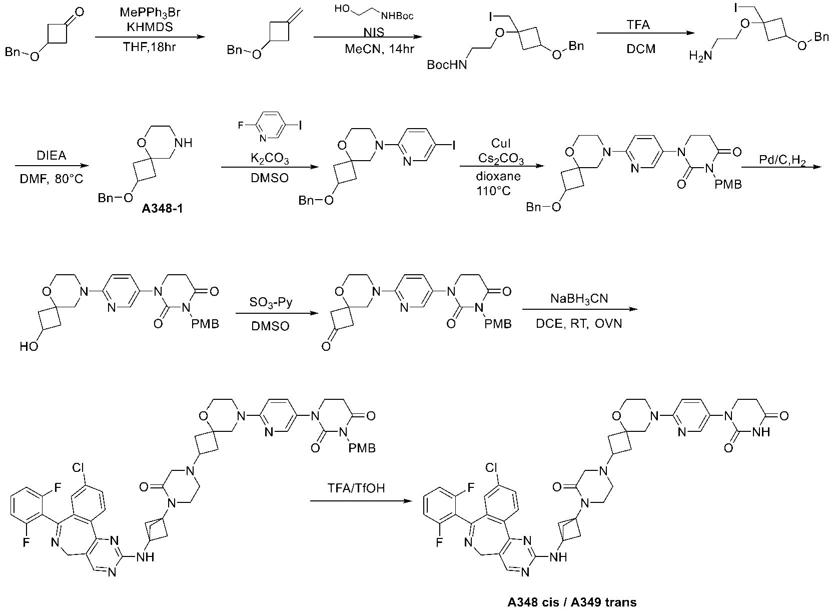

### Step 4:

**A348-1** (440 mg, yield 49.3%) was synthesized with reference to WO2019085996A1. LCMS (ESI) m/z = 234.1 [M+H]⁺.

### Step 10:

Trans-compound **A348** (20 mg, yield 62.7%) was synthesized according to the synthesis method of compound **A132** described in **Example 131.** LCMS (ESI) m/z = 835.52 [M+H]⁺. ¹H NMR (400 MHz, DMSO-*d*₆) *δ* 10.36 (s, 1H), 8.48 (s, 1H), 8.20 - 8.17 (m, 1H), 8.14 (s, 1H), 8.07 (d, *J* = 2.7 Hz, 1H), 7.83 (dd, *J*₁ = 8.6 Hz, *J*₂ =2.2 Hz, 1H), 7.57 - 7.50 (m, 2H), 7.28 (d, *J* = 2.1 Hz, 1H), 7.20 - 7.14 (m, 2H), 6.90 (d, *J* = 9.1 Hz, 1H), 3.70 (t, *J =* 6.7 Hz, 2H), 3.64 - 3.61 (m, 2H), 3.43 - 3.36 (m, 4H), 3.30 - 3.27 (m, 2H), 2.88 (s, 2H), 2.71 - 2.68 (m, 2H), 2.64 - 2.60 (m, 1H), 2.55 - 2.52 (m, 2H), 2.42 (s, 6H), 2.23 - 2.18 (m, 2H), 1.79 - 1.74 (m, 2H).

Cis compound **A349** (20 mg, 45.7% yield). LCMS (ESI) m/z = 835.57 [M+H]⁺. ¹H NMR (400 MHz, DMSO-*d*₆) δ 10.36 (s, 1H), 8.48 (s, 1H), 8.20 - 8.17 (m, 1H), 8.14 (s, 1H), 8.07 (d, *J* = 2.7 Hz, 1H), 7.83 (dd, *J*₁ = 8.5 Hz, *J*₂ = 2.2 Hz, 1H), 7.57 - 7.50 (m, 2H), 7.28 (d, *J* = 2.2 Hz, 1H), 7.20 - 7.14 (m, 2H), 6.90 (d, *J =* 9.1 Hz, 1H), 3.70 (t, *J* = 6.7 Hz, 2H), 3.64 - 3.61 (m, 2H), 3.41 - 3.37 (m, 4H), 3.30 - 3.27 (m, 2H), 2.88 (s, 2H), 2.71 - 2.68 (m, 2H), 2.64 - 2.60 (m, 1H), 2.55 - 2.52 (m, 2H), 2.42 (s, 6H), 2.24 - 2.19 (m, 2H), 1.79 - 1.74 (m, 2H).

### Example 346 Synthesis of Compound A350:

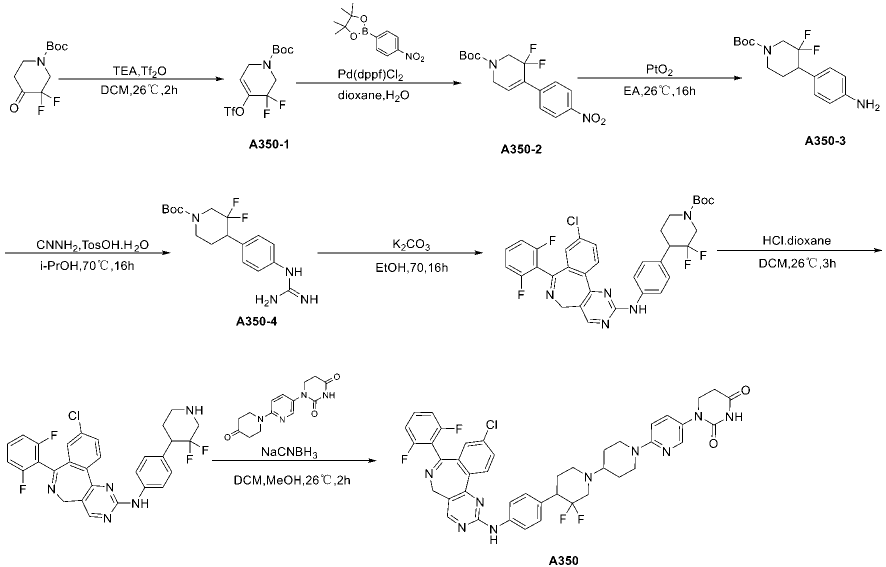

### Step 1:

Triethylamine (19.36 g, 191.30 mmol) and trifluoromethanesulfonic anhydride (26.99 g, 95.65 mmol) were added to a solution of N-Boc-3,3-difluoropiperidin-4-one (15 g, 63.77 mmol) in dichloromethane (150 mL) at room temperature. After reaction with stirring at room temperature for 2 h, the organic layer was washed with water and then washed twice with saturated brine. The organic phase was dried over anhydrous sodium sulfate and filtered, the filtrate was concentrated under reduced pressure, and the residue was separated for purification by silica gel column chromatography (ethyl acetate/petroleum ether = 1:5) to give a colorless oil **A350-1** (4.0 g, yield 17.0%). LCMS (ESI) m/z = 368.0 [M+H]⁺.

### Step 2:

At room temperature, 4-nitrophenylboronic acid pinacol ester (1.76 g, 7.08 mmol), Pd(dppf)Cl₂ (398 mg, 0.54 mmol) and sodium carbonate (1.15 g, 10.89 mmol) were added to a mixture of a solution of compound **A350-1** (2.00 g, 5.45 mmol) in 1,4-dioxane (20 mL) and H₂O (4 mL). The reaction mixture was heated to 100°C under argon protection and stirred for 16 h. The reaction mixture was filtered, the filtrate was concentrated under reduced pressure, and the residue was separated for purification by silica gel column chromatography to give a yellow solid **A350-2** (1.00 g, yield 53%). LCMS (ESI) m/z = 341.1 [M+H]⁺.

### Step 3:

Compound **A350** (6.7 mg, yield 21%) was synthesized according to the method of compound **A150** described in **Example 149.** LCMS (ESI) m/z = 824.3 [M+H]⁺. ¹H NMR (400 MHz, DMSO-*d*₆) δ 10.38 (s, 1H), 9.90 (s, 1H), 8.68 (s, 1H), 8.31 (d, *J* = 8.4 Hz, 1H), 8.08 (d, *J* = 2.8 Hz, 1H), 7.92 - 7.88 (m, 1H), 7.79 (d, *J* = 8.4 Hz, 2H), 7.61 - 7.55 (m, 1H), 7.51 (dd, *J* = 9.2, 2.8 Hz, 1H), 7.35 (d, *J* = 2.4 Hz, 1H), 7.27 (d, *J* = 8.4 Hz, 2H), 7.19 (t, *J* = 8.8 Hz, 2H), 6.93 - 6.88 (m, 1H), 4.37 (d, *J* = 12.4 Hz, 2H), 3.73 (t, *J* = 6.8 Hz, 2H), 3.24 - 3.17 (m, 1H), 3.10 - 2.92 (m, 2H), 2.83 (t, *J* = 12.4 Hz, 2H), 2.75 - 2.65 (m, 4H), 2.63 - 2.55 (m, 1H), 2.45 - 2.37 (m, 1H), 2.10 - 2.01 (m, 1H), 1.90 - 1.82 (m, 2H), 1.55 - 1.40 (m, 2H).

### Example 347 Synthesis of Compound A351:

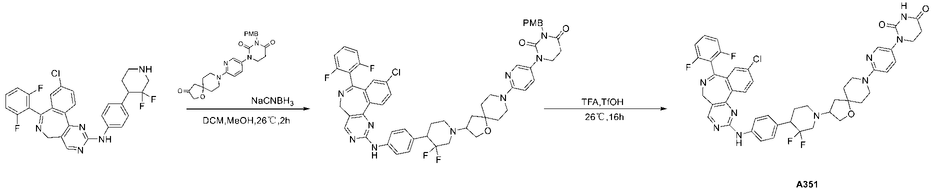

Compound **A351** (8.0 mg, yield 30%) was synthesized according to the method of compound **A4** described in **Example 4.** LCMS (ESI) m/z = 880.3 [M+H]⁺. ¹H NMR (400 MHz, DMSO-*d*₆) δ 10.37 (s, 1H), 9.91 (s, 1H), 8.69 (s, 1H), 8.31 (d, *J* = 8.4 Hz, 1H), 8.07 (d, *J* = 2.8 Hz, 1H), 7.90 (dd, *J* = 8.5, 2.4 Hz, 1H), 7.80 (d, *J* = 8.4 Hz, 2H), 7.63 - 7.54 (m, 1H), 7.51 (dd, *J* = 9.1, 2.7 Hz, 1H), 7.35 (d, *J* = 2.4 Hz, 1H), 7.27 (d, *J* = 8.4 Hz, 2H), 7.20 (t, *J* = 8.8 Hz, 2H), 6.90 (d, *J* = 9.2 Hz, 1H), 4.01 (t, *J* = 7.6 Hz, 1H), 3.75 - 3.72 (m, 4H), 3.70 - 3.63 (m, 2H), 3.57 - 3.46 (m, 2H), 3.26 - 3.14 (m, 2H), 3.10 - 3.01 (m, 2H), 2.72 (t, *J* = 6.7 Hz, 2H), 2.30 - 2.20 (m, 1H), 2.15 - 2.05 (m, 2H), 1.74 - 1.65 (m, 6H).

### Example 348 Synthesis of Compound A352:

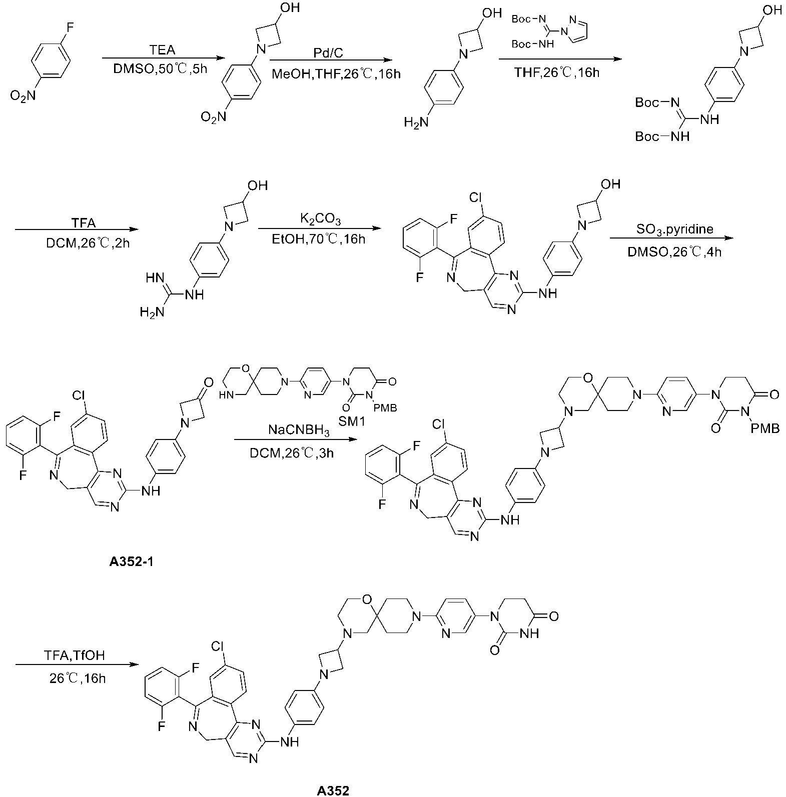

### Step 1:

Compound **A352-1** (50 mg, yield 30%) was synthesized according to the synthesis method of compound **A146** described in **Example 145.** LCMS (ESI) m/z = 504.1 [M+H]⁺.

### Step 2:

Compound **A352** (3 mg, yield 20.6%) was synthesized according to the synthesis method of compound **A132** described in **Example 131.** LCMS (ESI) m/z = 831.4 [M+H]⁺.¹H NMR (400 MHz, DMSO-*d*₆) δ 10.35 (s, 1H), 9.51 (s, 1H), 8.58 (s, 1H), 8.26 (d, *J* = 8.4 Hz, 1H), 8.07 (d, *J* = 2.8 Hz, 1H), 7.87 (dd, *J* = 8.5, 2.4 Hz, 1H), 7.60 - 7.55 (m, 3H), 7.50 (dd, *J* = 9.0, 2.8 Hz, 1H), 7.32 (d, *J* = 2.0 Hz, 1H), 7.19 (t, *J* = 8.8 Hz, 2H), 6.83 (d, *J* = 8.8 Hz, 1H), 6.45 (d, *J* = 8.4 Hz, 2H), 3.91 (t, *J* = 7.2 Hz, 2H), 3.85 - 3.80 (m, 2H), 3.75 - 3.70 (m, 4H), 3.60 - 3.54 (m, 2H), 3.29 - 3.22 (m, 3H), 2.72 (t, *J* = 6.8 Hz, 2H), 2.40 - 2.32 (m, 2H), 2.28 - 2.22 (m, 2H), 1.95 - 1.85 (m, 2H), 1.62 - 1.53 (m, 2H).

### Example 349 Synthesis of Compound A353:

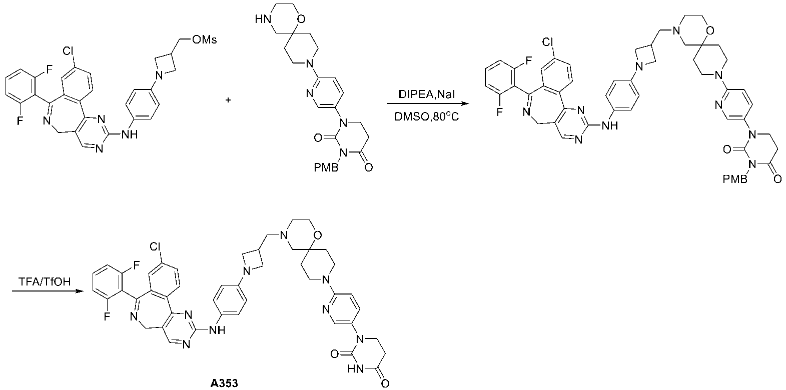

Compound **A353** (18.2 mg, yield 44.2%) was synthesized according to the synthesis method of compound **A202** described in **Example 201.** LCMS (ESI) m/z = 845.4 [M+H]⁺. ¹H NMR (400 MHz, DMSO-*d*₆) δ 10.33 (s, 1H), 9.47 (s, 1H), 8.55 (s, 1H), 8.23 (d, *J* = 8.4 Hz, 1H), 8.04 (d, *J* = 2.4 Hz, 1H), 7.86 - 7.81 (m, 1H), 7.58 - 7.46 (m, 4H), 7.29 (d, *J* = 2.0 Hz, 1H), 7.16 (t, *J* = 8.8 Hz, 2H), 6.85 (d, *J* = 10.0 Hz, 1H), 6.45 - 6.36 (m, 2H), 3.93 - 3.75 (m, 5H), 3.71 - 3.64 (m, 4H), 3.45 - 3.39 (m, 2H), 3.28 - 3.19 (m, 2H), 2.94 - 2.85 (m, 1H), 2.69 (t, *J* = 6.8 Hz, 2H), 2.55 - 2.53 (m, 1H), 2.35 (s, 2H), 2.25 (s, 2H), 1.92 - 1.80 (m, 2H), 1.59 - 1.47 (m, 2H).

### Example 350 Synthesis of Compound A354:

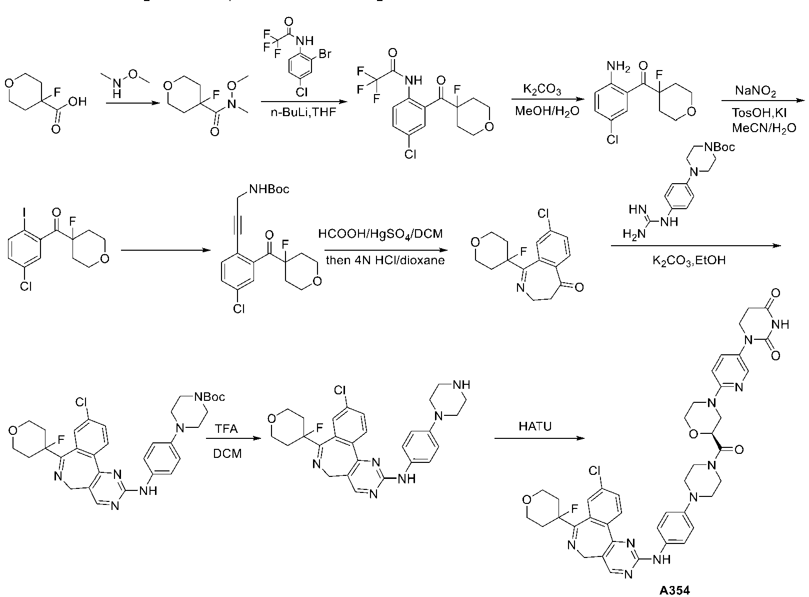

Compound **A354** (3.2 mg, yield 8.2%) was synthesized according to the synthesis method of compound **A128** described in **Example 127.** LCMS (ESI) m/z = 809.3 [M+H]+. 1H NMR (400 MHz, DMSO-d6) δ 10.38 (s, 1H), 9.61 (s, 1H), 8.60 (s, 1H), 8.16 (d, J = 7.8 Hz, 1H), 8.14 (d, J = 2.8 Hz, 1H), 7.91 (t, J = 2.4 Hz, 1H), 7.86 (dd, J = 8.4, 2.0 Hz, 1H), 7.67 (d, J = 9.2 Hz, 2H), 7.58 (dd, J = 8.8, 2.8 Hz, 1H), 6.98 - 6.94 (m, 3H), 5.79 (s, 2H), 4.73 (d, J = 12.0 Hz, 1H), 4.43 - 4.39 (m, 1H), 4.23 - 3.98 (m, 4H), 3.81 - 3.66 (m, 11H), 3.47 - 3.37 (m, 2H), 3.21 - 3.19 (m, 2H), 3.12 - 3.08 (m, 2H), 2.74 (t, J = 7.2 Hz, 2H), 2.26 - 2.20 (m, 2H).

### Example 351 Synthesis of Compound A355:

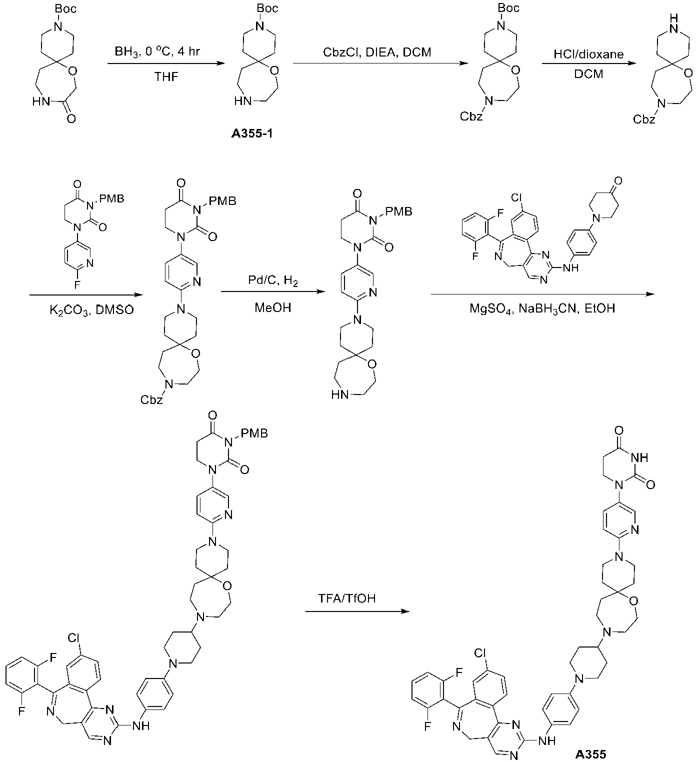

### Step 1:

At 0°C, borane dimethyl sulfide (2 M, 2.64 mL) was added dropwise to a solution of tert-butyl 9-oxo-7-oxo-3,10-diazaspiro[5.6]dodecane-3-carboxylate (750 mg, 2.64 mmol) in tetrahydrofuran (20 mL). The reaction mixture was reacted at 0°C for 4 h. After LCMS showed that the reaction was complete, methanol (20 mL) was slowly added to the reaction mixture, and the mixture was stirred for 4 h. After the reaction mixture was concentrated, saturated sodium bicarbonate and ethyl acetate were added for extraction, and the organic phase was concentrated to give **A355-1** (730 mg, crude) which was directly used in the next step. LCMS (ESI) m/z = 271.5 [M+H]⁺.

### Step 2:

Compound **A355** (4 mg, yield 25%) was synthesized according to the synthesis method of compound **A101** described in **Example 101.** LCMS (ESI) m/z = 873.9 [M+H]⁺. ¹H NMR (400 MHz, DMSO-*d*₆) δ 10.35 (s, 1H), 9.60 (s, 1H), 8.61 (s, 1H), 8.29 (d, *J* = 8.4 Hz, 1H), 8.07 (d, *J* = 2.8 Hz, 1H), 7.89 - 7.86 (m, 1H), 7.67 - 7.62 (m, 2H), 7.61 - 7.53 (m, 1H), 7.51 - 7.47 (m, 1H), 7.34 (d, *J* = 2.2 Hz, 1H), 7.23 - 7.16 (m, 2H), 6.97 - 6.92 (m, 2H), 6.86 (d, *J* = 9.1 Hz, 1H), 3.95 - 3.86 (m, 2H), 3.74 - 3.66 (m, 6H), 3.28 - 3.17 (m, 2H), 2.75 - 2.69 (m, 4H), 2.69 - 2.59 (m, 5H), 1.85 - 1.75 (m, 6H), 1.69 - 1.57 (m, 2H), 1.49 - 1.38 (m, 2H).

### Example 352 Synthesis of Compound A356:

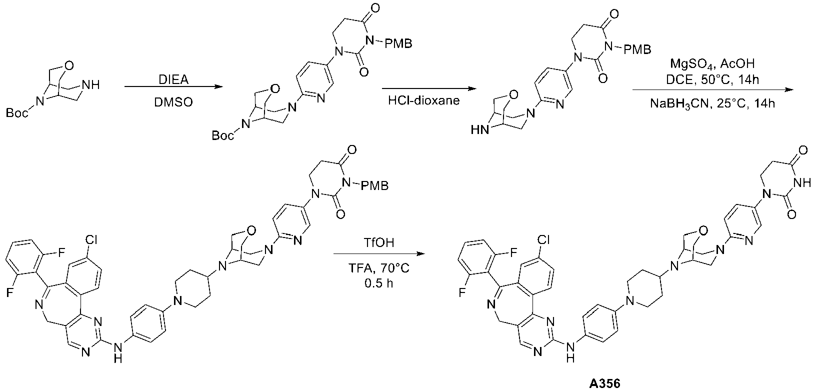

Compound **A356** (10.0 mg, yield 28.6%) was synthesized according to the method of compound **A132** described in **Example 131.** LCMS (ESI) m/z = 831.52 [M+H]⁺. ¹H NMR (400 MHz, DMSO-*d*₆) δ 10.34 (s, 1H), 9.60 (s, 1H), 8.60 (s, 1H), 8.28 (d, *J* = 8.5 Hz, 1H), 8.09 (d, *J* = 2.7 Hz, 1H), 7.87 (dd, *J*₁ = 8.5 Hz, *J*₂ =2.2 Hz, 1H), 7.64 (d, *J* = 8.7 Hz, 2H), 7.58 - 7.54 (m, 1H), 7.52 (dd, *J*₁ = 9.0 Hz, *J*₂ =2.7 Hz, 1H), 7.33 (d, *J* = 2.1 Hz, 1H), 7.21-7.16 (m, 2H), 6.95 (d, *J* = 9.0 Hz, 2H), 6.71 (d, *J* = 9.2 Hz, 1H), 3.85 - 3.77 (m, 6H), 3.72 (t, *J* = 6.7 Hz, 2H), 3.63 - 3.60 (m, 2H), 3.33 - 3.31 (m, 1H), 3.21 - 3.18 (m, 2H), 2.99 - 2.93 (s, 1H), 2.76 - 2.71 (m, 4H), 2.06 - 2.01 (m, 3H), 1.49 - 1.37 (m, 2H).

### Example 353 Synthesis of Compound A357:

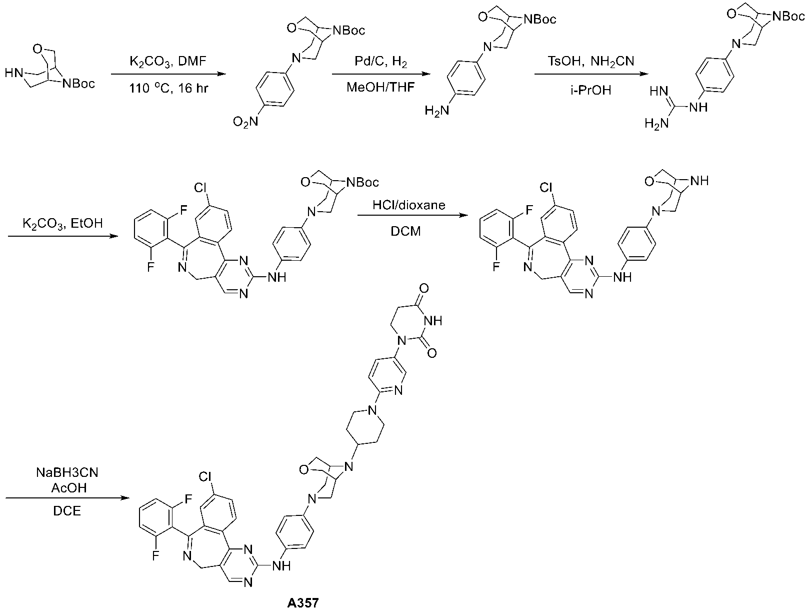

Compound **A371** (6.0 mg, yield 10.9%) was synthesized according to the method of compound **A101** described in **Example 101.** LCMS (ESI) m/z = 831.54 [M+H]⁺. ¹H NMR (400 MHz, DMSO-*d*₆) δ 10.32 (s, 1H), 9.52 (s, 1H), 8.57 (s, 1H), 8.24 (d, *J* = 8.5 Hz, 1H), 8.04 (d, *J* = 2.7 Hz, 1H), 7.84 (dd, *J*₁ = 8.5 Hz, *J*₂ =2.2 Hz, 1H), 7.59 (d, *J* = 8.9 Hz, 2H), 7.56 - 7.50 (m, 1H), 7.47 (dd, *J*₁ = 9.1 Hz, *J*₂ =2.7 Hz, 1H), 7.30 (d, *J* = 2.2 Hz, 1H), 7.19 - 7.14 (m, 2H), 6.86 - 6.83 (m, 3H), 4.20 - 4.16 (m, 2H) 3.85 - 3.73 (m, 6H), 3.69 (t, *J* = 6.7 Hz, 2H), 2.92 - 2.83 (m, 4H), 2.69 (t, *J* = 6.7 Hz, 2H), 2.59 - 2.56 (m, 2H), 2.25 - 2.20 (m, 1H), 1.81 - 1.77 (m, 2H), 1.44 - 1.35 (m, 2H).

### Example 354 Synthesis of Compound A358:

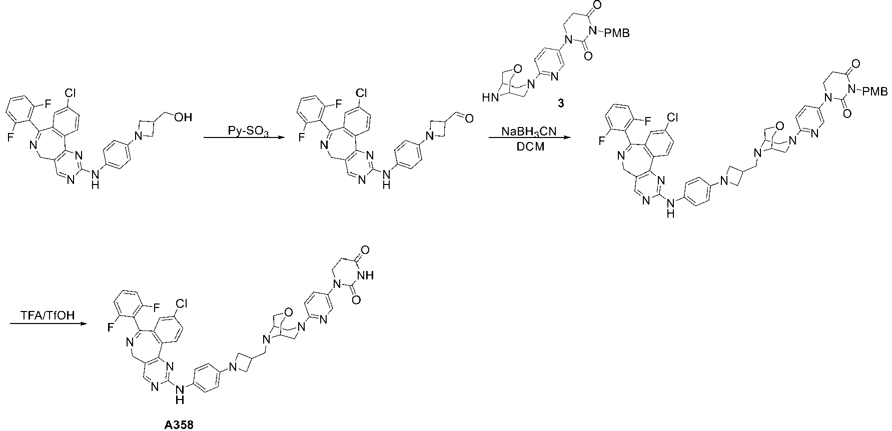

Compound **A358** (28.5 mg, yield 46.7%) was synthesized according to the method of compound **A223** described in **Example 222.** LCMS (ESI) m/z = 817.5 [M+H]⁺. ¹H NMR (400 MHz, DMSO-*d*₆) δ 10.31 (s, 1H), 9.47 (s, 1H), 8.55 (s, 1H), 8.23 (d, *J* = 8.0 Hz, 1H), 8.05 (d, *J* = 2.8 Hz, 1H), 7.86 - 7.82 (m, 1H), 7.56 - 7.52 (m, 3H), 7.49 - 7.45 (m, 1H), 7.29 (d, *J* = 2.4 Hz, 1H), 7.16 (t, *J* = 8.0 Hz, 2H), 6.69 (d, *J* = 10.0 Hz, 1H), 6.42 (d, *J* = 8.8 Hz, 2H), 3.91 - 3.78 (m, 8H), 3.76 - 3.66 (m, 5H), 3.51 - 3.46 (m, 2H), 3.02 - 2.98 (m, 2H), 2.88 - 2.83 (m, 3H), 2.70 (t, *J* = 6.8 Hz, 2H).

### Example 355 Synthesis of Compound A359:

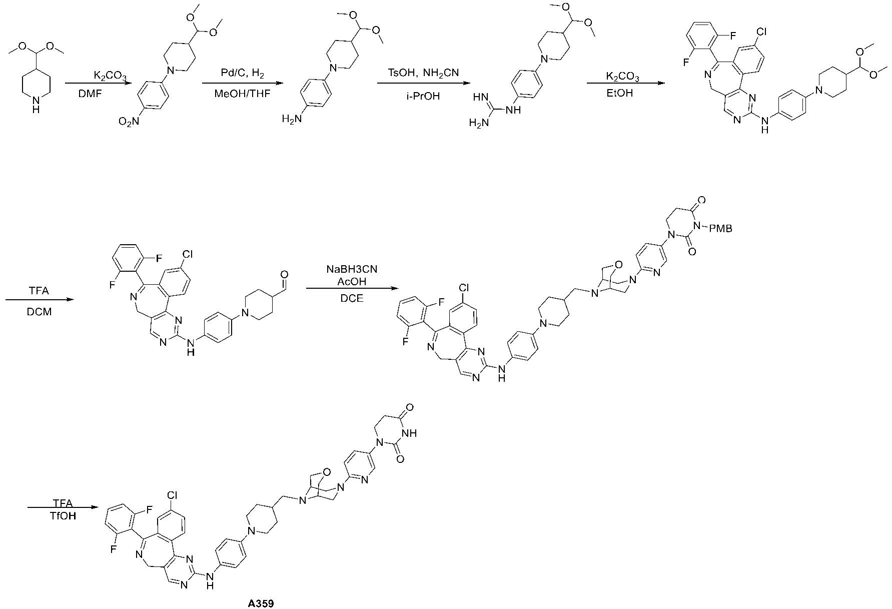

Compound **A359** (16.0 mg, yield 60.9%) was synthesized according to the method of compound **A101** described in **Example 101.** LCMS (ESI) m/z = 845.57 [M+H]⁺. ¹H NMR (400 MHz, DMSO-*d*₆) δ 10.32 (s, 1H), 9.50 (s, 1H), 8.39 (s, 1H), 8.05 (d, *J =* 2.7 Hz, 1H), 7.95 (d, *J* = 8.3 Hz, 1H), 7.63 - 7.59 (m, 3H), 7.50 - 7.45 (m, 2H), 7.20 - 7.15 (m, 2H), 6.91 - 6.88 (m, 3H), 6.69 (d, *J* = 9.2 Hz, 1H), 5.34 - 5.23 (m, 2H), 3.87 - 3.78 (m, 5H), 3.74 - 3.67 (m, 4H), 3.61 - 3.57 (m, 2H), 3.54 - 3.49 (m, 1H), 2.81 (s, 2H), 2.70 (t, *J* = 6.7 Hz, 2H), 2.63 - 2.56 (m, 3H), 2.00 - 1.95 (m, 2H), 1.86 - 1.82 (m, 2H).

### Example 356 Synthesis of Compound A360:

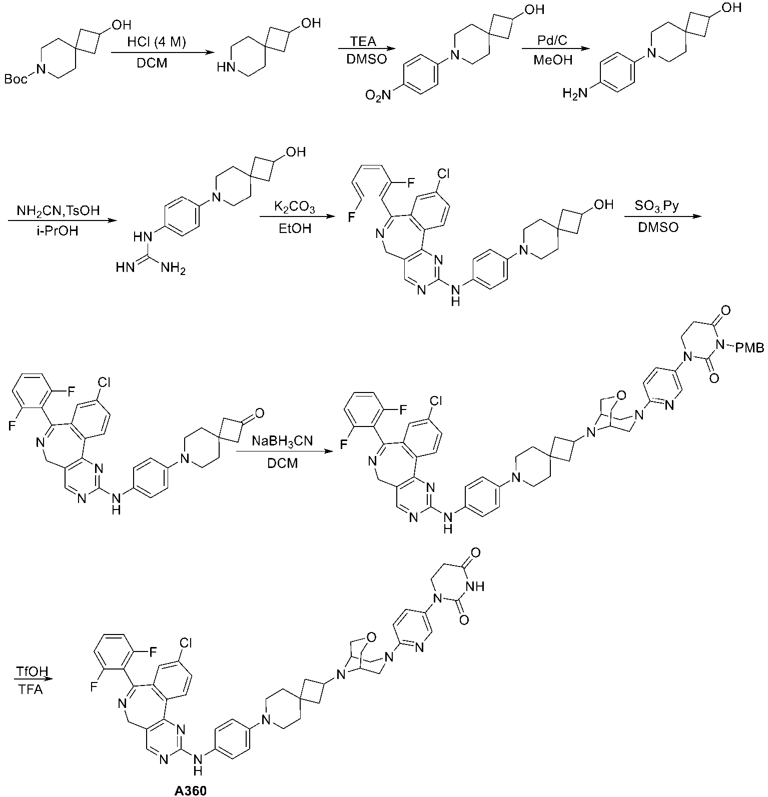

Compound **A360** (23.6 mg, yield 47.9%) was synthesized according to the synthesis method of compound **A189** described in **Example 188** and the synthesis method of compound **A132** described in **Example 131.** LCMS (ESI) m/z = 871.55 [M+H]⁺. ¹H NMR (400 MHz, DMSO-*d*₆) δ 10.32 (s, 1H), 9.57 (s, 1H), 8.58 (s, 1H), 8.24 (d, *J* = 8.4 Hz, 1H), 8.05 (d, *J* = 2.8 Hz, 1H), 7.84 (dd, *J* = 8.4, 2.0 Hz, 1H), 7.65 - 7.43 (m, 4H), 7.30 (d, *J* = 2.0 Hz, 1H), 7.16 (t, *J* = 8.8 Hz, 2H), 6.91 (d, *J* = 9.2 Hz, 2H), 6.67 (d, *J* = 9.2 Hz, 1H), 3.90 - 3.61 (m, 9H), 3.30 - 3.23 (m, 2H), 3.10 - 2.91 (m, 4H), 2.84 - 2.80 (m, 2H), 2.70 (t, *J* = 6.8 Hz, 2H), 2.04 (t, *J* = 8.8 Hz, 2H), 1.75 - 1.56 (m, 4H), 1.55 - 1.46 (m, 2H).

### Example 357 Synthesis of Compound A361:

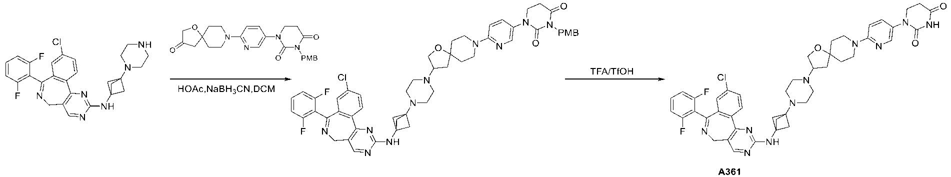

Compound **A361** (26.3 mg, yield 39.1%) was synthesized according to the method of compound **A223** described in **Example 222.** LCMS (ESI) m/z = 835.6 [M+H]⁺. ¹H NMR (400 MHz, DMSO-*d*₆) δ 10.32 (s, 1H), 8.46 (s, 1H), 8.19 (s, 1H), 8.07 (s, 1H), 8.03 (d, *J* = 4.0 Hz, 1H), 7.84 (d, *J* = 8.0 Hz, 1H), 7.58 - 7.44 (m, 2H), 7.27 (d, *J* = 2.4 Hz, 1H), 7.16 (t, *J* = 8.0 Hz, 2H), 6.85 (d, *J* = 8.0 Hz, 1H), 3.97 - 3.90 (m, 1H), 3.72 - 3.62 (m, 4H), 3.59 - 3.53 (m, 1H), 3.50 - 3.40 (m, 2H), 2.95 - 2.86 (m, 1H), 2.69 (t, *J* = 6.8 Hz, 2H), 2.45 - 2.30 (m, 6H), 2.07 - 1.96 (m, 7H), 1.67 - 1.49 (m, 5H).

### Example 358 Synthesis of Compound A362:

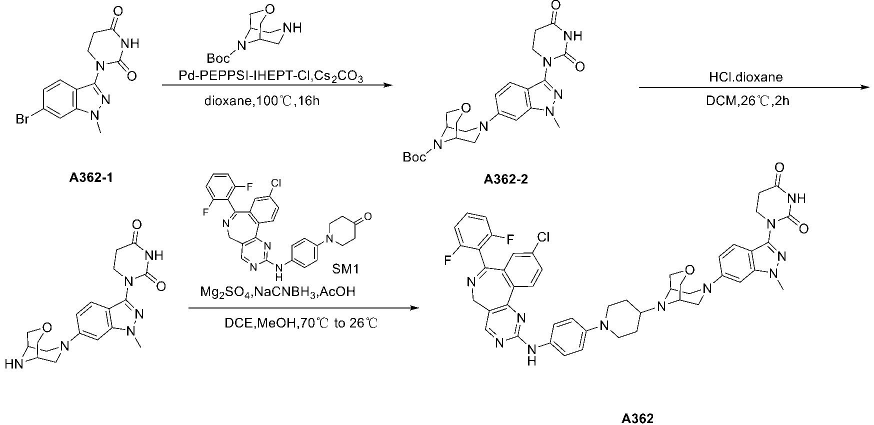

### Step 1:

At room temperature, tert-butyl 3-oxa-7,9-diazabicyclo[3.3.1]nonane-7-carboxylate (99 mg, 0.43 mmol), Pd-PEPPSI-IHEPT-Cl (42.0 mg, 0.06 mmol) and cesium carbonate (201.6 mg, 0.6 mmol) were added to a solution of compound **A362-1** (100.0 mg, 0.31 mmol) in 1,4-dioxane (5 mL). The reaction mixture was heated to 100°C under argon protection and stirred for 16 h. The reaction mixture was filtered, the filtrate was concentrated under reduced pressure, and the residue was separated for purification by silica gel column chromatography to give a yellow solid **A362-2** (40 mg, yield 27%). LCMS (ESI) m/z = 471.2 [M+H]⁺.

### Step 2:

Compound **A362** (3.7 mg, yield 12%) was synthesized according to the synthesis method of compound **A132** described in **Example 131.** LCMS (ESI) m/z = 884.3 [M+H]⁺. ¹H NMR (400 MHz, DMSO-*d*₆) δ 9.14 (s, 1H), 8.84 (s, 1H), 8.40 (s, 1H), 7.91 - 7.82 (m, 2H), 7.59 - 7.48 (m, 5H), 7.16 - 7.10 (m, 2H), 6.98 (d, *J* = 2.4 Hz, 1H), 6.89 - 6.83 (m, 2H), 6.79 (dd, *J* = 8.0, 2.4 Hz, 1H), 4.87 (s, 2H), 4.04 (dd, *J* = 7.0, 4.4 Hz, 1H), 4.01 - 3.95 (m, 1H), 3.98 (s, 3H), 3.89 - 3.79 (m, 2H), 3.78 - 3.66 (m, 3H), 3.64 - 3.60 (m, 1H), 3.55 - 3.48 (m, 2H), 3.46 - 3.37 (m, 2H), 3.25 - 3.20 (m, 2H), 2.90 - 2.86 (m, 1H), 2.78 - 2.72 (m, 2H), 2.65 - 2.60 m, 2H), 1.86 - 1.80 (m, 2H), 1.78 - 1.68 (m, 2H).

### Example 359 Synthesis of Compound A363:

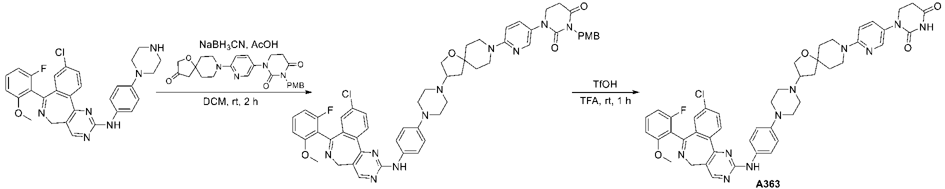

Compound **A363** (17 mg, yield 19%) was synthesized according to the synthesis method of compound **A132** described in **Example 131.** LCMS (ESI) m/z = 857.2 [M+H]⁺. ¹H NMR (400 MHz, DMSO-*d*₆) δ 10.36 (s, 1H), 9.60 (s, 1H), 8.59 (s, 1H), 8.24 (d, J = 8.4 Hz, 1H), 8.07 (d, *J* = 2.8 Hz, 1H), 7.81 (dd, *J* = 8.4, 2.0 Hz, 1H), 7.67 (d, *J* = 8.8 Hz, 2H), 7.56 - 7.38 (m, 2H), 7.22 (s, 1H), 7.07 - 6.78 (m, 4H), 4.01 (t, *J* = 7.6 Hz, 1H), 3.79 - 3.58 (m, 5H), 3.57 - 3.42 (m, 4H), 3.33 - 3.31 (m, 1H), 3.14 - 2.96 (m, 5H), 2.72 (t, *J* = 6.4 Hz, 2H), 2.64 - 2.57 (m, 2H), 2.51 - 2.43 (m, 2H), 2.16 - 1.99 (m, 1H), 1.79 - 1.49 (m, 5H).

### Example 360 Synthesis of Compound A364:

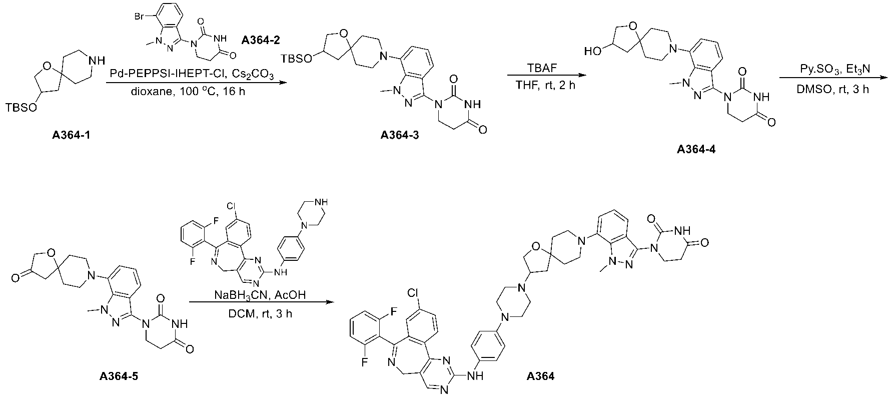

### Step 1:

**A364-2** (400 mg, 1.24 mmol), Pd-PEPPSI-IHEPT-Cl (241 mg, 0.25 mmol) and cesium carbonate (1.21 g, 3.71 mmol) were added to a solution of compound **A364-1** (672 mg, 2.48 mmol) in 1,4-dioxane (5 mL) at room temperature. The reaction mixture was reacted in a sealed tube at 100°C for 16 h under argon protection. The reaction mixture was filtered, and the filtrate was concentrated to give a crude product, which was separated for purification by silica gel column chromatography to give a yellow solid **A364-3** (300 mg, yield 47%). LCMS (ESI) m/z = 514.2 [M+H]⁺.

### Step 2:

To a solution of compound **A364-3** (300 mg, 0.58 mmol) in tetrahydrofuran (6 mL), a solution of tetrabutylammonium fluoride in tetrahydrofuran (1.75 mL, 1.75 mmol) was added, and the reaction mixture was stirred at room temperature for 2 h. The reaction mixture was directly spin-dried to give a crude product, which was separated and purified by silica gel column chromatography to give a yellow solid **A364-4** (110 mg, yield 47%). LCMS (ESI) m/z = 400.2 [M+H]⁺.

### Step 3:

Triethylamine (1.04 mL, 7.51 mmol) was added to a solution of compound **A364-4** (100 mg, 0.25 mmol) in dimethyl sulfoxide (2 mL), the reaction mixture was cooled to 0°C, sulfur trioxide pyridine complex (398 mg, 2.5 mmol) was added, and the reaction mixture was stirred at room temperature for 3 h. After the addition of water, the mixture was extracted with dichloromethane, washed with saturated citric acid solution, washed with saturated brine, dried over anhydrous sodium sulfate, filtered, and concentrated to give a crude product. The crude product was separated for purification by silica gel column chromatography to give a yellow solid **A364-5** (84 mg, yield 84%). LCMS (ESI) m/z = 398.2 [M+H]⁺.

### Step 4:

**A150-3** (40 mg, 0.08 mmol) and acetic acid (0.1 mL) were added to a solution of compound **A364-5** (45 mg, 0.12 mmol) in dichloromethane (1 mL), and the reaction mixture was stirred at room temperature for 0.5 h. Sodium cyanoborohydride (10 mg, 0.15 mmol) was added, and the reaction mixture was stirred at room temperature for 3 h. After the addition of water, the mixture was extracted with dichloromethane, washed with saturated brine, dried over anhydrous sodium sulfate, filtered and concentrated to give a crude product, which was purified by a preparative thin layer chromatography plate to give a yellow solid **A364** (7 mg, yield 10%). LCMS (ESI) m/z = 898.4 [M+H]⁺. ¹H NMR (400 MHz, DMSO-*d*₆) δ 10.56 (s, 1H), 9.63 (s, 1H), 8.62 (s, 1H), 8.28 (d, *J* = 8.8 Hz, 1H), 7.88 (dd, *J* = 8.4, 2.0 Hz, 1H), 7.66 (d, *J* = 8.4 Hz, 2H), 7.63 - 7.49 (m, 1H), 7.36 - 7.26 (m, 2H), 7.20 (t, *J* = 8.4 Hz, 2H), 7.11 - 7.00 (m, 2H), 7.00 - 6.89 (m, 2H), 4.28 (s, 3H), 4.10 - 3.97 (m, 1H), 3.91 (t, J = 6.8 Hz, 2H), 3.77 - 3.63 (m, 1H), 3.12 (s, 3H), 2.79 (t, *J* = 6.8 Hz, 2H), 2.73 - 2.68 (m, 1H), 2.68 - 2.55 (m, 3H), 1.36 - 1.27 (m, 4H).

### Example 361 Synthesis of Compound A365:

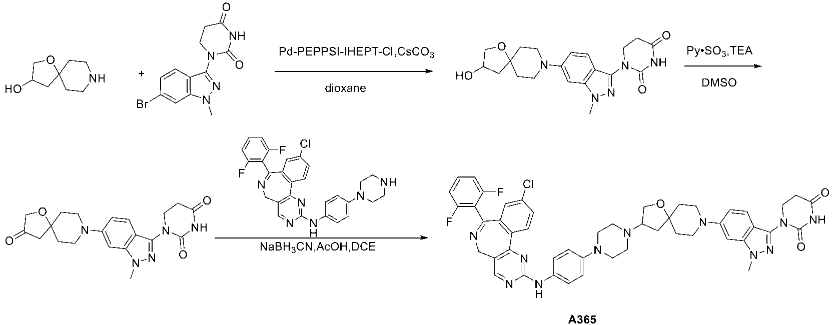

Compound **A365** (4.0 mg, yield 3.5%) was synthesized according to the synthesis method of compound **A364** described in **Example 360.** LCMS (ESI) m/z = 898.4 [M+H]⁺. ¹H NMR (400 MHz, DMSO-*d*₆) δ 10.52 (s, 1H), 9.62 (s, 1H), 8.62 (s, 1H), 8.28 (d, *J* = 8.6 Hz, 1H), 7.88 (dd, *J* = 8.5, 2.2 Hz, 1H), 7.66 (d, *J* = 8.9 Hz, 2H), 7.57 (ddd, *J* = 15.0, 8.4, 6.6 Hz, 1H), 7.47 (d, *J* = 9.0 Hz, 1H), 7.34 (d, *J* = 2.2 Hz, 1H), 7.20 (t, *J* = 8.7 Hz, 2H), 6.95 (d, *J* = 9.0 Hz, 4H), 6.87 (s, 1H), 4.02 (t, *J* = 7.5 Hz, 1H), 3.92 (d, *J* = 4.2 Hz, 6H), 3.67 (t, *J* = 8.2 Hz, 1H), 3.11 (ddddd, *J* = 6.3, 4.4, 3.7, 1.9, 1.1 Hz, 4H), 3.03 (t, *J* = 7.5 Hz, 1H), 2.76 (t, *J* = 6.7 Hz, 2H), 2.61 (t, *J* = 5.7 Hz, 2H), 2.10 (dd, *J* = 12.3, 7.6 Hz, 1H), 1.73 (s, 1H), 1.66 (dd, *J* = 12.3, 8.5 Hz, 1H).

### Example 1: Biological Effects

### Biological Example 1: Protein Abundance Detection via Western Blot (WB)

WB is a classical biochemical experimental method used to detect the abundance of a target protein in a sample of cells or biological tissue. It relies on specific antigen-antibody binding to detect protein signals from the sample processed by gel electrophoresis: the protein specifically binds to a primary antibody at an epitope; the primary antibody is then conjugated with a secondary antibody to amplify the signal cascade; the signal is read out by chemiluminescent or fluorescent detection. The intracellular protein content is ultimately characterized by the intensity of the optical signals. PROTAC compound molecules induce target protein degradation in a concentration/dose-dependent and timepoint-dependent manner. The DC50 value can be calculated by curve fitting of the protein WB exposure signals.

The experimental procedure is as follows:
1. Seed Jurkat cells into a 12-well plate at 1×10⁶ cells/500 µL/well.
2. Mix the culture medium with the compound to a concentration that is twice the desired final concentration. Add 0.5 mL of this mixture to the 12-well plate, mix well, and incubate the plate in an incubator for 24 h.
3. Transfer the cell suspension to a centrifuge tube. Rinse the 12-well plate with PBS and then transfer the PBS from the plate to the same centrifuge tube. Centrifuge at 3000 rpm for 5 min at room temperature. Aspirate the supernatant using a vacuum pump. Add 90 µL of pre-cooled cell lysis buffer containing protease inhibitor (Complete Protease Inhibitor Cocktail Tablets, Roche #4693116001) and phosphatase inhibitor (Phosphatase Inhibitor Cocktail Tablets supplied in EASYpacks, Roche #4906837001) to the centrifuge tube according to the manufacturer's instructions. Lyse on ice for 20 min.
4. Centrifuge the lysed sample at 13000 rpm for 20 min at 4°C. Mix the supernatant with 5× protein loading buffer. Boil at 100°C for 5 min and then cool. Store the denatured protein sample at -20°C.
5. Load 12 µL of the protein sample into each well of 4-12% Bis-Tris Midi Gel 26-well (invitrogen #WG1403BOX). Run electrophoresis at 80 V for 20 min, and then at 120 V constant voltage for 60 min.
6. Transfer the protein onto a nitrocellulose membrane in an ice bath at 250 mA constant current for 90 min.
7. Block the membrane with protein blocking buffer (LI-COR #927-50000) for 1 h at room temperature.
8. Wash the membrane with TBST (CST #9997S) at room temperature 3 times, 5 min each. Incubate with a primary antibody overnight at 4°C.
9. Wash the membrane with TBST (CST #9997S) at room temperature 3 times, 5 min each. Incubate with a secondary antibody for 1-2 h at room temperature.
10. Wash the membrane with TBST (CST #9997S) at room temperature 3 times. Expose and image the membrane using a LICOR Odyssey DLx imager.

### Biological Example 2: CTG Assay of Compound Inhibition on Cell Viability (HCC1806 Cells)

CellTiter-Glo (CTG) is a rapid cell viability test method based on ATP detection. It has the advantages of high sensitivity, rapidity and convenience, and is the gold standard for cell viability testing. Adenosine triphosphate (ATP) participates in various enzymatic reactions in organisms and is an indicator of living cell metabolism. Its content directly reflects the number of living cells and cell status. During the experiment, equal volume of CTG reagent is added to the cultured cells to measure the luminescence value. The chemiluminescent signal value is proportional to the amount of ATP, thus reflecting the number of viable cells. Therefore, cell viability can be obtained by detecting the ATP content. Compounds that affect cell viability will inhibit cell viability in a concentration-dependent manner, allowing a curve to be fitted to obtain the IC₅₀ value.

The experimental procedure is as follows:
11. Place the cell suspension in a 15 mL centrifuge tube, centrifuge at 1000 rpm for 5 min at room temperature, discard the supernatant, and collect the cell pellet.
12. Resuspend the cell pellet with preheated complete cell culture medium (RPMI1640+10%FBS+1%PS), and use a Countess^{®} automated cell counter to detect cell viability and density. The cell viability must be above 90%.
13. Seed HCC1806 cells in a 96-well plate (Corning #3925) at a density of 300 cells/100 µL/well.
14. Dissolve the compound in DMSO to prepare a 10 mM stock solution, transfer the stock solution to a dilution plate (Corning #3357) for 3-fold serial dilution at 8 concentration points. Transfer the compound solution to a 96-well plate seeded with cells (final concentration of DMSO 0.1%), and place in a cell incubator at 37°C, 5% CO₂ for 6 days.
15. Take out the 96-well plate, equilibrate at room temperature for 15 min, remove 100 µL of medium from each well, add 50 µL of CellTiter-Glo^{®} Luminescent Cell Via.Assay (Promega # G7573) reagent, shake and mix for 2 min, and allow to stand at room temperature for 25-30 min. Protect from light during the whole process. Perform optical signal readings using a SpectraMax i3x reader.
16. Data analysis: Remaining activity (%) = 100% * (1 - ((LumDMSO - Lummedium) - Lumsample) / (LumDMSO - LumPBS/medium)). Use GraphPad Prism 9.0 software to draw an S-shaped dose-survival rate curve and calculate the IC₅₀ value using a nonlinear regression model.

The AURKA degradation data of some degrader compounds in Jurkat cells measured in Biological Example 1 (percentage values of induced AURKA degradation at concentrations of 1 µM and 100 nM) and the cell viability inhibition data (IC₅₀ values) in HCC1806 cells measured in Biological Example 2 are listed in Table 1.

**Table 1.**

| Compound No. | Percentage of AURKA Degradation Induced by 1 µM Compound in Jurkat Cells | Percentage of AURKA Degradation Induced by 100 nM Compound in Jurkat Cells | IC₅₀ in HCC1806 Cells |
|---|---|---|---|
| alisertib | + | + | A |
| A1 | ++ | ++ | A |
| A2 | ++ | ++ | A |
| A4 | | | C |
| A5 | ++ | ++ | A |
| A6 | + | ++ | |
| A7 | ++ | ++ | B |
| A8 | +++ | +++ | A |
| A9 | + | ++ | A |
| A10 | ++ | ++ | A |
| A11 | ++ | +++ | A |
| A12 | + | + | A |
| A13 | + | ++ | A |
| A14 | + | + | A |
| A15 | +++ | +++ | A |
| A16 | ++ | +++ | A |
| A17 | + | + | A |
| A18 | + | ++ | A |
| A19 | + | ++ | A |
| A20 | ++ | ++ | |
| A21 | +++ | +++ | A |
| A22 | +++ | +++ | A |
| A23 | + | + | A |
| A24 | ++ | +++ | A |
| A25 | ++ | +++ | A |
| A26 | + | + | A |
| A27 | + | + | B |
| A28 | + | + | A |
| A29 | +++ | +++ | A |
| A30 | ++ | ++ | A |
| A31 | ++ | ++ | |
| A32 | + | + | C |
| A33 | ++ | ++ | A |
| A34 | ++ | ++ | A |
| A35 | + | ++ | A |
| A36 | + | + | A |
| A37 | +++ | +++ | A |
| A38 | +++ | +++ | A |
| A39 | + | ++ | A |
| A40 | + | ++ | A |
| A41 | + | + | A |
| A42 | + | + | C |
| A43 | + | + | C |
| A44 | + | + | A |
| A45 | ++ | ++ | A |
| A46 | +++ | +++ | A |
| A47 | + | ++ | A |
| A48 | + | + | A |
| A49 | + | + | A |
| A50 | +++ | +++ | A |
| A51 | +++ | ++ | B |
| A52 | ++ | ++ | A |
| A53 | ++ | ++ | |
| A54 | +++ | +++ | A |
| A55 | +++ | +++ | A |
| A56 | +++ | +++ | A |
| A57 | +++ | +++ | A |
| A58 | ++ | ++ | A |
| A59 | +++ | ++ | A |
| A60 | +++ | +++ | |
| A61 | +++ | +++ | A |
| A62 | ++ | ++ | |
| A63 | ++ | ++ | A |
| A64 | ++ | +++ | A |
| A65 | ++ | +++ | A |
| A66 | + | + | A |
| A67 | + | + | A |
| A68 | +++ | +++ | A |
| A69 | +++ | +++ | A |
| A70 | +++ | +++ | A |
| A71 | +++ | +++ | A |
| A72 | +++ | +++ | A |
| A73 | +++ | +++ | A |
| A74 | ++ | ++ | A |
| A75 | ++ | ++ | B |
| A76 | ++ | ++ | B |
| A77 | ++ | ++ | B |
| A78 | +++ | ++ | C |
| A79 | +++ | ++ | B |
| A80 | +++ | ++ | A |
| A81 | +++ | +++ | A |

+: Degradation ≤ 30%; ++: 30% < Degradation ≤ 70%; +++: Degradation > 70%; A: IC₅₀ < 1000 nM; B: 1000 nM ≤ IC_{5 0}< 10000 nM; C: IC₅₀ ≥ 10000 nM. Unlisted indicates not tested.

### Biological Example 3: CTG Assay of Compound Inhibition on Cell Viability (H82 Cells)

Except that the number of cells plated was 4000/well, other operations are the same as the method described in Biological Example 2. The AURKA degradation data of some degrader compounds in Jurkat cells measured in Biological Example 1 (percentage values of induced AURKA degradation at 1 µM and 100 nM concentrations) and the cell viability inhibition data (IC₅₀ values) in H82 cells measured in Biological Example 3 are listed in Table 2.

**Table 2**

| Compound No. | Percentage of AURKA Degradation Induced by 1 µM Compound in Jurkat Cells | Percentage of AURKA Degradation Induced by 100 nM Compound in Jurkat Cells | IC₅₀ in H82 Cells |
|---|---|---|---|
| A82 | + | + | |
| A84 | +++ | ++ | |
| A91 | + | + | |
| A92 | + | + | |
| A93 | +++ | ++ | |
| A94 | | | A |
| A95 | | | A |
| A96 | +++ | ++ | A |
| A97 | + | + | A |
| A98 | +++ | ++ | A |
| A99 | + | ++ | A |
| A100 | ++ | ++ | A |
| A101 | +++ | +++ | A |
| A102 | + | + | A |
| A103 | ++ | ++ | A |
| A104 | +++ | +++ | A |
| A105 | +++ | +++ | A |
| A106 | +++ | +++ | A |
| A107 | ++ | ++ | A |
| A108 | + | + | |
| A109 | ++ | ++ | A |
| A110 | ++ | ++ | A |
| A111 | + | + | |
| A112 | +++ | +++ | A |
| A113 | ++ | +++ | |
| A114 | +++ | +++ | |
| A115 | +++ | +++ | A |
| A116 | +++ | +++ | A |
| A117 | ++ | ++ | |
| A118 | +++ | ++ | A |
| A119 | +++ | +++ | |
| A120 | + | + | A |
| A121 | ++ | +++ | A |
| A122 | +++ | +++ | A |
| A123 | + | + | |
| A124 | + | + | A |
| A125 | +++ | ++ | A |
| A126 | + | + | A |
| A127 | +++ | +++ | A |
| A128 | +++ | +++ | A |
| A129 | +++ | +++ | A |
| A130 | ++ | ++ | A |
| A131 | +++ | +++ | A |
| A132 | +++ | +++ | A |
| A133 | +++ | ++ | A |
| A134 | ++ | +++ | A |
| A135 | ++ | +++ | A |
| A136 | +++ | +++ | A |
| A137 | +++ | +++ | A |
| A138 | +++ | +++ | B |
| A139 | +++ | +++ | |
| A140 | +++ | +++ | A |
| A141 | +++ | +++ | A |
| A142 | +++ | +++ | A |
| A143 | +++ | +++ | A |
| A144 | + | + | A |
| A145 | ++ | ++ | A |
| A146 | + | ++ | A |
| A147 | +++ | +++ | A |
| A148 | +++ | +++ | A |
| A149 | +++ | +++ | A |
| A150 | +++ | +++ | A |
| A151 | + | ++ | A |
| A152 | ++ | +++ | A |
| A153 | +++ | +++ | A |
| A154 | ++ | +++ | |
| A155 | ++ | ++ | A |
| A156 | + | + | B |
| A157 | + | ++ | A |
| A158 | + | ++ | A |
| A159 | +++ | +++ | |
| A160 | ++ | ++ | |
| A161 | ++ | + | |
| A162 | ++ | ++ | |
| A163 | + | ++ | |
| A164 | +++ | ++ | A |
| A166 | ++ | +++ | A |
| A168 | +++ | +++ | A |
| A170 | +++ | +++ | A |
| A172 | +++ | ++ | A |
| A174 | ++ | ++ | A |
| A176 | +++ | +++ | A |
| A178 | +++ | +++ | A |
| A179 | ++ | ++ | A |
| A181 | +++ | +++ | A |
| A183 | +++ | +++ | A |
| A185 | +++ | +++ | A |
| A187 | | | A |
| A189 | | | A |
| A193 | + | + | |
| A195 | +++ | +++ | A |
| A197 | +++ | +++ | A |
| A199 | +++ | +++ | A |
| A201 | ++ | ++ | |
| A203 | +++ | ++ | A |
| A204 | ++ | +++ | |
| A209 | +++ | +++ | A |
| A211 | +++ | ++ | A |
| A213 | +++ | +++ | A |
| A214 | + | +++ | A |
| A216 | ++ | ++ | A |
| A217 | + | + | A |
| A219 | +++ | +++ | A |
| A221 | + | + | A |
| A223 | +++ | +++ | A |
| A225 | + | +++ | A |
| A227 | + | ++ | A |
| A229 | +++ | +++ | A |
| A231 | +++ | +++ | A |
| A232 | +++ | +++ | A |
| A233 | +++ | ++ | A |
| A234 | + | ++ | A |
| A235 | + | ++ | A |
| A236 | ++ | +++ | A |
| A237 | +++ | +++ | A |
| A238 | +++ | +++ | A |
| A239 | +++ | +++ | A |
| A240 | +++ | +++ | A |
| A241 | ++ | +++ | |
| A242 | ++ | ++ | |
| A243 | +++ | +++ | A |
| A244 | +++ | ++ | A |
| A245 | +++ | +++ | A |
| A246 | ++ | + | |
| A247 | + | + | |
| A248 | +++ | +++ | A |
| A249 | +++ | +++ | A |
| A250 | +++ | +++ | A |
| A251 | +++ | +++ | A |
| A252 | +++ | +++ | A |
| A253 | +++ | +++ | A |
| A254 | ++ | +++ | A |
| A255 | ++ | ++ | A |
| A256 | +++ | +++ | A |
| A257 | +++ | +++ | A |
| A258 | +++ | +++ | A |
| A259 | +++ | +++ | A |
| A260 | ++ | ++ | |
| A261 | +++ | +++ | A |
| A262 | +++ | +++ | |
| A263 | +++ | +++ | A |
| A264 | +++ | +++ | A |
| A265 | + | + | A |
| A266 | ++ | ++ | |
| A267 | +++ | ++ | A |
| A268 | +++ | +++ | B |
| A269 | +++ | +++ | B |
| A270 | +++ | +++ | A |
| A271 | +++ | ++ | A |
| A272 | +++ | +++ | A |
| A273 | +++ | +++ | A |
| A274 | +++ | +++ | A |
| A275 | +++ | +++ | A |
| A276 | ++ | ++ | A |
| A277 | +++ | +++ | A |
| A278 | +++ | ++ | |
| A279 | +++ | +++ | A |
| A280 | +++ | +++ | A |
| A281 | +++ | +++ | A |
| A282 | +++ | +++ | A |
| A283 | +++ | +++ | A |
| A284 | ++ | +++ | A |
| A285 | +++ | +++ | A |
| A286 | ++ | ++ | A |
| A287 | + | + | A |
| A288 | + | + | A |
| A289 | +++ | +++ | |
| A290 | +++ | ++ | A |
| A291 | +++ | +++ | A |
| A292 | +++ | +++ | A |
| A293 | +++ | +++ | A |
| A294 | ++ | ++ | A |
| A295 | ++ | +++ | |
| A296 | +++ | +++ | A |
| A297 | +++ | +++ | A |
| A298 | +++ | +++ | A |
| A299 | +++ | +++ | A |
| A300 | +++ | +++ | A |
| A301 | +++ | +++ | A |
| A302 | +++ | +++ | A |
| A303 | +++ | +++ | A |
| A304 | +++ | +++ | A |
| A305 | +++ | +++ | A |
| A306 | +++ | ++ | A |
| A307 | +++ | +++ | A |
| A308 | +++ | +++ | A |
| A309 | ++ | ++ | A |
| A310 | +++ | +++ | A |
| A311 | +++ | +++ | A |
| A312 | | | A |
| A313 | | | A |
| A314 | | | A |
| A315 | | | A |
| A316 | ++ | ++ | A |
| A317 | | | A |
| A318 | | | A |
| A319 | +++ | +++ | A |
| A320 | +++ | +++ | A |
| A321 | +++ | +++ | A |
| A322 | +++ | +++ | A |
| A323 | +++ | +++ | A |
| A324 | +++ | +++ | A |
| A325 | +++ | +++ | A |
| A326 | +++ | +++ | A |
| A327 | +++ | +++ | A |
| A328 | ++ | ++ | |
| A329 | +++ | +++ | A |
| A330 | +++ | +++ | A |
| A331 | +++ | +++ | A |
| A332 | + | + | A |
| A333 | + | + | A |
| A334 | + | + | A |
| A335 | +++ | +++ | A |
| A336 | +++ | +++ | A |
| A337 | +++ | +++ | A |
| A338 | +++ | +++ | A |
| A339 | +++ | +++ | A |
| A340 | +++ | +++ | A |
| A341 | +++ | +++ | A |
| A342 | +++ | +++ | A |
| A343 | +++ | +++ | A |
| A344 | +++ | +++ | A |
| A345 | +++ | +++ | A |
| A346 | +++ | +++ | A |
| A347 | +++ | +++ | A |
| A348 | ++ | +++ | A |
| A349 | ++ | ++ | A |
| A350 | +++ | +++ | A |
| A351 | +++ | +++ | A |
| A352 | +++ | +++ | A |
| A353 | +++ | +++ | A |
| A354 | ++ | ++ | A |
| A355 | +++ | +++ | A |
| A356 | +++ | +++ | A |
| A357 | + | ++ | A |
| A358 | +++ | +++ | A |
| A359 | +++ | +++ | A |
| A360 | +++ | +++ | A |
| A361 | +++ | +++ | A |
| A362 | ++ | +++ | A |
| A363 | +++ | +++ | A |
| A364 | +++ | +++ | A |
| A365 | +++ | +++ | A |

+: Degradation ≤ 30%; ++: 30% < Degradation ≤ 70%; +++: Degradation > 70%; A: IC₅₀ < 1000 nM; B: 1000 nM ≤ IC₅₀ < 10000 nM; C: IC₅₀ ≥ 10000 nM. Unlisted indicates not tested.

### Biological Example 4: Mouse PK Study

Male CD1 mice (5-8 weeks old) were administered intravenously (IV) at a dose of 1 mg/kg by tail vein bolus or orally (PO) at doses of 10 mg/kg and 40 mg/kg by gavage. The compound was formulated in a vehicle of 10% DMSO + 10% Solutol HS15 + 80% saline. All animals were given free access to food and water during the experiment. Blood (n=3) was collected via the vein at 0.083, 0.25, 0.5, 1, 2, 4, 8 and 24 h after administration and placed in tubes containing EDTA-K2 as an anticoagulant. Blood samples were centrifuged at 3500 g for 5 min, and the resulting plasma was analyzed by an LC-MS/MS method. The PK parameters were calculated using WinNonlin software (Phoenix^{®}, version 8.3) through non-compartmental analysis methods. The results are listed in Table 3.

**Table 3**

| A307 | **IV** | **PO** | |
|---|---|---|---|
| | **1 mg/kg** | **10 mg/kg** | **40 mg/kg** |
| **C₀/Cₘₐₓ (ng/mL)** | 1527 | 2460 | 7283 |
| **t_{1/2} (h)** | 2.28 | 2.41 | 2.37 |
| **Tₘₐₓ (h)** | - | 2.00 | 3.33 |
| **AUC₀₋₂₄ (ng*h/mL)** | 2449 | 19525 | 78931 |
| **CLₚ (mL/min/kg)** | 6.33 | - | - |
| **Vₛₛ (L/kg)** | 1.04 | - | - |
| **F (%)** | - | 79.7 | 80.6 |

| **A321** | **IV** | **PO** | |
|---|---|---|---|
| | **1 mg/kg** | **10 mg/kg** | **40 mg/kg** |
| **C₀/Cₘₐₓ (ng/ml)** | 1307 | 4007 | 13233 |
| **t_{1/2} (h)** | 1.89 | 2.13 | 1.97 |
| **Tmax (h)** | - | 3.33 | 3.33 |
| **AUC₀₋₂₄ (ng*h/mL)** | 2629 | 41389 | 136773 |
| **CLₚ (mL/min/kg)** | 6.16 | - | - |
| **Vₛₛ (L/kg)** | 0.97 | - | - |
| **F (%)** | - | 157 | 130 |

Although the specific embodiments of the present invention are described above, those skilled in the art should understand that these are only examples and various changes or modifications can be made to these embodiments without departing from the principles and essence of the present invention. Therefore, the protection scope of the present invention is defined by the appended claims.

## Claims

1. A compound represented by formula (I), a pharmaceutically acceptable salt thereof, a solvate thereof, or a solvate of the pharmaceutically acceptable salt thereof: 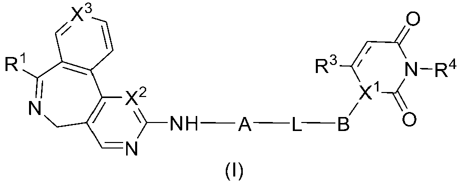 wherein,
" 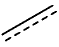 " represents a single bond or a double bond;
X¹ is CH, CD or N;
R³ is H, D, C₁-C₆ alkyl or optionally substituted C₁-C₆ alkyl;
R⁴ is H or C₁-C₆ alkyl;
X² is N, CH or CD;
X³ is CR² or N;
R² is H, D, cyano, halogen, -OR²⁻⁷, -P(=O)(R²⁻¹)₂, -S(=O)₂R²⁻², optionally substituted C₁-C₆ alkyl, optionally substituted C₁-C₆ alkoxy, optionally substituted cycloalkyl or optionally substituted heterocycloalkyl;
R²⁻¹ and R²⁻² are each independently optionally substituted C₁-C₆ alkyl;
R²⁻⁷ is optionally substituted cycloalkyl or optionally substituted heterocycloalkyl;
R¹ is optionally substituted aryl, optionally substituted heteroaryl, optionally substituted cycloalkyl or optionally substituted heterocycloalkyl;
R¹⁻¹ and R¹⁻² are each independently halogen, optionally substituted C₁-C₆ alkyl or optionally substituted C₁-C₆ alkoxy;
A is -R^{A1}C(=O)-, -R^{A2}CH₂-, -R^{A2}CD₂-, -R^{A2}CHD-, optionally substituted arylene, optionally substituted heteroarylene, optionally substituted cycloalkylene or optionally substituted heterocycloalkylene;
R^{A1} and R^{A2} are each independently optionally substituted aryl, optionally substituted heteroarylene, optionally substituted cycloalkylene or optionally substituted heterocycloalkylene;
L is a linker;
B is optionally substituted arylene, optionally substituted heteroarylene, optionally substituted cycloalkylene or optionally substituted heterocycloalkylene;
and the compound represented by formula (I) is not any one of the following compounds:

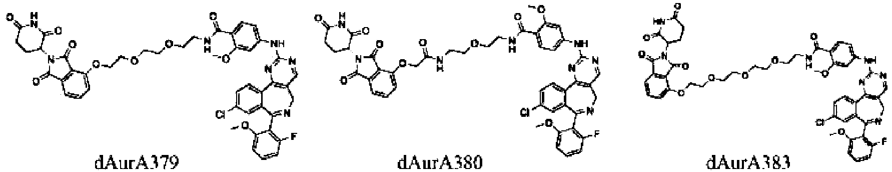

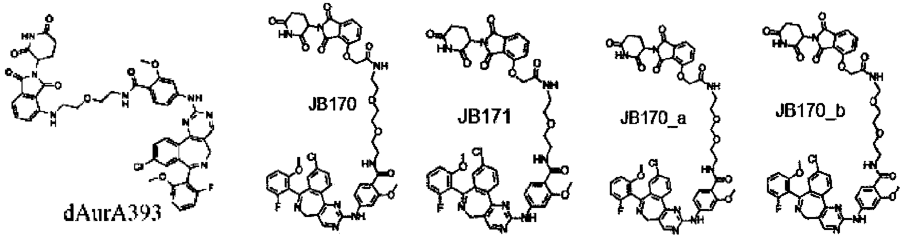

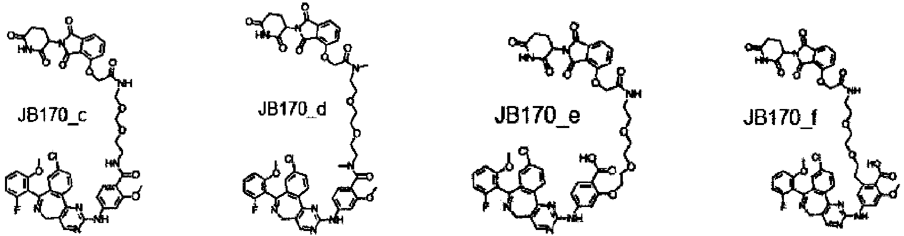

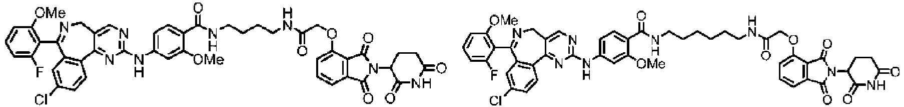

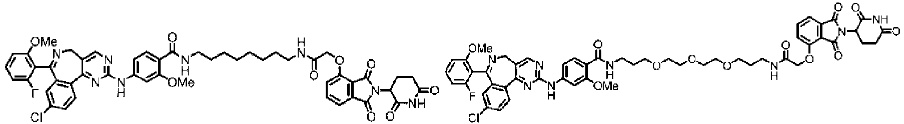

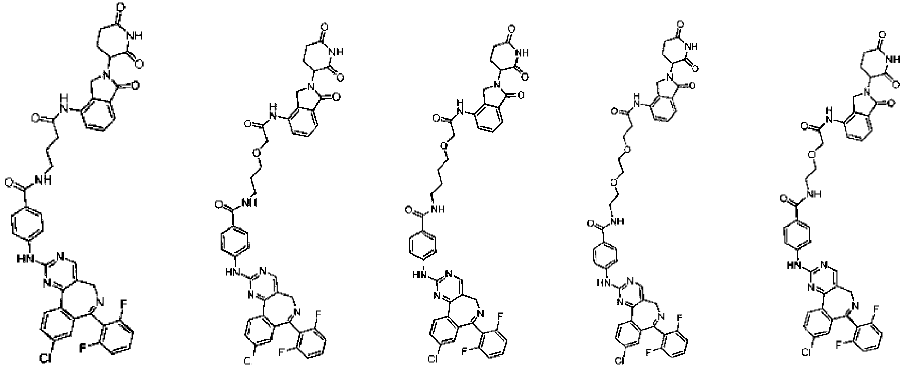

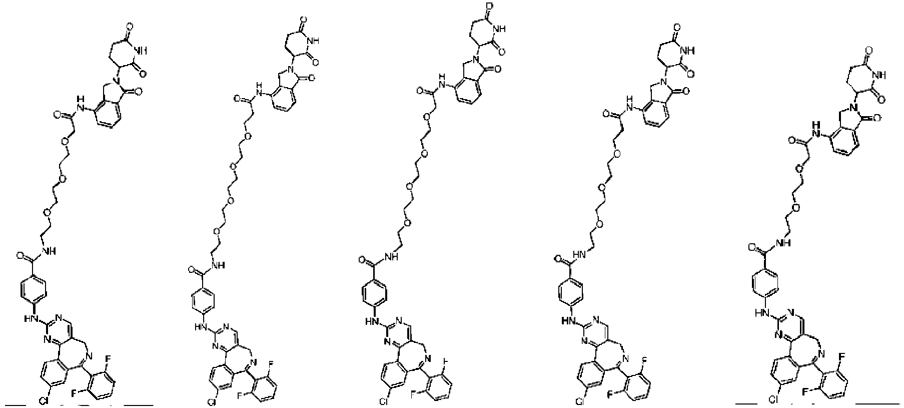

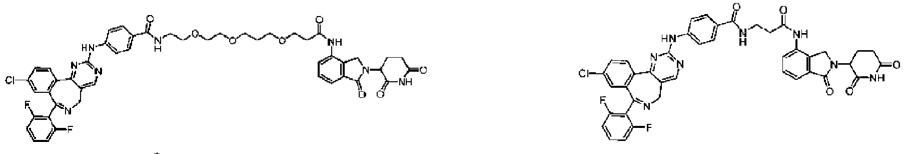

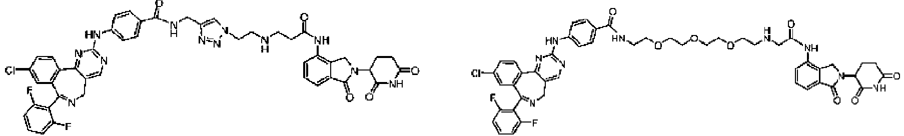

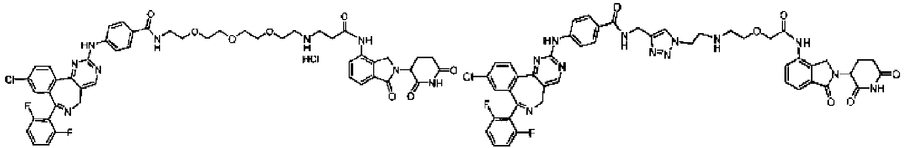

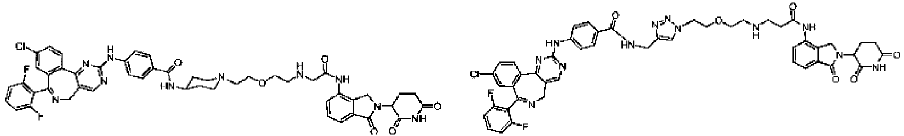

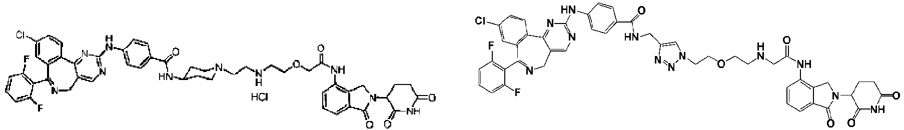

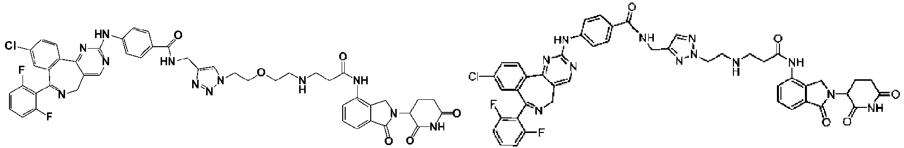

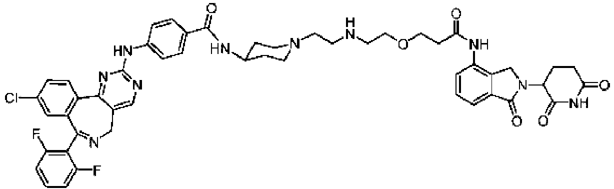

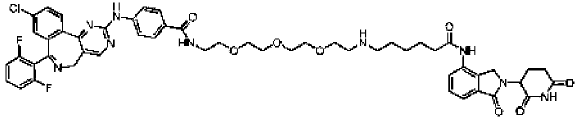

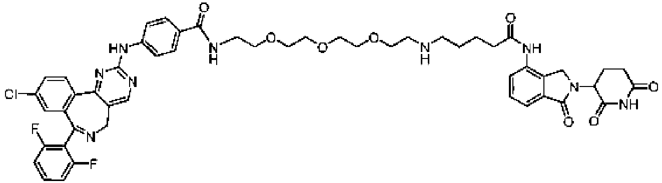

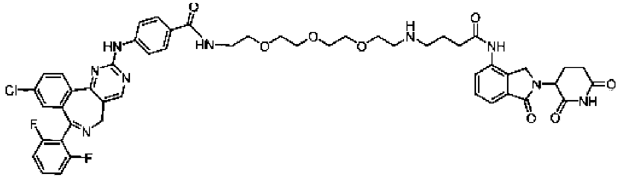

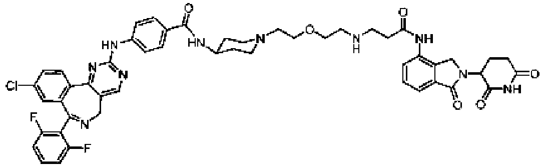

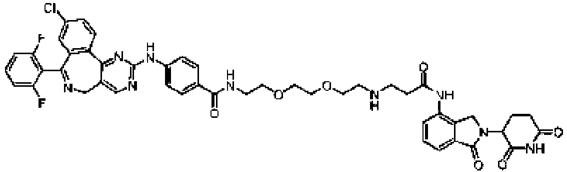

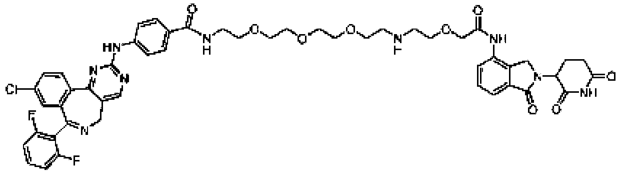

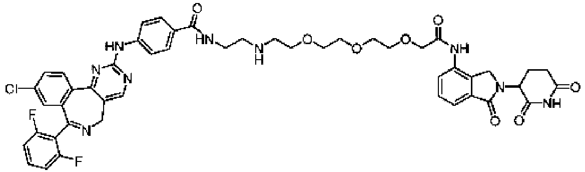

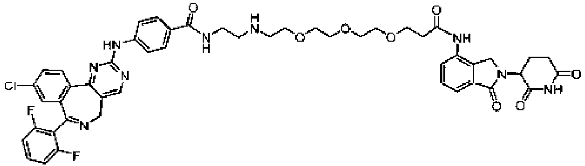

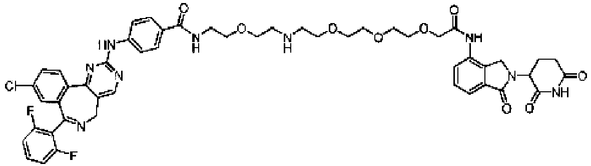

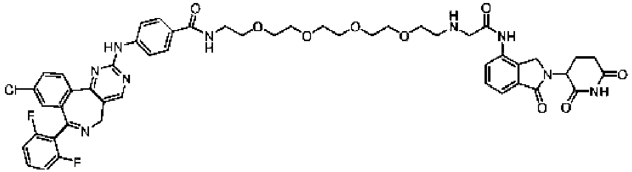

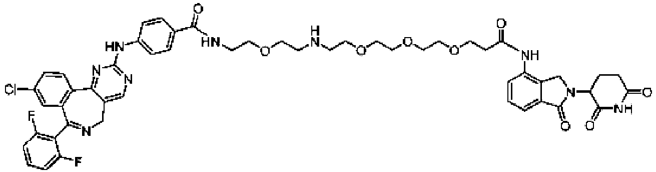

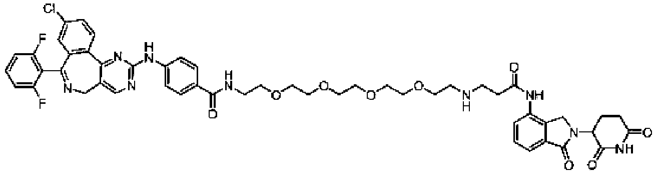

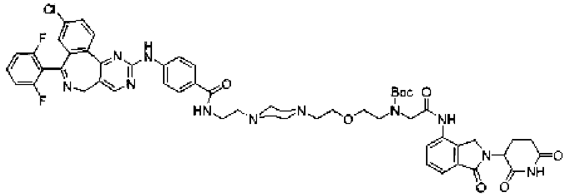

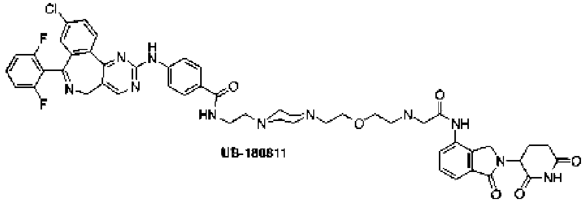

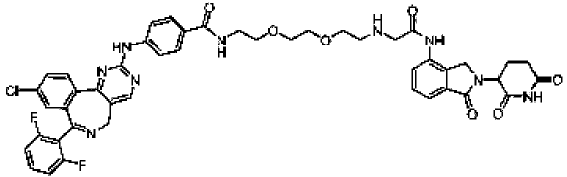

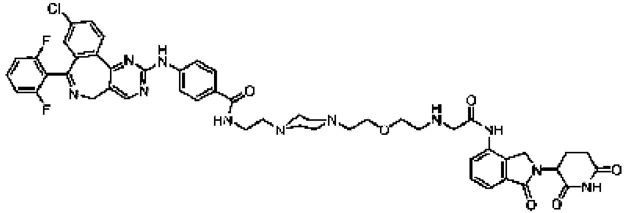

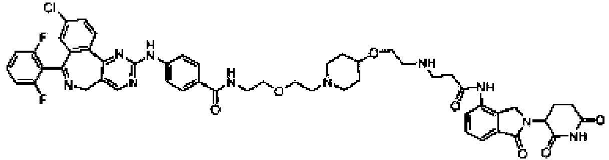

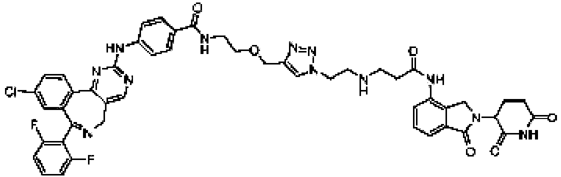

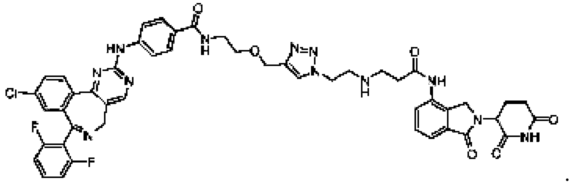

2. The compound represented by formula (I), the pharmaceutically acceptable salt thereof, the solvate thereof or the solvate of the pharmaceutically acceptable salt thereof according to claim 1, wherein,
" 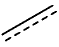 " represents a single bond or a double bond;
X¹ is CH, CD or N;
R³ is H, D, C₁-C₆ alkyl or optionally substituted C₁-C₆ alkyl;
R⁴ is H;
X² is N, CH or CD;
X³ is CR² or N;
R² is H, D, cyano, halogen, -OR²⁻⁷, -P(=O)(R²⁻¹)₂, -S(=O)₂R²⁻², optionally substituted C₁-C₆ alkyl, optionally substituted C₁-C₆ alkoxy, optionally substituted cycloalkyl or optionally substituted heterocycloalkyl;
R²⁻¹ and R²⁻² are each independently optionally substituted C₁-C₆ alkyl;
R²⁻⁷ is optionally substituted cycloalkyl or optionally substituted heterocycloalkyl;
R¹ is optionally substituted aryl, optionally substituted heteroaryl, optionally substituted cycloalkyl or optionally substituted heterocycloalkyl;
R¹⁻¹ and R¹⁻² are each independently halogen, optionally substituted C₁-C₆ alkyl or optionally substituted C₁-C₆ alkoxy;
A is -R^{A1}C(=O)-, -R^{A2}CH₂-, -R^{A2}CD₂-, -R^{A2}CHD-, optionally substituted arylene, optionally substituted heteroarylene, optionally substituted cycloalkylene or optionally substituted heterocycloalkylene;
R^{A1} and R^{A2} are each independently optionally substituted aryl, optionally substituted heteroarylene, optionally substituted cycloalkylene or optionally substituted heterocycloalkylene;
L is a linker;
B is optionally substituted arylene, optionally substituted heteroarylene, optionally substituted cycloalkylene or optionally substituted heterocycloalkylene;
and the compound represented by formula (I) is not any one of the following compounds:

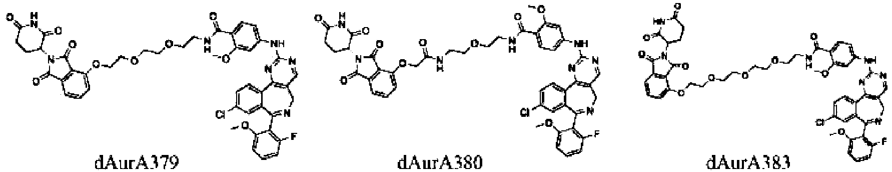

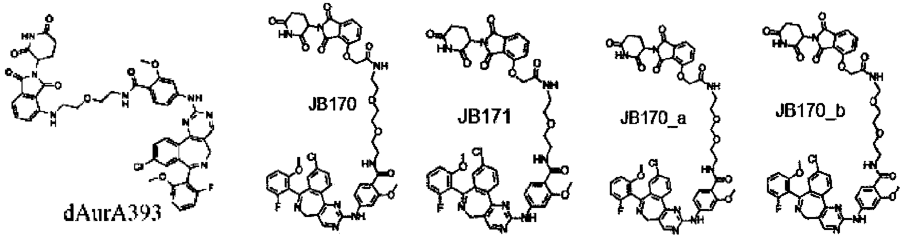

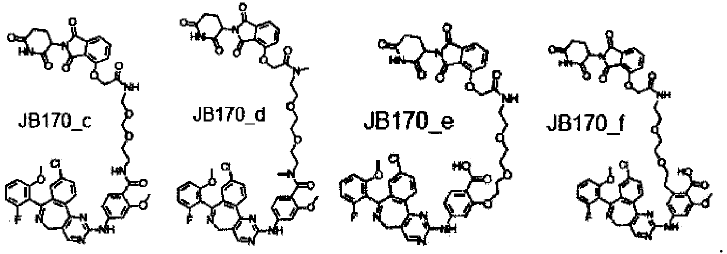

3. The compound represented by formula (I), the pharmaceutically acceptable salt thereof, the solvate thereof or the solvate of the pharmaceutically acceptable salt thereof according to claim 1, wherein
" 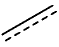 " represents a single bond or a double bond;
X¹ is CH, CD or N;
R³ is H, D, C₁-C₆ alkyl or optionally substituted C₁-C₆ alkyl;
R⁴ is H;
X² is N, CH or CD;
X³ is CR² or N;
R² is H, D, halogen, -OR²⁻⁷, -P(=O)(R²⁻¹)₂, -S(=O)₂R²⁻², optionally substituted C₁-C₆ alkyl, optionally substituted C1-C₆ alkoxy, optionally substituted cycloalkyl or optionally substituted heterocycloalkyl;
R²⁻¹ and R²⁻² are each independently optionally substituted C₁-C₆ alkyl;
R²⁻⁷ is optionally substituted cycloalkyl or optionally substituted heterocycloalkyl;
R¹ is optionally substituted aryl, optionally substituted heteroaryl, optionally substituted cycloalkyl or optionally substituted heterocycloalkyl;
R¹⁻¹ and R¹⁻² are each independently halogen, optionally substituted C₁-C₆ alkyl or optionally substituted C₁-C₆ alkoxy;
A is -R^{A1}C(=O)-, -R^{A2}CH₂-, -R^{A2}CD₂-, -R^{A2}CHD-, optionally substituted arylene, optionally substituted heteroarylene, optionally substituted cycloalkylene or optionally substituted heterocycloalkylene;
R^{A1} and R^{A2} are each independently optionally substituted aryl, optionally substituted heteroarylene, optionally substituted cycloalkylene or optionally substituted heterocycloalkylene;
L is a linker;
B is optionally substituted arylene, optionally substituted heteroarylene, optionally substituted cycloalkylene or optionally substituted heterocycloalkylene;
and the compound represented by formula (I) is not any one of the following compounds:

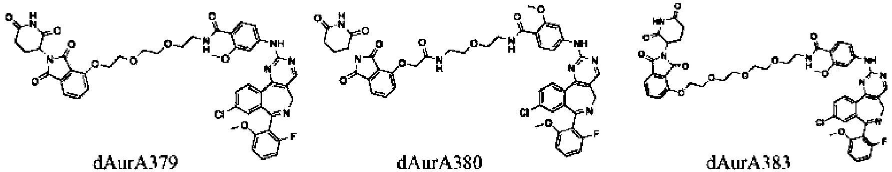

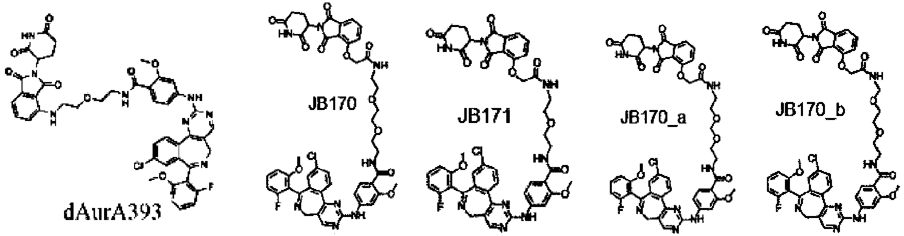

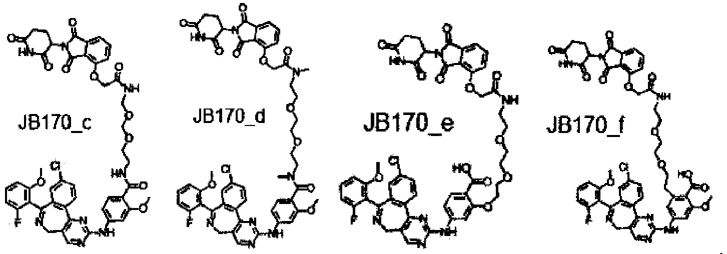

4. The compound represented by formula (I), the pharmaceutically acceptable salt thereof, the solvate thereof or the solvate of the pharmaceutically acceptable salt thereof according to claim 1, wherein
" 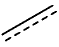 " represents a single bond or a double bond;
X¹ is CH or N;
R³ is H or C₁-C₆ alkyl;
R⁴ is H or C₁-C₆ alkyl;
X² is N or CH;
X³ is CR² or N;
R² is H, cyano, halogen, C₁-C₆ alkyl, C₁-C₆ alkoxy, saturated C₃-C₁₁ cycloalkyl, saturated 3- to 11-membered heterocycloalkyl, -P(=O)(R²⁻¹)₂, -S(=O)₂R²⁻², -OR²⁻⁷, C₁-C₆ alkyl substituted with one or more R²⁻³, C₁-C₆ alkoxy substituted with one or more R²⁻⁴, saturated C₃-C₁₁ cycloalkyl substituted with one or more R²⁻⁵ or saturated 3- to 11-membered heterocycloalkyl substituted with one or more R²⁻⁶, wherein the saturated 3- to 11-membered heterocycloalkyl and the 3- to 11-membered heterocycloalkyl in the saturated 3- to 11-membered heterocycloalkyl substituted with one or more R²⁻⁶ each independently have 1, 2 or 3 heteroatoms selected from one or more of N, O, S and C(=O);
R²⁻¹ and R²⁻² are each independently C₁-C₆ alkyl;
R²⁻⁷ is saturated C₃-C₁₁ cycloalkyl;
R²⁻³ and R²⁻⁴ are each independently -N(R²⁻³⁻¹)₂, saturated C₃-C₁₁ cycloalkyl, saturated 3- to 11-membered heterocycloalkyl, saturated C₃-C₁₁ cycloalkyl substituted with one or more R²⁻³⁻², or saturated 3- to 11-membered heterocycloalkyl substituted with one or more R²⁻³⁻³, wherein the saturated 3- to 11-membered heterocycloalkyl and the 3- to 11-membered heterocycloalkyl in the saturated 3- to 11-membered heterocycloalkyl substituted with one or more R²⁻³⁻³ each independently have 1, 2 or 3 heteroatoms selected from one or more of N, O, S and C(=O);
R²⁻³⁻¹ is independently H or C₁-C₆ alkyl;
R²⁻³⁻² and R²⁻³⁻³ are each independently C₁-C₆ alkyl;
R²⁻⁵ and R²⁻⁶ are each independently C₁-C₆ alkyl;
R¹ is C₆-C₁₀ aryl, 5- to 10-membered heteroaryl, saturated C₃-C₁₁ cycloalkyl, saturated 3- to 11-membered heterocycloalkyl, C₆-C₁₀ aryl substituted with one or more R¹⁻¹, 5- to 10-membered heteroaryl substituted with one or more R¹⁻², saturated C₃-C₁₁ cycloalkyl substituted with one or more R¹⁻³, or saturated 3- to 11-membered heterocycloalkyl substituted with one or more R¹⁻⁴, wherein the saturated 3- to 11-membered heterocycloalkyl has 1, 2 or 3 heteroatoms selected from one or more of N, O, S and C(=O), and the 5- to 10-membered heteroaryl and the 5- to 10-membered heteroaryl in the 5- to 10-membered heteroaryl substituted with one or more R¹⁻² each independently have 1, 2 or 3 heteroatoms selected from one or more of N, O and S;
R¹⁻¹, R¹⁻², R¹⁻³ and R¹⁻⁴ are each independently halogen, C₁-C₆ alkyl or C₁-C₆ alkoxy;
A is -R^{A1}C(=O)-, -R^{A2}CH₂-, C₆-C₁₀ arylene, 5- to 10-membered heteroarylene, saturated C₃-C₁₁ cycloalkylene, saturated 3- to 11-membered heterocycloalkylene, 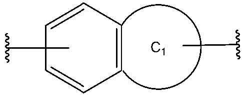 , C₆-C₁₀ arylene substituted with one or more R^{A3}, or 5- to 10-membered heteroarylene substituted with one or more R^{A4}, wherein the saturated 3- to 11-membered heterocycloalkylene has 1, 2 or 3 heteroatoms selected from one or more of N, O, S and C(=O) and the 5- to 10-membered heteroarylene and the 5- to 10-membered heteroarylene in the 5-to 10-membered heteroarylene substituted with one or more R^{A4} each independently have 1, 2 or 3 heteroatoms selected from one or more of N, O and S;
R^{A1} and R^{A2} are each independently C₆-C₁₀ arylene, 5- to 10-membered heteroarylene, saturated C₃-C₁₁ cycloalkylene, saturated 3- to 11-membered heterocycloalkylene, C₆-C₁₀ arylene substituted with one or more R^{A1-1}, or 5- to 10-membered heteroarylene substituted with one or more R^{A1-2}, wherein the 5- to 10-membered heteroarylene and the 5- to 10-membered heteroarylene in the 5- to 10-membered heteroarylene substituted with one or more R^{A1-2} each independently have 1, 2 or 3 heteroatoms selected from one or more of N, O and S, and the 3- to 11-membered heterocycloalkylene has 1, 2 or 3 heteroatoms selected from one or more of N, O, S and C(=O);
R^{A1-1} and R^{A1-2} are each independently CN, -P(=O)(R^{A3-1})₂, C₁-C₆ alkyl or C₁-C₆ alkoxy;
ring C₁ is a partially unsaturated 3- to 8-membered heterocycle or a partially unsaturated 3- to 8-membered heterocycle substituted with one or more R^{C1}, wherein the partially unsaturated 3- to 8-membered heterocycle and the 3- to 8-membered heterocycle in the partially unsaturated 3- to 8-membered heterocycle substituted with one or more R^{C1} each independently have 1, 2 or 3 heteroatoms selected from one or more of N, O, S and C(=O);
R^{C1} is independently C₁-C₆ alkyl;
R^{A3} and R^{A4} are each independently CN, -P(=O)(R^{A3-1})₂, C₁-C₆ alkyl or C₁-C₆ alkoxy;
R^{A3-1} is independently C₁-C₆ alkyl;
L is -(CH₂)ₙ-, wherein n is any integer from 1 to 18, and 1 or more of the -(CH₂)- in -(CH₂)ₙ- are replaced by one or more of the following groups: -NR^{L1}-, -O-, -S-, -CR^{L6}R^{L7}-, -C(=O)-, 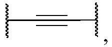 saturated C₃-C₁₂ cycloalkylene, saturated 3- to 12-membered heterocycloalkylene, C₆-C₁₀ arylene, 5- to 10-membered heteroarylene, 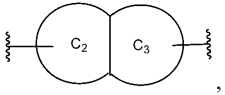 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4} or 5- to 10-membered heteroarylene substituted with one or more R^{L5}, wherein the saturated 3- to 12-membered heterocycloalkylene and the 3- to 12-membered heterocycloalkyl in the saturated 3- to 12-membered heterocycloalkylene substituted with one or more R^{L3} each independently have 1, 2 or 3 heteroatoms selected from one or more of N, O, S and C(=O) and the 5- to 10-membered heteroarylene and the 5- to 10-membered heteroarylene in the 5- to 10-membered heteroarylene substituted with one or more R^{L5} each independently have 1, 2 or 3 heteroatoms selected from one or more of N, O and S;
R^{L1} is H or C₁-C₆ alkyl;
ring C₂ is benzene ring or 5- to 6-membered heteroaromatic ring, wherein the 5- to 6-membered heteroaromatic ring has 1, 2 or 3 heteroatoms selected from one or more of N, O and S;
ring C₃ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
ring C₄ is a saturated C₃-C₇ carbon ring or a saturated 3- to 7-membered heterocycle, wherein the saturated 3-to 7-membered heterocycle has 1, 2 or 3 heteroatoms selected from one or more of N, O, S and C(=O);
R^{L2} and R^{L3} are each independently CN, OH, halogen, C₁-C₆ alkyl, C₃-C₇ cycloalkyl or 3- to 7-membered heterocycloalkyl;
R^{L4} and R^{L5} are each independently C₁-C₆ alkyl;
R^{L6} is H or C₁-C₆ alkyl;
R^{L7} is C₁-C₆ alkyl;
B is 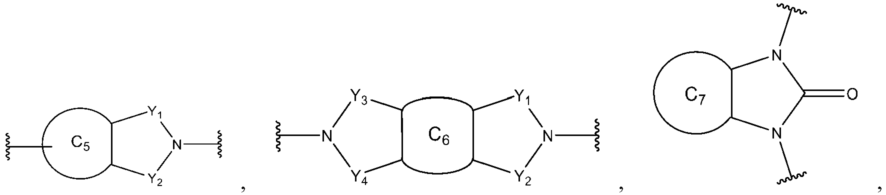 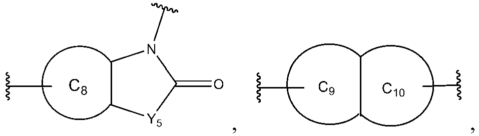 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1}, or 5- to 10-membered heteroarylene substituted with one or more R^{B2}, wherein the 5-to 10-membered heteroarylene and the 5- to 10-membered heteroarylene in the 5- to 10-membered heteroarylene substituted with one or more R^{B2} each independently have 1, 2 or 3 heteroatoms selected from one or more of N, O and S, and the saturated 3- to 12-membered heterocycloalkylene independently has 1, 2 or 3 heteroatoms selected from one or more of N, O, S and C(=O);
Y₁. Y₂, Y₃ and Y₄ are each independently CH₂ or C(=O);
Y₅ is NY⁵⁻¹, O or S, and Y⁵⁻¹ is H or C₁-C₆ alkyl;
ring C₅, ring C₆, ring C₇, ring C₈ and ring C₉ are each independently a benzene ring or a 5- to 6-membered heteroaromatic ring, wherein the 5- to 6-membered heteroaromatic ring has 1, 2 or 3 heteroatoms selected from one or more of N, O and S;
ring C₁₀ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
R^{B1} and R^{B2} are each independently halogen, cyano, -P=O(C₁-C₆ alkyl)₂, -C(=O)NH₂, C₁-C₆ alkyl, C₁-C₆ alkoxy, C₁-C₆ alkyl substituted with one or more R^{B1-1}, or C₁-C₆ alkoxy substituted with one or more R^{B1-2};
R^{B1-1} and R^{B1-2} are each independently halogen;
moreover, the compound represented by formula (I) satisfies one or both of the following conditions:
i: B is 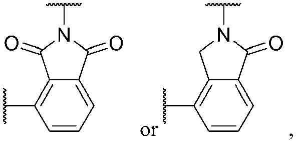 L is -(CH₂)ₙ-, wherein n is any integer from 1 to 18, and at least one -(CH₂)- in -(CH₂)ₙ- is replaced by the following groups: saturated C₃-C₁₂ cycloalkylene, saturated 3- to 5-membered heterocycloalkylene, saturated 7- to 12-membered heterocycloalkylene, C₆-C₁₀ arylene, 6-to 10-membered heteroarylene, 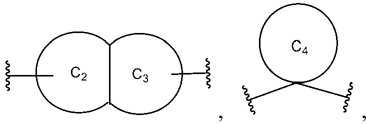 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4}, or 5- to 10-membered heteroarylene substituted with one or more R^{L5};
alternatively, at least two -(CH₂)- in -(CH₂)ₙ- are replaced by the following groups: saturated C₃-C₁₂ cycloalkylene, saturated 3- to 12-membered heterocycloalkylene, C₆-C₁₀ arylene, 5- to 10-membered heteroarylene, 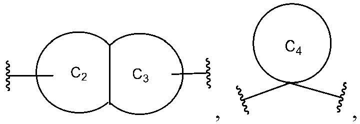 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4}, or 5- to 10-membered heteroarylene substituted with one or more R^{L5};
ii: B is 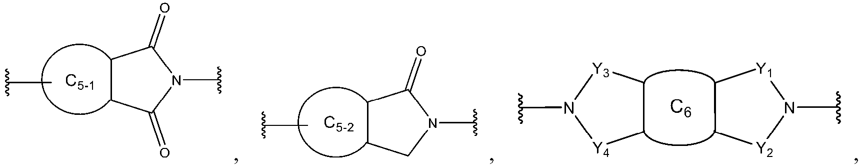 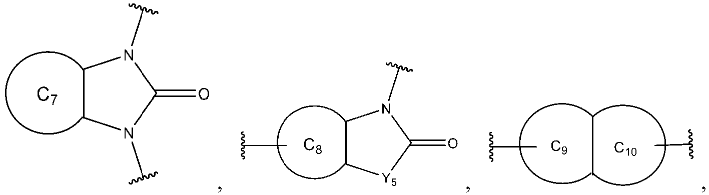 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1} or 5- to 10-membered heteroarylene substituted with one or more R^{B2}, ring C₅₋₁ same as the 5- to 6-membered heteroaromatic ring in ring C₅, and ring C₅₋₂ same as the 5-to 6-membered heteroaromatic ring in ring C₅.

5. The compound represented by formula (I), the pharmaceutically acceptable salt thereof, the solvate thereof or the solvate of the pharmaceutically acceptable salt thereof according to claim 1 or 2, wherein, 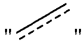 represents a single bond or a double bond;
X¹ is CH or N;
R³ is H or C₁-C₆ alkyl;
R⁴ is H;
X² is N or CH;
X³ is CR² or N;
R² is H, cyano, halogen, C₁-C₆ alkyl, C₁-C₆ alkoxy, saturated C₃-C₁₁ cycloalkyl, saturated 3- to 11-membered heterocycloalkyl, -P(=O)(R²⁻¹)₂, -S(=O)₂R²⁻², -OR²⁻⁷, C₁-C₆ alkyl substituted with one or more R²⁻³, C₁-C₆ alkoxy substituted with one or more R²⁻⁴, saturated C₃-C₁₁ cycloalkyl substituted with one or more R²⁻⁵ or saturated 3- to 11-membered heterocycloalkyl substituted with one or more R²⁻⁶, wherein the saturated 3- to 11-membered heterocycloalkyl and the 3- to 11-membered heterocycloalkyl in the saturated 3- to 11-membered heterocycloalkyl substituted with one or more R²⁻⁶ each independently have 1, 2 or 3 heteroatoms selected from one or more of N, O, S and C(=O);
R²⁻¹ and R²⁻² are each independently C₁-C₆ alkyl;
R²⁻⁷ is saturated C₃-C₁₁ cycloalkyl;
R²⁻³ and R²⁻⁴ are each independently -N(R²⁻³⁻¹)₂, saturated C₃-C₁₁ cycloalkyl, saturated 3- to 11-membered heterocycloalkyl, saturated C₃-C₁₁ cycloalkyl substituted with one or more R²⁻³⁻², or saturated 3- to 11-membered heterocycloalkyl substituted with one or more R²⁻³⁻³, wherein the saturated 3- to 11-membered heterocycloalkyl and the 3- to 11-membered heterocycloalkyl in the saturated 3- to 11-membered heterocycloalkyl substituted with one or more R²⁻³⁻³ each independently have 1, 2 or 3 heteroatoms selected from one or more of N, O, S and C(=O);
R²⁻³⁻¹ is independently H or C₁-C₆ alkyl;
R²⁻³⁻² and R²⁻³⁻³ are each independently C₁-C₆ alkyl;
R²⁻⁵ and R²⁻⁶ are each independently C₁-C₆ alkyl;
R¹ is C₆-C₁₀ aryl, 5- to 10-membered heteroaryl, saturated C₃-C₁₁ cycloalkyl, saturated 3- to 11-membered heterocycloalkyl, C₆-C₁₀ aryl substituted with one or more R¹⁻¹, or 5- to 10-membered heteroaryl substituted with one or more R¹⁻², wherein the saturated 3- to 11-membered heterocycloalkyl has 1, 2 or 3 heteroatoms selected from one or more of N, O, S and C(=O), and the 5- to 10-membered heteroaryl and the 5- to 10-membered heteroaryl in the 5- to 10-membered heteroaryl substituted with one or more R¹⁻² each independently have 1, 2 or 3 heteroatoms independently selected from one or more of N, O and S;
R¹⁻¹ and R¹⁻² are each independently halogen, C₁-C₆ alkyl or C₁-C₆ alkoxy;
A is -R^{A1}C(=O)-, -R^{A2}CH₂-, C₆-C₁₀ arylene, 5- to 10-membered heteroarylene, saturated C₃-C₁₁ cycloalkylene, saturated 3- to 11-membered heterocycloalkylene, 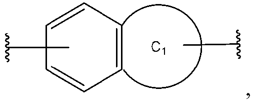 C₆-C₁₀ arylene substituted with one or more R^{A3}, or 5- to 10-membered heteroarylene substituted with one or more R^{A4}, wherein the saturated 3- to 11-membered heterocycloalkylene has 1, 2 or 3 heteroatoms selected from one or more of N, O, S and C(=O) and the 5- to 10-membered heteroarylene and the 5- to 10-membered heteroarylene in the 5-to 10-membered heteroarylene substituted with one or more R^{A4} each independently have 1, 2 or 3 heteroatoms independently selected from one or more of N, O and S;
R^{A1} and R^{A2} are each independently C₆-C₁₀ arylene, 5- to 10-membered heteroarylene, saturated C₃-C₁₁ cycloalkylene, saturated 3- to 11-membered heterocycloalkylene, C₆-C₁₀ arylene substituted with one or more R^{A1-1}, or 5- to 10-membered heteroarylene substituted with one or more R^{A1-2}, wherein the 5- to 10-membered heteroarylene and the 5- to 10-membered heteroarylene in the 5- to 10-membered heteroarylene substituted with one or more R^{A1-2} each independently have 1, 2 or 3 heteroatoms selected from one or more of N, O and S, and the 3- to 11-membered heterocycloalkylene has 1, 2 or 3 heteroatoms selected from one or more of N, O, S and C(=O);
R^{A1-1} and R^{A1-2} are each independently CN, -P(=O)(R^{A3-1})₂, C₁-C₆ alkyl or C₁-C₆ alkoxy;
ring C₁ is a partially unsaturated 3- to 8-membered heterocycle or a partially unsaturated 3- to 8-membered heterocycle substituted with one or more R^{C1}, wherein the partially unsaturated 3- to 8-membered heterocycle and the 3- to 8-membered heterocycle in the partially unsaturated 3- to 8-membered heterocycle substituted with one or more R^{C1} each independently have 1, 2 or 3 heteroatoms selected from one or more of N, O, S and C(=O);
R^{C1} is independently C₁-C₆ alkyl;
R^{A3} and R^{A4} are each independently CN, -P(=O)(R^{A3-1})₂, C₁-C₆ alkyl or C₁-C₆ alkoxy;
R^{A3-1} is independently C₁-C₆ alkyl;
L is -(CH₂)n-, wherein n is any integer from 1 to 18, and 1 or more of the -(CH₂)- in -(CH₂)ₙ- are replaced by one or more of the following groups: -NR^{L1}-, -O-, -S-, -C(=O)-, 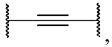 saturated C₃-C₁₂ cycloalkylene, saturated 3- to 12-membered heterocycloalkylene, C₆-C₁₀ arylene, 5- to 10-membered heteroarylene, 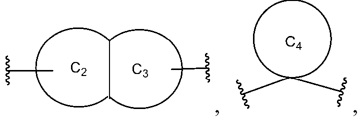 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4} or 5- to 10-membered heteroarylene substituted with one or more R^{L5}, wherein the saturated 3- to 12-membered heterocycloalkylene and the 3- to 12-membered heterocycloalkyl in the saturated 3- to 12-membered heterocycloalkylene substituted with one or more R^{L3} each independently have 1, 2 or 3 heteroatoms independently selected from one or more of N, O, S and C(=O) and the 5- to 10-membered heteroarylene and the 5- to 10-membered heteroarylene in the 5- to 10-membered heteroarylene substituted with one or more R^{L5} each independently have 1, 2 or 3 heteroatoms independently selected from one or more of N, O and S;
R^{L1} is H or C₁-C₆ alkyl;
ring C₂ is benzene ring or 5- to 6-membered heteroaromatic ring, wherein the 5- to 6-membered heteroaromatic ring has 1, 2 or 3 heteroatoms selected from one or more of N, O and S;
ring C₃ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
ring C₄ is a saturated C₃-C₇ carbon ring or a saturated 3- to 7-membered heterocycle, wherein the saturated 3-to 7-membered heterocycle has 1, 2 or 3 heteroatoms selected from one or more of N, O, S and C(=O);
R^{L2} and R^{L3} are each independently CN, OH, halogen, C₁-C₆ alkyl, C₃-C₇ cycloalkyl or 3- to 7-membered heterocycloalkyl;
R^{L4} and R^{L5} are each independently C₁-C₆ alkyl;
B is 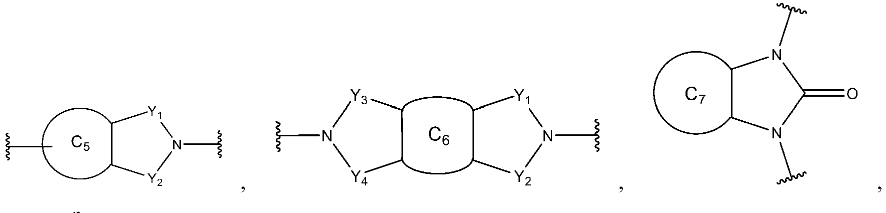 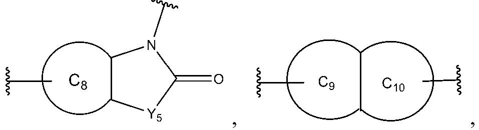 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1}, or 5- to 10-membered heteroarylene substituted with one or more R^{B2}, wherein the 5-to 10-membered heteroarylene and the 5- to 10-membered heteroarylene in the 5- to 10-membered heteroarylene substituted with one or more R^{B2} each independently have 1, 2 or 3 heteroatoms selected from one or more of N, O and S, and the saturated 3- to 12-membered heterocycloalkylene independently has 1, 2 or 3 heteroatoms selected from one or more of N, O, S and C(=O);
Y₁. Y₂, Y₃ and Y₄ are each independently CH₂ or C(=O);
Y₅ is NY⁵⁻¹, O or S, and Y⁵⁻¹ is H or C₁-C₆ alkyl;
ring C₅, ring C₆, ring C₇, ring C₈ and ring C₉ are each independently a benzene ring or a 5- to 6-membered heteroaromatic ring, wherein the 5- to 6-membered heteroaromatic ring has 1, 2 or 3 heteroatoms selected from one or more of N, O and S;
ring C₁₀ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
R^{B1} and R^{B2} are each independently halogen, cyano, -P=O(CH₃)₂, C₁-C₆ alkyl, C₁-C₆ alkoxy, C₁-C₆ alkyl substituted with one or more R^{B1-1}, or C₁-C₆ alkoxy substituted with one or more R^{B1-2};
R^{B1-1} and R^{B1-2} are each independently halogen;
moreover, the compound represented by formula (I) satisfies one or both of the following conditions:
i: B is 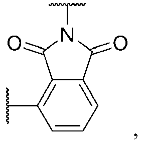 L is -(CH₂)ₙ-, n is any integer from 1 to 18, and at least 1 of -(CH₂)- in -(CH₂)ₙ- is replaced by the following groups: saturated C₃-C₁₂ cycloalkylene, saturated 3- to 12-membered heterocycloalkylene, C₆-C₁₀ arylene, 5- to 10-membered heteroarylene, 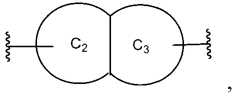 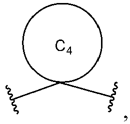 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4}, or 5-to 10-membered heteroarylene substituted with one or more R^{L5};
ii: B is 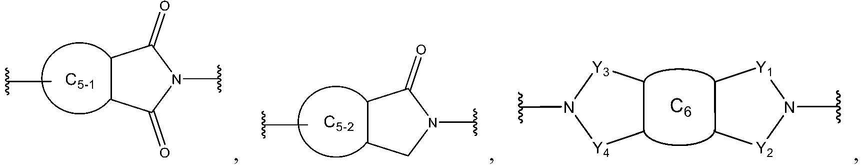 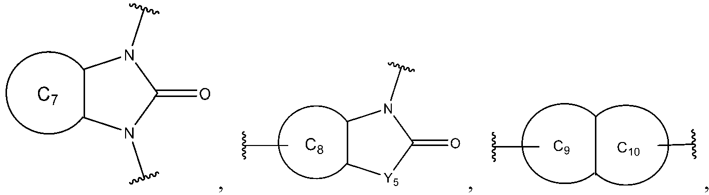 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1} or 5- to 10-membered heteroarylene substituted with one or more R^{B2}, ring C₅₋₁ same as the 5- to 6-membered heteroaryl ring in ring C5, and ring C₅₋₂ same as ring C₅.

6. The compound represented by formula (I), the pharmaceutically acceptable salt thereof, the solvate thereof or the solvate of the pharmaceutically acceptable salt thereof according to any one of claims 1 to 3, wherein, 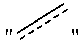 represents a single bond or a double bond;
X¹ is CH or N;
R³ is H or C₁-C₆ alkyl;
R⁴ is H;
X² is N or CH;
X³ is CR² or N;
R² is H, halogen, C₁-C₆ alkyl, C₁-C₆ alkoxy, saturated C₃-C₁₁ cycloalkyl, saturated 3- to 11-membered heterocycloalkyl, -P(=O)(R²⁻¹)₂, -S(=O)₂R²⁻², -OR²⁻⁷, C₁-C₆ alkyl substituted with one or more R²⁻³, C₁-C₆ alkoxy substituted with one or more R²⁻⁴, saturated C₃-C₁₁ cycloalkyl substituted with one or more R²⁻⁵ or saturated 3- to 11-membered heterocycloalkyl substituted with one or more R²⁻⁶, wherein the saturated 3- to 11-membered heterocycloalkyl and the 3- to 11-membered heterocycloalkyl in the saturated 3- to 11-membered heterocycloalkyl substituted with one or more R²⁻⁶ each independently have 1, 2 or 3 heteroatoms independently selected from one or more of N, O, S and C(=O);
R²⁻¹ and R²⁻² are each independently C₁-C₆ alkyl;
R²⁻⁷ is saturated C₃-C₁₁ cycloalkyl;
R²⁻³ and R²⁻⁴ are each independently -N(R²⁻³⁻¹)₂, saturated C₃-C₁₁ cycloalkyl, saturated 3- to 11-membered heterocycloalkyl, saturated C₃-C₁₁ cycloalkyl substituted with one or more R²⁻³⁻², or saturated 3- to 11-membered heterocycloalkyl substituted with one or more R²⁻³⁻³, wherein the saturated 3- to 11-membered heterocycloalkyl and the 3- to 11-membered heterocycloalkyl in the saturated 3- to 11-membered heterocycloalkyl substituted with one or more R²⁻³⁻³ each independently have 1, 2 or 3 heteroatoms selected from one or more of N, O, S and C(=O);
R²⁻³⁻¹ is independently H or C₁-C₆ alkyl;
R²⁻³⁻² and R²⁻³⁻³ are each independently C₁-C₆ alkyl;
R²⁻⁵ and R²⁻⁶ are each independently C₁-C₆ alkyl;
R¹ is C₆-C₁₀ aryl, 5- to 10-membered heteroaryl, saturated C₃-C₁₁ cycloalkyl, saturated 3- to 11-membered heterocycloalkyl, C₆-C₁₀ aryl substituted with one or more R¹⁻¹, or 5- to 10-membered heteroaryl substituted with one or more R¹⁻², wherein the saturated 3- to 11-membered heterocycloalkyl has 1, 2 or 3 heteroatoms selected from one or more of N, O, S and C(=O), and the 5- to 10-membered heteroaryl and the 5- to 10-membered heteroaryl in the 5- to 10-membered heteroaryl substituted with one or more R¹⁻² each independently have 1, 2 or 3 heteroatoms independently selected from one or more of N, O and S;
R¹⁻¹ and R¹⁻² are each independently halogen, C₁-C₆ alkyl or C₁-C₆ alkoxy;
A is -R^{A1}C(=O)-, -R^{A2}CH₂-, C₆-C₁₀ arylene, 5- to 10-membered heteroarylene, saturated C₃-C₁₁ cycloalkylene, saturated 3- to 11-membered heterocycloalkylene, 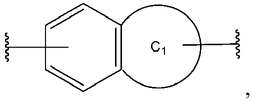 C₆-C₁₀ arylene substituted with one or more R^{A3}, or 5- to 10-membered heteroarylene substituted with one or more R^{A4}, wherein the saturated 3- to 11-membered heterocycloalkylene has 1, 2 or 3 heteroatoms selected from one or more of N, O, S and C(=O) and the 5- to 10-membered heteroarylene and the 5- to 10-membered heteroarylene in the 5-to 10-membered heteroarylene substituted with one or more R^{A4} each independently have 1, 2 or 3 heteroatoms independently selected from one or more of N, O and S;
R^{A1} and R^{A2} are each independently C₆-C₁₀ arylene, 5- to 10-membered heteroarylene, saturated C₃-C₁₁ cycloalkylene, saturated 3- to 11-membered heterocycloalkylene, C₆-C₁₀ arylene substituted with one or more R^{A1-1}, or 5- to 10-membered heteroarylene substituted with one or more R^{A1-2}, wherein the 5- to 10-membered heteroarylene and the 5- to 10-membered heteroarylene in the 5- to 10-membered heteroarylene substituted with one or more R^{A1-2} each independently have 1, 2 or 3 heteroatoms selected from one or more of N, O and S, and the 3- to 11-membered heterocycloalkylene has 1, 2 or 3 heteroatoms selected from one or more of N, O, S and C(=O);
R^{A1-1} and R^{A1-2} are each independently CN, -P(=O)(R^{A3-1})₂, C₁-C₆ alkyl or C₁-C₆ alkoxy;
ring C₁ is a partially unsaturated 3- to 8-membered heterocycle or a partially unsaturated 3- to 8-membered heterocycle substituted with one or more R^{C1}, wherein the partially unsaturated 3- to 8-membered heterocycle and the 3- to 8-membered heterocycle in the partially unsaturated 3- to 8-membered heterocycle substituted with one or more R^{C1} each independently have 1, 2 or 3 heteroatoms selected from one or more of N, O, S and C(=O);
R^{C1} is independently C₁-C₆ alkyl;
R^{A3} and R^{A4} are each independently CN, -P(=O)(R^{A3-1})₂, C₁-C₆ alkyl or C₁-C₆ alkoxy;
R^{A3-1} is independently C₁-C₆ alkyl;
L is -(CH₂)n-, wherein n is any integer from 1 to 18, and 1 or more of the -(CH₂)- in -(CH₂)ₙ- are replaced by one or more of the following groups: -NR^{L1}-, -O-, -S-, -C(=O)-, 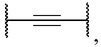 saturated C₃-C₁₂ cycloalkylene, saturated 3- to 12-membered heterocycloalkylene, C₆-C₁₀ arylene, 5- to 10-membered heteroarylene, 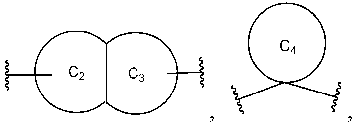 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4} or 5- to 10-membered heteroarylene substituted with one or more R^{L5}, wherein the saturated 3- to 12-membered heterocycloalkylene and the 3- to 12-membered heterocycloalkyl in the saturated 3- to 12-membered heterocycloalkylene substituted with one or more R^{L3} each independently have 1, 2 or 3 heteroatoms independently selected from one or more of N, O, S and C(=O) and the 5- to 10-membered heteroarylene and the 5- to 10-membered heteroarylene in the 5- to 10-membered heteroarylene substituted with one or more R^{L5} each independently have 1, 2 or 3 heteroatoms independently selected from one or more of N, O and S;
R^{L1} is H or C₁-C₆ alkyl;
ring C₂ is benzene ring or 5- to 6-membered heteroaromatic ring, wherein the 5- to 6-membered heteroaromatic ring has 1, 2 or 3 heteroatoms selected from one or more of N, O and S;
ring C₃ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
ring C₄ is a saturated C₃-C₇ carbon ring or a saturated 3- to 7-membered heterocycle, wherein the saturated 3-to 7-membered heterocycle has 1, 2 or 3 heteroatoms selected from one or more of N, O, S and C(=O);
R^{L2} and R^{L3} are each independently CN, OH, halogen, C₁-C₆ alkyl, C₃-C₇ cycloalkyl or 3- to 7-membered heterocycloalkyl;
R^{L4} and R^{L5} are each independently C₁-C₆ alkyl;
B is 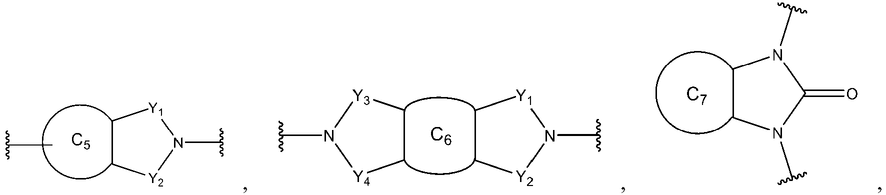 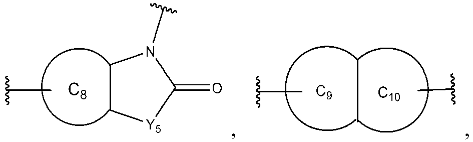 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1}, or 5- to 10-membered heteroarylene substituted with one or more R^{B2}, wherein the 5-to 10-membered heteroarylene and the 5- to 10-membered heteroarylene in the 5- to 10-membered heteroarylene substituted with one or more R^{B2} each independently have 1, 2 or 3 heteroatoms selected from one or more of N, O and S, and the saturated 3- to 12-membered heterocycloalkylene independently has 1, 2 or 3 heteroatoms selected from one or more of N, O, S and C(=O);
Y₁. Y₂, Y₃ and Y₄ are each independently CH₂ or C(=O);
Y₅ is NY⁵⁻¹, O or S, and Y⁵⁻¹ is H or C₁-C₆ alkyl;
ring C₅, ring C₆, ring C₇, ring C₈ and ring C₉ are each independently a benzene ring or a 5- to 6-membered heteroaromatic ring, wherein the 5- to 6-membered heteroaromatic ring has 1, 2 or 3 heteroatoms selected from one or more of N, O and S;
ring C₁₀ is a partially unsaturated C₃-C₇ carbon ring or a partially unsaturated 3- to 7-membered heterocycle, wherein the partially unsaturated 3- to 7-membered heterocycle has 1, 2 or 3 heteroatoms selected from one or more of N, O, S and C(=O);
R^{B1} and R^{B2} are each independently halogen, C₁-C₆ alkyl, C₁-C₆ alkoxy, C₁-C₆ alkyl substituted with one or more R^{B1-1}, or C1-C6 alkoxy substituted with one or more R^{B1-2};
R^{B1-1} and R^{B1-2} are each independently halogen;
moreover, the compound represented by formula (I) satisfies one or both of the following conditions:
i: B is 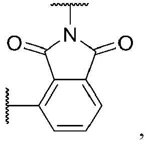 L is -(CH₂)ₙ-, n is any integer from 1 to 18, and at least 1 of -(CH₂)- in -(CH₂)ₙ- is replaced by the following groups: saturated C₃-C₁₂ cycloalkylene, saturated 3- to 12-membered heterocycloalkylene, C₆₋C₁₀ arylene, 5- to 10-membered heteroarylene, 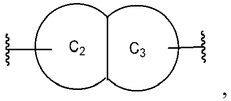 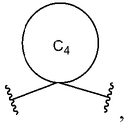 saturated C₃-C₁₂ cycloalkylene substituted with one or more R^{L2}, saturated 3- to 12-membered heterocycloalkylene substituted with one or more R^{L3}, C₆-C₁₀ arylene substituted with one or more R^{L4}, or 5-to 10-membered heteroarylene substituted with one or more R^{L5};
ii: B is 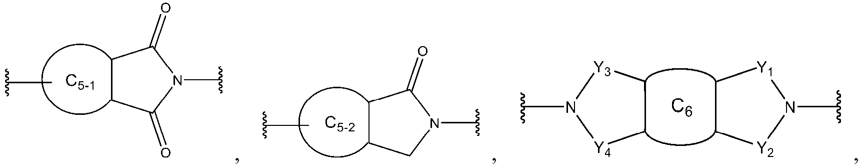 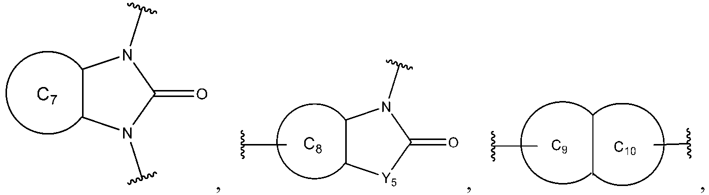 C₆-C₁₀ arylene, 5- to 10-membered heteroarylene, saturated C₃-C₁₂ cycloalkylene, saturated 3- to 12-membered heterocycloalkylene, C₆-C₁₀ arylene substituted with one or more R^{B1} or 5- to 10-membered heteroarylene substituted with one or more R^{B2}, ring C₅₋₁ same as the 5- to 6-membered heteroaryl ring in ring C₅, and ring C₅₋₂ same as ring C₅.

7. The compound represented by formula (I), the pharmaceutically acceptable salt thereof, the solvate thereof or the solvate of the pharmaceutically acceptable salt thereof according to any one of claims 1, 2, 4 and 5, wherein the compound represented by formula (I), the pharmaceutically acceptable salt thereof, the solvate thereof or the solvate of the pharmaceutically acceptable salt thereof satisfies one or more of the following conditions:
(1) R² is -H, -CN, -F, -Cl, -CH₃, cyclopropyl, -OCH₃, 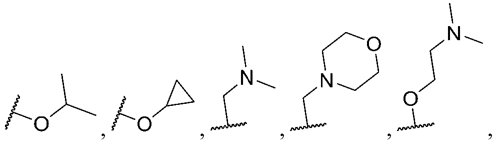 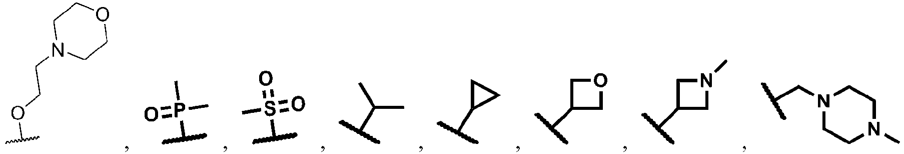 or 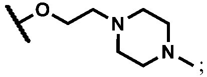
(2) B is: 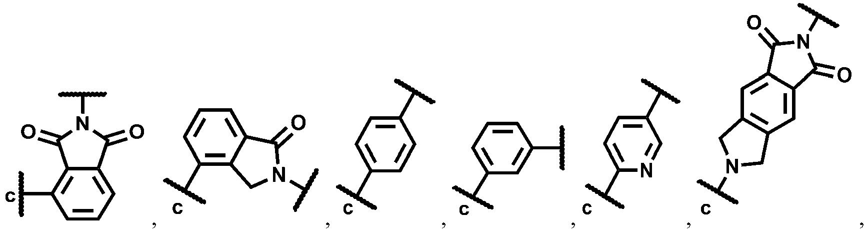 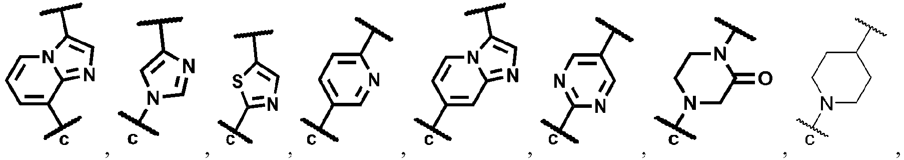 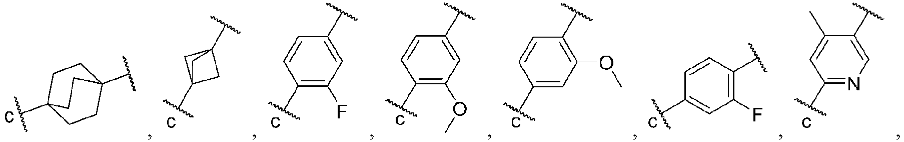 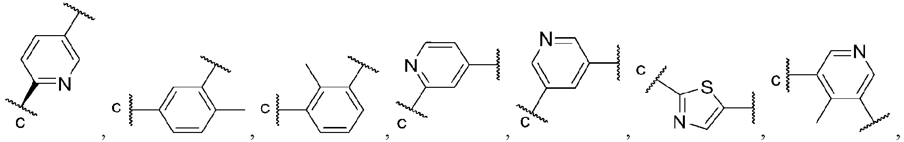 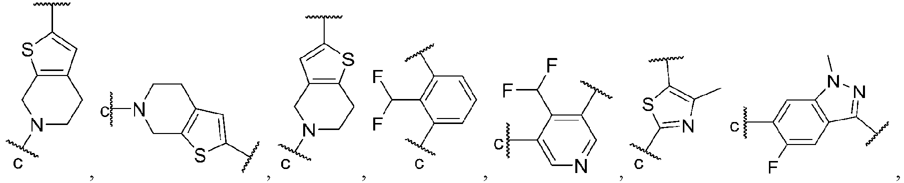 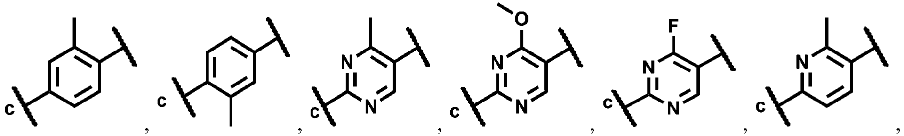 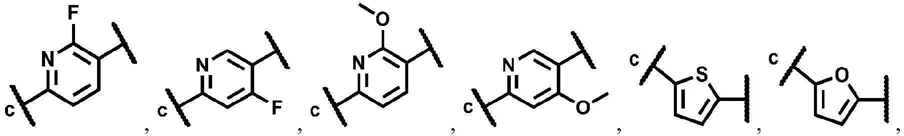 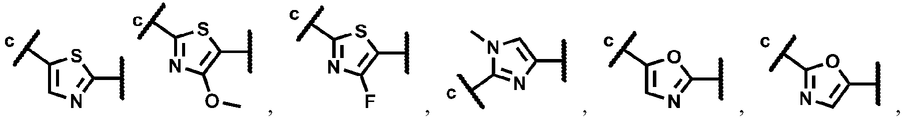 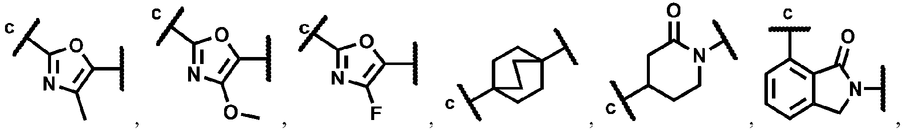 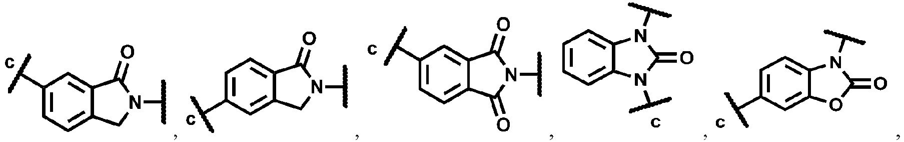 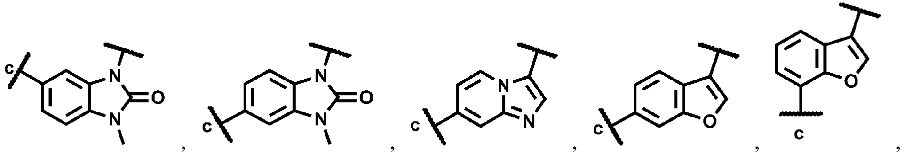 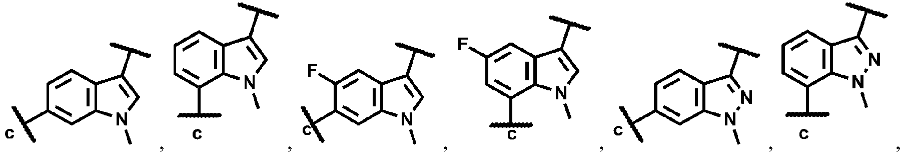 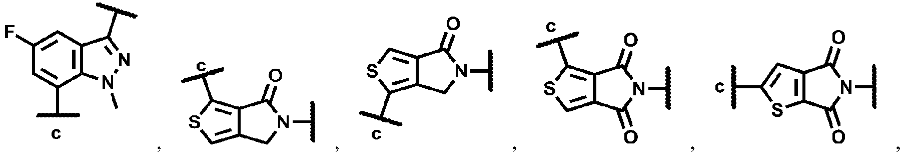 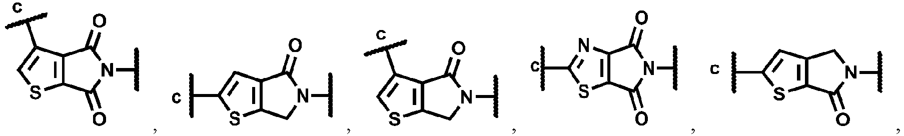 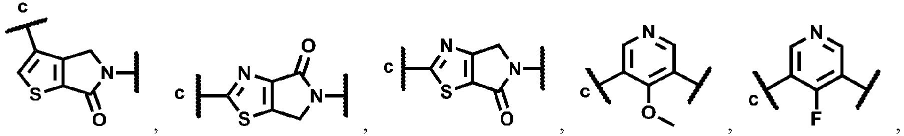 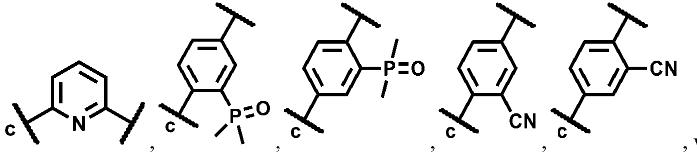 wherein side c is connected to L; in Scheme ii, B is any one of the above groups other than

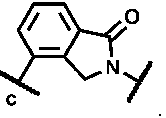

8. The compound represented by formula (I), the pharmaceutically acceptable salt thereof, the solvate thereof or the solvate of the pharmaceutically acceptable salt thereof according to claim 4, wherein the compound represented by formula (I), the pharmaceutically acceptable salt thereof, the solvate thereof or the solvate of the pharmaceutically acceptable salt thereof satisfies one or more of the following conditions:
(1) in condition ii, L is: 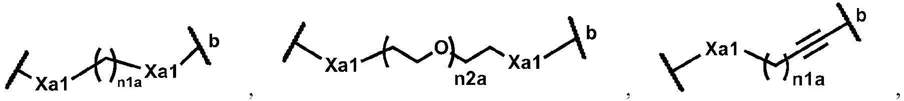 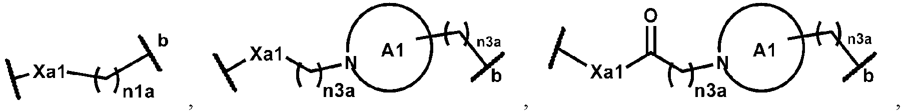 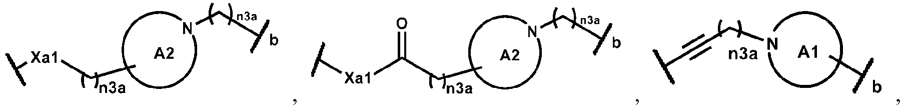 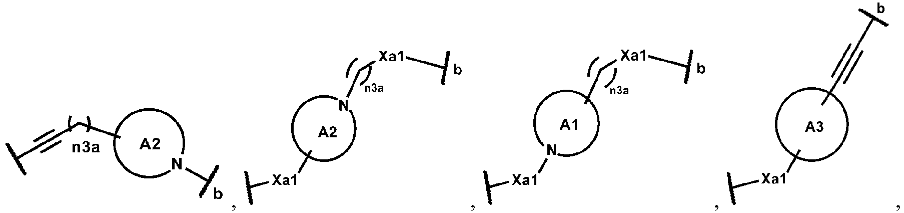 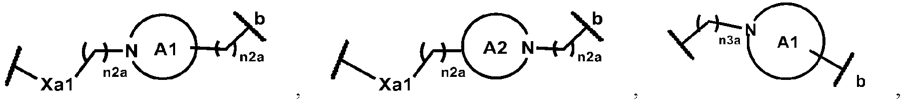 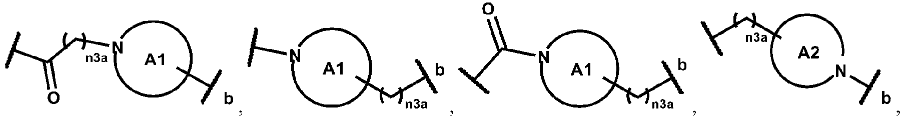 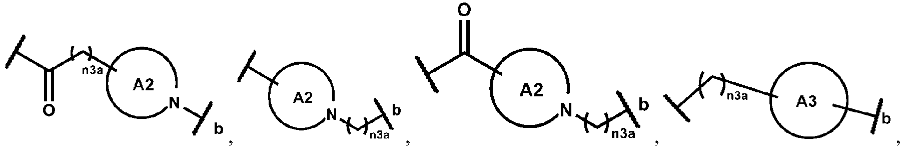 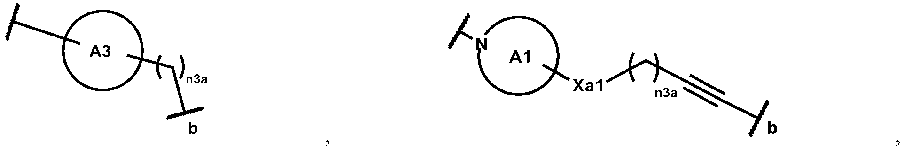 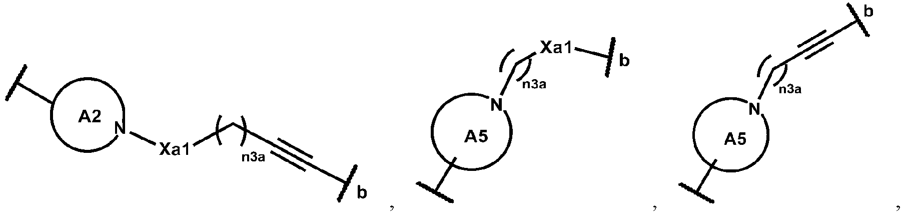 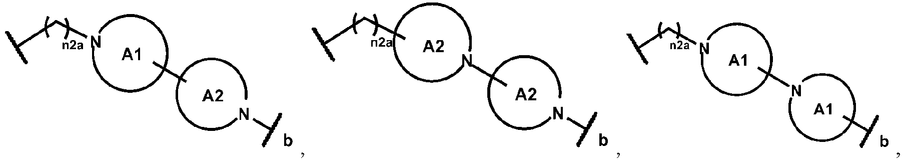 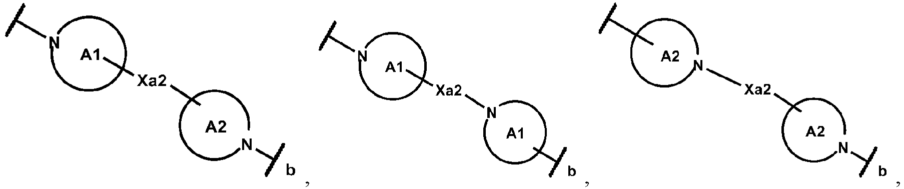 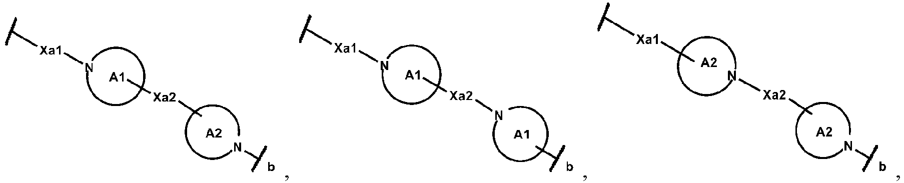 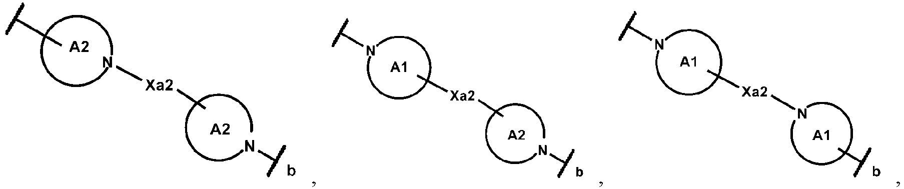 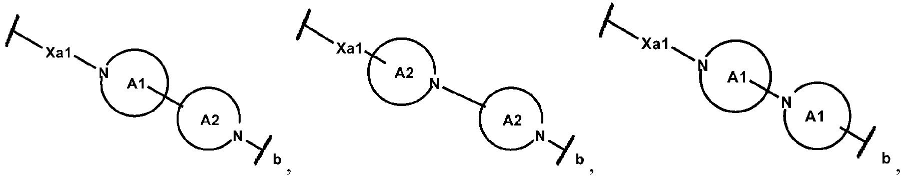 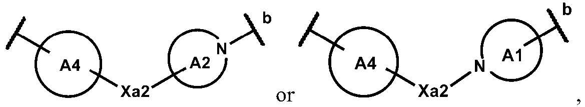
wherein side b is connected to B;
Xa1 is O or -NR^{L1}-, R^{L1} is H or C₁-C₆ alkyl;
Xa2 is 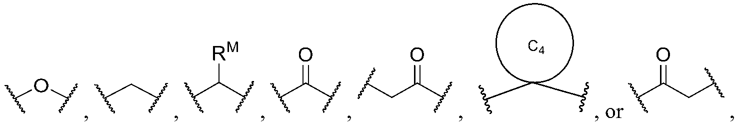 R^{M} is C₁-C₆ alkyl, ring C₄ is saturated C₃-C₆ carbon ring, or saturated 3- to 6-membered heterocyclic ring having 1, 2 or 3 heteroatoms selected from one or more of N, O, S and C(=O);
ring A1 and ring A2 are saturated 3- to 12-membered nitrogen-containing heterocycles or saturated 3- to 12-membered nitrogen-containing heterocycles substituted with one or more R^{L3}, R^{L3} is independently CN, OH, halogen, C₁-C₆ alkyl, C₃-C₇ cycloalkyl or 3- to 7-membered heterocycloalkyl, wherein the saturated 3- to 12-membered nitrogen-containing heterocycle or the 3- to 12-membered nitrogen-containing heterocycle in the substituted saturated 3- to 12-membered nitrogen-containing heterocycle is independently 3- to 6-membered monocyclic nitrogen-containing heterocycle, 7- to 12-membered spirocyclic ring or 7- to 12-membered bridged nitrogen-containing heterocyclic ring, with 1, 2 or 3 heteroatoms selected from one or more of O, S and C(=O) in addition to N;
ring A3 is independently saturated C₃-C₁₂ carbon ring or saturated C₃-C₁₂ carbon ring substituted with one or more R^{L2}, and R^{L2} is independently CN, OH, halogen, C₁-C₆ alkyl, C₃-C₇ cycloalkyl or 3- to 7-membered heterocycloalkyl, wherein the C₃-C₁₂ carbon ring in the saturated C₃-C₁₂ carbon ring or the substituted saturated C₃-C₁₂ carbon ring is independently C₃-C₆ monocyclic carbon ring or C₅-C₁₂ spirocyclic carbon ring; ring A4 is independently 5- to 10-membered heteroaromatic ring or 5- to 10-membered heteroaromatic ring substituted with one or more R^{L5}, and R^{L5} is independently C₁-C₆ alkyl; the 5- to 10-membered heteroaromatic ring or the 5- to 10-membered heteroaromatic ring in the 5- to 10-membered heteroaromatic ring substituted with one or more R^{L5} independently has 1, 2 or 3 heteroatoms selected from one or more of N, O and S;
ring A5 is independently 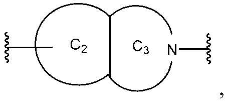
and ring C₂ is benzene ring or 5- to 6-membered heteroaromatic ring wherein the 5- to 6-membered heteroaromatic ring has 1, 2 or 3 heteroatoms selected from one or more of N, O and S; ring C₃ is partially unsaturated 3- to 7-membered nitrogen-containing heterocyclic ring, which has 1, 2 or 3 heteroatoms selected from one or more of O, S and C(=O) in addition to N;
n1a is independently any integer from 0 to 8;
n2a is independently any integer from 0 to 3;
n3a is independently any integer from 0 to 5;
preferably: 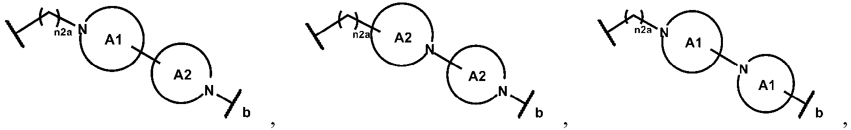 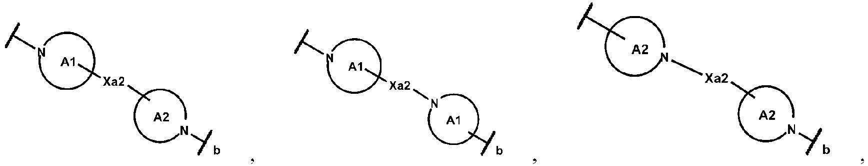 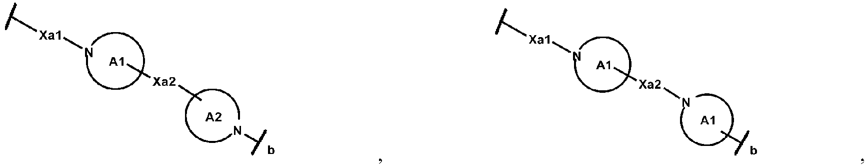 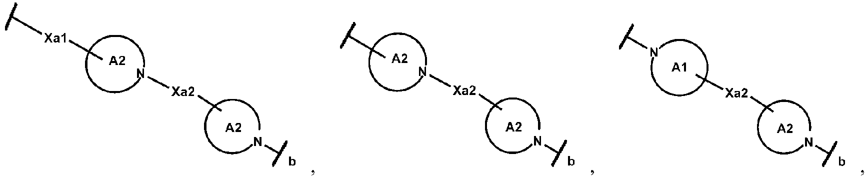 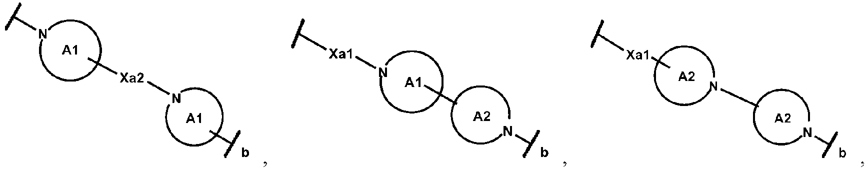 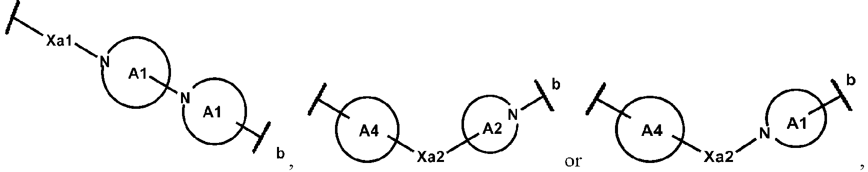
wherein side b is connected to B;
(2) in condition i, L is G1 or G2:
G1: 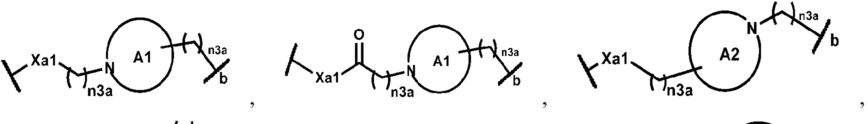 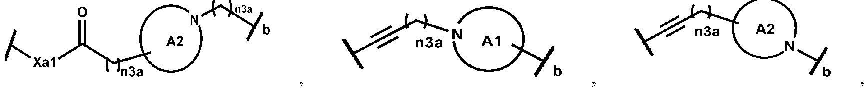 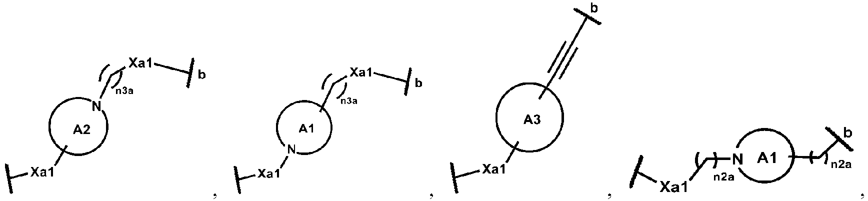 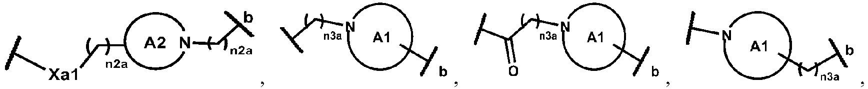 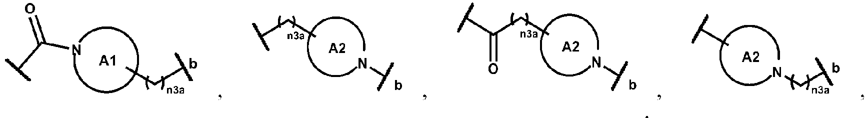 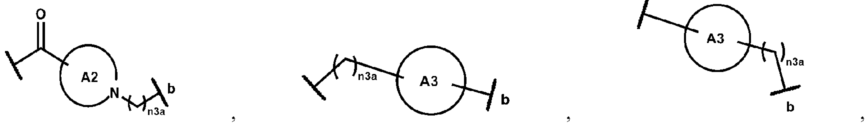 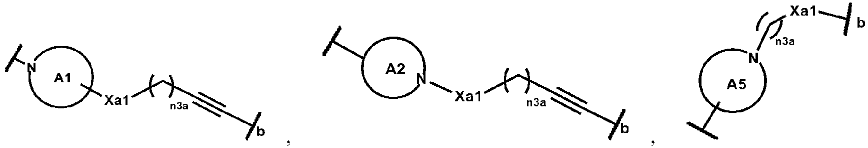 or 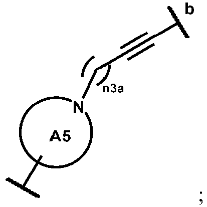
wherein side b is connected to B;
Xa1 is O or -NR^{L1}-, R^{L1} is H or C₁-C₆ alkyl;
ring A1 and ring A2 are saturated 3- to 12-membered nitrogen-containing heterocycles or saturated 3- to 12-membered nitrogen-containing heterocycles substituted with one or more R^{L3}, R^{L3} is independently CN, OH, halogen, C₁-C₆ alkyl, C₃-C₇ cycloalkyl or 3- to 7-membered heterocycloalkyl, wherein the saturated 3- to 12-membered nitrogen-containing heterocycle or the 3- to 12-membered nitrogen-containing heterocycle in the substituted saturated 3- to 12-membered nitrogen-containing heterocycle is independently 4-membered monocyclic nitrogen-containing heterocycle, 7- to 12-membered spirocyclic nitrogen-containing heterocycle or 7- to 12-membered bridged nitrogen-containing heterocycle, with 1, 2 or 3 heteroatoms selected from one or more of O, S and C(=O) in addition to N;
ring A3 is independently saturated C₃-C₁₂ carbon ring or saturated C₃-C₁₂ carbon ring substituted with one or more R^{L2}, and R^{L2} is independently CN, OH, halogen, C₁-C₆ alkyl, C₃-C₇ cycloalkyl or 3- to 7-membered heterocycloalkyl, wherein the C₃-C₁₂ carbon ring in the saturated C₃-C₁₂ carbon ring or the substituted saturated C₃-C₁₂ carbon ring is independently C₃-C₆ monocyclic carbon ring, C₇-C₁₂ spirocyclic carbon ring or C₇-C₁₂ bridged carbon ring;
ring A5 is independently 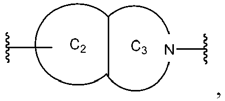 and ring C₂ is benzene ring or 5- to 6-membered heteroaromatic ring wherein the 5- to 6-membered heteroaromatic ring has 1, 2 or 3 heteroatoms selected from one or more of N, O and S; ring C₃ is partially unsaturated 3- to 7-membered nitrogen-containing heterocyclic ring, which has 1, 2 or 3 heteroatoms selected from one or more of O, S and C(=O) in addition to N;
n2a is independently any integer from 0 to 3;
n3a is independently any integer from 0 to 5;
G2: 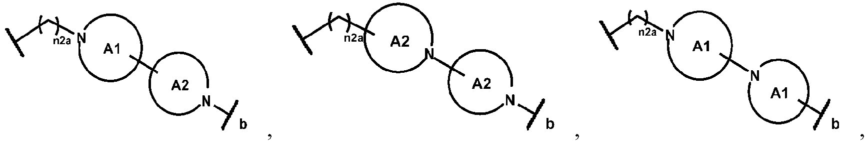 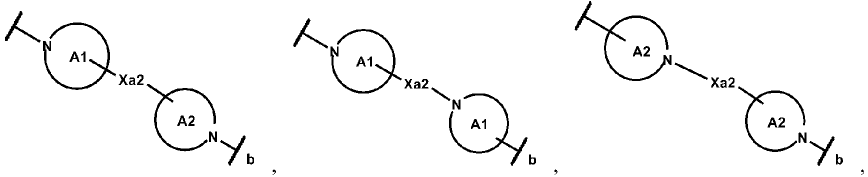 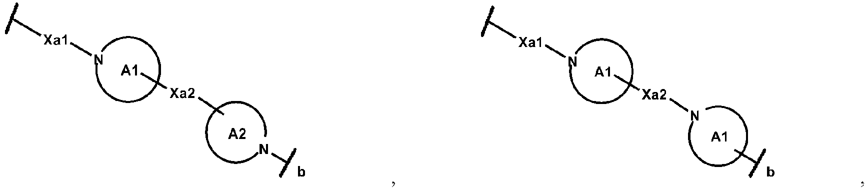 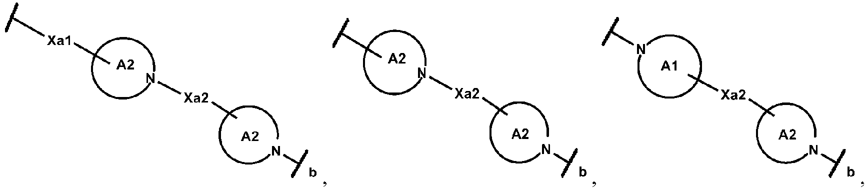 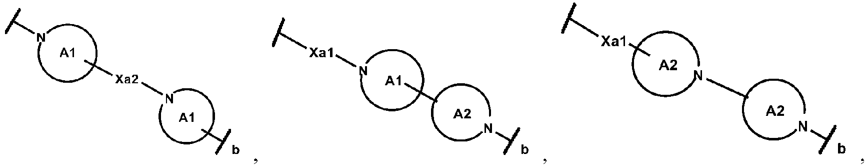 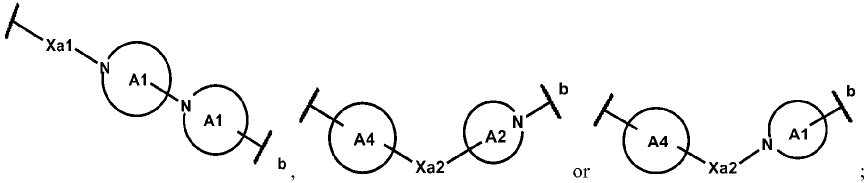
wherein side b is connected to B;
Xa1 is O or -NR^{L1}-, R^{L1} is H or C₁-C₆ alkyl;
Xa2 is 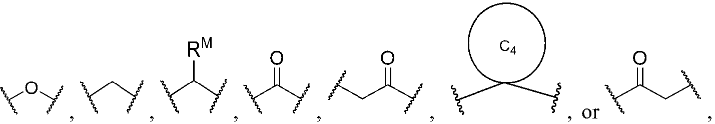 R^{M} is C₁-C₆ alkyl, ring C4 is saturated C₃-C₆ carbon ring, or saturated 3- to 6-membered heterocyclic ring having 1, 2 or 3 heteroatoms selected from one or more of N, O, S and C(=O);
ring A1 and ring A2 are saturated 3- to 12-membered nitrogen-containing heterocycles or saturated 3- to 12-membered nitrogen-containing heterocycles substituted with one or more R^{L3}, R^{L3} is independently CN, OH, halogen, C₁-C₆ alkyl, C₃-C₇ cycloalkyl or 3- to 7-membered heterocycloalkyl, wherein the saturated 3- to 12-membered nitrogen-containing heterocycle or the 3- to 12-membered nitrogen-containing heterocycle in the substituted saturated 3- to 12-membered nitrogen-containing heterocycle is independently 3- to 6-membered monocyclic nitrogen-containing heterocycle or 5- to 12-membered spirocyclic nitrogen-containing heterocycle, with 1, 2 or 3 heteroatoms selected from one or more of O, S and C(=O) in addition to N;
ring A4 is independently 5- to 10-membered heteroaromatic ring or 5- to 10-membered heteroaromatic ring substituted with one or more R^{L5}, and R^{L5} is independently C₁-C₆ alkyl; the 5- to 10-membered heteroaromatic ring or the 5- to 10-membered heteroaromatic ring in the 5- to 10-membered heteroaromatic ring substituted with one or more R^{L5} independently has 1, 2 or 3 heteroatoms selected from one or more of N, O and S;
n2a is independently any integer from 0 to 3.

9. The compound represented by formula (I), the pharmaceutically acceptable salt thereof, the solvate thereof or the solvate of the pharmaceutically acceptable salt thereof according to claim 4, wherein the compound represented by formula (I), the pharmaceutically acceptable salt thereof, the solvate thereof or the solvate of the pharmaceutically acceptable salt thereof satisfies one or more of the following conditions:
(1) in condition ii, L is 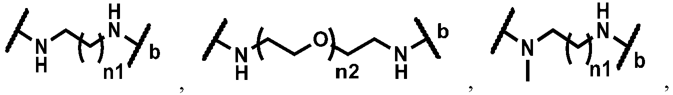 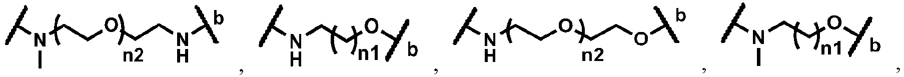 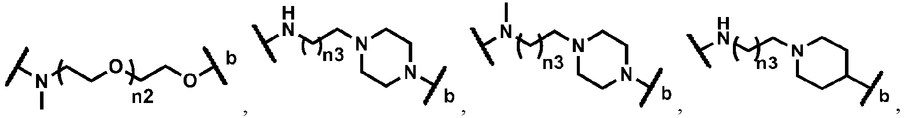 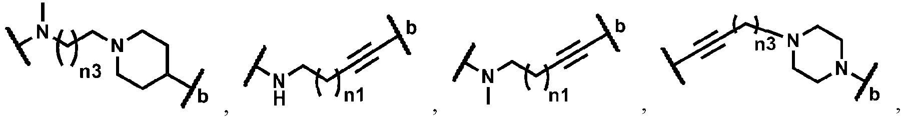 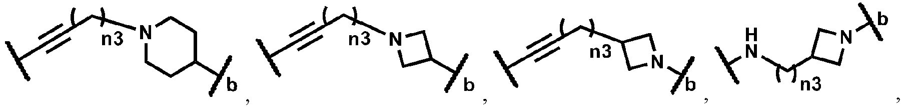 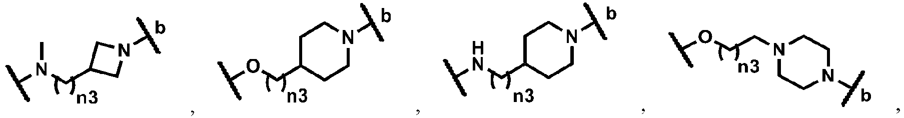 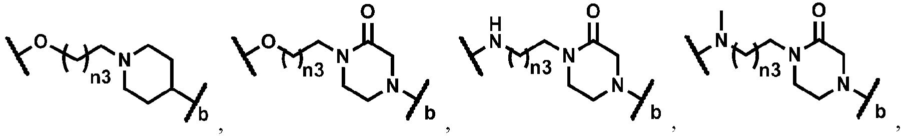 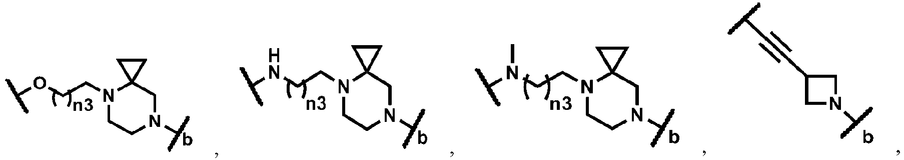 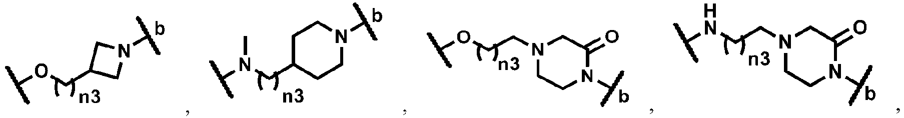 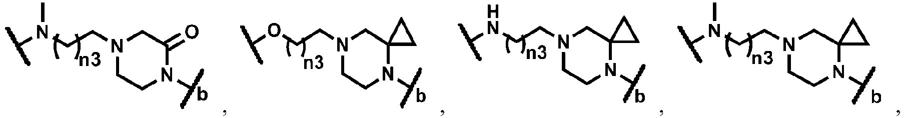 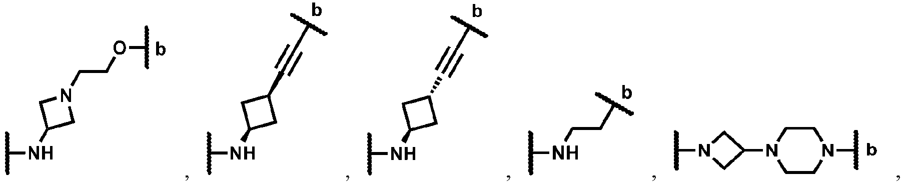 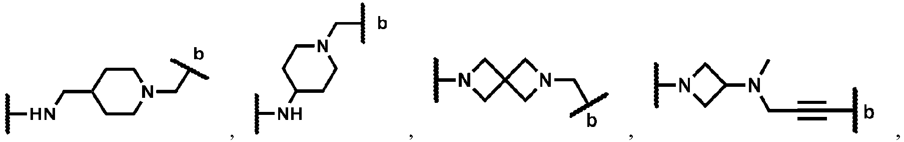 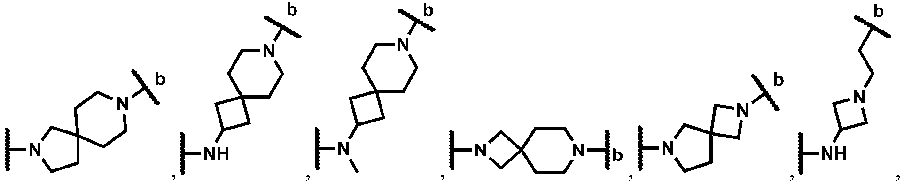 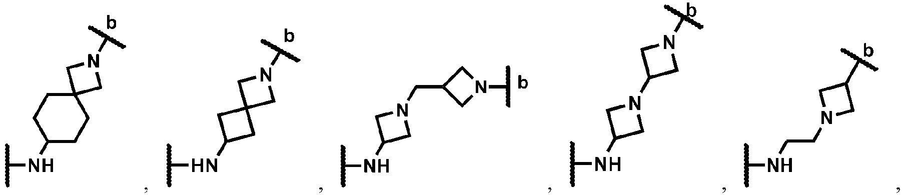 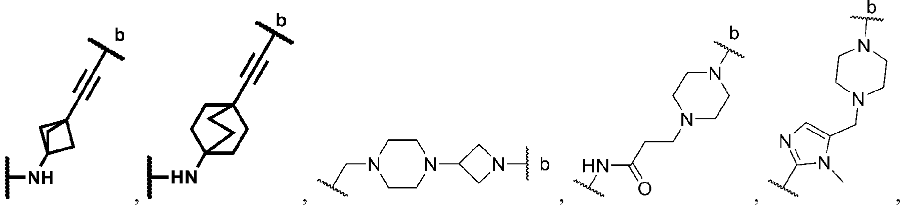 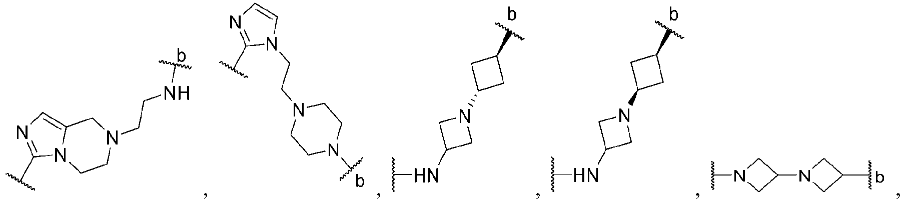 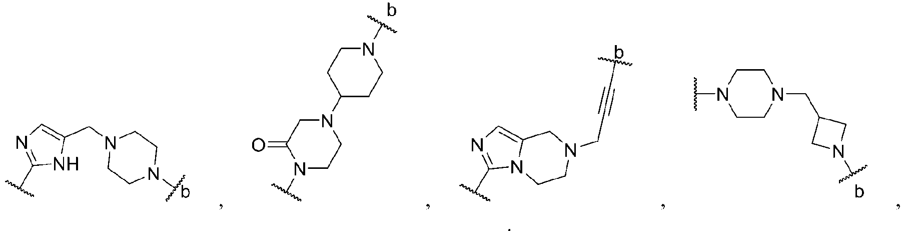 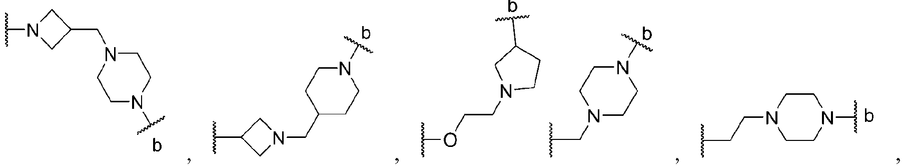 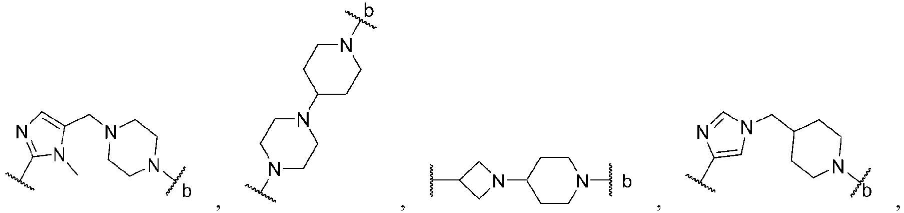 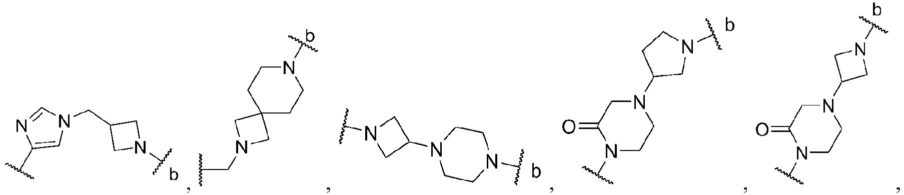 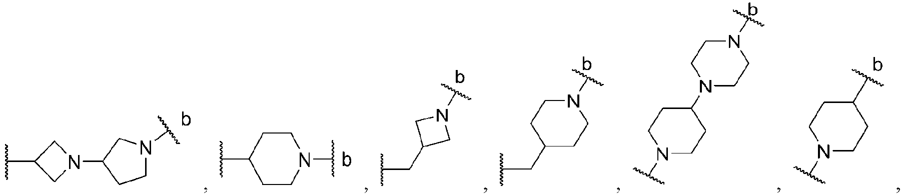 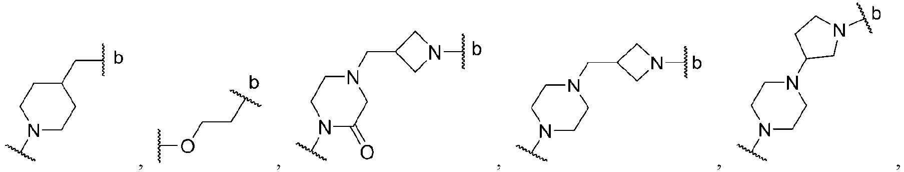 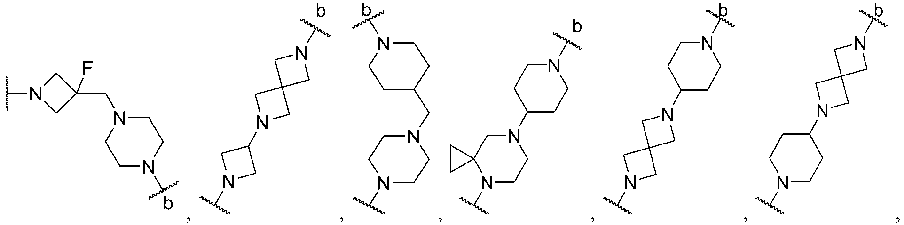 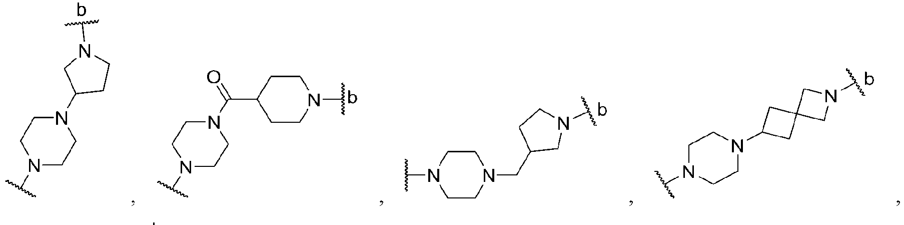 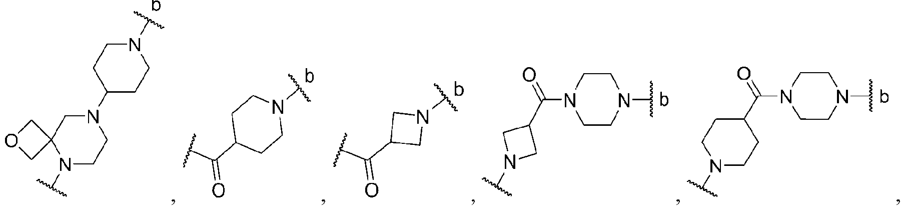 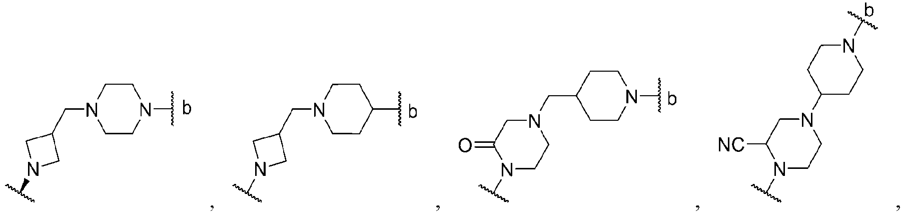 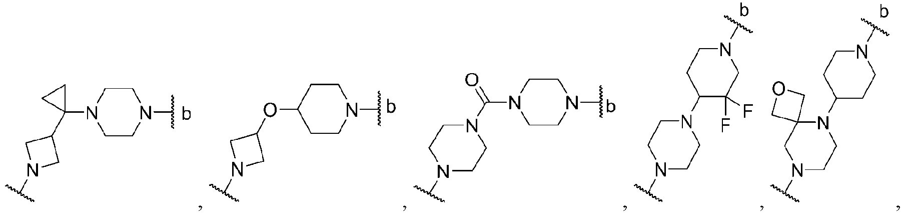 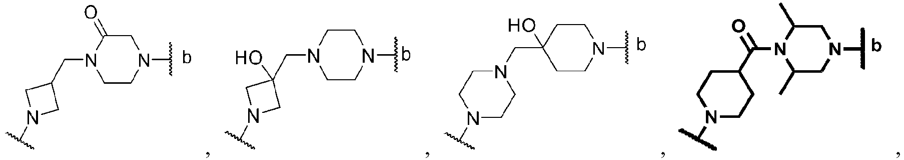 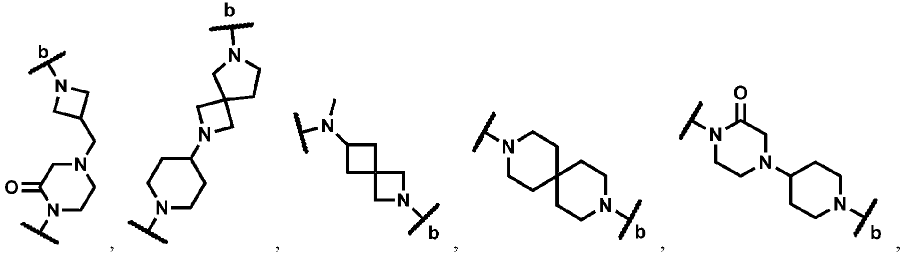 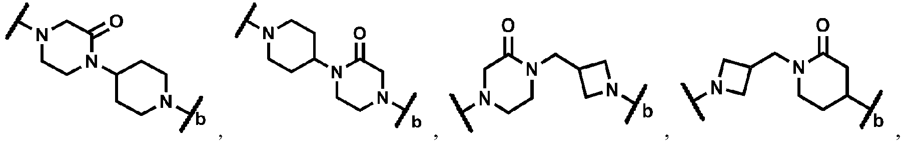 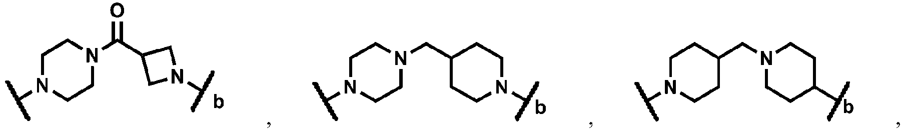 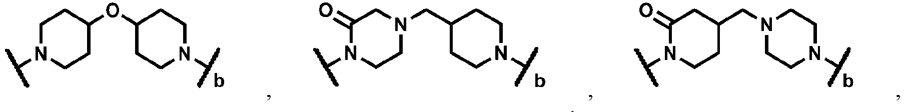 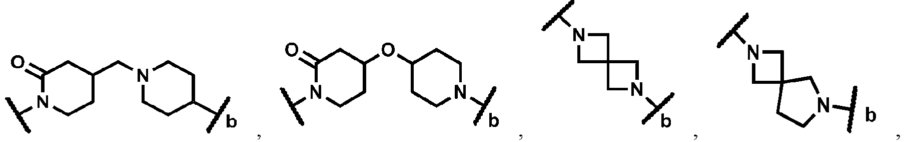 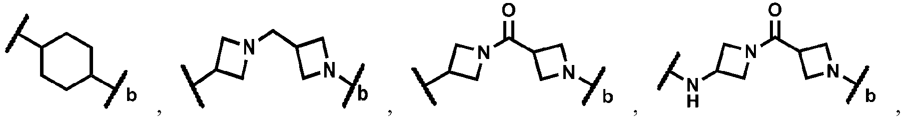 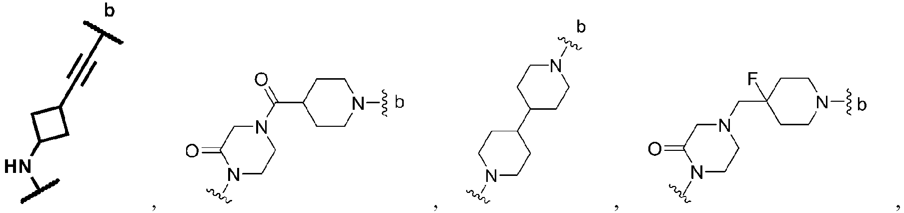 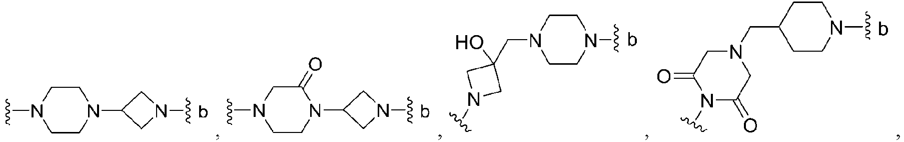 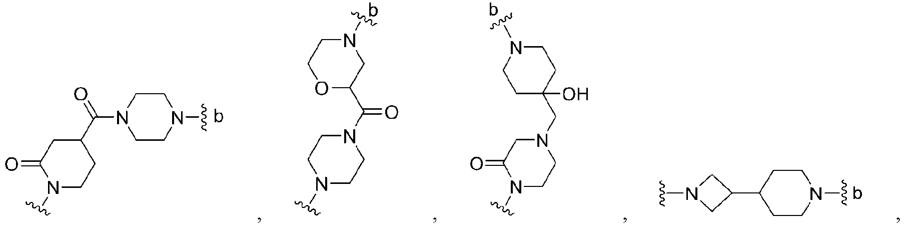 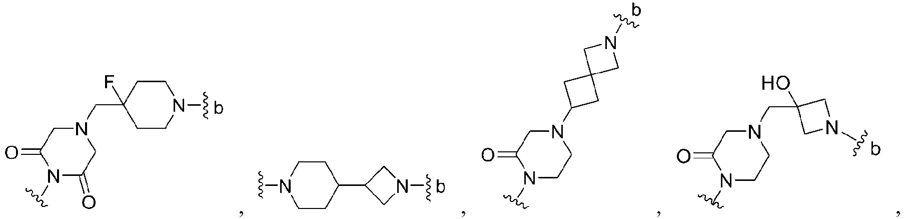 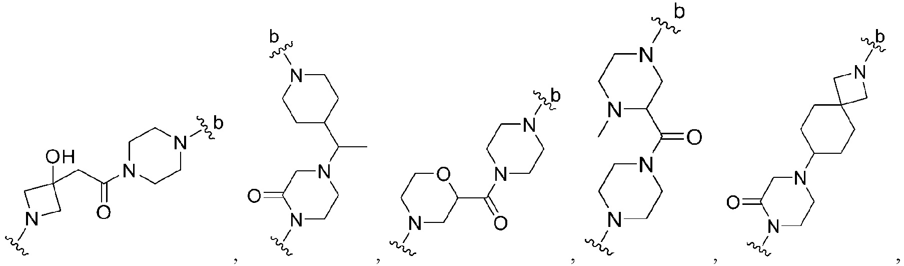 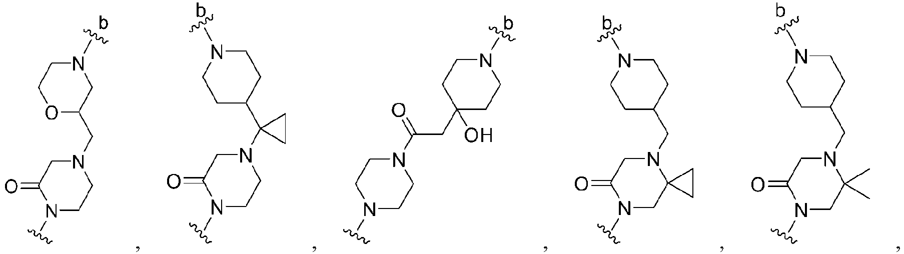 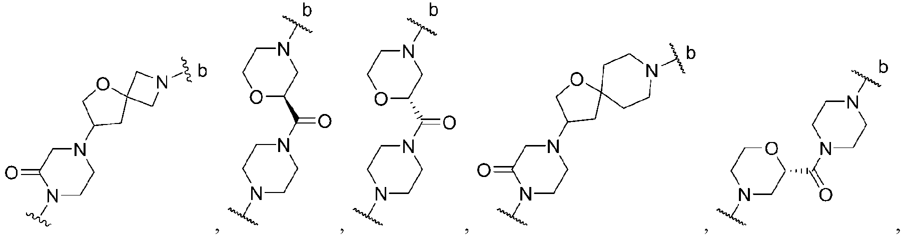 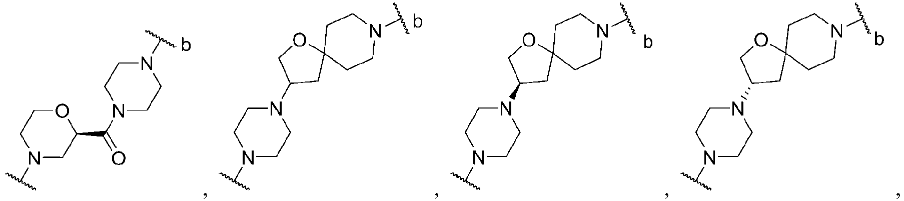 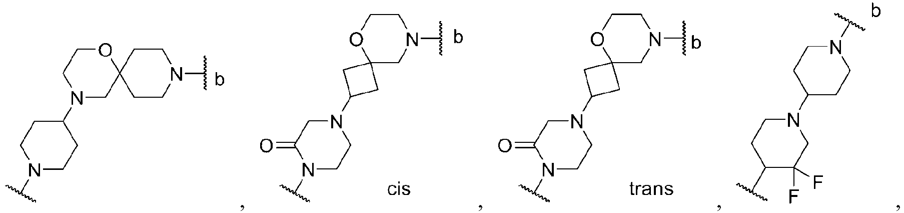 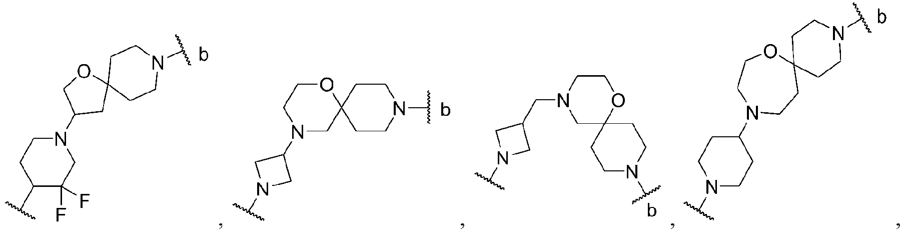 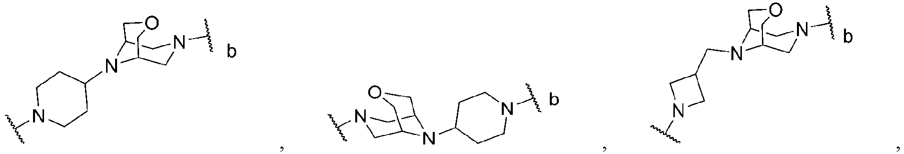 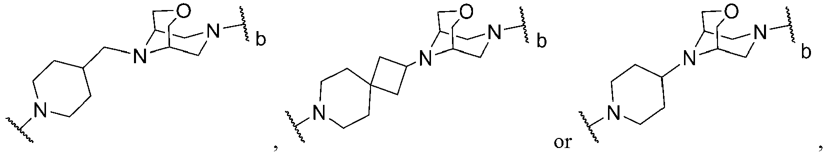 wherein n1 is any integer from 1 to 8, n2 is any integer from 1 to 3, n3 is any integer from 1 to 5, and side b is connected to B;
preferably 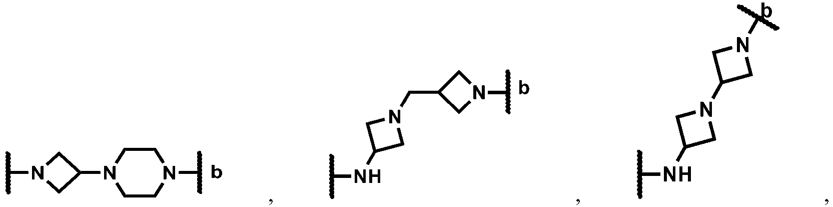 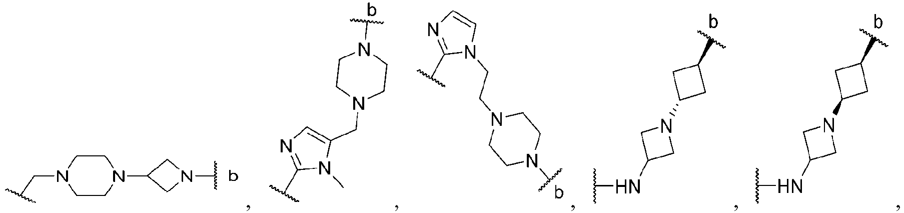 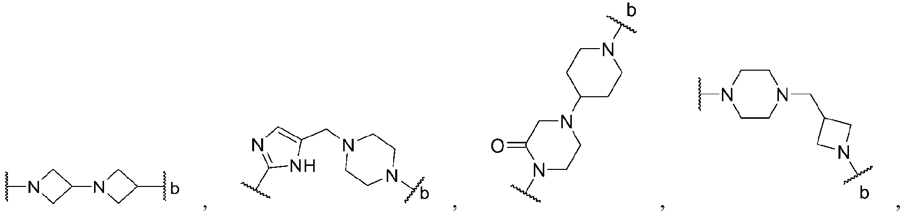 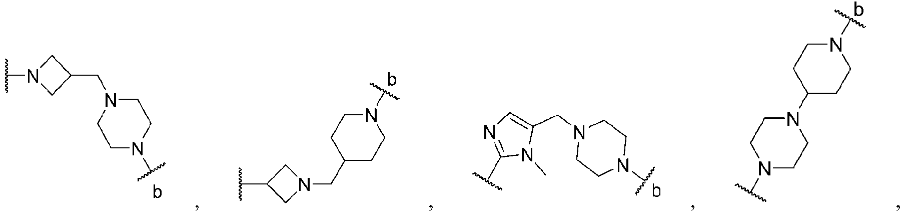 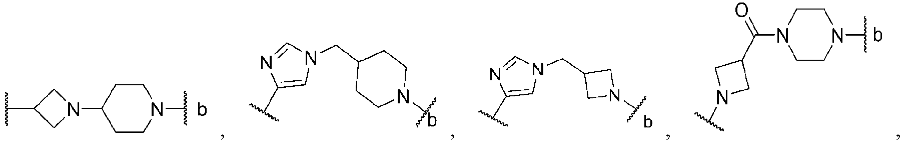 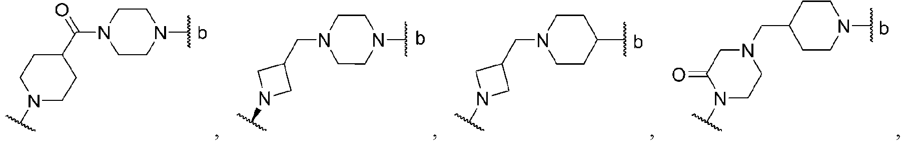 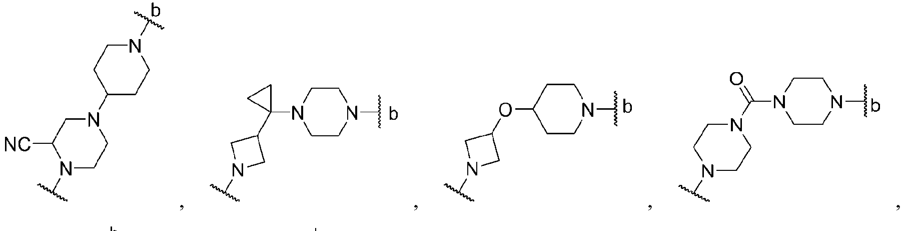 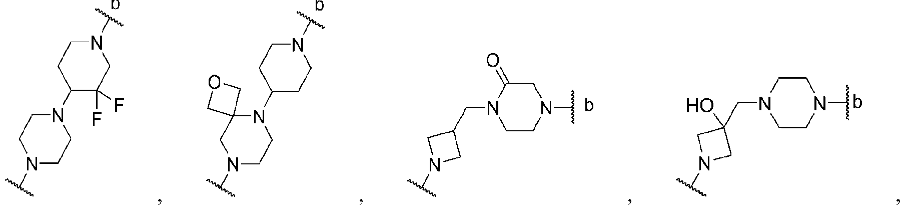 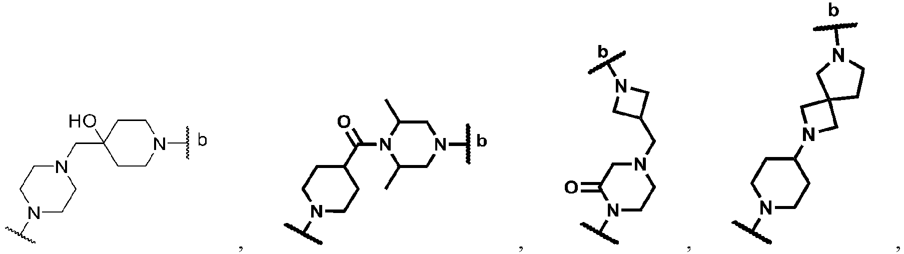 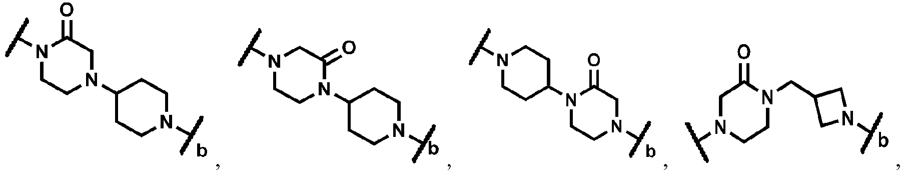 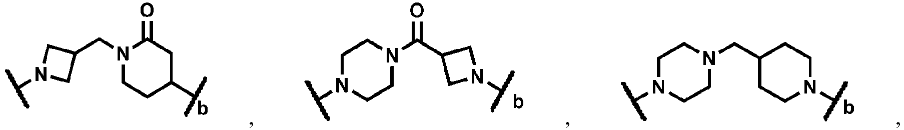 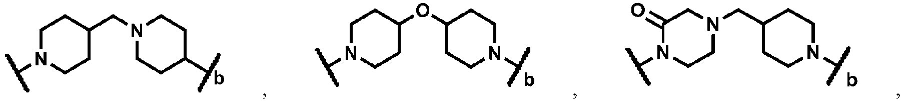 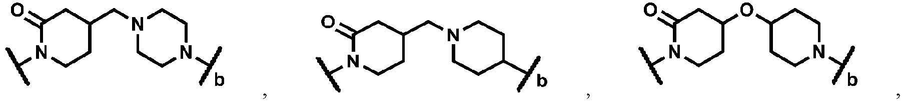 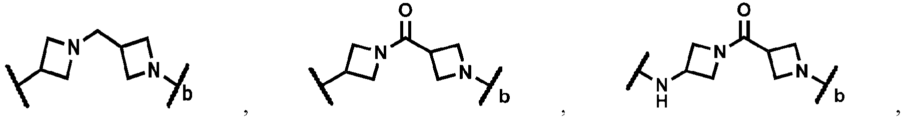 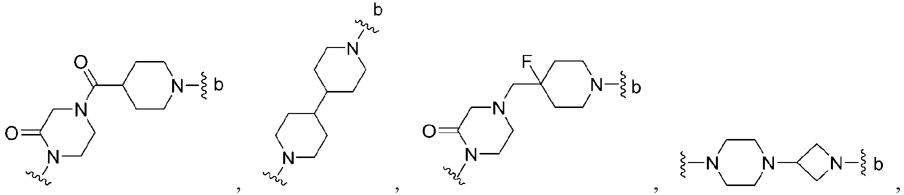 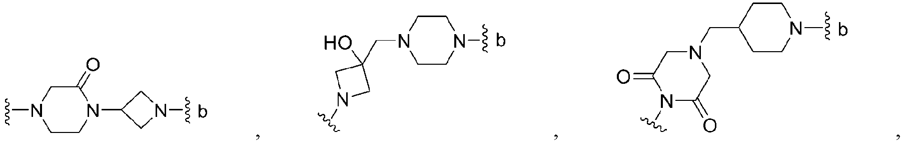 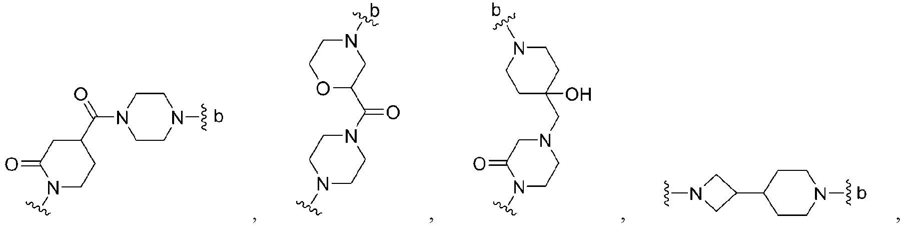 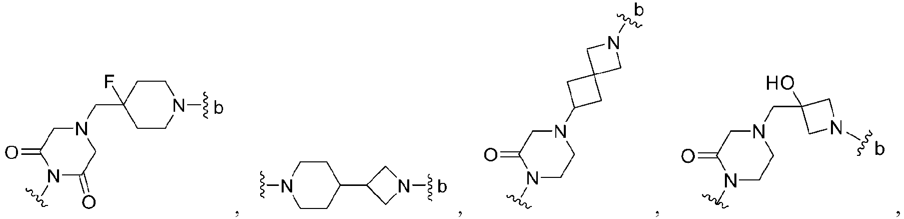 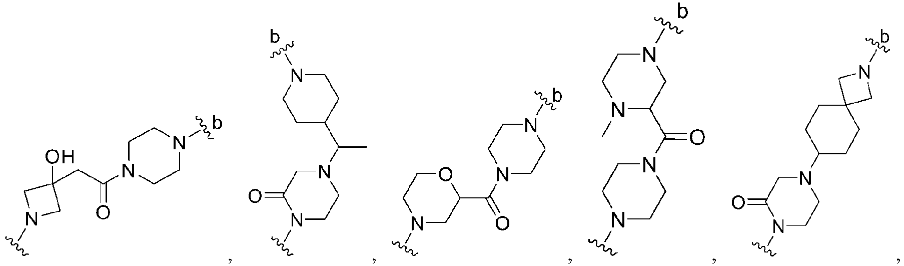 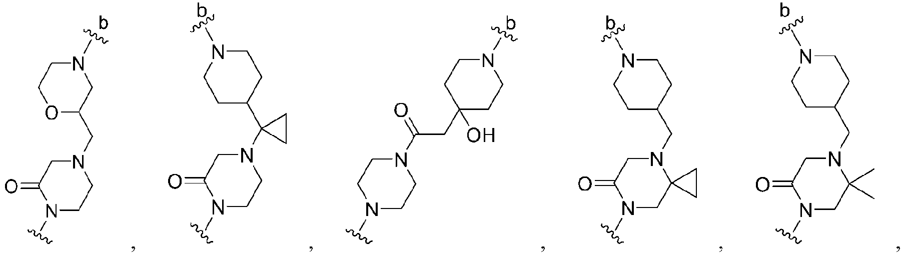 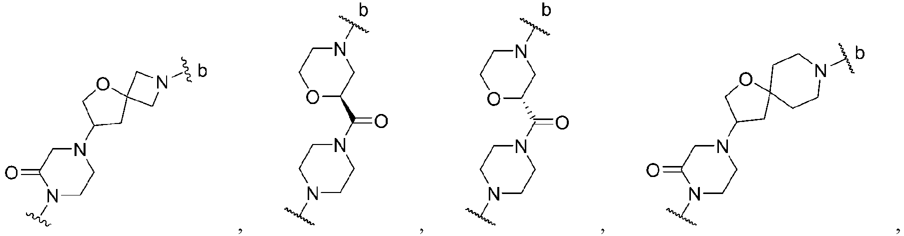 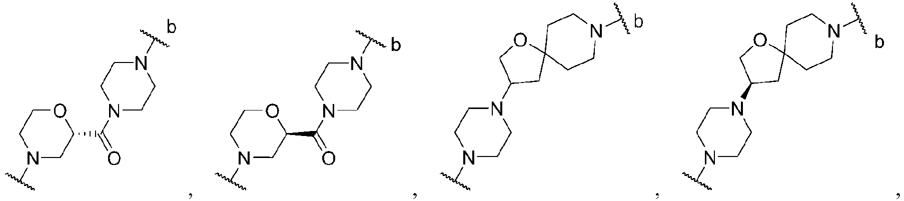 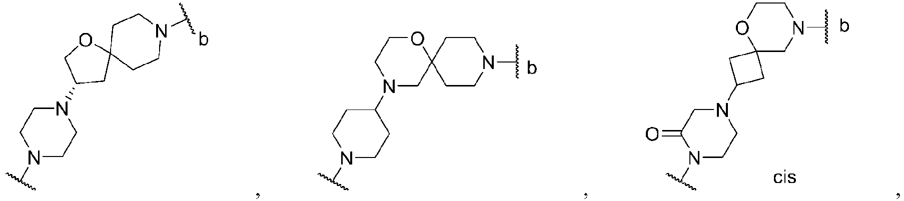 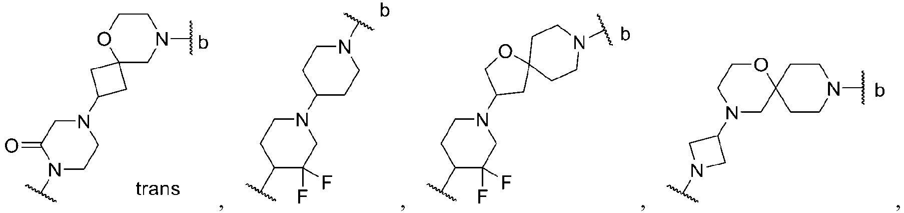 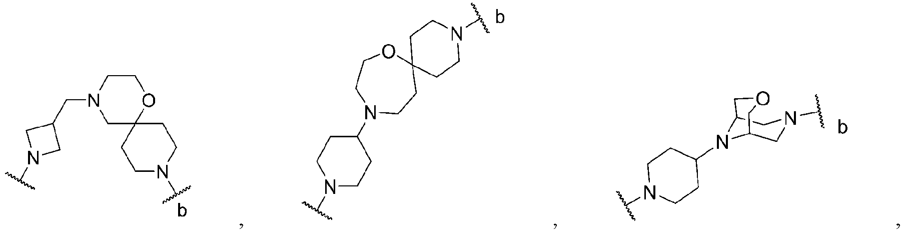 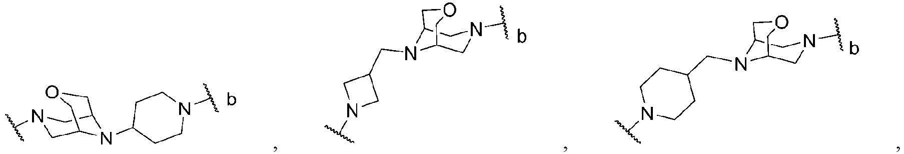 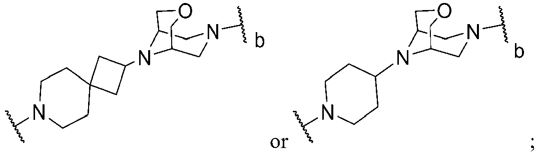 side b is connected to B;
(2) in condition i, L is G1 or G2:
G1: 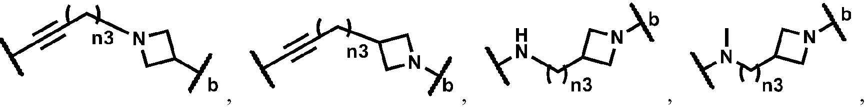 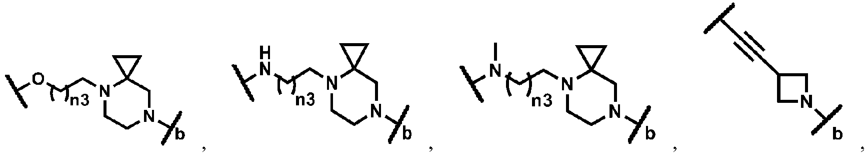 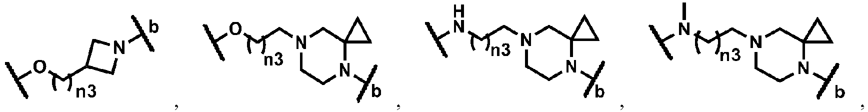 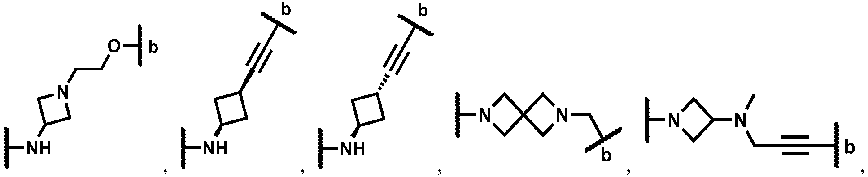 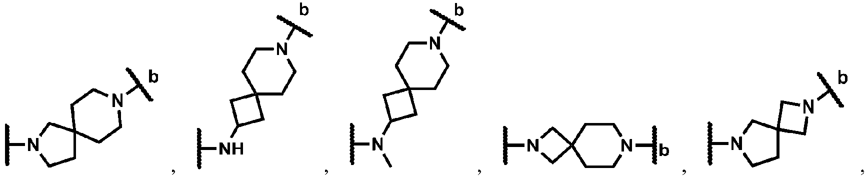 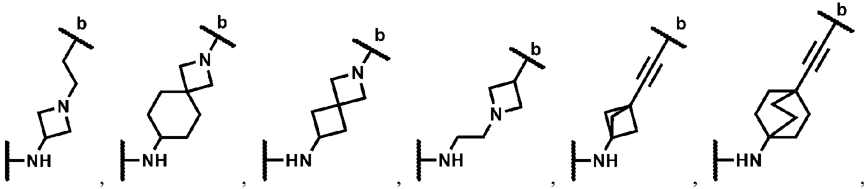 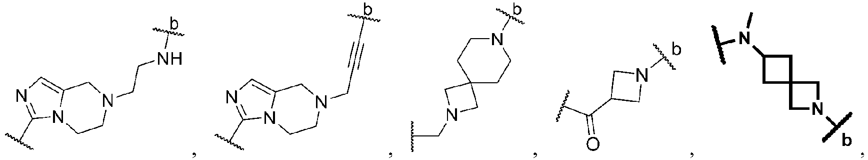 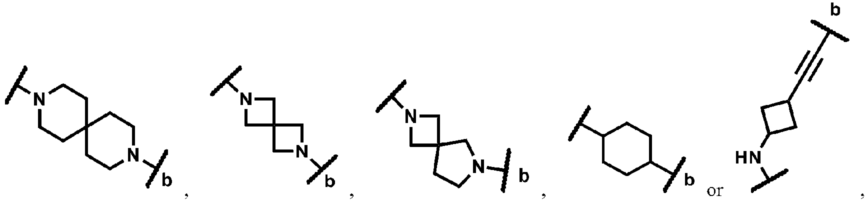
wherein n1 is any integer from 1 to 8, n2 is any integer from 1 to 3, n3 is any integer from 1 to 5, and side b is connected to B; 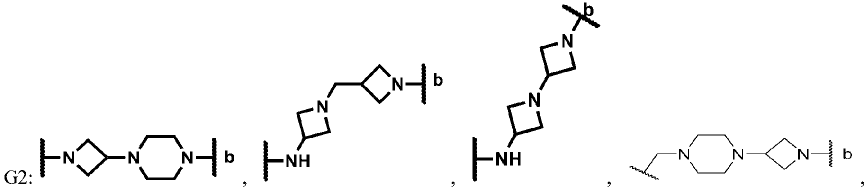 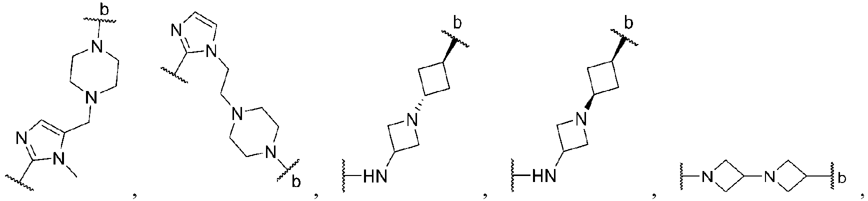 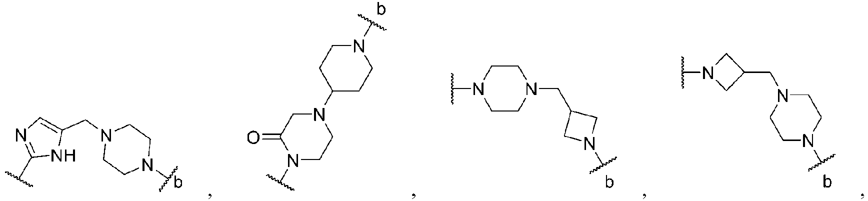 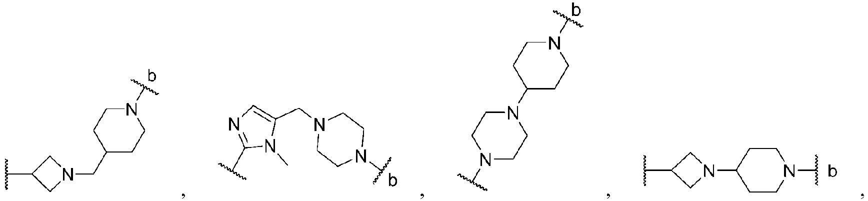 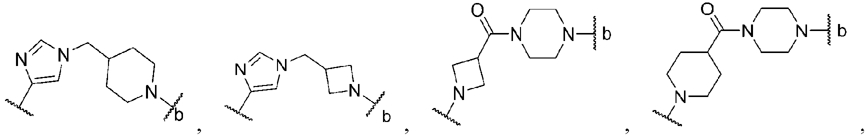 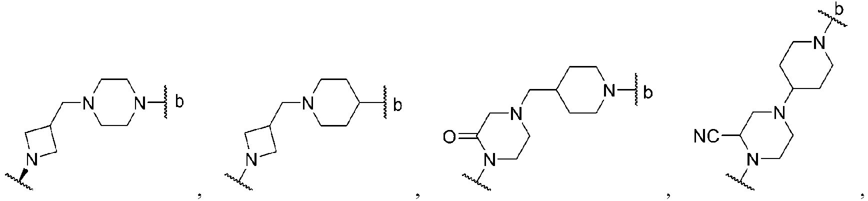 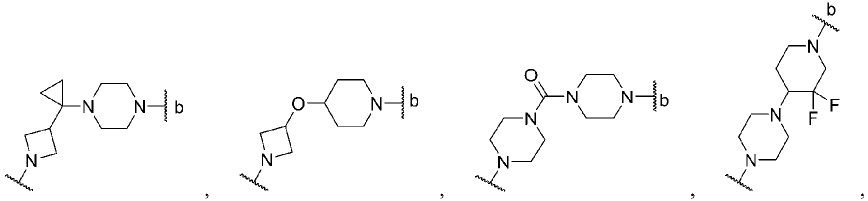 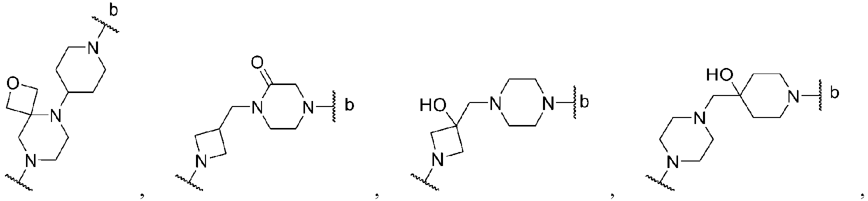 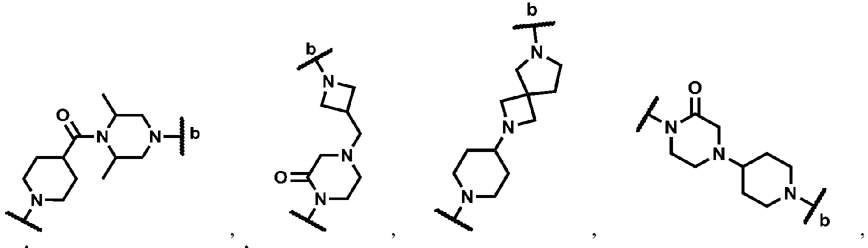 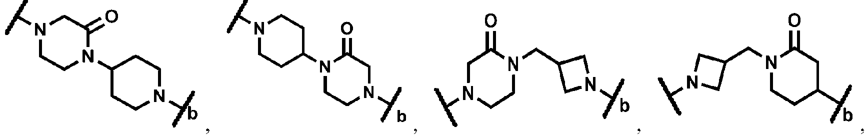 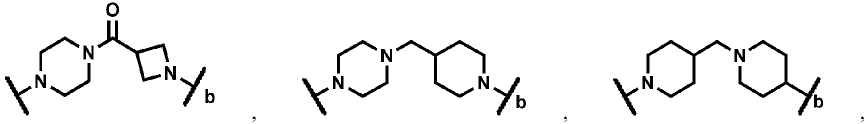 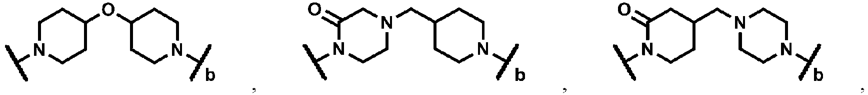 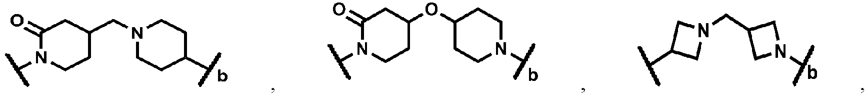 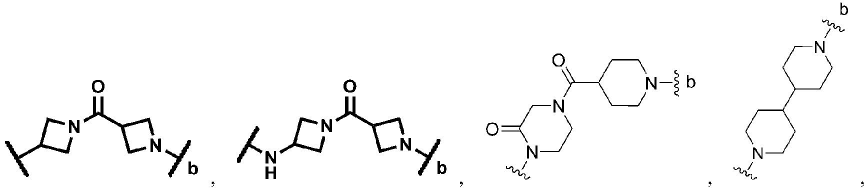 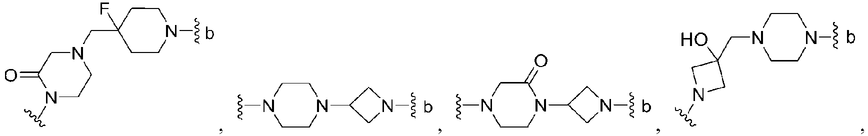 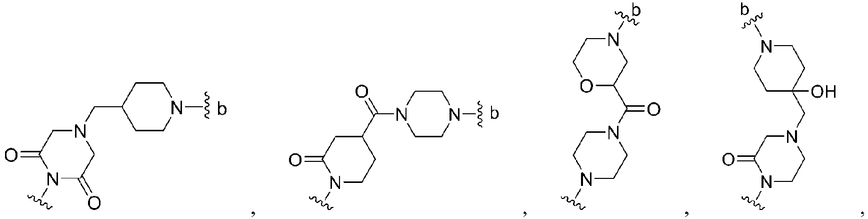 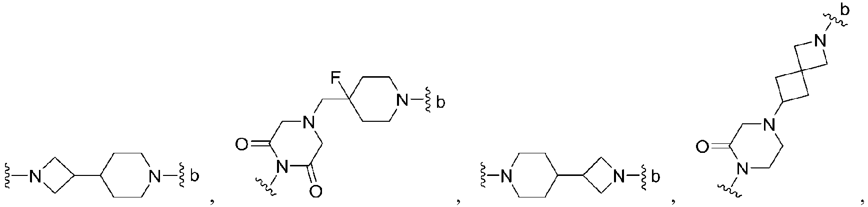 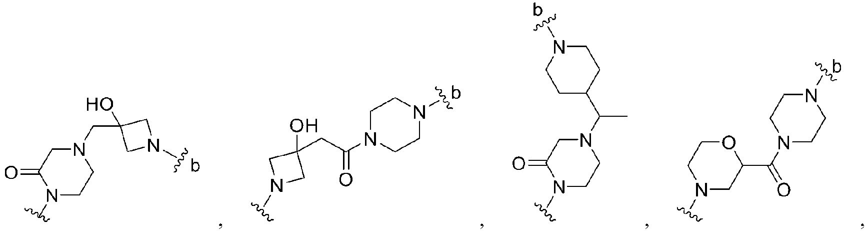 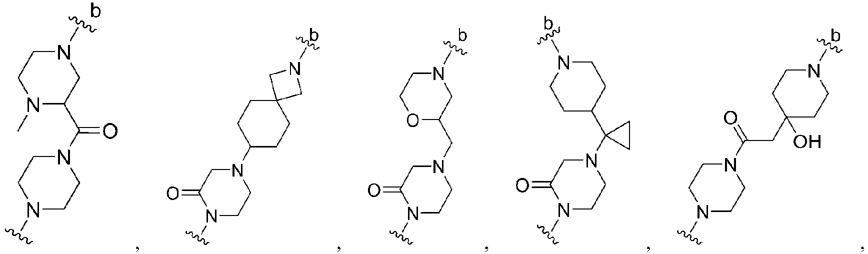 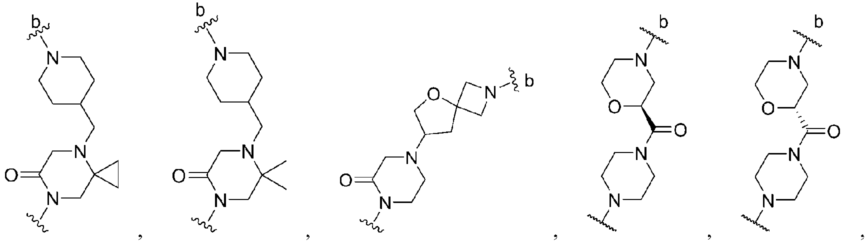 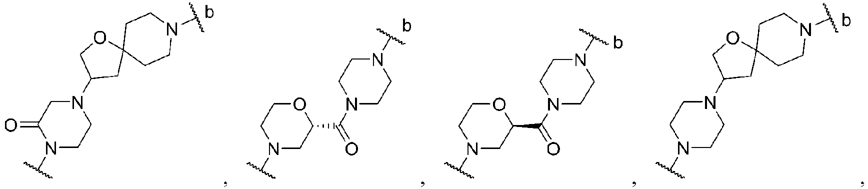 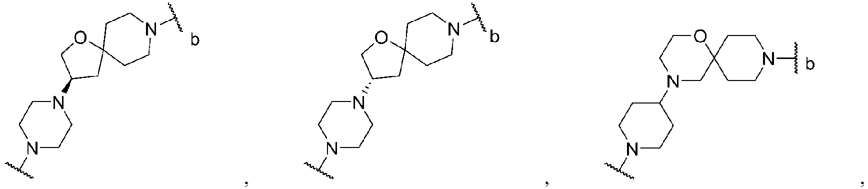 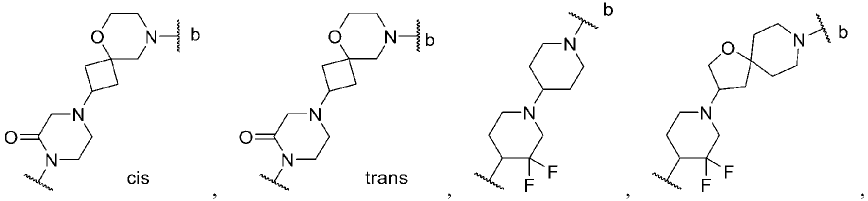 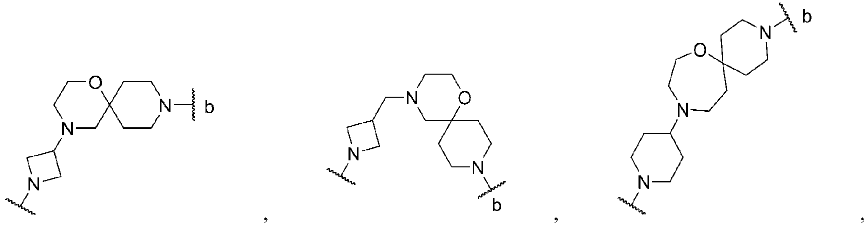 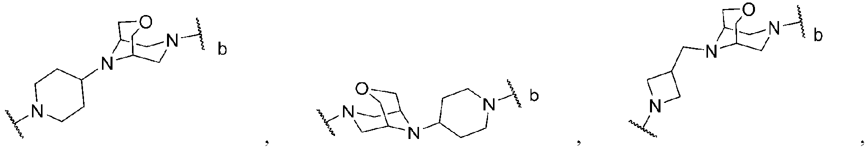 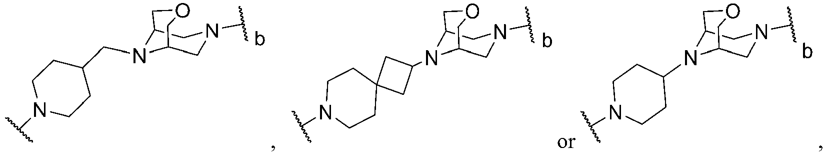
side b is connected to B.

10. The compound represented by formula (I), the pharmaceutically acceptable salt thereof, the solvate thereof or the solvate of the pharmaceutically acceptable salt thereof according to claim 2 or 5, wherein,
L is 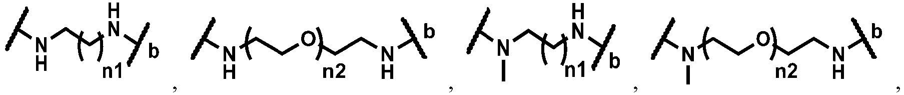 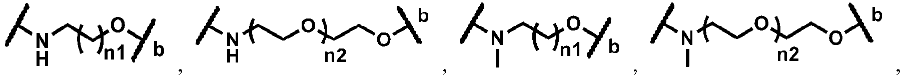 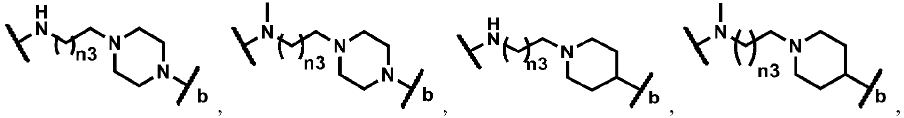 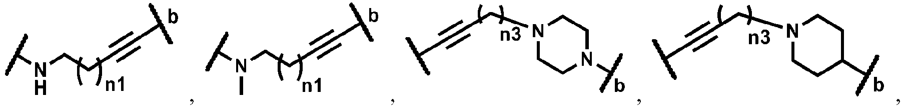 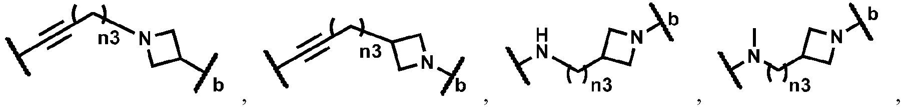 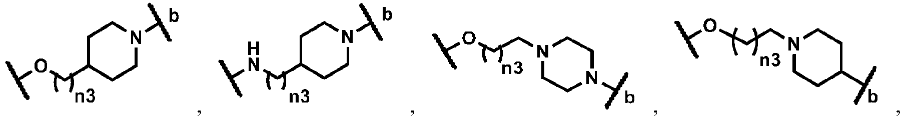 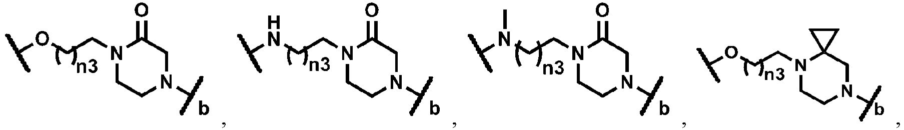 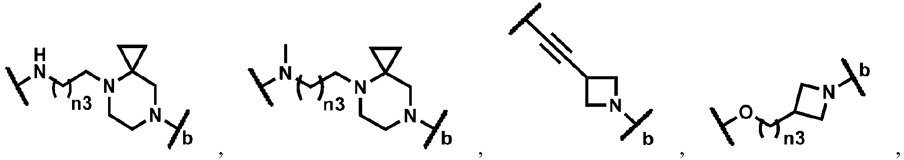 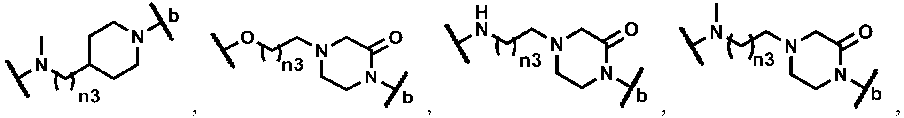 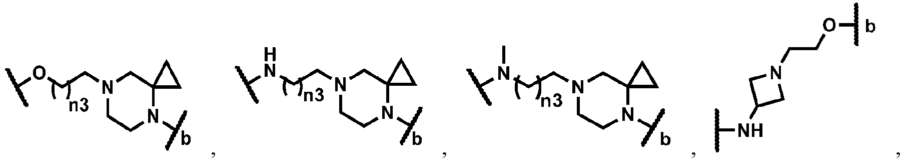 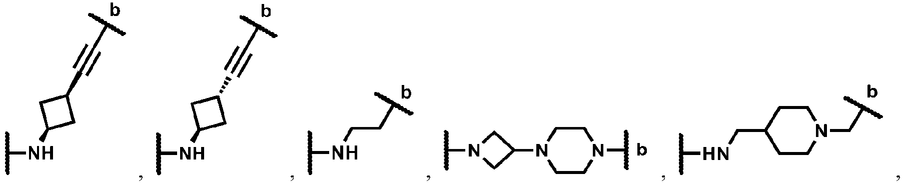 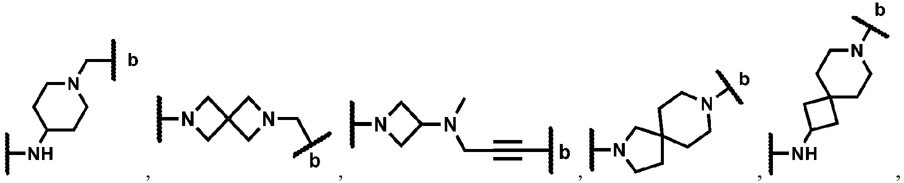 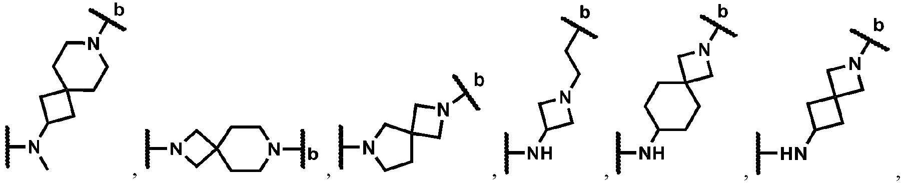 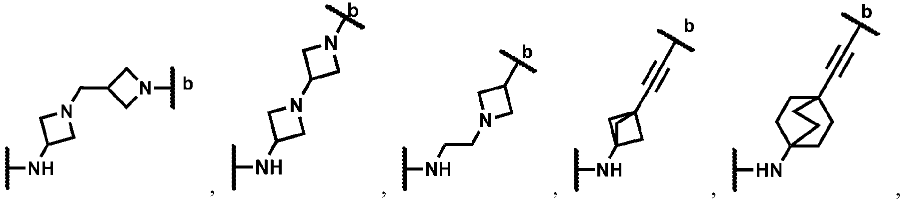 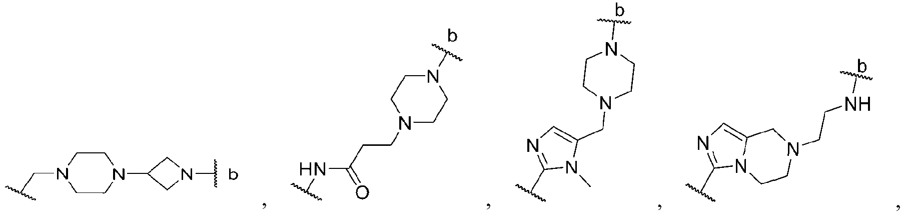 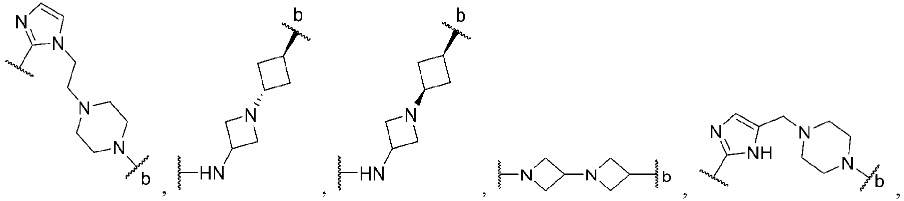 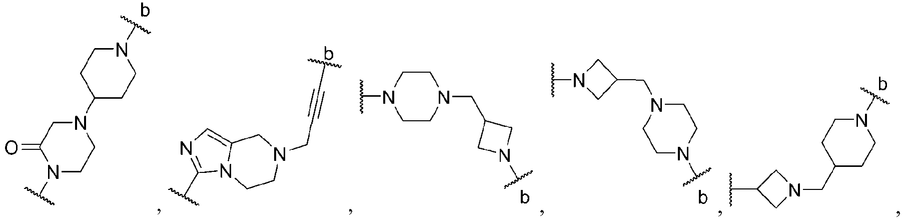 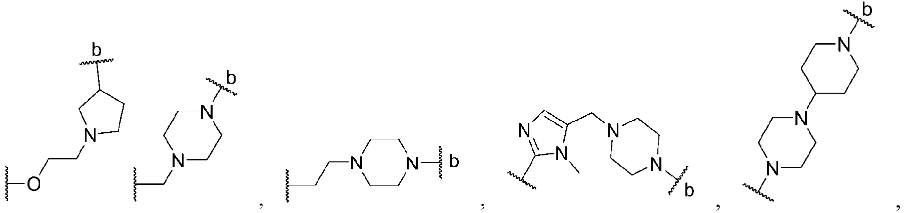 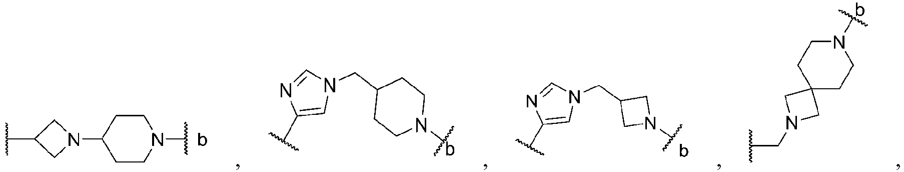 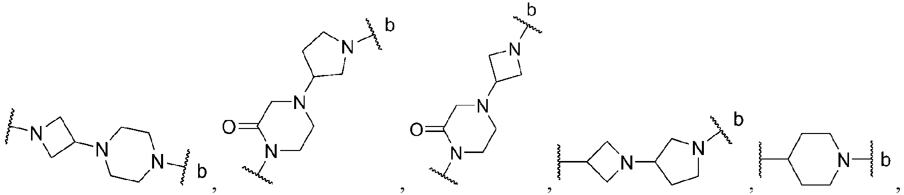 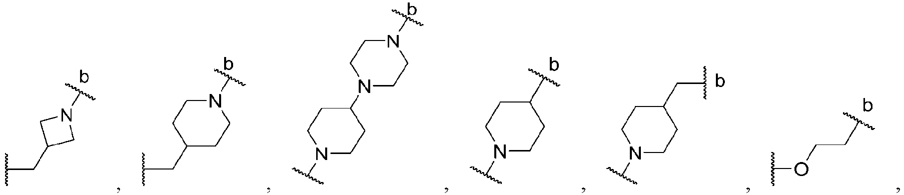 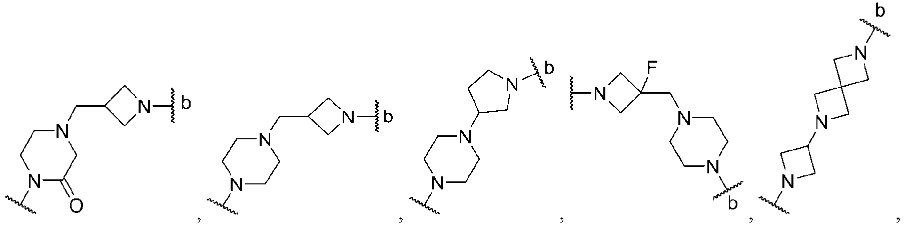 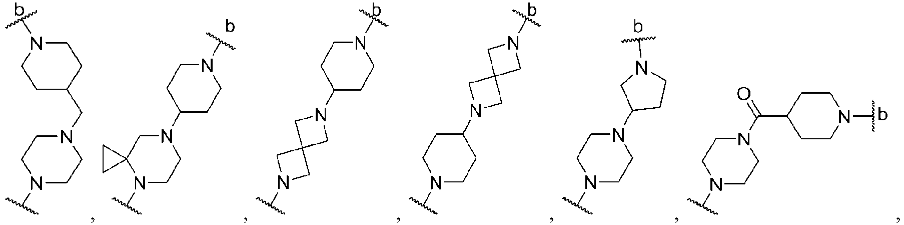 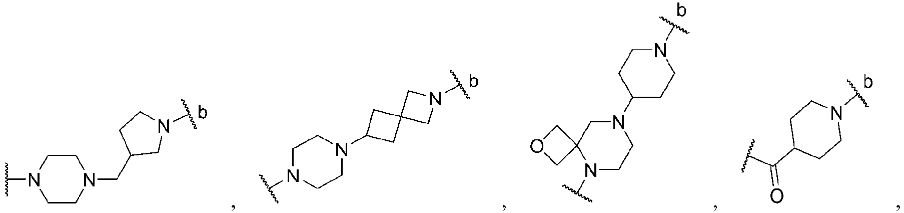 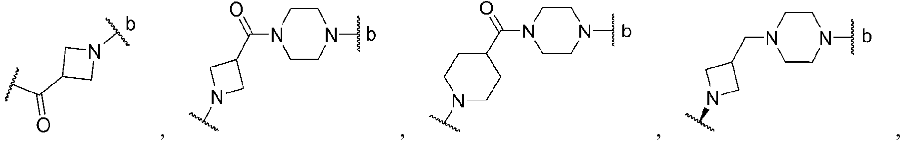 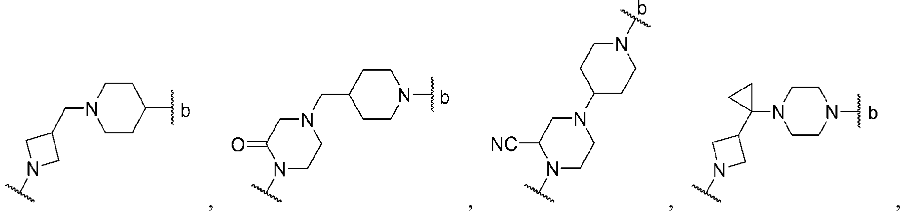 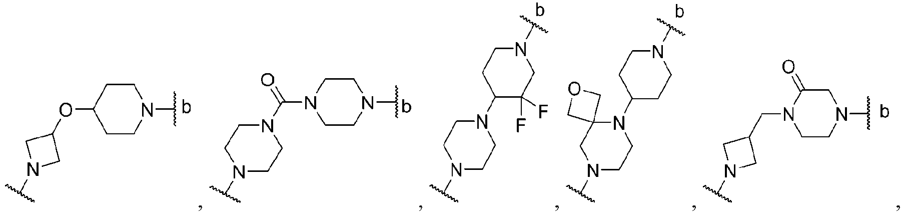 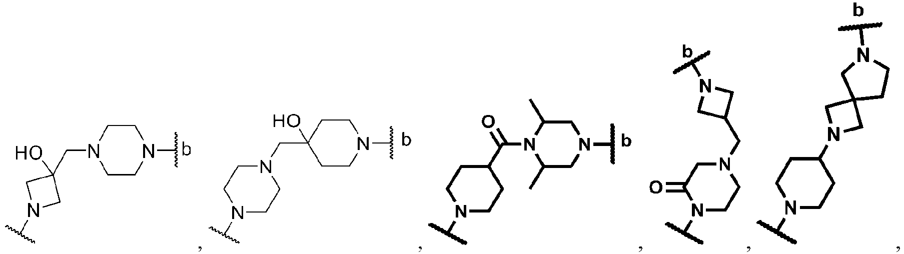 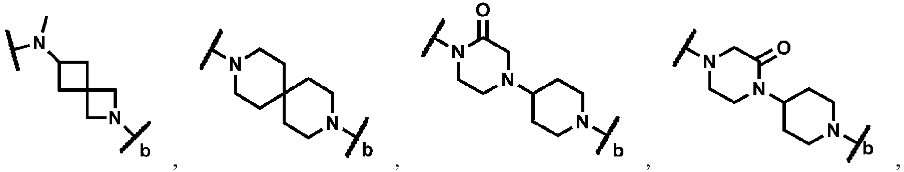 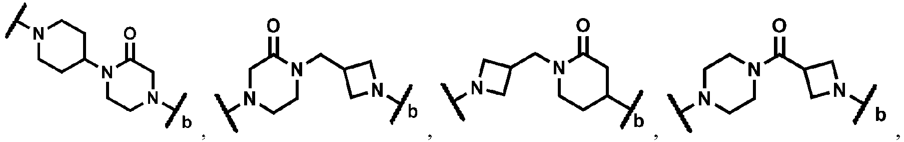 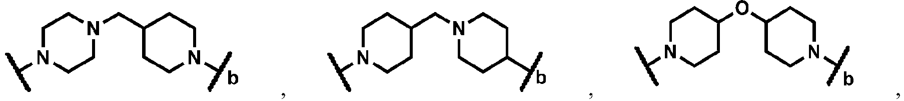 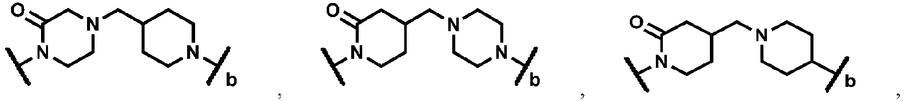 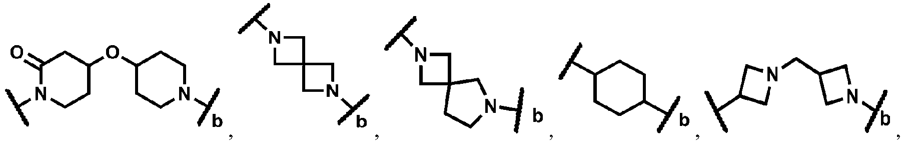 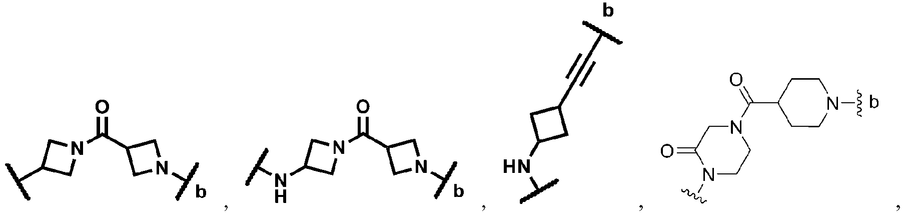 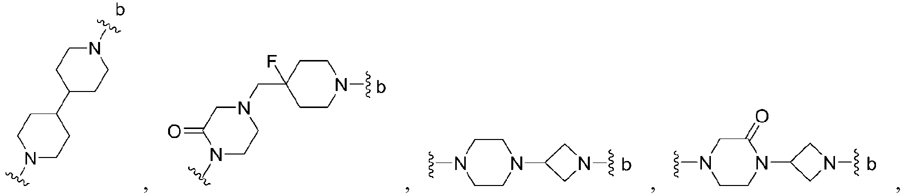 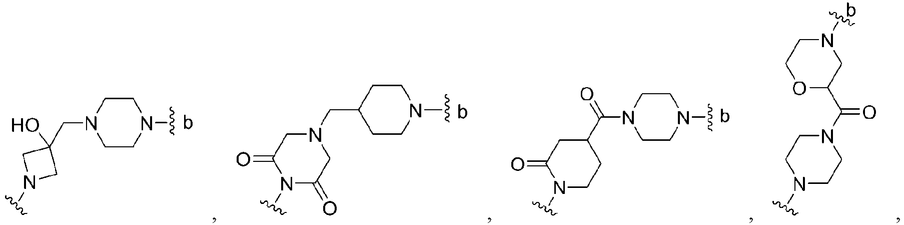 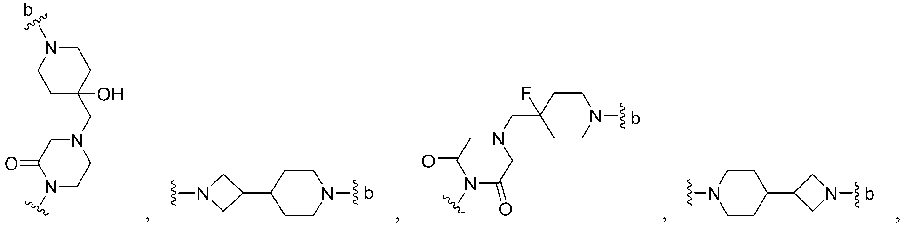 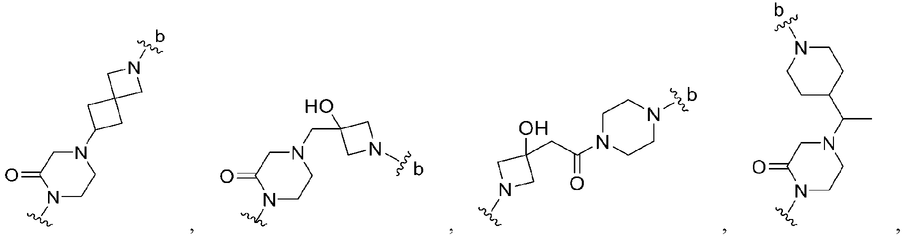 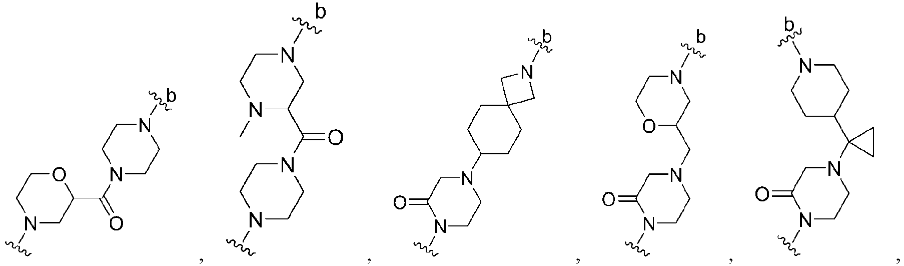 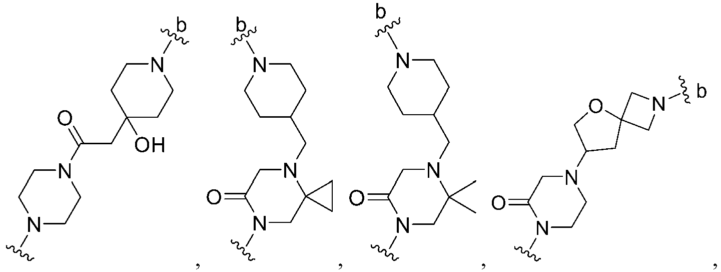 wherein n1 is any integer from 1 to 8, n2 is any integer from 1 to 3, n3 is any integer from 1 to 5, and side b is connected to B; preferably, in Scheme i, L is any group of the above groups except 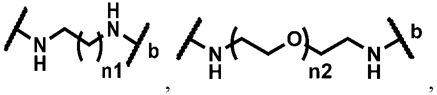 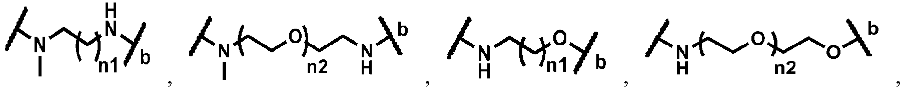 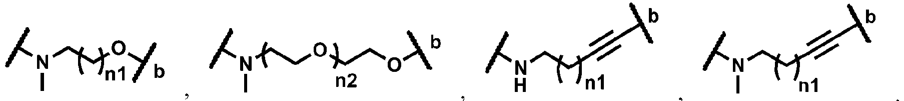 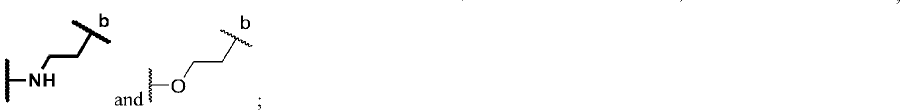
preferably, L is: 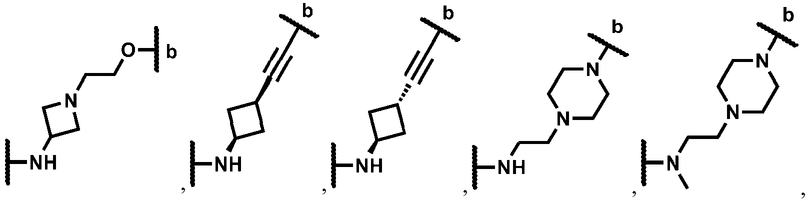 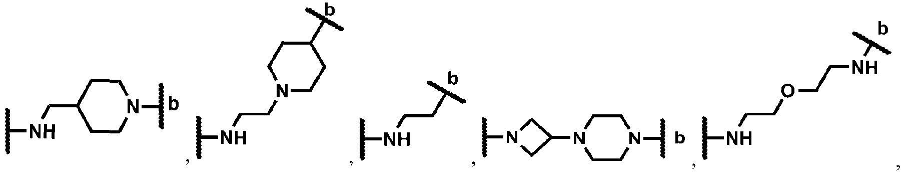 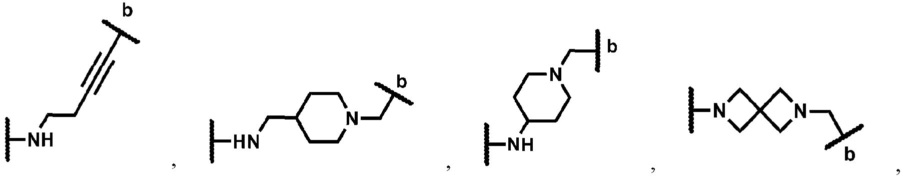 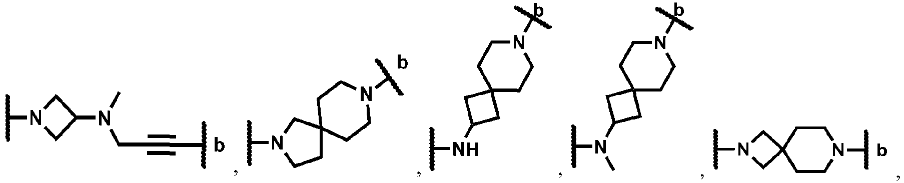 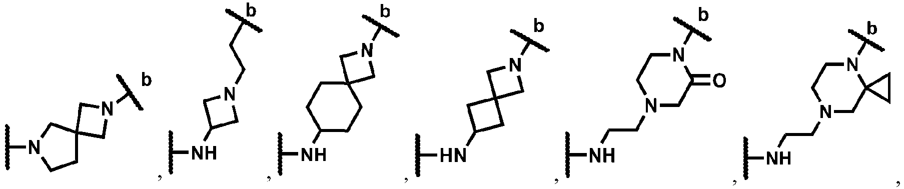 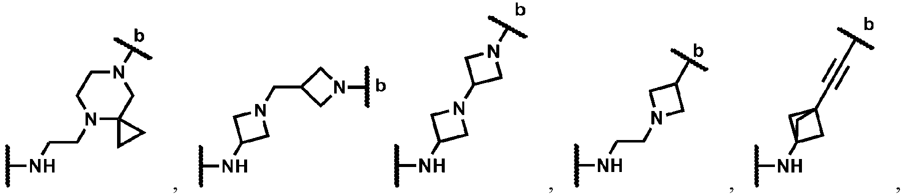 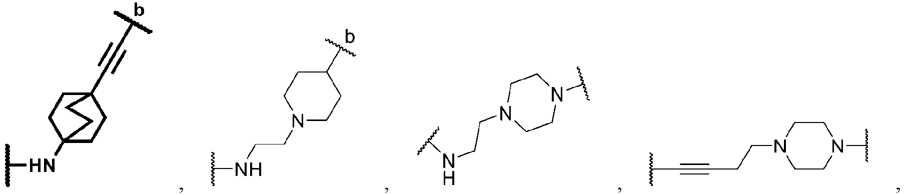 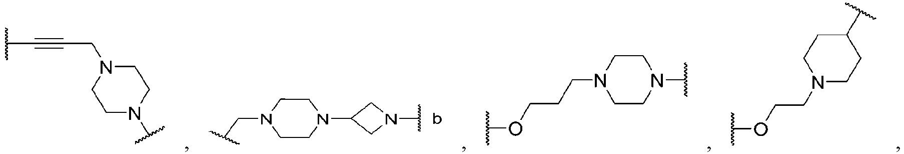 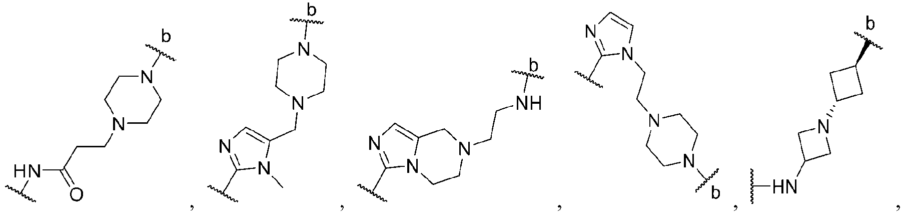 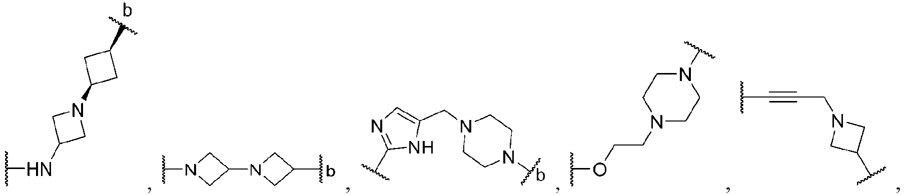 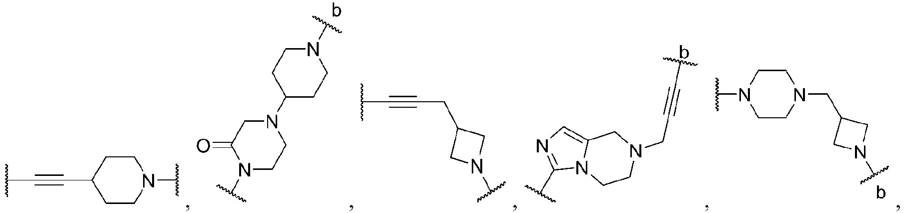 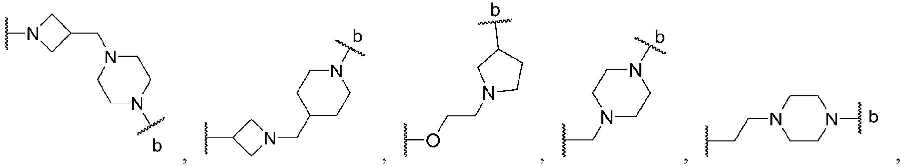 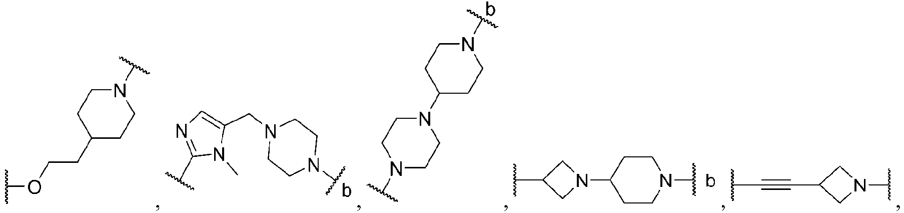 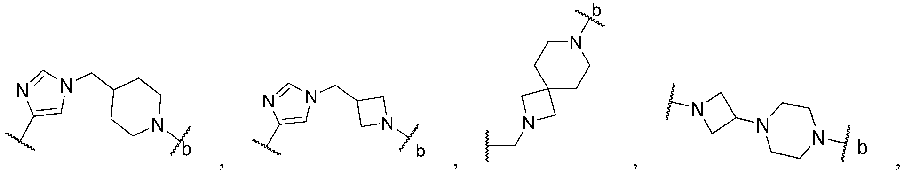 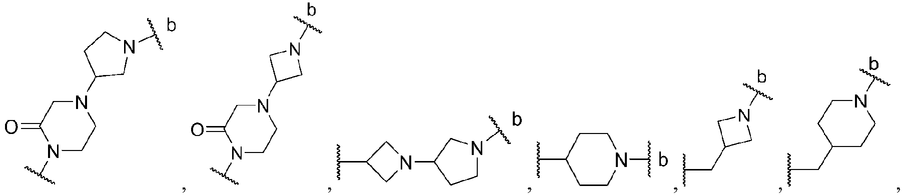 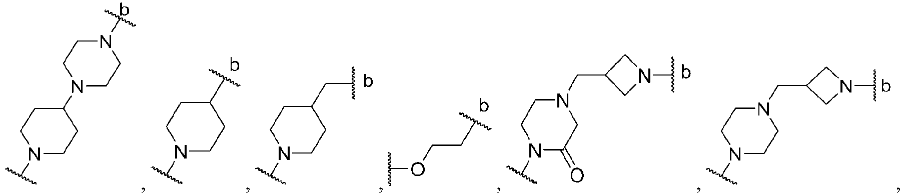 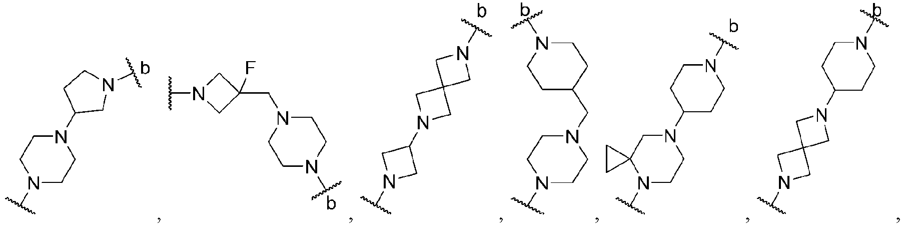 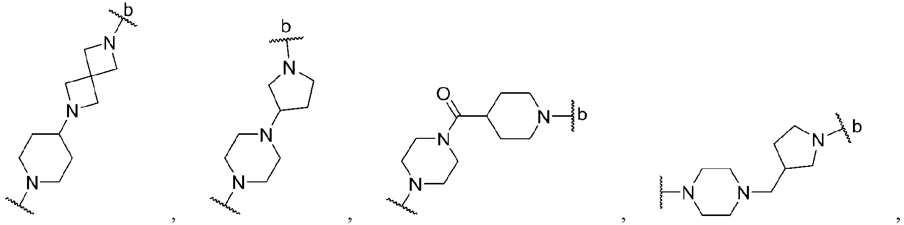 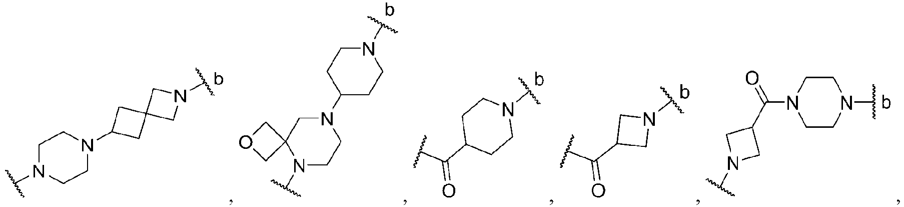 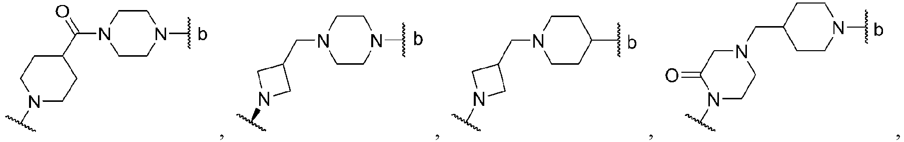 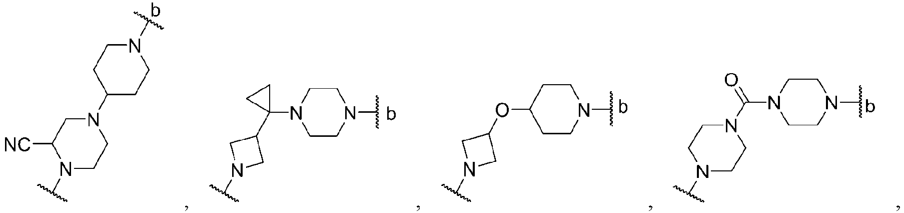 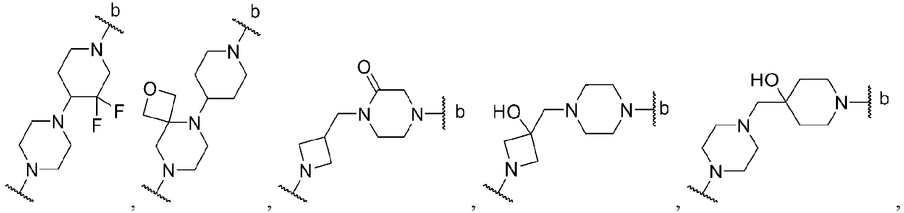 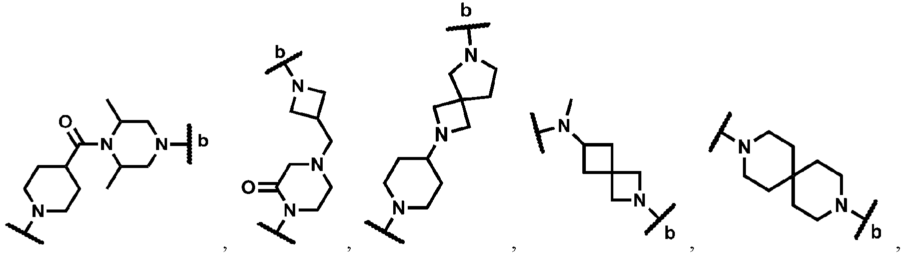 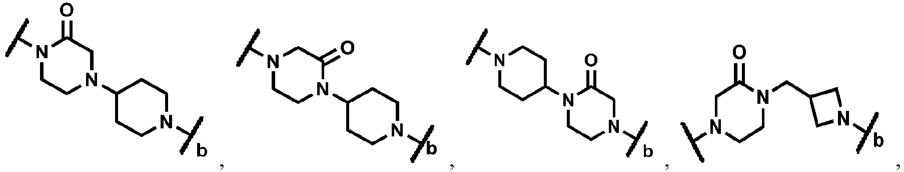 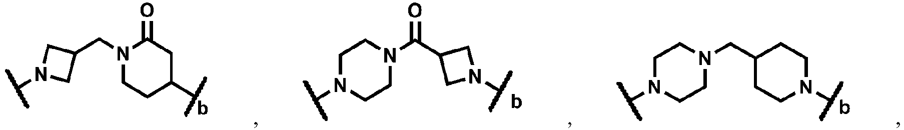 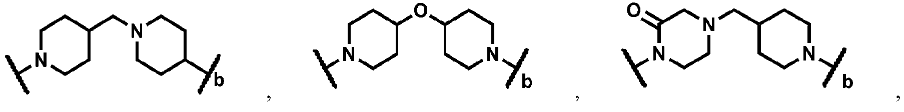 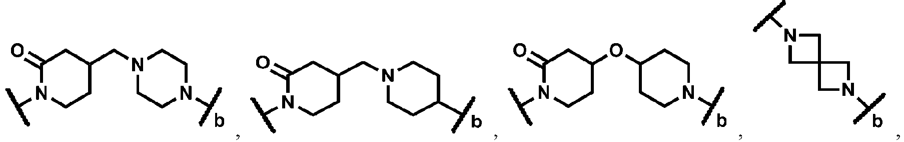 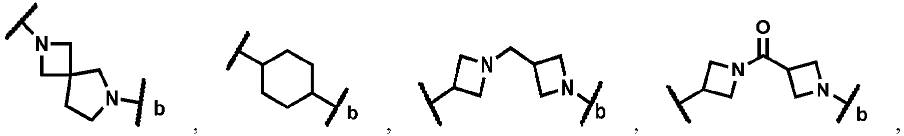 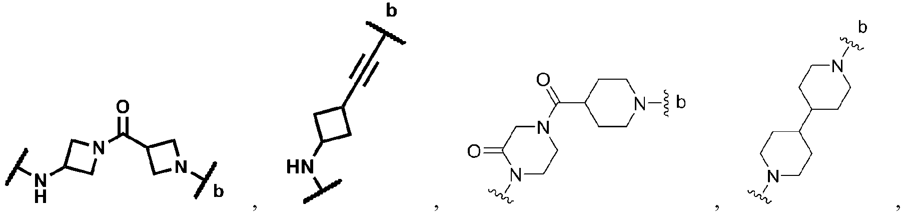 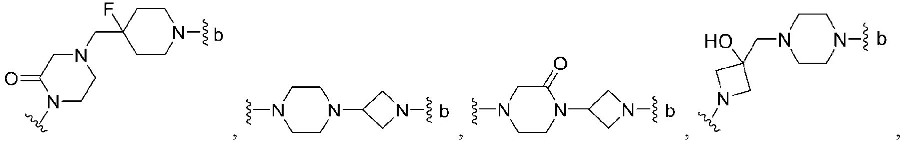 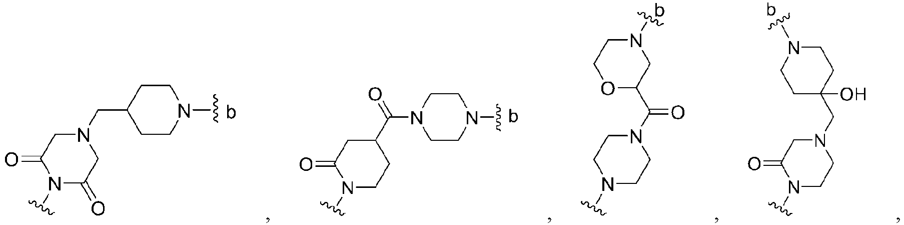 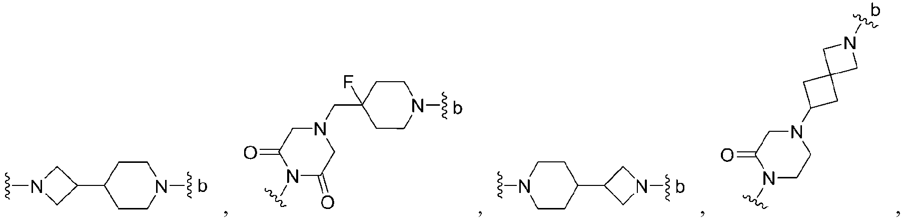 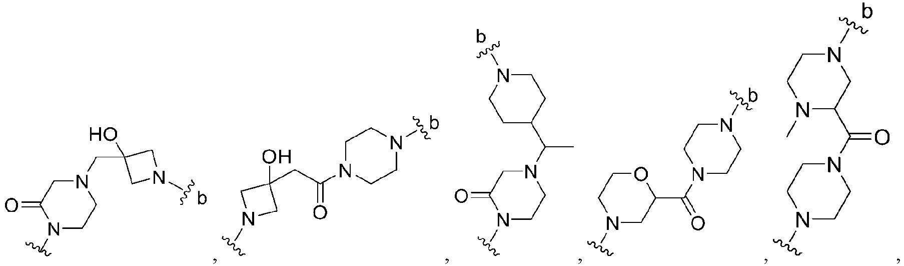 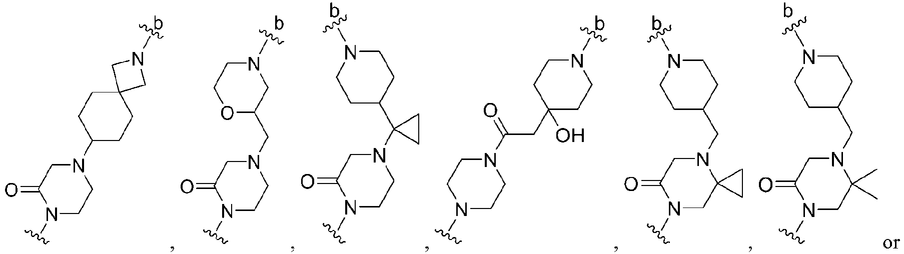 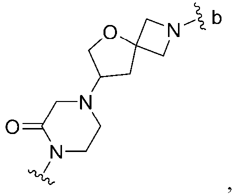 wherein side b is connected to B; preferably, in Scheme i, L is any one of the above groups except

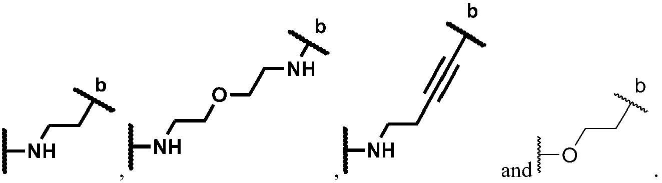

11. The compound represented by formula (I), the pharmaceutically acceptable salt thereof, the solvate thereof or the solvate of the pharmaceutically acceptable salt thereof according to any one of claims 1-6, wherein the compound represented by formula (I), the pharmaceutically acceptable salt thereof, the solvate thereof or the solvate of the pharmaceutically acceptable salt thereof satisfies one or more of the following conditions:
(1) R³ is H or methyl;
(2) R² is -H, -F, -Cl, -CH₃, cyclopropyl, -OCH₃ 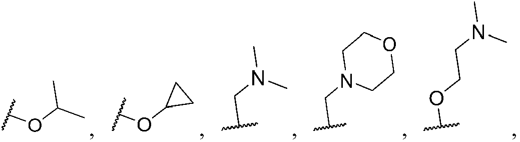 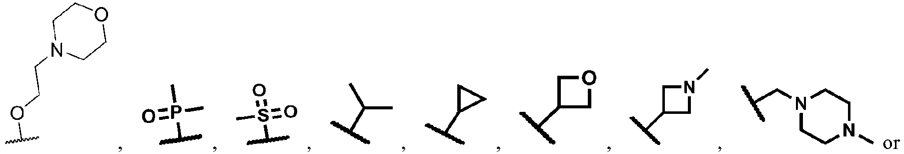 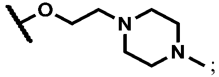
(3) A is 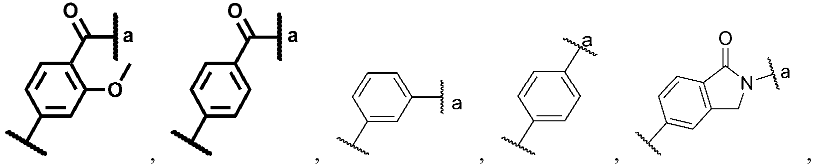 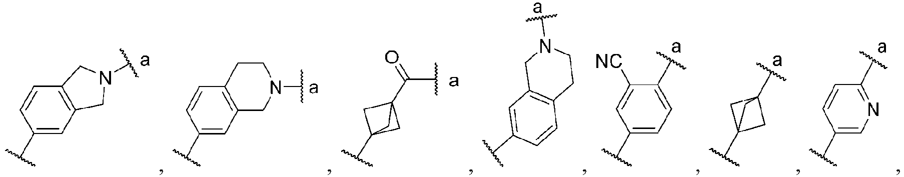 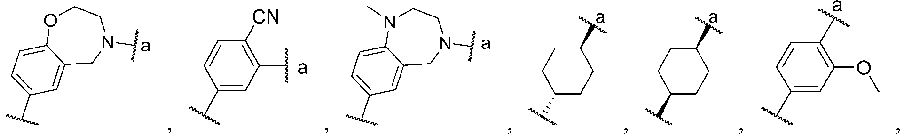 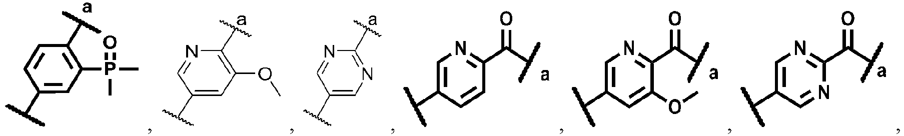 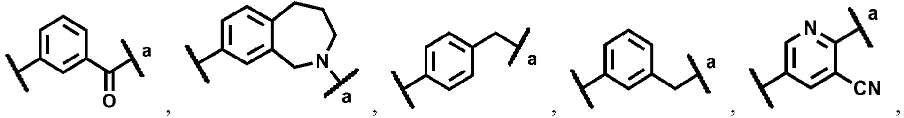 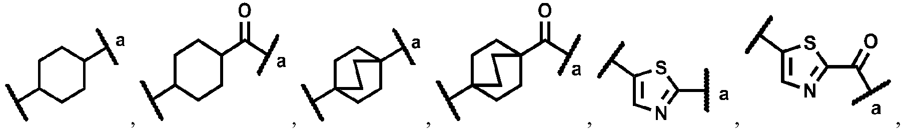 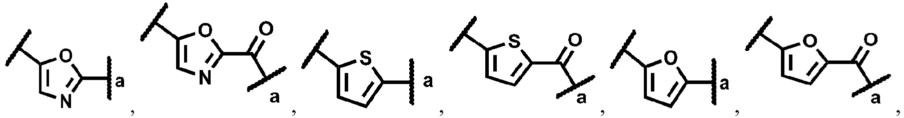 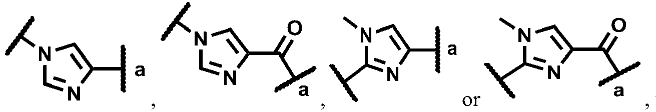 with side a connected to L;
(4) L is: 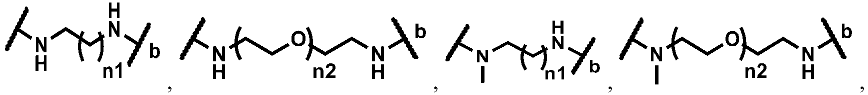 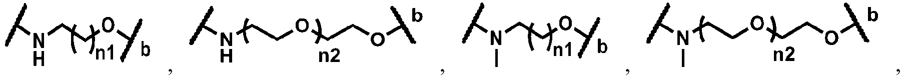 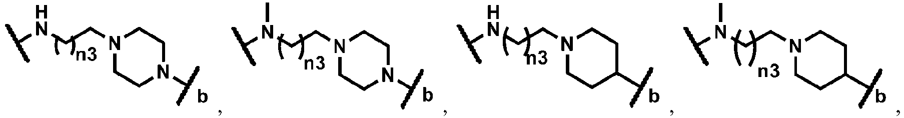 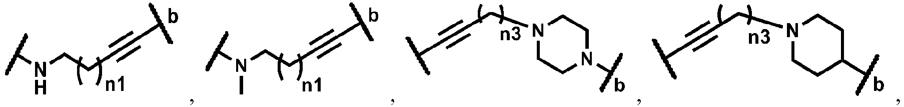 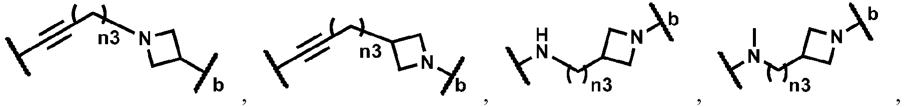 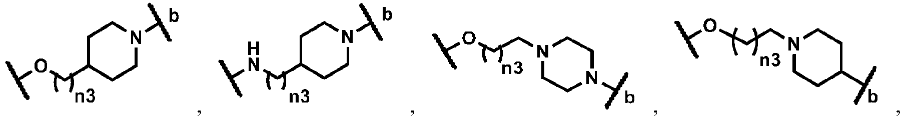 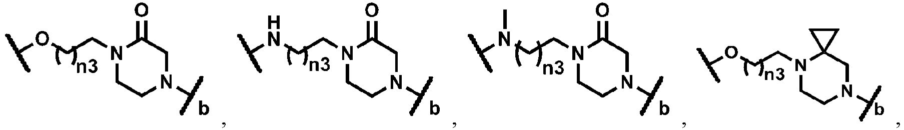 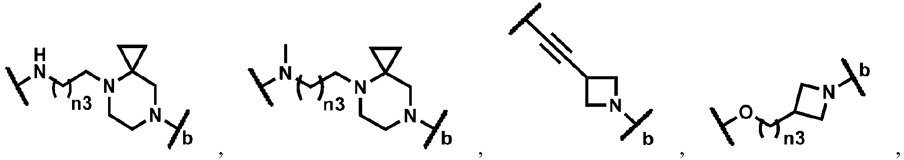 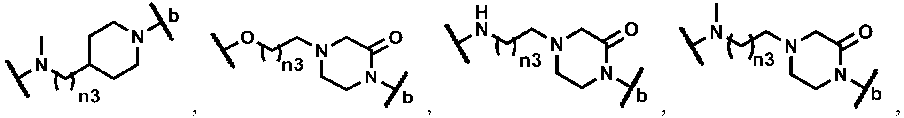 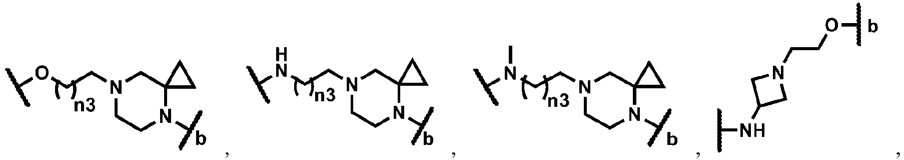 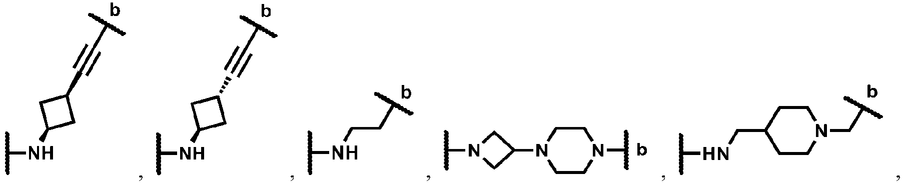 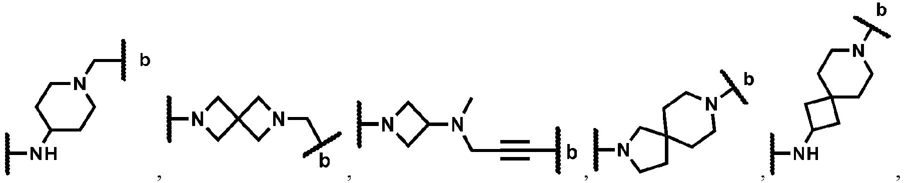 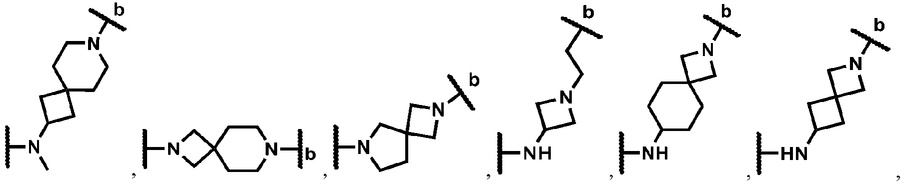 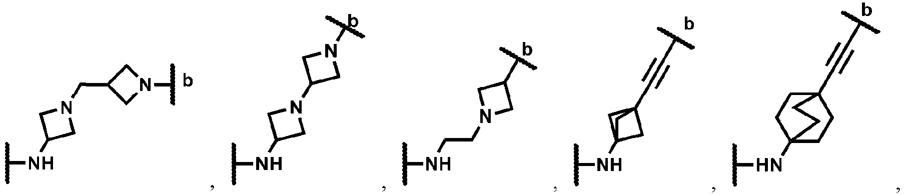 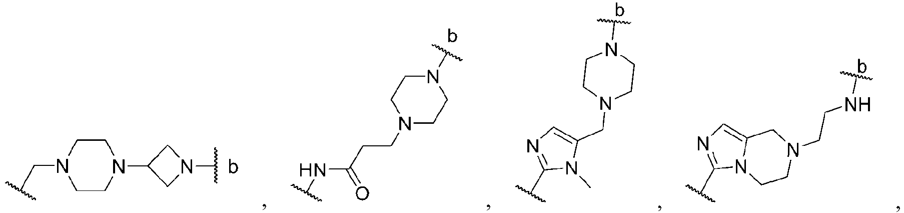 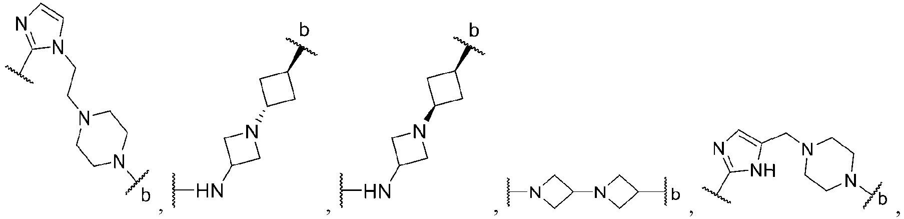 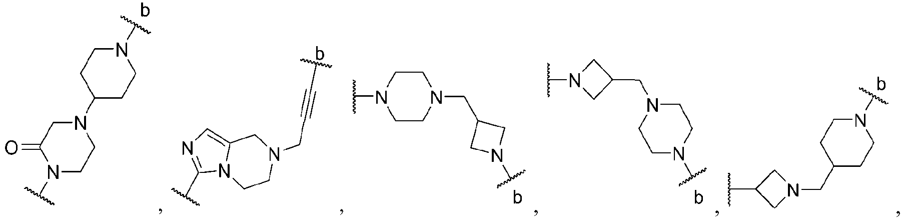 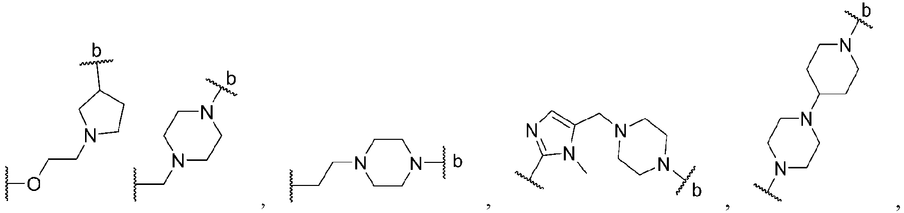 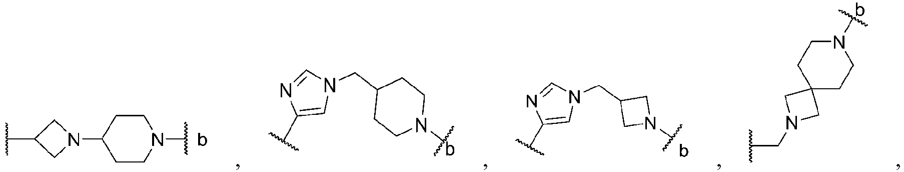 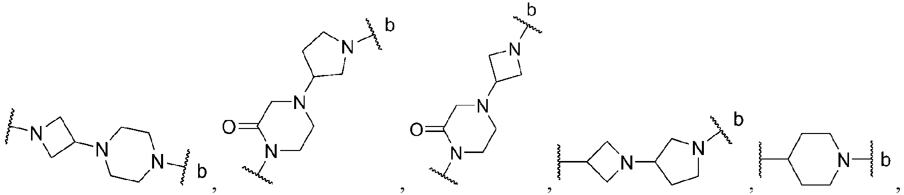 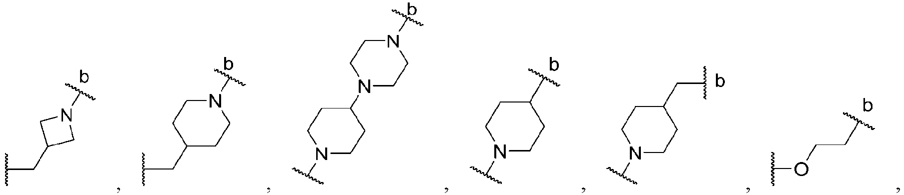 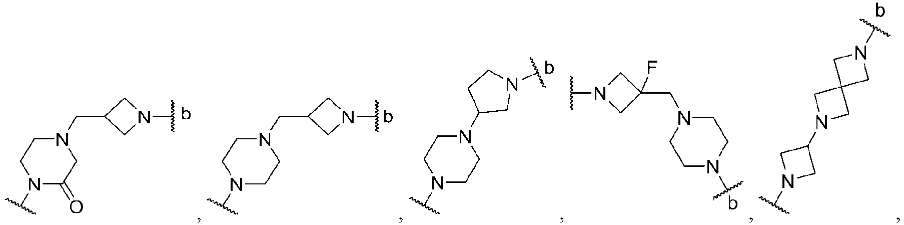 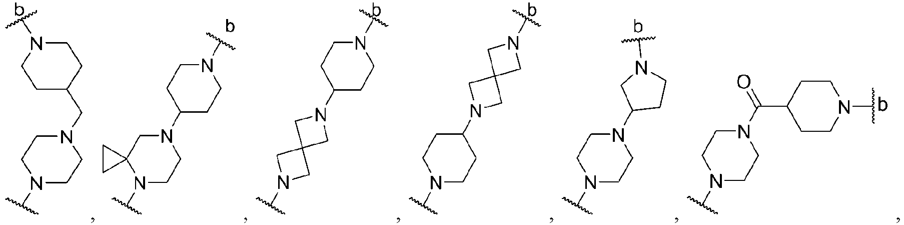 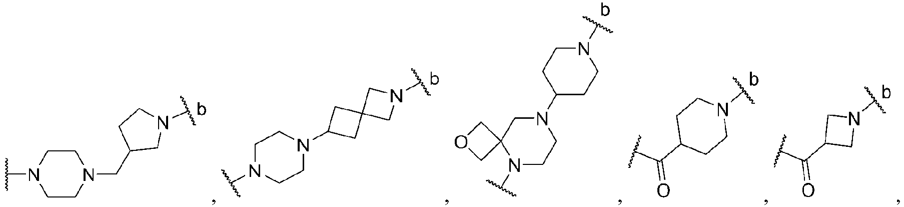 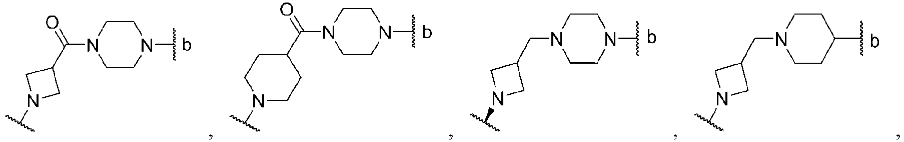 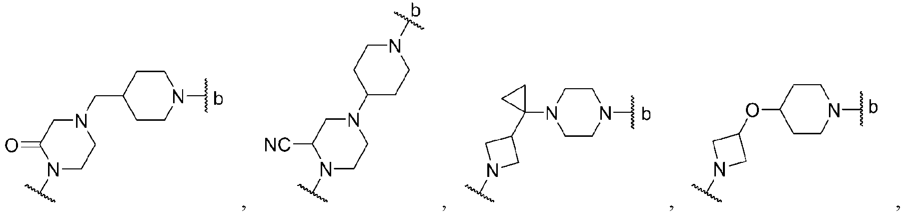 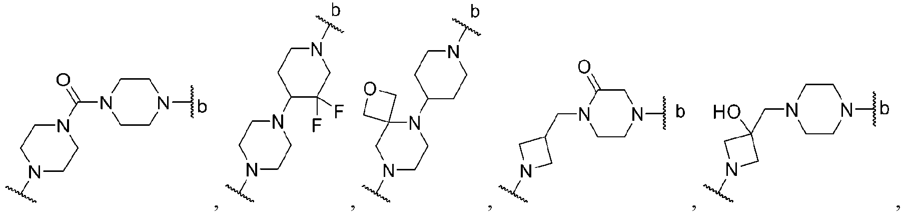 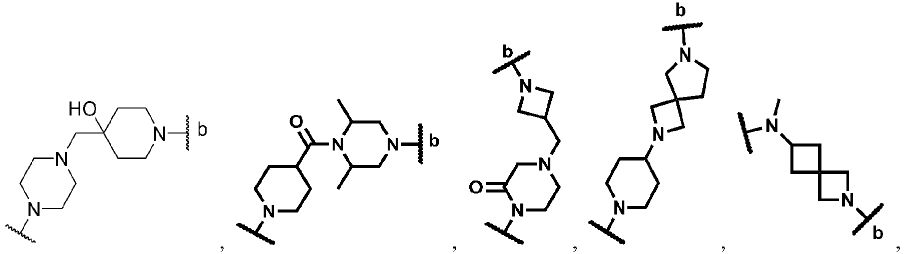 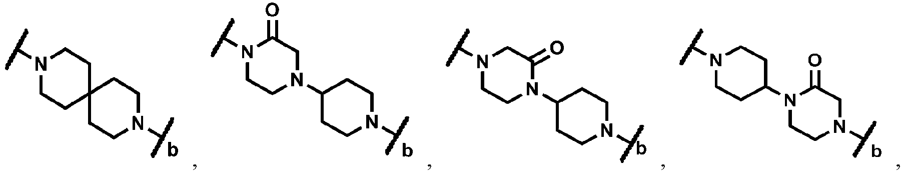 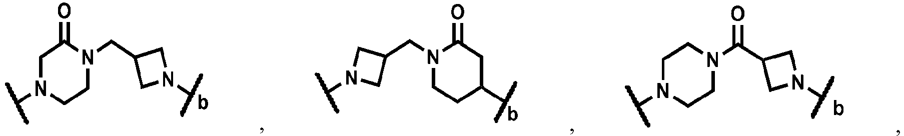 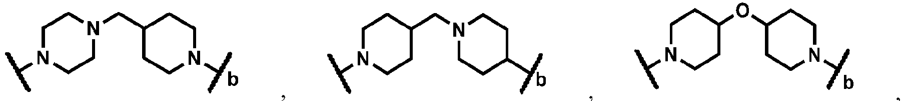 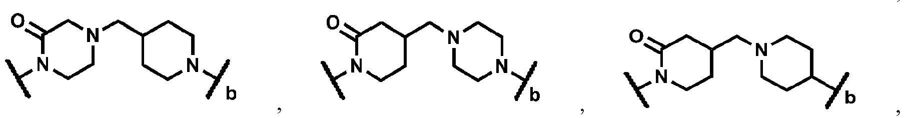 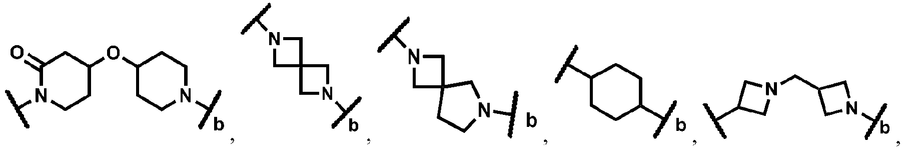 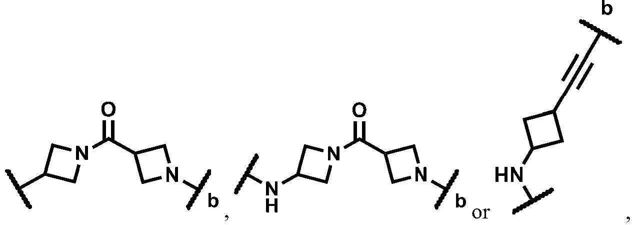 wherein n1 is any integer from 1 to 8, n2 is any integer from 1 to 3, n3 is any integer from 1 to 5, and side b is connected to B; preferably, in Scheme i, L is any group of the above groups except 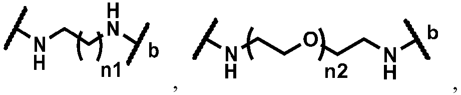 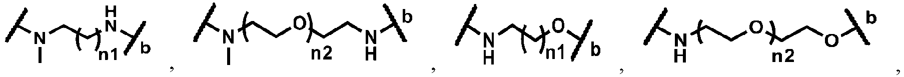 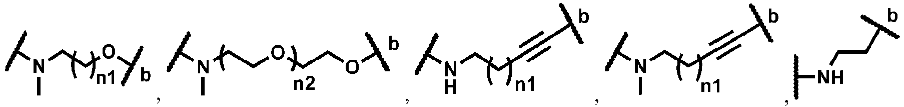 and 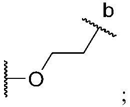
(5) B is: 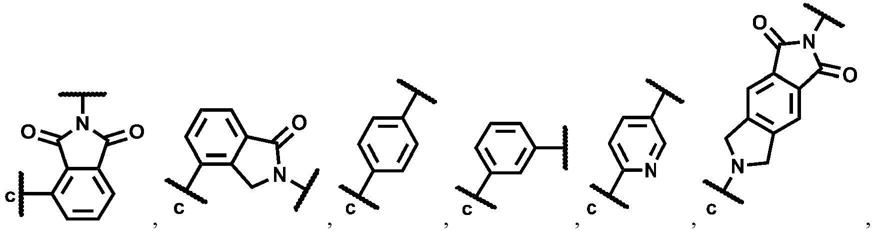 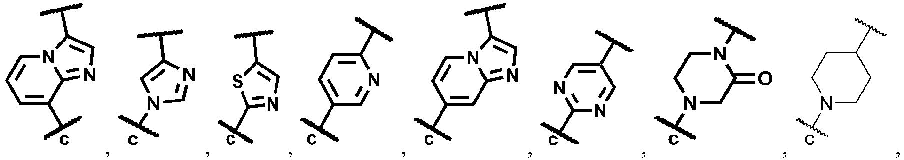 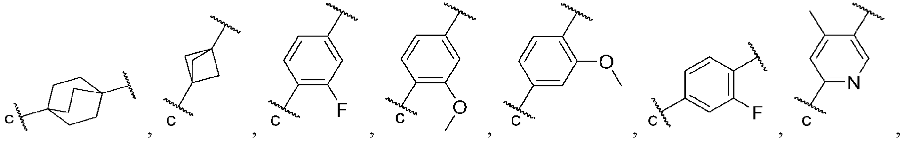 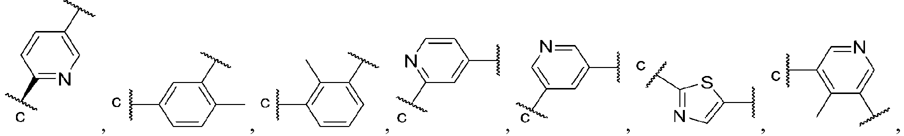 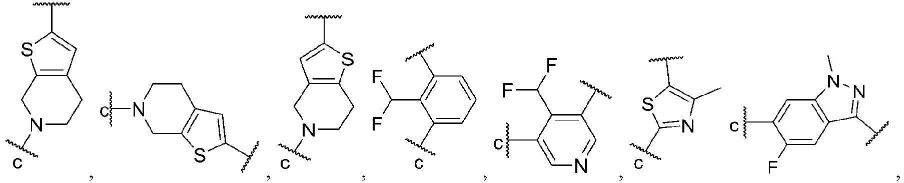 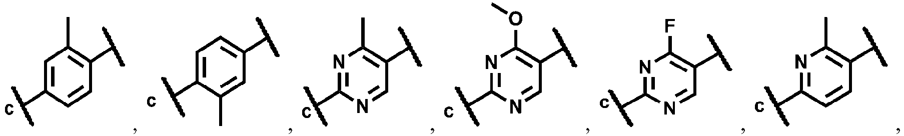 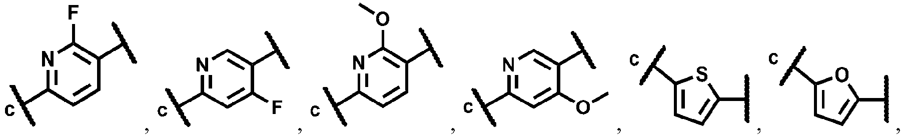 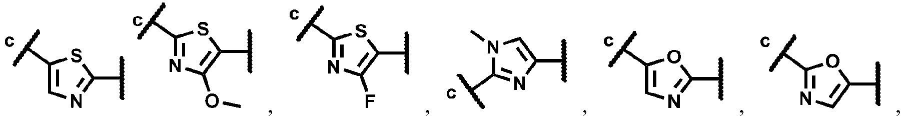 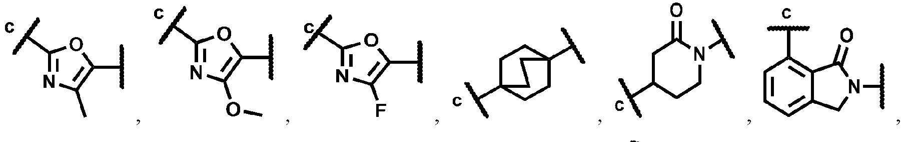 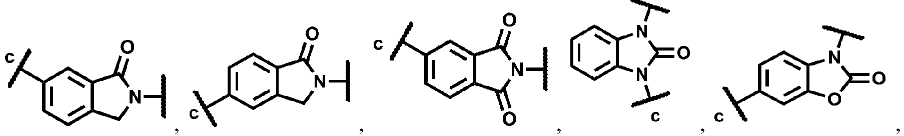 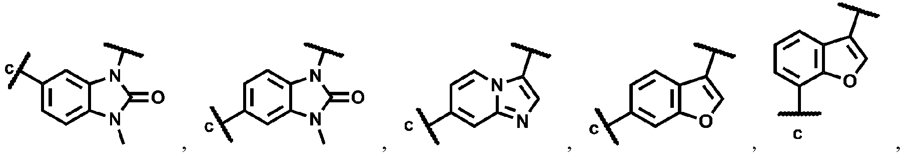 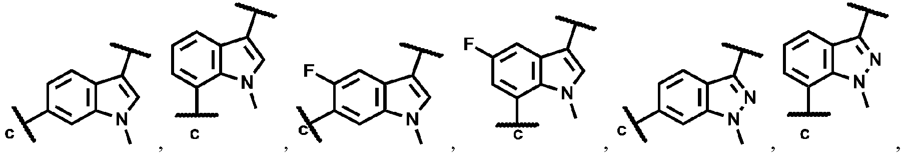 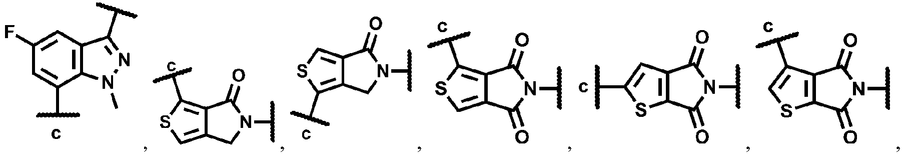 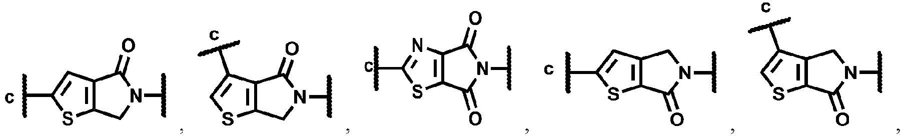 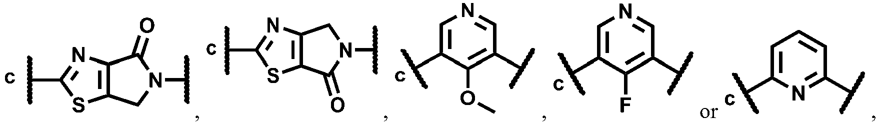 wherein side c is connected to L; in Scheme ii, B is any one of the above groups other than

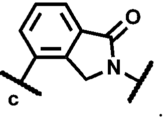

12. The compound represented by formula (I), the pharmaceutically acceptable salt thereof, the solvate thereof or the solvate of the pharmaceutically acceptable salt thereof according to any one of claims 1-6, wherein,
L is: 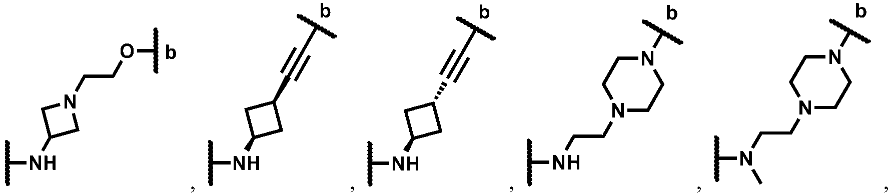 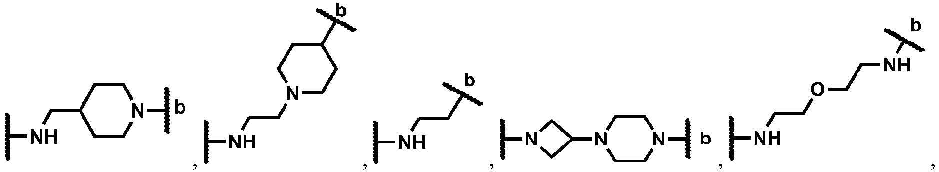 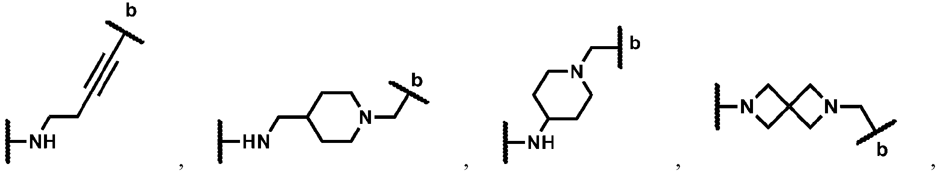 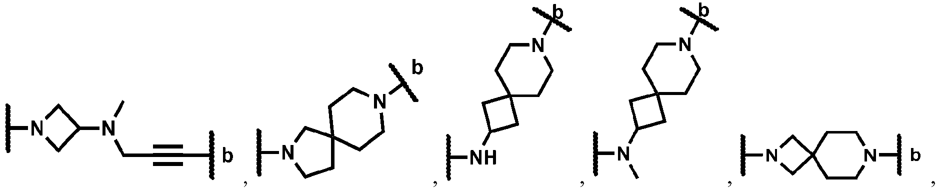 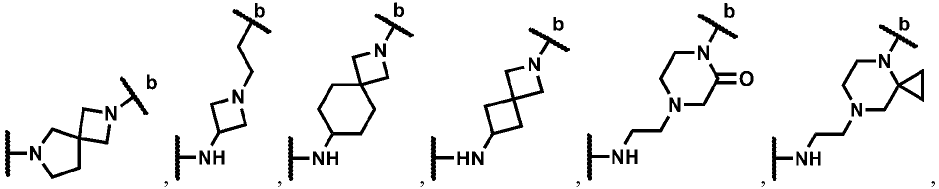 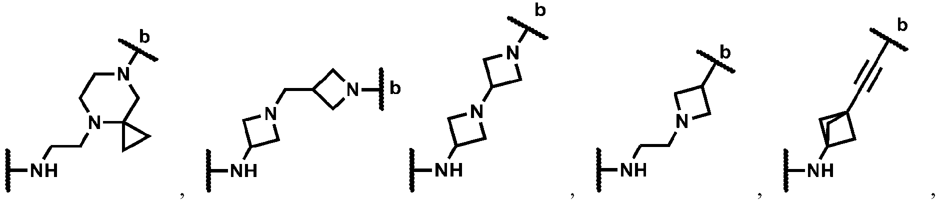 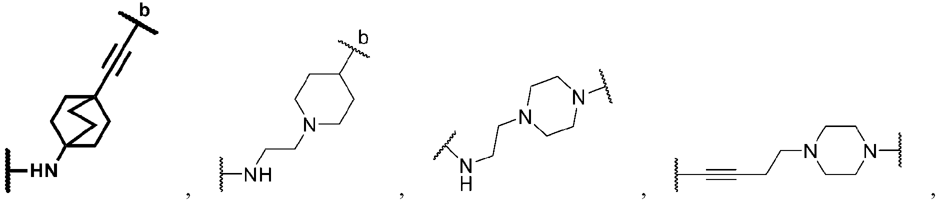 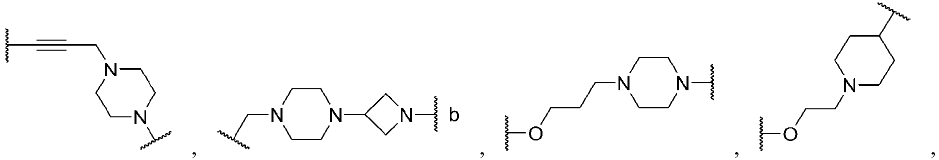 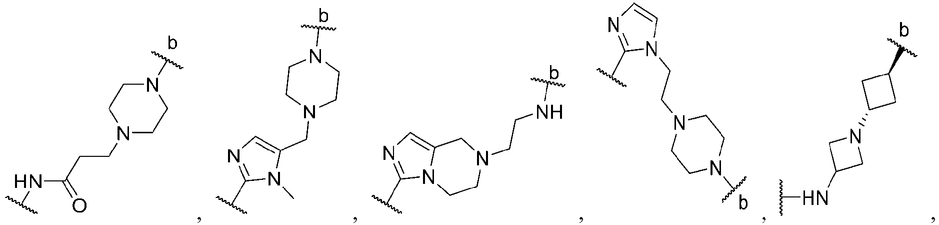 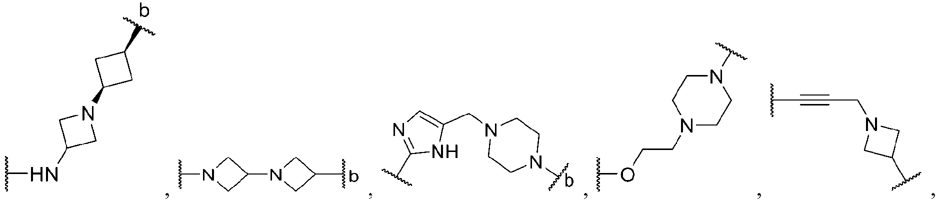 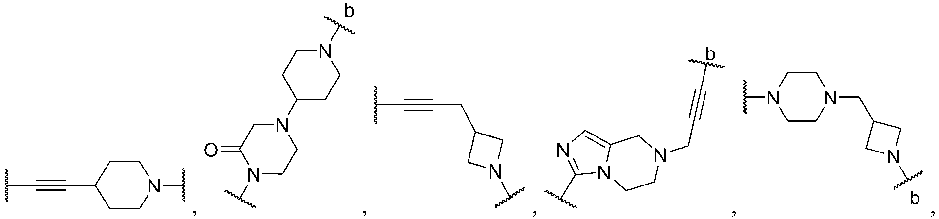 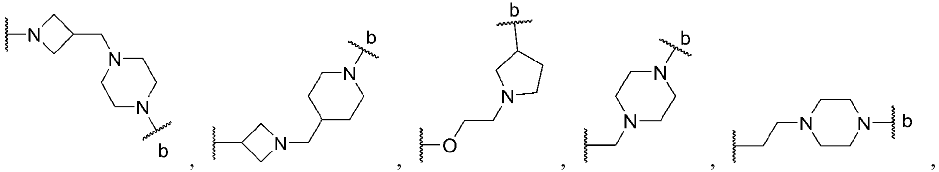 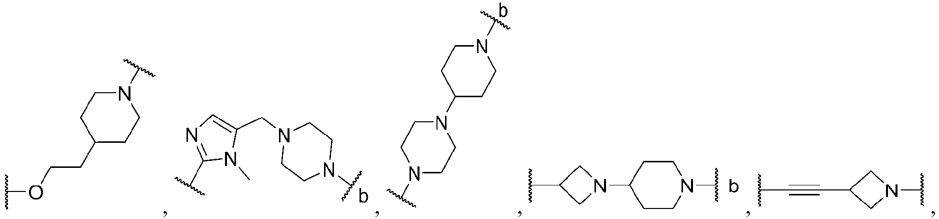 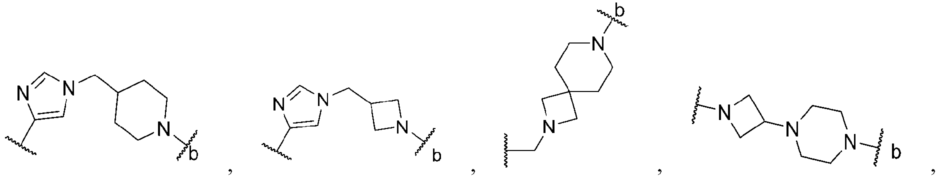 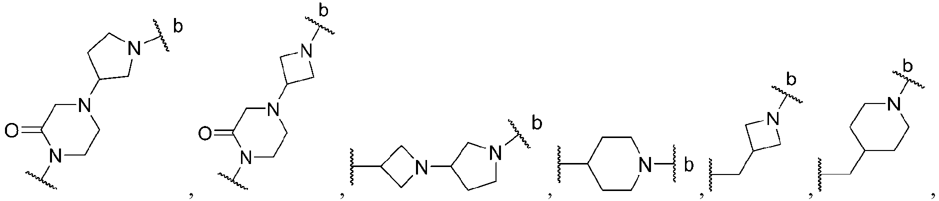 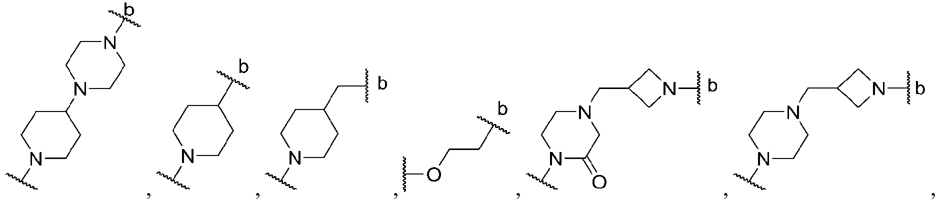 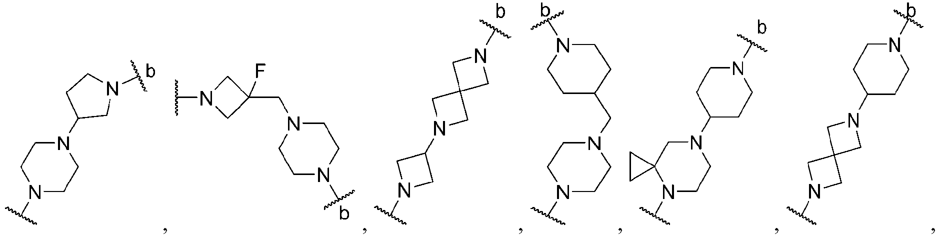 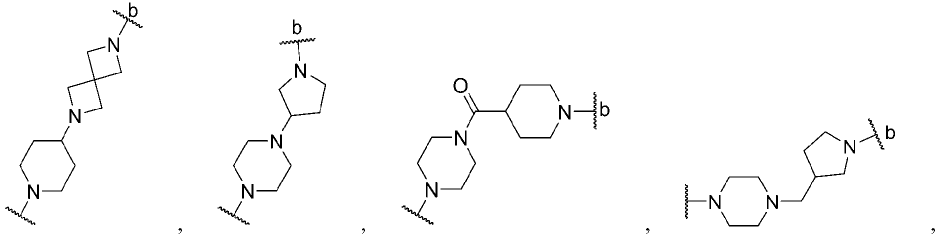 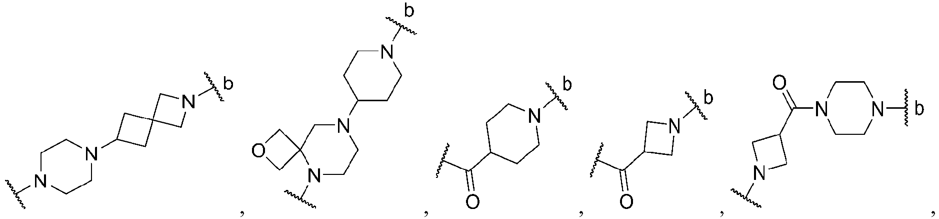 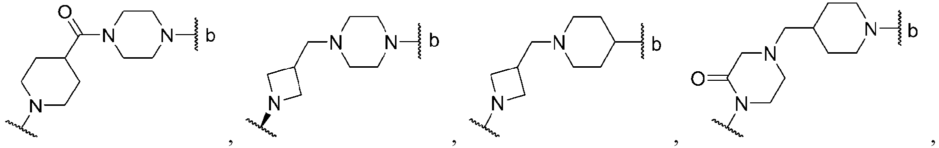 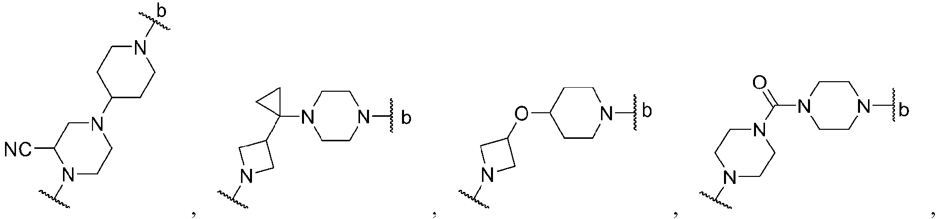 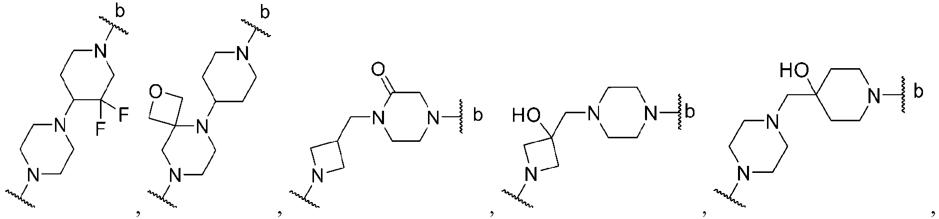 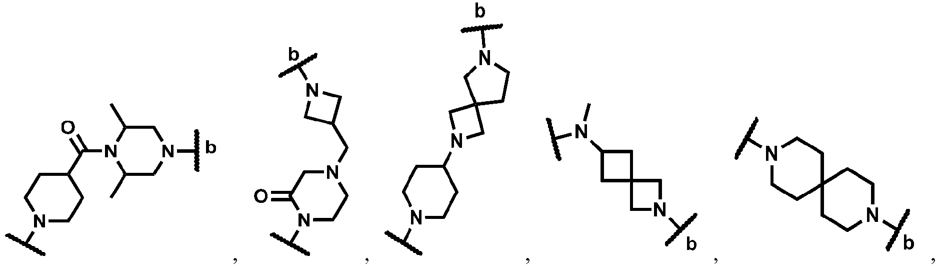 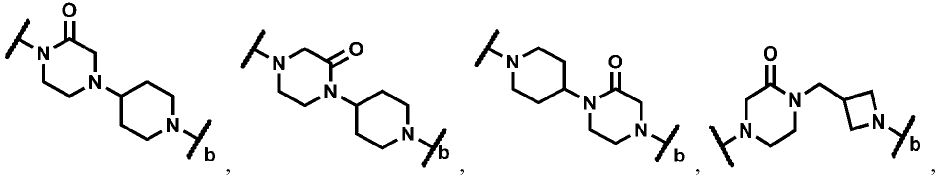 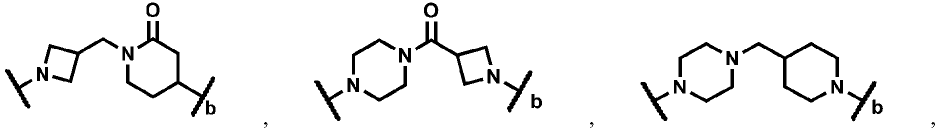 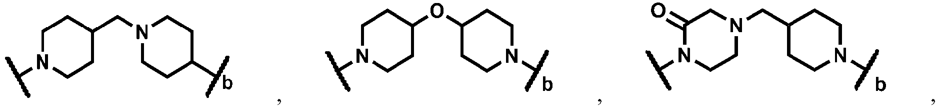 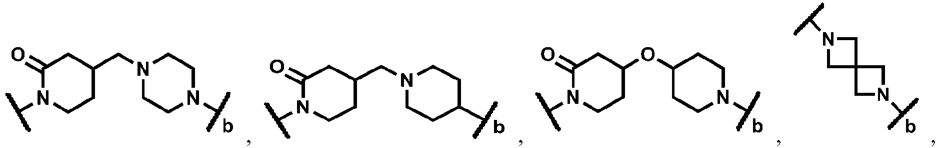 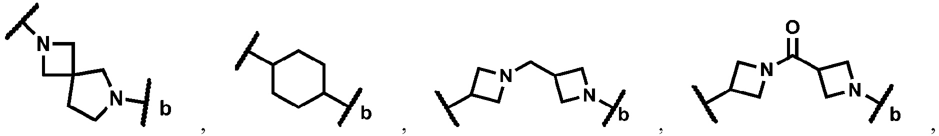 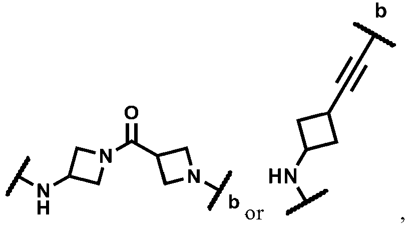 wherein side b is connected to B; preferably, in Scheme i, L is any one of the above groups except

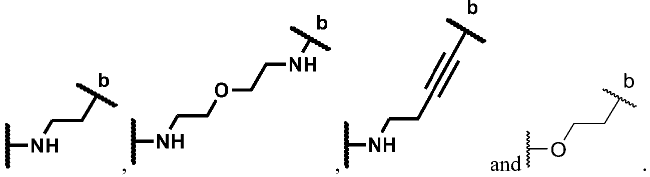

13. The compound represented by formula (I), the pharmaceutically acceptable salt thereof, the solvate thereof or the solvate of the pharmaceutically acceptable salt thereof according to claim 4, wherein the compound represented by formula (I), the pharmaceutically acceptable salt thereof, the solvate thereof or the solvate of the pharmaceutically acceptable salt thereof satisfies one or more of the following conditions:
(1) in condition ii, -A-L-B- is G11 or G12:
G11: 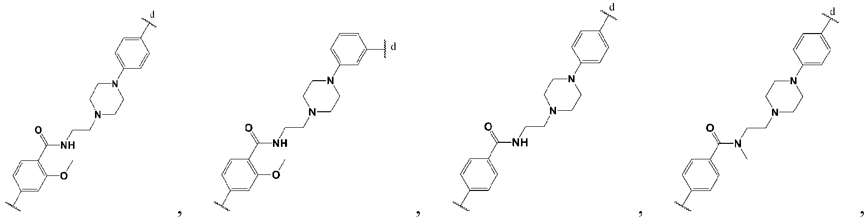 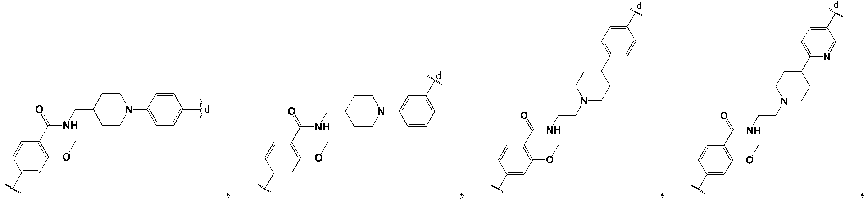 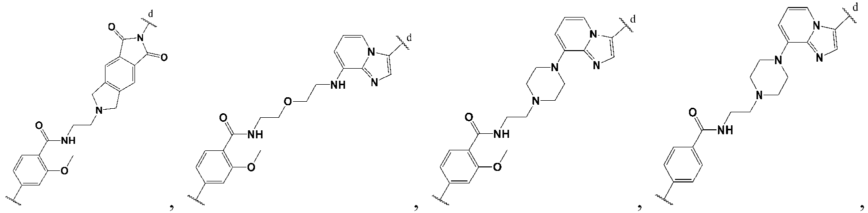 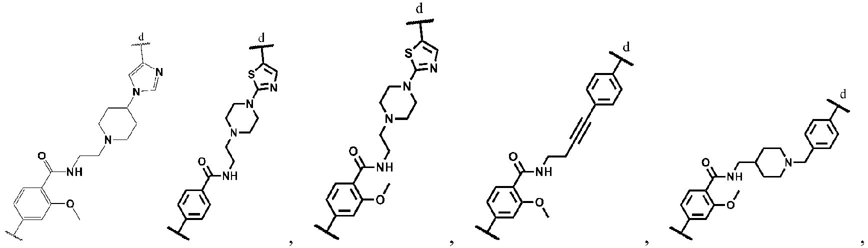 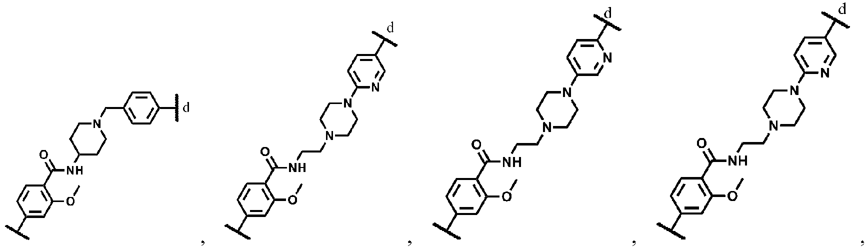 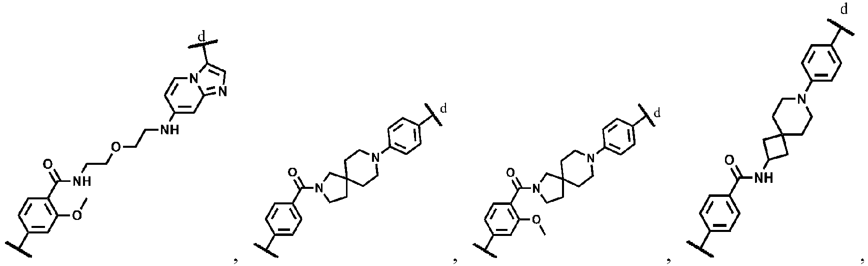 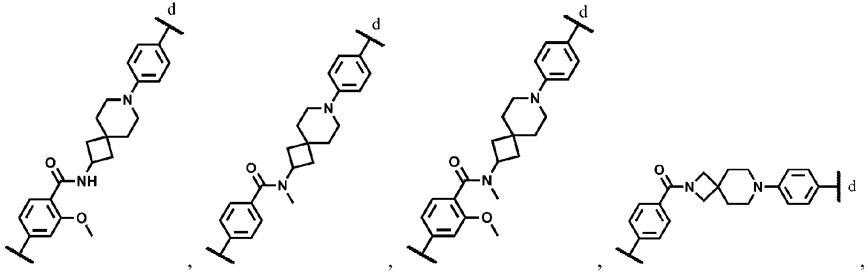 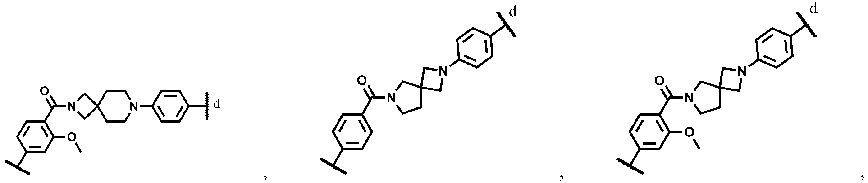 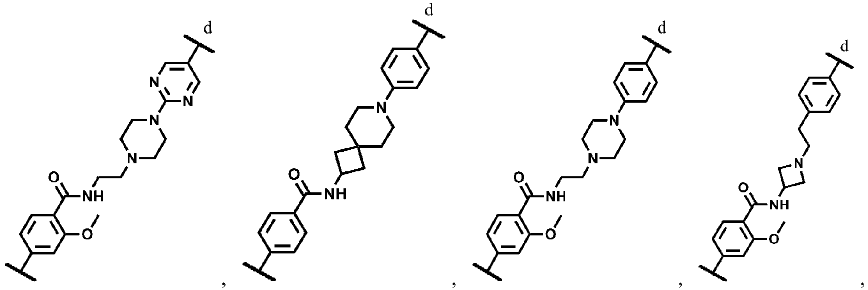 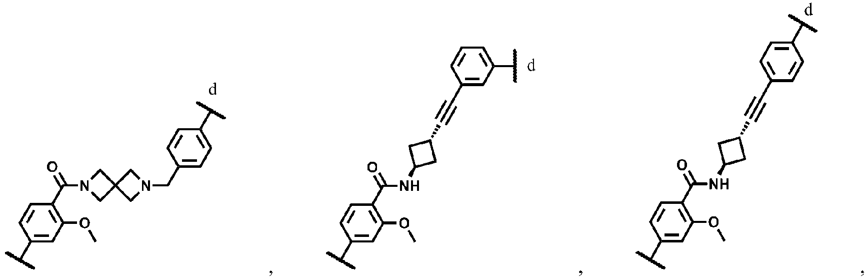 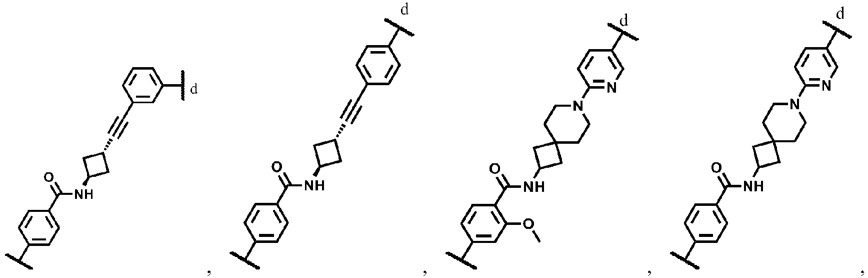 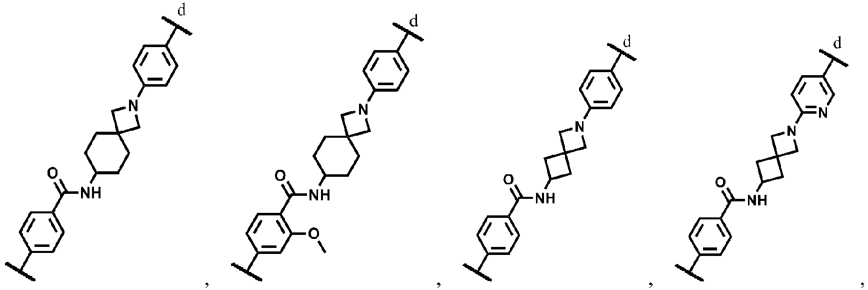 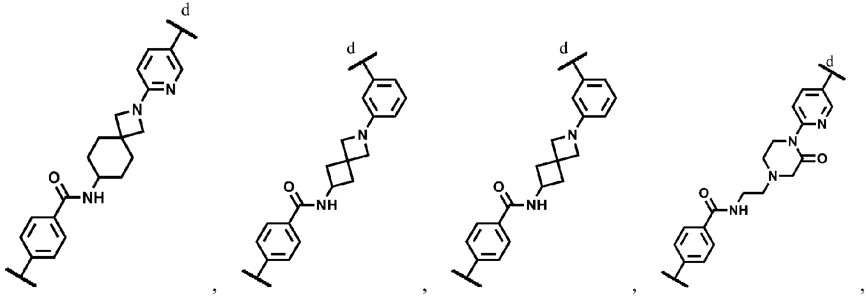 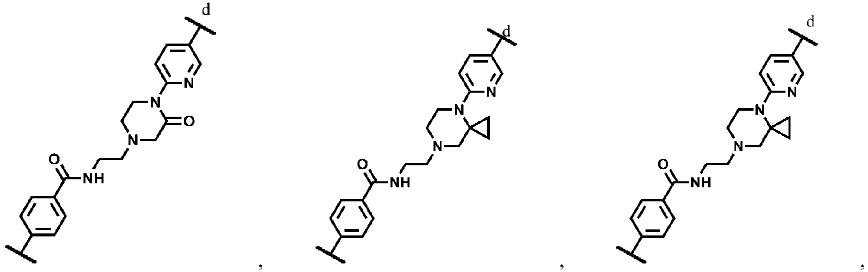 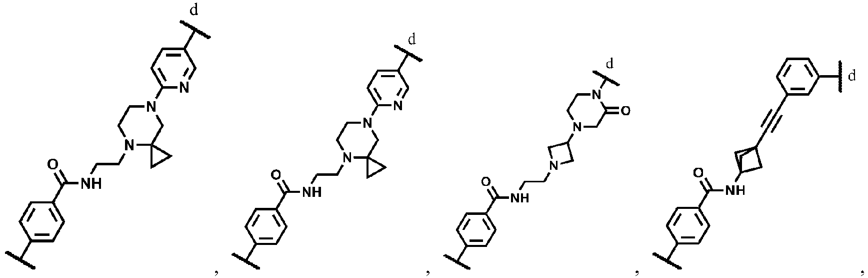 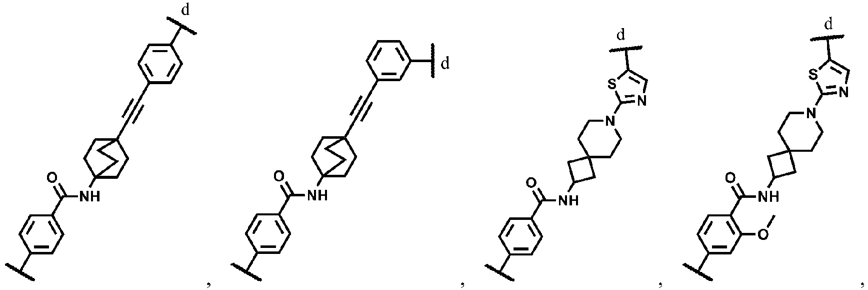 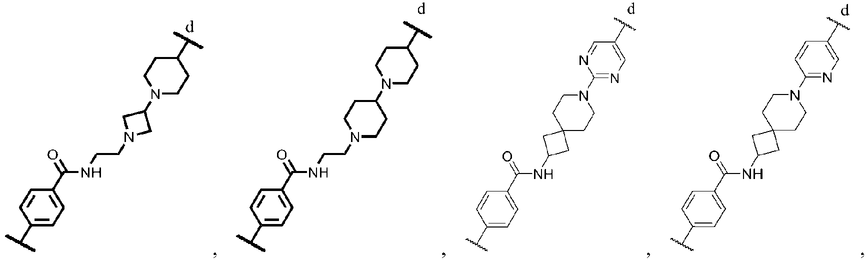 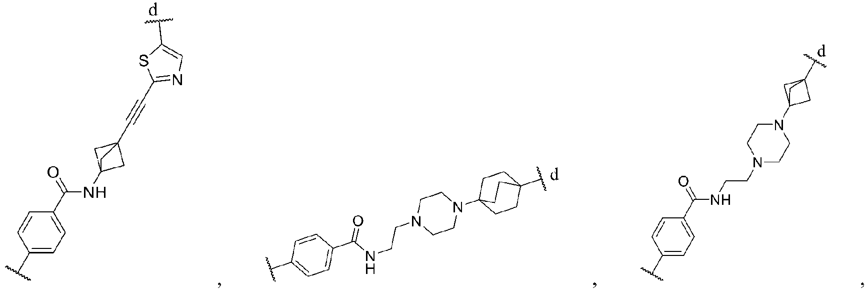 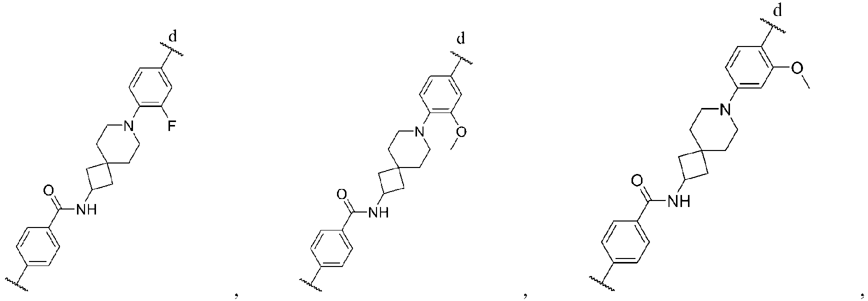 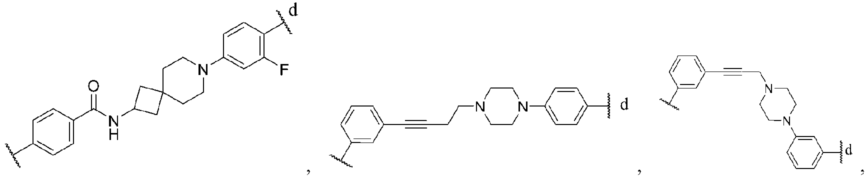 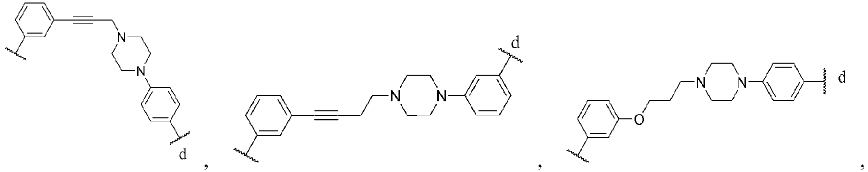 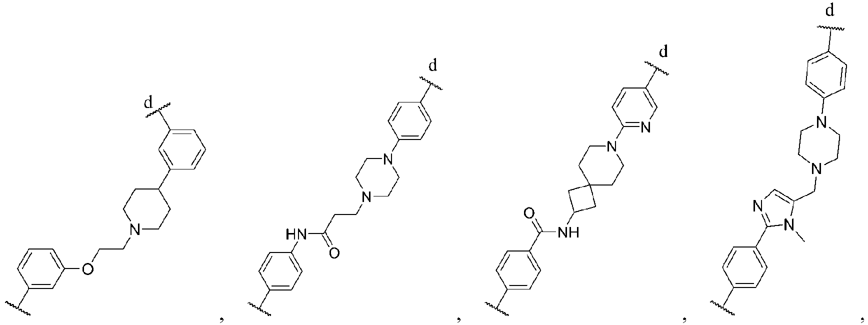 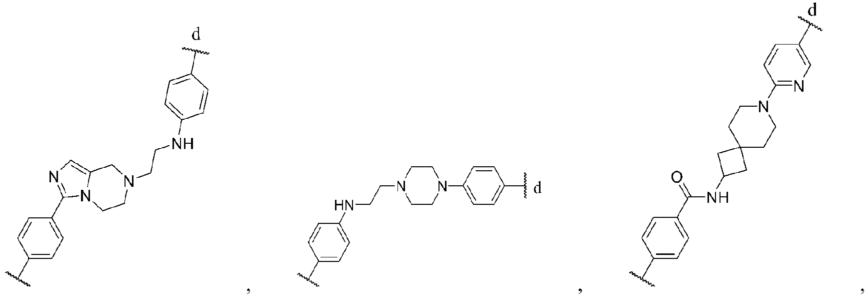 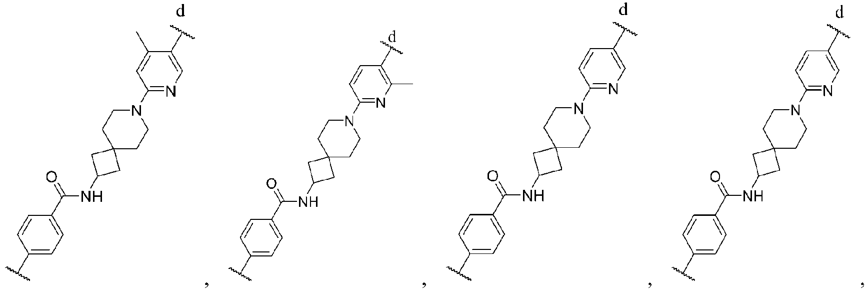 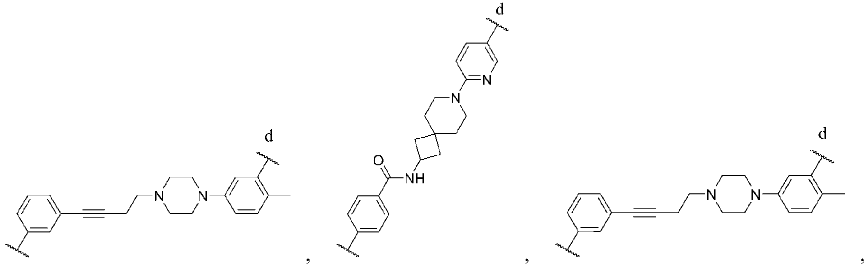 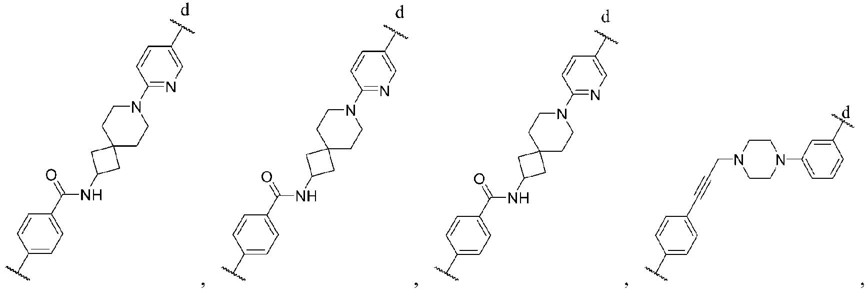 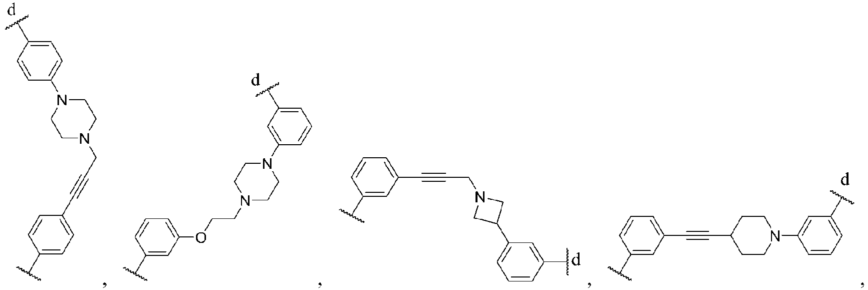 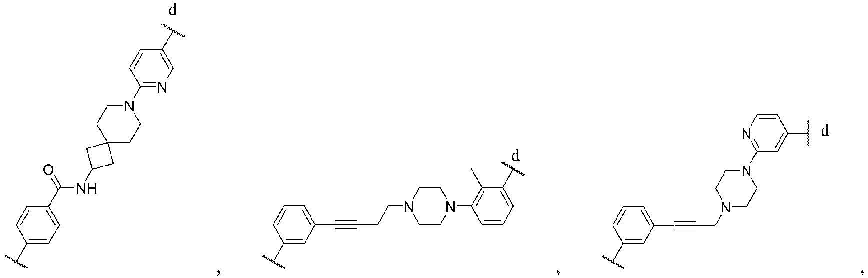 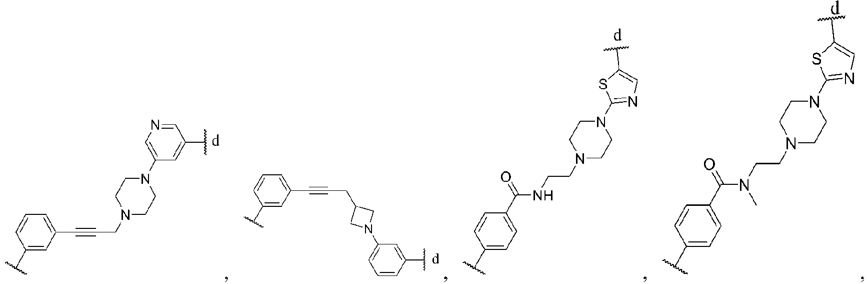 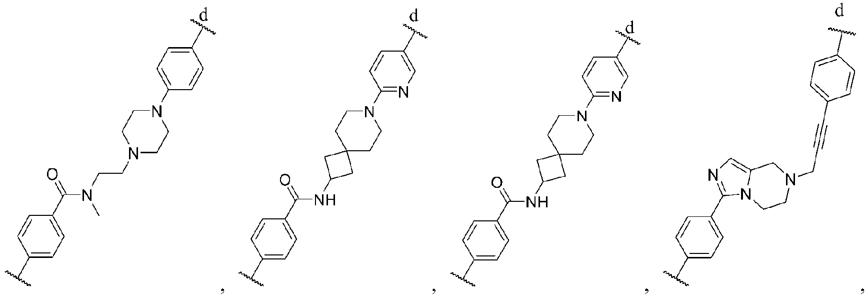 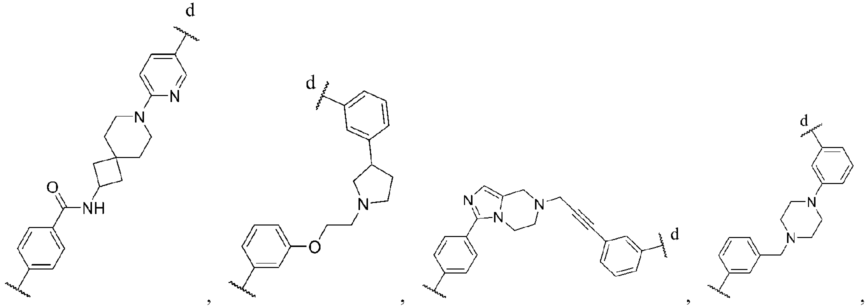 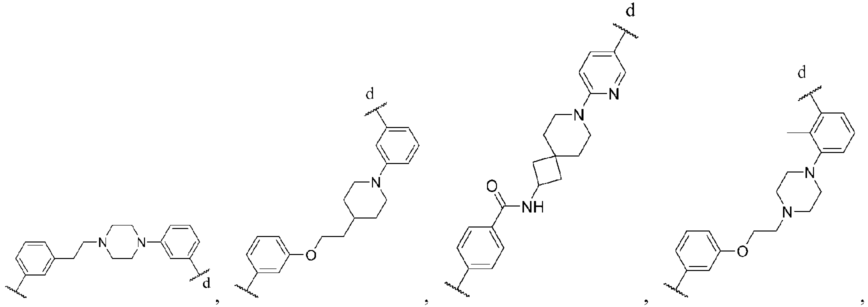 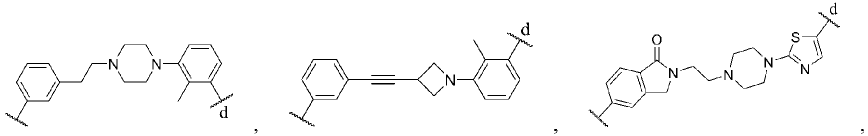 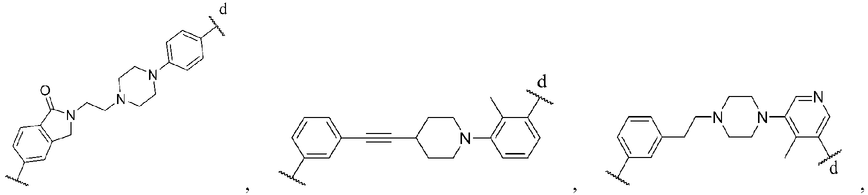 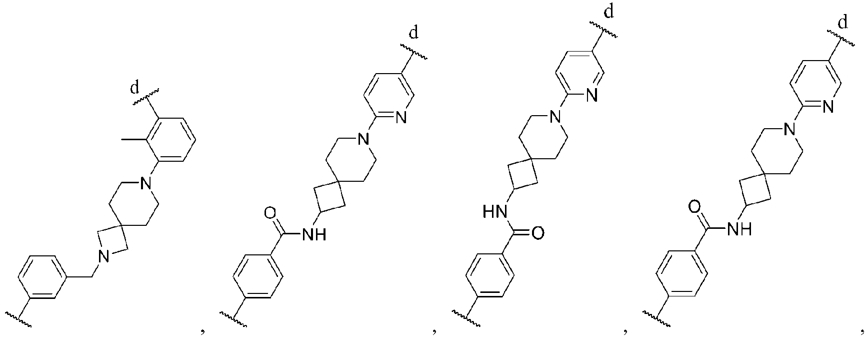 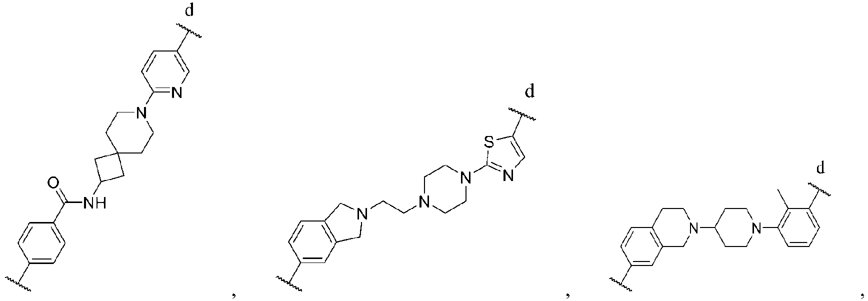 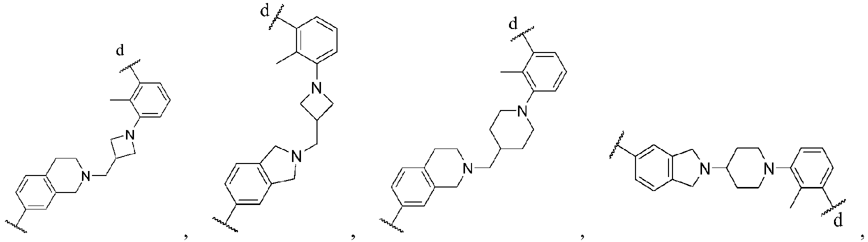 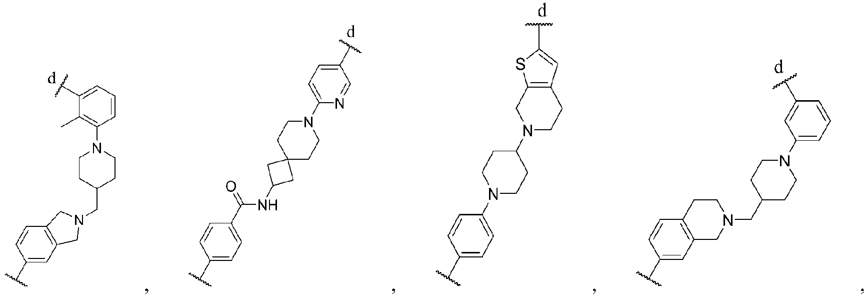 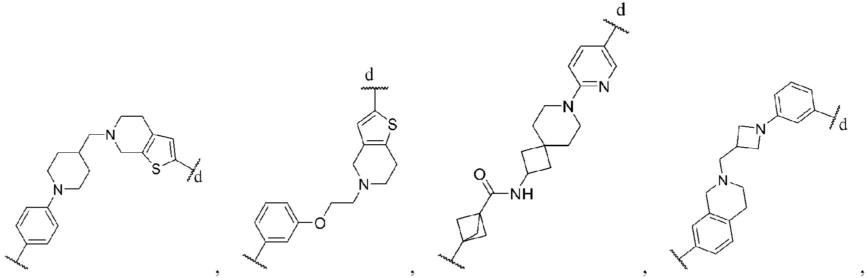 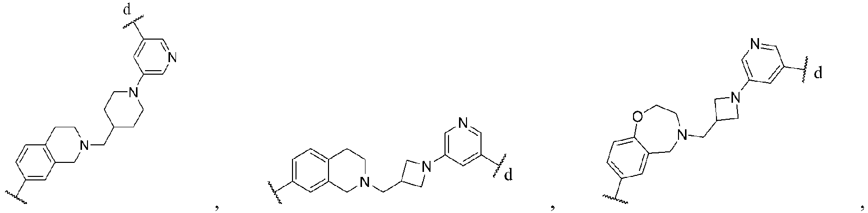 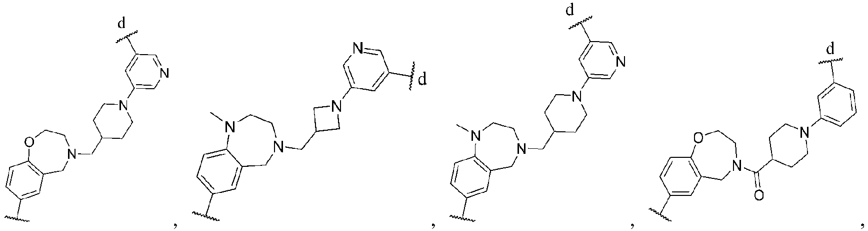 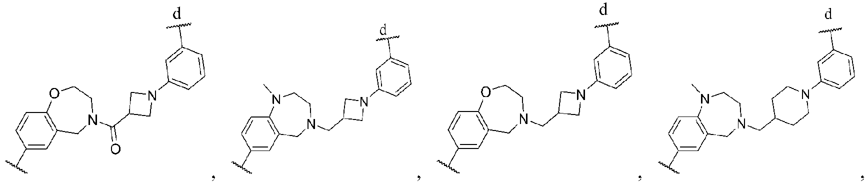 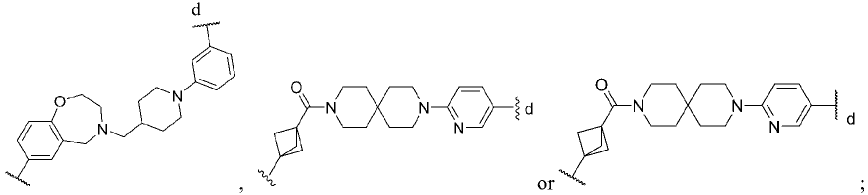
G12: 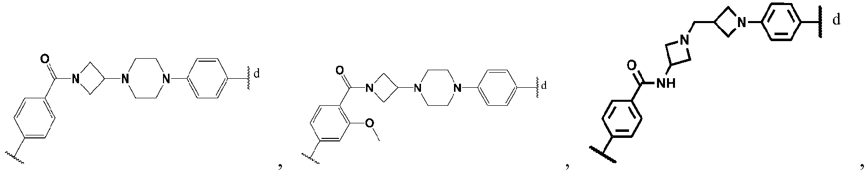 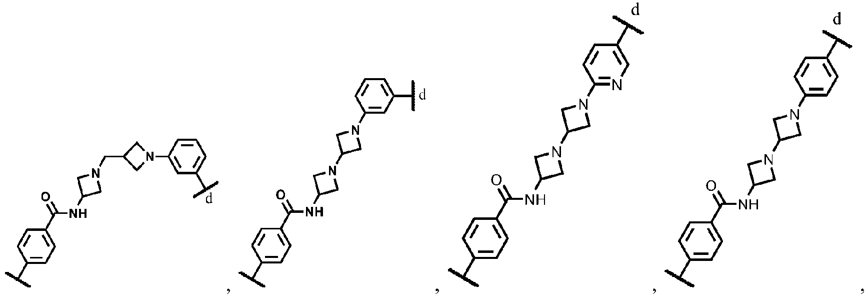 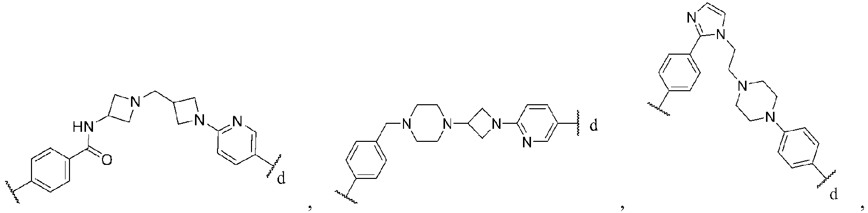 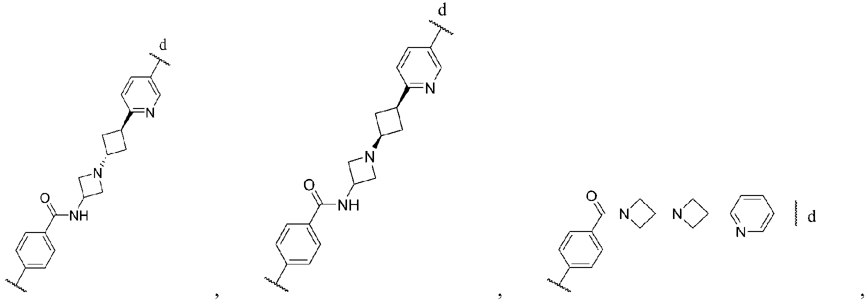 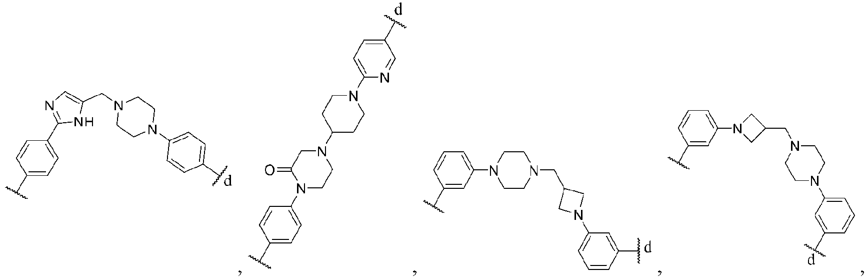 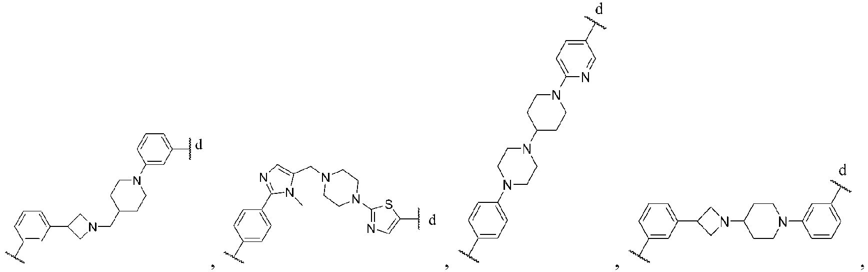 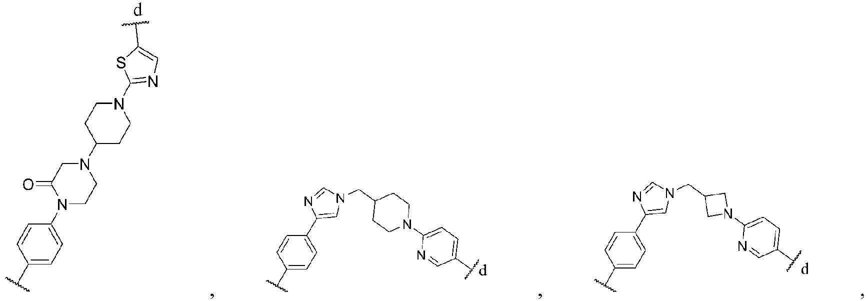 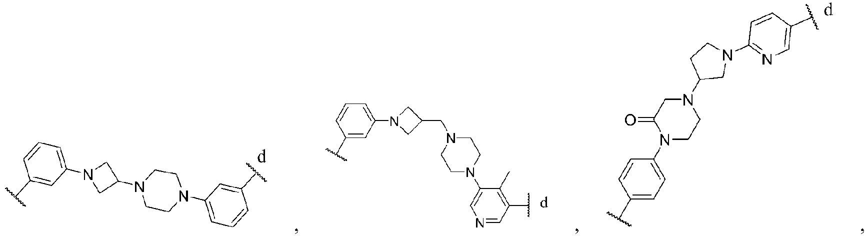 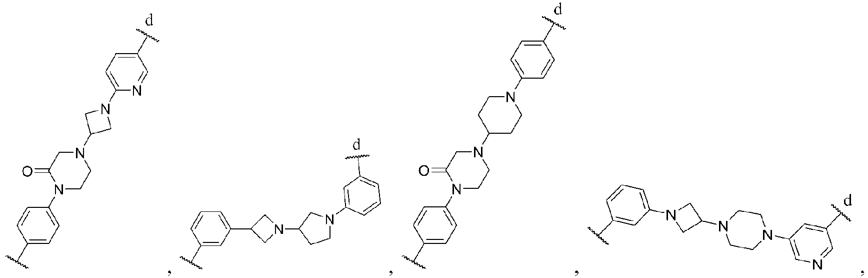 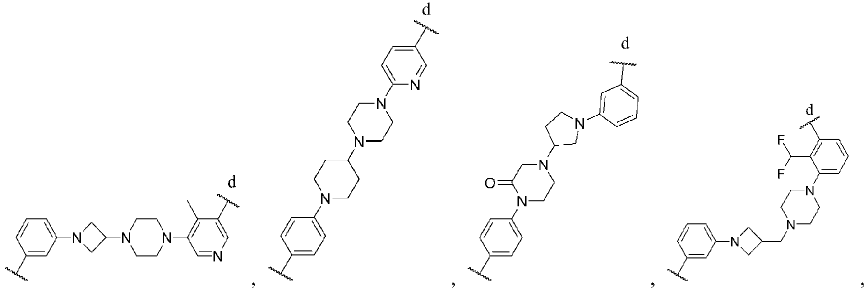 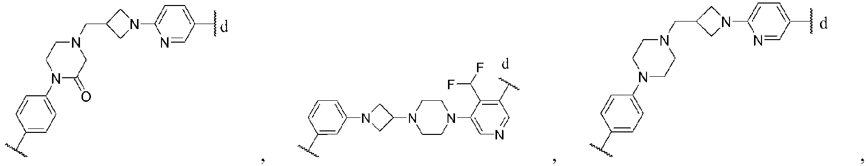 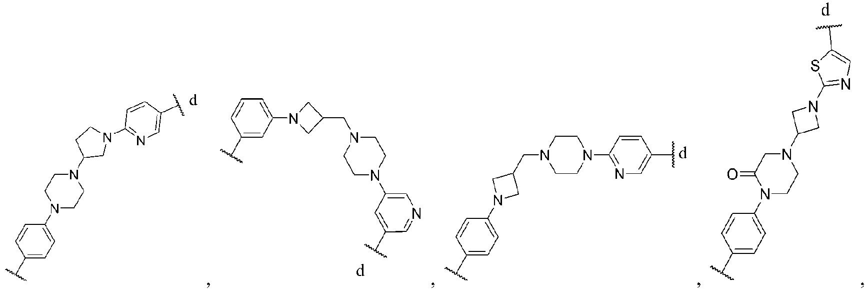 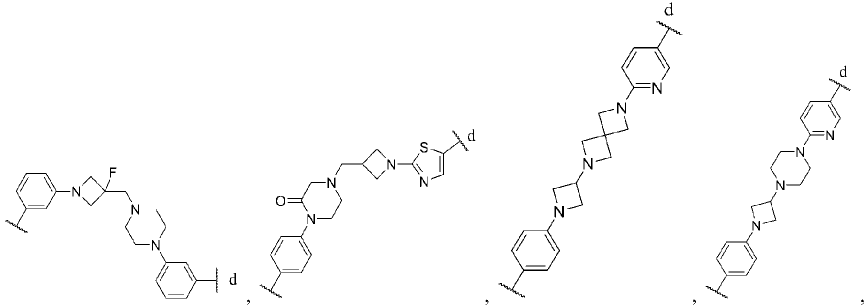 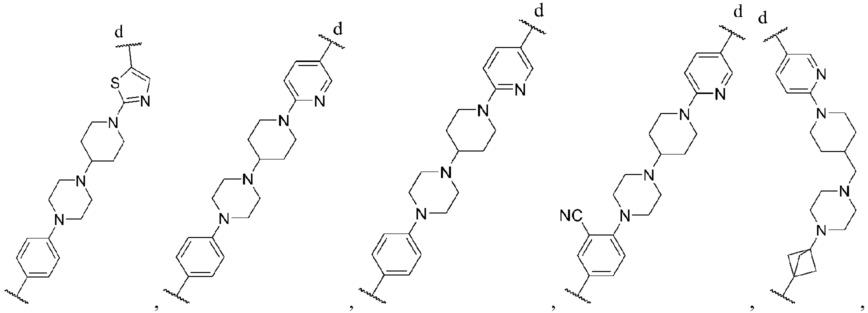 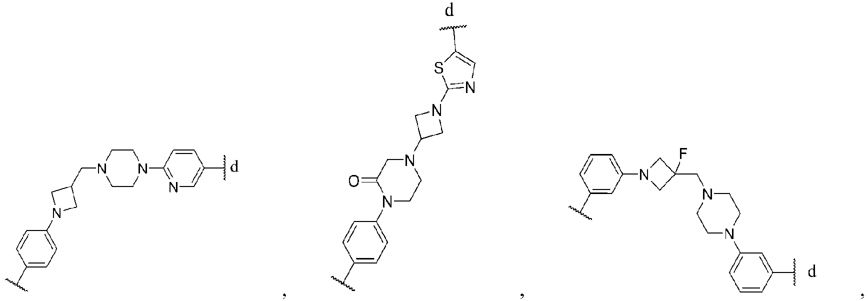 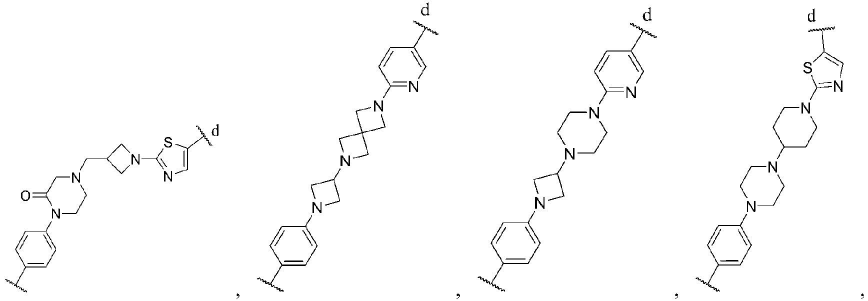 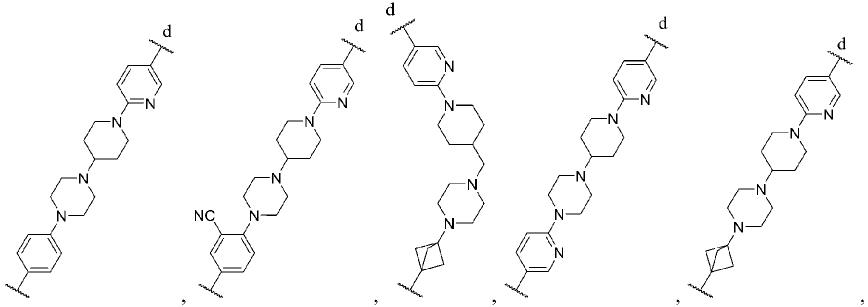 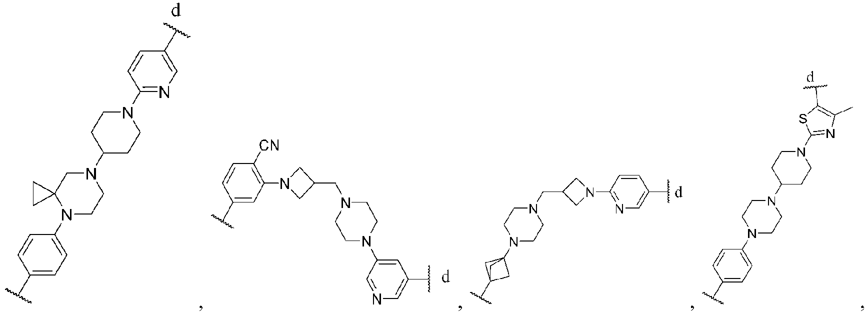 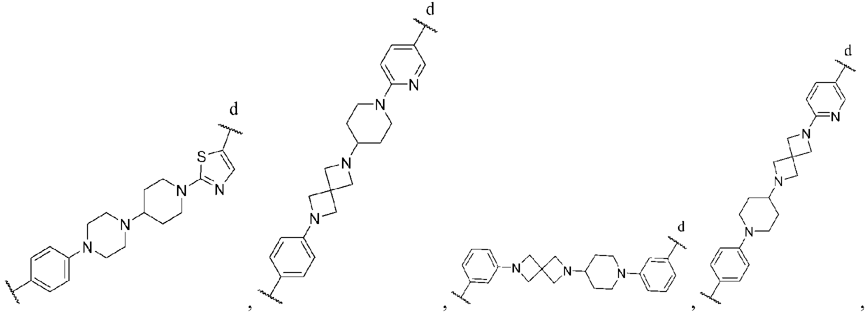 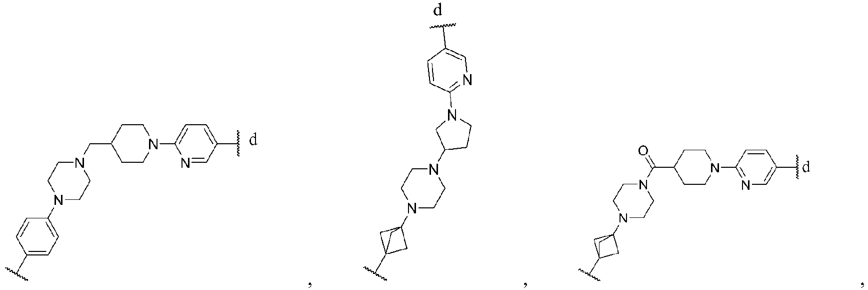 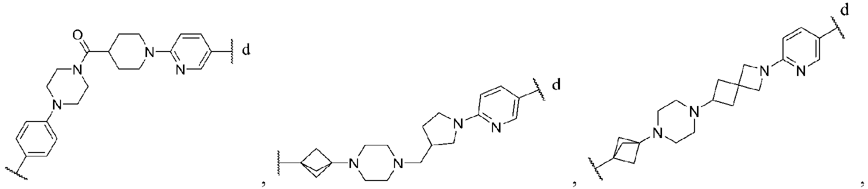 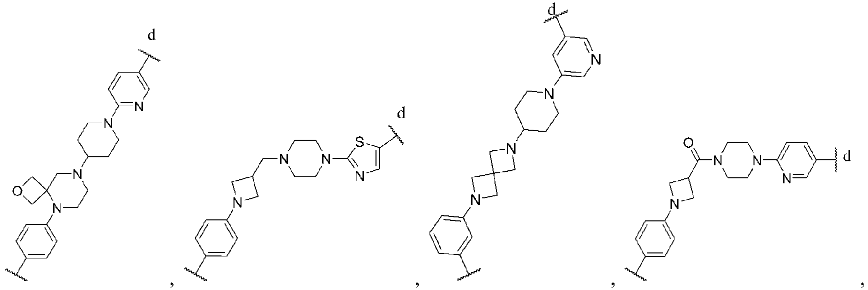 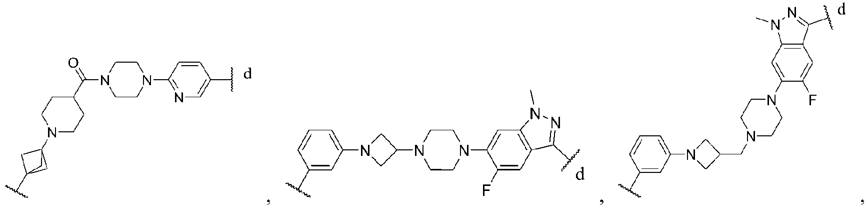 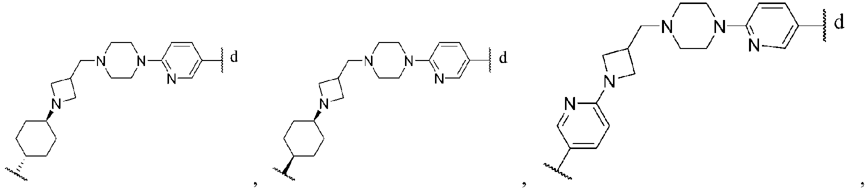 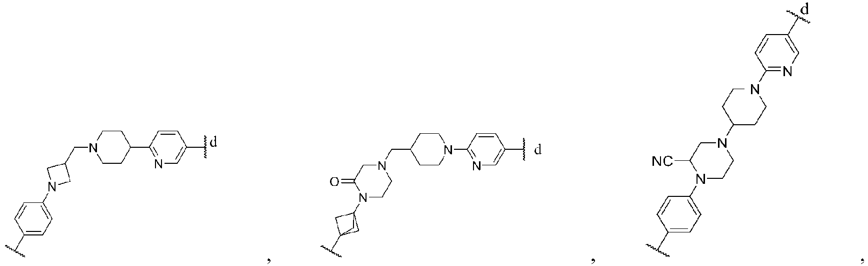 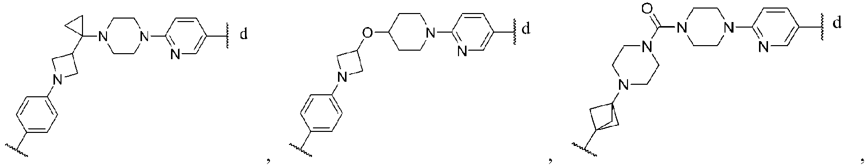 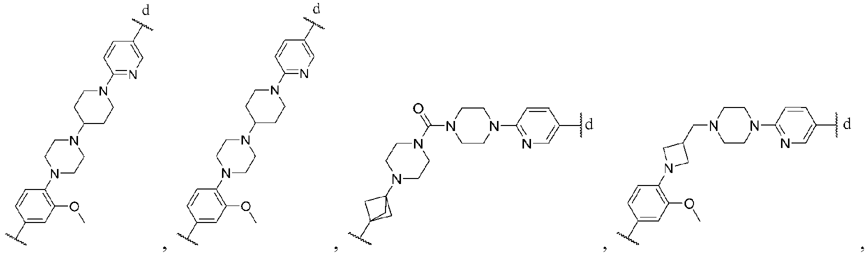 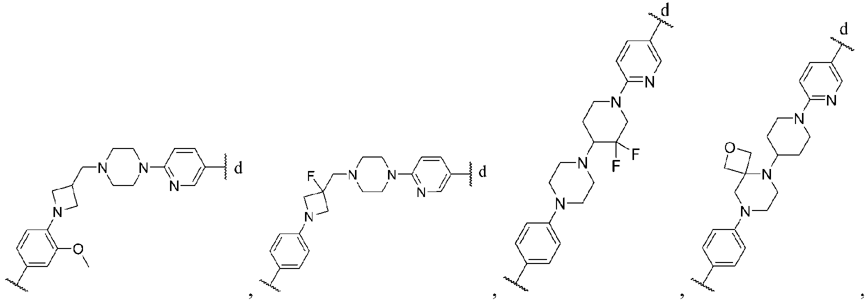 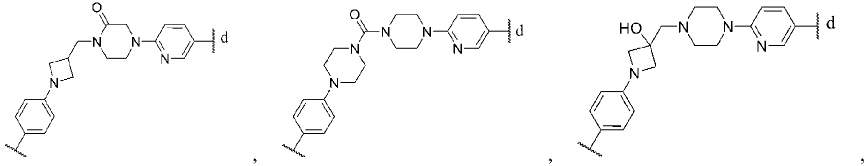 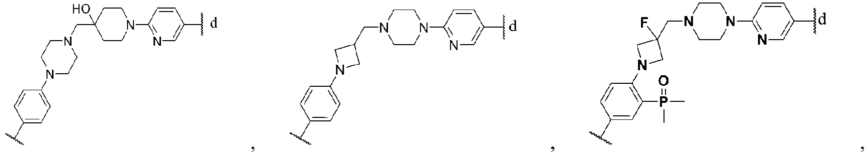 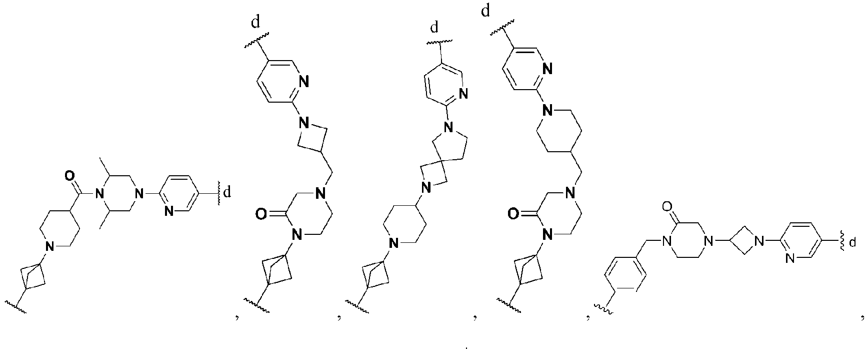 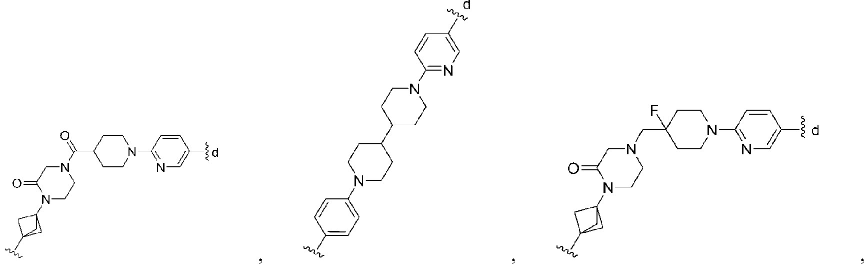 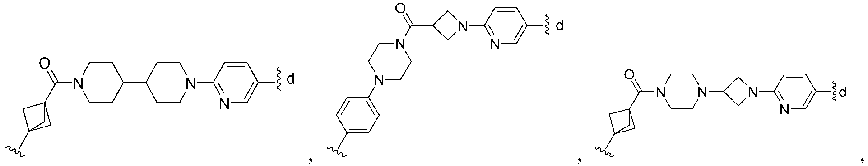 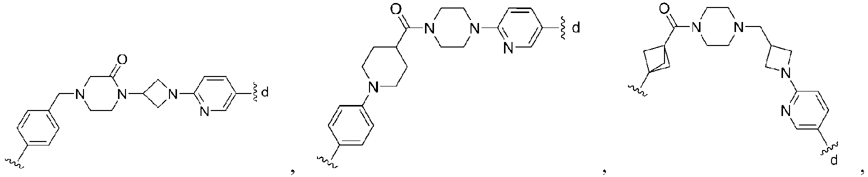 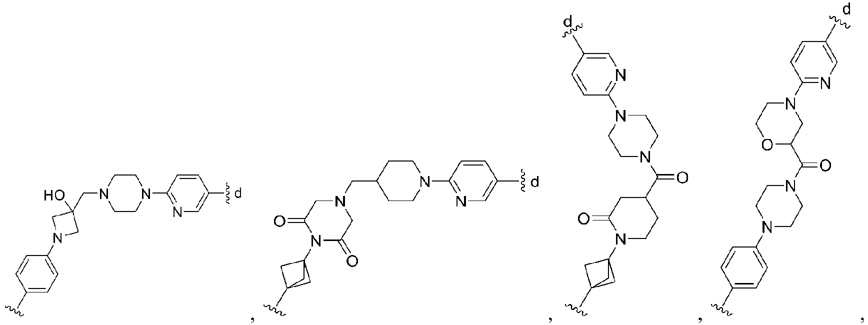 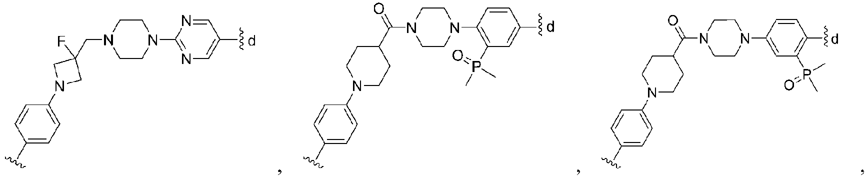 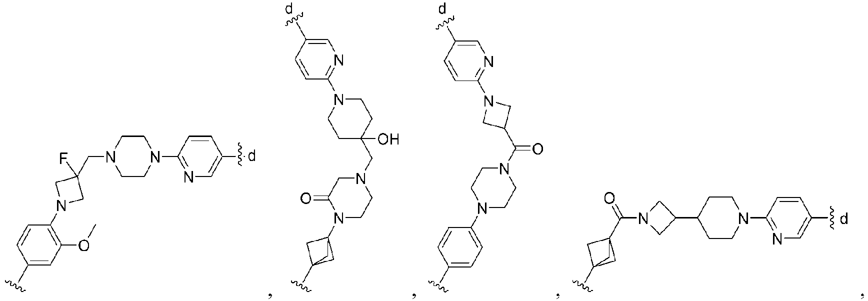 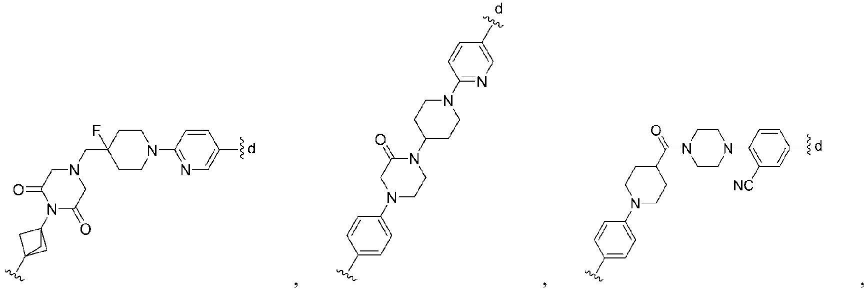 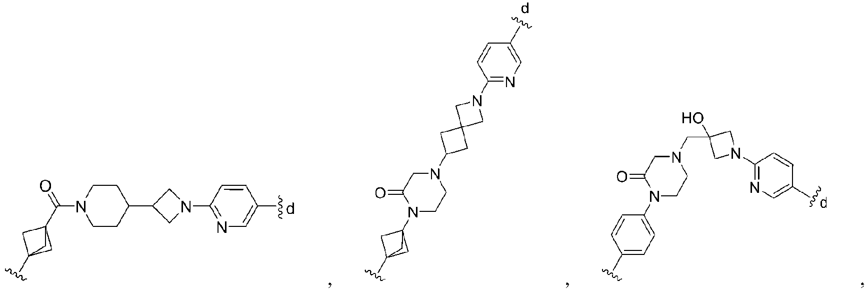 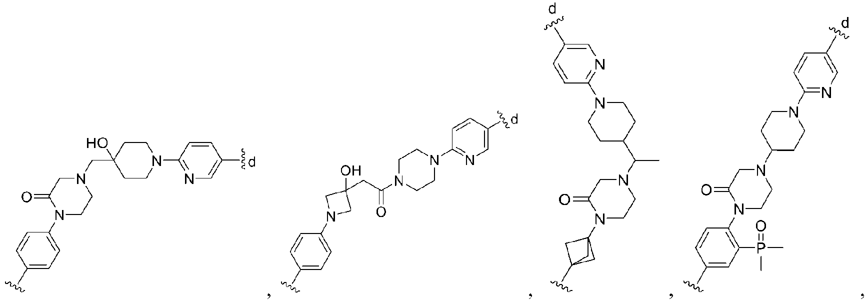 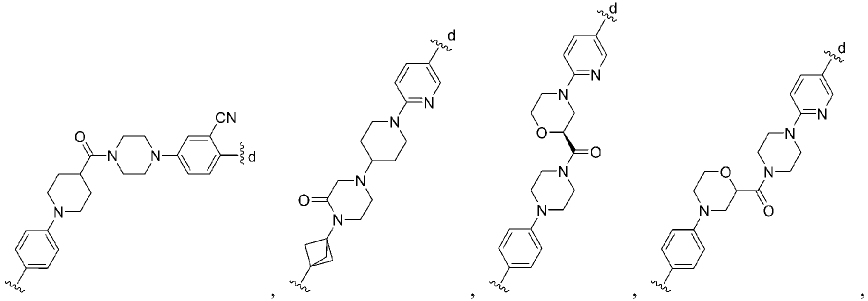 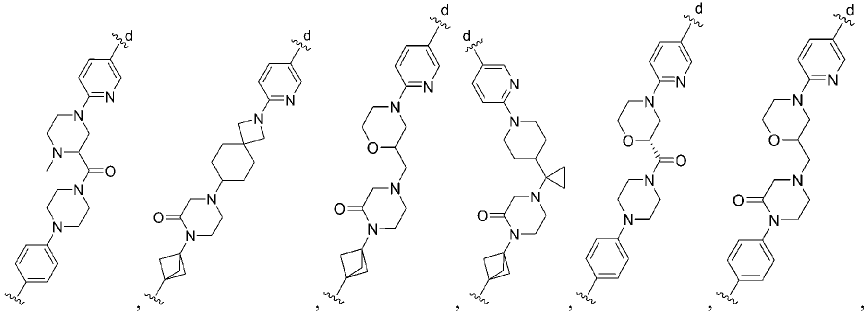 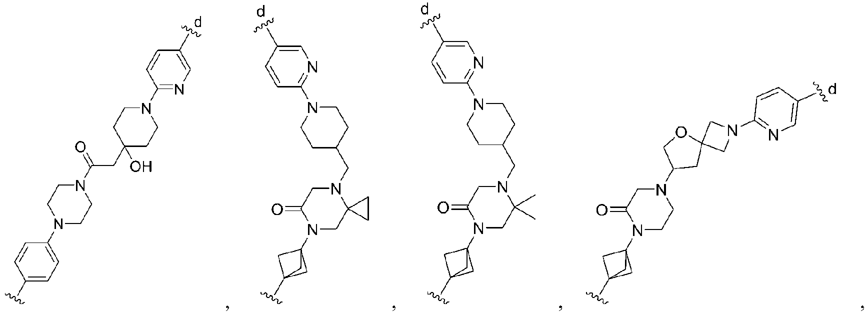 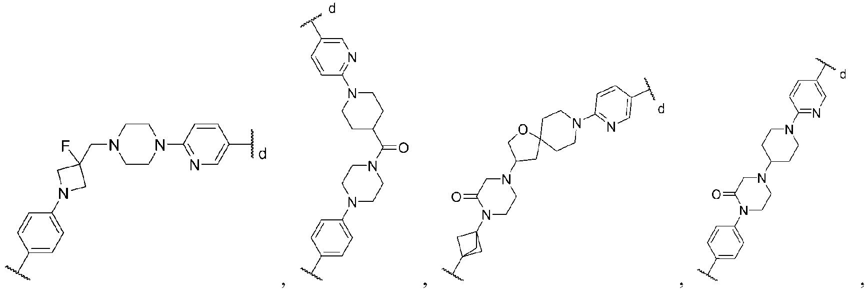 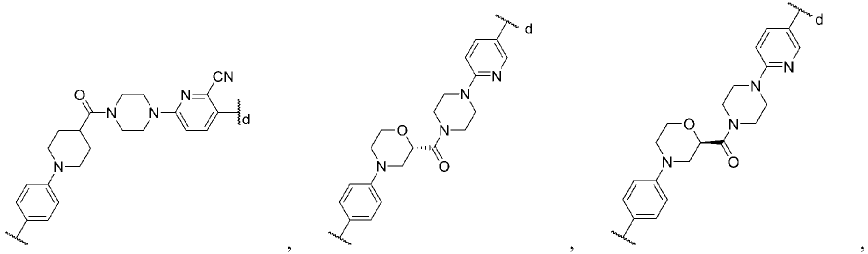 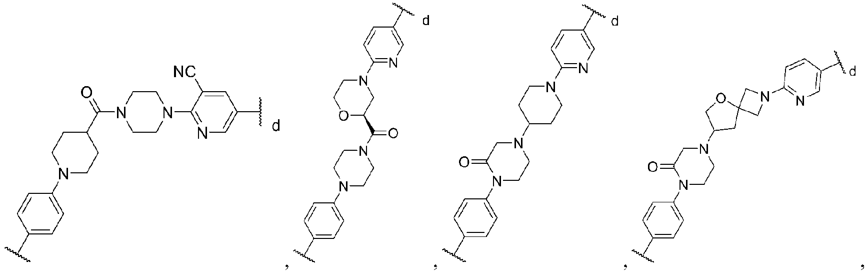 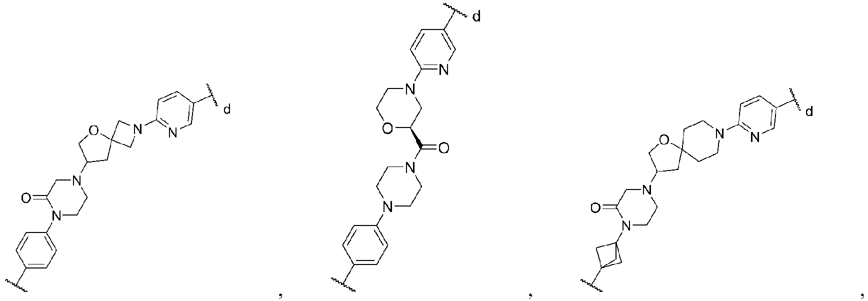 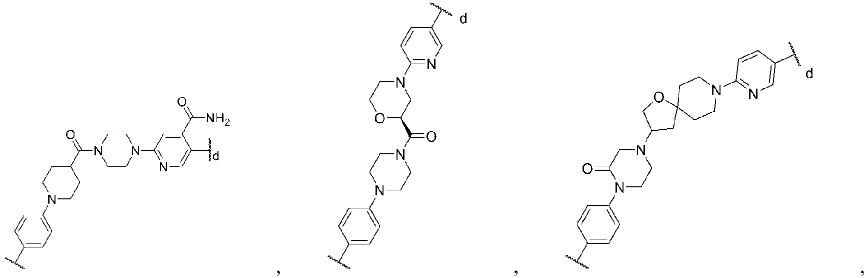 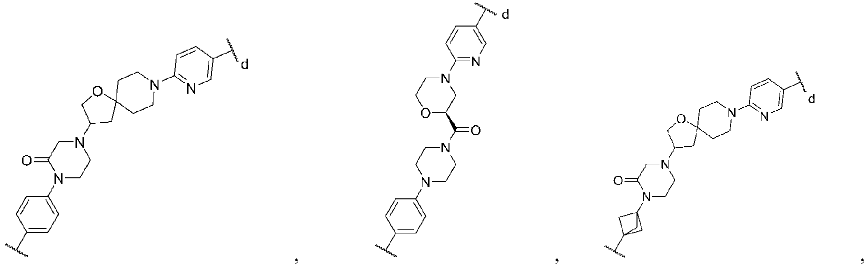 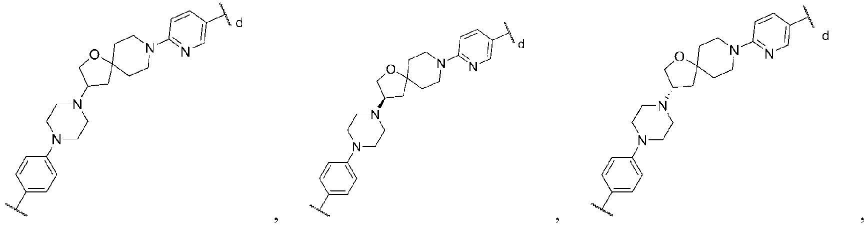 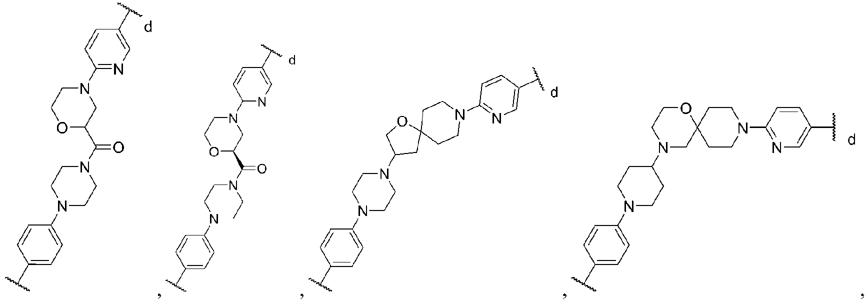 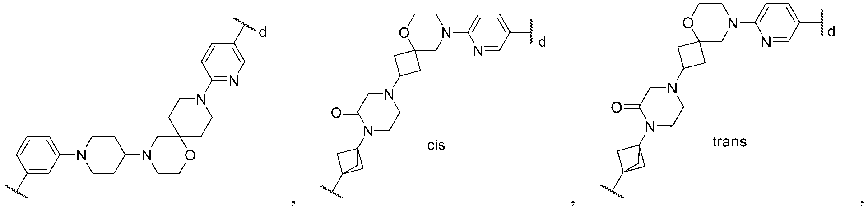 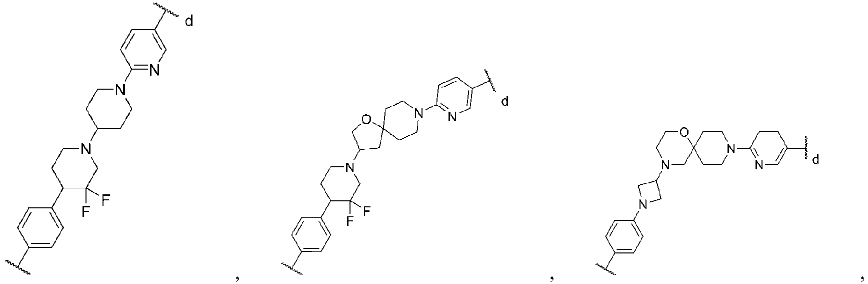 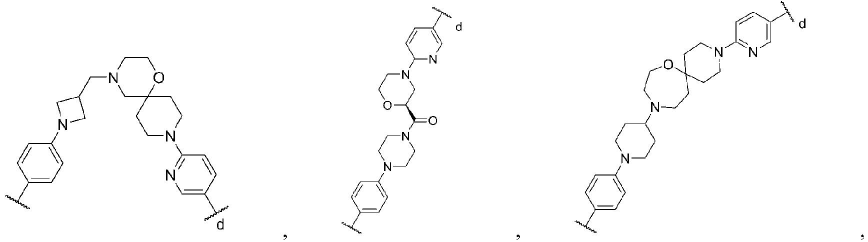 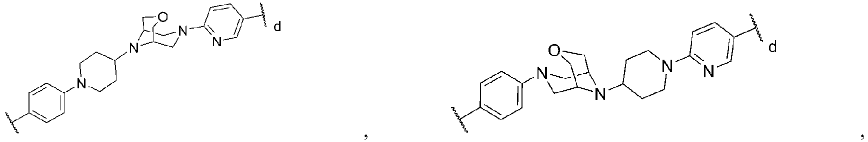 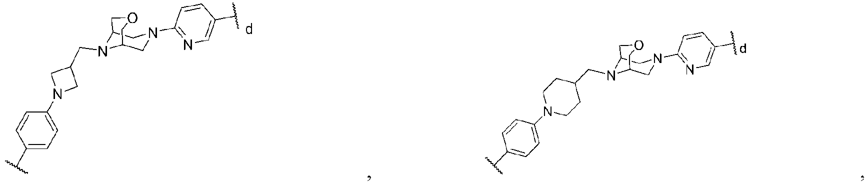 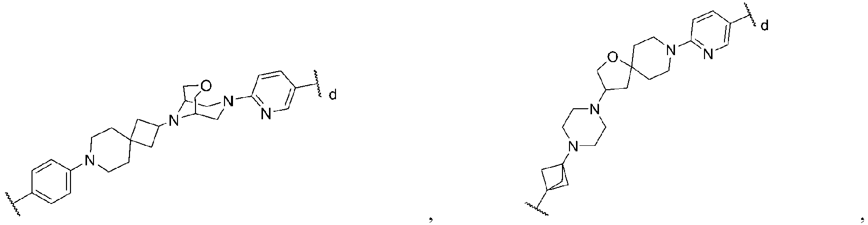 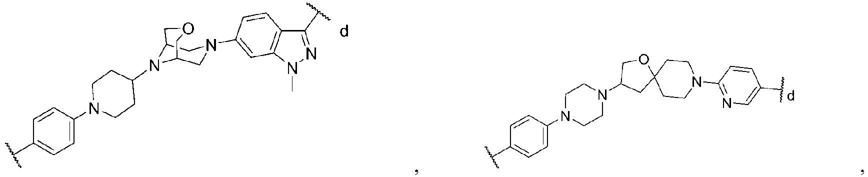 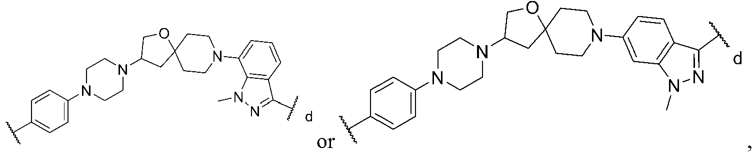
wherein side d is connected to 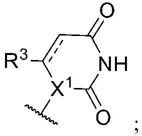
(2) In Scheme i, -A-L-B- is: 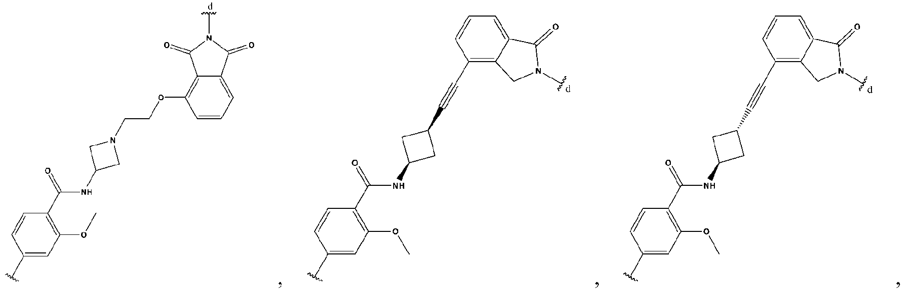 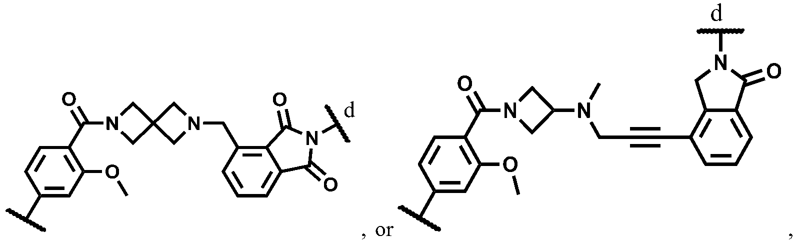 wherein side d is connected to

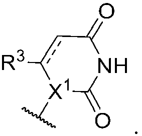

14. The compound represented by formula (I), the pharmaceutically acceptable salt thereof, the solvate thereof or the solvate of the pharmaceutically acceptable salt thereof according to claim 2 or 5, wherein,
-A-L-B- is: 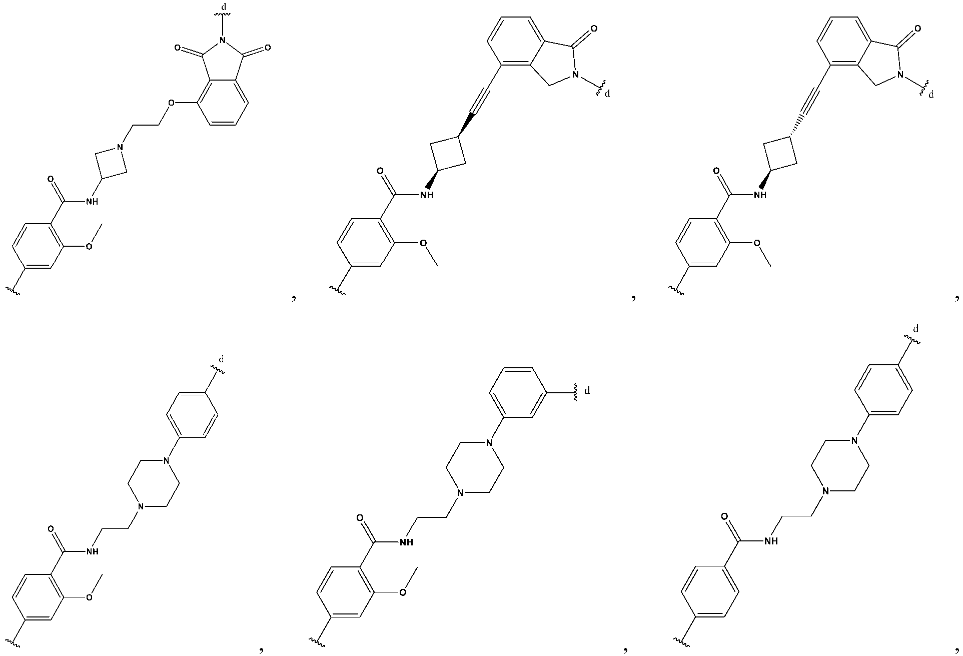 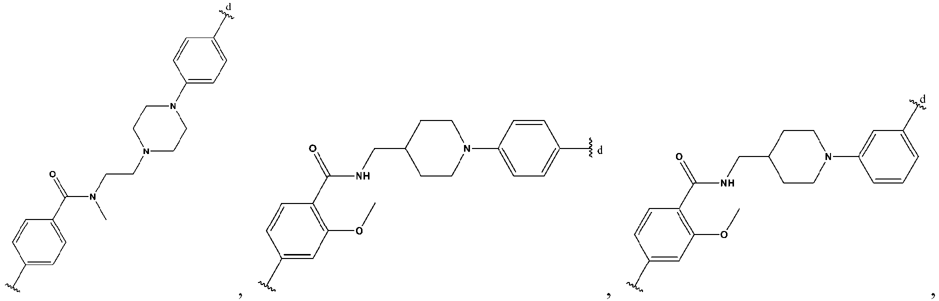 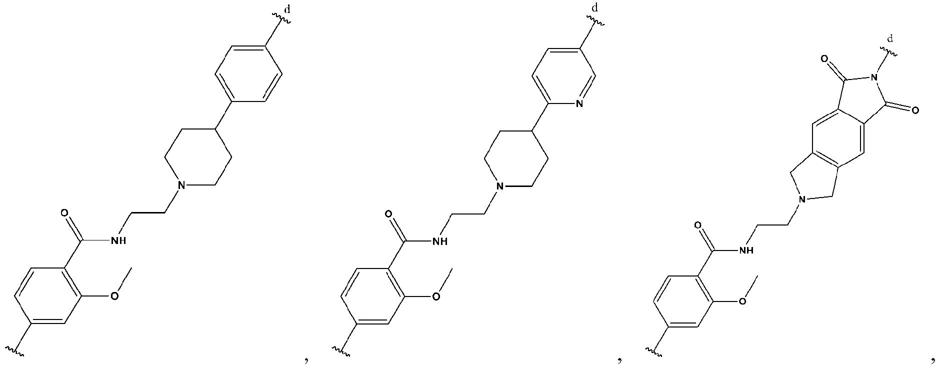 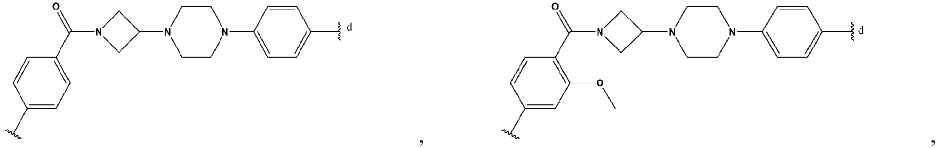 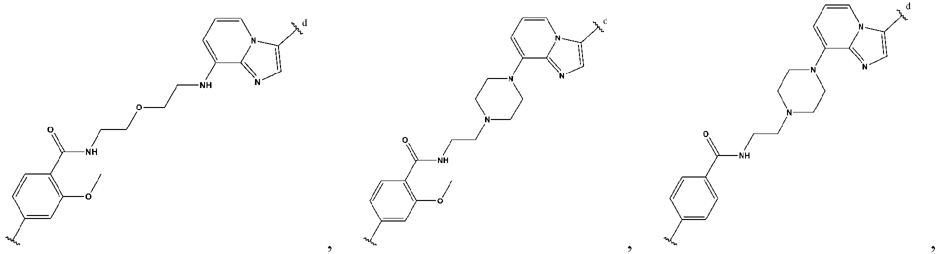 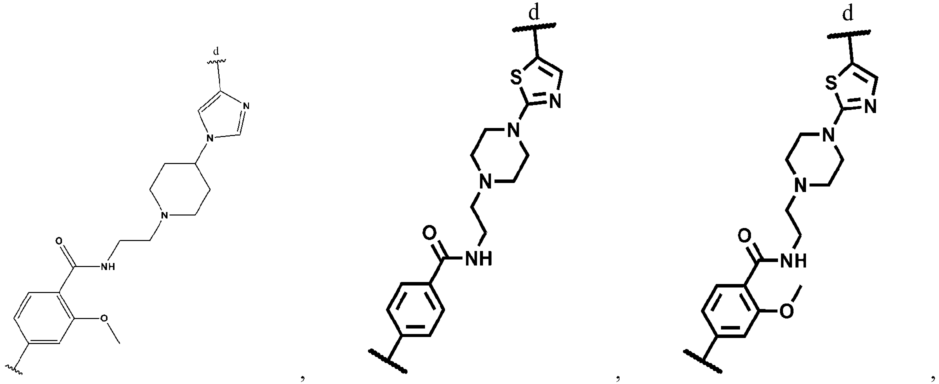 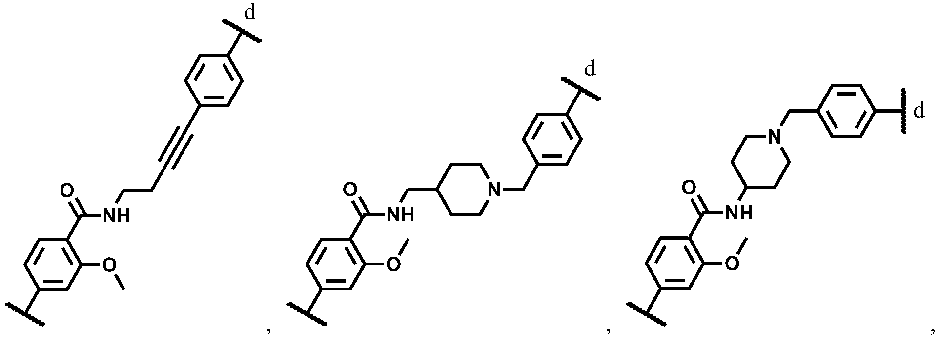 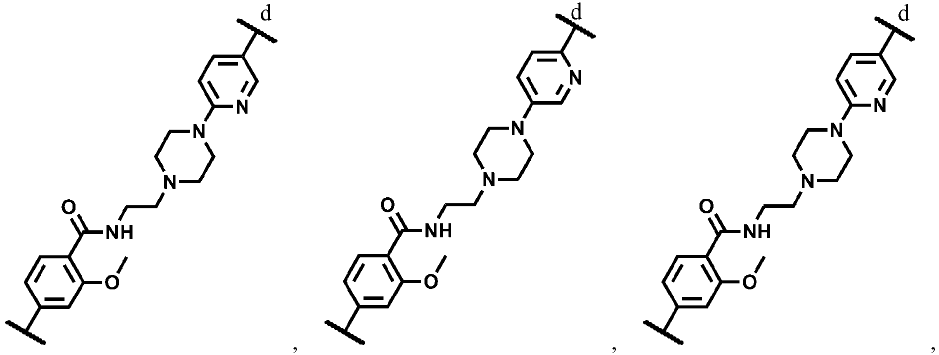 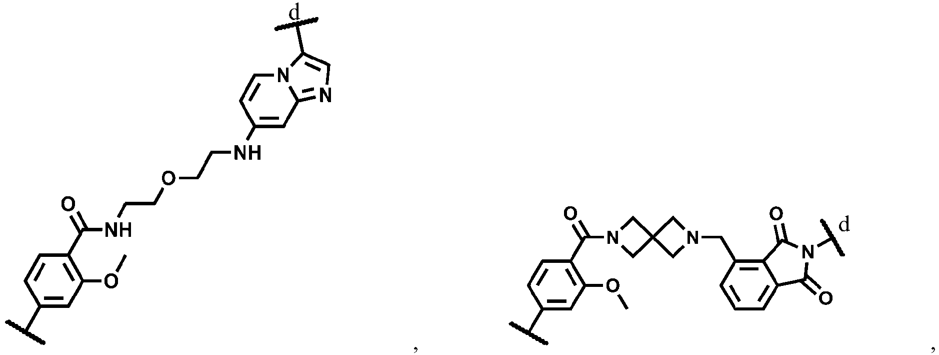 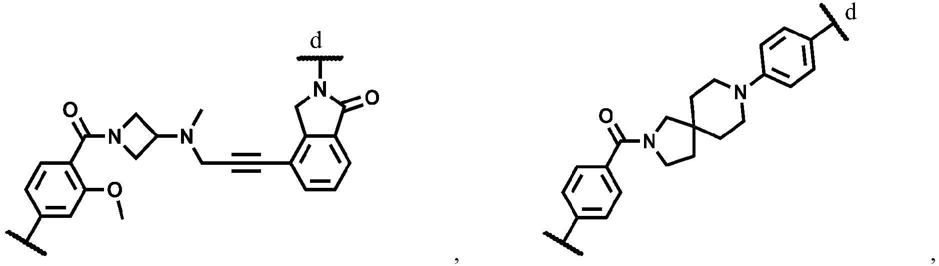 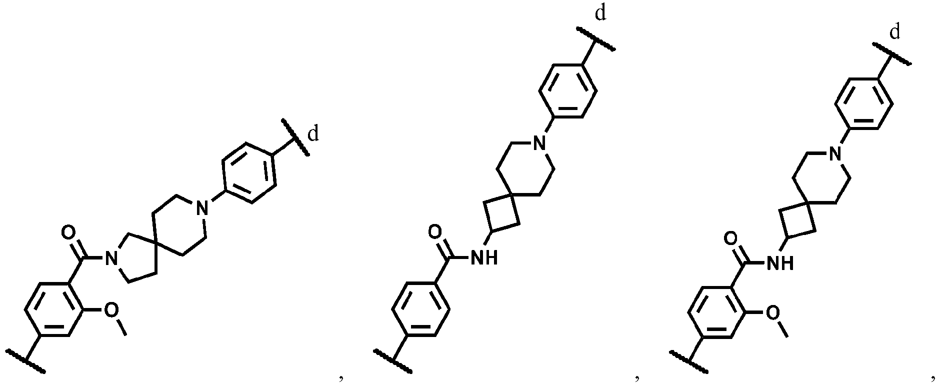 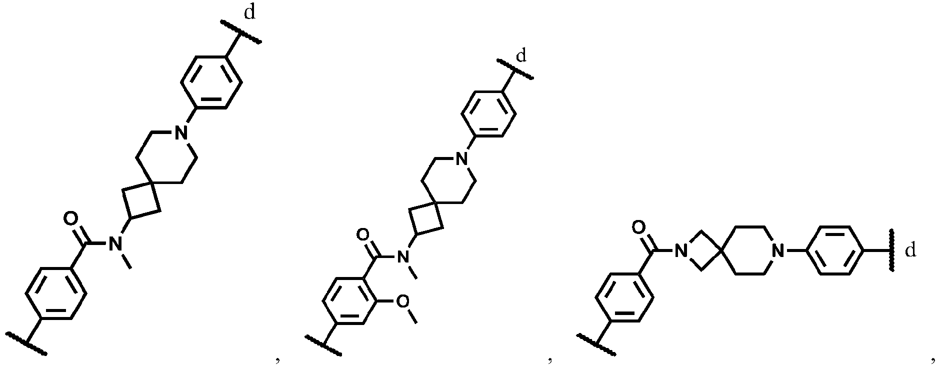 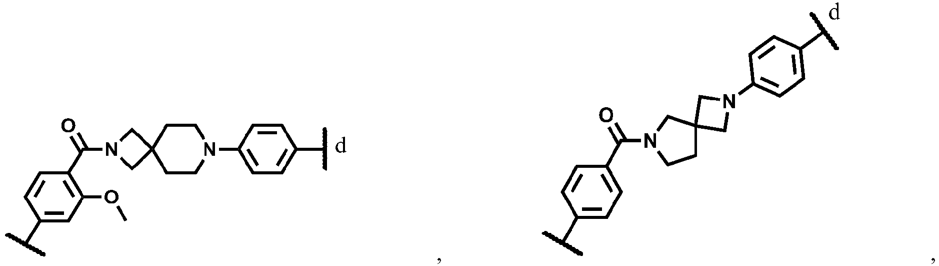 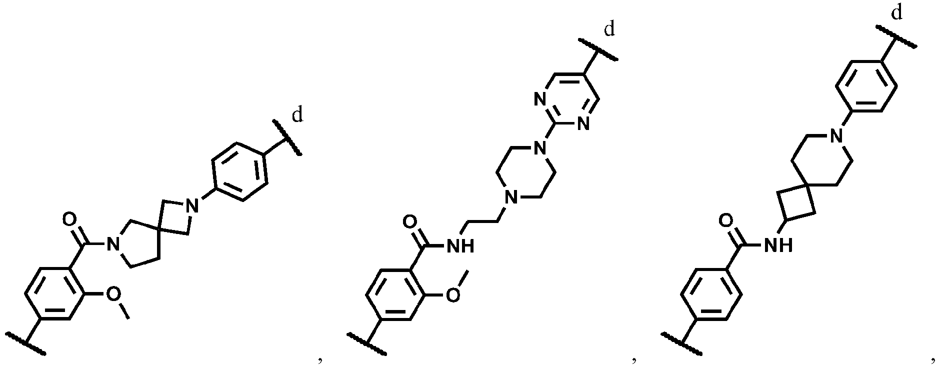 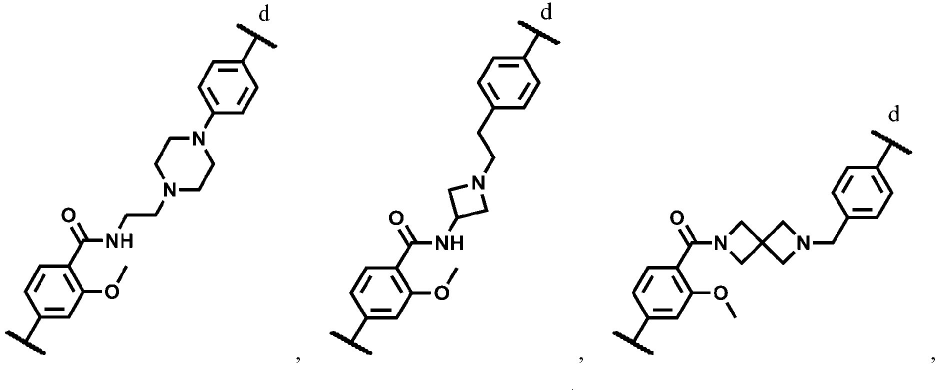 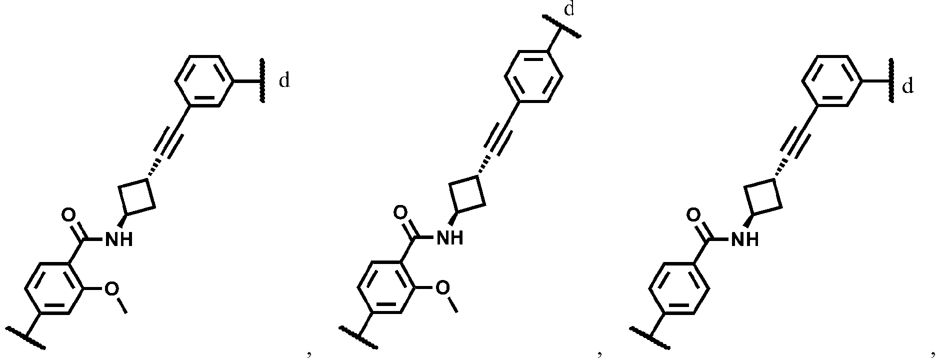 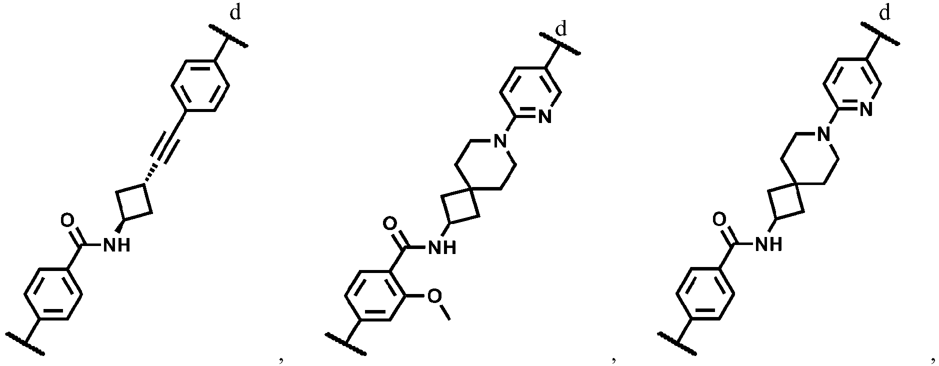 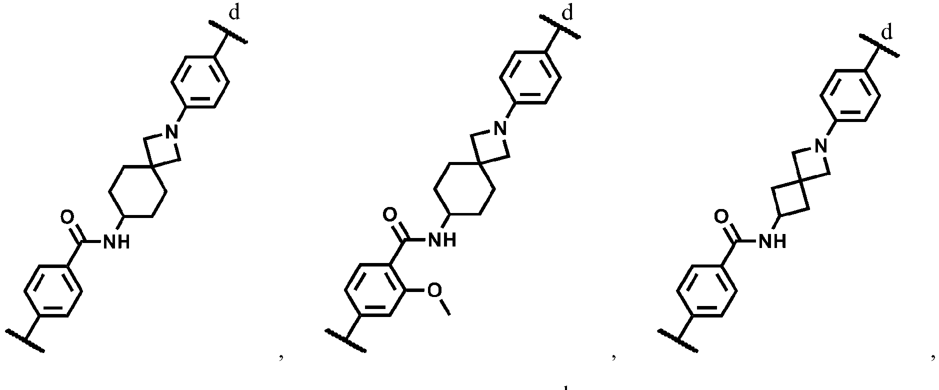 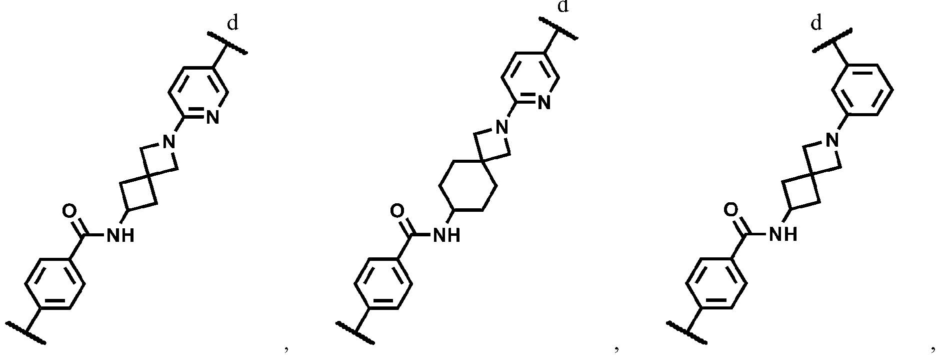 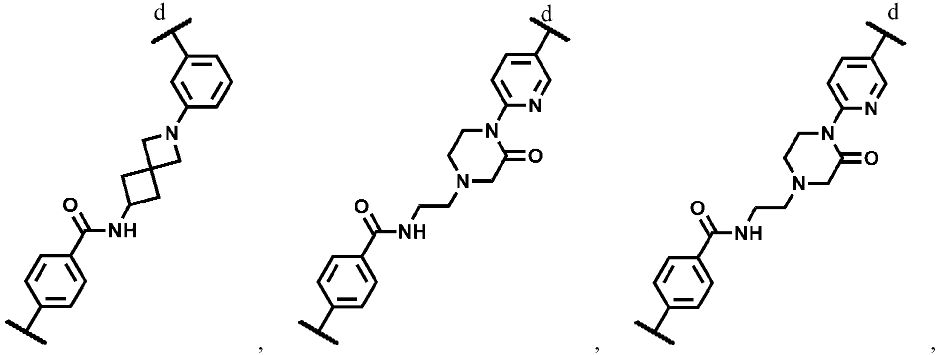 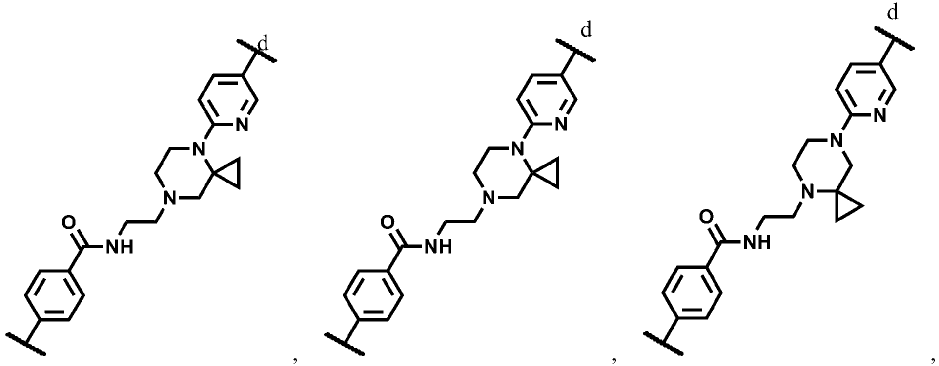 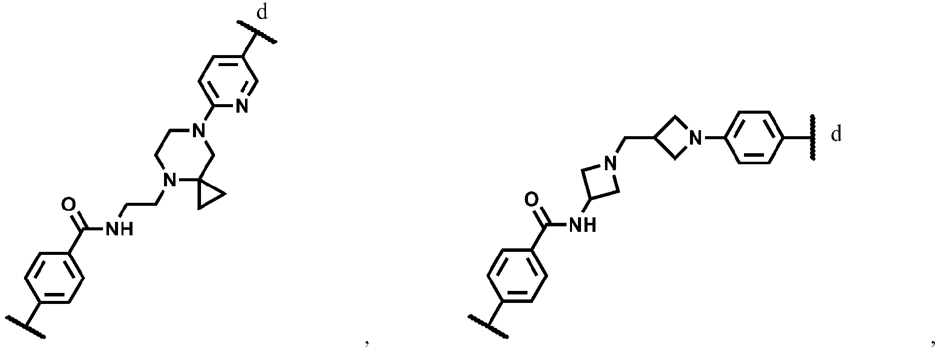 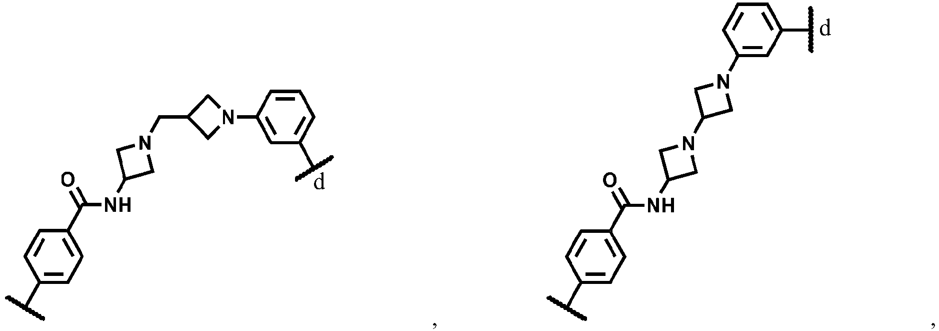 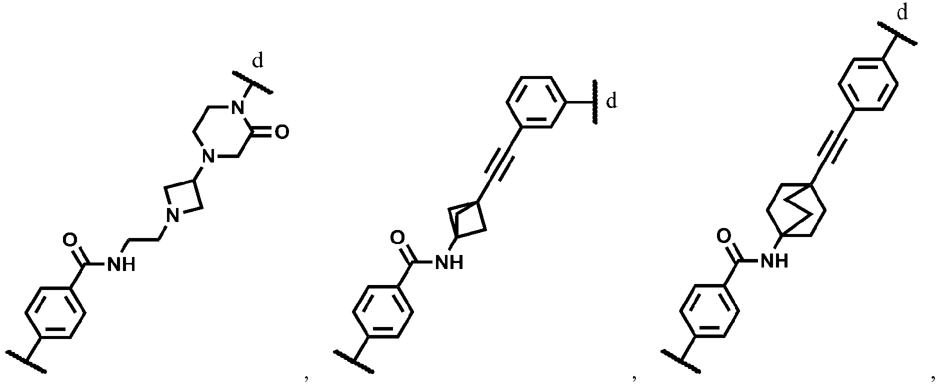 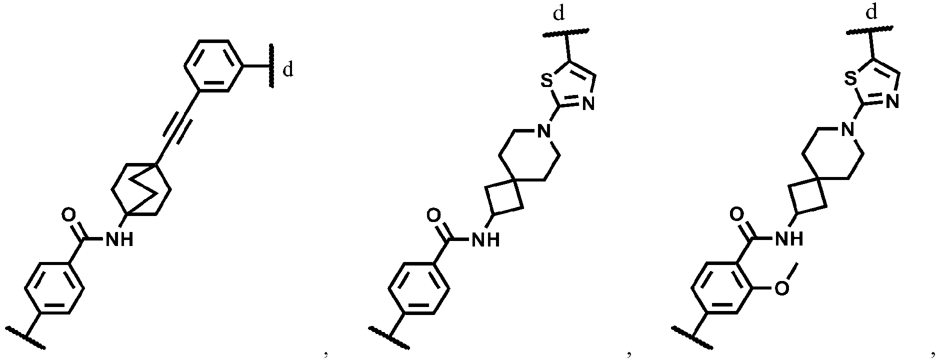 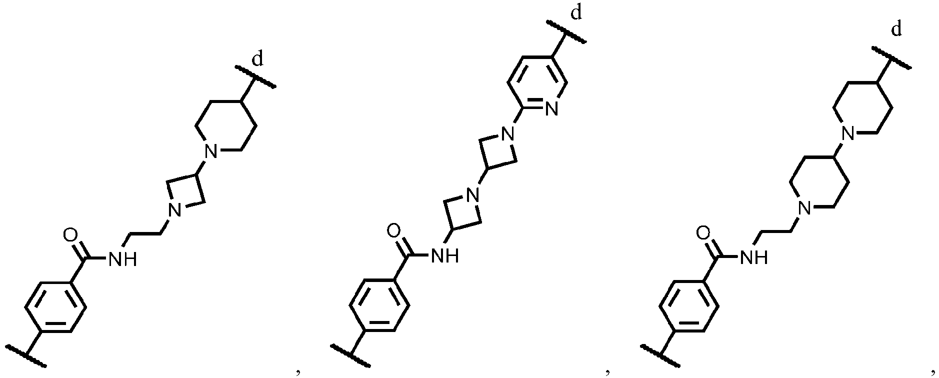 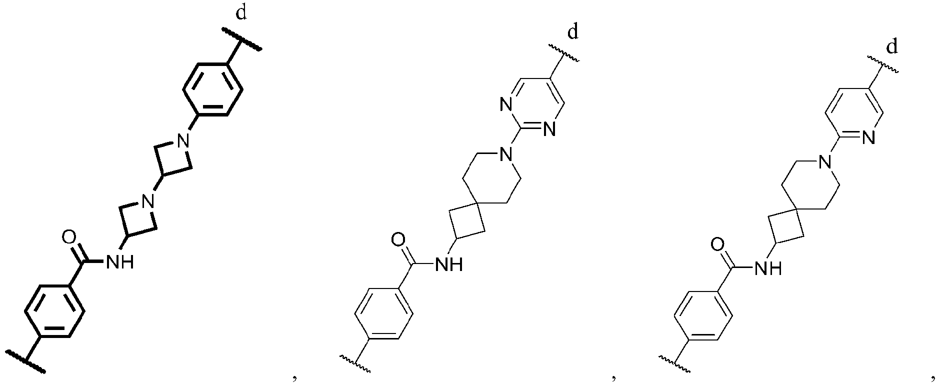 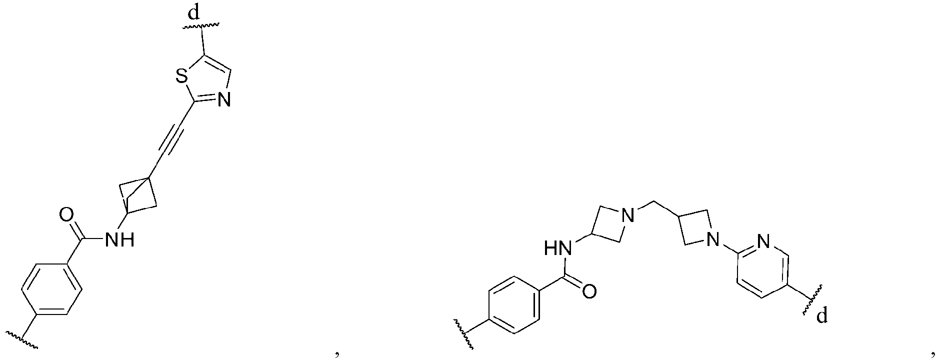 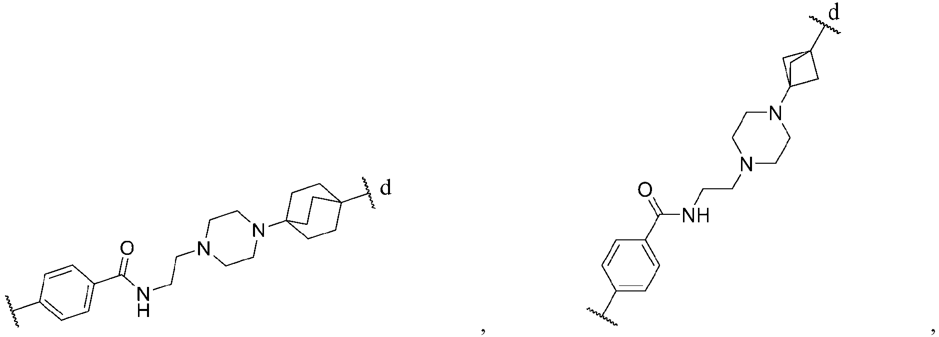 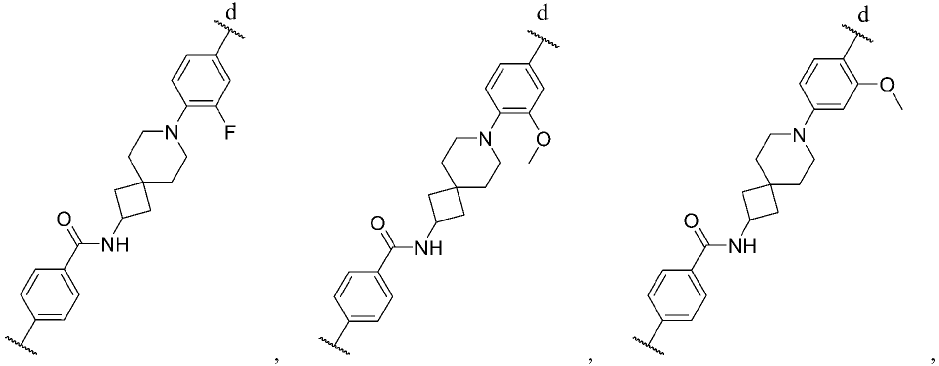 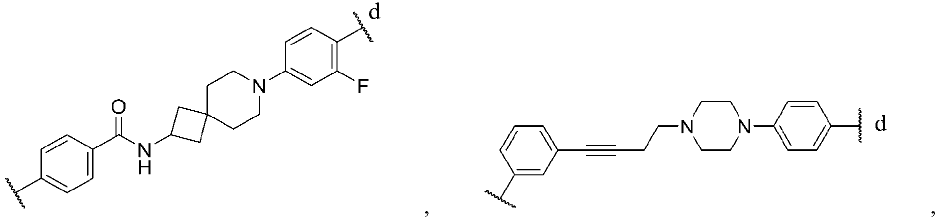 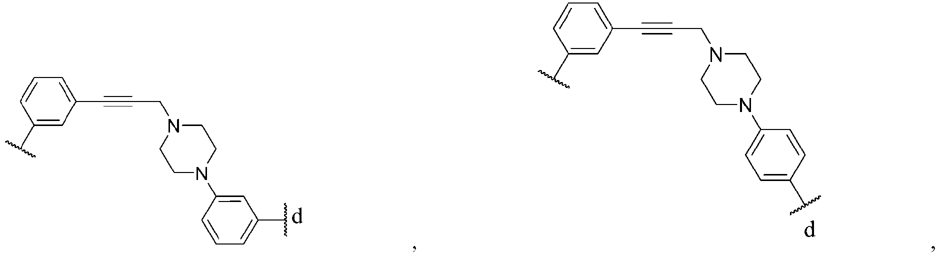 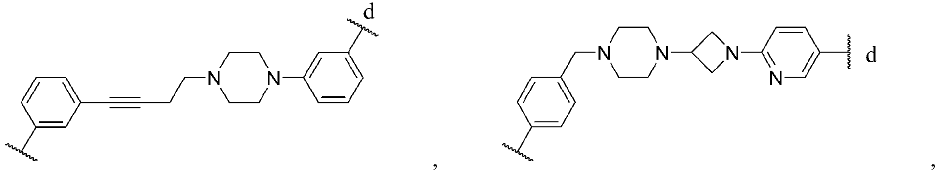 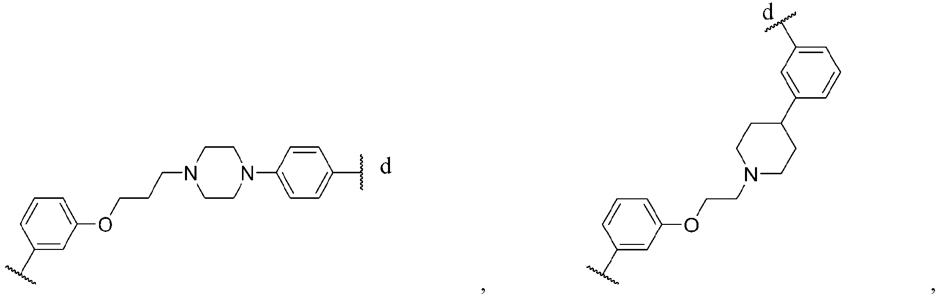 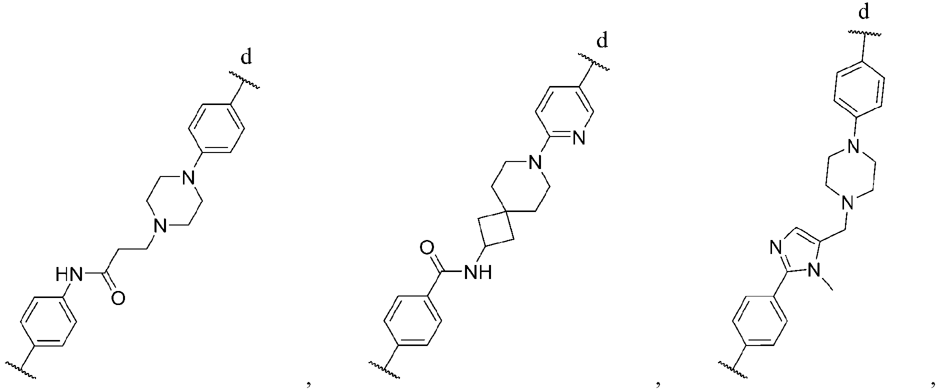 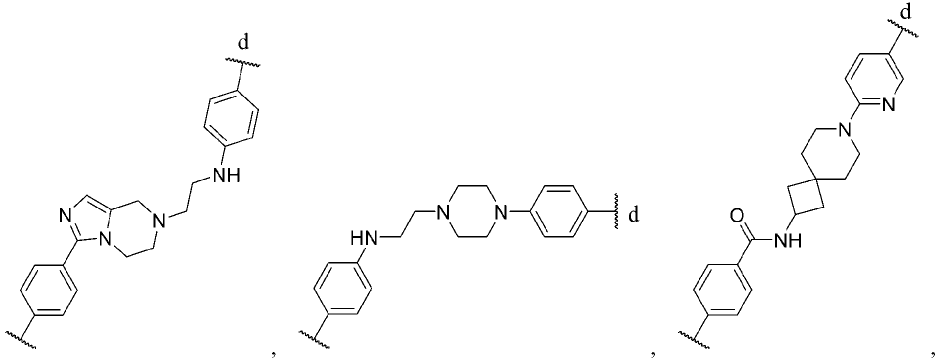 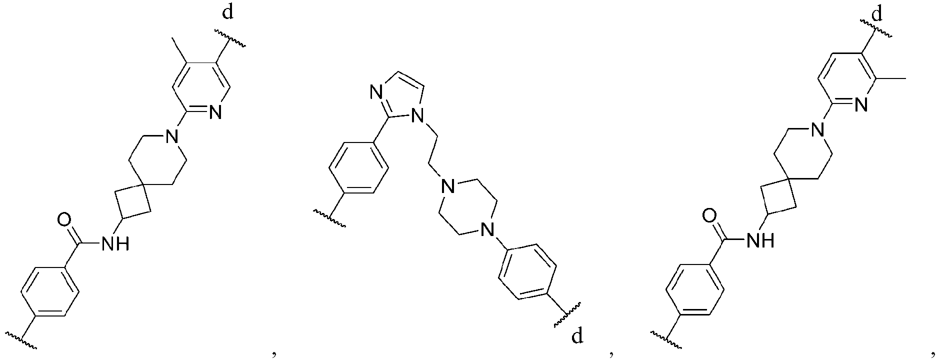 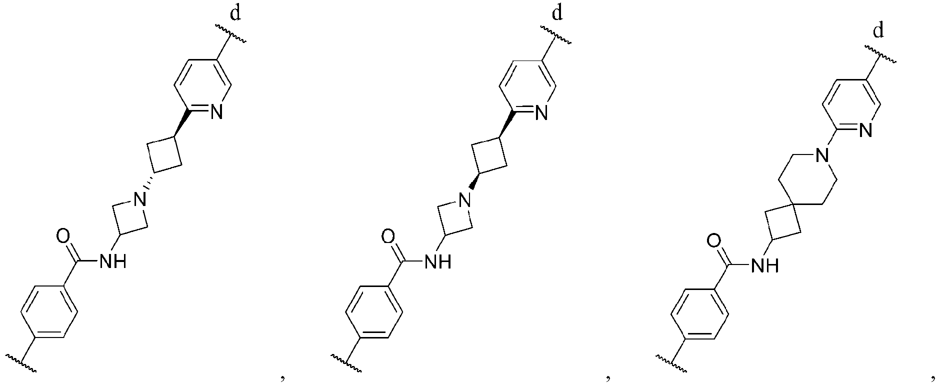 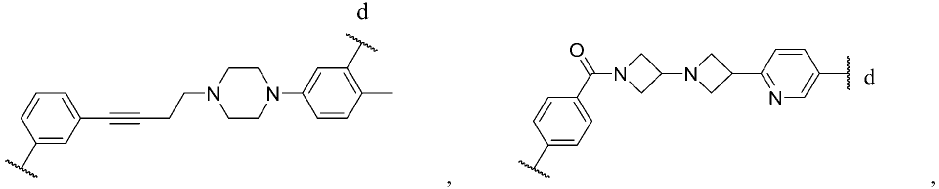 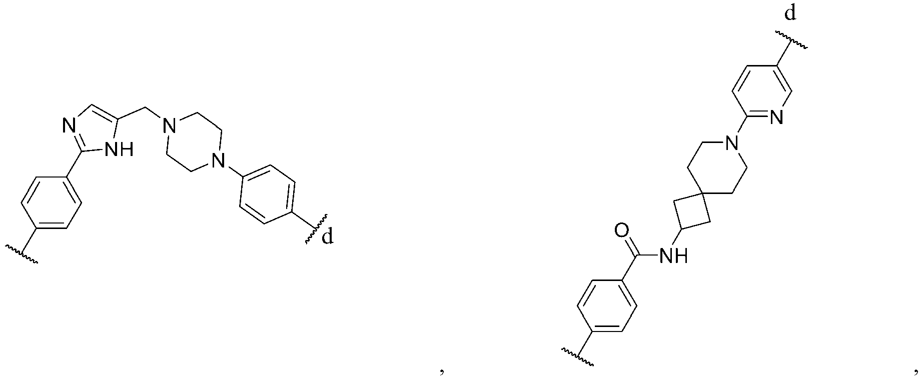 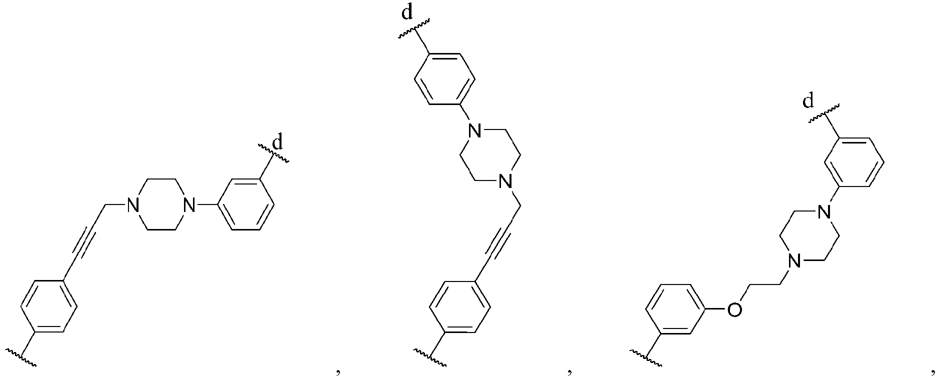 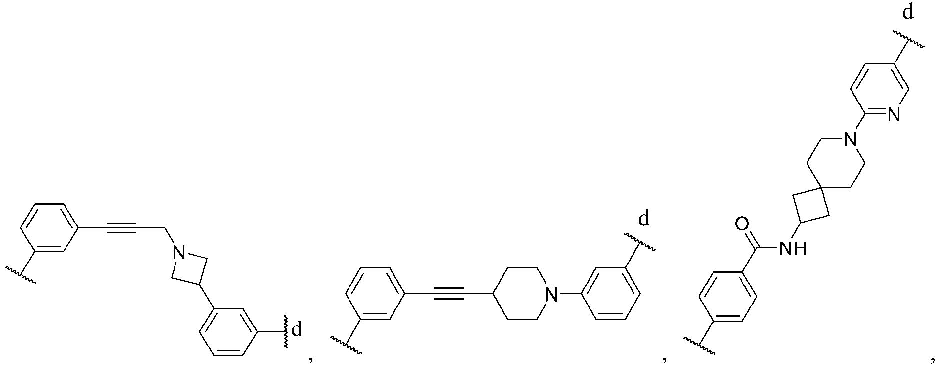 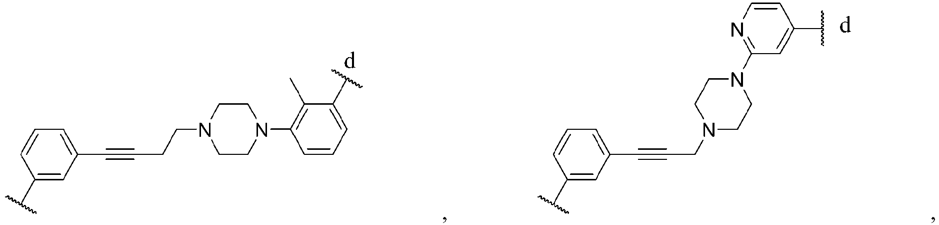 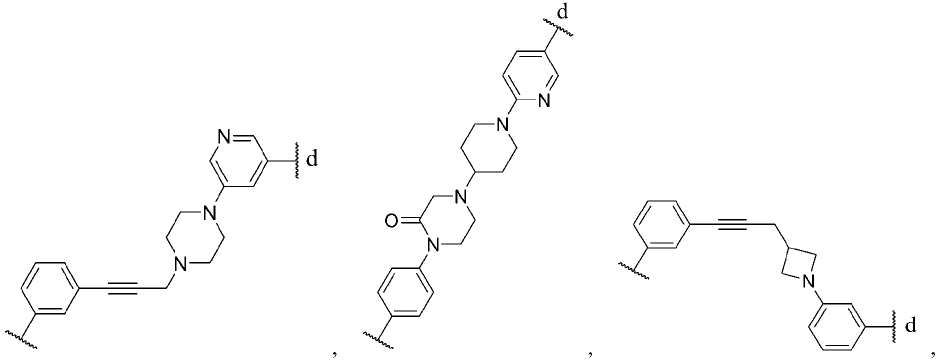 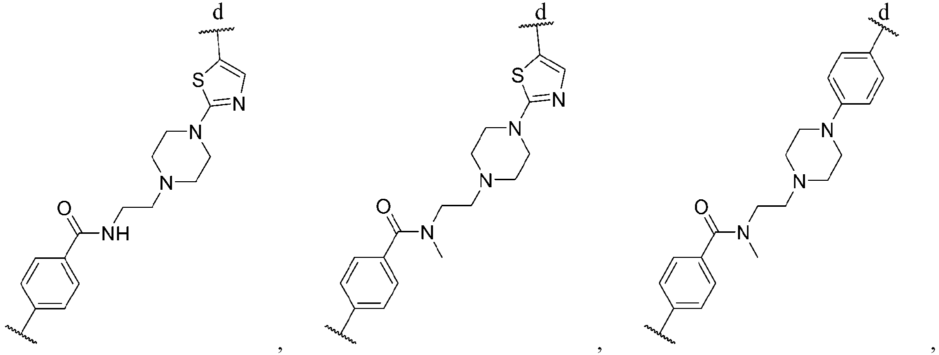 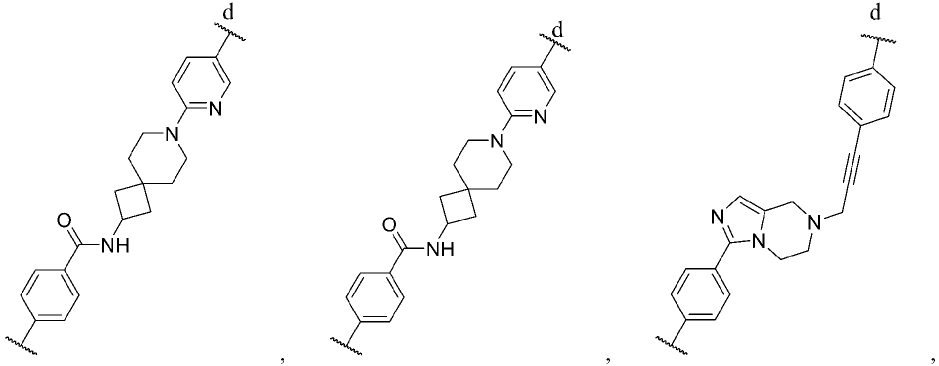 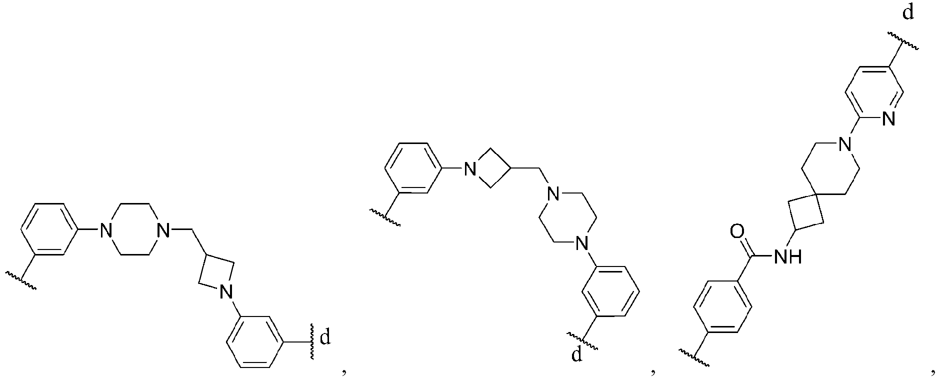 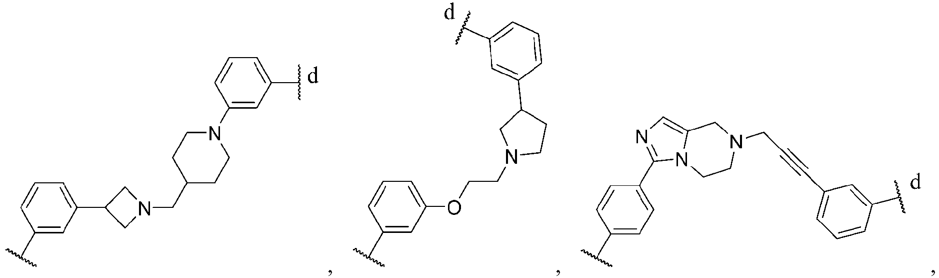 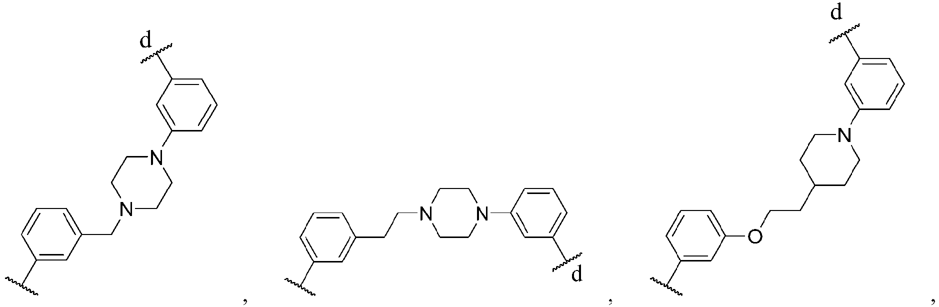 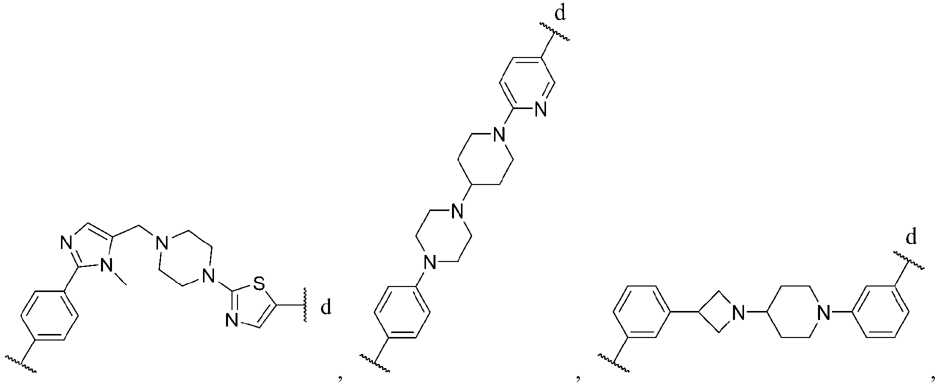 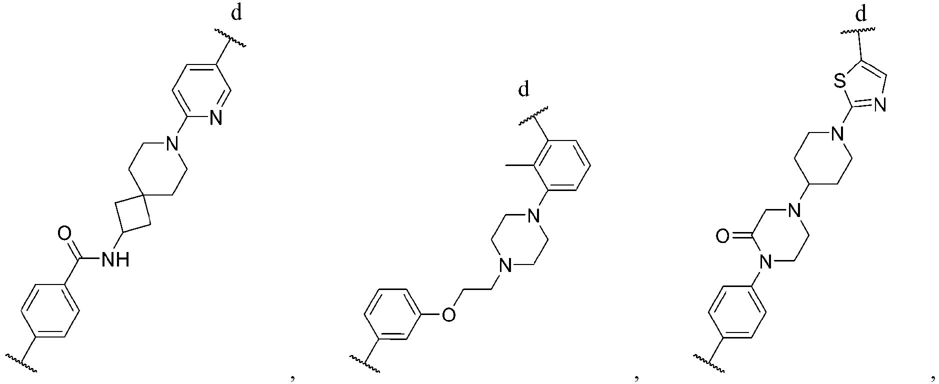 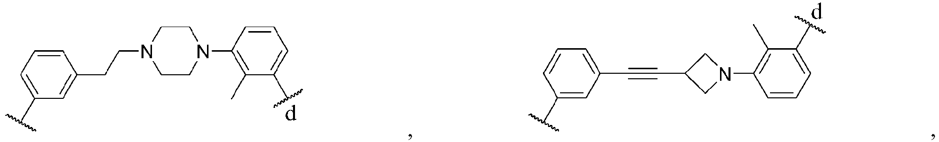 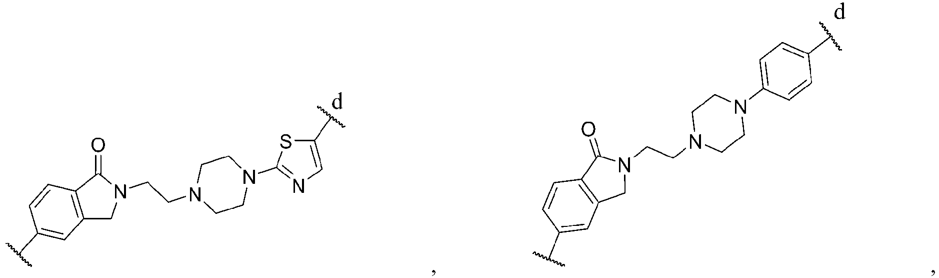 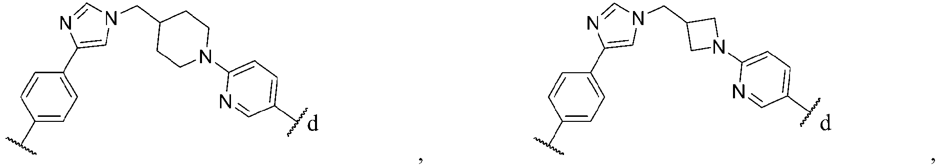 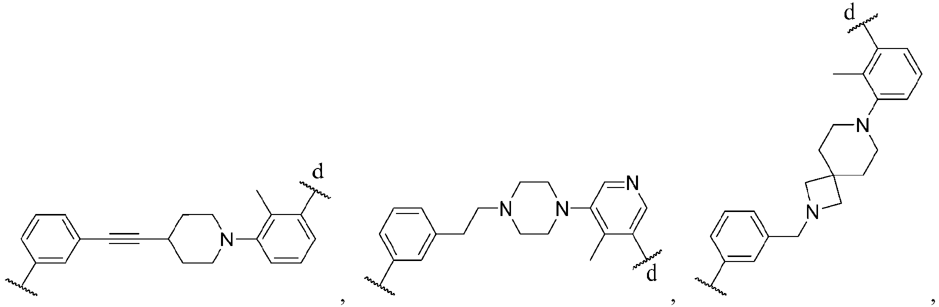 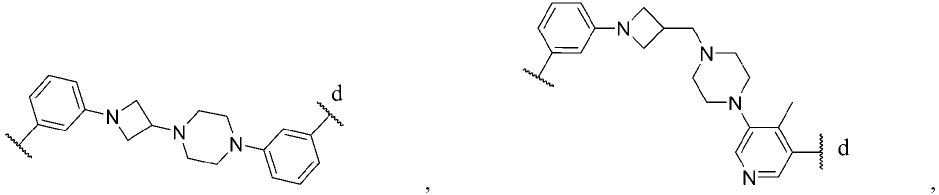 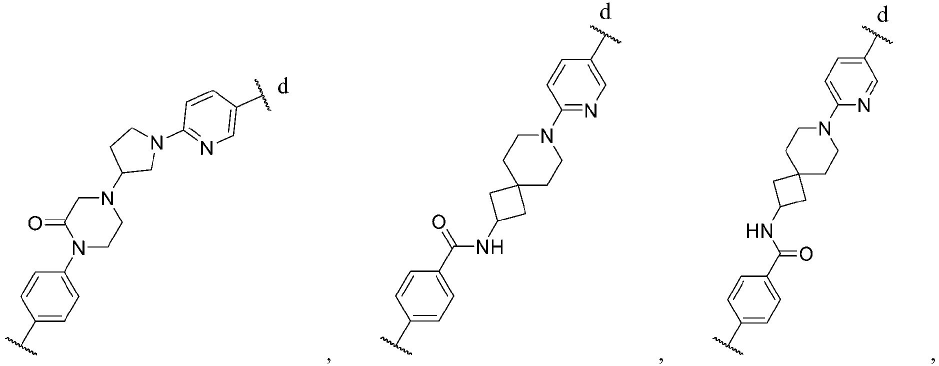 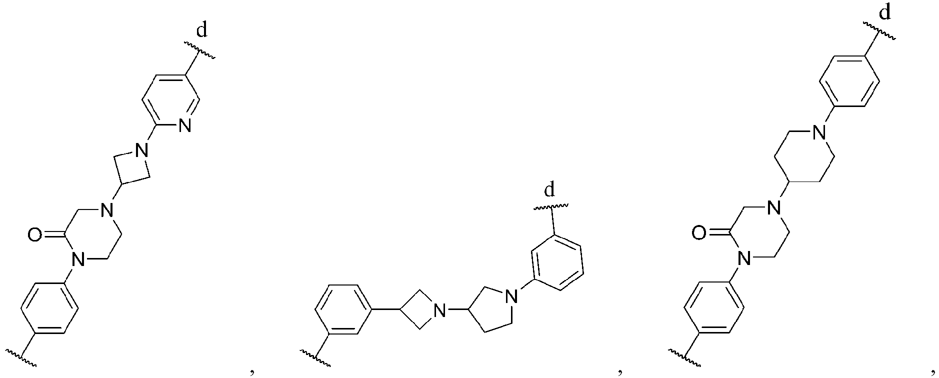 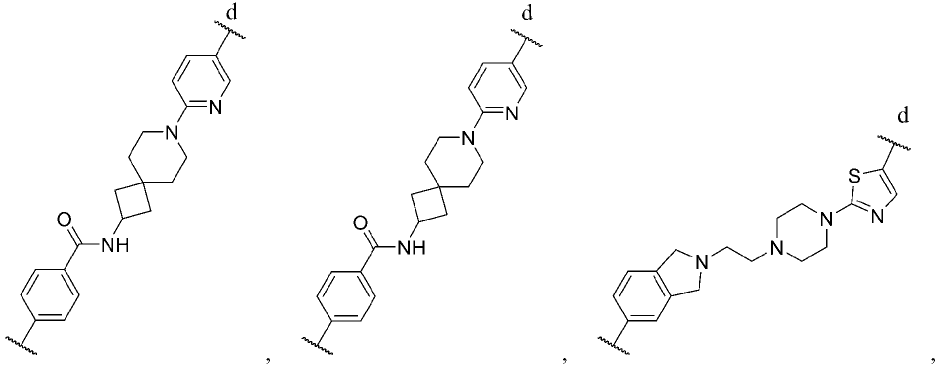 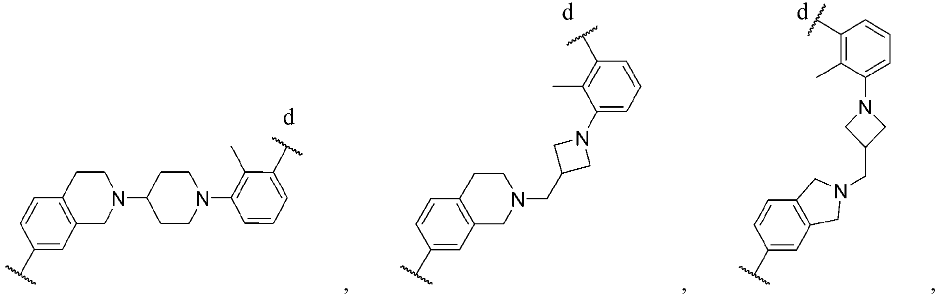 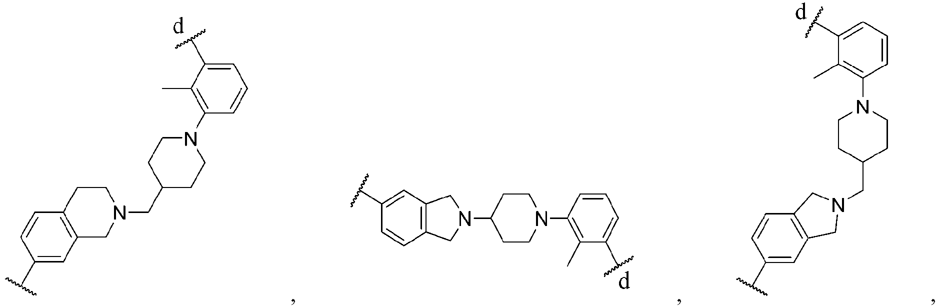 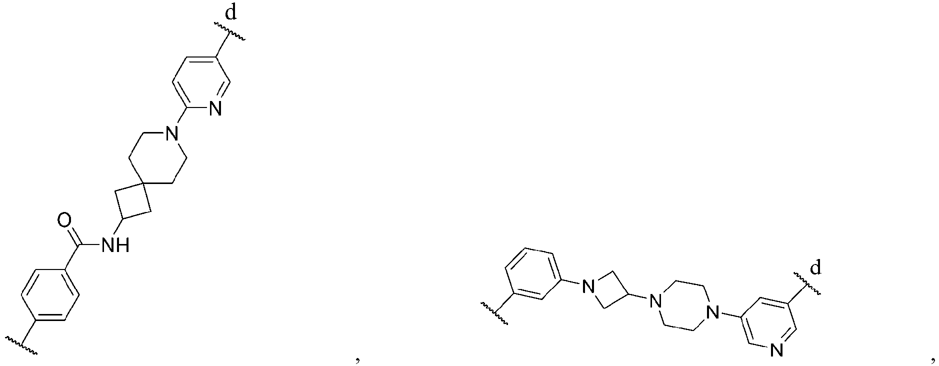 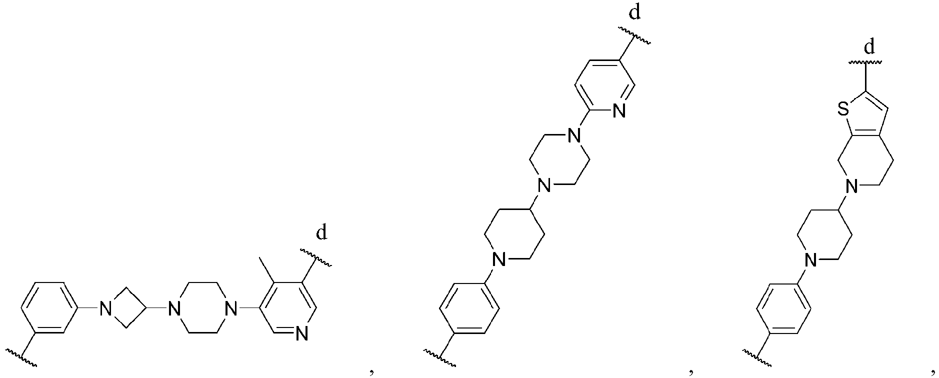 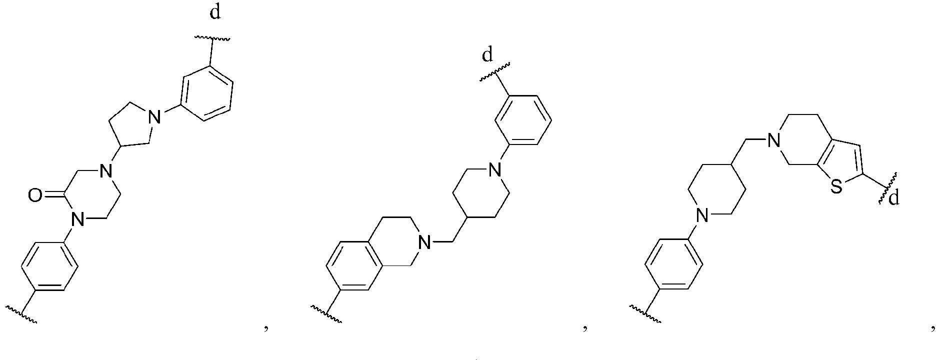 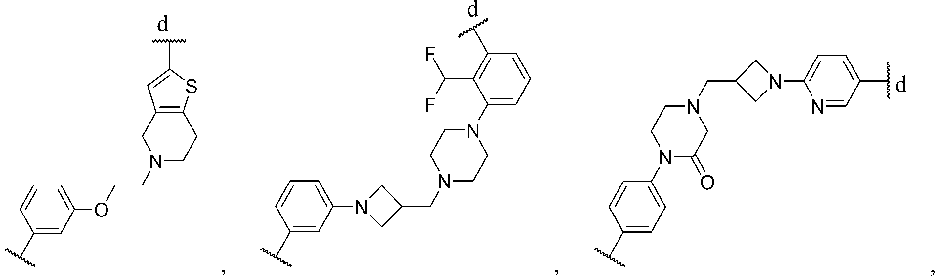 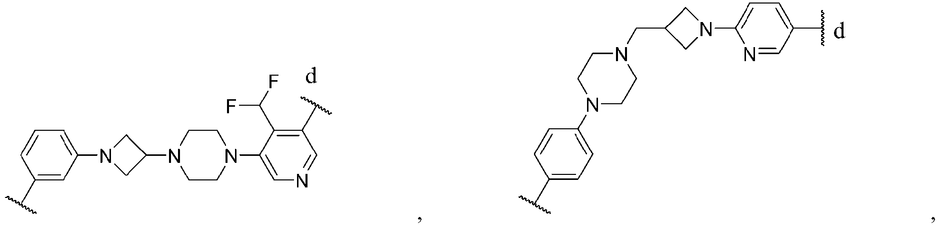 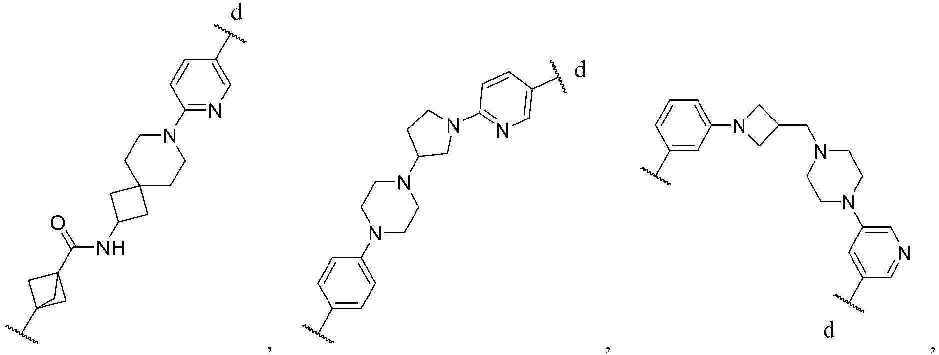 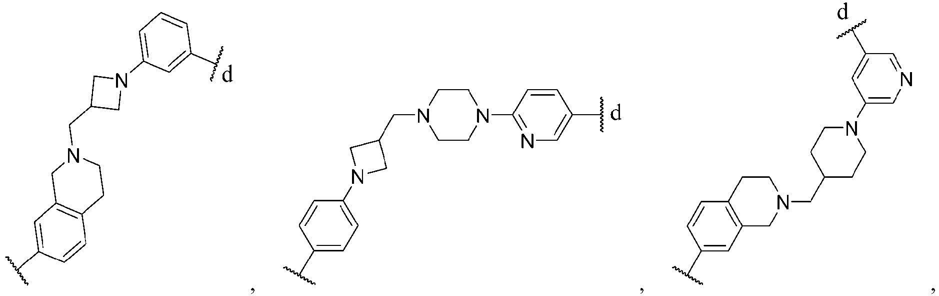 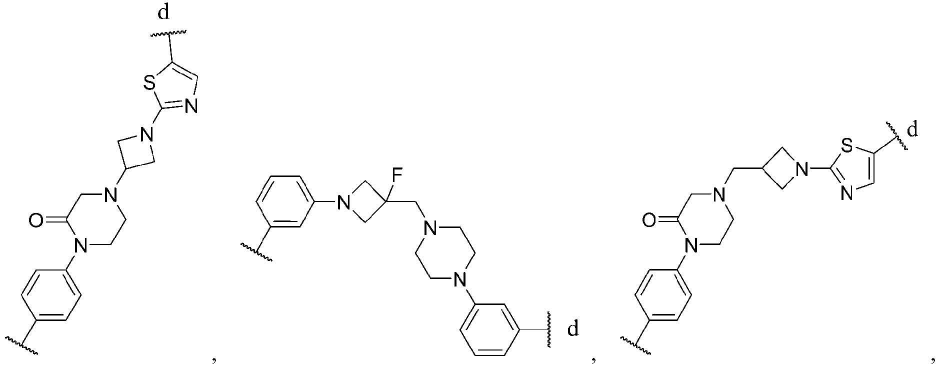 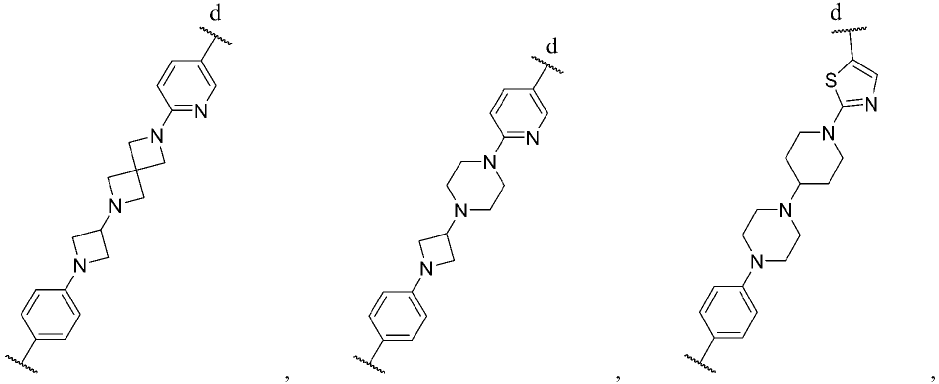 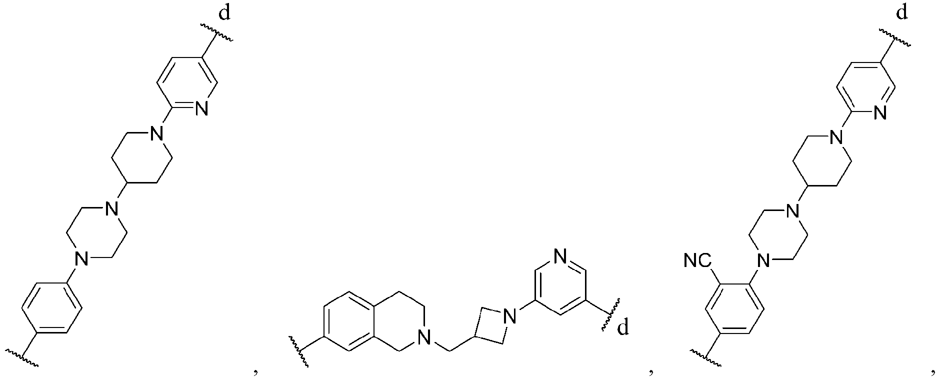 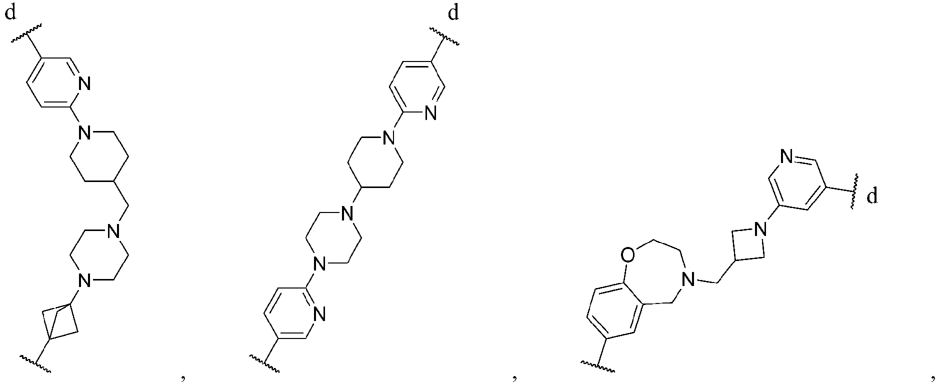 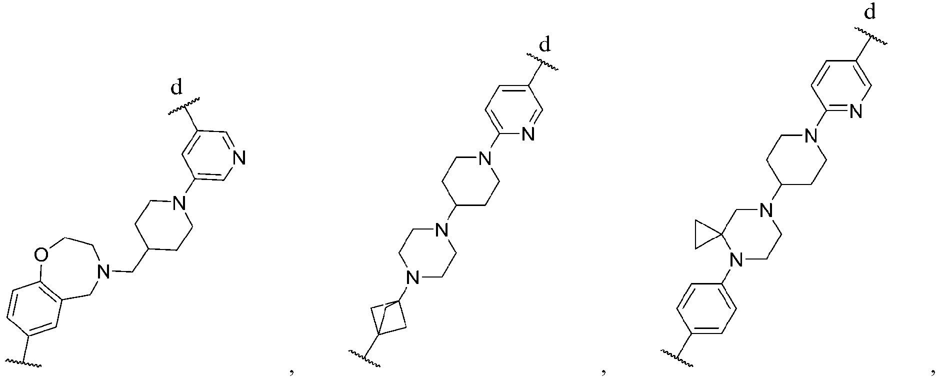 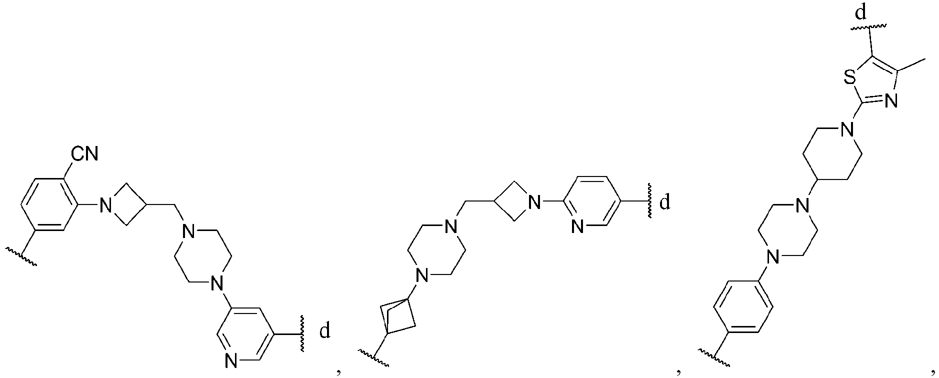 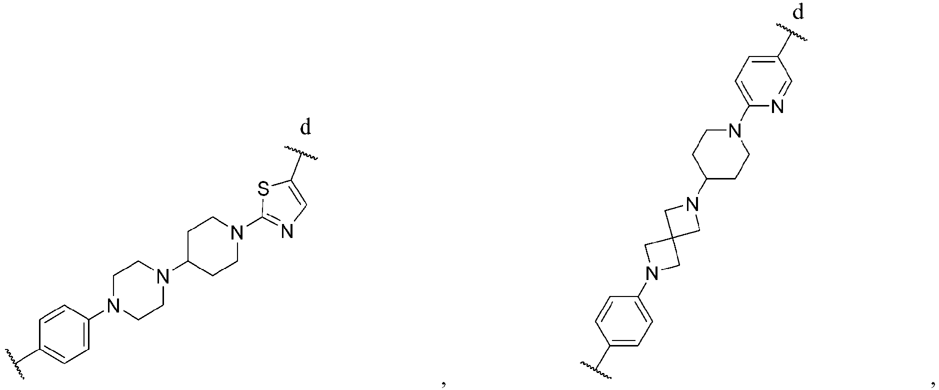 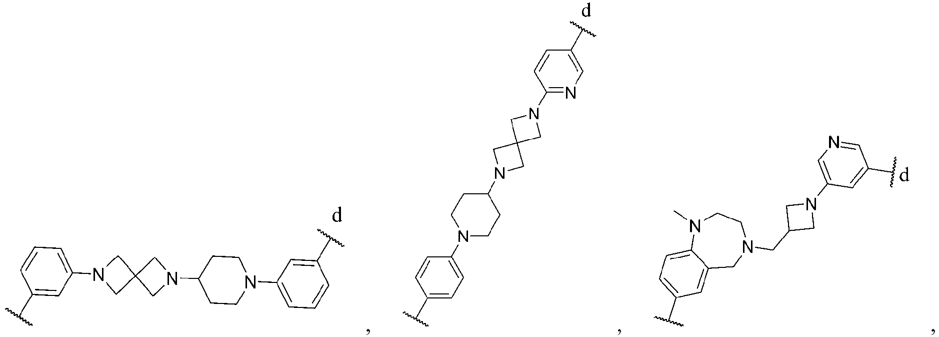 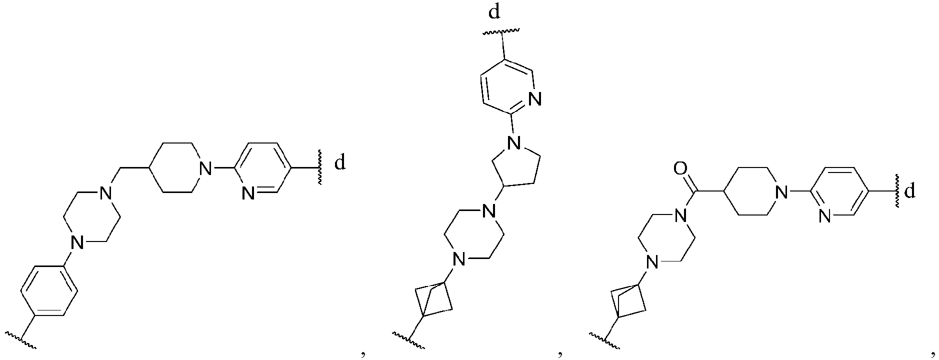 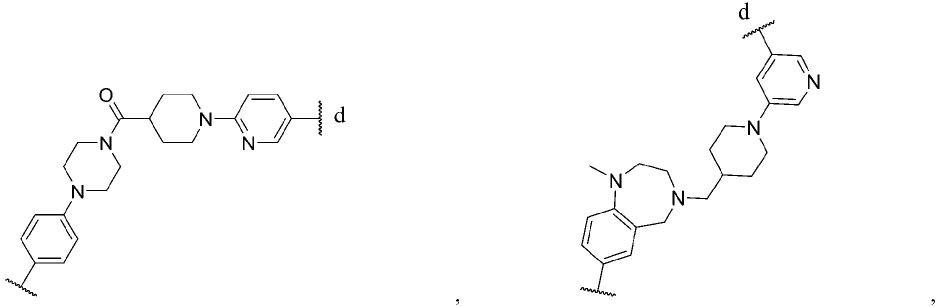 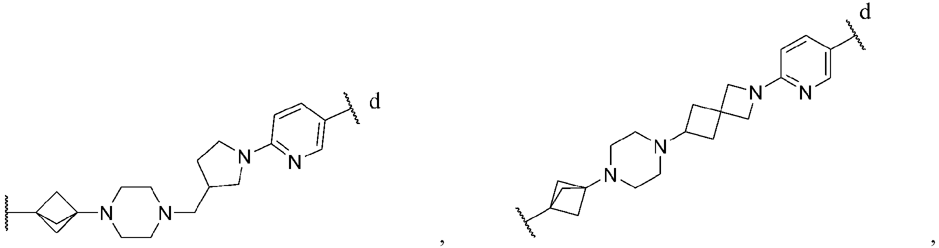 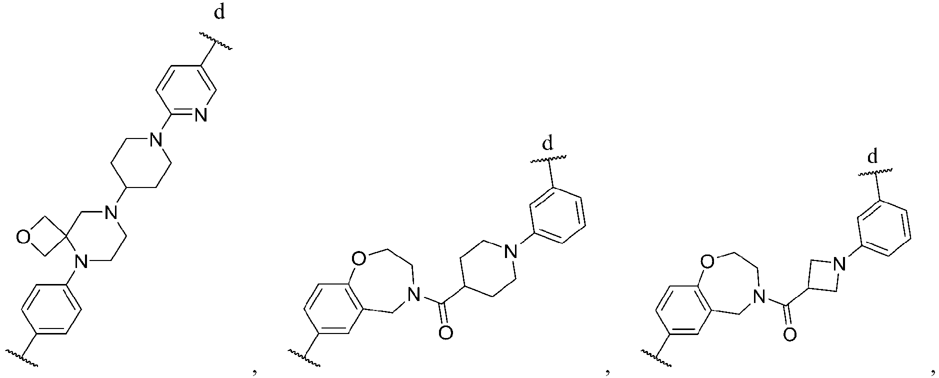 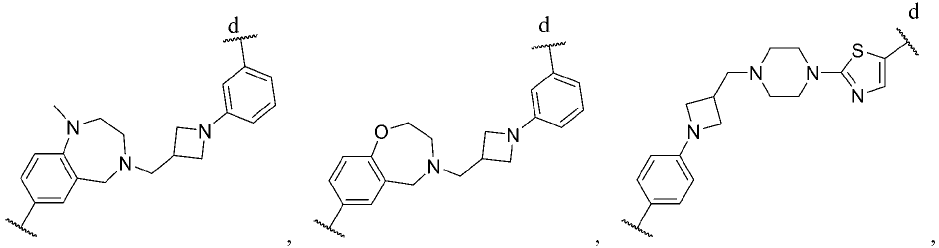 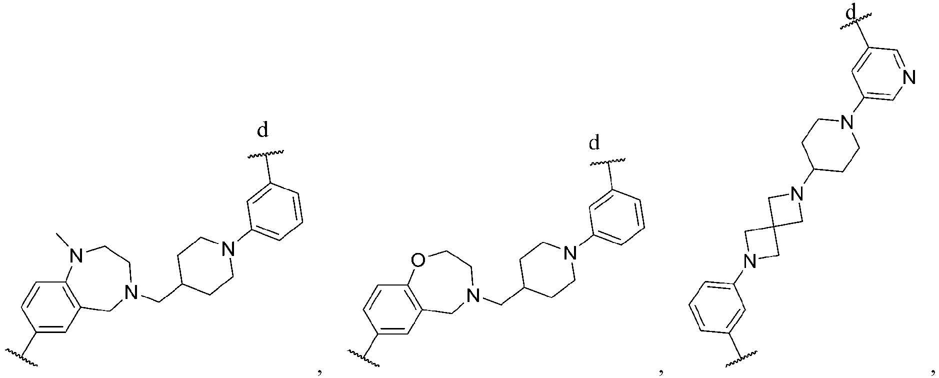 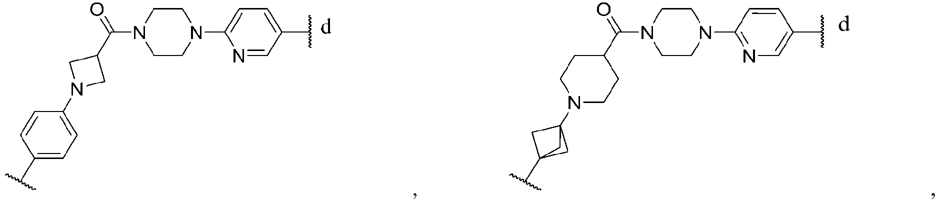 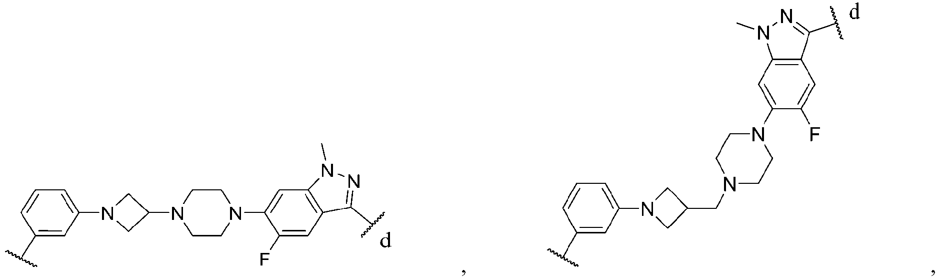 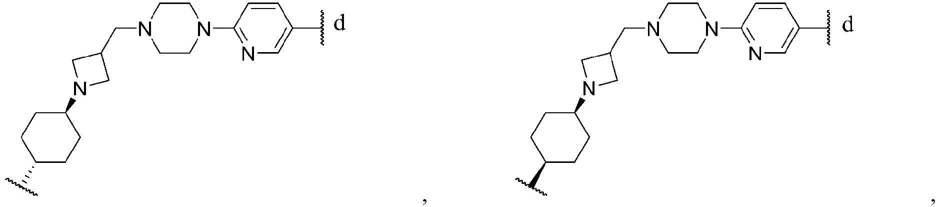 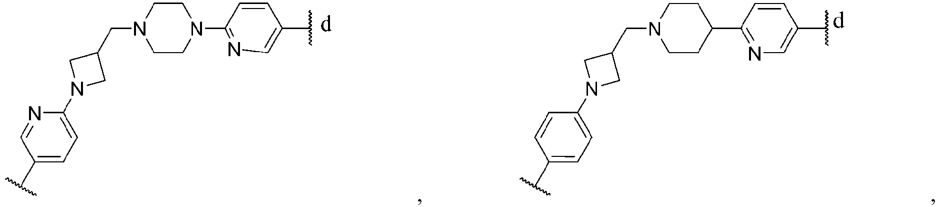 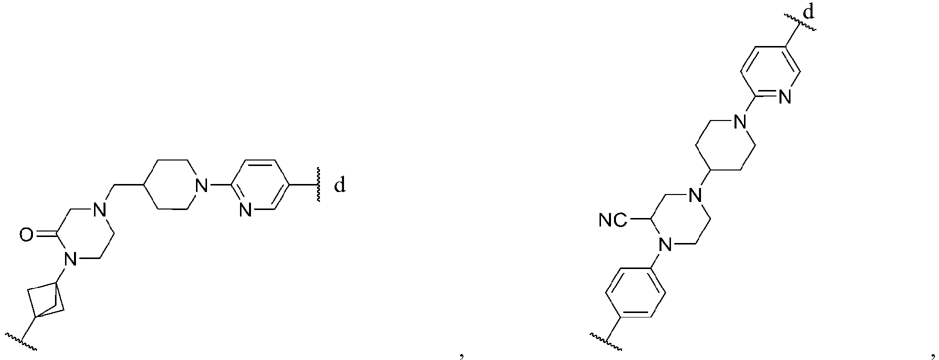 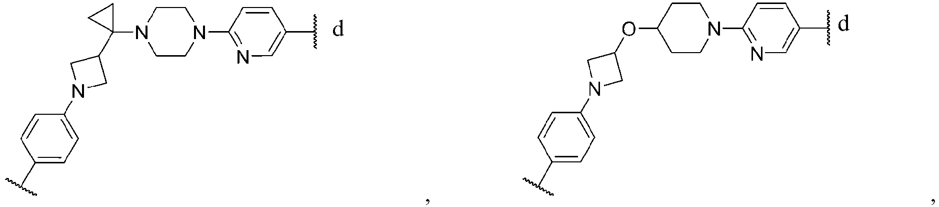 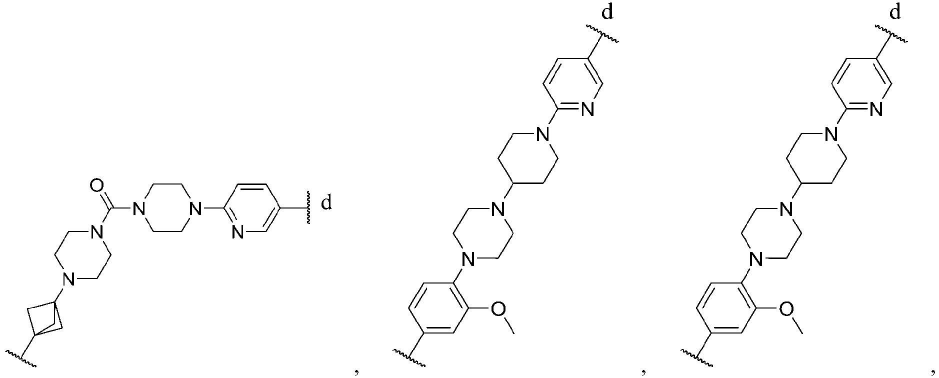 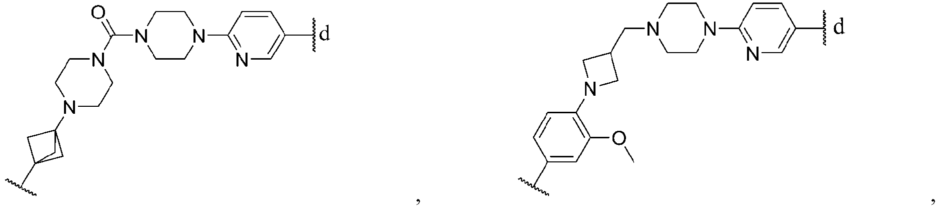 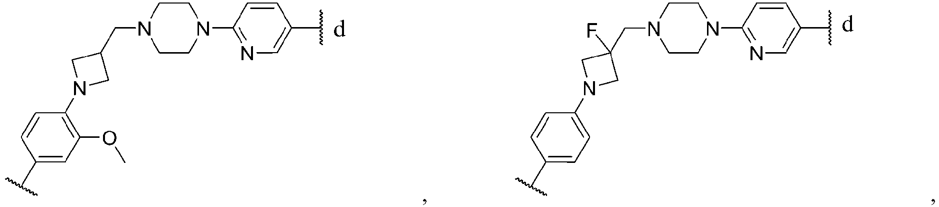 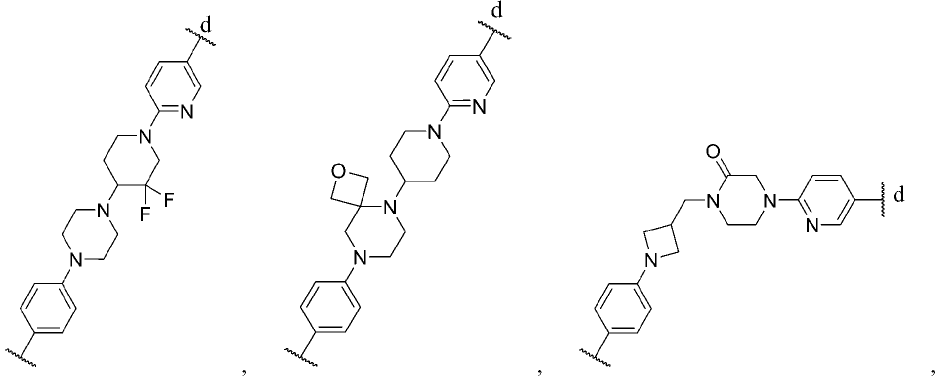 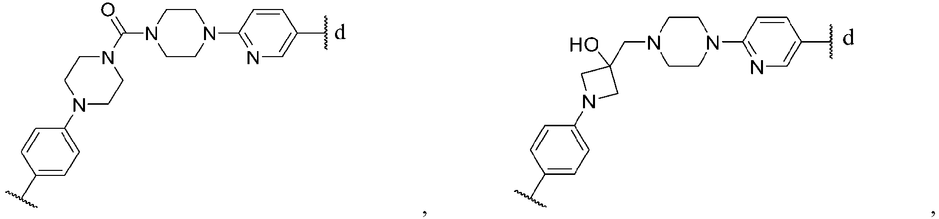 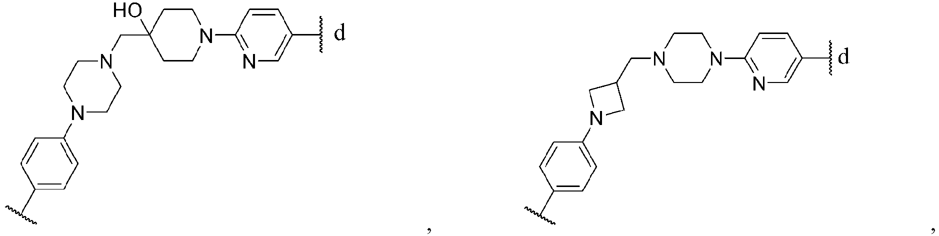 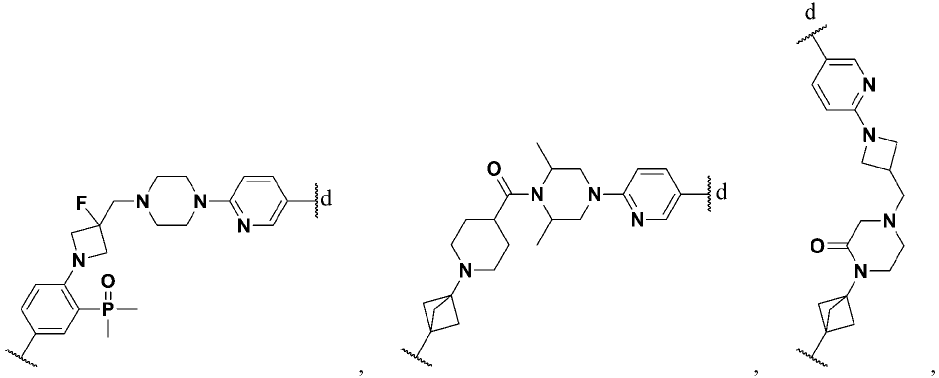 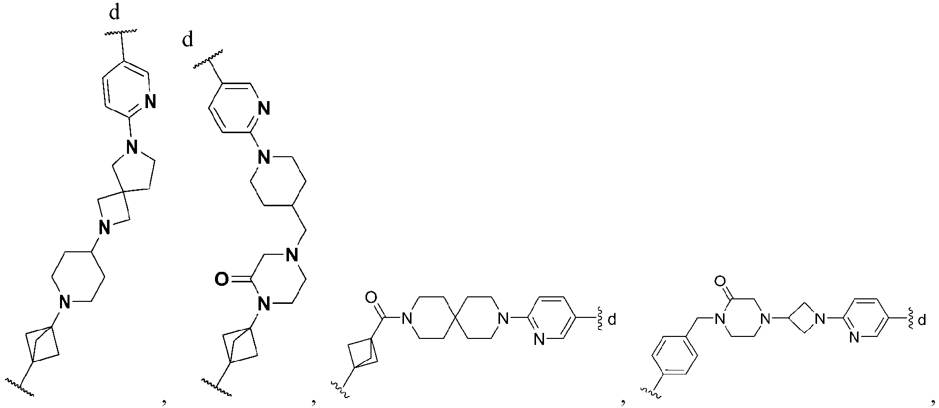 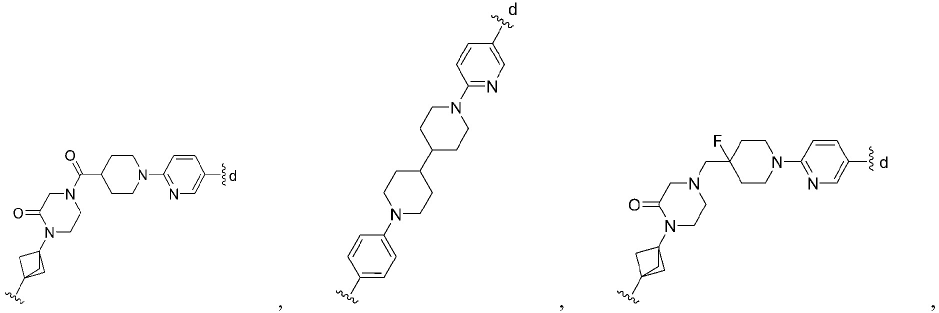 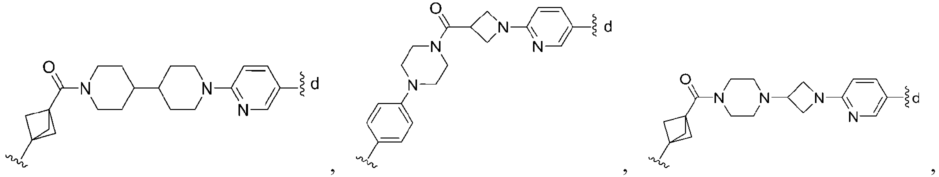 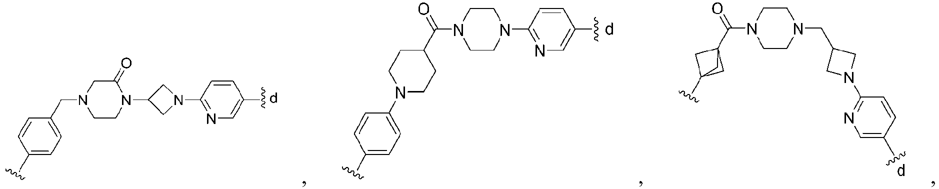 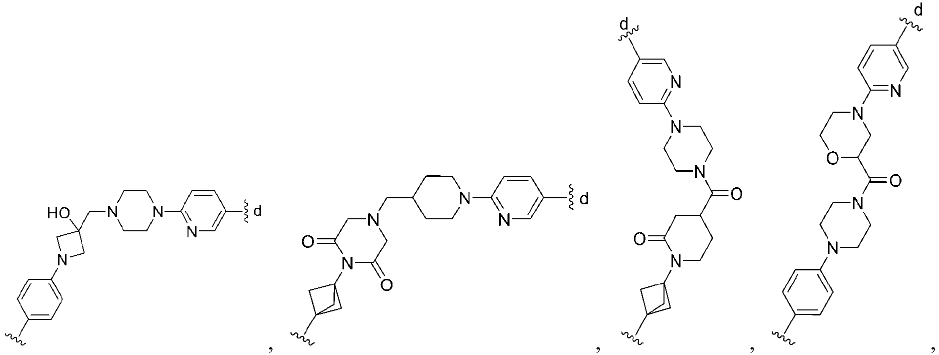 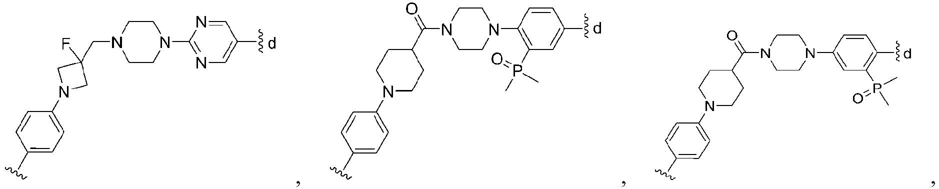 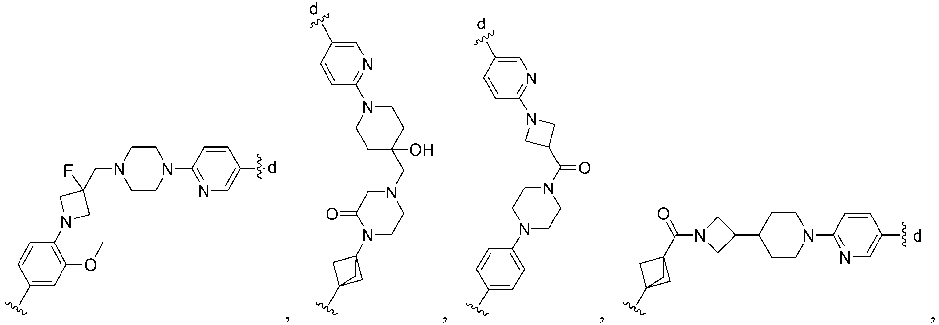 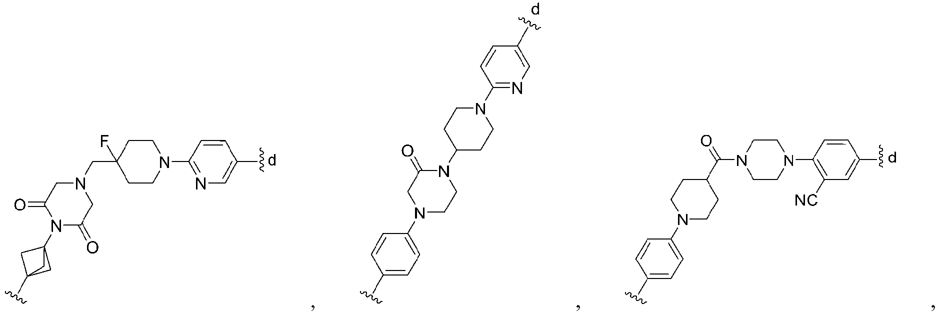 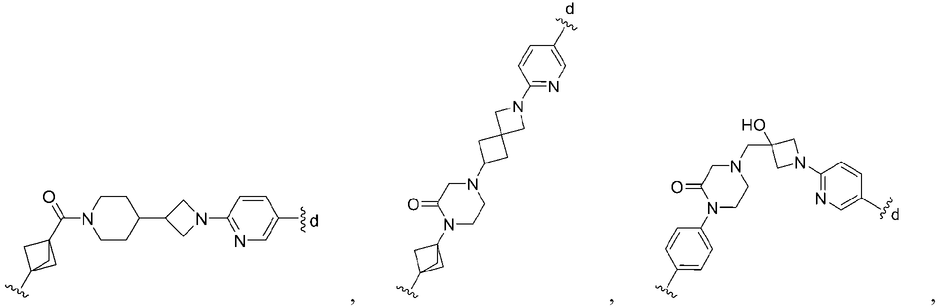 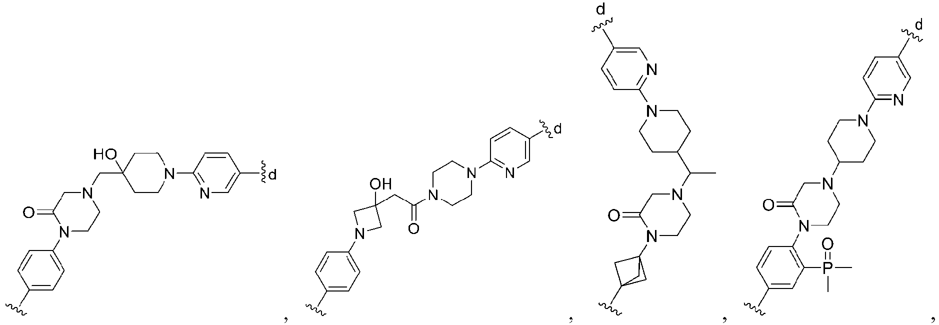 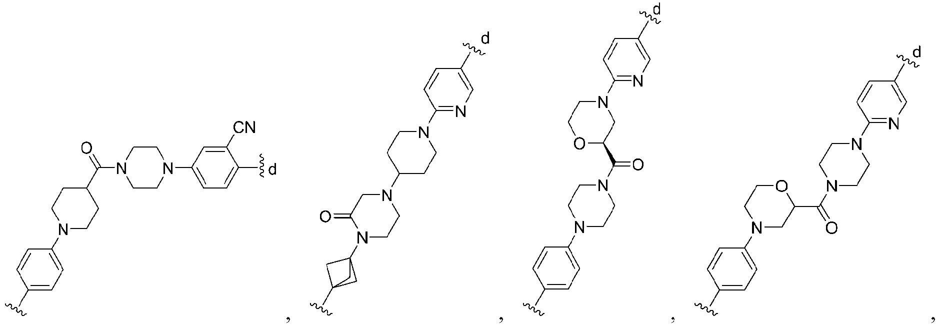 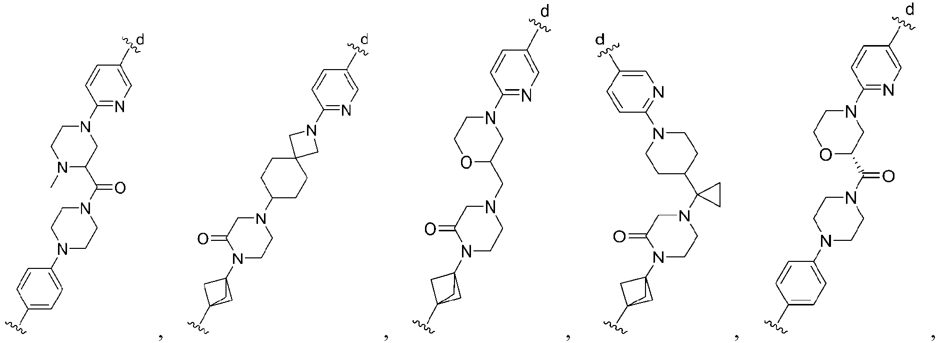 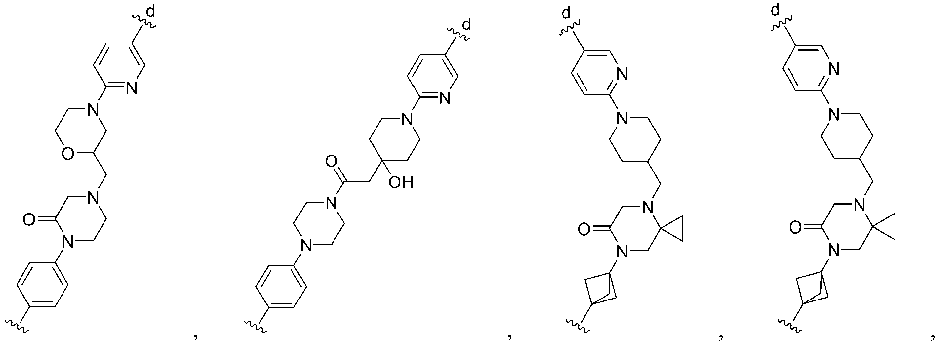 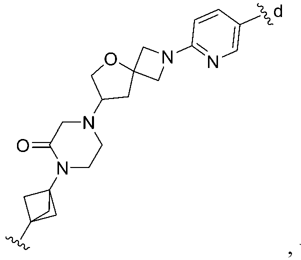 wherein side d is connected to 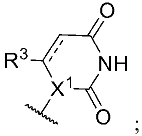 preferably, in Scheme ii, - A-L-B- is any one of the above groups except 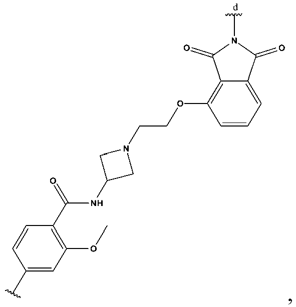 and in Scheme i, -A-L-B- is

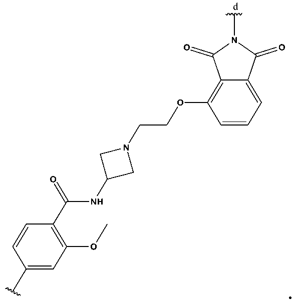

15. The compound represented by formula (I), the pharmaceutically acceptable salt thereof, the solvate thereof or the solvate of the pharmaceutically acceptable salt thereof according to any one of claims 1-6, wherein,
(1) -A-L-B- is: 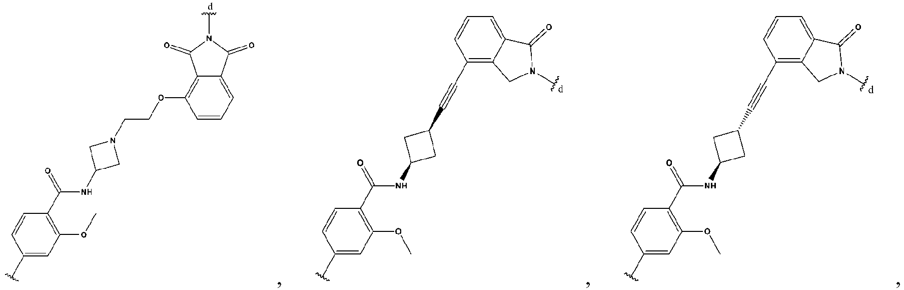 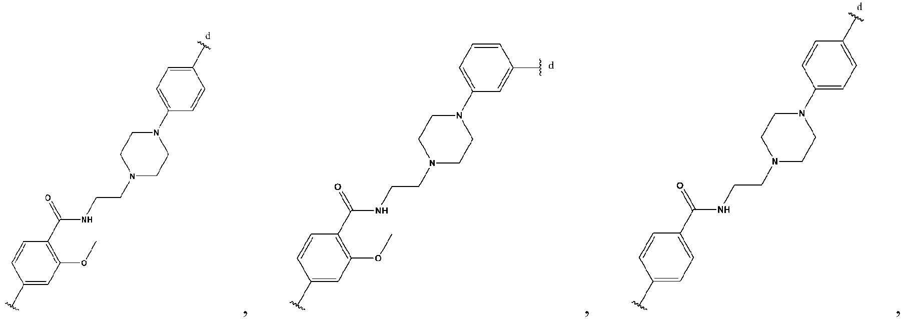 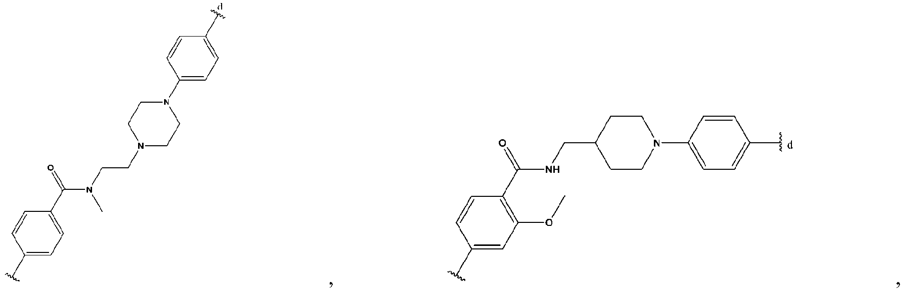 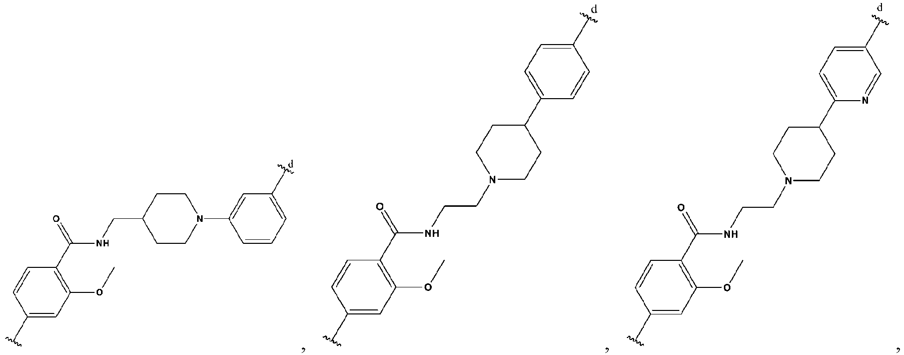 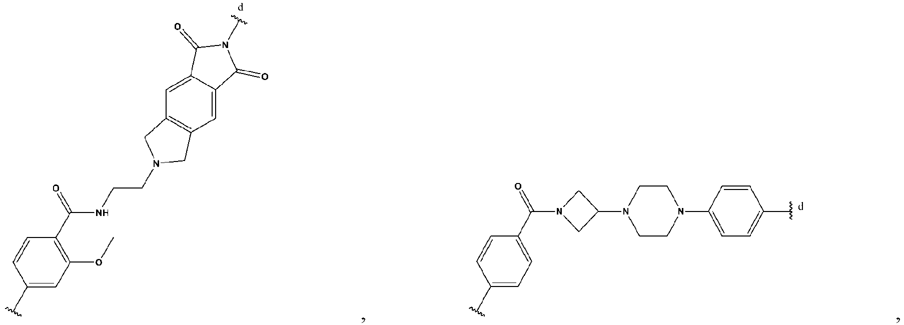 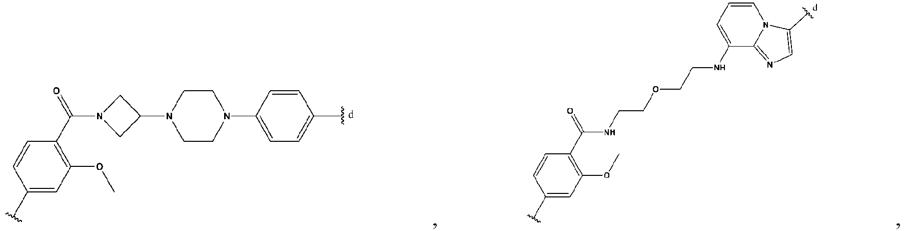 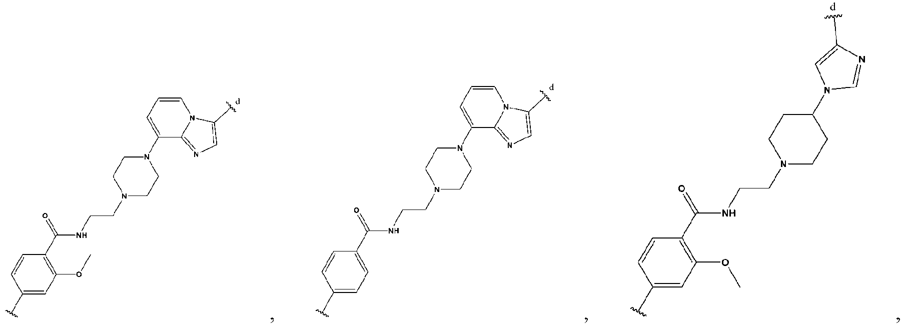 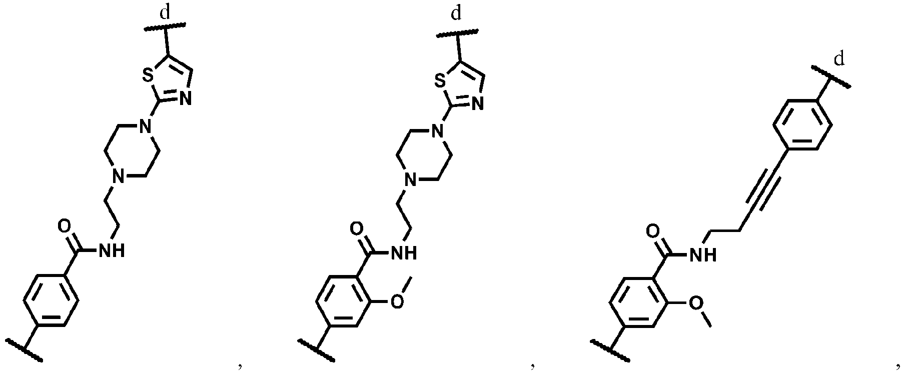 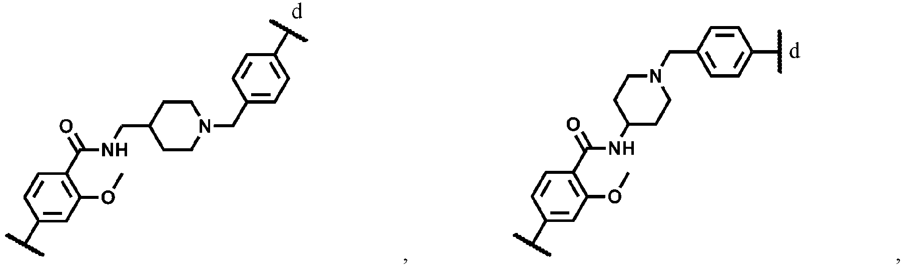 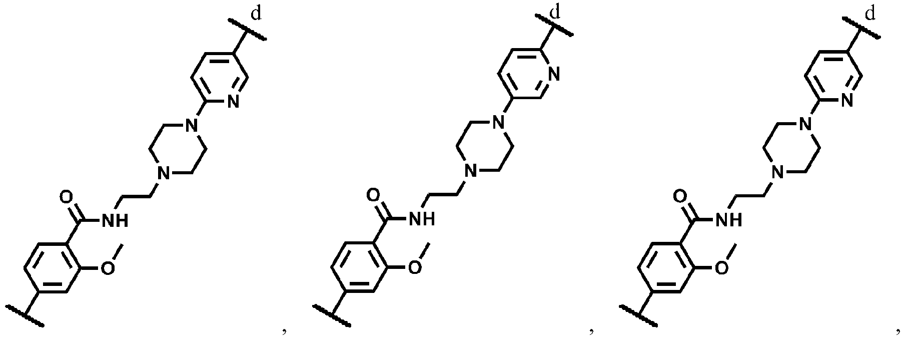 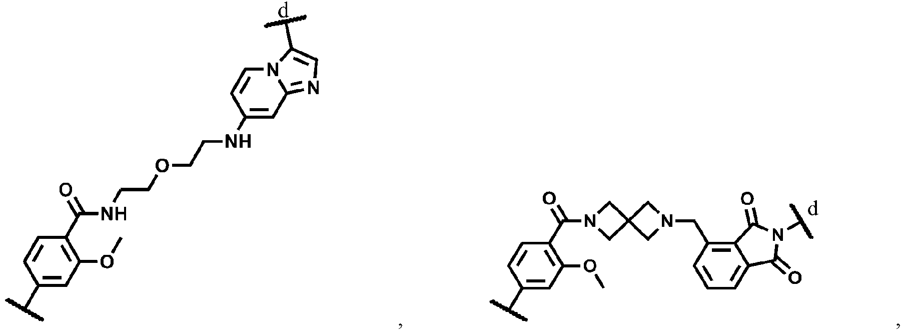 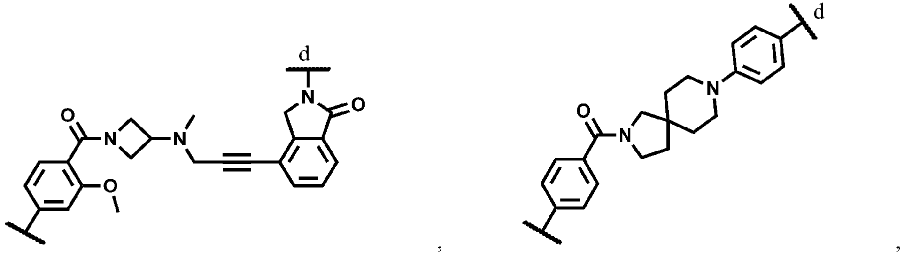 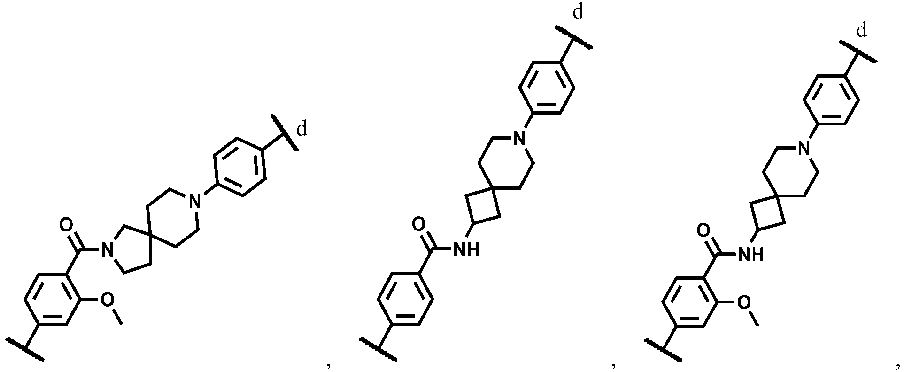 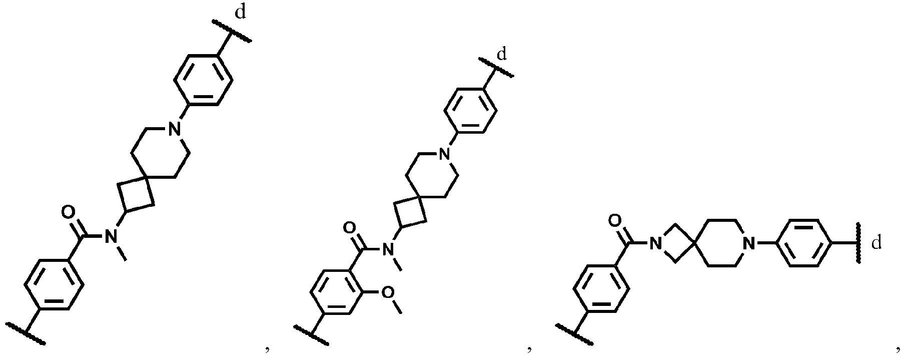 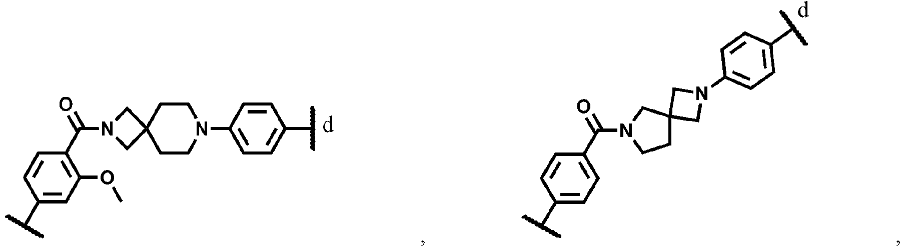 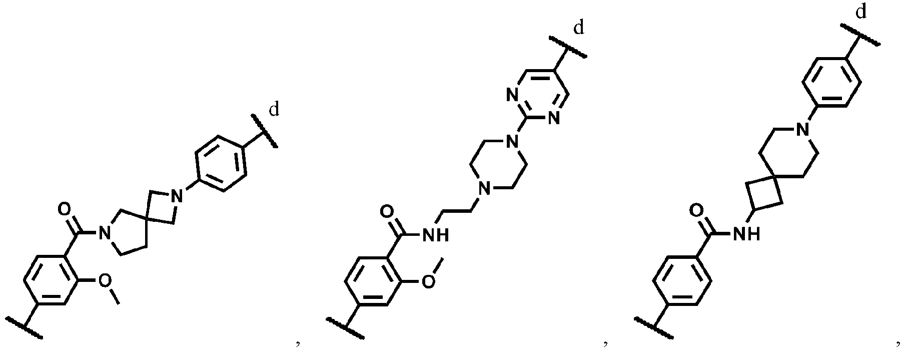 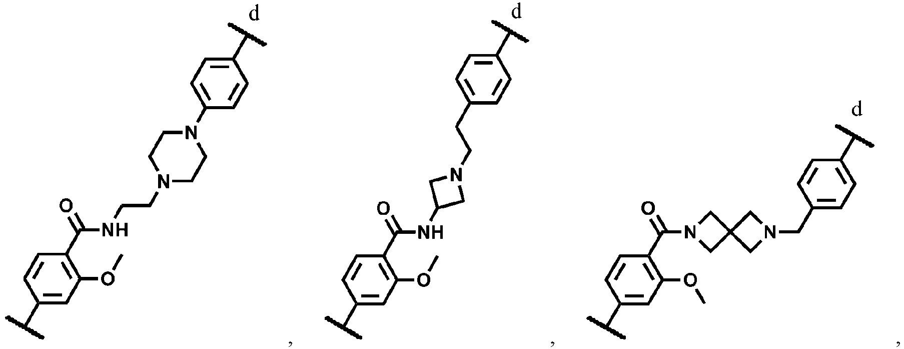 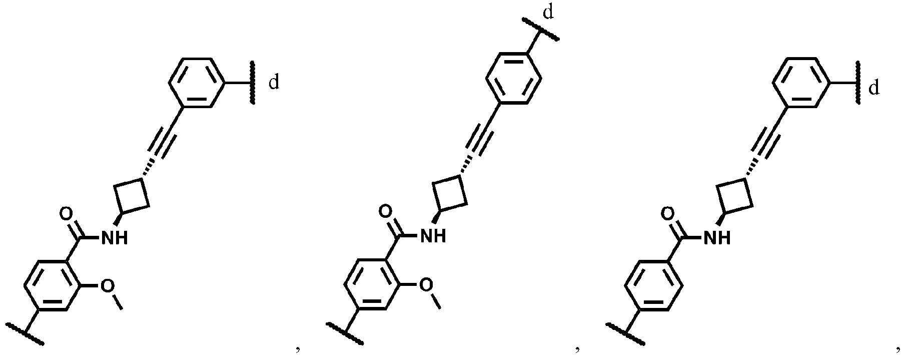 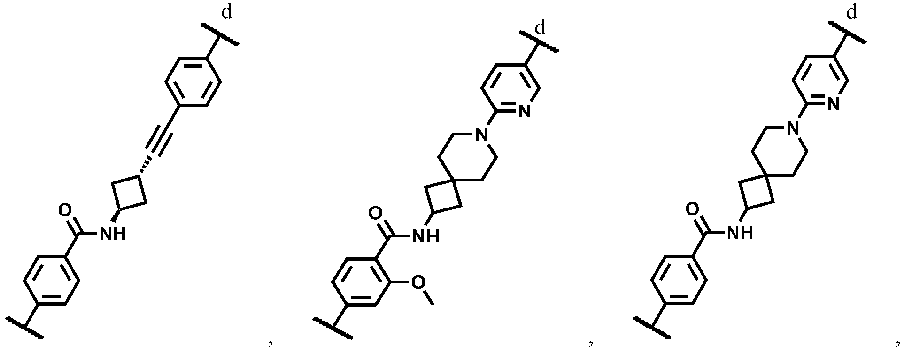 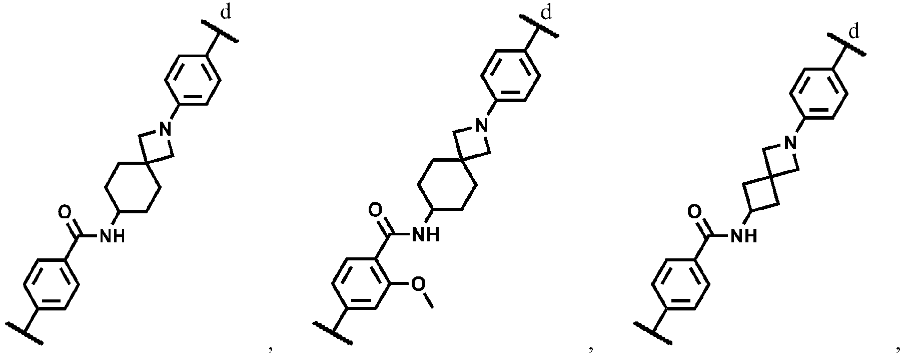 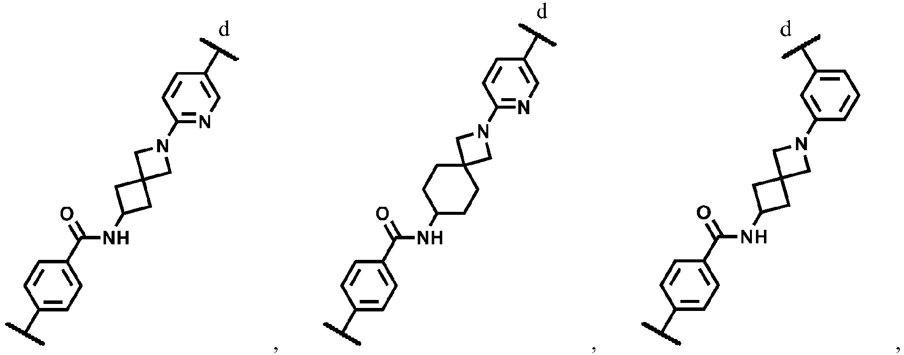 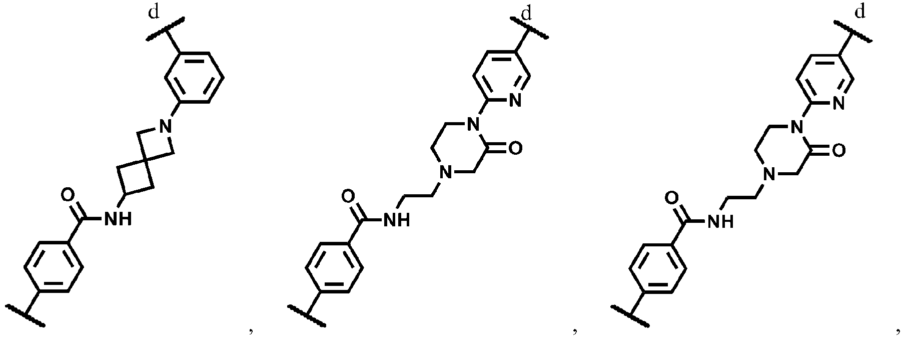 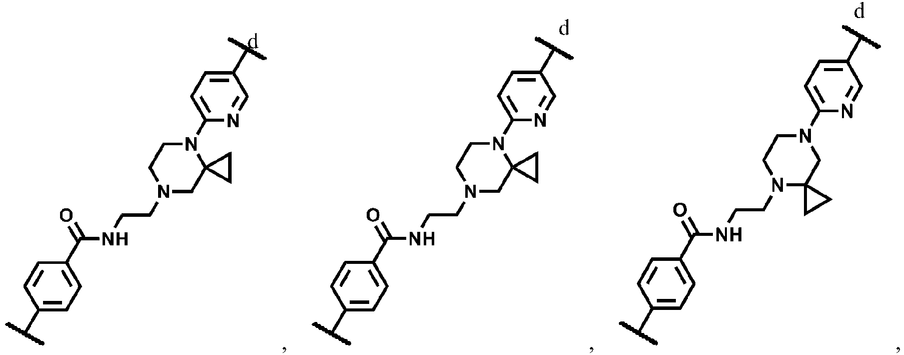 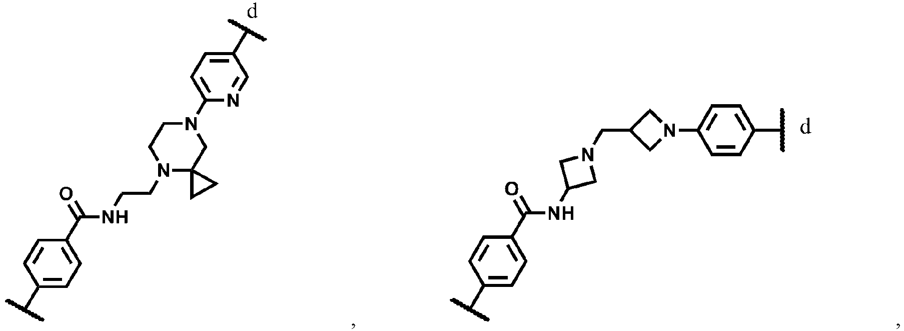 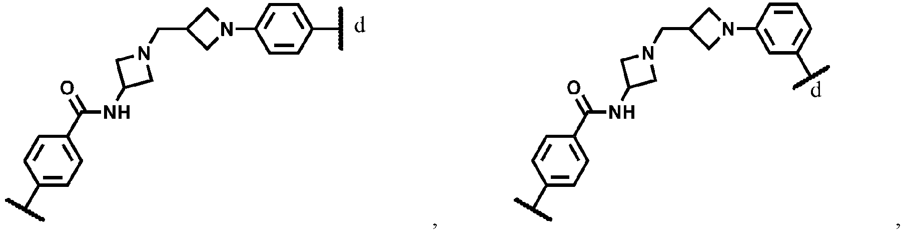 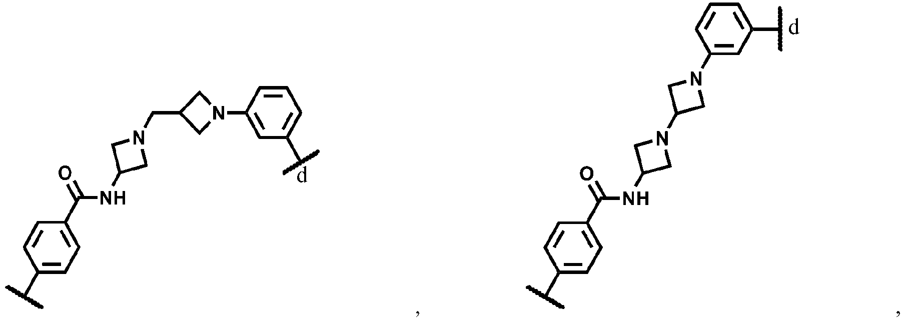 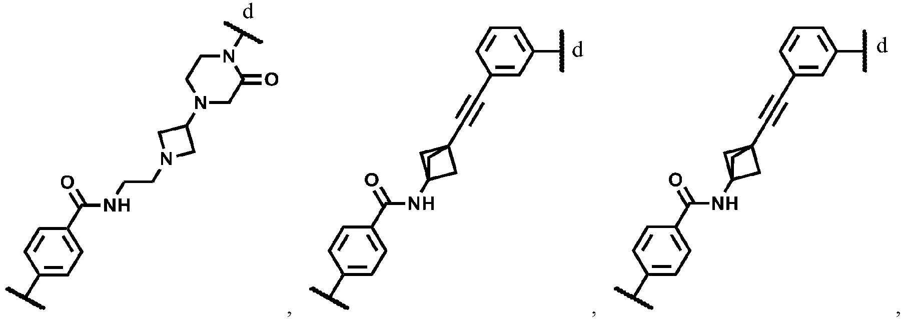 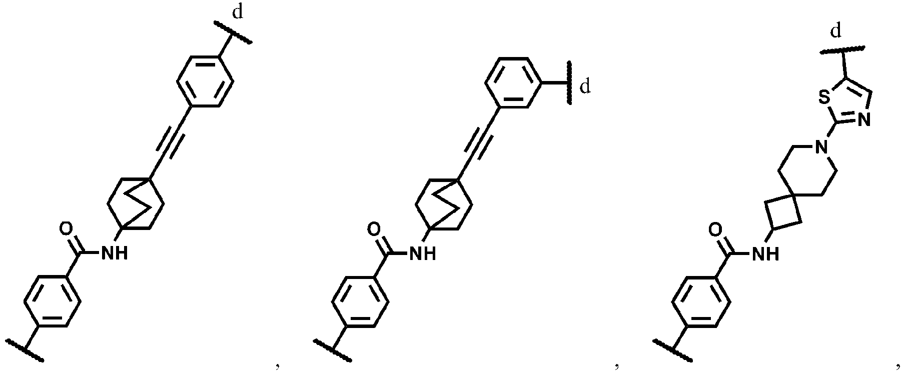 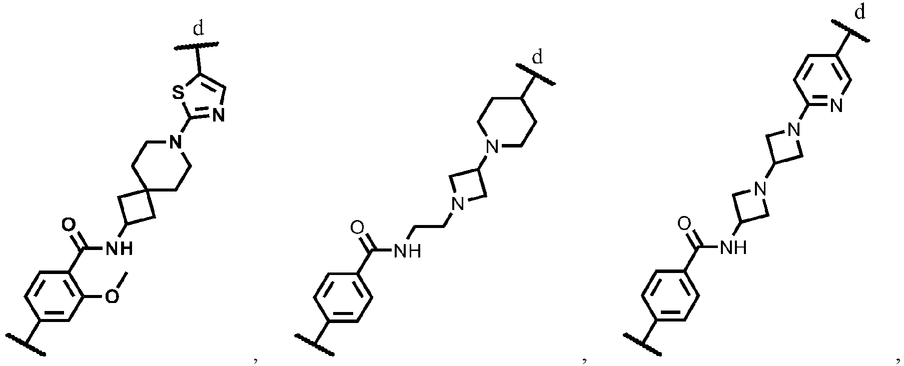 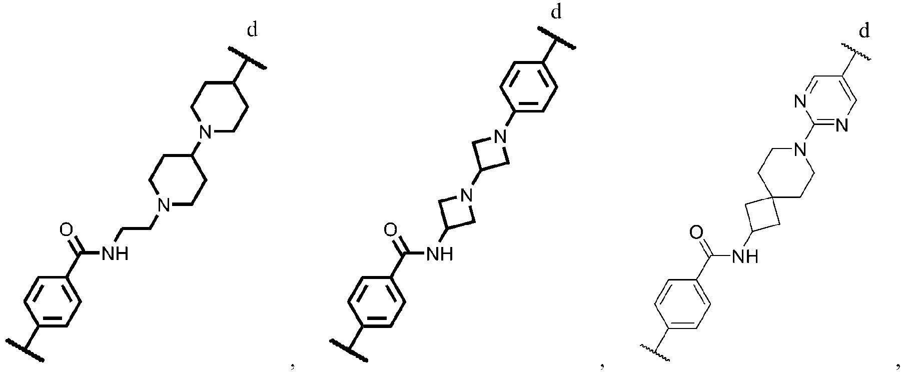 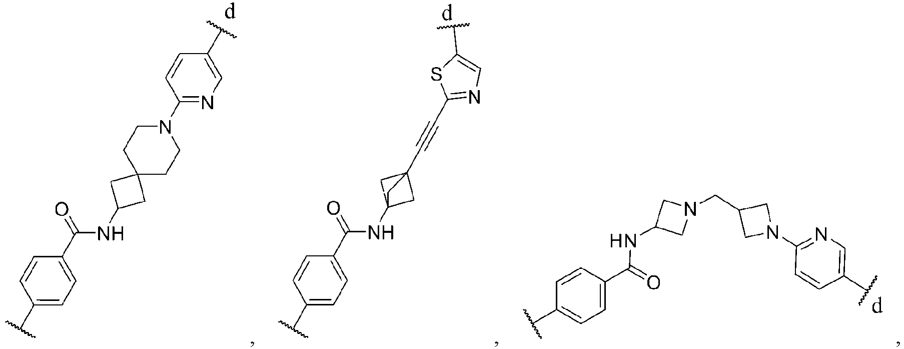 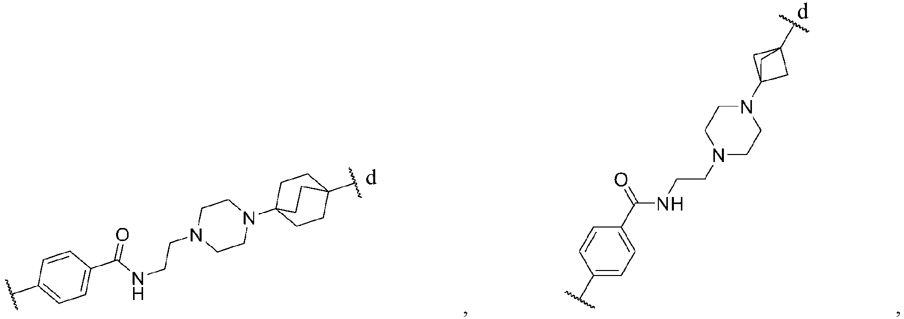 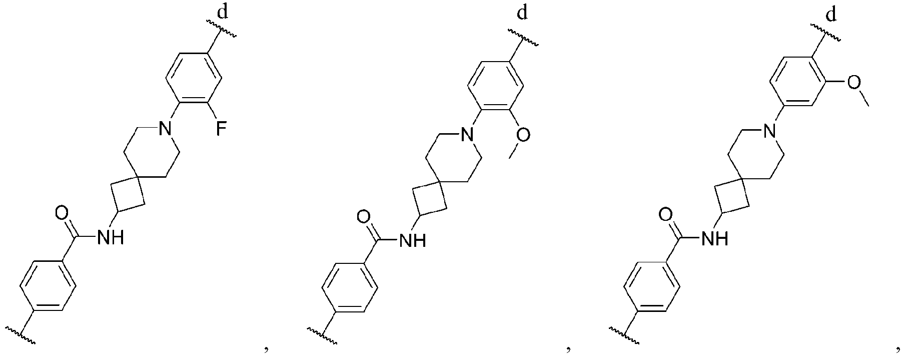 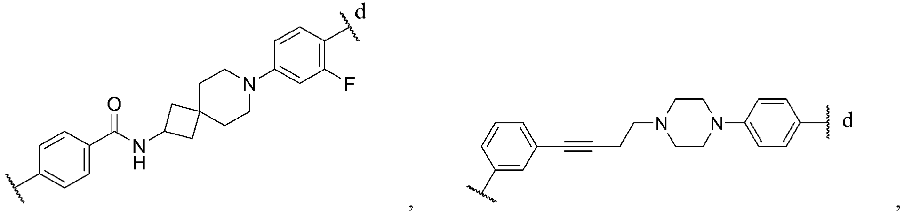 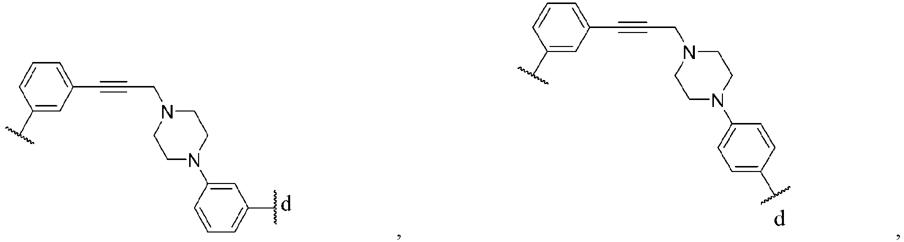 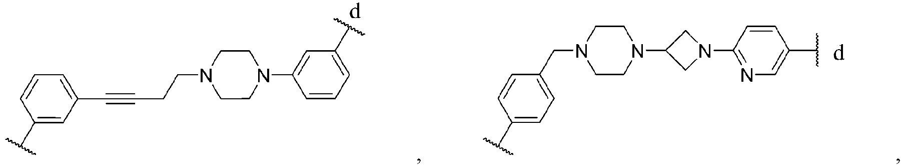 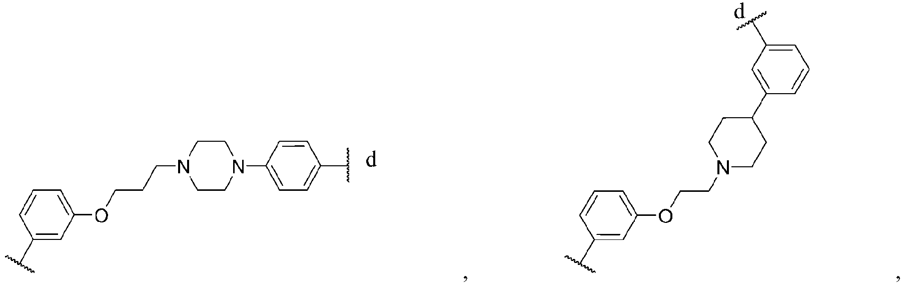 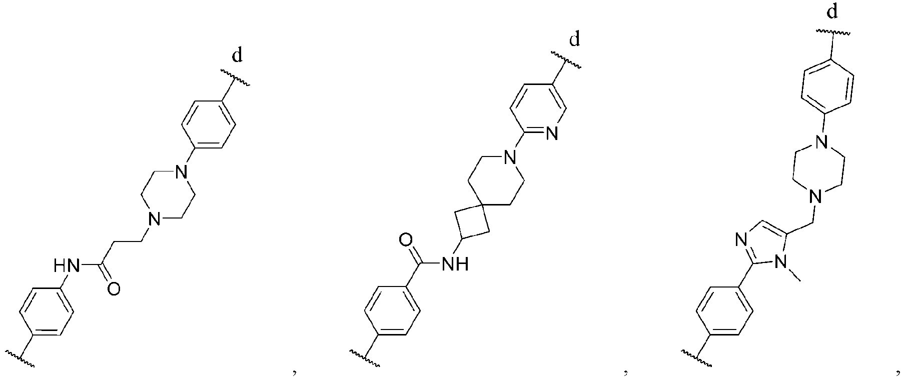 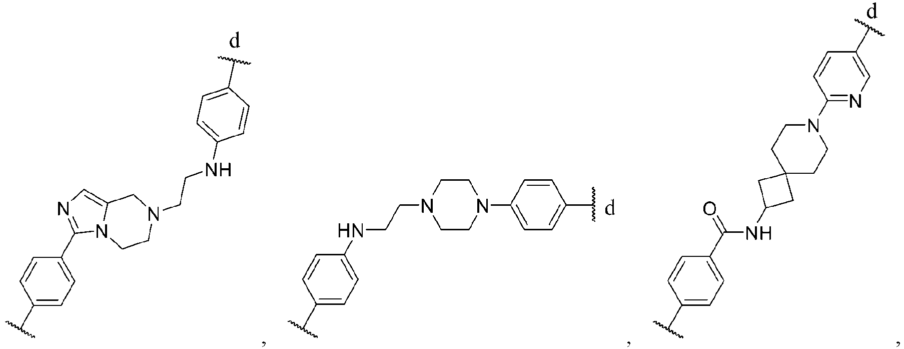 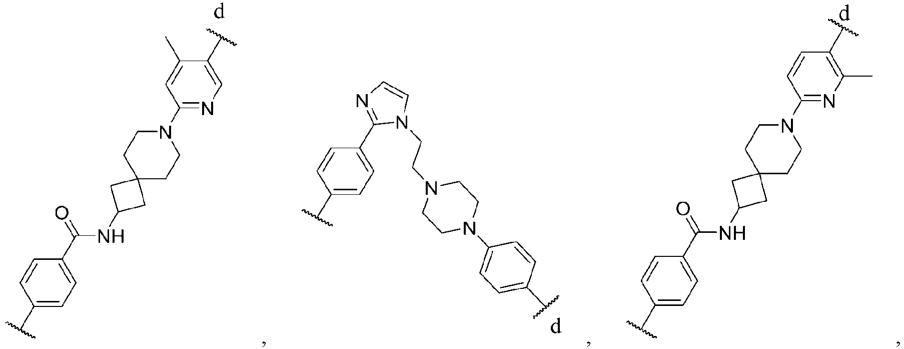 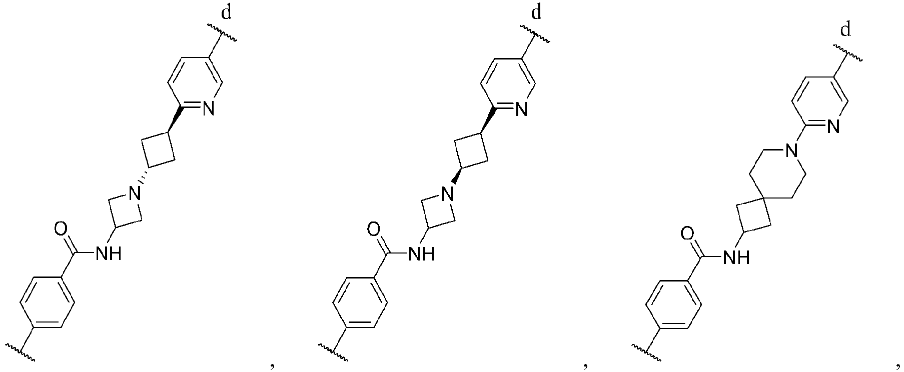 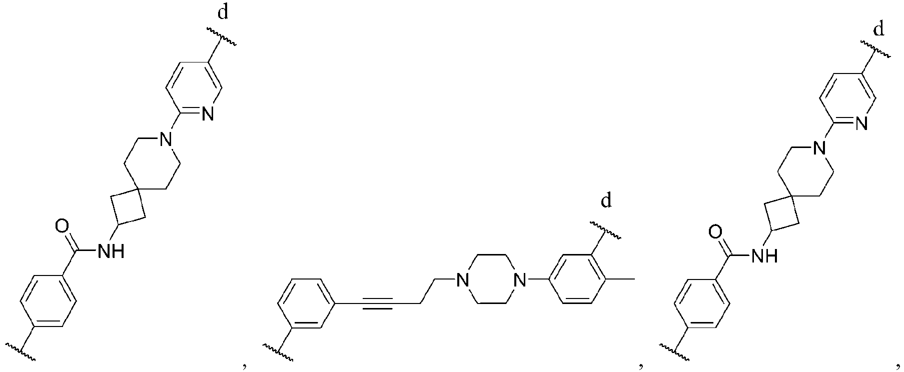 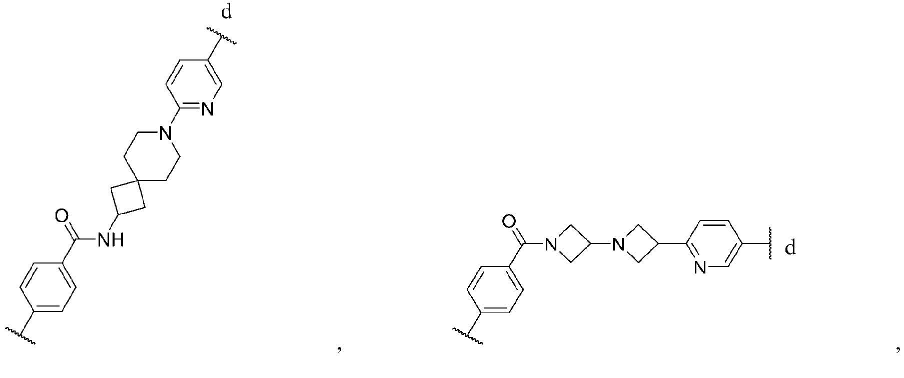 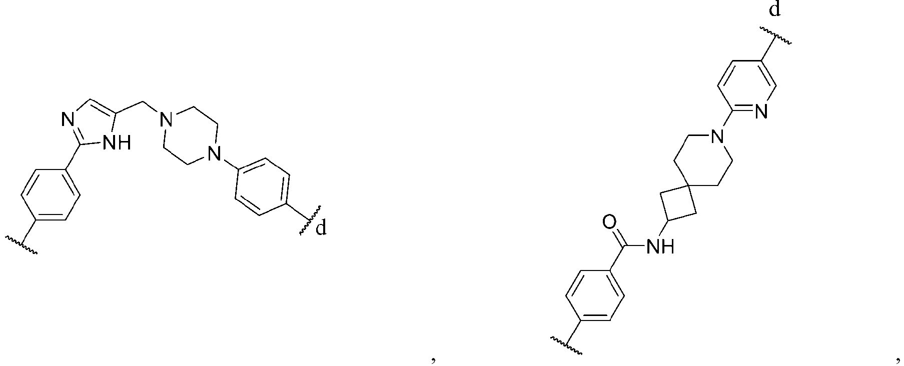 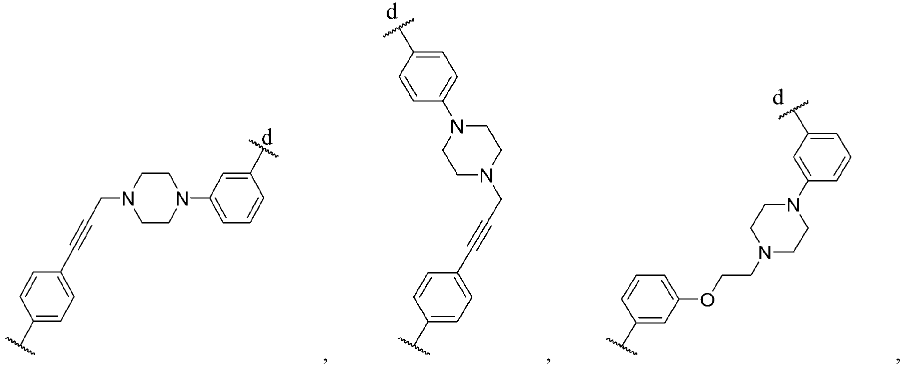 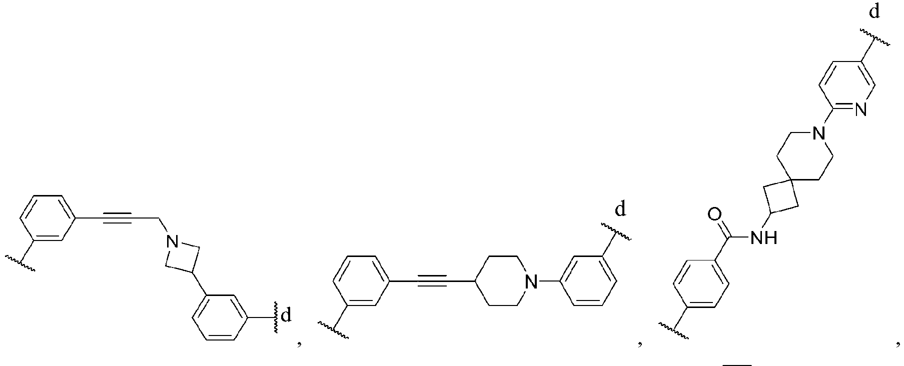 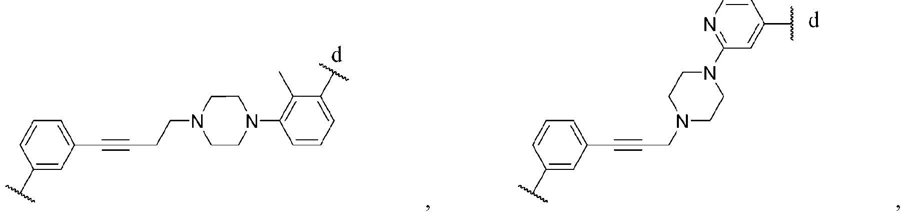 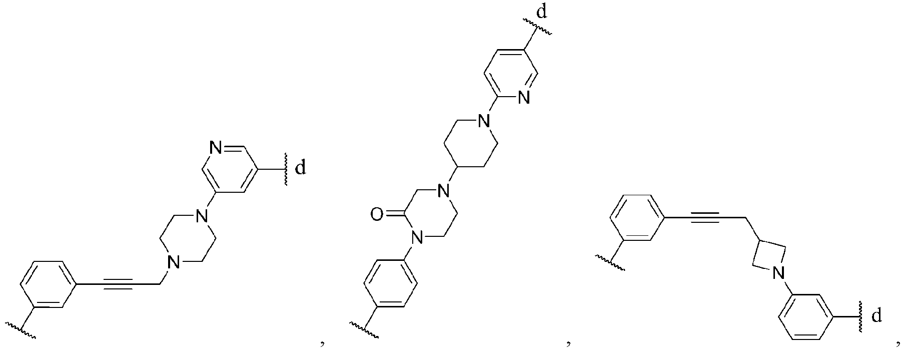 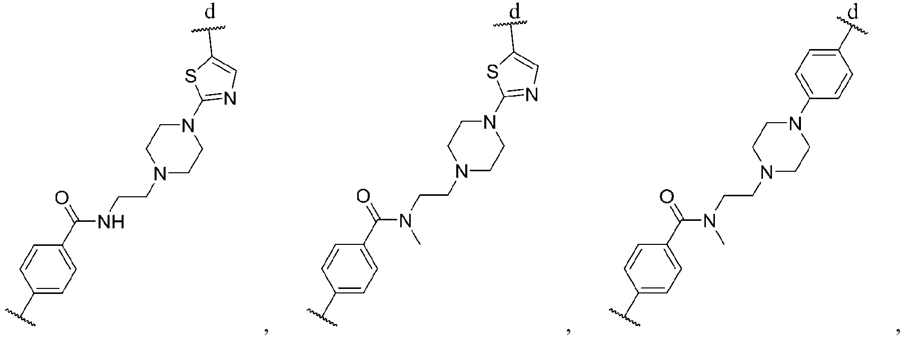 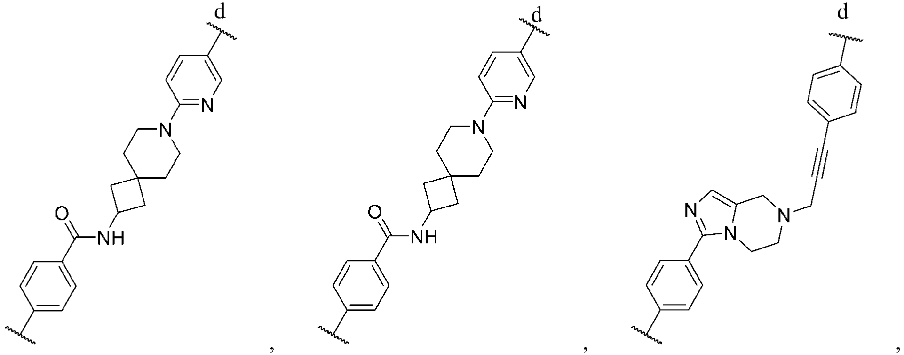 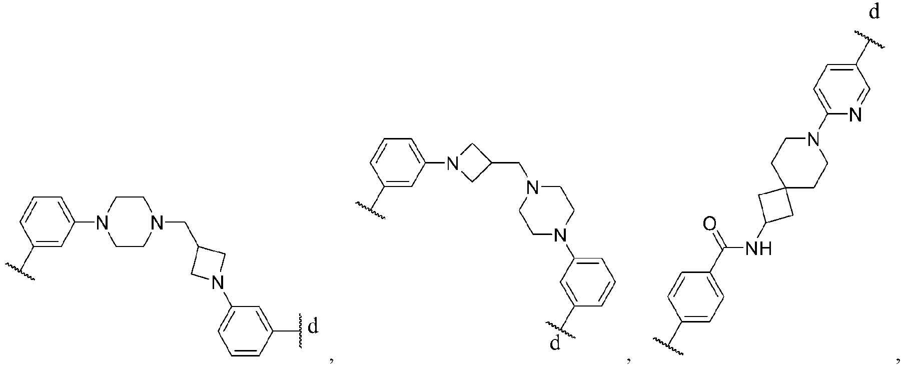 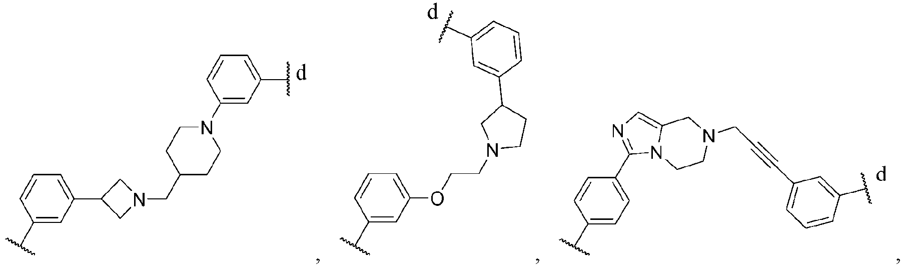 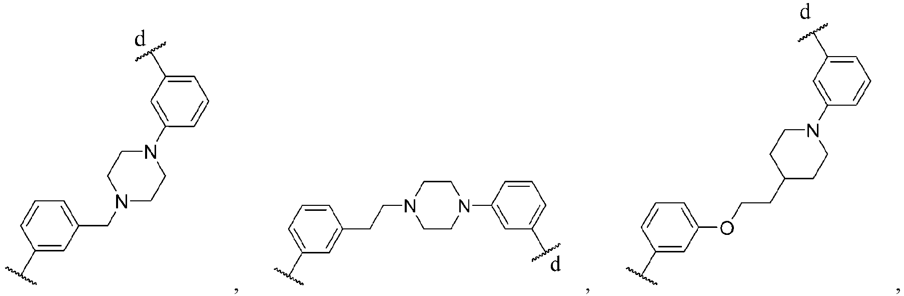 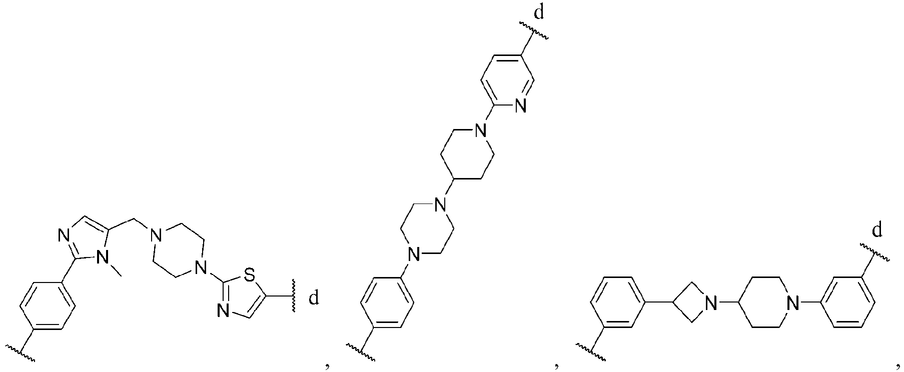 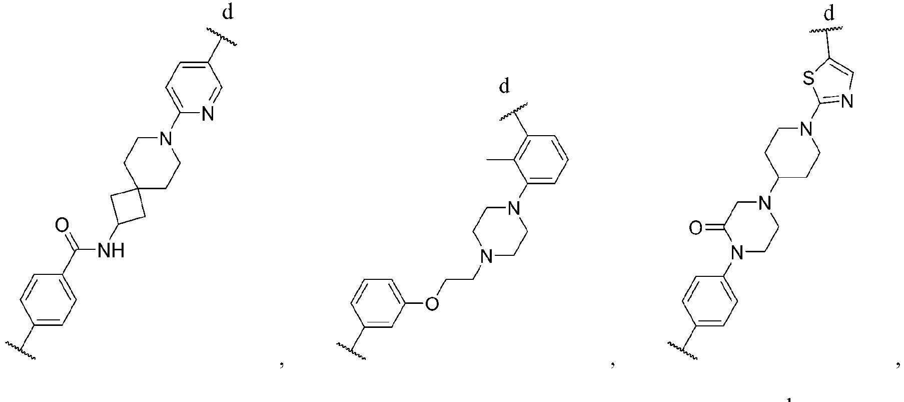 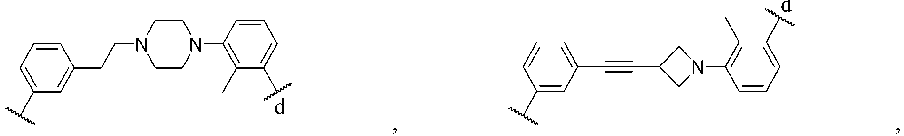 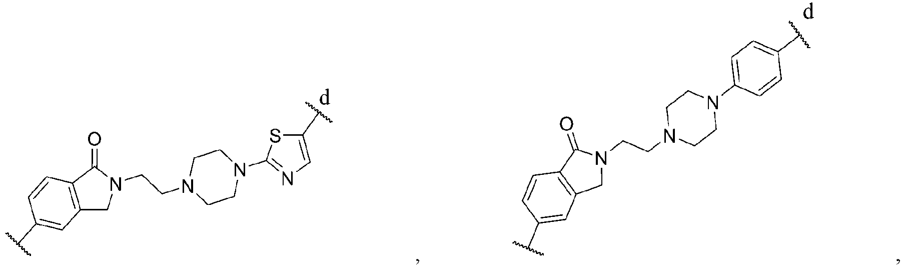 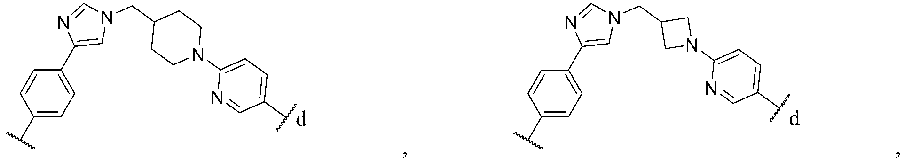 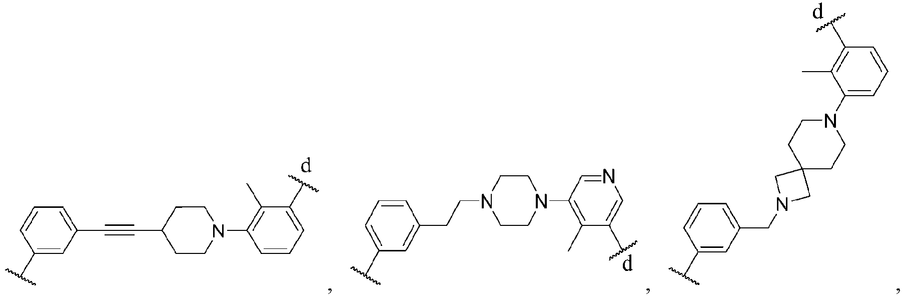 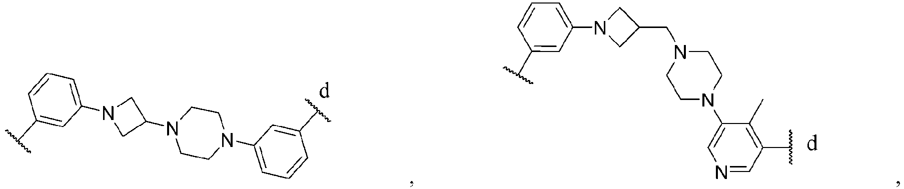 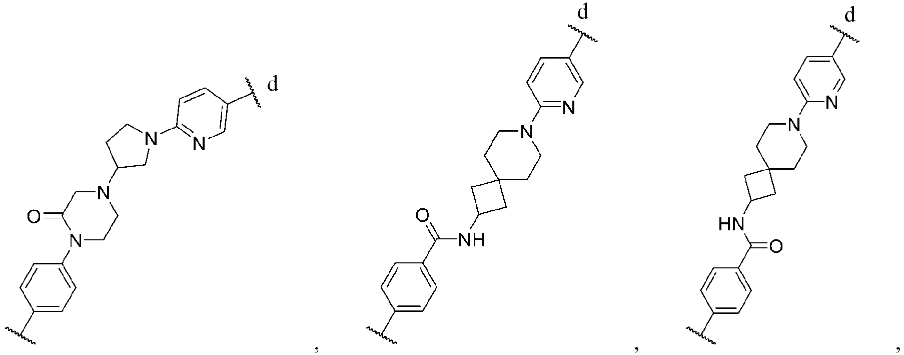 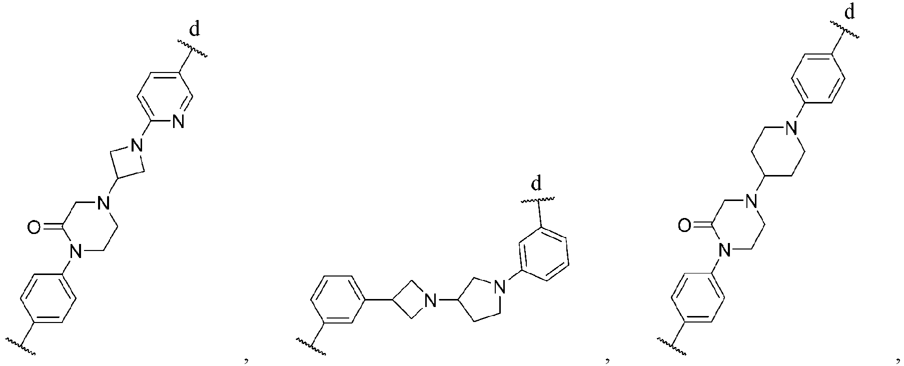 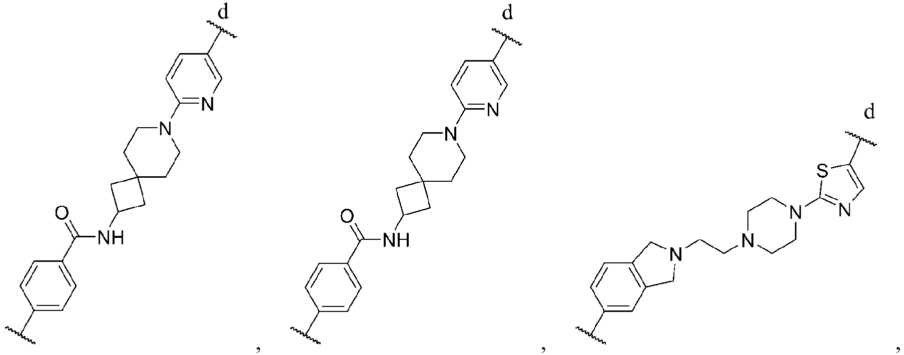 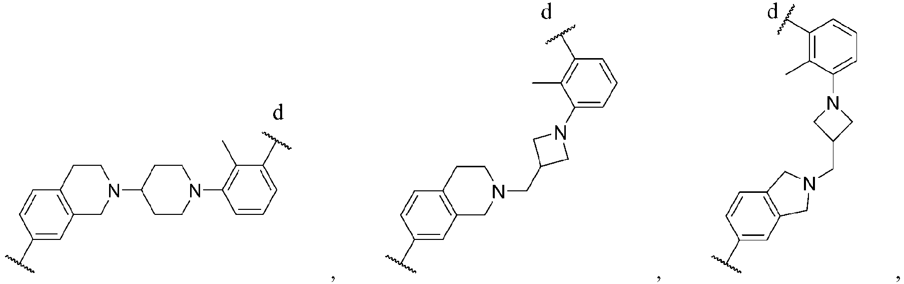 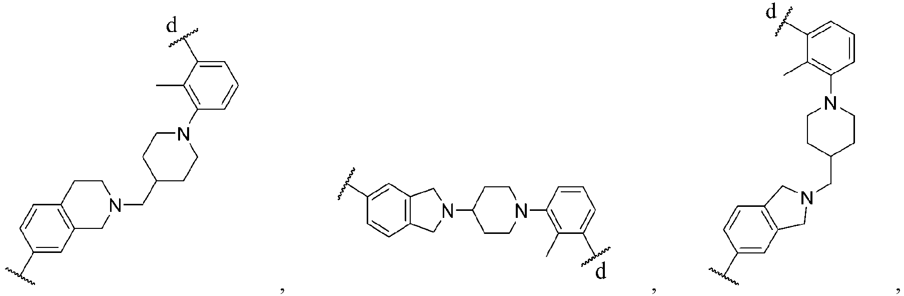 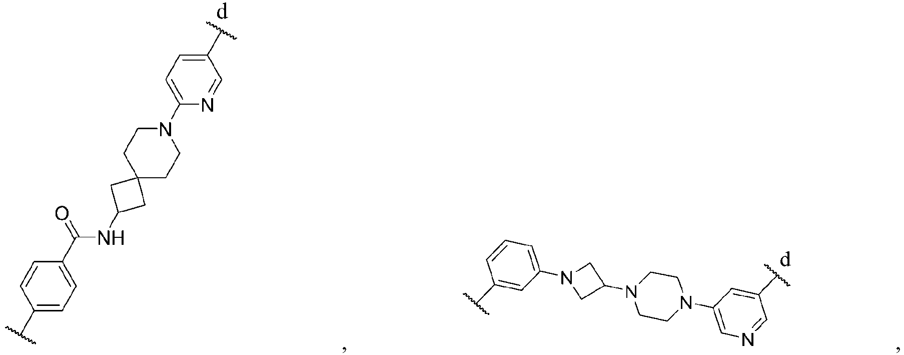 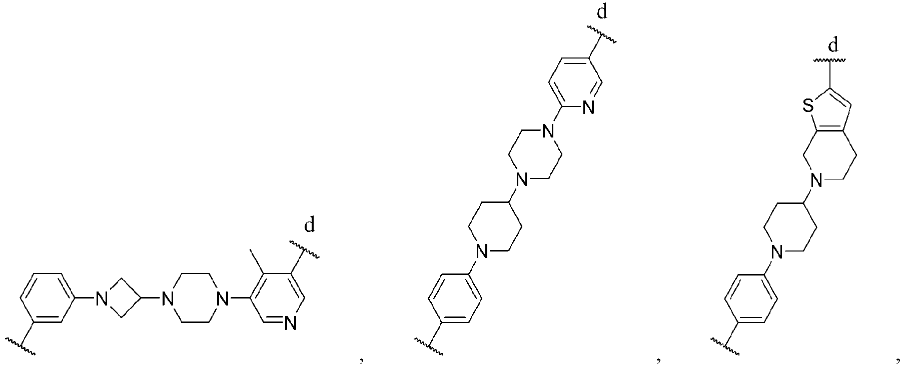 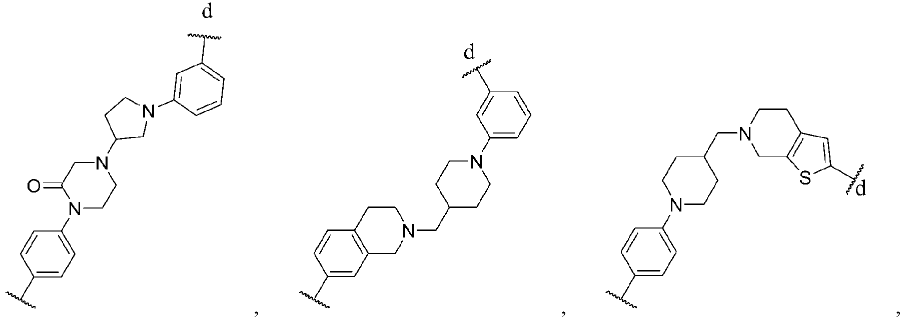 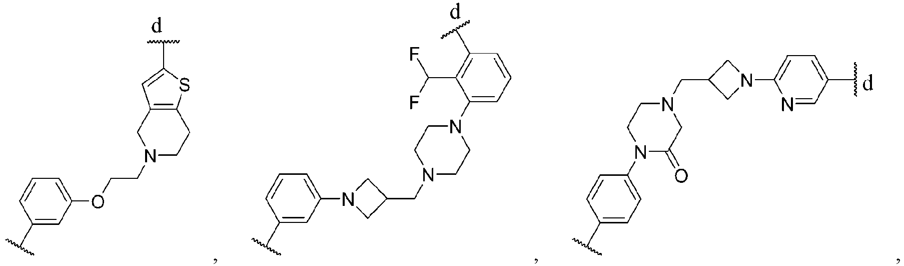 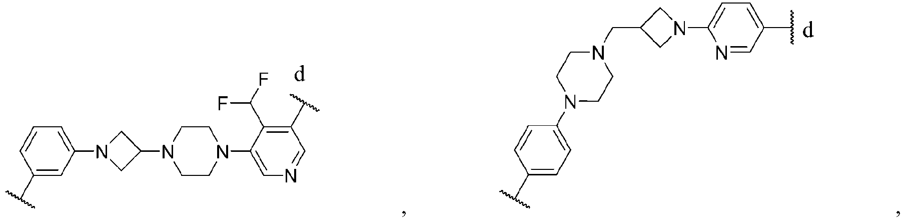 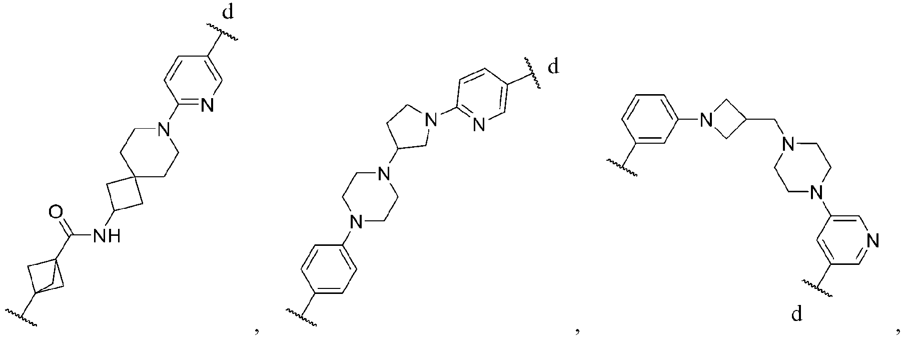 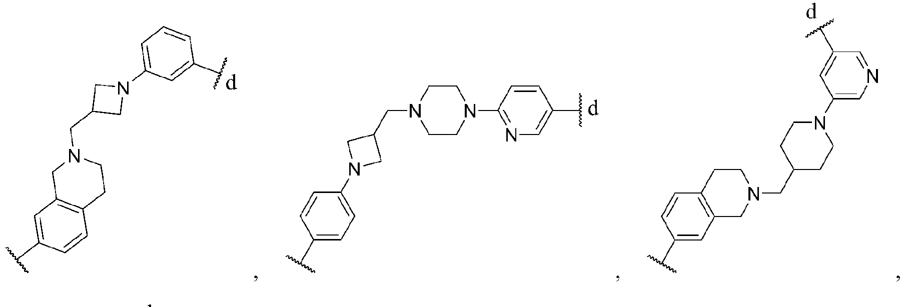 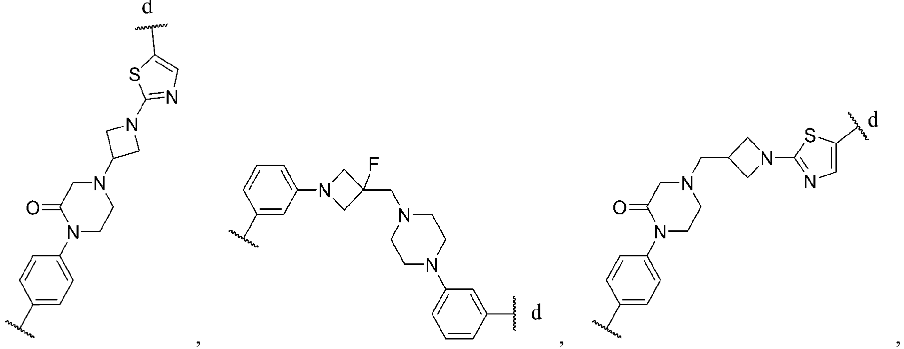 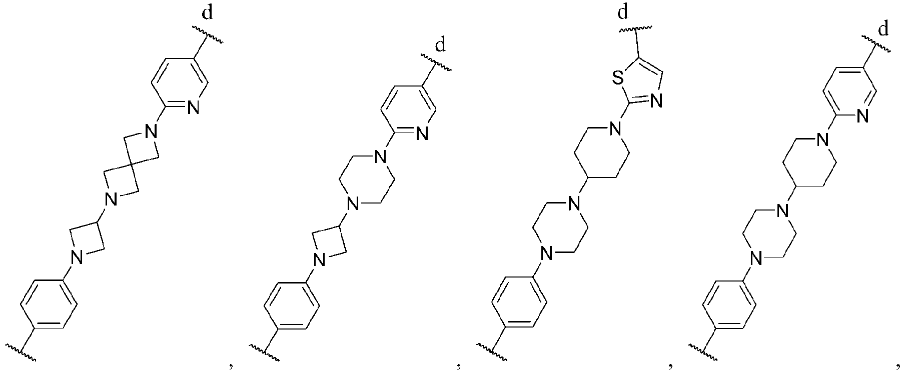 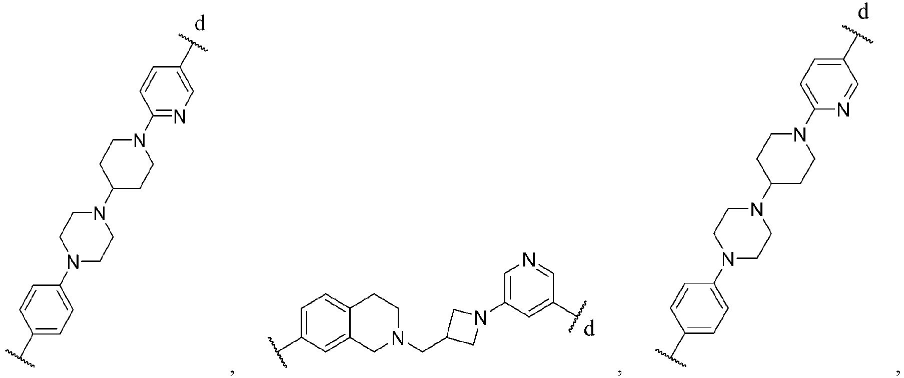 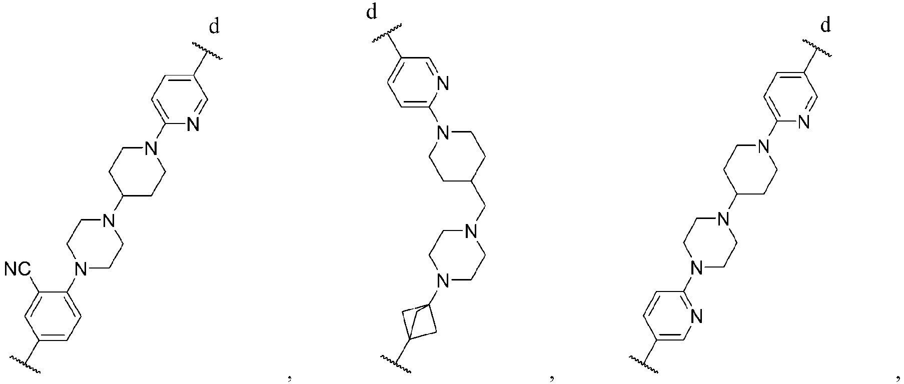 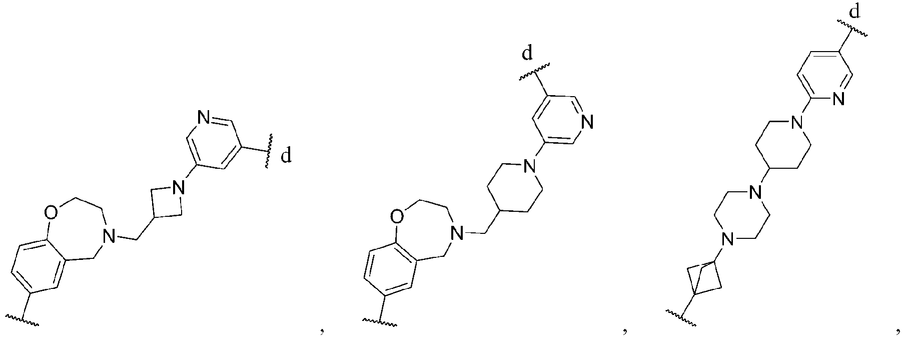 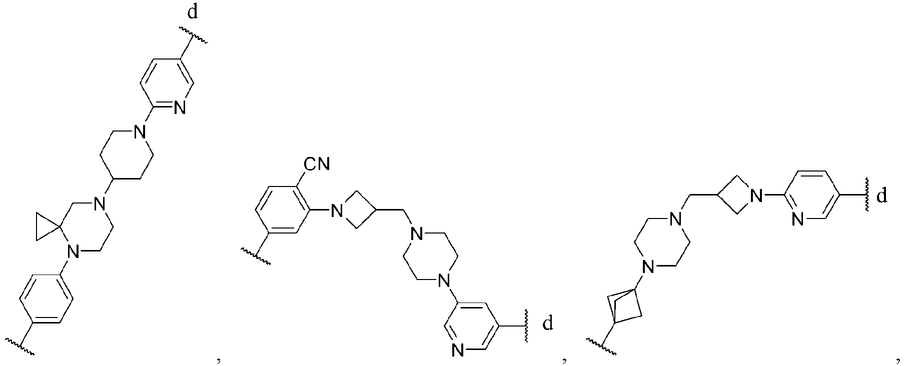 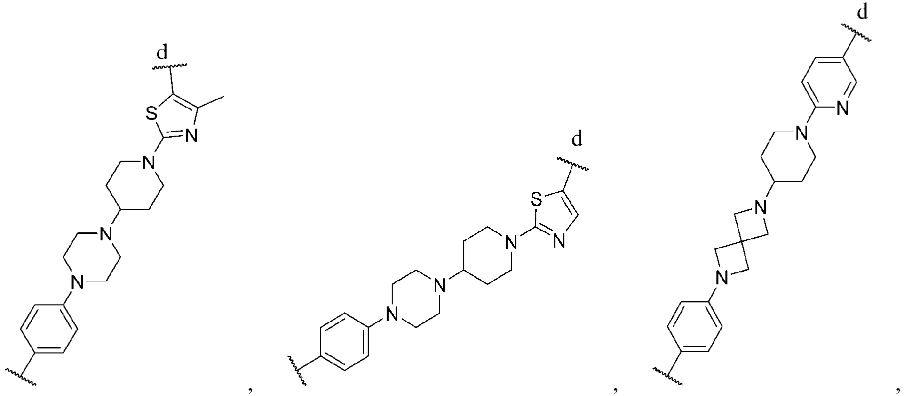 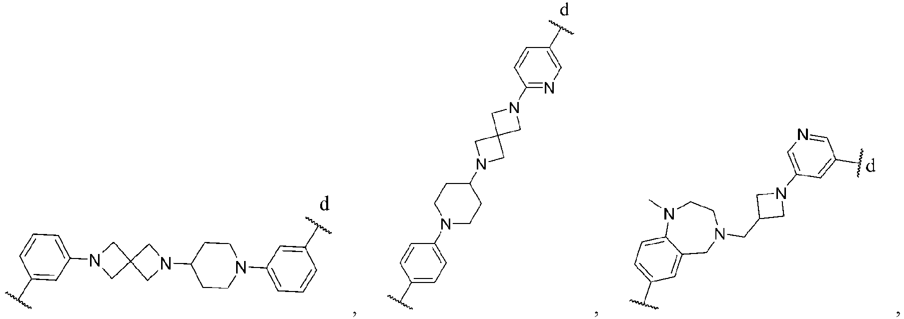 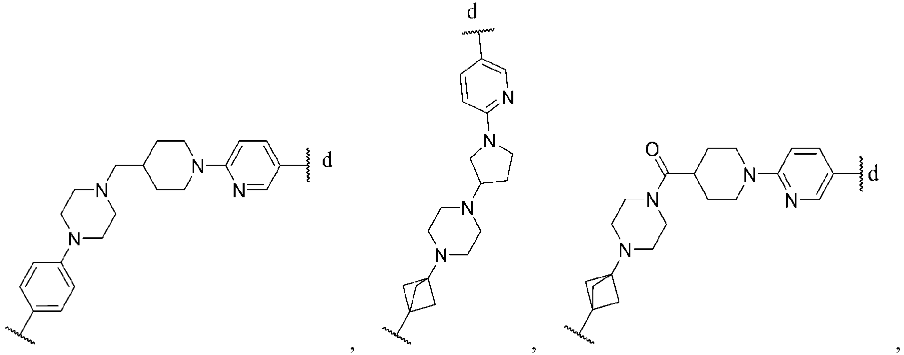 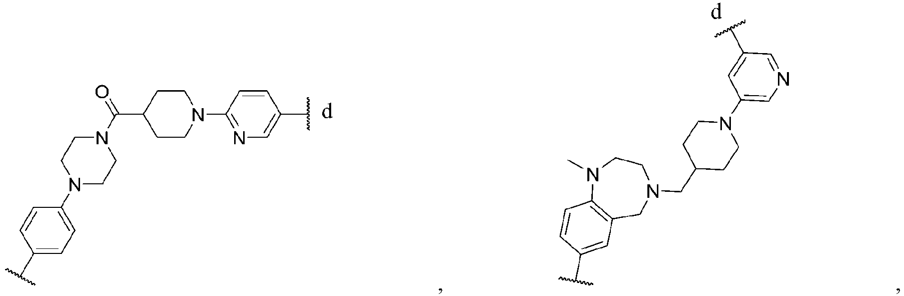 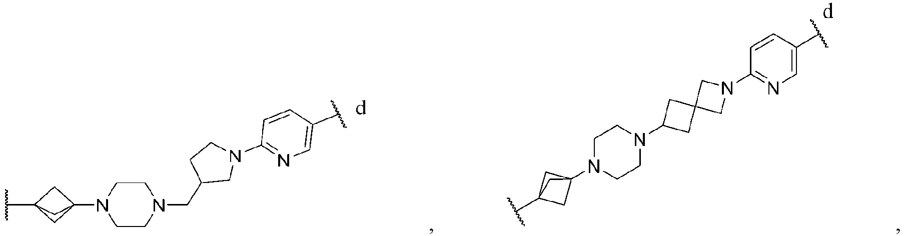 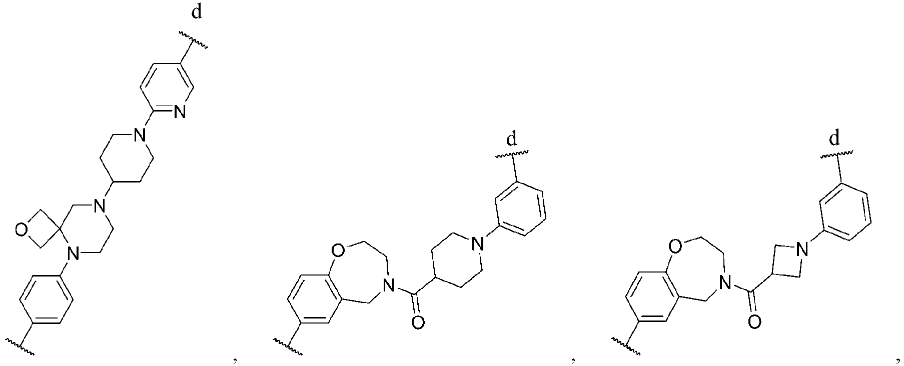 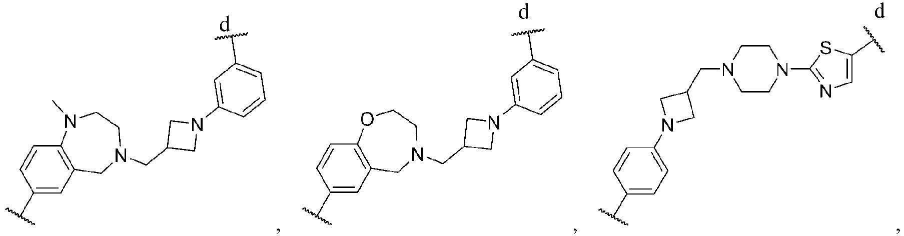 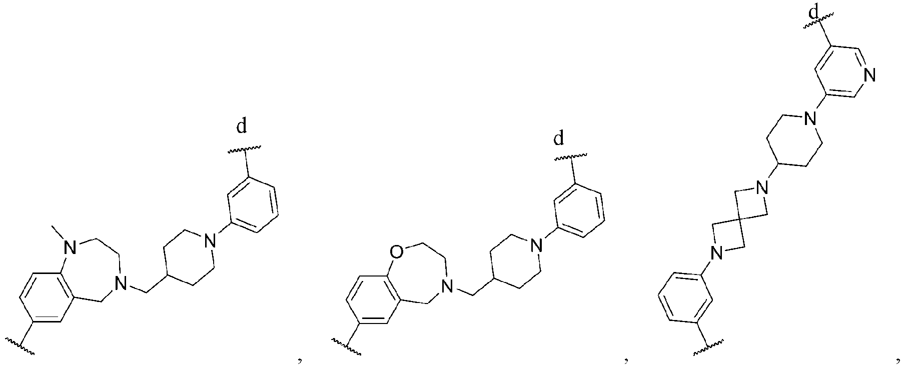 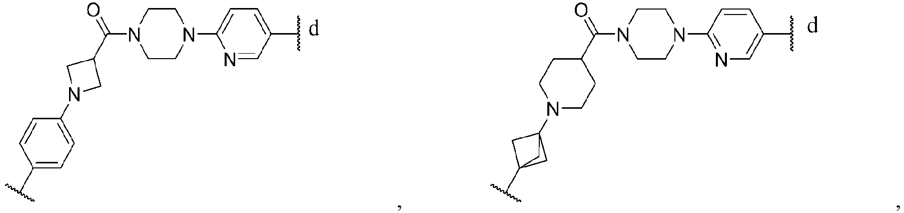 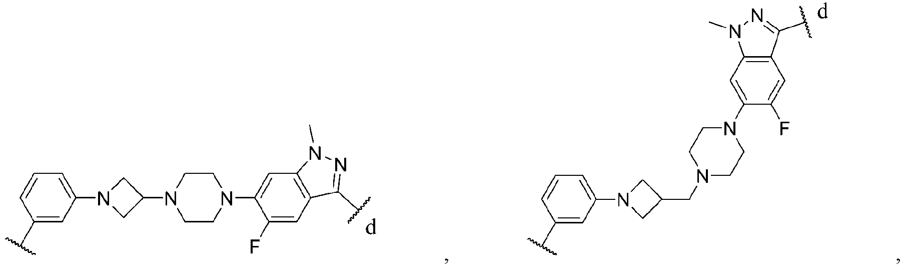 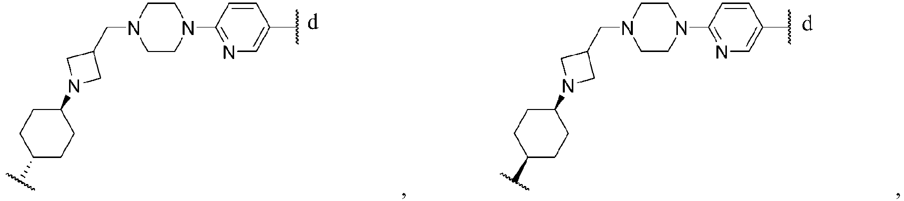 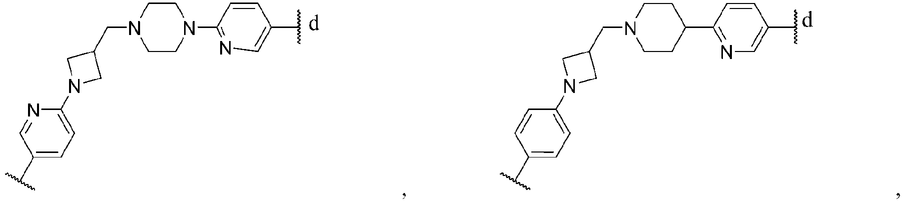 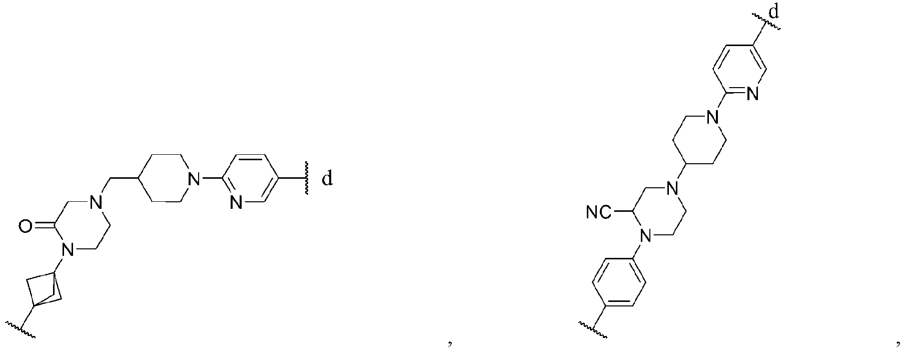 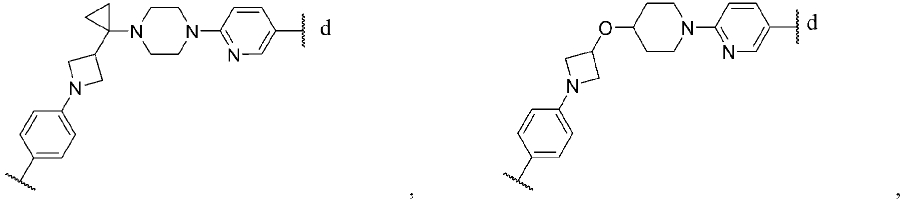 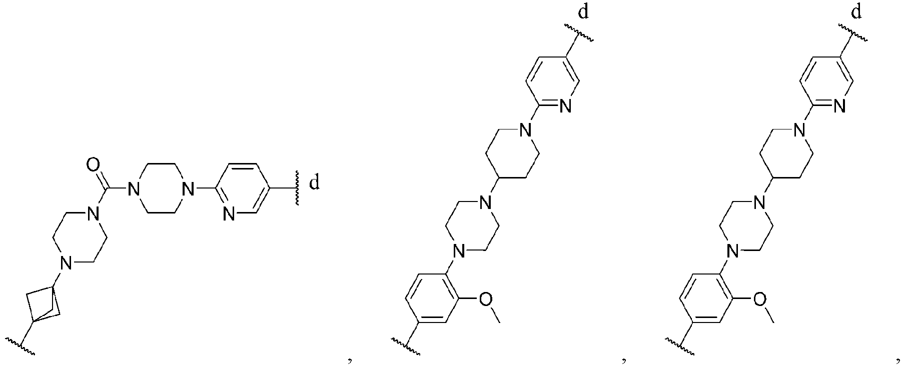 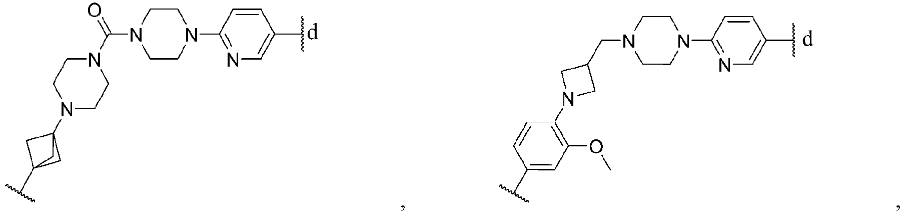 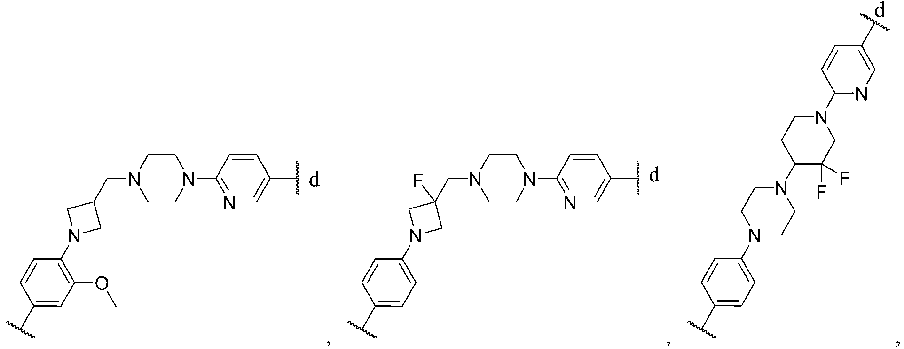 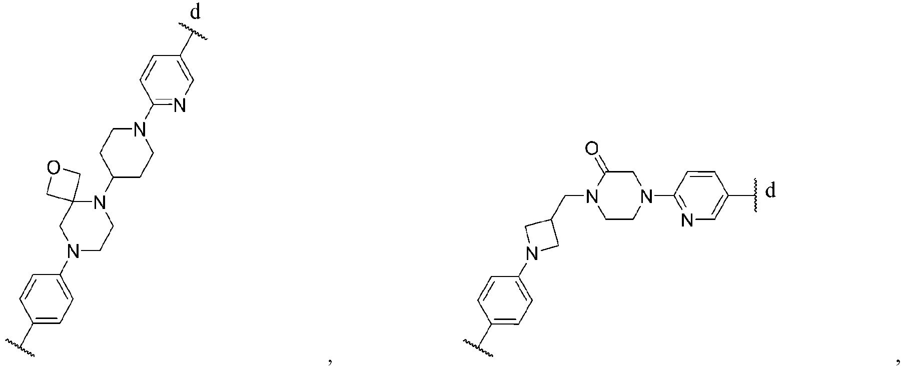 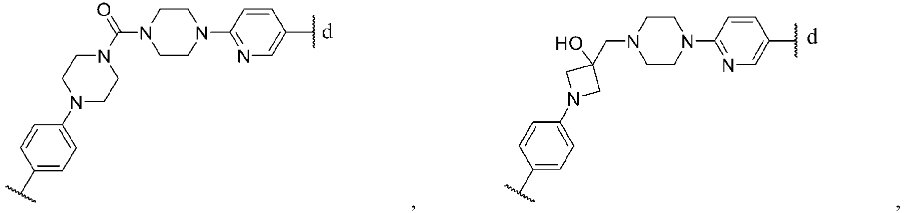 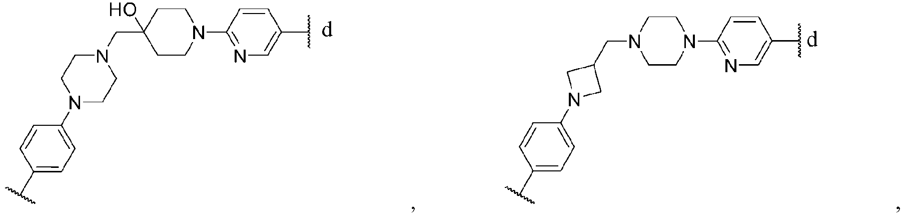 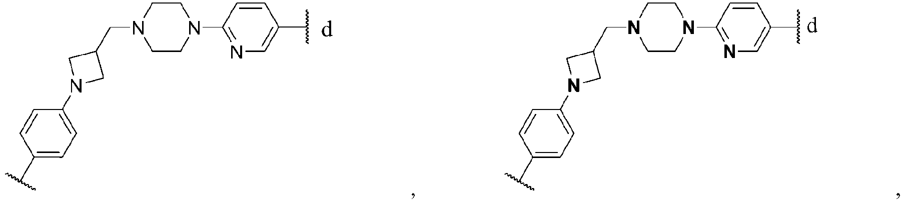 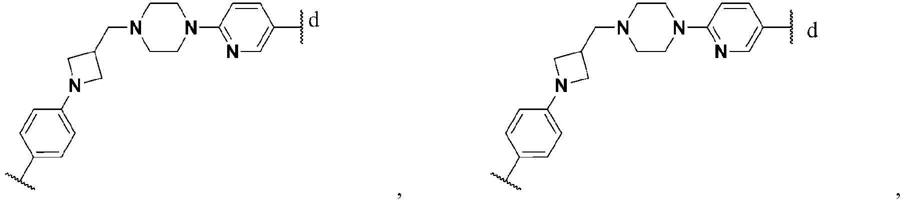 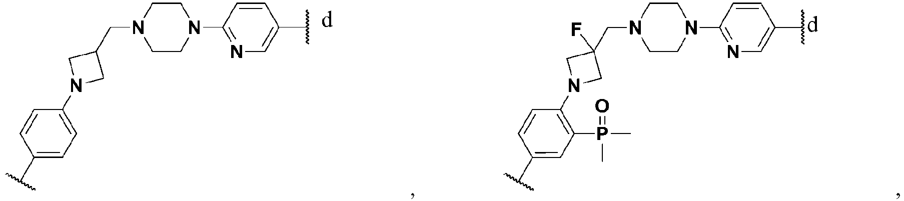 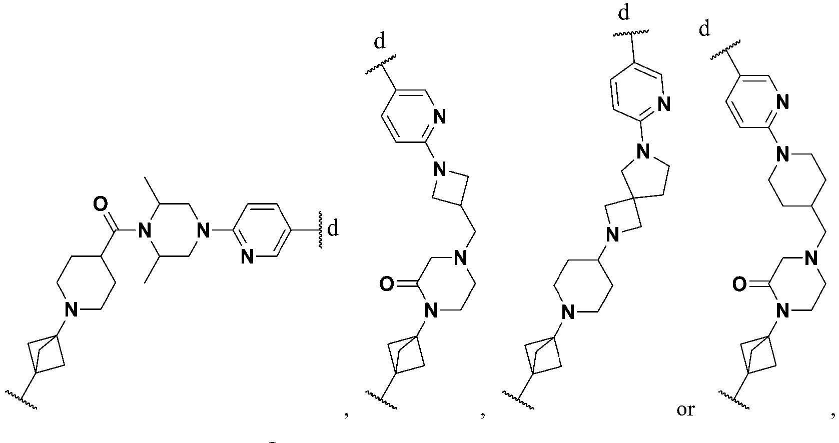 wherein side d is connected to 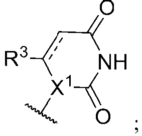 preferably, in Scheme ii, -A-L-B- is any one of the above groups except 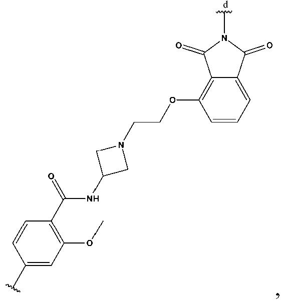 and in Scheme i, -A-L-B- is 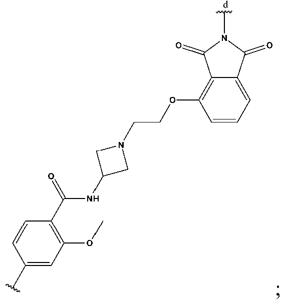
(2) 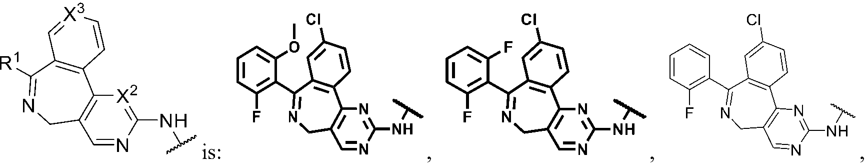 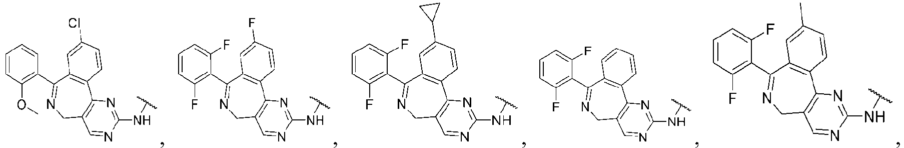 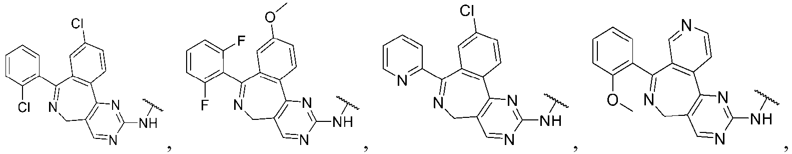 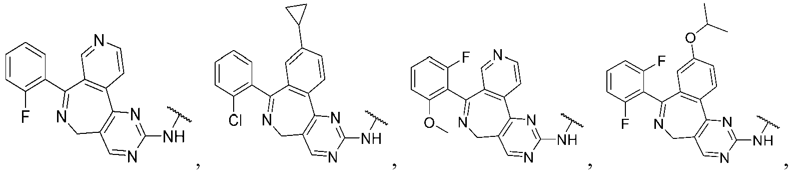 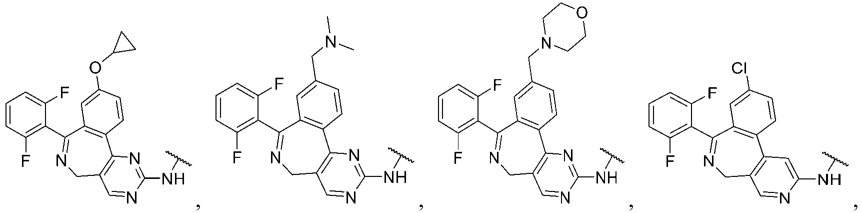 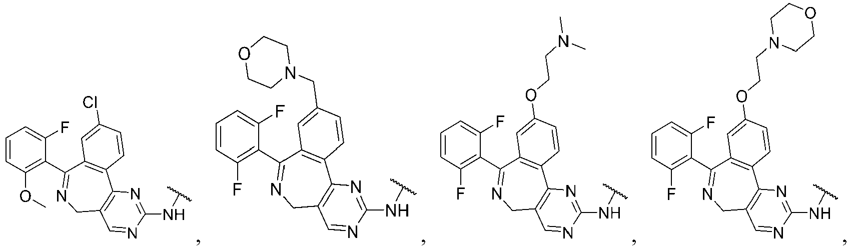 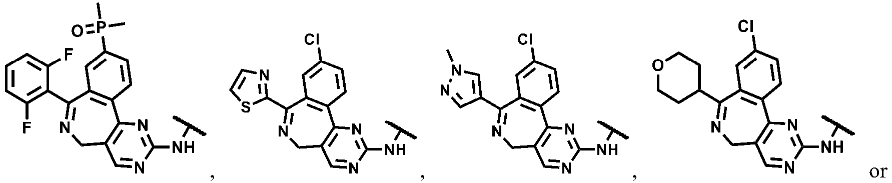 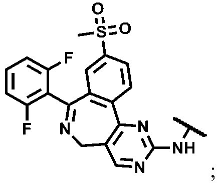
(3)

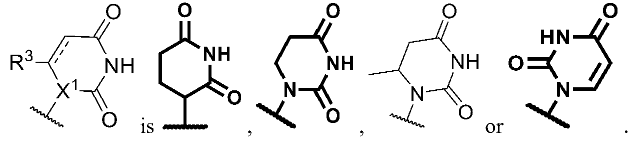

16. The compound represented by formula (I), the pharmaceutically acceptable salt thereof, the solvate thereof or the solvate of the pharmaceutically acceptable salt thereof according to claim 1, wherein the compound represented by formula (I) is any one of the following compounds:

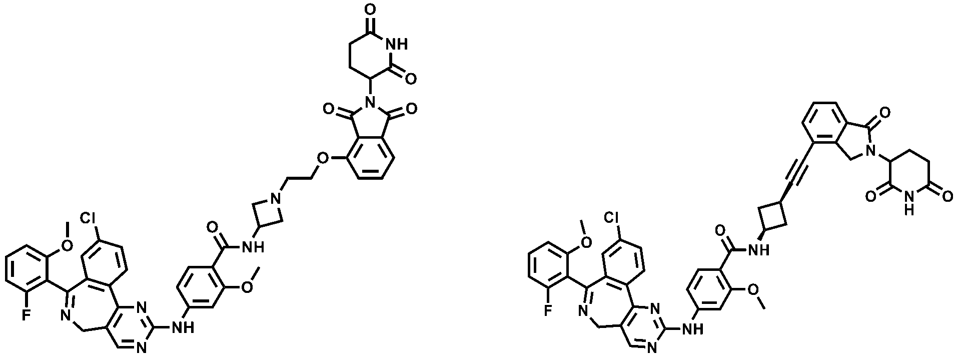

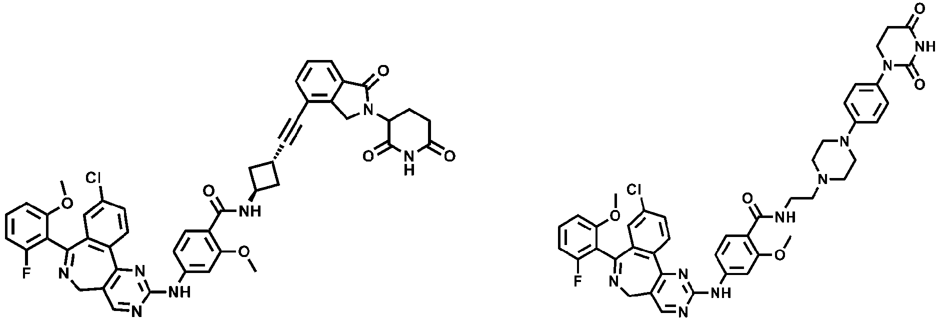

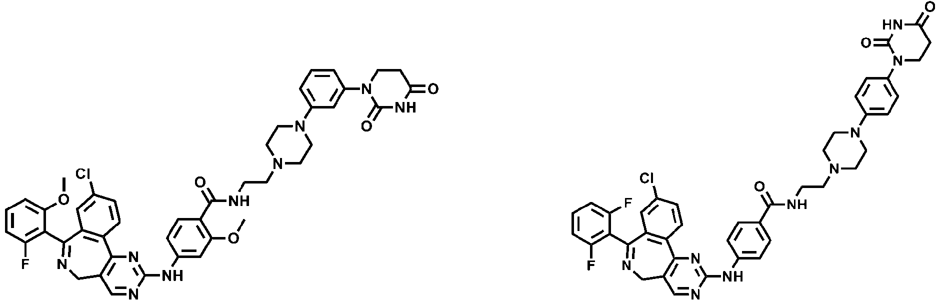

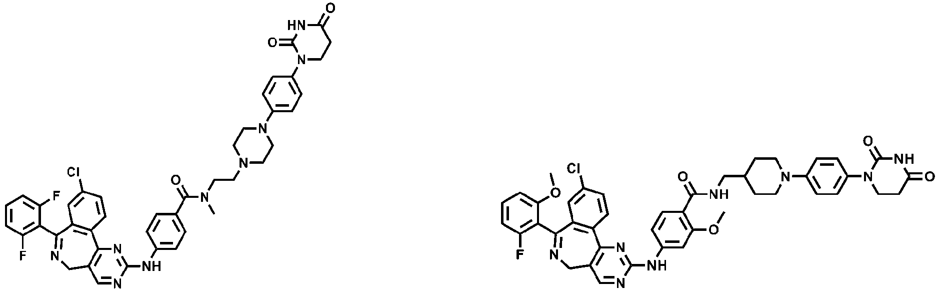

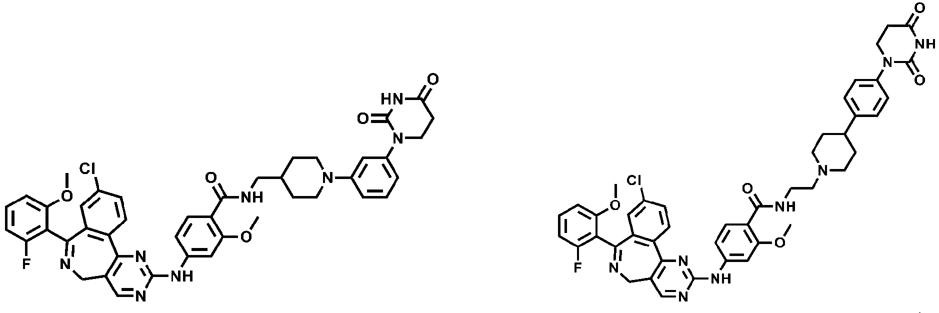

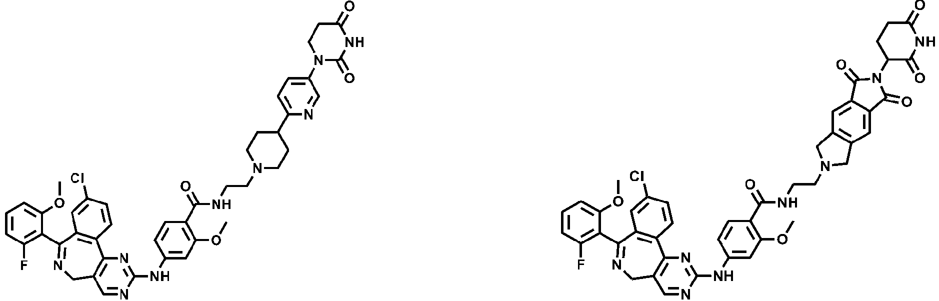

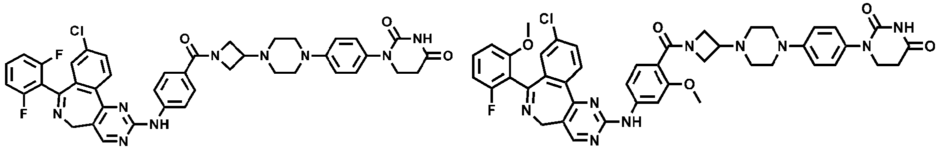

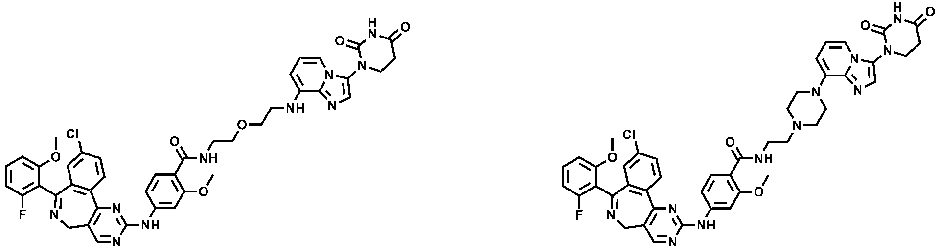

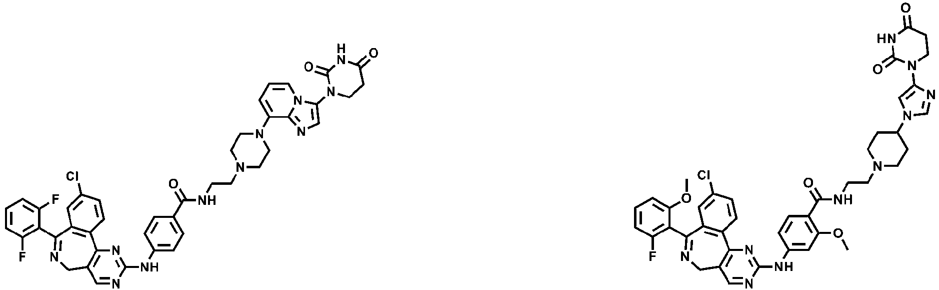

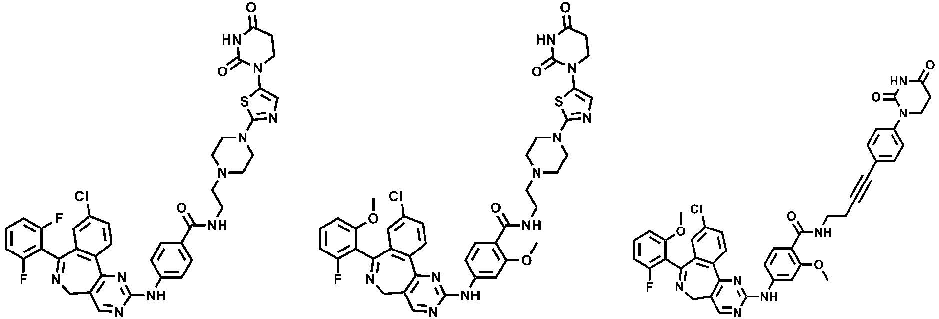

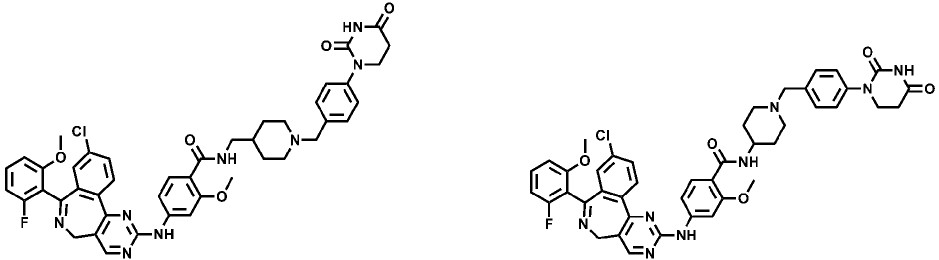

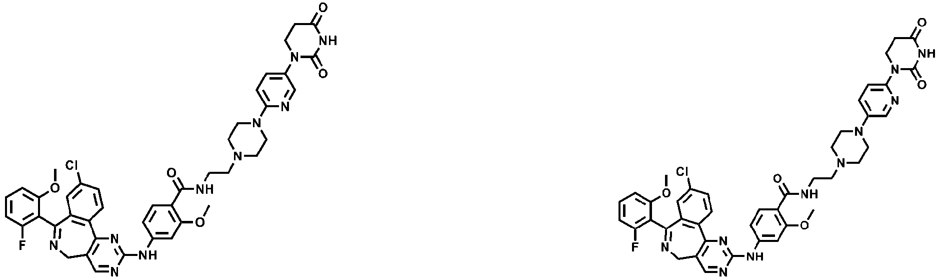

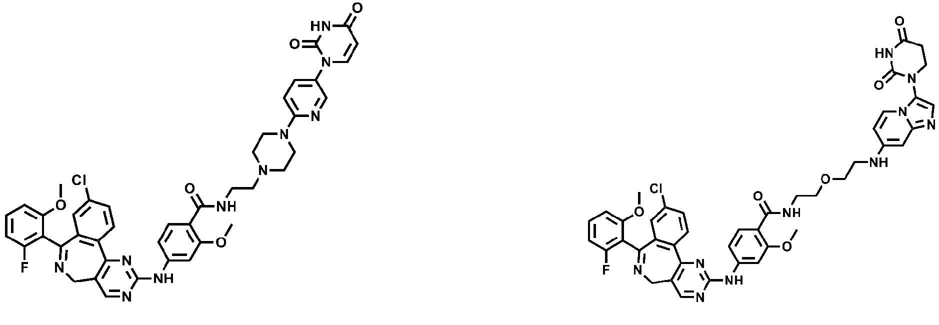

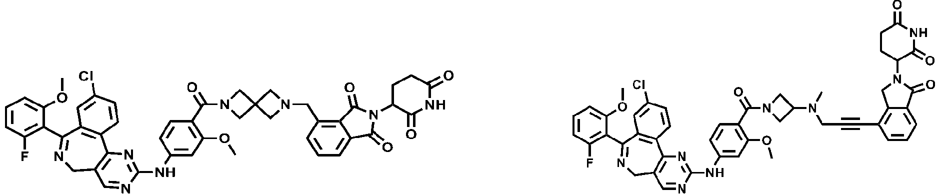

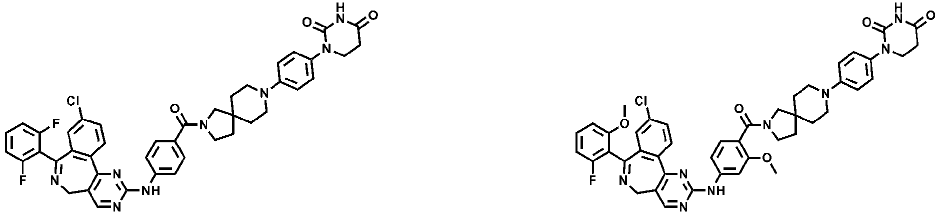

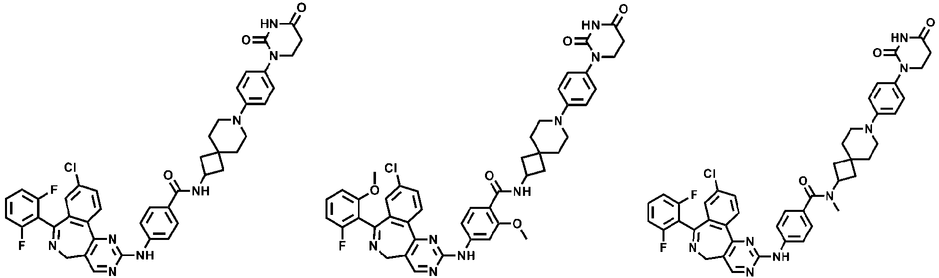

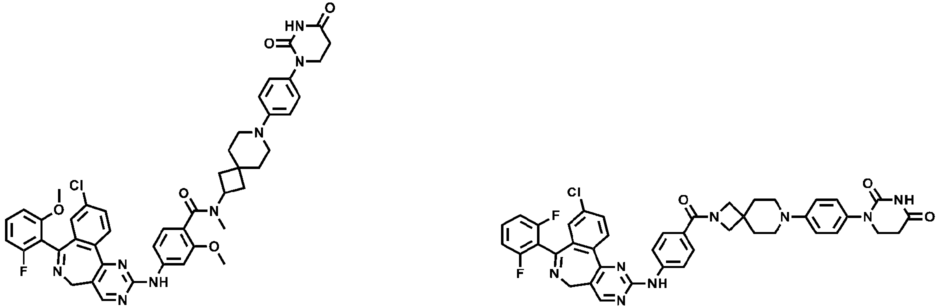

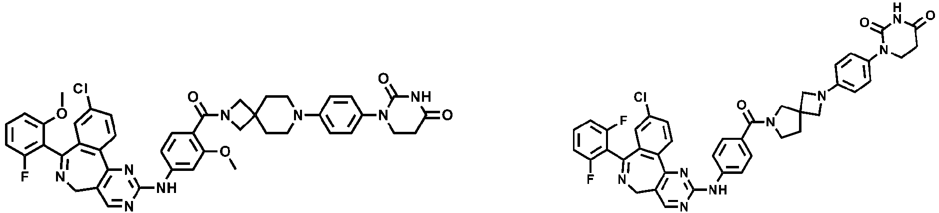

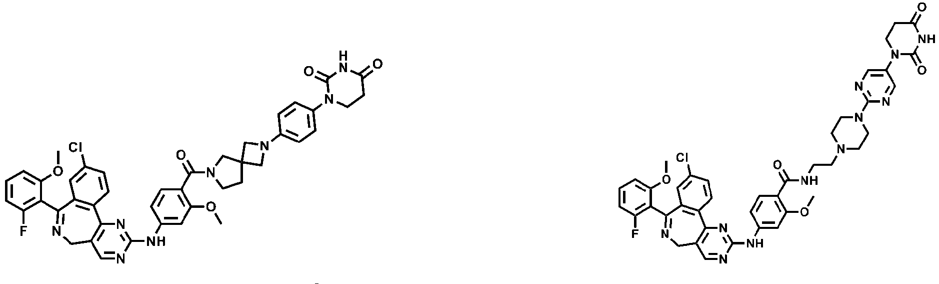

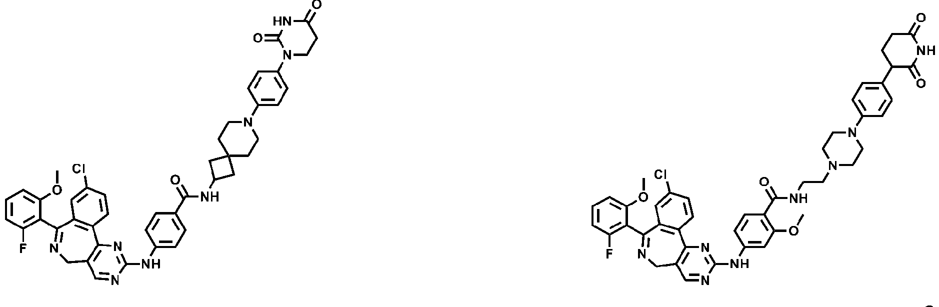

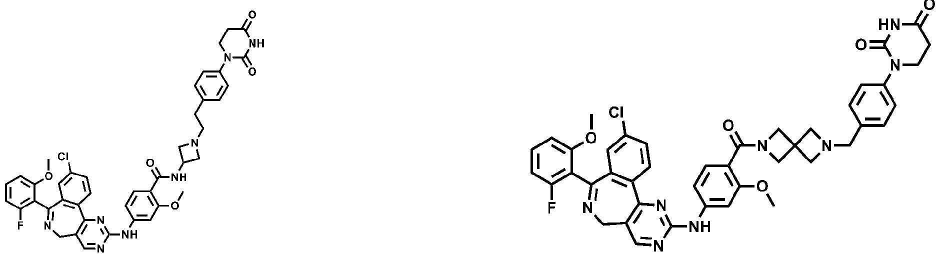

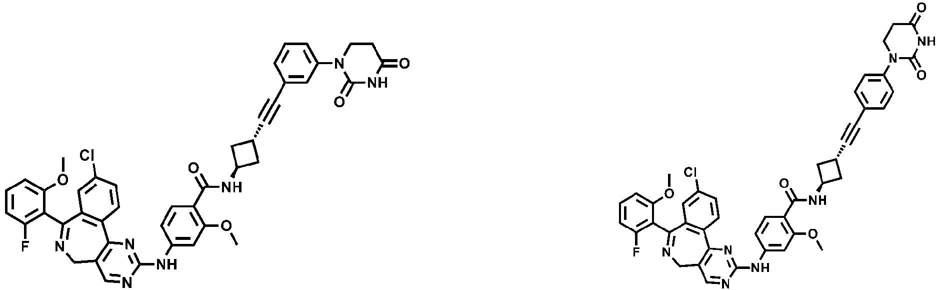

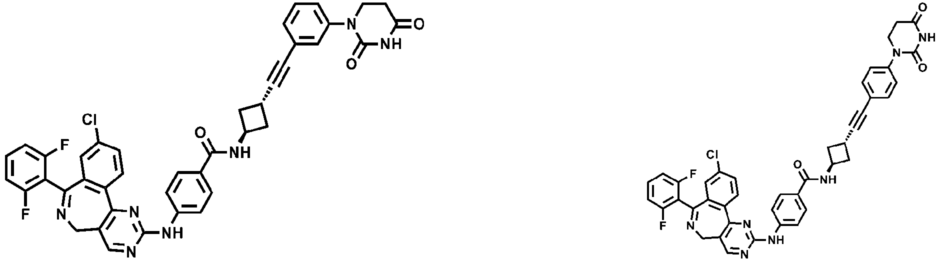

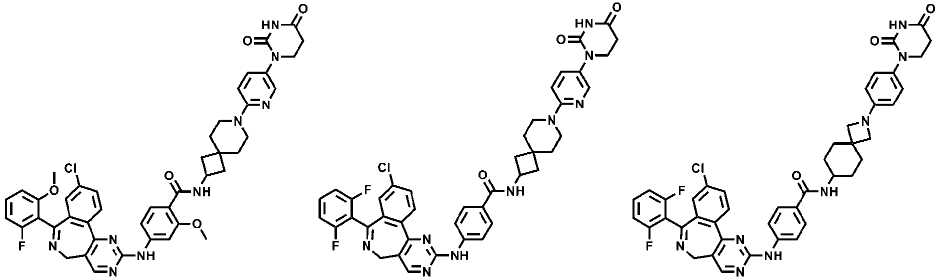

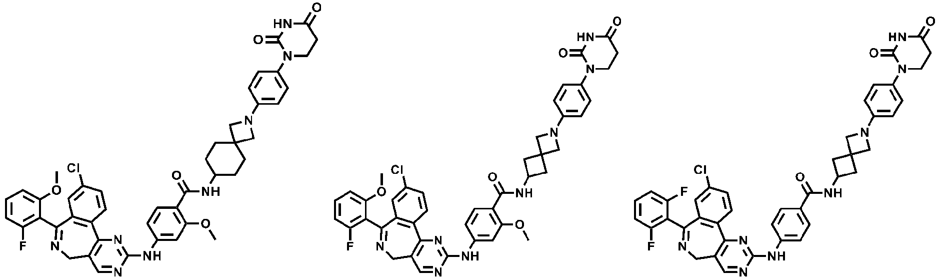

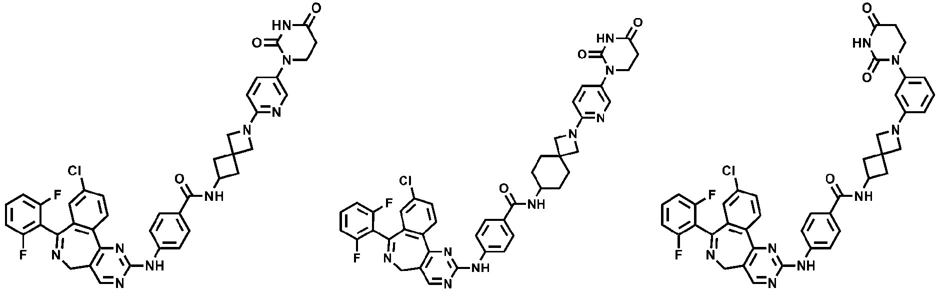

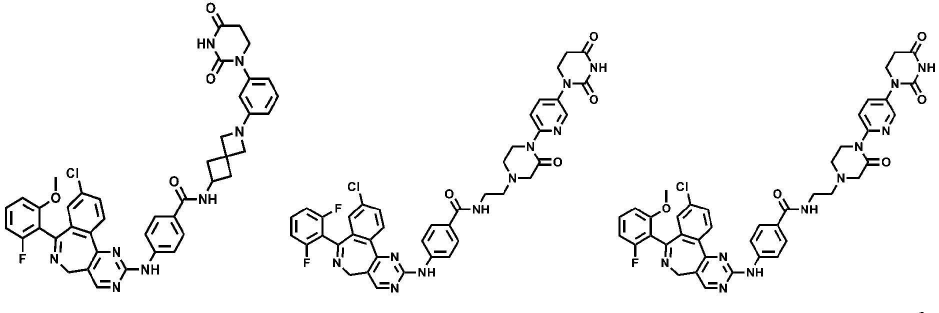

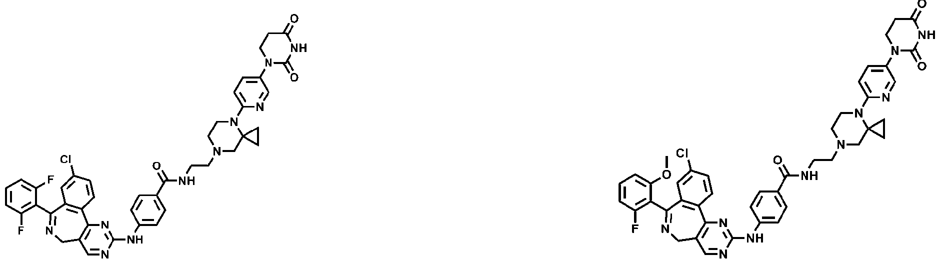

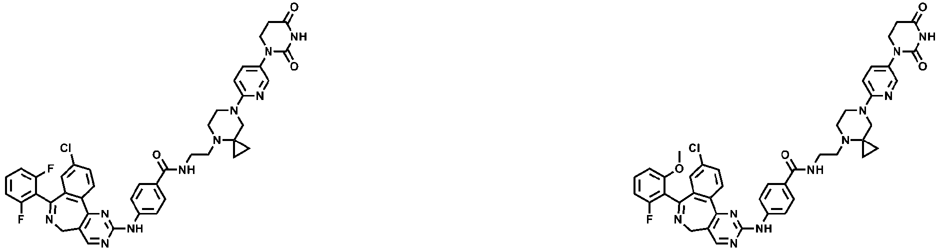

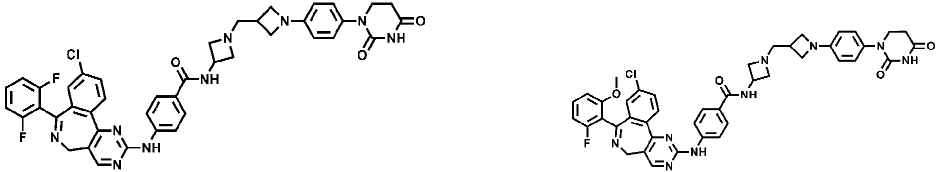

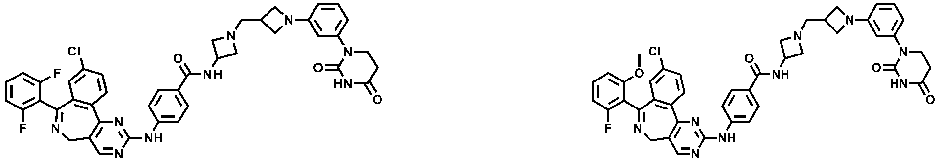

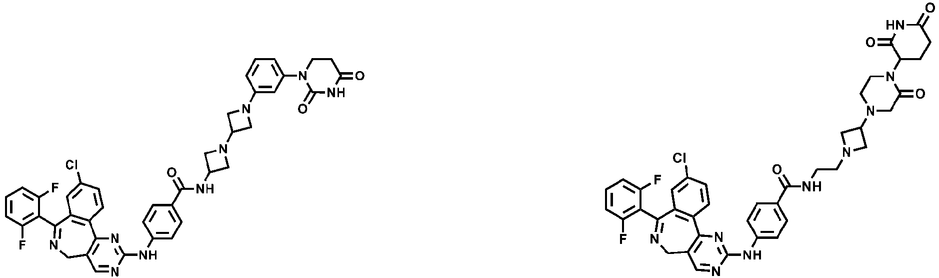

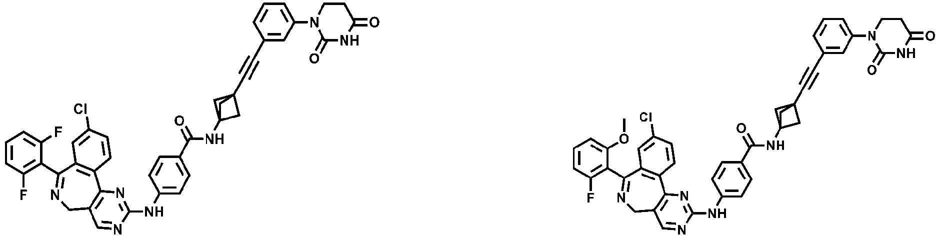

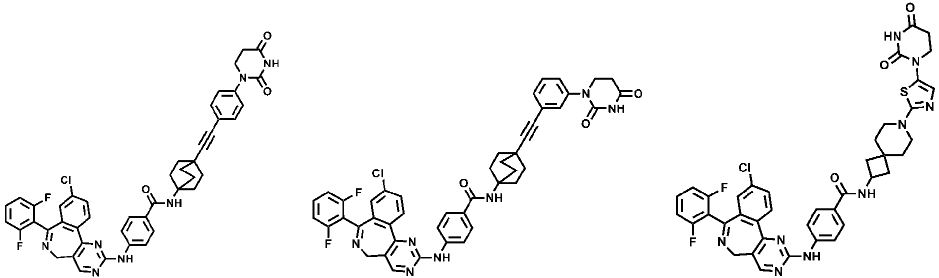

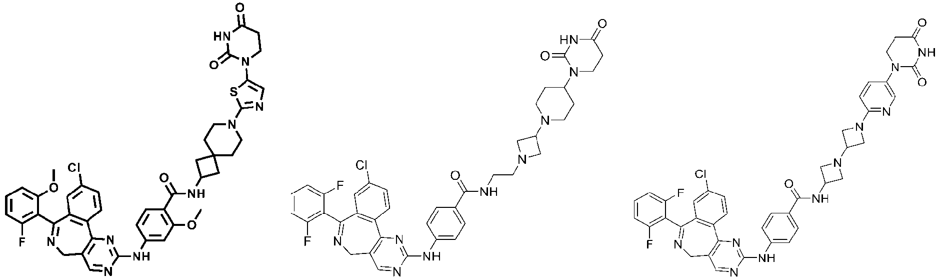

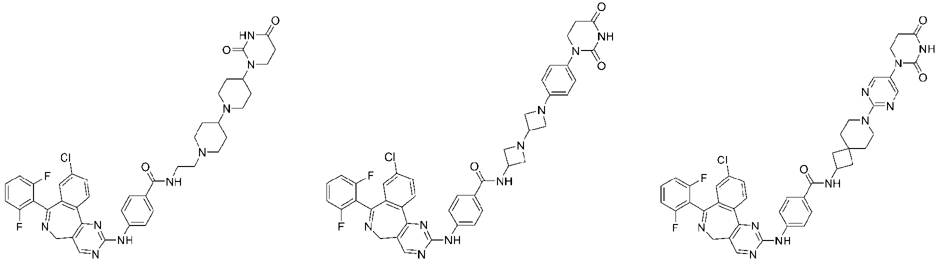

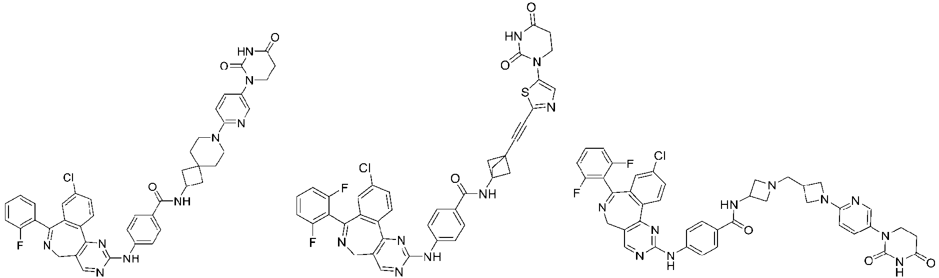

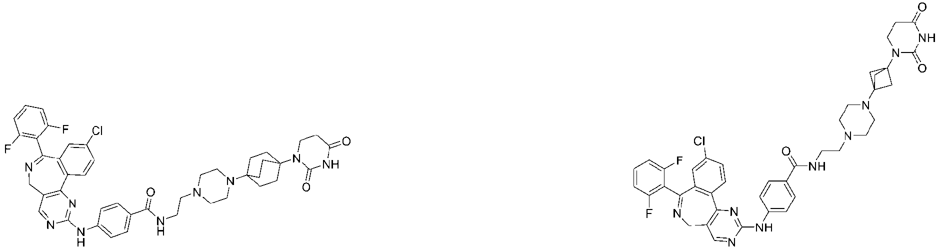

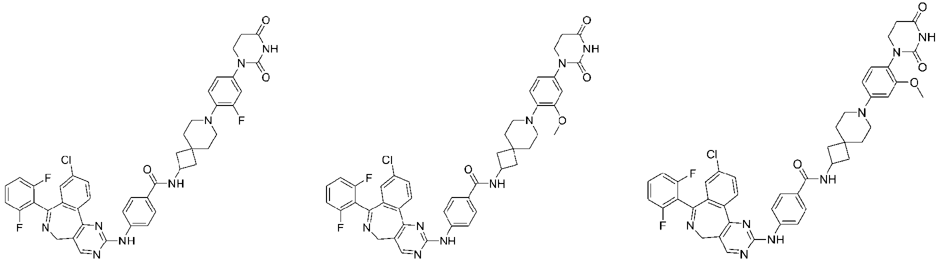

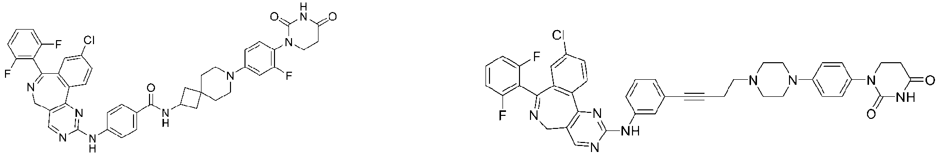

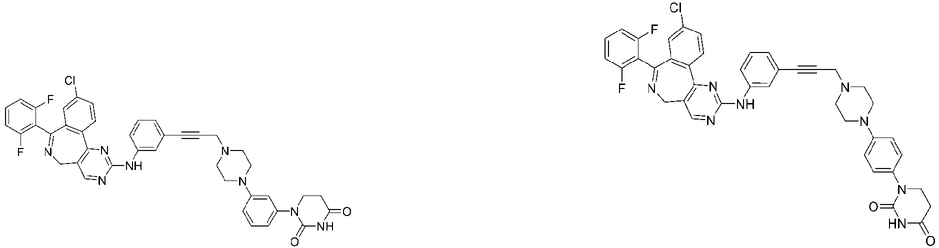

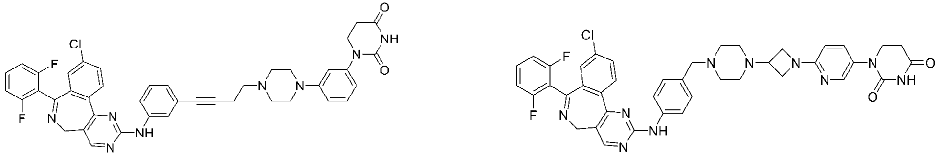

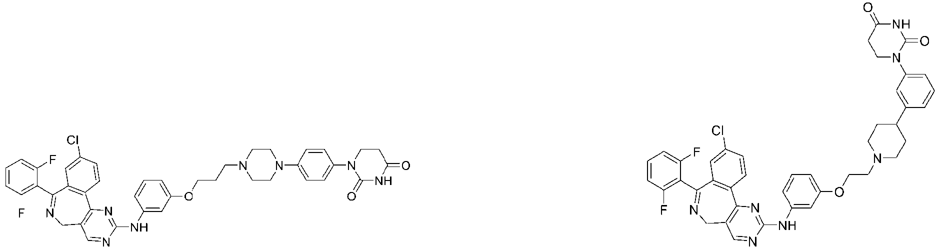

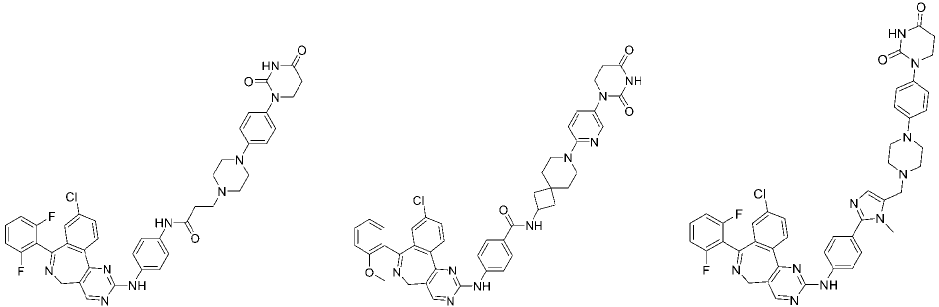

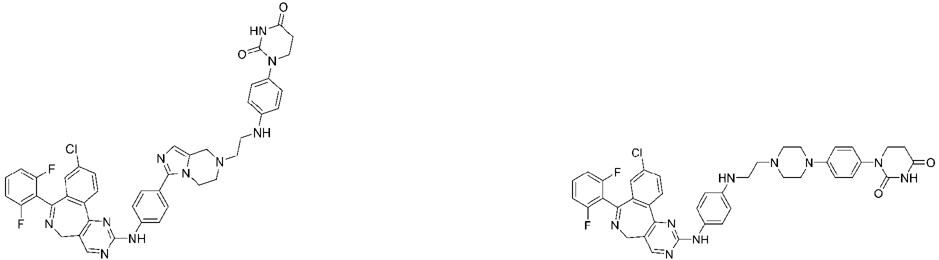

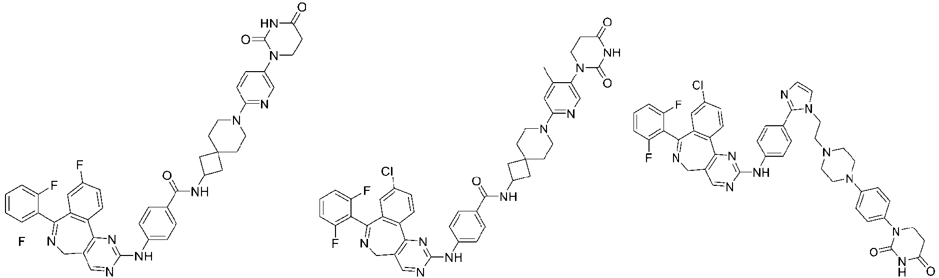

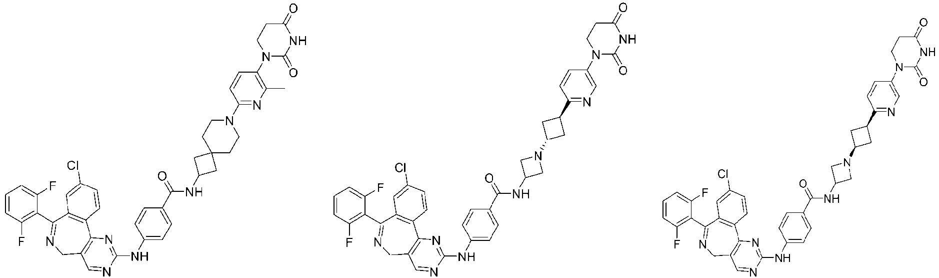

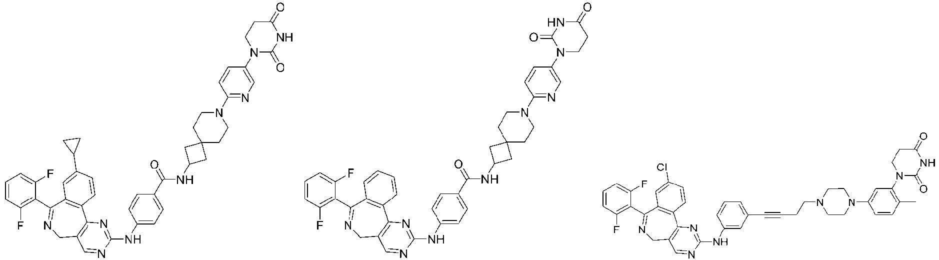

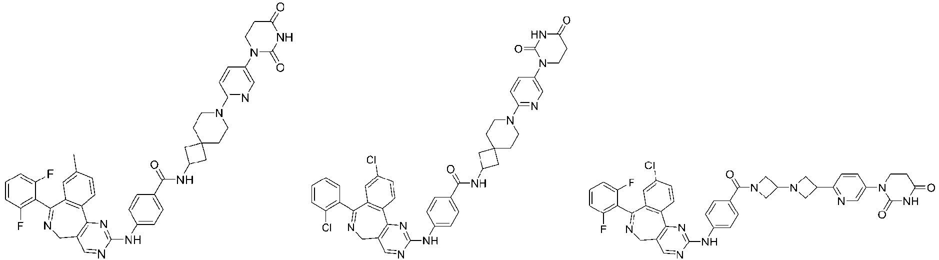

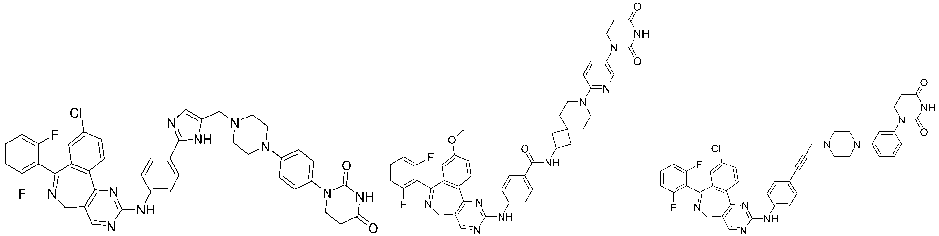

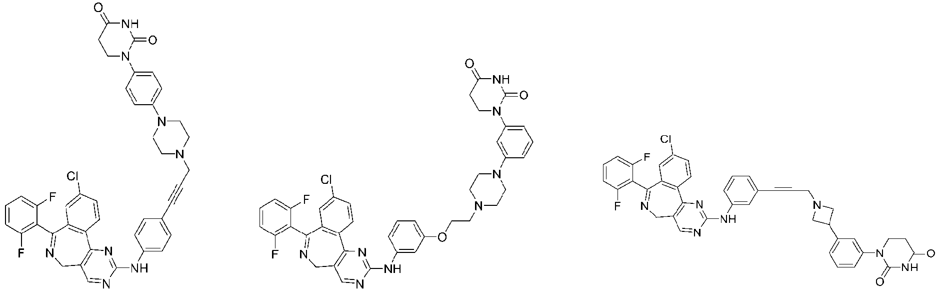

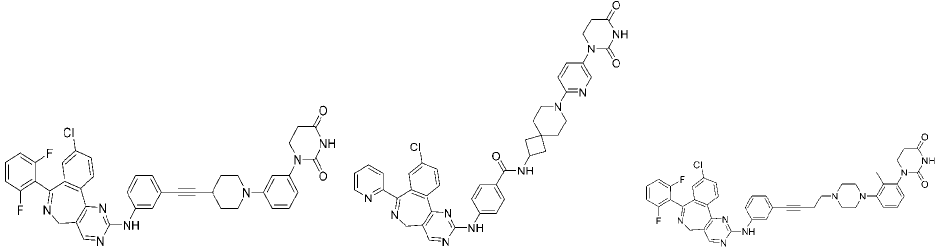

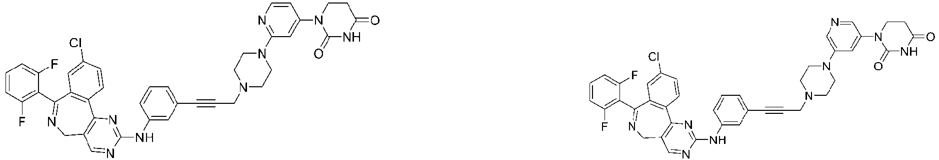

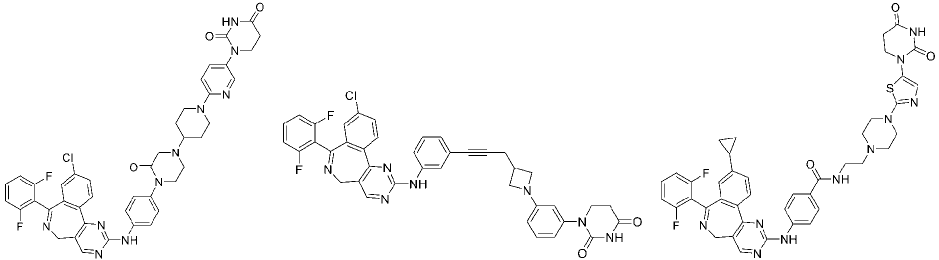

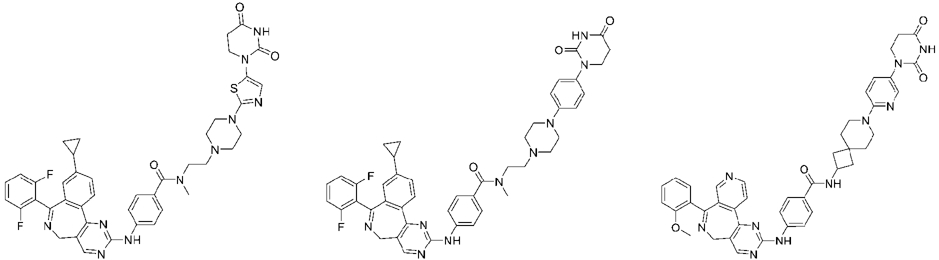

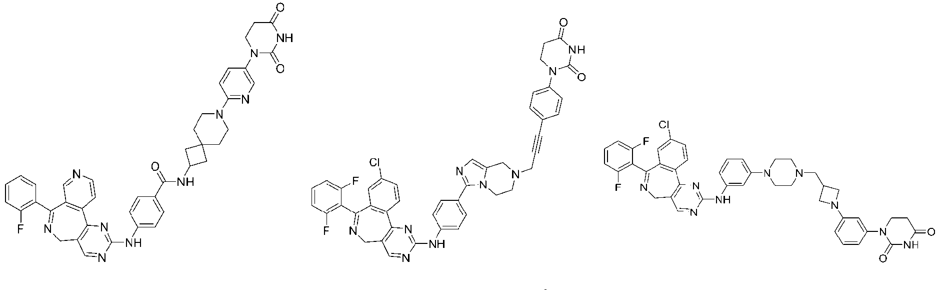

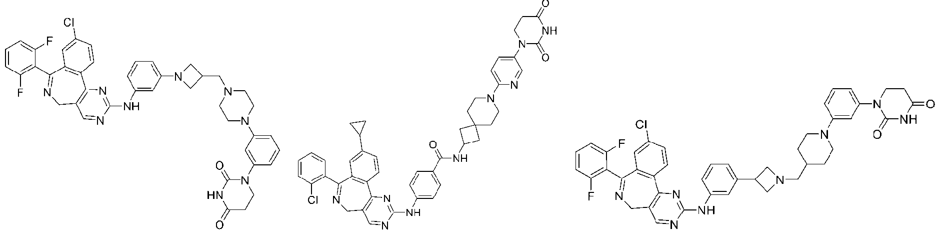

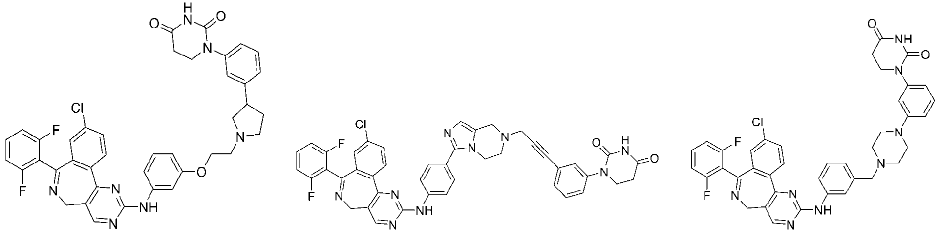

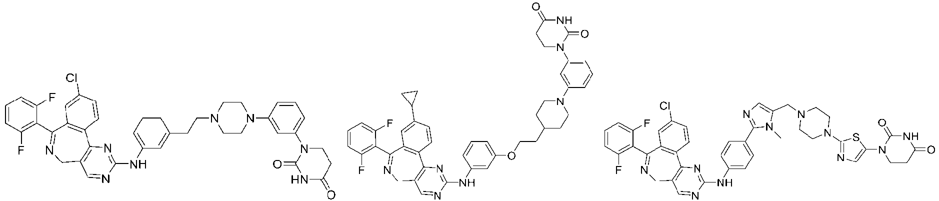

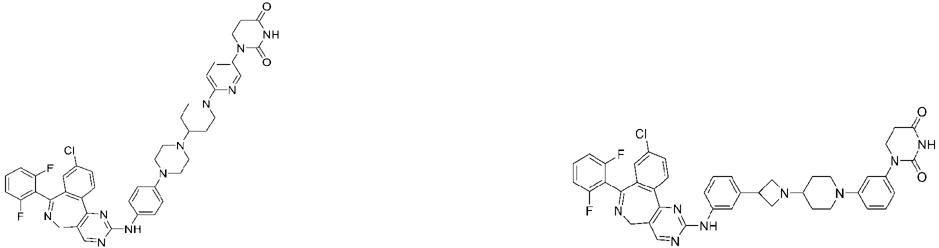

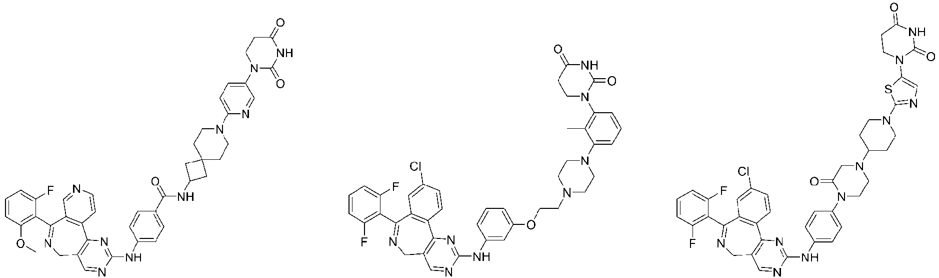

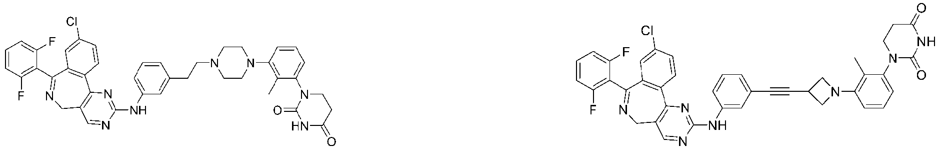

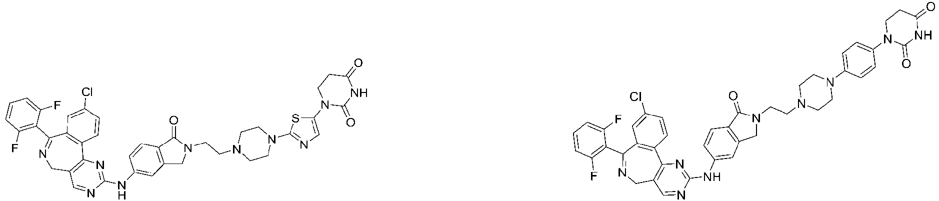

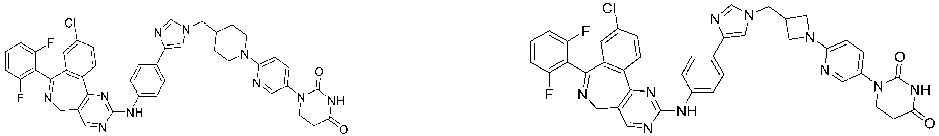

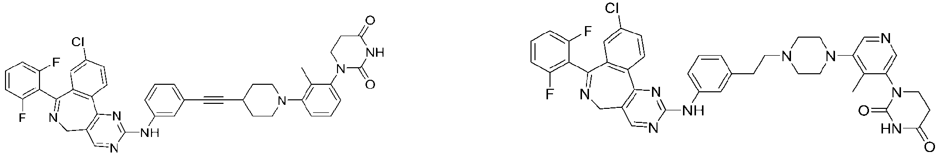

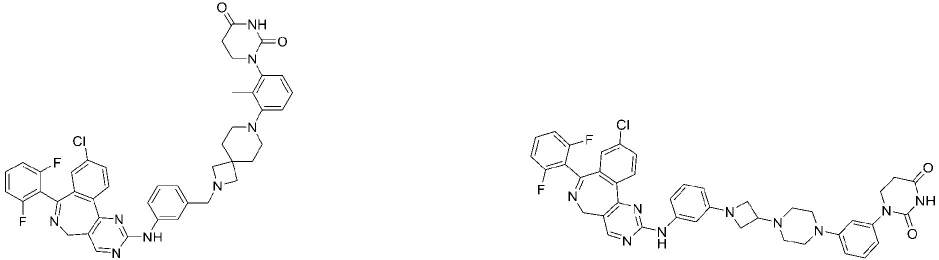

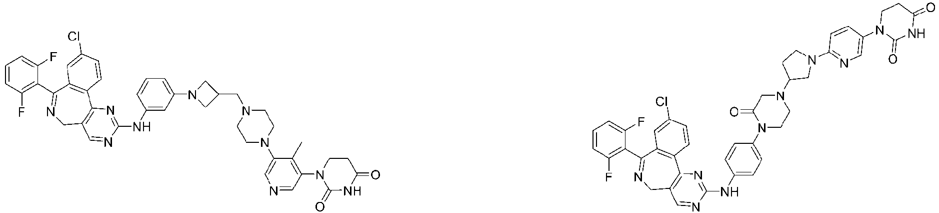

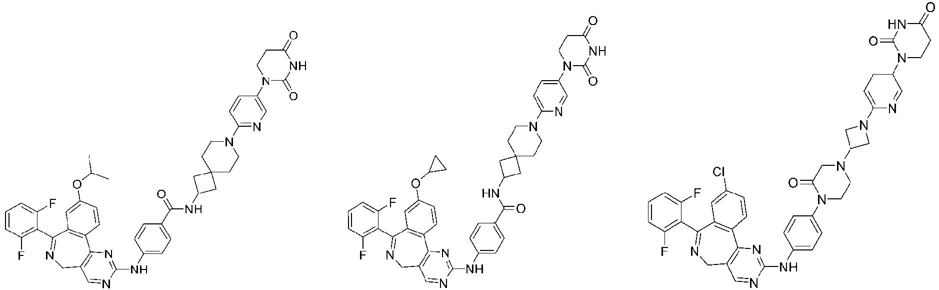

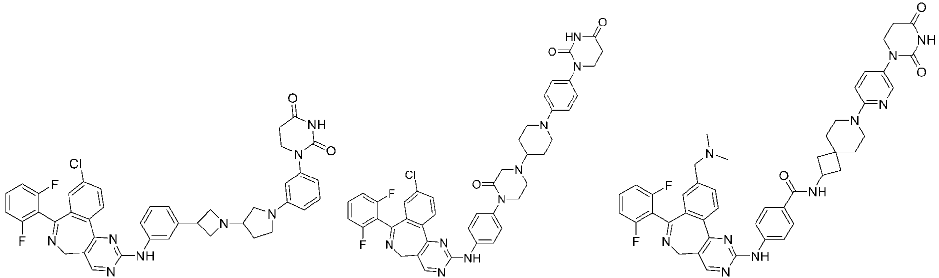

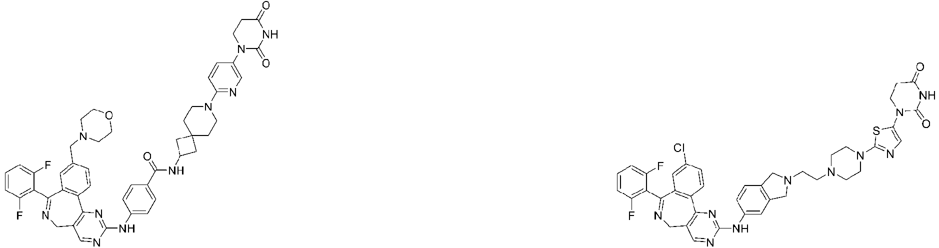

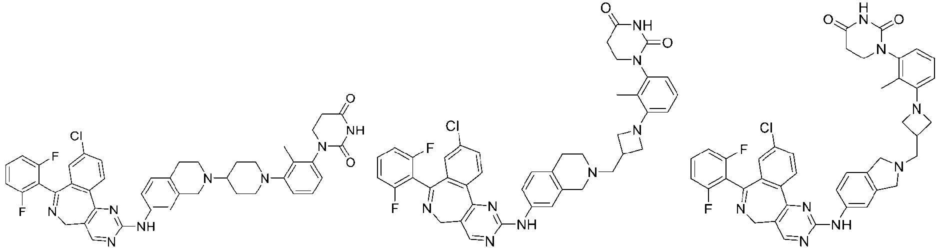

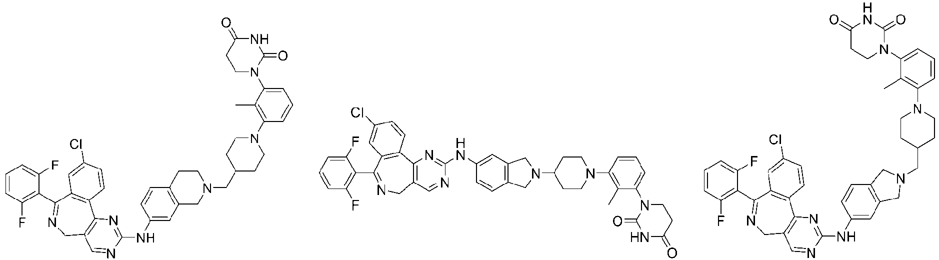

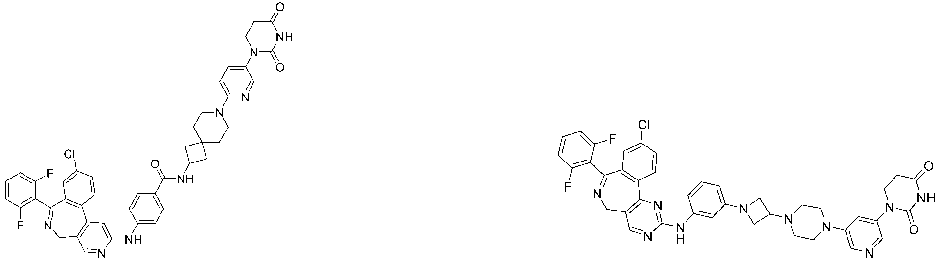

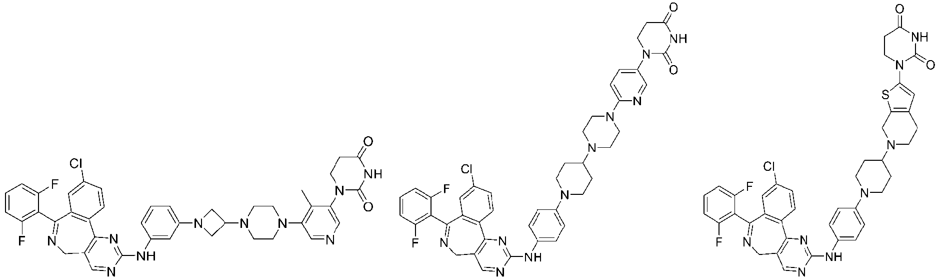

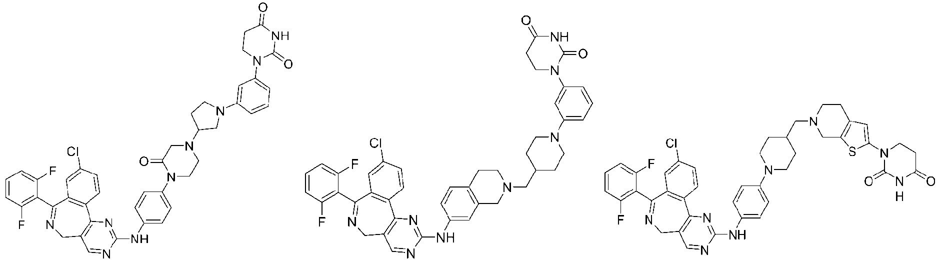

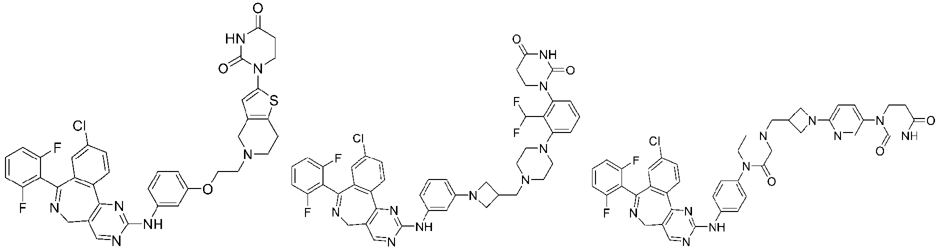

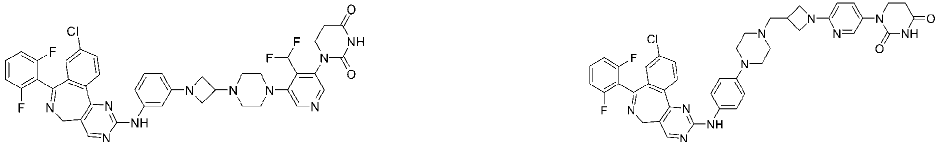

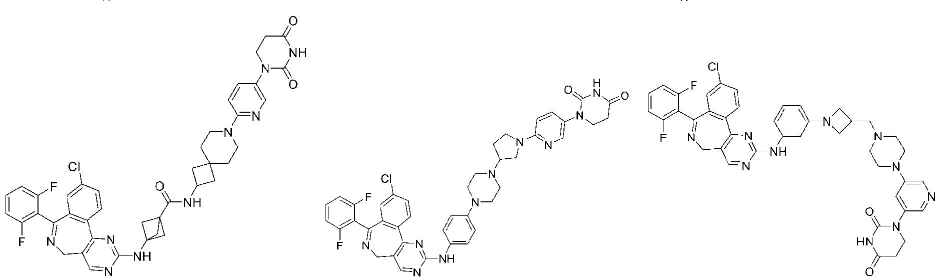

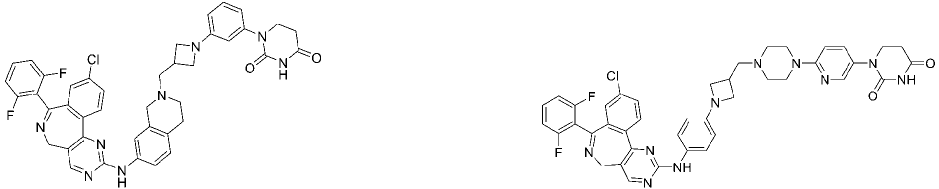

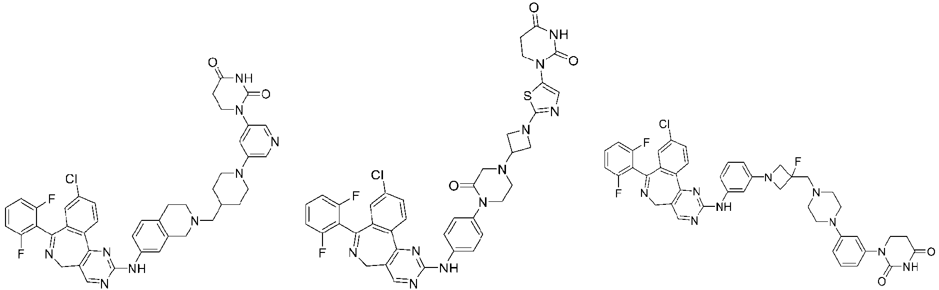

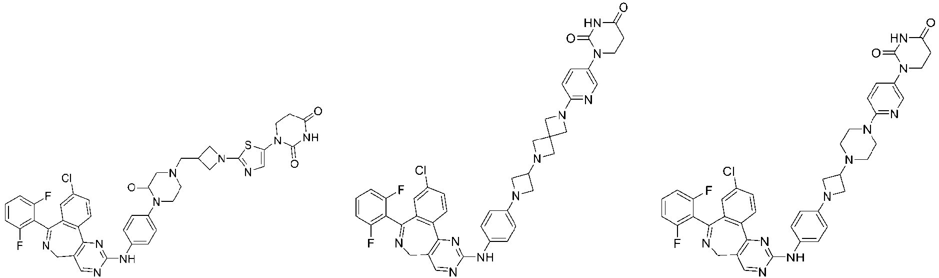

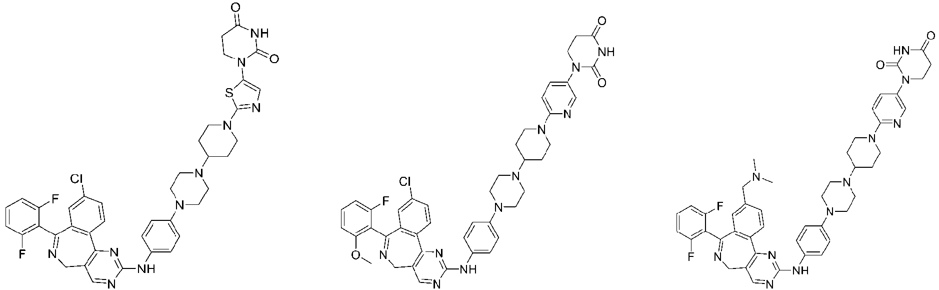

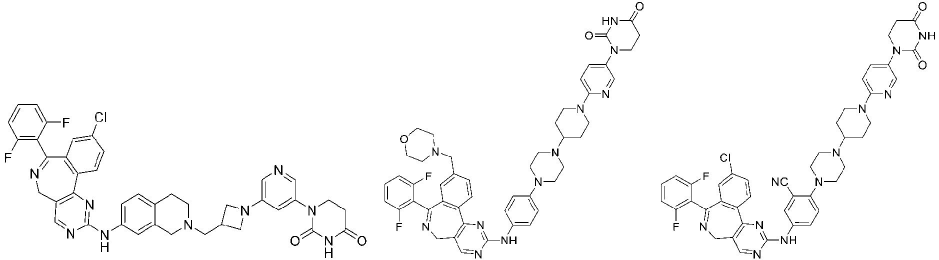

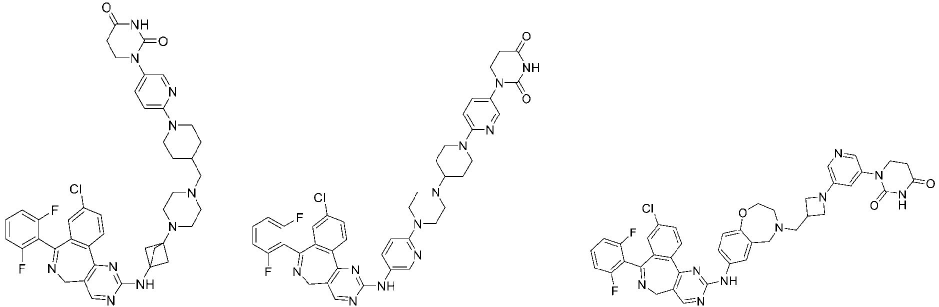

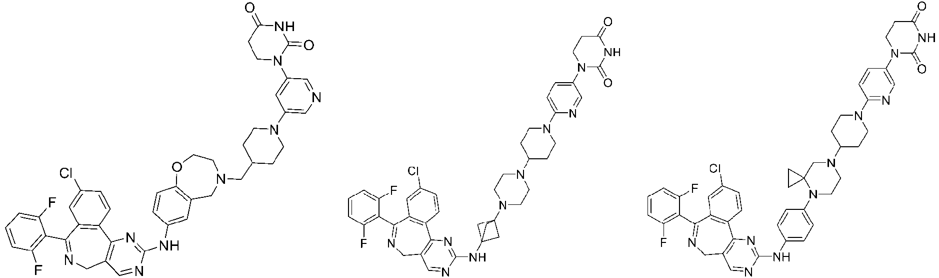

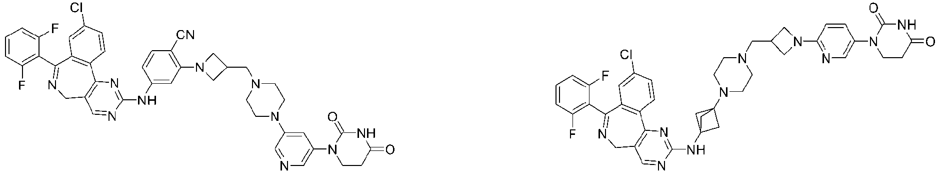

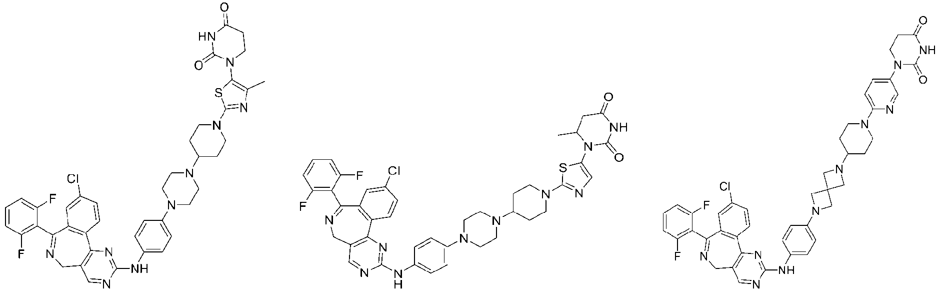

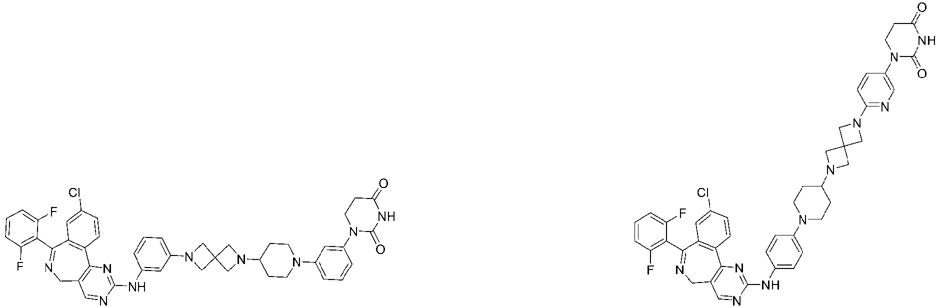

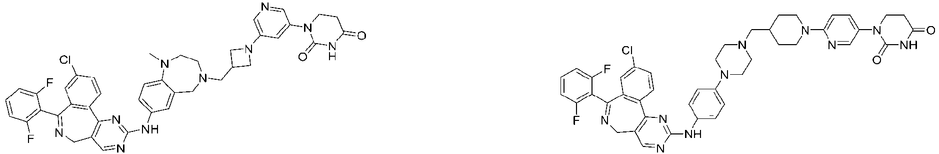

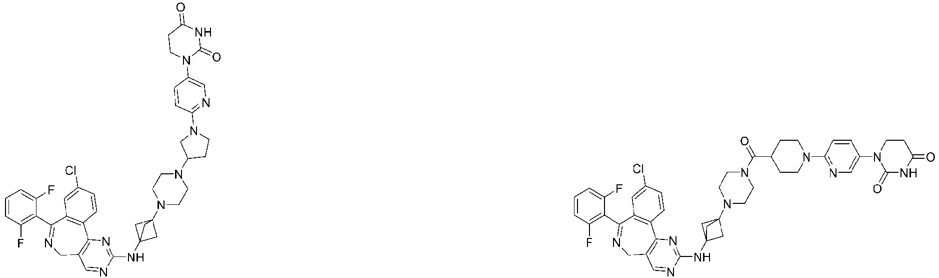

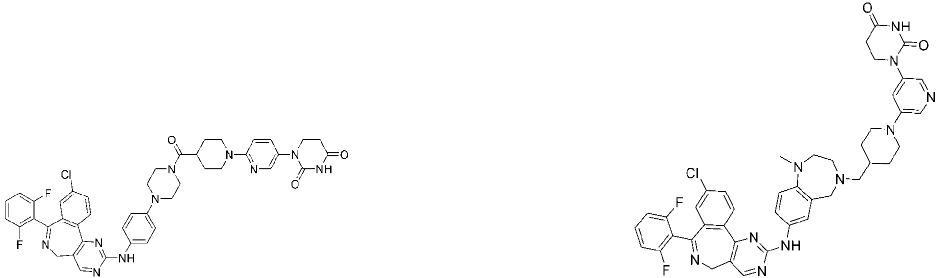

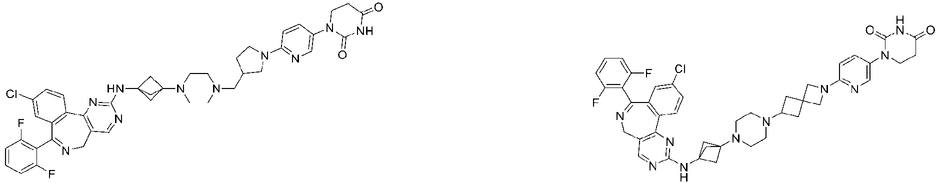

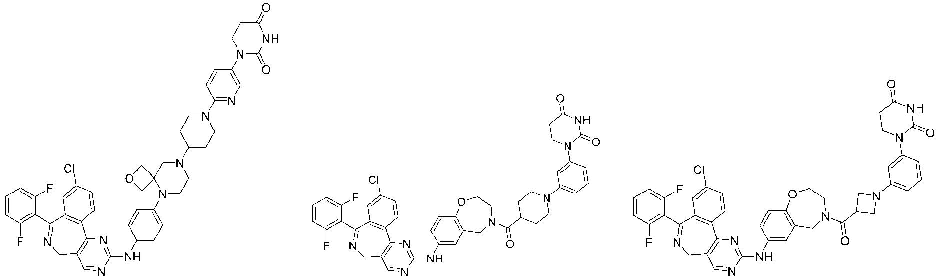

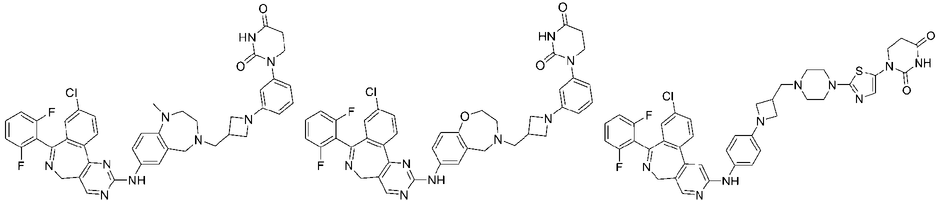

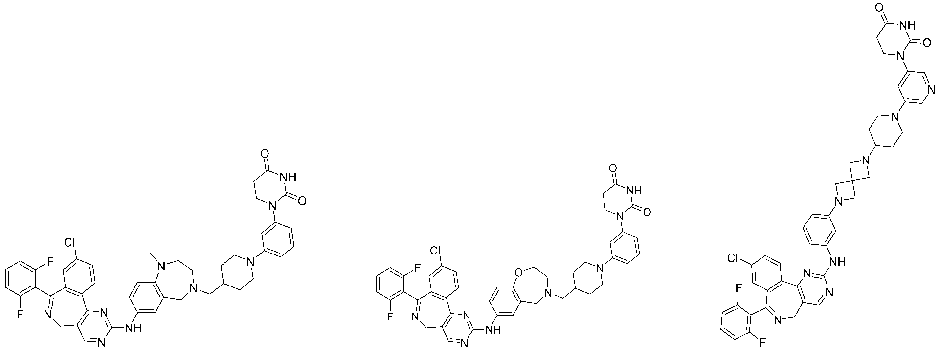

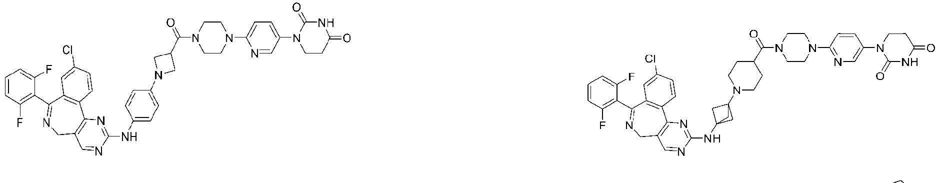

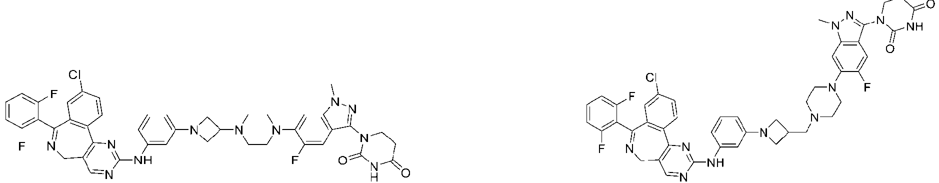

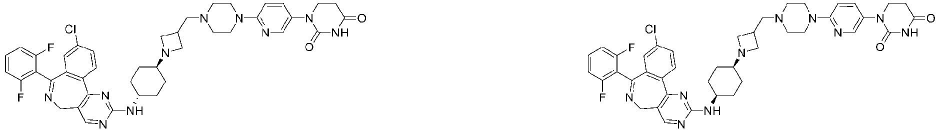

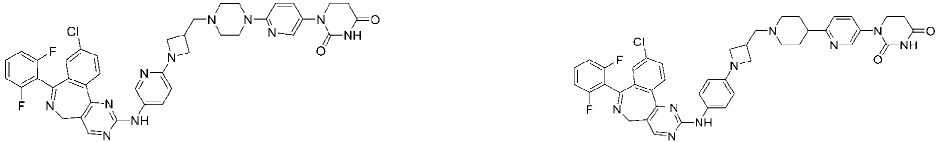

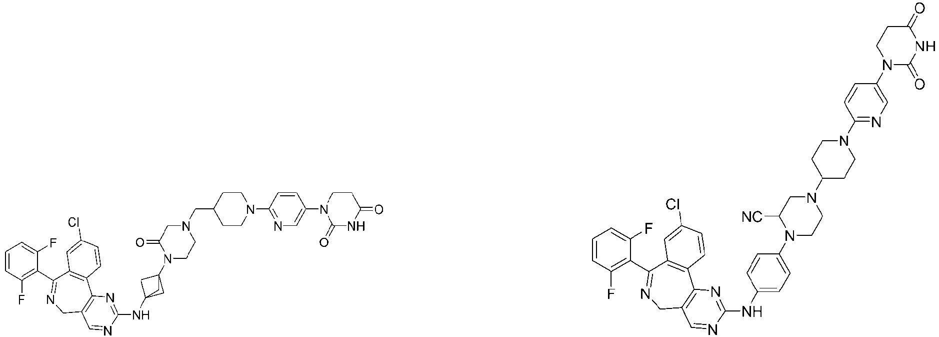

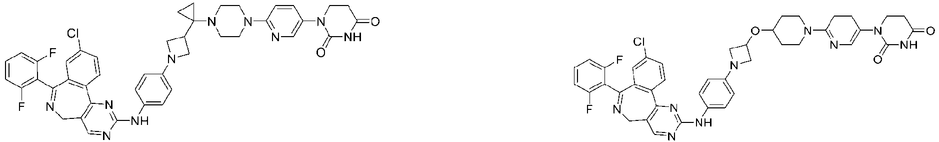

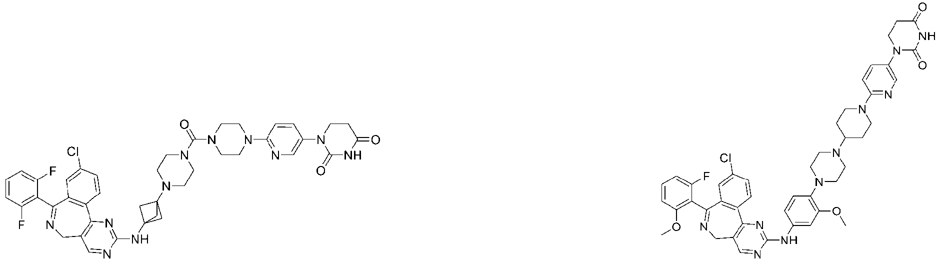

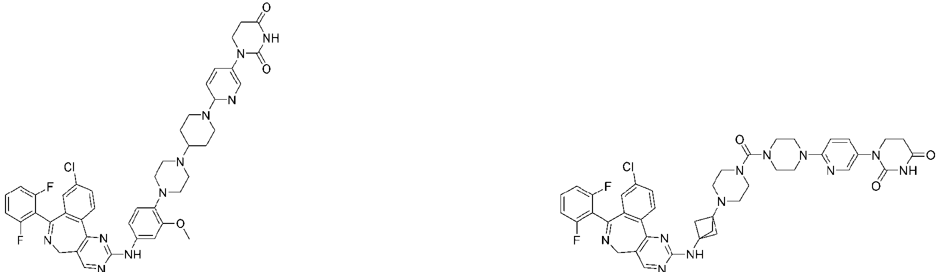

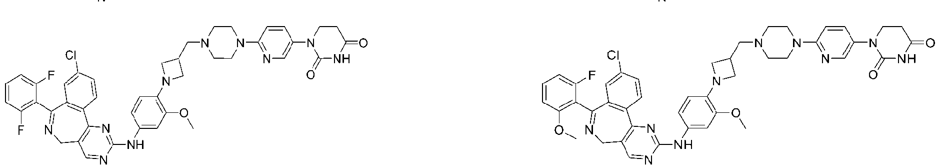

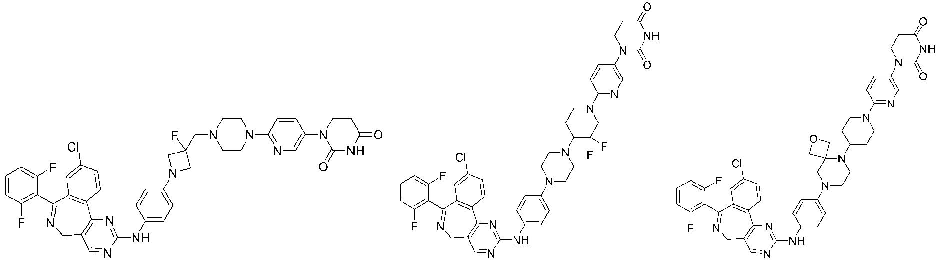

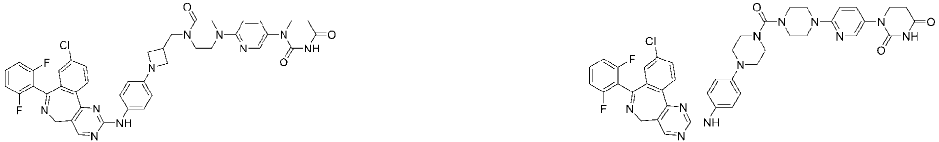

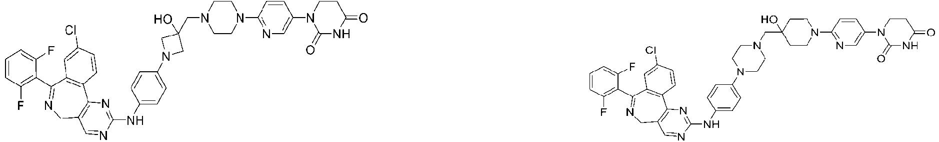

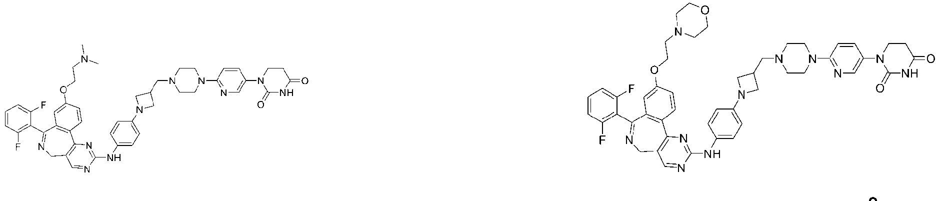

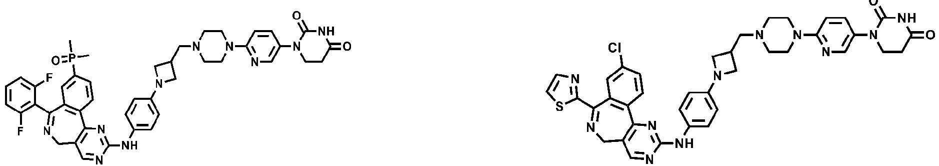

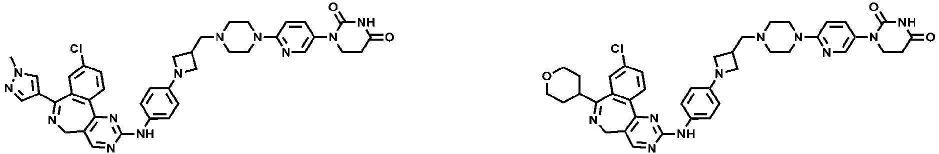

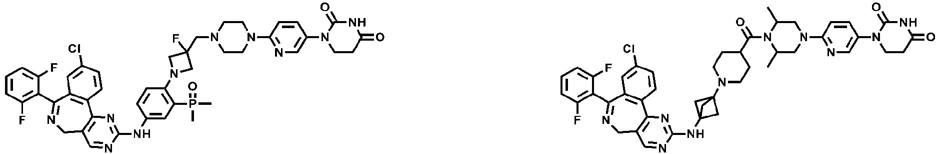

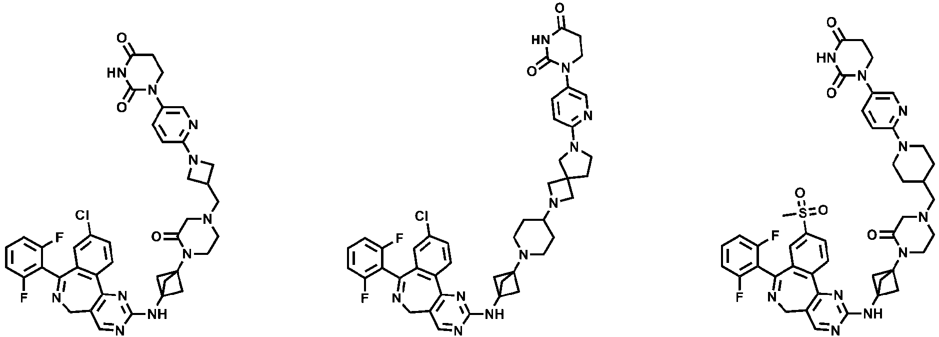

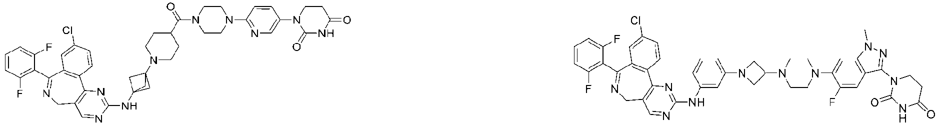

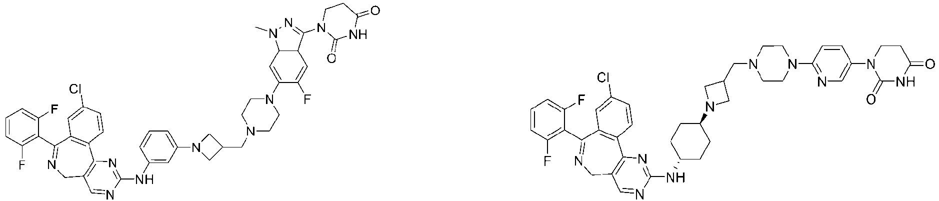

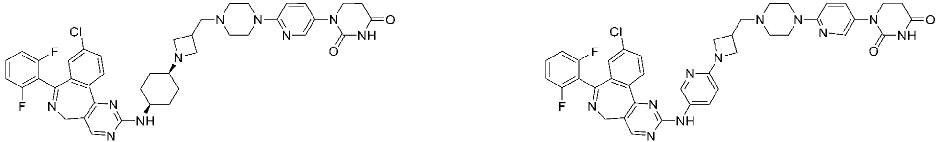

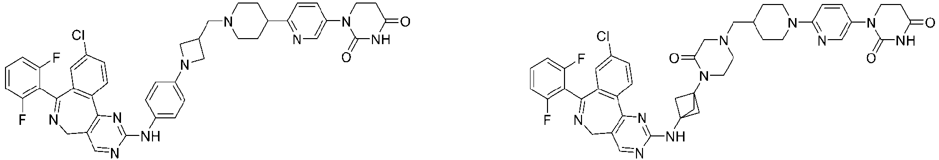

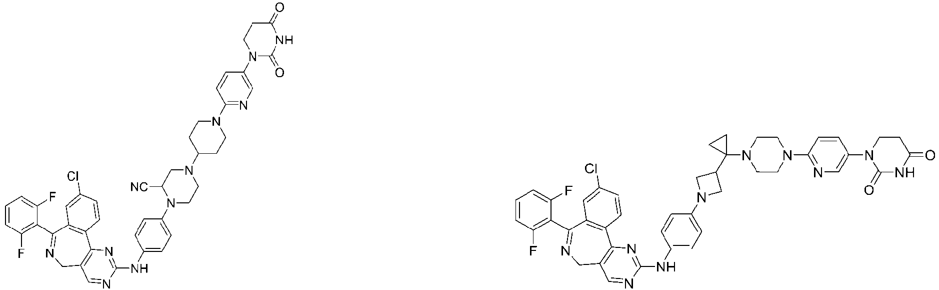

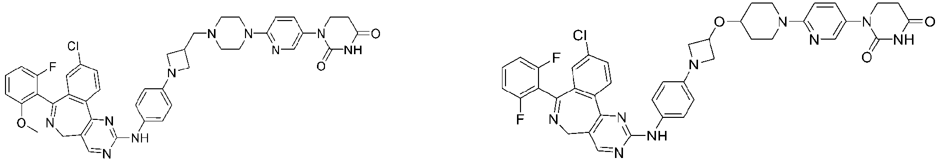

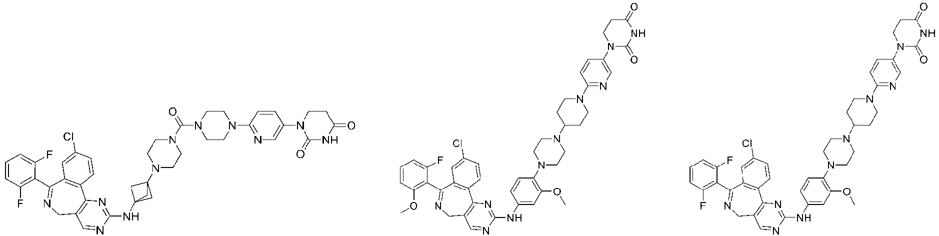

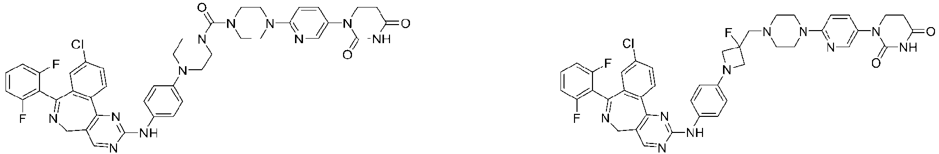

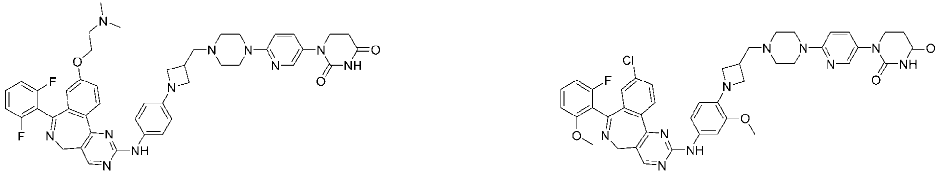

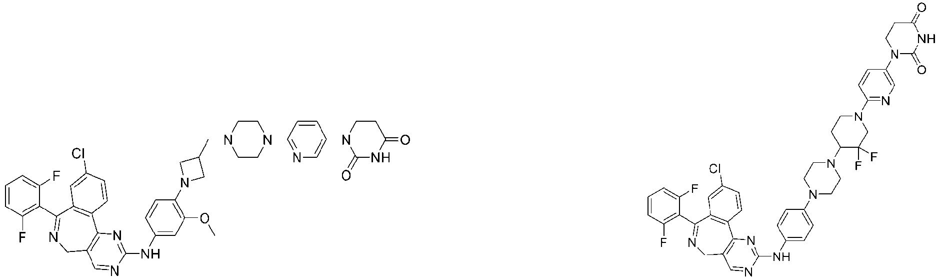

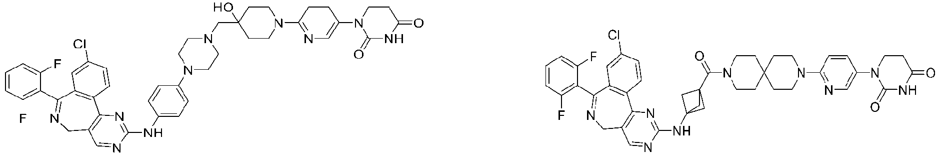

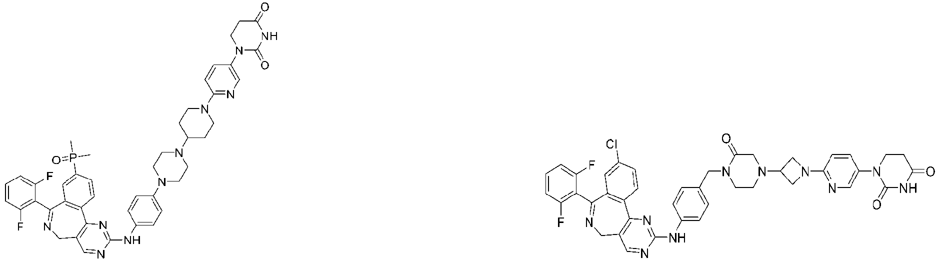

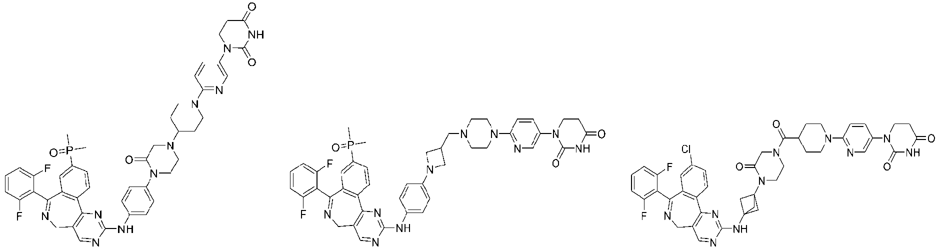

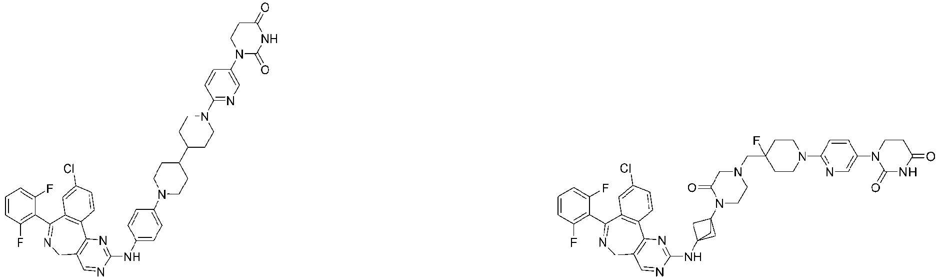

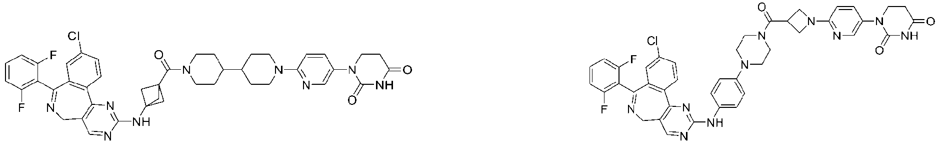

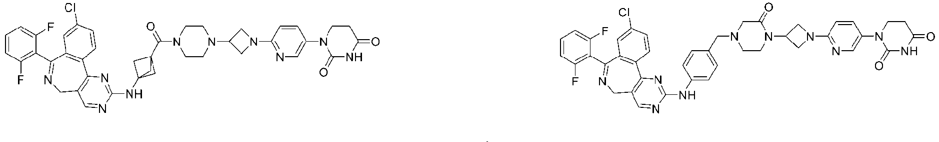

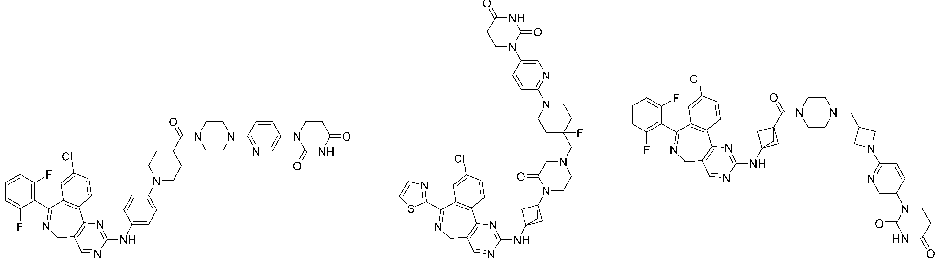

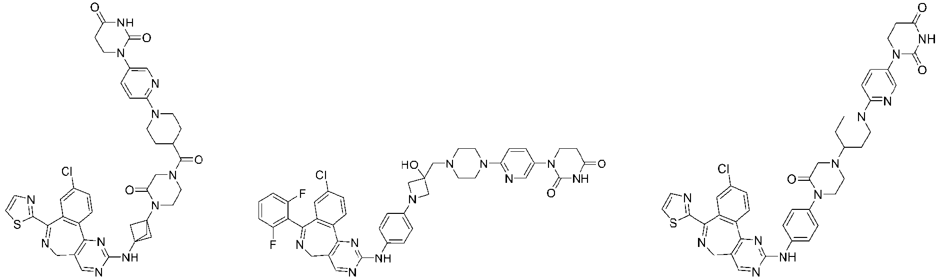

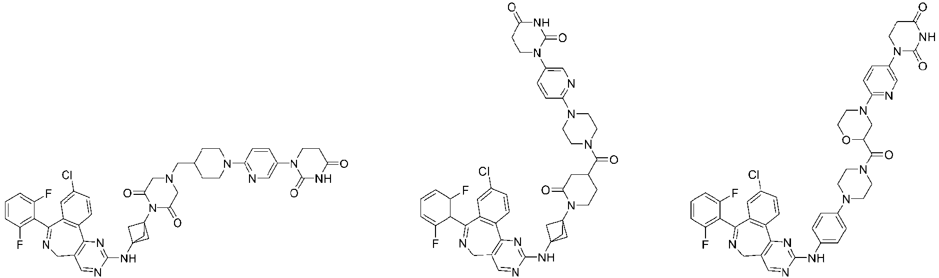

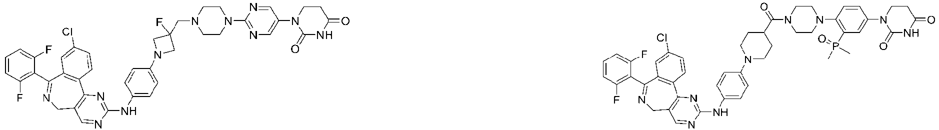

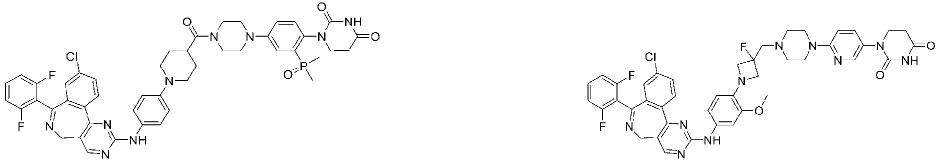

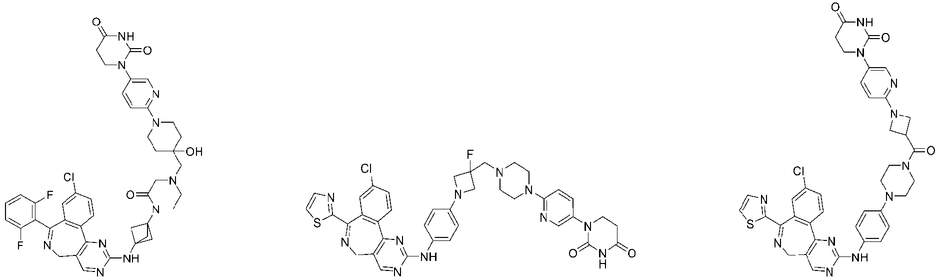

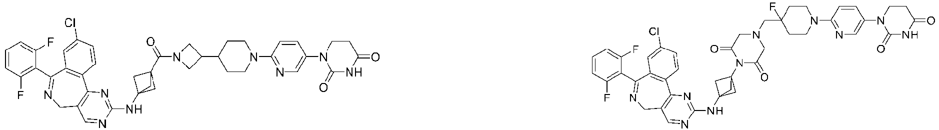

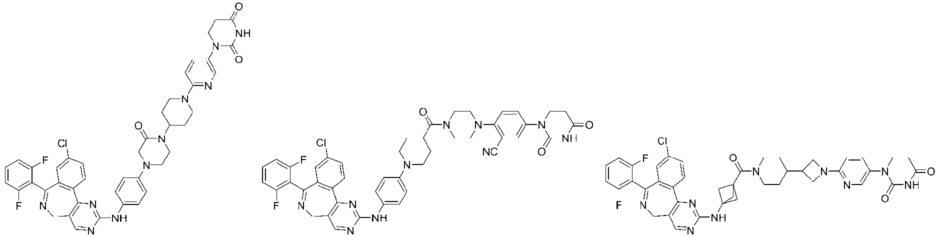

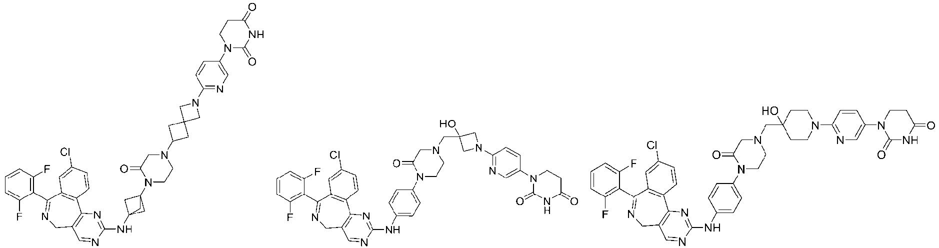

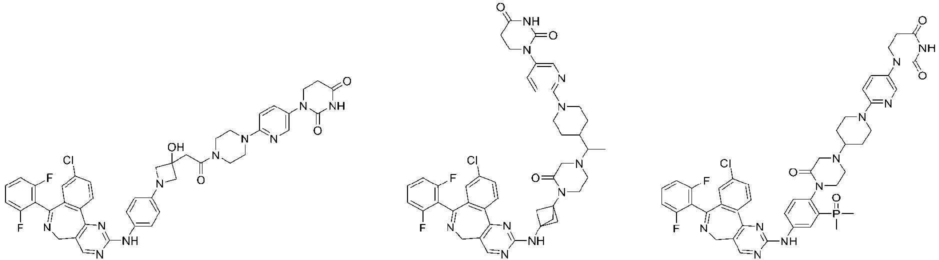

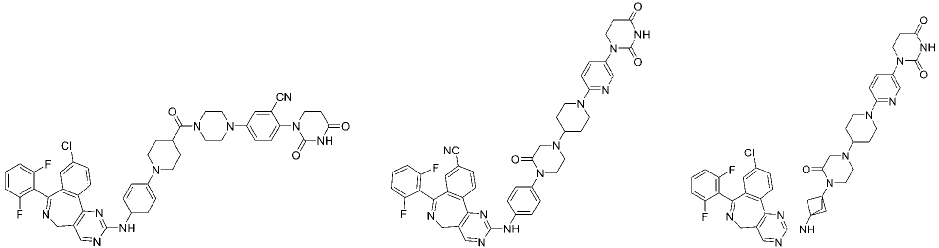

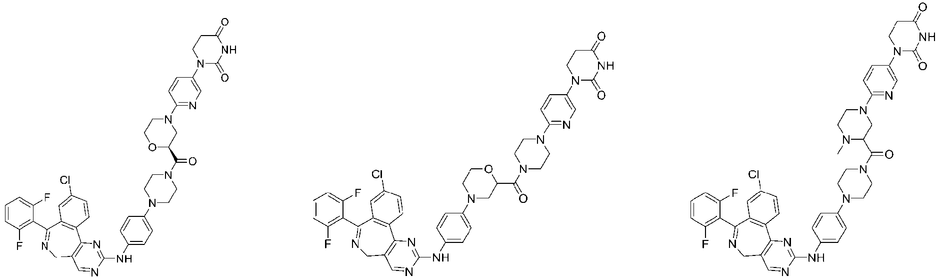

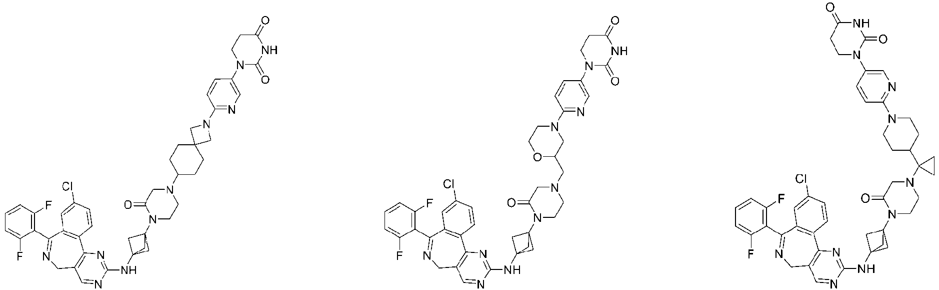

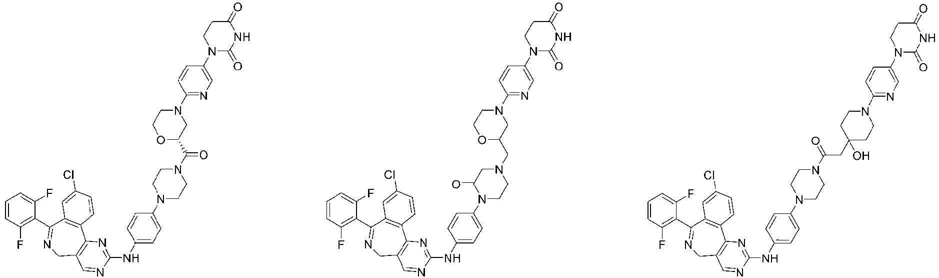

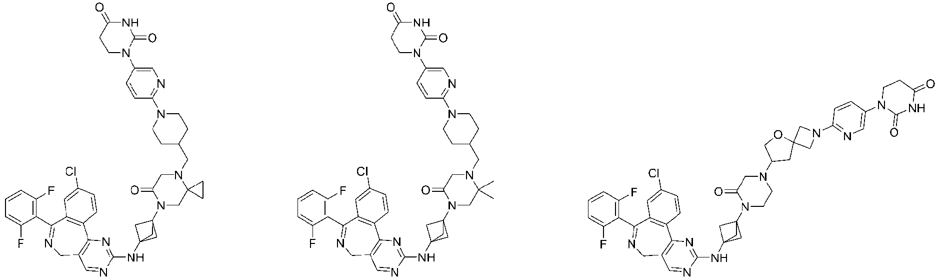

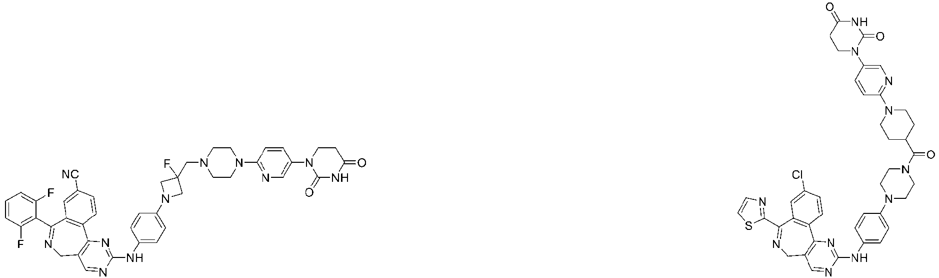

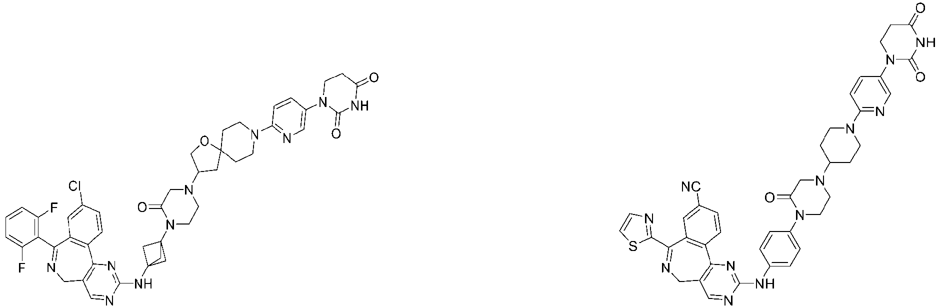

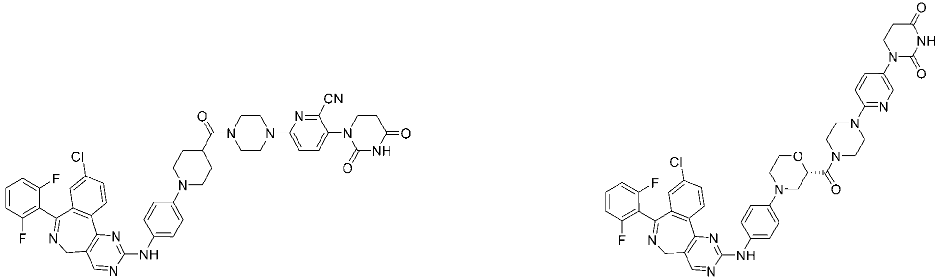

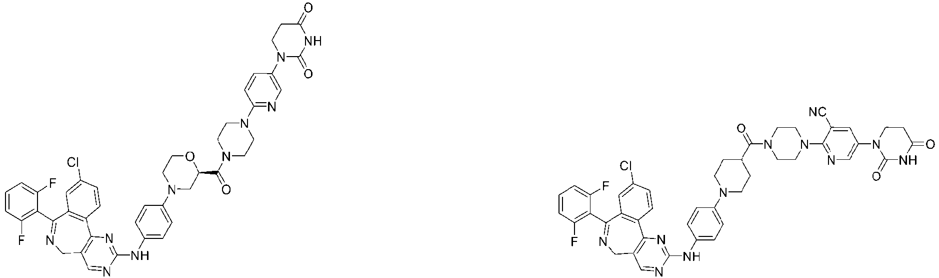

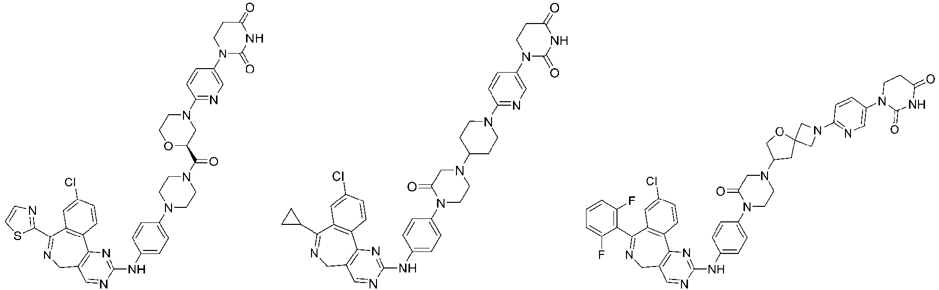

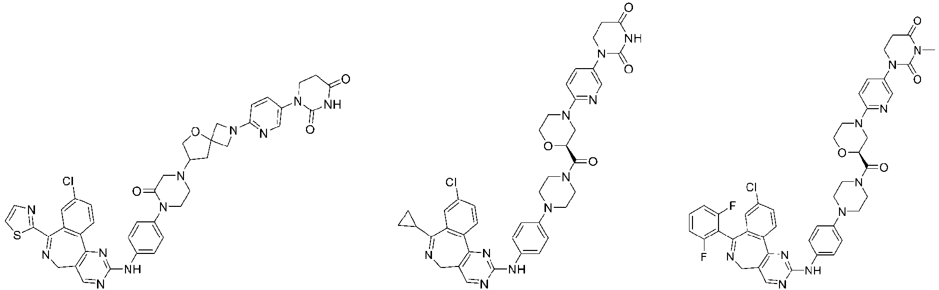

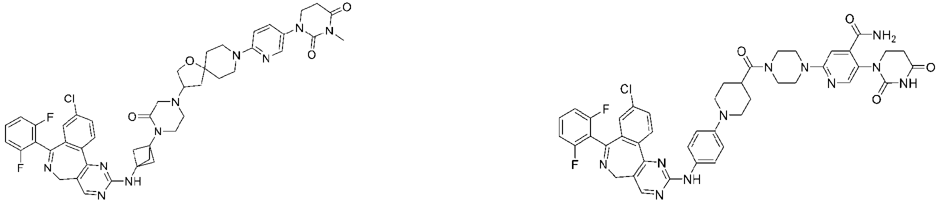

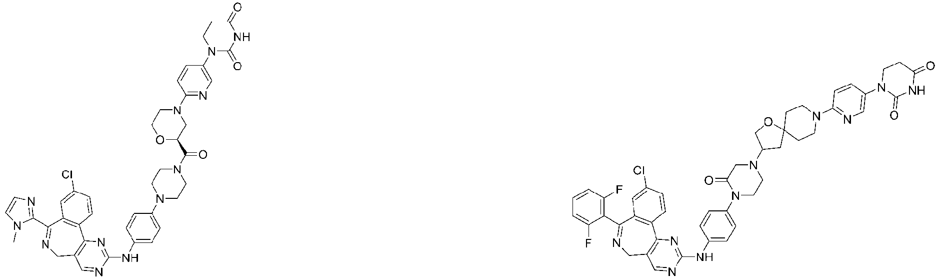

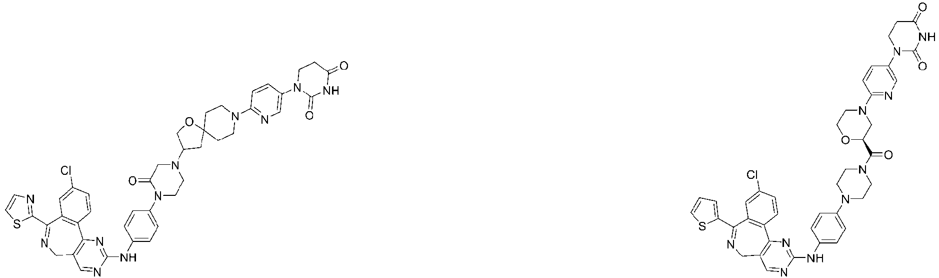

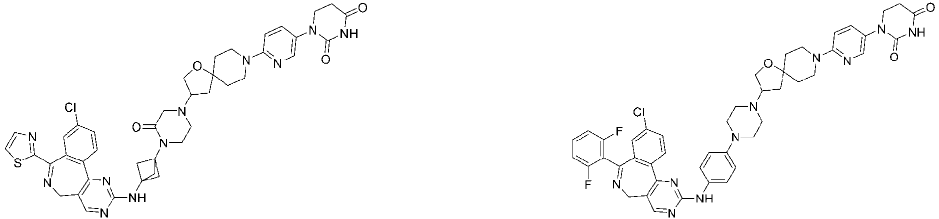

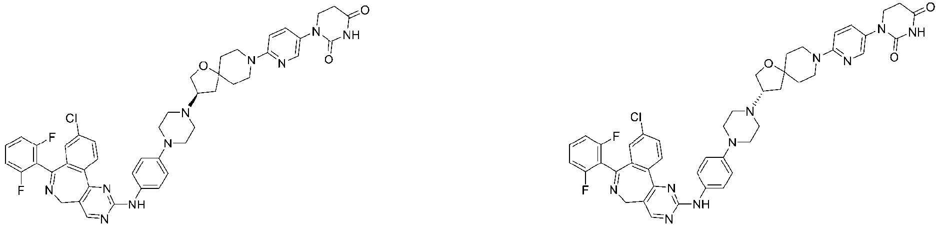

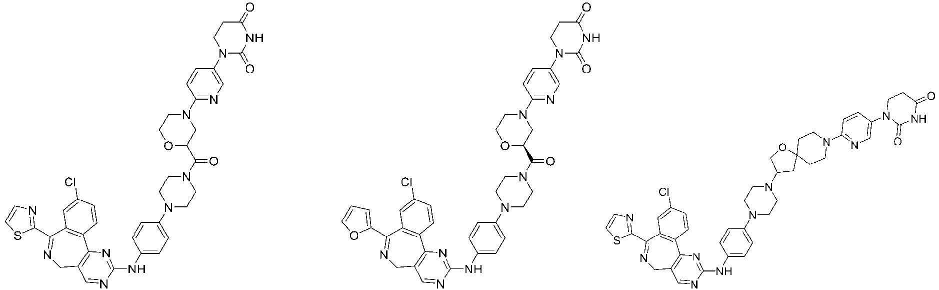

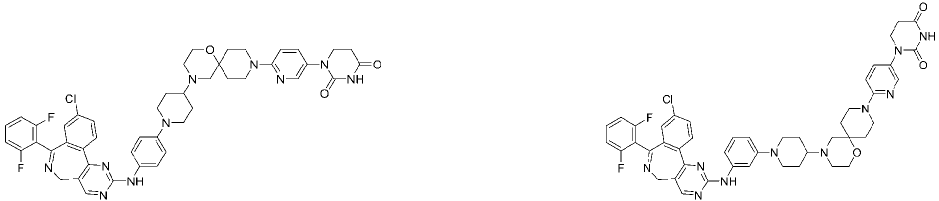

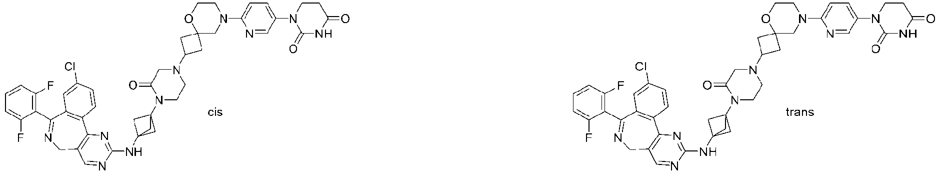

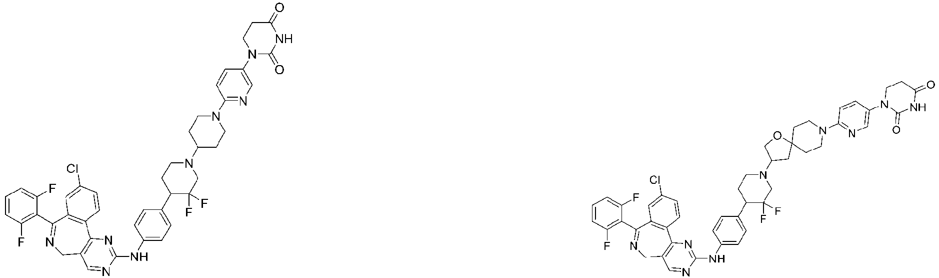

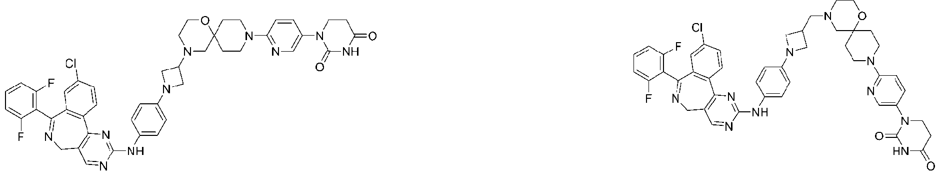

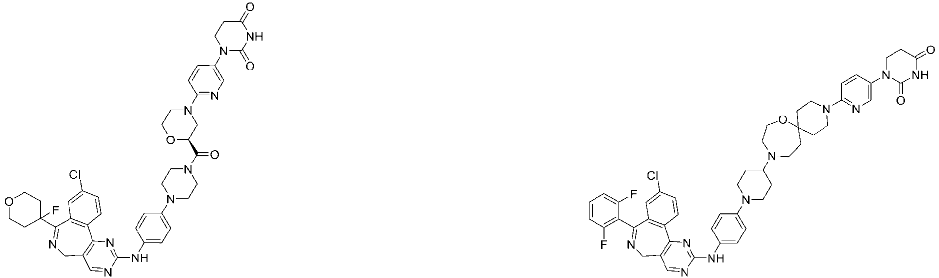

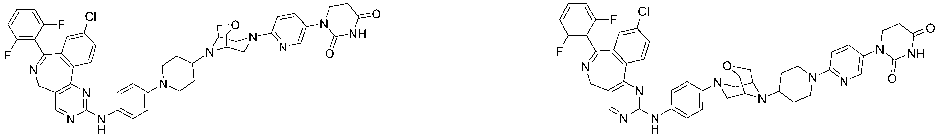

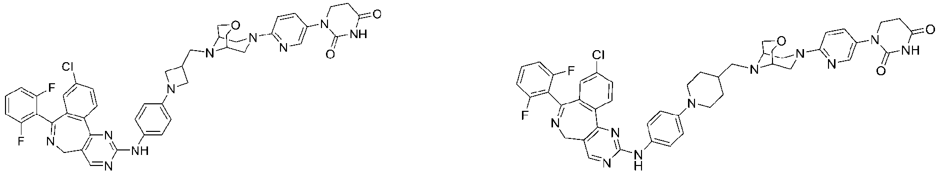

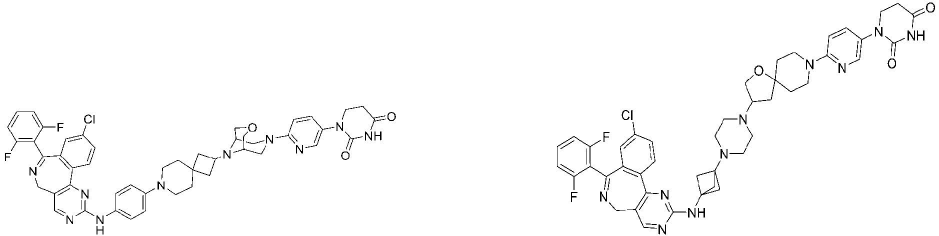

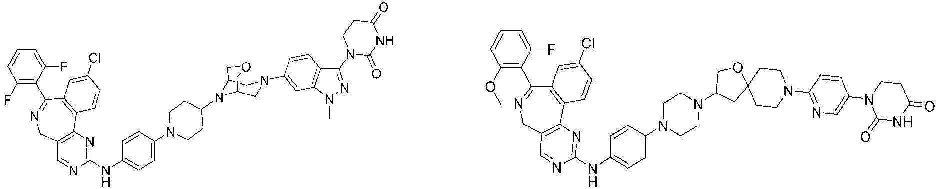

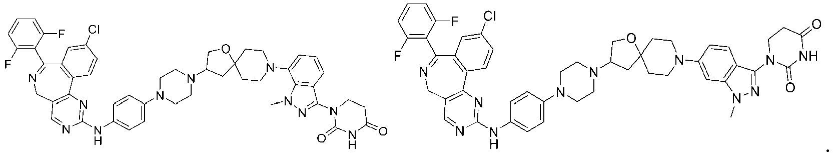

17. A pharmaceutical composition, comprising the compound represented by formula (I), the pharmaceutically acceptable salt thereof, the solvate thereof or the solvate of the pharmaceutically acceptable salt thereof according to any one of claims 1-16, and pharmaceutically acceptable excipients.

18. Use of the compound represented by formula (I), the pharmaceutically acceptable salt thereof, the solvate thereof, the solvate of the pharmaceutically acceptable salt thereof according to any one of claims 1 to 16, or the pharmaceutical composition according to claim 17 in the preparation of an AURKA inhibitor or degrader.

19. Use of the compound represented by formula (I), the pharmaceutically acceptable salt thereof, the solvate thereof, the solvate of the pharmaceutically acceptable salt thereof according to any one of claims 1 to 16, or the pharmaceutical composition according to claim 17 in the preparation of drugs for treating and/or preventing diseases associated with AURKA; preferably, the disease associated with AURKA is neuroblastoma, retinoblastoma, colorectal cancer, breast cancer, lung cancer, kidney cancer or blood cancer.

20. Use of the compound represented by formula (I), the pharmaceutically acceptable salt thereof, the solvate thereof, the solvate of the pharmaceutically acceptable salt thereof according to any one of claims 1 to 16, or the pharmaceutical composition according to claim 17 in the preparation of drugs for treating and/or preventing diseases mediated by AURKA inhibitors or degraders; preferably, the disease mediated by AURKA inhibitors or degraders is neuroblastoma, retinoblastoma, colorectal cancer, breast cancer, lung cancer, kidney cancer or blood cancer.

21. Use of the compound represented by formula (I), the pharmaceutically acceptable salt thereof, the solvate thereof, the solvate of the pharmaceutically acceptable salt thereof according to any one of claims 1 to 16, or the pharmaceutical composition according to claim 17 in the preparation of drugs for treating and/or preventing neuroblastoma, retinoblastoma, colorectal cancer, breast cancer, lung cancer, kidney cancer or blood cancer.
